(19) Europäisches Patentamt
European Patent Office
Office européen des brevets

(11) EP 2 093 233 A1

(12) **EUROPEAN PATENT APPLICATION**

(43) Date of publication:
**26.08.2009 Bulletin 2009/35**

(51) Int Cl.:
***C07H 21/04*** *(2006.01)* ***C12N 15/63*** *(2006.01)*
***C07H 15/00*** *(2006.01)* ***C12Q 1/68*** *(2006.01)*

(21) Application number: **08170786.1**

(22) Date of filing: **21.03.2003**

(84) Designated Contracting States:
**AT BE BG CH CY CZ DE DK EE ES FI FR GB GR HU IE IT LI LU MC NL PT RO SE SI SK TR**

(30) Priority: **21.03.2002 US 367025 P**

(62) Document number(s) of the earlier application(s) in accordance with Art. 76 EPC:
**03714347.6 / 1 501 855**

(71) Applicant: **Sagres Discovery, Inc.**
**Davis, CA 95616 (US)**

(72) Inventor: **Morris, David W.**
**Davis, CA 95616 (US)**

(74) Representative: **UEXKÜLL & STOLBERG**
**Patentanwälte**
**Beselerstrasse 4**
**22607 Hamburg (DE)**

Remarks:
This application was filed on 05-12-2008 as a divisional application to the application mentioned under INID code 62.

(54) **Novel compositions and methods in cancer**

(57) The present invention relates to novel sequences for use in detection, diagnosis and treatment of cancers. The invention provides cancer-associated (CA) polynucleotide sequences whose expression is associated with cancer. The present invention provides CA polypeptides associated with cancer and provides diagnostic compositions and methods for the detection of cancer. The present invention provides monoclonal and polyclonal antibodies specific for the CA polypeptides. The present invention also provides diagnostic tools and therapeutic compositions and methods for screening, prevention and treatment of cancer.

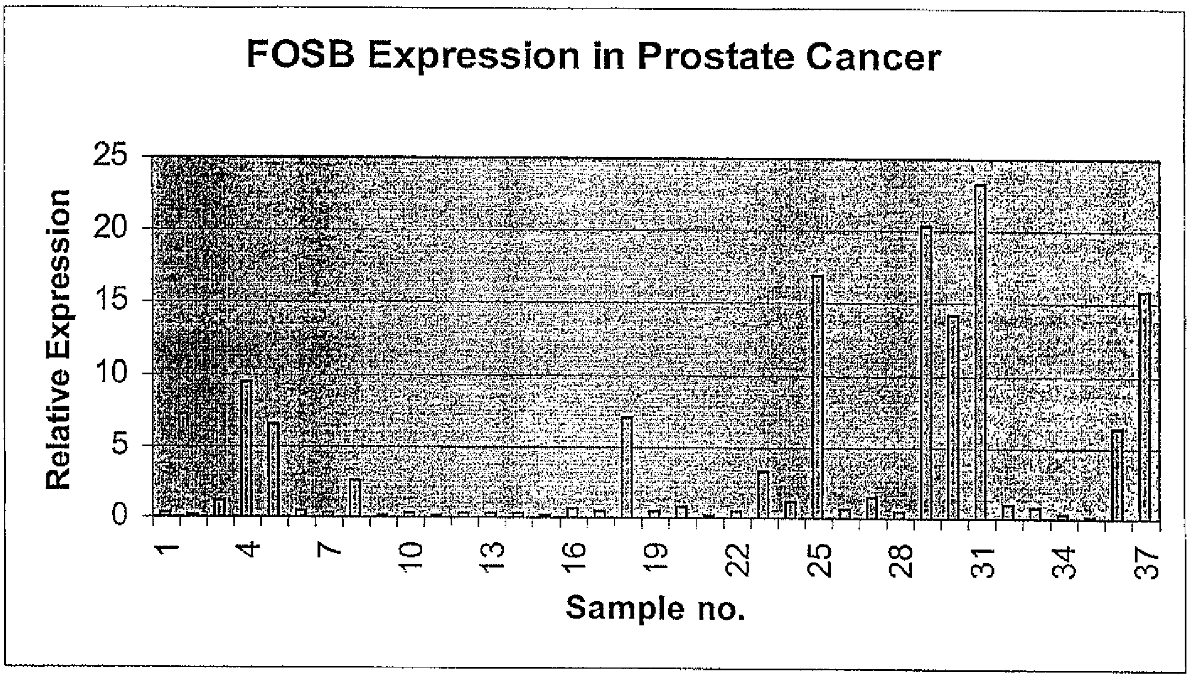


Figure 12

EP 2 093 233 A1

**Description**

CROSS-REFERENCE TO RELATED APPLICATIONS

This application claims priority to U.S provisional application Ser No. 5fly357,025 entitled "Novel Compositions and Methods in Cancer," filed March 21, 2002 This application is related to U.S. Applications entitled "Novel Compositions and Methods in Cancer," U..S Ser No. 09/747,377, filed December 22,2000, U.S. Sex- No. 09/798,586,filed March 2,2001, U.S. Sex ,No. 10/004,113, filed October 23, 2001, U.S. Ser No. 10/052,482, filed November 8, 2001 and U,S., Ser .No. 09/997,722, filed November 30,2001, all of which are expressly incorporated herein by reference in their entirety.

TECHNICAL FIELD OF THE INVENTION

This invention relates generally to the field of cancer-associated genes. Specifically, it relates to novel sequences for use in diagnosis and treatment of cancer and tumors, as well as the use of the novel compositions in screening methods. The present invention provides methods of using cancer associated polynucleotides, their corresponding gene products and antibodies specific for the gene products in the detection, diagnosis, prevention and/or treatment of associated cancers.

BACKGROUND OF THE INVENTION

Oncogenes are genes that can cause cancel Carcinogenesis can occur by a wide variety of mechanisms, including infection of cells by viruses containing oncogenes, activation of protooncogenes in the host genome, and mutations of protooncogenes and tumor suppressorf genes. Carcinogenesis is fundamentally driven by somatic cell evolution (i.e. mutation and natural selection of variants with progressive loss of growth control). The genes that serve as targets for these somatic mutations are classified as either protooncogenes or tumor suppressor genes, depending on whether their mutant phenotypes are dominant or recessive, respectively.

There are a number of viruses known to be involved in human cancer as well as in animal cancer. Of particular interest here are viruses that do not contain oncogenes themselves; these are slow-transforming retroviruses. They induce tumors by integrating into the host genome and affecting neighboring protooncogenes in a variety of ways. Provirus insertion mutation is a normal consequence of the retroviral life cycles. In infected cells, a DNA copy of the retrovirus genome (called a provirus) is integrated into the host genome A newly integrated provirus can affect gene expression in cis at or near the integration site by one of two mechanism. Type I insertion mutations up-regulate transcription of proximal genes as a consequence of regulatory sequences (enhancers and/or promoters) within the proviral long terminal repeats (LTRs), Type II insertion mutations cause truncation of coding regions due to either integration directly within an open reading name or integration within an intron flanked on both sides by coding sequences. The analysis of sequences at or near the insertion sites has led to the identification of a number of new protooncogenes

With respect to lymphoma and leukemia, retroviruses such as AKV murine leukemia virus (MLV) or SL3-3 MLV, are potent inducers of tumors when inoculated into susceptible newborn mice, or when carried in the germline. A number of sequences have been identified as relevant in the induction of lymphoma and leukemia by analyzing the insertion sites; see Sorensen et al., J of Virology 74:2161 (2000); Hansen et al., Genome Res. 10(2):237-43 (2000); Sorensen et al, J. Virology 70:4063 (1996); Sorensen et al., J. Virology 67:7118 (1993); Joosten et al, Virology 265:308 (2000); and Li et al, Nature Genetics 23:348 (1999); all of which are expressly incorporated by reference herein. With respect to cancers, especially breast cancer, prostate cancer and cancers with epithelial origin, the mammalian retrovirus, mouse mammary tumor virus (MMTV) is a potent inducer of tumors when inoculated into susceptible newborn mice, or when carried in the germ line Mammary Tumors in the Mouse, edited by J Hilgers and M. Sluyser; Elsevier/North- Holland Biomedical Press; New York, N.Y.

Breast cancer is one of the most significant diseases that affects women. At the current rate, American women have a 1 in 8 risk of developing breast cancer by age 95 (American Cancer Society, 1992) Treatment of breast cancer at later stages is often futile and disfiguring, making early detection a high priority in medical management of the disease.

The pattern of gene expression in a particular living cell is characteristic of its current state. Nearly all differences in the state or type of a cell are reflected in the differences in RNA levels of one or more genes, Comparing expression patterns of uncharacterized genes may provide clues to their function High throughput analysis of expression of hundreds or thousands of genes can help in (a) identification of complex genetic diseases, (b) analysis of differential gene expression over time, between tissues and disease states, and (c) drug discovery and toxicology studies. Increase or decrease in the levels of expression of certain genes correlate with cancel biology. For example, oncogenes are positive regulators of tumorigenesis, while tumor suppressor genes are negative regulators of tumorigenesis, (Marshall, Cell, 64: 313-326 (1991); Weinberg, Science, 254:1138-1146 (1991)).

SNL, which also in known as fascin, is an actin-bundling protein that was first isolated from cytoplasmic extracts of'sea urchin eggs (Kane, 1975: J. Cell Biol, 66:305-315) and was the first bundling protein, to be characterized in vitro. Subsequent work has shown that fascin bundles actin filaments in fertilized egg microvilli and filopodia of phagocytic coelomocytes (Otto et al , 1980, Cell Motil. 1:31-40; Otto and Bryan, 1981, Cell Motil. 1:179-192) Fascin is a widely expressed protein found in a broad spectrum of tissues and organisms.

*In vivo,* AP-1, or transcription factor activating protein 1, is a heterogenous mixture of heterodimers of several related protein subunits in addition to c-Fos and c-Jun including FosB, Fra-1, Fra-2, c-Jun, JunB, JunD, etc., (The FOS and TUN Families of Proteins, Angel and Herrlich, eds., CRC Press, Boca Raton, Fla, 1994). While AP-1 has been implicated in abnormal cell proliferation and tumor formation, events that thus might be controlled by modulating the expression of c-fos and/or c-jun, the precise contribution of each of these proteins to cell proliferation or tumor formation is unclear.

The c-myc protein is a member of the helix-loop-helix/leucine zipper (HLH/LZ) family of transcription factors that forms heterodimers with Max. In general, trans-activating Myc:Max heterodimers are found in proliferating cells, while trans-repressing Mad:Max heterodimers are found in differentiated cells. The c-myc protein level influences cell proliferation, differentiation, and neoplastic transformation, presumably by affecting the balance between Myc:Max and Mad:Max heterodimers.

Cyclin D1 is a protein derived from the PRAD1, CCND1 or bcl-1 gene on chromosome 11q13, which is involved in both regulation of the cell cycle. In the G1 (resting) phase of the cell cycle, cyclin D1 together with its cyclin dependent kinase (cdk) partner, is responsible for transition to the S (DNA synthesis) phase by phosphorylating the product of the retinoblastoma gene (pRB), which then releases transcription factors important in the initiation of DNA replication .The nuclear factor-κ B is an inducible transcription factor which participates in the regulation of multiple cellular genes after treatment of cells with factors such as phorbol ester, lipopolysaccharide (LPS), interleukin-1 (IL-1) and tumor necrosis factor-α (TNF-alpha) These genes are involved in the immediate early processes of immune, acute phase, and inflammatory responses, NF-κB has also been implicated in the transcriptional activation of several viruses, most notably the type 1 human immunodeficiency virus (HIV-1) and cytomegalovirus (CMV) (Nabel, et al., Nature, 326:711, 1987; Kaufman, et al., Mol. Cell. Biol., 7:3759, 1987; Sambucetti, et al, EMBO J, 8:4251,1989). NF-κB is a dimeric transcription factor that binds and regulates gene expression through decameric cis-acting NF-κB DNA motifs. Although a p50/p65 heterodimer has traditionally been referred to as NF-κB and remains the prototypical and most abundant form, it has been recognized recently that several distinct but closely related homo- and heterodimeric factors are responsible for NF-κB site-dependent DNA binding activity and regulation. The various dimeric factors are composed of members of the family of Rel-related polypeptides. One subclass of this family, distinguished by its proteolytic processing from precursor forms and lack of recognized activation domains, includes p50 (NFKB1) and p50B (NFKB2, p52), whereas the second subclass contains recognized activation domains and includes p65 (RelA), RelB, c-Rel, and the Drosophila protein Dorsal. All Rel-related members share a 300-amino acid regions of homology, RHD, responsible for DNA binding and dimerization, called the Rel homology domain. In the cytoplasm, NF-κB and Rel proteins form a "Rel complex".

NF-κB gene regulation is involved in many pathological events including progression of acquired immune deficiency disease (AIDS), the acute phase response and the activation of immune and endothelial cells during toxic shock, allograft rejection, and radiation responses. In addition, NF-κB gene transactivation may be critical for HIV and CMV replication.

The PVI-1 locus is associated with the c-myc locus. Frequently, mutations in one or the other loci correlate with disease such as lymphoma While rearrangements and amplification of the PVI locus have been found in lymphomas, the role of PVI-1 remains unclear

Accordingly, it is an object of the invention to provide polynucleotide and polypeptide sequences involved in cancer and, in particular, in oncogenesis

Immunotherapy, or the use of antibodies for therapeutic purposes has been used in recent years to treat cancel, Passive immunotherapy involves the use of monoclonal antibodies in cancer treatments See for example, Cancer Principles and Practice of Oncology, 6th Edition (2001) Ch 20, pp 495-508. Inherent therapeutic biological activity of these antibodies include direct inhibition of tumor cell growth or survival, and the ability to recruit the natural cell killing activity of the body's immune system These agents are administered alone or in conjunction with radiation or chemotherapeutic agents. Rituxan® and Herceptin®, approved for treatment of lymphoma and breast cancer, respectively, are two examples of such therapeutics. Alternatively, antibodies are used to make antibody conjugates where the antibody is linked to a toxic agent and directs that agent to the tumor by specifically binding to the tumor. Mylotarg® is an example of an approved antibody conjugate used for the treatment of leukemia

Accordingly, it is another object of this invention to provide antigens (cancer-associated polypeptides) associated with a variety of cancers as targets for diagnostic and/or therapeutic antibodies. These antigens are also useful for drug discovery (*e.g.,* small molecules) and for further characterization of cellular regulation, growth, and differentiation.

## SUMMARY OF THE INVENTION

In accordance with the objects outlined above, the present invention provides methods for screening for compositions that modulate cancer, especially lymphoma and leukemia. The present invention also provides methods for screening for compositions which modulate carcinomas, especially mammary adenocarcinomas Also provided herein are methods of inhibiting proliferation of a cell, preferably a lymphoma cell or a breast cancer cell Methods of treatment of cancel, including diagnosis, are also provided herein.

In one aspect, a method of screening drug candidates comprises providing a cell that expresses a cancer-associated (CA) gene or fragments thereof. Preferred embodiments of CA genes are genes that are differentially expressed in cancer cells, preferably lymphatic, breast, prostate or epithelial cells, compared to other cells. Preferred embodiments of CA genes used in the methods herein include, but are not limited to the nucleic acids selected from Tables 1-6. That is, they include but are not limited to PVI1, SNL1, CCND1, FOSB, MYC, or NFKB1. The methods further include adding a drug candidate to the cell and determining the effect of'the drug candidate on the expression of the CA gene.

In one embodiment, the method of screening drug candidates includes comparing the level of expression in the absence of the drug candidate to the level of expression in the presence of the drug candidate.

Also provided herein is a method of screening for a bioactive agent capable of binding to a CA protein (CAP), the method comprising combining the CAP and a candidate bioactive agent, and determining the binding of the candidate agent to the CAP.

Further provided herein is a method for screening for a bioactive agent capable of'modulating the activity of a CAP. In one embodiment, the method comprises combining the CAP and a candidate bioactive agent, and determining the effect of'the candidate agent on the bioactivity of the CAP.

Also provided is a method of evaluating the effect of a candidate carcinoma drug comprising administering the drug to a patient and removing a cell sample from the patient. The expression profile of the cell is then determined. This method may further comprise comparing the expression profile of the patient to an expression profile of a heathy individual

In a further aspect, a method for inhibiting the activity of an CA protein is provided In one embodiment, the method comprises administering to a patient an inhibitor of a CA protein preferably selected from the group consisting of the sequences outlined in Tables 1-6 or their complements.

A method of neutralizing the effect of a CA protein, preferably a protein encoded by a nucleic acid selected from the group of sequences outlined in Tables 1-6, is also provided. Preferably, the method comprises contacting an agent specific for said protein with said protein in an amount sufficient to effect neutralization.

Moreover, provided herein is a biochip/microarray comprising a nucleic acid segment which encodes a CA protein, preferably selected from the sequences outlined in Tables 1-6. That is, they include but are not limited to genes including PVT1, SNL1, CCND1, FOSB, MYC, or NFKB1. In different embodiments, a microarray of the invention comprises one, two, three, four, five or more human sequences from Tables 1-6 including, but not limited to, sequences related to PVT1, SNL1, CCND1, FOSB, MYC, or NFKB1.

Also provided herein is a method for diagnosing or determining the propensity to cancers, especially lymphoma or leukemia or carcinoma (including breast cancer) by sequencing at least one carcinoma or lymphoma gene of an individual. In yet another aspect of the invention, a method is provided for determining cancer including lymphoma and leukemia gene copy numbers in an individual.

Novel sequences associated with cancer are also provided herein Other aspects of the invention will become apparent to the skilled artisan by the following description of the invention

## BRIEF DESCRIPTION OF THE FIGURES

Figure 1 depicts PCR amplification of host-provirus junction fragments.

Figure 2 shows an example of average threshold cycle ($C_T$) values for a housekeeper gene and target gene.

Figure 3 shows an example of the calculated difference ($\Delta\Delta C_I$) between the $C_I$ values of target and housekeeper genes ($\Delta C_I$) for various samples

Figure 4 shows the $\Delta\Delta C_I$ and comparative expression level for each sample from Figure 3

Figure 5 depicts mRNA expression of SNL1 in breast cancel tissue compared with expression in normal tissue. Samples 1-50 are breast cancel samples. Samples 51 and 52 are normal tissues.. Bars represent the mean of expression level Error bars represent standard deviation..

Figure 5 depicts mRNA expression of FOSB in colon cancel tissue compared with expression in normal tissue. Samples 1-11 are normal samples Samples 12-31 are colon cancer tissues, Bars represent the mean of expression level, Error bars represent standard deviation.

Figure 7 depicts RNA expression of FOSB in lung cancer tissue compared with expression in normal tissue. Samples 1-9 are normal samples, Samples 10-43 are lung cancer tissues. Bars represent the mean of expression level.

Error bars represent standard deviation.

Figure 8 depicts RNA expression of FOSB in pancreas cancer tissue compared with expression in normal tissue. Samples 1-10 are normal samples Samples 11-31 are pancreas cancer tissues. Bars represent the mean of expression level. Error bars represent standard deviation.

Figure 9 depicts mRNA expression of FOSB in ovary cancer tissue compared with expression in normal tissue. Samples 1-16 are normal samples. Samples 17-44 are ovary cancer tissues. Bars represent the mean of expression level. Error bars represent standard deviation.

Figure 10 depicts mRNA expression of FOSB in stomach cancer tissue compared with expression in normal tissue. Samples 1-10 are normal samples Samples 11-39 are stomach cancer tissues Bars represent the mean of expression level. Error bars represent standard deviation

Figure 11 depicts mRNA expression of FOSB in breast cancer tissue compared with expression in normal tissue. Samples 1-9 are normal samples. Samples 10-30 are breast cancer tissues Bars represent the mean of expression level. Error bars represent standard deviation.

Figure 12 depicts mRNA expression of FOSB in prostate cancer tissue compared with expression in normal tissue.. Samples 1-7 are normal samples. Samples 8-37 are prostate cancer tissues. Bars represent the mean of'expression level. Error bars represent standard deviation.

Figure 13 depicts mRNA expression of MYC in breast cancer tissue compared with expression in normal tissue, Samples 1-50 are breast cancer samples. Bars represent the mean of expression level Error bars represent standard deviation.

Figure 14 depicts DNA amplification of MYC in breast cancer tissue compared with expression in normal tissue. Samples 1-49 are breast cancer samples. Bars represent the mean of expression level. Error bars represent standard deviation.

Figure 15 depicts mRNA expression of CCND1 in breast cancer tissue compared with expression in normal tissue. Samples 1-50 are breast cancer samples Samples 51 and 52 are normal tissues. Bars represent the mean of expression level Error bars represent standard deviation.

Figure 16 depicts mRNA expression of CCND1 in colon cancer (sigmoid) tissue compared with expression in normal tissue Samples 1-12. are normal samples. Samples 13-29 are colon cancer tissues Bars represent the mean of expression level. Error bars represent standard deviation.

Figure 17 depicts RNA expression of CCND1 in colon cancer (transverse) tissue compared with expression in normal tissue, Samples 1-12 are normal samples. Samples 13-30 are colon cancer tissues Bars represent the mean of expression level. Error bars represent standard deviation

Figure 18 depicts mRNA expression of CCND1 in lung tissue compared with expression in normal tissue. Samples 1-9 are normal sample. Samples 10-43 are lung cancer tissues.. Bars represent the mean of expression level. Error bars represent standard deviation.

Figure 19 depicts mRNA expression of CCND 1 in ovary tissue compared with expression in normal tissue. Samples 1-14 are normal samples Samples 15-41 are ovary cancer tissues. Bars represent the mean of expression level. Error bars represent standard deviation,

Figure 20 depicts mRNA expression of NFKB1 in breast cancer tissue compared with expression in normal tissue. Samples 1-50 are breast cancer samples. Samples 51 and 52 are normal tissues. Bars represent the mean of expression level. Error bars represent standard deviation.

Figure 21 depicts mRNA expression of NFKB1 in lung tissue compared with expression in normal tissue. Samples 1-9 are normal samples Samples 10-44 are lung cancer tissues. Bars represent the mean of expression level. Error bars represent standard deviation.

Figure 22 depicts mRNA expression of NFKB1 in skin tissue compared with expression in normal tissue Samples 1-9 are normal samples. Samples 10-46 are skin cancer tissues Bars represent the mean of expression levels Error bars represent standard deviation.

Figure 23 depicts RNA expression of PVT1 in breast cancer tissue compared with expression in normal tissue. Samples 1-50 are breast cancer samples. Bars represent the mean of expression level. Error bars represent standard deviation

Figure 24 depicts DNA amplification of PVT1 in breast cancer tissue compared with expression in normal tissue. Samples 1-50 are breast cancer samples. Bars represent the mean of expression level. Error bars represent standard deviation

## DETAILED DESCRIPTION OF PREFERRED EMBODIMENTS

The present invention is directed to a number of sequences associated with cancers, especially lymphoma, breast cancer or prostate cancer. The relatively tight linkage between clonally-integrated proviruses and protooncogenes forms "provirus tagging", in which slow-transforming retroviruses that act by an insertion mutation mechanism are used to isolate protooncogenes In some models, uninfected animals have low cancer rates, and infected animals have high cancer rates. It is known that many of the retroviruses involved do not carry transduced host protooncogenes or pathogenic trans-acting viral genes, and thus the cancer incidence must therefore be a direct consequence of proviral integration effects into host protooncogenes. Since proviral integration is random, rare integrants will "activate" host protooncogenes that provide a selective growth advantage, and these rare events result in new proviruses at clonal stoichiometries in tumors. In contrast to mutations caused by chemicals, radiation, or spontaneous errors, protooncogene insertion mutations can be easily located by virtue of the fact that a convenient-sized genetic marker of' known sequence (the provirus) is present at the site of mutation. Host sequences that flank clonally integrated proviruses can be cloned using a variety of strategies. Once these sequences are in hand, the tagged protooncogenes can be subsequently identified. The presence of provirus at the same locus in two or more independent tumors is *prima facie* evidence that a protooncogenes is present at or very near the provirus integration sites. This is because the genome is too large for random integrations to result in observable clustering. Any clustering that is detected is unequivocal evidence for biological selection (i.e. the tumor phenotype) Moreover the pattern of proviral integrants (including orientations) provides compelling positional information that makes localization of the target gene at each cluster relatively simple. The three mammalian retroviruses that are known to cause cancer by an insertion mutation mechanism are FeLV (leukemia/lymphoma in cats), MLV (leukemia/lymphoma in mice and rats), and MMTV (mammary cancer in mice)

Thus, the use of oncogenic retroviruses, whose sequences insert into the genome of the host organism resulting in cancer, allows the identification of'host sequences involved in cancer. These sequences may then be used in a number of different ways, including diagnosis, prognosis, screening for modulators (including both agonists and antagonists), antibody generation (for immunotherapy and imaging), etc. However, as will be appreciated by those in the art, oncogenes that are identified in one type of cancer such as lymphoma or leukemia have a strong likelihood of being involved in other types of cancers as well. Thus, while the sequences outlined herein are initially identified as correlated with lymphoma, they can also be found in other types of cancers as well, outlined below.

**Definitions**

Accordingly, the present invention provides nucleic acid and protein sequences that are associated with cancer, herein termed "cancer associated" or "CA" sequences. In one embodiment, the present invention provides nucleic acid and protein sequences that are associated with cancers that originate in lymphatic tissue, herein termed "lymphoma associated," "leukemia associated" or "LA" sequences. In another embodiment, the present invention provides nucleic acid and protein sequences that are associated with carcinomas which originate in breast tissue, herein termed "breast cancer associated" or "BCA" sequences.

Suitable cancers that can be diagnosed or screened for using the methods of the present invention include cancers classified by site or by histological type, Cancers classified by site include cancer of the oial cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, nos; ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and leukemias (lymphocytic leukemia; myeloid leukemia; monocytic leukemia; and other leukemias)

Other cancer, classified by histological type, that may be associated with the .. sequences of the invention include, but are not limited to, Neoplasm, malignant; Carcinoma, NOS; Carcinoma, undifferentiated, NOS; Giant and spindle cell carcinoma; Small cell carcinoma, NOS; Papillary carcinoma, NOS; Squamous cell carcinoma, NOS; Lymphoepithelial carcinoma; Basal cell carcinoma, NOS; Pilomatrix carcinoma; Transitional cell carcinoma, NOS; Papillary transitional cell carcinoma; Adenocarcinoma, NOS; Gastrinoma, malignant; Cholangiocarcinoma; Hepatocellular carcinoma, NOS; Combined hepatocellular carcinoma and cholangiocarcinoma; Trabecular adenocarcinoma; Adenoid cystic carcinoma; Adenocarcinoma in adenomatous polyp; Adenocarcinoma, familial polyposis coli; Solid carcinoma, NOS; Carcinoid tumor, malignant; Bronchiolo-alveolar adenocarcinoma; Papillary adenocarcinoma, NOS; Chromophobe carcinoma; Acidophil carcinoma; Oxyphilic adenocarcinoma; Basophil carcinoma; Clear cell adenocarcinoma, NOS; Granular cell carcinoma; Follicular adenocarcinoma, NOS; Papillary and follicular adenocarcinoma; Nonencapsulating sclerosing carcinoma; Adrenal cortical carcinoma; Endometroid carcinoma; Skin appendage carcinoma; Apocrine adenocarcinoma; Sebaceous adenocarcinoma; Ceruminous adenocarcinoma; Mucoepidermoid carcinoma; Cystadenocarcinoma, NOS; Papillary cystadenocarcinoma, NOS; Papillary serous cystadenocarcinoma; Mucinous cystadenocarcimoma, NOS; Mucinous adenocarcinoma; Signet ring cell carcinoma; Infiltrating duct carcinoma; Medullary carcinoma, NOS; Lobular carcinoma; Inflammatory carcinoma; Paget's disease, mammary; Acinar cell carcinoma; Adenosquamous carcinoma; Adenocarcinoma w/ squamous metaplasia; Thymoma, malignant; Ovarian stromal tumor, malignant; Thecoma, malignant; Granulosa cell tumor, malignant; Androblastoma, malignant; Sertoli cell carcinoma; Leydig cell tumor, malignant; Lipid cell tumor, malignant; Paraganglioma, malignant; Extra-mammary paraganglioma, malignant; Pheochromocytoma; Glomangiosarcoma; Malignant melanoma, NOS; Amelanotic melanoma; Superficial spreading melanoma; Malig melanoma in giant pigmented nevus; Epithelioid cell melanoma; Blue nevus, malignant; Sarcoma, NOS; Fibrosarcoma, NOS; Fibrous histiocytoma, malignant; Myxosarcoma; Liposarcoma, NOS; Leiomyosarcoma, NOS; Rhabdomyosarcoma, NOS; Embryonal rhabdomyosarcoma; Alveolar rhabdomyosarcoma; Stromal sarcoma, NOS; Mixed tumor, malignant, NOS; Mullerian mixed tumor; Nephroblastoma; Hepatoblastoma; Carcinosarcoma, NOS; Mesenchymoma, malignant; Brenner tumor, malignant; Phyllodes tumor, malignant; Synovial sarcoma, NOS; Mesothelioma, malignant; Dysgerminoma; Embryonal carcinoma, NOS; Teratoma, malignant, NOS; Struma ovarii, malignant; Choriocarcinoma; Mesonephroma, malignant; Hemangiosarcoma; Hemangioendothelioma, malignant; Kaposi's sarcoma; Hemangiopericytoma, malignant; Lymphangiosarcoma; Osteosarcoma, NOS; Juxtacortical osteosarcoma; Chondrosarcoma, NOS; Chondroblastoma, malignant; Mesenchymal chondrosarcoma; Giant cell tumor of bone; Ewing's sarcoma; Odontogenic tumor, malignant; Ameloblastic odontosarcoma; Ameloblastoma, malignant; Ameloblastic fibrosarcoma; Pinealoma, malignant; Chordoma; Glioma, malignant; Ependymoma, NOS; Astiocytoma, NOS; Protoplasmic astrocytoma; Fibrillary astrocytoma; Astroblastoma; Glioblastoma, NOS; Oligodendroglioma, NOS; Oligodendroblastoma; Primitive neuroectodermal; Cerebellar sarcoma, NOS; Ganglioneuroblastoma; Neuroblastoma, NOS; Retinoblastoma, NOS; Olfactory neurogenic tumor; Meningioma, malignant; Neurofibrosarcoma; Neurilemmoma, malignant; Granular cell tumor, malignant; Malignant lymphoma, NOS; Hodgkin's disease, NOS; Hodgkin's; paragranuloma, NOS; Malignant lymphoma, small lymphocytic; Malignant lymphoma, large cell, diffuse; Malignant lymphoma, follicular, NOS; Mycosis fungoides; Other specified non-Hodgkin's lymphomas; Malignant histiocytosis; Multiple myeloma; Mast cell sarcoma; Immunoproliferative small intestinal disease; Leukemia, NOS; Lymphoid leukemia, NOS; Plasma cell leukemia; Erythroleukemia; Lymphosarcoma cell leukemia; Myeloid leukemia, NOS; Basophilic leukemia; Eosinophilic leukemia; Monocytic leukemia, NOS; Mast cell leukemia; Megakaryoblastic leukemia; Myeloid sarcoma; and Hairy cell leukemia

In addition, the CA genes may be involved in other diseases such as, but not limited to, diseases associated with aging or neurodegeneration.

"Association" in this context means that the nucleotide or protein sequences are either differentially expressed, activated, inactivated or altered in cancers as compared to normal tissue. As outlined below, CA sequences include those that are up-regulated (i.e. expressed at a higher level), as well as those that are down-regulated (i.e. expressed at a lower level), in cancers. CA sequences also include sequences that have been altered (i.e., truncated sequences or sequences with substitutions, deletions or insertions, including point mutations) and show either the same expression profile or an altered profile. In a preferred embodiment, the CA sequences are from humans; however, as will be appreciated by those in the art, CA sequences from other organisms may be useful in animal models of disease and drug evaluation; thus, other CA sequences are provided, from vertebrates, including mammals, including rodents (rats, mice, hamsters, guinea pigs, etc.), primates, and from animals (including sheep, goats, pigs, cows, horses, etc) In some cases, prokaryotic CA sequences may be useful CA sequences from other organisms may be obtained using the techniques outlined below

CA sequences include both nucleic acid and amino acid sequences. In one embodiment, the CA sequences are recombinant nucleic acids, By the term "recombinant nucleic acid" herein is meant nucleic acid, originally formed in vitro, in general, by the manipulation of nucleic acid by polymerases and endonucleases, in a form not normally found in nature Thus a recombinant nucleic acid is also an isolated nucleic acid, in a linear form, or cloned in a vector formed in vitro by ligating DNA molecules that are not normally joined, are both considered recombinant for the purposes of this invention. It is understood that once a recombinant nucleic acid is made and reintroduced into a host cell or organism, it will replicate using the in vivo cellular machinery of the host cell rather than in vitro manipulations; however, such nucleic acids, once produced recombinantly, although subsequently replicated in vivo, are still considered recombinant or isolated for the purposes of the invention As used herein a "polynucleotide" or "nucleic acid" is a polymeric form of nucleotides of'any length, either ribonucleotides or deoxyribonucleotides. This term refers only to the primary structure of the molecule. Thus, this term includes double- and single-stranded DNA and RNA It also includes known types of modifications, for example, labels which are known in the art, methylation, "caps", substitution of one or more of the naturally occurring nucleotides with an analog, internucleotide modifications such as, for example, those with uncharged linkages (e,g., phosphorothioates, phosphorodithioates, etc.), those containing pendant moieties, such as, for example proteins (including e.g., nucleases, toxins, antibodies, signal peptides, poly-L-lysine, etc.),those with intercalators (e.g., acridine, psoralen, etc ), those containing chelators (e.g., metals, radioactive metals, etc.), those containing alkylators, those with modified linkages (e.g, alpha anomeric nucleic acids, etc.), as well as unmodified forms of the polynucleotide,

As used herein, a polynucleotide "derived from" a designated sequence refers to a polynucleotide sequence which is comprised of a sequence of approximately at least about 6 nucleotides, preferably at least about 8 nucleotides, more preferably at least about 10-1.2 nucleotides, and even more preferably at least about 15-20 nucleotides corresponding to a region of the designated nucleotide sequence "Corresponding" means homologous to or complementary to the designated sequence. Preferably, the sequence of'the region from which the polynucleotide is derived is homologous to or complementary to a sequence that is unique to a CA gene.

Similarly, a "recombinant protein" is a protein made using recombinant techniques, i.e. through the expression of a recombinant nucleic acid as depicted above. A recombinant protein is distinguished from naturally occurring protein by at least one or more characteristics. For example, the protein may be isolated or purified away from some or all of the proteins and compounds with which it is normally associated in its wild type host, and thus may be substantially pure. For example, an isolated protein is unaccompanied by at least some of the material with which it is normally associated in its natural state, preferably constituting at least about 0.5%, more preferably at least about 5% by weight of the total protein in a given sample. A substantially pure protein comprises about 50-75% by weight of the total protein, with about 80% being preferred, and about 90% being particularly preferred The definition includes the production of a CA protein from one organism in a different organism or host cell, Alternatively, the protein may be made at a significantly higher concentration than is normally seen, though the use of an inducible promoter or high expression promoter, such that the protein is made at increased concentration levels. Alternatively, the protein may be in a form not normally found in nature, as in the addition of an epitope tag or amino acid substitutions, insertions and deletions, as discussed below.

In a preferred embodiment, the CA sequences are nucleic acids. As will be appreciated by those in the art and is more fully outlined below, CA sequences are useful in a variety of applications, including diagnostic applications, which will detect naturally occurring nucleic acids, as well as screening applications; for example, biochips comprising nucleic acid probes to the CA sequences can be generated. In the broadest sense, use of "nucleic acid," "polynucleotide" of "oligonucleotide" or equivalents herein means at least two nucleotides covalently linked together, In some embodiments, an oligonucleotide is an oligomer of 6, 8, 10, 12, 20, 30 or up to 100 nucleotides. A "polynucleotide" or "oligonucleotide" may comprise DNA, RNA, PNA or a polymer of nucleotides linked by phosphodiester and/or any alternate bonds

A nucleic acid of the present invention generally contains phosphodiester bonds, although in some cases, as outlined below (for example, in antisense applications or when a nucleic acid is a candidate drug agent), nucleic acid analogs may have alternate backbones, comprising, for example, phosphoramidate (Beaucage et al., Tetrahedron 49 (10):1925 (1993) and references therein; Letsinger, J. Org. Chem. 35:3800 (1970); Sprinzl et al, Eur. J. Biochem. 81: 579 (1977); Letsinger et al., Nucl. Acids Res 14:3487 (1986); Sawai et al, Chem Lett. 805 (1984), Letsinger et al., J. Am. Chem, Soc., 110:4470 (1988); and Pauwels et al", Chemica Scripta 26:141 91986)), phosphorothioate (Mag et al., Nucleic Acids Res. 19:1437 (1991); and U.S. Patent No. 5,644,048), phosphorodithioate (Briu et al., J. Am Chem. Soc 111:232.1 (1989), O-methylphosphoroamidite linkages (see Eckstein, Oligonucleotides and Analogues: A Practical Approach, Oxford University Press), and peptide nucleic acid backbones and linkages (see Egholm, J. Am Chem. Soc. 114:1895 (1992); Meier et al., Chem, Int., Ed. Engl. 31:1008 (1992); Nielsen, Nature, 365:566 (1993); Carlsson et al., Nature 380:207 (1996), all of which are incorporated by reference). Other analog nucleic acids include those with positive backbones (Denpcy et al., Proc. Natl. Acad Sci. USA 92:6097 (1995); non-ionic backbones (U.S. Patent Nos. 5,386,023, 5,637,684, 5,602,240, 5,216,141 and 4,469,863; Kiedrowshi et al., Angew Chem. Intl Ed. English 30:423 (1991); Letsinger et al., J. Am. Chem Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y.S. Sanghui and P. Dan Cook; Mesmaeker et al., Bioorganic & Medicinal Chem Lett 4:395 (1994); Jeffs et al., J. Biomolecular NMR.34:17 (1994); Tetrahedron Lett 37:743 (1996)) and non-ribose backbones, including those described in U.S. Patent Nos 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within one definition of nucleic acids (see Jenkins et al., Chem. Soc. Rev. (1995) pp169-176). Several nucleic acid analogs are described in Rawls, C & E News June 2, 1997 page 35. All of these references are hereby expressly incorporated by reference These modifications of the ribose-phosphate backbone may be done for a variety of reasons, for example to increase the stability and half-life of such molecules in physiological environments for use in anti-sense applications or as probes on a biochip.

As will be appreciated by those in the art, all of these nucleic acid analogs may find use in the present invention. In addition, mixtures of naturally occurring nucleic acids and analogs can be made; alternatively, mixtures of different nucleic acid analogs, and mixtures of naturally occurring nucleic acids and analogs may be made

The nucleic acids may be single stranded or double stranded, as specified, or contain portions of both double stranded or single stranded sequence. As will be appreciated by those in the art, the depiction of a single strand "Watson" also defines the sequence of the other strand "Crick"; thus the sequences described herein also includes the complement of the sequences. The nucleic acid may be DNA, both genomic and cDNA, RNA, or a hybrid, where the nucleic acid contains any combination of deoxyribo- and ribo-nucleotides, and any combination of bases, including uracil, adenine, thymine, cytosine, guanine, inosine, xanthine, hypoxanthine, isocytosine, isoguanine, etc. As used herein, the term "nucleoside" includes nucleotides and nucleoside and nucleotide analogs, and modified nucleosides such as amino modified nucleosides. In addition, "nucleoside" includes non-naturally occurring analog structures. Thus for example the individual units of a peptide nucleic acid, each containing a base, are referred to herein as a nucleoside.

As used herein, the term "tag," "sequence tag" or "primer tag sequence" refers to an oligonucleotide with specific nucleic acid sequence that serves to identify a batch of polynucleotides bearing such tags therein. Polynucleotides from the same biological source are covalently tagged with a specific sequence tag so that in subsequent analysis the polynucleotide can be identified according to its source of origin. The sequence tags also serve as primers for nucleic acid amplification reactions

A "microarray" is a linear or two-dimensional array of preferably discrete regions, each having a defined area, formed on the surface of' a solid support. The density of the discrete regions on a microarray is determined by the total numbers of target polynucleotides to be detected on the surface of a single solid phase support, preferably at least about 50/cm$^2$, more preferably at least about 100/cm$^2$, even more preferably at least about 500/cm$^2$, and still more preferably at least about 1,000/cm$^2$. As used herein, a DNA microarray is an array of oligonucleotide primers placed on a chip or other surfaces used to amplify or clone target polynucleotides. Since the position of each particular group of primers in the array is known, the identities of the target polynucleotides can be determined based on their binding to a particular position in the microarray.

A "linker" is a synthetic oligodeoxyribonucleotide that contains a restriction site. A linker may be blunt end ligated onto the ends of DNA fragments to create restriction sites that can be used in the subsequent cloning of the fragment into a vector molecule.

The term "label" refers to a composition capable of producing a detectable signal indicative of the presence of the target polynucleotide in an assay sample. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical, chemical, or any other appropriate means The term "label" is used to refer to any chemical group or moiety having a detectable physical property or any compound capable of causing a chemical group or moiety to exhibit a detectable physical property, such as an enzyme that catalyzes conversion of a substrate into a detectable product. The term "label" also encompasses compounds that inhibit the expression of a particular physical property. The label may also be a compound that is a member of a binding pair, the other member of which bears a detectable physical property.

The term "support" refers to conventional supports such as beads, particles, dipsticks, fibers, filters, membranes, and silane or silicate supports such as glass slides.

The term "amplify" is used in the broad sense to mean creating an amplification product which may include, for example, additional target molecules, or target-like molecules or molecules complementary to the target molecule, which molecules are created by virtue of the presence of the target molecule in the sample. In the situation where the target is a nucleic acid, an amplification product can be made enzymatically with DNA or RNA polymerases or reverse transcriptases

As used herein, a "biological sample" refers to a sample of tissue or fluid isolated from an individual, including but not limited to, for example, blood, plasma, serum, spinal fluid, lymph fluid, skin, respiratory, intestinal and genitourinary tracts, tears, saliva, milk, cells (including but not limited to blood cells), tumors, organs, and also samples of *in vitro* cell culture constituents.

The term "biological sources" as used herein refers to the sources from which the target polynucleotides are derived. The source can be of any form of "sample" as described above, including but not limited to, cell, tissue or fluid. "Different biological sources" can refer to different cells/tissues/organs of the same individual, or cells/tissues/organs from different individuals of the same species, or cells/tissues/organs from different species.

## Cancer-associated Sequences

The CA sequences of the invention were initially identified by infection of mice with a retrovirus such as murine leukemia virus (MLV) or mouse mammary tumor virus (MMTV) resulting in lymphoma The CA sequences wre subsequently validated by determining expression levels of the corresponding gene product (e.g., mRNA) in breast cancer and other cancer tissue samples Retroviruses have a genome that is made out of RNA. After a retrovirus infects a host cell, a double stranded DNA copy of the retrovirus genome (a "provirus") is inserted into the genomic DNA of the host cell. The integrated provirus may affect the expression of host genes at or near the site of integration - a phenomenon known as retroviral insertional mutagenesis. Possible changes in the expression of'host cell genes include: (i) increased expression of genes near the site of integration resulting from the proximity of elements in the provirus that act as transcriptional promoters and enhancers, (ii) functional inactivation of a gene caused by the integration of a provirus into the gene itself thus preventing the synthesis of a functional gene product, or (iii) expression of a mutated protein that has a different activity to the normal protein. Typically such a protein would be prematurely truncated and lack a regulatory domain near the C terminus Such a protein might be constitutively active, or act as a dominant negative inhibitor of the normal protein. For example, retrovirus enhancers, including that of SL3-3, are known to act on genes up to approximately 200 kilobases from the insertion site Moreover, many of these sequences are also involved in other cancers and disease states. Sequences of mouse genes according to this invention, that are identified in this manner are shown in Tables 1-6.

A CA sequence can be initially identified by substantial nucleic acid and/or amino acid sequence homology to the CA sequences outlined herein Such homology can be based upon the overall nucleic acid or amino acid sequence, and is generally determined as outlined below, using either homology programs or hybridization conditions.

In one embodiment, CA sequences are up-regulated in cancers; that is, the expression of'these genes is higher in cancer tissue as compared to normal tissue of the same differentiation stage. "Up-regulation" as used herein means increased expression by about 50%, preferably about 100%, more preferably about 150% to about 200%, with up-regulation from 300% to 1000% being preferred.

In another embodiment, CA sequences are down-regulated in cancers; that is, the expression of these genes is lower in cancer tissue as compared to normal tissue of the same differentiation stage.. "Down-regulation" as used herein means decreased expression by about 50%, preferably about 100%, more preferably about 150% to about 200%, with down-regulation from 300% to 1000% to no expression being preferred.

In yet another embodiment, CA sequences are those that have altered sequences but show either the same or an altered expression profile as compared to normal lymphoid tissue of the same differentiation stage. "Altered CA sequences" as used herein also refers to sequences that are truncated, contain insertions or contain point mutations

CA proteins of the present invention may be classified as secreted proteins, transmembrane proteins or intracellular proteins. In a preferred embodiment the CA protein is an intracellular protein. Intracellular proteins may be found in the cytoplasm and/or in the nucleus. Intracellular proteins are involved in all aspects of cellular function and replication (including, for example, signaling pathways); aberrant expression of such proteins results in unregulated or disregulated cellular processes. For example, many intracellular proteins have enzymatic activity such as protein kinase activity, protein phosphatase activity, protease activity, nucleotide cyclase activity, polymerase activity and the like. Intracellular proteins also serve as docking proteins that are involved in organizing complexes of proteins, or targeting proteins to various subcellular localizations, and are involved in maintaining the structural integrity of organelles.

An increasingly appreciated concept in characterizing intracellular proteins is the presence in the proteins of one or more motifs for which defined functions have been attributed. In addition to the highly conserved sequences found in the enzymatic domain of proteins, highly conserved sequences have been identified in proteins that are involved in protein-protein interaction. For example, Src-homology-2 (SH2) domains bind tyrosine-phosphorylated targets in a sequence dependent manner. PTB domains, which are distinct from SH2 domains, also bind tyrosine phosphorylated targets SH3 domains bind to proline-rich targets. In addition, PH domains, tetratricopeptide repeats and WD domains to name only a few, have been shown to mediate protein-protein interactions. Some of these may also be involved in binding to phospholipids or other second messengers. As will be appreciated by one of ordinary skill in the art, these motifs can be identified on the basis of primary sequence; thus, an analysis of the sequence of proteins may provide insight into both the enzymatic potential of the molecule and/or molecules with which the protein may associate.

In a preferred embodiment, the CA sequences are transmembrane proteins. Transmembrane proteins are molecules that span the phospholipid bilayer of a cell. They may have an intracellular domain, an extracellular domain, or both. The intracellular domains of such proteins may have a number of functions including those already described for intracellular proteins. For example, the intracellular domain may have enzymatic activity and/or may serve as a binding site for additional proteins. Frequently the intracellular domain of transmembrane proteins serves both roles. For example certain receptor tyrosine kinases have both protein kinase activity and SH2 domains. In addition, autophosphorylation of tyrosines on the receptor molecule itself creates binding sites for additional SH2 domain containing proteins.

Transmembrane proteins may contain from one to many transmembrane domains. For example, receptor tyrosine kinases, certain cytokine receptors, receptor guanylyl cyclases and receptor serine/threonine protein kinases contain a single transmembrane domain. However, various other proteins including channels and adenylyl cyclases contain numerous transmembrane domains. Many important cell surface receptors are classified as "seven transmembrane domain" proteins, as they contain 7 membrane spanning regions. Important transmembrane protein receptors include, but are not limited to insulin receptor, insulin-like growth factor receptor, human growth hormone receptor, glucose transporters, transferrin receptor, epidermal growth factor receptor, low density lipoprotein receptors, leptin receptors, interleukin receptors, e.g. IL-1 receptor, IL-2 receptor, etc CA proteins may be derived from genes that regulate apoptosis (IL-3, GM-CSF and Bcl-x) or are shown to have a role in the regulation of apoptosis

Characteristics of transmembrane domains include approximately 20 consecutive hydrophobic amino acids that may be followed by charged amino acids. Therefore, upon analysis of the amino acid sequence of a particular protein, the localization and number of transmembrane domains within the protein may be predicted.

The extracellular domains of transmembrane proteins are diverse; however, conserved motifs are found repeatedly among various extracellular domains. Conserved structure and/or functions have been ascribed to different extracellular motifs. For example, cytokine receptors are characterized by a cluster of cysteines and a WSXWS (W= tryptophan, S= serine, X=any amino acid) motif. Immunoglobulin-like domains are highly conserved Mucin-like domains may be involved in cell adhesion and leucine-rich repeats participate in protein-protein interactions.

Many extracellular domains are involved in binding to other molecules. In one aspect, extracellular domains are receptors. Factors that bind the receptor domain include circulating ligands, which may be peptides, proteins, or small molecules such as adenosine and the like. For example, growth factors such as EGF, FGF and PDGF are circulating growth factors that bind to their cognate receptors to initiate a variety of cellular responses. Other factors include cytokines, mitogenic factors, neurotrophic factors and the like. Extracellular domains also bind to cell-associated molecules In this respect, they mediate cell-cell interactions. Cell-associated ligands can be tethered to the cell for example via a glycosylphosphatidylinositol (GPI) anchor, or may themselves be transmembrane proteins Extracellular domains also associate with the extracellular matrix and contribute to the maintenance of the cell structure.

CA proteins that are transmembrane are particularly preferred in the present invention as they are good targets for immunotherapeutics, as are described herein. In addition, as outlined below, transmembrane proteins can be also useful in imaging modalities.

It will also be appreciated by those in the art that a transmembrane protein can be made soluble by removing transmembrane sequences, for example through recombinant methods. Furthermore, transmembrane proteins that have been made soluble can be made to be secreted through recombinant means by adding an appropriate signal sequence.

In a preferred embodiment, the CA proteins are secreted proteins; the secretion of which can be either constitutive or regulate. These proteins have a signal peptide or signal sequence that targets the molecule to the secretory pathway. Secreted proteins are involved in numerous physiological events; by virtue of their circulating nature, they serve to transmit signals to various other cell types. The secreted protein may function in an autocrine manner (acting on the cell that secreted the factor), a paracrine manner (acting on cells in close proximity to the cell that secreted the factor) or an endocrine manner (acting on cells at a distance)". Thus secreted molecules find use in modulating or altering numerous aspects of physiology. CA proteins that are secreted proteins are particularly preferred in the present invention as they serve as good targets for diagnostic markers, for example for blood tests.

**CA sequences and homologs**

A CA sequence is initially identified by substantial nucleic acid and/or amino acid sequence homology to the CA sequences outlined herein. Such homology can be based upon the overall nucleic acid or amino acid sequence, and is generally determined as outlined below, using either homology programs or hybridization conditions.

As used herein, a nucleic acid is a "CA nucleic acid" if the overall homology of the nucleic acid sequence to one of the nucleic acids of Tables 1-6 is preferably greater than about 75%, more preferably greater than about 80%, even more preferably greater than about 85% and most preferably greater than 90% In some embodiments the homology will be as high as about 93 to 95 or 98%. In a preferred embodiment, the sequences that are used to determine sequence identity or similarity are selected from those of the nucleic acids of Tables 1-6. In another embodiment, the sequences are naturally occurring allelic variants of the sequences of the nucleic acids of Tables 1-6. In another embodiment, the sequences are sequence variants as further described herein.

Homology in this context means sequence similarity or identity, with identity being preferred. A preferred comparison for homology purposes is to compare the sequence containing sequencing errors to the correct sequence. This homology will be determined using standard techniques known in the art, including, but not limited to, the local homology algorithm of Smith & Waterman, Adv. Appl Math. 2:482 (1981), by the homology alignment algorithm of Needleman & Wunsch, J. Mol. Biol. 48:443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387-395 (1984), preferably using the default settings, or by inspection.

One example of' a useful algorithm is PILEUP PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351-360 (1987); the method is similar to that described by Higgins & Sharp CABIOS 5:151-153 (1989). Useful PILEUP parameters include a default gap weight of 3.00, a default gap length weight of 0.10, and weighted end gaps

Another example of a useful algorithm is the BLAST (Basic Local Alignment Search Tool) algorithm, described in Altschul et al., J. Mol. Biol. 215, 403-410, (1990) and Karlin et al., PNAS USA 90:5873-5787 (1993). A particularly useful BLAST program is the WU-BLASI-2 program which was obtained from Altschul et al , Methods in Enzymology, 266: 460-480 (1996); http://blast.wustl.edu/]. WU-BLAST-2 uses several search parameters, most of which are set to the default values. The adjustable parameters are set with the following values: overlap span =1, overlap fraction = 0.125, word threshold (T) =11. The HSP S and HSP S2 parameters are dynamic values and are established by the program itself depending upon the composition of the particular sequence and composition of the particular database against which the sequence of interest is being searched; however, the values may be adjusted to increase sensitivity. A percent amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical with the nucleotide residues of the nucleic acids of Tables 1-6. A preferred method utilizes the BLASTN module of WU-BLAST-2 set to the default parameters, with overlap span and overlap faction set to 1 and 0.125, respectively.

The alignment may include the introduction of gaps in the sequences to be aligned. In addition, for sequences which contain either more or fewer nucleotides than those of the nucleic acids of fables 1-6, it is understood that the percentage of homology will be determined based on the number of homologous nucleosides in relation to the total number of nucleosides Thus homology of sequences shorter than those of the sequences identified herein will be determined using the number of nucleosides in the shorter sequence.

In another embodiment of the invention, polynucleotide compositions are provided that are capable of hybridizing under moderate to high stringency conditions to a polynucleotide sequence provided herein;, or a fragment thereof, of a complementary sequence thereof. Hybridization techniques are well known in the art of molecular biology For purposes of illustration, suitable moderately stringent conditions for testing the hybridization of a polynucleotide of this invention with other polynucleotides include prewashing in a solution of 5x SSC ("saline sodium citrate"; 9 mM NaCl, 0.9 mM sodium citrate), 0.5% SDS, 1.0 mM EDTA (pH 80); hybridizing at 50-60° C, 5x SSC, overnight; followed by washing twice at 65° C for 20 minutes with each of 2x, 0.5x and 0.2x SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, e.g., to 60-65° C, or 65-70° C. Stringent conditions may also be achieved with the addition of destabilizing agents such as formamide

Thus nucleic acids that hybridize under high stringency to the nucleic acids identified in the figures, or their complements, are considered CA sequences. High stringency conditions are known in the art; see for example Maniatis et al., Molecular Cloning: A Laboratory Manual, 2d Edition, 1989, and Short Protocols in Molecular Biology, ed. Ausubel, et al., both of which are hereby incorporated by reference Stringent conditions are sequence-dependent and will be different in different circumstances Longer sequences hybridize specifically at higher temperatures An extensive guide to the hybridization of nucleic acids is found in Tijssen, Techniques in Biochemistry and Molecular Biology-Hybridization with Nucleic Acid Probes, "Overview of principles of hybridization and the strategy of nucleic acid assays" (1993). Generally, stringent conditions are selected to be about 5-10°C lower than the thermal melting point ($T_m$) for the specific sequence at a defined ionic strength pH. The $T_m$ is the temperature (under defined ionic strength, pH and nucleic acid concentration) at which 50% of the probes complementary to the target hybridize to the target sequence at equilibrium (as the target sequences are present in excess, at $T_m$, 50% of the probes are occupied at equilibrium) Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e g. 10 to 50 nucleotides) and at least about 60°C for longer probes (e.g greater than 50 nucleotides).. In another embodiment, less stringent hybridization conditions are used; for example, moderate or low stringency conditions may be used, as are known in the art; see Maniatis and Ausubel, supra, and Tijssen, supra

In addition, the CA nucleic acid sequences of the invention are fragments of larger genes, i.e. they are nucleic acid segments Alternatively, the CA nucleic acid sequences can serve as indicators of oncogene position, for example, the CA sequence may be an enhancer that activates a protooncogene. "Genes" in this context includes coding regions, non-coding regions, and mixtures of'coding and non-coding regions, Accordingly, as will be appreciated by those in the art, using the sequences provided herein, additional sequences of the CA genes can be obtained, using techniques well known in the art for cloning either longer sequences or the full-length sequences; see Maniatis et al., and Ausubel, et al, supra, hereby expressly incorporated by reference. In general, this is done using PCR, for example, kinetic PCR.

### Detection of CA Expression

Once the CA nucleic acid is identified, it can be cloned and, if necessary, its constituent parts recombined to form the entire CA nucleic acid Once isolated from its natural source, e.g, contained within a plasmid or other vector or excised therefrom as a linear nucleic acid segment, the recombinant CA nucleic acid can be further used as a probe to identify and isolate other CA nucleic acids, for example additional coding regions It can also be used as a "precursor" nucleic acid to make modified or variant CA nucleic acids and proteins. In a preferred embodiment, once a CA gene is identified its nucleotide sequence is used to design probes specific for the CA gene

The CA nucleic acids of the present invention are used in several ways In a first embodiment, nucleic acid probes hybridizable to CA nucleic acids are made and attached to biochips to be used in screening and diagnostic methods, or for gene therapy and/or antisense applications. Alternatively, the CA nucleic acids that include coding regions of CA proteins can be put into expression vectors for the expression of CA proteins, again either for screening purposes or for administration to a patient.

Recent developments in DNA microarray technology make it possible to conduct a large scale assay of a plurality of target CA nucleic acid molecules on a single solid phase support. U.S. Pat. No. 5,837,832 (Chee et al.) and related patent applications describe immobilizing an array of oligonucleotide probes for hybridization and detection of specific nucleic acid sequences in a sample Target polynucleotides of interest isolated from a tissue of interest axe hybridized to the DNA chip and the specific sequences detected based on the target polynucleotides' reference and degree of hybridization at discrete probe locations. One important use of arrays is in the analysis of differential gene expression, where the profile of expression of genes in different cells, often a cell of interest and a control cell, is compared and any differences in gene expression among the respective cells are identified. Such information is useful for the identification of the types of genes expressed, in a particular cell or tissue type and diagnosis of cancer conditions based on the expression profile

Typically, RNA from the sample of interest is subjected to reverse transcription to obtain labeled cDNA. *See* U.S, Pat No. 5,410,229 (Lockhart *et al.*) The cDNA is then hybridized to oligonucleotides or cDNAs of known sequence arrayed on a chip or other surface in a known order. The location of the oligonucleotide to which the labeled cDNA hybridizes provides sequence information on the cDNA, while the amount of labeled hybridized RNA or cDNA provides an estimate of the relative representation of the RNA or cDNA of interest. *See* Schena, et al. Science 270:467-470 (1995) For example, use of a cDNA microarray to analyze gene expression patterns in human cancer is described by DeRisi, et al. (Nature Genetics 14:457-460 (1996)).

In a preferred embodiment, nucleic acid probes corresponding to CA nucleic acids (both the nucleic acid sequences outlined in the figures and/or the complements thereof) are made. Typically, these probes are synthesized based on the disclosed sequences of this invention, The nucleic acid probes attached to the biochip are designed to be substantially complementary to the CA nucleic acids, i.e. the target sequence (either the target sequence of the sample or to other probe sequences, for example in sandwich assays), such that specific hybridization of the target sequence and the probes of the present invention occurs As outlined below, this complementarity need not be perfect, in that there may be any number of base pair mismatches that will interfere with hybridization between the target sequence and the single stranded nucleic acids of the present invention. It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions, as outlined herein. Whether or not a sequence is unique to a CA gene according to this invention can be determined by techniques known to those of skill in the art. For example, the sequence can be compared to sequences in databanks, e.g., GeneBank, to determine whether it is present in the uninfected host or other organisms. The sequence can also be compared to the known sequences of other viral agents, including those that are known to induce cancer

A nucleic acid probe is generally single stranded but can be partly single and partly double stranded The strandedness of the probe is dictated by the structure, composition, and properties of'the target sequence. In general, the oligonucleotide probes range from about 6, 8, 10, 12, 15, 20, 30 to about 100 bases long, with from about 10 to about 80 bases being preferred, and from about 30 to about 50 bases being particularly preferred That is, generally entire genes are rarely used as probes. In some embodiments, much longer nucleic acids can be used, up to hundreds of' bases The probes are sufficiently specific to hybridize to complementary template sequence under conditions known by those of'skill in the art The number of mismatches between the probes sequences and their complementary template (target) sequences to which they hybridize during Hybridization generally do not exceed 15%, usually do not exceed 10% and preferably do not exceed 5%, as determined by FASTA (default settings).

Oligonucleotide probes can include the naturally-occurring heterocyclic bases normally found in nucleic acids (uracil, cytosine, thymine, adenine and guanine), as well as modified bases and base analogues Any modified base or base analogue compatible with hybridization of the probe to a target sequence is useful in the practice of the invention The sugar or glycoside portion of the probe can comprise deoxyribose, ribose, and/or modified forms of these sugars, such as, for example, 2' - O-alkyl ribose. In a preferred embodiment, the sugar moiety is 2'-deoxyribose; however, any sugar moiety that is compatible with the ability of the probe to hybridize to a target sequence can be used

In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (*e.g,* U.S. Patent No. 5,470,967) and polyamide (*i.e*., peptide nucleic acids). Peptide nucleic acids are described in Nielsen et al (1991) Science 254: 1497-1500, U S Patent No , 5,714,331, and Nielsen (1999) Curr. Opin. Biotechnol. 10:71-75.

In certain embodiments, the probe can be a chimeric molecule; *i.e* , can comprise more than one type of base or sugar subunit, and/or the linkages can be of more than one type within the same primer The probe can comprise a moiety to facilitate hybridization to its target sequence, as are known in the art, for example, intercalators and/or minor groove binders Variations of the bases, sugars, and internucleoside backbone, as well as the presence of any pendant group on the probe, will be compatible with the ability of the probe to bind, in a sequence-specific fashion, with its target sequence. A large number of structural modifications, both known and to be developed, are possible within these bounds. Advantageously, the probes according to the present invention may have structural characteristics such that they allow the signal amplification, such structural characteristics being, for example, branched DNA probes as those described by Urdea et al (Nucleic Acids Symp. Ser., 24:197-200 (1991)) or in the European Patent No. EP-0225,807 Moreover, synthetic methods for preparing the various heterocyclic bases, sugars, nucleosides and nucleotides that form the probe, and preparation of'oligonucleotides of specific predetermined sequence, are well-developed and known in the art. A preferred method for oligonucleotide synthesis incorporates the teaching of US Patent No. 5,419,966.

Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target CA gene are used. That is, two, three, four or more probes, with three being preferred, are used to build in a redundancy for a particular- target. The probes can be overlapping (ie have some sequence in common), or specific for distinct sequences of a CA gene. When multiple target polynucleotides are to be detected according to the present invention, each probe or probe group corresponding to a particular target polynucleotide is situated in a discrete area of the microarray.

Probes may be in solution, such as in wells or on the surface of a micro-array, or attached to a solid support. Examples of solid support materials that can be used include a plastic, a ceramic, a metal, a resin, a gel and a membrane Useful types of solid supports include plates, beads, magnetic material, microbeads, hybridization chips, membranes, crystals, ceramics and self-assembling monolayers. A preferred embodiment comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the chip can assist in the identification of the target nucleotide sequence Patterns can be manually or computer analyzed, but it is clear that positional sequencing by hybridization lends itself to computer analysis and automation Algorithms and software, which have been developed for sequence reconstruction, are applicable to the methods described herein (R Drmanac et al., J. Biomol. Struc. & Dyn. 5:1085-1102,1991; P. A. Pevzner, J. Biomol. Struc. & Dyn. 7:63-73,1989).

As will be appreciated by those in the art, nucleic acids can be attached or immobilized to a solid support in a wide variety of ways By "immobilized" herein is meant the association or binding between the nucleic acid probe and the solid support is sufficient to be stable under the conditions of binding, washing, analysis, and removal as outlined below. The binding can be covalent or non-covalent By "non-covalent binding" and grammatical equivalents herein is meant one or more of' either electrostatic, hydrophilic, and hydrophobic interactions. Included in non-covalent binding is the covalent attachment of a molecule, such as streptavidin, to the support and the non-covalent binding of the biotinylated probe to the streptavidin By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules . Immobilization may also involve a combination of covalent and non-covalent interactions.

Nucleic acid probes may be attached to the solid support by covalent binding such as by conjugation with a coupling agent or by, covalent or non-covalent binding such as electrostatic interactions, hydrogen bonds or antibody-antigen coupling, or by combinations thereof Typical coupling agents include biotin/avidin, biotin/streptavidin, Staphylococcus aureus protein A/IgG antibody $F_c$ fragment, and streptavidin/protein A chimeras (T. Sano and C. R Cantor, Bio/Technology 9:1378-81 (1991)), or derivatives or combinations of these agents Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative Derivatives which have been found to be useful include 3 or 4 [bis-(4-methoxyphenyl)]-methyl benzoic acid, N-succinimidyl-3 or 4 [bis-(4-methoxyphenyl)]-methyl-benzoic acid, N-succinimidyl-3 or 4 [bis-(4-methoxyphenyl)] -hydroxymethyl-benzoic acid, N-succinimidyl-3 or 4 [bis-(4-methoxyphonyl)]-chloromethyl-benzoic acid, and salts of these acids.

In general, the probes are attached to the biochip in a wide variety of ways, as will be appreciated by those in the art As described herein, the nucleic acids can either be synthesized first, with subsequent attachment to the biochip, or can be directly synthesized on the biochip.

The biochip comprises a suitable solid substrate By "substrate" or "solid support" or other grammatical equivalents herein is meant any material that can be modified to contain discrete individual sites appropriate for the attachment or association of the nucleic acid probes and is amenable to at least one detection method The solid phase support of the present invention can be of any solid materials and structures suitable for supporting nucleotide hybridization and synthesis. Preferably, the solid phase support comprises at least one substantially rigid surface on which the primers can be immobilized and the reverse transcriptase reaction performed The substrates with which the polynucleotide microarray elements are stably associated may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon®, fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads Each tagged bead has different primers attached to it. Tags are detectable by signaling means such as color (Luminex, Illumina) and electromagnetic field (Phaimaseq) and signals on tagged beads can even be remotely detected (e,g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.

In a preferred embodiment, the surface of the biochip and the probe may be derivatized with chemical functional groups for subsequent attachment of the two Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes For example, nucleic acids containing amino groups can be attached to surfaces comprising amino groups, for example using linkers as are known in the art; for example, homo-or hetero-bifunctional linkers as are well known (see 1994 Pierce Chemical Company catalog, technical section on cross-linkers, pages 155-200, incorporated herein by reference). In addition, in some cases, additional linkers, such as alkyl groups (including substituted and heteroalkyl groups) may be used

In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleosides. In an additional embodiment, the immobilization to the solid support may be very strong, yet non-covalent. For example, biotinylated oligonucleotides can be made, which bind to surfaces covalently coated with streptavidin, resulting in attachment.

The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), US. Pat. No, 5,384,261 (in situ synthesis by mechanically directed flow paths); and US. Pat. No. 5,700,637 (synthesis by spotting, printing or coupling); the disclosure of which are herein incorporated in their entirety by reference, Another method for coupling DNA to beads uses specific ligands attached to the end of the DNA to link to ligand-binding, molecules attached to a bead. Possible ligand-binding partner pairs include biotin-avidin/streptavidin, or various antibody/antigen pairs such as digoxygenin-antidigoxygenin antibody (Smith et al., "Direct Mechanical Measurements of'the Elasticity of Single DNA Molecules by Using Magnetic Beads," Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established, See Pease et al., Proc. Natl. Acad. Sci USA 91(11):5022-5026 (1994). A preferred method of attaching oligonucleotides to solid-state substrates is describe by Guo et al., Nucleic Acids Res. 22:5456-5465 (1994) Immobilization can be accomplished either by in situ DNA synthesis (Maskos and Southern, Nucleic Acids Research, 20:1679-1684 (1992) or by covalent attachment of chemically synthesized oligonucleotides (Guo et al., *supra*) in combination with robotic arraying technologies.

In addition to the solid phase technology represented by biochip arrays, gene expression can also be quantified using liquid-phase arrays. One such system is kinetic polymerase chain reaction (PCR). Kinetic PCR allows for the simultaneous amplification and quantification of specific nucleic acid sequences. The specialty is derived from synthetic oligonucleotide primers designed to preferentially adhere to single-stianded nucleic acid sequences bracketing the target site. This pair of oligonucleotide primers form specific, non-covalently bound complexes on each strand of the target sequence. These complexes facilitate *in vitro* transcription of double-stranded DNA in opposite orientations. Temperature cycling of the reaction mixture creates a continuous cycle of primer binding, transcription, and re-melting of the nucleic acid to individual strands The result is an exponential increase of the target dsDNA product This product can be quantified in real time either through the use of an intercalating dye or a sequence specific probe SYBR® Greene I, is an example of an intercalating dye, that preferentially binds to dsDNA resulting in a concomitant increase in the fluorescent signal. Sequence specific probes, such as used with TaqMan® technology, consist of a fluorochrome and a quenching molecule covalently bound to opposite ends of an oligonucleotide The probe is designed to selectively bind the target DNA sequence between the two primers When the DNA stands are synthesized during the PCR reaction, the fluorochrome is cleaved from the probe by the exonuclease activity of the polymerase resulting in signal dequenching. The probe signaling method can be more specific than the intercalating dye method, but in each case, signal strength is proportional to the dsDNA product produced Each type of quantification method can be used in multi-well liquid phase arrays with each well representing primers and/or probes specific to nucleic acid sequences of interest When used with messenger RNA preparations of tissues or cell lines, an array of' probe/primer reactions can simultaneously quantify the expression of multiple gene products of interest. See Germer, S, et al., Genome Res. 10:258-266 (2000); Heid, C. A, et al., Genome Res. 6, 986-994 (1996)

**Expression of CA proteins**

In a preferred embodiment, CA nucleic acids encoding CA proteins are used to make a variety of expression vectors to express CA proteins which can then be used in screening assays, as described below. The expression vectors may be either self-replicating extrachromosomal vectors or vectors which integrate into a host genome. Generally, these expression vectors include transcriptional and translational regulatory nucleic acid operably linked to the nucleic acid encoding the CA protein. The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked means that the DNA sequences being linked are contiguous, and, in the case of a secretory leader, contiguous and in reading phase. However, enhancers do not have to be contiguous, Linking is accomplished by ligation at convenient restriction sites. If such sites do not exist, synthetic oligonucleotide adaptors or linkers are used in accordance with conventional practice. The transcriptional and translational regulatory nucleic acid will generally be appropriate to the host cell used to express the CA protein; for example, transcriptional and translational regulatory nucleic acid sequences from *Bacillus* are preferably used to express the CA protein in *Bacillus.* Numerous types of appropriate expression vectors, and suitable regulatory sequences are known in the art for a variety of host cells

In general, the transcriptional and translational regulatory sequences may include, but are not limited to, promoter sequences, ribosomal binding sites, transcriptional start and stop sequences, translational start and stop sequences, and enhancer or activator sequences In a preferred embodiment, the regulatory sequences include a promoter and transcriptional start and stop sequences,

Promoter sequences encode either constitutive or inducible promoters. The promoters may be either naturally occurring promoters or hybrid promoters. Hybrid promoters, which combine elements of more than one promoter, are also known in the art, and are useful in the present invention.

In addition, the expression vector may comprise additional elements. For example, the expression vector may have two replication systems, thus allowing it to be maintained in two organisms, for example in mammalian or insect cells for expression and in a prokaryotic host for cloning and amplification Furthermore, for integrating expression vectors, the expression vector contains at least one sequence homologous to the host cell genome, and preferably two homologous sequences that flank the expression, construct The integrating vector may be directed to a specific locus in the host cell by selecting the appropriate homologous sequence for inclusion in the vector Constructs for integrating vectors are well known in the art

In addition, in a preferred embodiment, the expression vector contains a selectable marker gene to allow the selection of transformed host cells Selection genes are well known in the art and will vary with the host cell used

The CA proteins of the present invention are produced by culturing a host cell transformed with an expression vector containing nucleic acid encoding a CA protein, under the appropriate conditions to induce or cause expression of the CA protein The conditions appropriate for CA protein expression will vary with the choice of the expression vector and the host cell, and will be easily ascertained by one skilled in the art through routine experimentation. For example, the use of constitutive promoters in the expression vector will require optimizing the growth and proliferation of the host cell, while the use of' an inducible promoter requires the appropriate growth conditions for induction. In addition, in some embodiments, the timing of the harvest is important For example, the baculoviral systems used in insect cell expression are lytic viruses, and thus harvest time selection can be crucial for product yield.

Appropriate host cells include yeast, bacteria, archaebacteria, fungi, and insect, plant and animal cells, including mammalian cells. Of particular interest are *Drosophila melanogaster* cells, *Saccharomyces cerevisiae* and other yeasts, *E. coli, Bacillus subtilis,* Sf9 cells, C129 cells, 293 cells, *Neurospora,* BHK, CHO, COS, HeLa cells, THP1 cell line (a macrophage cell line) and human cells and cell lines

In a preferred embodiment, the CA proteins are expressed in mammalian cells. Mammalian expression systems are also known in the art, and include retroviral system A preferred expression vector system is a retroviral vector system such as is generally described in PCT/US97/01019 and PCT/US97/01048, both of which are hereby expressly incorporate by reference. Of particular use as mammalian promoters are the promoters from mammalian viral genes, since the viral genes are often highly expressed and have a broad host range Examples include the SV40 early promoter, mouse mammary tumor virus LTR promoter, adenovirus major late promoter, herpes simplex virus promoter, and the CMV promoter. Typically, transcription termination and polyadenylation sequences recognized by mammalian cells are regulatory regions located 3' to the translation stop codon and thus, together with the promoter elements, flank the coding sequence. Examples of transcription terminator and polyadenylation signals include those derived form SV40.

The methods of introducing exogenous nucleic acid into mammalian hosts, as well as other hosts, are well known in the art, and will vary with the host cell used Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nucleic,

In a preferred embodiment, CA proteins are expressed in bacterial systems Bacterial expression systems are well known in the art Promoters from bacteriophage may also be used and are known in the art. In addition, synthetic promoters and hybrid promoters are also useful; for example, the tac promoter is a hybrid of the trp and lac promoter sequences furthermore, a bacterial promoter can include naturally occurring promoters of non-bacterial origin that have the ability to bind bacterial RNA polymerase and initiate transcription. In addition to a functioning promoter sequence, an efficient ribosome binding site is desirable. The expression vector may also include a signal peptide sequence that provides for secretion of the CA protein in bacteria. The protein is either secreted into the growth media (gram-positive bacteria) or into the periplasmic space, located between the inner and outer membrane of the cell (gram-negative bacteria) The bacterial expression vector may also include a selectable marker gene to allow for the selection of bacterial strains that have been transformed. Suitable selection genes include genes that render the bacteria resistant to drugs such as ampicillin, chloramphenicol, erythromycin, kanamycin, neomycin and tetracycline. Selectable markers also include biosynthetic genes, such as those in the histidine, tryptophan and leucine biosynthetic pathways. These components are assembled into expression vectors. Expression vectors for bacteria are well known in the art, and include vectors for *Bacillus subtilis, E. coli, Streptococcus cremoris,* and *Streptococcus lividans,* among others, The bacterial expression vectors are transformed into bacterial host cells using techniques well known in the art, such as calcium chloride treatment, electroporation, and others.

In one embodiment, CA proteins are produced in insect cells Expression vectors for the transformation of insect cells, and in particular, baculovirus-based expression vectors, are well known in the art.

In a preferred embodiment, CA protein is produced in yeast cells. Yeast expression systems are well known in the art, and include expression vectors for *Saccharomyces cerevisiae, Candida albicans* and *C. maltosa, Hanseraula polymorpha, Kluyveromyces fragilis* and *K. lactis, Pichia guillerimondii* and *P. pastoris, Schizosaccharomyces pombe,* and *Yarrowia lipolytica*.

The CA protein may also be made as a fusion protein, using techniques well known in the art, Thus, for example, for the creation of monoclonal antibodies. If the desired epitope is small, the CA protein may be fused to a carrier protein to form an immunogen. Alternatively, the CA protein may be made as a fusion protein to increase expression, or for other reasons. For example, when the CA protein is a CA peptide, the nucleic acid encoding the peptide may be linked to other nucleic acid for expression purposes

In one embodiment, the CA nucleic acids, proteins and antibodies of the invention are labeled. By "labeled" herein is meant that a compound has at least one element, isotope or chemical compound attached to enable the detection of the compound. In general, labels fall into three classes: a) isotopic labels, which may be radioactive or heavy isotopes; b) immune labels, which may be antibodies or antigens; and c) colored or fluorescent dyes. The labels may be incorporated into the CA nucleic acids, proteins and antibodies at any position For example, the label should be capable of producing, either directly or indirectly, a detectable signal The detectable moiety may be a radioisotope, such as $^{3}H$, $^{14}C$, $^{32}P$, $^{35}S$, or $^{125}I$, a fluorescent or chemiluminescent compound, such as fluorescein isothiocyanate, rhodamine, or luciferin, or an enzyme, such as alkaline phosphatase, beta-galactosidase or horseradish peroxidase Any method known in the art for conjugating the antibody to the label may be employed, including those methods described by Hunter et al., .Nature, 144:945 (1962); David et al, Biochemistry, 13:1014 (1974); Pain et al., J. Immunol. Meth , 40: 219 (1981); and Nygren, J. Histochem. and Cytochem, 30:407 (1982).

Accordingly, the present invention also provides CA protein sequences.. A CA protein of the present invention may be identified in several ways. "Protein" in this sense includes proteins, polypeptides, and peptide, As will be appreciated by those in the art, the nucleic acid sequences of the invention can be used to generate protein sequences There are a variety of ways to do this, including cloning the entire gene and verifying its frame and amino acid sequence, or by comparing it to known sequences to search for homology to provide a frame, assuming the CA protein has homology to some protein in the database being used. Generally, the nucleic acid sequences are input into a program that will search all three frames for homology This is done in a preferred embodiment using the following NCBI Advanced BLAST parameters. The program is blastx or blastn The database is nr. The input data is as "Sequence in FASTA format". The organism list is "none". The "expect" is 10; the filter is default. The "descriptions" is 500, the "alignments" is 500, and the "alignment view" is pairwise. The "query Genetic Codes" is standard (1). The matrix is BLOSUM 62; gap existence cost is 11, per residue gap cost is 1; and the lambda ratio is 85 default. This results in the generation of a putative protein sequence

In general, the term "polypeptide" as used herein refers to both the full-length polypeptide encoded by the recited polynucleotide, the polypeptide encoded by the gene represented by the recited polynucleotide, as well as portions or fragments thereof The present invention encompasses variants of the naturally occurring proteins, wherein such variants are homologous or substantially similar to the naturally occurring protein, and can be of an origin of'the same or different species as the naturally occurring protein (*e.g.*, human, murine, or some other species that naturally expresses the recited polypeptide, usually a mammalian species). In general, variant polypeptides have a sequence that has at least about 80%, at least about 81 %, at least about 82%, at least about 83%, at least about 84%, at least about 85%, at least about 86%, at least about 87%, at least about 88%, at least about 89%, usually at least about 90%, at least 91%, at least 92%, at least 93%, at least 94%, at least 95%, at least 96%, at least 97%, at least 98% and more usually at least about 99% sequence identity with a differentially expressed polypeptide described herein, as determined by the Smith-Waterman homology search algorithm using an affine gap search with a gap open penalty of 12 and a gap extension penalty of 2, BLOSUM matrix of 62. The Smith Waterman homology search algorithm is taught in Smith and Waterman, Adv Appl. Math. (1981) 2: 482-489. The variant polypeptides can be naturally or non-naturally glycosylated, *i.e.*, the polypeptide has a glycosylation pattern that differs from the glycosylation pattern found in the corresponding naturally occurring protein

Also within the scope of the invention are variant. Variants of polypeptides include mutants, fragments, and fusions, Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general change, hydrophobicity/ hydrophilicity, and/or steric bulk of the amino acid substituted Variants can be designed so as to retain or have enhanced biological activity of a particular region of'the protein (*e.g.*, a functional domain and/or, where the polypeptide is a member of a protein family, a region associated with a consensus sequence). Selection of amino acid alterations for production of variants can be based upon the accessibility (interior vs exterior) of the amino acid (see, e.g., Go et al, Int, J. Peptide Protein Res. (1980) 15:211), the thermostability of the variant polypeptide (see, e.g., Querol et al., Prot. Eng. (1996) 9:265), desired glycosylation sites (see, e.g., Olsen and Thomsen, J. Gen. Microbiol. (1991) 137:579), desired disulfide bridges (see, e.g., Clarke et al., Biochemistry (1993) 32:4322; and Wakarchuk et al., Protein Eng. (1994) 7:1379), desired metal binding sites (see, e.g., Ioma et al., Biochemistry (1991) 30:97, and Haezerbrouck et al., Protein Eng. (1993) 6:643), and desired substitutions within proline loops (see, e g., Masul et al., Appl. Env. Microbiol. (1994) 60:3579). Cysteine-depleted muteins can be produced as disclosed in USPN 4,959,314.

Variants also include fragments of the polypeptides disclosed herein, particularly biologically active fragments and/or fragments corresponding to functional domains. Fragments of interest will typically be at least about 8 amino acids (aa) 10 aa, 15 aa, 20 aa, 25 aa, 30 aa, 35 aa, 40 aa, to at least about 45 aa in length, usually at least about 50 aa in length, at least about 75 aa, at least about 100 aa, at least about 125 aa, at least about 150 aa in length, at least about 200 aa, at least about 300 aa, at least about 400 aa and can be as long as 500 aa in length or longer, but will usually not exceed about 1000 aa in length, where the fragment will have a stretch of amino acids that is identical to a polypeptide encoded by a polynucleotide having a sequence of any one of the polynucleotide sequences provided herein, or a homolog thereof. The protein variants described herein are encoded by polynucleotides that are within the scope of the invention. The genetic code can be used to select the appropriate codons to construct the corresponding variants.

While altered expression of'the polynucleotides associated with cancer is observed, altered levels of expression of the polypeptides encoded by these polynucleotides may likely play a role in cancers.

Also included within one embodiment of CA proteins are amino acid variants of the naturally occurring sequences, as determined herein. Preferably, the valiant are preferably greater than about 75% homologous to the wild-type sequence, more preferably greater than about 80%, even more preferably greater than about 85% and most preferably greater than 90%., The present application is also directed to proteins containing polypeptides at least 80%, 85%, 90%, 95%, 96%, 97%, 98% or 99% identical to a CA polypeptide sequence set forth herein. As for nucleic acids, homology in this context means sequence similarity or identity, with identity being preferred. This homology will be determined using standard techniques known in the art as are outlined above for the nucleic acid homologies.

CA proteins of the present invention may be shorter or longer than the wild type amino acid sequence. Thus, in a preferred embodiment, included within the definition of CA proteins are portions or fragments of the wild type sequences herein. In addition, as outlined above, the CA nucleic acids of'the invention may be used to obtain additional coding regions, and thus additional protein sequence, using techniques known in the art.

In a preferred embodiment, the CA proteins are derivative or variant CA proteins as compared to the wild-type sequence, That is, as outlined more fully below, the derivative CA peptide will contain at least one amino acid substitution, deletion or insertion, with amino acid substitutions being particularly preferred. The amino acid substitution, insertion or deletion may occur at any residue within the CA peptide.

Also included in an embodiment of CA proteins of the present invention are amino acid sequence variants. These variants fall into one or more of three classes: substitutional, insertional or deletional variants These variants ordinarily are prepared by site-specific mutagenesis of nucleotides in the DNA encoding the CA protein, using cassette or PCR mutagenesis or other techniques well known in the art, to produce DNA encoding the variant, and thereafter expressing the DNA in recombinant cell culture as outlined above. However, variant CA protein fragments having up to about 100-150 residues may be prepared by *in vitro* synthesis using established techniques Amino acid sequence variants are characterized by the predetermined nature of the variation, a feature that sets them apart from naturally occurring allelic or interspecies variation of the CA protein amino acid sequence. The variants typically exhibit the same qualitative biological activity as the naturally occurring analogue, although variants can also be selected which have modified characteristics as will be more fully outlined below,

While the site or region for introducing an amino acid sequence variation is predetermined, the mutation per se need not be predetermined For example, in order to optimize the performance of a mutation at a given site, random mutagenesis may be conducted at the target codon or region and the expressed CA variants screened for the optimal combination of desired activity. Techniques for making substitution mutations at predetermined sites in DNA having a known sequence are well known, for example, M13 primer mutagenesis and LAR mutagenesis. Screening of the mutants is done using assays of CA protein activities.

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the CA protein are desired, substitutions are generally made in accordance with the following chart:

**Chart 1**

| Original Residue | Exemplary Substitutions |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Substantial changes in function or immunological identity are made by selecting substitutions that are less conservative than those shown in Chat I. For example, substitutions may be made full length to more significantly affect one or more of the following: the structure of the polypeptide backbone in the area of the alteration (e.g., the alpha-helical or beta-sheet structure); the charge or hydrophobicity of the molecule at the target site; and the bulk of the side chain. The substitutions which in general are expected to produce the greatest changes in the polypeptide's properties are those in which (a) a hydrophilic residue, e.g., seryl or threonyl is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g., glycine.

The variants typically exhibit the same qualitative biological activity and will elicit the same immune response as the naturally-occurring analogue, although variants also are selected to modify the characteristics of the CA proteins as needed Alternatively, the variant may be designed such that the biological activity of the CA protein is altered. For example, glycosylation sites may be altered or removed, dominant negative mutations created, etc.

Covalent modifications of CA polypeptides are included within the scope of this invention, for example for use in screening. One type of covalent modification includes reacting targeted amino acid residues of a CA polypeptide with an organic derivatizing agent that is capable of reacting with selected side chains or the N-or C-terminal residues of a CA polypeptide. Derivatization with bifunctional agents is useful, for instance, for crosslinking CA polypeptides to a water-insoluble support matrix or surface for use in the method for purifying anti-CA antibodies or screening assays, as is more fully described below. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl, threonyl or tyrosyl residues, methylation of the a-amino groups of lysine, arginine, and histidine side chains [IE. Creighton, Proteins: Structure and Molecular Properties, W.H Freeman & Co., San Francisco, pp 79-86 (1983)], acetylation of the N-terminal amine, and amidation of any C-terminal carboxyl group.

Another type of covalent modification of the CA polypeptide included within the scope of this invention comprises altering the native glycosylation pattern of the polypeptide. "Altering the native glycosylation pattern" is intended for purposes herein to mean deleting one or more carbohydrate moieties found in native sequence CA polypeptide, and/or adding one or more glycosylation sites that are not present in the native sequence CA polypeptide.

Addition of glycosylation sites to CA polypeptides may be accomplished by altering the amino acid sequence thereof. The alteration may be made, for example, by the addition of, or substitution by, one or more serine or threonine residues to the native sequence CA polypeptide (for O-linked glycosylation sites) The CA amino acid sequence may optionally be altered through changes at the DNA level, particularly by mutating the DNA encoding the CA polypeptide at preselected bases such that codons are generated that will translate into the desired amino acids

Another means of increasing the number of carbohydrate moieties on the CA polypeptide is by chemical or enzymatic coupling of glycosides to the polypeptide. Such methods are described in the art, e.g., in WO 87/05330 published 11 September 1987, and in Aplin and Wriston, LA Crit. Rev. Biochem., pp 259-306 (1981).

Removal of carbohydrate moieties present on the CA polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo-and exo-glycosidases as described by Thotakura et al., Meth. Enzymol, 138:350 (1987).

Another type of covalent modification of CA comprises linking the CA polypeptide to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

CA polypeptides of the present invention may also be modified in a way to form chimeric molecules comprising a CA polypeptide fused to another, heterologous polypeptide or amino acid sequence. In one embodiment, such a chimeric molecule comprises a fusion of a CA polypeptide with a tag polypeptide that provides an epitope to which an anti-tag antibody can selectively bind. The epitope tag is generally placed at the amino-or carboxyl-terminus of the CA polypeptide, although internal fusions may also be tolerated in some instances. The presence of such epitope-tagged forms of a CA polypeptide can be detected using an antibody against the tag polypeptide. Also, provision of the epitope tag enables the CA polypeptide to be readily purified by affinity purification using an anti-tag antibody or another type of affinity matrix that binds to the epitope tag. In an alternative embodiment, the chimeric molecule may comprise a fusion of a CA polypeptide with an immunoglobulin or a particular region of an immunoglobulin. For a bivalent form of the chimeric molecule, such a fusion could be to the Fc region of an IgG molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)] Other tag polypeptides include the Flag-peptide [Flopp et al., BioTechnology, 6:1204-1210 (1988)]; the KI3 epitope peptide [Martin et al., Science, 255:192-194 (1992)]; tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci., USA, 87:6393-6397 (1990)].

Also included with the definition of CA protein in one embodiment are other CA proteins of the CA family, and CA proteins from other organisms, which are cloned and expressed as outlined below. Thus, probe or degenerate polymerase chain reaction (PCR) primer sequences may be used to find other related CA proteins from humans or other organism. As will be appreciated by those in the art, particularly useful probe and/or PCR primer sequences include the unique areas of the CA nucleic acid sequence. As is generally known in the art, preferred PCR primers are from about 15 to about 35 nucleotides in length, with from about 20 to about 30 being preferred, and may contain inosine as needed. The conditions for the PCR reaction are well known in the art.

In addition, as is outlined herein, CA proteins can be made that are longer than those encoded by the nucleic acids of the figures, for example, by the elucidation of' additional sequences, the addition of epitope or purification tags, the addition of other fusion sequences, etc

CA proteins may also be identified as being encoded by CA nucleic acids. Thus, CA proteins are encoded by nucleic acids that will hybridize to the sequences of the sequence listings, or their complements, as outlined herein.

**CA antigens and antibodies thereto**

In one embodiment, the invention provides CA specific antibodies. **In a** preferred embodiment, when the CA protein is to be used to generate antibodies, for example for immunotherapy, the CA protein should share at least one epitope or determinant with the full-length protein By "epitope" or "determinant" herein is meant a portion of a protein that will generate and/or bind an antibody or T-cell receptor in the context of MHC Thus, in most instances, antibodies made to a smaller CA protein will be able to bind to the full-length protein. In a preferred embodiment, the epitope is unique; that is, antibodies generated to a unique epitope show little or no cross-reactivity.

Any polypeptide sequence encoded by the CA polynucleotide sequences may be analyzed to determine certain preferred regions of the polypeptide. Regions of high antigenicity are determined from data by DNASTAR analysis by choosing values that represent regions of the polypeptide that are likely to be exposed on the surface of the polypeptide in an environment in which antigen recognition may occur in the process of initiation of'an immune response. For example, the amino acid sequence of a polypeptide encoded by a CA polynucleotide sequence may be analyzed using the default parameters of'the DNASTAR computer algorithm (DNASTAR, Inc., Madison, Wis.; http://www.dnastar.com/).

Polypeptide features that may be routinely obtained using the DNASTAR computer algorithm include, but are not limited to, Garnier-Robson alpha-regions, beta-regions, turn-regions, and coil-regions (Garnier et al. J. Mol. Biol., 120: 97 (1978)); Chou-Fasman alpha-regions, beta-regions, and turn-regions (Adv in Enzymol., 47:45-148 (1978)); Kyte-Doolittle hydrophilic regions and hydrophobic regions (J Mol. Biol., 157:105-132 (1982)); Eisenberg alpha- and beta-amphipathic regions; Karplus-Schulz flexible regions; Emini surface-forming regions (J. Virol., 55(3):836-839 (1985)); and Jameson-Wolf regions of high antigenic index (CABIOS, 4(1):181-186 (1988)). Kyte-Doolittle hydrophilic regions and hydrophobic regions, Emini surface-forming regions, and Jameson-Wolf regions of high antigenic index (i.e., containing four or more contiguous amino acids having an antigenic index of greater than or equal to 1.5, as identified using the default parameters of the Jameson-Wolf program) can routinely be used to determine polypeptide regions that exhibit a high degree of potential for antigenicity. One approach for preparing antibodies to a protein is the selection and preparation of an amino acid sequence of all or part of the protein, chemically synthesizing the sequence and injecting it into an appropriate animal, typically a rabbit, hamster or a mouse. Oligopeptides can be selected as candidates for the production of an antibody to the CA protein based upon the oligopeptides lying in hydrophilic regions, which are thus likely to be exposed in the mature protein. Additional oligopeptides can be determined using, for example, the Antigenicity Index, Welling, G.W. et al., FEBS Lett. 188:215-218 (1985), incorporated herein by reference.

In one embodiment, the term "antibody" includes antibody fragments, as are known in the art, including Fab, $Fab_2$, single chain antibodies (Fv for example), chimeric antibodies, etc., either produced by the modification of whole antibodies or those synthesized de novo using recombinant DNA technologies.

Methods of preparing polyclonal antibodies are known to the skilled artisan. Polyclonal antibodies can be raised in a mammal, for example, by one or more injections of an immunizing agent and, if desired, an adjuvant.. Typically, the immunizing agent and/or adjuvant will be injected in the mammal by multiple subcutaneous or intraperitoneal injections. The immunizing agent may include a protein encoded by a nucleic acid of the figures or fragment thereof or a fusion protein thereof. It may be useful to conjugate the immunizing agent to a protein known to be immunogenic in the mammal being immunized Examples of such immunogenic proteins include but are not limited to keyhole limpet hemocyanin, serum albumin, bovine thyroglobulin, and soybean trypsin inhibitor Examples of adjuvants that may be employed include Freund's complete adjuvant and MPL-TDM adjuvant (monophosphoryl Lipid A, synthetic trehalose dicorynomycolate), The immunization protocol may be selected by one skilled in the art without undue experimentation.

The antibodies may, alternatively, be monoclonal antibodies Monoclonal antibodies may be prepared using hybridoma methods, such as those described by Kohler and Milstein, Nature, 256:495 (1975). In a hybridoma method, a mouse, hamster, or other appropriate host animals, is typically immunized with an immunizing agent to elicit lymphocytes that produce or are capable of producing antibodies that will specifically bind to the immunizing agent. Alternatively, the lymphocytes may be immunized *in vitro* The immunizing agent will typically include a polypeptide encoded by a nucleic acid of Tables 1-6, or fragment thereof or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Monoclonal antibody technology is used in implementing research, diagnosis and therapy. Monoclonal antibodies are used in radioimmunoassays, enzyme-linked immunosorbent assays, immunocytopathology, and flow cytometry for *in vitro* diagnosis, and *in vivo* for diagnosis and immunotherapy of human disease Waldmann, T. A, (1991) Science 252:1657-1662. In particular, monoclonal antibodies have been widely applied to the diagnosis and therapy of cancer, wherein it is desirable to target malignant lesions while avoiding normal tissue. See, e.g., U.S. Pat.. Nos 4,753,894 to Frankel, et al.; 4,938,948 to Ring et al.; and 4,956,453 to Bjorn et al.

In one embodiment, the antibodies are bispecific antibodies Bispecific antibodies are monoclonal, preferably human or humanized, antibodies that have binding specificities for at least two different antigens. A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al., (1989) Proc. Nat. Acad. Sci USA 86:4220-4224; Shaw et al. (1987) J Immunol 138:4534-4538; and Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients. In the present case, one of the binding specificities is for a protein encoded by a nucleic acid of Tables 1-6, or a fragment thereof, the other one is for any other antigen, and preferably for a cell-surface protein or receptor or receptor subunit, preferably one that is tumor specific.

In a referred embodiment, the antibodies to CA are capable of reducing or eliminating the biological function of CA, as is described below. That is, the addition of anti-CA antibodies (either polyclonal or preferably monoclonal) to CA (or cells containing CA) may reduce or eliminate the CA activity. Generally, at least a 25% decrease in activity is preferred, with at least about 50% being particularly preferred and about a 95-100% decrease being especially preferred.

In a preferred embodiment the antibodies to the CA proteins are humanized antibodies "Humanized" antibodies refer to a molecule having an antigen binding site that is substantially derived from an immunoglobulin from a non-human species and the remaining immunoglobulin structure of the molecule based upon the structure and/or sequence of a human immunoglobulin. The antigen binding site may comprise either complete variable domains fused onto constant domains or only the complementarity determining regions (CDRs) grafted onto appropriate framework regions in the variable domains. Antigen binding sites may be wild type or modified by one or more amino acid substitutions, e.g., modified to resemble human immunoglobulin more closely. Alternatively, a humanized antibody may be derived from a chimeric antibody that retains or substantially retains the antigen-binding properties of the parental, non-human, antibody but which exhibits diminished immunogenicity as compared to the parental antibody when administered to humans The phrase "chimeric antibody," as used herein, refers to an antibody containing sequence derived from two different antibodies (*see, e.g,* U.S. Patent No 4,816,567) that typically originate from different species. Typically, in these chimeric antibodies, the variable region of both light and heavy chains mimics the variable regions of antibodies derived from one species of mammals, while the constant portions are homologous to the sequences in antibodies derived from another. Most typically, chimeric antibodies comprise human and murine antibody fragments, generally human constant and mouse variable region. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues that are found neither in the recipient antibody nor in the imported CDR or framework sequences In general, the humanized antibody will comprise substantially all of at least one, and typically two, variable domains, in which all or substantially all of the CDR regions correspond to those of a non-human immunoglobulin and all or substantially all of the framework residues (FR) regions are those of a human immunoglobulin consensus sequence. The humanized antibody optimally also will comprise at least a portion of an immunoglobulin constant region (Fc), typically that of a human immunoglobulin (Jones et al., Nature, 321:522-525 (1986); Riechmann et al., Nature, 332:323-329 (1988); and Presta, Curr. Op. Struct. Biol., 2:593-596 (1992)). One clear advantage to such chimeric forms is that, for example, the variable regions can conveniently be derived from presently known sources using readily available hybridomas or B cells from non human host organisms in combination with constant regions derived from, for example, human cell preparations. While the variable region has the advantage of ease of preparation, and the specificity is not affected by its source, the constant region being human, is less likely to elicit an immune response from a human subject when the antibodies are injected than would the constant region from a non-human source. However, the definition is not limited to this particular example,

Because humanized antibodies are far less immunogenic in humans than the parental mouse monoclonal antibodies, they can be used for the treatment of humans with far less risk of anaphylaxis. Thus, these antibodies may be Preferred in therapeutic applications that involve *in vivo* administration to a human such as, *e.g.*, use as radiation sensitizers for the treatment of neoplastic disease or use in methods to reduce the side effects of, *e.g.*, cancer therapy. Methods for humanizing non-human antibodies are well known in the art, Generally, a humanized antibody has one or more amino acid residues introduced into it from a source that is non-human These non-human amino acid residues are often referred to as import residues, which are typically taken from an import variable domain. Humanization can be essentially performed following the method of Winter and co-workers (Jones et al., Nature 321:522-52.5 (1986); Riechmann et al., Nature 332:323-327 (1988); Verhoeyen et al" Science 239:1534-1536 (1988)), by substituting rodent CDRs or CDR sequences for the corresponding sequences of a human antibody Accordingly, such humanized antibodies are chimeric antibodies (U.S.. PatentNo. A,815,SG7), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies,

Human antibodies can also be produced using various techniques known in the art, including phage display libraries [Hoogenboom and Winter, J. Mol. Biol., 227:381 (1991); Marks et al., J. Mol Biol , 222:581 (1991)]. The techniques of Cole et al. and Boerner et al are also available for the preparation of human monoclonal antibodies [Cole et al., Monoclonal Antibodies and Cancer Therapy, Alan R.. Liss, p. 77 (1985) and Boerner et al., J. Immunol., 147(1):86-95 (1991)]. Humanized antibodies may be achieved by a variety of methods including, for example: (1) grafting the non-human complementarity determining regions (CDRs) onto a human framework and constant region (a process referred to in the art as "humanizing"), or, alternatively, (2) transplanting the entire non-human variable domains, but "cloaking" them with a human-like surface by replacement of surface residues (a process referred to in the art as "veneering'"). In the present invention, humanized antibodies will include both "humanized" and "veneered" antibodies. Similarly, human antibodies can be made by introducing human immunoglobulin loci into transgenic animals, e.g., mice in which the endogenous immunoglobulin genes have been partially or completely inactivated.. Upon challenge, human antibody production is observed, which closely resembles that seen in humans in all respects, including gene rearrangement, assembly, and antibody repertoire. This approach is described, for example, in U.S. Patent Nos 5,545,807; 5,545,806; 5,569,825; 5,625,126; 5,633,425; 5,661,016, and in the following scientific publications: Marks et al , Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern Rev Immunol. 13 65-93 (1995); Jones et al., Nature 321:522-525 (1986); Morrison et al., Proc. Natl. Acad. Sci, USA, 81:6851-6855 (1984); Morrison and Oi, Adv. Immunol, 44:55-92 (1988); Verhoeyer et al, Science 239:1534-1536 (1988); Padlan, Molec Immun. 28:489-498 (1991); Padlan, Molec Immunol. 31(3):169-217 (1994); and Kettleborough, C.A. et al., Protein Eng. 4(7):773.83 (1991) each of which is incorporated herein by reference.

The phrase "complementarity determining region" refers to amino acid sequences which together define the binding affinity and specificity of the natural Fv region of a native immunoglobulin binding site. *See, e.g.,* Chothia et al , J. Mol. Biol., 196:901-917 (1987); Kabat et al., U.S. Dept. of Health and Human Services NIH Publication No 91-3242 (1991). The phrase "constant region" refers to the portion of the antibody molecule that confers effector functions. In the present invention, mouse constant regions are substituted by human constant regions. The constant regions of the subject humanized antibodies are derived from human immunoglobulins. The heavy chain constant region can be selected from any of the five isotypes: alpha, delta, epsilon, gamma or mu. One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region that disturb the structure of the CDRs until the predicted conformation of the humanized sequence model closely approximates the conformation of the non-human CDRs of the parent non-human antibody. Such humanized antibodies may be further derivavatized to facilitate uptake and clearance, *e.g,* via Ashwell receptor *see, e.g.,* US Patent Nos. 5,530,101 and 5,585,089 which are incorporate herein by reference.

Humanized antibodies to CA polypeptides can also be produced using transgenic animals that are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci, WO 91/10741 also discloses transgenic non-primate mammalian hosts capable of mounting an immune response to an immunogen, wherein the antibodies have primate constant and/or variable regions, and wherein the endogenous immunoglobulin-encoding loci are substituted or inactivated WO 96/30498 discloses the use of the Cre/Lox system to modify the immunoglobulin locus in a mammal, such as to replace all or a portion of the constant or variable region to form a modified antibody molecule WO 94/02602 discloses non-human mammalian hosts having inactivated endogenous Ig loci and functional human Ig loci. U S. Patent No. 5,939,598 discloses methods of making transgenic mice in which the mice lack endogenous heavy chains, and express an exogenous immunoglobulin locus comprising one or more xenogeneic constant regions.

Using a transgenic animal described above, an immune response can be produced to a selected antigenic molecule, and antibody-producing cells can be removed from the animal and used to produce hybridomas that secrete human monoclonal antibodies. Immunization protocols, adjuvants, and the like are known in the art, and are used in immunization of; for example, a transgenic mouse as described in WO 96/33735. The monoclonal antibodies can be tested for the ability to inhibit or neutralize the biological activity or physiological effect of the corresponding protein.

In the present invention, CA polypeptides of the invention and variants thereof are used to immunize a transgenic animal as described above. Monoclonal antibodies are made using methods known in the art, and the specificity of'the antibodies is tested using isolated CA polypeptides. Methods for preparation of the human or primate CA or an epitope thereof include, but are not limited to chemical synthesis, recombinant DNA techniques or isolation from biological samples, Chemical synthesis of a peptide can be performed, for example, by the classical Merrifeld method of solid phase peptide synthesis (Merrifeld, J. Am Chem. Soc 85:2149,1963 which is incorporated by reference) or the FMOC strategy on a Rapid Automated Multiple Peptide Synthesis system (E. I du Pont de Nemours Company, Wilmington, DE) (Caprino and Han, J. Org Chem. 37:3404, 1972 which is incorporated by reference).

Polyclonal antibodies can be prepared by immunizing rabbits or other animals by injecting antigen followed by subsequent boosts at appropriate intervals. The animals are bled and sera assayed against purified CA proteins usually by ELISA or by bioassay based upon the ability to block the action of'CA proteins. When using avian species, e.g., chicken, turkey and the like, the antibody can be isolated from the yolk of the egg, Monoclonal antibodies can be prepared after the method of Milstein and Kohler by fusing splenocytes from immunized mice with continuously replicating tumor cells such as myeloma or lymphoma cells (Milstein and Kohler, Nature 256:495-497, 1975; Gulfre and Milstein, Methods in Enzymology: Immunochemical Techniques 73:1-46, Langone and Banatis eds , Academic Press, 1981 which are incorporated by reference) The hybridoma cells so formed are then cloned by limiting dilution methods and supernates assayed for antibody production by ELISA, RIA or bioassay.

The unique ability of antibodies to recognize and specifically bind to target proteins provides an approach for treating an overexpression of the protein Thus, another aspect of the present invention provides for a method for preventing or treating diseases involving overexpression of a CA polypeptide by treatment of a patient with specific antibodies to the CA protein

Specific antibodies, either polyclonal or monoclonal, to the CA proteins can be produced by any suitable method known in the art as discussed above. For example, murine or human monoclonal antibodies can be produced by hybridoma technology or, alternatively, the CA proteins, or an immunologically active fragment thereof, or an anti-idiotypic antibody, or fragment thereof can be administered to an animal to elicit the production of antibodies capable of recognizing and binding to the CA proteins. Such antibodies can be from any class of antibodies including, but not limited to IgG, IgA, IgM, IgD, and IgE or in the case of avian species, IgY and from any subclass of antibodies.

By immunotherapy is meant treatment of a cancer with an antibody raised against a CA protein As used herein, immunotherapy can be passive or active. Passive immunotherapy as defined herein is the passive transfer of antibody to a recipient (patient). Active immunization is the induction of antibody and/or T-cell responses in a recipient (patient). Induction of an immune response is the result of providing the recipient with an antigen to which antibodies are raised. As appreciated by one of ordinary skill in the art, the antigen may be provided by injecting a polypeptide against which antibodies are desired to be raised into a recipient, or contacting the recipient with a nucleic acid capable of expressing the antigen and under conditions for expression of'the antigen.

In a preferred embodiment, oncogenes which encode secreted growth factors may be inhibited by raising antibodies against CA proteins that are secreted proteins as described above Without being bond by theory, antibodies used for treatment, bind and prevent the secreted protein from binding to its receptor, thereby inactivating the secreted CA protein.

In another preferred embodiment, the CA protein to which antibodies are raised is a transmembrane protein. Without being bound by theory, antibodies used for treatment, bind the extracellular domain of the CA protein and prevent it from binding to other proteins, such as circulating ligands or cell-associated molecules. The antibody may cause down regulation of the transmembrane CA protein As will be appreciated by one of ordinary skill in the art, the antibody may be a competitive, non-competitive or uncompetitive inhibitor of protein binding to the extracellular domain of'the CA protein The antibody is also an antagonist of the CA protein. Further, the antibody prevents activation of the transmembrane CA protein, In one aspect, when the antibody prevents the binding of other molecules to the CA protein, the antibody prevents growth of the cell.. The antibody may also sensitize the cell to cytotoxic agents, including, but not limited to TNF-α, TNF-β, IL-1., INF-γ and IL-2, or chemotherapeutic agents including 5FU, vinblastine, actinomycin D, cisplatin, methotrexate, and the like, In some instances the antibody belongs to a sub-type that activates serum complement when complexed with the transmembrane proteins thereby mediating cytotoxicity. Thus, cancers may be treated by administering to a patient antibodies directed against the transmembrane CA protein

In another preferred embodiment, the antibody is conjugated to a therapeutic moiety In one aspect the therapeutics moiety is a small molecule that modulates the activity of the CA protein In another aspect the therapeutic moiety modulates the activity of molecules associated with or in close proximity to the CA protein The therapeutic moiety may inhibit enzymatic activity such as protease or protein kinase activity associated with cancel.

In a preferred embodiment, the therapeutic moiety may also be a cytotoxic agent. In this method, radioisotopes, natural toxins, chemotherapy agents, or other substances (such as biological response modifiers) are chemically linked or conjugated to a monoclonal antibody to form "immunoconjugates" and "immunotoxins" which target the cytotoxic agent to tumor tissue or cells resulting in a reduction in the number of afflicted cells, thereby reducing symptoms associated with cancers, including lymphoma. Cytotoxic agents are numerous and varied and include, but are not limited to, cytotoxic drugs or toxins or active fragments of such toxins. Suitable toxins and their corresponding fragments include diphtheria A chain, exotoxin A chain, ricin A chain, abrin A chain, curcin, crotin, phenomycin, enomycin and the like. Cytotoxic agents also include radiochemicals made by conjugating radioisotopes to antibodies raised against CA proteins, or binding of a radionuclide to a chelating agent that has been covalently attached to the antibody. Targeting the therapeutic moiety to transmembrane CA proteins not only serves to increase the local concentration of therapeutic moiety in the cancer of interest, i.e., lymphoma, but also serves to reduce deleterious side effects that may be associated with the therapeutic moiety. A number of investigators have used monoclonal antibodies as carriers of cytotoxic substances in attempts to selectively direct those agents to malignant tissue More particularly, a number of monoclonal antibodies have been conjugated to toxins such as ricin, abrin, diphtheria toxin and Pseudomonas exotoxin or to enzymatically active portions (A chains) thereof via heterobifunctional agents See, e.g., U.S. Pat, No. 4,753,894 to Frankel et al.; Nevelle, et al. (1982) Immunol Rev 62:75-91; Ross et al. (1980) Eur. J Biochem 104; Vitteta et al. (1982) Immunol Rev 62:158-183; Raso et al. (1982) Cancer Res 42:457-464, and Trowbridge et at (1981) Nature 294:171-173.

In another preferred embodiment, the CA protein against which the antibodies are raised is an intracellular protein In this case, the antibody may be conjugated to a protein that facilitates entry into the cell. In one case, the antibody enters the cell by endocytosis. In another embodiment, a nucleic acid encoding the antibody is administered to the individual or cell Moreover, wherein the CA protein can be targeted within a cell, e.g., the nucleus, an antibody thereto contains a signal for that target localization, e.g., a nuclear localization signal.

The CA antibodies of the invention specifically bind to CA proteins By "specifically bind" herein is meant that the antibodies bind to the protein with a binding constant in the range of $10^{-4}$-$10^{-6}$ $M^{-1}$, with a preferred range being $10^{-7}$-$10^{-9}$ $M^{-1}$.

In a preferred embodiment, the CA protein is purified or isolated after expression. CA proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the CA protein may be purified using a standard anti-CA antibody column. Ultrafiltration and diafiltration techniques, in conjunction with protein concentration, are also useful. For general guidance in suitable purification techniques, see Scopes, R., Protein Purification, Springer-Verlag, NY (1982) The degree of purification necessary will vary depending on the use of the CA protein In some instances no purification will be necessary

## Detection of cancer phenotype

Once expressed and purified if necessary, the CA proteins and nucleic acids are useful in a number of applications. In one aspect, the expression levels of genes are determined for different cellular states in the cancel phenotype;

that is, the expression levels of genes in nominal tissue and in cancer tissue (and in some cases, for varying severities of lymphoma that relate to prognosis, as outlined below) are evaluated to provide expression profiles. An expression profile of a particular cell state or point of development is essentially a "fingerprint" of the state; while two states may have any particular gene similarly expressed, the evaluation of a number of genes simultaneously allows the generation of a gene expression profile that is unique to the state of the cell, By comparing expression profiles of cells in different states, information regarding which genes are important (including both up- and down-regulation of genes) in each of these states is obtained Then, diagnosis may be done or confirmed: does tissue from a particular patient have the gene expression profile of normal or cancer tissue.

"Differential expression," or equivalents used herein, refers to both qualitative as well as quantitative differences in the temporal and/or cellular expression patterns of genes, within and among the cells. Thus, a differentially expressed gene can qualitatively have its expression altered, including an activation or inactivation, in, for example, normal versus cancer tissue, That is, genes may be turned on or turned off in a particular state, relative to another state. As is apparent to the skilled artisan, any comparison of'two or more states can be made. Such a qualitatively regulated gene will exhibit an expression pattern within a state or cell type which is detectable by standard techniques in one such state or cell type, but is not detectable in both. Alternatively, the determination is quantitative in that expression is increased or decreased; that is, the expression of the gene is either up-regulated, resulting in an increased amount of transcript, or down-regulated, resulting in a decreased amount of transcript. The degree to which expression differs need only be large enough to quantify via standard characterization techniques as outlined below, such as by use of Affymetrix GeneChip® expression arrays, Lockhart, Nature Biotechnology, 14:1675-1680 (1996), hereby expressly incorporated by reference. Other techniques include, but are not limited to, quantitative reverse transcriptase PCR, Northern analysis and RNase protection, As outlined above, preferably the change in expression (i.e. upregulation or downregulation) is at least about 50%, more preferably at least about 100%, more preferably at least about 150%, more preferably, at least about 200%, with from 300 to at least 1000% being especially preferred

As will be appreciated by those in the art, this may be done by evaluation at either the gene transcript, or the protein level; that is, the amount of gene expression may be monitored using nucleic acid probes to the DNA or RNA equivalent of the gene transcript, and the quantification of gene expression levels, or, alternatively, the final gene product itself (protein) can be monitored, for example through the use of antibodies to the CA protein and standard immunoassays (ELISAs, etc.) or other techniques, including mass spectroscopy assays, 2D gel electrophoresis assays, etc. Thus, the proteins corresponding to CA genes, i.e. those identified as being important in a particular cancer phenotype, i.e., lymphoma, can be evaluated in a diagnostic test specific for that cancer.

In a preferred embodiment, gene expression monitoring is done and a number of genes, i.e. an expression profile, is monitored simultaneously, although multiple protein expression monitoring can be done as well. Similarly, these assays may be done on an individual basis as well.

In this embodiment, the CA nucleic acid probes may be attached to biochips as outlined herein for the detection and quantification of CA sequences in a particular cell The assays are done as is known in the art As will be appreciated by those in the art, any number of different CA sequences may be used as probes, with single sequence assays being used in some cases, and a plurality of the sequences described herein being used in other embodiments. In addition, while solid-phase assays are described, any number of solution based assays may be done as well.

In a preferred embodiment, both solid and solution based assays may be used to detect CA sequences that are up-regulated or down-regulated in cancers as compared to normal tissue. In instances where the CA sequence has been altered but shows the same expression profile or an altered expression profile, the protein will be detected as outlined herein.

In a preferred embodiment nucleic acids encoding the CA protein are detected. Although DNA or RNA encoding the CA protein may be detected, of particular interest are methods wherein the mRNA encoding a CA protein is detected. The presence of mRNA in a sample is an indication that the CA gene has been transcribed to form the mRNA, and suggests that the protein is expressed. Probes to detect the mRNA can be any nucleotide/deoxynucleotide probe that is complementary to and base pairs with the mRNA and includes but is not limited to oligonucleotides, cDNA or RNA. Probes also should contain a detectable label, as defined herein In one method the mRNA is detected after immobilizing the nucleic acid to be examined on a solid support such as nylon membranes and hybridizing the probe with the sample Following washing to remove the non- specifically bound probe, the label is detected. In another method detection of the mRNA is performed *in situ*. In this method permeabilized cells or tissue samples are contacted with a detectably labeled nucleic acid probe for sufficient time to allow the probe to hybridize with the target RNA Following washing to remove the non-specifically bound probe, the label is detected For example a digoxygenin labeled riboprobe (RNA probe) that is complementary to the mRNA encoding a CA protein is detected by binding the digoxygenin with an anti-digoxygenin secondary antibody and developed with nitro blue tetrazolium and 5-bromo-4-chloro-3-indoyl phosphate

In a preferred embodiment, any of the three classes of proteins as described herein (secreted, transmembrane or intracellular proteins) are used in diagnostic assays. The CA proteins, antibodies, nucleic acids, modified proteins and cells containing CA sequences are used in diagnostic assays. This can be done on an individual gene or corresponding polypeptide level, or as sets of assays.

As described and defined herein, CA proteins find use as markers of cancers, including lymphomas such as, but not limited to, Hodgkin's and non-Hodgkin's lymphoma. Detection of these proteins in putative cancer tissue or patients allows for a determination or diagnosis of the type of cancer Numerous methods known to those of ordinary skill in the art find use in detecting cancers. In one embodiment, antibodies are used to detect CA proteins A preferred method separates proteins from a sample or patient by electrophoresis on a gel (typically a denaturing and reducing protein gel, but may be any other type of gel including isoelectric focusing gels and the like). Following separation of proteins, the CA protein is detected by immunoblotting with antibodies raised against the CA protein Methods of immunoblotting are well known to those of ordinary skill in the art.

In another preferred method, antibodies to the CA protein find use in *in situ* imaging techniques. In this method cells are contacted with from one to many antibodies to the CA protein(s) Following washing to remove non-specific antibody binding, the presence of the antibody or antibodies is detected In one embodiment the antibody is detected by incubating with a secondary antibody that contains a detectable label. In another method the primary antibody to the CA protein(s) contains a detectable label. In another preferred embodiment each one of multiple primary antibodies contains a distinct and detectable label. This method finds particular use in simultaneous screening for a plurality of CA proteins. As will be appreciated by one of ordinary skill in the art, numerous other histological imaging techniques are useful in the invention.

In a preferred embodiment the label is detected in a fluorometer that has the ability to detect and distinguish emissions of different wavelengths. In addition, a fluorescence activated cell sorter (FACS) can be used in the method.

In another preferred embodiment, antibodies find use in diagnosing cancers from blood samples As previously described, certain CA proteins are secreted/circulating molecules. Blood samples, therefore, are useful as samples to be probed or tested for the presence of secreted CA proteins Antibodies can be used to detect the CA proteins by any of the previously described immunoassay techniques including ELISA, immunoblotting (Western blotting), immunoprecipitation, BIACORE technology and the like, as will be appreciated by one of ordinary skill in the art.

In a preferred embodiment, *in situ* hybridization of labeled CA nucleic acid probes to tissue arrays is done. For example, arrays of tissue samples, including CA tissue and/or normal tissue, are made *In situ* hybridization as is known in the art can then be done.

It is understood that when comparing the expression fingerprints between an individual and a standard, the skilled artisan can make a diagnosis as well as a prognosis. It is further understood that the genes that indicate diagnosis may differ from those that indicate prognosis,

In a preferred embodiment, the CA proteins, antibodies, nucleic acids, modified proteins and cells containing CA sequences are used in prognosis assays. As above, gene expression profiles can be generated that correlate to cancer, especially lymphoma, severity, in terms of long term prognosis Again, this may be done on either a protein or gene level, with the use of genes being preferred As above, the CA probes are attached to biochips for the detection and quantification of CA sequences in a tissue or patient. The assays proceed as outlined for diagnosis

**Screening for CA-Targeted Drugs**

In one embodiment, any of the CA sequences as described herein are used in drug screening assays. The CA proteins, antibodies, nucleic acids, modified proteins and cells containing CA sequences are used in drug screening assays or by evaluating the effect of drug candidates on a "gene expression profile" or expression profile of polypeptides, In one embodiment, the expression profiles are used, preferably in conjunction with high throughput screening techniques to allow monitoring for expression profile genes after treatment with a candidate agent, Zlokarnik, et al., Science 279, 84-8 (1998), Heid, et al, Genome Res, 6:986 994 (1996).

In another embodiment, the CA proteins, antibodies, nucleic acids, modified proteins and cells containing the native or modified CA proteins are used in screening assay, That is, the present invention provides novel methods for screening for compositions that modulate the cancer phenotype. As above, this can be done by screening for modulators of gene expression or for modulators of protein activity Similarly, this may be done on an individual gene or protein level or by evaluating the effect of drug candidates on a "gene expression profile" In a preferred embodiment, the expression profiles are used, preferably in conjunction with high throughput screening techniques to allow monitoring for expression profile genes after treatment with a candidate agent, see Zlokarnik, supra

Having identified the CA genes herein, a variety of assays to evaluate the effects of agents on gene expression may be executed. In a preferred embodiment, assays may be run on an individual gene or protein level. That is, having identified a particular gene as aberrantly regulated in cancer, candidate bioactive agents may be screened to modulate the gene's regulation. "Modulation" thus includes both an increase and a decrease in gene expression or activity. The preferred amount of modulation will depend on the original change of the gene expression in normal versus tumor tissue, with changes of at least 10%, preferably 50%, more preferably 100-300%, and in some embodiments 300-1000% or greater. Thus, if a gene exhibits a 4 fold increase in tumor compared to normal tissue, a decrease of about four fold is desired; a 10 fold decrease in tumor compared to normal tissue gives a 10 fold increase in expression for a candidate agent is desired, etc. Alternatively, where the CA sequence has been altered but shows the same expression profile or an altered expression profile, the protein will be detected as outlined herein.

As will be appreciated by those in the art, this may be done by evaluation at either the gene or the protein level; that is, the amount of gene expression may be monitored using nucleic acid probes and the quantification of gene expression levels, or, alternatively, the level of the gene product itself can be monitored, for example through the use of antibodies to the CA protein and standard immunoassays. Alternatively, binding and bioactivity assays with the protein may be done as outlined below.

In a preferred embodiment, gene expression monitoring is done and a number of' genes, i.e. an expression profile, is monitored simultaneously, although multiple protein expression monitoring can be done as well

In this embodiment, the CA nucleic acid probes are attached to biochips as outlined herein for the detection and quantification of' CA sequences in a particular cell. Ihe assays are further described below.

Generally, in a preferred embodiment, a candidate bioactive agent is added to the cells prior to analysis. Moreover screens are provided to identify a candidate bioactive agent that modulates a particular type of cancer, modulates CA proteins, binds to a CA protein, or interferes between the binding of a CA protein and an antibody.

The term "candidate bioactive agent" or "drug candidate" or grammatical equivalents as used herein describes any molecule, e.g., protein, oligopeptide, small organic or inorganic molecule, polysaccharide, polynucleotide, etc., to be tested for bioactive agents that are capable of directly or indirectly altering either the cancer phenotype, binding to and/or modulating the bioactivity of a CA protein, or the expression of' a CA sequence, including both nucleic acid sequences and protein sequences. In a particularly preferred embodiment, the candidate agent suppresses a CA phenotype, for example to a normal tissue fingerprint. Similarly, the candidate agent preferably suppresses a severe CA phenotype Generally a plurality of' assay mixtures are run in parallel with different agent concentrations to obtain a differential response to the various concentrations Typically, one of'these concentrations serves as a negative control, i.e., at zero concentration or below the level of detection.

In one aspect, a candidate agent will neutralize the effect of a CA protein By "neutralize" is meant that activity of a protein is either inhibited or counter acted against so as to have substantially no effect on a cell

Candidate agents encompass numerous chemical classes, though typically they are organic or inorganic molecules, preferably small organic compounds having a molecular weight of more than 100 and less than about 2,500 Daltons. Preferred small molecules are less than 2000, or less than 1500 or less than 1000 or less than 500 D Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Particularly preferred are peptides.

Candidate agents are obtained from a wide variety of sources including libraries of synthetic or natural compounds For example, numerous means are available for random and directed synthesis of a wide variety of organic compounds and biomolecules, including expression of randomized oligonucleotides Alternatively, libraries of natural compounds in the form of bacterial, fungal, plant and animal extracts are available or readily produced Additionally, natural or synthetically produced libraries and compounds are readily modified through conventional chemical, physical and biochemical means. Known pharmacological agents may be subjected to directed or random chemical modifications, such as acylation, alkylation, esterification, or amidification to produce structural analogs

In one embodiment, the candidate bioactive agents are proteins. By "protein" herein is meant at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and norleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard in vivo degradation.

In a preferred embodiment, the candidate bioactive agents are naturally occurring proteins or fiagments of naturally occurring proteins. Thus, for example, cellular extracts containing proteins, or random or directed digests of proteinaceous cellular extracts, may be used. In this way libraries of prokaryotic and eukaryotic proteins may be made for screening in the methods of the invention. Particularly preferred in this embodiment are libraries of bacterial, fungal, viral, and mammalian proteins, with the latter being preferred, and human proteins being especially preferred.

In another preferred embodiment, the candidate bioactive agents are peptides of from about 5 to about 30 amino acids, with from about 5 to about 20 amino acids being preferred, and from about 7 to about 15 being particularly preferred The peptides may be digests of naturally occurring proteins as is outlined above, random peptides, or "biased" random peptides. By "randomized" or grammatical equivalents herein is meant that each nucleic acid and peptide consists of essentially random nucleotides and amino acids, respectively, Since generally these random peptides (or nucleic acids, discussed below) are chemically synthesized, they may incorporate any nucleotide or amino acid at any position The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position. In a preferred embodiment, the library is biased. That is, some positions within the sequence are either held constant, or are selected from a limited number of possibilities For example, in a preferred embodiment, the nucleotides or amino acid residues are randomized within a defined class, for example, of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines or histidines for phosphorylation sites, etc, or to purines, etc.

In one embodiment, the candidate bioactive agents are nucleic acids. As described generally for proteins, nucleic acid candidate bioactive agents may be naturally occurring nucleic acids, random nucleic acids, or "biased" random nucleic acids. In another embodiment, the candidate bioactive agents are organic chemical moieties, a wide variety of which are available in the literature.

In assays for testing alteration of'the expression profile of'one or more CA genes, after the candidate agent has been added and the cells allowed to incubate for some period of time, a nucleic acid sample containing the target sequences to be analyzed is prepared. The target sequence is prepared using known techniques (e.g., converted from RNA to labeled cDNA, as described above) and added to a suitable microarray. For example, an *in vitro* reverse transcription with labels covalently attached to the nucleosides is performed. Generally, the nucleic acids are labeled with a label as defined herein, especially with biotin-FIIC or PE, Cy3 and Cy5

As will be appreciated by those in the art, these assays can be direct hybridization assays or can comprise "sandwich assays", which include the use of multiple probes, as is generally outlined in U.S. Patent Nos. 5,681,702, 5,597,909, 5,545,73 0, 5,594,117, 5,591,584, 5,571,670, 5,580,731, 5,571,670, 5,591,584, 5,624,802, 5,635,352, 5,594,118, 5,359,100, 5,124,246 and 5,681,697, all of which are hereby incorporated by reference. In this embodiment, in general, the target nucleic acid is prepared as outlined above, and then added to the biochip comprising a plurality of nucleic acid probes, under conditions that allow the formation of'a hybridization complex.

A variety of hybridization conditions may be used in the present invention, including high, moderate and low stringency conditions as outlined above. The assays are generally run under stringency conditions that allow formation of the label probe hybridization complex only in the presence of target. Stringency can be controlled by altering a step parameter that is a thermodynamic variable, including, but not limited to, temperature, formamide concentration, salt concentration, chaotropic salt concentration, pH, organic solvent concentration, etc. These parameters may also be used to control non-specific binding, as is generally outlined in U.S. Patent No. 5,681,697. Thus it may be desirable to perform certain steps at higher stringency conditions to reduce non-specific binding.

The reactions outlined herein may be accomplished in a variety of ways, as will be appreciated by those in the art. Components of the reaction may be added simultaneously, or sequentially, in any order, with preferred embodiments outlined below In addition, the reaction may include a variety of other reagents in the assays These include reagents like salts, buffers, neutral proteins, e.g. albumin, detergents, etc which may be used to facilitate optimal hybridization and detection, and/or reduce non-specific or background interactions. Also reagents that otherwise improve the efficiency of the assay, such as protease inhibitors, nuclease inhibitors, anti-microbial agents, etc., may be used, depending on the sample preparation methods and purity of the target. In addition, either solid phase or solution based (i.e., kinetic PCR) assays may be used.

Once the assay is run, the data are analyzed to determine the expression levels, and changes in expression levels as between states, of individual genes, forming a gene expression profile.

In a preferred embodiment, as for the diagnosis and prognosis applications, having identified the differentially expressed gene(s) or mutated gene(s) important in any one state, screens can be run to test for alteration of the expression of the CA genes individually That is, screening for modulation of regulation of expression of a single gene can be done. Thus, for example, in the case of target genes whose presence or absence is unique between two states, screening is done for modulators of the target gene expression.

In addition, screens can be done for novel genes that are induced in response to a candidate agent. After identifying a candidate agent based upon its ability to suppress a CA expression pattern leading to a normal expression pattern, or modulate a single CA gene expression profile so as to mimic the expression of the gene from normal tissue, a screen as described above can be performed to identify genes that are specifically modulated in response to the agent. Comparing expression profiles between normal tissue and agent heated CA tissue reveals genes that are not expressed in normal tissue or CA tissue, but are expressed in agent treated tissue. These agent specific sequences can be identified and used by any of the methods described herein for CA genes or proteins. In particular these sequences and the proteins they encode find use in marking or identifying agent-Heated cells. In addition, antibodies can be raised against the agent-induced proteins and used to target novel therapeutics to the treated CA tissue sample.

Thus, in one embodiment, a candidate agent is administered to a population of CA cells, that thus has an associated CA expression profile. By "administration" or "contacting" herein is meant that the candidate agent is added to the cells in such a manner as to allow the agent to act upon the cell, whether by uptake and intracellular action, or by action at the cell surface. In some embodiments, nucleic acid encoding a proteinaceous candidate agent (i.e, a peptide) may be put into a viral construct such as a retroviral construct and added to the cell, such that expression of the peptide agent is accomplished; see PCI US97/01019, hereby expressly incorporated by reference

Once the candidate agent has been administered to the cells, the cells can be washed if desired and are allowed to incubate under preferably physiological conditions for some period of time. The cells are then harvested and a new gene expression profile is generated, as outlined herein.

Thus, for example, CA tissue may be screened for agents that reduce or suppress the CA phenotype. A change in at least one gene of the expression profile indicates that the agent has an effect on CA activity. By defining such a signature for the CA phenotype, screens for new drugs that alter the phenotype can be devised. With this approach, the drug target need not be known and need not be represented in the original expression screening platform, nor does the level of transcript for the target protein need to change.

In a preferred embodiment, as outlined above, screens may be done on individual genes and gene products (proteins). That is, having identified a particular differentially expressed gene as important in a particular state, screening of modulators of either the expression of the gene or the gene product itself can be done.. The gene produces of differentially expressed genes are sometimes referred to herein as "CA proteins" or "CAP". The CAP may be a fragment, or alternatively, be the full-length protein to the fragment encoded by the nucleic acids of Tables 1-6. In a preferred embodiment, the CAP is selected from the human protein sequences shown in Tables 1-6 embodiment, the sequences are sequence variants as further described herein:

Preferably, the CAP is a fragment approximately 14 to 24 amino acids in length More preferably the fragment is a soluble fragment. Preferably, the fragment includes a non-transmembrane region. In a preferred embodiment, the fragment has an N-terminal Cys to aid in solubility. In one embodiment, the C-terminus of the fragment is kept as a free acid and the N-terminus is a free amine to aid in coupling, e.g., to a cysteine.

In one embodiment the CA proteins are conjugated to an immunogenic agent as discussed herein In one embodiment the CA protein is conjugated to BSA.

In a preferred embodiment, screening is done to alter the biological function of the expression product of the CA gene. Again, having identified the importance or a gene in a particular state, screening for agents that bind and/or modulate the biological activity of the gene product can be run as is more fully outlined below.

In a preferred embodiment, screens are designed to first find candidate agents that can bind to CA proteins, and then these agents may be used in assays that evaluate the ability of the candidate agent to modulate the CAP activity and the cancer phenotype. Thus, as will be appreciated by those in the art, there are a number of different assays that may be run; binding assays and activity assays

In a preferred embodiment, binding assays are done In general, purified or isolated gene product is used; that is, the gene products of one or more CA nucleic acids are made In general, this is done as is known in the art. For example, antibodies are generated to the protein gene products, and standard immunoassays are run to determine the amount of protein present. Alternatively, cells comprising the CA proteins can be used in the assays.

Thus, in a preferred embodiment, the methods comprise combining a CA protein and a candidate bioactive agent, and determining the binding of the candidate agent to the CA protein. Preferred embodiments utilize the human or mouse CA protein, although other mammalian proteins may also be used, for example for the development of animal models of human disease. In some embodiments, as outlined herein, variant or derivative CA proteins may be used.

Generally, in a preferred embodiment of the methods herein, the CA protein or the candidate agent is non-diffusably bound to an insoluble support having isolated sample receiving areas (e.g. a microtiter plate, an array, etc.). The insoluble support may be made of any composition to which the compositions can be bound, is readily separated from soluble material, and is otherwise compatible with the overall method of screening. The surface of such supports may be solid or porous and of any convenient shape. Examples of suitable insoluble supports include microliter plates, arrays, membranes and beads. These are typically made of glass, plastic (e.g., polystyrene), polysaccharides, nylon or nitrocellulose, Teflon®, etc. Microtiter plates and arrays are especially convenient because a large number of assays can be carried out simultaneously, using small amounts of reagents and samples.

The particular manner of binding of the composition is not crucial so long as it is compatible with the reagents and overall methods of the invention, maintains the activity of the composition and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the protein or agent on the surface, etc. Following binding of'the protein or agent, excess unbound material is removed by washing The sample receiving areas may then be blocked through incubation with bovine serum albumin (BSA), casein or other innocuous protein or other moiety,

In a preferred embodiment, the CA protein is bound to the support, and a candidate bioactive agent is added to the assay. Alternatively, the candidate agent is bound to the support and the CA protein is added. Novel binding agents include specific antibodies, non-natural binding agents identified in screens of chemical libraries, peptide analogs, etc. Of particular interest are screening assays for agents that have a low toxicity for human cells. A wide variety of assays may be used for this purpose, including labeled *in vitro* protein-protein binding assays, electrophoretic mobility shift assays, immunoassays for protein binding, functional assays (phosphorylation assays, etc.) and the like.

The determination of'the binding of the candidate bioactive agent to the CA protein may be done in a number of ways In a preferred embodiment, the candidate bioactive agent is labeled, and binding determined directly. For example, this may be done by attaching all or a portion of the CA protein to a solid support, adding a labeled candidate agent (for example a fluorescent label), washing off excess reagent, and determining whether the label is present on the solid support Various blocking and washing steps may be utilized as is known in the art.

By "labeled" herein is meant that the compound is either directly or indirectly labeled with a label which provides a detectable signal, e.g. radioisotope, fluorescers, enzyme, antibodies, particles such as magnetic particles, chemiluminescers, or specific binding molecules, etc. Specific binding molecules include pairs, such as biotin and streptavidin, digoxin and antidigoxin etc. For the specific binding members, the complementary ember would normally be labeled with a molecule which provides for detection, in accordance with known procedures, as outlined above. The label can directly or indirectly provide a detectable signal.

In some embodiments, only one of the components is labeled For example, the proteins (or proteinaceous candidate agents) may be labeled at tyrosine positions using $^{125}I$, or with fluorophores. Alternatively, more than one component may be labeled with different labels; using $^{125}I$ for the proteins, for example, and a fluorophore for the candidate agents.

In a preferred embodiment, the binding of the candidate bioactive agent is determined through the use of competitive binding assays. In this embodiment, the competitor is a binding moiety known to bind to the target molecule (i.e. CA protein), such as an antibody, peptide, binding partner, ligand, etc. Under certain circumstances, there may be competitive binding as between the bioactive agent and the binding moiety, with the binding moiety displacing the bioactive agent

In one embodiment, the candidate bioactive agent is labeled. Either the candidate bioactive agent, or the competitor, or both, is added first to the protein for a time sufficient to allow binding, if present Incubations may be performed at any temperature which facilitates optimal activity, typically between 4 and 40° C. Incubation periods are selected for optimum activity, but may also be optimized to facilitate rapid high throughput screening Typically between 0.1 and 1 hour will be sufficient Excess reagent is generally removed or washed away. The second component is then added, and the presence or absence of the labeled component is followed, to indicate binding.

In a preferred embodiment, the competitor is added first, followed by the candidate bioactive agent Displacement of the competitor is an indication that the candidate bioactive agent is binding to the CA protein and thus is capable of binding to, and potentially modulating, the activity of the CA protein. In this embodiment, either component can be labeled. Thus, for example, if the competitor is labeled, the presence of label in the wash solution indicates displacement by the agent Alternatively, if the candidate bioactive agent is labeled, the presence of the label on the support indicates displacement.

In an alternative embodiment, the candidate bioactive agent is added first, with incubation and washing, followed by the competitor. The absence of binding by the competitor may indicate that the bioactive agent is bound to the CA protein with a higher affinity. Thus, if the candidate bioactive agent is labeled, the presence of the label on the support, coupled with a lack of competitor binding, may indicate that the candidate agent is capable of binding to the CA protein.

In a preferred embodiment, the methods comprise differential screening to identity bioactive agents that are capable of modulating the activity of the CA proteins. In this embodiment, the methods comprise combining a CA protein and a competitor in a first samples. A second sample comprises a candidate bioactive agent, a CA protein and a competitor The binding of the competitor is determined for both samples, and a change, or difference in binding between the two samples indicates the presence of an agent capable of binding to the CA protein and potentially modulating its activity. That is, if the binding of the competitor is different in the second sample relative to the first sample, the agent is capable of binding to the CA protein

Alternatively, a preferred embodiment utilizes differential screening to identify drug candidates that bind to the native CA protein, but cannot bind to modified CA proteins The structure of the CA protein may be modeled, and used in rational drug design to synthesize agents that interact with that site. Drug candidates that affect CA bioactivity are also identified by screening drugs for the ability to either enhance or reduce the activity of the protein.

Positive controls and negative controls may be used in the assays. Preferably all control and test samples are performed in at least triplicate to obtain statistically significant result. Incubation of all samples is for a time sufficient for the binding of the agent to the protein. Following incubation, all samples are washed free of' non-specifically bound material and the amount of bound, generally labeled agent determined. For example, where a radiolabel is employed, the samples may be counted in a scintillation counter to determine the amount of bound compound.

A variety of other reagents may be included in the screening assays. These include reagents like salts, neutral proteins, e.g, albumin, detergents, etc which may be used to facilitate optimal protein-protein binding and/or reduce non-specific or background interactions. Also reagents that otherwise improve the efficiency of the assay, such as protease inhibitors, nuclease inhibitor, anti-microbial agents, etc., may be used The mixture of components may be added in any order that provides for the requisite binding

Screening for agents that modulate the activity of CA proteins may also be done.. In a preferred embodiment, methods for screening for a bioactive agent capable of modulating the activity of'CA proteins comprise the steps of adding a candidate bioactive agent to a sample of CA proteins, as above, and determining an alteration in the biological activity of' CA proteins. "Modulating the activity of' a CA protein" includes an increase in activity, a decrease in activity, or a change in the type or kind of activity present. Thus, in this embodiment, the candidate agent should both bind to CA proteins (although this may not be necessary), and alter its biological or biochemical activity as defined herein. The methods include both *in vitro* screening methods, as are generally outlined above, and in vivo screening of cells for alterations in the presence, distribution, activity or amount of CA proteins

Thus, in this embodiment, the methods comprise combining a CA sample and a candidate bioactive agent, and evaluating the effect on CA activity. By "CA activity" or grammatical equivalents herein is meant one of the CA protein's biological activities, including, but not limited to, its role in tumorigenesis, including cell division, preferably in lymphatic tissue, cell proliferation, tumor growth and transformation of cells. In one embodiment, CA activity includes activation of or by a protein encoded by a nucleic acid of Tables 1-6. An inhibitor of CA activity is the inhibition of'any one or more CA activities.

In a preferred embodiment, the activity of the CA protein is increased; in another preferred embodiment, the activity of'the CA protein is decreased. Thus, bioactive agents that are antagonists are preferred in some embodiments, and bioactive agents that are agonists may be preferred in other embodiments.

In a preferred embodiment, the invention provides methods for screening for bioactive agents capable of modulating the activity of'a CA protein. The methods comprise adding a candidate bioactive agent, as defined above, to a cell comprising CA proteins. Preferred cell types include almost any cell. The cells contain a recombinant nucleic acid that encodes a CA protein In a preferred embodiment, a library of candidate agents is tested on a plurality of cells.

In one aspect, the assays are evaluated in the presence or absence or previous or subsequent exposure of physiological signals, for example hormones, antibodies, peptides, antigens, cytokines, growth factors, action potentials, pharmacological agents including chemotherapeutics, radiation, carcinogenic, or other cells (i,e. cell-cell contacts), In another example, the determinations are determined at different stages of the cell cycle process

In this way, bioactive agents are identified. Compounds with pharmacological activity are able to enhance or interfere with the activity of the CA protein.

**Applications of the invention**

In one embodiment, a method of inhibiting cancer cell division is provided In another embodiment, a method of inhibiting tumor growth is provided. In a further embodiment, methods of treating cells or individuals with cancer are provided

In one embodiment, a method of inhibiting carcinoma cancer cell division, is provided. The method comprises administration of a carcinoma cancer inhibitor. In oned embodiment, the carcinoma cell is a lymphoma carcinoma, in another embodiment, the carcinoma cell is a breast camcer carcinoma.

In another embodiment, a method of inhibiting tumor growth is provided. The method comprises administration of a carcinoma cancer inhibitor. In a particular embodiment, a method of inhibiting tumor growth in lymphatic tissue is provided comprising administration of a lymphoma inhibitor, In another embodiment, a method of inhibiting tumor growth in mammary tissue is provided comprising administration of a breast cancer inhibitor.

The method comprises administration of a cancer inhibitor, In particular embodiments, the cancer inhibitor is an antisense molecule, a pharmaceutical composition, a therapeutic agent or small molecule, or a monoclonal, polyclonal, chimeric or humanized antibody. In particular embodiments, a therapeutic agent is coupled with a an antibody, preferable a monoclonal antobody.

In other embodiments, methods for detection or diagnosis of cancel cells in an individual are provided. In particular embodiments, the diagnostic/detection agent is a small molecule that pereferentially binds to a CAP according to the invention In one embodiment, the diagnostic/detection agent is an antibody, preferably a monoclonal antibody, preferably linked to a detectable agent.

In other embodiments of the invention, animal models and transgenic animals are provided, which find use in generating animal models of cancers, particularly lymphomas and carcinoma.

### (a) Antisense molecules

In one embodiment, the cancer inhibitor is an antisense molecule. Antisense molecules as used herein include antisense or sense oligonucleotides comprising a single-stranded nucleic acid sequence (either RNA or DNA) capable of binding to target RNA (sense) or DNA (antisense) sequences for cancer molecules Antisense or sense oligonucleotides, according to the present invention, comprise a fragment generally at least about 14 nucleotides, preferably from about 14 to 30 nucleotides The ability to derive an antisense or a sense oligonucleotide, based upon a cDNA sequence encoding a given protein is described in, for example, Stein and Cohen, Cancer Res 48:2659, (1988) and van der Krol et al., BioTechniques 6:958, (1988).

Antisense molecules may be introduced into a cell containing the target nucleotide sequence by formation of'a conjugate with a ligand binding molecule, as described in WO 91/04753. Suitable ligand binding molecules include, but are not limited to, cell surface receptors, growth factors, other cytokines, or other ligands that bind to cell surface receptors Preferably, conjugation of'the ligand binding molecule does not substantially interfere with the ability of the ligand binding molecule to bind to its corresponding molecule or receptor, or block entry of the sense or antisense oligonucleotide or its conjugated version into the cell. Alternatively, a sense or an antisense oligonucleotide may be introduced into a cell containing the target nucleic acid sequence by formation of an oligonucleotide lipid complex, as described in WO 90/10448. It is understood that the use of antisense molecules or knock out and knock in models may also be used in screening assays as discussed above, in addition to methods of treatment.

### (b) Pharmaceutical Compositions

Pharmaceutical compositions encompassed by the present invention include as active agent, the polypeptides, polynucleotides, antisense oligonucleotides, or antibodies of the invention disclosed herein in a therapeutically effective amount. An "effective amount" is an amount sufficient to effect beneficial or desired results, including clinical results. An effective amount can be administered in one or more administrations. For purposes of this invention, an effective amount of an adenoviral vector is an amount that is sufficient to palliate, ameliorate, stabilize, reverse, slow or delay the progression of the disease state.

The compositions can be used to treat cancer as well as metastases of primary cancer. In addition, the pharmaceutical compositions can be used in conjunction with conventional methods of cancer treatment, *e.g*, to sensitize tumors to radiation or conventional chemotherapy. The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e, arresting its development; or (c) relieving the disease symptom, i.e., causing regression of the disease or symptom.

Where the pharmaceutical composition comprises an antibody that specifically binds to a gene product encoded by a differentially expressed polynucleotide, the antibody can be coupled to a drug for delivery to a treatment site or coupled to a detectable label to facilitate imaging of a site comprising cancer cells, such as prostate cancer cells, Methods for coupling antibodies to drugs and detectable labels are well known in the art, as are methods for imaging using detectable labels

A "patient" for the purposes of the present invention includes both humans and other animals, particularly mammals, and organisms Thus the methods are applicable to both human therapy and veterinary applications. In the preferred embodiment the patient is a mammal, and in the most preferred embodiment the patient is human

The term "therapeutically effective amount" as used herein refers to an amount of a therapeutic agent to treat, ameliorate, or prevent a desired disease or condition, or to exhibit a detectable therapeutic or preventative effect The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of' therapeutics selected for administration. The effective amount for a given situation is determined by routine experimentation and is within the judgment of the clinician. For purposes of the present invention, an effective dose will generally be from about 0.01 mg/kg to about 5 mglkg, or about 0.01 mg/kg to about 50 mg/kg or about 0.05 mg/kg to about 10 mg/kg of the compositions of the present invention in the individual to which it is administered.

A pharmaceutical composition can also contain a pharmaceutically acceptable carrier The term "pharmaceutically acceptable carrier" refers to a carrier for administration of' a therapeutic agent, such as antibodies or a polypeptide, genes, and other therapeutic agents. The term refers to any pharmaceutical carrier that does not itself induce the production of antibodies harmful to the individual receiving the composition, and which can be administered without undue tonicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles. Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g , mineral acid salts such as hydrochlorides, hydrobromides, phosphates, sulfates, and the like; and the salts of organic acids such as acetates, propionates, malonates, benzoates, and the like, A through discussion of pharmaceutically acceptable excipients is available in Remington. The Science and Practice of Pharmacy (1995) Alfonso Gennaro, Lippincott, Williams, & Wilkins.

The pharmaceutical compositions can be prepared in various forms, such as granules, tablets, pills, suppositories, capsules, suspensions, salves, lotions and the like. Pharmaceutically grade organic or inorganic carriers and/or diluents suitable for oral and topical use can be used to make up compositions containing the therapeutically-active compounds" Diluents known to the art include aqueous media, vegetable and animal oils and fats. Stabilizing agents, wetting and emulsifying agents, salts for varying the osmotic pressure or buffers for seeming an adequate pH value, and skin penetration enhancers can be used as auxiliary agents.

The pharmaceutical compositions of the present invention comprise a CA protein in a form suitable for administration to a patient. In the preferred embodiment, the pharmaceutical compositions are in a water soluble form, such as being present as pharmaceutically acceptable salts, which is meant to include both acid and base addition salts, "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like" "Pharmaceutically acceptable base addition salts" include those derived from inorganic bases such as sodium, potassium, lithium, ammonium, calcium, magnesium, non, zinc, copper, manganese, aluminum salts and the like Particularly preferred are the ammonium, potassium, sodium, calcium, and magnesium salts, Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine

The pharmaceutical compositions may also include one or more of the following: carrier proteins such as serum albumin; buffers; fillers such as microcrystalline cellulose, lactose, coin and other starches; binding agents; sweeteners and other flavoring agents; coloring agents; and polyethylene glycol. Additives are well known in the art, and are used in a variety of formulations.

The compounds having the desired pharmacological activity may be administered in a physiologically acceptable carrier to a host, as previously described Ihe agents may be administered in a variety of ways, orally, parenterally e.g., subcutaneously, intraperitoneally, intravascularly, etc. Depending upon the manner of introduction, the compounds may be formulated in a variety of ways" The concentration of therapeutically active compound in the formulation may vary from about 0.1-100% wgt/vol. Once formulated, the compositions contemplated by the invention can be (1) administered directly to the subject (*e.g.*, as polynucleotide, polypeptides, small molecule agonists or antagonists, and the like); or (2) delivered ex vivo, to cells derived from the subject (*e.g.*, as in ex vivo gene therapy). Direct delivery of the compositions will generally be accomplished by parenteral injection, e.g., subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the ex vivo delivery and reimplantation of transformed cells into a subject are known in the art and described in *e.g.*, International Publication No. WO 93/14778. Examples of cells useful in ex vivo applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once differential expression of a gene corresponding to a CA polynucleotide described herein has been found to correlate with a proliferative disorder, such as neoplasia, dysplasia, and hyperplasia, the disorder can be amenable to treatment by administration of a therapeutic agent based on the provided polynucleotide, corresponding polypeptide or other corresponding molecule (e.g., antisense, ribozyme, etc.). In other embodiments, the disorder can be amenable to treatment by administration of a small molecule drug that, for example, serves as an inhibitor (antagonist) of the function of the encoded gene product of a gene having increase expression in cancerous cells relative to normal cells or as an agonist for gene products that are decreased in expression in cancerous cells (e.g, to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic compositions agents includes local or systemic administration, including injection, oral administration, particle gun or catheterized administration, and topical administration. Preferably, the therapeutic polynucleotide composition contains an expression construct comprising a promoter operably linked to a polynucleotide of at least 12,22,25, 30, or 35 contiguous nt of the polynucleotide disclosed herein Various methods can be used to administer the therapeutic composition directly to a specific site in the body For example, a small metastatic lesion is located and the therapeutic composition injected several times in several different locations within the body of tumor Alternatively, arteries that serve a tumor are identified, and the therapeutic composition injected into such an artery, in order to deliver the composition directly into the tumor. A tumor that has a necrotic center is aspirated and the composition injected directly into the now empty center of the tumor. An antisense composition is directly administered to the surface of the tumor, for example, by topical application of the composition. X-ray imaging is used to assist in certain of the above delivery methods.

Targeted delivery of therapeutic compositions containing an antisense polynucleotide, subgenomic polynucleotides, or antibodies to specific tissues can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al, J. Biol Chem (1988) 263:621; Wu et al., J Biol. Chem. (1994) 269:542; Zenke et al., Proc. Natl. Acad. Sci. (USA) (1990) 87:3655; Wu et al., J. Biol Chem (1991) 266:338 Therapeutic compositions containing a polynucleotide are administered in a range of about 100 ng to about 200 mg of DNA for local administration in a gene therapy protocol. Concentration ranges of about 500 ng to about 50 mg, about 1 μg to about 2 mg, about 5 μg to about 500 μg, and about 20 μg to about 100 μg of DNA can also be used during a gene therapy protocol Factors such as method of action (e.g., for enhancing or inhibiting levels of the encoded gene product) and efficacy of transformation and expression are considerations that will affect the dosage required for ultimate efficacy of the antisense subgenomic polynucleotides. Where greater expression is desired over a larger area of tissue, larger amounts of antisense subgenomic polynucleotides or the same amounts re-administered in a successive Protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect.

The therapeutic polynucleotides and polypeptides of the present invention can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters, Expression of' the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., WO 90/07936; WO 94/03622; WO 93/25698; WO 93/25234; USPN 5,219,740; WO 93/11230; WO 93/10218; USPN 4,777,127; GB Patent No, 2,200,651; EP 0345 242; and WO 91/02805), alphavirus-based vectors (e.g., Sindbis virus vectors, Semliki forest virus (ATCC VR-67; ATCC VR-1247), Ross River virus (ATCC VR-373; AICC VR-1246) and Venezuelan equine encephalitis virus (AICC VR-923; ATCC VR-1250; ATCC VR 1249; ATCC VR-532)), and adeno-associated virus (AAV) vectors (see, e.g., WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed

Non-viral delivery vehicles and methods can also be employed, including, but not limited to, polycationic condensed DNA linked or unlinked to killed adenovirus alone (see, e.g, Curiel, Hum. Gene Ther. (1992) 3:147); ligand-linkedDNA (see, e.g., Wu, J. Biol. Chem. (1989) 264:16985); eukaryotic cell delivery vehicles cells (see, e.g, USPN 5,814,482; WO 95/07994; WO 96/17072; WO 95/30763; and WO 97/42338) and nucleic change neutralization or fusion with cell membranes Naked DNA can also be employed. Exemplary naked DNA introduction methods are described in WO 90/11092 and USPN 5,580,859. Liposomes that can act as gene delivery vehicles are described in USPN 5,422,120; WO 95/13796; WO 94/23697; WO 91/14445; and EP 0524968. Additional approaches are described in Philip, Mol. Cell Biol. (1994) 14:2411, and in Woffendin, Proc Natl. Acad. Sci (1994) 91:1581

Further non-viral delivery suitable for use includes mechanical delivery systems such as the approach described in Woffendin et al., Proc Natl. Acad. Sci USA (1994) 91(24):11581. Moreover, the coding sequence and the product of expression of such can be delivered through deposition of photopolymerized hydrogel materials or use of ionizing radiation (see, e.g., USPN 5,206,152 and WO 92/11033) Other conventional methods for gene delivery that can be used for delivery of the coding sequence include, for example, use of hand-held gene transfer particle gun (see, eg, USPN 5,149,655); use of ionizing radiation for activating transferred gene (see, e.g., USPN 5,206,152 and WO 92/11033).

The administration of the CA proteins and modulators of the present invention can be done in a variety of ways as discussed above, including, but not limited to, orally, subcutaneously, intravenously, intranasally, transdermally, intraperitoneally, intramuscularly, intrapulmonary, vaginally, rectally, or intraocularly. In some instances, for example, in the treatment of wounds and inflammation, the CA proteins and modulators may be directly applied as a solution or spray.

In a preferred embodiment, CA proteins and modulators are administered as therapeutic agents, and can be formulated as outlined above. Similarly, CA genes (including both the full-length sequence, partial sequences, or regulatory sequences of the CA coding regions) can be administered in gene therapy applications, as is known in the art. These CA genes can include antisense applications, either as gene therapy (i.e. for incorporation into the genome) or as antisense compositions, as will be appreciated by those in the art

Thus, in one embodiment, methods of modulating CA gene activity in cells or organisms are provided. In one embodiment, the methods comprise administering to a cell an anti-CA antibody that reduces or eliminates the biological activity of an endogenous CA protein. Alternatively, the methods comprise administering to a cell or organism a recombinant nucleic acid encoding a CA protein. As will be appreciated by those in the art, this may be accomplished in any number of ways. In a preferred embodiment, for example when the CA sequence is down-regulated in cancer, the activity of the CA gene product is increased by increasing the amount of CA expression in the cell, for example by overexpressing the endogenous CA gene or by administering a gene encoding the CA sequence, using known gene-therapy techniques, In a preferred embodiment, the gene therapy techniques include the incorporation of the exogenous gene using enhanced homologous recombination (EHR), for example as described in PCI/US93/03868, hereby incorporated by reference in its entirely Alternatively, for example when the CA sequence is up-regulated in cancer, the activity of the endogenous CA gene is decreased, for example by the administration of a CA antisense nucleic acid

### (c) Vaccines

In a preferred embodiment, CA genes are administered as DNA vaccines, either single genes or combinations of' CA genes. Naked DNA vaccines are generally known in the art Brower, Nature Biotechnology, 16:1304-1305 (1998).

In one embodiment, CA genes of the present invention are used as DNA vaccines. Methods for the use of genes as DNA vaccines are well known to one of ordinary skill in the art, and include placing a CA gene or portion of a CA gene under the control of a promoter for expression in a patient with cancer. The CA gene used for DNA vaccines can encode full-length CA proteins, but more preferably encodes portions of the CA proteins including peptides derived from the CA protein. In a preferred embodiment a patient is immunized with a DNA vaccine comprising a plurality of nucleotide sequences derived from a CA gene. Similarly, it is possible to immunize a patient with a plurality of CA genes or portions thereof. Without being bound by theory, expression of the polypeptide encoded by the DNA vaccine, cytotoxic I-cells, helper T-cells and antibodies are induced that recognize and destroy or eliminate cells expressing CA proteins

In a preferred embodiment, the DNA vaccines include a gene encoding an adjuvant molecule with the DNA vaccine Such adjuvant molecules include cytokines that increase the immunogenic response to the CA polypeptide encoded by the DNA vaccine. Additional or alternative adjuvants are known to those of ordinary skill in the art and find use in the invention.

### (d) Antibodies

In one embodiment, a cancer inhibitor is an antibody as discussed abover. In one embodiment, the CA proteins of the present invention may be used to generate polyclonal and monoclonal antibodies to CA proteins, which are useful as described herein. Similarly, the CA proteins can be coupled, using standard technology, to affinity chromatography columns, These columns may then be used to purify CA antibodies. In a preferred embodiment, the antibodies are generated to epitopes unique to a CA protein; that is, the antibodies show little or no cross-reactivity to other proteins. These antibodies find use in a number of applications. For example, the CA antibodies may be coupled to standard affinity chromatography columns and used to purify CA proteins The antibodies may also be used therapeutically as blocking polypeptides, as outlined above, since they will specifically bind to the CA protein.

The present invention further provides methods for detecting the presence of and/or measuring a level of a polypeptide in a biological sample, which CA polypeptide is encoded by a CA polynucleotide that is differentially expressed in a cancer cell, using an antibody specific for the encoded polypeptide. The methods generally comprise: a) contacting the sample with an antibody specific for a polypeptide encoded by a CA polynucleotide that is differentially expressed in a prostate cancer cell; and b) detecting binding between the antibody and molecules of the sample.

Detection of specific binding of the antibody specific for the encoded cancer-associated polypeptide, when compared to a suitable control is an indication that encoded polypeptide is present in the sample. Suitable controls include a sample known not to contain the encoded CA polypeptide or known not to contain elevated levels of the polypeptide; such as normal tissue, and a sample contacted with an antibody not specific for the encoded polypeptide, e.g., an anti-idiotype antibody. A variety of'methods to detect specific antibody-antigen interactions are known in the art and can be used in the method, including, but not limited to, standard immunohistological methods, immunoprecipitation, an enzyme immunoassay, and a radioimmunoassay. In general, the specific antibody will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g, $^{152}$Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the live The antibody may be attached (coupled) to an insoluble support, such as a polystyrene plate or a bead. Indirect labels include second antibodies specific for antibodies specific for the encoded polypeptide ("first specific antibody"), wherein the second antibody is labeled as described above; and members of specific binding pairs, e.g., biotin-avidin, and the like. The biological sample may be brought into contact with and immobilized on a solid support or carrier, such as nitrocellulose, that is capable of immobilizing cells, cell particles, or soluble proteins, The support may then be washed with suitable buffers, followed by contacting with a detectably-labeled first specific antibody Detection methods are known in the art and will be chosen as appropriate to the signal emitted by the detectable label Detection is generally accomplished in comparison to suitable controls, and to appropriate standard

In some embodiments, the methods are adapted for use *in vivo,* e.g., to locate or identify sites where cancer cells are present. In these embodiments, a detectably-labeled moiety, e.g., an antibody, which is specific for a cancer-associated polypeptide is administered to an individual (e,g., by injection), and labeled cells are located using standard imaging techniques, including, but not limited to, magnetic resonance imaging, computed tomography scanning, and the like. In this manner, cancer cells are differentially labeled.

**(e) Detection and Diagnosis of Cancel**

Without being bound by theory, it appears that the various CA sequences are important in cancers. Accordingly, disorders based on mutant or variant CA genes may be determined. In one embodiment, the invention provides methods for identifying cells containing variant CA genes comprising determining all or part of the sequence of at least one endogenous CA genes in a cell. As will be appreciated by those in the art, this may be done using any number of sequencing techniques In a preferred embodiment, the invention provides methods of identifying the CA genotype of an individual comprising determining all or part of the sequence of at least one CA gene of the individual. This is generally done in at least one tissue of'the individual, and may include the evaluation of a number of tissues or different samples of'the same tissue. The method may include comparing the sequence of the sequenced CA gene to a known CA gene, i.e., a wild-type gene. As will be appreciated by those in the art, alterations in the sequence of some CA genes can be an indication of either the presence of the disease, or propensity to develop the disease, or prognosis evaluations

The sequence of all or part of the CA gene can then be compared to the sequence of a known CA gene to determine if any differences exist. This can be done using any number of known homology programs, such as Bestfit, etc. In a preferred embodiment, the presence of a difference in the sequence between the CA gene of the patient and the known CA gene is indicative of a disease state or a propensity for a disease state, as outlined herein.

In a preferred embodiment, the CA genes are used as probes to determine the number of copies of the CA gene in the genome. For example, some cancers exhibit chromosomal deletions or insertions, resulting in an alteration in the copy number of a gene.

In another preferred embodiment CA genes are used as probes to determine the chromosomal location of the CA genes. Information such as chromosomal location finds use in providing a diagnosis or prognosis in particular when chromosomal abnormalities such as translocations, and the like are identified in CA gene loci.

The present invention provides methods of using the polynucleotides described herein for detecting cancer cells, facilitating diagnosis of cancer and the severity of a cancer (e.g., tumor grade, tumor burden, and the like) in a subject, facilitating a determination of the prognosis of a subject, and assessing the responsiveness of the subject to therapy (*e g.*, by providing a measure of therapeutic effect through, for example, assessing tumor burden during or following a chemotherapeutic regimen). Detection can be based on detection of a polynucleotide that is differentially expressed in a cancer cell, and/or detection of a polypeptide encoded by a polynucleotide that is differentially expressed in a cancer cell, The detection methods of the invention can be conducted *in vitro* or *in vivo,* on isolated cells, or in whole tissues or a bodily fluid *e.g.*, blood, plasma, serum, urine, and the like).

In some embodiments, methods are provided for detecting a cancel cell by detecting expression in the cell of a transcript that is differentially expressed in a cancer cell. Any of a variety of known methods can be used for detection, including, but not limited to, detection of a transcript by hybridization with a polynucleotide that hybridizes to a polynucleotide that is differentially expressed in a prostate cancel cell; detection of a transcript by a polymerise chain reaction using specific oligonucleotide primers; *in situ* hybridization of a cell using as a probe a polynucleotide that hybridizes to a gene that is differentially expressed in a prostate cancer cell. The methods can be used to detect and/or measure mRNA levels of a gene that is differentially expressed in a cancer cell. In some embodiments, the methods comprise: a) contacting a sample with a polynucleotide that corresponds to a differentially expressed gene described herein under conditions that allow hybridization; and b) detecting hybridization, if any.

Detection of differential hybridization, when compared to a suitable control, is an indication of the presence in the sample of a polynucleotide that is differentially expressed in a cancer cell. Appropriate controls include, for example, a sample that is known not to contain a polynucleotide that is differentially expressed in a cancer cell, and use of a labeled polynucleotide of the same "sense" as the polynucleotide that is differentially expressed in the cancer cell. Conditions that allow hybridization are known in the art, and have been described in more detail above. Detection can also be accomplished by any known method, including, but not limited to, *in situ* hybridization, PCR (polymerase chain reaction), RT-PCR (reverse transcription-PCR), IMA, bDNA, and Nasbau and "Northern" or RNA blotting, or combinations of such techniques, using a suitably labeled polynucleotide, A variety of labels and labeling methods for polynucleotides are known in the art and can be used in the assay methods of the invention. Specificity of hybridization can be determined by comparison to appropriate controls.

Polynucleotides generally comprising at least 10 nt, at least 12nt or at least 15 contiguous nucleotides of a polynucleotide provided herein, such as, for example, those having the sequence as depicted in Tables 1-6, are used for a variety of purposes, such as probes for detection of and/or measurement of, transcription levels of a polynucleotide that is differentially expressed in a prostate cancer cell As will be readily appreciated by the ordinarily skilled artisan, the probe can be detectably labeled and contacted with, for example, an array comprising immobilized polynucleotides obtained from a test sample (e.g., mRNA). Alternatively, the probe can be immobilized on an array and the test sample detectably labeled Ihese and other variations of'the methods of the invention are well within the skill in the art and are within the scope of the invention.

Nucleotide probes are used to detect expression of a gene corresponding to the provided polynucleotide In Northern blots, mRNA is separated electrophoretically and contacted with a probe. A probe is detected as hybridizing to an mRNA species of a particular size. The amount of hybridization can be quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of'detectable labels can be used such as chromophores, fluorophores, and enzymes. Other examples of nucleotide hybridization assays are described in WO92/02526 and USPN 5,124,246

PCR is another means for detecting small amounts of target nucleic acids (see, *e.g,* Mullis et al., Meth. Enzymol (1987) 155:335; USPN 4,683,195; and USPN 4,683,202). Two primer oligonucleotides that hybridize with the target nucleic acids are used to prime the reaction. The primers can be composed of'sequence within or 3' and 5' to the CA polynucleotides disclosed herein. Alternatively, if the primers are 3' and 5' to these polynucleotides, they need not hybridize to them or the complements. After amplification of the target with a thermostable polymerase, the amplified target nucleic acids can be detected by methods known in the art, e.g., Southern blot. mRNA or cDNA can also be detected by traditional blotting techniques (e.g., Southern blot, Northern blot, etc) described in Sambrook et al., "Molecular Cloning: A Laboratory Manual" (New York, Cold Spring Harbor Laboratory, 1989) (e.g., without PCR amplification). In general, mRNA or cDNA generated from mRNA using a polymerase enzyme can be purified and separated using gel electrophoresis, and transferred to a solid support, such as nitrocellulose. The solid support is exposed to a labeled probe, washed to remove any unhybridized probe, and duplexes containing the labeled probe are detected.

Methods using PCR amplification can be performed on the DNA from a single cell, although it is convenient to use at least about $10^5$ cells. The use of the polymerase chain reaction is described in Saiki et al. (1955) Science 239: 487, and a review of current techniques may be found in Sambrook, et al Molecular Cloning: A Laboratory Manual, CSH Press 1989, pp. 14.2-14.33. A detectable label may be included in the amplification reaction. Suitable detectable labels include fluorochromes,(*e.g.* fluorescein isothiocyanate (FIIC), rhodamine, Texas Red, phycoerythrin, allophycocyanin, 6-carboxyfluorescein (6-FAM), 2',7'-dimethoxy-4',5'-dicloro-6-carboxyfluorescein, 6-carboxy-X-rhodamine (ROX), 6-carboxy-2',4',7',4,7 -hexachlorofluorescein (HEX), 5-carboxyfluorescein (5-FAM) or N,N,N',N'-tetramethyl-6-carboxyrhodamine (TAMRA)), radioactive labels, (*e.g.* $^{32}P$, $^{35}S$, $^{3}H$, *etc*), and the like The label may be a two stage system, where the polynucleotides is conjugated to biotin, haptens, *etc.* having a high affinity binding partner, *e.g.* avidin, specific antibodies, *etc.*, where the binding partner is conjugated to a detectable label. The label may be conjugated to one or both of the primers. Alternatively, the pool of nucleotides used in the amplification is labeled, so as to incorporate the label into the amplification product.

The detection methods can be provided as part of a kit Thus, the invention further provides kits for detecting the presence and/or a level of a polynucleotide that is differentially expressed in a cancer cell (*e.g.*, by detection of an mRNA encoded by the differentially expressed gene of interest), and/or a polypeptide encoded thereby, in a biological sample. Procedures using these kits can be performed by clinical laboratories, experimental laboratories, medical practitioners, or private individuals. The kits of the invention for detecting a polypeptide encoded by a polynucleotide that is differentially expressed in a cancer cell may comprise a moiety that specifically binds the polypeptide, which may be an antibody that binds the polypeptide or fragment thereof. The kits of the invention used for detecting a polynucleotide that is differentially expressed in a prostate cancer cell may comprise a moiety that specifically hybridizes to such a polynucleotide. The kit may optionally provide additional components that are useful in the procedure, including, but not limited to, buffers, developing reagents, labels, reacting surfaces, means for detection, control samples, standards, instructions, and interpretive information. Accordingly, the present invention provides kits for detecting prostate cancer comprising at least one of polynucleotides having the sequence as shown in Tables 1-6 or fragments thereof

The present invention further relates to methods of detecting/diagnosing a neoplastic or preneoplastic condition in a mammal (for example, a humans) "Diagnosis" as used herein generally includes determination of a subject's susceptibility to a disease or disorder, determination as to whether a subject is presently affected by a disease or disorder, prognosis of a subject affected by a disease or disorder (*e.g.,* identification of pre-metastatic or metastatic cancerous states, stages of cancer, or responsiveness of cancer to therapy), and therametrics (*e.g.*, monitoring a subject's condition to provide information as to the effect or efficacy of therapy).

The terms "treatment", "treating", "treat" and the like are used herein to generally refer to obtaining a desired pharmacologic and/or physiologic effect The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

An "effective amount" is an amount sufficient to effect beneficial or desired results, including clinical results. An effective amount can be administered in one or more administrations.

A "cell sample" encompasses a variety of sample types obtained from an individual and can be used in a diagnostic or monitoring assay The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides. The term "cell sample" encompasses a clinical sample, and also includes cells in culture, cell supernatants, cell lysates, serum, plasma, biological fluid, and tissue samples.

As used herein, the terms "neoplastic cells", "neoplasia", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation (i.e., de-regulated cell division). Neoplastic cells can be malignant or benign

The terms "individual," "subject," "host," and "patient," are used interchangeably herein and refer to any mammalian subject for whom diagnosis, treatment, or therapy is desired, particularly humans. Other subjects may include cattle, dogs, cats, guinea pigs, rabbits, rats, mice, horses, and so on. Examples of conditions that can be detected/diagnosed in accordance with these methods include cancers. Polynucleotides corresponding to genes that exhibit the appropriate expression pattern can be used to detect cancer in a subject. For a review of markers of cancer, see, *eg,* Hanahan et al. Cell 100:57-70 (2000).

One detection/diagnostic method comprises: (a) obtaining from a mammal (e.g., a human) a biological sample, (b) detecting the presence in the sample of'a CA protein and (c) comparing the amount of product present with that in a control sample. In accordance with this method, the presence in the sample of elevated levels of' a CA gene product indicates that the subject has a neoplastic or preneoplastic condition.

Biological samples suitable for use in this method include biological fluids such as serum, plasma, pleural effusions, urine and cerebro-spinal fluid, CSF, tissue samples (e.g, mammary tumor or prostate tissue slices) can also be used in the method of the invention, including samples derived from biopsies. Cell cultures or cell extracts derived, for example, from tissue biopsies can also be used.

The compound is preferably a binding protein, eg., an antibody, polyclonal or monoclonal, or antigen binding fragment thereof, which can be labeled with a detectable marker (e.g., fluorophore, chromophore or isotope, etc). Where appropriate, the compound can be attached to a solid support such as a bead, plate, filter, resin, etc. Determination of formation of the complex can be effected by contacting the complex with a further compound (e.g., an antibody) that specifically binds to the first compound (or complex) Like the first compound, the further compound can be attached to a solid support and/or can be labeled with a detectable market.

The identification of elevated levels of CA protein in accordance with the present invention makes possible the identification of subjects (patients) that are likely to benefit from adjuvant therapy. For example, a biological sample from a post primary therapy subject (e.g., subject having undergone surgery) can be screened for the presence of circulating CA protein, the presence of elevated levels of the protein, determined by studies of normal populations, being indicative of residual tumor tissue. Similarly, tissue from the cut site of a surgically removed tumor can be examined (e.g., by immunofluorescence), the presence of elevated levels of product (relative to the surrounding tissue) being indicative of incomplete removal of the tumor. The ability to identify such subjects makes it possible to tailor therapy to the needs of the particular subject. Subjects undergoing non-surgical therapy, e.g., chemotherapy or radiation therapy, can also be monitored, the presence in samples from such subjects of elevated levels of CA protein being indicative of the need for continued treatment. Staging of the disease (for example, for purposes of optimizing treatment regimens) can also be effected, for example, by biopsy e.g., with antibody specific for a CA protein.

**(f) Animal Models and Transgenics**

In another preferred embodiment CA genes find use in generating animal models of cancers, particularly lymphomas and carcinomas. As is appreciated by one of ordinary skill in the art, when the CA gene identified is repressed or diminished in CA tissue, gene therapy technology wherein antisense RNA directed to the CA gene will also diminish or repress expression of the gene. An animal generated as such serves as an animal model of CA that finds use in screening bioactive drug candidates. Similarly, gene knockout technology, for example as a result of homologous recombination with an appropriate gene targeting vector, will result in the absence of the CA protein. When desired, tissue-specific expression or knockout of'the CA protein may be necessary.

It is also possible that the CA protein is overexpressed in cancer. As such, transgenic animals can be generated that overexpress the CA protein Depending on the desired expression level, promoters of various strengths can be employed to express the transgene Also, the number of copies of the integrated transgene can be determined and compared for a determination of the expression level of the transgene Animals generated by such methods find use as animal models of CA and are additionally useful in screening for bioactive molecules to treat cancer

**(g) Characterization of CA sequences**

The CA nucleic acid sequences of the invention are depicted in Tables 1-6 The sequences in each Table include genomic DNA sequence, sequence corresponding to the mRNA and amino acid sequences of the proteins encoded by the mRNA for both mouse and human genes. The different sequences are assigned the following SEQ ID Nos:

Table 1 (mouse gene: Fscn1; human gene SNL)

Mouse genomic sequence (SEQ ID NO: 1)

Mouse mRNA sequence (SEQ ID NO: 2)

Mouse coding sequence (SEQ ID NO: 3)

Human genomic sequence (SEQ ID NO: 4)

Human mRNA sequence (SEQ ID NO: 5)

Human coding sequence (SEQ ID NO: 6)

```
MOUSE SEQUENCE - GENOMIC (SEQ ID NO: 1)
CCTCTCAAACTCTTGGTAATTCTCCTGCTTCGGCACGCTGGGGACTGGGACTACACCTGTGTGCCACCATGGCTGGCTATTTCTCT
CTCTTGAACACTGGAAGAGTGCTCAGAGTTTACTGGATCTGGGAGAAGCTCGAGGCTGGGTTATGGAAGCCGGCTCTGCTTGCTCC
AGCTCAGGGAGGAGTTGCCTGAGGGCCAGGCACTTCGAAGGACAGATGGGACCCAAGGCCAGACACTGGGCCCCAGCTCACCAGAA
GTGGAGCCCTGACTGTCTCTGAGAGGGTAAGCTGGGGTGGCTGCCAGGCGGGCAAGGCCAAAGCCTGGCAGCAGCCGGTGGCCCCT
CTTCTGGCAGAGATATCTTGGCATGAGCCTGGCTCTGCCCATGACACTAAAGTGCCCTCTTAATTAGCCAGGCTCTTGCCAAGCAC
TGGCCACGATTGCCTTGTTTCACAGAATTCACCTTGGACTGGCACAGATGGGGAGGGTGGATAGCCCGGTGTTTTGTCTTTCTTCT
AGGGGAGCTGGGCTCAAGGGCAGGACTCCTGGGCCAGCCCAGGTTTTTCCCTCAGAAACCACAGCATGAATACTGGCATGATGGAG
CACACCTGTAATCAGCAGAAATTGAGACAGGAAGATTGTTGAGAATTTGAGGCCAACCTAGGCTAAATAGGGAGACCCTATCTCTA
CACACTCCCCCTCCCCCCGCCCCTGGAAATGCTGGAGGGTCTGGGGAAGATGGCTCAGTGGTTAAGGGCACTTGCTGCTCTTGCAG
GGGACCCAAGTTTGATTCCCAGCATCTATAACTTGGTGGCTCACAATTCCAGTTCCAGGCACTCTGACACCTTCCTGTGGTCTGCA
AGGCACCTGCATTCATTCTCACGTGCATGCGCACACACATGCGCGCGCACACACACACACACACACACACACATCTCATAAA
```

TACAAATAAAATAAATCTTGAAAACAACAGTGACAACAACACATGCTGAACATGGTGGTGCTCCTGACATGGTGGTGCTCCTGACA
TGGTGGTGCTCCTGACATGGTGATGCTCCTGACATGGTGGTGCTCCTGACATGATGGTAAGCCTGACATGGTGGTGCTCCTGACAT
GGTGGTGCTCCTGACATGGTGATGCTCCTGACATGGTGGTGCTCCTGACATGGTGGTAAGCCTGACATGGTGGTAAGCCTGACATG
GTGGTGCTCCTGACATGGTGGTGCTCCCTGCACTGACAGTTCTGAGGAGGTGGAGATAGGAGGATCCAGACTTGGAAGCTTGCCTG
AGATAGACCCTGTCTCAGAATAGGCTGGAGGCTGCCTCTGCCCTTTTGCTTCTATCTCTTTTTCCATATGTGTGTGTGCGTGTGTG
CATATGTGTATGCATATTTGTAAGTATATGTATATAAATAAACACACACACACACAAACTTACTCTGTATACTAGGCTGGCCTTGA
ACTCAGAGACCTGCCTGTTTCTGCCTTCCCAGTGCTGGGATTAAAAGCGTGTGCCATGGGCTGGTGAGATGGCTCAGTGGGTAAGA
GTACCTGACTGCTCTTCCAAAGGTCCAGTGTTCAAGTCCCAGCAACCACATGGTGGCTCACAACCATCCGTAACAAGATCTGACGC
CCTCTTCTGGTGTGTCTGAAGACAGCTACAGTGTACTTAAATAAATAAATCTTAAAAAAAAAAAAAAAAAAAAAAAAAGGCGTGCA
CCACCACTGCCCGGCTAGTTAGCTTTATCTTCCGTGGATGTTTTGTCTGCAGTATGTCTGAGTGAGGGTGTCAGATCCTCTGTAAC
TGGAGTTATAGACAGTTGTGAGCAGCCATGTAAGTGCTGGGAATTGAATCCGGGTTCTCTGGAAGAGCAGTTCATGCTCTTAAGCA
CTGCGCCATCTCTCCAGCTGTCCTTATTTATTTTTAAGGGTCTCTTGTAGCCATACAGGCCAGAAACCCACCATTAGCCACGGACC
TTGAGCTCCAAGTGCTGGGTGACAGGCCCGCATCTCTCCTGGTTTGTGATCTGCTAGGGATGGGGTCTAGGACCTCACACAGGCTG
CATAAGCATTCCCCTCACCCACCCTCATCCCCACCCAGTCCTGGACAGGCTCTGGCTGTGTAGGCTGGTCTCCTCCTTTCTATCCC
CCTGCCTCAGCCTCCTGAGTGCTGAGGNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNCTGTGGTGGCTCCCGAGTGCTGTCATCCCAGCACTGGGGAGGTTGAAGCAGGAAGGAGAA
TCACAGGTTCAAGGCCAGCTTGGTTTACTTGAGACTCTGTCTCAAAAAGACCAAACCAGGGACTGGGAGGATGGCCAGTAAAATGC
TTTTCCAGCTATCGAGGGAACCTGAGTTTCATCCTGGAACTCATACAAAAAGCTGGCTGTGGTCAGGTGGCGCATGCCTTTAATCC
CATCACTCAGGAGACAGAGGCAGGCAGATTCTTGAGTTCGAGGCCAGCCTGGTCTACAGACTGAGTTCCAGGATAGCCAGGTCTAC
ACAGAGAAACCCTATCTTGGAAAACAAAACAAACAAAAAAAACTAGGTGTGGCTAGCCTGTAACCCCAGTGCTGGGTAAACAGGGG
CAGGTGAATACATCTCCGGGATTCGCCAGCCTGTCCTATCTATGAGCTCTAGGTCTGGGGAGACAGCCTGTCAAATCAATGCAAGC
AGGTGACAACCGAGGAAAGACACCCAAGGTTGATGCACGGGCAAGTGTGCACACACACACACCACAGCAAACAAAAACTAACAAAA
CCCAAACCATATTTTGCTGTGGTGGTGGTTGTTAAATTTTATTTATTCTGTGTGTATGGGTATTTCACCTGCATGAATATCTACCT
GTGTAGCATATACATGCCGGGTACTTTCGGAGGTCAGAAGAGGACATCAAATCCCGTAGAACTGGAGTTACAGAAAATGAACTTGT
GGCTTCTGGGAATCTAACTTGGATCCTCTAAAGGACAGGCAGGACTCTGAATTCTTGAGCCACAGATACAGCTTTCCAAACCCATG
TTTTGTAAGAGCCACCAGGCCTGAAAACTCTGGGCCATGTCCCGTTTGTTGTGTCTAGGCCTCAGTTTCCTGGAAGGCAGAGAATG
CGTAGGTCCGTTGTTAGTGGAGACAGCAGTGGTGGTAACAGCCTGGTCTACCCTCTTGGTCAGAGGGAGGCAGGCGGAGAGTAACT
CAGGGCCTGGGGGCAACACCCAGCTGGGCCTGGGGCTAGTGGGGGCTCAGCCAGGCAGGACGTGGGTCCTCCTACTTCCTAAGCTC
TGATGGGCAAGTGGTGCCAGTGGTACCAGGCTGCCCTGAGGGGCCCTTAGAGCAGGTGACCACAGAGCCACAAAGAGGCTATTCAT
GGCCTCCGGTTCACGAGGCTGCCCCTTTATTGAGGGCTTGAACCACAGAAAGCTGTGTGGTCTGCAGGGAAGCTAGTTCTGAGCTG
TCTGGCCAGCTACAGTAGTGTGTGTGTGTGTGTGTGTGTGTATACACATGGTACTTTTTTCTATCTATCTATCTATCTATCTATCT
ATCTATCTATCTATCTATCTATCTATCTTTGTGTCTATGTATCTAATAATAAATTGATCTATTTAACTATGTAGATATCAATCCAT
TGATCTATGTATCTATCAACTGATGTGTATGTATGTATGGATGTACCTACATGCGTACTTGAGGCAGGACTTAAAGTAGCCCAGGC
TAGCCTTACCACATTTCATAGCAGAGGATGGTCTTGAACTTCAGGTCCTCCTGTCTCTACCTCGTGAGTGCTGGGATTACAGGTTT
GTGCCACCACGCCTGGCTTAGGAGGTTCTGTGGTTAGAATCCAGGATTTCACTATCAACTGGGCCACATCGTCAGCCATATTCAAT
ATGCTTTCTTTTACATTCTTACTTATTTACTACAAATGGGTCCCAGGAACCAAGGTCGGCAAGCCTGATGGTGAGTGCTCTTCCCT
GCTGGCTGACTTATTTGCTCTTTTTTTTTTTTTTTTTTTTTTCTAGACAGGGTTTCTCTGTGTAGCCCTGGCTGTCCTGGAACTCA
CTTTGTAGACCAGGCTGGCCTCGAACTCAGAAATCTGCCTGCCTCTGTCCCCAACCCCACCCCCAGTACTGGGATTAAAGGCATGC
GCCACACGCCCAGCTCTTATTTGCTCCTTTGAATTAAGGTCTCATGTACACAAGGCTGACCGTTCATGCACAATACAGCTAAGGCT
TGCTTTGAATTTCTCAGTTTCTTTCTTCTACCCAACAGTTTCTGAGATTTCAGGTCCGTCCTATGTCATGCCCAGGTTTTTTGTTT
GTTTGTTTGTTTGTTTTTTGGTTTTGTTTTGCTGTTTTTCAACACAGAGTTTCTCTGTGGAACCCTGGCTATCCTGGAACTCTGTA
TACCAGGCTGTCCTTGAACTCAGAGACCCTCTGCCTCCCTAGTGCTGGGTTAAAGGTCCCTGCCATCACCGTGAAACTTAAGCCCA
GCTTCTCATTTGGTGGCTGGAAATTTGCCTGCCTCTGCCTCTCTCCAGCTGCCTAGCACTGATGTCTGGTTGAGAGCAGGGAAAC
TGAGGTGCCGCTAGGACAAGATCTCATGCAACCGTGAACAGCCCTACTGAGCCATCCTCCTGGGACAGCCTCTCAGGCCAGGCCTT
CTGTGTCTTCTGCTATAGTAGCAGTACAAAGTTGACACAACCTTGCTCTTCCCAGCAGCTGGGAGTTCCTGAGAAGTGTAAATGAG
GTTTCCAGGAAAGCCAAGAGGTGAGAGACTGAAAAGTTGGAGGGATAAGGGGGTGCCCATCAAGCTGCAGTGGAGACTTGTTATCT
GTTGCGCATCCCTGACATCTCAGCAGGAGGATCAGGAGTTCAAGGGCATCCTCAGCAACATAGTAAGTCTATGGAGCTAGCCTGGG
CTACACAGATGCCATTACAAAAAAAAAAAAAAAAAGTAAAAAAAAAAATCTTGGCATATGGGGGATGGGTGGGTGAACTGTGTAGCCC
AGGGAGTCACTACTGGTTCCTTTGGAAGTCCCTGTTCTGCATGGAGATCATGGAGATTCTGGGGACACTCCAGGAGTCCTCCTTCA
GGGAACTAAGTTGGGGAGCAGGGCAGCCTATCCTGTGGCCTTATTTCCTGTCTGGTGCAAGCGCCAGGCAAGGCATCCAGCCAGGA
GCGACTCAGGCCTGACCTCCGCTAACTGTTCTGTTTTGTGCTGCACCCTCATCTCAGCAGGGATCAGGTGGCACTTGGGTCCCCAG
GGGATCCAGGGACACAGTGTCCATGTCCCCATCTTTCCCCACAGGGACCTGGGGCAGGGCCGAAGAGGAGACCTCGTGATGTGTAC
AGGTGTAGCTCATCCCTCCGAGGACTCAGGGGACACTTGGGGAAGGTGTGACACAGAGCAGGCTGGAGCTCTGTGCGGCTCTTTAT
TAAGTGTGCCTACAACAGGGGCAGAGACACAGCAGCGACAATGATAACAGCCCACTCCCAACCGAAGCTCAATGGTTACCAGACTC
AGCCAACTGCAGAATTCCAGTCCATAACGGCACCTCCTTTCCTTTCTCAGGAGCTTACCAGAGAGGTCTCACCCCAAGCCTTCACC
CCATCTAGAGCAAATGCTTATATCCCCCATGTTTGCTCAGAGCAGAGCATGGAGGGACCTTCCTGTCACCAGAGGCAGAGGCATTC
TGTATACGTGCATACACGCCTCTGTGTGTGTGTGTGTGTGTGTGTGTGTGTATTTGGTACAGATCTAAGCATAGAAAATTGAAAAG
CATTGAGGTTGGTGATTGAAAGGCTCACTGATGGCAAGTCGGGTTCGAGTGCGCTAACTGTATGACTCTATGGATCCGAATATCA
TGCTGTTTTGGAAAGGAATGAGGGAGCTACTGTTTCTAAGGAAGAGAAGAATGGGCACCAAGACATAGGGAGGGAAGGTGGGACAG
TAAGCACTTACAGCCGAAGCCCAAGACCTGGAACCCAAGGGGAAAAACAGGTGTGGCCAGGCTTGGTGGCACGCGCCTTTAGTCTC
TGAGTTCAAGTCCAGCCAGGATTACATAGCAAAACTTTCCCTCAAGAAAAAAGGTAGGGGCTGGAGAGATGGCTCAGCAGTTAAGA
GCTCTTCCGAAGGTCCTGAGTTCAAATCCCAGCAACCACATGGTGGCTCACAACCATCTGTAATGAGATCTGACGCCCTCTTCTGG
TGTGTCTGGAGACAGCTACAGTGTACTTACATATAATAAATAAATAAATCCTAAAAAAAAAAAAAAAAAGAAAAAGAAAAAGAAAAA
AGGTAGAGGAGGAGGAAGAGGAGGAGGAGACGGCAAGACAAAACTAACTACATAGCTGGGGGAGGTGGTGCTTGCCTATAATCCCA
GCACTTGGAAGGCTGAAAGAGGAAATCAGGAGTTCCTATGCAGCTTTGGCTTATGTGAGCCTTTCTCATAAAACCAAGAGCAAAAA
AGCAAAACATGAGGTGGTAACTCAGCTGGAGAGTGCTTGTCTAGATTCCCTCAGTGAGGGGCTGGGGTGTGACTCAGTGCCTAGAA
TCCCCCAGGGAGGGGCTGGGGGCGTGGCTCAGTGGTAGAGCCCCTGCCTAGAATCCCCCATTGACGGGCTGGGGTGTGGCTCAGTA
ACAGACTGCTCACCTAAGATGTTCAAGGCCATGACTTTCATTGAAAACATCACAAAACAAACAAAACCATCAGTGAGGTTGAATGT
GTATCCATGTTTGCTTGTCTCCATTTAAAGAACACCTGGAAGATCTCTAGCTGCTGGTGGAGGCGGGAGGGTGAAGGCTGCTTCAT
CCTTTGTATTTGAACTGTGTGGCTGTGGGTGTATTATCTGTTCCCAAGGACTGCACATGAAATGAGAACAATGAAGGCTTCGGTTC
TGCTGAGATGAACCTCAATTCTCCACAGATGATCTCACCCAGAGCCAGATGGAAGTCCCCCAACATTTAAGCCTCTGTTCTGAGTG
CTGGACCCAAACTCATCTTAAGGGAAAACCCAAAGAGCCTTTCTGTGGTTTCCTTCCACAACTGTCTTTCAATGTGTGTATGTGTG
CACGTGTGTTTGCTTGTGTGTGTGTGTGTGTACTATGTGCATGTGTGTGAGCATGTGTGTATATGTATGTGCATGAGTGTGTGTGT
GTGTGTGTATACATGTGAGCATGTATGTATAGGCCAGAGGTTAGTGATGGGTAACTTTCTCCAGAGCTCTCCACCTTAGTTTTATT
GTCTGTGTGTGAGCGGCTGTGGGCACTGTACACCACAGCACATGTGTGGAGGTCAGAGAACAACTTGTGGCTGTCACTTCTCTCCC
TCTGTGTGGGTCTAAGACCTGGATCCAGCGCTTTCGCTTGCTTGAGACATCTCGTTGACTAGCTCCTCCGTTGTTAACACAGGGTC

TCACTATGTAGGTCCAGTTATCTTGGAATTTGCTCTGTAGACCAGGCCGGCATCAAACTCAGAGATAGACCTGCCTTTGCCTCCAG
AGTACTGGGATTAAAGGTGTGCACCACTACCACCCAGTTTAAAATTCTAAAAAGATTTGTGGGTGCAGGGATTTGAGGAGGCCAGG
AGAGACTGTTGGATCTCTGGGAACTGGAGTTAGAGGTGGTTGTGAGCCCCCACGGGTGCTGAGAACTGAACTAGGGTGCTTGAGAG
GAGCTCACTCACTGCTGAGCCCCCACTTTAGATTTGAGTCCCTCGTACTGAAGCTGAGTTCAGTGATTTGGCTGAGCTGACCGCTC
AGGGTGGGCTTTTCGATGTGCACCACAGGCCTGGCTTTATAGGTGAGGTCATGCTTGCACAACTGGCCTTTTGGTGACTCAGCCAA
CTCCCCAGACCTTTTTTCTTTTGCATTTACGTGTGTGTGTGTGTGTGTGTGTGTGTGTGTGTGTGTGTGTGTACATGAAGTGAGAG
GGGGCAGTCTGGGAAGGGTCAGTGGACAACTTAAGGGAGTTGGTTTTGCCTTCCATCATGATGGTCTAGAGACTAAATTCAGCCCC
TCAGCCTGGGAGCGTGCCTTTACCCTCCGAGCTCCCAGCTTTGGGTTTTTGAGGCAAGGTTTCATATAGCTTAGGCTGGCCTGGCA
TTCAAGGTCATCTCAGAGGCCTAGGATGACCTTGTGTTGTCGGTTCTCCGGCCTTTACCTCTGGTGTGCTGGCGTTTGCCTTACCA
CACCCAGGCATCCCCCATAGCTTAAGCCTAAAGCTCTCGGATGGCCAGCACGCCCATGCCCTGACGTGAAGTTCAGTGACTAATCA
ATACAGAGCGTTCACGGAGCCCTGACACATAGCAGCATTGACTTAACCTTAACAGCCTGGTGTGTAATGAGGCCAACTTGTCCGAT
TCTGCCCACGATGCGATGCTCTGGGTTAAACAGGTTTGAACAGAGTACCGGATGGCAGGGAGGGCACCCTCAGTACAGGGTTTTGG
CTGTTACTGCTTCCAGTCTCCTGACCTGCTCCCCCAAGGCTGGTCTTAAACTCAAAGCAATCCTCCTGTCCCTGCTTCTCAAGTTC
TGGAATTACAAGTGTGACTCACTGTACCCAGGAATATGTGTCTTTATTACAGAGGAAGGAGAGCTCAGAGAGTGAGAGTAACCTGT
GCAAAGTCCCACAGCAGGAGAGGGTGGCTGGCTATCGGCCGTGAACTTGGTGACCTTTCCTGTGACCTCTAAGGGAGGAACTAGAT
GTCCCAGCAAGGATTCTAGGGACTTGTCAGGACTGACCTTTGATGATGGGAAAGCCATGCCATCTCCCCTTCCCCTTTGATGGTCG
CTGGAGGGGTTTGCCTGGGACAGAGATGTGTTTGGACACAGATCACCGTGAAGACTGGTCAGTTTCAGTACCCTGCCCAGCCTGTG
GCTGGGAAGGGCCAGGGGCCCAGATCTTGTGGGCAGCCTGGGGTAGGGCCAGCATTCCAGGGGCCTGCCTGGCATGTGGGGAATGT
CCCAGGAGAAGTCTCAGTCCTCCAGAAACTCAAAGGGAAGTGTTCTTACGTGGGGTGGTGTGTGGTGGGAGGTGGGAGGTGGTGTT
TGTGGGAATGTCCCTCTGGTCTGGAACCCTGAGGGGCTGCAGTTAGGAGGTACATTGGGGCTGTGCCATTCTAGGGAGCTGACCTG
CTTTTCTGATGTCTTCATAAACAGAGACCACTATTCCTGCCTTACCTACCCTGCTTCCTGGTAGACCAGCACCCCGGGGCTTGAGC
ATCACCACAGTAGGGACTTTTGTGGGCCACTGACTTGGGCCACAGTTACCACAATTTATTTAGAGTCAGCAAGGTGGCTGTACTCT
ATGCTGACCCGGGAATAGAAATCTCGGTTTCTGCTCAGGAGGCCCAGGCTACAAGGCCTTGAAAAACCCTGAGGCGGGCGGGGCAG
GTAGGGCTCTGCATGCGCCTCTCTAGAAATTAACCTCCTTTCTACCCCAGACCCTTCTGGCGTCTCTGGCTTCTTCAAGGACCGGC
TCTAAGGCTACTTCCTGGTACACCCTGGACCCTCCCTCCATCCTGAAGGGATCATTATCTTAAACCTGGTCCTTCCCATCGACTGG
GTTTGGGGGTAGGGGTGTTTCAGGATGGCCTCAGAGAGCAGGAGAAAGTTGGAGATCAGCCAACTACTGCAGAGCACGACTGTGGA
GGAACAGTTAAATGGACTAGCAAACACGAACGGGCCAGAGGCAGAAGGACTGCTATGAGTTCGAGTCCAGCCTGAGCCACACAGAT
ACTTCAAGCCTAGTCTGAGTTACAGAGTGAGACCCGGTCTTAAACCCCAGAAAAACACTCCGGGGAAGCGCAGGGGACCGGGCGCG
CGCTGCAGAGCCCCCTCCCCGGCAGGCCCGGGTAGGGGCGTGGCCACGGTGACGTCATCCTCCTATAAAACCCTGGCGCCGCCGG
GCTGGCTTTGTGGAGAACTGCAGCGGGCTAAGCCGTGTTGAACAAAGGAGGTCGGCACAGCTATCCAAGCTCCCGGGGCCACCGG
GCCGCCCTCCGCCACCATGACCGCCAACGGCACGGCAGAGGCTGTGCAGATTCAGTTCGGGCTCATCAGCTGCGGCAACAAGTACC
TGACAGCCGAGGCGTTCGGGTTCAAGGTGAACGCATCCGCTAGTAGCTTGAAAAAGAAGCAGATCTGGACGCTGGAGCAACCTCCC
GATGAGGCGGGCAGCGCGGCCGTGTGTCTGCGCAGCCACCTGGGTCGCTACCTGGCCGCCGACAAGGACGGCAACGTGACCTGCGA
GCGCGAGGTGCCCGACGGCGACTGCCGCTTTCTCGTCGTGGCGCACGACGACGGCCGCTGGTCGCTGCAGTCCGAGGCTCACCGGC
GCTACTTTGGCGGCACCGAGGACCGCCTGTCCTGCTTCGCGCAGAGCGTGTCGCCGGCCGAGAAGTGGAGCGTGCACATCGCCATG
CACCCGCAGGTTAACATCTACAGCGTTACCCGCAAGCGCTACGCGCATCTGAGCGCGCGGCCGGCCGACGAGATCGCGGTAGACCG
CGACGTGCCTTGGGGCGTCGACTCGCTCATCACCTTGGCCTTCCAGGACCAACGCTACAGTGTGCAGACGTCCGACCACCGCTTCC
TGCGCCACGACGGGCGCCTTGTGGCACGGCCGGAGCCCGCCACGGGCTTCACGCTGGAGTTCCGCTCCGGCAAGGTGGCCTTTCGC
GACTGCGAAGGTCGCTACCTGGCTCCGTCCGGGCCCAGCGGCACCCTCAAGGCTGGCAAGGCCACCAAGGTGGGCAAAGATGAGCT
CTTCGCCCTGGAACAGAGCTGCGCTCAGGTGGTGCTGCAGGCGGCCAACGAGAGGAACGTGTCCACGCGCCAGGTGAGTTGGGGA
CATGTCCCCTCCCTTGGGTCCTGACCCAGTGCACAAAAGCATCTCTGCACTCCTTCTATTTCCCTATTTCCTCCTGGGCTCCCTTT
AGGCTCACAGGCCTCTGGGCTCCGCCATGGGGAGGTGGGCTTCTATTTTGAAAGACCTGATCTCACCAGATAGCCTTGGCTATCCT
GGAACTCGCTATGTAGACTGGGCTGGCCCCGAACTCGCAGAAATCTTCCTTCCTGTCTCTTGTGCTGGGAATAAAGGCATGTACCA
CCCCTCTGTTCAGGACGTAGGCTTCTTCTGCTGGGAGAAGGTGACGTGGGGAAGTGGGTAAGGCCTAACACATTCTAAAGCAATAC
TGTCCACCACCACCCCCCCACCCCCCCACCCCCCCGGCTGGGGGCGTGGGACTCCATAGACCTAGCTTAGATCTTCTCCGTTTCCT
CTTCCTTCCCAGTCTGAGCCATCCCCATTCATGAGCCGATTCTTTAGGATAGGCTGTCCCAGGTCTTGGAGAGGAAGGGTTCCCCC
TACACCCCCCCTTGTTCATTTCCTCGTGCGTCCTCTCCCGTTTCGCGGGCCTTTGGCTGGAGGGCCAGGGGACAGTCACCCCTGCC
CTCCCCGCCTTAAATGGCGAGATAGAGGAGGTTGGAGGGGCTTTTGATCCCGGCGGGGTGTGCTGGGCGGGGTCGGCCCTGCGACT
CTTGGTGGTGGTGGGGTTGTCCGTAGGGGCTTCCCCTCCCCCACTCCATCTCCCCTCGCTAAGGCCTGGCGCTCCGGGCCGCGGCC
TTTGTGAGCAGGGGGGCGGGTCGCCACGGCTGGGCCCTCCCTCGTCCCCTTTGTCCTGCTAAGGCTCTGCAGATGGGAAAACCAGA
TGCGGCGGAGCTGGGGGAGGGGATCGGCTTAGGCGGGTCCCTCTGCGGAAGAGCCACCCCCAACCCTCCAGGCGTTGTCCCTGGTC
CAGGGTTCATTGAGAAGGCGCGGAGTGAAGCCGCCTGGCTCTTCCTTCGCACCCCGGCTGGCAGGCGGCGTGGCGCGGGTGGCTGC
GCGCCAGGCGCCCTCCCTGCCCTCCTATGCACGCCTTGCGGTCCGGCACTCGGCGGAGTGTCCCTCCAAAAGCCTCTACTCTCGAG
TGAACCGGTCCCCAAGGCCTCTCTGGCTGGGAACAACCGTTTGCAGCTCTATTTGTCCCCATCCAGGGCCTCCAGCAGGCTGGCAG
GCCATTTCCGACCGGCATGGCATGGTACAAGTAGGCGCCTCCTTTCTGGCGTACCCCCGACTCTTCAAAGGGTCTTTGCTGCCTG
CTGGTAGGGGCTTGTGAGATCTCGCTTCCTTGGTTGGAAACTGAACCCCAGGCTTTGGAGATAGCTGGATGGGAGCCAGTCTGTG
GGGAGGAAGACCCCCCTCCCCCAATGCAGAGGGCAAAGCCTTGGGTGGGGCCTGCTTGAGTTTGGCTGAATAACGCTCAGAAAAGT
GCGACCATTTGCTTACTGTTACACAGTAAGTCAGCACCATGCTGAACTGGGTGTACTTGATGCCAGAATGTTCTCTCATTATACAC
ACACACACACACACACACACACACACACACACACACACCAATGAACCATGGCCTCTCTTGTTTCACAGTGCTGGGCGAGGGGGTCAGA
GTTCCCACCTGTTTAGCAAACATTGAGATACGAGCTGCGGTAAACGTCTCTTGGGAATGTGACTGGTATGTAAAGCAGAAGATTGG
GGGTACAGGATCGGGGTAGTAGATTATAAAGATCTGGGGTCCCTTCTGAGGACCCAGGAGCTGCCCCAGGAGAGTGAATAGAAATG
AGCATGCACCAAGTTTATGGACTGGTTTTGCAGGCAGGGGCCTCAGCAAAGGCCCGGAGGCAGGGAGTTGCAAATAGAGAAGCCCT
GCATAGCTAGGAGGAAGGCTGGGATTGGAGGGAATCCTGCAGATGACGGGTACTCCAGAGCCTAGAGAGACCAGCTCTCTGCTTAC
AGAGCTGTGGCCGAGGCTTGTGCCCTGGGGAAGGTTAGGCAAGGCACAGGTGACTTGGGCCCTTGGGGCCTGAAAGGAGCTAGAGG
GTGTGTTTTGCGAGCCTGGACAGGTACCCTTTTCTGAGGGTGCCATGATTGTGCATGATTCACACTGATGCGGCAGGAGGGGGCGG
GAATTGAAGGGGTGAGAATCTGACGGGAACTTGGGGTCACACACAGTCAGACCTCTTCTGCCCTCCTATGTCTGGGTCCAAGTCTC
ATTCCCGAGTCAGCCAACTCCTCCCCCGGCTTTTTGAGGGTGAGCTGTTGGGTGGGTGTGCCGGGGAGGCCATTTTGGGCAGGCTT
GCGTGGCAGGGAGGCGGGGCTGGGCTTCGGGGAAACACGTGTTGAACTGCTAGCCAGGAGTAGGGAGAGGGAAGGCTCAATCTGGT
CTGGGGTTGTGAATCTTCCATGCAGATTTGCTGCTTCCGGCTTCCTGGCCTCAGTTTCCCCATCTGAAAGCACAGAGGTACATTGA
GCCAGGATCTTCCTGGGAGGTGGAGATCCTATGAGAGTAGGATGGGGGGTAGTGGGTAGGGTCTTTTCCTCCTTGCTTCTCATGAC
CCTCCCAAGCCCCTGCTGGCTCAAGGAGACCCCGTGCTATGTCTCCCCACACTGCCCACCCTGGCTTACTTGTTGGGTTCTAATGC
TTTCAAAGAATTTTGTCCCCATCATTTCTAGGGCGGCTGGCTAAGAATGAGTGACAGGTGAGGCTGGGCTTTGTAGGGTATCCTTT
TGACGGCTTCCCTCTTGGGTCGAGGAATGGCTTAGTTTGAGCACTGTACGACTTTGGGCGGTGAAGTCCTGGAGGGTTCTAAACT
AAGCCGCCTTCTGGGTTGTGAGGATTGCTTATTTCGAACAACAACGCTAGGGAGTCTGGCAAACAGCTAAGCCCAGGAAGGGGGTT
GCGGGTTAGAGGACCCTCTGAGTCACCGCAGAGACGCCTAGGAAGCTGGAAGACAGCCACGCCCCCTCCGTGGGCTCCTAGGAGCC
AGTGACTCAGGTTTTCTGTGCTGCTGCTGCTGAAAAAAAAAAAAATCCCACAGAAAACCTGTTACCATAGCAACGCAGCTCCAATA

TGATTCCAAGGCCAAGTGGGGCCTTTCCCCCTGGCTGAGCCTTAGAGGGCCCATTCTGGAAGGGGGCTAACTTCCCTTTCCGGCTG
AGGTGGGAGGGCCCACCATCTCCTTGGCAACAGGCCCTCACGTCCTTTCACGGTCCCAACAGAATTGAGAGGCGATTCTTTTTGGC
AAGGGAGGGAAACTGAGGCAGAGAGCGGAGAAGACATTCCCCGAAGGGTGCCTGGAGTCCACCTTTGGTGCCTTGGTGGCCTCTTT
ATTAAAAAGTGGTTGCACAGGCCCCTTTGTTCCAGTACCTGGAGATGAGACCCACATGGTGAGGCCCTTTTTGTTCTCTGTCTGGA
CGTCTCATTTTTCAGAGGTACTGGGAGGTTGAAGGTTGGTGTATGTCCACAGCACTGTGCTGTCTTGAACTCAGTGCGCATGACAT
CTTGGGCCAGGGCCTCTCCTTTGTGTGAGGGAAGCTAGCATGCTGGGAAACTCCGACTCCTTTAGTCCACCCACTTTAGTTGTAGT
TTTTGTCTTGTAGCCCATCTGGCTTCCAACTCTCCATCCTCCTGCCTCAGTCTCCAGGGTGCGGCGATGACAGGCATAGGTCACCA
CAGGCTTCTGGCCTCCACTTGATGGTTGCCGGAGTGGCTGGGAGTGGCTTTTCTTTTCTTTTCTTTTCTTTTCTTTACTTTCAGGC
AGGGTTTATCTGTGTAGTCCGGCTGTTCTGGAACTATGTNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNAGAGAGAGA
TTGGGACTGACTGGTCCCAGTTCTGGCTTTCCCTGACTGTTCCATTCCCCGCAGTGGGCACGGTTAGATCAGGTGGGGTCACTTAG
GCTCGCTAGCATTGGGTGTGGCTCTTTTCTGCCCAGTATGCTGTGCTAACTCAGCTGTCCCTGCACGTGGCAGGTCAATTACAGGT
AGGCTGAGGGGACAGTGGGTGACACCCTGTATATGTGTAGCCAATTGTTCTCTTAATATTTAGCCATCCCTAGTGTGGGCCAGGGG
CAGGAGAGCCATTTCTGCCTGAGAAGGTTGAACAGACTACAGCCCAGAGCTGTGGCCTTGGGGTGGCTGTCTAGCCTGGGATGGCC
CTCCTACTCTCCTCTGTGCTTCAGTGGTAGATCCTGTGGAGGCACTGGGCCAGGGTCCTTGGGGACCCATCATGGAAGCCATGTCT
GCTGATCCATATGTCTAGAAGGGGAGGCATCTGTCCATCCCCACTCCTGAAGTCAAGGGACCCCTTGGTCTTGCAGGGTGACCTTG
GGCCAAGGCCAGCCACCTCTGAGCTCCATCTTCTGCTTTGTGAGGTAGAGGTAAGAGCCTTTGGCATTGACAACACAGATAGAGAC
TCGGTTGGGTCAGGGTGGCATTGGCTGGCATACTCTTGGGTGTAGTGTCAACTAAGGATGAGTTCCTAGGGGCAAGGCTGAAGGTG
GCATCTACCATCCCATACCACGTTCTGCTGCCTAAGGACGCCTGAAGACAACTGTGAGACTGGCAGTGTACACTGCTTCCAAGCTG
AGGACATTGTTAGAATCCCAGATCCTTGAAACAATGAGTTTATCTTGTTAGGACCCAAGATCCCACTCCTAGGTGGAGTCAAAGCC
TTGGGAGCGGCACCTTATTGGGGCTCAGTGAGGTCATGTGACATGGAAACCATGTAGTGTTACTATGTAGCAAGAAGTTAGAAAGC
CATGCAACAAACCTGTAATTCCAACTCCTGAGGCAGAGGCAGGTGGATCTCTGATTTCAAAGGTAGCTCCAGATACACTGTGAGAC
CTTCTCAAAACAAGACAAGCAGACTGGAGAGATAGCTCAGAGGTTAAGAGCACTGGCTACTCTTCTAGAGGTCCTGAGTTCAATTC
CCAGCAACCAGTTGGTGGTCACAACCATCTGTAATGGGATTCGATACCCTCTTCTGGGGTGTCTGAGGAGAATGACAGTGTAGTCA
CATACGTAAAATAAATAAATCTTTAAAAAAAGAAAAGAAAAATGAAAAGGGAGGGGGGTGATGGAGACATGGCTCAGACAGTTTTT
TGTTTGTTTTGGCTTTTTGGTGTCTTTTCTTCTTTCTTTCTTTCTTTCTTTCTTTCTCTCTCTCTCTCTCTCTCTCTCTCTCTCTC
TCTCTCTCTCTCTCTCTTTCTCTATCTTTTCTCTTCTTGTCTTGTCTTGTATCTGTGTAGCAGCCCTGGCTACCCTGGAACTCACC
TATAGGTCAGGCTAGCCTTGAACTCAGAGATCAGCCTGCCTTTGCCTTCCAATGCTGGGTTAAAGGTGTGCTCCAGCACTGTCCAC
TCTGGACCACTTGTTCTTGTATTCACCTAGTTCTCACAGGGTGGCTCATGACAGTTCCAGGCGACCTGACCTCTTAGGGCACCAGG
CACATGTGCGCATATTCATACACACAGGCAAGACGTTCACACACGATACATACAATACAAGACGTATCTTTCTAAAAATTAAAGAA
AATAAATGAGAAGCGAGAGGGAAAGGAGGGAGAGCCACTGTTTGGACTGGACCACCACTGGCAGGGAAATGCCAGAAGGTTCAGAC
AGCATTCAGGGTTTGTGGCTGGTGGGGGCAGGGATAAAGGAGGAGTTTTCTAAAGGAACATGTGGTCTGTGCGTGTGTGTGTGTGT
GTGTGTGTGTGTGTGTGTGTGTGTGTTACTAGGGGTAGACTCCAGGGCCTTCCACCATGCTGGGCAAACAATTTACACTGAGCT
ATATTTTCGACCCCCTCTTTATTTTATTTTTATTTTATTTTATATATAGCTCTGACTGGCCTAGAACTCATTATGTAAACTAAGGT
GACCTCAAACTCACCCATACTCAAATGAGATTCACCTGCCTCTGCCTCCTGAGTGCTAGAGACAGTCATGAAGCACCACGCCCACC
TTCTCTTTAAAAAAAAAAAAAAAGCCTATTGCTTTTGTTTTTATTTTTGTCTGTGGAGGGTGGTGCCCACACCCAGTGGTGCCAGTT
TGGAATCAGTTCTCCTGCCCTATTCTACCTAGGGATCGAACGCAGGTGGGTAGGTTTGGCAGCAGCTGCTTTTACCCCACGAGCCA
TCTCGCTGGCCTGTAGCCCTTTGTGTGTCTGTCTGTCTGTCTGTCTGTCTGCATGGAGTGGAGTCTCATGTAGCTCAGGCTGGCCT
GCGAGTTATGATCTTCCTGACCCTGGTATCCAAGTGCGGAGACTGCAGGCTCACTCTGCCACGCCCTTTTACACAGTTCTGGGATG
GACTCTGCATGTCAAGTGAGTTATAGCCACGGCCCTATTTTATTTTATTATTTTTGAGATCGGGCCCCACTCATCATGGAGGCTAA
GGTCGGCCTCCTGAGTGGTGGGATGACAGGCGTGGGTCGATGTGCCTGGCTCCTACTCTATTCTTTTACTTTCACACATACCCACT
ACAGGAAGCGAGTAGCGAGCCCCCTTTTACCAGCACCCCGCCATGTGACATTCCGCAGAACCAGCGCCATCTGTGTGTTTATTCAA
TGGTTTTGGTTGTATTTAAAGCAAATTCCCCACACTGGTGCTTCTTACCCGGAAACGCCTCCATCTGTAGCTACAAAACCAGGCCA
TTCCTTACATAACGGCAGCGTTGCCTAACTGGAACCGATCTTTAAGACCCAATCCAGGCCCACGGGAGAGTTCTGCTAGGCCTTAG
AACATCTTTGTGGGTGGTTCGTTTGAAGTTTGATGGCTTCCTTCCTCCCCTGCTCCCCTTTCTCCTTGCCATTTTGGGCACTAGAG
TTTAAAGAAACCCGCCTGCCACTGAACTAAATCCCCAGATTTTTTTTTTTTTTAAGTTTTTTTTCGAGACAGGGTTTCTCTGTGTAG
CCCTGGCTGTCCTGGAACTCACTCTGTAGACCAGGCTGGCCTCGAACTCAGAAATCCACCTGCCTCTGCCTCCCGAGTGCTGGGAT
TAATGTCTTATGTATCCCAGACTGGTCTTGAACTTGTGGTCATGCCTGGAGTATCCTATTTTGATTGTTTATGTGTATACCCCTAA
TATGGATGTCAGAGATTCATATCATATGTGCCGTCTTTGGTCATCCTCCATACTTTTGGGGGATGTATGGGGGCAGGGATGGAGTC
TAGCCTGGGCTGTGGTAACACACGCTTTTCATTCCAGCATTAAAGCAGAGATAGGTCGATCTCTGAGTTCTAGGACAGCCTGGGCT
ACACAGGGAAATTCTGTCTCAAAAAAAAAAAAAAAACAAAAAGAAAGAAAGAAAGGAAAAGAAATAAGAGTCTCCCTATATAGCCCT
GGCTGTCCTAGAACTTATTATGTAGACCACGCTGGTCTTGGACTCACAACAGAGATCCATCTGCCTCTGCCTCCTGAATGTCCACT
CTCTGCTTGGAGACAAGAATCGCTCAGTGGCCCGGATTCAGGGATTCAGCTAAGCTGGCTGGTCCCTGAGCTCCAGGCCTCCATCT
GTCTTGGCTTCCCTGTGACAGGATTATCGGCTCATGCCACCGCGCCTGGCTCTTACACCATTCCTAGCGATCTGAATTTAATACTC
TATGGTCAAGCCATCCTTCAATGCTCCCTCCCCTCCTGTTGAGACAGAGTCTTACTCACTCGGTAGCCTGGGGTCCCAGAACTCAC
AGTTAGTCTCCCGTTTGTTTCCTGAGTGGTGAGATCGTTCCTGGCTAGCCTTGGCTGCTGATTTGGTAGCCTGGTCCCGCCCACTG
CTTAGGATTATCACTGAGGAGTTTAAGCCACACTTCAGGAAGCCCTGTGGATCCTGGAATCTGAGTGAGACAAGGGTCACACAGTG
GCCTGACCGGCTCTGGTCTCTCTCTGCAGGAATGGACCTGTCAGCCAATCAGGATGAAGAGACCGATCAGGAGACCTTCCAGCTGG
AGATCGACCGCGACACAAGAAAGTGTGCCTTTCGCACCCACACGGGCAAGTACTGGACACTGACGGCGACCGGAGGTGTGCAATCC
ACTGCGTCCACCAAGTGAGTGACACCCTACACCCCTTCATCACCTGGCCTGGCTCTTCCCCCAGGACTGGGCAGCCTGCTCGATGC
CCCCACGTTGCCAGCCCCTCTTCTCTTCCCCAGGAACGCCAGCTGCTACTTTGACATCGAGTGGTGTGACCGCCGGATCACTCTGA
GAGCCTCCAACGGCAAGTTTGTGACCGCCAAGAAAAATGGCCAGCTGGCCGCCTCGGTGGAGACAGCAGGTAGTCACTTGGGGGCA
CGTACCCTAAGCCTGTTTCCCTAGTACCCCGTGGTCAACCATCAGTCCCACCTGGACCTCTCTGTGTGTTCAGGGAATCCCTGTAA
GCTGGTGTACCCTCAGGCCAGCAGCCTGTGACCCTTAGCTTCTTGGTATCCCTCTCTGCTGAGCCATTCCCTGACTGGCCCATCTT
TGTTGCTGTGAAGCTCACTGGCTCCCTTTCCCTGTGGCAGGGGACTCGGAACTCTTCCTCATGAAGCTGATTAACCGCCCCATCAT
CGTGTTCCGGGGGGAACACGGGTTCATTGGCTGCCGCAAGGTCACGGGCACTCTGGATGCCAACCGTTCCAGTTACGATGTCTTCC
AGTTGGAATTCAATGACGGCGCCTACAACATCAAAGGTGGGTTCACTGGGTGAGGATGCACCTGGCCATTCAAAGCCGACATTAGG
GAACGGGTGTCCTATGACCGCCTGGGGTAGCCCCTCCCCCCTTGTCTTAGAACTTTCCTGGCCCAACCTGGGCAGACAGCGAAGGT
GGGAAGCAGCTAGGGAGAGTGGTCGTGGCTCCAATCTGGGAATCGGAGACAGGAGAATTATCTTTTTTTTTTTTTTGGTGTTGAGG
ACAGAACCCAGGGCTTTGAGCTTATTAGGCAAGCATTCTACTGCTGAGTTAAATCCCCAACCCCAAGAATCATCTCAAAAAGAAAG

AAAGAAAAGGGAAAAAGAAGGTGGAGCCTGGTGGTGGTGGTGGCGGCGGCGGCGGCACACGCCTTTAGTCCCAGCACTCAAGAGGC
AGAGGCAGGTGGGTCTCTGAGTTCTTGCCAGCCTGCTCTACAGAATACATTCTAAGACAGCCAGAGCTATGCAGAGAAACCCTGTC
TCAAAAACAAACAAAAAAAGGGGGTGGGGAGAGAAGATAGAAGATAAGGATATTCCTTCTTAGCTAGCTGTGGGACCAAAACCAGT
GAGAGGACACAGCAATCCAGAGGTGTCAGTCAGAATCAGAGCCCTGTGCTGTGTGTGGCCTTAGCAAGGCAAACGGGACTCCTTTC
TTACAGTGCTGAGGACTGGACTCAGAGCTAAGATCCAGCCCCTCCCTGGGGGATTCTAGGCAGGGGCTCTACCACTGAGCCACGCC
CCCAGCCCCTCACTGGGGGATTCTAGGCAGGTGTTCTACCACTGAGCCACGCCCCCAGCCCCTCACTGGGGGATTCTAGGCAGGGG
CTCTACCACTGAGCCACACCCCCAGCCCCTCACTGGGGGATTCTAGGCAGGGGCTCTGCTGCTGATATATGTCCTTTTTGTTTGTT
TACTTGTTTGTTTGTTTGTCTTATTGTGGCCCTAGCTGTCCTAGAACTTGCTTTGTAGACCAGAGATATACCTGTCTCTGCCTCCC
AAATGTTGGATTTAAAGGCATGTGCCACCATGCCCCACCCTAGCCTAGCCCTCTTATGTTTTGTAGTTTGAGATGGCATGTCAGTA
GATTTCCCAGGATGGCCTTGAATCTAGGTAATCCTCCTGCCTCAGTTTCTTGAGTATTTGAATTATGGGACTGTACCTTACATCCA
GCTGAGCCCACTCAGTTCTGGAAGCTGACAGCGGGAGGGAGTGTTAATCCTTTGGGGACTGACAAGGTGGGGTTAACATGTAGTCT
CCCTCCTCAGACTCCACGGGCAAGTACTGGACGGTGGGTAGTGATTCCTCGGTCACCAGCAGCAGCGACACCCCTGTGGATTTCTT
CCTTGAGTTCTGTGACTACAATAAGGTGGCTCTCAAGGTGGGCGGCCGCTACCTGAAGGGGGACCACGCTGGGGTCCTGAAGGCCT
GCGCGGAGACTATCGACCCCGCCTCACTCTGGGAGTACTAGGGCCACCTGCCCTCTGCAGGCCGCTCTCGTCAGTCCCTCCTGTTA
TCCTTACTCATCGGGTGGCCCTGCAGCAGGTGGCAAACCCCTTGCCTTTCAAACTGGAAACCCAAGAGAAAACGGTGCCCTTGCTG
TCACCCTCTGTGGACCCCTTTTCCCTAACTCACTGCTCCCCATGGGTCGGTGGCTGCAGACTGTCCCCAGGAGGGACTCTGGTTCC
CTCTGTCCCCTTCTTTCCATGGGGAACTCTGGCACCTTTCTTCTGACCTCAGTCAACTCTGAGCCTTATTTCCCCCCAGGAAGTGG
CCTAGGAGAAGCTACAGGGCCTAGGGACTTACCCTGAGCTTGTAACTGGAAGACCCCGTCCCTATCCCCGCTCCCGCCCCCACCCC
ACCCCACCCCTGCTCTGGCCCCAGCCTCTGGAGGCCAGCCTTTTGGCGGGACTGAAGCCGGGCATGGCCAACCTTGCCCACAAGTG
TTTTTCTGGATCTTGGCTGGAAGGCAGTCTGTCCCATCCTGCAGTGTTTGGGCCTGGCTCTTTGACTCAAAGCTAGCTAGGTGGCA
CTCCGTGTCGCTCCTGCACATTCTGGAAGGGGCGGGCCTCTCACCCACCTCATTCCTTTTCCCCCTGGCCTGACTGGAAGCAGAAA
AATGACCAAATCAGTATTTTTTTTTTTTTCTTTAAGGAAATGTTACTGTTGAAAGGCCCTAGGCAAGCCTGCCCTGTTGGTTGTA
GTCGTGAGTGGTCTTGGGGGGAGATGCTTGGCTCCTGTCCCTGCCTCCCCAGCGGGTTCCCTCCCTCCCTCCTGCCTGACCACCCC
AGCTCTGGCTCTGTGATTGGTGCTCCACGTCTTCCCAGACACCTCGGGGCTCCTGGGCGGGAGAAAGCCGGATGTGCCCCTCCCTG
GGAGCCCTGGAGTAAACCTCAGGGGGCCCTTTCCCAATCACCCCCTTCCACCGACCCCTCAACACCATGCATCTCACTCTGGGTGT
CTCGCTCCTTTATTTTTTTGTAACTGTCATTTCTATAACTCTGAAGACCCATGATAGTAAGCTTTGAACTGGAAAATAAAGTAAAA
TCAAGTCTGCGGCCCGTGTGTGTCTCAGGGGAAGTGGATGCTGGAGTGGGCAGAGGGCCGGCTGGGAGGGAGGCAGCGCTGATTGA
TTGGCAGCCCGCAGACTCTTGTTTCGGTCTCGGACCCTAGCGGCTTCAGCATATGCCTTAGTAACTCTGGACTGAGAAATGGCTGA
CACGAGGTTCCCGAGCCTTACAAGCTGGGCAGGCTAGAGGGTTCAGCAGGTAAAGTTGTGTGCTGTCAAGCTTGATCAGACTGAGT
CCCCAGCACCGTGTGAAAAGTTGGGCACAACGGTGTACACCCACCCCAGGGCCTCTGGCAAGCACAGAGACAAGAGGATCCTTGGA
GCCTGCTGGCGGCCCAACCTTGCCCAATCTGTGATTCCCATTGAGTGAGCCATAAGGTGGAGATGGGTTTTGGAAGATACCCCACA
TAGGCCTGCGGCCCCCCACACCCGGAAATAAAAACAAGCCAGGCTTGTCATCCCAGTTATTCGGGGAGCTGAGGCAGGAGGATCAC
AGATTTAAGACTGCCCAGGTTGAAGAGTAAGTTCAAGGGCAGCCTGGACATCTTGCTAGATTGTTTTTTTGTTGGTTGGTTGGTTT
TTGTTTTGTTCTTTTTTTCGAGACAGGGTTTCTCTGTGTAGCCCTGGCTGTCCTGGAACTCACTCTGTTGACCAGGCTGGCCTCGA
ACTCAGAAATCCGCCTGCCTTTGCCTCCCGAGTGCTAGGATTAAAGGTGTGCGCCACCATGCCAGGCTTAGATGTTTTAAATGAAG
GGCTGTGGAGTGTGGTTCACGGGTAGCCTCGCTCAGACAGGCTGGGGCAGGAGAAATGGATGGCTCAGCATTGGCTGCTGCCTTAG
GGTTTCCATTGCTGTGAACAGACACCATGACCTGGCAGCTCTTCTGAAGGACATTGGACTGGGGCTGGCTTACAGGTTCATCAAGG
CAGGAAGCAAAGCACAGGCATGGTGCAGGAGGGGCTCAAGTCTACGACCCGGGTCTCAAAGCCCACCCCCACAGTGACAGACTTTC
TCCAACAAGGCCACACCCACTCCATCAAGGCCACACCTCCTAGTAGTGCCACTCCTTGAGCCAAGCTTATTCAAACCACCAATCAC
AACTGCTTTTCTAGAGGACCCAGGTTCAATTCCCAGCATCCACATGACAGCTTACAATTGTAACTACAATTCCAGGGGATCCAACA
CCCTTACACAGGCACACTTACAGGAAAAGCACCAATCAGTGCACATAAAAATACATAAATCGTTAGAGAGAAGAGACCATTAGCTA
CCTTCACCCTTAACAGGCAAGCTGAGGTCCTGGGAGTGTGTTAGAGGTCCGCAGCTTGGAGTCTATAGGGTAGTGGCATCGTCTAT
ACGTTCCTTCCTTGACAGGTAGGTACTTAGCTGAGACCTCTGGGGTATCCCAGAGACCCATTTTACAGAGGGAGGCACTGAGGCGG
AGGGAAAAGACAACCTTGTTTGCTGGCTCAGAAGAGTGCCAGTGGGGTACCCACCCACAAGGCTCAGCATCCCGAGGCACTGTGGA
GGCAGTTACAAAGCCGCATTGATTGGCTGCTGATGACGAAATGAAGAGCCCCGCGGTGTGATTGGCGGTCCCAGAGGGAAGGCATG
TGGGGAGATTGACAGGGGCAGATGGCCACAAAGGGTCCAGTGACTTCCCGCAGGGTGGAAGCTCTGGGCCCAAGCCAGTAAAGTGT
CAATGACATGGAGAGACAGATGAATCCTTCCCAGTTGAATTCTCAGGCTCTGGTGGGGCCGCGCTGGGGAGAAAAGACACCCTGTC
CGTTACAACCACCAAGCGCAGCCAGGCTACGTGATGACTGGGGGAAGTCCCAGACGTGACTTCCTGCAGCACTGGAGGGGAGGGTC
CGACGGAGGAGGCTGAGAGTCTGTTGGAGGCAGCCGAGGATCGGAGGGAAGAGGCACAGGTCTTACATGGGAGGCATAAAGTCTGG
TGGGAGTCCAAGGGAGGACGGATGTAGGGTGATGGGAAGAAAGGACCTAATCCAGAAAAAGGAACAGAAGAGCCACCTACCTGGTT
AGGGGACGCTGGCCCTCCGCAGGGAAAGCTGTCAAAGAAAGGCCTGGCTAATGAGACCATGTCGTGGGGGGCCCAGGCAACTGTGG
AGGCATCAGACACCTTGGGGCAAGGAGGGCACCCATGAGCTAGTACACACCACTAAGTATCTCAGAGTCCTGCACACACACTCATT
GACACAGGTGGGTGAGCAGCACCCACGGGCAGCTGAAGCTGAGTATCCTGCCCGCCCCTTTGTTTCTTCCAACTGAGCAGGGACAA
GAAGGATCCCTATGAATATGGCTTTTTTTGTTTGTTTGTTTGGTTGGTTGGGTTTTTGACTTGGTTTGGTTTTTCCAGACAGGGAG
TAAAGACATACACCACCATAACCCAGCTTTGTAAATCTGGTTTTTGTGTGTGAACCTATGAATTCCACTCCTGTTGCACTGATGGG
AAATTGAGGCAAAGTCTCAAAGAAGCTGACAGCACAGGCTAGTGTGTTCATTTTCTCTCTACAAAAAGAGGAAAAATTGGGGCTGG
TGAGATGGCTCAGCGGTTAAGAGCATTGACTGCTCTTCCAAAGCAGGTCCCGAGTTCAAATCCCAGCAACCACATGGTGGCTCACA
ACCATCTATAATGAGATCTGAGACCCTCTTCTGGGGTATCTGAAGACAGCTACAGTGTACTTAGATATAATAAATAAATAAATCTT
TAAAAAAAGGAAAAATTATTTATTATTTTATTATTAAATTAATTGTTGTATTTATTTATTTTATTAATCTATATTTAATTTATTTT
TTTATTTTGTGTGCATGAGTGTTTTGCCTGCATGTGTGTGCACTACCTGTGTGTCCACTGCCTATGGAGGTCAGAAGAAGGCGTTG
GGTCCGGGAACTGGGGTTGTATGGTTGTTAGCCGCCGCGTTCATGCTGGTTCTCTGCGAGGCGGCACTTAGTCCTGAGCCATCTTC
TGTCAGCTCCTCTACTTTCTTTTAGACAGGGTCTCCTGTAGCTCAGGCTGGGCTTCAGCTTGCCTGGTGGTTAAGGGTGACTAAGT
TAAATCATCTTACATTTCTGCCTCACTTCCACCTCCTGAGTGCTGGGCTTGCAGGCATGTGCCACTGGGATGGGTTTTGTTTCCTG
CTGGCTTCTCACCCCGTAGCTCCGTGTGTTGGGTCAGGGCCAGCCCTGTGCCCACTGAGCCATTAACCCTACTGAGCCCACTGTAG
GGAAAAACCATAGAGGAGCTTCTATGACCCTCATGGGTCCCTCCAGGTCTGTCAGAGTTCAGGAGGCATGGATCTCTCTGGCAGGG
AGCTGTTGACTTTAGCCTTGGAATTTCCCAACCAAGTCTACACTGAGCACCCTGTCGACGACCTGCTTCTGACTCCTCCTGTGAGG
CTCTTTTGGAGTGATCTTAGTCCCCACAGGGCCCCCATCTTCTCCAGAGCTGAGTGGCCTCTCCTACTGGACAGAGAAGAGCTGTC
CTGACTTGGTTAAAATGTTGAATGCAAGGAGCGGAGGAAACGGCCCAGCTGGTGAAGTGTTTGGCTCGATCCCCAGAGCTGTGGAG
TTCCCAACAGGATGGGGCTCACTGGCTGCCCTGATCAGCAAGCTCAGAAACCAAGATGGATAGCTCCTAAGGACTGGAATTGGAGG
ATGACCCCTGGTGACAGACACACTTGCATATGCATCTGTCTGCACACATACACATATGCATGTAAAACCCTGATCCACGTGGTAGT
GCCTGACTGTAATCCCAGTACTTGGGAGACAGGAACAGAGAATCACCTTCAGTTTGAGGCCAACCTCGTCTACATATCCAGCACCT
TAGGAGACTCTGTTTCCAACTCTTCAGGGTGGCTCACATCTCTAATCTCAACCCTCAAGAAGCCAAGACTGGAAGATGCCTAGAGA
GGAAGGTTTGCTTGGGTTCAGTGCCAGCCTCCAGAGGCAGAGGCAAGCAGATTTCTGAGTTCGAGGCCAGCCTCTACAGAGTGAGT
TCCAGGACAGCCAGGCAGCCAGGGCTATACAGAGCAACCCTGTCTCAAACAACAACAATAACAACAACAACACAACAAGAAAACTT
GAGTGTGCGTGTGCGTGTGCGTGTGCGTGTGTGTGTGTGTGTGTGTGTGTGTGTTTACTATTTACAGTAAACGAGTCTTTGGGG
AGGCAGCCAAAGTAGGCGAGGAACTCCTTGTCCCCTGCTGGGCTCTCTGGGTTGAGATCTTGTCTTGATGGGGAGATAGGAAGGGA

TTCTCTGTCCCTCAGGGTGCTATCAGCAGTGGGAACAGATGGGGTCGGGAGAGAGACTGGGGGAGGGGAGAATCAGGGTGGAGGAG
GGCTGTGATTTAGGTTTACCATGAAACCGGAGTCATCACCGGTGGGAGAGGCTGGTCAGAGTTCCTCTTTTTTCCTCTTCTTTCCC
TAGGACCCAGGCCTGATCCGGTGGGGAGGGGAATGATTGGAAAATGCTGCCTTCTAGAGTCCCTAGCGAGAGCCACACCCCAGGCC
TCAGCCATCAATGACAACCTTTCTGTTCTCACTGAAAGCAATTAAAGATCTGTAAGATGAGTCAGTGTTACCCATTGTAAGCTGCT
GACTGCCTTGGTCCCATCCCTGGGACCCACATGGTGGAAGGAAAGAATGGACTGATGCCCTTGGACCCCCACGTGTGTGCTGTGGC
ACACACACACACGCATGCACACAAAAATAAGTAATGTAGTTCAAAATTTAGAATAAATCAGGCGTGGTGGTGCACACCTTTAATCC
CAGTACTCGGGAGGCAGAAGCAAGTAGATCTCTGTGAGTTTGAGACCAGCCTGGTCTACAGAGTGAGTTTCAAGCCAGCCATAGAT
AGCTACATAGCCAGATCTTGTGTTAGCCTCCTCCTTCTACCAACAGAAATTAAGATCACAAATAAACAGGAAAGAGGGTGGAAGAG
ACAACTCAGTGGCTATGAACATTTGCTGTTCTTGCAGAGAAACCAGGATTGAGTCCCGGCACCCACATAGTGGGTAAACATTATCT
CTAGCTCAAATTCCAGAGGACCTGGCTTCTGAGAGCACGGTACACATGGGGTGTTCTCTCTCTCTCTCTCTCTCACACACACAC
ACACACACACACACACACACACACACACGCAGGAAAAACAGAAAAGCACATAAAATAAAAACAAATATATAAAAATAGAATCCA
AGGGGATTCAACGATGTCATGAGTCCACAGAAATCCTATCCTCCCCTTTTTCGGGGTGTTACACGGGCGGATCAAACCCAGGGNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNTGACTGCTCTTCCAAGGT
CTTGAGTTCAATCCCAGCAACCACATGGTGGCTCACAACCATCCGTAATGAGATCTGACGCCCTCTTCTGGTGTGTCCGAAGACAG
CTACAGTGTACTTACATATAATAAATAAATAAATCTTAAAAAAAAAAGAAAGAAAGAAAGAAAGAAAGAGAGAGAGAAAGAAAGAA
AGAGGTGAGGGCTGGGTGTGGTGGCGCACACCTTTAATCCCAGCACTTGGGAGGCAGAGGCAGGTGGATCTCTGTGAGTCTGAGGC
CAGCTTGGTCTACAGAGTGAGTTCCAGGACAGCCAGGGCTACACAGAGAAACCCTGTCTTGAAAGAAAGAAAGAAAGAAAGAAAGA
AAGAAAGAAAGAAAGAAAGAAAGAAAGAAAGAAAGAAAGAAAGGAAGGAAGGAAGGAAGGAAGAGGTGAATAGGATAAAATG
AGGTCCAATAGGTTGGGGGCATGGCTCAGTGTTAGAGGGCTTGCCTAGCAAGTGAGAGGCCTGGGTTCAACCTCTGGCACTGAAGA
GGGATGGTAGGTAGGTAGGTAGGTAGGTAGGTAGGTATCTAGATAGATAGATGATAAATAGGTAGATGGATGGTAGATATATAGAT
AATAGATGTGGGATAGATAGATGATAGAGATATACATAGATGATAGATTATAGATAGATAGATAGATAGATAGATAGATAGATAGA
TAGATAGATAGACAGACAAATGGTAGGTATATAGATGATAGAAAGATGAGGGATAAATAGATGGTAGATAAGTAGATGATAGTAGG
TAGATGAGAGGGAGAGAGAGAGAGAGCGAGAGAGAGCGAGAAAGGATATAATAAGGTCATTAGGCTAATTTGATCCAAAGACAGAT
TGGGGTTCTTATAAGACAAGTGATCAAGCAGGCCAGATATGGGGGGAACTGATCTGTAATTCTAGCACCTGGGGTGGGAGATGGAG
GCAGGAGGAAGGGGAGGTCGAGGGTATCCGCAGATACTTAGTGAGTTTGAGGCTATCCCCAGCCACGTGAGACTCTGGCAAATCCT
TCCGCACTAACAACAAAAATTCTGAAAGGCAAACCTCCCTTAACTCATAAAGCATGAATTGGGGCGTATGCGGGCCTTCAGAGCCA
TGTGGTGTGTTTGGGAACAGACGACAGGAAACACTGTTGAGAGGACTCAGACCCAGAGAGGAAGGGACAGCAGAGTCACCATGTGG
ACAGGCGTTGGGGCTTGCTGGCCGCTCACAGGAAGCAGCGCACACCAGAGCCATCCGCCTAGGAGCTGGGCCACAGTTTCCTGCA
GAGTGGGCCCTGACACACCCGGCCAAAGCAAGCACTGGCCTGTGGACAGAAAGCAGACACAGGACGGAGCCCGTTCCGGGGTCAGC
TCACTGTACTTGAAGATGTGAGCCCAAAGAAGTGGGGGGTGCCACGGCGTCTTTTGCAGATGTTACTATGCCAGTTGGTGTTAGAC
ACTTAGATATATTTTTAAGAAGAGCAGTCGGTACTCTTAACTACTGAACCATCTCTCCAGCCCCACAATGACATTCTTCAAAGAGC
AGAGCTTAGAGGCACAGGCACTTGTCACCAAGCAGCAACATGAGTCAGATCCCTGAAAATCCACAGGCAGAAGGAGAGAGCTGACT
CCTGTCCGTTGACCTTCATGTGAATACAGTGGTGTGTGGTGGTGTGTGTGTGTGTGTGTGTGTGTGTGTGTGTGTGTGCATTTA
TGCCATGTGTGTCAAGAGTGTGAGGAGGACCCAAGAGAGTTCCTAGACTTAAAGCTGGTTNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
N

MOUSE SEQUENCE - mRNA (SEQ ID NO: 2)
GAGCTAAGCCGTGTTGAACAAAGGAGGTCGGGCACAGCTATCCAAGCTCCCGGGGCCACCGGGCCGCCCTCCGCCACCATGACCGC
CAACGGCACGGCAGAGGCTGTGCAGATTCAGTTCGGGCTCATCAGCTGCGGCAACAAGTACCTGACAGCCGAGGCGTTCGGGTTCA
AGGTGAACGCATCCGCTAGTAGCTTGAAAAAGAAGCAGATCTGGACGCTGGAGCAACCTCCCGATGAGGCGGGCAGCGCGGCCGTG
TGTCTGCGCACGCACCTGGGTCGCTACCTGGCCGCCGACAAGGACGGCAACGTGACCTGCGAGCGCGAGGTGCCCGACGGCGACTG
CCGCTTTCTCGTCGTGGCGCACGACGACGGCCGCTGGTCGCTGCAGTCCGAGGCTCACCGGCGCTACTTTGGCGGCACCGAGGACC
GCCTGTCCTGCTTCGCGCAGAGCGTGTCGCCGGCCGAGAAGTGGAGCGTGCACATCGCCATGCACCCGCAGGTTAACATCTACAGC
GTTACCCGCAAGCGCTACGCGCATCTGAGCGCGCGGCCGGCCGACGAGATCGCGGTAGACCGCGACGTGCCTTGGGCGTCGACTC

GCTCATCACCTTGGCCTTCCAGGACCAACGCTACAGTGTGCAGACGTCCGACCACCGCTTCCTGCGCCACGACGGGCGGCTTGTGG
CACGGCCGGAGCCCGCCACGGGCTTCACGCTGGAGTTCCGCTCCGGCAAGGTGGCCTTTCGCGACTGCGAAGGTCGCTACCTGGCT
CCGTCCGGGCCCAGCGGCACCCTCAAGGCTGGCAAGGCCACCAAGGTGGGCAAAGATGAGCTCTTCGCCCTGGAACAGAGCTGCGC
TGAGGTGGTGCTGCAGGCGGCCAACGAGGGGAACGTGTCCACGCGCCAGGGAATGGACCTGTCAGCCAATCAGGATGAAGAGACCG
ATCAGGAGACCTTCCAGCTGGAGATCGACCGCGACACAAGAAAGTGTGCCTTTCGCACCCACACGGGCAAGTACTGGACACTGACG
GCGACCGGAGGTGTGCAATCCACTGCGTCCACCAAGAACGCCAGCTGCTACTTTGACATCGAGTGGTGTGACCGCCGGATCACTCT
GAGAGCCTCCAACGGCAAGTTTGTGACCGCCAAGAAAAATGGCCACGTGGCCGCCTCGGTGGAGACAGCAGGGGACTCGGAACTCT
TCCTCATGAAGCTGATTAACCGCCCCATCATTGCGTTCCGGGGGGAACACGGGTTCATTGCGTGCCGCAAGGTCACGGGCACTCTG
GATGCCAACCGTTCCAGTTACGATGTCTTCCAGTTGGAATTCAATGACGGCGCCTACAACATCAAAGACTCCACGGGCAAGTACTG
GACGGTGGGTAGTGATTCCTCGGTCACCAGCAGCAGCGACACCCCTGTGGATTTCTTCCTTGAGTTCTGTGACTACAATAAGGTGG
CTCTCAAGGTGGGCGGCCGCTACCTGAAGGGGGACCACGCTGGGGTCCTGAAGGCCTGCGCGGAGACTATCGACCCCGCCTCACTC
TGGGAGTACTAGGGCCACCTGCCTCTGCAGCCGCTCTCGTCAGTCCTCCTGTTATCCTTACTCATCGGGTGGCCTGCAGCAGGTGG
CAAACCCCTTGCCTTTCAAACTGGAAACCCAAGAGAAAACGGTGCCCTTGCTGTCACCCTCTGTGGACCCCTTTTCCCTAACTCAC
TGCTCCCCATGGGTCGGTGGCTGCAGACTGTCCCCAGGAGGACTCTGGTTCCCTCTGTCCCCTTCTTTCCATGGGGAACTCTGGCA
CCTTTCTTCTGACCTCAGTCAACTCTGAGCCTTATTTCCCCCCAGGAAGTGGCCTAGGAGAAGCTACAGGGCCTAGGGACTTACCC
TGAGCTTGTAACTGGAAGACCCCGTCCCTATCCCCGCTCCCGCCCCCACCCCACCCCACCCCTGCTCTGGCCCCAGCCTCTGGAGG
CCAGCCTTTTGGCGGGACTGAAGCCGGGCATGGCCAACCTTGCCCACAAGTGTTTTTCTGGATCTTGGCTGGAAGGCAGTCTGTCC
CATCCTGCAGTGTTTGGGCCTGGCTCTTTGACTCAAAGCTAGCTAGGTGGCACTCCGTGTCGCTCCTGCACATTCTGGAAGGGGCG
GGCCTCTCACCCACCTCATTCCTTTTCCCCCTGGCCTGACTGGAAGCAGAAAAATGACCAAATCAGTATTTTTTTTTTTTCTTTAA
GGAAATGTTACTGTTGAAAGGCCCTAGGCAAGCCTGCCCTGTTGGTTGTAGTCGTGAGTGGTCTTGGGGGGAGATGCTTGGCTCCT
GTCCCTGCCTCCCCAGCGGTTCCCTCCCTCCCTCCTGCCTGACCACCCCAGCTCTGGCTCTGTGATTGGTGCTCCACGTCTCCAGA
CACCTCGGGGCTCCTGGGCGGAGAAAGCCGATGTGCCCCTCCCTGGGAGCCCTGAGTAAACCTCAGGGGGCCCTTTCCCAATCACC
CCTCCACCGACCCCTCAACACCATGCATCTCACTCTGGGTGTACTCGCTCACATTTATTTTTTTGTAACTGTCATTTCTATAACTC
TGAAGACCCATGATAGTAAGCTTTGAACTGGAAAATAAAGTAAAATCAAGTCTG

MOUSE SEQUENCE - CODING (SEQ ID NO: 3)

ATGACCGCCAACGGCACGGCAGAGGCTGTGCAGATTCAGTTCGGGCTCATCAGCTGCGGCAACAAGTACCTGACAGCCGAGGCGTT
CGGGTTCAAGGTGAACGCATCCGCTAGTAGCTTGAAAAAGAAGCAGATCTGGACGCTGGAGCAACCTCCCGATGAGGCGGGCAGCG
CGGCCGTGTGTCTGCGCACGCACCTGGGTCGCTACCTGGCCGCCGACAAGGACGGCAACGTGACCTGCGAGCGCGAGGTGCCCGAC
GGCGACTGCCGCTTTCTCGTCGTGGCGCACGACGACGGCCGCTGGTCGCTGCAGTCCGAGGCTCACCGGCGCTACTTTGGCGGCAC
CGAGGACCGCCTGTCCTGCTTCGCGCAGAGCGTGTCGCCGGCCGAGAAGTGGAGCGTGCACATCGCCATGCACCCGCAGGTTAACA
TCTACAGCGTTACCCGCAAGCGCTACGCGCATCTGAGCGCGCGGCCGGCCGACGAGATCGCGGTAGACCGCGACGTGCCTTGGGGC
GTCGACTCGCTCATCACCTTGGCCTTCCAGGACCAACGCTACAGTGTGCAGACGTCCGACCACCGCTTCCTGCGCCACGACGGGCG
CCTTGTGGCACGGCCGGAGCCCGCCACGGGCTTCACGCTGGAGTTCCGCTCCGGCAAGGTGGCCTTTCGCGACTGCGAAGGTCGCT
ACCTGGCTCCGTCCGGGCCCAGCGGCACCCTCAAGGCTGGCAAGGCCACCAAGGTGGGCAAAGATGAGCTCTTCGCCCTGGAACAG
AGCTGCGCTGAGGTGGTGCTGCAGGCGGCCAACGAGGGGAACGTGTCCACGCGCCAGGGAATGGACCTGTCAGCCAATCAGGATGA
AGAGACCGATCAGGAGACCTTCCAGCTGGAGATCGACCGCGACACAAGAAAGTGTGCCTTTCGCACCCACACGGGCAAGTACTGGA
CACTGACGGCGACCGGAGGTGTGCAATCCACTGCGTCCACCAAGAACGCCAGCTGCTACTTTGACATCGAGTGGTGTGACCGCCGG
ATCACTCTGAGAGCCTCCAACGGCAAGTTTGTGACCGCCAAGAAAAATGGCCACGTGGCCGCCTCGGTGGAGACAGCAGGGGACTC
GGAACTCTTCCTCATGAAGCTGATTAACCGCCCCATCATTGCGTTCCGGGGGGAACACGGGTTCATTGCGTGCCGCAAGGTCACGG
GCACTCTGGATGCCAACCGTTCCAGTTACGATGTCTTCCAGTTGGAATTCAATGACGGCGCCTACAACATCAAAGACTCCACGGGC
AAGTACTGGACGGTGGGTAGTGATTCCTCGGTCACCAGCAGCAGCGACACCCCTGTGGATTTCTTCCTTGAGTTCTGTGACTACAA
TAAGGTGGCTCTCAAGGTGGGCGGCCGCTACCTGAAGGGGGACCACGCTGGGGTCCTGAAGGCCTGCGCGGAGACTATCGACCCCG
CCTCACTCTGGGAGTACTAG

HUMAN SEQUENCE - GENOMIC (SEQ ID NO: 4)

TTGCATTTGAAGCTACCATGACTGCAAGAGGGAGTTCCCGAAGGGGTCTGGGAATGGCCCAGGGAAATAGGGGTGAGGTGAGACTC
CACTGCCCCGACAGTGCCCATGTATGAATTAGAAAAAGTGGGCCAGGCGTGGTGGCTCACACCTGTAATCCCAGCATTTTGGGAGG
CTGAGTGGGCAGATCATGAGGTCAGGAGTTCGAAACCAGCCTGGCCAACATGGTGAAACCCCATCTCTACTAAAAATACAAAAATT
AGCTGGGCTTGGTGGCACACATCTGTAGTCCCAGCTACTCAGGAGGCTGAGGCAGAAGAATCGCTTGAACCTGGGAGGTGGAGGTT
GCAGTGAGCCGAGGTCATGCCACTGCACTCCAGCCTGGGTGACAGAGTGAGACTTTGTCTCAAAAAAAAAAAAAAAAAAAAAAAAGA
AAAGAAAGAAAGAAAAAGAAAAAAAGTGGGCCAGGTGTGGTGGCTCATACCTGTAATCCCAGCACTTTGGGAGGCTGAGGTGGGAG
GATTGCTTACAGCCAGGAGTTTGAGACCAGGTTGGACAACATGGTGAGACCTTGTCTTTACAAAAAAATACAAAAACCTATCTGGG
CATGGTGTTGCATTCCTTTAGTCCCAGCTACTTAGGAGGTTGAGGTGGAAGGATCACTTGAGTTAAGGGAGGAGACCACCCCTCAT
ATTGTCTTATGCCCAATTTCTGCCTCCAAAGAAAGAAAAAGTAAAAACTAAAAGGCAGAAATGAAAACCACAGGCAGACAGCCCAG
CGCCACACCCTGGGCCTCGTAGTTAAAGATCGACCCCTGATCTAATCGGTGATGTTATCTATAGACTACAGACATTGTATAGAAAT
GCACTGTGAAAATCCCTATCTGGTTTTGTTCTGATCTAATTACCGGTGCATGCAGCCCCCAGTCACGTACCCCCTGCTTGCTCAAT
CACGACCCTCTCACGTGCACCCCCTTAGAGTTGTGAGCCCTTAAAAGGGACAGGAATTGCTCACTCGGGGAGCTTGGCTCTTGAGA
CAGGAGTCTTGCTGATGCCCCTGGCCAAATAAACCCCTTCCTTCTTTAACTCGGTGTCTGAGTTTTGTCTGCGGCTCATCCTGCTA
CAGAGTCTAGGAGGCAGAGGTTGCAGTAAGCCAAATTCACGCCACTGCACTCCAGCCTGGGTGACAGAGCAAGACCCCACCAAAAA
AAGAAAAGAGGCCAGGCGCAGTGGCTCACGCCCAGCTAATTTTTGTACTTTTAGTAGAGACGGGGTTTCACCATGTTGGCCAGGCT
GATCTCAAACTCCTGTCCTCAGGTGATCCGCCCACCTTGGCCTCCCAAAGTGCTGCGATAACAGGAGTGGAGGCTCAACTTTTTAA
AGAAGAAAAGGACGAATCAGGACAGGAGACAATTACAAGCTCTGTTCATTCGGAATTCTCATTGGCTTACAGAAATAACTTTGGCT
AGTGATTGGTTATATGTTGTAGACAGACCCACAGGGTGGATGGCGTCTGTGGCCACTTGGGCATTAGCTGGTCCAGAGCCCAGAGT
CCATGTAGCAAGTAGCTTCAGGACGTAATTATTTAGCTCAATAGAAAGTGAGATGTGACTGCTGTTACTTTTTTTTTTTTTTGAGA
CGGAGTTTCACTCTTGTTGCCCAAGCTGGAGTGCAATGGCGCGATCTCGGCTCACTGCAACCTCTGTCTCCCAGGTTCAAGTGATT
CTCCTGCCTCAGCCTCCCGAGTAGCTGGGATTACAGGCGTGTGCCACCAAGCCCGGCTAATTTTTGTATTTTTAGTAGAGACAGGG
TTTCACTATGTTAGCCAGGATGGTCTCGAACTCCTGACCTTGTCATCCACCGGCCTCGGCCTCTCAAGGTGCTAGGATTACAGGCA
TGAGCCACCACGCCCGGCCTTTTAACTGCTGTTACTTTTTTTTTTTTTAATCATTTTTATTCTGCTAGTTCCATAAAAGCAATGTAA
ACACCAGCATTCTATACATCTTGCTGGTGAACTCACATAATGCTTAGTTCCCTGATCCTTTGACCTCCTTGTCTTCTCCAGTTATT
TTCTGTTTGGACCACTGGCCACAGGAATGGAGTGGGGTTGGGGCTTAGGAGAGAGCAATGTGGCTTTTTGGTATGACTTGTTTCAT
TGACTGCTGTTACATTTTAAATGCCTTTCTGGGCCTGATAATTTAAGGGGGCTGGCATTTCTCACATCAAAGGGCAAAGGGTTTTT
TTGTTTGTTTCTCACTATTGCTCAAAAGCCACATGTGTCAAGGACAGCTCTTGTTAACGGGCAGAGAAGCTTATAGAACCTTTCCA
GGGCTGGCGCGGTTGTTCATGTCTGGGGGACAAGAGCGAAACTCCATCTCAAAAAGAAAAAAAAAAAAAAAGAACATTTCCAGGCCA
GGCACAGTGGCTCACACCTGTAAGTCCCAGCACTTTGGGAGATTGAGGTGGGAGGATCACTTGAGGCCAGGAGTTTGAGACCAGCA
TAGGCCACATGGCAAGAACCCAGTTTCTACAAAAACATTTTTTTTTTAAATTAGCTGGGCTCGGTGGTGCACACTTGTGGTCCCAG

CTACTCGGGAGGCTGAGGTGGGAGAATCGCTTGAGCCTGGGAGGCGGAGGTTGCAGTGAGCTGTGATCGCACCACTGCATTTCAGC
CTGGGCAACAGAGGGAGACTCTGTCTCAAAAAAAATCAAATAAAGAAAAAAGAACATTTCCATTATTGCAGAATGTTCTATTGGAC
GGCGCTGGGCCAGATGCCTGCCCCAAGCCCTGGGACACCCAAACCTGGTGAAAGTGCCAAGCTCCCATCTGGGGGTGCTCACGGAA
GGGGCCGGGAGCTGGGATTACAAGCGTGGAGGCAGGCGCCACCCCAGAGGAGGTGGGTGATGTCTGAGCCAGAGTCTTTGGGATGA
GCAGGGCTTTGGCAGGCAGCTTTGTTGGGCAGGCAACAGGCACAGCAGGTGCAATGGCATCGAGGTTGGAGAGGTGGACTGGCGGG
GAGAAGAGAAGGGAGGGTGGCAGGAGAGATGGGCAGAGGCCCCCCAGGAGCCCAGAGCCTTCAGGGCTTTGGGCCCTCTTGGGGCA
CTGGGGAGCCACGGGAGAAGTGTGGGGTGGAGAGGGGCGCCCTGGATTTGACCACCTTCAGGAACCTACCTTGGCTGCCCGGGTGG
GTGGGATGGAGGTGATGAAGGTGGAGGTCGTAGCATTGTCACTGAGAGCGATGCTTATTCTGATTTTTGCCCCCGTGAGCCTCAGA
AATCGGTCAGAACAGTGCTCGGGCTGGGCGTGGTGGCTCACGCCTGGAATCCCAGCACTTTGGGAGGCCGAGGCGGGCGGATCACC
TGAGGTCAGGAGTTTGAGACCAGCCTGGCCAACATGGCAAAACCCCGTCTCTACTAAAAACACAAAAATTAGCTGGGTGTAGTGGT
GCATGCCTGTAATCCAGCTACTCCAGAGGCTGAGGCCTCCCTGGGAGGCGGAGTGCAGTGAGCCAAGATTGTACCACCGCCCCTCC
AGCCTGGGTAACAGAGTGAGACTCTGTCTCAAAACAAACAAAAAAGAACAAACCAAAACCAGTGCTCCAATTCACCTTGCTGAATT
GGAGAAGAACAGGTGCCTGGGTCCTCCCGGCTGACGGTGACGTCACTATGAGCCACCACGCCTGGCCCGGTGGGTGTTTACAGCAG
AGGTGGTTCAGGGAGGAGTGAGTGGCAGATGGTGCAGGCTCAGGGAGGAGTGAGTGGCAGGTGGGGCCTGAGGCCAGAGGCAGGAG
GACCGTTCAAGACTGAGGTGTCTTGGGCACTGGTGCCGGGGCCGGGGAGAGCCCTGGCATGCCGGGCCCCGGCCCTCAGTGAAAGC
AGGATGAGGTGGCGGCCAAGCAGGGAGGCCAAGGCCTGGCAGCAGCCGGCAGCCCCTCTCCTGGCAGGGACATCTTGGCAGGAGCC
GGGCTCTGCCCAGGGCGCTAATGTCCTCTTAATTAGCCAGGCACTTGCCAAGCACTTGCCGCTGCGGCCTCACTTTGCAGAACGCA
CTTGGGTTTGGAGCAGGACCAGCATCGGCCACCGGGTGAGTAGGGGGGATGGCCAGGTGAGTACCATGGCCAGAAGGTGCGGCCTC
TGAGTCCCCACTGAAGCCAATCCAGGCCCCGACCTCTGGTCATCATCAACAGAAGAGTGACAGAAACAGAAAAGATATCAAGGAAC
ACGTTTTCAGGGCCAGATTCCAGGTCCCCCACCTATTTTCCTTTGCTCCTGCCTTGCCTGGCTCTGCCCCCTTGAACTCTTATTCA
CCCAGTGTAGGCATCACCTCCTCCTGAAGGCCCTCCTGGACTGCACAGTTAACTCTGGAGCCTCCCCTGCACAGGGCAGCTCATGT
ATGGATCCACAGGTGAGTTCCTGTCTTTGAAGGGTTACAGTTTAGGTCCTGGAGCAGTGGTGGGGAGGTCACACCTCCACCCACAT
CCCTCTTTGCAAATCCCATGGGAACTATGAGCCTGGGTCCCCCAAACTGCATGTTCCCCACTTAAAGCTGGGCCAGATCCTGTTTG
CTGTATCCAGGCCTCAGTTTCCCTGCTTGTAACCAGAAGTCCATGCAGCCAGAAATTGTCAGGGAGCTGGGCACGGTGGCTCATGC
CTGTAATCCCAGCACTTTCAGAGGCTGAGGCAGGAGGTTCACTTGTGATCAGGAGTTTGGGACCCCAGCCTGGGCAATACAATGAG
ATCCCTGCCTCTACCAAAAAATTAAAAACAATTAGCCAGGCCTGGAGGCATACACTTGTAGTCCTAGCTATTCAAGGGAGGCTGAG
GTGGGAGGATCACTTGAGTCCAGGAGTTTGTGGCTGGGGTGAGCTCTGATTGTACCACTGCACTCCAGCCTGGGTAAGAGAATGAG
ACCTTGTTTTATTTTATTTTATTTTTATTTATTTTGAGATGGAGTCTCACTCTGTCACCCAGGCTGGAGTGCAGTGGTGCAATGTC
GGTTCACTGCAACCTCTGCTTCCCGGCTTCAAGAGATTCTCCCGCCTCAGCCTCCCAAGTAGCTGGGATTACAGGCGTGTGCCACC
ACGCCTGGCTAATTTTTTGTGTTTTTAGTAGAGACAGGGTTTCACCATGTTTGCCAGGCTGGACTTGAACTCCTGACCTTAGGTGA
TCCATGCCCCCTTGGCCTCCTAAATTGCTGGGATTATAGGCATGAGCCACCGGGCCTGGCCGAGACCCTGTTTTAAAAAAATTAAT
CAGGTCCAGTGCGGTGGCTCACGCCTGTAATCCCAGCACTTTGGGAGGCCGAGGAGGGCAGATCACCTGAGATCGGGAGTTCGAGA
CCAGCCTGACCAACATGCAGAAACCCCGTCTCTACTAAAAATACAAAATTAGCAGGGCGTGGTGGCGCACACCTGTAATCCCAGCT
ACTTGGGAGGCTGAGGCAGGAGAATCACTTGGACCTGGGAAGCAGAGCTTGTGGTGAGCCGAGATTGCACCATTCCACTCCAGCCT
GGGCAAAAAGAGTGAAACTCCATCTCAAATAATAATAATAATAATAATAATAATAATAATAATAATTGGTAGAGATGTTGGTA
GAGGCAGGACAGTGGAGTGACAGCCTGGTCCTGTCCTCCCGGGCTCTGGCTGACCATGCTGTAGTGTGGCTCAGGGCCCTGGAGG
GCACAGCTGTCCTGTGGCCCTGTGGTCAGCCGGGAAGCCAGGCCTGGCAGGACTCCTGCCCTAGAATCAGCTCCTCCCACTCCCCA
GGGTCCCATGCAGGGCGCTGGCCGTACCAGGCCTCCTCGCGGGTGCCACAGAGCCAGCGGCTGCACAGCAACAGAGACGCCATTCA
CAGGCTCTCGGCCACGAGGCCGCTCATTTACAGAGGGCTGAGCCGCTGACATCTGTGCGTTTGTAACATGGACAGCATTTCTGGTC
CCATACAGACGCCTGGCCAGCCACATTTATCTCCCCTTGAGAGAAGGGAAACGAGGCTCGGGAGGGAATGAGGGATTGGCTTGGG
GTCACACAACTTTAATGATGACCCTGGGCAGGAAGCTCCCAGGATGGCAACAGAGACTCAGGGCAGGCTGGGCATGGTGGCTCACA
CTTGTCATCCCAGCCCTTTGGGAGGCAGAGGTGCAAGGATCACTCGAGCCCAGGAGCTCAAGGCCAGCCTGGGGAATATAGTGAGA
ACCCATCTCTAAAAAAAAAAAAAAATAGGCCGGTCGCGGTGACTCACGCCTGTAATCCCAGCACTTTGGGAGGCTGAGGCGGGTGGA
TCACGAGGTCAGGAGATTGAGACCATCCTGGCTAACACGGTGAAACCCCGTCTCTACTAAAAATACAAAAAATAGGCCGGGCGTGG
TGGCTCATGCCTGTAATCCCAGCACTTTGGGAGGCTGAGGCCGGCGGATCACGAGGTCAGGAGTTCGAGATCAGCCTGACAAACAT
GACGAAACCCTGTCTCTACTAAAAATACAAAAATTAGCCGGGCGTGGTGACACGCGCCTGTAGTCCCAGCTACTCAGGAGGCTGAG
GCAGGAGAATCGCTTGAACCATCGAGGTGGAGGTTGCGGTGAGCCGAGATCGCACTACTGCACTCCAGTCTGGGAGACAGAGTGAG
ACTCCGTCTCAAAAAAAAAAAAAAAAATTAGCCGGGCGTGGTGGCGGGCGCCTGTAGTCCCAGCTACTCGGGAGGCTGAGGTAGGA
GAATGGCGTGAACCCGGGAGGCGGAGCTTGCAGTGAGCCGAGATCGCGCCACTGCACTAAAGCCTGGGCAACAGAGCGAGACTCTA
TCTCAAAAAAAAAAAAAAAAATTTAGCCGGGCATGGTGGTGCATGTCTGTACCCCCAGCAATTTGGGAAGCTGAGGTGGGAGGATT
GCTTAAGCCCAGGAGTTTGAGGCGGAGTGAGCTGTGATGGCACCACTGCACTCCAGCCTGGGTGACAGAGTGAGACCCCGTCTCT
CCCTATCCCCCGCAAAAAAAAAAAAACAACTCAGGGCTTTCCCACCCCTGTCCTTGCACAGATGAAGAACCGAAGCTTCTAGAAGG
GGTATATTTTGCCCCCAGGCCCCAAATCCTGGTCTTTGGACTACAGTCAAGACCTAGCACAGGGCTCAGGCCTAAAAAAAAGCTGT
TGAGGAGGGTGTGAGGCTGCAACGTTGCGGTGAGAAGGGGGTCCCCAGGGAGGGCGCAGCAGGAAGCCCCAGGGAAGTGCCTGGGA
GAGGGAGGTTGTGCAGCCAGAAGGAGCAGCCCGCAGCCTTTGGTTGTTGACTCCTGCTTTGTAAGTGGCAGTCTGGTTGGGTAGGA
CAGGGTCCGAGTCCTCACTCAGGGAACTGAGTCCAGGGTGAGCTGAGCAGCCCTGCCTGGTGGCCTCACTTTCCCCTGCAGAGCCT
GCTGCATGGTGTCCAGTGGCCAGCCTGGGAGGAGCTCAGGACTGGCCTCACCTCGTGCCACGCCCTCGTACCAAGGTGGCTCAGAT
GGCACCTGGGTTCCCGAAGGGCCCAGGAACACAGCGTCCATGTCCCCATCCTTCCCCGGAGGGACTTGGGGTGGGGCCTGCAGGAA
GGATCATGTGACTTGTTCAAGGGCAGCTTGTCCTGCCCCGGACACAGAGGTGCCCATCGTAAGCGAGATGCAGGCAAGGTGAGGAC
AGGGCATGGTGCCGAGCAGGAATGATTTTCGAAAATGCTACTCTGTGGTCGGGCACGGTGGCTCACTCCTGTAATCCCAGTACTTT
GGGAGGCCCAGGCGGGCAGATCATGAGGTCAGGAGTTCAAGACCAGCCTGGCCAACATGGTGAAACCCCTTCTCTACTGAAAATAC
AAAAACTACCCGGGCGTGGTGGTGGATGCCTGTAATCCCAGCTACTCAGGAGGCTGAGGCAGGAGAATCGCTTGAACCCTGGAGG
TGAAGGTTGCAATGAGCCGAGATTGCACCACTGCACTCCGACCTGGGCGACAGAGAAAGACTCCGTCTGAAAAACAAACAAACAAA
ACCCCGAGATTCTCTTTTCCCCCGTCCGGAGCTCTATGGCCATCTGGAGCTCGTTCTGTCCACAAGGACACATTTCCTGGCAGCAG
CTGTGGACCAGGGCTCACTCACTCACATCTGTACCCAATCTAGAGCAGGACAAACACCTATCACCTGCACTGGCAATGGACAGAGG
ACAGTGGCAGCCCCATCACCAGAGGCATTCAAGCCAAGGCATTTTCTATCGTCTATTTATCTATCTATCTATCTATCTATCTATCT
ATCTATCTATCTATCTAGATGAGATCTTGCTATGTTGCCCAGGTTGAACTCCTGGCCTCAAGCGATCCTCCTGCTTCAGCCTCCCA
AAGTGTTGGGATTTCAGGTGTGAACCACTGTACCTGGCTGGAGTGCAGTGGCACTGTCATAGCTCACTGCAGCCTCCACCTCCTGG
GCTCAAGGAATCCTCTTTCCTCAGCCTCCTGAGTAGCTGGGACCACAGGCATGCACCATCACACCCAGCTAAACCTTGATTTTTTC
CATAGTATATGGCTCAGGGCAGAGCAAAGAAGCACAAAAACATGTTGGCTTGCGGTTGAGGGCTGATGGAGGGGGTTCTGGCCAA
AGCCCAAAATTAACAGCCACAACGTCAGTGTCTGTGGGAGGGGTTGCCAGGGGCGTGCGGGTTCTGGGGCTCAAGGCCCTGCCGGT
AAACCCATTTGAAGCAGGATGGCAAGAAGGTGACACCATCTTCCCCCGCGCACTGAAAGCCCCTGGCTGGGATTGCCTGGGGCAGA
CACAGGCTCGGACCAGCCCCAGCAATCCCAGTTTATCAGCGAGCCGGCTGAGGGCCCGGAGTTATCTCAGTGCCCGGCCTGAGACC
TTGTGGGCAGCCTGTGGTCATGCTGGCATTCCAGGGGCCTTTTGGCATGTGGGGAATGTCCAGGAAAAGCCTCAGCCTTCGGTGAG
GCGCAGAAAAGGGAAGTGTCCCTAGAGGGGGTGGGTGAGGGCGTGGGAGGTGGTGTCTGCAGGGAATGTCCCCTTTGGGGGAGGAG
GATGGAGGGTTGGGATTCTGAGGATGGGGGGGGGGCTGTAGCCAGCACCATGTCCCTCCTGTGTGACCAGCTCAGAGTCCCATGA

AATTGGGGCTTGGGAGGGGAAGGGACACTGGCCTGGGAACCAGAGACCTGGGCTGGTCTGGCTCACAGTTGCTGGACCTCTGTGAT
CCGTGTCAAAAAACGAGAACACCAATTCCTGTCCTGCCCACTCACCACCAGGTGGGACCTGAACCCTGGCATCGCCAGCATTGGGA
ATGTCGGCCACTGACTCAACCACTCTCCCGGAGACCTATTTGGGCCACCCGAGGCGGGTGCCTGGGCCACACGGAGGGGTCCTGGT
GGTCTTCAGGGCAGCGGCTGTGGGGCTGAAGCCTCAAGGAACCACATCTCTGCATAGGAGGGCCAGGCTGCAGGGCCTCGGAGACA
ACCTAGTTGGGCGTGTTGGGGTCTGTGGGTCCCAGGTCCTGGCCTCACCGGGTCCCCACCGCGCTGTCAGCTCCCAGCCTCTTTCC
CTCGTCTGCCTCTGGGCTTCTGTAAGGCTATGTGCTCCAAGGCCACCTCCTCCAGGCAGCCCTCAGACCCCCACCTTCGCCCAGTA
CCGATCTGCACCGTGGTCTCTGAAGTCTCCATCGTGACCTCAAACCTCGCTCGTCCTTGCTCCTAGCGAGGCTTGGGGTCGGGGTG
TCCGAGGTGGGGGACATCCGGGGGGGTTAGGTGGCTGGCGCGGGGAGCCGGGGTTGTGAGGGGTGATGTCCTCAGGCGGCGGCGCT
GCGGGGTGCGGCGAGGACACCGGTGGGGTGAGAGCACCGGCGGGGCAGCAGCGGGGGCCGCAGCGCCGGGTCCCTCGGCCCGGGGC
CCCTCCCGCGCGGAGCCAGGGGCGGGACAGGGGGGCGTGGCCTGGTGGCGCTGACGTCACCTCGCCTATAAAATGTCCGGGGCGCC
GCTAGCTGGGCTTTGTGGAGCGCTGCGGAGGGTGCGTGCGGGCCGCGGCAGCCGAACAAAGGAGCAGGGGCGCCGCCGCAGGGACC
CGCCACCCACCTCCCGGGGCCGCGCAGCGGCCTCTCGTCTACTGCCACCATGACCGCCAACGGCACAGCCGAGGCGGTGCAGATCC
AGTTCGGCCTCATCAACTGCGGCAACAAGTACCTGACGGCCGAGGCGTTCGGGTTCAAGGTGAACGCGTCCGCCAGCAGCCTGAAG
AAGAAGCAGATCTGGACGCTGGAGCAGCCCCCTGACGAGGCGGGCAGCGCGGCCGTGTGCCTGCGCAGCCACCTGGGCCGCTACCT
GGCGGCGGACAAGGACGGCAACGTGACCTGCGAGCGCGAGGTGCCCGGTCCCGACTGCCGTTTCCTCATCGTGGCGCACGACGACG
GTCGCTGGTCGCTGCAGTCCGAGGCGCACCGGCGCTACTTCGGCGGCACCGAGGACCGCCTGTCCTGCTTCGCGCAGACGGTGTCC
CCCGCCGAGAAGTGGAGCGTGCACATCGCCATGCACCCTCAGGTCAACATCTACAGCGTCACCCGTAAGCGCTACGCGCACCTGAG
CGCGCGGCCGGCCGACGAGATCGCCGTGGACCGCGACGTGCCCTGGGGCGTCGACTCGCTCATCACCCTCGCCTTCCAGGACCAGC
GCTACAGCGTGCAGACCGCCGACCACCGCTTCCTGCGCCACGACGGGCGCCTGGTGGCGCGCCCCGAGCCGGCCACTGGCTACACG
CTGGAGTTCCGCTCCGGCAAGGTGGCCTTCCGCGACTGCGAGGGCCGTTACCTGGCGCCGTCGGGGCCCAGCGGCACGCTCAAGGC
GGGCAAGGCCACCAAGGTGGGCAAGGACGAGCTCTTTGCTCTGGAGCAGAGCTGCGCCCAGGTCGTGCTGCAGGCGGCCAACGAGA
GGAACGTGTCCACGCGCCAGGGTGAGTGGGGACGCTGCCCCCGCCTCTCCTGGTCCGTGCACAAAGCGCACCCCACCCGCGCCCCT
CCAGCCTCCCGCCCTTTCTCGCTCGCGGCGCCGCTGCGGTCCGGAGCACTGCCCATTGCGCCCCCGCTAGGCACGCGGGCTACCCC
GCCTGGAGGGGGCGAGGAGTGGGGCTTTGCCCATCCTCGGGTGCCGCTGCCCACCTCCCACCCCGGGCTGGGATCATGGGCTCCCC
TAGGCCCCGCGGAGTCGCAACCGTACGTGCACCCTCCTAACCCCCCCCCCGCCCAATCTTGGCTCTCCCCACGCGCGCTGATCCC
TCGGATCAGCTGTCCCAGCTCTTGCGGAGTGGGAGCCCCTCACCCATTTCCTCGTGCCCCTCCCCCCGCCGGCTGTCCTGGAGCTC
GGGGGTCCGAGGCAAGGGTCGCCACCCGCAAGGGCGCGCCTCCACCCCCACCGGCAGCCTTTCGCGGGCGAGATGGGGGAGGTTAG
CCAGGCCTTTGATCCCGGCGGGGCGCGCCTCCACCTCCCCGTCTGCCCGGCCTTCCTCCACCTCCTCCCGCTGCCGGGCGGGGTCG
GCCTCCGCTGGTGGGGGGGGCGCGGGGTGTCAGCCCTTCCCCCAGCCCCTCCTCCCGCGTCTGCCCCGCGCTCGAGGCCGCGGC
CTTTGTGAGCAGGGGGGCGGGTCGCCTCGACTGGGTCCTCCCTCGCCCCCTTTGTCCTGCTCCATCTCTGCAGATGGGAAAACCAG
ATGCGGCGGGGCGGGGGAGGGGATCGGCTTTTGCGGTTCACCCCTGCAGAGGAGCCCCCCGCGCCGCCCCCGGGCCCAGGGCTCGG
GTCCCGAGGTTCCCCAGGAGGCGGTCTCCCTCCTCGCGCCGCGGCCCGGGAACGGCGTGGCGCGGATGGCGGCCCTCCAGGCACCC
CGCCCTTCGCCCGCCGCGCGCGTCTCCAGGCCGGTGCGCTGAGCTCCGCTCCGCGGGCGACCGAGGGCGGCTTCAGCGCGAGCCGG
GAGACCCCAGGCCAGCTCCCTCGGGAAGCCCCTACCCTCTGGTGAACCCATCCCCTAGGGCGCTCGCCGGGAACAATTGACTGCAA
CTCGATCCGTCCCCTCCGGGGCCTCCTGCAGGCACGGCTATTTGCGACCGGCCTGTGCGCCACTCTTTCCCTTCTTTCTCAGATTC
TCCCCGAGGGGCTTTCTCTTCCTTTTGGCGTCCTCTTCCACTCCTGGCGGAGAAGCTGTTCCTTGCTTGCAGACAGAGGCCCCGCT
GGAGGGAGGGGCGTGGGGGATCTTTTTCCCTGGATAGGAAGTCGGACCCCAGGCCTTGGAGATGGCTGGACGGAGCCCAGTTTG
TGTCCTCATCCCTCCTCTTGGAAGGAGCCCTCCTGACGGCCCCCCTCTTGGCTGTGGGCTCTGCAGGAGGGGGAAAGACCCCCCAT
GGCAGTGGGGGTGAGGGTGGAGGCCTGGGTGGGGTAATGGCCATTTTGTGCCTGAAGTTTGCTACCTTAAGTCCCAGAGAGGTGAA
GCTGCTTGTCATGGGTCACACAGCAAGTGACCACAAGGAAGCAGCATGCAGAAGTGGGTGCATTTGGCTCCAGAAACCCCGTTTCC
TGTTTCCCACAGCGCATGGCGGGCAGAGCTCCTCACCTGATCAGCAGGCATTGAGCCCTACTAAGGGGACCTGCTGAGCCATGAGT
GAACCAGTGGGGTTCACGTTCTCTTGGGGACATTACGAAGTGTCAAGGAAAAGGCAGCGGGTACAGGTTATTAGATGGCTGAGTCC
TTTCTGAGCAGGACATTTGAGCTGGCCTGAAGGATGAATAGAAGGTGGCAGGTATGGGGGGGGTGCTGTGTGGACCAGCGTTGTAG
GCTGTGGGTGTCTCCTGTGCAAAGGTCTTATGGCAGGAAGGTACAGAGAAAGCCCAGTGTGGCTGGGAGGAAGCTTGTGAGGTCTG
GGCCACGGGACCTTGGGTGAGAGCTTAGTGCAGGGCTATGGTCCTTGCCTTGGGGTTGCAGCCACAGCCTCCCGTGGGGAAGAAGT
CGCAGAAGTAGGTGGCCTTGGCCCTGGGGGCCAGGAAGTTGCAGAGAGCCTGAGGGTGTTTTTGGCGAGCCTGGGCAGATAACTCC
CCTCTCCTGAGAAGCTTGCTGGGGGCGTCTGGTGTGTGATTCAGGCTGATAGGGTGGAGGAACCAGAAATTGCAGCCAGGAGAGG
TGGTGGTGATGGCTCAGCTTGGAGTTGAAAGGGATGTGGACGGCGCGGGGGTGGGGGTACTTGGGGGCCGAGGTTGGGGCTCCTC
TGGCCTCAGATCTTGGGGTCCTTATCTCGTTTCTATGTCAGCCAACTCCCGGGGGGCTTTTGGGAACTGGAGTCTGCAGGGGTGGG
CGTGGGGGGCGTGTCTGCAGAGGCCATTTTGGACGGGCCTGCGTGGCAAGGGGGAGGCGTCTGCTTCCGACACACATGCTGGGGAA
CGCCAGCCAGGAAGGGGAGGATCCGGACTTAGCTGGCAGGGGGGATGTGAATCATCTCTGCAGGCCTGCACGGGCGGGCCCGGTGG
GGGCAGGGTGCTCCCCTTTGAAAAACGCTGCGCCCTGCTCTTCAGCCTCAGTTTCCCCATCTGTAAAGTGTGCATTCCAGGCCTCC
TTGGGCTGGCCCAGAGCTGCCCTGGGCAGGGGCTTTCAGCCCTTCACACCAGAGGCTGGGTCAGCTGAGTGAGTGCTCCTTTGTCC
TCCCGCCATGCCCCAGGCTCCTCCCTGCTCCCCAGGATCCACACCTACCCTGGCCCCGACCCTGGGCCATGCCTCACCACACTTCC
TGTCTTCTCTCACATCTCTCACATCTGGGAGTCCTCTCCCGCCAGCCTGTGCTTGCATCTGGGGATCCATGCCAGGGATAGGACCT
GTCCTCCCTGCTGGCTTGGGACATGCCCTCTCCCAGCCACTCTGAGGAGGGCTGACTGAGGAAGGGCTCGTCAAGCTGGGCTTTGC
AGGATGTGTAAGAGTTCTCCCCACGAGGCGCAGGGCATTCTGAGCCCAGGGAATGGCTTGTATAAGGATGCAGAGGCATTTGAAAT
GGCCAAGTAGTTGCAGGAATCGACTGGAAACCGGGGTGGTAAGGTGAAGCCATAGAACATTCCAGGCCCCCTCCCCTAAATGAGAT
GGAAGGAGTGCCTGTTTTGAACAAGCCAGGGGCATCTGGGGACCGTTAAGGCCTGGGGGTGGTGATGGGGACTGGAGGGTGTGAGG
CAACCAGGGGGTGCCCCTCTGAGCCACCACAGACAGAATGGTTCAGAAGGCCAGGCACACTGGCTCTCGCCTGTAATCCAAGCACT
TTGGGAGGCCAAGGAGGGAAGATGGCTTCGGCCAAGGAGGGAAGATGGCTTCAGCCCAGGAGTTCAAGGCCAGCCTGGAAAACATG
GCAAAACCTCTGTCTACAAAAAATACAAAAAATTAGCCAGGCATGGTGATGCACGCCTGTCGTCCCAGCTACTCAGGAGGCTTAGA
TGGGAGGATCACTTGAGCCGGGGAGTTTGAGGCTGCAGTGAGCCGAGATCGTGCCACTGCACTCCAGCCTGGGCAACAGAACGAGA
CCCTGTCTTAAAAAAATTTTTTTTGGCCTGGTGCGGTGGCTCACGCCTGTAATGCCAGCACTTTGGGAGTCCAAGGTGGGTGGATC
ACCTGGAGGTCGGGAGTTTGAGACCAGTCTGACCAACATGGAGAAACCCCATCTCTACTAAAAACACAAAAATTAGCCGGGCGTGG
TGGCGCATGCCTGTAATCCCAGCTACTCTGGAGGCTGAGGCAGGAGAATCCCTTGAACCCGGGAGGCGGAAGTTGCAGTGAGCCGA
GATCGCACCATTGCACTCCAGCCTGGGCAACAAGAGTGAAACTCCATCTCAAAAAACAAAACAAAACAAAAAAAACAACCAAAACA
AACAAACAAAAAACTGTAATTAAAATAATTAAAAGAAAAAAGTTAAAAAAAAAAAAAAGAGAGGATGGCTCAAAGGCTGGAAACAG
GGAAGGCCACTGTGAGGGGAGGACAGGCAGGGCTAGACCTCTCTGGAAGGAGGAGGGAGAGGTACCCGTGGGCCCGGCACAGGAGA
CTCTTAATCTCCTGGCTCCCGGGGGCTTCTGTGGGCCTGTGACTCAGGATTTCTGTGCCTCTGTTGATGAAAAGAAAAATCCTGAA
GAAAACACTGTTCCCATAGTAACACAGCTCTAATTAGAGACTCCAAGGCCAAGCGAGGGCCTTGGAGCCAGGAGGGGCCCTGCTCT
ACTGGGGGGACGACACCCCTTTCCCCTATCCCTCCCTGCTGGGGTGTGGCCTGATGTCTACGTGGCACCAGGCCCCTAAATCCTTT
CAACATCCTGGCGGAGCGGAGAGGCTGGGCCCTTTTGACAGGTGGGGAAACTGAGGAAGGTTGCAGGGAAGGCACTCATCCAAGGG
TGCCTGGCCCCCCGCGGTGGGTGCCTCACTGGCTGGCCTAAACCTTTGCATCAAACCAGTTCCAGGATTGAACGCAACAGGCTGAT
GGGGACTGAGTCGTGGTACAGATGGCGGCAGTTGTGTGGCCCTGGGCCCAGGAATGGCTGGGAAGCTCCCTTTCTTCTCTGGCTTG
GGGGATGAGGTTAGTGTAAATTTCATGGAGTTGCCAGGAGGATTAAGGCCGACGTGCATTCACACGTGGCCCCGTGGCTGCCGCCA

GTTAAGCCCTGCGCAGCCATTGGAAGTCTCATTGAGAAGGAGCCTCCCGGCTTCCTGCATTTGTTCCAGGCGGGCTGGTAAGCGCC
CTGCTTATTTGCAAGGCTGTGTGAGGACGAGCCCTGTAAACCCTAGGACGAGGCTGTGCTAATGTGGTTTCTGTTCCACTGTCTCC
CCTTCTCCTTTTGCCCCTCTTTCTGGGGAACCTGGAGCCCCTGATGCCCTCTGTTTTTTCCTGCTGCTGGGGTGGACCTAGAACCT
CTCCCTCCATCCTTTCTGCTGCTGGGGTGGGCCTGCAGGAGGCTGTCCTCTTACCTGGTCCCTAACTTCCCTCCGATCAGCGGGGC
TTCAGCGGAGCCCCAGCGCATTGGACAGGTGGTGCTCTGTGGCCGGCACAGGCCAGGGCCCAGGGTGCGGCCCCTGCCTGTAGGTA
GACATGCAGGCCCTTCTGAGCAGAATGAGGGGGCGGTGAGGGCAGCTGTGCGGGTGTGGCTGGCCTGACGGCTCCCTGCAGCCCTG
CGGCTTCTGTGCAGTGGGGGTGGGGCGGGCCAGACCTTTGCAGGGCCTCTTGGCTGGTGGAGGGCTGAGTGAGCAGAGGCCCCAGC
CCTCGTGTTCCCTGGGGTGTGGCCTTAGGATGGTCCCGGCACCCTGGGGACCCAGCCCCTGCTCACCTTATCCTCCTCCCGTGCTT
GGCTGGGGTGTGGTGGCTGAGCCAGCCCACCCAGTGCGGTGGATGCTCGTCGGAGGGCAGAGGGTGGGCTACCGGCTTAAGGGGCC
CCAGGGAACCTGGGGGGTGGAGCCCAAGGGGTTCCAAGAAGGGGCGGGGCCAGGAACCCATAGACAAGAGGGTGGGGCAGGGAAAG
GGCGTGGCAAGAGGGCCACCCACCTCCGGTCCAGAGAGCCAGCCAGACCCTTACTTTCTCTTGCTGTGTGACCTTAGGCAAGGACA
TGCCACCACCGGCCTTAGTCTCCCTCTTTGTGAAGTAGGATGAAGATCCCCACCTGTGGAGCAGATGTGGGGCTGCATGGTTGGGT
CAGGATGGAACAGGATGGGGAGGCCGGGCGTGGTGGCTTACCCCTGTAATCCCAGCACTTTGGGAGGCAAAGGTGGGAGGACTGCT
TGAGTCCTGGAGTTTGGGCAACATAGCGAGACCACCCCCATCTCTACAAAATAATGTTAAAGTTAGCCAGGTGTAGTGGTGAGTGC
CTGTGGTCCCACCTACTGGGGAGACTGAGGCAAGAGGATCCTTTGAGCCCAGGAGGTGGAGGCTGTAGAGAGCCATGCTTGGGCCA
CTTGCACTCCAGCCTGGGCAACAGAGCAAGACCCTATCTCTAAAAAAAAAATGGAATTGAGGATGTGTCCTCTGGGTGTGGGCTCT
ACCACCCACCAGCCTCCCTCTCCTGTGGGTGCTGCCTGCCACCCACCGCTGAGGTCCCTGCAGCATCAAGGTGCCCAAACGAAGAC
ACTCTCCAGCTCTGAGCCAGGCACCCATACAGAGCCCGGCAGACGCCTCTGCTGCTGCAGTTCTTAGAATCCAGAGCGCGTGGGGA
ATGTGAATTTGTGCTGCTGGAGCCAAACATCCCACCTCCAGGTTTTAGCTGGAGGAATCCATGTGGATATGCAAAGCAATGTAGTT
ATAATGAATAGCAATTGTGGGCTGGGCGCAGTGGCTCATACCTGTAATCCCAGCACTTTGGGAGGCTGAGGTGGGTGGATCACCTG
AGGTCAGGAGTTTGAGACCGGCCTGGCCAACATAATGAAACTCCGTCTCTACTAAAAATACAAAAAAATTAGCCGGGCATAGTGGC
GGGTGCCTGTAATCCCAGCTACTCAGGAGGCTGAGGCAGGAGAATCACTTGAACCCAGGAGGCAGAGGTTTCAGTGAGGCGAGATC
GTGCCATTGCACTCCAGCCCGGGAGACACAGCGAGACTCTCTTTCTCAAAAAAAGAATAGCAGCTTTGCTATAGCTTAGGGGAGCA
AAACCCGAAAGCCACCTTAAGTTTCCCGAAGATAGAGATCCCTGGGGTCCTATTCTGAGACAGAGTCTTGCTCTGTCGCCCAGGCT
GGAGTGCAGTGGCGGATCTCAGCTCACTGCAGCCTCCGCCTCCTGGGTTCAAGCAATTCTCCTGCGTCAGCCTCCCGAGCAGCTGG
GATTACAGGCGCCCGGCACCACCCCCAGCTAATTTTTTTTTTTTTGAGACAGAGTCTCGCATTGTCGCCCAGGCTGGAGTGCAGTG
GCGCGATCTTGGCTCACTGCAAGCTCCACCTCCCGGGTTCACGCCATTCTCCTGCCTCAGCCTCCCGAGTAGCAGGGACTACAGGC
GCCCGCCACCGTGCCCGGCTAATTTTTTTTTTTTGTATTATTAATAGAGACGGGTTTCACCGTGTTAGGATGATCTCGATCTCCTG
ACCTCGTGATCCGCCTGCCTCGGCCTCCCAAAGTGCTGGGATTACAGGCATGAGCCACCTTGCCTGGTCCTTTTTTTTGTATTTTT
AGTAGAGATGAGGTTTCACCATGTTGGCCAGGCTGGTCTCAAACTCCTGACCTCATGATCCGCCCGCCTCGGCCTCCCAAAGTGCT
GGGATTACAGGTGTGGGCCACTGTGCCCGGCCAGTCCCATTCTACTTTTAGGGACATGGAATCATGTAAAGATGCCATACAGAAA
ACAGTATGCAAATCCAGAGACAGAGGACATCTGTGCATGTTTCTTTCTTTCTTCCTTTTTTTTTTTTTTTTTTTTTTTGAGATGGA
TTCTTGCTCTGTCGCCCAGGCTGGAGTGCAGTGGTGCAATCTCAGCTCACTGCAACCTCTGCCTCCCAGGTTCAAGTGATTCTCCT
GCCTCAGACTTCCAAGTAGCTGGGATTACAGGCATGCCCCACCATGCCCAGCTAATTTTTGTATTTTTAGTAGAGGCGGGGTTTCA
CCATGTTGGCAAGGCTGGTCTTGAACTCATGACCTCAGGTGATCCACCCGCCTCAGCTTCCCAAAGTGCTGGGATTACAGGCGTGA
GCCGCCGTGGCAACCCGCATGCTTCTTATGTATAAGAAAAAAACAGCTAGAAAGTTGTGGGTTCACTGGCTGGGCACATGGTGGCT
TGTGTCTGTAACCTCAGCACTTTGGGAGGCTGAGTTAGGAGGATCATGTGAGGCCGGGAGTTCAAGACCATCCTAGGCAACATAGC
CAGACCCTGTCTGTACCAAATAGAAAAAAAAATTAACTTGATGTGGTGGTGTGTGCCTGTAGTCTCAGCTATTTGGGAGGCTGAGG
TGGGAGGATCACTTGAGCCCAGGAAGTCGAGGCTACAGTGAGCTATGATTGTGCCACTGCACTCCAACCTGGGCAACAGAGCAAGA
CCCTGAATCAAAAAGAAAGAGATGTATTTGCCAACACAGGGGTGTTCTGCAGTGGGGTGGGAAGGGGACGTGTAGGACTTCATAGT
TTCCTTTTTTTTGTTTTAGAGACAGGGTTTAAAAAAAAATTTTTTTTTTTTTTTGAGGCGGAATTTCATTCTTGTTGCCCAGGCT
GGAGTGATCTCGGCTCACTGCCATCTCCGCCTCCCAGGTTGAAGTGATTCTCCTGCCTCAGCCTCAGGCAATTTTGTATTTTTAGT
AGAGACGGGGTGACTCCATGTTGGTCAGGCTGGTCTCAAACTCCCGACCTCGGGTGATCTGCCTGCCTTGGCCTCCCAAACTGCTG
GGATTACAGGTGTGAGCCACTGTGCCTGGCCCCTTTTTTTTTTTTTAAGTAGGGTCTTGCTAAGTTGTCCAGGCTGGTCTTGAAC
TCCTGGTCTCAAATGATCCTCCTGACTTGGCCTCCCAAAAGGCTGGGATTGCAGGAGATATGAGCCACCGCACCCGGCCCTTTTAT
AAGTTTTCTAAAGGAAAGGAACATGTACCATCGTGTTTTATTTTAATTTTTACATAGAAGCAGGAACAATAGCATGAACCCCCAGA
CACGGCCCCCCAAGATGCGCTGTTCCGCTGTGCTGCAGCCCCATTTGTCCTTTTCTATACCATTTTTGGTTGTATTTGAAGCAAAT
CCCTGACATCAGGGCATTTCATCCCAAAATACTTCCATCTGTGTTTTCTAAAAAAACGAGGCCATTTTCTTATGTAACCACAACGTG
ATCAACAGTAATCAATGCTTAACATCTAATTCTGGGCCACGTTAAGACCCTCCTGATTGGCTCAAGAATGTCTTTGTGCAGTTGG
TTTCTTGATTCTTTTTGTTTTTTTTGAGACAGGGTCTCACTGTGTCACCCAGGCTGGAGTGCAGTGGTGCAATCACGGCTCACTGC
AGCCTCAGCCTCCTGCCTCAGCGATCCTCCTGCCTCAGCCTCCCAAGTAGCTGGGACCACAGGTTTGCACCACCACTCCCAGCTAA
TTAAAAAAAGTAATTTGTAGAGACGGGGGTGGGGTGGGGGTCTTGCTATGTTGCCCAGGCTGGTCTCAAACTCCTAGACTCAAACA
ATCCTCCTGCCTTGGCCTCCCAAAGTGTTGCGATCACAGGCATGAGCCCCTGTCCCTGGCCCCTATTATTTCTCTAACTGGGGAAA
GTCATGTGGGAACAGATGTAGCTTGCCTTGGCCTCTGACCGGCCCTGCCTGCGTTCCTGGGTGCTCTCTGCTGCTTCTCATGTGTG
CCACTGTGGGGACTCGGCCGCCCACCCCACCCCGTGGTGTTACCTTGCGTGTGTAGTTCTGTGAGCTCAGGGCTATGGTCTGCCAG
AACTAGGGGGCGTGGGGCCCCAGTACCAGCCCAAGGCCTCCTCTCTGCAGGTATGGACCTGTCTGCCAATCAGGACGAGGAGACCG
ACCAGGAGACCTTCCAGCTGGAGATCGACCGCGACACCAAAAAGTGTGCCTTCCGTACCCACACGGGCAAGTACTGGACGCTGACG
GCCACCGGGGGCGTGCAGTCCACCGCCTCCAGCAAGTGAGTGCCTCGCTCCCACCTGTCACCGCCCCCACCACCTTGCCTGGGCTA
CCCCGCCTGACCCTGTCCCGCCATCCCCCAGGAATGCCAGCTGCTACTTTGACATCGAGTGGCGTGACCGGCGCATCACACTGAGG
GCGTCCAATGGCAAGTTTGTGACCTCCAAGAAGAATGGGCAGCTGGCCGCCTCGGTGGAGACAGCAGGTAACACTAAAGCCCCAGT
TCCCTGGAGCCGTCCTGGAGTCCTGGAGGGTCTGGCCATGCCGTGGTCACTTGGTAGCCCCAGCCAAGGCCTGCTCTGTGCTGGGC
ATCCCCCCGGACTGGCCCCGCACTGTCCTACCCTGGGGCTGACTGCTGTGTGACCCCAGCTCCTGGCCCTCCCTCTCTGGTCACCC
CAGCCTCCACCCCACTCCCTGCCAGGAGGCTCACTGACTCCCCTCTTTCTGGGACAGGGGACTCAGAGCTCTTCCTCATGAAGCTC
ATCAACCGCCCCATCATCGTGTTCCGCGGGGAGCATGGCTTCATCGGCTGCCGCAAGGTCACGGGCACCCTGGACGCCAACCGCTC
CAGCTATGACGTCTTCCAGCTGGAGTTCAACGATGGCGCCTACAACATCAAAGGCAGGTTCTCCTGTGGGCAGCTGCTGGGCAGGG
AACCCCTCGGTCGGGGCTGGGGTCAGTGCTGCGGGGAGCGCCCTCTGCATCCACACTGGACCCTGGCTTGGCTCAGGGCCATTCCA
GGCCCTAAAGGACAGGTGTCTGATGGCCACCAGGGGCTCTGGGATGCAAGCAGCCCCTTTCCCTCTTGTCTGTGTGGTTGGGGGG
ACTTACCTTGCCCACCTGACAGAGAGGTGTGTGGAGGGGAGAGCAGGGAGGGGAAGGAGAGCAGGGAAGGGGAGGGAGGAGAGCAG
GGGAGGGGAGAGCCGGGAAGGAGAGGAGAGCAGGGGTGGGGAGGTTCTGGAAAGGGTGTGCAGGGGAGGACGCGCCTCGGTTATGG
GACTGGAGCCCCTTCCCAGGAGGACCCCCAACAATCCAGAGGTGCCTGTTAGGATTCAGAACATGGTTTTTTTGTTTGTTTTTTTG
AGACTCACTCCCTCACCCTGGCTGGACTGCAGTGGCGTTATCTCGGCTCACTGCAACCTCTGCCTCCTGGGTTCAAGTGATTCTCC
TGCCTCAGCCTCCCAAGTAGCTGGGATTACAGGTGTGCACCACCACGCCTGGCTAATTTTTATATTTTTAAAATTTATTATTTATT
TATTTTGAGACCCAGTCTGGAGTGCAGTGGCGTTATCTCGGCTCTCTGCAACCTCTGCCTCCTGGGTTCAAGCGATTCTCCTGCCT
CAGCCTCCCGAGTAGCTGGGACTATGTGTGGGAGCCACCATGCCTGGCTAATTTTTTTGTATTTTTCATAGAGACGGGTTTCACCA
TGTTGTCCAGGCTGGTCTTGAATTCGTGGCCTCAAGTGATCCGCCCACCTCAGCCTCCCACAGTGCTGGGTTTATAGGTGTGAGCC
ACCACACCCGGCTAATTGTTTTGTATTTTTAGTAGAGACGGAGCTTCACTATGTTGGCAAGGCTGGCTCGAACTCCTGACCTCAAG

TGATCCGCCCACCTCAGCCTCCCAAAGTGCTGGGATTACAGGCGTGAGCCACTGCGCCCGAGCAGAACACGTTCTAGGACCCTTGT
TCATGTGTCCATCATGGACAGGAGGACGTGCGGGCCATAGGGACCCTGGCTCATTCCGGAGCCGGGACTGGAGGGTGGGGCGTCAC
CCTTGGGAACACCCGTGCCCACCCTCCGCTGCCCAGGGTAGGGGTGGGGAGCCAGGCTTTGGGCCCCACTTGATAAAGTCCCCTCC
CCAGACTCCACAGGCAAATACTGGACGGTGGGCAGTGACTCCGCGGTCACCAGCAGCGGCGACACTCCTGTGGACTTCTTCTTCGA
GTTCTGCGACTATAACAAGGTGGCCATCAAGGTGGGCGGGCGCTACCTGAAGGGCGACCACGCAGGCGTCCTGAAGGCCTCGGCGG
AAACCGTGGACCCCGCCTCGCTCTGGGAGTACTAGGGCCGGCCCGTCCTTCCCCGCCCCTGCCCACATGGCGGCTCCTGCCAACCC
TCCCTGCTAACCCCTTCTCCGCCAGGTGGGCTCCAGGGCGGGAGGCAAGCCCCCTTGCCTTTCAAACTGGAAACCCCAGAGAAAAC
GGTGCCCCCACCTGTCGCCCCTATGGACTCCCCACTCTCCCCTCCGCCCGGGTTCCCTACTCCCCTCGGGTCAGCGGCTGCGGCCT
GGCCCTGGGAGGGATTTCAGATGCCCCTGCCCTCTTGTCTGCCACGGGGCGAGTCTGGCACCTCTTTCTTCTGACCTCAGACGGCT
CTGAGCCTTATTTCTCTGGAAGCGGCTAAGGACGGTTGGGGGCTGGGAGCCCTGGGCGTGTAGTGTAACTGGAATCTTTTGCCTC
TCCCAGCCACCTCCTCCCAGCCCCCCAGGAGAGCTGGGCACATGTCCCAAGCCTGTCAGTGGCCCTCCCTGGTGCACTGTCCCCGA
AACCCCTGCTTGGGAAGGGAAGCTGTCGGGTGGGCTAGGACTGACCCTTGTGGTGTTTTTTTGGGTGGTGGCTGGAAACAGCCCCT
CTCCCACGTGGCAGAGGCTCAGCCTGGCTCCCTTCCCTGGAGCGGCAGGGCGTGACGGCCACAGGGTCTGCCCGCTGCACGTTCTG
CCAAGGTGGTGGTGGCGGGCGGGTAGGGGTGTGGGGCCGTCTTCCTCCTGTCTCTTTCCTTTCACCCTAGCCTGACTGGAAGCAG
AAAATGACCAAATCAGTATTTTTTTTAATGAAATATTATTGCTGGAGGCGTCCCAGGCAAGCCTGGCTGTAGTAGCGAGTGATCTG
GCGGGGGGCGTCTCAGCACCCTCCCCAGGGGGTGCATCTCAGCCCCCTCTTTCCGTCCTTCCCGTCCAGCCCCAGCCCTGGGCCTG
GGCTGCCGACACCTGGGCCAGAGCCCCTGCTGTGATTGGTGCTCCCTGGGCCTCCCGGGTGGATGAAGCCAGGCGTCGCCCCCTCC
GGGAGCCCTGGGGTGAGCCGCCGGGGCCCCCCTGCTGCCAGCCTCCCCCGTCCCCAACATGCATCTCACTCTGGGTGTCTTGGTCT
TTTATTTTTTGTAAGTGTCATTTGTATAACTCTAAACGCCCATGATAGTAGCTTCAAACTGGAAATAGCGAAATAAAATAACTCAG
TCTGCAGCCCCAGGCCGGCCTGTGTGTGTCTTGGGGCTGAGGTGGGTGGGGGGGCTGAGGTGGGTGGGAGGGCTGGCGGGACAGGT
AGGCGCCCTGGCTCCCCAGCTCAGTGCTGGGAGTGTGCAGTGGGAGGGAGGCCGTGGCTCCAGTGGGTGCTCCGGAGCTCGTGGGC
CCAGCACACCTCCTTAAGCGGGGGATGGAGCGCTGGGAGGGGGTGGACTGTGGCCCATGCGACCCCCAGAGCCATTAGGAGGAGTT
CTGTGGTGAGAAGTGGCTGTGGCTCCTCGTAGGCTACGTCCACCATGCGGGGGACCTCGGGGGTGTCTGGCGGTGGCACGCTGGAT
GTTGAGAAGGCGCAGCCCAGGGAACACTCAAACCAGGAGACCCCACATTATCCTCTAGTTGACATGTGCCCTTCGACTAGGGGACT
TGGTGGTAGGGGCAGCTCCGCCCCCATACTGCGAGGATCCGGGCCTTCCACTTCCCCAGACACAGCAGGTTGGGGTGCCCCTGGGG
CTGGGAGATGCTGCCCTGGGCCCCTCAGGGGGCGGCCGTGGTAGGATGGACCGGGCCGAGCGTGCAGACCCTGGGCAGGTCCCTAC
ATCGGAACCAGCAGCCCTGGGGATATCTGGCATATCAGAGGCTGAGGACTGTTTCCAGACTTTACCAAGGCCACAGTCAGGGCGAC
CTGGGTTCTGAGCCTCCCTTTGCATCCTAGGCTGCAGGGACAGGGGCTGGGCAGAGGCTCGCGGACCTCGTGCAGCTTCATTCACT
CCCAGGTGCCCCTAAGGATAGCAGCTGCGAGAAGACTGAGGGGAGGAGCAGTAAGGCGCAGCCTGGAGCGGAGGGACCTGGCGTCC
AGTCATTGTGCGCTGGGAAGGCGGTGATGGCGACGGAATTTGGGACACGCGGGGCGTCCCGGGGGCAGCCAGGGCACTGCAGGCCG
GAGCCCCCTGTTCCCCCGCATCCTCCCCGCCGTCGGCAGCAGAGCAAGCGCTGATTGGCTGCTGATGACGCAACTGGGAGGGCTGC
GCTGTGATAGGTGGTCCCTCGGGGGCGTGGGGCGCAAGTCTTGAGATTGGCAGGGGCAGGTGGCTGCTGCAGAGGGAAGTCGGGGT
CACCTCAGCGAGGTTCCGGCGGCCACCCCTCTCCCCTCCCCCATGAAAGCCAGGCTGGAAGCCAGAAAAATCCCAGTGACTGGAAG
GGACAGATTAATCCTCCGGAACCAAACTCTTTGAGACCTGGGGAGGAGGGTGGGAGGCACTGGGAAGGGGGATTGGGGGGAGCCTG
GCTCATTTCCACCCATGGAGCTGACCTAGAATGACCCTAAGGTCCCCGGAGCCACCAGCTGATTCCGAATTCCATTCATTCTGCAA
AATTTGGCGCCTACCACGTGGCCGAGATTCCCGAGGCTGCAGCCAATGAGCAGCATAGTCCGATGGGGGAGGCTGGAAGGGCCGTT
CACGAGATCTCTCGGGAGGAATCGGTAGGAGCTGCCCAACTGAAAGGGACGGGGGAATAACCCGGGGCGAGGGACCTGCCCAGGCC
CTGCCCTCTGGGGGGAAGCCAGGCCAGGGGCCGGGGGCATCTCTGGGGTCCCAGGTGAGATGCGGGTCGAGGGAGCCAGGCCACGG
TTCTCCAGACTCGCGTGGAGGCCACGAGCGTCTTCTGGAGGAGCGGGCACTGCGCGGACCGGCAGACTCTGGGGCGCTCGTGCTCC
CCATCTCCTGACCTTTTCCTACCTTCAGTTCCCTCCTGTCAGGACAGGTTCTGGGGCTCGCCGGGGCAGGGAGCCAGGGCAGAGGG
TGCAGTTTCCGCTCCTGCCTGAGTCACCTTCGCTTTCTCAGCGATTCTTCATTCACAAGGGCGTGGCCCATCACACGCACTCACAC
ACTTGTGAACTTGCACGCACACACACATATGGACCTGTGCAAAGAGCCCCATGCACAGGCAGGTACACGTGCATGTAGGCGTGCAC
ACAGACACGCACAGATGCAGGCAGACCAACTCCCAGGAATCTGTCTCCAGCCTACAGGGATTCAGCACTCAGGGTACCAGCCTCTG
TCCCGTGCACCCCCATTCCCGGCAGTTCTCCCAGGTGGGTCGGGGGCCACTAGGGACCCCATACCCCGAGGGTGGCTCCTCCATTA
AGCCTGCCATGGGGTTGGGAGTGAGAGTCCCACCTCCCACTTGAGAGTCCCCAGTGAGTCAAGGCAAGTGCAGCCAGGGCTCCAAG
ATCCTTGTTGGGATTCACTGTGATCCCCAAAGATCACGACTTAAGGGAAAACATTCAATTAAACTTGTTAGAGCAAAGAAAGGAGA
GACTGGGCTGGGAGCAGTGGCTAACGCCTATATAGTTCAGCTACTCAGGAGGCCCAGGTGTGAGGATTGCTTGAGTCCAGGAGGCT
GAGGCTGCAGTGAGCTATGATGGCACCACCCACTGCACTCCAGCCTGGGTGACAAAGCAAGACCCTGTCTCAAAAAAAAAAAAAAA
AAAAAAAAAAAAGGAAAAAGAGGCCGGGCACAGTGGCTCACACCTGTAATCTACTAAAAATAAGAAAATTAGCCAGACATGGTGGT
GGGTGCCTGTAATCTCAGCTACTTGGGAGGCTGGGGCAGGAGAATCGCTTGAACCCAGGAGGCGGAGGTTGTGTGAGCCGAGATTA
CACCACTGCACTCCAGCCTGGGCGACAGAGTGAGACTCTGTCTCCAAATAATAATAATAATAATAAAATAAAAAGAAAAAAGA
AAGAAAGAAAGGAGAGACTGAGCTATATTGCCCAGTCCAGGCATGTGGGCAGTCTGGGGGACAGTGGGCATACTCGGTGTCTCAG
GGTCCCAGCCCTGGGTGCCCCCACTGCTGTCTGCTGTGACCTCCTCAGGAGGCCTCATTGGGAGGGACCCTGAGTCCTGCGGGGGG
CTGTGGAGCTGTCACCAGCCCCAGGAGGACTCAGGCAAGTCCCTCTGGTGGTCGAGGCCTCTGAGCTTCCCAGTGTGTCTCCACGG
GACACCCCCCCTCCAGACCTTCTGCCCCAGCTCAGCCTCTTCCAATTCATTATCAGGGGGTGGGGGAGGGGACAGGAAGCCCCAGA
CCCAGCTGGGCCCCATTTCCTCCAGGGCCACAGTGGCCTCTCCCAGAGTCACGAGGATGGGGGCTGATCTCACTCTTTTTTTTTTT
TGAGACGGACTCTCACTCTGTCGCTTTTTTTTTTTTTTTTTTTTTGAGACGGAGTCTCACTCTGTCGCCCAGGCTGGAGCGCAGT
GGCGTGATCTCGGCTCACTGCAATCTCCACCTCCCACGTTCAAGCGATTCTCCTGCCTCAGCCTCCTGAGTAGCTGGGACTACAGG
TACGTGCCACCACACCTAACTAATTTTTGTATTTTTTAGTAGAGATGGGGCTTCACCATATTAGCCTCCCAAAGTGCTGGGATTAC
AGGCATGAGCCACCATGCCCGGCCTTGACTTCACTCTTTTAAAAAAGTCAAATACTTGGTGCTTGAAATCCCAGCACTTTGGGAGG
CTGAGGCAGGAGAACTGCTTGAGCTCAGGAGTTTGAGACCAGCCTGGGCAACATGGTGAGACTCCTGTCTCTGTAAAAACTACGAA
AATTAGTCAGGCATGGTGGTGCGTGTCTGTCGTCCCAGCTGCTCAGGAGGCTGAGGTGGGAGGATGGTTTGATCCTGGAAGGTTGA
GACTGCAGTGAGCTGTGATTCCACCACTGCAGTCCAGCCTGGGCCGCAGAGCAAGACTCTCTCTCAAAAATAAATAAAATAAAATA
AAAAGTCTAAAACTTTTCTTTCAAACGACGGCTTTTGGGGCTCATAAGGTGCGTGAAGATGGAAAGGGGGCCCGGCAGTGCCCATT
TCGGCTCCCTTGGGACCCCGGCTTCCCTGAAGACCCCTGAGCAATGGTTGGCTGGGTGCCTGGTCTCCCTGAACGGCTGTGGGCA
ACAAGGTTGTCTGGATGGGGGAGGGGCTCTGAGACCCTTGGGGGCAGCAGAGGGAGAAGGGAACAGATGGGGTCTCCCAGGCAGAG
AGGCCTGGAGGGGTGGGGCGGGGCAGGGCATTTGGCGTGAAACCCAAGCCCCTTAACGGGGCCCTAGCTTAAACCGGGAGGAGGGG
CCTCTGTGATGTGCAAATAATTAACACAGAAGCCTTCACTGTGCTCCCCTCCTCATCCCAGGACCCAGGTCTGACCCTCAGGAGGG
GAGGAGGTGAGCAAGGGGGGAACCCACCTTCCAGGGAACCCCCAGATAGTGCCAGGCACCAGGCAAGTGCTCTAAAAGGCTTTACT
AATATTATTTGAAGAAACTAAGCTAATAAATAAATACGGTATAAAATCAAAGGGTACCCACAGATATAAACAAAAATCCACAGCTA
ACCCGGCCCCAGCCCTCTTTCCTTTGGAGGTAACTGTGTTAATGGTTTTTTACACCCCTCTGGAATTTTTTTTTTTTTTTTTTTT
TTTTTTTTTTTTGGGAAGGAGTCTGGCTCTGTCGCCCATGCAGCGCAGTAGCGTGATCTCGGCTCACTGCAACTTCCACATCCCG
GGTTAAAACGATTCTCCTGCCTCAACCGCCTGAGTAGCTGGGACTACAGGGGCGCACCACCATGCCCGGCTAATTTTTTTTTTTT
TTTTTTTTTTTTTGAGCTGGAGTCTTGCTCTGTCGCCCAGGCGGGAGTGCAGTGGCGCAATCTCGGCTCACTTGCAAGCTCTGCCT
CCCGGGTTCACGCCATTCTCCTGCCTCAGCCTCCTGAGTGGGTGGGACTACAGGCTCCAGCCACCACGCCCGGCTAATTTTTTGTA
TTTATTTTTTAATAGAGATGGGGTTTCACTGTGTTCGCCAGGATGGTCTCGATCTCCCTTTTTTTTTTTTTTTTTTAGACGGAGT

CTCGCTCTGTCGCCCAGGCTGGAGTGCAGTGGCATGATCGTGACTCACTGCAAACTCCACCTCCCGGGTTCACGCCATTCTCCCGC
CTCAGCCTCCCGAGTAGCTGGGACTACAGGCGCCCACCACCACACCCGGCTATTTTTTTGTATTTTTAGTAGAGACAGGGGTTTCA
CCATGTTAGCCAGGATGGTCTCGATCTCCTGACCTTGTGATCCTCCTGCCTCGGCCTCCCGAAGTGCTGGGATTACAGGCGTGAGC
CACCGCGCCCGGCCTCTTGTTTTTTTTTTTTTTTTTTTTATAGACTTCTAAGGAAAGCAACACTAAGAAAATATATTTTTAAGAA
ATTATTCAGCCAGGCGTGGTGGCTCACGCCTGTAATCCCAACACTTTGGGAGGCTGAGGCGGGTGGATCACTTGAGGCCAGGAGTT
TGAGACCAGCCTGGCTCACATGGTGAAACCCTGACTCTACTAAAAATACAAAAAATTAGCCGGGCACAGTGGCATGCTCCTGCAAT
GCCAGCTACTTGGGAGGCTGAGGCAGGAGAATCCCTTGAACTGGGGAGGCAGAGGTTTCAGTGAACCAAGATCGAGCCACTGCACT
CCAGCGTGGGTGACAGACTGAAACTGTGTCTCAAAAAAAAATTTTTGTTTTTGGAGATGGGGTCTTGCTCTGTCACCCAGGCTGGA
GTACAATGGGGTGATCTAGGCTCACTGCAACCTCAAACTCCTGGGCTCAGGTGATCTTCCTGCCTCAGCCCCTGGAGTAACTGGCA
CTACAGGTGCATGTCAGAACACCTGGCTAGTTTTTAACTTTTTTGTGTGTAGACAGGGTCTTGATATGTTGCCCAGGCTGGTCTCC
ATCACCTTCTGGGCTCAGTGATCCTCCTGCTTCAGCCTCCCAAAATGCTGGGATTACAGGTGTGAGCCACCATGCCCGACCCTGAG
GGATACCTTAATGTTTATTTCTCACATTTCTTCTAGAACAAACATTGCCAGCGCATCCCAGACTCTGAACTGTGGCTGGGGACTGC
AGCATGGAGGTCTTTGCAGGCACTCTGGGAAACATGCCACAAGCAGGGTATTAACCCAAGACAGTCAGTCCCTCCAATGAGAAAAA
GGAGCCGAGGGACTGTGCATGCCTCAGAGCCAGATGCAGCAGAACTGGTGGGTTGAGGGGGCACTGAGCGCCTGGTGCCTCCTGCT
GCCCCCCACCAGCTCCGACAGTCCTCGTCCTTGTCCTCCCCATCAGAAGGGAAGAGTGAATTGTCTGTGGGATGTGCCAGTGTCCC
CGGTTCTGAGGTTGGGCAGGCTGGGGAGCCCCAGACATGGCTTGCTGCCCACTGTGGAGCCTCCTGGACTTAGTTCCAGGCTCTGC
CCGATCCTCTGAGGGTGTGGCTTCAAAGCCCTGCTTGGGCCTAAGGCCATCCCAGGGCAGTGACTGAGGGTAGACTGAGATGTCCC
TTGGGCACCAGTCCTAGGGTACCTGGGCTGGGGGTCTTCAGCTCAGGCCCCCTAGTGCCCGTCCTTTGGGCTGTGGGCAGGTTTCC
ACAGCACTGGCCCTTTCTGACCTTACACCCCTGTGCATCCTCACTGGGGCTCTGTGAAGGTGAAAGTCACACTAAGGACTGTGCCT
ATGGTGGCCAGGGCACCCCACCGGCAGCACACCTCAGAGGCCTCATCCTAGGCGCTGCTCACTTTTTTTTCTTTTTTTTTCCTTTTT
TTTTTTTTTTTTTTTCCGAGACAGAGTCTTGCTCTGTCACCTAGGCTGGAGTGCAGTGGCATGATCTCGGCTCACTGCGACCTCC
TCCTCCTCCTGGGTTTAAGCAATTCTCTTGCCTCAGCCTCCTGAGTAGCTGGCATTACAGGTGCCCGCCACCACGCCCGGCTATTT
TTGTAGTTTTAGTAGAGACGGGGTTTCACCATGTTGGCCAGGCTGGTCTCAAACTCCTGACCTTGTGCTCTGCCCGCCTTGGCCTC
CCAAACTGCTGGGATTACAGGCGTGAGCCACTGTGCGTGGCCCTGCTCATTTTCTTACCACAGCTTTCCAGAAATAAAGTTAGGAT
GGAAAGAGAGAGAGAGAGATTGAGAGAGAGAGAGAGCACCACATTCAGATAGAGACAGGAGAGTTCTCAGCAAGAAGAGAACTCAG
CTCAGAGTTGAGAATGTCTCAACTCAGCTGTCTCCCTGCTGAGACGTCCTGGGCTGAAGTGTCCCCTCACCCCTGCGGGGCTGTGA
CTGCACACTGCACACATGGGTCACAGCAGGGCTTGGCGTTTGGAGCAGGCCGGGTTGGTGGCTTGTCCTCATCTCTTGCCTGGAGC
TGTGCTGGCCTGTGGTTTGCTTTGGCTGCTACCATGTGGAGGGAGAGGTGCTTGTGTCCATGCCTGGCCTCTTGGAAGGCCACCAC
TGTGATGGAGAGAGGAAGGCCACCACTGTGATGGAGAGAAGCCGGGGCCAGACCGATGAGGGCTGAGAGCCCATGGCCAGGGGTTG
TGGTCTCGGCCAAACCCCAGCTGGCAGCTGATGGCAGCCACCCCGGGGTCCTCAGATATGGCACAGCTGCCTGGCTGAGCCTCGGC
AAAGGGAAGCCTTGTGGAAAATAGCAACTCAGGGATGTTGTGAGTAGTGGGCTGAGCTGTGGTTTATTTTTAGAGACAGGGTCTT
GCTCTGTCACCCAGGCTAGAGTCCAGTGGTGTAATCATAGCTCACTGCAGCCTTGACCCCCCAGGCTCAAGTGATCCTTGTGTCTT
GGCCTCCTGAGTAGCTAGGACTATAGGTGCATACCACCATGCCTCGCTTGTTTTTTGTTTTGATAGAGATGGGGTCTTGCTAGGT
TGCCCAGGCTGGTCTGTAACTCCTGGGCTCAAGTGATCCTCCTGCTTTGGCCTCCCAAAGTGCTGGGATTACAGGCGTGAGCCACT
GTGCTGGCCTTGGGGTGTGGTTTTATGTGGCCATAGATGACTGAGATGGGTGAGAAGCGGGACCTAGGGGAATATACAGGGCAGGG
GGATCCGACAGGGGGACAGGAGGGCAGACAGCTCCTGGAAGGCCTGGGAAAGAGATATCAGGGCTGGACCCTGGAGAAGTGAGGCT
TTGAGGGAGGGGAGGAGATGGGGCCTGGCGGGGTACGGGCGCTGCTCCTAGGTCTGAGGGCATCGGCGGTCTGAGAGCTTGCGGGA
GGGAGGGCTGCCTCCCGGTGCTCGGTGCCCTGGCTCTCCTCCTTGCTGCTGGCTGGCCTCCTCCTCCTCATGCATCTGTGTGTCCT
GGAACACAGGGCACTGTCCCTCTGCCTGTCCAGAAAAGCACGTGCTCCTTGGGGCCTGGCCTGCTCAGCCCTATGTCCCACCCGCT
CCCTGCTGCTATTCCCAGGCCTTCGGGCCTCACTGCCTGGGGTGGCCGTGCTCAGCCCCACCCTGTGGTGCCATGGCCTCCCCACC
CCTGCCCCGAACTGCCCCTGGACCAGTTCCTTCAGCTCCCAGGACAGGGCCTGGCCATCCCTGTGGATTCTCCCTGTTCGTGGAC
CCCCATTGGTGCCCAGTCTCCTTGGACTGTCTTCTGAGCCTCTCTCAGATGTACCCCCAACTGTACCCTTTCACCCAGAGGCCATT
TGGCAGCGTCCTCCAGGACTCCTGCCGTGGGGGAAGTCTCCACCAAGGCCAACGCGTGACTGCATCCCGAGGAGCTGATTTAAGAT
CTGGTTCTCAGGAACACCTGGAACTGGCCAGTCAGCATCCCCGGCCCTGCGGTCTGCACTTGGACAAGGGTCAGGCAATGAGGATG
AGCAGCAGGCGGGGCTTCTCCTGGAAGCCTTCCCAGCCTCCTGGGCCACTCCAGTGGGTCCCCTCCACAGACTCAGCCTGTTCCCT
GCCACCTGGTGCCGGTTTCTCTGTGGCACCTACTAGGGGCCGAGTGCCCCGGGACTGCCCAGCTGAGCAGCGCCGTCCAGGTCTGC
ACCCAGCTACACTCAGGGCACGTGCACAGGTGAGCAGCCGGGCACGCAGGCACCTGCCAGGTGTGGGTGTGGACTAGGTGCTCCCG
G

HUMAN SEQUENCE - mRNA (SEQ ID NO: 5)

GCGGAGGGTGCGTGCGGGCCGCGGCAGCCGAACAAAGGAGCAGGGGCGCCGCCGCAGGGACCCGCCACCCACCTCCCGGGGCCGCG
CAGCGGCCTCTCGTCTACTGCCACCATGACCGCCAACGGCACAGCCGAGGCGGTGCAGATCCAGTTCGGCCTCATCAACTGCGGCA
ACAAGTACCTGACGGCCGAGGCGTTCGGGTTCAAGGTGAACGCGTCCGCCAGCAGCCTGAAGAAGAAGCAGATCTGGACGCTGGAG
CAGCCCCCTGACGAGGCGGGCAGCGCGGCCGTGTGCCTGCGCAGCCACCTGGGCCGCTACCTGGCGGCGGACAAGGACGGCAACGT
GACCTGCGAGCGCGAGGTGCCCGGTCCCGACTGCCGTTTCCTCATCGTGGCGCACGACGACGGTCGCTGGTCGCTGCAGTCCGAGG
CGCACCGGCGCTACTTCGGCGGCACCGAGGACCGCCTGTCCTGCTTCGCGCAGACGGTGTCCCCCGCCGAGAAGTGGAGCGTGCAC
ATCGCCATGCACCCTCAGGTCAACATCTACAGTGTCACCCGTAAGCGCTACGCGCACCTGAGCGCGCGGCCGGCCGACGAGATCGC
CGTGGACCGCGACGTGCCCTGGGGCGTCGACTCGCTCATCACCCTCGCCTTCCAGGACCAGCGCTACAGCGTGCAGACCGCCGACC
ACCGCTTCCTGCGCCACGACGGGCGCCTGGTGGCGCGCCCCGAGCCGGCCACTGGCTACACGCTGGAGTTCCGCTCCGGCAAGGTG
GCCTTCCGCGACTGCGAGGGCCGTTACCTGGCGCCGTCGGGGCCCAGCGGCACGCTCAAGGCGGGCAAGGCCACCAAGGTGGGCAA
GGACGAGCTCTTTGCTCTGGAGCAGAGCTGCGCCCAGGTCGTGCTGCAGGCGGCCAACGAGAGGAACGTGTCCACGCGCCAGGGTA
TGGACCTGTCTGCCAATCAGGACGAGGAGACCGACCAGGAGACCTTCCAGCTGGAGATCGACCGCGACACCAAAAAGTGTGCCTTC
CGTACCCACACGGGCAAGTACTGGACGCTGACGGCCACCGGGGGCGTGCAGTCCACCGCCTCCAGCAAGAATGCCAGCTGCTACTT
TGACATCGAGTGGCGTGACCGGCGCATCACACTGAGGGCGTCCAATGGCAAGTTTGTGACCTCCAAGAAGAATGGCAGCTGGCCG
CCTCGGTGGAGACAGCAGGGGACTCAGAGCTCTTCCTCATGAAGCTCATCAACCGCCCCATCATCGTGTTCCGCGGGGAGCATGGC
TTCATCGGCTGCCGCAAGGTCACGGGCACCCTGGACGCCAACCGCTCCAGCTATGACGTCTTCCAGCTGGAGTTCAACGATGGCGC
CTACAACATCAAAGACTCCACAGGCAAATACTGGACGGTGGGCAGTGACTCCGCGGTCACCAGCAGCGGCGACACTCCTGTGGACT
TCTTCTTCGAGTTCTGCGACTATAACAAGGTGGCCATCAAGGTGGGCGGGCGCTACCTGAAGGGCGACCACGCAGGCGTCCTGAAG
GCCTCGGCGGAAACCGTGGACCCCGCCTCGCTCTGGGAGTACTAGGGCCGGCCCGTCCTTCCCCGCCCCTGCCCACATGGCGGCTC
CTGCCAACCCTCCCTGCTAACCCCTTCTCCGCCAGGTGGGCTCCAGGGCGGGAGGCAAGCCCCCTTGCCTTTCAAACTGGAAACCC
CAGAGAAAACGGTGCCCCCACCTGTCGCCCCTATGGACTCCCCACTCTCCCCTCCGCCCGGGTTCCCTACTCCCCTCGGGTCAGCG
GCTGCGGCCTGGCCCTGGGAGGGATTTCAGATGCCCCTGCCCTCTTGTCTGCCACGGGGCGAGTCTGGCACCTCTTTCTTCTGACC
TCAGACGGCTCTGAGCCTTATTTCTCTGGAAGCGGCTAAGGGACGGTTGGGGGCTGGGAGCCCTGGGCGTGTAGTGTAACTGGAAT
CTTTTGCCTCTCCCAGCCACCTCCTCCCAGCCCCCGCAGGAGAGCTGGGCACATGTCCCAAGCCTGTCAGTGGCCCTCCCTGGTGCA
CTGTCCCCGAAACCCCTGCTTGGGAAGGGAAGCTGTCGGGAGGGCTAGGACTGACCCTTGTGGTGTTTTTTTGGGTGGTGGCTGGA
AACAGCCCCTCTCCCACGTGGGAGAGGCTCAGCCTGGCTCCCTTCCCTGGAGCGGCAGGGCGTGACGGCCACAGGGTCTGCCCGCT

GCACGTTCTGCCAAGGTGGTGGTGGCGGGCGGGTAGGGGTGTGGGGGCCGTCTTCCTCCTGTCTCTTTCCTTTCACCCTAGCCTGA
CTGGAAGCAGAAAATGACCAAATCAGTATTTTTTTAATGAAATATTATTGCTGGAGGCGTCCCAGGCAAGCCTGGCTGTAGTAGC
GAGTGATCTGGCGGGGGGCGTCTCAGCACCCTCCCCAGGGGGTGCATCTCAGCCCCCTCTTTCCGTCCTTCCCGTCCAGCCCCAGC
CCTGGGCCTGGGCTGCCGACACCTGGGCCAGAGCCCCTGCTGTGATTGGTGCTCCCTGGGCCTCCCGGGTGGATGAAGCCAGGCGT
CGCCCCCTCCGGGAGCCCTGGGGTGAGCCGCCGGGGCCCCCCTGCTGCCAGCCTCCCCCGTCCCCAACATGCATCTCACTCTGGGT
GTCTTGGTCTTTTATTTTTTGTAAGTGTCATTTGTATAACTCTAAACGCCCATGATAGTAGCTTCAAACTGGAAATAGCGAAATAA
AATAACTCAGTCTGC

HUMAN SEQUENCE - CODING (SEQ ID NO: 6)
ATGACCGCCAACGGCACAGCCGAGGCGGTGCAGATCCAGTTCGGCCTCATCAACTGCGGCAACAAGTACCTGACGGCCGAGGCGTT
CGGGTTCAAGGTGAACGCGTCCGCCAGCAGCCTGAAGAAGCAGATCTGGACGCTGGAGCAGCCCCCTGACGAGGCGGGCAGCG
CGGCCGTGTGCCTGCGCAGCCACCTGGGCCGCTACCTGGCGGCGGACAAGGACGGCAACGTGACCTGCGAGCGCGAGGTGCCCGGT
CCCGACTGCCGTTTCCTCATCGTGGCGCACGACGACGGTCGCTGGTCGCTGCAGTCCGAGGCGCACCGGCGCTACTTCGGCGGCAC
CGAGGACCGCCTGTCCTGCTTCGCGCAGACGGTGTCCCCCGCCGAGAAGTGGAGCGTGCACATCGCCATGCACCCTCAGGTCAACA
TCTACAGTGTCACCCGTAAGCGCTACGCGCACCTGAGCGCGCGGCCGGCCGACGAGATCGCCGTGGACCGCGACGTGCCCTGGGGC
GTCGACTCGCTCATCACCCTCGCCTTCCAGGACCAGCGCTACAGCGTGCAGACCGCCGACCACCGCTTCCTGCGCCACGACGGGCG
CCTGGTGGCGCGCCCCGAGCCGGCCACTGGCTACACGCTGGAGTTCCGCTCCGGCAAGGTGGCCTTCCGCGACTGCGAGGGCCGTT
ACCTGGCGCCGTCGGGGCCCAGCGGCACGCTCAAGGCGGGCAAGGCCACCAAGGTGGGCAAGGACGAGCTCTTTGCTCTGGAGCAG
AGCTGCGCCCAGGTCGTGCTGCAGGCGGCCAACGAGAGGAACGTGTCCACGCGCCAGGGTATGGACCTGTCTGCCAATCAGGACGA
GGAGACCGACCAGGAGACCTTCCAGCTGGAGATCGACCGCGACACCAAAAAGTGTGCCTTCCGTACCCACACGGGCAAGTACTGGA
CGCTGACGGCCACCGGGGGCGTGCAGTCCACCGCCTCCAGCAAGAATGCCAGCTGCTACTTTGACATCGAGTGGCGTGACCGGCGC
ATCACACTGAGGGCGTCCAATGGCAAGTTTGTGACCTCCAAGAAGAATGGGCAGCTGGCCGCCTCGGTGGAGACAGCAGGGGACTC
AGAGCTCTTCCTCATGAAGCTCATCAACCGCCCCATCATCGTGTTCCGCGGGGAGCATGGCTTCATCGGCTGCCGCAAGGTCACGG
GCACCCTGGACGCCAACCGCTCCAGCTATGACGTCTTCCAGCTGGAGTTCAACGATGGCGCCTACAACATCAAAGACTCCACAGGC
AAATACTGGACGGTGGGCAGTGACTCCGCGGTCACCAGCAGCGGCGACACTCCTGTGGACTTCTTCTTCGAGTTCTGCGACTATAA
CAAGGTGGCCATCAAGGTGGGCGGGCGCTACCTGAAGGGCGACCACGCAGGCGTCCTGAAGGCCTCGGCGGAAACCGTGGACCCCG
CCTCGCTCTGGGAGTACTAG

Table 2 (mouse gene Fosb; human gene FOSB)

Mouse genomic sequence (SEQ ID NO: 7)

Mouse mRNA sequence (SEQ ID NO: 8)

Mouse coding sequence (SEQ ID NO: 9)

Human genomic sequence (SEQ ID NO: 10)

Human mRNA sequence (SEQ ID NO: 11)

Human coding sequence (SEQ ID NO: 12)

MOUSE SEQUENCE - GENOMIC (SEQ ID NO: 7)
CTCACTCCGCCAGTCTGAAGCATCCGGGCTCTGTCTGTGAAACAGTCCGCGAACTGGGAGGGTCTACCCACTGGCCCCAGCACCCC
TCCTCCCAAGGCCCCCCATGCTGCTGCTTCATGTGACCAGGGCTGGGCCGAATCTCTGAGCAGACAGAGAGAAGCATGGTGTGACC
GCATGAGTGGAGTGAGTTCTGGGGACAGTGCCCAGTTGTGCGATAGACAGCAGATTCCGGAGGACCACTGTGCATGTGTGTGTGTT
GTTGTATTTGGTTTGTGTTTGTGAGTATGTCTGTGTGTGTGTGTGTGTGAGAGAGAGAGAGAGAGAGAGAGAGAGAGACAGACA
GACAGACAGAGACAGAGACAGAGACAAAGAGAGTTGTGTGAGGACCACTGTGCATGTGTGTGTGTTGTGTGAATGTTTGTGAGTGT
GTGTGTTGTTGTATTTGGTTTGTGTTTGTGAGTGTATCTGTGTGTGTGAGAGAGAGACAGAGAGACAGAGAGAGAGAGAGAGAGAG
AGAGACAGACAGACAGACAGACAGACAGAGAGACAGACAGAGAGAGACAGAGAGAGACAGAGAGAGAGAGAGTTGTGTGAGGGC
CTCCCAGGTGATCTGGGAAGCTTGTGAATGTGAAAGTGCCTGTGGGTGGCTTCACCTCTGGCAGAGGCATTCTGCCTCCTGTATAT
TAACTATGTGTGCTCAGACAATCCACTCAAAGATGCTCTGGGGCTGCAGCTGATAGAGCAGCTTACTTAGTGTGCACAAGGCCGTG
GGTGCAAACACCCCAGAACTACAATCCCAGAACTCTCTCTGCGCACTGAACTCGACTTCCTCATTTGTTTCTTGTTTCTTTTTTTT
GACGGAGTCTAACATTGTCCGGATTGCTTCAAACTTTATAGCGGAGGAAGATCGATGGCAATTCTTCTGCCTCCACCTTCTGAGTG
CTAGGATTGCAGGCATGTACCCTCATGCCCAGTTGAGACAGTATCAGTGATTGAACCCAGGGCCTCACGCCTCCTGGAGAAGCCCT
GTACCATGGAGCTACAAGTCTAGTTCCTTTTCTTGTCCTTAAAAAAAATAAAAAGCTGGGTCTTCTTATAGCCCAGGCTGGCTTTG
TACTCCCCATCCTCCTGCCTCAGCCTCCCAAGTGCTGGGATGATAGGCTTATGGCACGGCCATGATAGCACCTACAATTTCCAGAG
TTGCCTGTCCTTTGCAGTGTACTATGCCTTGTGAGGGACAGGCTCTGAGGGTCTGCTTGTGATCACACACCTGAGGACCTGTTTGC
TGGGAGTTCTGTCCCCATTTCTTTGCCAGGCTGGAAGACACTGACACACCCACCATGGTGACAGTACCCATGGACTTAAATGGTGT
GACTCCCTCTGCTCTAATGGGAGGGTCTCAGTGCCCCGTATCTGCTGTTTCTCAAATGGGGTAGGTAATACAGAGATAGGAGGGCT
GACACAAAACATATCAAACACCAAGATAGCAAAGGCTAGAATGAGACCCTGTCTCAAGCTTTCCCATAGAGAAGGGGAGTGTATAA
TTGCACCCACTCACAAGACTGTTGGCCATAAACAAGTTTGTATGGAAAATGTTTACAAAGTGCTTGGCACATCCAGGGGTGGTGGC
GCACACCTGGATTCTCATACTTGGAGAGAAGAGCCAGGAAGATTAGGGACGGTTCCAGTCAGGCCTACATATTGAGACCTTATCTC
AAAAACAAAACAACACAACACACACACACACACACACACACACACACACACCTCAAAAACCTCTGGAAGTTCACATTTTGGGAAAGGC
ATCTGAATGGTAAAGTGTAGGGATTAAGAGTAGAGACTAGAGTCCCTCCATGGGACCGAGATGTAGAAAAGAAAGAAAAGAGAGCT
TGCTAAAGCAGGCCAGCCTAAGGGCAGACTCCTGAAAGGACTTGCTCCAAATGATTGTTGAGGGTGGAGGACTCTAAGAAACTGGA
ATTGTGGGAAAAACTCTCGGGGAATGACAAAAGCCGGTTTGCAGGTTGGTGAGATTAGAAAGTAATGGCCACAATATAGGGCACAA
TATACAGGCTGGTCTTGAACTTGTAATCCTCCTGCCTCAACCTCCCAACTATTATAGGTATATGTCACCAGCTGGATGCTTTTGCC
TGTCTTGTTTCTTTCTGTGCCCCAGGGCTTCAGTGAATAGGGATGGCTGCCGATTAAATAACTGAATTGCTAGCAGGGTGGGTGAG
TCAGGGCTTTAATCCCAGCCTTCAGGAGGCAGAGGCACACAGATCTTTGTGAGTTCGAGGCTAACCTGGTTAACAGAGAGAGTTCC
AGGACAGTCAGAGCTACACAGAGAAACCTTGGCTCAAAAAACAACAAAATATCTGAATGGCTGATTGAATGACTGGGCAGATGGAC
AGATAGACAGATGGACATGTACGATCCACAGACATGTACGATCTTTGGGATGGTGGGGAAGGCTTGGTAGCTAACACAGGCTGGCT
CAAGACCTGCTTCTGGGAACCCTTTCTTTCCTTTGTATCCTCAGCTCTGGGTCATGAAGGGTGTGGAGTGGGCCGGGAAGGGAGTG

TCAGGCTCAGTGGCTGCTTGCCTTGGATTCTGGCCCAGAGTGTCTGGAGGAAGAAGAGGGTATCTCGGCATCTGAGTCACCTTAGA
GAAACCCTAGCCACATACCTGACTTCTGACATCACACAACCAGGGCAACCCTGGTGGCCGAGACAAGGAGACTGAATCATGGACTG
AAATCAGTCACCTTGGGCATGGGCACGTGGCCGCTAGTGACCCCCTGAGACACCCCCGAGTGGCTGTCTGGCTGTGTGGGCATGCA
AACCACATCAAGGTCAGTAAAGGCTCTCCAGCCTGAGTCAGGGACAGGGTGGCCACTGCCAGAGACCACCGACCACAGCATCTGCA
GGACAGGAAGAGAAAGTTGAGGGAGAAAAGGCAGAGGGTAGGCCCCAGTCAGGGAGAGGGAAGTGCCTGTGGGAGAAAGGGGAAGC
TGTGGCTTGCGGCCAGTAGCCTGGTGACCTGTGGCTGGGATGTGGAATGGGGTCTTAGTGTTTGCGCACAGAATGGCGAAGTTGCA
AGCGAGGCTCAGAGGACAATGGAGTCAGCAGACAATGGGCTCTTACACTTGCACACGCAGGCACAGGCGCACAAAAGCCAGCGTGG
GAGCCCTACCCTCCAGCCACCCCAGAGCTACAGCTTGCCACACACACACAAGACTGTCCCCACAAGACTCGCTTCCTTTACCCTAT
TAAGTGACATCCGTAATTCTCATTGTGAAGATTGCAACTCTCGGTATTTTGATGATCTCATTTGATATGAATAACTTAGACATTTG
CAGTGCCAGGCACTGTTGTAGACTATTTAGCTGTGCTAGGGATTGAACCCAGGGCTTCTTGCATTCTGGGCAAACATCCTGCCACT
GAGCTTGCTCCCAGACCCACGTGCCCATGGTTTGTCCCCAGACCCCTGTTTTATATGTAATTCTGAGACAAGGTCTCACTGTAATG
CCCAGAGACCCTTGAGCTCACTCTATTACCCAGCCTTGAACTCACTGATCCTTCTGCAGGGATTGTAGGCATGACTAGCTAGACAC
AGCTAAGTATCTGTTAGAGCGTGTATGTGTACCTTGGGGCAGACATCTGTCCAACTGTATATGTCTATACATTGTTAGGTTTCAAA
CAAATGGCTGAGGTGCCTATTTAACATATCAAGAGGACCTGGCCCCCAATTTCTCCCTGCAATAACTCAGCTCCTATATGCCCTCC
TGGCTGGCATACCCCACCCGCTATGCTGAACTTCTCCAGCCCAGGGGCTGGGCTGTTCATCCCTATGTAATCCAACTATTTTAGCT
CTCCCCTCTCTTTGTACCTTAAGGCTGCACCTGGCTCCCATGGCTCCCCTCCCTCTCTCCCCACACGGCTCAGGGTTAAGTCGACT
CTGGACTATCCTAGAGGTCCCTGCCTCTGGCTATGCTCTCCCACATATCTATAATAAACTCTCCTCTGCATACCTAGGAACAGTCA
TGTTCCCTTCCTTTCTCTTTCTTAATTTCCTTTTCTTTTCATTCATACATACGTTTCTTTGGATTCTGTATTCTATTCTCCCTCCT
TAAAAATATACACAGCTAGCCAGGCATGGTGATGCACACCTTTAATCCCAGCACTCGAGAGGCAGACACAGGTGGATCTCTGTGAG
TTCGAGACCAGCCTAGTCTACAGATTGAGTTCCAGGCCTGTCTGGCCTATACAGTGAGATCCTCAAAAAGAAAAATGTACACACAC
TACCCCCTTCAAGTCAGAAGACCCTGAGACACAAGCCCATCCATCGCCGGGCAGCATGGGAAAAGCAGGTTGTTACAAATGCCTGG
AGTCAGGTCTGACACTCACACACAAGGTCCCTGCCACCAGTTGATCCAGCAGCTACATTCGAAGGATCAGAGAGGTCCAGATAACT
GTGCTGCCCTGTCAGACCTTATGAATGAAGACCTGAATGGAGGCTGATGTCTGTAATTCCAGCACTCGGGAGGCACTCAGGAGGCA
AGGAGGATTGGAGGGAGTTAGCTGGAGGCCAGCCTATGCTATAGAGTAAGACTGCTTCAAACAAGAGAAATGAGAACTGAAAAGAT
TGCTCAGTGGGTAGAGGCGCTTGCCTGCAAGTCTAAGGAACTGAGTTCAATTCCTGACATTTGCATGATGGAAGAAAAGAAACTCC
TTCAAGGTCACACACACACACACACACACACTCAAAGAAGTAAAAATACTTTTTAATTTAAAAGAACAGAGAAACTTGAACACATG
GTCGGTCTGCGATGTGGCTCTGCAGTAATGCCTGCCTTGGATGTATGTAGTCCTGGTTTTGACCCCACCATTATACAGACCAGGTG
TGGTGATACAAACCTGTAATTCCAGCACTGGGGGCTGGAGCCAAGACGATGGGAGAAGTTCTAGGTGGCTCATCCTTGGCTAAAAA
AACATAGCCTGAGCTACCTGACACTGGTTGCTAGTGTGAGCTATCTGAGACTCTGCCTCACAAATAATTAAGTAAAAATATGGTAG
ATTCATGACGACAGCACCTACAGATTGCCTGTCCTGTGCTGGGACACAGGGCAGCTTCACACTAACCCTGATGGGAACACCTGGGA
GACGACATGCTTGTTGCAAGGCGCTGGCTCATCTGAGACCAAAGCGACTTCAGGAGCCAAGTAGACCAGAATCCGTGTGCTTCAGA
CAGTGGGAAGGGCATACAGAAACCTCAGGAACTGAGGCACAGCAGGGAGATGGGAGGCTGGGACAAGGGACCACTGACAGACACCA
TACATCATACCATCTTGTCCCTCTGAATCTCCATGGGCACCCCCTCTGGGAGGACTCCAGGACTGCAAGCAAAGGTTAACTGAGTC
AGCCAATTCAACCCTGGCCTAGGGGGTGGAGGCAGGGGGTCTATCAGCGCGAGGAGACAGGACAAGGACACTCATGTATTGCCATA
GGGATGGCTCTCTTGTCAAACCATCAGAACACCCACAAACATCCCGGCTCACAGGGGTCTGCAGGGGTAGGGCTGGGATGTGGCCC
GAGACTTGTGGGAACTGAGGAATGTAGTGGCAAAAGTTTGGCCAAGGGAAGACCAGAGGACTCTGTATGCATGAAGGCCAGGGTGA
TGACATTTCAGGAAAGAAAAGCACAGAATCAAGCATGGTAGGTAGTTGGAAAGTGGAAGCGGGNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNACCACATTTACTAAGCCCCTCCCTGCCAAAGTAAGCAATAAAT
TATGGCTAAGAGTCTCCCCACTCCTAAGATGGAAAGATGCCAAGCTGCAAAGGAGATCCTGACAACAGACCAGCTACTTGGAAGAC
TCAGAACCTAGCCAAGCTGCCTGCAAGAGGTTTAGACCAACTAAGACATCTGGAGAGAGCACCCTCCAATCTGTGGAGCAGTCTTC
AGGCTCTGCAGGGTGCTCCAGGCTCCCAGCTTTGTAAGCTGTCACTCATGCTGGGGCGGGCTTGGGTGACGCAGCTGTCTTTGAGT
GACTTCTCCCGTAAGTCACCCCTCACCCATGCACACTCCTGTAAGTAACTCTAATAGAAAATTCATCGGTTCAGCAAATTGGACTT
TGGTCTCTTCTGAGCTGGCTCCCTGTCTAGGGTGAATGGACATGTGTGTGTGGCATCTCCCCTGGAAAAGTTTTCTTTCCCTAGGC
TTGGCCTGGGGTCTCCCAGGCTTTTTGCAGTTGCTGGTGGCATCCTTCTTAGTGGGTACTTAAGAATCAGGACCCTGGCCAGCGAT
GCAGCAGCCGTTGGCAGAGGGCTCCCCTAGCATGCAGGAGGCCCCTGGATTACATCCCCAGTAATAAACCAGGCCTGGCGACAATA
TAGAGGTGAGAGCAAGATGTTCATTGTCATCCTTGGGTACATAGTAAATTCTAGTCTTTGTTTTAAAAACAAACAAACAAACAAAC
AAGCAGGACATAAGATCAGATATGCCTGGCATGGTGGAAGGTGCCTTTAACTCCAACACTCAAGAGGTAGAGGTCATCCTGGCCTA
CAGAATGAATTCCAGGCCAAAGGTGGTACATAATGAGACCCCATCTCAAAAACAGACAGAGAAAGGAGGAGGCAAAGGGTAGAAA
GAGGAGGGGATGTTCACAATAGGGGGAGGGGATGTTCACAATACGTGTATATATATTCTTTCCTTCTGGATGTGGATAGAACCCAG
GACTTCACACATGCTAGACATTCATTCCACTTTAAGCTACATTCCCGCCCTCACATCCCCTTTTGTTTAAATCATTAATATTGATA
TTATTATTATTATTATTATTATTATTATTACTACATATGAGTACTGGTTGTTTTCTGTCTTTGCATGCATGTGGAGATCAGAGAAC
AATTTTTAGGAGTCATTTTTCTTCTTCCACCGTGAGTCCCAGGGATGGGACTCAGGTTGTCAGGTATGTGTAGCAAGCTCCTTTAC
CTGTGAACCATCTTGCTGGGGCTGATACCTGCTGATTGCTAAACACATGCCACCGATTATATTAATGCACTAGTATTTTATTTATT
TATTTTTCGAGACAGGGTTTCTCTGTGTAGCCCTGGCTGTCCTGGAACTCACTCTGTAGAACAAGCTGGTTTCGAACTCACAGAGA
TCTCCCTGCTTCTGCTGGGATTAAAGATGTGTGCTGCTGCCGCAGCTGCCACCACCACCAGACACCACCAATTATTATTGTTCAAT
TATTATTGTATACACACTTTCTTTTCTGTCTCCTGAGCTAAGTGGGAAGGAGAGGCTTCCAACACCATCAAGCCTGAAGAACTCAC
CGGGTGTGGGTTCCTACCTGCTCCAATTCTGCAGGTCCCCCAGATTCAGGGACCCGGAAAAGCCACCTATCCTTTGCTTAGCTATG
AGTACTGAAGTTTTAGATCAGGGAGGGAGCACATTTTCCAAGCTCACCGAGTCACACAGAATCCGTGACAAAGCTAGTGGGGAGGG
GACTGCATCCTTACCCATCCCTACACCTGGGATATCACAGACTCTCCAATTCCCCCTGAGCCTCAGTTTCCCCACCTAGACTGAT

TATACCCCTCTCGCCAGAATCTGCACTGGGGACCTTGCTCTTTTTCACCAGCAGGGGCGCTGCACCGGTTTGGGGAGGGTGGGGTC
CCAGGGGTATAAGCAGACCTGGGATCTGGAGTTGCACCTTCTCCAACCCGGGTCAGCAGGGGCCTTCTGAGGGAGTTTAGGCGCCT
GTCAATCTCAGCCTCCGGGACAGCGTGGAACTGCGCAGGCGCGGGCGGGTTCCGCACAGCGCAACAGCGGGCGCGCGCGGGAGCGA
GGGATTCCCTCTGACGTAATTGCTAGGATACCAAACAAACACTGGGCCGCGCTGGCCGAGCTCCTTATATGGCTAATTGCGTCACA
GGAACTCCGGGGAGGGCGGGGCGGGATCCCCTCCCGCGAAGCCCCTCAGAACGCAGCCTTGGGGACCCCCGAGACCCCCAGGGTCA
CACTATGGGCAGGTGGCAGGTGCACGAGGCCCTGGAGGGCGCCACAAGGAGCTCAGTCGGCGGGAAGGGAGAGTTTGGGAGGTTTG
TGCATGGAGTTGCGGGTGACGCAAGCGCGGGGGGCGGGTCCCGGAGGCATAAATTCAGCCCGGCAGCTCCCGGTTTCATTCATAAG
ACTGGAGCGGCTACGCCGGGGACACGCGGAGCCGGGGCTTGCTGGACTTGACTACTGGTGACTCTTTCTTTTTCTTCTTCTTGGAG
GCCGTGAAAAATTTATATATATATATTTATATATATTTTTAAACTGGAGAGAAAGTTTTGTGGGTTTCCTTTTTGCAAGACTCTTT
CTAGATCGTTCTTCCGATTCGTCCGGAGGATAGACTTTCTTTCTGTGGGCTTCTGGATCGCGCCCCTCTGCTCTCCCTCATTTCTT
GGGACGCTTCGACAGGATTGGAACTGCATCTGAAGCGCACGCTGCACCGCGGCACTGCCCGGCGGGTTTCTGGGCGGGGAGCGATC
CCCGCGTCGCCCCCCGTGAAACCGACAGAGCCTGGACTTTCAGGAGGTACAGCGGCGGTCTGAAGGGGATCTGGGATCTTGCAGAG
GGAACTTGCATCGAAACTTGGGCAGTTCTCCGAACCGGAGACTAAGCTTCCCCGAGCAGCGCACTTTGGAGACGTGTCCGGTCTAC
TCCGGACTCGCATCTCATTCCACTCGGCCATAGCCTTGGCTTCCCGGCGACCTCAGCGTGGTCACAGGGGCCCCCCTGTGCCCAGG
GAAATGTTTCAAGCTTTTCCCGGAGACTACGACTCCGGCTCCCGGTGTAGCTCATCACCCTCCGCCGAGTCTCAGTACCTGTCTTC
GGTGGACTCCTTCGGCAGTCCACCCACCGCCGCCGCCTCCCAGGTAAGTTCTAGATCGTAAAGATTCTACTTTAGTGGTTGGGGGG
GGGTGTTTCTTTCAGGCTAAAGTGTAGATTGAGCATCCTCTCAAATAGAGACGCGCCAAAACCCAGGATCTGGGATTGCAATAGTT
CGGTTTGCACTTTGGTTTTTTGTTGTTTGCAAACTGCAAAGAATGGAGTGATGTTTGCAAAAGGTTATTTGCGCGGAGCGCGGGAA
AGGAATGCAGCTGGGCAAACGTTGGCGATGCCCGGTGCAAAGTATATACCCGGTGGTTAGCAGAAGCTGAGAACTTTTAGCCGAAA
GCCGGCTCCCTAAGCCGAAGCTAGGCAAGTAGGGGAAGAAAAAGAAACAAAAAATTCCAGAGAAGCTTCCAGGAGCCTCCTCCTCT
TCCCTCTTCCTTCAAAACGCGGACTGCAAGTCCGCAGTCACCCTCCACCCAGCAAGAGTTAGGGCCTCGAACCCCGGTCACGCTGC
CTCCGCCTCCTGCGCGGAGACGTAACGGGGGACCCGTGCGTAAAGGCTGACGCGCTGGAATCCTCCGTCTGACGCGGGGCACGCAC
AGCGCGCAGCGCCCCCTCCGCCCGCCCCGCCCCTGACGTCCCGGGCACGTTCTATTTTGGAACGCCGAGGCCACGTTGCTAAGGGA
GGGGGCAGCGTGGCTTTGTGATTGGCTGTCGCGGCGCGAGCTTTAGCCAATCAGCGTTCCCTTCCTATTTGTAGAGCGTAGCTCCC
TTCCTTGCTTTTTGTGGTTCTTCCCGTGCTGGGGGTCTCCAAGAGGGAGAGCTAGGGGATTCTTGTCGCGATCGGGGGACTCGTTG
TCACCCCATGGGTCTGCGAGGACCTTGTGTGGACCTGGTCCTGTTGTCATAAGCTAGAGGCTTTTGGCTGAGTGTTAGCGCCTCTA
AGGGGGAACTGAAGGCCTCATCCCTTCTCCAGGCACACATATACGTGCTCCCTGAGCTCTAGACACTCAGTCCTTCCGAGGTGTTC
AAACACTAGATGAGCTAGCCTACGGAGAGGCAGCCAGGTGGTCTCTAAAAGGTCCGCCTTCCCTTAGTTCCCAGGGCTCTGATTGG
CCAGGGATTCAGCCCTTCCCTCGCCACGCCCCCTAGAGTAGTTAAGCCTCTAGGATTCCACTTGCGGGAAGGGGGGGGGGGGCGTG
ATGGACGCTTCTTGGGTTCGGGGACGCAGATCCTATGTCACCCCATCCCCTGCAAGACAGTCTGAGAGATTCTCGCTGTCACTTTT
CTCTGCCTATCAGTTCACTGAAACCTGTCAGTCTCACTGGGGAAGAGACAGACACTCGGAAGGGATGCTCTCAACTCTTAGGCCGG
TCCCCCAACACCGTTGGAACTGGGATCTCCGCCCCTGCGGGAGCCCTCATGCAGTGGGGGGTGTGTTTGTGTGTGAGTGGAGGAGA
GGAAGGCTTGGGCTAAGGCCTCTCCCTCTCCCTACCTACTGTGGTGGGGGTGGGGTGTTTTGGCTGTATGTGTGTGTGANNNNNNN
NNNNNNNNNNNNNCTGACTTGAAGGGGGTAGTGTGTGTACATTTTTCTTTTTGAGACAGGATCTCACTGTATAGGCCAGACAGGCC
TGGAACTCAATCTGTAGACTAGGCTGGTCTCGAACTCACAGAGATCCACCTGTGTCTGTGGCTCCAGTCCCGGGATTAAAGGTTTG
CATCACCAGGTTCTGTCCAGCCTCCGTCTCCCACCCACCCCCCCACACCTAAGAGTCACCAACCCGGGGTGTGATTCACCACCCGC
TGGAACCGTGCAACCTTTCCCCGAGGAAGAAGGAGGAGGTAGAAGGCAGTTGAACAGAATCTCTCATTAACCACTGCGTCACGGTG
TAGTGGAAGGGTGGGTGTTGTGGCTTTTTGCCTGTGACACACACATCCACACCCGCTCACCCTGTGCTCACTCACAGGGGTCGGTG
TGTGTTATGTGTGTTGGGCGTGTGTGTGTCGGTGGCTTTGTTTGTGTGTCTACGCCTGTGTGTGTATGTCTCACCCCGTAGGAGTG
CGCCGGTCTCGGGGAAATGCCCGGCTCCTTCGTGCCAACGGTCACCGCAATCACAACCAGCCAGGATCTTCAGTGGCTCGTGCAAC
CCACCCTCATCTCTTCCATGGCCCAGTCCCAGGGGCAGCCACTGGCCTCCCAGCCTCCAGCTGTTGACCCTTATGACATGCCAGGA
ACCAGCTACTCAACCCCAGGCCTGAGTGCCTACAGCACTGGCGGGGCAAGCGGAAGTGGTGGGCCTTCAACCAGCACAACCACCAG
TGGACCTGTGTCTGCCCGTCCAGCCAGAGCCAGGCCTAGAAGACCCCGAGAAGAGACAGTAAGTATGAGGCCTCAGGAGTTGGGAT
GGAGGAGCCTAGCTAGGGATGTGGGCTCAGTTTGTACAGTGCTTGCTGCCATGCATGAAGATCCCTAGCACAGCATAAGCCAGGAG
TGGTTATGCAGACCTGTAACCCCAGCTCTCAGAAGGTGGAGGCAGGAGGAGCAGGAGTTCGAGGCCAGCCTGTGCTACTTATGGAG
TCCAGCCTGCACTGCAAGAGATCATTATTTTCAAAAGTTGGCCTTGGGGGGAGGTGGGTGAGGGAAGTAAGAGAAAGTGACAGTAA
TTTTATCACTTAATAGTTGGAGGTTCCTCTGAGGCCTCAAGTCTGAAGGAACTTTACCATTCTGGCCAGTGAGGAGTAGGGGTTAT
TATTTGGGGTTCAGGAGGAAGGAGTTTTCTTAGGGCTGATAGAGGTACCCCCAGATCTCATGGTCCTTATCTCTGACTCAGCTTAC
CCCAGAAGAAGAAGAAAAGCGAAGGGTTCGCAGAGAGCGGAACAAGCTGGCTGCAGCTAAGTGCAGGAACCGTCGGAGGGAGCTGA
CAGATCGACTTCAGGCGGTAAGGAGGAGTCTGGGGGTGTCTTGAGGCCGTGCTGGGAGCACTCTGCCTTGTTCTTCCCCCGTTTCT
CACTGTGCCTGTGTCCTAAACGAGGAAACCCCCTCTTAGGGAACAGGGGTCAGTATAGGCTGATGGAGTGGCTCCATATGCATGCT
CAGACCCATGCCCACTTACTTTCGACTGTTCCCCACTTTCCCTGAATATGTCCCCACATGTCACCCTCCTGGCTTTCTCTCAGCCT
AAGGAGACAAGCTGGAGGAGGTAATTCTCTCACCTTCTTTCCTTCACTAAATAATAATCCATTTTGCCTTCCTGCCTCCATTTTTT
TTTCCTGAGCTGGGGATCTACCTGTGTAGTTCAGCCCTCCTCCCCCAACTTGATAGCCTCAAGTTTCAGCCCTTGGCTGAGATGCC
ATCATCCTGACTGGCTCTGGCTGGAAACTATTTTGTGCTAAGTCAATTCCTTACCTGCTACTCCAGCTATCTACAGTTCTGCCGAA
CTTGAGCTGGTGGCGCCCACCAAGCCCACTTCTTTCTCTCTTCTACCTCAGTGCAACCCCCACACACACACACACACCTCATGCCT
GCCCCTTGAAACCAGGGTGTGTCTCTGATTTCCCGTCGGGAGGCTGAAGGAGATGGGTAACAGAACCTCATTAAAAACAACACATA
AGCATTACCTACTGACTCAACAAACTGTAGTGTTTTTCTTTTTTCCTCTCAAAAAATTATTTCGTTTGTTTATTTATTATTTGCTT
ATGTTTGAGTGAGTGCTGGTGCACCACAGCACACATACGAGGTCAGAGGGAAATTTTCATAGTTTGTTCTCTCCTTCCGTGTTGTG
GGTGCTTGCTGGCAATCTCCTTCACTCAGTGAGCTACAATGCCCCCTTCTGCCCTTTAAGGCAGAGTACTCCTTAGTACAGGGGGA
CCCTTTCCTCGGCCTCTCAAAGTTGAGATTACAAATGTTCACCATCACACCAGGCTTGGAGTTCTTGCCTATCAGTGACGTCCACT
CCTGCCTAGCTTCTTCCCAACCATCTCTTAGTCTGATGGGGAAACCGAGGCACGAGTAGCATGGTCTACCAGGATTTCCTCTTAGG
GGACGGTCCCCTCAGTTGGGAGGGAGCTGTCCAGCCCCCTGGATCAGCAGCAAGAATGTATGAGTGTGGGGTTGGGCGGGTGAAGC
TACTCTGTGTGGTCGCTGACCAGCAATTCTCCTTTCTCTGTCTCCTATGACCTGGCCCTGCTGGGATCCATTAGGAAACTGATCAG
CTTGAAGAGGAAAAGGCAGAGCTGGAGTCGGAGATCGCCGAGCTGCAAAAAGAGAAGGAACGCCTGGAGTTTGTCCTGGTGGCCCA
CAAACCGGGCTGCAAGATCCCCTACGAAGAGGGGCCGGGGCCAGGCCCGCTGGCCGAGGTGAGAGATTTGCCAGGGTCAACATCCG
CTAAGGAAGACGGCTTCGGCTGGCTGCTGCCGCCCCCTCCACCACCGCCCCTGCCCTTCCAGAGCAGCCGAGACGCACCCCCCAAC
CTGACGGCTTCTCTCTTTACACACAGTGAAGTTCAAGTCCTCGGCGACCCCTTCCCCGTTGTTAGCCCTTCGTACACTTCCTCGTT
TGTCCTCACCTGCCCGGAGGTCTCCGCGTTCGCCGGCGCCCAACGCACCAGCGGCAGCGAGCAGCCGTCCGACCCGCTGAACTCGC
CCTCCCTTCTTGCTCTGTAAACTCTTTAGACAAACAAAACAAACAAACCCGCAAGGAACAAGGAGGAGGAAGATGAGGAGGAGAGG
GGAGGAAGCAGTCCGGGGGTGTGTGTGTGGACCCTTTGACTCTTCTGTCTGACCACCTGCCGCCTCTGCCATCGGACATGACGGAA
GGACCTCCTTTGTGTTTTGTGCTCTGTCTCTGGTTTTCTGTGCCCCGGCGAGACCGGAGAGCTGGTGACTTTGGGGACAGGGGGTG
GGGCGGGGATGGACACCCCTCCTGCATATCTTTGTCCTGTTACTTCAACCCAACTTCTGGGGATAGATGGCTGGCTGGGTGGGTAG
GGTGGGGTGCAACGCCCACCTTTGGCGTCTTGCGTGAGGCTGGAGGGGAAAGGGTGCTGAGTGTGGGGTGCAGGGTGGGTTGAGGT
CGAGCTGGCATGCACCTCCAGAGAGACCCAACGAGGAAATGACAGCACCGTCCTGTCCTTCTTTCCCCCACCCACCCATCCACCC
TCAAGGGTGCAGGGTGACCAAGATAGCTCTGTTTTGCTCCCTCGGGCCTTAGCTGATTAACTTAACATTTCCAAGAGGTTACAACC

TCCTCCGGGACGAATTGAGCCCCCGACTGAGGGAAGTCGATGCCCCCTTTGGGAGTCTGCTAACCCCACTTCCCGCTGATTCCAAA
ATGTGAACCCCTATCTGATTGCTCAGTCTTGCCCTCCTGGGAAAACTGGCTCAGGTTGGATTTTTTTCCTCATCTGCTACAGAGCC
CCCTCCCAACTCAGGCCCGCTCCCACCCCTGTGCAGTATTATGCTACGTCCCTCTCACCCTCACCCCCACCCCAGGCGCCCTTGGC
CGTCCTCGTTGGGCCTTACTGGTTTTGGGCAGCAGGGGGCGCTGCGACGCCCATCTTGCTGGAGCGCTTTATACTGTGAATGAGTG
GTCGGATTGCTGGGTGCGCCGGATGGGATTGACCCCCAGCCCTCCAAAACTTTCCCTGGGCCTCCCCTTCTTCCACTTGCTTCCTC
CCTCCCCTTGACAGGGAGTTAGACTCGAAAGGATGACCACGACGCATCCCGGTGGCCTTCTTGCTCAGGCCCCAGACTTTTTCTCT
TTAAGTCCTTCGCCTTCCCCAGCCTAGGACGCCAACTTCTCCCCACCCTGGGAGCCCCGCATCCTCTCACAGAGGTCGAGGCAATT
TTCAGAGAAGTTTTCAGGGCTGAGGCTTTGGCTCCCCTATCCTCGATATTTGAATCCCCAAATATTTTTGGACTAGCATACTTAAG
AGGGGGCTGAGTTCCCACTATCCCACTCCATCCAATTCCTTCAGTCCCAAAGACGAGTTCTGTCCCTTCCCTCCAGCTTTCACCTC
GTGAGAATCCCACGAGTCAGATTTCTATTTTTTAATATTGGGAGATGGGCCCTACCGCCCGTCCCCCGTGCTGCATGGAACATTC
CATACCCTGTCCTGGGCCCTAGGTTCCAAACCTAATCCCAAACCCCACCCCCAGCTATTTATCCCTTTCCTGGTTCCCAAAAAGCA
CTTATATCTATTATGTATAAATAAATATATTATATATGAGTGTGCGTGTGTGTGCGTGTGCGTGCGTGCGTGCGTGCGAGCTTCCT
TGTTTTCAAGTGTGCTGTGGAGTTCAAAATCGCTTCTGGGGATTTGAGTCAGACTTTCTGGCTGTCCCTTTTTGTCACTTTTTTGT
TGTTGTCTCGGCTCCTCTGGCTGTTGGAGACAGTCCCGGCCTCTCCCTTTATCCTTTCTCAAGTCTGTCTCGCTCAGACCACTTCC
AACATGTCTCCACTCTCAATGACTCTGATCTCCGGTCTGTCTGTTAATTCTGGATTTGTCGGGGACATGCAATTTTACTTCTGTAA
GTAAGTGTGACTGGGTGGTAGATTTTTTACAATCTATATCGTTGAGAATTCTGGGTGGAAATGTCTGATCAGGAGAAGGGCCTGCC
ACTGCCGACCACAATTCATTGACTCCATAGCCCTCACCCAGGCTGTATTTGTGATTTTTTTCATTTTGTTTTTTTGTATTTTGCAC
CTGACCCCGGGGGTGCTGGGGCAGTCTAGCACTGGGCAGCTCCCCTCCCCCCCTTGGTTCTGCACTGTCGCCAATAAAAAGCTTTT
AAAAAACTGTATTCTTCAGGTCAAAGTGTCTGTTTTCCCTGGACATCTACTACATGGCTTCCTTTCAGAAAAACGGAGTTTGGATT
GCTAGGGAGGTCTTGCTGGCACTTAGTGGGACGCCTAATGAATCAGAACCTACAACGGGACTAAAAGGAAGTGGAGACTTGCTAGG
TTTTCCCATGTTCCCAGGCTGGGCCACCTACTTGAAAAAATAAGGGGCGGAAAAGTGTAAGGTACAAATTTGGTGAAGGGTCTGGA
AGACTTCATGATCGGAAAAGAATTTATTCACCTTGGGTGTGCAATGACTTCAGCAACAGCTAAGGGCAAGGTGTAAAAGCTGGGCA
CACTTGTAAATCCTAGCATTTGAGAGGTGGAGGCAAGGGGATCACTGGTGGAGTTCAGTGTCATGTGGATCGTAGATACCAAGCGC
AAAGATCTGCTATGGGGAGAGGGCTTGGTACACCAGGGGAGCCAGAAGTTCGTGGTGAGGGTAGTGGAGGGCAGGTGGAGAGTGAG
AGTTAGCCTCAGGGAGATTCTACAGGCAATGATGCAGAGTTCAGACGCTCCTTTGAAAGCACTAGAGAGCCGCAGCAGGTTTTGAG
CAGAGAAGGTTAGCGTTAGGTGGTCTCTTCTAGCCCATCCCAGGCTGAGGAGGACGCTGAGGGTTTCAAGAAGGATCGAGAATGGA
AAGCAGAGGAGAAAAAGGATCCAAGAGGCATGGAGGAGGCAGAACACATTTCTCTTCTTTAATAGCAAGCCTGGAAAGGATAACTT
GCTGCAGGAGGAGATGCTCACCAGTCGGGTGGTCTAGGGGGTTCTTGGAAAAGAGAAGGCATTTGCTCAAGCCTCGGTTCCCCCAT
TCTCGCTCTTCTGTCAGCTTGTCTTCCATTAAGTGTGTGTCTCAAGGCCACCCTGCTCAGGACTCCTTGTGAGACGACCTTCTATG
CTCGAGTTCATTAAAAACACAATTGCCTGGTGCCCTGCTCTCTCCACTGGCTCAGTTACCTCAAAAGACCAGGGCTAAAGGTGTGA
TCACAACTCTATCCCCATTACTGCTCCAACGCAGAGACAGGACTGAGCCGGAGTGAACAAATGAACAAAAATGACTAATAATGCAT
GCGTGATTAAATACATAAAAGAGCAGATGACTGGATGAGCAAATCGTTTAAGGAGAGACAGCAAGATCCTAGAATTTTGGAGACTA
ATTTAAATCCATCTTTGAGATGTATTTGGTCGGAAATTCCTGGGAGGAAAAAAAGTGTAAGTATGAAGAGAGAATAAATGAGAATA
GGGGTGGCTTCAGAGAGGTTAACTGCGCGCTGGTCGCTTTTGTACAAGAATGTGAATTGCAGGGAGCAAAATGGGATAGATACTCC
CGCCCGAAAGGTGGAATTGAACCACTCTGTCGCTAAACAGCTACAGGTTTGAAGCCTGCACCCCAGACCACTGAGGATCATCCGGG
CGAAAGGAGCTATTTTCGGTTAGTTATATAAAGGCCAGATACTACTACTTTTTTCACTTATGGTCATTATTTGTGGTATACAGTAG
ATAATTAATTTCAATGGTTTCGAACATTTTTTTTCACTTTTTCTTGTGAACATGTGTTTCCTCAGTAAAGTGTTCCGTGAATGACT
CTACTAACTAAAAAGTAAGTAGCTTCATTTGCATAGCGCCTTGCATTCTGGGAAGCAGCGCCTAAAGTGCCTGTCTCCCTAACTAA
AAGCAGAATTTTTTGCAAAGTGAAAAGTCAGTTTTATTTTTGTTTGTTTGTTTGTTTGTTTTTAATGGAAAAACTTCTCACG
CGGCCCATTCGTAGCAGAATTCGAGATTTTCTGCAAGCGAGACCGTAGGGTCTGACGGCACGCGGCCGCAGAGCGACACCTGCCGT
TGCTTTATAGAACTGCAAGTATGTAGGGAATCTACTGAGTCCCTAAGGTGATGGAGTTGACAACCAACTCCCCTTGCGTTTAGACG
CTAAAAACCATCCCTTTTTATATCTATGTGATTAGCCCAGGGAAACTAAGGCTCAGACATGGATAATACCACAGCCGAGTTCCTGT
AGCCCAACTCCCTAGGGGAAATGAAACCTACAGTTGTGGTTTTAATATGCTTGGCCCAGGGGCAGTGGCCCTATTGGCAGGAGTGG
CCTTATTAGAGGAGGTGTACCTTGTTAGAGGAAGTGTGTCACTTGGAGGCGAGGTTTTGAGGTACGTATGCTCAAGTCTGGCCAGT
GTGATCCTGGCTGTCTGCAGAACGCGGTCTCCTGGCTGCCTTCGGATCAAGGTGTAGAACTCTCAGCTCCTTCTCCAGCACCATGT
CTGCCTGCTTAATGCTTTGCTTCTTTCCATGACGATAATGAACTGTGCCTCTGAAACTGTAAGTCAACCCCAATTACACAATTACA
TGTTTTCTTTTATAAGAGTTGCATATATATATATATATGTCACACTCTTTAGCTCTGGCTGGCCTGAAATTCACTATGTAGCCCAG
GATTGCCTGAACTTTGAAGCAATCTTCCTGCCTCAGCCTCCCAATGGTATTACAGGCATGAGTCACAACAAGCCATTTAAATCTTA
TGATGACTTATAAGAAGACAGAAAATCAGAGTTCCTTTACCTAGTTCACAGATCCCTACAATCTAACCTCGTTCGCTCCATAAACA
GCCCTACCCCACCCTCCTGGAACTGCTTTGAGGAATGCTGCAGGCTCTCACAGGCACACTCCTCCTTGGTTAATCTCTTCAGCCTG
GTTGCCTTCCCTCCCCATGTCCATGTGGCCCAAAGCCTCTCATCCTGTTCTCAAATACCACTAGCTAGTAAGGCCTCCCCGACCTG
ACCCCGGTTTAAATATTAGAAAAGGGTCACTTTCTCCCCTGCCACAGACAACCAAACCACCATATGCTTGTCACTTACTACCTGAC
TATGAAGGTGAATAGATGTCTTCACAACCTTTCTCTGAGCCTCAGTTTCCCCACCTGCATAATGCATCTGAGACACAGAATTCCCT
AGAGCTGTGGTTCTCCTCATTCCTAGTGCTGGGACCCTTTAATACATTTCCTCATGTTGTGGTGACTCCACCACCACCATAAAATT
ATTTTCATTGATACTTCATAACTGTAATTTTTTCTATTGTTATGAATAGTAATGTAAGCATTTGTGTTTCCCAGTGATCTTAGATG
ACCCTGTGGAAGAGTCATTCCACCCCCAAAGGGGTCCCCACCACAAGTTAAGAATTCCTGCCATAGAGGAATCACAAGGAACCAAT
GAATTACACTTTGGGTCGACTTTTGGGCTGGCCTCTGGGAGGCGNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN

NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNCTTGGATTACAAACATGTGCCACCAAGTCTGGCTGCCCATTATGCATTTCTGTGGCAGGCGTTGATGGCTATTAAACCCAA
GCCTTGTATATACTAGGCAAGTACCCATCCACTGAACTACAGCCCTAGCATGGACCTCACAGGGCAATGGCCTGACATCTGGGAAA
CACTATCACAGAGTATAATAATAAAACCGTAGCATGCTTTTCAGATTGAGCATTTCTGCTGGGTCACACTTGGAAAACCTTCTTAT
TGTTATCATGAGTATGTGTGCATACATGTGGAGGTGAGAGGATGACGTCATAGAGACAGCTCTGTCTCCCATTCTTTATTGCCAAT
ATTATTTTATGTTTTGTCTGCGTGTATGTTTGTATACCAAAGTGCATGCAGTGCCTGTAGAGACCAAAAGGGGGCATCCCTGAGAC
TGGAGTTAGAAGTGGCTGTGAACCACTCACTCTGTGGGTGCTGGGAATCAAACCCCGGTTCTCTGGCAGAGCAACTGGTGCTCTTA
ACCACCGAATCAACTTCTCCAGCCCTGCATTGCTTTAATCTTTTGGTCGATTCCCCCAATCCAACTCATACCGCCAAGTCTGAGTA
GCAAGTGCCTTTATCTGGGAGACTCACTGATCTTATTTTTTTTCCTTTCTTTTTCTTTCTTTTTTTCTTTTTTTTTTTGAGTCAG
GTATGATTATTTAGATGGCCTCGAACTCACAGAGATCCACCTCCCTCTGTTATGCCAGGCTTTTAAAAAACAACATTTATTTTTGA
ATGCATATATAGGGCTGTGCAAACACAGCCTGAAGGAGTCGTTTCTGATTGCTCACAACGTGGTTCCAGGGATGGAACGCGGGTGA
CCAGCTTGAAGGTAGGTGCTTTCACCAGCCCCAAGAATTTATTTTTTAACTTTATTTTTTTAATTAAATGATTAAATGACACATGT
TCATTGTGGGTAGTGCAGGTATGCGATGGAGCACATGTAGAGGTGACATTACTGCAGGCGGTCTCTCTACCATGGGGATTCAACTC
AGACCACTGCTTCCTTTACCAGATCATCATTCTTGCATCCCCCAATCATTTCTTTTTGTTGATGGTTTTGTTTGTTTGTTTTAGTT
TTGGGGGGTTTTTAAAGTTTTTTTCGAGACAGNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNATTAAAGGCGTGTACTACCACTGCCTGGGCATAACCGGCCTTTCTGCCTTATTT
GTTTCGAAACGGAGTCTCATGTAGCCTGAGGTGGCCTCAAACATGCAATGTAGCTGCAGCTGGGATTACATATGTTCACCACCACA
ACCTGTTTTATGCACTGCTAAGAGATTAAACCTGGGCCTTCGTGCTAGGTAAGGACTCTATTAACAGAGCTACAACCTCTGCCTGG
TTTTTAGTTTTTCAGACAGGGCTTTGCGACATAGCCCTAACTGGCCTTGAACATGCAATGATCCTCCTGCCTCGGCCTCCTGGGTC
CTCAGATTGTTGATGCTACACCCACATATCTCAGACCTGAGGGTTGGCAGCTTGGGCCATGCTCTGATTTGGGAGTGACATATTAA
CAGTAGTTTAATACGAATTTGAATCCACCTTACTTTTTTGGTGGCACTGACATTGAACTCATGGCCTTCCACAGGCTTTTACGTGA
GAAATGTTTTACCTCCCTTCCTTGAGCCATGAGCGAGCCCCTCACTGGTGGAGTCTATGCAGCTGGTCTGAAACTCATACCATCTT
CCTGCCCCACTCTCCGAGTGCTGAGTGTCTACAGAGCTCCTGACTCTGCCTGGGACGTGATGTCTGTGGGACCTTGGCTATAGAAG
GGAGGCAGTCACACCACCCATTTTTGGCTTCCATGTCGGCAATGGAGAGCTTGCCATACTCACCTGATGTTGTCTGTACAGAAGGG
GGACGGTAAACAAAGCAACCACTCCTACAGATGCAGAGAGAGAGATGGGGGATGGGGGGTGTCAGGTTCTCCTGGCAAAGGGTCAG
CTCCGTTGAGGGCTTTTCCACTCCCGACCAAATTCCCAAAATAACGACTCTGAGACTCAATATTTCATGAACAAATGCTTGGGCTA
GCTTGGGCTTGTTCCCTGACAAGCCCCTATCTCATTTAACCCGTTTATTCTATTCTATGAATGCCACATGGCTGGTTACCTCGTCT
CAGTTTCATGAGACCCTCTCATCAGCACTAGGGCAAACCCTGATTCTATTACTCTCTTCCTGCAGGAGCTCGGGCCCCCTATTTCC
TCCCTAAGCTAATAGCCCAACAGCTTTTGATTGACAGGTGATGCTTCCACACAGTGCACACGAAGATTCTCTTTACCCTCCATGTG
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNATTTTTGTAGAGAGGGCTGAGGTCAAGTTTAAGATTAGGAAAGA
GAATCAAGAATTATCAAGGATAAAGGGAGAGGTCAGGATTTACAGGGGAGCAGGGTTCATGGTCAGAATGAAAATGGATGAGAGAG
GGCAATGCGTGAGCTGAGGTCAAGGGTCACAATGGACGGTCCGTCGGCGGGGGCTGGGCAGGGCCAAGCATTCTCACTGGAAAGGG
TTGGTGCCGTCGCCCCGGTGCACGGCCTGCAGCACTTTGCGGTAAACCCTGAGAGAGATGGTGGCGCAGAGACCCAACAGGGCCAG
GTGCGCAGCCACGGACACGATGCTAAAGTGCAGGAGGCAGAGGAGAGAGGCCATGAGGCCCGTGAAGACCGCTCCCGACGTCCTGG
TGTCCTTCCAGTACAACAGGTCTGCCACTGTGGAAAAGAAGACAATGTTGGGACCCGACCTGACCAAGCTCACTCCCTTTCTGTTT
GTTTTTTAGAGATAAGGTCTCACTATGAAGCTAAGGCTGACTTCAAACTATGAAACCCTGGCTGGTCTTGAACTCCTGATTATTCT
AATTCATCCTTTCTTTTTCTTAGACTGAGTTAAGTTTGACCGGCTTCAAACTCAAGACAAATACTCCTGCCTCTACCCTTTAGTGC
TTCAAGGAGGTGCTAAGGATCAAATTTAGGGCTTCATGCATGCTAGGTAAAACCCAACTGAGCTACTTCCCTGAC

MOUSE SEQUENCE - mRNA (SEQ ID NO: 8)

ATAAATTCTTATTTTGACACTCACCAAAATAGTCACCTGGAAAACCCGCTTTTTGTGACAAAGTACAGAAGGCTTGGTCACATTTA
AATCACTGAGAACTAGAGAGAAATACTATCGCAAACTGTAATAGACATTACATCCATAAAAGTTTCCCCAGTCCTTATTGTAATAT
TGCACAGTGCAATTGCTACATGGCAAACTAGTGTAGCATAGAAGTCAAAGCAAAAACAAACCAAAGAAAGGAGCCACAAGAGTAAA
ACTGTTCAACAGTTAATAGTTCAAACTAAGCCATTGAATCTATCATTGGGATCGTTAAAATGAATCTTCCTACACCTTGCAGTGTA
TGATTTAACTTTTACAGAACACAAGCCAAGTTTAAAATCAGCAGTAGAGATATTAAAATGAAAAGGTTTGCTAATAGAGTAACATT
AAATACCCTGAAGGAAAAAAAACCTAAATATCAAAATAACTGATTAAAATTCACTTGCAAATTAGCACACGAATATGCAACTTGGA
AATCATGCAGTGTTTTATTTAAGAAAACATAAAACAAAACTATTAAAATAGTTTTAGAGGGGGTAAAATCCAGGTCCTCTGCCAGG
ATGCTAAAATTAGACTTCAGGGGAATTTTGAAGTCTTCAATTTTGAAACCTATTAAAAAGCCCATGATTACAGTTAATTAAGAGCA
GTGCACGCAACAGTGACACGCCTTTAGAGAGCATTACTGTGTATGAACATGTTGGCTGCTACCAGCCACAGTCAATTTAACAAGGC
TGCTCAGTCATGAACTTAATACAGAGAGAGCACGCCTAGGCAGCAAGCACAGCTTGCTGGGCCACTTTCCTCCCTGTCGTGACACA
ATCAATCCGTGTACTTGGTGTATCTGAAGCGCACGCTGCACCGCGGCACTGCCCGGCGGGTTTCTGGGCGGGGAGCGATCCCCGCG
TCGCCCCCCGTGAAACCGACAGAGCCTGGACTTTCAGGAGGTACAGCGGCGGTCTGAAGGGGATCTGGGATCTTGCAGAGGGAACT
TGCATCGAAACTTGGGCAGTTCTCCGAACCGGAGACTAAGCTTCCCCGAGCAGCGCACTTTGGAGACGTGTCCGGTCTACTCCGGA
CTCGCATCTCATTCCACTCGGCCATAGCCTTGGCTTCCCGGCGACCTCAGCGTGGTCACAGGGGCCCCCCTGTGCCCAGGGAAATG
TTTCAAGCTTTTCCCGGAGACTACGACTCCGGCTCCCGGTGTAGCTCATCACCCTCCGCCGAGTCTCAGTACCTGTCTTCGGTGGA
CTCCTTCGGCAGTCCACCCACCGCCGCCGCCTCCCAGGAGTGCGCCGGTCTCGGGGAAATGCCCGGCTCCTTCGTGCCAACGGTCA
CCGCAATCACAACCAGCCAGGATCTTCAGTGGCTCGTGCAACCCACCCTCATCTCTTCCATGGCCCAGTCCCAGGGGCAGCCACTG
GCCTCCCAGCCTCCAGCTGTTGACCCTTATGACATGCCAGGAACCAGCTACTCAACCCCAGGCCTGAGTGCCTACAGCACTGGCGG

GGCAAGCGGAAGTGGTGGGCCTTCAACCAGCACAACCACCAGTGGACCTGTGTCTGCCCGTCCAGCCAGAGCCAGGCCTAGAAGAC
CCCGAGAAGAGACACTTACCCCAGAAGAAGAAGAAAAGCGAAGGGTTCGCAGAGAGCGGAACAAGCTGGCTGCAGCTAAGTGCAGG
AACCGTCGGAGGGAGCTGACAGATCGACTTCAGGCGGAAACTGATCAGCTTGAAGAGGAAAAGGCAGAGCTGGAGTCGGAGATCGC
CGAGCTGCAAAAAGAGAAGGAACGCCTGGAGTTTGTCCTGGTGGCCCACAAACCGGGCTGCAAGATCCCCTACGAAGAGGGGCCGG
GGCCAGGCCCGCTGGCCGAGGTGAGAGATTTGCCAGGGTCAACATCCGCTAAGGAAGACGGCTTCGGCTGGCTGCTGCCGCCCCCT
CCACCACCCCCCTGCCCTTCCAGAGCAGCCGAGACGCACCCCCCAACCTGACGGCTTCTCTCTTTACACACAGTGAAGTTCAAGT
CCTCGGCGACCCCTTCCCCGTTGTTAGCCCTTCGTACACTTCCTCGTTTGTCCTCACCTGCCCGGAGGTCTCCGCGTTCGCCGGCG
CCCAACGCACCAGCGGCAGCGAGCAGCCGTCCGACCCGCTGAACTCGCCCTCCCTTCTTGCTCTGTAAACTCTTTAGACAAACAAA
ACAAACAAACCCGCAAGGAACAAGGAGGAGGAAGATGAGGAGGAGAGGGGAGGAAGCAGTCCGGGGGTGTGTGTGGACCCTTTG
ACTCTTCTGTCTGACCACCTGCCGCCTCTGCCATCGGACATGACGGAAGGACCTCCTTTGTGTTTTGTGCTCCGTCTCTGGTTTTC
TGTGCCCCGGCGAGACCGGAGAGCTGGTGACTTTGGGGACAGGGGGTGGGGCGGGGATGGACACCCCTCCTGCATATCTTTGTCCT
GTTACTTCAACCCAACTTCTGGGGATAGATGGCTGGCTGGGTGGGTAGGGTGGGGTGCAACGCCCACCTTTGGCGTCTTGCGTGAG
GCTGGAGGGGAAAGGGTGCTGAGTGTGGGGTGCAGGGTGGGTTGAGGTCGAGCTGGCATGCACCTCCAGAGAGACCCAACGAGGAA
ATGACAGCACCGTCCTGTCCTTCTTTTCCCCCACCCACCCATCCACCCTCAAGGGTGCAGGGTGACCAAGATAGCTCTGTTTTGCT
CCCTCGGGCCTTAGCTGATTAACTTAACATTTCCAAGAGGTTACAACCTCCTCCTGGACGAATTGAGCCCCCGACTGAGGGAAGTC
GATGCCCCCTTTGGGAGTCTGCTAACCCCACTTCCCGCTGATTCCAAAATGTGAACCCCTATCTGACTGCTCAGTCTTTCCCTCCT
GGGAAAACTGGCTCAGGTTGGATTTTTTTCCTCGTCTGCTACAGAGCCCCCTCCCAACTCAGGCCCGCTCCCACCCCTGTGCAGTA
TTATGCTATGTCCCTCTCACCCTCACCCCCACCCCAGGCGCCCTTGGCCGTCCTCGTTGGGCCTTACTGGTTTTGGGCAGCAGGGG
GCGCTGCGACGCCCATCTTGCTGGAGCGCTTTATACTGTGAATGAGTGGTCGGATTGCTGGGTGCGCCGGATGGGATTGACCCCCA
GCCCTCCAAAACTTTCCCTGGCCTCCCCTTCTTCCACTTGCTTCCTCCCTCCCCTTGACAGGGAGTTAGACTCGAAAGGATGACC
ACGACGCATCCCGGTGGCCTTCTTGCTCAGGCCCCAGACTTTTTCTCTTTAAGTCCTTCGCCTTCCCCAGCCTAGGACGCCAACTT
CTCCCCACCCTGGGAGCCCCGCATCCTCTCACAGAGGTCGAGGCAATTTTCAGAGAAGTTTTCAGGGCTGAGGCTTTGGCTCCCCT
ATCCTCGATATTTGAATCCCCAAATATTTTTGGACTAGCATACTTAAGAGGGGGCTGAGTTCCCACTATCCCACTCCATCCAATTC
CTTCAGTCCCAAAGACGAGTTCTGTCCCTTCCCTCCAGCTTTCACCTCGTGAGAATCCCACGAGTCAGATTTCTATTTTTTAATAT
TGGGGAGATGGGCCCTACCGCCCGTCCCCCGTGCTGCATGGAACATTCCATACCCTGTCCTGGGCCCTAGGTTCCAAACCTAATCC
CAAACCCCACCCCCAGCTATTTATCCCTTTCCTGGTTCCCAAAAAGCACTTATATCTATTATGTATAAATAAATATATTATATATG
AGTGTGCGTGTGTGTGCGTGTGCGTGCGTGCGTGCGTGCGTGCGAGCTTCCTTGTTTTCAAGTGTGCTGTGGAGTTCAAAATCGCT
TCTGGGGATTTGAGTCAGACTTTCTGGCTGTCCCTTTTTGTCACCTTTTTGTTGTTGTCTCGGCTCCTCTGGCTGTTGGAGACAGT
CCCGGCCTCTCCCTTTATCCTTTCTCAAGTCTGTCTCGCTCAGACCACTTCCAACATGTCTCCACTCTCAATGACTCTGATCTCCG
GTNTGTCTGTTAATTCTGGATTTGTCGGGGACATGCAATTTTACTTCTGTAAGTAAGTGTGACTGGGTGGTAGATTTTTTACAATC
TATATCGTTGAGAATTC

MOUSE SEQUENCE - CODING (SEQ ID NO: 9)
ATGTTTCAAGCTTTTCCCGGAGACTACGACTCCGGCTCCCGGTGTAGCTCATCACCCTCCGCCGAGTCTCAGTACCTGTCTTCGGT
GGACTCCTTCGGCAGTCCACCCACCGCCGCCGCCTCCCAGGAGTGCGCCGGTCTCGGGGAAATGCCCGGCTCCTTCGTGCCAACGG
TCACCGCAATCACAACCAGCCAGGATCTTCAGTGGCTCGTGCAACCCACCCTCATCTCTTCCATGGCCCAGTCCCAGGGGCAGCCA
CTGGCCTCCCAGCCTCCAGCTGTTGACCCTTATGACATGCCAGGAACCAGCTACTCAACCCCAGGCCTGAGTGCCTACAGCACTGG
CGGGGCAAGCGGAAGTGGTGGGCCTTCAACCAGCACAACCACCAGTGGACCTGTGTCTGCCCGTCCAGCCAGAGCCAGGCCTAGAA
GACCCCGAGAAGAGACACTTACCCCAGAAGAAGAAGAAAAGCGAAGGGTTCGCAGAGAGCGGAACAAGCTGGCTGCAGCTAAGTGC
AGGAACCGTCGGAGGGAGCTGACAGATCGACTTCAGGCGGAAACTGATCAGCTTGAAGAGGAAAAGGCAGAGCTGGAGTCGGAGAT
CGCCGAGCTGCAAAAAGAGAAGGAACGCCTGGAGTTTGTCCTGGTGGCCCACAAACCGGGCTGCAAGATCCCCTACGAAGAGGGGC
CGGGGCCAGGCCCGCTGGCCGAGGTGAGAGATTTGCCAGGGTCAACATCCGCTAAGGAAGACGGCTTCGGCTGGCTGCTGCCGCCC
CCTCCACCACCCCCCCTGCCCTTCCAGAGCAGCCGAGACGCACCCCCCAACCTGACGGCTTCTCTCTTTACACACAGTGAAGTTCA
AGTCCTCGGCGACCCCTTCCCCGTTGTTAGCCCTTCGTACACTTCCTCGTTTGTCCTCACCTGCCCGGAGGTCTCCGCGTTCGCCG
GCGCCCAACGCACCAGCGGCAGCGAGCAGCCGTCCGACCCGCTGAACTCGCCCTCCCTTCTTGCTCTGTAA

HUMAN SEQUENCE - GENOMIC (SEQ ID NO: 10)
CACTAAGCACTCTCACCACCCAATGCCTGGAGTGGTTGTAGTCAGTGAGTGACACATTGCACAGTGTGTGCCCCCTGGATTGGGGG
TGGGTGAGAGACAGCCCCCACAGTGAGTGGCACCCCTGGCCAGGGCCTGGGACAAGTCTGTATCCAAGGGTGGCTCTCTGCTTAGG
TCTGTGTTTGTACCTGGGTGTGTCTGTGTCTGCCCTACTCTGTGCATACTCATATATGTGAACCCGTGTGTGTGTGTGTAGTTGTG
AGTGTGTGTGGAAGAGGCTGCATGGCAGTGGAAGCTAGGTGTGTGATTCGTGTATCTGTGTGCCTAGAGTGCCTTGTTCTATAGAT
TTGGATGCCACTCCAAGCAAGTCAGTGGGTCTTTTTGTTTTTTGTTTTTTGAGATGGAATCTCACTCTGTTGCCCAGGCTGGAGTG
CAGTGGCACGATCTTGGCTCACTGCAACCTCTACCTCCTGGGTTCAAGTGATTCTCCTGCCTCGGCCCCCCGAGTAGCTGGGATTA
CAGGTGCCCACCAATACACTCAGCTAATTTGTTGTATTTTTAGTAAAGATGGGGTTTCACCATGTTGGCCAGGCTGGTCTCAAACT
CCTGACCTCATGATTCTCCTGCCTCGGCCTCCCAAAGTGCTGGGATTACAGGCATGAGCCACCGCGCCCGGCCAAGTCAGTGGGTC
TTTAGGAGCTGTTTCGTACGGTGTGACTGTGAGTGAACCTTCGCACACGTGTCTGTACGGATATCTAAGAAATTCTTCAAGTAGGC
CGGGCACAGTGGTTCAGGCCTGTAGCCTTAGCACTTTGGGAGGCCCAGGTGAGTGGATCGCTTGAGCTCAGGAGTTCGAGAACAGC
CTGGGCAACATAGTGAGACTTCGTATCTATAAAAAATATGAAAATTAGCCAGGCATGACAGTGGGCACCTGTAGTCCCAGCTACTA
CTTGGGGGGCTGAGGCAGGAGGATCCCTTGAGCCTGGGAGGTGGAGGCTGCAGTGAGCTGAGATCGAGCCACTGCACTCCAGCCTG
GGCGACAGAGGGAGACCCTGTCTCAAAAAAAAAGAAAAGAAAAGAAAAAAAGAAGACATTCTTCAAGTCCACAACTCTGAGGGTAT
CACTGTGAGAGCAGGGGTCCTAGCTCTACCCATTTGTGTGTGTGTGGAGATGTGTAAGTCTATGTAGACAAAAGTGTGTGTCATTT
ACTGTGTTTGGGGTGTGAACACCTATGTGATGTGTTTGCACAACTGCACGTGTTTTGTTCTGTGTGTGTGATCGTGTGTTCAAATA
AGTCATCTTGTCGCCGGGCGTGGTCGCTCATGCCTATAATCCCAGCACTTTGGGAGGCCTAGGCAGGAGGACCATTGAGCCCAGGA
GGTCCAGACTGCAGTGAGCCGAGATTGCGCCACTGCACTCCAGCCTGGGCGACAGCAAGACCTTGTCTCAAAACAAAAGCAAAAAC
AAAAATAAACAAATAGTCATCAGGTGCCTGCACAGACAAAGGTGAAAGGTGTCTGCCTGTTGAGATCTGTGGATAGGGTGTATATA
TGGACATCTCAGCCTGTCTACGTGTGTATCTGTCTCTGTCCTCACGGCAAAAGAGAGGTTGGCCGGGTGTGTGGTGGCTCACGCCT
GTAATCCCAGCACTTTGGGAGGCTGAGGCGGGCGGATCACCTGAGGTGAGGAGTTCAAAACCAGCCTGGCCAACATGGCGAAACTC
CATCTCTTCTAAAAATACAAAAAAATTAACCGGGCGTGGTGGCACACGCTTGTAATCCCAGCTACTCAGGGAGGCTGAGGCATTAG
AATTGCTTGAACCCAGGAGGTGGAGGTTGCAGTGAGCCAAGATTGCGCCACTGGACTCCAGCCTGGGCAACAGAACAAGACTCCGT
CTCAAAAAAAAAAAAAAAAAAAAAAAGAGGCTCAGATATGTTTCTGTCTGAGAGTCTGTCAGAGTTTAGGAATTAGTAAATGAATG
AATGGTGAAGATCCATTTACTCAACAAACATTTATTTATTTATTTATTTATTGAGACAGAGTCTCGCTCTGTCACCCAGGATGGAG
TGCAGTGGCGCAATCTCGGCTCACTGAAGCCTCTGCCTCCTGGGTTTAAGAGAGTCTTGTGCCTCAGCCACCAAGTAGCTGGGATT
ACAGGTGTGTGCCACTGCGCCCAGCTAATTTTTGTATATTTAGTAGAGATGGGGTTTCACCATGTTGCCCAGGCTGGTCTCGAACT
CCTGGCCTGAAGTGATCTGCTCACCTCAGTCTCCCAATATGCTGGGATTACAGGCATGAGCCACCAGTCCTGCCTCATTTATTCTT
ATATTATATTATATTGTTTTTATTTTAAATTTTTTTGTAGTGACAGGGTCTTACTATGTTGACTGGGCTGGCCTCAAACTGGCCTC
AAGTGATTCTCCTGCCTCAGCCTCCCAAAGTGCTGGGATTACAGGCATGAGCCACTGTGCCTGGCTTGAACAAATATTTAATAACC

ACCTATTGGGTACCACGTGCTATGCTGGGGACAAGGCAGTGACCGGGATGGTTTTGGCCGAGCTTTCACCCAGCTCACAACCCAAT
GAGGGAGACAAACCTATCCCCAGACAATGATGAGCCCAGATTGGCAGAGTTGGAGGGAGGGACCCCAGGAGAGGGGGCCTTGACTC
AGCCTGGAGATCAGGGAGGGCTTCCTGGAGGAGAGGTTATGGGAGTTAAGACTTGGAGGAAAAGACTATGAGCCACAGGAAGCGAA
GGGAAGAGTGACCCAGGCAGAGGGAAAAGCACCTGCAAAGGCCTGAAGTCCAGGGAGAGAGGCCAGGCATCTGGAACACAACGGGG
AGAGGAGAGACGAGACTAGGGCACCCTCGGAGCAGGTCGTTCAGGGCCTCAGGAGCCATGGGGAGGAGTATAATGCAGGATGCAGG
GGTACTGGGAGGGTCAAGCTAACAGTGTCCAATCTGGGTTACTCTGCTTCCCTGAAACTCCAGGGACATCTCCTCCAGGAAGCCCT
AGGGATTGCTCAGTGTGGAGCGGCTCAGCCACCCAGTCACACTCACTATTCAATGCTGAGCACTGAGGAGGCAGCCGTTACTAAAT
CAGCCTGGATTTGCCCTCCCAGAGGTCACAGTCACAACAGTCAAACACACACCTGCACGTGCACACTCATGAACGTGCCCCTCCCC
TCCTCTGGAGCCTCAGAAACCCACAGACCTTGTTCCTCCCAGGGGGTGTCCCTGGGGCCAAAGCTTCTGCACTACCAGGGGGCTTG
TTGGAAATGCAAAAGATGGCTGGGCACAGTGGCTCACACCTGTAATCCCAGCAGTTTGGGAAGCTGAGGCAGGCAGATCATTTGAG
GTCAGGAGTTCATTTGAGGTTGGGAGTTCAAGACCAGCTTGGCCAACATAGCGAAACTCTGTCTCTACTAAAAATACAAAAAAATT
AGCCGGGCGTGGTAGCACACGCCTGTAATCCCAGCTACTTGGGAGGCTGAGGCAGGAGAATCTTTTGAACCCGAGAGGTGAAGGCT
GCAGTGAGCCAAGATGGCACCACTGCACTCCAGCCTGGGTGACAGAGTGAGTCTTAAAAAAATAATAATAAAATAGGCTGGGCGCG
GTGGCTCACGCCTGTAATCCCAGCACTTTGGGAGGCTGAGGCAGGCGGATCACCTGAGGTCAGGAGTTCGAGACCAGCCTGACCAA
AATAGTGAAACCCCGTCTCTACTAAAAATACAAAAAAATTTAGCTGGGCGTAGTGGCTAACGCCTGTAATCCCAGCTACAGGCTGA
GGCAGGAAAATTGCTTGAACCCAGGGGTGCAGAGGTTGCAGTGAGCCAAGATGGCACCATTGCACTCCAGCCTGGGCAACAACAGT
GAAACTCCGTCTCAAAAAACAAAATACAAAAATTAGCCAGGCATGGTGGCACACACCTGTAATCCCAGCTACTCGGGAGGCTGAGG
CAGGAGAATCACTTGAACCCGGGAGGCAGAGGTTGCAGTGAGCTGAGATCGTGCCACTGCACTCAAGCCTGTGTAACAAGAGTGAA
ATTCCGTCTCAAAATAAAATAAAATAATAAAAATAAAAAGATGACCACAGCAGATCTGCTGGCTCTCCAGGCAATTCGTGATCACC
ACGAATTGAGAAGCGCTGCTCTCAGGCTTAACCCCCCTGTACCTCAGTTTCCTTTTCTGTACAAGGAGATCAAAACAGAATCCATG
TAGAGGACTGACGTGGAGTGAGGCTTAACTGAGATAATGAGTGTGAAAGTGCTAAGCACAGCTTCCTAAGTGCTTGTTTTCAGCAG
CTCCTCTTCCTCTTTTCTATTTTTTTATTTTTATTTTTTTTGGGGGGGAACAGGGTCTCGCTCTGTTGCCCAGGCTGGAGTATAG
TGGCAGAGTCATAGCTCACTGCAACCTCAATCTCTCAGGGCTCAAGTGATGCCCGTGCCTCAGCCTCCTGAGTGGCTGGGACTACA
GGTGTGTGCCACCATGCCCAGCTAATTTTTAAATTTCTTGTAGAGGTTGGGGGTGGGTCTCACTATGTTGCCCAGGCTGGTCTTGT
ACTCCTGGCCTCTGGCGATCCTCTTACCTAGGCCTCCCAAAGTGCTGGGATTACAGGCGTGAGCCACCACACCCAGCCCTCCTCCT
CCTCTGTATTATTAAATTTCAGAGCTGGAAAGAAATTTCGAAGACCGCCATCACTCAACCTTCTTGCTCTACAGATGGGGGAACTG
AGGAACAGAGGAGCAGAGGTTTGGCCAGAGGAAGGTGGGAAGGACGTGGTGTGGCTGGCCTTCACAGACTCTGCCGGGGGCTGTTT
ACAGGATACTCTGAGGAAAGCCAGGATGGGCACTGATACCCACCTCCACCCTGCGGGAGGCAGAAGGCTAAGGCTCCCCCAGCCGT
CTCCCCCAAGCAGAGCTGAGCCCTTGGGAGCCAGGGCAGAGATGCAACCCTCTCCCCCATGCTCAGGTCCTCCTCCTCCTGGAAGC
TCTCCTTCCAAGAGGAGCCTCTTATTTTATTTTTTATTATTATTATTTTTGAGACAAGGTCTCACTTTGTCACCCAGGCTGGAAGT
GCCGTGGCATGATCATGGCTCACTGAAGCCTTGCCCTCCCCAAGCTCAAGTGATCCTCTCACCTCAGCCTCCCAAATAGCTGAGAC
TACAGGCACACGCCACCACCCCTGGCTAGTTTTTGTATTTTTAGTAGAGATGGGGTCTTGCCATTGTCCAGGCTGGTCTTGAACTC
CCGGGCTCAAGTGATCTGCCCACCTTGGCCTCCCAAAGTGCTGGGATTACAGGCATGAGACACTGAGCCTGGCAAGGAATGAGCCT
TATGGAGAAGAACTTGATTTACCCACTCCATGCTCTGCAGGCAGGTGTGTCATGTCTGTGAGTGCAGGTGTGTGTGTGTGTGTA
TGTGTGTGTGTGTGTGTCTCCAGGGCCAATTTCTCCCTTAGGCCCAGGGGCACAGTGCCTAGGGCCCATGATAAATTTAACCGACC
CACGGATATGGGTCAGGAGGGTATACATGTATAAAGTTCAGGAAGACTTTGTTTTAGTAACAAAACGAGCATGTACAATCTGTCAG
GAGCCTTATGATATCTTCCCACCAGTCTCATGAGTGGGCACTACATCTTGTTCTCCTTTGTCGGGTAGGGAAACTGAGGCCCCGGA
TGTCCTAGCTCAAGTTCACCAAGCTACTTGGTGATAGAAGCGGAACATAAGCCTAGGCCACCTAGCTTCTGGGTCTGCGATCGTGT
GAGGATAAATGCCCAGTGTGTTCGTGAACATGAGCATCCCTGTGTGAATAAATTGACATACATAAACCCATTTAATAAATTTCAAA
GCCAGGCGCCGTGGCTTACTCCTGCAATCTCAGCGCTCTGGGAGGCTGAGGTGGGAGGATCGCTTGAAGCCAGGAGTTTGAGATCA
GCCTGCAACAAAGTGAGACCCTGACTCTAAAAACATTTTTTTGAATAAAAAAATTAGGCTGGACACAGTGGCTCACGCCTGTAATC
CCAGCACTTTGGGAGGCCAAGGTGGGTGGATCACTTGAGGTCAGGAGTTTGAGACCAGCCTGGTCAACAGGGTGAAACCCTTTCTC
TACTAAAAATAGAAAAATTAGCCAGGCGTGGTGGCACATGCCTGTAATCCCACCTACTGGGGAGGCTGAGGCCGGAAAATTGCTTG
AATCTGGGAGGTAGAGGTTGCAGTGAGCCAAGATCATGCCATTGCACTCCAGCCTGGGCGAAGAAACGAGACTCTGTCTCAAAAAA
AAAAAAAAAAAATTAGTCAAGCATGGTGGTACGCACCTGTAGTTGTTAGTTACTTGGGAGGCTGAAGCAGGAGGATTACTTGAGGC
CAGGAGTTCGAGGTTACAGTGAACTATGATTGCATCACTGCACTCTAGCCTGGATGACAGAGCAAGATCCTATCTCAAAATAATAC
TAATAGTTCAAGGAACAGACTTTGAAGCCTGACACCCTGCATGGTGTTATTCCAGCTTTGCTACTTACTTGCTGTGTGACTCTGGG
TGAATAACTTAACCTCTCTGGGCTTCTGTTTCCCTTCCTGTAAAATGATCATTTGTACCTCACAGGAGTGTTGTGAGAATTAAATA
AGTTAATATAAGCTCTTGGGAAGAATTAGCTCTTGTTATGGTTGTGTGAAGAGCTTTACACGAGTCCCTCTGCAGGATGCATGGTT
GTTGGTCTGTGTGTGTGTATATATATATATGTGTGTGTGTATGTGTGTATCTATGTGTATATGTGTATATGTGTGTGTTGCTCTCT
TGATCTCTGAGTGTTGTGTGTGTGTCAATGTGTGAGATCATATATGCACCTTTAGTAAAGATCTAGGGGTCTCTGTGTGTTTTGGG
GGTCTGTACCTGTCCTTGGCTGTACACCTGAACCCTACGTCTTCTTGTGTGTGTAGGGATGTGTGTCATGTGTGTTGATAACCATA
TGACAGTGTGTGTTTCCACATGCCAGTGTGTGTCTGTGAAGCTGTTTCTATGTGGCTGTGGGTGATTGCCCACGCGTGACCTGGTG
AGCGGCTGTGTGGAAGTCTGTGTTGCCTGTGTCGGGTGTGGAGTCTCTGGCCAGCCAGGGTAGGTCTTTGCATGTTTCCTTTATTC
CATGGAGGAGGAGATGAGGGGCTTGGATGAGACAGAAGAATGAGGGGTCACTCCCTGTCTGATGCTGGGGCCGAGTCACTGCCTAC
TAGTGGCTGCTGTGTCATCTCCCAGTCTCTGTCCCTCCCTCCTGGCTGGTGGCAGACAGAGGGGGCGTGGAGGAACAGGGCAGGGG
GTGGGCTTGAGGCTGGACTTTTGTCTGGAAACTTGAACCTCCACCTATGCCCCCCACGTCCCTCTAGTCTTCCACATCTCCCTACA
CCCTCCCTACTATTGGGGCAGGGAATCAGGACTCCCTGAAAATTAAGAACCATCGCCTTGGCTCTGCCACAGGCTTGCTGTGTAGC
CCGGAGACAGGCCCTGCCCCTCTCTCAACCTGTGAAATTCAATGTTTTAAAAGTGCTTCTTCCTTTGTGCCCAGGGCTGGGCCAGG
CTGTGCACTGGGAGGGAAGTGCTGGGTTCTCTGAGGTTTGTGAAATAGCTGGTAGCACCCTTCCCAGTGGGTGCTTAAGAGTTGGG
GGCTGTAGCATCTGAAATACCCAAGTCAGTGCAGGCATAGTGGCTCATGCCTGTAATCCCAGCACTTTGGGAGACCGAGGCAGGCG
GATCACCTGAGGTTGGGAGTTCGAAACCAGCCTGGCCAACATGGTGAAACCCCCATCTCTATTAAAAATACAAAAGTTAGGCCGGG
CACAGTGGCTTACGCCTGTAATCCCAGCATTTAAGAAGGCCAAGGCGGGAGGATCACCTGAGTTTGGGAGTTTGAGAGCAGCCTGA
CCAATATGGAGAAACTCTGTCTCTACTAAAAATACAAAAAAACTAGCCAGGCATGGTGGCACATGCCTGTAATCCCAGCTACTCGG
GAGGCTGAGGCAGGAGAAGCACTTCAACCCGGGAGGCGGAGGTTGCGGTGAGCCGAGATCGTGCCATTGCACTCCAGCCTGGGCAA
CAAGAGCAAAACTCTGTCTCAAAAAACAAAACAGAACAAAACAAAAATTAGCCAGGTGTGGTGGCGCACACCTGTAATCCCAGCTA
CTCGGGAGGCTGAGGCAGGAGAATCACTTGAACCCAGGAGATGTAGGCTGCAGTGAGCCGAGATTGTGGCACTGCACTCCAGCCTG
GGCAACAGAGTGAGACTCTGTCAAAAAAAAAAAAAAAAAACGTCAGATTCATGAACCCCATTTGCTAAGAAGATTTGCTCAGGTAG
TCACCTGTACCCCTGTGAGCCAAGATTGCAACAGTACATGCTAAGAGCAGTAAGAAGGAGTTTGTAAAGCTTAGAAAGACCTGACA
GGCCAGGCGCTGTGGCTCACGCCTGTAATCTCGACACTTCGGGAGGCCGAGGCAGGCGGATCACCAGGTCAGGAGTTCAAGACCAT
CCTGACCAATATGGTGAAACCCCGTCTGTACTAAACATATAAAAATTAGCTTGACGTGGTGGCCCGCGCCTGTAGTCCAGCAACTC
GAGAGGCTAAGGTGAGGCGGAGGTTGCAGTGAGCCGAGATCGCCACTGCACTCCAGCCTGGCGGCAGAGCAAGACTCCGTCTCAAA
AAAAAAAAAAAAAAAAAAAAAAAAAGACCTGACAAGTGAAAGCTACTAATATTGCCATTATCATTTAAAAAAACCCCAGACACAGG
TTTTTCAGGGAGTTTCATCCAACCAGGCAGGTCTCAGAAATCAGAAAAGAAATGGAAAGGGTAGGAAATCTGGAGTTTGACAATTC
TGAGTTTGAATTTCTTGTGATGGGATCTTGGGCAAGTCATTTAACCTCCCTGAGTATCATTTTTTTCTTTTATAAATGAAGATTT
TTCTCTCTTAACCTTCCAGAGCTGTTTTAAGGATTACAAATCTTCTACTGAAAGGCGTAGCACAGGAGCTGTGCTTGGCAAGTGCC

AAATACAAGGCATTAATTATTATTATTAGAATTAATAATAATATCCCCTCCCTCTTACACATTCTTTGTCTCCGGGTGGATTAAAA
GGTGGAAGGAGAGGCTACCAACACCATCAGAAGAGAGGCTTCTCTCTAAGTTTCATTTCCCATCTCCTCCAAATCCGCATCCCTCC
CAAACGCCGGACCTGTAAGGCCAGCAGGGTCCAAGACACACATCCTTTGCCCAGCGGGGAAGATTAAAGCTAAAGCTCAGAGAGGG
AAAACATTTCCTAAGCTCGCACAGCGAATCAGGACAGAAACCAGGACGAGCCTCGGAATCCCTCCATTACCTCCACTTTCACCTGA
GCATCACAGCCCGCTCGGGACTCAGTTTCCCCACCTACGTGACCACACCACACTAATCAGGGTCTCCTTTTGGAGATCTGCTCTTC
TTCTCGAATGGGGGCGCTGCACCATCGGTAGAACAGGGTAGGTGGGGGCGCCAGAGGTGAAGGGGACCTGCAGGCTGGGGTCTTCC
CCGCCCGGGTCAGCGGGGTCCCTGCGGGGCTAGTCTAAGCGCCTATTATTACCAGCCCCCGGGGCGGCGTTGCACTGCGCAGGCGC
GGGCGGGGCGCGGGCGCGCGCGAGCGAGCGAGGGATTCCCTCTGACGTCATTGCTAGGATACCAAACAAACACTCCGCCGCGCC
GGCCGAGCTCCTTATATGGCTAATTGCGTCACAGGAACTCCGGGAAGGCGGGGCCGGGATCCCCTCCCGCCGAGTGGCCCGGAACG
CAACCCCCGAGACCCCCAGGGCCCCGAGGGTCATGCAAGTGACCAGATCGAGTCTAGAACAGACCTCTTGCTGGACAGTGCGGGAC
TCGATTTGGCGGGGCCGGAGATTTGGGAAGTTTGTCCAGCAAGGGGCGGGTGACGTAAGCAGGGGGGCGGGTCCCGGGCATATAA
ATACAGGCTGGCGGGTCTGTGCTTCATTCATAAGACTCAGAGCTACGGCCACGGCAGGGACACGCGGAACCAAGACTTGGAAACTT
GATTGTTGTGGTTCTTCTTGGGGGTTATGAAATTTCATTAATCTTTTTTTTTCCGGGGAGAAAGTTTTTGGAAAGATTCTTCCAGA
TATTTCTTCATTTTCTTTTGGAGGACCGACTTACTTTTTTTGGTCTTCTTTATTACTCCCCTCCCCCCGTGGGACCCGCCGGACGC
GTGGAGGAGACCGTAGCTGAAGCTGATTCTGTACAGCGGGACAGCGCTTTCTGCCCCTGGGGGAGCAACCCCTCCCTCGCCCCTGG
GTCCTACGGAGCCTGCACTTTCAAGAGGTACAGCGGCATCCTGTGGGGGCCTGGGCACCGCAGGAAGACTGCACAGAAACTTTGCC
ATTGTTGGAACGGGACGTTGCTCCTTCCCCGAGCTTCCCCGGACAGCGTACTTTGAGGACTCGCTCAGCTCACCGGGGACTCCCAC
GGCTCACCCCGGACTTGCACCTTACTTCCCCAACCCGGCCATAGCCTTGGCTTCCCGGCGACCTCAGCGTGGTCACAGGGGCCCCC
CTGTGCCCAGGGAAATGTTTCAGGCTTTCCCCGGAGACTACGACTCCGGCTCCCGGTGCAGCTCCTCACCCTCTGCCGAGTCTCAA
TATCTGTCTTCGGTGGACTCCTTCGGCAGTCCACCCACCGCCGCCGCCTCCCAGGTAAGTTTTTGATAGTAGGGGTGCTGCTTTGT
AGGTTTTATTTTTAAGTCAAGGGTGAAAAGAATAAACCCCAACCCCCACAAAAAGGCGCATCAGAACCCTAGATCTGAGATGGAA
AAGGCTCACAGCGCACTTTGCAAACTGCAAAGAGTCGGGAGATGTTTGCAATTGGTTGCGTGCGTGGAGCGCAAGGAGGGAACGCG
GCAGGGAGGGTAGGCTTTGGGGCGAGGTGGGGTGGGGTGGGTAATGCGCTGCTCAATGCAACGTGTATGCGGTAGCGGGGCTGAG
AACTTTGAGCCGGCCCCGGGACTGCCCCCTGCTCGGGTCCCAGACCTGAAGCTAGCGCAGTTAGGCAGGTGGGGAAATCCCGGGG
AAGCTTCCAGCAGTCTCTTTTCCTTTCCTCCTCCTTCGGAGCGCCCACTTCGGTGCCGGGTCGCCCTCCACCCATCGGGAAGAGGG
GCCTCGAACCCTCAGCCGCGCTGCCTCCGCCTCCTGCGCGGAGACGTAACGGGGGACCCGTGCGTAACGGCTGACGCGCTGGAATC
CTCCGTCTGACGCGGGGCACGCACGGCGCGCGGCGCCCCCTTCGTCCGCCCCGCCCCTGACGTCCCGGGAGCGTTCTATTTTGGAA
CGCCGGGGCCACGTTGCTAAGGGAGGGGGCAGCCCGGCGTTTCGATTGGCCGCCGGGGCGCACGCCTTGGCCAATCAGCTTTCCCT
TCCTATTTGTAGGGTGCATTTTCCTTCCCCCCTCTCTGTCCCCGGAACCCGTGGTTCCTTGGCGGCTGGGTCTCTTTTCGGCGCCT
CTAGAGGCAGAGGGAGGGGATCCCTGTCGTGACAAGAGCGCCTGTCTGCGACCCAATGGATCTGCGAGGCCCTTGCGGGGATCTAG
TCCCTGGGCTCTCAGGAGAAGGGGGTGTCTGCTTGTGTGCTGGCGTTTCTTGGAGAGATACGGTGGCTGTCACCCTCTTCTCCAGG
CACACACAGACACATTCCCACTCCCTCTGCTCCTCATGCCCGGTTCCTCCGGTGTGTCCCAAGACAGGACTAGAACCGCGAACCGA
AGGGCAACCAGCCAGGTGGTCTCCAGGAGCTCCGCCCCCTCTGGGTTCCCAGGACTCTGATTGGTCGGCGAGCCAGCCCTTCCCTC
ACCACGCCCCCCGAGAGAGTAGTTAAGCCTTCAGAGCAGTTCCAGGAGTCCATTTACGGGAGGGGGGAGATGAGCGCTGCTGAGGC
TTGGGGGCTCAGGTCCCGCACCATTCCCCCTCCGCGACAATCTGAGAGAGCTCCAGTGGTTACTTTTATCTACCTGTCCGTTCACC
CTAAACTGTCACTCGTCAGTCTCACTCTGAGAAGAGACAGTAACTTGAAACGTTGTTCTAAACTCCTAGGCCCGTCCCCCAAACAC
CCTTTTGACTGGGACCCCCGCCCCTGCATGGGACCTCGCGCAGAGGGGGGTGTGTATGTGTGTGAGTGTAGAGGAAGGCTTGGCCT
AAGGCCTCTCCTTCTCCCTCCCCTTGCCTCTGGGGTGGGGGTGGGGTGTTGTGGCTGTGTGTGTGGCTGTGGCTCCGTCCCGGGGG
TTCTGTCACCCGGCTGTGTCCAGCCTCCTCTCCACCCCCCATACCTAAGAGTCACCAACCCGGGGTGTGATTCACCACCCGCTGGA
ACCGTGCAACCTTTCCCCGAGGAAGAAGGAGGAGGTAGAAGCCAGTTGAGCAGAAATCCTCTCATTAACCACTGCGTCACGGTGTA
GTGGAAGGGTGGGTGTTGTGGCTTTTTCCCTGTGACACACACATCCACACTCGCTCACCCTGTGCTCACTCACGGGGTCGGTGTGT
GTTATGTGTGTTGGGTGTGTGTGTGTCGGTGTCTTTGTTTGTGTGTCTACGCCTGTGTGTGTATGTGTCACCCCGTAGGAGTGCGC
CGGTCTCGGGGAAATGCCCGGTTCCTTCGTGCCCACGGTCACCGCGATCACAACCAGCCAGGACCTCCAGTGGCTTGTGCAACCCA
CCCTCATCTCTTCCATGGCCCAGTCCCAGGGGCAGCCACTGGCCTCCCAGCCCCCGGTCGTCGACCCCTACGACATGCCGGGAACC
AGCTACTCCACACCAGGCATGAGTGGCTACAGCAGTGGCGGAGCGAGTGGCAGTGGTGGGCCTTCCACCAGCGGAACTACCAGTGG
GCCTGGGCCTGCCCGCCCAGCCCGAGCCCGGCCTAGGAGACCCCGAGAGGAGACGGTGAGTAAGGGACATCAGAACTTGGCCTGGG
TAGGGGGAAGCAAGAGAGGCAGGAAGTTTCTTATGAATGGAGGGGGGCTCCACTAAGGCCTCAGTGTTACAGAAACCCCAAGATCC
TTGCTACACGAGCGAGGACCGGAGGCTTGTTTTTTGGCTCTTGGGGGTTCTGAAAGAAGTAGAGGTTCGGGATGGGTGGAGGAGTG
CTGTATCCCCAAATCTCATGGCCTCTATCTCCCTGACTCAGCTCACCCCAGAGGAAGAGGAGAAGCGAAGGGTGCGCCGGGAACGA
AATAAACTAGCAGCAGCTAAATGCAGGAACCGGCGGAGGGAGCTGACCGACCGACTCCAGGCGGTGAGGACAGGCCCTGGGGTGGG
AGAGGGGATGTTGAGGGGAGCTCTCTCCCCATTCTCTGCCCCCTCTCCACCTGTACCCTTATCCTGGGTTGAGAACTAGACGTTCC
ACACATGGAACTAGGTACTGCTGTGGCCAGACTGGGTAGCCCAGGGCACAAACACAGACCCCCATGGACTTAAGTCAACTCCTGGT
CCCCCCCCCATTTCCTGACCCCACGGACTACTCTCCTAGCCTTCATTTCATCCCAAGGGGCCACATGGGGCCCTTGAGGAGAGGGG
CGCCCCCATCATTTCTCCCATCTGGTCTTCAGCAAGTAACAACCCATTTTGCCTCAGTTTCTCCATCTCTGCAAACCCTCATCAAA
TCTCCCGGGCTCTTTCTACCCTTAACACTCTGGAAAGCCTGTGAAATGAAATTATTCCACCTCCTGCCCTAGCCACCCACAGCTCT
CCTGGTGCTGGTGGCATCCCCCAAAACCCACTCCCTTCCTACGTCCTCCCTTGGTCTGAGAGTTCCCTGCTGTATGCCTGCAGGGT
GAGCTGTTACTCCTTGAGGGAACAAGGGAATTGTCAACTTTCCTTCTCTACTTTTTCTCTTCCCCGGGAGGTAGAGAGGGAGGGGT
AATAGAAGGGAACACATTAAAAACACATAACAGTGGCTCATGCCTGTGATCCCAGCACTTTGAGAGGCCAAGGCAGGAGGATTGCT
TGAGCCCAGGAGTTTGAGACCAGCCTGGGAAACATAGGGAGGACTTGTCTCTACCAAGAAAAAAAAAAAATTAGTTGGGCATGGTGG
TGCACACCACTGTGGTCCCAGCTACTATGGAGGCTTTAGTGGAGGATCGCTTGAGCCGAGGAGGTCCAGGCTGCAGTGAGCCATG
ATTGCACTATTGCACTCCAGCCTGGGGGACAGAGCGAGACTCTGGCTCAAAAGCAAAACAAAACCAACCACATAACGATTGTTCAT
TCATTCAACAAACCTCCTGCACACCAGAAACTTCCCCATAAACCTGGCCCCTTCTGTACCATCCTTTGGACAGATGGGGGAAACTG
AGGTTCCTGGAAGGCAAGACCCTTGCCCCAACTCCCATGGCCACCAGGACTCCATCTCAGGAGGTGTTTTTTCTCAGCCCGGGGCT
GCCTTCCAGCAGCAAGTCCAAGGGAGCCATGACCCGAGTTTCCCGGTCACTGACATGCTTTTTTCTCCTTCCTCTCTCTCTTCTGT
GACCTGGCCTCCCTGGCCTCAGGAGACAGATCAGTTGGAGGAAGAAAAAGCAGAGCTGGAGTCGGAGATCGCCGAGCTCCAAAAGG
AGAAGGAACGTCTGGAGTTTGTGCTGGTGGCCCACAAACCGGGCTGCAAGATCCCCTACGAAGAGGGGCCCGGGCCGGGCCCGCTG
GCGGAGGTGAGAGATTTGCCGGGCTCAGCACCGGCTAAGGAAGATGGCTTCAGCTGGCTGCTGCCGCCCCCGCCACCACCGCCCCT
GCCCTTCCAGACCAGCCAAGACGCACCCCCCAACCTGACGGCTTCTCTCTTTACACACAGTGAAGTTCAAGTCCTCGGCGACCCCT
TCCCCGTTGTTAACCCTTCGTACACTTCTTCGTTTGTCCTCACCTGCCCGGAGGTCTCCGCGTTCGCCGGCGCCCAACGCACCAGC
GGCAGTGACCAGCCTTCCGATCCCCTGAACTCGCCCTCCCTCCTCGCTCTGTGAACTCTTTAGACACACAAAACAAACAAACACAT
GGGGGAGAGAGACTTGGAAGAGGAGGAGGAGGAGGAGAAGGAGGAGAGAGAGGGGAAGAGACAAAGTGGGTGTGTGGCCTCCCTGG
CTCCTCCGTCTGACCCTCTGCGGCCACTGCGCCACTGCCATCGGACAGGAGGATTCCTTGTGTTTTGTCCTGCCTCTTGTTTCTGT
GCCCCGGCGAGGCCGGAGAGCTGGTGACTTTGGGGACAGGGGGTGGGAAGGGGATGGACACCCCCAGCTGACTGTTGGCTCTCTGA
CGTCAACCCAAGCTCTGGGGATGGGTGGGGAGGGGGGCGGGTGACGCCCACCTTCGGGCAGTCCTGTGTGAGGATTAAGGGACGGG
GGTGGGAGGTAGGCTGTGGGGTGGGCTGGAGTCCTCTCCAGAGAGGCTCAACAAGGAAAAATGCCACTCCCTACCCAATGTCTCCC
ACACCCACCCTTTTTTTGGGGTGCCTAGGTTGGTTTCCCCTGCACTCCCGACCTTAGCTTATTGATCCCACATTTCCATGGTGTGA

GATCCTCTTTACTCTGGGCAGAAGTGAGCCCCCCCCTTAAAGGGAATTCGATGCCCCCCTAGAATAATCTCATCCCCCCACCCGAC
TTCTTTTGAAATGTGAACGTCCTTCCTTGACTGTCTAGCCACTCCCTCCCAGAAAAACTGGCTCTGATTGGAATTTCTGGCCTCCT
AAGGCTCCCCACCCCGAAATCAGCCCCCAGCCTTGTTTCTGATGACAGTGTTATCCCAAGACCCTGCCCCCTGCCAGCCGACCCTC
CTGGCCTTCCTCGTTGGGCCGCTCTGATTTCAGGCAGCAGGGGCTGCTGTGATGCCGTCCTGCTGGAGTGATTTATACTGTGAAAT
GAGTTGGCCAGATTGTGGGGTGCAGCTGGGTGGGGCAGCACACCTCTGGGGGGATAATGTCCCCACTCCCGAAAGCCTTTCCTCGG
TCTCCCTTCCGTCCATCCCCCTTCTTCCTCCCCTCAACAGTGAGTTAGACTCAAGGGGGTGACAGAACCGAGAAGGGGGTGACAGT
CCTCCATCCACGTGGCCTCTCTCTCTCTCCTCAGGACCCTCAGCCCTGGCCTTTTTCTTTAAGGTCCCCCGACCAATCCCCAGCCT
AGGACGCCAACTTCTCCCACCCCTTGGCCCCTCACATCCTCTCCAGGAAGGGAGTGAGGGGCTGTGACATTTTTCCGGAGAAGATT
TCAGAGCTGAGGCTTTGGTACCCCCAAACCCCCAATATTTTTGGACTGGCAGACTCAAGGGGCTGGAATCTCATGATTCCATGCCC
GAGTCCGCCCATCCCTGACCATGGTTTTGGCTCTCCCACCCCGCCGTTCCCTGCGCTTCATCTCATGAGGATTTCTTTATGAGGCA
AATTTATATTTTTTAATATCGGGGGGTGGACCACGCCGCCCTCCATCCGTGCTGCATGAAAAACATTCCACGTGCCCCTTGTCGCG
CGTCTCCCATCCTGATCCCAGACCCATTCCTTAGCTATTTATCCCTTTCCTGGTTTCCGAAAGGCAATTATATCTATTATGTATAA
GTAAATATATTATATATGGATGTGTGTGTGTGCGTGCGCGTGAGTGTGTGAGCGCTTCTGCAGCCTCGGCCTAGGTCACGTTGGCC
CTCAAAGCGAGCCGTTGAATTGGAAACTGCTTCTAGAAACTCTGGCTCAGCCTGTCTCGGGCTGACCCTTTTCTGATCGTCTCGGC
CCCTCTGATTGTTCCCGATGGTCTCTCTCCCTCTGTCTTTTCTCCTCCGCCTGTGTCCATCTGACCGTTTTCACTTGTCTCCTTTC
TGACTGTCCCTGCCAATGCTCCAGCTGTCGTCTGACTCTGGGTTCGTTGGGACATGAGATTTTATTTTTTGTGAGTGAGACTGAG
GGATCGTAGATTTTTACAATCTGTATCTTTGACAATTCTGGGTGCGAGTGTGAGAGTGTGAGCAGGGCTTGCTCCTGCCAACCACA
ATTCAATGAATCCCCGACCCCCCTACCCCATGCTGTACTTGTGGTTCTCTTTTTGTATTTTGCATCTGACCCCGGGGGGCTGGGAC
AGATTGGCAATGGGCCGTCCCCTCTCCCCTTGGTTCTGCACTGTTGCCAATAAAAAGCTCTTAAAAACGCATTCGCCAGGCTACAG
TGTTTATTTCCTCCTAACACCCATTGCCTGGCTTCCTTTTAGTAAGAGAGGATGAGGTTAGATTGTCGAAGGACTACACACACACA
GAGATCATACACCCATGCACACACACACACAGACACACACCTATGCAGGAACTCCTTCTAGTCAATGGGTCTACAGCAGGATAAAA
GGAAGGCAGACAGTCAGGAGGACTTCCTGTACTCGCAGGCAGTGCCTCCTACTAGAAAAAGTAAGTGGCTACAGCTGGGCGCAGTG
GCTTGCGCCTGTAATCCCAGCATTTTGGGAGGCCAAGATGGGCGGATCACTTGAGGTCAGGAGTTTGAGACCAGCCTTGCCAAGAT
GGTGAAACCCTGTCTCTACAAAAAATACAAAAATTAGCCTGGTGTGGTGGTGCACACCTGTAATCCCAGGCTACTTGAGAGGTTGA
GGCAGGAGAATCACTTTAACTTAGGAGGCAGAGGTTGCAGTGACACAAGATTGCACCACTGCACTCCAGCCTGGGTGACAAAGTGA
AACTGTCAAAAAAAAAAAAAAAAGTAAGGGGTCAGAAGGTTAGCCTGCAGGTGTGGGATCACAGAGGTCTCCTAGTGATGGAGCTT
GTATGCTCTATGGGTTAAAAACAGACGCTAAGGAGATAAACTATACACAGAAGAAGCTTAATGGGCTGTGCACAGTGGCTTGCACT
TGCAATCCCAGCTCTTTCGGAGGCCGAGGTAGGAGGCTAGGAGTTCGAGAACAGCCTGGGGCAACATAGTGAGACACCCCCCACCC
CAACCCATCTCATTATGTTGAGAAAAAAAAAAAAAAAGAGGATCTTGAGAAACTTGCACAGCAAACTACTAAAGACCACTCAGGCTA
GGAATGAGGCGTCATCTTGGATTTTTTACAAACTACAACTACAAAAAACAATTATTTTACACTGAAGATGTGTTTCCTTTTGTTTT
CCATATGACCACTATTGGGGCCAACCCAAGACTGACTGAAGCAAATATATGACCATTTTTTTTTTTTACACATAGAGTGGAGCCAT
CCCCATTGCAAAATCCTAGCCGTCTCTTATTTGCATAACAGCAAGTCCTCTGGGAATTGAGGTGAGGGTCCAGGGGTCGCTCAGAC
GCTTTGGTAAATCAATAAGCAAGGGAATTTGTCATGGTGGAATCCATTTATTAATTCACTCAAAAAAATGTGCTAAGCACCTACCC
AACTGTGCTGGGAGTTGGGGACACTATAGGGACTGAGCCTCAGGTCTTGCCCTCCTGGGGCTCACAGTCCATTGGGGACACACAC
CGTTGCCAGTCAGGGACAACCTAAAGTGGATTGGGTTTGGAAGCCCATGGGGCTAGGGAAGCACAGCAGGGACACCTAGCTTGGA
CTGGGAGTCGGGAAAAACTTCCTGGCGGAGGGGACAGCTGAGCTGAGTCCTAGAGGAGGATAGAGAGTATAGAAAGAAATAGAAAA
AGGCCAAGGCCAAGAAAATAGCACAGCCATAGATCTGTTGGTGGGGAGAAGGCTTGATACATTGAGAACGCTTAGAGGGTCGGGTG
CGATGGCTCATGCCTGTAATCCCAGCACTTTGGGAGGCCGAGGTGGGTGGATCACCTGAGGTCAGGCATTCAAGACCAGTCTGGCC
AACGTGGCGAAACCCCGTCTCTACTAAAAAATATATATATATATATACAAAAAATTAGCTAGGCATGGTGGTGGGCACCTGTGATC
CCAGCTACTCGGGAGGCTGAGGCAGGAGAATCGCTTGAACCTGGAAAGTGGACATTGCAGTGAGCTGAGATTGTGCCACTGCACTC
CAGCCTGGGCAACACAGCGAGACTCTGTCTCAAAAAAAAAAAAAAAAAGAAAGAAAAAAAAGAGAAAACTCAGAGATTCGTGGAGAC
TGGAACCACGGGTGTGGAGAGAGGGGTTAGTAGAGACCAGATTCTGCAGGTACTATAATGACATTCCCAGGCTAAGGAGTTTAGAT
CTTTTCTTCAGGGCACTGGGGAGCTATTGCAGGTTTTGAACACGAGAGGGGCAGGGGCAGGTTTGTGATTTAGGAAAGACCCCTCT
GGCCCCAGACTGAGCGTGACTGGAAGGGAGAGGAGTGAGACTGAGCAGGAGACCAGGGAGGAAGTGGGGTGGGGACCCTGCAGGGT
GGGGTGAGGAGGAGTCCAGAACAGGGCCAGGGACATGGGACAGAGAGGAGGGGAAGGATTCAGGAGACACTCGGGAGGCAGGACAG
TTGGGCTTGGTGAGCAGCCGGCTTGGGAAGGAAAATACAGGGCAGTAATAAATAGCATGGGTCAAGTGGTCTGAGTGAGGCTGGGA
GTGGTGGCACGCACCTATGGTCCCAGCTACTCAGGAGGCTGAGGTGGGAGGATTGCTTGAGCCTGGAAGTTTGAGGCTGCAGTGAG
CTATGATCGTACCGCTGCACTCCACTTTCGGTGACAGAGTAAGACCCTGTCTCAAAAAAAAAAAAAAAAAAGGAAAAAAAAAAAAAG
ACACTGCCTATGAGTTAACCCTGCTCTACAAGGAGCAGTTTTTAAAGTAAAATTAAAAAAAAAAAAAAAAAAAGAAAGAAAGAAAGA
AAGAAAGAGAGAGAAAAAAGAAAAAAGGCTGGGTTGCCTTGGACAACAGACTTCATCTCCCTGAGCCTCCATTTCCTCATCTGTAG
AATGGGGGCTGTTAAGAGGAGTTGCAAGGCTTGTGCATGCCAGCAGTAAGTGCAGAGTGACGGTGCAATTATCATTACCCCCATCA
TCTTTATTGGGGTCAGCCTGAACCCTCGATATCCCATAATATTCACCCCCATCCTTCAAGGGTCTGCCCTAATGTTCCCATGACAC
CCGACCAGCTCAGCTCTCCTTATGAGAGGGCCTCACTTTTCTATTCCCTTTGCAGCCGTTATCCCCTTTGTAGTTGTTAATTAAGT
GTGTAATTACATGATGGCTTAATGTTTGTCTCCCCCACTGGGCTGACTGCACCAGGAGACCAAGGCCAGGGCTGTCACCCACCGCT
GCGTCCTCAGCAAATGCTTGTTTGATGAGTGAATGACAGATGAACAAATGGGCAAGTATTTGACTGATTAATCACGGCATGCGTGA
TTAAATAAATGAGTGAGCAAACGACTGAATGAGTAAGCAATTGAAGGGGAGAGATCTAGGATAATTTCCAAACTGCCAATATCCCA
GAACTGGGTAGATGACTTTTTCTCCGTCTTTTGGAGTGGGTTTTTATACTCCGGAGGGGGAAATAAGTAACGACAATCAGAGATCG
GAGTGACAGAGAATGAGTTTGCGATGCGGAAGGCCGCAGCGACCTCTCCAGGTGCAGGCTGGAGGAGCGCCTCCCGGGCAACAAGC
AAAATAAGTCCGGTTCGATAAGTAAGATTCAAGGCGCTTAGTTACTACCGCCCGAAAGGTGGAATTGAACCACTCTGTCGCTAGAC
AGCTACAGGTTTGAAGCCTGCACCCCAGACCACTGAGGATCATCCGGGCAGGACAACCTTCTTCCCGCGCAGCTATATAAGGATCG
CAAAAGCTTACCTTTTAGAGTTATGGTCGCGTCCTTTGGAAAGGTGTGAGATGGTTGACTTCAAGGGCTATAAGTTCTCCCCACTC
ATTTATCGTGAGAGATGTCCTTGTAAAACGTTGCCTTCACTTCATATAGTATGAATCATCCTCGCTCCCCAAACCGTCTCATTTAC
ATAGCGCCACGCATTCTGGGAAGCAGCGTTTGAGCACCTGGCAAGCTGTTAAAGGGCCCAGGACCCCCTTTTTCCAAAATGAAAAG
TCAGCTTTTTAAGAGGAGAGATTCATGATTCGGCATTCAGAAGCGCCTTCTCGGAAAATTCCGAATTCTCTTTCTGCGGAGGAGTG
GTGAGTGGGTTCCTGTTAGAGTCTGATTCACCCTGGCCCCAAAGGCGCTGAGCAGGCGGCTATAGGGCTGGTGCGACACCTGCTGT
TCTTTTATGGAATTGCGTAGGCCGATGGAGCTCCGCGTTCATTCAAACGTTTATTGAGCGTCTACTGAGTCCCAGGAACTGTCTCG
GTGCTGGTGACGACAGACATCCTGTCCCGATTGAGTTTAAACACTAGACATAACCAATTTCTGCACTTGCGTTTTATGGTTGGAGA
AACTGAGACAGACTCAGAAACGGATAGTACCATATTCCGGTTAAAACAGCCCATCCACTTAGGGAAATCGAACTTGTGTTACAATT
TTTATTACAAAAAAGGTGATTCTTTTTTCTTAAATTTTTACATATTTAGGAGGTACAGGTGCCGATTTCTACATGCATATGTTGAT
TGCATCGTGGTGAAATCTGGGCTTTTAGGTGTACTCATCACGAAAAATTATTCAATATTCTAAGTCAGTGATTCTTTGACAATACC
ATTCTCCTGCATGAAACTCTCCCATGACTTCCATTTCAGAGTAAAAGTCAAAGTACTCACCCTGCTTTACAAATCCCTGTAATCCA
GCCCCTTTGGTCCCTCTGACCCTGTCTCACATACTCCTCTTCCCTTGGATATTATTGGATTTAGGCACACAGATGTCTTGCTGCAC
ACATGGATCATGCTACCACCACAGGGACTTTGTGCTTGCCGATCCCCTAGCCTGGATCTCCTCTTCACATGTTCCCATGGCTCATC
GCCTCCCTTCACTCTGTCATCTGATCAAATGTCAATACCAAAAACTCACTGAGTGACCAGCACTCAGTGGGGCCTTCCCTGATCCC
AGTTTAAAATAGCGAGTGGCGGCCGGGCGCGGTAGCTCACGCCTGTAACCCCAGCACTTTGGGATGCCAAAGCGGGTGGATCACGA
GATCAGGAGATGGAGACCATCCTGGCTAACACGGTGAAACCTTGTCTCTACTAAAAATACAAAACATTAGCTGGGCGTGGTGGCGG

GCGCCTGTAGTCCCAGCTACTCGGGAGCCTGAGGCAGGAGAATGGAGTGAACCCGGGAGGCGGAGCTTGCAGTGAGCTGAGATTGC
TCCACTGCACTCCAGCCTGGGGGACAGAGCGAGGCTCCGTCTCAATAAATAATAAATAAATAAAATAGCTAGTGGCTGGGATCGGT
GGCTCAGGGCCTGTAATCCCAGAACTTTGGGAGGCCGAGGCGGGTGGATCACCTGAGGTCAGGAGTTCGAGGCCAGCCTGGCCAAC
ATGGTGAAACCCCGTCTCCACGAATGATACAAAAATTAGTCAGGCATAGTGGCTCACGCCTGTAATCCCAGCTACTCGGGAGTTTG
AGGCAGGAGAACCGCTTGAACCGGGGAGGCAGAGGTTGCAGTGAGCTGAGATGGTGCCATTGTACTCCAGCCTTGGTGAAAAGAGC
AAAACTCCATCTCCAAATAAAAGTAATAATAATAATAAATAAAATAAAAATTTTTAAAAAGCGAGCTCTGTCACTCTATCCCCTTA
CCCTGCTAGACATTTCCCCACCACTCTGATCACCACCTGCCATTTTCTGTGTCTATTTGCTTACCGTCTGTCTGCTTCTGTAGAAT
ATCAGCACCATGAAACTATGCACTTTATTTTTGATCATTGCTGTATCTCTAGTGCCTAAAAAGTGCTTGAGAACATAGCAGATGTT
CAGTAAATGTTTGTGGAATGAATAAAAGAATATCATCACTGTCTTTTTTTCATTCTTCTTCCAGACAGGATCTTACTTTGTCACCC
AGGCTGGAATGCAGTGGCGCAAAAACGGCTCACTGCAGCCTCGACCTCCCAAGCTCAAGTGATCCTCTTGCCTCAGCCTCCCTGGG
ATGACAGGCATATACCATCACGCCCAGCTAATTTTAATTTTTTTTGTAGAAACAGGGGCCTCACTTTGTTGCCCAGACTGGCCTCG
AACTCCTGGCCTCAAGTGATGCTTCTGCCTTGGGCTCCTAAAGTGCTGGAATTTCACGCGTGAGCCACAGCACCTGGCCTCACTAT
CCTTCTTTCAGCCTCAGTTTTCTCATTTGTATAACCAGACTAGTACAACTGATCTCACTGGAGAAATCATGATATAAAATTCTGAC
ACTGGCTGAGGCAACTGGGAGGAGCTCAGTAAAGGCTGTTTCTGCTGGGCACGGTGGCTCACACATGTAATCCCAGCACTTTGGGA
GGCCGAGGTGGGTGGATCACAGGAGATTAGAAGTTCCAGACCATCTGGCAAGCATGGTAAAACCCCATCTCTACTAACAATTCAAA
AAGTAGCCAGGCATGGTGGCTCACACCTGTGATCCCAGCTACTCGGGAGGCTAAGGCAGGAGAATCCCTTGAACCCAGGAGGCTGA
GGTTGCAGTGAGCCAAGATTGTGCCACTGCACTCCATCCTGGGCGACAGAGCAAGACTCTGTCAAAAAAAAAAAAAAAAGTTTTTTT
TTTTTGGCTGGAATTACAGGCGCCTGCCCCCACACCTGGCTAATTTTTGTTTTTTGTTTTTTTAGGAGAGACGGGGTTTCACCATG
TTCACCAGACTGGTCTTGAACTCCTGACCTCAGGTAATCCAACTGCCTCAGCTTCCCAAAGTGCTGAGATTACAGGCGGGAGCCAC
TACACCTGGCCAATAAAGGCCGTTTCAGTCTTCAATCTGTTTTGAGCTTGGAGGCTTTAGTCATTCCCAGACCCAAAATCTCAATC
AGACCCTCTTCCACCACTTTTTGTGATAGATCAATAAACATTTTGTCTTATGGAAGTTTAACTAAGAGTATCTTTAGAAAGTTTT
GGACAGGCGCTGTAATCCCAGCACTTTGGGAGGCCGAGATGAGCGGATAGCTTGAGCCCAGGAGTTAGAGACAAGCCTGGGCAACA
TAGTGAGACTCTGTCTCAAAAAAAAAAAAAAAGAAAGAAAGGAAAAGAAAAAAAGAAAAAAAAAGTATTCCCTCTGCTTGGCAAATC
CAGATTCAAGATATATCCCCTAAACCCTCTTTGTTTTAATTAGATAGTTGCTGCTAGGCACCTCTGTATACAAATTCTGAGGATGT
AAGGACTCCTTGGAACACCGTTATCTGTCTCCTAATATGTGACGTGTGTATGACGAACAAGTGAGTTTTTTTTTTTTTTGTTTCTT
TTTTTTGGTTTTTTGTTTTGAGACGCAGTCTCGCTCTGTCGCCCAGGCTGGAGTGTAGTGGCGCGATCTTGGCTCACTGCAAGCTC
TGCCTCCCAGGTTCACGCCATTCTCCTGCCTCAGCCTCCCTAGTAGCTGGAACTACAGGCGCCCGCCACCACTCCCGGCTAATTTT
TTTGTATTTTTAGTAGAGACGGGGTTTCACCGTGTTAGCCAGGATGGTCTCGATCTCCTGACCTCATGATCTGCCCGCCTCGGCCT
CCCAAAGTGCTGGGATTACAGGCGTAAGCCACCGAGCCCGGCTTTTTTTTGAGATGGAGTTTCCCTCTTGTTGCTCAGGCTGGAGT
GCAGTGGTGTGATCTCGGCTCACTGCAACTTCCGCCTCCCGGGTTCAAGCGATTTTCCTGCCTCAGCCTCCCAAGTAGCTGGGATT
ACAGGCATGCACCACCACGCCCGGCCAATTTTGTATTTTTAGTAGAGACATGGTTTCTCCATGTTGGTCAGGCTGGTCTCCAACTC
CCGACCTCAGGTGATCCACCTGCCTTGGCCTCCTAAAGTGCTGAGATTACAGGCGTGAGCCACTGCGCCTGGCTGCACAAACATTT
TAAACGTCAATATTTTTGTGTTTATTTTTACTATTGTCTTCTATTTCTGGCAAGCAATACTGGTTTCTGGTGGCAAGGTAACCTTT
CTTCTTAAAATACATTTGTTATTATATTATTAAATATTAAACCAATTTTCAAGAAAAATATTAGATAAATAGCAGAACAGGTTGTA
GAAAGATGAAGGTCATGTGGGGAATGACTGAGGTTTGGGAAACAATATTATAACTCCTTACTGTGCATTTATTATATGCCAGGCCC
AATGCCAAGGGCTTTACATGTAGGTATAGTTATGGTTGACAGTTTTCTTCTTTTTCTTTTCTTTTTTTTGAAACAGGGTCTTGCT
CTGTCGCCCTGTCTGGAGTGCAGTGACGCAATCTCAGCTCACTACAACCTCCACCTCCTTAGTTCAAGTGATTCTCCTGCCTCAGC
CTCCCGTGTAGCTGGTCTTACAGGTGCCCACCACCACATCCGGTATTTTTAGTAGGGACGAGGTTTTACCATGTTGGCGAGGCTTG
TCTTGAACTCCTGACCTCAGGTGATCCACCTTCCTTGGCCTCCCAAAGGCTGGGATTACAGGTGTGAGCCACTGCACCTGGCCTCT
TTTTTTCTTACTTAATTTTTTTGTAGAGATGAGGTCTATGTTGCCCAAACTTGTCTCGAACTCCTGGCCTTAAGTGATCCTCCCTC
CTTGGCCTTCCAAAGTGCTGGGATTGCAGACGTGAGCCATCGTGCCCGGCCTAATAAGTTTTCCATTTGAGGAAACTGAGGCTTTG
AGAGATGAAGTCATATGCTCAAGGTCATACAGCGAAGGGGTAGTAGAGCCAGGATTCAAACCCAAGTCTGTGGATCTTTAGACCTC
AGTGTTCTCCCAGTCTCCCCACGGGTCCCTTTGCTTCATCCTTTCCGAGTTTAGTTGTCTTTTTTGTTGTTGTTCTTGTTGTTGCT
GTTTTTTGATAGAGTCTCGCCCTGTTGCCCAGGCTGGAGTGCAGCGGCATGATCCTGGCTCACTGCAACCTCTGCCTCCTGGGTTC
AAGTGATTCTCCTGCCTCAGGCTCCCGAGTAGCTGGGATTACAGGTATGCGCCGCCACACCCAGCTAATTTTTGTATTTTTAGTAG
AGATGGAGTTTCATCATGTTGGTCAGGCTGGTCTGGAACTCCTGACCTCAGTGATCTGCCCACCTTGGCCTCCCAAAGTGCTGGGA
TTACAGGCGTGAGCCACTACGCCCAGTATGTAGTTTTTTTGTGTTTTGTTTTTGTTTTTGTTTAGAGACAGGGTCTCCCTCTGTAA
CTCAGGCTGAAGTGCAGTGGTGTGATCATAGCTCTCTGTAACCTCGAACTCCTGGCCTCAAGCAATTTTCCTGTCTTAGCCTCCCA
AGTACCTGGGACTACAGGCATGCACCACCACGCCCGGATAAATTTTTTTTTTTTTTTAGAGACGGGGGTCTTGTTATGTTGCTCAGG
GTGGTCTCGAACTTTTGGCCTCAAGTGATCTTCCCACATTGGCCTCCCGAAGTGTTGGGATCACAGATGTGAGTCATCATGCCAGG
CCTCAGATTAGTGTTGAAACTGGAAGTCCAGGGAGGCCCTGTATTTGTTCCCGAGGCAATCAGGGTGGGGAGACGTGGGTGGATGA
GAGAAGTTTGTGAGGCAGGATAGACACAGCATTGTGACTGATTGTTTGTGGGAGGTGAGTGTGTAGAAGTCCAGAGCCATACCTGG
GTGTCTGGCCTGGTGACACCGTGGGCAGTGAAAACATCTCTGAGTTGGAAATCAGGTAGGATGAAGAAGACGTTTGGTTAGATTTA
GGGTGGAAGGGGCATCCAGAGGCCGTTGGACACGTGGGTTTGGGCTCAGTTGAGGTGGCTGCACAGTAGGCAGGGTGTGGGATCCA
TTAGTGATGTCTGCCTTGAGCACAGGCATGGGAAGTGGCTGCCAAGGCTTGGCTAACCCTGGAATGGGTGGTCTGTCAGCATCTTC
CCCTCAATCATTTCTTTCTTTTTTTTTTTTTTGAGACAGAATCTCGCTCTGTCGCCGAGGCTGGAGTGCAGTGGCGCAATCTTGGC
TCACTACAACCTCCATCTCCCAGATTCAAGCGACTCTCATGCCTCAGCCTCCCGAGTAGCTGTGATTACAGGCATGCACCACCACA
CCCGGCTAAATTTTGTATTTTTAGTAGAGATGAGGTTTCACCATGTTGGCCAGGCTGGTCTTGAACTTCTGGTCTCAAGTGATCCG
CCCTCCTCGGCCTCCCAAAGTGCTGGGATTACAGGCGTGAGCCACTGCGCCCGGCCTCCCCTCAATCATTTCATAACCTACACAGA
TGGCAGAG

HUMAN SEQUENCE - mRNA (SEQ ID NO: 11)

CATTCATAAGACTCAGAGCTACGGCCACGGCAGGGACACGCGGAACCAAGACTTGGAAACTTGATTGTTGTGGTTCTTCTTGGGGG
TTATGAAATTTCATTAATCTTTTTTTTTTTCCGGGGAGAAAGTTTTTGGAAAGATTCTTCCAGATATTTCTTCATTTTCTTTTGGAG
GACCGACTTACTTTTTTTGGTCTTCTTTATTACTCCCCTCCCCCCGTGGACCCGCCGGACGCGTGGAGGAGACCGTAGCTGAAGC
TGATTCTGTACAGCGGGACAGCGCTTTCTGCCCCTGGGGAGCAACCCCTCCCTCGCCCCTGGGTCCTACGGAGCCTGCACTTTCA
AGAGGTACAGCGGCATCCTGTGGGGGCCTGGGCACCGCAGGAAGACTGCACAGAAACTTTGCCATTGTTGGAACGGACGTTGCTC
CTTCCCCGAGCTTCCCCGGACAGCGTACTTTGAGGACTCGCTCAGCTCACCGGGGACTCCCACGGCTCACCCCGGACTTGCACCTT
ACTTCCCCAACCCGGCCATAGCCTTGGCTTCCCGGCGACCTCAGCGTGGTCACAGGGGCCCCCCTGTGCCCAGGGAAATGTTTCAG
GCTTTCCCCGGAGACTACGACTCCGGCTCCCGGTGCAGCTCCTCACCCTCTGCCGAGTCTCAATATCTGTCTTCGGTGGACTCCTT
CGGCAGTCCACCCACCGCCGCGGCCTCCCAGGAGTGCGCCGGTCTCGGGGAAATGCCCGGTTCCTTCGTGCCCACGGTCACCGCGA
TCACAACCAGCCAGGACCTCCAGTGGCTTGTGCAACCCACCCTCATCTCTTCCATGGCCCAGTCCCAGGGGCAGCCACTGGCCTCC
CAGCCCCCGGTCGTCGACCCCTACGACATGCCGGGAACCAGCTACTCCACACCAGGCATGAGTGGCTACAGCAGTGGCGGAGCGAG
TGGCAGTGGTGGGCCTTCCACCAGCGGAACTACCAGTGGGCCTGGGCCTGCCCGCCCAGCCCGAGCCCGGCCTAGGAGACCCCGAG
AGGAGACGCTCACCCCAGAGGAAGAGGAGAAGCGAAGGGTGCGCCGGGAACGAAATAAACTAGCAGCAGCTAAATGCAGGAACCGG
CGGAGGGAGCTGACCGACCGACTCCAGGCGGAGACAGATCAGTTGGAGGAAGAAAAAGCAGAGCTGGAGTCGGAGATCGCCGAGCT

CCAAAAGGAGAAGGAACGTCTGGAGTTTGTGCTGGTGGCCCACAAACCGGGCTGCAAGATCCCCTACGAAGAGGGGCCCGGGCCGG
GCCCGCTGGCGGAGGTGAGAGATTTGCCGGGCTCAGCACCGGCTAAGGAAGATGGCTTCAGCTGGCTGCTGCCGCCCCCGCCACCA
CCGCCCCTGCCCTTCCAGACCAGCCAAGACGCACCCCCCAACCTGACGGCTTCTCTCTTTACACACAGTGAAGTTCAAGTCCTCGG
CGACCCCTTCCCCGTTGTTAACCCTTCGTACACTTCTTCGTTTGTCCTCACCTGCCCGGAGGTCTCCGCGTTCGCCGGCGCCCAAC
GCACCAGCGGCAGTGACCAGCCTTCCGATCCCCTGAACTCGCCCTCCCTCCTCGCTCGGTGAACTCTTTAGACACACAAAACAAAC
AAACACATGGGGGAGAGAGACTTGGAAGAGGAGGAGGAGGAGGAGAAGGAGGAGAGAGAGGGGAAGAGACAAAGTGGGTGTGTGGC
CTCCCTGGCTCCTCCGTCTGACCCTCTGCGGCCACTGCGCCACTGCCATCGGACAGGAGGATTCCTTGTGTTTTGTCCTGCCTCTT
GTTTCTGTGCCCCGGCGAGGCCGGAGAGCTGGTGACTTTGGGGACAGGGGGTGGGAAGGGGATGGACACCCCCAGCTGACTGTTGG
CTCTCTGACGTCAACCCAAGCTCTGGGGATGGGTGGGGAGGGGGGCGGGTGACGCCCACCTTCGGGCAGTCCTGTGTGAGGATGAA
GGGACGGGGGTGGAGGTAGGCTGTGGGGTGGGCTGGAGTCCTCTCCAGAGAGGCTCAACAAGGAAAAATGCCACTCCCTACCCAA
TGTCTCCCACACCCACCCTTTTTTTGGGGTGCCCAGGTTGGTTTCCCCTGCACTCCCGACCTTAGCTTATTGATCCCACATTTCCA
TGGTGTGAGATCCTCTTTACTCTGGGCAGAAGTGAGCCCCCCCTTAAAGGGAATTCGATGCCCCCCTAGAATAATCTCATCCCCCC
ACCCGACTTCTTTTGAAATGTGAACGTCCTTCCTTGACTGTCTAGCCACTCCCTCCCAGAAAAACTGGCTCTGATTGGAATTTCTG
GCCTCCTAAGGCTCCCCACCCCGAAATCAGCCCCCAGCCTTGTTTCTGATGACAGTGTTATCCCAAGACCCTGCCCCCTGCCAGCC
GACCCTCCTGGCCTTCCTCGTTGGGCCGCTCTGATTTCAGGCAGCAGGGGCTGCTGTGATGCCGTCCTGCTGGAGTGATTTATACT
GTGAAATGAGTTGGCCAGATTGTGGGGTGCAGCTGGGTGGGGCAGCACACCTCTGGGGGGATAATGTCCCCACTCCCGAAAGCCTT
TCCTCGGTCTCCCTTCCGTCCATCCCCCTTCTTCCTCCCCTCAACAGTGAGTTAGACTCAAGGGGGTGACAGAACCGAGAAGGGGG
TGACAGTCCTCCATCCACGTGGCCTCTCTCTCTCTCCTCAGGACCCTCAGCCCTGGCCTTTTTCTTTAAGGTCCCCCGACCAATCC
CCAGCCTAGGACGCCAACTTCTCCCACCCCTTGGCCCCTCACATCCTCTCCAGGAAGGCAGTGAGGGGCTGTGACATTTTTCCGGA
GAAGATTTCAGAGCTGAGGCTTTGGTACCCCCAAACCCCCAATATTTTTGGACTGGCAGACTCAAGGGGCTGGAATCTCATGATTC
CATGCCCGAGTCCGCCCATCCCTGACCATGGTTTTGGCTCTCCCACCCCGCCGTTCCCTGCGCTTCATCTCATGAGGATTTCTTTA
TGAGGCAAATTTATATTTTTTAATATCGGGGGGTGGACCACGCCGCCCTCCATCCGTGCTGCATGAAAAACATTCCACGTGCCCCT
TGTCGCGCGTCTCCCATCCTGATCCCAGACCCATTCCTTAGCTATTTATCCCTTTCCTGGTTTCCGAAAGGCAATTATATCTATTA
TGTATAAGTAAATATATTATATATGGATGTGTGTGTGTGCGTGCGCGTGAGTGTGTGAGCGCTTCTGCAGCCTCGGCCTAGGTCAC
GTTGGCCCTCAAAGCGAGCCGTTGAATTGGAAACTGCTTCTAGAAACTCTGGCTCAGCCTGTCTCGGGCTGACCCTTTTCTGATCG
TCTCGGCCCCTCTGATTGTTCCCGATGGTCTCTCTCCCTCTGTCTTTTCTCCTCCGCCTGTGTCCATCTGACCGTTTTCACTTGTC
TCCTTTCTGACTGTCCCTGCCAATGCTCCAGCTGTCGTCTGACTCTGGGTTCGTTGGGGACATGAGATTTTATTTTTTGTGAGTGA
GACTGAGGGATCGTAGATTTTTACAATCTGTATCTTTGACAATTCTGGGTGCGAGTGTGAGAGTGTGAGCAGGGCTTGCTCCTGCC
AACCACAATTCAATGAATCCCCGACCCCCCTACCCCATGCTGTACTTGTGGTTCTCTTTTTGTATTTTGCATCTGACCCCGGGGGG
CTGGGACAGATTGGCAATGGGCCGTCCCCTCTCCCCTTGGTTCTGCACTGTTGCCAATAAAAAGCTCTTAAAAACGC

HUMAN SEQUENCE - CODING (SEQ ID NO: 12)

ATGTTTCAGGCTTTCCCCGGAGACTACGACTCCGGCTCCCGGTGCAGCTCCTCACCCTCTGCCGAGTCTCAATATCTGTCTTCGGT
GGACTCCTTCGGCAGTCCACCCACCGCCGCGGCCTCCCAGGAGTGCGCCGGTCTCGGGGAAATGCCCGGTTCCTTCGTGCCCACGG
TCACCGCGATCACAACCAGCCAGGACCTCCAGTGGCTTGTGCAACCCACCCTCATCTCTTCCATGGCCCAGTCCCAGGGGCAGCCA
CTGGCCTCCCAGCCCCCGGTCGTCGACCCCTACGACATGCCGGGAACCAGCTACTCCACACCAGGCATGAGTGGCTACAGCAGTGG
CGGAGCGAGTGGCAGTGGTGGGCCTTCCACCAGCGGAACTACCAGTGGGCCTGGGCCTGCCCGCCCAGCCCGAGCCCGGCCTAGGA
GACCCCGAGAGGAGACGCTCACCCCAGAGGAAGAGGAGAAGCGAAGGGTGCGCCGGGAACGAAATAAACTAGCAGCAGCTAAATGC
AGGAACCGGCGGAGGGAGCTGACCGACCGACTCCAGGCGGAGACAGATCAGTTGGAGGAAGAAAAAGCAGAGCTGGAGTCGGAGAT
CGCCGAGCTCCAAAAGGAGAAGGAACGTCTGGAGTTTGTGCTGGTGGCCCACAAACCGGGCTGCAAGATCCCCTACGAAGAGGGGC
CCGGGCCGGGCCCGCTGGCGGAGGTGAGAGATTTGCCGGGCTCAGCACCGGCTAAGGAAGATGGCTTCAGCTGGCTGCTGCCGCCC
CCGCCACCACCGCCCCTGCCCTTCCAGACCAGCCAAGACGCACCCCCCAACCTGACGGCTTCTCTCTTTACACACAGTGAAGTTCA
AGTCCTCGGCGACCCCTTCCCCGTTGTTAACCCTTCGTACACTTCTTCGTTTGTCCTCACCTGCCCGGAGGTCTCCGCGTTCGCCG
GCGCCCAACGCACCAGCGGCAGTGACCAGCCTTCCGATCCCCTGAACTCGCCCTCCCTCCTCGCTCGGTGA

Table 3 (mouse gene: Ccnd1; human gene: CCND1)

Mouse genomic sequence (SEQ ID NO: 13)

Mouse mRNA sequence (SEQ ID NO: 14)

Mouse coding sequence (SEQ ID NO: 15)

Human genomic sequence (SEQ ID NO: 16)

Human mRNA sequence (SEQ ID NO:17)

Human coding sequence (SEQ ID NO: 18)

MOUSE SEQUENCE - GENOMIC (SEQ ID NO: 13)
CACGGGATCCAAAACCCTCTCCTGACTTCTGCAAGCGCTGCACACACATGGTACATAGACATGCACACGCACGTGCGTAAACATGC
TGGCTTTCTGATCCTACGGCTTCTAATTTAGCACTAACACTTAGACTTTTTATTCCCTGTTGCTGCCATGACTGACCCCAGACCCT
GCCTTTCAGGCTTCTCCTTGCTTTTCAGACATGGGACAGTTCTGGGGTCTCTTTTGCAAATTGAGACCTGTGCTCACCTAGCACAC
ATGTGGCCTTGGGTTCCATCCCCAGAACTGAGTGAATGAGGCATGGTGGCTCACCCTGTGATTCCAATATTCAGAATGTGGAGACA
GTAGAATGAAGAGTTCCAGTTTCAGGTACACCAAACCTTGTACCAGAAAGAAGAGGGGGAAGAGAAGGAAGAGGAGGGAGAAATCT
AGCAAAAGATGGAGGAAGCATACAGTGACTTTGAAGATGTGTTAACTTTCCCTTGCTGTAAGGAAATACCTGCGGTAATTAACTTA
TAAAGAACAGAGGTTATGTGAACTCACAGTTTGGGGATTTTAGTCCAGAACAGGACTCTATTCTACTTCGAGTCTCTAATGCAGAT
GATGAGAGCGAAATGCTGAGCAAACAGAACTGAACTGCCCTGTACTACATGGCAAATCAGAGGAAGAGTAGTCCCATCGTCCTCTC
TGAGGCCACCTTCAGTAACCTAAGGACTGCCAGGCTGTGCTGTTTGTTTGTTCTCTGTCTCTAGGGTGTTTGTTTTCGGGTTTGTT
TTGGTTTTGTTGGTGGTAGGTTTTGTTCATTTGCTTGTTTGTTTTTGATATAGAATCATGCTATGCAGCCCAGGCTGTTCTCAAAC
TTGTAATCCTCCTGTCTTCGCCCCCTGACTACTAGGATCACAGTATGCGCCACCACACCTGGCTCTAGAATCCATCTCTTGGAAGT
TCTACCACCTCCCAATAAAGCCACCCTGAGAATGAAGCCTTGGACAACCAGAGAATATTGCTGAGCCAAACTACAGCAAGAAGGCT
GACCAGAAGGCTCTGCCAAATTCACAAGCCACTGGCCCATCTGCAATAGATGCCATCTGTAAAGGAGTCACTTCTCCCCAGGGTGA
CAGATTCAATGCAACCTCAATCAAAACCCTAGCTGTGGTGGGTTTGTGTGTGTGTGTGTCTGTGTGTGTTTATGTGTGTGTCTC

TCTATGCATATCTGTGTGTGTGTGTTGGGGGTGCTAACCTGTGTCTGTGCATGCATGCACAGTGTCTGAGAAAGAGGGTCCAAGATGC
CGGCCACTGTAGGCCATGTTACTGTTCGCATGGGACAGGCCTTAAAGAAGAGCTGCAAAAGAGCCTTGTACCCACCTCCTCCAATG
AGACCCACCCCAGTCTGCTTTCTCAGTTAATCTCTTCCTCTCTCAACTTGAAGCCTGTCTGGGAATTTGCTGTTGAGTAGATTATA
GGCTTGGCCTCAGCTGGTCCACTAAAAGCAGGAGGTGAGGGCCCAGGCCTGAGGAAGCATTGGGGAGGCAGATCCCAGGCCTAAGT
TTGTTGGAGACACATTCTCCAGGACCACAAATAGCTGACATGCTGTTACCAGATTGTCATCCCAGTACCTGCAAAGTGGGAGAACC
AGAATCAGGTGTTCAAGGCCAGCCTCTCAAAAAACAAAACTGAAGTAAGTACATCAACTCTACTCTGCCTTTTAAGAGTTAAAAAG
GGGCCTGGAGAGATGACTCAGCAGTTAAGAGCACTGGCTGTTCATCCAGAAGAACCTAGGCTCAATTCCCAGCACCCATATGGCAG
CTCACAACTGTCCATGACTTCAGTTCCATAATTCCAGGGGATTTGTCATGTTCACACAAACATACATGCAGGCAAAACAGCAATGC
ACATAAAAAAAAATAAATCATAAAACAAACAACAAAGAGTTAAAAGGACAGGAGAAGAGGAACAAGAGAAGGTTCATGTGGCCAGG
TTTTCTTTTCCCCAATTTAAAAGTTTCTTCTGAGGTTTACATCTCACACCCATAAAGAGCTCAGAAGTCAACAAAGCGAGGAGGCT
TGATTCATCATGAAGTCATTTCAAAATTTCTCTTTCTGAATATAAACCAAAATATTTAATTAATTCCACATACCACAGGAAGTACA
TTACAGCCTTCAAATTCACTCTGCTTAACAGTAAACTCGTGAACGAATGCACAGAACAGACCCCAGTCACCCACCAGCACTATGCT
AAATGTCCTATGCACATTACCTGAATTACTTCTGGCCATGGCCTGGCAGAGGAAATCATCCCACTGCCCAGCAGAGATGAGGGCAT
CTTTGCCCACAGCCTCTCCAATGCCAGAGAGCACAGCTCTGTACGTTGCCTCCTGTAGTTACAGAAATGTTTTCCAACTTCCTGGC
ATCCTGAAAAGAAGTGGCTTGCCTTAAAGTGACTTTTCTTTTTCCTTTTCACAAACAGTGCAGGATGGCCTCCTGTGCCAATGACA
GGTATGTGCTACCTCATCTAGTTCTGGTAGTACGTGGATTTTCTCTCTCTCTCTCTCTCTCTCTCTCTCTCTCTCTGTGTGTGT
GTGTGTGAGAGAGAGAGAGAGAGAGAGAGAGAGAGAAAGAGAGAGAGTGAGAGAGAGAGTGTTTGTGTACCTGTTCTTTCTTGTGT
ATGTGTGTACCTGCTCTTTCTTGTGTGCACATATGTGCACCTGTTCTTTCCTCTGTGTGTATGTATCAGCTCTTTCTTGGGGGGGG
GTGTATGTCCCCCTGCTTCTGCCTTACAAGGGCTAGTATCTCAAGCAATGAGTTGGTGGAGTTACAGATGCCTGTGTCCAGCTCTC
TATGGATTGTTAAGCTGCTAACCTCAGCACACTGGTTTTCTGCCCTGTCAAATGCCTGCGATGACAGCATCCACCTGGTAAGCTGG
AAGGTGGGGTCTGCTCCTCCATCCCACCTGAGAGGGAATGATTTCCTTAGATAGGCTGCGGCTGGAACTCACCTGATCTAGAAGTA
CACTGGGGGCACACAGGAGCCATGGAGCCCCTAAGGCCCACCAGCAGCTTCTGTCCTATCCCTTCTGGCAAAGGGGCCATCTGTCA
AAGGAGGGGTTGAAAGATGGCCCAGAAGAGGTCTCCTCAAATCTCTGACCCTTACAGGACTCTGATGATAAATGATACAGTCTCAG
TAGGCCTATCCTGGCTTCATTTCTTCTGCTGTGATAAAATGTCATTGGGGGAAAAAAAAAAAAGCAGCTTAGGGAGAAAGGGCTTA
TTCTGGCTCGGAACTCCAGGTTTCCAGTCCATTACTACATGAAAGGCAGTGTGGCAGGAACTTGAAGCACATGGTCACATCATATC
CTCCCAGAGCAGTGAGATATGAATCCAGGGGTGGGGACTGGAATGATGGCTCAGGGGTTAAGAGCATCGGCTACTCTTCCAGAGGA
TGCCGATTAAAACTGTCTGTAACTCCAGTTCCAGGGGATCTGACACCCTCACGCAGACATACTTCAAATAGACATACATGTGGGCA
AACACCAATGTACTTAAAATAAAAATAAATAAATTATAATAAATAAATAGGTGCTTAGTTACTTTTCCCACCTTTAATCAGTCCAA
GACCCAAGCCTAGGGAATGTGTCACCCACTCCCACACTGGGTCCTCTCACATCAGCTAAGATAGCCAAGACACTACCCACAGCCGT
ACCCATAGGGCTTCTTGAGACTCTCTAGCAACGTGAATCGAGGTTGTGTCAAGTTGACAATTGAAACTATTATGGTGCTTAACTGC
TCCCTGATGAACAGGATGTGGCAGAGACAGGCATCCCATTGGTTAATGTCAGAAGTGTCATCCCTCCGTGGACTGAATTTTTGCCT
TTAACTTATATTTATTTGCTTGTTTGTTTATTAGTGTTTGTATCTATGTGCATGTGTGAGTGCGGCTTCCCTCAGAGTCCAGAAGA
ATGTGTAGAATCCCTCTAGAGCTGGAGTAGGGGGCTGCCGGGTATGGGTGCTGGAAATCAAACCCGGTCCTCTGGAAAAATCAGCA
AGCACTCTGACCTGTTAAATCATCTACCTAACCTCATCCTTGCTCATGTTTTAAAAATAGTTATAGTGGCCAGGTGGTGGTGGCGC
TCGCCTTTAATCCCAGCACTTGGGAGGCATTGGCAGGAGGATTTCTGAGTTCGAGGTCAGCCAAAGTGAGTTCCAAGACAGCCAGC
AGGGCTATACAGAGAAACCCTGTCTCAAAGAACCAAAAATAAATGAAATAATTATAGTGGGTTTTTGGACATTCTCTGTATGCTAA
GCACCTTCCAGATGGGCTTCCTGACAAGAGATCCGTGGCCTGACCCACACTGATTTGAAATAATGATGAAAATTCAACTTTCAGAA
GCAAAGGCTGTAGTTCCTCTCTGGCTTCTACCACAGTCAGTAACCAGTCCAGTTCCTTAGAGAGTCAGTCACCTTAGTACACATTC
CAGCGGAGCCAAGACAGTATAGTGCTCCTTATAAACATGGAAGGGCAGTTCCTACCCAGCGAGTCACTGCGGGCTTGGTGCCTCTA
CTGAAGCGCAGGCTCAACCACCGTTCACCGCGGGAGCGTCCTCAGGCTCTCGCGAGCCACTGCTGCGCGTCGCGTCCAAGGTTTA
CGGTAAGCGCGGCGCTCAGGACGACCACGTGGCGGCGAAACACCGGCCGGCGGGAATGCGCGGAATGCGCGGTGCGGCCTCGCGCG
CTCCCCAAGCCGTGACCCCGGAGTTCCCGGCGCGCGTTTGTGGGACCGCGGAGGCTAGTGCCATGGGTCCGCCCGCGCGTCCAAGC
CACCAAGCACCACAGGGAGCAGGGGCCCCATGGAGGGCTCAGCACGTGAGGCTAGACAACCTTTTCCAAAACCTGGGGCTAGGTTT
GCCCTCCCTGTTTGGGCTCCATCACAGATCTGGAACCCGCTTAGTCCCCATTCTAAAGCCCCCACTGATCTGGATTAAGAATGGAG
ATGCCTGGTTTTGCAGGGAAGCAGGTCAAAGGGCTCTGCGCCCTGACCGCCCTTCGGAGTGCTGAGGGACCAGTGCCAGGGGCTGA
AAGACCCCAGTTCCAGCAGCTGCGCCCTGGGATCGCATGAGGGTTCGCTCACCACGATTGAGCCACGTGGTCCTGCCTGGCCACTG
GTGGCTCACCTTGGCCCGAGTGCCCGCTCCCCACCATTTAGTCTGGGAAGGGCCGAGGGCCAAAGCCCAAAAGGACCCTAGGGTTC
TTCAGAGCACCTGTGATTCGTTCAGAGAGCTGGAGACCGAGCAGACATCCCTCCTTCAAATTGCATCTCTACCCTTGGGGGTCCCT
GGAGCCAGGCTTGCCGCTCCAGAGCTCATTGCACGGCGCGCGTCTATTCGCTCTGGGTGGTGGGATTTAGGGACTGAAACTTATTT
GGTAAGGCACGCTTGGGAAGATAGGATGCGACGGTCCCAGGTGTGCACAGGGGATGAGCTGAGCATACACACCTTAGCCGAAGGGT
GCCTGAAATCCGCTCAGGGTAACCTAGGCGGAGCAGCCGTGTAGCACGTGGGCTGCCACGCGCGCCCCAAAACGCCTTCTGGGTGA
GGAGAGGGAAGCCGTAATGCCTCCGGAACCTTGGGGTCCATATTCGGAAGTGTTTTCTCTGGGCGACTTGAAGGCTAAATTAACAA
TGGCTAGCTGGAGCAGAAACAGCCAAGTCCTTTCAAGTTGCCGCCAGGTATGCGGCTGCAGGTGACCCCACCTAGGTGCGTCCGCT
CTTCTCCAGGAGGGGCTACAGCCAGAGGCTCAGTATTGCCGCCCAGCCCCGCACCCCTAACCCGAGCCCCGCGCTTAGTCGCGAGG
GTTCCTCAATGAACGCGCTCCCTCCCACTTCTCAATGAAGTTCCCACAGCCAGGGATAGTGGCAAACGCAAGACTAAATCTCCGCT
CTCTTTGGAACCTTGGCTCCAGTCAGGTGGGGGTAGGGGTAGGGTTCGAGGAGGCAGGAAATCCGAAAGGGGATTAAAGTTACTTT
GAGATTTTCTTTCCAAATAACGTGGTTCCCTCTCGGCTGGGCAAGTGGCCTTTTGTCTCCAAAAGGTCACTGACAAATAAGCGCAG
AACACTTTAAGCCCAGAGCGGACAGTTCCTGCCACGAGATTTTTTTTAAAGCCAATGTAAAGTTAAAATTGCAAAAGAAAGTGGGT
GTTTGTGTTTAAGTTACTATTTCGCTGGAAAATAAAAATCCAAGTTCTTTCTCTGAGATTCCTAAAGCAGTTGTCCCGGAAAGAAC
TCTGAATTGCAGCCCACCTCCTGGGCTCGGGATTCGGACAAGCAGGGTTTCCAGGATGGAGGATGGGCGCTCCCCGCCGGGTCCCA
AATTCCTGCGCCTCCCCTACGGGTTCCTGGACGGCTCTACACGCCCGCCAACCGGACTTGCACTTTTGCTCCTCTCCCTGAGCGCA
GAGCTCAACGAAGTTCCTCGTGGAGATCTGCCCGGTCCGCCTAGTAACAGCACTGAGTCCGGATTGGCTCATGCAAATTTCAGTTT
CCTGGTCTTTCCCGCGGTGGCGGTGGTGGGGGGTGGCTGCGGGTCGCTAGCTCGTGCTGCAGGGTGGGCGAGCCCCTTTATGCCCC
GCTGCCCTTGGTGGCGCACCCTGGCTAGGCTAGCCTGCTCAGTTCGCAGGCACTCAGCCGCGGGTGATAGCGCGCAGCCCTATAAA
TCATGCTTACTGCCGCCCGGTAGGGATTTTATGAATGAAAAGGCAGCCTGGCCGCCCTCGTGCTTAGGCTAAGGCTCCCAGGCTTG
GCGGGGCCTGTGGCCAGAGCAAGCGTGGGCACTCTGCCCGGGCCACCCACCACCTTCGGAGCTACAGTGGAATCACTGTCCCGAAG
GGTCCGGACTTTAGGGACCCCAATGTCGGGGGAGGGGGAGCGGAAGGCATGAGCTCGGAAGGTGAGGGGTTTGCGTCTTGTGTGCC
GCCTGGACGCCGTGCGCTCCTTTACCAGTTTCTTCTGGGACACGAAAGTATTCCGTGGGAAATGTGTGTGAATAGTTCGCCTAGCT
TGAAGTCGGGTATTGTGAAAACGTTTCCAAAACTAGGGAACTAAATAATGGACGAAGGTGGTGGAACCGCTTTATTCTAAAAATAT
TTTATTTGAATCTATAAATTATAAACTTAGGAGTCCATTCAGTTAACCACGACCATCTATAGATTCTCTTTAAATATCACCTTATC
GGCTCACAAGTTTATCTTGATTCTCACCAGCCGCCCCCCCCCCCCAACTCAAGACTGCAATTCTAAAGGTGGAGAAACACCACCAC
CCTCAACGAAGCCAATCAAGAAGCTTCCGGTGGTCTGGTTCCTGGAAGGGCGACTAATAACTTGCAGCGATTTACTTTTCTTAATT
AAAAAATAAATTAGGAAGGAGCCTATCGTGTCTCAACCTTTTCTTCAACGGTTTATTTTTCTTGGGCAAACCGCCTGCAGTGGAGG
AGGGGATACTGGGGGACCCTGGCCAGGATAAACCGGTCACTGTATGAAGACAAATCTCAGATCCCACCCCACCCCCCAGCGAGGA
GGAATAGATGAAATAATGGCCACCATCTTGAGCTGTTGCTGGAATTTTCGGGGTTTTATTTTATTTTTGAGCGAGCGCATGCTAGG
CTGGGGATCCTTTAAAGTTCAGATACCCCTCTGGCCCTTTGCAACCACCCCAGTGCGCCAGGATGGAGCCTGCACGAGAGCTTAGG

GCTCGTCTGGCATCTTCGGGTGTTACACAGTTCCTGAATTTTACACGTGTTGATGAAATTGAAAGAAGACAGGGACGCTGGGATTT
CTAAGCAATGGGTCCGCCTGTGGGTGCCTCGTGGCGTCCTCGGAAACGCACCCATTCTCCCGGTTTAAGAACAGGGTGTCCTTGCA
CCCCCAGGCTCCCCTTCCATACATTCTTCCTTGGCTTGCGTGTGGCCTGGCCTCCCTCCTAGCTGTCCTCCTGTCCAGAGCCGCCA
CTACCCCACCTCCACAGGTCTCGGAGGACCCTCTTAGGGAAAGAAGCCCCCCTCCCCCTTGCCCCGCTCTTTCCCAGTTTGGAGAG
AAGCAGTCCGAGCGATTTGCATATCTACGAAGGCTGAGGGGGAAGGGNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNCTTTATTGCAAAGCAAAAAGTGTTTATTAATAATTGGGGGCAGGGCAGGGAGCGGC
AGTAGGGGCGCTGGGACAGGCACGAGGGGCCTCGTGGCGGCCGGGAATCACTAAATAAGACAGTGTGAGGAATATTTGGGGTGGGG
GATTCTTTGTTCAAACAGCGGACCTGGGTACCCCGCACACGCTGCCTGGCCCAGAGAAAACGCCAGGCTGCAAGGTCGCGTGGCCC
CCTAGTCAATGAGACTGACTGTTGTGCGTGCGGTTCTGGATTTGGAGGAACCTGTCTAGGTCAAACTGGGGGCGGAGGGGGACAAA
AGCACATTTTCAGATGTTCAGCAGGAGACTCAGGGGTCCCAAAATATTTTAAAATAATTTTTAAAGTGAGGCATAATGCCCTTGTA
GAGGCAAACAGCGCCCGCACCCTGCAAAAGGGGGGCCCCCGGAGTTTGAGTTGCTGGAGCTCACCCCCCAATGTCCACTGAGCTC
CTGACCCTCGGCTTGGATCAAGGGTCGTCCGATGGTTATTTTCAGGTCGTTTTTAGGCACCTAGTTATTTTTTAAATATTTTTAAA
TATTTTTTGAAAAGATGACGTCTGGGAAATGCAGCGCGGCGGCCTGGGACGCCACCATTGTGTCTCGCGTGCGCTCGAGCGGCGCC
CAGCCCCCGGCGCCCCGCCAGCCCCGCGGTGGGGTTCTCTGGCTTGTGGCTTCCTAATTTCAACAGGGGACCGGGGTCTCCGTGGC
CACCATTGCTTCTACCGGCCCCGCCAGCTGCCCAAAGACTTTCCCTTTCAGTTTCAGGGTGGGTGGGCACTGGGAACCCTCGGAGC
AGGAGCGCATCCCAGGGAGTTGGCCACCGGAATCCCTCTCTGTGCAATCTAGGAAAAGGGAATGGGGTCGTATCGAGCGACGCCCC
CCTCCCACGTTGACGGTCAGGGTCCGGATCCCCGCAGGTCTGTGAGGAGCAGAAGTGCGAAGAGGAGGTCTTCCCGCTGGCCATGA
ACTACCTGGACCGCTTCCTGTCCCTGGAGCCCCTGAAGAAGAGCCGCCTGCAGCTGCTGGGGGCCACCTGCATGTTCGTGGCCTCT
AAGATGAAGGAGACCATTCCCTTGACTGCCGAGAAGTTGTGCATCTACACTGACAACTCTATCCGGCCCGAGGAGCTGCTGGTAAC
CACGTAACCCACCACCGACAATCCCTCTTTATGCTGGGTGGTGGGAGCTGCAAATGGCAGGAGATCCAGCCATCCATGGACAGATC
TCCTGCCCCACGAAAGGCACCCGCACCGCACCGCACGACACCGAAATGCCAGTGAGCAGAATGCCAACCCATTCTACCTCTGTGCC
CGCCCTCTTTCCGTACTGGACTCCCCACCATGTCTGTGGTGAAACCACTCTGAAATGTGGGCGGTGTGCGCCCCCTAGCGACGTGC
AGGCCACGGCGCTGGCGGTGGGAGATTCTTTTTGTCCGCAGTCTGTCCCCTTAGTATTTCTACAGGCACATTTTGTTGTTTACCTG
TATGGGGTCCCGGGTTGACCCAACTCTTTAGACCTTCTCGAATCAGCCTGCCTTCCTTTTGACCGTGTTCCTCATTCTCTGCAGCA
AATGGAACTGCTTCTGGTGAACAAGCTCAAGTGGAACCTGGCCGCCATGACTCCCCACGATTTCATCGAACACTTCCTCTCCAAAA
TGCCAGAGGCGGATGAGAACAAGCAGACCATCCGCAAGCATGCACAGACCTTTGTGGCCCTCTGTGCCACAGGTAAGGCTCCACCC
CCATTCTTCCCAGGAAGAGCGGGCTCCTTGCCACTCCCCCTGGGGTTAAGGCCTCTTTCCTTGCTATCTATTCTGAGCCCAGGGTG
CAGATAGTTTGTTGGAGAGATAGATGATACTGGGTGGCACATTCTCTTGGCTAGTATCTTGGAGACCTAACAGAATGACACAGCTT
CCCTGTGCCAACCTTTCCACTGCCAGGAAGTGGTACGGCCACAGCATGCTTTCTGAAGAGGTTTTTGCCTCTTTTCCTAGGCTTCC
TGCCTTGCCTCTGTTCTTAAGGAGCTCGAGCTATAGAGCACACACACACACACCAGCTTCTCCCAGGCTGAGCCACCTGCTAGGGG
TTCCTACATGGATGGGACAAATGGTCCAGCCTCAGCCCCACGAGCTACATACAGTACAGCCCCAGAGCAGGAACTTAAGAGGCCCC
ACCAGGGGTACTGCAGGCCAGTACTTTTTGGCTGAGTCCTGCCTGGGAGGGTTGGCTGGAAACAGAGAAACTAGAACCTCCCTATG
ACTCCTCCAAGGGACCCTCTCTGGCCCCAGACCCCAGGTAGATTAGGCTGACATAAGTCATGGTAGACAGCCATGCAGGAACCCAC
ACACATAGACAACCCTGCTACTTCTGAGTGGTTTCAGTACTACTTCTGACAGCTGCTCCTCACCTCTAACTGAGAACCCTACCCAG
GAGGCTTAGCAATATTGAGGGGGTGTGGTGTTCTCCCACCTGTTGAGGACCCCCTTCCTCTGTGGCCCTGATCAGACCTCAGTTGC
TATAGCTGTTTGGCCTGTGATGTCAGAAGGGCCGGTAGAGTATCTCCCCCCACCCCCAACCTCCACCTCACCCCCGCTGTCTCAGT
GACATCTTCTTATGTCTGACAGCCCCATAGCCAGTGTTTCTAAGCTGCTGGTGTGAGAATTTGGAAATTCTAGCTTGCCAACTACC
TACTGAGAGTGCTGAGCCCGCCCCAAGGGTACCAAACAAGGGGGTGGCAGATGGGAGTAGCAAGGCCATCGGTAGGCTGGAAGGAT
CTAAGGGGTGGGGGTGGCCCTGCTGGCAGTCAGGGAGGCAGCTGGCTGGGGTTCCTCTGAGGAGAGCGGAGCCTTTGGAAGCTCCA
GGCAGCCTTTTATGGAGCTGAAAGTGCCTTCAGAGAGGCAGCAGAGTTTCCCAGAGATAACTTGGGGATGGAGAGGCACAACCTCC
CCCAGGATAGTTCTGGCAGTAGTGTGCCCCTCCCGGGACCCCTAGATGCCAGCAGAGTAATCAGTAAGCGCTATATCTGGGTACAG
CTGGACTCAGCTCTTCCAGCTCTCCTTACTGCCTTTGTCTGTGCCCCTGGAATACAGGTGGGGGTAAAGGACAGCTTGGTGACCTG
CGGTAGGCCAGTGTTCTGTGGTCTGGAGCATTTGTTTCTCTGTAGGAGCAACCAGCTCCTTTGTCACACGGGAACCACTTTGGGAA
GAGGCCAGTGTGACATCACCGTGAATGGAAAGATGCTAACATCAAGGCCAGGAGATCTGTCGTCTGTGTGACAGGGGTTCCCCCTA
TAGGAGATGGGAAGGCATAACTTCCCTTCTGCAAACCGCCCCCCCCCCAACCTGGATTCCTGACTGCAGTTGAACAATGTGCTAGC
TAGGGTTGGGGTTGTTAACACAGGTGGCCAGAGCGCTGTGTGGATGGATACAGACAGTGGGAAACTGAACTTAATGAAGTATGTAG
GTGTCAGACATGCCACTAAGCCAGGGTACACCTATGTCATCAGCAACCAGTGTTAGTAGCTAGGGATCCTTATAGGACATAACAGA
TCTTGCTACTGCCCGTGGGAGGGAGAAAAACAGGCTCTGGAAGGGGCTGGGGACTCCTGGTCCTGCTGGCTGGAGTCTCACTCCCT
GTAAAAGGGCTGTTTGTGATTTGAACTTGACTCCATAGCCAGGTTTTGGTTCACTGGACTCACTAGCATGAACTGTCCTTGGTGAC
ACCTGGAGGTATTGCTTGTCTGCTGGATCCCTTTAACAGCCAAAGCTGTATGTTCTCTCAGGGTGGTCCCCAGAGGGCCACCTCGC
TACAGCAACTCTCTGCCTTCCTGATCCCATTCCTTCCAGCCTCCTTGATCCCAGGCTCTGGGTGACACACAAGATGATTGATGGGA
GCTGACATCTGTTCATTCTTGTGTCCTCCTCTGGCCTGGAAGCCCTGATTCGCAGGCCAAGTGACCAGCTGTCCCTGTCACTATTT
CTTTGTTGGACGTGACAGGGAAGCAGTTTTCAGACTCATCTTTGGTGGGTTTATGCGGAGAGGCATTCCATCGGTGTGACATACAA
CTGCAGGATGCTGAGTCCAGTGGACCTGAGAGGTGTGGAGTTGGTGACCCCAGATGAAGTCCTAAAATGACACCCCACTACTTCCC
AGTTGGGACATGTACATATGAGAGTCCCTAAGACTCCAGGGCCCCTCATGAGGGACCTTGGCCTGGAGATCTGAGGTGGCTATTCC
CAGCTCCCTCTGAGTCTATTCCTACTGGGTTTTCCTGGACTGGGGTCACATGTCTTCCCCAACCTGGGTCAGTAAGCCGGGAAGGC
TAACTCCCTTCTGTGGAGTCTAGCCTGGCCTCTGAGGAACCTGGGCCTGTCATCTCTACAGCTTTCTCCTGTGTCTGAGATGTGTC
TCTAGTCCTTGGGGTGCTGCCAGGCTGACTCTGGAAGAGACAGAGATCCCTGCCTCCCACCTCAGCCGCGCAAATACTGGGATGCA
GATGTGAAAGCACCACACCTTCATAGATTCCATCCTCTTAGGGCGACATAGCCAACCCCTCCTTGATGTTTGCTGGAAGTGCTGGG
GATTGAACCCGGTGTGTATGCCAGGCAGCATTTTAAAGTTTGAGCCAAGGCCTTATTGACAGGTAGCATCAAGGCTCTGGCCTGGA
CACCGTAATCCTCCGATGTGCTGGGGTCTAGAGATGCAGCACTGTGTGCACCTAGTGCCACTTAGGTGTCTCCAAGGGAGCTAGAG
TTGATGTGGGCATCCATTGGAAGGGCTTGAAGACCAGCCCCGAGGCTGTGGGTGGGAGCTGAATCATAGGCATGTCTCTGTCCCTC
TCGCTGTAGATGTGAAGTTCATTTCCAACCCACCCTCCATGGTAGCTGCTGGGAGCGTGGTGGCTGCGATGCAAGGCCTGAACCTG
GGCAGCCCCAACAACTTCCTCTCCTGCTACCGCACAACGCACTTTCTTTCCAGAGTCATCAAGTGTGACCCGGTGAGTAAGTCTCA
TCAAAGGAGGCTACAGCTGGCAAGGGCCTGGGAACCTTGGTGGGCAGTGCCAAGGCACTGTGTGACTGGGCTGGAGCTGAGAGGTG
CCAAATCTGGGTCCCCACGAGCCTCTTCCCAGAGTCATGGGTCCCAAAGGCATAGAGGGCTGTGGTGTGGTGACAGTGCCCCTGCA

ATGTGTATGGGTAGTGGCATGTGAAGCCCGGTCTGGCCCAGCCAGCATAGGACCATATCTCCCTCAGTGCAGACGTTGGGGATTGA
ATCCATAAAGGACTCTGGTATAGACCTACACATTCTAGAACTTTCTTTGTGTGAGAGAGGGGTTTGCATCAGGCAGACTGTGCTAC
GTCTAACTGTACCTTTCTGCCAAGGAGTACAGCTGATCATGCTTGGGGCTCTGGCCGTGAATGTTTTCTCAAGGGCATCACAAGTT
GGCATAGACAGGCAAAAGTCCAGTTCTGGCTTGAACTTCAGTTGGGGGTTCAATGGAGGTCAGAGCCACTGGCATGTGGAAGGCTT
CCAGGTGTGACAGCTGTTCTGCCCTGCCTGGCATCAGCCACAGGGACACCCCGTGCCCCATTCCTACATATCTCAATTGTAGAACA
TGCAGCCCCCTTCCCCTAAGTGTCTGGGGCTCCTGAGAACTGAGAATAGGGTATGTGGGCAGGTTGGAGCCCACAGGCTTCAGAGA
GGGAAGGGTGTCTGTGGGTCCATACTAGGCTACTTGGGCCTCACTTTCTCCATCTGTGAAATGGGGCCATTGCCCTTCCATCCTCT
AGCCATGGTGTTCATCAAACAAAGGGGGAACAATACACAAGTTTTGAATGTGGGGGCTTCCCAGGTGTTCCCCAGGAATCCTCCTG
GCTTCAGGAATATAGACAGTGCCCTAGCTTCAGCCTGCCTAGGCAGGGCTTCAGTTCATGAGTCTTATTCCTGGGCAAATGCTTTC
CAGACCCTACCTGTGCTGGAGCTGTCCTCTCTCAGAGGCCACCCAAGGCAAAAAGTTATGAGAGATAGACGCTCCTTGTGGGGAAG
TTCAGGGTACAGCCTGGGAGGAGGAACCTAGAGAGTAGCAGTACCTTTCTAGTCTCTGGCAGCTCTCACCGGATTTAGGGGTGGCT
TGGGGAGTTTCCTCAAGCCTTGCTCTTAGCAAAGCCTGCCTCGCAGGTCAAGGCCCTGGCCACCGGCCTCTGGCTAAACAAGGACC
CCCTCCATCTCCGCCCGCCTCTCCAGGACTGCCTCCGTGCCTGCCAGGAACAGATTGAAGCCCTTCTGGAGTCAAGCCTGCGCCAG
GCCCAGCAGAACGTCGACCCCAAGGCCACTGAGGAGGAGGGGGAAGTGGAGGAAGAGGCTGGTCTGGCCTGCACGCCCACCGACGT
GCGAGATGTGGACATCTGAGGGCCACCGGGCAGGCGGGAGCCACCAAGTAGTGGCACCCGCAAAGAGGAAGGAGCCAGCCCGGGTG
CTCCTGACGACGTCCCCCTTGGGGACATGTTGTTACCAGAAGAGGAAGTTTTGTTCTCTTTGTTGGTTGTTTTTCCTTAATCTTTC
TCCTTTCTATCTGATTTAAGCAAAAGAGAAAAAAATATCTGAAAGCTGTCTTAAAGAGAGAGAGAGAGAGAGATAGAATCTGCATC
ACCCTGAGAGTAGGGAGCCAGGGGGTGCTACAAAAATAGAATTCTGTACCCCAGTAATCAACTAGTTTTCTATTAATGTGCTTGTC
TGTTCTAAGAGTAGGATTAACACAGGGGAAGTCTTGAGAAGGAGTTTTGATTCTTTTATATGTTTTAAAAAAAAAAGCTTAAGAAA
CATTGCTTTAAAAAGGAAGGAAAAAAAAATACAGCAAACCATTGTTAAAGTAGAAGAGTTTTTAGGTTGAGAAATGTACTCTGCTTT
GCTGAAAAGCCACAGCTTAGGCCCTCAGCCTCACTCCCTGGCTTGCTCAGTGCCTACAGCCCTGTTACCTGATACCTGTGCTTTAT
CCCAGGGGTGGGCAGACCTCTTAACCTTATAGATGGTCAGTGCGACCTCTAGTGGTCTCATGGCGTGTGGCACAACCCCCCTCCCC
AGGGCTCAGCTTAATGTGCCCTCTCCCCCCAACAACCTGCAGGTTCACAGCGCCAGCCACACAGCGGTAGGGATGAAATAGTGACA
TAATATATTCTATTTTTGTAACCTTCCTATTTTGTAGCTCTGTTTAGAGAGATGCTGGTTTTTGCCTGAAGGCCCTGCAGCCTGCC
CACATCAGGTTAAACCCACAGCTTTTGTGTGTGGTTTGTTTTGTTTGTGTTTTCTTTCTCTATGTTCCAAAACCATTCCATTTCAAA
GCACTTTTGGTCAGCTAGCTGGAGGCAGTGTTGCTGGTGTGTGTTGGGGGGAGGGGTTCTAATGGAATGGATGGGGATGTCCACAC
ACGCATTCAGATGGCTGTACAACAGGTTGTAGGGCTGGTAGTATGAGGTGCTTGGGAAGTTTTGTTGGGTCAAGAAGAGAGAACTC
TGTTCTCGCACCACCGGGATCTGTCCTGCAAAGTTGAAGGGATCCTTTGGTGCCAGCTGGTGTTTGGAAGTAGGAACCATGATGGC
ATTACCTGGACAAGGAGATTGGGGACAACTCTTAAGTCTCACACAGGAGGCTTTTAAACACTAAAATGTCTAATTTATACTTAAGG
CTACAGAAGAGTATTTATGGGAAAGGCTGCCCATGACCAGTGTGACTCAAAGCAATGTGATCTCCCTTGATTCAAACGCACACCTC
TGCCCTGCTGGAGAAGGTTTAGGGCCATGTCTGAGAGATTGGTCTTTTATTGGGCAACGGGGGGGGGGGGGGGGTCCTTAAAAAAA
AAAACCACAAAGACAGAGATTTGGTCTGCTTGACTTTCCCAACCCAATTGGCCCCATTGGAGAGCCATCCAAACTGAGGAAAATTA
GGGGACTCCAAAAGAGTTTGATTCTGGCACATTCTTGCCGCTGCCCCCAAGTTAACAACAGTAGGTAATTTGCACACCTCTGGCTC
TGTGCCTTTCTATTAGGACTTTTTGGCAAAAGGTGGAGAGCGGGAGGCTTAAGAGGGGATGTGAGGGAAGAGGTGAAGGTGGGACC
ACATGGGACAGGCCACGGCTCCTCTCATGGCGCTGCTACCGATGACTCCCAGGATCCCAGGCGTTCAGAACCAGATTCTCATTGCT
TTGTATCTTTCACGTTGTTTTCGCTGCTATTGGAGGGTCAGTTTTGTTTTGTTTTGTTTTGCAATGTCAGACTGCCATGTTCAAGT
TTTAATTTCCTCATAGAGTGTATTTACAGATGCCCTTTTTTGTACTTTTTTTTTTTAATTGTGATCTATTTTGGCTTAATGTGATT
ACCGCTGTATTCCAAAAAAAAAAAAAAAAAAAAAAAAAAAACAGGTTCCTGTTCACAATACCTCATGTATCATCTAGCCATGCACGAGC
CTGGCAGGCAGGTGGGCGGTCTGCCTCCAGGGATCCTGGGACCCTGATGGCGATCGTCCTGTCATGCTGGGCCCTTCATTTGATCT
GGGACATAGCATCACAGCAGTCAGGGCACCTGGATTGTTCTGTTATCGATATTGTTACTTGTAGCGGCCTGTTGTGCATGCCACCA
TGCTGCTGGCCCGGAGGGATTTGCTCTGAGTCTCCGGTGCATCATTTAATCTGTTAGGTTCTAGTGTTCCGTCTTGTTTTGTGTTA
ATTACAGCATTGTGCTAATGTAAAGACTCTGCCTTTGCGAAGCCAGCTGCAGTGCTGTAGGCCCCCAAGTTCCCTAGCAAGCTGCC
AAACCAAAACGGGCACCACCAGCTCAGCTGAGGCATCCCAGCCAGGCAGGACCCTTGAGGGCCGCTGTGTCCATGGTGATGGGGTG
AGGTTTTGGCCAAAAGGCCAAAGACTGGTGGTGGGTCCACGGAATCTGCCCTGTGACATGAAAGGCTTTGAGGGACTCTGGCTGGT
GGCCAGGTTGGCTTTTTGTATTTCTGGTTGACACACCATGGCGCTTCCCAGCACAGACATGTGACCAGCATGGTCCAGGAAAAAAA
AAAGACAAAAAATCTAGAAAATAAAATTGGTAAAATCTCAGCTCACTGTTGTCTGTGTTTTCTGGAACAGGAGTGGGGTGATGCC
ACAGCCATGTGGGGTGGCATCTCTGGCCCTGGCACCAACCTGGGATTCCCAAGGGGAAGGGTGTATGCAAGTGACAGCCAAGAGCA
AAGTGGGAGCCTCTGGCGTCAGGGAGGCCAGCGACAGCCACTGATCAGAAGCGGCTCTGGGTCCATCACTGCCAGCTCACCCTCCT
GCTTATTTGCAGATGGAAGATCAACAGAACCCGCGTTCACTGCCCCTTGTCTGGCGCAAATGGGTGTTTGCTCTGGGTGAATCACC
GTTTCTTAGGTGCTGCCAAGCGCCCAGAGAGCCATGCTTAGTGTTAGGGCCAAGACCCAAGGTGGCCTTTCACATAGTTCTGCAGA
CCTGAGTGGCCTGGCAAGACTCCTGTGATGTCTTCGGGGTGGCAGTTACTCATGACTAGTATTGTTTCCAAACCCTGTGGGGACAG
GTGGCTTTTAGACATCATCTTCTACAAGGTAGCTTTTGGGTCTCAGCTTGGCTGTTTTGGTGCCTGGTGGGCCCCTGCCCTTCTGG
AAAGTGGAGGGTGTAGAGGACACCTGGTCTGAGTGGTGCCACCCAATGATGAAAGCAGAAAGAGATGGACGGAGGGAAGCCCACAG
TGACCTTGCCAGGCGACCTACTCAATGAGAGTATATGGGAACACCAGAGCCAGAGGTGGCTTATGAAGACACAGTACTCATCTTGT
ACCAAATTGTCTGATGGCAGGATCCAGAAGGTATTTTGTCAGCCCAGCTCCATATAAGCATCCGAGCCGGGTCCACAGTCACATGG
CATCCAGGAGAATGGGCATGGGGCCGTCAGCATTCCAGGAAATGGCCAAAGGGCATTGAGGTGGAAGAGTGCTTTCCCCCTTGGTT
CTTAGAAGCACTGGGGAGCGAGCTTATCGTGTCCAGGTAGGCTGACATGAGGAAATCCGCTGGGGTGCAGCAGCCAGGCAGCCAGT
GCAGCAGAGTGGTACTAGGAATGCAGACACAAATAGCAAGGGGCTAAGCATCCACTCAAAGGCTGCTGCTGCTGCTGCAGCTGCTG
CTTTCCACCACTGCCTATCAAAGGTGCTCATGGAAGGGCGCCCAACTGGAATGGGCAAGCATCACCATCAACTGCACAGGGCTCAC
ACATGGTGCCGTCTCTTAACGGTGCAATTGGCTTGTCTCGTCATCCAGTGGTCTGTGATTCCCAGCTGTGGCTGATAGCCAAGCCT
CTTTTCTTGGTTTTAGCAGTGAAACCCCAGATACCACTGGCCACTCCAGGCTCCAACCCTCAAGTTTAACTGGTAACACTGGAGGC
TAGATCCTCTGCTGGGTGTAGAGATACTCCTGTTCTAAGGGTGATGGTAAAATAGCTTTTTGAGGGCCCTCAGAGATAAGGACTCA
GGGAGCCAGCACACCCAACTGTCTCCTCTCAGAAGCTCAATGGCTGGTTCTCTCTCTGCCTCTACCAGCTCCATGGTCCCCTGCCC
TGTGTCCTGGCCAGGCTCTCCTTTTGCAGGCGGCCTCCATCCTGCTGAGCCATCCTACCTCTCTCAGCCAAGCCAAGCTCTTGAAC
CTGCCTGGCCTCTCTGAGCCTCAGCTCCCTGAACTGGAAAGATTGCCCCTGGCTCCCCATTCTGTGTTGCCCCACTGCCTGGACCT
GCTCCAGCCTTCCTGTTTGTCCTACAGGACTCACAGTGAATGTGGCCCCTGTGGAGCATGTGGAGCATGACATTGCACAGGTGACT
CTGTGTAGCCTCTCCCCTCCTGACTCCACATCCTAGAACCAAGTGTGAGGAAGTTAGCCTGTTGCCCATGGGTCAGCTCAACTGCT
GCACTTTGTCCCAGAAAGCAGGCCAACTTCACCAACCCTACAGAGGATCTGGGCTGCTGGGGCCCACAAAATGCCAGCAGTGGGAG
CTGAGGACTCCAAGGGGTGGGACACAGGCAGAAGAGTAGGACTGTGTGTTCTCCCTGCAGTGCCCCAAAGCCCTGGCCACAACATC
CCAGGAGACAGTCAATGCGGGTTGGGCTGTGTGTTTGGTGGCAAGGCTATGCAGGCCACATAAGGCCAGTGCTGGGAGCTGGCACT
GCCAAGTGTGCTTGCCAAGCAAACCTGTACTTGAGCTGATAAAGCAGTCTGTTGAGTGTGTGGGGTGGGGTGTATAGGTAACATGA
GGCGGGCATGTTGTATACAGGGCCCCACGTGGGCACAGCACAGTGCTGGTGGGATGCAGTACCCCTAGTTCTGAGCTGATCTGGCC
TCTCTTACTCAGGGATGTCATCCCAGGAATGTATTTGATCAAACAGTTGTGCAAGAGGACAAAGCAAGCACAGGANNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN

NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNACTAAGTAACTGCTAGATCCTTGGACTTCCATTCACAGCTGCTACTGAACCATTGTTGGGAA
TTGGACTGTAGACTGTAAGTCATCAATAAATTCCTTTACTATATAGAGCCTACCCATAAGTTCTGTGACTCTAGAGAACCCTGACT
AATACAACATGCATACACATAAAATTAAAATGTGCCAGGCAGTGGTAGCACATGCCTTTAATCCCAACACTTGGGAGGAAGAGGCA
GGTGGATTTCTGAGTTTGAGGCCAGCCTGGTCTACAGAGTGAGTTCCAGGACAGCCAGGGCTACACAGAGAAACCCTGTCTCGAAA
AACCAAAAAAATAAAAATTAAAAAAATAAGTAGGTAAATTAAATTAAATTAAAATGTTTAAAAAGAAGAAATCTTGGCTGAATATT
CTACAGTTTTTTAAGAGTTTGTCAAACATTTTGATGTTACAATGGCCAATGATGACAGTAGCCCCTCACATAAATACATAGTACAG
AGGGCAAAAGTGTGCACAGGGCCAGTTCAGAAATTGCTGGAAAATTCTGTCCTGACCCTAAGACAAGTTTCACTGAACAATTTTTT
TATGAAAATGGTCGGGCTGGAGAGATGGTTAAGAGCACTGACTACTCTTCCGAAGGTCCTGAGTTCAGGTCCCAGCAACCACATGG
TGGCTCACAACTATCCATATTGAGACCTGACGCCCCCTCCTGGTGTGTCTGAAGATAGCTACGGTGTACTTACATATAATAAATAA
ATAAATCATCAAAAAAAAAGAAAATGGTCAATTTTTAAAATCAGACATTCTAATATAATACAAACCATAGCTATACTAATTTGACA
CCTGTTGAGGCCTGAGAGCACAACACATTAAAGACTTGGCTTGCCATAAGGAACCCGTCATGCCTCTGTGAGAGCAGAGAGCTGTG
TACAAGATGGATGGACGTTGTACTTCTTACAATGCTGGTCTGGGTTGAGGGGGTTTCATTCGGGTGCTGTGTGTTGGTGCAGATGG
AATGACCTCGTGCAGTACAGAGCACAGAACCGAGACTGCATTGAAGTCTTGGGATGCAATATAGGTGAGCGTCTAGAAGCACCCTT
AGATCTGATTATGCATTTGATGTACTTCATTTTTAGACAGGACCTCACTATGTAGCTTTGGGTGGCCTGGCTGAGTAGACCAAGCT
GGCCTCAAATTCCAGAGGATGCATCTGCCTCTGCCTTCTGGGTGCTTGGAATCAAGGTATGCGTGCCACCAGGCCCAGTAAATTTA
ATTTTTTCAAATTAATTCTTTTCTCATGTTTAGAAATCTTTTCCTGGTTTTTGTTGTTGTTTGTTTGTTTGTTTGTGACTCCTACA
TTGGGTTTTTGTGACTCCCATACAGAATTGGGATCCACAGTTTTTAAGAAACTGGGCACTAAATTACCCCTGCCTTATGCAAAGCC
CACAAGGACAATCTGGAGAGACCGCTTATGTGAACAGCAGGAGCTGACTCTGTTCAGGGAGCTAAAGGGTAAAACTGTGTATGCTG
GACCACTCCATTTCTGGGGGTTCCTGGCAAGCCTTAATGTTACTCATCTGGACCTCCCTCTATTTTCAGGCTGTCTTGGGTCTTTA
CGTTGTAGCTTCTCATGGCCTCCTCCTCAGGGTCCACTACCACCCACCTTAAACAATGCCAGTTCCAGCCTTTCAAGCTGAAATCA
AGTGTAAAAGGCCCAGCCATGCTTGAGCAGGCCTTCCCTGCACAAGTTCAACCACAAGCAAACTTCTGAGTGAACTGGGTGGCTAG
GACCCTTCCAGAGCAGACTTGGCAGCAGGCCTCTGAGCTTGAGAGCAGGTTCCAAAACTCTTGGTTTCAAGGGTCCGCACCCACTG
GCAACAGTAAGGAACCTTCTGGGAGAATGGGCAAGCACTTCAAGTCTTCAATCCTGCAGACACTTGGTCTTTAGGCACTGATGCAA
CCTCTCCAATAGAGGACTAAAGGACCCACCATCCATGAAGCATGGGGACAGGCACTCCAGCCCTGCAACCCAGGAATGGCCAGGGT
CTTATGGAAAGTGCCTCTGGGAAGCTGACCACAGAAAGGCAAGGAGTGACTTCCCTTCTTAGAATATTAACCTGAGGTCACCCTAA
CTTCTAGGAAATGTGAGCAAAGCCCTATTCACCCCAGATGGGGCTCCAGTGACAGACCAAAGTATACTTCTGCCACAGCCCAATTT
GGTGAACCAATGAGTTTCTAAGTGGTTAGTTGGTTATAGGAGTGTGGGAGACACCCCAGCTACAGATAATGATCTTGTGGGAGTTA
AAAAAAAAGGGTCCCCACTTCTAGTATACTCCTGAATCCCCTAAGAGCTCTGCTGCTGGGGACAACCAGGTGAAGTAGTGGCTGGA
ATCCCAGGGTCCTCCCTGCCTTCTCCCTGTCTGTCACCCCCTTACCATGTGGTTATCTCTGTAACTAGTTGCCACAGCAACCTACC
CTACATCATCATTAAGATGGTGGTAGAGTGAACTGAACTACAACACAGAGCCACAGGCTGCTGACCTTTGAGACAGGTTCCTGGTT
GTCAGGGCTCTGGGGTCCCATGCCTCATTTTCTCTGTCAGCCCTGTGACTTCAGTTTCCTGAGATACACAGCAATTTCTAGCTCTG
CTCATAAAGCTGGGAGCCACTAAGGTCAGTGGGGCAATCTTTCTAGTCAGCACACTTTGGTCATCTCCAGGTGGAACCTCAAAGTG
AACATGGGAGTCTCCCTCCCAGGAAGTCTCCCTGCCACCTCCTCAGGTAACTGAGTGCTCCCCTACCTCTGGGCTCCCAGAAGGTG
GCAGCCATTATCAGAGAGCCCACACGGGGTCTGAAGCATCTGACAGCCCTTCAACACTGCTCCTTCTCTATAAATCCATGCTTTCC
TAGACCTGCTAGATTCAAGACATGCCATAGGCTCAAATGAGCAAGAAACTAGGCTGGAGCAGCAGGCACAACTCTCACCCCACTAT
GCTTTGTCCCGCCTGGAATTGGCCCTTTACTGTGACCTCCTGCAGTCTCTCCACCAGCCCACGTTGCTGACTACAGTCTGGTACAT
GTAGACTTAGTGGTATGGGGAAGTCAGGGCTTAGCAAACAGAACTGTGGCCCCTTCTTCCAGTTTCCTCATCTCACTACCAGGCAG
AGGAAGAAAAGGTTTGGGGTAAATAGAAAATAAGGAGGGAGTGAGCCAGAGTTTCTGGGTGTTTGGTCTCAACACAACTATCCTAC
AAACATCACCATCAGCAGGGCTGGGGGATGGAGAGGTGGTTAAGGGTGCTTGATGATGCTCTTGAAGAGGACCCTGCTTCAGTCCT
GACCACCATATGACAGCTCACAAGCACATGGAACTCCAGTTNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNAGGCAGGCGAATTTCTGAGTTCAAGGCCAGCCTGGT
CTACAGATTGAGTTCCAGGACAGCCAGGGCTACACAGAGAAACCCTGTCTCGAAAAACCAAAAAACAAAACAAAACAAAACAAAAC
TCCTCACTAACACTAGCTTTATACAGAACTTCAATCTGTATTTAGCTCCAGGCTACAATTGGTCCTGCAGTCACAGTTAGGGATTA
CATAGTGTCTCTGCAGGCCCAAAAGGCCTTCTGGATCACAGTTTAGACAGACTGCAGGACCATTCTGCCAGGCCCTCTCTCTGTTT
AGTTACTTTTCTGTGTTGACAAAAAAAAAAAAAAAAAAAAAAAAAAAAAGGCAATTTGCGGGATGAAGGAGTTTATTCTGTCCAGT
TTATAGGTGGCAATCATTATGGTGGAGGCATGACAGAAGGAGCCTGAGGCAGATGCTCATATCGCATCCACGGCCGGTGAACACT
GTTGCTCAGCTCACTTTCTCCTTTCTTTTCAGTCCAGGACCCCACTTCATGGGACACAGCGCATTCAAGGTGGGTCTTCCCTTCTC
AGTTAAGCCTCCCTGAGAAGACCCTCCCAGGCACACCCAGAGGTATCTACGAGGTGATTCTAAATTGTCTCAAGGTGACTATCAAG
CAGGCAGCAAGCTGAGCAGAAAGACTTGGGAAAGCCAGCCCAGAAACCTCTGCCTCAGACCTCCAGCATGTGACAAGTGATTGCT
CATAAGGGATGACATCTAAGCTAGAGTCACAGGAAGCCAGAACAGGGTGGGATGAGTGATAGAAAACAGACCGGTAGTGGGGACAC
ATAGCTCACAGGACATGGTCTGAGAAGGCAGGGGATTGACCAGCAGCTCAGTGATTATCAAGGACACAGGTTCCACTGGTACCTGA
CTCTGCCACCTTCAGCGACAGCTTGGCCACAGCTCCCACAGTTGTCTATGGCAGTCCAAGTAACACCTTCATGGTACAGCATCCAA
AAGCAGAGCATTCTTCTGACGACCTATGCCCTCCCCTTTCACAGCAGGTGTGACTCAGTGGTGGGAGGAGGCGGCATGCCCACGTG
GCAGAATGGTCCTGTAGTCTGTCTAAACTGTGATCCAGAAGGCCTTTTGGGGTAACCAAAGCATTCTGGGGACTTGATGGGGACTT
TGAGAAAGGGTCAAGTGGGCAGAGGTCAAGGCTTGTACAGGAACATGGAAAGTTATCAGGCAAGCGGCTGTGGTGATGCTGATGGG
TGGGAGATCTGACACCTGATGTACACAAGGTGAGGGGATAGGAGGTAGAGATGAAGGGAGAAGAGAGGGGAAGAGACAAGAGAGGA
ACTAGCAACTGATTGATACCTGTAGTGAATGTGACAGGCACAGTAGGCCCATATCTGTGAACCTACATCTTGACATGCACCATCTT
GGCACTGAAGGACTGCTGCAGCTGTCAGTAGAAATGATCCCAGACAGACTACTGCTTTAAAATACACTATTTATTCTTTTTTCTGA
ATATAATAGGAAAGTAAAATATGGTGATAACAGCAGCTCAGTTCTCACACCGAAGAGTCCCTGATCCTGGGCACCTGAGAGAGTGG
TGCTGAATACCTGAGAGGTAGCCGCCAGGCCCAGGAGGCCAGGTGCATGGTGCCCCAAGCCTCAGACTCAAAACATATTTACAAAT
CCTTCGGACATACAGCTAGGCTCTATTCTGACTAGATTATAACTGAAGATAACCCATTTATTTTAACCTGCATTCTGCCAGCTGGC
TGGTTACCTGTGCTCAGGTACCATGTGTCCATCTCCTCACATCCTCCCTGGTAGATCTCCCACCTGGCTCTATCCCAGAATGCTTT
CTCTCTCCCAGATGTTCCGCCTATTTCCTGCCTAAGCCATAGGCCATAGGCTTTTTGCCACAGGTGAGGCATCCATACAACACAAA
GATATTCTCTCTACGAAATGCCTGAGAGCACTGTGCCACTCCTGCCAGCTCGTCTTATATCTGCCTGTAAATACCATGCCAGACAC
ACAAGCTGTTGCTAAAGGAAGTACAGTGTCACCAGGCAAGCCCTACACATTCACAGTGGCGGCCACCAATTAGTCCTGCTTTGGTT

TCCAATAGTTCCCCTAATGCCACTTTTTGTTTGTTTGGTTTTGAATTCACTTTGTAGACTAGGCTGACCTCAAACTGAGAGATCTG
CCTACTATCTCTTGAGCACTGGGATTAAAGGCGTGCACCACCAGCACCCAGCTCCATTGCCACTCCTTAATGTATAGAGCTCACCC
CACACCTGTTAGGAGCAGCATGAAGGCCCTGCTTCAGCTTCCTCCCTTGTCTCGTCACCCTTGTCTTCTCAGCTGGGCATCCAGAG
GCAAACCAAGTCCAGTGGAGCCTGAGTCCTGTGCAGATAGCAGGAATCAGGTCCTCCAGTGGTGTTGGCAATGACAGCGCATGTTG
GAGGCAGAGGCACCATGTGGTACTTCAGGGCATGGACCTGGGGTGGGGGCCAGCTCTGCCACCAGGCCCTTAGACTAGATGGGTCT
ATTTTATCACTTCTAAGCCATAAGGTATCCTCCACCTCTGGGGCTGCCAGAGGGATGGAGTGAAACAAGGAGGCCCAAGCATGGCC
AGCAGAGGCTCAGCAGGCAGCAAAGATCCTGTCACGGCTTCCCATGACCCATTTCACATCGGCCTGCCTCTGTGAGTCCCACCCTC
AGGGTAACCCTACCCAGGGCCACTCACTCTGGCCAGTGACAAGCGGTCTTTTGAGTGTGTTGCCCCTGTGCAGAAGGCAGGGTGGT
CAGGTAAGCCACCTAGGCAGAGACTCAGGGAAGAGGCCAAGCTGGTCCTCCCACAGAACAATGGATTCCTCTCAGGCTTGCCTTTA
ATCACTGTGACACTTAATGTGCAGACATCTGCTCTTCTGTGGGTCCTCAAAACGCAAGTCCAGAGCCTCCTGGAGTCACCTTGAAG
TCTGGCTGCACGTTGAGCAGTGAGCACAGCTGAAAGTACCCAAGAGCCCATGCCTTACACCTCCTGCACTTCTGCTCAGCTTTCCC
TATCCCAGCACAAGCCCAAAGGCTATGTCGCCTTAACGTCCCCAACCCACTTTCAGTCATTCTACAAGGGCCAAGGAAACAAGTTC
CTCTGGGTCTATATCTGGGCACACATTGCCCAACACAACCATCACATGAACCCTAGGGCCCACGCCAACCTGGCCTTCAAAGCCCC
CTCAGGCACACCCCATAGAC

MOUSE SEQUENCE - mRNA (SEQ ID NO: 14)
GAGGGCTGTCGGCGCAGTAGCAGAGAGCTACAGACTCCGCGCGCTCCGGAGACCGGCAGTACAGCGCGAGGCAGCGCGCGTCAGCA
GCCGCCACCGGAGCCCAACCGAGACCACAGCCCTCCCCAGACGGCCGCGCCATGGAACACCAGCTCCTGTGCTGCGAAGTGGAGAC
CATCCGCCGCGCGTACCCTGACACCAATCTCCTCAACGACCGGGTGCTGCGAGCCATGCTCAAGACGGAGGAGACCTGTGCGCCCT
CCGTATCTTACTTCAAGTGCGTGCAGAAGGAGATTGTGCCATCCATGCGGAAAATCGTGGCCACCTGGATGCTGGAGGTCTGTGAG
GAGCAGAAGTGCGAAGAGGAGGTCTTCCCGCTGGCCATGAACTACCTGGACCGCTTCCTGTCCCTGGAGCCCCTGAAGAAGAGCCG
CCTGCAGCTGCTGGGGGCCACCTGCATGTTCGTGGCCTCTAAGATGAAGGAGACCATTCCCTTGACTGCCGAGAAGTTGTGCATCT
ACACTGACAACTCTATCCGGCCCGAGGAGCTGCTGCAAATGGAACTGCTTCTGGTGAACAAGCTCAAGTGGAACCTGGCCGCCATG
ACTCCCCACGATTTCATCGAACACTTCCTCTCCAAAATGCCAGAGGCGGATGAGAACAAGCAGACCATCCGCAAGCATGCACAGAC
CTTTGTGGCCCTCTGTGCCACAGATGTGAAGTTCATTTCCAACCCACCCTCCATGGTAGCTGCTGGGAGCGTGGTGGCTGCGATGC
AAGGCCTGAACCTGGGCAGCCCCAACAACTTCCTCTCCTGCTACCGCACAACGCACTTTCTTTCCAGAGTCATCAAGTGTGACCCG
GACTGCCTCCGTGCCTGCCAGGAACAGATTGAAGCCCTTCTGGAGTCAAGCCTGCGCCAGGCCCAGCAGAACGTCGACCCCAAGGC
CACTGAGGAGGAGGGGGAAGTGGAGGAAGAGGCTGGTCTGGCCTGCACGCCCACCGACGTGCGAGATGTGGACATCTGAGGGCCAC
CGGGCAGGCGGGAGCCACCAAGTAGTGGCACCCGCAAAGAGGAAGGAGCCAGCCCGGGTGCTCCTGACGACGTCCCCCTTGGGGAC
ATGTTGTTACCAGAAGAGGAAGTTTTGTTCTCTTTGTTGGTTGTTTTTCCTTAATCTTTCTCCTTTCTATCTGATTTAAGCAAAAG
AGAAAAAAATATCTGAAAGCTGTCTTAAAGAGAGAGAGAGAGAGAGATAGAATCTGCATCACCCTGAGAGTAGGGAGCCAGGGGGT
GCTACAAAAATAGAATTCTGTACCCCAGTAATCAACTAGTTTTCTATTAATGTGCTTGTCTGTTCTAAGAGTAGGATTAACACAGG
GGAAGTCTTGAGAAGGAGTTTTGATTCTTTTATATGTTTTAAAAAAAAAAGCTTAAGAAACATTGCTTTAAAAAGGAAGGAAAAAA
AATACAGCAAACCATTGTTAAAGTAGAAGAGTTTTTAGGTTGAGAAATGTACTCTGCTTTGCTGAAAAGCCACAGCTTAGGCCCTC
AGCCTCACTCCCTGGCTTGCTCAGTGCCTACAGCCCTGTTACCTGATACCTGTGCTTTATCCCAGGGGTGGGCAGACCTCTTAACC
TTATAGATGGTCAGTGCGACCTCTAGTGGTCTCATGGCGTGTGGCACAACCCCCCTCCCCAGGGCTCAGCTTAATGTGCCCTCTCC
CCCCAACAACCTGCAGGTTCACAGCGCCAGCCACACAGCGGTAGGGATGAAATAGTGACATAATATATTCTATTTTTGTAACCTTC
CTATTTTGTAGCTCTGTTTAGAGAGATGCTGGTTTTTGCCTGAAGGCCCTGCAGCCTGCCCACATCAGGTTAAACCCACAGCTTTG
TGTGTGGTTTGTTTTGTTGTGTTTTCTTTCTCTATGTTCCAAAACCATTCCATTTCAAAGCACTTTTGGTCAGCTAGCTGGAGGCA
GTGTTGCTGGTGTGTGTTGGGGGGAGGGGTTCTAATGGAATGGATGGGGATGTCCACACACGCATTCAGATGGCTGTACAACAGGT
TGTAGGGCTGGTAGTATGAGGTGCTTGGGAAGTTTTGTTGGGTCAAGAAGAGAGAACTCTGTTCTCGCACCACCGGGATCTGTCCT
GCAAAGTTGAAGGGATCCTTTGGTGCCAGCTGGTGTTTGGAAGTAGGAACCATGATGGCATTACCTGGACAAGGAGATTGGGGACA
ACTCTTAAGTCTCACACAGGAGGCTTTAAACACTAAAATGTCTAATTTATACTTAAGGCTACAGAAGAGTATTTATGGGAAAGGC
TGCCCATGACCAGTGTGACTCAAAGCAATGTGATCTCCCTTGATTCAAACGCACACCTCTGCCCTGCTGGAGAAGGTTTAGGGCCA
TGTCTGAGAGATTGGTCTTTTATTGGGCAACGGGGGGGGGGGGGGGGGGTCCTTAAAAAAAAAAAACCACAAAGACAGAGATTTGG
TCTGCTTGACTTTTCCCAACCCAACCCAATTGGCCCCATTGGAGAGCCATCCAAACTGAGGAAAATTAGGGGACTCCAAAAGAGTT
TGATTCTGGCACATTCTTGCCGCTGCCCCCAAGTTAACAACAGTAGGTAATTTGCACACCTCTGGCTCTGTGCCTTTCTATTAGGA
CTTTTTGGCAAAAGGTGGAGAGCGGGAGGCTTAAGAGGGGATGTGAGGGAAGAGGTGAAGGTGGGACCACATGGGACAGGCCACGG
CTCCTCTCATGGCGCTGCTACCGATGACTCCCAGGATCCCAGGCGTTCAGAACCAGATTCTCATTGCTTTGTATCTTTCACGTTGT
TTTCGCTGCTATTGGAGGGTCAGTTTTGTTTTGTTTTGTTTTACAATGTCAGACTGCCATGTTCAAGTTTTAATTTCCTCATAGAG
TGTATTTACAGATGCCCTTTTTTGTACTTTTTTTTTTTAATTGTGATCTATTTTGGCTTAATGTGATTACCGCTGTATTCCAAAAA
AAAAAAAAAAAAAAAAAAAGAGGTTCCTGTTCACAATACCTCATGTATCATCTAGCCATGCACGAGCCTGGCAGGCAGGTGGGCGG
TCTGCCTCCAGGGATCCTGGGACCCTGATGGCGATCGTCCTGTCATGCTGGGCCCTTCATTTGATCTGGGACATAGCATCACAGCG
GTCAGGGCACCTGGATTGTTCTGTTATCGATATTGTTACTTGTAGCGGCCTGTTGTGCATGCCACCATGCTGCTGGCCCGGAGGGA
TTTGCTCTGAGTCTCCGGTGCATCATTTAATCTGTTAGGTTCTAGTGTTCCGTCTTGTTTTGTGTTAATTACAGCATTGTGCTAAT
GTAAAGACTCTGCCTTTGCGAACGCAGCTGCAGTGCTGTAGGCCCCCAAGTTCCCTAGCAAGCTGCCAAACCAAAACGGGCACCAC
CAGCTCAGCTGAGGCATCCCAGCCAGGCAGGACCCTTGAGGGCCGCTGTGTCCATGGTGATGGGGTGAGGTTTTGGCCAAAAGGCC
ATAGACTGGTGGTGGGTCCACGGAATCTGCCCTGTGACATGAAAGGCTTTGAGGACTCTGGCTGGTGGCCAGGTTGGCTTTTTGTA
TTTCTGGTTGACACACCATGGCGCTTCCCAGCACAGACATGTGACCAGCATGGTCCAGGAAAAAAAAAAGACAAAAAATCTAGAAA
ATAAAATTGGTAAAATCTCAAAAAAAAAAAAAAAAAA

MOUSE SEQUENCE - CODING (SEQ ID NO: 15)
ATGGAACACCAGCTCCTGTGCTGCGAAGTGGAGACCATCCGCCGCGCGTACCCTGACACCAATCTCCTCAACGACCGGGTGCTGCG
AGCCATGCTCAAGACGGAGGAGACCTGTGCGCCCTCCGTATCTTACTTCAAGTGCGTGCAGAAGGAGATTGTGCCATCCATGCGGA
AAATCGTGGCCACCTGGATGCTGGAGGTCTGTGAGGAGCAGAAGTGCGAAGAGGAGGTCTTCCCGCTGGCCATGAACTACCTGGAC
CGCTTCCTGTCCCTGGAGCCCCTGAAGAAGAGCCGCCTGCAGCTGCTGGGGGCCACCTGCATGTTCGTGGCCTCTAAGATGAAGGA
GACCATTCCCTTGACTGCCGAGAAGTTGTGCATCTACACTGACAACTCTATCCGGCCCGAGGAGCTGCTGCAAATGGAACTGCTTC
TGGTGAACAAGCTCAAGTGGAACCTGGCCGCCATGACTCCCCACGATTTCATCGAACACTTCCTCTCCAAAATGCCAGAGGCGGAT
GAGAACAAGCAGACCATCCGCAAGCATGCACAGACCTTTGTGGCCCTCTGTGCCACAGATGTGAAGTTCATTTCCAACCCACCCTC
CATGGTAGCTGCTGGGAGCGTGGTGGCTGCGATGCAAGGCCTGAACCTGGGCAGCCCCAACAACTTCCTCTCCTGCTACCGCACAA
CGCACTTTCTTTCCAGAGTCATCAAGTGTGACCCGGACTGCCTCCGTGCCTGCCAGGAACAGATTGAAGCCCTTCTGGAGTCAAGC
CTGCGCCAGGCCCAGCAGAACGTCGACCCCAAGGCCACTGAGGAGGAGGGGGAAGTGGAGGAAGAGGCTGGTCTGGCCTGCACGCC
CACCGACGTGCGAGATGTGGACATCTGA

HUMAN SEQUENCE - GENOMIC (SEQ ID NO: 16)
CATGCCTGTAATCCCAGCACTTTGGGAGGCCAAGGCGGGCAGATCACCTGAGGTCAGGAGTTCGAGACCAGCCTGGCCAACATGGT

GAAACCCGTCTCTACTAAAAATACAAAAAAAAAAAAAGAAAAAAAAGAAAGAAAAGAAAAATAGCTGGGCATGGTGGCGCATGCCTC
TAATCCCAACTACTTGGGAGGCTGAGGCAGGAGAATCGTTTGAACCCAGGAGGTGGAGTTTGCAGTGAGCTGAGACCGCGCCACTG
CACTCCAGCCTGGGAGACAGAGCAAGCCTCTGTCTCAACAAAAAAAAAAAAAAAAAAAAAAAAAAAAGGGGAAAAATCACTCCAAT
CATCCCAAGTCTCAGCTCCTACTCTTTTGCTATATGTCCTTCAAACCAGCTATGTATTTATTTTGCTGAATTTATGCTGGTTTTAT
CTTTTGTATCTTGCTTTTTTTCATTTAATGTTTTTCCATCCAGTGAACACACCCTAATTTGCCATTCCCCTCTTCTTGAACATGAG
ATTGTCTCTAGGGTATCTCTTGCAAATAGGATAATTGTCAATGATGTAACGGGTCTCTATGCAACTTGCAAAAGTGATCAGTCTTG
GTGTCCTCCTCATCCTAGTGTTTGGTTCCAAGCAACAGAAACCAATGCTGGCAGATTTCAGCAGAAAAGGAAAATCAGATGCAGAA
GATAGGCAGGAACAAGGGAGGCAAGACAGCACGAATCACAATGGAAGCATGCTGCAGATCTTCTCTGATGAGAAGCCAGGGCTACC
CCTACCCATCCCAACACCAGACCCTCCAGTAAAACTGCAGCGGCCCACACCACCTTGTCACCAGAGCCCACAGCTCTATGATGGGC
AGCACAGATACTGCTGCTCATAAACTTGAATGCCTCACCCAGTTCTACACTAGAAAACATTTCCTACCTTTTCTGATTCTTCCCAC
CACCAGCTCCCAATTCAAAATCTTGATCCAAAAAGATGAAATGTATAAATAATACTATTTATAATGACATCAAAATATCAAGTACT
TAAGAAGAAATCTAATAAAAGATGCACAACACCCAACACACACACACACACTCACACACACACACCTGCAATTAAATTAAGGACAA
TCTAAATAAACAGAGGTCTATATCATGCCCATGAATTGAATGACTCTGTTAGTAAAAGTGTCAATTCCCTCAAAATTGATCTATAG
ATCCAATACAATCCCAATCAAAATTCCAGCATTGTGTGTGTGCACATGTGTAAGTTGATTCTGAAATGTTTATGGAGCCCCAAGGG
CCAGGAGTAGCCAGGGCAACCATAAAAGAGAAGTGGGTTGGAGGATTTAGCCCGCCAGATATCAAGAATTCGTAGCGTATAGCTTC
CATGATGCTTCTGGGGTAAGGACTGAAAAAGAGACCAGTGGAACGGGATAGAAAGTCCAGAAACAAGAGATTTGTGTTAAGTGAGC
AGATTTTAGCTGCTCTGTTCACAAAAAAAAGTAACTATGTGAGATGACAGGTATGTTACACTAATTCACTATGGTAACCATTTTAC
TATGTATATGTATCTTATAACATCATGCTGTCAAGTTTATTTCAAAAATAAATTTTACATAAAATCTTAGCAGTGTTATTCATAAT
AGACAAAAAGTGGGAAAATTCTATTTTTTTAAAGTACAGAAACTTTATTCCTCAAAGGGGAAATAGGTTAATAATAATAAGAAGAA
GAATTAAGGCCAGGTGTGGTGGCTCATGCCTATAATCCCAGCACTTTGGGAGGCCAAGGCCAGAGGATTACTTGAGCCCAGGAGTT
TGAGACCAGCCTGGGCAACATAGTGAGACCCCATGTTTACAAAAATTAAAAAATTAGCTGGGTGACGTGACAAGTGCCTATAGTCC
CAGCTACTTGGGAGGCGAAGGCAGGAGGATTGCTTGAGACCAGGAGGTCAAGGCTACAGTAAGGTATGATCGTGCCACTGCACTCC
AGCATGGGCAACAGAGCAAGACCCTGTATCTAGGGGGAAAAAAATGATAAGCCTTGTGAAAGAGAAATGAAAACAGCATCCACACA
TAAACTTGTACCTCTGTGTTCATCGGCAGCATTGTCCACAGTAACCAAAGGTAGAAACCATCCTAAGGCCCATTGACTGATGAATG
GATAAGCAAAATGTGGTCTATCAATACGATTGATAGTATTCAACCATAAAGAGGAATGAACCATGGACACATGCCACATGTGGGCC
TTGAAAACGTGATGCTAAGTGGAAAAAGCCAGATGCAAGAGGCTGCATAGCATATGATTCCATCTGTTGGAAATGTCCAGAAAAGG
CAGATCCACAGAGAAAGATTAGTGGTTGCCAGGGGCTGGGGAAAAGGGGGAAGAAAAGGGTGAATAACTACAAATGGGTACAGGGT
TTCTTTGGCGGATGATGAAAATGTTCTCAGATGGTGATGGTGGCTGTCCAACATAACAAAATCCATCGAACTATATACTCTAAACG
AGTGAACTTTACGGTATGTAAGTTATTTTTCAATAAAGTGGTTTCAAAAAAATAAAAAGGTGAACCCTACCATTTCTAGGTGCTTT
GGAGTTTACCGTTTTTCTCCTTGCATTCTGGAGTCAGAGTGAGAAGGCATCTCAGCTCTACCACAGCTGAATGATGTGTTGGGCTT
CGGTTTCCTCATCTGTAAAATGGGGATAAAGGGCCTCTCCTTGGAGCTGCCATGAAGATTGAATTAGATGCCCTCTGTGAAGATGC
AGGGCAGGTGGAGAGCACAGTAAGCGTCTCGGGGGGCCACGCCATCACCGCCAGCAGGGCTCACCAGTATCTTTGCCCAGAGAATA
GCCGGGGTCTAGGGAGCAGCCAGGGTCTAGGGAGAAGGGCTGCTTCCCTGTGGAGGCTCCTAACTTTCCCATCTCCTCCCTGTTTC
TCCCCTGCCCACCCCTTACTCCTGGAAGTGTGACTCGCTGCCTCCTCCCGTGACTCCCCTACATTGCATCTCCCCAGTTACTGTCG
TTATCTCTCATCTCTTCTTTCCAAACAGCCTCTGTCTAGCACCTTGGAGCTCGCATGGGAATTCATGGGTGGGTGGATTCCAAGCT
TGGCTTCATCAGATGACAACTAAAAGGCAGAGGCCTGGGGGGGCAAGGGAGCTCCAGGATCTGTCTCCCTTTGGAGAAGCCCAGGT
TCACCTTCCCAGCAAGCTGCTCCGGCCCCACGCCCCTGGCCCTCAATAACCTTCAGTGGCTCCCTTCTTGCAGCTATAATTCCCCT
CTTGGGATGCTACAAAAGGGATTCCAGAGATTTTTTTTTCAATAACTCAAAAGGGATGGAGTAAACGATATATGTGCCCAGGACAA
GGCAGCTAAGGGAAGGCCTCAGGCTTCCTTTGCCTTTGCCTGGAAGTATACCAACCCGCAGCCCGGAGGGCCAGCAGAGAACCAAT
GCTCAGAGCCTGGGGGCCCAAGGAATGCTTTCCAGGCTGGCGGTCCCAGGAGCCCCCTCTCCCTTCCCTATAGCAAGATACTAAGA
GCAGAGGCTGGCCTGGCAGGCCGTGTCGAGGCCCTGAGCTCACATGGCCTTGGCAGCTTCTTGACAGTCGTGTCTTTTTGTTTCCA
GCAGTGACACTCACTGACCCACACCTTTGGCCAGCGTCCCTTGTGACTGCCCCCTCCCGCTTGAGCAGAATGGCTCCTATTACACA
ATGGGTGCCAGGAACCTGGCCAGTCCATCCAGAGACAGGGCAGAGCCCTGGGCTACCGACCCTCTGGGGCCAGCAGAAACCCTCCC
TATGGCTCTGCCTCTCATCCTGGCATTGACACTTGTGCCCTTAATTAAAGTGCTTCTTTTTTAAATCAACATTTCTACTCCTTTTG
CAGAGTAAGAGAAAGAAGAGTTAGGGGAGAAGAAAGAAGGAAGGATTCATGGGGTTATTTTTTTTTTTTCATGTAAAAGTTTGTTC
CGTGATTTAAATCTCATGTCCACAAACAGCTCAGGAGAAGTCAGCATAGAGAAGCAAGCTGACAGATTATAAAGTCATTTGTAAAA
TCCTCTTTCTGCATATAAACTGAAATGTGTAATTAATTCCACATACTGCGTGTGGCACATGGCCTTCATCCTGCATAAGAACAAAT
ACATGAATGAATAAATCGAGCAGGCCTTGACACTCACCAGGCCCTGTGCTAAATGCTTTCTACATATTACCTTAATTACTCCTCAC
CGTGGCCTTGCGAAGAAAACGATCACAGCCCGTTTTACAGATGGGGACAGAGATTTGTCCAAGGCCACACAACTAGTGGCAGATGT
AGGTCTGTATGTGACATTCTTGTTCAGTTTCATAAATGTGGCTGCCACCTGCCTGAAATTTTGAGGGAAAAAAAAGTGGTTTGGCT
TTGCATGAATTCTTGAAAAAGTAGATTTTTATCTTTACCAGCTTCTAAGCAAGCTGGATTTATTCTGACAGTGTCCATGAAATGGC
TACAGACCATCACCGTTGCCAATGTTGTGAACCTGAACAAGGCACCTGGTTTCTCTGTGCCTCGGTTTCCTTACCATTAAACGCAT
TTCCCTGAGATTAAGTGTGTGAAACTATAAGTTGGCTGGAGGAAACACCTCATAGAAGTGAGAAGATGAGGATGGGTGGTGAGTGA
AGGCTTTTTCTCCAAACCCTACAGGAGATGGACCTCAAAGCAAATGGCCATGCTTCCCTTAGACTGGGTGAACTGGTGCTGCTCAG
GGCTAGAAGTGTGTCTAGAAGCATGGGGACCAATGCAGCCCCCTCCACCCTAGGGCACATAACCTGTCTCTCTCCCATAGGATAGC
AGAGACAGCTCTCTAAGTCAGGATTCAGCGAGATGGTCCAGGAGGGGCCTAAGGATTCCTCTGACTTATCCAAGCCTGGGGAGGCA
ACACAACCTAGCCTGTGTCTCAGGGGGCCCCAATCATGCCCTGAACCTCATGAGCCCAGGCAGGCATCCTGTTGGTTAAGGTCGGC
CTCAGGGTCCAGGGATGCACTAGATTCTTTATCTTAAAGTGACCCTCAAGAAAAAAAAAATAAAATGTAGCTATAGGCAGTTTCTG
GACGTTTTCCTATTTGTTAAGCACCTTCTTGCTCCGAGCTTTCCAGCCACAGTGCACTGGACGCCATCGGAAGGCTGCAGCTGCTA
TTTGGTGCTTTACCACCGCTGAGAAGGTGGAATGGTCACGGCCCGGTTCTGTCACTTTCTTCAGCCGGACAGTCGCCTTATTACGG
ATTCCAGTAGGGCCGAGCACACTCCTTCCTGCCCGTCTTTACAGATGAACATGCTATCGCTCCAGCAGTACAACCCGCCTTATTAC
GTAAAAAAGCAGCCCCTATCTACCCGCAGGGAAGGAGTATGTTCGGCACCACAGCTGGACTGGTGCTCGAGTTAAGCGTCCTGGGA
GGTCCGCATGCGCTCCGGAACCGTAATGCGCGCTTTTTCTAAGCCTTACGGTAAACGCGGACGCAGGGCAACCACGTGGCGGTGGA
ACCGAGGCCCGGCGGGAATGCGCGGAATGCGCGGCGCGGCCTCGCGCGGTTCCCGAGCCACGGCCCAGGGTCCGGCGGCGCGCGCT
CTCGCCTCCTCCCCTCACCTCTCCCAGCCGCACCCCGGCCCTGGCCCTGCCACCCAGAACTCGCTGGGCAAGTCGTGCCCCGCGTG
AACACACAGAAGGGGCTTGGGGACCGAGCGCGGCCCATCAGTCCCTCAGACCCTGAGGACCCAGAATTCCCTAAGGGGTCCGAATC
CGAGTCCTGCCCCCAGCCCTTAAGGCACGGGCTCCAGGGACCCCAGGGGAAGGGCGCGGGGCATTAGGTACGCAACCCGTTTCCCC
GCACCTGGAAAAAAACTCCCTTTCCCTCCCCTCCCCTGCTTGTTGAGTGTGCGGATAACCAGAACTCTAAGGCGCCCCGTAATAAC
GACCCCGCTGTCCCTCCACCCACCCCCAAGTGCCAAAGCGAGGGATGGAAGCGCTTTCAAGCGTTCCAAGGGCATTGAGGAGCGAG
CTGGAGAGGCGCGGGGATGCGGGGTCCTCCCCGCAGTCTTCCGGAAAGGGCGGGGGAGGGCGCGGCAAGTTCCGGAGTGGGGCATG
CCGTGGGAGCCCACGAGGGCCTCAGCGCGGATCCTCCGCCGGAAAACCGGCTCCCGCGAGCCGCCGCCGCAGGTTTCCTAGGCCCC
GCGAGTCCCGCAGCGAAGCCCTGCGTCTCCGTCCGACGCGGGGGTCTGCTCAGCCTCGGGTGGCCGCGGCCAGGCCTGACTGCGG
GGGAGAGGGCCGAACGTGACCTCCGAGGTCACCCCCAGCCAGCTTTCTCTCCTGTGGTCGGAAGTGGTTTTCTTCTCGATCTGGGC
GCCTACTCCCCACCACTTGGTCTGAGAGGGGCTGGGGCCGGAAGGCCAGGGAATCTCTGGTGGATTTGGGGGTTCATATTGCTCAG
GGTACCAGCCGATGCGTTTTGAGGGGCGGGAGTCGAGGAATTAGAATCGCCTTTAACCCTCAAGAGTTGCGCCTTCAGCCTCGGGA
TCCCAGATGCGTCGTTGGAGCCAGGGCCGCCCCCCTACCTGTTGGGTTTGCGTTTTAACTCCAGCGCACACCTTGCCGGCAGCCCT

CGGAGCTAGGGGAGGGGTCTCGTTTCCCCGCAGCCCGCCGGACAGACGACTGGGGCACGGGAGGGGCGGTGGCAGGGTGGTCTGTG
TGTGGCTGAAACTAATTGATCTGGAGCGGAAACGCACGTCTGCGGTTGGGGCGATGGGGGGGCGGTGCGGCTGTCCATGTGCCGA
GCGTGTGGCTGTCTCGGGTGGGCACTGGGGCCGGAGTTCGCCCCGGCCCACCTCGCAGTTTTGGGGCGCCTGGGATCGGCGCTACG
TAAGCGAAGCAGAGCTGCCATAGCACGTGGGCCGCCACGCGCACCCCAAAAGCAAGCAGTGTGGGGGAAGGGGAGCTCGAGCGCC
TTCGGAGCCCAGGGGCCGGCTTTCGGAAGCGTTTTCCCGGGCGACTTAAGGGCTTAACAATGGAAAACTCGCGGAGCCTGAGCCAA
GTCCTTTCAAGTCGCCGCCAGGTATGCGGCTGCAGGTGACCCCACCTGGGTGCGCCCGCCCGCCAGCCGCCCTGGTGGAAAAGCGG
GTGCGGGAGGTCGCTGGCGAAAGGTCGGGACTGGTCCCTGCACCACCCGCCCCCAACCCAAGCCCCGAGCCCCGCGGCGCGCAGCC
GCGCTGAGTCCCGGGGTCTGCGTCGCGGCGCGCCGGTTCCTGAATGAACGCGCTCCCTTCCCCCGCCTGAATGAAGGTTCCCACAG
CCAGGGACGGTGGCGAACACGCGCCTGCAGCGGAATTCGCTTTCTCCTGACCGACCATCCGCCCAGGCCGCGGTCACCGGGGCGGG
GGCCAGGGGGCGAGGAAAGCGTGAAGGTGATTTCAGTTAATTTTGGATTTTCTTTCAAACAACGTGGTTACCCTCCCGACTGGGCC
ACTTGCCCTTTGTCTCCAAATGGTCACCAAGAAATAAGAACAGAGCACTTTAAATGAGCCCAGAATCCGCAGTTCCTGCTTCGTGG
TGGGTTTTAAGAAGACAGTGTAAAGTAAAACTGCAACCGAAAAGTTTTTTAAAGTTGCTTTTCTCTTTGGAAAAAATAAAATCAAA
ATGCTTTCTCTGCGCTTCTTGAAGCAATGACCCTCAAAAGCCCAGAGGTATTGGCCCCCTCGGGGGACCCGGGGGCCGCCAAGCAG
GGTTCCCCCAGGTGGGGGCTGGGCAGCTGGCGCTCCCCGCCGGGCCCCAAATTCCAGCGCCGGGCCCGCAAATTCCAGCGCCTCCC
CCGCGGGTTCCTGGACGGCTCTTTACGCTCGCTAACCGGGCTTGCAATTTTGCGCTCGTCCCTGAGCCGGGAAATCAACGAAGTTC
CTAGTCGAGATCTGCCCGGTCCGCCTAGTAACAGCGCCGCGCCCCCATTGGCTCATGCTAATTCCAGTTTCCTCTGTCTTGCGCCC
GGGATGGGGGGGTGAAGCTCCCTCCTGGACCCAGAGCCGGTTGTGCCGGAGTGGGCGAGCCTCTTTATGCCCTGCTGCCCCTAGCC
GACTTCGGCCCGCTTCGCGCCTCGGGCTGGGCCAGGGCGCACGCGGGGCTCGGGGCCCCTCGCCCCACGGGATGGAGAGGCCGGG
TGATAGCTCCGGGCCCCATAAATCATCCAGGCGGCCGCCGGGTCGGGATTTTATGAATGAAAAAGCAGCTGGGCCGCCCTTGTGCG
CGGGCTGATGCTCTGAGGCTTGGCTATGCGGGGGCCAACGCGATTGTGGGTGCTCGGGGAGTGGGGGGGGGCACGACCGTAGGTGC
TCCCTGCTGGGGCAACCCATCGCTCCCCATGCGGAATCCGGGGGTAATTACCCCCCCAGGACCCGGAATATTAGTAATCCTAATTC
CCGGCGGGGGAGGGGGCGCGGGAGGAATTCACCCTGAAAGGTGGGGGTGGGGGGGGTCGCATCTTGCTGTGAGCACCCTGGCGAAG
GGGAGAGGGCTTTTTCTATCAGTTTTCTTTGAGCTTTTACTGTTAAGAGGGTACGGTGGTTTGATGACACTGAACTATATTCAAAA
GGAAGTAAATGAACAGTTTTCTTAATTTGGGGCAGGTACTGTAAAAATAAAAACAAAAGTTAAGACAGTAAAATGTCCTTTTATTT
TTTAATGCACCAAAGAGACAGAACCTGTAATTTTAAAAACTGTGTATTTTAATTTACATCTGCTTAAGTTTGCGATAATATTGGGG
ACCCTCTCATGTAACCACGAACACCTATCGATTTTGCTAAAAATCAGATCAGTACACTCGTTTGTTTAATTGATAATTGTTCTGAA
TTATGCCGGCTCCTGCCAGCCCCCTCACGCTCACGAATTCAGTCCCAGGGCAAATTCTAAAGGTGAAGGGACGTCTACACCCCCAA
CAAAACCAATTAGGAACCTTCGGTGGTCTTGTCCCAGGCAGAGGGGACTAATATTTCCAGCAATTTAATTTCTTTTTTAATTAAAA
AAAATGAGTCAGAATGGAGATCACTGTTTCTCAGCTTTCCATTCAGAGGTGTGTTTCTCCCGGTTAAATTGCCGGCACGGGAAGGG
AGGGGGTGCAGTTGGGGACCCCCGCAAGGACCGACTGGTCAAGGTAGGAAGGCAGCCCGAAGAGTCTCCAGGCTAGAAGGACAAGA
TGAAGGAAATGCTGGCCACCATCTTGGGCTGCTGCTGGAATTTTCGGGCATTTATTTTATTTTATTTTTTGAGCGAGCGCATGCTA
AGCTGAAATCCCTTTAACTTTTAGGGTTACCCCCTTGGGCATTTGCAACGACGCCCCTGTGCGCCGGAATGAAACTTGCACAGGGG
TTGTGTGCCCGGTCCTCCCCGTCCTTGCATGCTAAATTAGTTCTTGCAATTTACACGTGTTAATGAAAATGAAAGAAGATGCAGTC
GCTGAGATTCTTTGGCCGTCTGTCCGCCCGTGGGTGCCCTCGTGGCGTTCTTGGAAATGCGCCCATTCTGCCGGCTTGGATATGGG
GTGTCGCCGCGCCCCAGTCACCCCTTCTCGTGGTCTCCCCAGGCTGCGTGTGGCCTGCCGGCCTTCCTAGTTGTCCCCTACTGCAG
AGCCACCTCCACCTCACCCCCTAAATCCCGGGGGACCCACTCGAGGCGGACGGGGCCCCCTGCACCCCTCTTCCCTGGCGGGGAGA
AAGGCTGCAGCGGGGCGATTTGCATTTCTATGAAAACCGGACTACAGGGGCAACTCCGCCGCAGGCAGGCGCGGCGCCTCAGGGA
TGGCTTTTGGGCTCTGCCCCTCGCTGCTCCCGGCGTTTGGCGCCCGCGCCCCCTCCCCCTGCGCCCGCCCCCGCCCCCCTCCCGCT
CCCATTCTCTGCCGGGCTTTGATCTTTGCTTAACAACAGTAACGTCACACGGACTACAGGGGAGTTTTGTTGAAGTTGCAAAGTCC
TGGAGCCTCCAGAGGGCTGTCGGCGCAGTAGCAGCGAGCAGCAGAGTCCGCACGCTCCGGCGAGGGCAGAAGAGCGCGAGGGAGC
GCGGGGCAGCAGAAGCGAGAGCCGAGCGCGGACCCAGCCAGGACCCACAGCCCTCCCCAGCTGCCCAGGAAGAGCCCCAGCCATGG
AACACCAGCTCCTGTGCTGCGAAGTGGAAACCATCCGCCGCGCGTACCCCGATGCCAACCTCCTCAACGACCGGGTGCTGCGGGCC
ATGCTGAAGGCGGAGGAGACCTGCGCGCCCTCGGTGTCCTACTTCAAATGTGTGCAGAAGGAGGTCCTGCCGTCCATGCGGAAGAT
CGTCGCCACCTGGATGCTGGAGGTGCGGGGCTTCGGGCGGCTCTCTTAAGACTTCCCTGCAACTTGTTGCCCAGACCCACGTTTCT
TTGCTACTCACCCCCCTCCCTTCTCTCCCGCTAGAACTTTGAAGTTTGCCGTGGTGTTTCTAGGGATCCGTATTTTCAAAATAAAA
ATTGCGGGTATTTTCTGAAGGAGGAAGGGGTGGGGGTGGGGGTGCTAGAAGTAGCGTTTCGTGGGAGGGGAGAAGGGGTCCGGGA
GGGGTGCCTTCGGGAGAAGCCAGTGCCAGGGGCACCCCAATGGCCCGAGGGTGCGGGCTGGCAGGCTGGGTGCGCTTTGTGTCCC
CCGCCTGCGCCCCAGCCCGGCTGCGCCTCAGCGGCCGGGAGCCGCCAACTCCGGGGGGAGGGGGCATAGATTTGATTTTTAAATTA
ATATCCATGGACACGTATGCAAGGGCCGCTCGTGCCAGTATTATGCGCCATCTTTGCTCTTTTATTGCAAAGCAAAAGTGTTTATT
AATAATTGGGGGCAGGGTGGGGGCGGGGAGCGGCCGCCGGGCGCTGGGGCCGCAGCTAAGGGCCGCGCGGCTGCCGGGAGCCCGCG
GGAGGGGCGCAGGGACGCGGCATGGGTAGTTTTGGGGGGACCCCGCTAGGGAAGGGGGGGCCTTTGTTCAAGCAGCGAGTCCCGGG
GCGCCCCGAACGGGCAGCCTGGGCCGGAGAGCACGGCGAGCTGCAAGGTCGCGTGGCCCCCAAGACGCCAGGGCTTGATCCCCGTC
TGCAGGGATATCGGCTTGGAGGACCTTCTCCGAGCGAGCCGGGGGCCTGGGAGCACATTTTCAGACCTTCGGTGGGCGCCTGAGGG
GCCCGCAAGTATTTTAAAATAATTTTTGAAAGTGCGGCGTGGTGCCCTTGCGAGAGGGAAACGCCGCCCGCGCCCAGGGGGAAGGG
GGGGCCCCGGAGTTTGAATTCCTGGGGCTCCCCCCGGAGCCTGTAACGAACTCCCAACCCCCGGCCTGGGTAAAGGGTCGCCCGAG
GGTCATTTTCAGGGTTTTTTTATGCACTTAGTTATTTTTTTAATATTTTTAAATATTTTTTGAAAAGATGACGTCTGGGAAATGC
GGCGCGGCGGCCTGGGACGCCACCTTTGTGTCTCGCAGGCGCGGCGCCCAACCCCGCGGCCCGTTCCGCGGCCCCGCACCCCAGTT
GGTGTCGACCCCCAGTCAGAGGGACCACGGAGCTCCAGGGCGGGCCAGGGTCCCGGGGGCCGGCAGCCCGCGCCGCCGCGCACGCC
GCCCAGCTGTGCCCGCTCCCGCCCCCACCGTGCCAGCCTCGCGGGGACTTTCCCTTTCAGTTTCGGGGAGGGTGGGTACTGGGGAC
GCGCGGGGGAGGGGGCGCATCACGGGAAGCTCCTGCCGCCCCCAGCCCCGACCCCTCGGCGCCCTCCAGACCTGCCGGCCCTGCCA
AGCGCGATGGGGGGTGCGGGGGCGTGCGGGGGGCGGCGCGACCTGGCGGCGGTCACGGGCCCCGTGCCTCCGTAGGTCTGCG
AGGAACAGAAGTGCGAGGAGGAGGTCTTCCCGCTGGCCATGAACTACCTGGACCGCTTCCTGTCGCTGGAGCCCGTGAAAAAGAGC
CGCCTGCAGCTGCTGGGGGCCACTTGCATGTTCGTGGCCTCTAAGATGAAGGAGACCATCCCCCTGACGGCCGAGAAGCTGTGCAT
CTACACCGACAACTCCATCCGGCCCGAGGAGCTGCTGGTAACCACTGGACCCCGCCGCCCCCCGCCCCCCGCGAGCCGCACGCAGG
ACCACGGGGCCGGGGAAGGTGCAGGCGGTGGCGGCCGGCCCGCCTCTGACATATCTGCTCCTCCGAGGGAGGGCGGCCCCGCCGCC
GGGCGTCCCTGTCCGGGGAGCGGGCGGGATCCTAGCCGCCCTCGTCCCGCCGCCCTGTGTGCGCTTGCCTGCGACTCCCACCGCGT
TCGCGCCCCGCGGTGTGGCCGAAAAGTGGGCGGCGCGCGCCCTCCAGCGGCTGCACGAGGAGCGCCGCGCTCGGCGCTGAGCCTCC
AGTTCCAGGTGGTGGGAGGTCTTTTTGTTTCCACTTGCAGAGTCTTTTCACGCGGCGGGCGCCTTTTCTGTTTTGATCTGGGATTG
CGTGTTGCCCCAGCTCCCTTGAGTCCCCAGCATTCGCCAGCCCTCCCCTCCAACATCCAGGACCGCACGAGACGCAGGGGCCAGTG
CTCTGAGCCGGAGGTGCGGCGTGGCCCGGCCCCCGTGCTGCCGGCTTCCCCGCGCCCCCGGGCTGGCCCGCACCTCCCCTGATGGC
CGCTCACCCTGTGTTCGCAGCAAATGGAGCTGCTCCTGGTGAACAAGCTCAAGTGGAACCTGGCCGCAATGACCCCGCACGATTTC
ATTGAACACTTCCTCTCCAAAATGCCAGAGGCGGAGGAGAACAAACAGATCATCCGCAAACACGCGCAGACCTTCGTTGCCCTCTG
TGCCACAGGTAGGGCAGGCCCGGCAGCCCCCGGCCTCCCCTTGAGAGCCGGCTCCTTAGGTGACCCTGGCCGGCTTCTTGCTCTCC
ACCTGGGTGCTGTCTGGGAAGATGTCCCCAGACCCCCTCCTGCGCTGGAGAGCGCTCTTCCAGCTCTGGTGAGCAGAGGCCCTGGA
TTGTTTGTCGCGCTGGATGGAGGGAGATTTGCTCCCTCACGGCCACCATGCAGTACCTTGGGCATTGGTGTGGACGGCTCAGCCTG
CCTGTGTCCCGTTACTCTGGCCTCGTCCTTCAGGCCAGGCAGCCTGTGGCCACTCCATGCTGAAAGGGGTTTACCTTGGCCACAGG

GCCGCCTCCTTTCTCCACCCACCTCCAGCCCTTCTTGTGTCCTTAAGGAGCCTGAGCTGCAGAGGCCCCCTCCTGGCCTCTCCCAG
GCTGGGCCACCTGCCAGAGGCGCCTCCAGGGGCGGGAGAGCTGTCGGCCTGCCTGCACCACGTGCTCTGGGCAGCCGAGTGCAGG
GGTGTCCAGCAGAGGAGCTCGGCTGCCTGAGGCCCTGCCAGGGGTGCCGGCAGCCAGCCGGGCTCAGCTGAGCCCTGAGGGGGCGC
TTCAGAGCACTCTCAGCTTGGGCCGCCACCGTGGGCAGCAGAAGCACCCAGTCCTCACTTCCCCTGGCATGGCCCCAGAGGCCCCT
CCCTGACATGGCCTTGGCCCCAGAACCCAGTGGGGACAGACTCGCACATACACAGGGTGCCGCCTCCTGCTGTCCCCAGCCCTGCC
TCTGACCCCCCTGTGACCGCCTCCTTCCCTGGCCCAGGAGGCCTGGTTACCTTCATGGGGGAGCATGGCCCCATCCCACCCAGCTC
TGCTGTGGCCCACCTTTGGTCAAGCCTCAGTTGTCACATCTGTTTGGGGGCTCACTCTGGGTGACCTAGGCCACAAGGCCCACGGG
GCATCAAAGAGGCAGTAGCATCTTCTCCCCTCCCCAGAGGGCAGAGCCCCCCAAGCCTACTTCAGAGCTCCCTTCTGACACCGGTA
GCCCGCAGCCGGTATTCCAGAATGGGTTCTGGTTTAGGCGTGAGGCCTCCCCCACCTCCTCCACCTGCTTGGGGCATGAACCCCTC
CCCCACGTTTCCAAGCGAGTCCCCAAGGTGGGCAGATGAAGATGCCAAGGATGTCGACCAGTCTGGATGGGTCTGGGGTGGGGGGG
CATGCGGCAGACAGGGAGGCATTCTCTGGCTGGTGCTCCTCAGAGGAGAGAGGCCTCCGGAGACTCCAGACAGCCTTTTATGGAGC
TGAAAGTGGCTTCAGAGAAATGCAAAGTTTCCTGGAGAGAACGTGGGGCGTGGTTCTTGCACAGCCTCCCTACAGGGTGGCTCCAG
CAGTGGAGCTCCCCTCCCAGGACCCCTGGGTGCTAGTGGGAGGCAGTGGGCAGGTGCAGATTCTCGTCCTTCCCACTACTGCACAC
CCTTTGTCTGCGAAGGCGCCCCCAGCGGTGGGTGAAGGAGGAGGGACACTTGGGGACCCAGCTGTGCACGTGCTCTCAGTGACTGT
GGAGTCCACTCCAGGGTGGGTCCCGAGGGAGGGGCAGGAGACCAGGGGACCCACCCCTGCAAAGTGCTCCGGGTCCTGACCCGTGG
CCACCCCATGGAACGTAACTGAGCAGCCAGTGCCTTGTTCCTGCTGGACATCTGTGGAGACAAGAGTGACTTACGGCTGCTTAAAG
TCAGAAACAGGTTGAAGGAGGTGGAGGCGTGGGAAAGAGTCTAGGAAGGTGTTTTTGCCCTCCACGTGGCAAAGGTTACATTTAAA
GGTGATGCTGGGTGTTCTCCCTGCACTAGGCATTCCTGGCCCCAGGTCCCCAGCAGGTGTGCACATGCTGCATACACTCACGCATG
GGGGTTTCAGGGCAGGTGCGCCCTTGGCTCCGTGGGAGGCCAGGTGAGGAACGTCCAGTGCCAAGGAGCTTCCGGGACAGCTGTCA
CTTCCCTTTACAACCAGGCAGCGGATAGGGTCAAATCCTGGAGCTTTGGTGTCTAATTCTGGGTGGCTCCTAATCTAAGCACAGAC
AGCACCACACACTGGGGTGGGGGCACGAGCTTCTGAAACAACGTGGCCCCAGTGACTCCACGCTGTGTGTGCCCCTGGAGACGGGG
GGGTGCACAAGGTGCGGAGCCAGCTAGAACCTGTCGCTCCCTGCAGAAGCGGTTTCTGTGTGCGGTTCTGATTTGCCTCAATGAGA
AGGTTTTCATTCATGGCTCCCGGCTCTCAGACTGGGTGGAACTGCTCCCATTTAAAGGGGAAAAGAGGTGGCTCGGCTCGTTAAGG
ATTTCTTTTTCTAAGTTGTTACGGCGCCCAGCAGCCGGCTTTGTCTCCCCTTCAGGGTGGCTGCCTTTCTTCCCGGCCCCTCGCCG
GCGGCCCTCTCTTTAACAAGGCCGAAGTTGTTTATTCTCTCGGGATGAAGTCTCGGATGGGCCGCCACACCCCTGGCGGCCCGTGG
GGGCCCCTCTCCCTTTGTGCCTGGGTCGGCTCCCATTCAGCTCCCCCGACCCCCCTTGTTCCCGGGCGCTCAGTGGCGCGAGATGA
GGCGATGGGGCCGACAAAGATGCCACACTCATCCCTGCCGACCTCCGGCTCCCAGCCCAGGGCCCCTGGTTCCTGTGCAGAATTCC
TCGTGGGTGTGACAAAAGGCTGCCCCCAGGCTCCGCTGGGGTGGGGGCCAGGCCAAGAGGCACATCCCACACTGGCCCACCTGTCC
ACGGTAGGCGCATGACTGCCCTGAGGAGGGGAGGCCGGCATTCCCCGCCACAAACCAGGACGTAATTGGTGGCAGGGCTCTCTGTG
GAAAGAGCCAGTCTGCTGTTTGTCTAGGAGGTCAGTCACAGAGGCCCCGAGACGCCCACTACTGCAGCCTGGCAGGCGGATGAGCC
CAGTATCTGGCAGTGACCAGAGGGAGTTTTGTGCAGACCACAAAGGCTGATGGGCTGCCCTAGATTGGTGTCCCTCTTGGAAGTGG
GCCCAGATGTGCGGGACAGTCCCCAGGAAGCCCCAGGTGAGGGCACTGGTGCCCTCTTGGGAAAGCTGCTCCCTCCTGGGGCCCGG
CTCCCGGCCCAGTCCTCCAGGGGTGTCCCATGGTGACTGGTGCTAGGAACCCCACACCTCTTCCCTTACTTGGGAAGTCACTGGAA
TTGTTGGGCTACATCAGACGGCCCAGAAAAGTGTTTTTGTCATCGGCCAGAAATAGGAGAGTTGTGAGTAGAGGGCCCGGGTGGAG
TTGGGGTGTACTTGGTCTGTGCTCTGAAGGTCACTGTGACAGTCATGGTCCCATGGTAAGGGGCATGGGTTGCTGGAAGAGCTCTT
CCTTCCCGAGTGAGCCAAGCCGGGCTCTCCTGGCGCCAGGGCCTGAGCCGCAGCCACACCACAGCCGCCCTGAAGGCTGCCGGCCA
GGGCTTACCCCTCAAGGGACACGGAATGGCTTCATCAGTACCCTGCAGCCCCGTGGCCTGGCCCGGGTGGAGGCCTAGGCTTCAGC
CATGCGATGTCCCTTCAGAATATGACTTGTCTGCAATCCCTGCTGCTGGGGGGTGGCAGGTACTTGGGGTGAGGGTTAGGGTCATA
GAAGCGACATCTCTACGTCCTCATATTTGCGTCATCTAATTTTGTTTTTGTGAATACGTGATAACATTCACAAGGCTCAAGATGCT
AAAAGGATGAGAAGGCAGTGATGTCCCCATCACCTGTCCTGTGTCTTCCCGTGGCTTTCTCTTTCCTTGGTTATGTTTGAGTCAAC
AGTGGGGCTGACGTTCCAGGAGGGTCCGTGGGCCAGGCTCTTGCTCTCCGAGTGCCCAGGGATGGCTGGAGGCTGAGGAGGGCCTG
GATGTGGAGCCTCAGATACCAAGTGCTTCCCTTCAGGCCGGGCCGCTTGCTCAGAGCCAGCACACAGGGATGCCCGGATCACGGGG
GCCCTGAGAGGGTCCCCTGCTCACAGCCTCCTTCCCTCTCTCCTTCTGCCTCAGATGTGAAGTTCATTTCCAATCCGCCCTCCATG
GTGGCAGCGGGGAGCGTGGTGGCCGCAGTGCAAGGCCTGAACCTGAGGAGCCCCAACAACTTCCTGTCCTACTACCGCCTCACACG
CTTCCTCTCCAGAGTGATCAAGTGTGACCCAGTAAGTGAGGGTGATGTCCCAGGCAGCCTTGCCGGGGCTTACAGGGGGAGACACC
TAGTGCCACGGAAATGCCGAGGCTGGTGCCAAGGCCCCCAAGGGTGACAAGGTTGGGGCTGGGGCTGGCCCCTCGGACCCCAGGC
CACAGACTGACAGGGCACCGGCTTCTTCCACTGCTCCTAGAACTTACTGACTGGCTGGGAGGTCCTCACAGCCTTCTCACGTCCCC
TGGGGCTTCCAGGAGCCGTAGAGTTTCTGGGCGAAGCGTCCGGGACGGAGGCCCCAGGCGGCCCCAGCCAATGGTCTGTGTGGTGA
TGGTGTGTGGGGTTAGGCCCAGGCGAGCTTTGTTTGGCCACAATGTGCGTGGCCAATAAATAGATGCTTGAAAAGGGCTCCTGTG
AGGTCCGAGACACCGGACAACGGGCGGATAGAGACAGCCTTGTTGTTTACGGCCTCTTTGAGAGGCTGCTGCTGTTAAACCCTGGG
ATGACTGTGTCTTTCTTCTTAAAAATGCCATTGTTTTATTCCCGAGTCTTTTCTTAAAGAAAGAATTAAAATGACAATCAAAAGGG
TTTGTGGCATTTACCAAATTAGACCAGAGAGGTGGCCGGGTCAGCCGCCGGCCCCGCGGTGTGTGAGGGAGTGACCGCCTGACCCC
AGCTTGGGGCTGGGTGGGCCTGCAAGACCCGTTTTGGCTCTGGCCTGGGCCGCCTCTTGGTGGTCTGCCCTCGAGCCTCCCGGGGA
CTCCGCACGGGTCTCAGCAGATGCTATCTAGGGTCCACCTGCCTGTCCCCTGCCTAGTGGTGCCTCTGTCCCGGGGACACTGGGAG
TAGCGGCTGCCCAGCCCATGTGTGTCTCGGAAGAGGAAGAAGCTTTTTTGCCGTGGGACACCGAAGTTGGCAGGGGCCTCCCTTCT
GTGTTCTCGGCCATGGCCTCCCTTGCACCCTGCCCCGTGTTATCCTTGGGGGTGGTGAGGTGTCCTCACCCGCTGTAGGGTGGAG
GCCAGCAGCCCGCAGCTCTCTCAGGAAAATGGCTCAGAAACACCATCGAGGCCTCCAGAAGCCCAGCAAAGAGAAAGCCCCTCCAT
CAAAATGAAACTCGCGTCTGCACTTTTCATTTCGAACTCCACGCCCTGAGTGAAAACCGCTTCCCCGCCAGGGGTGACTGCCCTGG
GATGTTGCTGTCTTCGGGCAGTTGTGGGAAGTTGGGCGCTGGCCCTTATTTGAGTAGAGACCATCTTAACTAGATTGGAGGCACAC
GTCTCACAGCTGACAGACACACGGGGTGAAGTTACCCGAGGCGGAGTCCACTCTGCCTGATCAGCTAGTGACCAACGTAGCTGAGC
CCAGACTCAGAAAAACCGTCCACAGCAGAGGCCCCTGCATTTTCTAGGGCGTGTTCTAGAATTTTCTTTGGTGGGTGGAATGTCCA
TCTGTGCAAATCGGGTGCGCAGTGCCACACACCAGTGACTTTTCGCGGAGGAGCGTGCTGCCTTTTTGGAGCTTCTGGCTGTGGGA
GAACAGCTTTGTCCACCGGGGTAGCCTTGCAGGCAGCTGTGGGGCCAGAGGAATGAAGGAAGGTCCTGGAGTCTAGCTGCATGTGT
GACCCTGGAGTGGGTCATGGGCGAGGGACGGGCCGCAGGTGAAGAATCCCTGGATGGAGCTGCCAGGCCCCTGGGGCTGAGAATTG
AAGCTGGCTGGTGTTTTAGGTTGAACGTCAGGAGTCTTGTATCTCACCCCAGGCCTCTGGCCTCAGTTTCCCCATCTGTACAGTGG
GACTGTTTGTGCAGCCAGCCCGGCCAGCTTCATTTGCCATGATGAGAATTTATCTGAGGGGCGGGAGAGGAAAGCCCTCCCTATAA
AGGTACAGGCGCTAAAATGTCATGACCTCAGTGGTCCACCTAAAAGTCGTTCTGGCCTGGGTCATCGCCTGTCGTGCTATGCCTTT
GTCCAGCCCCTTCTGGTTGGGAGTTAAGTGGCACCTGTGCGGCACGTGGTGGGGCTGTGGCCCAGCCCTGCTCCTTGTGGAAGGTC
TGTTTCCTGGGCTGCCTAGAGACTTGGCTTGAAGCCCTAGCGTGGCTTCCTGGCAGTTGGGACACACACAGCCCCAACACATGGAG
CCGGTTCTCCATCCAGAAGCCCCCGGGCAGTAAGCAGCCACTTCAGGCTGCGTGGGACTTGCCCGTGGTGGAGCCTAGGAGAGGCC
CCTGGCTGGGCGTGGCGTTCCAGATTTCACGGCTGCTCTTTCCCACTGACAGTGTGGTGTGGACGCTGCCAAGGGAGTCTGGAGCC
CCAGAGGGTGGAGGTGCAGGACTTCCAGGAGCGTCCGTCGCACTCCACCCGAGGGCGAGCACCTCAGTGGCCGCAGTGGGTGGATG
CATGCTGTGCCAGGCTGATGGCTGGCCCCGGGGCACAGGCCTGAGCGGGAGAGGATGGAGGGGAGGGATCAATGGTCCAGGTCCCC
CTGGCCACCCAGCATTCATCCTCAGTCATGCACGGCCCAAGGCTTCGACAGCCATTGATCATGGAAGGCCAGGTTCACCTCAAGGG
CTGCCACATGGAGAGGTTAAGTCTGAAAAGGCTGAAAAGGCAGGGTTAAAAGGGCCTCCTGTCCAGATCAGATGGCACTGAATTCC
CCAGGGAGCTGGCACGGCCAGTGGGAACAGGCGGTGAAGGCGCTGTTGGACATGGGGACGGGCAGGGGGTGTGCAGGGTGGGCGGG

CAAGCATCTGGTGTCTTGTGGCTCCAGAGACCAGGTGGGAGGTGGAGGCGTTTGGTCCTGAGTGTCCTGACAGGTGATGGCAGCTC
CCACATCTCGCTCAGGTTCAGAGGAGGCAGCATGGCCGAGGGACAGTTTTTGGCTTAGTCTTGCTCTTATAAAGGCTTCCGGGTC
ATGGCACCTGGGAAGGGGCCCTCGCTGCAGGCCCCTTCTAAGGACCCCCTCTTCCCACCTCTCCCCACCCTCTCTCTCTCAGGACT
GCCTCCGGGCCTGCCAGGAGCAGATCGAAGCCCTGCTGGAGTCAAGCCTGCGCCAGGCCCAGCAGAACATGGACCCCAAGGCCGCC
GAGGAGGAGGAAGAGGAGGAGGAGGAGGTGGACCTGGCTTGCACACCCACCGACGTGCGGGACGTGGACATCTGAGGGCGCCAGGC
AGGCGGGCGCCACCGCCACCCGCAGCGAGGGCGGAGCCGGCCCCAGGTGCTCCACTGACAGTCCCTCCTCTCCGGAGCATTTTGAT
ACCAGAAGGGAAAGCTTCATTCTCCTTGTTGTTGGTTGTTTTTTCCTTTGCTCTTTCCCCCTTCCATCTCTGACTTAAGCAAAAGA
AAAAGATTACCCAAAAACTGTCTTTAAAAGAGAGAGAGAGAAAAAAAAAATAGTATTTGCATAACCCTGAGCGGTGGGGAGGAGG
GTTGTGCTACAGATGATAGAGGATTTTATACCCCAATAATCAACTCGTTTTTATATTAATGTACTTGTTTCTCTGTTGTAAGAATA
GGCATTAACACAAAGGAGGCGTCTCGGGAGAGGATTAGGTTCCATCCTTTACGTGTTTAAAAAAAAGCATAAAAACATTTTAAAAA
CATAGAAAAATTCAGCAAACCATTTTTAAAGTAGAAGAGGGTTTTAGGTAGAAAAACATATTCTTGTGCTTTTCCTGATAAAGCAC
AGCTGTAGTGGGGTTCTAGGCATCTCTGTACTTTGCTTGCTCATATGCATGTAGTCACTTTATAAGTCATTGTATGTTATTATATT
CCGTAGGTAGATGTGTAACCTCTTCACCTTATTCATGGCTGAAGTCACCTCTTGGTTACAGTAGCGTAGCGTGGCCGTGTGCATGT
CCTTTGCGCCTGTGACCACCACCCCAACAAACCATCCAGTGACAAACCATCCAGTGGAGGTTTGTCGGGCACCAGCCAGCGTAGCA
GGGTCGGGAAAGGCCACCTGTCCCACTCCTACGATACGCTACTATAAAGAGAAGACGAAATAGTGACATAATATATTCTATTTTTA
TACTCTTCCTATTTTTGTAGTGACCTGTTTATGAGATGCTGGTTTTCTACCCAACGGCCCTGCAGCCAGCTCACGTCCAGGTTCAA
CCCACAGCTACTTGGTTTGTGTTCTTCTTCATATTCTAAAACCATTCCATTTCCAAGCACTTTCAGTCCAATAGGTGTAGGAAATA
GCGCTGTTTTTGTTGTGTGTGCAGGGAGGGCAGTTTTCTAATGGAATGGTTTGGGAATATCCATGTACTTGTTTGCAAGCAGGACT
TTGAGGCAAGTGTGGGCCACTGTGGTGGCAGTGGAGGTGGGGTGTTTGGGAGGCTGCGTGCCAGTCAAGAAGAAAAAGGTTTGCAT
TCTCACATTGCCAGGATGATAAGTTCCTTTCCTTTTCTTTAAAGAAGTTGAAGTTTAGGAATCCTTTGGTGCCAACTGGTGTTTGA
AAGTAGGGACCTCAGAGGTTTACCTAGAGAACAGGTGGTTTTTAAGGGTTATCTTAGATGTTTCACACCGGAAGGTTTTTAAACAC
TAAAATATATAATTTATAGTTAAGGCTAAAAAGTATATTTATTGCAGAGGATGTTCATAAGGCCAGTATGATTTATAAATGCAATC
TCCCCTTGATTTAAACACACAGATACACACACACACACACACACACACACAAACCTTCTGCCTTTGATGTTACAGATTTAATACAG
TTTATTTTTAAAGATAGATCCTTTTATAGGTGAGAAAAAAACAATCTGGAAGAAAAAAACCACACAAAGACATTGATTCAGCCTGT
TTGGCGTTTCCCAGAGTCATCTGATTGGACAGGCATGGGTGCAAGGAAAATTAGGGTACTCAACCTAAGTTCGGTTCCGATGAATT
CTTATCCCCTGCCCCTTCCTTTAAAAAACTTAGTGACAAAATAGACAATTTGCACATCTTGGCTATGTAATTCTTGTAATTTTTAT
TTAGGAAGTGTTGAAGGGAGGTGGCAAGAGTGTGGAGGCTGACGTGTGAGGGAGGACAGGCGGAGGAGGTGTGAGGAGGAGGCTC
CCGAGGGGAAGGGGCGGTGCCCACACCGGGGACAGGCCGCAGCTCCATTTTCTTATTGCGCTGCTACCGTTGACTTCCAGGCACGG
TTTGGAAATATTCACATCGCTTCTGTGTATCTCTTTCACATTGTTTGCTGCTATTGGAGGATCAGTTTTTTGTTTTACAATGTCAT
ATACTGCCATGTACTAGTTTTAGTTTTCTCTTAGAACATTGTATTACAGATGCCTTTTTTGTAGTTTTTTTTTTTTTTATGTGATC
AATTTTGACTTAATGTGATTACTGCTCTATTCCAAAAAGGTTGCTGTTTCACAATACCTCATGCTTCACTTAGCCATGGTGGACCC
AGCGGGCAGGTTCTGCCTGCTTTGGCGGGCAGACACGCGGGCGCGATCCCACACAGGCTGGCGGGGGCCGGCCCCGAGGCCGCGTG
CGTGAGAACCGCGCCGGTGTCCCCAGAGACCAGGCTGTGTCCCTCTTCTCTTCCCTGCGCCTGTGATGCTGGGCACTTCATCTGAT
CGGGGGCGTAGCATCATAGTAGTTTTTACAGCTGTGTTATTCTTTGCGTGTAGCTATGGAAGTTGCATAATTATTATTATTATTAT
TATAACAAGTGTGTCTTACGTGCCACCACGGCGTTGTACCTGTAGGACTCTCATTCGGGATGATTGGAATAGCTTCTGGAATTTGT
TCAAGTTTTGGGTATGTTTAATCTGTTATGTACTAGTGTTCTGTTTGTTATTGTTTTGTTAATTACACCATAATGCTAATTTAAAG
AGACTCCAAATCTCAATGAAGCCAGCTCACAGTGCTGTGTGCCCCGGTCACCTAGCAAGCTGCCGAACCAAAAGAATTTGCACCCC
GCTGCGGGCCCACGTGGTTGGGGCCCTGCCCTGGCAGGGTCATCCTGTGCTCGGAGGCCATCTCGGGCACAGGCCCACCCCGCCCC
ACCCCTCCAGAACACGGCTCACGCTTACCTCAACCATCCTGGCTGCGGCGTCTGTCTGAACCACGCGGGGGCCTTGAGGGACGCTT
TGTCTGTCGTGATGGGGCAAGGGCACAAGTCCTGGATGTTGTGTGTATCGAGAGGCCAAAGGCTGGTGGCAAGTGCACGGGGCACA
GCGGAGTCTGTCCTGTGACGCGCAAGTCTGAGGGTCTGGGCGGCGGGCGGCTGGGTCTGTGCATTTCTGGTTGCACCGCGGCGCTT
CCCAGCACCAACATGTAACCGGCATGTTTCCAGCAGAAGACAAAAAGACAAACATGAAAGTCTAGAAATAAAACTGGTAAAACCCC
AGCGTGGTGCCTGCCTCTTTGCTTCCTGGGCTGGCCGTGAGCCAGGGACGCGTGTCCTGGTGCCCTAGAACCAGGGCAGGGTGGCA
GGCTTGGCGGATGTGGGAGGCCGCAGCCTGTCCTGTGCGCTGTGGGAAGTTCAGCAGCATCCTGACCTCCATCCCCGGGATGACAG
TCACGCCACCGGCCGTGACAACCAAGAATGTCTCCTGACACTGCCACATCCCCGGGGGTGGGACAGAATCCAGCCAGGAGCAGGC
ACACCCCTCCCAACTGGAGGAAGCCCTCAGCACAGGTGTGTGAGGTGGAGGCGGTGTCCTGTCCCCGGGAGGCTCCAGAGAATA
ATTTGCAGGCTGCCTGGCTGGGTGAGCCCACCTCCAACCACGCGAGACAACAGCTCCGGCCTGGGTGACGTGAGCGGTGCCCATTG
ATGGGGAACATCTTCCCCCTCTTCCTTGCCCCACCAGTTTGTCTTCCCGGGTTATTTGCAGATAGGAAAATAAATAAAGCCGGCAT
TCGTTAACCCTCTTCTGGCGCAAACTGCTGTTTGCTCTGGATGAATCATGGTCCTTTGGCGACGCCAGGCTCCGGGAGAGCAAAGC
ACCGTGTCAGGGCCATGATCCGGGGTGGCCTTTCACTGGGATCGTGGGGACCTGGAGGCCGCCTTATAGGACACCCATGACGCCCA
CCTCTGGATTTCAGGTGCACGTGACTGGACTTAACTTCAAACCCCAGGGTGGAGGCAGGTAGTGGGAGTGCCCTGGGAAGGTGTCC
TCGGACCTTGGTCACTGCTCCTGAACCCATCTGTGAGGCTGGTTTGTCCTCATCCCAAGCTAAGTGGAAGCTCAGGTCCCAAGCCA
CCGATGGGTGCTACTTGTCAGCTGCAGGTTGAATCTCCGTGGCCTTTATGAAGCACCTGCTGTCTACCCTTCCTGCCTTGTAGAGC
ACTCCTCCCAGGGCTCAACAGTGGGGCCGGGGTGGTCGGTGTGTTGGCTCCACAGGCGCCTGCCCTGGGAGGAAGGTGGGGTGTGG
AGGGAAACGCTTGGCCCCTGTAGGTCTCCACCAGCCTCTCCCCTGAGGGTGGGGGCTCCGGGAGCCTTCCTCGAGGGAGTCCTATA
TTGAGTGGGTGGGGGAGCCTGCAAGGTGCCCCTGACAGGTCACATCAGAAAGAGCTCAAGGGACAGTCGGAGCCAGAGGTGACACT
GGTGGCCACTCGGGTGGCTCACAAGGCCCAGCTCCTCCTTGCTCCTGGGCAAATTACTCTGAAGGCAGGGACCAGGTCTGCACCAT
TGCGGCTCTCCAGTTCCAGGCAATGGCCAGGTCCTGTGTCAGGGCTGGGGTCCTAGGGAAGCCATGTCCCCACCCCCGGCCTGCAG
CTGGGTTTACATTCATCCCCCGAGAGCACATGGGTGTAGCAGGAGGCCTGTGCAGAGAGCTCCGACCATCGCACAGGGCACCTTTG
GTTGTTTCACGGAGCAGGCAAGGGAGCCATCGGATCCTGTTAGGTTTGAGCAAGGATGTGGGGAAGAAGCTGGAGAGCCACTTTGC
CATGCAGGGAGAGGAGCACATGGGTCTAGGGATCTACTTTAGTGTTTGGAAGGTTTTTTAAGATGAAAGAGGGATGTGTAGGCTGA
TAGGTCTGGCAGAGCCAAAAGGCAGCGACATGTCTACTGGGAGAGATGGAGCTGAGCGCGGGGCTCAGGCAGGGTGGCAGGGCAGG
GCCGGGGCCCTGGGTGGGTCAGGTGGGTTCACAGCCAAGTGTGTAGAGAGGGCTTGGGCCCAGAGTGAAGCAGTTGCAAGCTCTCC
CACAACCCATTCTCTCTGTCTCGGCATCTGTGGCATCCCGTGATGGGTGGGTCTGTACACACCCCACCCCTGGCTGTGCCACAATG
GGGGTGTCTGTACACCCCTCACCCCTGGCTGTGCCACGATGGGGGGTCTGTACACCCCCCATCCCTGGCTGTGCCACGATGGGG
GTTCTGTACATCCCCCAGTGATGGGTGGGTCTATACATCGTGGTTATGCCACGAGCTCCAAGGCTGTATCAGTCCGTTTTCACACT
GCTAACAAAGACATACCCGAGACTGGGTAATTTATAAAGAAAAAGAGGTTTAATAGACTCACAGTTCTACGTAGCTGGGAGGCCTC
ACCATCACGGCGGAAGGCGAAAGGCCCGTCTTCCATGGCAGCAGGCAAGAGAGAATGAGACTCAAGTGAAAGGGGAAACCCCTATA
AAACCATCAGATCTGCTGAGACTTGTTCACCACCATGGGAACAGTATAGGGGAAACTGCCCCATGATTCAATCACCTCCCACCGGG
TCCCTCCCACAACACGTGGGAATTATGGGAGCTACAATTCAAGATGAGATTTGGGTGGGGACACAGCCAAACCATATCAAAGGCAT
CGTCTGGCCACTCTAGGCCCTTGTTCCCAACTCTCAAACTCCACTGACATCACCTCTGTGCCTGCAACCCCTCCTGGGATTCAGTG
GAGACCTCGTCCCTTGTTGGAGTATCTGCGCTACCCAGAGGGTGATGGTAAAAACTGACCTTTGAGGAACTTCAGAGACAAAGCCT
TCCGCAGTCAGCTCTCCCTGCAGCCCCTTCGCAGAAGCCTGTCCTCACACGCCCGCCACCCCTACCTGCCCTGCCTCCTGGCCCAT
CTCCCTGCAGCCAGACAGTCCTCATCCATTCTTCATCCTCTGCCCCCAACCCCCAGCCTTCCTATCTCCCTTCAGCTCCGTCCAAG
GTCCCTGGGACTCAGCCCCATCTCTCCCCAGCCTTGACCCCAATAAGCTCGATGTCTTTGCCCAGGAGCTCCTGACACACCAGACT
GCATCCAGCTTCCTGTTCTGCGCTTGGCCCACCGCCTCGGTCCACAGGAACCTTCATCTCTGCTCTGACTGTAAGGATGGGCTCTC

TGAGGGCTTCCCCACCCCACAGCAGGCAGGTGGTTCTGGGAGTCTGGGATTGTCATGCCTGGCACCTTCCTGCTCTGGCTGAGCC
CCTCCAGGACGGAAATCTCCCAGATTTCTCTCTGCCTCCGTCTCCCCACCCTGGCATACAGTGGGTCCTAGGAAGGGGGCCGAATG
GAAGGGAGGTCGTGTCGGCTCCTACAGGGCAGCCCCAAGCTGCTGGGCTGAGGTGCAGAGCCATGGGTCCAGCCTGACCCGCATGC
CGGGAGCTGATTCCATGGCTGGAAGGGTCGGGGGTTCCCCAGGAGCCCAGACAGCACCTAGAGTTGTCACAAGGAGTGAAAGGCCC
TGAGGGGACAGAGGAAGGAGGCTGGGCAGAGTGGGCACGTGGACTTCGGACCGCGCTTGTGGGATAACAGTGGCTAGGGCTGTGCA
GAGGGTGGTGCACTGCCCATGGGAGTCCAGAGAAGCCAGGTCCAGTGGCAGTGGCATGCATCCGGCAATCCCTCCGGCTACCCTGC
ATGCAGCGTGACCCTCCTTGAAGCACCCCAGGAAGCAGTCACAGCCTCGCTCTGCCCAGCATTGGGGGCCTGGCCTCTGAATCTGG
TGGGGAAGGCTGTGTTGCCTGCTGGCACCCGCTCTATCACCAGTCCCACCATAAGGCTAGTCCCCAAAGTCAGCACTGCAGCCAGG
GCCAGCGAGGGCCAGCAAGGGCAGGCCTGTGCAGGGGCTGCAGCGTGGGGTGTGCGTCCCGCACTCTGGGCCAGCCTCGCAGCTCA
CAGGGCTCTGTCATCCCAGGAATGTCTTTGATCAAGAGTTTATGCAAGACGAGGAAAGCAAGCCCAGGACCTCTAATCCGTTCCCA
CAGTCCCCTGCCCGCCAGGGCCCCACAAGCCCAGCGCCACACCGCCAGGCCTGAGCAGACAATAGCAGGCGGACGGGTGGGCGGGC
AGCTCTGGGGCCGCTGGCCTTGGGGCCCACATCCGGGAGCATTGTCACCGCCACTCACGAGGGCACACAAGGAGGGCTGGGGAAGA
GAGAATGCAGAGCCCTCTGCTGAAGGGGCTTCGGTGGGCCTTGAGCCTTTCAAAGACGGCGGTGTCCATTAATCCTTTTAATTTGC
ACCTAGCGGCAGCTGAGACAAGCCTCTCAACCGCCAGCCACTTTAAGAGCTGAGTGGTCTAACCCGAGGATCAGCTCCTATTCCAG
ACCCTGGGCCCTCTGAGCCTGTCGAGGCAGTCCTCCTTGCCTTTGTTTAATCACGGGAGGAATCGCAGCTGGAGGTTACTGAGCAC
GCACTGTGTGCCTTGTGCTCAACACCGAACACAGTTTCTCTCTGAGTTTTGCAACGACCCCATGGGTTGGTGACAGGGAGGGGACA
CTCTGGTGGCAGCCACAGACACAGCCCTGCCCTCCCAGAGCCTACACTCTCCCGCACACAATCATCCCATCTGCTAGGTCAGGGAT
TGGCAGGTTTTCTGGAAAGGGCCACAGAGAGAGGCTTCAGGCTTTGTGGGCCACAGGTTCTCTTGCCCTGTGCAAAAGCAGCTGCA
GCCGCTCTGTGAATGAGTGAGCATGGCTGTGTGCCAATAAAGCTTTATTTACAAAAATAGGCAGCAGACTGGATTCAGGCTGAGGG
TGGTAGTTTGCCGACCCCTGTTGTAGATGAGTAAACCGAGGCCCAGAGAGGTAAAGTAACTGAGCCAGGACTGCACAGCTAGTGTG
TGACCAAGCCAGCCTGGTGGGTCAGCCTCATGGAGAAGAAAAGGCTCAAGCTTCAAAAAGGGAAATGCACGGAGGCTTCATTGGGC
CATTTGCAGCCAGGCCCCACGCACACAGCTCAGGGCCCTGCTTCGCCAGCTCCACCTGTTTCGAGGCTCCCAGTAGGTTGCCCCTG
CGGGCGGCACTGTGTCTTGGCTGCTGGCCTGGAGAAGGGCAGCCAAACAGGCCGGCAGTGGACATCTGAGGGTGACCAGCTGTTTA
AAAAGAAAACTCACCGGCATGTATAGGCACACATTAAGCACACACGTTTTGGAGTAAAAGGCCCTGGGGTTGGATCTCGGATCTGC
AGCTTTCCAGCTGTGCGGCCTCATTTCTCTGGGCCTCAATGTTCACACCTATAACATGGGGAAATCCTGCAAGAACACAGCCCATG
ATGGGCAGTCGTCTCCATCTCCTACTCACGACAACGACCAGCACAGAACGGGGGCTCCATAAATCTCTGTCAATAACTGAATTCTT
TCTGGGGTGCTGAGGATTCTATGGCTCAATGTGTGTGAAGCACTGCTGAGCACCGAGCTTGGGGTGCAGTGATCACTCAGGGAACG
CCATCATCCCCTCGGCGCATCCGCATAATCACCATCAGCATTCCATGCACTGCCCAGGTCTGGAAGCACAGGCCTCCATGCCACGG
CGGCCGCCTGGGTGGTGGCACCAAAGCCGATCTTACTTCCCTCCTTTGGCACATTAAGTAGACAGTTCTGGGTCTTCTTGACACCA
CGGATGCCCCCTCCTGGTGTTGACAGCACCCAGACTTTGCTCCAGGGAACCACCATTCCCAGATCTGTGGTTTGAGTGGGACCGAA
CCCATCCCCAGCTCCAGGCCAGGTCTTCATAGGCTGAAACCCAACTGCACATCCACACCCACCAGAAGAGCAGCAAGTTAATGGAT
GGGTGAGAAATCAAATAGAGCCATTGAGTCTTGAGGAGACATTTTCTGGAGCTTCTGAAAAAGATAATGCTTCCCCCTTCCTTAGG
ATTCCCAGCAGGGAAACACAAGCCCAGGGGTGGCACTGCCCCCCTGCAGCCCTAAGGAAGACTGAGCTGCTCCCAGGACCTCACA
GGGGAGGCTGAGCGTAAGGCCAGCCCTGAGGGAAAGCAGGGAATGGGTCTCCAGACAAACTGCTGGATCAAGCCTCTCCTGAAGTC
ATCCCCCTTTTGTTCCAGAAGCCAATCATTGTCCTTCATTAAGTCAGTTTGATATGGGCACTGTGTCATTTGGTTTTCAAAGACAC
TTAACTATTACAGCTTGTGTGTGTTTTCTTTTTTTTTTTTGAGACGGAGTCTCACTCTCGCCCAGGCTGGAGTGCAGTGGCCTGCT
CTCGGCTCACTGCAAGCTCTGCCTCCCGGATTCACGCCATTCTCCTGCCTCAGCCTCTCGAGTAGCTGGGACTACAGGCGCCCGCT
ACCACGCCCAGCTAATTATTTGTATTTTAGTAGAGACCGGGTTTCACCGTGTTAGCCAGGATGGTCTCGATCTCCTGACCTCGTGA
TCCACCCGCCTCGGCGTCCCAAAGTGCTAGGATTACAGGCGTGAGCCACCGCACCCGGCCGCTTGTGTGTGTTTTCTACCTCTTCT
AGATCTAACATGTTATGATTGATATAAACAACAGGAGCGACTGACAAAGTTACCATGTGATCTTGGAATTTCAACATGCCTGTCTT
GCTTAGCTGGGAGTTCCACAGGGTCAGGGAATGACTCCTCTTGGGGCCACCAGTGCCCAGAAGTATGCCTAGAACTGCTCTGTCTG
AAGGGACAGGTCCCTCTTCCATGGCACACACTCTGTGTGTAACAGAGCACACGTGTGACCAAACTGACCTGGAGAAAGGGCCTGAC
CACATCCATATCCCAAGTTCTGAGGCCGGATAGTGCCTGCAGGGGCTGAGACCAGGGGAGAGGCTCAAGCCAGCCTTCTCTGGTGG
ACCCTGACTCAGTCCTCTGTGCAGGGCAGCTGCGCCTCCCAGAAGGCAGAGGCCTCTTGGAGAGTGAGCCTTGCGGTCACAGCGCC
TTTCGGGGGCTCCCGCCACTTGCCCTCAGTGGCGGCTGAGACCTGGGGGAGTCCAGGCAAATCCCTGAGGAGGCCCCACATGCAAG
GCCAGAAGTGTCTGGATGGTGTGGCCGGCACCCTTACCCTCCAGACCAGCAAACGAAGCCCAGGCAGGGAGAGGGGCCTTGTAGTT
TTCTGTGGCTGCTGTAATGAATTTTCACACACTCAGCAGCTTAAAACAACCAACATTTCTCTCATAGTTCCAGAGGCCAGCAGTGT
CTGCAGTGCCCGCTCTCTCCCAGCTTCTGGTGGTTTCGGGCGACCTTCGGCTCTTCTCAGCTGGTCCATGTGCA

HUMAN SEQUENCE - mRNA (SEQ ID NO: 17)
GCAGTAGCAGCGAGCAGCAGAGTCCGCACGCTCCGGCGAGGGGCAGAAGAGCGCGAGGGAGCGCGGGGCAGCAGAAGCGAGAGCCG
AGCGCGGACCCAGCCAGGACCCACAGCCCTCCCCAGCTGCCCAGGAAGAGCCCCAGCCATGGAACACCAGCTCCTGTGCTGCGAAG
TGGAAACCATCCGCCGCGCGTACCCCGATGCCAACCTCCTCAACGACCGGGTGCTGCGGGCCATGCTGAAGGCGGAGGAGACCTGC
GCGCCCTCGGTGTCCTACTTCAAATGTGTGCAGAAGGAGGTCCTGCCGTCCATGCGGAAGATCGTCGCCACCTGGATGCTGGAGGT
CTGCGAGGAACAGAAGTGCGAGGAGGAGGTCTTCCCGCTGGCCATGAACTACCTGGACCGCTTCCTGTCGCTGGAGCCCGTGAAAA
AGAGCCGCCTGCAGCTGCTGGGGGCCACTTGCATGTTCGTGGCCTCTAAGATGAAGGAGACCATCCCCCTGACGGCCGAGAAGCTG
TGCATCTACACCGACGGCTCCATCCGGCCCGAGGAGCTGCTGCAAATGGAGCTGCTCCTGGTGAACAAGCTCAAGTGGAACCTGGC
CGCAATGACCCCGCACGATTTCATTGAACACTTCCTCTCCAAAATGCCAGAGGCGGAGGAGAACAAACAGATCATCCGCAAACACG
CGCAGACCTTCGTTGCCTCTTGTGCCACAGATGTGAAGTTCATTTCCAATCCGCCCTCCATGGTGGCAGCGGGGAGCGTGGTGGCC
GCAGTGCAAGGCCTGAACCTGAGGAGCCCCAACAACTTCCTGTCCTACTACCGCCTCACACGCTTCCTCTCCAGAGTGATCAAGTG
TGACCCAGACTGCCTCCGGGCCTGCCAGGAGCAGATCGAAGCCCTGCTGGAGTCAAGCCTGCGCCAGGCCCAGCAGAACATGGACC
CCAAGGCCGCCGAGGAGGAGGAAGAGGAGGAGGAGGAGGTGGACCTGGCTTGCACACCCACCGACGTGCGGGACGTGGACATCTGA
GGGGCCCAGGCAGGCGGGCGCCACCGCCACCCGCAGCGAGGGCGGAGCCGGCCCCAGGTGCTCCACATGACAGTCCCTCCTCTCCG
GAGCATTTTGATACCAGAAGGGAAAGCTTCATTCTCCTTGTTGTTGGTTGTTTTTTCCTTTGCTCTTTCCCCCTTCCATCTCTGAC
TTAAGCAAAAGAAAAAGATTACCCAAAAACTGTCTTTAAAAGAGAGAGAGAGAAAAAAAAAAAAAAAAAAAAAAAAAAAAAAAAAA
AAAAAAAAAAAAAAAAAAAAAAAAAAAAAAAAA

HUMAN SEQUENCE - CODING (SEQ ID NO: 18)
ATGGAACACCAGCTCCTGTGCTGCGAAGTGGAAACCATCCGCCGCGCGTACCCCGATGCCAACCTCCTCAACGACCGGGTGCTGCG
GGCCATGCTGAAGGCGGAGGAGACCTGCGCGCCCTCGGTGTCCTACTTCAAATGTGTGCAGAAGGAGGTCCTGCCGTCCATGCGGA
AGATCGTCGCCACCTGGATGCTGGAGGTCTGCGAGGAACAGAAGTGCGAGGAGGAGGTCTTCCCGCTGGCCATGAACTACCTGGAC
CGCTTCCTGTCGCTGGAGCCCGTGAAAAAGAGCCGCCTGCAGCTGCTGGGGGCCACTTGCATGTTCGTGGCCTCTAAGATGAAGGA
GACCATCCCCCTGACGGCCGAGAAGCTGTGCATCTACACCGACGGCTCCATCCGGCCCGAGGAGCTGCTGCAAATGGAGCTGCTCC
TGGTGAACAAGCTCAAGTGGAACCTGGCCGCAATGACCCCGCACGATTTCATTGAACACTTCCTCTCCAAAATGCCAGAGGCGGAG
GAGAACAAACAGATCATCCGCAAACACGCGCAGACCTTCGTTGCCTCTTGTGCCACAGATGTGAAGTTCATTTCCAATCCGCCCTC
CATGGTGGCAGCGGGGAGCGTGGTGGCCGCAGTGCAAGGCCTGAACCTGAGGAGCCCCAACAACTTCCTGTCCTACTACCGCCTCA

CACGCTTCCTCTCCAGAGTGATCAAGTGTGACCCAGACTGCCTCCGGGCCTGCCAGGAGCAGATCGAAGCCCTGCTGGAGTCAAGC
CTGCGCCAGGCCCAGCAGAACATGGACCCCAAGGCCGCCGAGGAGGAGGAAGAGGAGGAGGAGGAGGTGGACCTGGCTTGCACACC
CACCGACGTGCGGGACGTGGACATCTGA

Table 4 (mouse gene: Myc; human gene MYC)

Mouse genomic sequence (SEQ ID NO: 19)

Mouse mRNA sequence (SEQ ID NO: 20)

Mouse coding sequence (SEQ ID NO: 21)

Human genomic sequence (SEQ ID NO: 22)

Human mRNA sequence (SEQ ID NO: 23)

Human coding sequence (SEQ ID NO: 24)

```
MOUSE SEQUENCE - GENOMIC (SEQ ID NO: 19)
CTTCCTTCTCCCTTCACTGAAACTGAACTGACCTTGGGATGGGAAACCCCCACTTCATGGCACCCACTCTAAAAGATGGCTGTTG
GTCAAACAGCCTAGCAAGCTCTAAACCAGGTACTCTGCACCTGCCCTCTGACGCCCTGGGATTATATGGCAGAGGGTAAGAACAGG
AATGACATAATTAAAACCAGCCTCAGAAGCTGCTGAAGGCATTAGAACTAAAGCCCAGAAAAGCAACAAACTGTTACCATCAAAGC
GTGAAAGAGGAAACGGTTAATCTTTTCAAGGGAAGTGAAGCAACTCGGAAGGCCATAGGAATCCACATCACCTGAACTGCAGACCA
AGCCTTGGAACTGGGGCTTTCAAGCTACATCTGCTACTCACACCTTTGACCTCTCTGGGTGAATTGTTTTCTTACTAGTCACCAAT
GTTTTCAAACCTGGATTTTCACACTCCCTGGAATGCCTGGAAGGGCTGCTACCAGCGGACCTATATTGCAGGGCCACCAACTGCCA
GGTTCTGAGCCACAGCTCTTAGCTAGGACCAAGCAAATGGTCCCCAGTGTGTTACAGTCTCCTGTCCTCCAAAGTGAGTTCCTCAG
ATGCACTCCTTTCCAATCCTNNNNNNNNNNNNNNNNNNNGATTTTCCTGTGGGCACATCAGCTTCCTATGCTGAAAAAAACAGAG
AGTAGTAGAAAGGGAAAGCCTAATCAAGCCTGCAGCTGAGAGATTGCCCTGTGCCACAACCCCCAAGTCCACCCCCTTATAGGACA
GCACCTTAGTCATTCAGGGAAAGCCTTAATGGATTGCCTTGATTCGCCTTGATTCCATAATGTAATTGACCATGAGTAGAGCAAGC
TGCACCAACTCCTATCGTAAATGAAGACAGGGGTGAGACTGGAAGGGTCAGATACTGGAAAATCTGAGATTCTCTGCCTTGGTTTC
TCATCTTGATCAGTTTCAACCAAAAAAGAAGGAAGCTTTAAGCAGGACTCCTTGTTTTTGTCAAAAGAATATGCTAAAGTACACAT
TCCCATTATTGCTCATGAGACCTTGTTCATATGTCACCAAGAATATTCAGTGGTATATGTTTGTTCATACCTTCTTAAACATAATT
TAAGATTTCTTTATTTGATGTATGTGTTTTGCTTGCATAATGTATATGTACCACGTGTGTGCTTGTACCTGCACAGGTCAGGAGAA
GGTTTCAGTTTCCCTGGATTGATGACTGTGAACTATCATGTGTATGCTGTGAACTGAACTAAGGTCCTCTGTGGAAGCAACAAGTG
TTCTTAACTGATGATCCATCTCTCCAGCTTCCGCTCAGAAATTTTCACACTTAAAGAAATAAGGGCTGGCTGGTGAGATGGCTCAG
CGGTTAAGAGCACCGACTGCTCTTCCAAAGGTCCTGCGTTCAAATCCCAGCAACCACAAAGAAATAAGGGCAGGGGCTAGAGAGAT
GGCTCAGCAGTTAAGAGCACTGACTGCTCTTCCAGAGGTCCTGAGCTCAATTCCCAGCAACCACATGGTGGTTCACAACCATCTGT
AATGTGATCTGATGTCCTTTTCTGATATGTCTGAAGACAGCTAAATTTTACTCATATACATAAAATAAAGAATTCTTTAGAAAGAG
AGAAAGGAAGGAGGGAAGGGAGGAAGGAAGGGAGAGAGAGAGAGAAAGAACGGAAGGGAGGGAGGGAGGGAGGGAGGGAGGGAGGN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNCCCTCCTCCTAGCTTTCTGAAGCCA
GTCTTCTCCGGTTTGCCTTCAGAACAAGATGTAAAACTCTCAGCTCCTTTAGCTCCATGCCCGGACTGGATGCTGCCACACTTCCT
GCCTTGATGATAATGAACTGAACCTCTGAGCCTGTAATCCAGCCCCAATTAAACATTGTCCTTTATAAGAGTTGCCTTGGTCATGA
TGTCTCCTCACAGCAATGGAAACCCTAACTAAGACAGCAAGTTTAGCAGTAAATGGGGTGGCAGAGACAGGGTGATAGCAGGAAAT
ACTTTGACATAGCTAGTGAGACAGCAGGTAATGACAGTAGCCAAACCAGCCTGATAAACAAACAGCTAGCCCAGAATGCATAGCAC
AGCAGCAAAAACATTGGAGAACTGGCTCAATGACAAAGTGGAAGGAGGAAAGGCAATTCCTAAATGTTGTCATCTGATCACCATAT
CTGCACACTGAAGCACACACACACACAAATAAACAACACTTTAAAGAAATAAATATTACATGATGATTGCAACAACCACAGGCCAT
CGGAAAAGAAAGAGGAAAAAAAATCAATGGAGCAAAGAGACCAGGCTGCACACACAGGAGGAACATCAGGAGACCCAAATTCACCC
AAATGTTCTCAATGTCAGCATGTTTGCATCTAGGTCCCAACACTCTAAGGGGATTGGCAGGATCCCTGACCCCGACTCACTAAATA
CCAATAGGAAGCCCCAACCCCCACCACCTATAACAACCTCAAATATCTCCTCCTGTTGGTCAATGTCCCGTGGAAGCAACATTACC
TCTAGTTGAGAGGTACTGATCTCTAGAATCCAAAAGTGTGAGCATTTCCAGAAGCTTTCCCAGCAAGCAACAAAAAAGAGATGGTC
TCACGGTCACCGGTTGTGGGGAAGATAGCTTGAGTGCCTTTAGGGAGGTTCATTCAATGATAAAGTCCACATACAGTAGTCAGGAG
GGAAATGGAGAAGGTGAAAGTGAGAGCAAGCAAGGCTGAGCTGCACTCAAAATTCTACCTACAGAAGTCAAGAGCTGTGACCCATA
GTTTGGGGTCTGTTCTAGCCAATAAGAGGCACAGGCTACTATTGCACTGGCTTCTAAATGAAAGTCAAAGATTACGGACAGAAAAA
AAGGAAGAAAAAAAAGTAAAAAAGAAAGCAAGACCCGTCATGCACCTCCTCCCCAGTCACCTTTACCCCGACTCAGCAAGGGTCCA
TCATGGAGACAGATGCCGCCCAAAAGGACCATCACAAAGGCCGGCCAGTGAACAAAAGTGCAAGATTGACTTGGGTTTGCCATTTA
CAGAACAATAAACGAAGCCTTTGAAGCACTGTTAAACAAAAGAGCCATTTGTCTATGCTGGTGGCTACAAAGGAGAACAGGATTGC
AGCAGAGGACTAAGAAAAGAAAGAGAAAATGCCAAACTGAGATGTGCCTGGACTTTACCGGGTCCTGCCCAGAGACCTTATATGCC
AGGACCTAAGATCTAAAAGCCACTGGGTAAGAAAACAATCAAATATGCTTAATGCAATACAGGCAATGTAGGTTCTTGGGTATTGT
GTTAAAAATATCTACTAATTTTTTTTCCTTGTCAGCTGCTTTAATTTATGCAGTATGGGAAGGGATCACATCGGAAAAGACAAGGG
AAGAACAGAAAAGAAACACCGTACATTTTCAAATGCACAAAGATCTCATGCACTGCCTTCTGTTTCTGTGCCTATGAATTAGTGAT
GCTAAATTTTATCTTATTTTCTTGCCTGGTGGGGGGGGGGGGAGGGCGGGGAGGGTGACTGTGTTCCTCTCTGTGGTGGCATAGC
TGCCATTTACATGCCTGTGAGGTCCTCTTTTCCTTTAGCATGCCATGGCTAGCTTGGTTTTTAATCTAACAGCGCTTCTTTCCAAA
```

TGATGTTTCTGCGGCAGACAGGTATTTATCATTGTCTTTGCTCTCGTCACTTCCCGACAGGACTCGTGAGAACTGAAGTAATTTTG
AAATCGTAGCCCGTCCTTAGGGTCTCCTGCTTCTCTCCCGAGTAGGAGCTAGGTTTTTGATCCAGTTTGTGTGGATTTAGTGACTG
TTTTTATACATGGAAGGTTAGCTCAGTGGTGATGGTTGGGGGGGGGGGCTCTTTGAAGCATGCAGAAAAGAGATTCAAGAACATTG
GGGGATGTTATCTAGTGGTTTTCTCCTTGTTGTTACAAGAGTGACTTTGGAGAAGAGGGGTATACTTTGGCTCAGAGTTTGACGGA
AAAATCTGGGATGGAAGAAGTCATTGTGGGAGCAGCTGGAAGCTTTGTCACTTGGGGAGCCTTGTCATACTCTAGCCAAAGTTAGG
AAGCAGGTTGGAGGGTGGGGAGACAGATTCTGGAGTTTGGCGCTTCTGTTTTATACCTTCTCTACCCACATTGTGAATAGACCTGA
CCCCTGGGTTGACACAGATCACATTAGTGTGAGTAGGTCTTCCTTCTACACATATACCTCTCTAGAAATACCCTCATAGGTAACAC
CAGATCTGCATCTCCTAAGTGATCAAGTCGACAATCAGAACTAACCATCATAGGCAGGGCTGCAACACACCTTGAATCCCGTGACT
TAAGAGGCAAAGACAGGTGGCTCTCTGAGTTCAAGGCCTGCCTGGTCTACAGAGTTTCAGGACAGTCAGGGAAACCCTACCTTGAA
AAACAAAAAACAAAAAACAAAAAACAAAAAAAAAGAAAAGAAAAGAAACACCCAAATTAAGCGTCTCAAGGATGACCGTTCTTGGT
ATGTTATTCACACCACATATTTGGACCTCCCCTCTTGTGTCTTTGCAAAGTAAGATGTTGGGCCGACTTGTTCATTCTAGCTGGGA
ATATACAGTTCAGTGACTGAATGAATGCTGACCACAAATGAGGCCCTGTTTCAATACTGAGCACACCTTCCTCTCCTTCCCATAAC
AGCCCTTTCTTTCTGGAGGTCTTCCTTTTTTTCTTAAAAATGTAAGAGCATCATGACTGTTAGTATGAGACAGTGAGGTTCTGGGC
ACTGTGATTCTTAGTTTTTCATACTGTAAGAGGGAATGTACTCTCTGCCATCGGGACACCCAGTGGAACTGCTCACCTGGAGTCTT
GCCTCCACGAAGACTAGGATCTTCTGGAAAGCTGTGTGCTTTGGACATCTAACTCACATCCAGCAATAGCTCCCAGAAAAGGACCT
CAGAGTTACAACGTTCCAGAGGAACAAAACAGTGGGACATCTTCCACCATCTTCCAAAAGGAAACCTCAGAACAGAACAAGCAACC
TTTGTCCTTAGTAAGCTTGTCCACAGAGGCACGCATGGCTGTCATCCGAACACTGGGAAGTCCAAGGCAAGCGAACAGCAAGTTCA
AAACTAGCTTGAACTGGCTGCATAGTGAGGCCCTATCTCAAAACATCACGGGCTAACTATGTAGCTCAGAGACAAAGCCCTTGTCC
AGTGTTTGAAAGACTCTGGATTTGATTCCCAACAATAAAAACAACAACATACACTCAGATACTCACACACACACACACACACACAC
ACACACACACACACCCAACTCCTTAAACACTAAAATTAGTGTAGGTAGAGTGGCACAGGAAACTCTAATTCCAGCACTACAAACC
CTGAAGCAGTTTTTATTTTAGTTAGAAGCCATCCTAGGCTACCTTCTCAACAAGAACATTCCAATCACTACACTTTATTGACAAGA
TTATAAACTTGTGGCTTTCCTGTCCTTTTTAAAGTATTTTTTAAAATTTATTTTACAATGAAGTGGTACCCTTTCATTTCCTAATA
TGTAAAATGGGGATGTCCCCTACCTTCCTGTTACTTAAAAGCAACACCCCGGGTGATGTCATCAGGCTGGGGTACAGTACGGGCAA
GTCCCCAAACCACAGGCTATTCCGCTACCTGGGTTCCCACACCTACACTGGACCGGCATGATTTGGACGTAAGATCTTAAAATAAT
CCATAATTACACCTTTTCAAAAAGGACCTTTTGAGGGTCAGCTCTACCCACTGTCATATCTGTTGGTGCTGTCCTGTGTTTAGGGA
GAGGCAGAGAGAAAGCGGGTGTGCGCAACCTCTAAGAGAGTAATAGTAAAGAGACAAGCCTAGGAAATCTTCCCCAAGAGGCCTTT
TGGGGAAGGCTATCCCCCCTTCCGTGGATGTTGAATACCTTGCCTGTTGCTCAGATGCAGTCAAGCATCTGATGATGCTTGAGGCA
TGATGCTGAGACCACTGTGTAGGTTGGTACAAATTCCCTTTCTATTATACCCACCCTTATGCAGTTATTTGGGTTTAAATTTTCAA
CGTTTTAAAAAAAAAAAAAAAAAGTATTGCCGGGCAATAGTGACGCAGGCCTTTAATCCCAGCACTCGAAAGACAGAGGCAGGCAG
ATCTCTGAGTTTGAGACCAACCTGGATGGTCTAAGGCCTTGAACTCAAGAGATCTATCCACCAGCTTGTATCCCATTTAATTGAGT
GATTTGTTTGCTCTCTCTCTCTCCCGCTCCCCCTCTCTCCCCCCTCTCCCTCCCCTTTTTCAGTTCTTTATTTATATTTGAATATT
ATCTCTCTCTCAGATGTGTACTTGGTGACGGTCTTTTCTACAAAGGATTATCTGTTTCATGACACGCCATTTATTAATCTGCTTGT
CCCTGGAGCTTCTGTACAGTAGCGTGACCTGATCCTCTAGCTTCTCCATCCCAGATGATTGGGATTCAAGGCATTCATCACCACGC
CTACCTAAAATTCCAACCTTTTAATAAAATGGCTCTCTAAAATATTTTGGCAAAGGAATCTCACTCTTTCTCTTCACTAATATAGT
TCAACCACCCAATTCGTAAGCAGTCGGGCTGAGGGTGAGCATCCTTAAGAACATAAAAGCAAAATTTGGAGGCTCAAGATTCAGTT
TAGTTGCTAGAGGGCTCACATAGCATGCCCTCCCCACCCGGGATTCCATTCTCATTTATCGAGGCATAAGGCCAGGTGTGGTGGGA
TATGTGCTGGGATGCATAAGATCTTTTATAAAGAAGAGGAAGAGGAAAAACAGTTATACAAAACTAATAAAACTGTGTGAGTTTCA
GGCTAGCAAAGAATAGTCGTGAGAATCTCTCTCAAAAAAAAAACCCCAAAATAATAATAATGATAATAATGATAATAATAATAATA
ATAATAAAACAAGGCAATAATAAGCTAATGCTCCTGCCTTGCATTCTGACTCCTTTGCCCAGTAAAATTCAATGCTCTGCTTTGA
CACGTCCAGCTTACAACACGACAAAGGTGTGAGACATGGGTGCTAAAACTTGTTCTATTGCCTTTCCGTTTCTGTTAGATCTCCTT
CACTTGTATACCTGTGACTCATTCATTTTCCCCATCCACAACTAGGGCTCTGTTTTGGGTGACTGTCAGAAAGTAGGGATGGGTTC
ATCTACTCCCCCTCTTTGCAACTGAATAGCCACCTCTGAACCATTTTTTTCTCTAGTAATTTCTCTTCCTTTGCCTCCTTTGCAGC
CTAAAAGAGTCATTTAAAGGATGACCGGAAGCTTGTCTTAGGCAAGGAAGCATCTTCCCAGAACCTGGAAACCCTGCAGCCCTGCC
CCCATCCGACCTCCGCCCTCGTTGGCTTCGCAACGCTGTGGTCTCTGTGGCCAGTAGAGGGCACACTTACTTTACTTTCACAAATC
CGAGAGCCACAACCCGGGTGGTGGGGGGTGAGGGGGCGGGAAAGAGTCTCTGCAGCAAAACGCAGACTAGGGATTGGTGGCTCTT
GGTGTTTGAGGCAAAATCCTAGAGCCTGTAGTCATTTTGCAATCCTTAAAGCTGAATTGTGCAATGAGCTCGATGAAGGAAGATAC
TATCATTCAACAGCTGAATCCTAAATTGCAAACTCAGTGGCTAATAACAACTTTGAACAATGAGCACCTTATACACGCTACTGTAT
TTTCTTTTCTTTCTTTTTTTTTTTTTTTTTTTTTTTTAAACCGGGTAGCAGTGAGAGAGGTTTCTTTAAGTGCCTTGGGGCGAGGA
GTCCGGAATAAGAAGACTTCTTTGGGTTTTAAAGTGTAGGATAAGCAAATCCCGAGGGAATATGCATTATATAATAAATCTAGAAC
CAATGCACAGAGCAAAAGACTCATGTTTCTGGTTGGTTAATAAGCTAGATTATCGTGTATATATAAAGTGTGTATGTATACGTTTG
GGGATTGTACAGAATGCACAGCGTAGTATTCAGGAAAAAGGAAACTGGGAAATTAATGTATAAATTAAAATCAGCTTTTAATTAGC
TTAACACACACATACGAAGGCAAAAATGTAACGTTACTTTGATCTGATCAGGGCCGACTTTTTTTTTTAAGTGCATAATTACGATT
CCAGTAATAAAAGGGGAAAGCTTGGGTTTGTCCTGGGAGGAAGGGGTTAACGGTTTTCTTTATTCTAGGGTCTCTGCAGGCTCCCC
AGATCTGGGTTGGCAATTCACTCCTCCCCCTTTCTGGGAAGTCCGGGTTTTCCCCAACCCCCCAATTCATGGCATATTCTCGCGTC
TAGCGCCTTGATTTTCCCCACCCCAGCTCCTAAACCAGAGTCTGCTGCAAACTGGCTCCACAGGGGCAAAGAGGATTTGCCTCTTG
TGAAAACCGACTGTGGCCCTGGAACTGTGTGGAGGTGTATGGGGGTGTAGACCGGCAGATACTCCTCCCGGAGGAGCCGGGTAGAG
CGCACCCGCCGCCACTTTACTGGACTGCGCAGGGAGACCTACAGGGGAAAGAGCCGCCTCCACACCACCCGCCGGGTGGAAGTCCG
AACCGGAGGTGCTGGAGTGTNNNNNNNNNNNNNNNNNNNNNNTGACGCGGTCCAGGGTACATGGCGTATTGTGTGGAGCGAGGCAGCT
GTTCCACCTGCGGTGACTGATATACGCAGGGCAAGAACACAGTTCAGCCGAGCGCTGCGCCCGAACAACCGTACAGAAAGGGAAAG
GACTAGCGCGCGAGCAAGAGAAAATGGTCGGGCGCGCAGTTAATTCATGCTGCGCTATTACTGTTTACACCCCGGAGCCGGAGTAC
TGGCTGCGGGGCTGAGGCTCCTCCTCCTCTTTCCCCGGCTCCCCACTAGCCCCCCTCCCGAGTTCCCAAAGCAGAGGGCGGGGAA
GCGAGAGGAGGAAAAAAAAATAGAGAGAGGTGGGGAAGGGAGAAAGAGAGATTCTCTGGCTAATCCCCGCCCACCCGCCCTTTATA
TTCCGGGGGTCTGCGCGGCCGAGGACCCCTGGGCTGCGCTGCTCTCAGCTGCCGGGTCCGACTCGCCTCACTCAGCTCCCCTCCTG
CCTCCTGAAGGGCAGGGCTTCGCCGACGCTTGGCGGAAAAAGAAGGGAGGGGAGGGATCCTGAGTCGCAGTATAAAAGAAGCTTT
TCGGGCGTTTTTTTCTGACTCGCTGTAGTAATTCCAGCGAGAGACAGAGGGAGTGAGCGGACGGTTGGAAGAGCCGTGTGTGCAGA
GCCGCGCTCCGGGGCGACCTAAGAAGGCAGCTCTGGAGTGAGAGGGGCTTTGCCTCCGAGCCTGCCGCCCACTCTCCCCAACCCTG
CGACTGACCCAACATCAGCGGCCGCAACCCTCGCCGCCGCTGGGAAACTTTGCCCATTGCAGCGGGCAGACACTTCTCACTGGAAC
TTACAATCTGCGAGCCAGGACAGGACTCCCCAGGCTCCGGGGAGGGAATTTTTGTCTATTTGGGGACAGTGTTCTCTGCCTCTGCC
CGCGATCAGCTCTCCTGAAAAGAGCTCCTCGAGCTGTTTGAAGGCTGGATTTCCTTTGGCGTTGGAAACCCCGGTAAGCACAGAT
CTGGTGGTCTTTCCCTGTGTTCTTTCTGCGTCTTGAATGTAGCGGCCGGTTAGGACAGTCTTTCTTCCATTCCTGTGCTTTTGACA
CTTTTCTCAAGAGTAGTTGGGGTAGGCTGGGGTAGATCTGAGTCGGGGTAGAGCGACTTGTCAAGATGACAGAGGAAAGGGGAAGG
GAAAAACCGGGATGCATTTTGAAGCGGGGTTCCCGAGGTTACTATGGGCTGACGCTGACCCGGCCGGTTGGACATTCTTGCTTTGC
TACATTAATTGATATGTGTCCTTTGAGGGGTCAAACCGGGAGGTCGCTTCGTGGTGGCCAAAGAAAGCCCTTGGAATCCTGAGGTC
TTTGGAGAAGGGATTACCTTTTGCGTTTGGGAGCGAGAAGGCTCCGTAGCTTCTGACTTACCAGTCTCTGAGAGGGCATTTAAATT
TCAGCTTGGTGCATTTCTGACAGCCTGGGACCGACACGGAGGTGCGTCCCGCCCGCCAATCCCCGGCGGCGATCGCAACCCGTCCC
TGAGCCTTTTAAGAAGTTGCTATTTTGGCTTTAAAAATAGTGATCGTAGTAAAATTTAAGCCTGACCCCCGCGGCACTAGGACTTG

ATGTTGGGCTAGCGCAGTGAGGAGAAGCAAAAITGGACAGGATGTGACCGATICGTTGACTTGGGGAAACCAGAGGGAATCCT
CACATTCCTACTTGGGATCCGCGGGTATCCCTCGCGCCCCTGAATTGCTAGGAAGACIGCGGTGAGTCGTGATCTGAGCGGTTCCG
TAACAGCTGCTACCCTCGGCGGGGAGAGGGAAGACGCCCTGCACCCAGTGCTGAATCGCTGCAGGGTCTCTGGTGCAGTGGCGTCG
CGGTTTAGAGTGTAGAAGGGAGGTGTCTCTTATTATTTGACACCCCCTCCCCTTTTATTTCGAGAGGCITGTGATAGCCGGAGACT
GAGCTCTCTCCTCCAAGTCAGCAATCGGAAAGAAAAGCCGGCAAAGGAAGGAAGGGGGCGCGCTGGGGGTGGAGAAAGAGGAGGGC
GGAGAGGGGCGGCGGCGCCGGCTGGGTAGGAGCGCGGCGACGGCGCGAATAGGGACTCGGACCCGGTCGGCGGCGCAGAGAGCCGG
CACACGGGAGGGGGCCGAGCGACGCGGCGCCTCTCGCCTTTCTCCTTCAGGTGGCGCAAAACTTTGCGCCTCGGCTCTTAGCAGAC
TGTATTCCCTACAGTCGCCTCCCTCAGCCTCTGAAGCCAAGGCCGATGGCGATTCCTGGGCGICTGCAGGGCTAAGTCCCTGCTCG
AAGGAGGCGGGGACTCGGAGCAGCTGCTAGTCCGACGAGCGTCACTGATAGTAGGGAGTAAAAGAGTGCATGCCTCCCCCCCAACC
ACACACACACACACACACACACACACACACACACACACACACACACTTGGAAGTACAGCACGCTGAAAGGGGAGTGGTTCAGGATT
GGGGTACGCGCTGCGCCAGGTTTCCGCACCAACCAGAGCTGGATAACTCTAGACTTGCTTCCCTTGCTGTGCCCCCTCCAGCAGAC
AGCCACGACGATGCCCCTCAACGTGAACTTCACCAACAGGAACTATGACCTCGACTACGACTCCGTACAGCCCTATTTCATCTGCG
ACGAGGAAGAGAATTTCTATCACCAGCAACAGCAGAGCGAGCTGCAGCCGCCCGCGCCCAGTGAGGATATCTGGAAGAAATTCGAG
CTGCTTCCCACCCCGCCCCTGTCCCCGAGCCGCCGCTCCGGGCTCTGCTCTCCATCCTATGITGCGGTCGCTACGTCCTTCTCCCC
AAGGGAAGACGATGACGGCGGCGGTGGCAACTTCTCCACCGCCGATCAGCTGGAGATGATGACCGAGTTACITGGAGGAGACATGG
TGAACCAGAGCTTCATCTGCGATCCTGACGACGAGACCTTCATCAAGAACATCATCATCCAGGACTGIATGTGGAGCGGTTTCTCA
GCCGCTGCCAAGCTGGTCTCGGAGAAGCTGGCCTCCTACCAGGCTGCGCGCAAAGACAGCACCAGCCTGAGCCCCGCCCCGCGGGCA
CAGCGTCTGCTCCACCTCCAGCCTGTACCTGCAGGACCTCACCGCCGCCGCGTCCGAGTGCATTGACCCCTCAGTGGTCTTTCCCT
ACCCGCTCAACGACAGCAGCTCGCCCAAATCCTGTACCTCGTCCGATTCCACGGCCTTCTCTCCTTCCTCGGACTCGCTGCTGTCC
TCCGAGTCCTCCCCACGGGCCAGCCCTGAGCCCCTAGTGCTGCATGAGGAGACACCGCCCACCACCAGCAGCGACTCTGGTAAGCT
ACCCCATTCACAGCAGGGTAGGAAGCGAGAGGTTGGATGGACCTCCTTCTCCACCACTCATTGGCATTAATTCAATTGGCCTCCGG
GGCTCCCCCTTTCTTTCCCTTCTGTCTAAGAGCTCTTCATCCCTGGATTCCCGTGNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNTCAGGAATTTCATTTGGGTTTTTAAATCTTCTGGC
TTATCTTTCAGCTCCATCCACTCCCTTTACCCCTCCTAAGCATTTTAATTACCCTGGGAAGGGTGTGAATGAGGATAAAGGAACTG
ATCIGGAGGGGGGTGAATTACCTGCTTCTTTTCITGACTGCCAGAAGAATATTTGAATTTAATGGATACGTTTGTCTAAGACCCAA
GAGAAGCAATGACAGAAGCTGGGACAGCCTTTATAGCCTTAGAGCCAGGCACTAGTGAAAGTTCCTAAAGAATTGAAGAGCTGGGC
TCTTTTGGGTGTTTTGTTTTGCTTTGTTTTTCGTCCTCTTTTGAACACTTCAAAGCAAATICTGTTCAATTTGGACTTCTCCCCC
CGTCCCAACACTCCCCCAACACCAGGACGTTTGGCAAAGCTGCAAGACTTTTTTTTTTTTTTTTTTTTTAATTGTGCTTCCAGT
AAAATAGGGAGTTGCTAAAGTCATAGCAAGAGATTTGCAGCTATCCCTCACGGGACCTGAAAGGTTCTCGGTAAAGTCCCTTAAAA
ATAGGAGGTGCTTGGGAAATGTGCTTTGCTTTGGGTGTGTCTGAAGCCTCATTAAATCTTAGGTAAGAATTGGCAAGGATACCATA
TCCTGGTACATGGTAATTTTCTCACCTGTGCCCTAACCCIGTTCTGCCTTTCTGGGAGAAGGGAAGATGGTGTCTGGATCTGATTC
TTACTTTCTTCCCTTTCCAACTTGGTATTTGGATAGCATCGGTCAAATCCTATGTATAGCGTCCGGGATTCAGGAGGCGTGGCTAA
CTGTGATCTTCCACTTCCTCCCTTACAGAAGAAGAGCAAGAAGATGAGGAAGAAATTGATGTGGTGTCTGTGGAGAAGAGGCAAAC
CCCTGCCAAGAGGTCGGAGTCGGGCTCATCTCCATCCCGAGGCCACAGCAAACCTCCGCACAGCCCACTGGTCCTCAAGAGGTGCC
ACGTCTCCACTCACCAGCACAACTACGCCGCACCCCCCTCCACAAGGAAGGACTATCCAGCTGCCAAGAGGGCCAAGTTGGACAGT
GGCAGGGTCCTGAAGCAGATCAGCAACAACCGCAAGTGCTCCAGCCCCAGGTCCTCAGACACGGAGGAAAACGACAAGAGGCGGAC
ACACAACGTCTTGGAACGTCAGAGGAGGAACGAGCTGAAGCGCAGCTTTTTTGCCCTGCGTGACCAGATCCCTGAATTGGAAAACA
ACGAAAAGGCCCCCAAGGTAGTGATCCTCAAAAAAGCCACCGCCTACATCCTGTCCATTCAAGCAGACGAGCACAAGCTCACCTCT
GAAAAGGACTTATTGAGGAAACGACGAGAACAGTTGAAACACAAACTCGAACAGCTTCGAAACTCTGGTGCATAAACTGACCTAAC
TCGAGGAGGAGCTGGAATCTCTCGTGAGAGTAAGGAGAACGGTTCCTTCTGACAGAACTGATGCGCTGGAATTAAAATGCATGCTC
AAAGCCTAACCTCACAACCTTGGCTGGGGCTTTGGACTGTAAGCTTCAGCCATAATTITAACTGCCTCAAACTTAAATAGIATAA
AAGAACTTTTTTTTATGCTTCCCATCTTTTTTCTTTTTCCTTTTAACAGATTTGTATTTAATTGTTTTTTTAAAAAAATCTTAAAA
TCTATCCAATTTTCCCATGTAAATAGGGCCTTGAAATGTAAATAACTTTAATAAAACGTTTATAACAGTTACAAAAGATTTTAAGA
CATGTACCATAATTTTTTTTATTTAAAGACATTTTCATTTTTAAAGTIGATTTTTTTCTATTGTTTTTAGAAAAAAATAAAATAAT
TGGAAAAAATACAATTGGGCCAACTTGTGTTTTCTTTTTCCTCTTCCTCAAACTTCCTTTCCTCAATTACAGATTAAAAGAATTTG
ACCATTTTCACAGGGTAGGTTTACAAATATGGAAGGGGTTATCATTGTTAAAAIGGGGCTGGGGGTCCTCAGGATTTCTAAGITG
TCTACAGGATGCTTTCTGTGGATAGTAATAAAAACCAGAGCTGTTAGTIAGGAATGGGCAAAAGGCAAGTGAGAAGGCTAGATGCA
GGGAAGGGAAAAGCAAGAGGTTAAAGATAACAGCTAAATATACAGGAGGAAGAGATGGCAGAATCTCCTACAGTTAACCGAAGCCA
TTCCCTGGTTCACCTCAACCCAAGGACTCTGCCCTGCCAAAGAACTGGTGAGGGGAGGGAGAGAGAACCACCGTTTGTTCCTTGCC
TCTTGCTCCCAGGTGATAGTCCCTTCACATCAGTATCTCCTATGCITCTGAAAAAAACAGAGGAAGAGCATTACCACTGCTAAGTT
GATCCTGGTTTTCCAAACAAGGACATACAAAGGITCAGAGGGTTGCAGAGCTTAATGGGTACGAACACAACAAAAATAACCCAGGG
CCCCTGTCTTGAAAACAAGCCTTGCTGCCTTGCTTAGTTGGGTGTCCTTCCGCTGGTTAGGGGTCTGAGAATGAGCGCTACAGGCT
CCATTAGGAGCTTTGACAGAGTGCCACGAAAGAGGTACTATGATCTCITTATACCTAGTACAGGTAGAAACAGACATGAAATAACC
CATCCATGCACCCAGGGGATTCAAAACCACTTTATCCTTAGTGCATCCAGGAGGAAGATCCTAGCTACGAAGGAGAGGCAGGAGT
CATTAAAGCTTCACTACTCAAGAACTGGTAGACTTCAGTTCCCCTCCTTGTTTGTGGATTGGGGGGTTGGGGTGGAGGGTGTTGTG
TGTACTCAGAGGCACATAGCTCACTCCTACCTTACCCACTTAGTTTTTAACATCAGATCCCTGCTTGCTCCTGGGAAGAAGCCAGT
TTAGAAGTGCTACTGGTCAGATCATAAAATAAAACCTTGTITTACATGAGTCATTATTTTAGAAATTGCAAGCTCGCCTTCCTCCC
AAGCACTTTCACACACTCATGACACACGCTCATCAATGGCCCATGAGAAAGCCTTTTGGAACAGGTATCTTATTAATTATAAGCCT
CTGAAAAGCCACAATCCAAACCAGAAACTGAAACATGTAGGTAAAGTCCAGGGAGAAAGGTACCCTAAATGTCCTAAATGACCCCA
TTGTCTCAGAGAAGGTCTCTAGCATCCTGGAGTGTCTTTGGGACTTTAATTCACCATCATTCATAAACCAATAAGTAATTACTATC
TTTGACACCCCCCCCCCATGATCTATTGAAGCGTTTAACATTTAGTTTTTGATCTTATTTTTGGCTCTTTTAGACTCACCCTCAGC
TTCAAACTGTTACACCCTCTATCCTACATTCAAAAGAAAACACAAACCCCAGTAGTAGTTTGAAATACTITGAGTAGTTCAAAGGA
TTAGCAAAAGGGGGGAGCAGGGGGTGCTCTTAAATAGTTCCCTCTTTTTCCCCTTTGTGAGCCTAGGCTGGAGTGCAGCCCTGGGG
TGACTCACTTGAGACCTGGGAAGGTGTTAGGTTGAATCACTCAGTCCAGGCAAGCCCAAAGAATAGAGGAAAGCATTCCTCATTAG
GAAAACAACTCCTGTTGCAAATGATCAGGAAACAAGTTTAGAGATTCAGATTTGGCTGTGGGGATGGAATCGAAGTATCACAGCTC
CCTATCTGGGCACTTCTCAGCTTTACCAAGCCAGGGAATGGTCTGAAAACAGGACATCGGCCAGCTTCCTTCCAGAAAGTCAGGCT
GATCTIGACCATAACACAGGAAGGCTCTCCCAGCCAATCTGGAGTCCCAGGGGTCTGCAACATTGTGCAATCAAATTTATAGATAA
CAATCACGATGAGGGGTGAAGTGGAGGAGCTTTCAGCTTGGTCTGGGATATACAAGAATTAGCTAGTCTCTTCTGTTGTTCACCC
ATGACACTGGAACTCAGTTTCCCCGAGAGGATCCCTGGGATGGTGCTCTTCAGGATAAACTGAGTGAGGAGGAAGTGTGACTTTAT
GCTATCATICGGGGACAACACTAAAAAGCAATCAATTACACTTTAAGTTGAACAAAAGTTTCACAATACCAGGATAGTGCTTGAGT
CATCCAAAGCTTGAACACGTATGTAGAGTCTTCTGCTGTTCTTTAAGATGGACTTTGGCACTAACCTTGGAAAAGAGGCCAAAAA
ACCCACCCCCCAAAAACAAACAGAACAAAAAACGTGAGGGGATTTTCTGTGGTTTGTTTACAATAAGAGATGAGTCACAATAATGA
TTTTTTTTTTTTTTTTTAGCATGACTGCTAAGGAACCCTGAAGCATTTCTTCCCAACAAAAACTAATCTTTAGGTTATAACATGCT
GGAAGATGAGCITCAAGCCTGTCTATAAATACATCACTGGGTATCTTCAGGGAACAAACTCTTCACTTAAGGTTAGAGACCATCCT

GGATCGATTTCAAGAACAGAGGTTTATTTTAAACAATGAAGCCTGGGCTAGAGATGTCGTTCAGTGATAAGACACTTGTCTAGCAT
GTATAAGGACCCATGCTTGACACCACAGGAATAAATAAAACCAAAAATGAGGTTCCTACTCATTCCCCAAACTTTAGCAATTTTGG
TGGTGGTGGTGGTGGTGGTTCGACGTCTTTCCAGAGAAATCCAACAAACTGCCAATTTAGGGAAAACATCTAGCTCTAGCGACTTT
GCCAAACTGGTGTGAGTTGACTCAATGACTGAGTGGATTTCCTTTTTGGGTTTTACTTCACTCAATATTGGTCTACACTAAGCTCT
TTAGGAAGACAGGGTTGAGAGAGGAACAAGTGTTAATGGGATGTAGATCCTCTGAGGCTGAAAGGAATGTCCTTTGTCTCTAAACA
ATGCCGAGGGGCTCAGGAACACTCTCTTCCAGTCTTCACCCGACCATCTCATTAAAGGCTAAACTCTCCAGTTCGGGTCTATATGG
CTAGGAAAGAGGAGCTTTGGGGAAAGCTCTGTCAGAATATACCTGCTTTCTGCAGGGGGCGGGAGGACCCCCACAAACAACTCAGC
TGGAGAACAATTGTTAGATGTGACAATACTGAAGTTTGTACACAGACATGTGAAATTGCTGGCCTGAAGTCACATAAGCACTAACT
AACGATAATAGCAATTAATGTTTACTGTGTGCTAAAAGTTTGACATACATTATCTCCTTTTATTCCCAAAGCAACCCCCTATACAC
ACACACACCTCCTTTTTGGTTTTGGTTTTACTTTTTAAATTTTACTATTAAATTTTAAATATGTTTTTTTTTAAATTTACTGCAC
TAGAGAGGGAACCTAGAACCTCCTGCATGCAAGGTAAGTGATCAATGGGTGAGTCACATCTCTATAGAGATTGTAGGTTGTTTCAC
CAAAAGTATTAGGGCATTTCCTACAGAGTGGTGGAAGTTCGAGTCCTGGGAACATGGAATAAGGATGGAATCCAGTAAGTCTTGAG
AAAGAAGCTAAAGGAGAAAATGTCAGCAGCAGATGGCAGGGAAGCAAAGAAGAAGGAGAGCTAAAACCAACAAGCTAAAACCAAAA
ACGCTTGCTATTTGAACTGTGCCTAAGGTAGCAGGAAGTACAGCAAGGATCCACTGGCTTAGTGAGGGAATGCTTCTCTAGGCCTC
TCCTATCCACTTCCCCTTCCACCCAGTATCCTCCTACTCCTTCATCTGCTAGAGACAACTGCCCATAGGTAGTTAATTAGCTGTCC
CAGCACCCAACTCTCCTGCAGTACTTTGGTTCTGTTTATTTGTTTTGGCAATAGGATCTCATTAGGCAGGCCATACTGGCCTCAAA
CTCTGGAATTTCTTGCCTCAGCCTCTTAAGTGTTGTGTTGGGATTACCAGTTTGGGACACTTATTCTCCAGCTTTCATGCAGGGCT
TTATTCAGGAATTTCCACTGTGTGATTCTAGGATGCAAATACTAGTTCTCATTCCCTGAGGGTTTAATTGTTGTTGTTGTTTCGTT
AGTTGGTTGGTTTTGGGTTTTGTTCATTGGGTTGGTTTGGGTTGGTTTTGGTTTTTCAAAACAGGGTTTCTCTGTGTCTGAGAACC
ATTGTCTTTTCTACCACTGCAAACCCAACCATCTTTTCAAACCACTGGTAAGCCAAGTCCTTTCTGGATTAAGTCTGAAGCCATTC
TCCACTCCTATTCGATACACTTACTACCTACCACAAACACGGCAGCCTGGCAGCCTCAATGCCAGTTTTGGGCTTCTCCTTGTATG
CTTGGAGACCAGTTGTCTTCATCATCAGATCAACCCCTTGAAGAATGGAAGTTCATTGGTCTTGCTTAATCATGCCTGACAAGAAA
GTGCAATTCACTAATTCTGTAAACATCTAAGTGAAGGGCTCTTTGATGGGCTCAATGGTGCATCAGACCTGAGTAAGGGGTGGAGT
AGAAGCAAAGTTGTTTCAAGTTGCATAGCGAGAAAGGTCAAGGAAAGAGATGGGGGTTCATCAGGTGAAGGGAAGGATTTTGATTA
GACTTGCTTTGCTTGATTTGGTTTTTGAGCTGAAAAGACAGGAAGCCTAATTCATTTCCATGATAGTAGGCTAGACTTGAGAGGGA
AAGAACTGCTGACAGAAAAGGAGGGGAATTGTACTGACTGGTTTTGTGTGTGTGTGTGTGTGTGTGTGTGTGTGTGTGTGTGTGTG
TGTGTGTGTGTGTGTGAACTTGACACAAGCTGGAGTTATCACAGAGCCTCACTTGGGGAAAAGCCTCCGTGAGATCCAGCTGTAAG
GCATTTTCTCAATTAGTGGTGAAGGAGGGCCTATGTGGGTGGTGCCATCCCTGGGCTGGTAGTCTTGAGTTCTCTAAGAGAGCAAG
CTGAGCAAGCCAGGGGAAGCAAGCCAGTAAGTAACATCCTTCCAAGACCTCTGCATCAGCTCCTGCTTCCTGACCTGCTTGAGTTC
CAGTCCTGACTTCCTTTGGTAATGAACAGCAGTGTGTAAGTGGACAGTGAATAATCCTTTTCTTTCCCAACTTGCTTTTTGGACAT
GATGTTTTGTGCAGGAATATAAACCCTGACTAAGACAAGAATCAAAGTGTCTAGTCAGGTGAGTTACTGGACCGGTAGGTAGCTTT
GTTTTGCTTGCTTGATGTCTGGAGCCCTCTCTTAGCTGGGTATGGCTGAACAAATGAAAAAAGAATATGTTCCCCCTACTTAGGTA
AGTATTCTTCCAGGTGGTGATGTCACTGGCCAAGCTGTTCTCCCTTGGCTTCCCACAGCTCTCTTCCTTTACCAGATACCATGTCC
ATCTTTACACAAATGACTCTAAACCTCTTCTCTCCACAGACACCTTCAATCCACATGAGATAAAGCCTCCACATTTTGTAGCTCTG
GCTGACCTGGAACTCACTATGCAGACCAGCCTTGCATTGAACTTTCAGAGATCTGCCTATATCTTTCTTAGGAGTGCTAGGACTAA
AGGCATGCACCACATTTAGTCCTGCTAAACTTCTTTTTTTGTTTTTTTGGTTTTTTCGAGACAGGGTTTCTCTGTGTAGCCCTGGC
TGTCCTGGAACTCACTCTGTAGACCAGGCTGGCCTCAAACTTAGAAATCCACCTGTCTCTGCCTCCCAAGTGCTGAGATTAAAGAT
GTGTGGTACTACTGCTTAGACTGACACACAAAATATTGTTGAAGCCAGTGAGATAAAGCTCACCTTCAATCTCAGAGGTAGGCCAG
GCAGACTTTTGTGAGTTCTGGGCCAGCCAGAGCTTCATATGAAAAGTAATAATAATGATAATAATAATAATAATTTTAATAAACAA
ATGGGTGTATGAAAGAGTAAATGTGCACATATTTCTGGCATCTGTTTTAAGATTCTACTTCCTTAGGCTGTCAGTCATTCTCACCC
CATTCAACCCTTTGCTCTGCACTGTGATTGACAAATCTTCTCTCCCTCTGGTGACTACCTAATTCATCCCAGACAGCATCAGCCTT
GGCAACAACTTCTCTATGCAGGGACTGTAAGTTCCAGACAACAGAAACTCTGTTCCAATTCACAAAATCAACTAGGAAAGGAGAAG
AGACAGAGGACAGAGGAAAGGAAGGAAGAAGGGAGAAAAGGAGGGAGGGAGGGAGGGAGGGAAAATGTCAATTTATGCAACT
GTTTCATGATTCATCCAAGGATCCTCTCTGATTCTTTTTTGGTTGTTGTTGTTTTTGTTTTTTCGAGACAGGGTTTCTCTGTATAG
CCCTGACTGTCCTGGAACTCACTTTGTAGACCAGGCTGGCCTCGAACTCAGAAACCCACCTGCCTCTGCCTCCCAAGTGCTGGGAT
TAAAGATGTGTGCCACCACGCCCAGCTAAACCTCTTCCTTCTAGCCAGAAGAACACAGCAGAAAGAACTATGATCAGTCTTCTGGA
ACCATGTGCTTACCCTTAAACCAGACAATTCACTTGGTTCCCAGGACCACACTCCTCCTGCAAAAGTTGGAACCCTGTGATTCTCA
GCTTCTGAGGCAAAGGGAAGACCTATCCAAGATAAAAAACCAACAACAACAAAAAAGTCAATATAAGAAAAGAAAAAAATGTTCTC
TAGATACATTCTGGGTAGTGATGGTTCTCACTGTTCAGAGATATAAGAAAAGAGCAGATGCTTAAAACCCTGACTCTTGAGAACAA
CTTTTGGTAAGAGAGACTCCTTTTTTTAAAATTTTATTTATTTATTTATTTACTTACTTATTATATGGTAAGTACACTGGTAGCTG
TCTTCAGACACTCCAGAAGAGAGTATCAGATCTCATTACAGATGGTTGTGAGCCACCATGTGGTTGCTGGGATTTGAACTCAGGAT
CTTTGGAAGAACAGTCAGTGCTCTTAACCACTGAGCTATCTTACCCAGCCCCCCACCCACCCCCCCAACCCCGTTAAGAGAGATTC
GAATGGTCCATCTTAGAGGTATTGAAATTGAGTCTGAAGTGGAGGCCTAGGGGCTGTTTTGAGCCAACTCAGCCCAGGTGATTTG
CAGTGAGGTGGGAATTCCAAGGGCAAACAAGGATCAGCAAAAGCTATACCCCTCTACCACAGATTTGGAGAGAATTCAGATGGGAG
GCCTGTTCCAGCCATTGCTCAGTAAGTAAGCAAGAGTCTAGCTACCTCTTCATCTCCCTCCTTCTACCACACCATTAAGTCCCTCC
AGGCCATCAGCCTCAATGATTCTTTCTTTTTTTTTTTTTTAATTAGGTATTTTCCTCATTTACATTTCCAATGCTATCCCAAAAGTC
CCCCATACCCTCCCCCTCCACTCCAACTTTTTGGCCCTGTTGTTCCCCGCCTCAATGCTTCTTGGCTCTATCCATGTCTCCTTGCC
CACTTTGCTTCTGCCAACCGATATTCTTTACCTGGGACTCTCTAGCAGATGCCAACTGCCTGTCTCTCATCTGGTTTCCTCAAAGG
TAGAAAAATGGTCTCCCAAATAAGCCCGATCAGTTTACTCCCTGGCGTAGGAGAACTTGATGATTCATGTGCTCTGCTTCTAAAAT
ACAGATCTGTGGGCCTGGTAGTGCAGATGTTAATCCCAACTACTCTACTTGGGAGGCCTAGGGAATGGACTCCATGTTCAAGGTCT
GCCTGGACAGCAAGATGAGTGCTGGGGAAGCCAGAGAAAATGAGTAAAACTTTCTGAAAAGTAAAGCATTATAAAAACAAACAAAC
AAAGGTCCAGAGAACTGGGGAGATGATTGGCTGGTTAAGAGTACTTGCTCTTCTGCATGGGACCCAAGTTCAGTTCCTAGAAGCCG
AGTAGCTTGCACCTTCAATTATGGCCCCCTCTTCTGTTTTCTCAGGACCAGGTGCACACAGTGCATATAACATACATGTAAGCAAA
ATACTCACACACATAAAATTAAAAATAAATAAATCTTTCAGGCAAAACTGACCTGGGATATAGCTCAAGGATAGAGGATTTGCATG
GGATGCTAGCAGCCCTGACTTCCATCTACAGTCCAGAAAGACAAGTAAATCAGAAATGCCTTTGAGGGACTGAAGAGATGATTCAG
AGGTTAAGAGCACTGGCTGCTCTTCCAGAAGACCTGGGTTCAATTCCTAGTACCTGCATGAGAGCTGACTCCAGTTCCAGGGAATC
TGACCCTATCACTCAGACACTGTTCTTGTCATCTGCACACGGGATGATCACACACATATCATTTCACATGTGGGGATACTGAGAAT
GAGAGTCAGCAGGTAACTGTCTCAACTTCAACTAAGCAGGGCAGTTTGTTAGCAGGGATGGAATCTGGATTCTTATTGCCCCCCAG
CTTCTTCACTTCCTGCTGCCTCTCTACAACCACACCATATTGAAATTACCTTCTTCCCTCTCTTGCTTCCGCACTGAAACTGGGGC
TGAATCTAGCTGGCTACCTGACCTGATTCTCTCCTATCAGATGGATGACTTGCTGTCCTCCTGGGTCATCTCCCGTGTTTAGCAAG
ACAGGGTATAGAGTTTTAAATGAGTGTAGGCAGAGTGCATGAATCATCACACTTTTAGGCAGAGTGCATGAATCATCAGACTTTCC
TACGCCAGGGAGTAAACTGAGCACGCATATTTTGGAGACCACTTCTCTACTCCTAGGGTCAGAAGAGCCTGCAGATGATCTACTCA
AAGCAATTTTATCTGCCATTCTGTTGAGTAGTGAGGCAAGGATCATGTTACCTGCAACGGGGCCAATCTTTGCCTCTGTTTTTGAA
GTCTCTGGAGAAGTAGCCGTCTTCCTGCGCTGGGGATATGATTAAGCAGTAGAACATGCACATAATTTCATGAGGCTAACATACAG
CTCTTCAGAGATCAGCCTACTTATTTATGACACAATTGTTTTAAGACATCAGGTATTCTAGGAGATAAGATTCCTTCTCTTGCTTT
CCTGGTACCCAGAATGATCTCCTAAACCACAAGTAATTGGCACAGAGATGGATCTTCCAAACTGACCCAGATCTACACAGATCTTA

TCTTCATACCCAAGAACTCCCCAGTCAATGGATATTTGGGGCATTTGCTATGCACACAGACGTCAGTGTGGAATAACCGTACTCCA
GCACTTAGTAGAAAGGATGTATACTGAACGAGTATTTACAAATAATTAACCAGATTATTTTCAGGGCACAAATTAGTGGAGCAAGG
ATTGCAGAGTTACATGAACATCTGATAGCATTGCGGTTCAGGGAAAGTTCCCAGCCAGGGTACAGAGGATGGTGATGAAGAAAGCA
TGCTCAATATCTTGAGGCGCTGCTATGAGAGAGAATGAAACTCTCTAGGGGAACTGAAAGCATACAAGGATGTAAAGAATAGGAAG
AGCAAAAGGACTTCAACCCTTGGATGAGACACAGGGTGATCCAATACCCGGTGACCTGCAGGGACTCAGGCCCAGTGGTTATGATG
GCAACCTACCCACAGTCGTGAGTATGATGCAGTGGGAAGTCACAGTACTGAAAACCAACACACTTAACTTGCCTGTGCTGCTGTGA
ACGTCCATTCAATGAGGGTCAGGAAGCTCAAATAAGATAATCGCAAATACTGAGTGGTTCTGAGAGGAATGCAACCATGAAAAAAA
AAGTGCTGACACTTGCAGAATGTTTATGTTTCTAATGCTGGGCTAAGTACTTCACCTGGATTATTTCACCAATTCCTCCTCCTCCT
CTCCCTGTTCTCCCTTCTCCTCCTCTTCTTCCTGTTCTTCCTCCTCCTCTTCCTCTTCTTCCAAAGATTCATGTGTATCAAGGTAT
TCTCAA

MOUSE SEQUENCE - Mrna (SEQ ID NO: 20)
GATTGGGGTACGCGCTGCGCCAGGTTTCCGCACCAACCAGAGCTGGATAACTCTAGACTTGCTTCCCTTGCTGTGCCCCCTCCAGC
AGACAGCCACGACGATGCCCCTCAACGTGAACTTCACCAACAGGAACTATGACCTCGACTACGACTCCGTACAGCCCTATTTCATC
TGCGACGAGGAAGAGAATTTCTATCACCAGCAACAGCAGAGCGAGCTGCAGCCGCCCGCGCCCAGTGAGGATATCTGGAAGAAATT
CGAGCTGCTTCCCACCCCGCCCCTGTCCCCGAGCCGCCGCTCCGGGCTCTGCTCTCCATCCTATGTTGCGGTCGCTACGTCCTTCT
CCCCAAGGGAAGACGATGACGGCGGCGGTGGCAACTTCTCCACCGCCGATCAGCTGGAGATGATGACCGAGTTACTTGGAGGAGAC
ATGGTGAACCAGAGCTTCATCTGCGATCCTGACGACGAGACCTTCATCAAGAACATCATCATCCAGGACTGTATGTGGAGCGGTTT
CTCAGCCGCTGCCAAGCTGGTCTCGGAGAAGCTGGCCTCCTACCAGGCTGCGCGCAAAGACAGCACCAGCCTGAGCCCCGCCCGCG
GGCACAGCGTCTGCTCCACCTCCAGCCTGTACCTGCAGGACCTCACCGCCGCCGCGTCCGAGTGCATTGACCCCTCAGTGGTCTTT
CCCTACCCGCTCAACGACAGCAGCTCGCCCAAATCCTGTACCTCGTCCGATTCCACGGCCTTCTCTCCTTCCTCGGACTCGCTGCT
GTCCTCCGAGTCCTCCCCACGGGCCAGCCCTGAGCCCCTAGTGCTGCATGAGGAGACACCGCCCACCACCAGCAGCGACTCTGAAG
AAGAGCAAGAAGATGAGGAAGAAATTGATGTGGTGTCTGTGGAGAAGAGGCAAACCCCTGCCAAGAGGTCGGAGTCGGGCTCATCT
CCATCCCGAGGCCACAGCAAACCTCCGCACAGCCCACTGGTCCTCAAGAGGTGCCACGTCTCCACTCACCAGCACAACTACGCCGC
ACCCCCCTCCACAAGGAAGGACTATCCAGCTGCCAAGAGGGCCAAGTTGGACAGTGGCAGGGTCCTGAAGCAGATCAGCAACAACC
GCAAGTGCTCCAGCCCCAGGTCCTCAGACACGGAGGAAAACGACAAGAGGCGGACACACAACGTCTTGGAACGTCAGAGGAGGAAC
GAGCTGAAGCGCAGCTTTTTTGCCCTGCGTGACCAGATCCCTGAATTGGAAAACAACGAAAAGGCCCCCAAGGTAGTGATCCTCAA
AAAAGCCACCGCCTACATCCTGTCCATTCAAGCAGACGAGCACAAGCTCACCTCTGAAAAGGACTTATTGAGGAAACGACGAGAAC
AGTTGAAACACAAACTCGAACAGCTTCGAAACTCTGGTGCATAAACTGACCTAACTCGAGGAGGAGCTGGAATCTCTCGTGAGAGT
AAGGAGAACGGTTCCTTCTGACAGAACTGATGCGCTGGAATTAAAATGCATGCTCAAAGCCTAACCTCACAACCTTGGCTGGGGCT
TTGGGACTGTAAGCTTCAGCCATAATTTTAACTGCCTCAAACTTAAATAGTATAAAAGAACTTTTTTTTATGCTTCCCATCTTTTT
TCTTTTTCCTTTTAACAGATTTGTATTTAATTGTTTTTTTAAAAAAATCTTAAAATCTATCCAATTTTCCCATGTAAATAGGGCCT
TGAAATGTAAATAACTTTAATAAAACGTTTATAACAGTTAAAAAAAAAAAAAAAAAAAAAAAAAAAAAAAAAAAAAAA

MOUSE SEQUENCE - CODING (SEQ ID NO: 21)
ATGCCCCTCAACGTGAACTTCACCAACAGGAACTATGACCTCGACTACGACTCCGTACAGCCCTATTTCATCTGCGACGAGGAAGA
GAATTTCTATCACCAGCAACAGCAGAGCGAGCTGCAGCCGCCCGCGCCCAGTGAGGATATCTGGAAGAAATTCGAGCTGCTTCCCA
CCCCGCCCCTGTCCCCGAGCCGCCGCTCCGGGCTCTGCTCTCCATCCTATGTTGCGGTCGCTACGTCCTTCTCCCCAAGGGAAGAC
GATGACGGCGGCGGTGGCAACTTCTCCACCGCCGATCAGCTGGAGATGATGACCGAGTTACTTGGAGGAGACATGGTGAACCAGAG
CTTCATCTGCGATCCTGACGACGAGACCTTCATCAAGAACATCATCATCCAGGACTGTATGTGGAGCGGTTTCTCAGCCGCTGCCA
AGCTGGTCTCGGAGAAGCTGGCCTCCTACCAGGCTGCGCGCAAAGACAGCACCAGCCTGAGCCCCGCCCGCGGGCACAGCGTCTGC
TCCACCTCCAGCCTGTACCTGCAGGACCTCACCGCCGCCGCGTCCGAGTGCATTGACCCCTCAGTGGTCTTTCCCTACCCGCTCAA
CGACAGCAGCTCGCCCAAATCCTGTACCTCGTCCGATTCCACGGCCTTCTCTCCTTCCTCGGACTCGCTGCTGTCCTCCGAGTCCT
CCCCACGGGCCAGCCCTGAGCCCCTAGTGCTGCATGAGGAGACACCGCCCACCACCAGCAGCGACTCTGAAGAAGAGCAAGAAGAT
GAGGAAGAAATTGATGTGGTGTCTGTGGAGAAGAGGCAAACCCCTGCCAAGAGGTCGGAGTCGGGCTCATCTCCATCCCGAGGCCA
CAGCAAACCTCCGCACAGCCCACTGGTCCTCAAGAGGTGCCACGTCTCCACTCACCAGCACAACTACGCCGCACCCCCCTCCACAA
GGAAGGACTATCCAGCTGCCAAGAGGGCCAAGTTGGACAGTGGCAGGGTCCTGAAGCAGATCAGCAACAACCGCAAGTGCTCCAGC
CCCAGGTCCTCAGACACGGAGGAAAACGACAAGAGGCGGACACACAACGTCTTGGAACGTCAGAGGAGGAACGAGCTGAAGCGCAG
CTTTTTTGCCCTGCGTGACCAGATCCCTGAATTGGAAAACAACGAAAAGGCCCCCAAGGTAGTGATCCTCAAAAAAGCCACCGCCT
ACATCCTGTCCATTCAAGCAGACGAGCACAAGCTCACCTCTGAAAAGGACTTATTGAGGAAACGACGAGAACAGTTGAAACACAAA
CTCGAACAGCTTCGAAACTCTGGTGCATAA

HUMAN SEQUENCE - GENOMIC (SEQ ID NO: 22)
ACTCCCACCACACCTATTCAACATAGTATTGGATTCCCTGGCCAGAACAATTAGGCATGAGAGAGAAACAACAGGCATCCAAACAA
GAAGAGAGAAGGTCAAATTATCCCTGTTTGCAGACAATATGATTCTGCATCTAGAAAACTCCATAGCCTCTGCCCCGAAACTCCTT
GAGTTGATAATCAACTTCAGCAAAGTTTCAGGATACAAGATCAATGTACAAAAATTAGTAGCACTCCTATACACTAACATCACCCA
AGCCAAGAGGCAAATCAGGAACACAATCCCATTCACAATTGCCACAAAATAATAAAAAGCTTAGGAATACAGTTAACTAGCGAGGT
GAAAGATCTCTACTATGAGAATTAAAAAACACTCTTCAAAGAAATCAGAGATGACCCAAACAATTGGAAGAACATTCCATGTTCAT
GGAAGGGAAGAATCAATATTGGTAAACTGGCCATACACCTCAAAACAATTTACAGATTCAATTTTATTCCTATCAAACTACCAATG
ACATTCTACACAGAAATGGAAATAACTACTCAAAAATTCATATGGAGCTAATAAAAGAGTCTAAATACACAAGGCAATCCTAGGCA
AAAAGAGCAAAGCTGAAGGAATCACATTGACCAACTTCAAATTGTACTACAAGGATGTAGTAATCAAAACAGCATGGAACTGATAC
CAAAACAGACACATAGACCAATGGAACAAAATAGTGAGCACAGAAATAAAGCCACATACCTACAACCATTGGATCTTTGACAAATC
TGACAGAAACAAGCAAGAGGGAAAAGACTCCCTATTAAATTAATGGTGCTGTGATTACTGGTTAGCCATATGAAGAAAACTGAAAC
TAGACTTATGCCATATATAAAATCCATATGCCGGAAAGCTGAGGCAGGAGAATCACTTGAACCCGGGAGGTGGAGGTTGCAGTGAG
CCGAGATCACACTACTGCACTGCAGCCTAGCGACAGAGTGAGACTCCGTTGCAAAAATAAATAAATAAATAAATAAATAAATAAAT
AAATAAATATCAACTCAAGGTGGATTAAAGACCAAAATGTAAAACTCTAAAACTATAAAAACCCTGGAAGATACCCTAGGAAATAC
CATTTTGGACATAGGAGCTGACAACGATTTTTTGAAAAAAACACCAAAAGCAAAAGTTGACAAATAAGACCTAATTAAACTAAAGA
ACTTCTGCACAGCAAAAGAAACTATCAACAGAGTAAATAGGCAACCTGCAGAATGGAGGAAAATATTGACAAACTATATATGTGAC
AAAGGCCTAATATACAAAATCTACGAGGAACTTAAACATACAAGGAAAAATCAAACAACCCACCTCACTAAAAAGTGGGAAAAGGA
CATAAACCGACACGTTTCAAAAGAAGACATACAAGCATATACATACAAGCCAACAAGCATATGAAAAAATGCTCATCATGACTAAT
CATTAGAGAAATGCAAATCAAAGCAGCAATAAGATACCATCTCACACTAGTCAGAATAGCTATTATTAAAAAGTTAAAAAATCACA
GATGCTGGTGAGGTTGTGGACAAAAGGGAACGCTTATACACTGCTGGTGGGAGTGTAAATTAGTTCAACCATTGTGGAAAGCAGTG
TGGTGATTCCTCAAGTAATTAAAAACAGAACTACCATTTGACCCAGCAATCCCACTACTGGATATAAACCCAAATAAATATAAATC
TTTCCACCACGAAAACACATGCAAGCATGTGTTCATTGTAGCTCTATTCACAATAGCAAAGGCATGAAATCAGCCTAAATGCTCAA
CTACTGCAGACTGGATAAAGAAAATGTGGTACATACACACGATGCAATACTAAGCATCCATTAAAAAAAAAAAAGAAATCATGTCC
TTTTCAGGAACATGAATGGAATCAGAGGCCATTATCCTTAGCAAACTACCACAGGAACAGAAAACCAAACCTATGTTTTCACTTAT

AATTGGGAGCTAAATAAIGATAACCCATGGGCAGAAAGAGGCCTCACACTGTGAGGCCTGCGIGAAGGAGGAGGATGGGAGGAGAA
AGAAGTCCAGGGGAAAAAAACTTCGGGTACTGTGCTTAGTACCCAGACAGCAAAATAATCTGTACACATTGATGGGACGTATCTCA
AAATAATAAGAGCTATCTATGACAAACACACAGCCAATATCATACTGAATGGGCAAAAACTGGAAGCATTCCCTTTGAAAACTGGC
ACAAGACAAGGATGCCTTCTCTCACCACTCCTATTCAACATAGTGTTGGAAGTTCTGGCCAGGGCAATCAGGCAGGAGAAAGAAAT
AAAGGGTATTCAATTAGGAAAAGAGGAAGTCAAATTGTCCTTGTTTGCAGATGACATGATTGTATATTTAGAAAACCCCATCGTCT
CAGCCCCAAATATCCTTAAGCTGATAAGCAACTTCAGCAAAGTCTCAGGATACAAAATCAATGTGCAAAAATCGCAAGCATTCTTA
TACAACAATAACAGACAGAGAGCCAAATCCTGAGTGAACTCCCATTCACAATTGCTTCAAAGAGAATAAAATACCTAGGAATCCAA
CTTACAAGGGATGTGAAGGACCTCTTCAAGGAGAACTACAAAACACTGTTCAATGAAATAAAAGAGGACACAAACAAATGGAAGAA
CAITCCATGCTCGTGAATAGGAAGAATCAATATCGTGAAAACGGCCATATTGCCCAAGGTAATTTATAGATTCAATGCCATCCCCA
TCAAGCTACCAATGACTTTCTTCACAGAATTGGAAATAACTACTTTAAAGTTCATATGGAACCAAAAGAGAGCCCGCATTGCCAAG
TCAATCCTAAGCCAAAAGAACAAAGCTGGAGGCATCACGCTACCTGACTTCAAACTATACTACAAGGCTACAGTAACCAAAACAGC
ATGGTACTGGTACCAAAACAGAGATATAGACCAAAGGAAGAGAACAGAGCCCTCAGAAATAATGCTGCATATCTACAACCATCTTA
CCTTTGACAAACCTGACAAAACCAAGAAATGGGGAAAGGATTCCCTATTTAATAAATGGTGTTGGAAAACTGGCTAGCCATATGTA
CAAAGCTGAAACTGGGTCCCTTCCTTACACCTTATACAAAAATTAATTCAAGATGGATTAAAGACTTAAATGTTAGACCTGAAACC
ATAAAAACCCTAGAAGAAAACCTAGGCAATACCACTCAGGACATAGGCATGAGCAAGGACTTCATGTCAAAAACACCAAAAGCAAT
GGCGACAAAAGCCAAAATTGACAAATGGGATCTAATTAAACTAAAGAGCTTCTGCACAGCAAAAGAAACTACCATCAGAGTGAACA
GGCAACCTAAAGAATGGGAGAAAATTTTTGCAATCTACTCATCTGACAAAGGGCTAATATCCAGAATCTACAATGAACTCAAACAA
ATTTACAAGAAAAAAACAAACAACCCCATCAAAAAGTGGGTGAAGGATATGAACAGACACTTCTCAGAAGAAGATATTTATGCAGC
CAAAAGACACATGAAAAAATGCTCATCATCACTGGCCATCAGAGAAATGCAAATCAAAACCACAATGAGATACCATTTCACACCAG
TTAGAATGGTGATCATTAAAATGTCAGGAAACAACAGGTGCTGGAGAGGATGTGGAGAAACAGGAACACTTTTACGCTGTTGGTGG
GACTGCAAACAAGTTCAACCATTGTGGAAGTCAGTGTGGCGATTCCTCAGGGATCTAGAACTAGAAATACCATTTGACCCAGCCAT
CCCATTACTGGGTATATACCCAAAAGATTGTAAGTCATGCTGCTATAAAGACATGTGCGCACGTATGTTTATTGCGGCACTATTCA
CAATAGCAAAGACTTGGAACCAACTGAAATGTCCATCAATGATAGACTGGATTAAGAAAATGTGGCACATATACACCATGGAATAC
TATGCAGCCATAAAAAATGATGACTTCATGTCCTTTGTAGGGACATGGATGAAGCTGGAAACCATCATTCTCAGCACACTATCTCA
AGGACAAAAAACCATACACCACATGTTCTCACTCATAGGTGAGAATTAAACAATGAGAACAGATGGACACAGGAAGGGAAACATCA
CACACCGGGGCCTGTTGTGGGGTAGGGGGAGGGGGGATGGATAGGATTAGGAGATATATCTAATATTAAATGACGAATTAATGAGT
GCGGCACACCAACATGGCACATGTATACATATGTAACAAACCTGCACATTGTGCACATATACCCTAAAACTTAAAGTATAATAAAA
AAAATCTGTACACTAAAACTCCAAGTCATGAGTTTACCCATATAAAAAACCTGAACCTAAAATAAAAGTTAAAATATTCAAATATA
AATAAGTAAATAAATAAAATGACAAACCCATGGCTAACATCATGTTGAATAAGGAAAAGTTGAATGCTTTTTTTCTAAAATCCAG
GAGAAGACAAGGATGTCCATTCTCTCTTCTACTCAACATGGTACTGGGAGTCTTAGGTAGAACAATTACACAAGAGAATGAAATAA
AGGTCATTCAAATTGGACAGGAGGAAGTCCTTTTCTGCAGTAACATGATCTTATAGGTATAAAACCCTATAGACTCAACCAAAGAA
AAATAACTGGTAGTATTGATAAATGAATTCAGIAAAACTTCAAGTTACAAATTTAATGTACAATAATTAGTAGTGTTTCTACACAG
CAACAGCAAACTATCTGAAAAAGAAATCAAGAAATCTATCTCTTTTACAATAGCTACAAAAATTTCAAAAAATTTCAAAAAATACC
TAGAAATAAATTTAACCAAGGAGGTGAAAGATCTCTACAGTAAAAAATAATAAAATATGGATGAAAGAAATTGAAGAGGATACTAA
TAAATGTAAAGTTAACCCATGCTCATAGATTTGTAGAGTTAATGTTGTTAAAATGTCCATACTACCCAAACCAATGTACAGATACA
ATGCAATCTCTATCAAAATACTAAGGACTTTCCTCACAGAAGTAAAATATATATATTTTTTAATATATAAAATGTATATATAAATA
TAATATATAATATGAATATTATATATGATTATAATATTATATGATTATATACTATTAATATTATTAATAATATATATTATTATATA
TATTATATATATTATTATATATTGTATATAAATATAIATTATATACTATATTATTATATATTGIATATAAATATATATTATATATA
CTATATATTTATATACTATATATATAGTGTATATATATATTTATATAGTATATATAATATATACACTATATATTTATATAGTATGT
ATATATATATACTATATATAATACATAAAATATATATACATATATAGTATATATATGAAATATATATATACTAAATTTGCACAGAA
CCACATGATTCCAAACAGCCAAAGCAATCTGGAGCACAAAGAACAAAGCTAGATGCATTACACTACCTGGCTTTTAAATTTTCTAC
AAAGCAATAGTAAACAAAATAGCATAGTACTAACATGAAAACAGATCCATAGACTGATGGAGGAGAATAGAGACCCCAAAAATGAA
TCCATACAATTATAGCCAACTAGTTTTIGACATATGTACCAAGAAAACACATTGGGGAATGGAAAGTCTCTGCAATAAATGGTGCT
GGATAAACTGAATATCCACAAGCAGAAGAAAGAAACTAGGTGCTTCTCTCTCCCAACATATAAAAATCAACTCAAAATGGATTAAA
GACTTGAATATAAAACATGAAACTAAGAAGATCCTTGAAGAAAACACAGGGGAAACAATTCATGATATTGGTCTGGGAAAGAATTT
TTCAAATAAGACCTCAAAAGCACAAACAACCAAAGCTAAAATAGATGAATGGGATAATATCAAGCCAAAAAGCTTTTACATCTTAA
AGGAAACAACAGAATGAAGAAATGACCTACAGAATGAAGAAATATTCACATACTATGCATCTGACAAGAGGTTAATATGAAACTAT
GTAATCAATGCAAGCAACTCAATAGGAAACACACAAATAATCCAATCAAAACTTGGGCAAAGGATTCGAATAGATATTTCTTAAAA
GGAGTCACACAAATGGGCAGCAAGTATATGAAAAAAGGCTCAACATCACTAAGAACCAGGGAAATGCAAATCAACACCACAGTGAG
CTATCCACTCATCCCAGTTAGAATAGTTATTATTTAAAAAAATAAAATAAAAAGACAAGAAAATGGCCAGTCATGGTGGCTCATGC
CTGTAATCCCAGCATTTTGGAGGCCAAGGAGGGCAGATCACTTAATGCCAGGAGTTCAAGAACAGCCTGGCCAACGTGACGAAACC
CCATCTCTACTAAAAATACAAAAAATTAGCTGGGCATGGTGGCACATGCCTGTGGTCCCAGCTACTCAGGAGGCTGAGGCACGAGA
ATTGCTTGAACTGGGGAGGCAGAGGTTGCAGTGAGCTGAGTTCGCACCACTGCACTCCAGCCTGGGTGCCAGAGCAAGGATCTGCC
AAAAAAAAAAAAAAAAAAAAAAAAAAAAAAGACAAGAAATAACATATGCTGGTAAGGATGTGGAGAAAAGTGAACTGTTATACAATG
CTATACACTTGGTGTACTACTATATTATCCAGCAATCCCACTGCTGGATCCCCCTCCCCCCCAAAAATGAAATCAACATATCAAAG
AGTTCAGATCCCCATGTTTATTGCAACACTATTTGCAATAGCCAAAATATGGAATCAACTTAAGTGCTCATCAACAGATGAAACAG
GAATGAAAATATGGTATATATACACAATGGAATGCTATTCAATCTTTAAAAAAGAATGAAACCCTCAAAAAGTGGGTGAAGGACAT
GAACAGACACTTCTCAAAAGAAGACATTTATGCAGCCAAAAAACACATGAAAAAATGCTCACCATCACTGGCCATCAGAGAAATGC
AAATCAAAACCACAATGAGATACCATCTCACACCAGTTAGAATGGCAATCATTAAAAAGTCAGGAAACAACAGGTGCTGGAGAGGA
TGTGGAGAAATAGGAACACTTTTACACTGTTGGAGGGACTGTAAACTAGTTCAACCATTGTGGAAGTCAGTGTGGCGATTCCTCAG
GGATCTAGAACTAGAAATACCATTTGACACAGCCATCCCATTACTGGGTATATACCCAAAGGACTATAAATCATGCTGCTATAAAG
ACACATGCACACGTATGTTTATTGCGGCACTATTCACAATAGCAAAGACTTGGAACCAACCCAAATGTCCAACAATGATAGACCGG
ATTAAGAAAATGTGACACATATACACCATGGAGTACTATGCAGCCATAAAAAATGATGAGTTCATGTCTTTGGAGGGACATGGATG
AAATTGGAAGTCATCATTCTCAGTAAACTATCGCAAGAACAAAAAACCAAACACCACATGTTCTCACTCATAGGTGGGAATTGAAC
AATGAGAACACATGGACACAGGAAGGAGAACATCACACTCTGGGGACTGTTGTGGGGTGGGGGGAGGGGGGAGGGATAGCITTAGG
AGATATACCTAATGCTAAATGACAAGTTAATGGGTGCAGCACACCAGCATGGCACATGTATACATATGTAACTAACCTGCACATTG
TGCACATGTACCCTAAAACTAAAAGTATAATAATAATAATAAAAAAGTAAAAATGATTAAGGACTATAATAGGATAAGAACTCTAG
GAAGATGATAAAGTATGCCCAAAAGAAAGCAATGCTATAATAAAAACAAAATAAAACAAAACAACAACAACAAAAAGAATGAAACC
CTGTCATTTGCAACAATGTGGACAAACCTAGAGGACTTTATGTTAAGTGAAATAAACCAAGCACAGAACGAGAACTACCACATGAT
CTCAGGCATATGTGGCATCTAAAAAAGTTGACCTCAAAGAGAGTTTAGTAGTGGCTATCGGAAACTGAGGAGAGTAGTTGCGAGGG
GGAGGGGAACAGGTTGCTCAACAGCTACAAAGTTACACTTATATAGGACAAATAATTTCTGATAATCGACTGCACAGTAGGTTGAG
TGTAGTCAACAATAATGTATTGTGTACTTTAAAATAGTTAGACGAGAGGATTTTGGACGTTCTCACCACCAAGAAATGACAAATGT
TTCAGGTGATAGACATATGCTAATTATGCTGATTTGATCATTACACAATATATATGTATCAAAACATCACACTGTATCCCATTAAT
ATGTACAATTATACATCAATTTAAAACAAAATAAAATGTATTTTATAAAAATTTTAAAACTTCAACCAGCAAGTTTGAATCTCCTA
TTCCCAGTTTTGGTCAGCCCTACAGTGTATACCAACCAGGCTGGCCTGGGGCTGCTGTTAGAGGAAAGAAAGAAACATCTGGACTT
GGAATGAACAGATCTGGAAGGAATCCTAAATTTTCCAATAAAGTCTCTGTATTCCTTTCATCCTGGTCCCTACCCAGGAAGCAGTC

TAGAGGAGATGATCCTTTTCTAAGAACAGCAACATTCAGTAAACAAACTTCCTTACTAATGTGTGTGGATCAAGAGATGGTGAGGA
AAGTAGAGTCAGTGATATTTTTGAATCAATTATGAGAAGGCCACCATGCTATTGCTCAGTAATATTAGATGCCCATGGACAGTAAG
ATTCATCGATGGTCCGTCTTTATTATTAGGTCATTACTGAGTGGCTTTTGCAATAAAATACACCATTGGCAGAATAAAAATATTCT
CTAATGCAAAAACATAGCATGGATTGTTTTTAAGGGGCACAATAGTGTGCTTGATCAGACTGGAATAGCAACATTGTAATCTGAAA
AAAGCTCAACAGTGGTGAGGCATATTCAAAAGCCCCAAGAGGAGAGTCAACAGTCAGATCCATCAGAGTTGAGAGGTTGCCACAAC
TTATGTAATGACGTCTCCACGCCATGAAACACATGTGTCAGCTTTTGTCAGGAGTCACAGATCTGGCATGCAGCAGGAGATTCTGC
ATTAGATATATAAAGTCAGGCCCAATCATCCCCTTGATTAGTTAGTCTCATCAGAGAATGAGCCCTTGCCATGGTCTGTATATGA
GGAATCCAGGCAGTTCAGTAAGTAGTCAAGATTTGTTTCCACATGCGGTCCCAAAGAAGTCTTTAATCTGACAGACTAGTTTTCCA
AGTGGCATACCAAAAGGCTGGACCATTGGTAGTGTCCCAAGAATTGGGTTCTGACCATTGAGCAGAGAAACCAAGTCCTCTTTTTA
TTTCTGCATAGCTGGTTCAGAGGCTGAACAGACACAGGAGCCAGTGAACACCATCAGTTTTCAGCTTAGCCAATCCTTCACTGAGG
CAAGCCCAGATATTTGGGGAAACCTCTGTAAACAGGGTCCAATTGAACTAGTAGTGTTGTTTCAAGCAGGAGAATAAAAGGTTAAG
TTTCCCCCAGAGAGACAGTTGCCACTCTTACATATAAAGCAGAGATGCAGAAGAAATTCGAGTTGATGCCATCGCCCACCCACCAA
CCCACCCCCGCATTTCCGCCCTGAGACTAGGATCCCAGCGCCAGGGACCCAGACCCTGGGATTGATCGCACAGAGCCGTGATCCGG
AGAGTGCGCCGGGGATGAGACGGAATCTCGGGGCCACTCGAGAGCTTGGGGCAGGAACCATGCATCCATTGTCCATCGTGACTGCA
TGTGGAGCAGCTATTTGGCCTGGGAACAGCTGTAGAGAGCGGGGAGCGACTGGCTCGCCACTGGCGCGCCCTGGGGAACCCGCCC
AAGGTGCCCTCCTCCCCAGACTGCCCTCCCAGCCTCGAAGACGGCAATCCAGCGCCCACTGCTCCCTGGACTCTGGGCAAGGCCAA
GACTCAGGCCTGCTCCAGATTTGAGAACCCAAACAACTCAGAGGGTGAGGAAATTGACCTTGTGACAGCAAAGAAAAGGCAGATTG
GGTTTACAGAAGTCGATCACCATCATGGTTCGAGTAGACCGTCTGAACCCCTGCATGAAACACTTCCATACCTCCATCCACCAGCA
ACAACACAACTGCGCTGCCCATATTTCTCCAGAAAGCTGCTCCCAAGAAGAGGCTCCAGAGAGAGGTCTCCAAGAAGAGGTTCTGG
AGGGAGATGCTCCAGGGGAAACGGAAGATGAGGAGGATGAGGGGATTGTGAGCCCCTCACCTATAGAAAGCAAGGCTGCCCAGTGC
TGCCAGTTCTGACACTGAGGATGTGACCAAGAGGAAGGGCCACAGCTTCCTGCAGCACAAGAGGTGGAATGACCTGCGCGTTCTCA
GTTCTTGGCCCTGAGGGACCAGGTACCCACCTTGGCCAGCTGCTCTAAGGTCCCCACGGTAGTGATCCTAAGCAAGGCCTTGGGAT
ACTTGCAAGACCTGGCGTGAACCGAGAAAAAGATGTCTATGGAGAAAACGCAGCTCCAACGCCAGCAACAGCAATTGCAGAAATGA
ATTGCGTACCTCAGTGGCTACTAACTGACCAAAAAGCCTGACTGTTCTATCTTACAAAGACACAAGTTTATTTTTTGACCTCCCCT
TCCCCTTTAGTAATTTTCACATTTGGTTATGACGGACAGTCTTTGCAGTAGGTCCCAGAATGCATTGCAGCCAGTACACACAGA
ATAAGGGCTTGCATTCTTGGAAACCTTGAAACCCAGCTCTTTCTCTCCCCTGACTCATGCTGTGTCTTCTCTGGCGCCTTTGGCTT
CTCAGCAGATAGCTGACTGAGGAGATTTGGGGTCTGTTTACCTCACTAGCTCCGAAGAAAAGGCTGACAGATACTATGCAACAGGT
GGTGTATGTTGTCGGGGACTCCAGCCTGCATGAAATCTCACACTCCGCGTGAGCCTTAGACTAGGAAAGAATGCTCCCTGGTATCT
CTGGGGTGATGCTAGAATGCTCCCTGGTATGTCTGGGGTCATGCAAGGACAGCTGGGCCTGGACAGCACTCTCGCTGTGGCTTTTT
TTTCCAGGAGACACACAAGCTGTCTTGGGTGATGACAAGCTTGAAGATTTGATCAACATGACCATTGCTTCACTGTCAGACACTTT
ACAGTATCTGAGGAGTTGGAAACCTTTAACGTATATATTGTGATATTAGCTGACACCTCTCCTTCCAGTTTCAATGCTGAGACCCT
GAGAACATTTAAAGAGCTTGCACTCTAGGTTCGTTGTCTCAGAGCTCTCTGGGCCTTCTCTGATGAAGGGACCTTTCTGTCCTCAT
GAGAGACTTTTGTTTCATTTTGCCTTTGTTGTGCAATGGGCTTTACAGCATCCTTTCCCACAGGTTAGAAATGTTTCCCCAAGTTA
CAGGGAAGTGGGGATCCTAGCCTGGGGCCTGAGGAAATCTTGGAGTCCTGGCTCCTGAACTTGTTCCCTGTCCGAGTGGCACTTGA
GGCCACCCATCTTATAATCTCTTCTCAAGGCACATCTAAGTCACCTCAGAAGGGAGAACTGTACCGCTTCCTCTTTTCCATGAAGC
TCTCATCTCAATCTTTGACTGATGAGTGTGAAATTCTACCGGAACCATGCAAACACGTCCACCTTGGGCATCTCCAAGGAACTTGT
GGGGTCTGCAGCATACTTGGCTCCCTACCAGCCTGCCATGAGTCATATTTCTTTTCCAGAAGGTGGACTTGCTTCCTGGTATGTTT
TAAAGGAGCCTGCAGGAGCTCTGCTTAGCCAATCATGATGGATTTTTGCCCCAGCTGGACTCTGCATGTCCAAGGAGAATCCAGGT
ACAGCCTCCATTCCGGGAAAGACATCCAGCCCAGCAGTTGTCATGTGGGTAACCTCAGGAACCCCTAACCCTGTCCTGGAAAAAGG
ACAAGCCCCTCCAGAACTCTGCCCAAAATAGCAGGTGCTTGATGGTTCTGAATTTGGAAAGGGATGGGGGTGATAAGTACTATCT
GTGGCTCTGGAAAACCAGCTGCTACATTCAAATCTATTTTCCATAATGGTTTCTTTCTGAGGTTGCTTCGTGGCCTCAGAGAATCC
CAGAGGATGTTTTGAAATAGCCTCTCTACCCTTCAGGAGCATGGTAGTTTACAGGAACCAACTGACTTCTGGAACTGTCTATGGAG
GGAGAACAGGCCAGGTGTAGGTTACTAATGTCCTACATGAATAGCTTGGTTTTATAAGCTGCTGTTGGGTATTATGTTGGGGCAGT
CTTTTTAATATATTGTATTTTTGTACGCATTTTGCAAAGTGGAGTTAACTGTTTTGTAAAAGAAAAAAAAGAAACCCTTGGGTGCT
TCTTCTGTTTCAAGGGTCTGATTTATTTGGAAAGGCAAGTTTATCTGAAATTTTGTATTATTGATTGCCCAATTTTAAAATGTTGC
CTTCTGGGACATCTTGACAAAAAATATTTCTCAAACATGAAGAAAATAAATATAATAAAAAAGAAATGTCTGTGGGATCAGATGAC
TGTTCTTGATTTTACCGTTTCCATTTCATAAGCCAAGCTTATTGACCCCTTCCTACCTGCTAGTCATTGAGTCTGAGTTGACCAAC
TCAAAGATGGAAATATCGAGCCAGATGGTAACAAATTCTCCATGAATCGATTGTTCAGTTTTGACAGAATCCCAATTGCAAGCCAT
TAGCTGCTTTTCAAAAGGGATGTACCCTGTAGCCATATTAGGGAGGCAACAAGTCCAGTTCCCCAAGGGATCTCTCTGGGTGTTGA
CTGCTTCCCCTTGCCAGGTGCCCCCATCGGCAAAATCATCAGTTACACAGATTTGTCCTGTATGTAATATTCAAGGAGTTGATTGA
GGTTCTGAGCCTCTTTTTCGGTGGCAGGTTGTATGAAGAGACGTCAATTTTTTCCTGTTAGAGGGATCTAATGTTGTGAATTTGCC
TGCTTAGTCCCAAGAGATTACTTGGCAAGCAGGCCCCTGTATTTGGGAGCATAAATCAGCCACCCTTGCTGACAGATATGTAATAA
CACTGCATTGAGGACCCTTGAGACTGAGACTTCTAGCCGGCCAGCTGACACAATTATTATGTATATAGTGAAAATATTGAACATTA
GAAGGTAGAGACACTAATGCTAAATCCTGAGCCAACCACTGCTGACAAATCTCATTGGAGTTTGTTCCACTAACACTTCCCTCTAC
CTCTGTCTCTCTGTGATGGGGATGACTTTATATGAGACCTGTAGAATGAGAAATTACAAGCATATGACCCATCAAATCCTGCCATT
TATAAGTAGAAGCAACAAGAACAGTTTATTCAACTTTCCCAAAGGCGTCCCTGTACAAAGTCATATTGGCTTCCAAACCCCACTGT
GAACCATGCCCAAACCCCAACAATTGCTTTTCTGGACTTTTTTCCCTTAAATGAACTCAGACCTAGAGTTCACTACAGGAATTTCA
GGGGTTACAGCAAGAGCTGTTAGGGGATTTCCCTATGTCCAGGCCAGAATGTAGGGATTGCAGGGGAGTCTTGGGATGAGGCGAGG
GGTGGGGACTGTAAGCCTTTTTTGTAATCCCTCTGGCTCTTTGCCCCAGCTTGTATGCTAAATCCTCTCCTGTGTACCTCCATTTG
TGCAGAACTGTAAGGGCTCAGGGATTCTTCCATTCTGTCCCTTGATTTCCCCAGCAGCTCTGCTCCACAGTCTTGGTGCTTTCATC
TACCTCATACCTATTACCCCTTCCCTCCACTCCCCAGAAAATCAGATAAGATAGTGTCATGGGAGATTATATGTAACAGTTCTGTG
AACATGTGAGTCCGTCCCTTTCCTGAAGTACCCTACCATGCATATGCCTTTTAGTCTGTCTTGAGGACAGAGAGACTTCTGGCTCA
CCACTAATCATCTTTCTTCTTATAGCACCTGAAGTGAGAAAAGGAAGAGAACCAGGAGTTAGGAAATGCAGAGAGAGAGAGAGAAA
AGACTCTGTCTATACTAGTAAGCAAGGCATGTTTGCATTTGGTTTCCAGTAGCCAGCAGTGTGGTTCAACCAAACAAACTTCAGAT
GGTTTTTTTTCCACTGAAAACTCTATATCTTCACTGCTGACGCCACTTATTCAGGTTTGGCAAGTCATTGACTCAGCACCATGACA
TCATCCCTTCTTCCTCCCATGCAGAGGAGAGTGAACAACAACCAAACCATGATTCAAACAGCTTGGTTTAATTCTGCATCCTGCCC
CTCAGCTTCATTAATAGGTAGGAGAGTGAGTGGGGAAGGGCCTTTAATGCACTTAGTGCCAATCCATTCGGGAAAAAGCATTTGCT
CTCCAATCCCGAGGAGTCTTCTCATATCCCCTAACCAGGCCAGTGAAGCACCCTTGTTTTATCACTTTCCAGAATGCTATGTTTCA
TCTTCATTACCAAAATTGGCAAGAAATGTGAAGAGTTTGAGACTGTACCCTACTTGCAAGCTCACAAACTAGCATACCTCAGTTTC
ACAGATGTTGGCAGAATATATGAGATTCTTGGGTCAGAGTTTGTTTTGGTTCATGTTTGCATTAATTCCCCTTGCCCTCTGCCCCA
AATCACACAGGAGAAATGAGGAGCAGCCAGTCAAATGTTGACATACAAGTATTCTTCAAACTATAGTTAGAGACAAAAGCTGTTAC
AATAAATGTAGAAAGGCCATGGACAATTATCTCAACAATATCTGACCATTGTTTCTCCACTATCTTGGCTTCTGACAAAGTTTGCC
ATTAAGTATGTGTGTTGATTTTCTGGATATCAGGGCTGAGCAAGGAAAAAGTCATGACCATTAGATGGCAGTAGCACACAACATGC
TTTATTTGGTTGGCCCTTTGAAAGGTTTCAGGAGAGTGGAACTTCCCTCACAGCAGGAGACCTTCCAGAGGCAGCAGCAAGAGGCC
ACCACCCAGAAGAGGAAGAGTTCAAGGCAACTTCCAAAGGAGAGAAGAATTGGACAGGAGGCTCATGTGTCTATGGATGTTACTC
AGCAGCCAGGTGTGGAGTCTCTGTGTCAGAGAGTTCCCATGGGCATCAGAGGCTTGGGGCTTTTGTCCAGCCCCAGAGTATGTCCT

ATCTACGGCTAGCAGATGGTGTGTCTGGTTTTACAGAGGGTGCAATGTGGGTGGGTTCTAAAGGGCCAAAAATGTGCTTACTGGGG
GCCATATTTAAAGCAATTGGGTGTGTAAACATTTGATTTTAGTTCCAATGAGCTTTGAGCTAATGGTCTTAGCTGTCTCTGAATAT
AGGGAACAAGATACAAGGCACCATCTAAAGATATTAATCCACTTATTTAACAGTCCATGCCTTGGCCCCATGTTGTTTTACAATCT
AAGTTGTATGCTATTTCATGGAGTCTCAGTTGGGAGCCAGCGATGAAGCATCCTCAAGACCCAGAATGCTATAAGGCTACAAATTA
GGATTTTAAAAGCCAGTTCTCAGCCAATCTGCCCACTTGGGCAGCCAGTAGAATAAATTTGAAAGGAGACAATTTTGTTTTGACCA
GTCCGTTTAGTCAATAGAGTGGCATTTATATCCCCTTTGGTACATGACCCCAGCTATCTGTAAAACTTGGTGTTATCTACGCACAT
GCAGGAAGATAATTTTAGCAAGCACACAGCCCCTGTGCCCTGTGACCCCTCCAGCAGCCTGACCTAGGGCTATGCAGTTATCTCAC
ACCATGGCCACTAAAGTACAGTGAGATTCAGCAAAGAGTTTCAGTCCTTGGGCTGTTTATCTAATTTCTGTGGGTAATTCCTGCAA
CATGGCTATGTGTTTCTCTTAATTGTGGTGTGCTGTTCCACACATATGCTGTTCCTACTGCCTCCAAAATTAGTGATCCTAGGGAA
GTTAGTACCATGAGGAACTATTTGGAAAGCTCATTTGTGACATTGGGGAGTCACATCTAGTGTGGTTTGGTGGCGGAAAGTACTTG
AGGGAGGGGGTTCCACTGTTGAATAAAATTCTACTGGAATTTTGCACCAATCCCAAGTGCAGGAAATATTCTTCAGGGTTGGATGA
GGAGAAGATTGTGATAGGGAAGCCATCTGATTCCTTTACAGGACCTCCAGGCTCCCTCTGCTGCCTGTATTCTCTGTGTTCCACTG
AAGGGAAGTTCACATGGCTTTGTGAAGTTCCAGAAGTTCCAGGTTGTGTTAATAGGAGCAGAAAATGGTATAGTCAAACAAGCTTG
TGTTATCCACCTTGCATTCAGCACCTGGGTTAACTAAATTTGTCGTGGGCTCAGCCCACCATTGTCTATACATTTATGTGAGATAA
GCCTCTGGATAAGGTTGTTGAATGTCAATGGTTAGGGGAATCGGCTCATCTAAATAAGTGAGGTATATGATGCAGCGTTGCCAGGA
AAGAAGTATTACACTATGTTTTCTAAAATCAGGTTGGCAGAAGCAAACCCTGCATTAGGTTAGATTTACCATCTTATATGTTGCTG
TGGCTAAGGCTATAGGCACGTTTGGTGTAGTCGGCAATTGCATGTTCCTCCTTGCTGTGACTGTTGTGTGTTGTGCTTGAGTTTCC
TGTTGATTGCCAATAATAAATAGCATTGATGTGTTCCCATGAGTGTGGGATAGGTTTTGCAGGTGTTATTGGCAGTCCAAGAAGAA
CGTTGTTAGTCTAGCCACACACCAAATTAGATTCTGAATTATGAGGCACTCGCATATGAGTGAACAAAACAGGTAAACCAAGTATT
TTTTAAAAAAATAGATATCAGCTGGGCACAGTGGCTCATGCCTGAAATCCCAGCACTTTGGGAGGCCGAAGTGGGCAGATCACGAG
GTCAGGAGTTCCAGACCAGCCTGGCCAATATGGTGAAACCCCGTCTCTACTAAAAATACGAAAAAAAAAAAAAAGATTACCCGGGTG
TGGTGGCATGCTCCTGTAGTCCCAGCTACTTGGGGGCTGAGGCAGGAGAATCACTTGAACCCAGGAGGCAGAGGTTTCAGTGAGCC
GAGATCACGTTACTGCACTCCAGCCTGGGCGACAGATCCAGACTCTGTCTCAAAAAAAAAAAAAAAAAAAAGATATCAATTGTATCA
AGGCATAGGTCCCTTTTATGTTCCCTTGTATTGCAACATTATTGTGTTTCAGTGGGGGACCAAAAGCTTTATGGACTCCATTTTAG
CGGAAACTTTCATAGGGAAGAGGTTTGTATACCTTTAATAACCTCAGGAGTGGCCCCTTGGGAGGTGGCAGTGTGACTACACCAAT
AAATTTCCTCCCAAGTACTCATGGACATAAAGTGTCTACTGAGACACTGGAGACTACTAGAGGGGGACGGGGGAGGGGAGCAAGGG
TTGAAAAACAAACTGTTGGGTACTATGCTCAATACCTGGGTGATGGGATCATTTGTACCCCAAACCTCAGCATCATGCAATATACC
CAGGTAACAAACCTGCACATGTGCCCAATGAATCTAAAATAAAAGTTGAAAAAGAAATGACAAGGCATAAAAATGTTTTAATGAAG
ATGAAAACATTCCTCCCAAGTTAGTTTCATTACATGTCTTCTCATTATCGTTTATGTCTTCTGTAGGTTTGGAGTGTGTATGGATT
TTTAAGGAGCCCTCAGAAAAGTAACATTGGGCAACAACTGCCCTCAAATAGTTTCTAAGTTTTTAATAACTGTGGTTTTGGACAAA
TTTCACAATAGCTATAAGCAAATAGTTATCAATGTGGGTGGAGAAACTCCCATGCAAACAGTAGTTAGATTTCTCCCTGAGATCAA
ATATGTTTCTTGTAAGAACTGTCTGTGCTGTGACACCACGAGCTATGAGTTGTTCACTGTGGGTAGCCAGCATTGCAATTGCTGTA
GAGTTCACTTCCATTGAGTGTTATGTAAAACTTGCAGTTAGTATATTAAGATTAGGTTAGGGAAGAATTCCCAAGCTGGTTGTTTT
GTGTGTGTGTGTGTGTGTGTGATTTTTTTTTTAAGAAATGTCTTGGCCCAGGTAATGTCATGTAGTATGCATTGCCTTAGAAGCCA
ATAATTGTAAAGGTATACACTGTGGGTGTCGATTGCACTTATTATAATTCCAGGGATAAGAAAAAGCTATCATTTTATCTATGCAA
TCCCGTAATGGCTTGTGGGTATTGTATCTGGATTATAGTATTAATAACAACACCAATCACAAGCATCCTGGTTGTAATATTTGGTG
CATACTATCTCTGGGCATTCCTTAAACGAGGCTCAATAGATAGTGGCCCATTGCTCCATTTGTTTAATGGCACATGAGTTAATAAT
AGTGGGTATCATCTGATGTGAAGGAAAAGGCGGCCCACAGAGCTTAGAAGGCATCTATGAGTCACAAGTGAAATTGTACAATCTGA
CTTAGCTGGAAGGATGTTACTAAGTGGTAGTAGAGAGAGGAGGAGAAGTGCTAACCTCAGTGCTGATGTCTGTGGACTGACCCAGT
GAAATGGGAGCATGGTTCCCTTTGTTCCCAGTCTGACCTATGTTGGGTGTCCCTGTGGTGTGTATGAGGCATGCTGTTTCCATACA
TACTCTGCCAGCAGTAGGGTGTGGATTACCAGGGATAGGGTGATCCACCAATCGAGGTATAATTGACATTGGTGAAGGTAGGAACT
TTCTCAGGTAAGGAAAGGGCAGAACAACAGCATAGGCATAAATGTTGGAGGTCATTTGCAGCCAGCAGTAGGGAATCTGATTGTCC
TCTGGGAGTCACCCAAATGGTGTTACCAGATACAAGGGTTGTTGGTTCAATCTCCTGGGTGTCCGGAGCAGGTCAAGGTTTGTTCC
ATAGTGCCCTTAGTGGAGCAAAGTACATGGGCAGAAAATGAGGATGCTTCCCTCTCCAGATAGTGGTGGGTGGTTTGCTAGTTTTA
TGCTGGGTAATGAGCAGACTAAGACTGAGAATGCACCCAGAGTGAGTGGTCCATGCCAGATCCTTGTCAGGATTGAGAGGGGAAAA
AAATGGCCAGGCTCTGGATTTTCTATGAGGGAGAAGGAAGAGGTGAAAGAGCCAAGTTCCTTTGCTGTGGGCAGTCACTTCAGGGC
CCCCATGTTCGTGAATACTGGTAAATCATGTTGTGTCCAGTGGGTACATACATTACCAGAGGTTGCAGCATCTCTGTTGGTGGTCT
CCTCATTTGCTGTGAATTAAAATATGGTGCATTTATGCCAGAGAGGTATGGATTTAATGTAAGCATGACTATGGGCAGTATTTTTG
GCTACGGGGAATGCTATTGTCCTGCATGTTCTTTCAATATCCCTTGTTTTAAAAAGCCCATTGTGTCATTCTGTGACACCTGCTAC
TGTAGGACCAGAGAATAAATGAGAAATCCAAGCAGTGTTCTTTGGTAATGCCTATGGTTGAATCTTCTTTGCAGAGAAGTGAGAGT
CTTGATCTGACTGGATAACTTTGTGTAACTTTTGTACTCCCAAAGGCCATACAAACTATTTTATCAGTTCCTCAATAACATTAGCA
GAGCTTGGTGCCTTTCGTGGTAAGCCAAGAGTAAGCCTGTGTAGGAATTACTACATTGTTGTGGCATATGTAATAGTTCCTGCTG
CTTCAGAGAGGGGCCCAATAAAATTGACTGGATTTGCCAGCTCTGATATAGCCCTTCTCCTGTGCAGTAGGTCCAAGTCTCTAGAT
TTCTGGTCTATGGTACGGTCCCTAACAGCTTCATGTAAATGTCTCTCTGCTGGGTCTATTTCTCATTAACAGTTGATTTTCATGGT
CTCATGATGCCCAAATGCCCTGACCTCTCCTTTATAGGGTGAAGGGTAGCTCTTACCAAAGTTCTGCTGTCTCCCTTCTTTTCAAA
AGGATTAACTACTTTGGTACTTTAGCTAGTTTGTCAGTTTCCAGGTTGTATTGTTTGCTGTGAGCTGAGGTATGGTGTACAAAGAT
GTGCAGAGTGGAGGCCAATGTGCTGATATTTTCCCCCACATCTCCTTACCCCAAATGTGTCAGCCTTGTATGTTCCAGCCCTGATT
TTGTCCAAGTCCCAACCCAATAGCCTATTAGCCACTGACCAGGAGTTGCCAAAAATGTGAAACTTAGTAAAATGTTTGTCGAAGGC
CTTCTCTATTTCTAGGGGTGTTGCACACAGCCTGGCCCATTGGGCACTTTGGCCCATCCCCATATGGAAGTTGGAGTATTTGAATA
AGGGTAGTAGGCTGTTGCTGTCCTTTATCTGACTCCCTTATACCCTGTGCTCACTCCATTGGTAAAGAAAAACATTAATTTTCTTG
TTATGAAAGTGCTGGCCATACTCCCTCCATTCCCCAATGAGCAAGAGCCAGGGGCATTTCCAGGGGTTTTGGCAAGTTAACCTCAC
TGACAGAGTATGTGGCCATTTCCTCACACAGTAGTGACACTCCAGGAGGCCAGGTGTAGTTCAATCCTGTAGATACCATTTTCAT
TTTAATAAAGAAGCTTCTGTTGCTGATCCAATTTTATGGGCAGCTCTGTCCATTACCCAGGGCATAACAGGAATCTCGGTCCATAG
GGTAATTTTCTCTGCCCCACCAGGGCTTCAGTTTTGATAAGTGCCCCATATGCTGCCAGTGGGTGTCTGTCTAGTGAATACTTTAG
GCTGAGGCTGAGAGCTACTTACACCAAAAGCCCATGGGTAGGCATTTACCTGTCCATTTGGTCCAGAGGCTCCAGGAAGCAAATTT
GGGAGCAGTGGAAACTTATAATTGCAAGTCTTGTCCTTGGGAGCACTAGAGGGAGAGCCAAAGTATTAGCCTCCTTTAGCTCAACC
AGTGACTGTAGATGTTCAGGTCCCTACTGAAAAAAGGAAGCTTTAGAGGTGTCTCGATAGACAATTCAGCAAGGGTCAGCAAATAT
TGGAGGTGAGGAAGGCATTGTGCTATAATTCCTAATGAATGTTGGGGGTTCTTTACTGATGTGAGCGGTTTAAGCATGAGGAGCTT
GTCTTTTATCAGATTGGAAATAGTCTGAACACTATTTCTGTCCTGTCAAAGTGTAGAGATCTTATTCCTAGATTTAGACTGCCCTT
AGGAATCGGCTCAGATAATGGGCCAGCGTTTGTGGCTGACTTGGTACAGAAAACAGCAAAGGTATTGAGGATCACATGGAAACTGC
ATGCTGCCTACCGACCGCAAAGTTCTGGAAAGGTGACCGAGTGTGGATCAAAGACTGAAACATAGCCCCTTTGCATGCACGGTGGG
AAGGACCCCAGCCCATCATCCTGACCACTCTCACTGCTGTGAAGGTAGAAGGAATCCCGGCCTGGATCCACCTCAGCAGTGTAAAA
CCTGCAGCGCCTGAAACTTAGGAGGCAAGACCAAGACCGGATAACTCAGGCCGTGCTCCGCCTGTCAGATAGTAAATCCCAGGTGA
TGTACCTGCTGTCAGCAGATCTAGGCTTTCTTCTTGGACACCTTATACCCACAGTCCTCCAGGTGCCAAAGTAGGGCATCCGTTCC
CTTGGCAAACCCGACTGCTGTGGGGTGTCCTAGCAGAAGGTCCTCGACGTACTGGAGCAACACGCACCCTGGGTCTCTGGCAGGAA
ACTTCTGGAAGTCCCGAACCAATGTTTCCTCGAAGAGATGGTGGGGGAGTTCTTGAACCTTTGGGAAGGCGGGTTCAACTGTACT

GAGTGGTGACACCCGACCCCAGATCTTCCCACTGAAAGGCAAACAGTTTCTGGCTCTCAGGGGCTAGTCTGATGCTAAAGAAAGAG
TCTTTCAGGTCCAAGCAGGTGAACCAGCTGTCCTCAGCTGACAGCAACCCTAACAACATGTACAGTTAGGCACTGTTGGATGTAAA
CTCACTGTAGCTTGATTAACCAAGCACAAGTCCTGTACTGGCCTGTAGTCCTTGGTCCTTGGCTTGGGAACAGGCAGGAGGAAAGT
GTAGTATTCTGAGGTCTGAACAGGCAGGAGTGGAAAAACACCCTTCCGAGTTCCTAGTGTAGCCTTCAAATTTTACAGACTTATAC
CTTACATGGTGTAATTGCCAATCCACTCTCAGTGAGAGGTGGTTTACTGAGGGTTGTGATCAACTGTGAAACAGAGTCTTGGTCAG
GTTCTGCGAGGAGGATGTCACCGATATAATGCCAAAGCATAGTTTCTGGTGAAATTGAGGGGCAGGTGTCTATGTCCTGTTGGCAA
TGACTGTGCAATTGGAGGGCTATTAAGTATCCCCCAAAAAGCTAGCATCACAATGAAGATGAGGGGAAACAAATACCCCAAAAGCA
AGTGGGTAAAGGCGTACTATTGACTCTCAAAGGTGGAAGTGAAATGTGTTTGACTCAACTGTGAAATTTTTACTAAACAGAACATG
TTAGCCAAGTCTACAACTCCAGATTCAAATTCTGGTGGCTTTAGATGCCCTGGATTAATATAAACTTACCACTGATGTTTTGAATG
ACACCCATTAAGGCAACAATGTCCTATACAGGTGCTCTAATAAAGGAAACAGCCCTGCTGAACTCTCTATGGTCTATAGTCTCCAC
TGATTAGTAGTGTCTCTTAGGGCAGGCCATATGGGGAATTGATAGGTGAGGTAGTAGGAGTCAGTGTTCCTTGTTTATCTATGTCA
CCTGTGACAGGCCATAATCCTTCAGTGTCCTGCTTCAGCTTCTGCTGTGGTATATGAACCACCTGAACCAGGAGGGGCAGGTTAAC
TGGTTCCCTCCGGGAAGTGCCCACCAGTAGCATAAAAACCTTAGTCTTATGTCATGAGTTTATCTGCGGCCTCAGGAGGTCTCTTC
CAATAATGGGGAAATGGATAATCTTGGAGGGGAGGCAAGCAGGACAGAAGCATATTTTTAGCAAAACTGAGCCAATTTTAATAGTT
AAGAGGGGAATTATCCCTTTAATTAATCCCTTTAATTAACCCCCTTCCAAGCAGGTAGCCTACTTGCCAGGAGGGGGTTCCTTTTA
AAGCATGTGTGTGTGTTGGCATTAGGGAGATTTGGGCACCCCTGACTGACAGAGTTAGCCAGTGTTGTTTGTCAGTGCCCAAGCCA
CAGTTAATATGGTGAAAGGGCAACTGTCTGTCAGAAGGGGCCTTCCGACACTGCAGGATCCCTGTGTGAGAGAAGATTCTTAGCCA
TTTGGCAAGCATCAGAGACAGGTGTGGAAGATAAGGTTACCTCCAAAGGGAAAGCCAGCTGGGGTGGGAGGGGGTCGTAATCTAAC
CCTAGTAAGTATTGCAATTGCTCCAAACATTCACCAGAGATCATCCAATTTGGGACCTTCTTGTGCCTTAGGGTCTAACATAACCT
CTTTTCGTCATCTCGTGGTTGATGCATACATGGGATCCTGGCCACAGGTGGTTTGTTTGGCCCAGGAGGGGCAGCCTGAGTGGTGA
TAGTAACTTTGATTTAGGCCAGGTAGGGTATTATAGTGTTAGCAGGCAGCACCTGACTCTGAGTTTGTCTTTCATGAGTTTCTATT
AAATCCAGTGCAGCTATGGTCTCTTGAATTGTTTTAGCAGTAGCCACCATGCCCAGTAAAGAAGATTTTCCCAGGAGCCCCGCCCC
CCGAGGAACAATTGAGTGGACTCAGGACTCAATTCAGACTCCTCCAGTCTTGTCAAGTCATCCTGCCCCACCTCATTTGTTGCCTC
CACTCCAACATGTCCAACTGGTGGAGGAGATGTTTGGCCTTGGCTAACCTTTTCCCTGGACAGTCTTCCCAGGCCATTATGAGGTC
TAATGGTGGGCAGTCTTCTAATCCACAAAAATACACATCAGCAATAAAGATGTGAACAGAGGTCCAATGTGGCTTCTTCCCAAATT
CTGCTCTAATTATACAGGTTCCTTTAGTGGGGACACAATTTTTCCATCATGTACTATCGCTCCTAATTGTTTAATTTCATTCCCCA
TTAGGTGGAATCAGTTCCCTGCCAGATGCATTCTGTAGAGCCAGGCAGCCCCTTGTTTATTTGGTTCTTGGCCTGGGTACTTAAGG
AGAGACTGGGGCTCGGGTAAGAGAGAGGATAGGACTCCTCCTCTGAGCAGGAACAGACCCTTCCTTCTTGGCTCTCCCTTTTTTTC
TTTTCCATTTTTATTACTTTCTGGGTGGCTACAGGCAAAGCAGAGGGTAAGTTTTCCCAGTCCCTACCGTGGTGACACTGTGCTGC
CTTTTCTTCTGGAGAGCACCTATTCTCCTCTCCAGGGACCTCTTGTCCAAACTGTCTCTCCCAAGAGTTTGGGCTCTTACCTCTTC
TCCAGGTGGCATCCTGGTGATAAATTGCTGCCAGCAAGGTCAGCACATGCCTCATGGTTAGGAATCCTTCTTCGACCTGACAGCAA
CCTCCTGTAGGGTTCCGGGAGGGGTAGGAATATAAATTTCTTTGCCTTCAGTCACTTTAAGTACCACTCTAGCTAGCTCAGTCCAA
AGGTTGAGATATTAATATCCCTCTGGAGAGGGGATAGCATGATGACAACTGCAACATCATAATTGAGTGTAACAACAGTAGAGGCA
TGCTACCTGCTTGCAAAGGCCTTTCCACCAGCTGCCCTTAGGAGACCAGCCTTGGCCTGATGTCTCTGCCTTATTGGCTCATTTAC
TTGCCTATCTGTCTCCGGGGAGGAGGAAATCCTCACATCTCAGACTACCCCAAATCCTCAACCCAGCCACAGGGAGCACTTCCCAC
CATAGCCTGTTATGCAGTCCAGGGTGGTTCACCCTTATTCCAGAGGCATGCACTCCCTCTCCTTTTCTATTCAGGTACACAGGTGC
ACAATGTCCAATGTCAACTGAGGGGGGGACCCAGGTAAGTGGGCTGACCTCCTTCCCAAGTACCTTGGCATCTAAGACCCCAAAAG
TCAGTTCTAGTGGTGTGGGTTCACTCAACTACTGGTGCTACCTCTGGGCCAGGTCAATGAGGTATAGAAGGAGACTGCAAAAAGTA
TATTGTTGCGGTACAGCAATGAGCGATGCTAAGCAAAACTGCTACAGAGGCATTCTAAATGTAGAGTCTGAGCACTTAAGGCTGCC
TAGGACTGATTTTTCAATTGATTGTCCTACTGACTGGGTAGACGCTATGCCAATTTCTTGTTCACGAAAATCCCATCCTCCTCACC
ACTTGTGGTGAGTTCCAAGATTTCTGGGCTGATCAATGAAGAAGACATGACCACGAGGTGGCAGTAGCGCCCAAAAACATTTTTTT
TTTTTTTTGAGAAGGGAGAAGGAGTCTCACTCTGTTGTCCAAGCTGGAATACAGTGGCGCAACCTCTGCTCACTGCAACCTCCGCC
TCCCGGGCTCAAGTGATTCTCCAGCCTCAGCCTCCGAAGTAGCTGCGATTACAGGCTCCCGCCACAATGGCGGGCTAATTTTTGTA
TTTTTTAGTAGAGACGAGGTTTCACCCTATTGGCCAGGCTGGTCTCAAACAGCTGACCTCAGGTGATCCACCCGCCTCGGCCTTCC
AAAGTGCTGAGATTACAGGTGTGAGCCACCGCGCCCAGCCAAGAAGCTTTTTATTTCGGCGGTGCTTTGACAGGTTGCCAGAAAGG
GGAGGTCTCTCACATCAGACCTTCCAGAGGCAGCAGCAGAGGGCCACTGAGCAGAAGGGGAAGAAGGCAAGGTAACTCCTGGGGTA
GAGGAGAATAGGAGAGGGCCTTATGTGTCTGGGCGATATGACTCAGCAGCAGGAGAGGGAGTCTCTGGGTCAGAGGGTTCTGGAGG
CAAGCAGTGGCATGGGGTCTTTTATAGCCCCTGTGTTTTCTTTAAATGTGGCTAGCAGATGTCAGTGCAGTTTTGTGGGGCATGCA
AGGCAGGCAAGCCCTAAGTGGCTACACATATGCTTACTTGAGCTATATTTAAAGCAATTGCATATGTGAAAAATTCGAGTTTGGCG
TTGGCAGGCTTTCTGCCTGCTGTGAGGAAGTATAATGGCAATATAAAGGCTAATATATGGAGGTCATCTTTAACCCACTGATATAA
TAACATGCCACTTAGTCAGCCACTCTGATTAATCCGACTAGCATGGTCTGAGATAAAATCCATTCAATCTGTACTGTCAACAGTAC
TCTAAGCAGCAAGGTGAGATCAATCTGCAACGTTAACCCGAACTTAAAACGTTAAGCGCACACGTACAATACTACTGCTCCTGAGT
TTTTATCTAAGAGAAATGAAAGCATAGGTCCACATAAAGACTTGTATGTGAATGTTTATAGCAGCTTCAATTGTGCTAGTGAAAAA
CTGGAAACAATCCAAATTACCATACAATGAAATACTATACTATGACAAATAGTAATGAACTATTGATAGAAATAACAGCATGGGTG
AATCTGAAAATAACTACGCTGATTTTAAGAAGACAAAAAAGAGTATACAGTGTATAATTCTATTTATATAAAATTCTATAAAGCAC
AAACTAATCTATAGTGAGAGAAAGTAGATCAGTGGTTGCCTAGGGACAGGGATGGGATGGGGATGGGTAGGGAGGGAAGCAACATA
AAGGGGCATGAGAAAGCCTTAGGAGGTGATGAAAATGATCGTCATTTTGAATGTGGTAAGGGCTTATGATTATTGGTAAGGAGTTA
TGCTTATAAAATATAAATAATTTGTATTTTATAAATTATATAAATTACATATAATTTCATTTAGTTTTAAATTTAAGGAATGGCAA
TATGTCTTGCATCAAAGAACCCTCAAAACAACACCTTAAGTACTGCAAAGTCTTTGACGATGTATTTGCCAGTTAACTTATCTTTA
AACATCAATAATAAATAAAAATGGTCAAGACTTATTTGTTTCTATGTGTTTATTTTAGTGTGCTATTGCTTTTTCACATTCTGGGT
CATATTTTCAAAGTATTATTTCACATTGTGTAAATGTATTTTTTTTTCACTATTAGTAAAGAGCATCTCAAGTAAGGTCCTACTTT
CTACTTTGATGGTATAGGAGCATGTTTGACTCAGATATGAACCACTTGTGGACAGAGTTATGGGTGAGAAATCTGATATTTAAGGA
ACACTCTGGATTAGTTTCCACTGGTGAGAAAATAAAAAAGTGATGAGCACTTTTTCCAAATACCACGTGATGAATCAAGTACTCAA
ACATGCTAACTGCATTTAGACTATTCTATTACCAAAATATTCCAATCAATGCCTGTGGACAGTTTGTAAACTGTCATTCCCAATGC
CTCCTCATTCTTCAAGTTCCCACTGAAAGGTCTCATAGCTTATCAGACCTTTGGGGGAAGTGCTTTTTTTTTTTAATTAAAAGTTA
AGTCATGTGCAGGGCAGAGTGAGGCAAAATGCAGCCAGTGGTTAGGACCTAAAGACCTCTCCTTCACCTGGGCCTTGAGAGTTTGA
GAGAAGCTCAGGAAGAAAAGCCAACCATCTGAGTCCAGTGGGCTTTGAAGCAGTGGTCTTGCATCTGTACATGTGGATAAATGAT
GTGAAAACCATTAACTCTGCTGCTGATCCAGCTGCAGGGAAAAGCATTTCCTCTGATCAAGGTGAACATCTGGCTGGTGGACCTCA
TTTTGCACCAGGTCCTGATCGATGAGCTGAGCCTGAGTCCACAGTTCACCTGATGGAGAACTCAGAGTCAAAGCCCAGGCCCAAGT
GAGGCTGTCTGACTGCTGCTTGAGGGCCTAGGGACCACACCCCTGGAGTCAGGAGGGCAGAGGTGAGGATGACAGTGGAATGGCCA
GAAGGTAAAGATACATGTGAATATTCTATCTCCATTAGGTCACATGGATGAAAGGCAAGAGAAAAGTAGCATATACATTTTTGAGA
AGTTAAAGCTGATAGGACACAAAGATGGTTGATTGTTCTACAGACGTGGAAGGCAGGGGCTTAGGTCCTGTAAAGTCCCCCACTGA
GCAAATAACTGAGACAGTACTTCCTAGTGATGCATTCTGCTGTCAAACAGTGTGAAATTTTCCTGGAGAAGGCTCAGGCCATCAGT
CCCTGGTCCATCTTCTGGCCCCACAGAGATGCTGATCGAACACCCTCCACAGGGAGAGCATTGCACAAGTGATTCCCTTTTTAGAA
ATTCTTCTAAGCCAGTCAGAAGAAGACAAGCTTGCAGATGGAAGGGAATCAGAACCTGGGCAATATCTTGTAGTGGCCGAGCTATC
CCTGTGTGTGGTTTGGAGTGTTTTCACTAAGACAAAACAGTGGGAGAAAGAAAAACCCTACACCCATACAAACTTTGAGGGAATAC

TGGAGCTTATTGTAACAGACACTGGTCATGCTATGAAATTTTATTACCTTTTCTGGAGGGGTCGCAGGGCTTTGTGTTTGGGCTGC
TTTCCGTGGTGCAGTAATTGTCAAGCTTGCCAAAAGTGTGACAGAATCACGGAATTACCCTTGGCAGAAACGCAGAATTACTCCTA
ACCTCTACTCTCATCCTCCACCTGGACCAGACAGAGAAACAACTTCTTGAGTTTTCAGAAACTACCAAATCTTGGCTCTAGCTCCG
CATCCTTGGCCAGAGCATGGAAGGTTGGGGTGAGGGACACGGTGAACAAAAACTGCATCCTCGAGCTGCTCGCACTTCCTCTTTCC
CTCTTTGGTCTTGTGTGTGGGGTCAGAGGTTACCAGGCAGGGGTCACTAAGGTTGTCAGTGGGAGGATGTGAGCCCTAGTGGGTTC
CTCCCAGGCAGCTCAACATGGAGGCTCCCAATTCCCCTTTCTCCTGCAAGGAAAGATACCACAGCTGTGTCATTCTCCTTCAGAG
ACTGCAGATTGGAGGTCTGTTTTTCCACAAAACCTAGTTCTAAGACATTTCTAGCAAGTGAGAGTGACTGATTGGAAGTCTAGTGG
TGACAGCTACCCATGGGGCAGGCTTAAATAGCGACTCCTCCTTGTAGTGAACGAATTTCAGATTTCACACAAGAAGGGCAAAACCA
TGGGCTATTTTCTCCCATGAATTTTTGCCTATTTTGGGCCCTAAATCAAATTGAGAAAAGTGTGACTAAAACACTCAAACTGCCAA
TATAGTTAGAGATATACACTTTGTGAGACTAACTCGGGAATCTACTGCCCTGCTTCTGCAAGGATAAGGCTCTGTAAGGCATGACT
TGAAATGAAATCCAAGCACCTTTTATCTGTTTAACTCTGGTGCAGGAGAACAGAGCAGCATTTAAGGTATAAATTAAATTAGTCTA
TTCCTGAAGAGAACATTTGCTACTCACATTTATGGTGGAAGATGCAATCTCACAACACAACAAAGCTCTCCTTGTGAGTGGAGCAG
GCCAATACTGGGTAGGGCTAGACTATAGGGCTCACAGGCTCTATGCCCTAAGCTACTGATGCCCCTTTGTGCCAGAGGCTGGGTTT
TGCAACAAACTCAGTTCAGGTCTGGGAACACTGGGCCCACCGAGAATCCTAAATAGTCAACTAAAATCCCTGAAATACCCAGGCCT
GTTTCTTTAATCATTAGTATTGTTTGCATACCTTTAGGAGGTACAAGCGCAGTTTTGTTACATGGTTATATTACCCACTGGTGAAG
TGTAGGCTTACCAAAGCAAAAACAGGGTGGTAGATAATCTCCCTTGTGCAATCAACAACTATAACCCTGGCAGGTAGGGTGCCTAC
TATTCATACTGACTACAGAGAGCCTGGACTCCTCTAACCTGATAAAGGATATGAAGCCAGTTATCTATTTATTACCTCTTAGCTCC
AAATTCACCAGTCAATATATATTCTTCGATAATAGAGGGATTCTTTAAGCATCTCTCCTTTAAAGTAGCATCAAAGAGCACGATGT
TACACTTACTTAATAGAGGGCTCTGGAGGGACAGACATTGAAAAAGGAAGGGTACTTCTATGATTTCTGGCTCTCAGAGGTGTGGA
GGTAAGGACATTCAGGATGCTTTGTCCCAGGCCTGGGCCCAGAACAACTGTCCCTCAGTAAACCTGCAGTCCTGGTGTGCCCTGAT
GATCAGCTTCTTGTGGCCCTCTTGATAGGGACATCCTGTACTCCAAGTCATCTGCCCACTGCTTCCCTCTGCTCACTTGCTCCGCA
GCCACACACACACACACACACACACACACACACACACACACACGACCAGGTTCATGTAGTCCTTTCAATACTGCAGCCCTCTCAAC
ACCGAGCCCCCACCCTGATCCTGTGCAAGGGGCATTGTTTACTCCAGGCCTCCAACTGAGACAGGACTCCCAACCCCTACTTCACA
CCACATACTAGGCACCAGCTATGGCTTGTCCACACTACCATGCTCCAAAGGGTGCCTTCTTGCTTCCTCTGCGACCGTAGACCAGT
TCTGCCTCTGTAGACCAGCTTAGGTACTGGCAAACCAGTGGAACTTCTCTGCCATCCAGTAGGTTCTTCTCCAATTAGGTATAAAA
TCCATCCTTGGGGAGAAGCTTTCATTCTAAGCTTGTCTTTCCTTGGGTACTCTCTCCCAGCCCCAGAAAACCCCACATAGAGTTGT
CTTGTATCCTATACAAGATCTTTTATCATCGCTTAATGATTCTTTATATTAAATTTTCCCCTTCTAGATCACTGTGTGCTTTCTAT
CTCCTGATTGGATCCATATTGCTGCAGAACACAACATCTACAGCAAGCATCATTCTTAATGGAGAATCACTGAAAGCTTTCTCTTC
AAGAGCTACATCAAGTAAGGATATCCACTATCACCACTTCTATGCAATAGTTTACTGGGGGTAGGGGCGGGGTCCCAGGCAGAGCA
GTAGGGCAAGAAAAAGGAATAAGATATTTGGAAAGGAAGAAGAAAGACTGGAACTATTTTCAGATGATATGACAGTGTATATAAAA
TTCTACAAAGTATTACAGACAAATTATTAGAATCGATAGAAGAGTTTATATGCTTATAGGATAAAATCTTAACGAACAAAAGCCAA
TTGTATGCTATTTAGAGCTATAAATTTAGACAATAAAAATTTCAAACATATGATTTGCAAAATATTAAATGCCTAGGATAAAAAAG
TTGCCTTGTGGGAAAAATATAAAATAATATTTGGGCAAATTAAAGGTGATCTTATTAACAGAAGAGATAGACCATAACCACGAATC
GAACATTCAATATTGAGAAGTCAAATCTAAAATATCAGCACAATTCAAATTAGAACCCCAAACTAGCTGTTGGTTGAAGTTTTTAC
GATTAGTTTATAATTTAAATGGAATTGAAATGGACCAAAAGTAGCCAAGACACTTTTGAAGAGCAAGGTAGAATTGATTTGCCTAA
TCCAAATATCAGAATTGACTATGGATCTGTAGTAATTAAGACAGTGTGGTATTTCAAACAGACAAGTGCTTAATAGAGAGTATAGA
ACTAGCCCACATATGTGTAGATACTTGATTTATGGCACTGTAAATCAACTGGCAAAAGGCAGACTTTTCAATAAGTGGTTCACTGA
GAAAACTGGATATTCACTTACTTACATACATATATACATAAATGGAACTCTACTTAACACAATACACAAACATCAATTGCAGGTGG
ATTAAGACCTGACTATGAAGGACAATAGCAGACATATTTTAGAAGAAAATATAGAGAATGCTTTCATGGTCCAGGGTTCCTTAAAA
CAGAAAAGATTTCTTAAAACAAAAAAGCACTAACCATCAGGAAAAGAATGATGAAATTGACTATATTATAATTAATAGCTTCTGTT
CACAAACCGGAGCACAAAGCAAGTGAATATACAAACAGAGCAAGGGAGTATTTTGGCCGCAGGTGTGGAAGAGTATTTAAAATATT
TAACAGACTCATTAAGTCAGTATCAGATGAACAATAAAATAGAAACGTGGGTTAAAAGAGGTAAATTTGAATAGGCCTTCCTCAAA
ATAGAAACTCCAAAAGGCTCATTAACACAAAAATGTGCTAAAGCTTAATAGTAATTTGGATATTGCCCACCAGTAGACCGGCAAAA
TTTAAATTCGGGCAATACTTGGTGTAGGTGAACATGTGGATCAGTAAGGACTCTCTATGCTGCTGGTGGGATTGACAACTCTCCCA
ATCACTACAGAGAACAATTTGGCATCATCTAGTAAAGCTGAGGATATCCATTCCCTATGGCCCTGTGATTCTATTCTTGGATATAT
ACCCTAGAGAGATTTTTACACGTGTCCCATTAGAAATGTGCAAGAATGTTCACAATAATCATTGAAAAGTAGAAGCAATCCTAATC
TCCATCTGCTGTAGAATGGAGTGGAATAATCATACAATAATCACTTTGCTATAAGCATATGCTGGAAAGACAGTCGATGGAGACAA
AGGATCTACTTACTTATAGCTATTATGAACAACATGGACGAGTCTCTCAAGTATAATACAGAGTGAAAAAAGCAAGCTATAAAAGG
AAGCATGCCGTATGATTCCACTTATACAACTTCATATCAACTCTATTGTTTAGGGATGCGTACAAAAGTGATAAAGCTATAAAGGA
AAGCAGTGAAATGATTATTACAAAGTCGGAGCAATTACTATATCTATATAGGAGAGTGTGGTTTGTGACTGGAAGGAGTACAGCAG
CTGTATTCTGGGAGGTTGGCAGTGGTCTATTGCTTGACCTTGGTCCTGGTTAAGTAGGTGTCTGCTTTTGTAAAATAATCTAAACT
ATACAGATACGTTATATGCACTCTTCTAATTTCAGATTTGAAAAGTGCTTCCTCCAGAAAAAGTAATAGAAAGAAAAGTATGTGGA
GTAGAAATTTTGATGAGGTTCGAATAGTTATGGTTGAAAAACTTGATTTTCAAGAGACCAAGTGTCCACTGTTATTAAAAGAGGGC
GAGAGAGCTGGGAGTATTTAAACAACAGGTTACACCCCCCCACCCCCACCACCACCAATCTGTTCATCACTCTAAGCCTTCTCATC
TCAGATATTTCACCCTATATTTGTGTCAATTCCAAACTCATTCGTGACAGACAAATTGGAGGATTGTCGTCATCATTAATAAGGAC
ATTAGGCACCAAAGGCCCAGCAAGACTGGGCCACCACACTTAAATGTGAGCAGGTACACCGGGGCGAGTGGACAAGATCGTCTGTG
CACATTACTTGGCAGAGTCCATTGAGTGACTGAGGGTTTAACCTTAACACATTTAACTGGAACTCCTGCCCAGAGAATGTGCTGGA
GAATGCAGAATAAACTGAGCAATGCCCTCATTGTGCTATGAGAGTGGACATGAAGTTTATCATGGTACAACCGATCGTCGAAGGGC
AAAGAGTTGGTTTAGGTTGTGCACTGTATAGGGAGAAGCCAGGGGCGGGTTGGGTTTTTTTCCAGATTTCAGTATAAATTGTTTCA
TCCTCCAGACTTTGCATTTGTAGGCTGAAGAGTGCTAATCTGTTTTGGCCGCATCTCTCACTCATTCTAATCTCTGTTATCAGCAG
ACAAAAATTGAACTATGTGGCAAATTGTACCGAGCACTTTGCTCAAGACAATTGTGAGTATGGAAGTTGTCCCGCGCAGAGGTGGG
AAGGGGAAGGGCAATTTCCGCAGAAATGCCAGCACTATGGTGGTTTAATGCTGACACAGAGACAGTTTTTTCTCTCAGGAATTGTC
TTTCATCCATCAATCCAGCCAGGTAGCCAGCCAATAAACAAATTTTAACACACAAAAAGTATGAAGCAACATGTCTAACACTGGGA
GGGGCTGGCGTGTGTTTATTTGGGTATATACAATGAGTCACGGATTAAAATTGCTTTTACCAGATAGATACCTAGGTGTTCCACAG
CATTATTGAACAAGATGTCGTCATTTCCCCATTACTTCAGAAGACCTCCTTAATTACTTATATTAATTGTAGAATATATTTATGCA
TACCAGGCTCTACCTCTGGGCTTTCTATTCCGTTCCAGAGACCCAGTACCAGAATATTTAAATCACTGTGGCGTTGAATACATTTT
AATATCTGATAGGGCCAGTTTCTGCGCATTGCACATTTTTTTTTCCCCTTTCAGGAACGTGTTCAGGCCCGCGGCAGGGGGCGGGA
TCGCGCCTCCTCCTCGGCTCTGGTTCCAGCCGAGCCTCTCGGACGCAGAGATGGAAATCCCGAAGCTGCTCCCGGCTCGCGGGACA
CTACAGGGCGGCGGCGGCGGCGGTATCCCCGCGGGTGGCGGCCGAGTCCACCGAGGCCCTGACTCGCCGGCTGGCCAGGTCCCCAC
GCGCCGCCTCCTGCTGCCCCGGGGCCCCCAAGATGGCGGGCCCGGGCGGCGGCGCGAGGAGGCCAGCACGGCATCACGGGGCCCTG
GCCCAAGCCTGTTCGCGCCGAGGCCCCATCAACCTAGCGGCGGCGGCGACGACTTCTTCCTGGTGCTGCTTGACCCGGTGGGTGGC
GACGTGGAGACCGCGGGCTCCGGTCAGGCCGCAGGGCCTGTGTTGAGGGAGGAGGCCAAGGCGGGCCCGGGGCTCCAGGGGGACGA
GAGCGGCGCGAACCCCGCGGGCTGCTCTGCGCAGGGCCCCCACTGCCTGTCCGCGGTTCCCACTCCGGCCCCGATCTCCGCCCCCG
GCCCCGCCGCGGCCTTCGCGGGCACAGTCACTATCCACAACCAGGACCTGCTGTTGCGCTTTGAGAACGGCGTCCTCACCCTGGCC
ACGCCCCCACCACACGCCTGGAGCCAGGGGCCGCTCCTGCCCAGCAGCCCAGGTGTCTGATCGCCCCCCAAGCTGGGTTCCCGCA
AGCCGCGCACCCGGGTGACTGCCCAGAGCTGCGGTCCGACCTCCTGCTAGCCGAGCCCGCAGAACCCGCGCCTGCTCCGGCGCCCC

AGGAGGAGGCGGAGGGCCTGGCCGCCGCCCTGGGCCCCCGCGGACTGCTGGGCTCTGGTCCAGGCGTGGTGCTGTACCTGTGCCCC
GAGGCGCTGTGCGGGCAAACCTTCGCCAAGAAGCACCAGCTGAAGATGCACCTGCTGACGCACAGCAGCAGCCAGGGCCAGAGGCC
CTTCAAATGCCCCCTGGGTGGCTGCGGCTGGACCTTCACCACCTCTTACAAGCTCAAGAGGCACCTGCAGTCGCACGATAAACTGC
GGCCCTTCGGCTGCCCTGCGGAGGGCTGTGGCAAGAGCTTCACCACCGTGTACAACCTCAAGGCGCACATGAAGGGCCATGAGCAG
GAGAACTCGTTCAAATGTGAGGTGTGCGAGGAGAGCTTCCCCACGCAGGCCAAACTCGGCGCCCACCAGCGCAGCCACTTCGAACC
CGAGAGGCCTTACCAGTGCGCGTTTTCTGGCTGCAAGAAGACATTTATCACAGTGAGTGCTCTGTTTTCCCATAACCGCGCCCATT
TCAGGGAACAGGAACTGTTTTCCTGCTCTTTCCCTGGCTGCAGCAAGCAATATGACAAGGCTTGTAGGCTGAAAATTCACCTGCGG
AGTCACACCGGCGAGAGACCTTTCCTTTGTGACTTTGATGGCTGTGGCTGGAACTTCACCAGCATGTCCAAACTCTTAAGGCACAA
AAGGAAGCACGACGATGACCGGAGGTTCATGTGCCCTGTGGAAGGCTGTGGGAAATCTTTCACGAGGGCCGAACATCTGAAAGGCC
ACAGCATTACCCACCTGGGCACAAAGCCTTTCGTGTGTCCTGTGGCAGGCTGCTGTGCCAGGTTCTCTGCTCGCAGTAGCCTCTAC
ATTCACTCCAAGAAACACCTGCAGGATGTGGACACTTGGAAAAGCCGTTGCCCGATCTCCTCTTGTAATAAACTCTTCACATCCAA
GCACAGCATGAAGACGCACATGGTTAAAAGGCATAAGGTGGGCCAGGATCTCTTAGCTCAGCTAGAAGCAGCAAATTCTCTCACAC
CCAGCAGTGAACTTACCAGCCAGAGACAGAATGATCTCAGTGATGCAGAGATAGTGTCTCTCTTCTCTGATGTACCTGACAGTACT
TCTGCTGCATTGCTGGACACAGCATTGGTGAACTCTGGAATCTTGACTATTGATGTGGCTTCTGTGAGCTCGACTCTGGCAGGGCA
CCTCCCTGCTAATAATAATAATTCCGTAGGGCAGGCTGTGGACCCTCCGTCCTTGATGGCCACCAGCGACCCTCCTCAAAGTCTGG
ATACCTCTCTCTTTTTTGGAACGGCGGCCACTGGTTTTCAGCAGAGCTCCTTAAATATGGATGAGGTCTCAAGTGTAAGTGTGGGG
CCATTGGGATCTCTGGACTCTTTGGCCATGAAAAACTCCAGTCCAGAGCCTCAGGCTTTGACACCCAGCAGTAAGCTAACAGTGGA
CACAGATACTCTGACTCCTTCGAGCACCCTTTGTGAAAACAGTGTCTCAGAACTACTGACACCAACCAAAGCGGAGTGGAACGTAC
ATCCTAACTCTGACTTCTTTGGACAGGAGGGAGAAACCCAGTTTGGATTCCCCAATGCAGCAGGAAACCATGGTTCTCAGAAAGAA
AGAAATCTTATCACTGTGACTGGCAGCTCATTTTTGGTATGAAGCAACTCTATTCATTCCTTGCCATGTGGCTAACTTTTATTACA
GTCAATTTTGAGGATATTCTGGACTAAATATTTAAGTGCAGTCATTTCTTTTTGGTTTGCAAAAAGAGCACAGCCCTGGACTATGA
GTTTGGAGATCTAAATTCTGATCTTGAGTCTGGAACTGACAAGTTGTGTGACCCTCAGCAAGTCACTTAACCTATCTGAGCCTTAA
TTTCCTTATTTATAAATTGTGGTGGTTTGAACACATTGCTCATAAGGTCTTTTCAGTTTTGTTTTGTTTTGTTTTGTGATTTTGTG
CTTTTTCTTGAAAATTTTCGGGCATTTTGCAATTATTATTGTTTGTACTTGTAATCAGAAGCGGTTTGAGCCCTGTAGCACTAAAT
AATGAAAGATTGAGGAACTGGTGTTTTACATTAAAAATTTAATGGAAATTTTACACAGTACGAAAATCTAATGATAGAGCTCAAAA
AAAAAGAGCTAAAAATAGGAGTTGGTTCTTCTTAGCTGTTTATCCTTCTAACCTTTTTTTAAATGATGAAGGTATGTTTTTGTTG
TGGAAAATACAAGGGCTTTTGTTATCACTCAGACCCAGAGTGAAATGTTTGCTTTGTGGTTTTGAATAAGTTGGCCTTTAATAAGT
TATTTAGTCTCTCTTAGCCTCAGTTTTCTATCTGTAAAATGGTCATAGCAATGTGAACATATGTGTTACATCCTAGACTTATTTTT
CTACCCCAGTAGGTTGTATTGAAAGGAAAATGTTATATGTGTTTCAGCATGTTTTGGTGAATCTTCATTCCCTTTCCTGCCCCTTG
TTTTCCCTCCTCAAAGGGGAGAAATTAGCACAAATTAGTATCAGGATTGTGCAGGAAATAAACATTTGTGAAGGTTAAGAAAGAGG
AAAAGGAAGTTATTTCTTCAACAGATTAAGGATTTTTCTACACACAGTTCTTTTGTGGCATTGGCCACATGTCCATTAGACCAATT
TGATAGTATCTTCGGTCTGCATTCAAAGCCAGCTCATGCAATGAGTATTCAGCCTATTCTTCCAAGACAATCTGGACATAGACCAG
AGGGAGATTTTTCTCCCCTCTGTGCCCTAGAGAGCTCATTGCTGGCTTACTCTTAGAGTTGAAATGAGAGGGTTTTG

HUMAN SEQUENCE - mRNA (SEQ ID NO: 23)

CTGCTCGCGGCCGCCACCGCCGGGCCCCGGCCGTCCCTGGCTCCCCTCCTGCCTCGAGAAGGGCAGGGCTTCTCAGAGGCTTGGCG
GGAAAAAAGAACGGAGGGAGGGATCGCGCTGAGTATAAAAGCCGGTTTTCGGGGCTTTATCTAACTCGCTGTAGTAATTCCAGCGA
GAGGCAGAGGGAGCGAGCGGGCGGCCGGCTAGGGTGGAAGAGCCGGGCGAGCAGAGCTGCGCTGCGGGCGTCCTGGGAAGGGAGAT
CCGGAGCGAATAGGGGGCTTCGCCTCTGGCCCAGCCCTCCCGCTTGATCCCCCAGGCCAGCGGTCCGCAACCCTTGCCGCATCCAC
GAAACTTTGCCCATAGCAGCGGGCGGGCACTTTGCACTGGAACTTACAACACCCGAGCAAGGACGCGACTCTCCCGACGCGGGGAG
GCTATTCTGCCCATTTGGGGACACTTCCCCGCCGCTGCCAGGACCCGCTTCTCTGAAAGGCTCTCCTTGCAGCTGCTTAGACGCTG
GATTTTTTTCGGGTAGTGGAAAACCAGCAGCCTCCCGCGACGATGCCCCTCAACGTTAGCTTCACCAACAGGAACTATGACCTCGA
CTACGACTCGGTGCAGCCGTATTTCTACTGCGACGAGGAGGAGAACTTCTACCAGCAGCAGCAGCAGAGCGAGCTGCAGCCCCCGG
CGCCCAGCGAGGATATCTGGAAGAAATTCGAGCTGCTGCCCACCCCGCCCCTGTCCCCTAGCCGCCGCTCCGGGCTCTGCTCGCCC
TCCTACGTTGCGGTCACACCCTTCTCCCTTCGGGGAGACAACGACGGCGGTGGCGGGAGCTTCTCCACGGCCGACCAGCTGGAGAT
GGTGACCGAGCTGCTGGGAGGAGACATGGTGAACCAGAGTTTCATCTGCGACCCGGACGACGAGACCTTCATCAAAAACATCATCA
TCCAGGACTGTATGTGGAGCGGCTTCTCGGCCGCCGCCAAGCTCGTCTCAGAGAAGCTGGCCTCCTACCAGGCTGCGCGCAAAGAC
AGCGGCAGCCCGAACCCCGCCCGCGGCCACAGCGTCTGCTCCACCTCCAGCTTGTACCTGCAGGATCTGAGCGCCGCCGCCTCAGA
GTGCATCGACCCCTCGGTGGTCTTCCCCTACCCTCTCAACGACAGCAGCTCGCCCAAGTCCTGCGCCTCGCAAGACTCCAGCGCCT
TCTCTCCGTCCTCGGATTCTCTGCTCTCCTCGACGGAGTCCTCCCCGCAGGGCAGCCCCGAGCCCCTGGTGCTCCATGAGGAGACA
CCGCCCACCACCAGCAGCGACTCTGAGGAGGAACAAGAAGATGAGGAAGAAATCGATGTTGTTTCTGTGGAAAAGAGGCAGGCTCC
TGGCAAAAGGTCAGAGTCTGGATCACCTTCTGCTGGAGGCCACAGCAAACCTCCTCACAGCCCACTGGTCCTCAAGAGGTGCCACG
TCTCCACACATCAGCACAACTACGCAGCGCCTCCCTCCACTCGGAAGGACTATCCTGCTGCCAAGAGGGTCAAGTTGGACAGTGTC
AGAGTCCTGAGACAGATCAGCAACAACCGAAAATGCACCAGCCCCAGGTCCTCGGACACCGAGGAGAATGTCAAGAGGCGAACACA
CAACGTCTTGGAGCGCCAGAGGAGGAACGAGCTAAAACGGAGCTTTTTTGCCCTGCGTGACCAGATCCCGGAGTTGGAAAACAATG
AAAAGGCCCCCAAGGTAGTTATCCTTAAAAAAGCCACAGCATACATCCTGTCCGTCCAAGCAGAGGAGCAAAAGCTCATTTCTGAA
GAGGACTTGTTGCGGAAACGACGAGAACAGTTGAAACACAAACTTGAACAGCTACGGAACTCTTGTGCGTAAGGAAAAGTAAGGAA
AACGATTCCTTCTAACAGAAATGTCCTGAGCAATCACCTATGAACTTGTTTCAAATGCATGATCAAATGCAACCTCACAACCTTGG
CTGAGTCTTGAGACTGAAAGATTTAGCCATAATGTAAACTGCCTCAAATTGGACTTTGGGCATAAAAGAACTTTTTTATGCTTACC
ATCTTTTTTTTTTCTTTAACAGATTTGTATTTAAGAATTGTTTTTAAAAAATTTTAA

HUMAN SEQUENCE — CODING (SEQ ID NO: 24)

ATGCCCCTCAACGTTAGCTTCACCAACAGGAACTATGACCTCGACTACGACTCGGTGCAGCCGTATTTCTACTGCGACGAGGAGGA
GAACTTCTACCAGCAGCAGCAGCAGAGCGAGCTGCAGCCCCCGGCGCCCAGCGAGGATATCTGGAAGAAATTCGAGCTGCTGCCCA
CCCCGCCCCTGTCCCCTAGCCGCCGCTCCGGGCTCTGCTCGCCCTCCTACGTTGCGGTCACACCCTTCTCCCTTCGGGGAGACAAC
GACGGCGGTGGCGGGAGCTTCTCCACGGCCGACCAGCTGGAGATGGTGACCGAGCTGCTGGGAGGAGACATGGTGAACCAGAGTTT
CATCTGCGACCCGGACGACGAGACCTTCATCAAAAACATCATCATCCAGGACTGTATGTGGAGCGGCTTCTCGGCCGCCGCCAAGC
TCGTCTCAGAGAAGCTGGCCTCCTACCAGGCTGCGCGCAAAGACAGCGGCAGCCCGAACCCCGCCCGCGGCCACAGCGTCTGCTCC
ACCTCCAGCTTGTACCTGCAGGATCTGAGCGCCGCCGCCTCAGAGTGCATCGACCCCTCGGTGGTCTTCCCCTACCCTCTCAACGA
CAGCAGCTCGCCCAAGTCCTGCGCCTCGCAAGACTCCAGCGCCTTCTCTCCGTCCTCGGATTCTCTGCTCTCCTCGACGGAGTCCT
CCCCGCAGGGCAGCCCCGAGCCCCTGGTGCTCCATGAGGAGACACCGCCCACCACCAGCAGCGACTCTGAGGAGGAACAAGAAGAT
GAGGAAGAAATCGATGTTGTTTCTGTGGAAAAGAGGCAGGCTCCTGGCAAAAGGTCAGAGTCTGGATCACCTTCTGCTGGAGGCCA
CAGCAAACCTCCTCACAGCCCACTGGTCCTCAAGAGGTGCCACGTCTCCACACATCAGCACAACTACGCAGCGCCTCCCTCCACTC
GGAAGGACTATCCTGCTGCCAAGAGGGTCAAGTTGGACAGTGTCAGAGTCCTGAGACAGATCAGCAACAACCGAAAATGCACCAGC
CCCAGGTCCTCGGACACCGAGGAGAATGTCAAGAGGCGAACACACAACGTCTTGGAGCGCCAGAGGAGGAACGAGCTAAAACGGAG
CTTTTTTGCCCTGCGTGACCAGATCCCGGAGTTGGAAAACAATGAAAAGGCCCCCAAGGTAGTTATCCTTAAAAAAGCCACAGCAT

ACATCCTGTCCGTCCAAGCAGAGGAGCAAAAGCTCATTTCTGAAGAGGACTTGTTGCGGAAACGACGAGAACAGTTGAAACACAAA
CTTGAAACAGCTACGGAACTCTTGTGCGTAA

Table 5 (mouse gene: Nfkb1; human gene NFKB1)

Mouse genomic sequence (SEQ ID NO: 25)

Mouse mRNA sequence (SEQ ID NO: 26)

Mouse coding sequence (SEQ ID NO: 27)

Human genomic sequence (SEQ ID NO: 28)

Human mRNA sequence (SEQ ID NO: 29)

Human coding sequence (SEQ ID NO: 30)

MOUSE SEQUENCE - GENOMIC (SEQ ID NO: 25)

TCTTCTGAATATCATGTTTAGATTATGTAAGTATTCACCGGCTTTCTGTCACTATAAACCAATACCTGAGTTGATTAATGAATAAA
GAAAAAAAGGTTTAGTTGATGAGTTTTGAAGGTTCCAGTCTACCAGTCTGTTACTGCAGACGTCCACCGTGATAGTATGTCATGGC
CAGAGCACCTGGCAAAGCATAAACTACCTGCTCGTCAATCAGGAAGCAAATAGAAAAGAGAACTGGTTGCGGTCCCACAACATCCT
TCCAGGTCATGTCTTAGTAGAGCACTTCTCCCAGGAGAAGCACCCTGTTGGCCTTGCCGTCAACACATGAACAGCTTGGGGACTCC
TACTGAGGTAACAGTACCCATTCTGCATACATTCCCTGAGATTAACTGAGACTTGATTCATGGCCCGCTGTTTTGTCTGCCTTTGT
GAGTGTTCTGCTTGCAGCCCTACTAAACACAGATGGCCAATGTGGTATTCAGAGTAGTGGCCTTTAAGAGGTTATGAGGTCATAG
CACATACCCTGCCCCTGCCATGAATAGGATTTTAAAGGGGGCTGATGAAGGAGGGTTGTCCCCTCTGACTCTTCCACCTTTACTGT
GTGACAATAGAGTGATTGTTTCCTCGTGACATTCCTCATTTGCGTGCTTTGACCTTGCACTTCTTGCCTCCAGAGTGATAAAAAAT
AAACTTATGTTTTTTATAAACTACCCAGACTCAAGTACTGTTTAACAGTACAGACATATGAAGACAGTTGGTCGTGTTTGTAGTCA
GCACCGAAAGAGGAAAGTTGAAAACTTCCACTCTACTTGTGGATTTCTGTCTCTTCCACAAATGTTTAGGTCTACTACTTTGTACT
TCATGTATTTTGGAGACCTATTAATAAGTGCATGCAGGTGGAGGTCATTATTTTTATAAGGGCTCAGTAGTGCATACATATAAGGT
TCCAACACTCAGCATCTAGAGGCAGGAGGGTTGCTGGGAGTTTGAGCCCAGCCAGAGCTATCTATAGAGACCCTGTCATAAAACAA
CAAAATTATTACTGCCCATTGATGACCTGGCCCCTTTATTCTTATATATATATCCTTCTTTGCCCTTAGTAATATTCCACCCCCTG
ATCTTCTCTATTGGTGGTTATGATATAAACTACTTTGCTGTGTTTAGTATTTGTATTATTAGCACAGTATCCCAGGCTTCTGCTTT
TAAACACTTTGCGTCTTTCATGTTAAAAATAATTCATAGGAAAAGCATTTTCTTGCGTCTTGCTTTTTCTGTACTGTTTACCATGA
ATGCTTTTTATAGCTGAGTTTATTTTATTTGCATTTAATATTGGACCTCTTCTTTATCCACATTTTGTCTTTCATTGTCATCTTTT
AAATGAAATAAACGTAGCACTTGAAGAGTTCCATTTTATCTCCCGTCTGGTAGTTCTGTAAAACGGTGGCTTTCTGTAGTAGGACT
TTCTGCATCCTGCGTCTATAGCGAGTGAAGCGCTATGTACCTAATGTGGAAGCTTTGATTTGTTCTCACCTCTCTGCTAGTGCTGG
TAGACTTTTTTCCCTCCATGGGTTCAGAATACTCCATAATATATTGTGTTGTTCAATGTGTTTGTTGTACAGTAAGTTTGAACATA
CTGCAAAGCGAAAGCCCCATGTGCCCACACCTTTATCACCAGTAGTGTTTTCCTTGTTTCTTAAAGGTCCAATTCTATCTCATTTC
CCTTCACCACAAAGATCTGCTTTTAGGATGTCTTCTGTCACTTCTACGCGTTATGAATTCTCTCAGCTCTGCTTTATTAGAAAGTG
TCAGTTTACCTTTATTGTCAAACCATTCTGGGACAAAGCTACCTGCTTCTTTGTGGCTTCAATGTTTTTGTTGTTCATTATAGATA
TCATGATGTGAAGAGTTTGCATGTTTTTCTTTTATTCAGAGTTGAGTTTTGTCCTAGTAGGCCTTACTAAGATGCATTTAAAGTCG
CCCTTATTTTATGATGTGTTCTTGACTCTTATAACACGGTTTTCCCCTAGACTGCTATAAGCACACATGTTCAGAACGTTTACTGA
TGTCTCTTCAGCCCACGTGATTGCACCCTCAGTGTCTCTCAGCTCTGTATGGCGTCTGTAATCTTATCAGAGCATTTATAGAACAA
CAGTGGCTTTCACTGAGCTTTTTGGGATCTCTGTTAGGATTTCATCAGAGTAAAGGTGGGCTGATTATAGATTTTTGAGGCTTTGC
ACATATACCATTTCTCTGACCAATGAGATCACTGCTCTTTATTTGAACTCTGCTTCCTGTGGCCTGGATTAGAAAGTGCCCTCAGT
CAGAAGGTGAGCTTGGAGTTCACTTAAACTGTGCAGCCTGTATCTCTTGTTTAGTGTCTCAGGCTATTGTGTTTCATAATTACATA
TGAATTTGGCAAGATCAAATAGTGGTTGATTTATAATGACTGAAATTAGATTTGTGTGGATTTTTAAAGATTTTTTTTTCAAGAC
AGGGTTTCTCTGTGTAGCCCTGGCTGTCCTGGAACCCACTCTGTAGACCAGGCTGATCTTGAACTCAGATCTACCTGCCTCTGCCT
CTGGAGTGCTAGGATTAAAGGCATGCTCCACCACTGCCCAGCTGTAGCATGCTATTAATGATTTTGAAAGACATTATTCCAGGGCA
TTTATATATTCAATATGTTGGCTAGCTAGCATTTCTCTACAGCTTTGTTTTGAAACTTTCTCATCATCCCAGAAGAGTGCCTTGAA
TGTGGTAAATGATGGCTCCCTTCCCAGTCTTCACTCACTCCCCTGGCCTCTGGCAAGCAATGATGTGTTCCTCTTCCTACGAATGT
GCTTGTTCTTGGTATTCAATGTGAAGGGAATTGAGTATAGGTGAGCGACCCTTTCTGTCTGGTTTCAGTATAAATCATTTAGTCTA
CTGGTTTTGAAATACAAAATCAAAACTCCCATTGCTGGCTGTAAGGCACACCATTTAAGGAGACTGTAAATATATAATGTATGATT
AAGTTGCATAGAAACAGTGAACCATAGTGCAAAACCACCAGGAAATGTTATAGACCTTATGGACTGAGGCGATAACAGTGGAAGAG
AAAATACAGGTTTTATGGAAGAAGTTGATTTTGAAGTACCATGTTGTGGAATACATAATATCTAAACAGGATAGAATCTCGAGTGT
GAAGAGATGTACACACTGGAAAGAAATGACATAGAGCTGTTACATGTGTGTGTGTACACATGCGTGTGTGCGTGTATGCAGTGAGC
ATTCACATGTGTAGAGGTCAAAGGACAATTTTGGAAGCTGGTTCTTTCCTTCGAGCATGTAAGTTCCAAGTAAGGACTTAGGTCCT
TGGGCTTAGCAGCAAGTGTTTTTACCTGCAGAGCCCTCTTGTTGGCCAAGCAATGCATCTTTAAATGATTGATTTCCCTATAGTAA
AGTGCTTGTGTTGGATCATGGTGATAGTTGTGGTGCTTATATAATATATAATATAATATAATCATATGCTGAGTAGTTGCTCTTTG
GACTTCACTATGTAAAGCACCTGGCTGTGATCTGAAAGATATTTAATCAGGAAGATACCCTGCCATGGGGGTGAGCGGTAGAAAAG
CTGTTTCTGTCAGAGTGAGGTAATAAGGTCTGCCCTAGGGTGTGGCTATGGACATAGAAAGGAAGTGCACACACAAGAAACAGTGT
GAGGAAAGAAGTGAGCGACAACAAGACTGTTCACTGCATTGGTTGGAGAAGGCTACATTTGCAAGAATGAGACAGTCAGGAGTCGG
GCTTGTTGTCGAGTAGTAAACTGTAGACATTAGGGTTGATGGGTTTAATGTATCATTAACTTGTTTTCTAATGGCATCGTTACCTA
AAGCTAACAGGGGCTCATTACCAAATACACCTTCAGAGCCTTAACAGTTCTAATCATGTACTGATGTCACGGGGGTTGGGCTCGTA
TCTTGGATAGCTTGGGTTTTTTGTTTTGTTTTGTTTCTTTTTGCTCTTTCCTTTTTTTAAAAAAAATTATTTATTTTTTGTTCAAT
TGAGAACTCAGTAGCCGATGGAGAAAAGCCAGTGCAAAGTCCAGACCGTGACTAAGAGTGTAATTAAAGCTGTGATACTGAATTGC
CAGGGTAATTAACCCATGCTCCACACGCCCTCTGTTCCCATGACACTGACCAAGCTGTATCGTGTAGGCTGCTGTCTTTCCATTTA
CTAACCGTTCTGATTGGCTAGCCTTTGCTGCGGCGAGATAAATCATGAAGAAGCACATAGAAACACAGATATGCCTCATTTCTTGC
GACCTGTGACTGTGTGTGGTGATTTGTCTGGCTGCTCTGTGGAGGTCCTCAGCTGCTCTGCCACCGAGGGCTCAGCCTGGGAAGTC
TTGGCTGAGCTCTGCTGCGTTTCCACTCCACACGTGGGTTCTCATCCTCCACTGGGCTGGCCAGGCCTTTCCCTTCCAGGAAAGAA
AATCCAGTCATTGAACGGCTTCTTGAAAGCCATGTTCATGACTCATGTCTGCATCCTCCTGTTGGTCAGGGCCTGCCAAGGGCCTG
ACAACATTCAAGGGGCAGAGATGTCAATTTTATTGTCCCTGTGTTGTGAGGGATAGAAAAGGGCAGTGGCCATTGTTTTGAGCCTA

CTACAGCTATAAGCCCTGAGTTCAGACTATGCCACTCTTGTGTAGTTTTGTTTCTTTGGAAACTGACAGGGTAGGCAAACCTCTTT
CTGATGGGTTGATGAAGGGCCAAAAACATCCAGGTAACATTTCAAACATGGTTTCCCTGTGATACAGACCATGGCATACACAAGTA
TCACAAATCTGTTCCTACAGCACTGTGGATGACACACACACACACTCTCTCTCTCTTTCTCTCTCTCTCTCTCTCTCTCTCTCT
CTCACACACACACACACACACATGTTTTAATTTTTAGTTTAGCTAACTGTGGCTTTTGTGAGATATTCTCTCTCTCTCTCTCTC
TCTCTCTCTCTCTCTCTCTCTCTCTCTCTCTCTCTTTCTCTCTCTCTCTTTCTCTCTCTCCCTTCACGTTTGGATTGCCTTG
TAAGTAGCTCATCTGAAATTCATGCACTGTAAGGTCCTATTCTGTTCTCTGGGTTACTCTGTAGAAGAGTTCTATGGGCTCACCAG
GTACTAGATGTAGGAACAGAAAGCACACTCGGGAGAAGGCATGTTGCAGAAACGAGAGAAACATGCTGAGACTCCTAATGTGCATC
TCCCCAGTGGAGACAACACAATTTGACTGGACAGTGTGTGCATGAAGGCTAAAGAGCGCTCAGGGAATATAGAGGGAGCCTCTTCC
CGCTCCAGTCACCGTGACAGCATGCTACTTCGTGTGTTGGCATGCACAAAATTGGCAGAACGCCTCAAGTGTGGAGAATTCACCAT
TATAACAAGATTATAGTAAGCAAAAAATCACTCCACATCACGTAGAAGAGGGAGTTGGCAAAGGCGGCTGGTTTAAAGCAGCTCTT
TGCAATCTTCTCTAGAGGGTCTTATCTGAAAGCAAACAAAAAACAACAGGAACAGCTTGAATGTTAGCAGTTATCCTGGCAGGCAG
CACTCCAACCCAGCCCAGCCCACCCAGCCCCCAGCACAGGTGTGTAACCCTTTAGTAGCCTACTTAATATGGCTGAAGAGATAGAT
GGCTTAACTAGTAAGAGTGCTTACTGCTCTTGCAGAGGACTCAGGCTGGTTCCTAACTCCCACATGATGACTGACAACCTCCTGTC
ACGCTGGTTCCAGGGGATCTGGCACTCCCCTCCTTGCCTCTGCCAGTACCCTTACATTTGTGGTGCACAGTCATGCTTGCAGGCAA
AACACTCAAGCCACATAAAATAAAAATTAATAAAACCTTGAAAAAAAGATCCTAGCTAGCAGTTAATATCATGAGTAGACTAGCTC
CAGCCAGAACTTTTGAGAGGCTTGAAGCAAAGATGTTTTTAAATGAATAATACTTCTGTCAAGAGGAAAATCACATCACCCATTAC
GCTAGTACAAGGGCCATAAGCTGTGCAGTTAGAGAGGCCAGCTCTAGTCTGTCAGCCTGAGCAAATGACTCATTCTCTCTGAAGCT
TGGCTCTTCAGGTGGACTCTACTTTCTGGGCTCAGCATAGCAAGAGGGCAGCATACGTAAGCATTACCTCCGGTTCCTTAGTTATG
TGGAAGCAGTCTGTCAAGCTTAGCTTTCCCCATACAAAGGAGAGCACTTGTCCTGAGGCCAGAGCACTCACTGGCTTCTCCTCCTC
AGCGTAACTGGCACCAGGGATTAGGTAAATTGTCCCTGGGTTAATCCTGAGCCTTCCAGGATCTTTGGATCACTTCTAGAAGATGG
TAGGACCAGCCATCCAGTCCTGTGAGCCCAGAATACTTCAGAGAATAACTTCTAAACTCCCCCTCAGGGAAAAATACAAGTTATAG
GGTAATGGCATGCAACCACTTCAGACTCAAATATGTTAGACTGTCAGTAGCTATTGGCAGAAAATTTATCTGTCTTTGCTACAGTT
CTATTGCTGTGAAGAGACATCATGACCAAGGCAACTCTTCTAAAAGAAAGCCTTTAAATGGGCCTGGCGTACAATTGTAGACGTTT
AGTCCATTATCATGATGGCAGAGAGCAGAGGACACAGGCAGGCACAGAATCAGGAGCTGAGAGTTCTGCATCATGGTCTGTAGGCA
GAGAAAGACAGACTCTGGACTTGGGATGGGCTTTTGAAACCTCAGTGCTCCTCCTCAGTGACAGACTTCCTCTAACAAGGCCACAC
TTCGTAATCCTTTTAATCCTTTCAAATAGTGTCACTCCCTGGTGACTAAGCACACAAATATATGAGCCTGTGTCTTTCTCAGGACA
ACGAAAAGTTTATCTTGCATCTTTCTTCCTATAATTTATCATGAGTGAGCTATTAGACTGTCTGGTGAGTTAACATATGTTCTGTA
TCTCCCACTCACTAATAAGCTTCGCATGGATTAGCTAGCTGAGTGTTTCCCATGGGTCTGCCAGCCCATTAGGCTGACACTCCATC
ATGAATGGGTTTCTTCGTCAGCTCTCAGATTTGCATTTGCAGTGCGTGCATGTATTGTTCTAGGTCATTGTCACGATGCTCCTGGT
GAAATAATATTGAAAATAAAAGAAGTGGGAAACCAGGTTCTGTAAATCTCAGTGTGGTAGCATGCACAGAAACATTGATTTATTTT
TAAAAGAAAGCTTTTAGAAATCAGCATTACTGAAAAAAAAAAAGTCCATTGCCAGCCTTTATCATTAGTGTGACATACTGTCAGAC
AGGAGGACACACAGAAAGGGGGACGCTTCTGAAGAGCAGTCAGGAATAATTAGGAGTGTTGACGAATTCATACTTCATATTTTATT
TAACACTGTCAGGGCATTTTAGGTTCAATGATTTAAATTTAGATTCAGCCCCCTTTTTATTCTAATACATGTCTAATGTGTATTAG
ATAAATTTCCATGTCACTGTACAAATCTCCCCATTGTTATAGCAATTCCATAATATTTAACTTACAGTTGTAGAACAAGAATGCAA
ACAGTTAGATCAGAGCACATCACAAACTCCTGAAAATCGACTTGCAGAATTTACGCCCTCTGAATATTTTACATGGTATACACTTT
ATGTGGCCATAGCCCTTACATTATGTTTTCTTCAAAAAATTCTCTATGGAAAAGAAACTTTAACATTTTATAATTTAATCTTTAGC
AATTTATTGTGCGTGCAAGATACAAAACAAAAAATGTTGTGAAAAGTCTCTTTTCCCAGTATCTTTGCCCCATTGCTAAACAAAAC
AGTACATTAAATGACACCTAAGTGACACCTGTTATGGTTACTAGAATTTTAAACCACCCTGCCACCCCACCCCCTCCCTGATTTCC
AAGTGCACCTTCTAGCTGGTGTCTGACCGCCTACTTTTCATAGTGCGCATTGTGGTTCTCTTTGGATTATGGTATTTTAATGGGTT
TGGAAATTTAGAGAGCCTTTTCCCCCAAAGAAATCGAACAGAACAGAAAGAGTTGTTGGTGTTGGTGGTGTTGTTGTTGGTGGTGC
TTACAGTGAGTCAGGCCTCGGCTGCCTATATCTCTGAACCTAGATGCTATATAGTTACTGACAGGAATTCTCCTGACTTTGTCCTT
GATGGCATACAGCCATGCCTTTAGAATTTCTCCACTCAGTCCAGCTGCTTCACCAAGATGCCCCTGTGTCAGATTGAGTTACTAGG
AAGATTGCCACAGGAATGCAGGAAACAGTTCGTTCAGCATTCATAGAATATGTCACATACTCAGGACTTGGCTGGTTTTGTTACAT
TGTGCTTTGTATGAAAACCACAACTCCCCGGATATTTGCTGAGACCTAGCTACATTTTCTGCGTCTCCTTATGTGGTGAGAAGATC
CCTAAAGATTCCTGGTTGGGGTAGACAAATGCATAAATGAGACCCAGGCTTGTGTCTCCCTTTGTGTCTCAAGGTCCTTCCTCAAA
AGCGCAAAAGGAAAGTGGGGGGAAGGCTGAGAAAGCAGGGGTACATAAGGGACTTTCCCACTGCTTTCTCATCCCCAGCTCCAAAG
CTTAGTCGATATTTCAACATTTTAGGTATAAACTTGCTTTTGCAATCAATGCAAATATTACTCAGTGATTCATCCTTTCCAAGTGA
CTCTCGGTTTCTTTCTTTTGACATTTAATTTAAAATTAGAAATTAGCATTTATATCATGCTAGAGCTGCACTTACCTTTCAACGCC
TCAGTAATGGCCAAGGCTTTATGCGTCAAGATTCTAAGGCACCCGAGTGTTCCTTGAGCTTCTGTAGTACCTGACGGAGTTAAGTT
TTTAGCCACATATAATCTAGAAACCGTAGGAGAGGGTGCCATGATGACAAGGTGCCCAGTATGGTCGTTTGTCAGACATTTAAAGT
GGAGCCGTCTTCATTAGGGCCTCATGTAGGGAATGGACCAGGAGGATACCCTTACGTCTGTGAAGTGCGGTTGGAGTTGAATCTTC
CAAGTTGGAGTGAGACTTCTTTATGTTAAATTCCCCCACTCAAACTTCTACAAATCATCCAATTTTAAAGAATGATTTTTAAGATG
ATAATATATTATATCATTTCCCCTTCCCCTGCCTCCAAGTTCTTTCATGTACCTCTCTCTCAAAACTTCATGACCTCTTTTTCTTT
AATTGATGTATCTTTTTCTAAGTACAAAAAAAAAAAAATCAATCTGCTCCATCCATCTAATGTTACTTGTATGAATGTTTCCATTG
GCATTGAGTAACCAATCAGTGTACATTTTTGGGAAACACAATTTCTCATGCTCTCAGCTTTCCCTATGTGCCTGTAGTTCTTAGTC
TAGGGTTGAGACCTCCTGAGCAATCTCCCTTTCACATTGCCTGTCTGTTGGTGCCATCCTTGTTCAGGTACACACAGAATCAATGA
CATAAAGGTACTAAGTGGGTTTGGACAGTTTAATAAGGCAATAATGTCACCTCCACCTTAGAAGATTGTCTTTGGATTGTTAGGAA
AGAATATCATCACCCAGGTTAATGGAAAACCTAGAGTCATAAGGATAGTGTAAAACATTGTTTGTGGAAATTAATTAGTATCCAAA
GTCACATACCAGACAGTGGCAGAACCCTGGTATTTTCAATGATGTCATACCCCTTTACTGCATCAAGTCAAAATTCCCCTGTTTTA
ACTTTTTTGTCTGAATAAATATGTTTAGTTGCAGCTGCTTAGAGAGTTTTGTTTTGTTTGTTTCATTTTGTTTTAAGACAAGAATC
TTACTGTGTAGCCCTGGAACTCACCATGTAGACCAGGCTAACCTCTACCACAAAGACATCTACTTGCCTCTGCCTCCCAAATGCTG
AGATTAAAGGCGTGTGCCACTACACCCAGCTCGTGTTTTCTTTTTAAGTATGTATGTAAATTGTGTGTTTGTGTGTGCCTGCATAA
GTTTATGTATACTTTATGTGTGCAGGTACCCTCAGAGGCCAGAAGAGGGTGTCAGAGCCCTTGTAACTGGAGTTTGACGGTCGTGA
GCTGCGCATGTAAGTACTGGGAACTGAATCAGGGCCTTCTGCACGCTTAACTGTTGAGCCAGCTCTCCAGCTTGTGTTTAAACATT
TTCAAGTGTTTCTTGTTTTAAACAACCCCACTTCCTTGCACACACCTTTGTGAAGTTGTGCCAAAGGACTTAGAGCTCCTACTTTT
CCTTTAGTTGGTGCTGGCCCTGCTCCATGTGGCCCTGGGAGTTCTCCAGAGCCTCCCGTGCATCCCATTAGTCACGTGGTGTTATA
GCACAGAGCCTTTCACTGGAAATGAAGCCTCAAGTTATGGGAATGTCCCGAGATGTTTTTATTAGTGTAAGATAGGCTTTTGATGG
AGGCTAGACAGTACATTGGATTGAAAACTAACTAGCCTCCATTCTTCCATCAGCCTATGTTCAAGCTCCAAGGTGGGCACAACAGC
CCCATCCTTTGATACAGAGTCTGTTCCCTCATGTGCTAGAAGCCAGGGCTTTTCTACATTTGGGTTTTATTCAGCATGGAACGCAG
TTTTTTAAAAAAAGATTTATTTATTTATTTAATGTATGTGAGTACACTGTAGCTGTACAGATGGTTGTGAGCCTTCATGTAGTTGT
TGGGAATTGAATTTTTAGGACCTCTGCTCACTCTGGTCGGCCCTGCTCACTCCAGCCCAAAGATTTACTATTATACATAAGTACAC
TGTAGCTGACTTCAGACGCATCAGAAGAGGGCATCAGATCTCATTACAGGTGGGTGTGAGCCACCATGTGGTTGCTGGGATTTGAA
CTCAGGGCCTTCAGAAGAGCAGTCAGTGCTCTTACGCGATGAGCCATCTCACCAGCCCAGAATGTGATTTTGAGGTGCAGATAGAT
GGTTCTATGTGTTTATCAGGGTCTCTGGGTGCCTGAAGGCCGCTGAGGCTGCTTATATTAATAGCTTGCTAAGTTCAGTAAGTTTG
AGACTTTCAGGTTTGATGTTTTCACTGGTTATTTTTTTAGTGCACTCTTTAGAGTTACAGAATTTTCATTGCACTTTGACAAAGGC
AGAATGACTCTCACCAGTCCTCACAAGAGGGCAGGGGGTGTCGTTCTATGCCCACTCAAGTGTTATGTAAAATGGTATCAGGACTT

CCTCAGCCTCTCAACCGAGTCTAGACAGTCCIAGGACCAICTCAGAGTTCAIGAGAAAAAAAATGCACAAAGIAGAAGTGGCCACA
GGGTTCCATCTGGACCTGATTTTTACCCAGAGGTAGGACCAGCTGTATCAAACCGAGGACCCTGAGCTTAGCCAAAGGGTTTAGAT
CCCGTGGTCTCCCTACAATGAGAGGTAGATGCCAAGGAGGTACAGCACGCATGATGCCCTCCTACAGAGGTCTTTTTTGCAGACAT
TTGTGGTCCCTGTAGAAATACAGCCTAGCCCTGCATATGTGCCAGAGTTGTAGGATCTTTAAGATTCACTCCAGTGCTACAGCTTT
CATTTCTTGTTCTGTTTTGTACAGCTTCACCATGGCAGACGATGATCCCTACGGAACTGGGCAAGTAAGTCCTCAGCTCACTAGCA
ACTTTGTTTCAACGCGTTCTTATCCCAAACACACAGAGGCAACGCTAGTAACTCAGTATCAAGCAAACCCTAAATTAGTAAACACT
ACTCTTAATTTATTTTCAGTTTTTCTGTTCTTCCAGAGTTGGAGAGGGCCCTTTCTTATGTATTTTTGCATAAAACCGTATATACT
ACATGCCAGAAAGTAGCCACTGAGTCAGTTGTGGTGTTGAAGGTCTGACCCCAGTATTTCATATGGTGACTGGTATATTAAAAAAA
TTGCTATTCAGTGAGATTTCTAGGGCTGCTATTAACATCGCTACAAACCTGGTGACTTGACACAATAGAAGTGTATCCTTTCATAG
GTCTGGAGGCAAGAACTCTGAATTTGGGGCTGGCAGAATAGTTCCCACCTCCTACCACTGCTGGGTGTGTTTAGAGATCCTTGGCA
TCACACCAGCCACAGTCTGTTGTTGTGTGGCTTTCCTCCACGTCTTCACACATACCATCTCCTCTGTCTTAAAAACACATCACTTT
TTGGATGTAGGATCCATCTCTGTCTAGTGTGAATTCATCTTAACATATATAAAGGCAAAGGCTGCCGTGTCCAAATAAGTCACATG
AAGGAGGAGCACTTATGAATTCTGAATATATTTTTTTCAAAGGTACTGTAGTTAAGCCCACTGCAGTCTGIGGGAGTGAGTGTGC
TTTGAGAGCGGAGCAGTGTTTATCATCACGTCATTGTCGCTGTCATCATAGGTGTGACAGCAGCCATGACACAGACTGACACGAGG
ATATCTGTATCTTCACTTICCTTCTAGTTTTTTCTCTTAAAAAATCTAAACCCATAGAAGIGTTAGACAGTCTGATGATACTTCCA
TTTCTTTCTCTTCTCCCCATAGAGAATATAGGGAGCATAGAACTGATTCTTGAAAGCAGTTTCTAGCTGCTAGCATGATTACAGTG
GCAATATTAAAAGTGGACTCTAATGGTCTATTAAATTATTTAAAATACTGGTATCTCTTTGCTCCTCAGCTATCTACTGAGACTTIT
ATAGAAGACTTTATGCCCAGAACATTAGCATCCTAGAGACGAAGGGACAGGIGATATGCTCACTGGGCTCAGGACTGCATCCCGGC
TGGAGTAACGGAACCACACTTTACACAGTCTGTGGATGTTGCCATGGTTCACTGAGAGACAGAAGCAGTTGGAGGGATTGCTGTGT
GTTCCTTTAGGGAAAGAGCAGGTTAAGGATGACTTTGCAAAGGAGGACTAAGCTGAGCTTTGAAAGCTACAACCAGTGCAGGGGCA
CTATTTACCAAGGTAGCCCTGCCACAACATTCATTACTACTTCACATTTAAATTTTGGTGGTAAACACCCGAGACTACCATTTCCC
AAATTTATGCTCGCAAAGAGTCAGCCAAAAATAAAACTGAAAATGAAATATTTGGGGGAGACCTGGTGTTTCTATAGTCACACAGC
CCCAGAGCCGCAGTCAGAGCTGCCATGGCCTGCACCGTAGTGACAGCTGCAGTGGCCTCAGAGCTGCAGTCAGAGCCGCAGTGGCC
CTCTTCTTCTCACCCAGCGGGCGGAGTGAGCATGCACCATCCGCTCCTGACCATGTTATAAAGATGGTGGTACTGGGTAAACCTAA
GGATGTTTTCTTTTATGGCAGCATAAAAGACACCTGGCAACCATACGCTCAAACCCCTTGTTTAACCTTTCACTTTTAATGGGCCT
CCTCTAACACAGGCCAGCCCTCACCACCATGAGAGCTGTCTGCTTGGTITCAAACCATGCTTTTCTGTCTGTACACAGGAAATCTT
AGCAACCAGAGATGGTCTTTTATGCCACAAAAGAGTAAGACCTGCCAAGGACAGTCATTTTGAAAGTGTCTCATGTGGGCAGCAGT
TCTGAGCTCATTAGCAGGGTGGGCATGTTCTGATAGTTCAGTTGTGGGGCAAGGGGAGGCCCTTGGAGGCTTTTAAGTTTTTGTCT
TTGGGTTGGTTTTTTCTTCCTGTCTTAAGCTTTAAGTCTATCTCTTCTAAAGTTTGCGATTTTTGAGGGAGGTGTCCTCCGTGTTT
TAGCCACACTGGAGTTTGTTCTCCACCTGTCACCAAGCAAACACCACATGCCGCCTTCAACAGGACGTCTGTCTTAGTGGGCTTCT
CCCCCAGCCAGATCACTGCAGACAGAGGCGGAACCACAGCAAAGGACTTCTTTGTGAGGTCGTTCTCTTTGCTCAGAGATGGAGGC
CTGGAGCTTATTATAAAACCTAGTTTCCCATTCAGTGTGTCTTTCATGGTTAATAGCGAATGGCACAGATGCGCTTTGCTTTTAAG
GATTGAAATATAACACACTCTTTGCTCCTCAGTTGAAAATTIGCATCCCICAAAAGTGGATTTGAAGTGACTCGCATTTAAACATC
ACATTTTAGGGGCCAGTAAGATTTTTCAGAGAGCAAAGTGCTTACTAAGTIAGCTTGACTTIGACTTCCTCCACTACCCACACATA
AGCACACTCATGTTACACACACACACATATCCATCTCATGTTAAACACACATACTACACATACATATGCACTCTTACACTATGCAC
ACATACACACCTTACACAAACATATATGTGCATACACTCAAACACACACACTCACATACTCCCACTACACATACATACACTTTCAC
ACTATACACACATACATGCACACACACTCACACTACATATACATACATACTTTCATACTACACATGCATACACACATACTACGCAT
ACATATACACTCTCATACCACACACACATACACTCACACTACACACACACAIACACATTCTCACACTACACACACAAATGTAATAA
ACATGCAAGCATTACTCTTCTTATTCTTGATTTAACTAGACATAGTAGGATTTGAGGTACCCAAGAATATCTAGTGATTTACTAAT
AAGGACAAAAAATCCCAAAAACCAAAAACCAAAAAAAAAAAGAAAGAAAGAAAAAAACCGTCTAGCTTGCAAGCTGACCAGAGAAA
GTAAAAGAAATAATTTGGTTTCTATGTTGTCCATAAACATTTCAACAAGTCACTTCTGGTGAGTGAGACAGATAGTAAGCTCACAC
ATACAAATGTGATTTTCTAAATGAGCTAACTGGGAAAATTCCTTTTGGAGTAATTATTAGTCATAACTTACCAAGGTAGAGCTTAA
TTCTATCAAAACGTTATATGTTCACTTTTTCAGTTTTCTTAGTTGTGTGTTGTTTCIGTAACAAGTTACCATAGTATTTGAAACAA
CACAAATATCTTCTAAAGATTAGAATAAAAATGTCAGAGTCGAGGCATCAACAGGGCTACACTCCTAGCAGCGTTTATTTCTTGTC
TTCCAAGCTCTAGGGGCTGCCTGTAAGTCCTGCTATCATCCCCCAGTCCTGCCTCTGTAGGCCTGGGTGAGTCCTGCTAGCATCCC
TGACTCCTGCTTCTGTAGGCACACTGCACTTTGATTTCTCACTCTCCTAAGTAGCICACTTGGATATTCCAGGATAATCCTGTCAT
TTAATTGTCCTACATTGATGGCACATGCAATATATCTTTTGCCACAGAAAGCATAGGTTCTGGGGAATAGGAAGGTTCTGTGGAGT
AGCATGTAGACATCTTTAGAGCCATTGTTCAGIGTGCCTTCATCTACTAGATTCAGGTATITATATTGTAATAGAAAATAGAGCTG
GGCAGTGGTGGCACATGCCTTTAATCTCAGCACTTGGGAGGTAGAGGCAGGTGGATTTCIGAGTTCGAGGCCAGCCTGGTCTACAA
AGTGAGTTCCAGGACAGCCAGGGCTACACAGAGAGACCCTGTCTCGATAAAAAAAAAAGAAAGAAAGAAAGAAAGAAAGAAAGAAA
GAAAGAAAGAAAGAAAGAAAGAAAGAAGACGACGACGACGACGACGACGACGAAAAGAAAAAGAAAATAGAGGCGGGAGAAAG
TGAGCCCTCTGCTCCATCATGTTCTCAGTTGTATTGCCAGTCATTTTGTAAGTTGTGTAACTCCTGCCTGTAATTCTTTCAAGGCC
TTGAAGTTCTGTAGCTCAGTAAGTAATCTATTTATCATCAGAACAGAAGCATACATATCTAAGATCATGTGCACGTATGTGCTGTC
TGTCTGTACGTGTACATTCACAAGTGTGTGGAGGCTGAAGGTGGCTCCCCATCTTTGATTTTGAGAAACGGTGTCTAACCTAACCT
ACAGCTCCTGGATTCAGCTAAGACCTCCAGCCAGCAAATCTTCATGTCTCCACCTTCCTGTAGCTGGGACTGCAGACAACTGCCGC
CAGCCCAGCTTTTCACTGGGGTACTGGGGCTCTGGACTCAGGTTCTTGTGCTCTACAGCAAGCACCTTATCAACTGGGCCATCTCC
CCACCCTCTGGGATGTAATGAACTCAAGTTCCTAAGAGGGTGCGAGATTCAGATCACTTACTCTACACATTTTTCTGAAGGGTCCA
GCATGCCTGAAGCTCTCTTGGCTCCGAAGATGCACAGCTTGCGGGATCCACCAGGCCCTTGTCTTTAGGGACCCCATAGCCATTAT
TCTCCTCAAATTTCTTTGTTACATAAGTTACACACACAGGGGCCTAAGAAGGTCCCAGAAAGGCACGTTAAAATTTTGATTTCAGG
AATCCTATTTTTGCGTTTTTATTTCTAGTTTTCAATCATGTAATTAGTTTTCTGGCTTTGTGTATTAGTGCAAGAAGAGAAATTAA
AATATTTGTATTTTCTTCTTTACAGATGTTTCATTTGAACACTGCTTTGACTCACTCAATATTTAATGCAGAATTATATTCACCAG
AAATACCACTGTCAACAGGTAAGAAAAACACCCCCTTTTCCTGTTGTTTTGCTTTCCCTTAAAAATATTTGGGTCAGGAGTCAGG
GAGATGTGGATCAACCAAACTAAGTATATATGAAATTACTATAAGGAAATCTGTTGCTTTGTGGGTGCTTGGACCTAGCCCAGGC
CCTCTGGAAGAGCCACAAGCACTTTTAACCACAGAACCATCTCTTTAGCCACAACAGTCTCTTTTAAAATTCAACAATATTCATAG
GAAGGAAGTGGTGATCCGTGTTTTAAAACCACCCTTTCAGTATGCTTTCTGGAAATGTGTCTCACAAAAATGCTCCGGCGTGCGCG
CACACACATCGTTTIAGCAAGTGTGGTTTCTGGTCTCTGTTAGAATACCCTGAAGCCCTGTCTTCACCCCGTGTAAGGACTTTCCT
CTTCTCCGTATCTCATTACCCATTAGTGCTCCTTTTTGCTTTACCGCAGCTCACAGAGAGCCAGTCTTAGCCCCTCAGTTCTGTAT
CTGAGGGCCAGTCTTGTCAGCCAGGACACGAGGCTCTGGGGAAACAGTAGGAAAGTAACCTTTATATTCTGACCAGACCAAGCGTA
CAGAACTAATGCTCCTGACATGACTGCTAATTAACGAACCCACAAACTGTTTAAGGTCCCTTTATGCTATAGCGCACGGGCTTCCT
TTAGACTCATTATTGACTGGCATGTGTGTAGCCTTGGCCTGATGGCTCTTTTGCTGCTTTGATCACCTGTTGGAGCTAAGCCGTCT
TCAGCAGCCACTTGTTAGTTGGTGGAGATGCGTATGGAAGTGATTGTCCATTACTGAGGTGGACAGCAGGTGCTTTGTGTGCTCCG
GATTTCTGTGTCTCGTGATGTAGTTTTTATCATTCTCATTTAAAATGACTATGGTTTTGCCAGCGCCTGCCTTACGGAAAGTTTTC
TACAGACGTGGGAGGAAGGAGAGTTAGTATGTCACCCTCCTCACTGGTGCCCAGAAGTTACATCACGGAGGCCTGTCTGTCCTTAC
CCCAAATAACCACCACAGTTTATCTGTAATCTTAAGCTGTCCAACACGCTCTAGGTCCCCTTCTTGTGACCAAGTAAATGCAGGGG
AGGCAACCGTGTTTCCTGTGAACTTCTAAACTCTGAGACCCCAAGAGCTTCCTTTAGCATTTCTGCTTTATTGAAAATGTTGCAGA
CTCGGTTCTGCCCTCAGGGAAAGCACAGGTTTCTAGAAAATGCCTCTAATTAGTTAGAATACTAAACAGCATCCTGCAGGCAGATG

ATATTCTAATGTGTTCTCCTTTTCCTTTGGTTTATGGACTTAGAAATAAAGTTATCCAAAGAATCATCTCAGAAAGTCGTTGGTGT
TCATATCATTGCGCTTCGGGAATCTTCTTTTCCATAGTGTTAGAGAATCCTTCAGAATTGCATGGAAGATGCATTCTTTTCCTGTT
GTAATGGCTCCTTGCTTGCACTAGCTCTCCGGCTCTGTTTCAGTGCGTTGTCCTGTTTTTATAGTCCAAGATGGATGGCACATTTT
GTTCAGATTTTAAAGGACTAGAAAGGCTATCTGTGACAGTGTTTGCTTGTAATTTTCATGTGGATTTTTTTTCAATCTTCTCAACT
TCTTAAAATCTCAACGTATTAGTATTTCTAACCCTGTGTTGGGAACTCTCTAACAAGGGACAGCTGATAGGTCTGTGTCATTTTGC
TGTGATTTAATTTTCTTTTTACAATGGAAGATCATTTTTTTTCTGGCACAGAAATGTAGCCAACAGAATATTAGCCTTAAAACAAG
TATCTGTGTATCAGCTCCAGTGTTCTACTTCTCTTACCCTTGGGTGTGCTCTTCTAAGGGTAATGATTGCCCCCTCTCCGCATGGA
ACTTCACATATGTAAGGAAAACCTAAAACCAATCCTGTGAGTGTTTCAGACGGGCTGTTAAGAAGGAAGAGACTTGGAGCCTAACA
CGAGGATGTCTCCTCTGCCTCCATATTTCAGCAGCATCTTATCCAAATGCTACCACCTTCCTGGCCTGGCTTCTCAGTAGGCTCTT
TTGGATGCCTAGAGGAAATGTATATTTTTAATATTGTTATACAAGCTGTCTGATATGCCTAGCATGAAATCTTTTTCTAATCAGTT
TTATTTCCTCATAAAAGTTAAATTCGAGCAATTTTCTTTTTGAAATTTAAAGTTAACAGTTACCTTTCTAAAAGTTTAAAGTGTCA
AGAGATGAATCCCATGAGACAGAAGAAGGTAATTTGCTACATTACTGACCCCAGTAACCACCCATCTGTCTTTCTTATTTGGCTTC
AAATTAGGTTTCCAATTGCTTTCCTGGTACAGTGTTTAGGCTTCCGTGTTATCTGATCAGTCATAATATTGAGAAGGTAAAATGTA
TTCTCTTTGAAGTTACAGTTTATTAAAAATTACAACCTGAACTTTTATCTCACCTCAAATTTACATATGCAAAATAAGGCTTAAGC
TTCAGCCGAGAAAGTGAATTTTCAAATGAGCACAGAAGTGTTCTTCTAGACTGTCTTCAGCTTCAAGTCTTAATTTTCACTCAAAC
AACTATAAAATGTCGAATATGTTTGTGTATTACACAGCTATTGGTTATCATACTGGGACATAGAAGCCATCTGACGCCCTTATGGA
ATTTTTTATCATAACATTTATATTTATTTATTTTGAAATTTCAAGTCAGATATCATTGGTATATAAATTGAGCCTTTAGGTCCTTT
TCATAGTAATTAATTGGCATGCATTTATCTTCAATTACATTATTTTTGGTATTTTATCATAACTCTGGGCTGTTTTAGAACAGAGT
TCTTATGAGCACACACTGTTAGATACTTTATTATATCTGCATCAAAAGCTGTGAGTAAGATGCCAGGGTAGTGGTCCATGCTTTTA
ATCCCAGCACTTGGCAAGAAAATCTCTGTGAGTTTGAGGCCAACTTTGTCTACATAGTGAGCTCCAGGCGAGCCAAGGATACATGG
TGAGTCTCTGTCTAACAAAACAAAACAAGCCATGGAGAAGCAAGAAAAGTTCTTCAGGTCTCACAGAGGGCAAGGAAGAAGCCAGT
TAGGCAACAGTGGCTGTCCCTTGGCTTGAAGTAAGGGAAAGACTACATTTCAGGAGCAGTGTTAGGAGAGGCAGGTGGCCCAGGAT
CTCCCTTCTGTGGGTTTCTAGCTTCAGTTCTTCATTTACTCCTGGGCAGTTCTCTTTGAGGGAGGAAAGTAGTCTGCAGGTGAAGG
CCACCTGACACCACAGCACTCAGAGGTGCTCCCTGCACTCAGCTGTATTGTGCCCTAAATGTTTGTTGACACATGAGTCTGAGGAC
GGCTCGGATCAGAATCTCTGTCAGCATTCAGACCTTTTGTTCAGTGCCAGTGGTGTACCATTACCATTCAGAAAAAGCCACTTATT
TACCGTTAGTCACTTAAACTATAAAACTANNNNNNNNNNNNNNNNNNNNNNNATCAGACACTCTCATCTGGCTTCCTTGGGCACTAAGC
AAAGCACTTATGAGCAAAAAATAAATATTTTTAAAACATTTATTTAATATTTCAAATGATAACACAGATCAGTAGAACACACTTC
ACCTATAAAAATTGATTTGAAATCTCTGTTTAAAGCATAACTTCAGCATTTGTCTTGCTTTGTATTTCATACAAGATTCCAGAAGT
CTCACAGTTTTCTTTTGGTTTTCTTGTGTTTTCCTTGTTTTTAACAGATGGCCCATACCTTCAAATATTAGAGCAACCAAAACAGG
TAAGCGTAAAGGGGTCGGGTGTTTGGTAATATTACATCTCATGGTAAGGCTCGGTCTCTGAGTAATAGTTAAATCTGATGACAGAG
GTAAGGGTTTTATCTGAATAATCGGCAGCCTGCCGTGTTATGCAATAACTTCCTGTGTCTACATTTTTCTTACAAATGGCTTTGAA
ACATCAGTTAATTATAAATCCTTGGTAGTCTGTCCTTTTTCCAGCTTAGCTCATCAGCTGCTTCCAGGGAAAGCACTAAAGAGTCT
CATTCATGAAGAAATACTCACACACACACAGGTTCACAGCCACTCTGACGCTCTATTGCTCATGGCCATGCCCACAGGGTACCCCT
CCTGCCTTCATAAGCTCTGGCCTTTTCTGTGCATAGAATGTGTAGCTTAAGGCAGTAAACGCACTGGGAGACATTAGAGTGGACTG
GGAGATCTCCCCTCCACCCCTCTGGCTTTAATCCCAGCCTTTTTGAAGCAAGGAGAGGATTTTTTTGTTTGGTTGTTGTTGTTGTT
TTTGTTTGTGTTTTTGTCTGGCTCTTTCGCTTTCTACTAAATATTGTGTTCACCAGCTGAAGATTAAAATAGCCTCAAAACTGCAA
ATATGCTCATTAGAAGTTGCATGGCTCTAGTACTGTAAGAGAGAAGAGAATCTTCAATATCTGCCGACAGAACATTTCAGTCATTA
TGGGATGGAAGTTATGGTATAGCCTCCCACAGAGTGAGAACCAAGTCACTAGATCTCTGGGTGGATATGCTCATCACAGCCGTGGC
GCTGAAGGACTGTATACCTGGCCTACCGAGTTGTGTCACCCAATGTCTTTTCTAGGCTGTTCCTTCTTGTAACTCAGGAGAGTGGG
TTAACACATTGTGTTGTATGACACTCAATGCTAAGAAGTGAATGCTGACAGCATTCGGTGCTGTGCTGTAGAATTCTGATGTGGTC
AGCTGTTAAATAGGAGAAACATCTTAGGGATGTCCATGCTACTTTGTAAAGCTGAAATCAGATGGAAATGTTAATTATAAAGACAG
GAATGTCACATGGAACTTTGGGGCATTGTTTGGTTGCTTATAGGCGGCCTCTTAACACACACACACACACACACACACACACACAC
ACACACACACACGCACACACACATACTTATACACATGTACACCCTCCCTGGGCTGCAGATAACACCTGCCCTTGCTTTATCTCCAC
GTCCATCTTTGTAAAGATGATTTCTCACATAAAGTCGCTGTTCTCGGAGCTCTTCAGTAGAGTTGTGTTCTGTGAAATTCTGCCCA
GTAATCTGGAATCCTGGAAAATGTCCTAGCCTGGAAGATTTGGTGAATTCATAAAACTGATGAGTCCAAAATGTACCTACACATCA
AATAGATTTTTCAGATCCTTTAGAAAATTTTAAATAATTTATTTAATTTTATTTTGTGCGCATTGGTGTGATGGCGTCAGAGCCCT
TGCAACTGGAATTACAGACAGTTGTGAGCTATGACGTGGGTGCTGGGAATTGAGCGCAGGTTCCTTGGGAGAGCGGACAGTGCTCT
GCTGAAACATTTCTCTAGCTCCCCTTTTTAACAATATTAAAATTGCATTGATTTATTTTTTAATGTGTTGCTCTCTTTGCCCCCAC
CCCGTGTGTGTGTGTGTGTGTGTGTGTGTGTTATTATGAACCCCAAAGTCATAGGTATTTATGATGTGTTTGAACAGCTAGTATAG
TTCCAGAATTTAAAATTTAATTCATTTTGTGTGAATATATAAAATCATATATGTATAAAACATCATGTTTGATTGCCAGTCCGATT
TTATTTTGGTATCTGGCTTTAGTGAAAGTAAGAAGGTTGTATGTAAGCCAGGCTTTTTGACTGGATGTATGGAGTCTCTCTGAGGT
TAATGACTCATTTGAAGTAAATGGCGTTCCTGAGATGAACAGCGATAGATGGTGAACGTTTAATTTCAGAATACTAGAATTAGAAA
TGTGTCCAGAGCGTGCATTTTAAGACTTCCTAATTTTAGGTTGTTATTTAATTTAAAACATTAGTTTATTTCTGAGTCAAAAAATG
TCTGTAGTCATTGCTGGCTTCAAACTGGTGCCAGTTTTCCTACCTCCCCTCTCCAAGTGATAAGATGACAGGTGTGAGCCACCATG
CCCAGCTCAAAGGCATCGATGCTCCAGCCGAACAGCGCAATACACATTACAACCTTCAGAATAAATGCTGCCTTGGATAGGACGTT
CAATATTCTTATTCGACCGAGAATAATACACAGTGTCACACGCTGTGCAATAAATCATGCTGGCTACAGGGTTAAACGTCACTCCC
CATAAGGTATGAGAGGTACAGGTACAGATTTTTCCAATGCCACAAAGAAATTTTGTAATTAAAGATAAGGAACCACTATGTTAAGT
TTTTGAGTTAGAAATGTTCTTTTTCTTGGAGGCCTTTTGGGCACAGATGATGAAAATGAACCAAACCACAGACTACATGTCTTTAG
ACTGTCCAGTTCCGTGGCATGTTTCTCACTGTTAACTGTTTATTCATGTTGTGTTGTGTTGTGTTGTGCTGTGTGAGTTTTGTACT
GCTTTGTTTGGAGGCAGGGTCTCAGCAGTGTAGCCTTGGCTGGCCCCAAACTCACAGAGCTCCATTTACGTGCCTCTGCCTCACAA
GCACTGGGGTAAGATGTGTGGCAGTTATAATCTGAAGGTGTGTTTGAATTTTCTCAGTTAAGTGGATCCCCATCCTAACGGCAGCC
CATTTGGGGCTCTTGGTGTTATACATGCTATTATTGTATAGTTTGGGGGTGATTGAGTTCAAGCGTTATCCAGACTGGATCCCAGT
AATATTAATTTTTACAAACTTTATGATCTTATTCCTTGAAGTGCTCAGAAGACTGCTAGAGTCACAGTGTCGTAGCATACAGCATG
TCTGTAACACAGATGCACTTAATCCTCCAGAGACTTGAGAAGGGGCACCAGGAGAAGGGGGATGTGGGGGGGAGCACCCTCTCAG
AGTCGAGGAGGAGGAGGAATGGGGTGAGGAACTTGGGGGGGTGATGTAAATAAATAAAATAATTCTTAAAAAGACACCTTTATGGT
CTGTGATCAGAGCAGATAAAGGAACTCCTTTAAGTAATTAGCATAACTTTTACTGATGGCAGAGCGGAGAGCTTGGCAGCAGCCAT
TGGTAACGAGAGGCTCCAGTTGAATAAAGTGCTAACGTATATCCAAAGGCTTAAACTCTTCATCACTCCTGTGTTGAAGGTGGGTG
CCTGGCTTCTCCGCCACTGCTCAGTGCATCATTTTCATAGTGGCTGTTAGAAAGCATTTCTCCGTCTGCAGAGTTGTCTTCCACAG
TTTTGCATGTTTAGAGACGTGTGTGTACATCTGTCATTGTTGCGTTCAGTAAATACCGCTGTGTTCACCTCTGCAGAGGGGATTTC
GATTCCGCTATGTGTGTGAAGGCCCATCACACGGAGGGCTTCCGGGAGCCTCTAGTGAGAAGAACAAGAAATCCTACCCACAGGTC
AAAGTAAGTTTGAAACCTCTGCTCTCACTGCCCAGGAGCAAAGCCAAAACGTTCAAGTGAATGTGACTGGGATGTTCCTTTGCCCC
TCAGCGGGAGTGTTTACATGGTTCTTAGAATTTTTAAATAGAAATTCACCTCATGAAAATGACGAATCGTCAAAACACCATTAACA
TCTCCATTTAAGTGAGATGGTTCCAACATCAACTAAGAAAAACTTAACTACCTCTCCAGTGATCTTAAAGCTGTCTTTTCATCTTA
AAGTATTTTTAAATATTCTCTGATGTTTTAGTAAAACTTGGAGCCAGGTGTCATAGTCCATACCTGTAGTCACTGGACTTAAGAGG
CTAGCAACAGGACTGTTAGATATCCCAAGCTAGCCTGAGCTACATAGCATGCTTGAGCTCAGCCTGGGCTAGGTGAGACCCTTATC
TCGTAAGAAAACGAAGAAAATAAAAAATGTCTTTGAGGCAAATTTCAAATACTGGGTTTATAGAGATTTCATGACAAGACCCTTGA

CAGAATATATCGGCTATGGAGTATATTAATTATTIAAGATCATAAGTTCAGACCCAAGTGATGGAATAAACAGGTTGTAAGCAGGC
TTATCTCACTGACCAAACATATAAATACACAAATACCACTAGCATGTACTTCACAAGGTGTTTTAGAGTTTCAATGAGGAAATATG
CTTAAGTTCTCTGTAGAGAGCCTGAGAGAAACCGTGTGCAGCTACCATGAATGAGTTCTGTAGCATGGGGGCAGAGGGGTGGGGG
AAGCTTCACTGTCTTTACCTTGGGGTGTCGGGTGTAGCTTCGCTGCTCTTCGCTTTTTACGTTGTTGATCTAGTTGAGACTACCTT
CCTCTAGTTCAGAGTTTCTACTTTCCTAATCCAGTGTTGTGTTTCAGAGACTGACAGTGATTTTCCAAGGAAACAGTTAAAAACTT
TCGAGTGGTTTAAAAGGTTTTATTTATTTGGTAATCTGGGCTTAGACTAAATGTTACCAGCAGTAAACGTTATGGCACTCCAGCCT
CTGTCTTTCTGCTGGCTTGAGATCTCAGACGCTCTTGCCCTCAGCTGAGCTGACACTGGGGAAGTTATCTTCAGTTTTGCCTCCTA
GGTTGCTTGTCCCCCTTTTAATGTTGTACTTCCAGGCTCAGGCCTTCCAGGTTCTTGCCCTCTGTCTAAGACAAAAGAGCCATACC
ATCCTTTATCTTTCCAGTGAAAAAAATGTGAAAAAGCCATACCATCCTTTATCTTTCCAGTGAAACTCAGCTCCTGGCTTGGGTCC
TGAGAGTCACATTTTCATTTTAAGGCTCCCAGCTCTAGTTGTGGAATGAGCCGTTTTTTTTTTCTCAGTAAAGGTAACTGAACGTG
GAAGTGCAGTGGCTTTAGTGCACAGAAGGCCACAGTTATATTAGACAGTGTTTTTCAGAGAAGTCGAACCAGGTCGGTGAAGACAG
GGAGATAGTTAGATGGTTGACTGAGCGATAGCGGAGGAGATGAGAGTAATCGGCCTCAGCTGTGACGATTATGAACGACACCAAGA
CCCACTCTGCAGCGTCTGTAAAATGTGCGCAGGGACACTGCTAGGATTCCAGCACAGAAGCCTGGGCGCCCTGTAACCAAGGGGAC
TCTTCACTAAATCTGGGCGTGGCTATGCTTTTCCTCTAAAGGCAGGAGAGGTTGGACTGCTGATGCCCTGGAGGGGCAAGAAATGA
AGAGTGTCCTCCCTCCCATTCAGGTGGGGCAGTAGCCGGAGGGAGGGAAGGAGAGGATTCTCTGGTGTTTTGGTCCAGCAGGTCTT
CTAGCTGAAGGGAAGGTCTTCCATCAGTCCAGCAAACTTTCCAGTGTCCTCTGGAAACGTGCACACAGCTAGGAAGCAAATGCTTT
ATCAGCTTTCAGCATCTGCCCAGTGTAGTCGACTGCACACCGGAAACTTCCCTCAGGACAGTCTGTGGCTTCCAGTCCTGCAGAGG
CCAGGCAAGGAATGCAGAGCAGAGCACCGGCGAAGAAAACGGTTATTCCACACCCCTGGTGGTCCTCGTGTCTCCCATCTGTTCCC
TCCTGTAGCCTTATTAACACTGAGGAGCTCAGGCTGAGGTTAACTGGCCCTCTTCAAAGGTGCTGGGCTTCTTTCTCCTGCAGCTC
ACTGGCCCTCCCATGTGAGCGTGTGCAGCTGAGGGGCCAGGGGCCAGGGGCTGAAGAACAAGTCAAGAAGGACTGATAAATCAAGT
CAGGGAGGATGAGCATTCTGAACAGCAAAGAAGAAATGGGGGCAGAAGTCATCAGGGGTCAGATCTAGAATTTCCTACCTGTGTGT
GTCCTGTACAAATATTGTGTGCAATTATGTGTCCTTATCTGCACCAGGGTCACTGAACATCCCTTGTCAGCACTGATACTGATCAT
AACTACTTAAGAACCGATGTTAGGAGAAAAAAGTAGGGTTTTTTTCTCTATTGGCCTTCTACCAAAAAAAAAAGAAAGAAAATGTA
CATTGTGTTCTATTTATGAACTCTTTCCTTCTGAGGCACTAGAATTCTGTGGTCTGCCATGCATGAAAGATGGAGTGTCTGTAGA
AAGAGGCTTCCAGGGGTATCATGGCTTACTGCATACCCACCCATAATTCATCGTAGAGGTGGGTCCATGTACTTCCTGAGGGAGCG
ACTCTTCCGTAGCCTTGCACATGCAAGGCTGTGACTTGCATGAAGTATATGTCTTCAGACTCACCTTTTCTTCACCCCCACTGACA
ATCTCTGTCTCCCTGGACTCAGGCCCTCTTTCCTGCCCTGCCTCTTCAAGCACCCCTCCCTGCTGICCTTAGGGAGCGCCCCTGCA
AGGTTAGTGGTTGATGTGATGTGAAGATTGCAGTGAAGATCTGAAGGCTCTTGAAAGCTTTGGGAACTGGTCATGACTTAAGGCTG
CAGAAACCAAGCTTTGCTTTATGGAGCTTGTAAACTTACATCTTGTTTGGTCGTCTACTTGTTGACTGTTTGAAGCATCAGGTTGA
CTTGCAAGCTAACTCTAGGAATCTCCCAAAACCAGCACTTCCTATTAGAAGTATTTCCACGCTGTGGTAGATGCTGGGATGCAAGC
TCAGTCGTCACCCCCTCCCCCCACTGAGGTACCACCTTTTCTAGAACTCAGGCGGCTGTACCATGTGTATGTTCCTCTCGTGTAGT
TGAGAAGAAAGAACCTCTTTGGCTCTCTAGGATCTTGTTGTACAAGTCTTCACGTTCTTATTTCCTCTCTCTCTTTTGATTAATTT
CTCTGGGATTACTGGTTCCTATGTCCTCACAGGTATCATGAATGGTTTCTTCAGATTGAAGCATTCCTTCCACTGCCTTCAGGAGC
TCGGGGTGGGTGAATAGTCTCTTTTAAACCAGCTACTTCATATTTCAAAATATTTTAAGCATACAAGTTGTAAAGTTCTATATAAC
AAATACCATGTACCAAAATTAATAAATTTAATAGATGGTAATATTTTGCCTATTTGCTATCTGTCTATCTGTTCTCTCTCTCTCTC
TCTCTCTCTCTGATTCTCTCTGAAGTACAACATAGTTACAGTGGGAGCCCCTACTCAGCCACTGCTCTGTCCTCTTATCCTCTT
TCTTTGCCCAAAGGAAAACCACTGTTCTGAACATTCTTTATCTGAGATGCATTCATATCATATACTAAAATCACTTTCAAGAAAGT
TACTTTTTACTATCTTTAATCCAAAGTTTTTTATTATATATGGTGGCTCTTTTTCAAACTATATCCAGGTTAAAACTGGGCCCCTG
AAGGCTTAAACATTGATTGCTTTAATCCAGTCTCTTCCTCTTTATTGTTTGACTAAAAATGTCTTCAGTCACAGTGTGGAATTTAT
GCTGACCCTTAAAGCAAAAAATCATATTCATGAGGAAGCTAGGGAAAATAAATATCCAGAATTTAAGAAAGTTTTCCACTTTTAAA
AAATGGAGTAATCCACTGTCTAGCCTGTAAGCTCTCCCTCCAGTGTTTGCTTGGTTCTGACTTTATCTGCGGCTCCTGGAGAACA
GGAGAGACAGCAGAGCTCCTCTGGGCCAAGCCTGTCGCTACCTGTTCTTGACTCAGCAAGGAGTGCCAGGGAAGGCTCACTGCCCT
CCTTTTCCCGAAAGTGGTGCAGAAAAATTTTAAATCAAAGTGTCTTCCTTGAGCAAATGGAACAGGGCCAAGAGGAGAAGCCACAC
GTGGCTTTAGGAATGCGCCTGCAGCCCGTCCTCACTTGCTTGAGTCCTTCTGTGCTGGGCACCTTGTGAACATTGCACTTGGACAT
CAGTGACTTTCATTTCTAAAAGACAGTTTGTTTGTAATCATCCAATGCCAGGAGGTTGGAACACAGTGATGCTAATATGACGATTC
TGATCAGCTTTGCTGGCAGCTGCCCTGCCTGCCACTCTCCTGGGGTTTGATGGTTAAAGAACGGGTTTATTGGAATGTTTCTGTGA
ACAACAGCTTTGGCTCCGTTCTCCCACCTCCTGTAGTGTGCACTCTTACGTGCCTCTCACTGAACAGTCCTTAATGTTACGTTTCC
GTCAAGGTTAATAAAGCCCGGCTGATCTGCTGTAGGCTTGAGGATATCACATATCTTTGCAAAAGCGCCCTTTCCCTTGCACAGGC
TTGTCTGCATCCGGAGACTTGCCATCCTAAAGATCTAGTTGGAATACTAATTTTGAATAATTTAGCCTGATCCAGTTACCTAACAT
GCCAGGGGGGAAAGATCACCACAACAGATTGGGCATTCAGAACCTACCTAGTTCATAGCATTTATTAGAATGTCCTTTGACACTTC
CAAGTGTTTACTGTGCTCAAATAAAAGAAAAGAGCTATGTTCCCTACCAATACTATAGTCTGTGCTTTGTAACACACACACACACA
CACACACACACAAACACAGAGTTTATTAAAACAAGGGCTTTGTAACCCAAGGTTCATGGATCCTTTTGTGGTTTCAGAAAGGTTTG
CAACCTCTGACTCTACACATAGAGTTTTATGCATATTTGTATGCATTTTTAAAGACTCGAACATGTGTAAAAGTAATGCTGAAGGG
CATTGGCTTTTTTACCACAGCTGATTAAATCGATCTCTACAATTTTGGAGGGATGGGGGCGGGGGCGGGGGAGGGCGACTGGCTC
TCACTGTGTAGCTTAGGCTGGCCTTGAACTCATCTTCCTACCTCTGCCTCCAGAGTATTGAGAAATTCCAGGGATGCAACTGTCTT
GAATGTTTTTCTTCTTTCTTTTTTCATTTCAAAAATTTACATTTACACTTTTTAGTTTTACCCATTTTAGGCAAGTTGAAGCGCTT
TGTGTTTCTCAGCATGTAATTCAAGCTAGTTGCTCTTTAGTATTCCATTGACTGGAAAAGATGTGAGATTTAGCGCCTCCTCACAC
ACACACACACACCACACCCCACCCCCGCCTTTCTGTTCTCTCCTTCCTCCCCTCTCCTCCCTCTCTCCCACTCCCACCGAGTCTCT
TGGCACTGACTAAATACGATGGTTCAAAACCAAAAAGAAAAGAGAATCATTTCCCTGATATTCACTCAGCACGGTGGCAAGTCCTG
CACTACAAGGAGGTAACATCCTCTGCGTGGCCATAATAATCTAGACTTCTGCATAACTTTTCTTCTACGTATCCCAGGCGTTTTGC
GATTGCCTCCATGAGTAAAATACTGACTTGTTAAAAAACAAACAAACAAACAAAAAAACCAGCCCTTTATTTCACTGTTAGCATTA
GAAGTTAGAGAAAGCTGCTTACTGAAGGAAACTGTTGAGCAGTGAGAATTGATCCTGAGAGTTAGGAGAGCCAAGCTCCTGAGGGT
TGCAGGTTGTCTGACTTTCCAGGCTTGGTATGGTGGCCTGCCTCGCCTGGCATTTCCTCTTCCCCTCTCCTGTTTCTCTGCATACT
CATTGCTCTTGGGATGCTTGTTCCTAGTGGCAGTGTTCAGTCTGAAATCATCTCTGATCAAAGTGGTCTCTGTTTGAAGAGGATCA
TTACATTCAGGCCGGGAATCCCGGTTTCGTAAAGCCACATGTGAAGATTGGTTTTGATGAGGCAGAGTGAGTAACCAGAGCCTATG
ATCCTCATGTCCCCAGCCACCTTCTCCATAGTCAGGGTCTAGGGTCTTTGGTGGGTCTAGGGATGTTCTGCTGGCTCTCCTGGAGT
CCCCATCTTCCCCCTCCCACTCTAAGTGGATTTTAGGTCAGTCTTCCAAACAACTTTCACTTTCTTGAGGTCTAATGGAGGTGAGT
GGGAGGATATCTGGGCAGACATGTGAAACAATCCAGTCTGAAGACCAGGGGAATGCAGAGTAAGTCCCCAGCATCTTCAGATAACC
GAGTTATCTATCCATTGCTTGATAGATAAGTACAAAACAGGTTTGCTTGTATTAGAGAAGACTTGGAATTGAAAACAAGCCAGAAG
AAGGTAAGGCCAAACACACATTTTTCAAATCACATATCATTGAAATGGTCTAGACATTGGAAAAACAATGATCAGGTGGGAGAGTG
GATAATGAGGTTTCTCGGGGTGGAACAGAGAACCGTGGAGGCTGGGAGAAAGGGAGAGCTGAAAGCAGAGCAGAGGGTGTCTAGCT
GAAGTCCTGGGAGAAACTAACATGGCTGCATGTGTGTGTGTGTCCTTCTCTACGCACAGCTCTCAGATTGGCATGCAGTACTCAGC
TTGTGGGCAACATATTCACGGTGTTAAGCACCAGCTGTGAACTGATGGCCAGCGTGGGGTGTGACATTACTTCCTAGATTCTCATT
GGCTAAGCTTTGTGAACTGATGGCCAGCGTGGGGTGTGACGTCACTTCCTAGATTCTCATTGGCTAAGCTTTGTGAACTGATGGCC
AGTGTGGAGAGTCTCACTGGCTAAGCTGCAGCTTCTTCATCTGTAAAGCTGACGTAGAGAACCAACTCAATGGAACATGTGCGTAC
ATTCAGTTGCTCAATGCCGGCACACAGTCAGCCTGGAAACACAATTACACCCGTTTTCATATAAAATTTCAGGAAATTGGGTACTA

CTCCAAAAGCTTGTGTTTAGAGGTAACACATGATGTCATAGGAAACTCACAGAAAACAAGAGGATAGAAACAAGTGTTAGGTTTTG
TTAGGTCCCTTCACTGTGTCCTGGTAGGCCCTATGTGTCTAATTTGCACAACGCTGTATTCATTCCTAGGTTGCCATAACAAATAA
CTGCAACCTGGGTCATATTAAACAACAGAGTTTATTGTTCCAAAGTTCTGGAGGCCCAGAGTCCACAGTGAAGTTGCCAGTGTGGT
CTTGCTTCCCACAGAGGCTTCAGGAAAGAGTCAATCCTGGCCTCTTCCAGTTTCGGGTACTTGTTGGCAGTCCTCAGCAAGCTCTA
GCTGCAGCCACTTCTCTGATCTCTGCTTCTGTCTTCACAGGGCTCTTCTCCCTGACTTTGTATCTGAATCTCTCCCTCCTTTCCCT
CGTAAAGACACCAGTCAGCGTGGTTAGGGACCACCACGCTGTGTAGCAGGACCTCTTCTTGACCAATCGCATGTGTAGGAAGCCTG
TGTGTAGATGAAATCATGTTCTTAGCTCCTGCATAGGCCCGAGTCCTGGGAGATGCTCTTCTCACTTACTACAGGCACGATGTGCA
GTGGTTACAGACTGTTCCCGAGTGTGTGCATTTAAGGCCATGTCGTTCCGTGCTGTGCTCAGAAAGAAGCCTGTGTGCACCACGTG
CTCCCCTGTGTACCTTTGTTAGATTCAGGTAGTGGAAGAAAATCTCTCAGGTCTGAGGAAATTGCTGTACTAGGGTTTTGAACATT
TATTCTAGTAGATGGAAGGTGTGCTAGAAAGTGTCTGGCATCAGACTCTTGGATGGCACTCTGAATTGTAGCATCTGCCAGTTTCC
TTCCCATAGTAAATTTCAGGCTGCTAACATGAAGTCAACTGGCTTGCAATAAATAAATAAATAGATAAATAAATAAATATTTTAAA
AAACAGTGAAAATTTAACTATTCTGAAATTAACTCCATGAACTAGTCAACCGGTTTCTAACCTACTAGGTGTTAAGCCAGCAAAGT
TAGAGCGAGAGAAGGTGATACATTGTTTCCTGTAGGAGCTACTTTCTATGCACATGTTACATAATATAAAGATGTGCAGGCTATGA
GGGAACCCGATAGGTGTTACAGCTGGGACTTCATTGTGTGATAGTTAAAGAGAGCTGCGGTTGAGTGGGATTACTGCTGTTTAGAA
GCAAGGCTAGACCTCGGTCCCATTCTGCCACTCGCTCGCAGGTGGGGTGTTCTTCAGAAAGTTGCTAAAGATCTTCAAACTTGGTT
TTGTTTATGAGATGAAAGTAGAAACAACTCCTTCACAGGAACCATGCGTGCTTACGTAAAAAACTGACCTCTTAGAAAGGCAGCTG
CCATAGTCCTGGCACATCTGCGTCATTAAGTGCTCATTCGGACACGTGTGTAGAAAGAGCCATGGAGATGACTGTTTTTTTCCTGA
GAGCAGAAGCTTGCAGGTACTTTGTCAACAGCACCTAGAAGGATGCCCATGTATAGCAGACAGGCAGCAGTTACTTGTACAGTGAG
CAGGTGGTCAGTGCTCAGAAGCTGGAAGGATCATCTAAGGAGGCACTTGTCCTCCCATGGTGGCGCTAAGCAAGTCACTGGAGTGG
CCACCAGGTCTCCACCTGTCACTGCTGCTCACTCCCCACCAGCCTGCTCCTCTTTGCTTCTCCAGCCTGGCATGACTGCCAGCCAG
GACAGCCACATCCCAGACCTGGCTGTACACTGTAGTGTAGCTCTTAGACATAGCACCAATCTATTCTGGGTTGAAACTGGCTTTTC
TGCCTTAGTTTCTAAACCTATTTTAACTGTCCAATTGCAAAATAACACTTAAAGAAACTAAAAAGGTTTAAGCAAAATAGACATTT
ATTTATTTTAAAAACTGAAAATTCTAAGGGAAGAAGTAGAAAAAAGAAACCCAAACAAACAAACAAATTTGCCAAATCCGTAGAAA
AACTCCCATTGGGCTTTTTAGCATCTCGACTTCTACCTTAGAGGCCACTCAGCAGCAGCAGGCTCACTGTGAGAAGACCTGCATTC
CCAAAGCTGACATCAAAGCTTATGAGACACCCAACTGGCAGTTCCTAGGCCTTTGGTACTCTGAGTTGTTCCACTGCCTGCCCCTG
CAGCAGTGAGACAGGGGATAGGGTTGAGCCCTGTGTCCGTCAGTCGCATACTCTGCCGCTGGTTCTACAAGTGGGATTAGTCACTG
ACTAGGAACATGGGGAATGAACTGAGGTTAGGATTCACTGAGCAAAGAGCTATGCGCTAGTGACACGCGGATGCTCCAAGATCACA
AGGATTAGCTCATTCGTCCATCCCGTGACCAGCCTACAGTTTCTGAACTCTCATTCCTAACAGTTGGTAAACCCGGGTTCCTCTCT
TGAGAATGACTGTTCCATGCTACTCCTAATATATGAAACTTTTGTTTTTAAAATAAACCAAAAAGTTTTTTGTTTATTTACAGATA
TCACTTGCATTTAATAAAATTTCAAGAAACACAAAGTTATAGGTAGTAAGTCCCCTTCTGTCCAAGATTCCTTTGTTCCCTGGAGA
GGAGAGCTGTTAGGGGCTTCTCAAGTGCACTCCCAACCGTGCCTGCCGTGTGTTTACCATGTGTGTTTATACCTGCTGGCACACTT
CCACTCACGCTTCCTCGTGTAAGTAAGCATTAGTGTAGCGCTTGTGAGCACGGATGTAGTCTACTAATCTATGAACCATCTGCTGC
TTACTTTTTTCACTTACTGAGTCTGAAATTTGCTCAGATTAACAGCAAACTCCAGAGCTGGCAAATCCAGATTCAAGTCCAGCTCT
GTCCCCTGAGTAACTGTGTAACCTTGAGCTAACTGACCACCTTCTCTGTGCCCCTATCCAACTAGCATAAGAACAACTCCATGTCT
CATAGTGTTGTTGGGAGTACTAATTGAATTAGTGTTGAAAGAGCAATGTCTGCTCCAGGGAAGAGTTCTGATGTTGAATTTCCTAC
CCATCTACTCTGTAAAGGCTGTGTGCTTTCCATGGCTGCTTGGGGCATGCTGTCTTCTCTAAGCTCCATCTGTCTTCTATCACAGG
CCAACCTTAGTAACCCTGCATGATAGTAACTTCTCCCTTATCCTGGGGTCTTTGTCACTTTGGGATGTTTTGTTATTGAGATAGCT
TTTTAAAAATACTTATTCATTTTTATTTTATGTGTCTGAGTGTTTTTGCATACATGTATGTATGTGCACCATATGTATGCAGTGCC
CACGGGGGCCAGAAGAGGCGTTGGAATCCCTGAAACTGGCGTTGCAGTTGGTTTTGAGCAGCCATGGGGATTTGCTAGAAGAGCA
GAAAGTGTTTTTAACCACAGAGCCATTTCTCCAGCCCAGAGATACCATTTTAAAATTATAATCTGTGACTGTTTTGTTTTGTTTTG
TTTTGTTGAAACTAGGTTTTACCATGAAGCCCCCTAGCTGGCCAGGAACCCACTGTATAGACAAGTCTGGAATGGGACTCACAGAG
GTTGGCCTGCCTCTGCCTCCCAAGTGCTGAGATTAAATGCATGTGCCACTGTGTCTAACCCTTTCTTAATGTAGTGTGTTTATTCT
TTGAAGAGTCATAAACTAGATTTTCACCATATTCACTTCCCACTGTTCTTTTTAGCTCTTCTGAGGTCTACCTCCTACCTCCTCTC
AGTTTCTTCCTTCCTTCCTCCCTCCCTTCCTTCCTTCCTCCCTTCCTTCCTTCCTTCCTTAATTCATTCATTCCTTTCTTTCTGCC
TATGTACTAATGGATATGAGACTATCCACTGACCATGGTCATTCCTATAAACATTTTCCTTTCATAAATGTTACTCTATGCATGTG
GAGGTCAGACGCAGCTTTCTAAGGTCAGTTCTCACTTTCTGCCTGTCTCTCTTGTTCCCCACTGTGATGCATCCTCCAGACTTGAT
GGCCCTGGAGCTCCCAGCTGGTTCTCCTGTCTCTGCCTCCCATCTCTCTATGGGGTGTTGGGATTATAGATGTGCATCACCTAATA
GGGCTTTTTACCTGGGTCCCAGGGTCTCAAACTCAAGTTGCTGTGATAGGCATATATAACAATGCTTTTACTCATGAGCCTCCCTG
GATGCTGAATTTTTTTTTATCATAGCTTTATTTTGACTCTTTATTGTGCCATTTGCTTCTTATATGTTTAATGATGTGTTTTTCAA
TGTGATGACCCTTTCAGAGAGTCTGCCCTGAGTTGTTCCCAGGCCTCGCCCACTTGAGTGTGTTCTGGGGATGCCTTCCTAATGAG
CCCCATGCGGACTCTGATGGCTGCCGTACTATGTCTTACTCAGATGTGCCCCTCCTCATTTCTAGACCTTATTTCATGTGCATAAG
AACTGTGTCTTTTTAGATTCCCTAACACTGTGCTCAGTTAAATTCCTTTTCATTCTTGGGTACTAGGTGATTACCCCAGTTAGCAA
TAGAAATATGCAATTAGTATTAGACATAAGAGGAAACATGTCTCTCCCGTTCATCTCTTACTGAGCTCTTGCTGTGTCTCACAGCC
AATCATATTCAGCTCCACCAGGGAGGCGTTGGTATCCTAGTTTTCAATTGGGTTTGATGAACACTTGGCCAAAAACAGCTTGTCTG
TCTACCTTTCTTATGTACATGCACACACACATATCTCCTAATTACTTTCCCCAGCCTAGACTTCTCCAGAAAACCAGGCTTCAGAT
CTGGCTACTTGGTCGACTTTACCATTTGGATACCTGGTAGGCTTGCTGAGCTCAAAGTTGAATGCTTGATTCCCTACTTGAAGTTG
TCCACTCTTAGAAAATGGTAACCCATCCCCCAGTTTCTCAGACCAAGCGGTCTTGAAGTTATTCTTGACGCTCCTCTTCTTGATCA
CCACTTATAATCTACCCTACTGGCTATTTCTTCAAACTAATTAGAATCTACTTGCTTGTTGCCAGCTCCACCCCATGAGCCCTGGT
ATAAACCATCCGCCTCCTAATGGCTGCCATTTGCATGGGTGATGCCTGTCTCAAGCAGGTATTGCTAACTGATTACAAATTTAGGG
TTTGCTCTATCATTTTTTTTGTTAGATAGCATTTATCTTTTTGTACTTTTCAAAGTAGTTTTCTCTTCTTCACACTCACTAAAAAA
TATTTCAGACTAGTTTAAATGTTTCTAATCTAAAAACACAAAGTCAAAAAATGCTCTGAAAGAACCAGGGGAAAATGAATGGGGGT
GGGTGGGGGGGTAAAGTACTTGTCATACAAGCATGAAGAGCTTGAGTCCCCAGAACCCACACGACAGCTCAATGCAGTATAGTAGT
GCAAGGCTCTATAATCTCATTGCTCCTGTACAGAAATGGGAGGTAGGCACTGGAGACTCCCTGGAAATTTACCTGTGGTCCTGAGC
GTGTCAGCTGAGTGCAGTTCTGGATCCTCTTCTCATCCGGGAGCCACGCATCACTCCTGCCATGATTTACTGTGATATTTGAACGG
TGCTTGTCACCAACCAGCAGAAGTGTTGCATGCCAGCTCTCGTGTCCGCTAGACCTCGCTCCCCCACTGCTAACTGACTGGGCTCA
TCCTCCATCTCCTGCTCTGTCCTGAAATGGCTCATCTGTCCAAGGGACCTGATTCCTTAGCAGAGTCGCTTTTCCTAGGTTTTCCT
GAGGCTCCATCATTGATCTTTTTTGTTGTTGTTGTTTTTAAGTCTTGAGTTCAGAGCCTATTTCACACATCTCATTGTTGCCATCA
TCACAGACTCTCCTTCCCTCCCCATCCTAGGTTTGTATCTCCTTTTCCCAATAAAATGCAAACCCTCATTGCCAATAACACTAGAA
GTGCCTACTCAGTTGATTGGTCCTACAATACTTCCAAACACATGGGGAGATAGTATGGTTTCAGGATGACGACGCCAGTATCAGCC
CTAGCAACTTAACAAAGAGTTGCGTTTTCTTTTTATTTCTTTAACCAAAATTCAGCAGGGAATAATCAACCAGAATAGCGTTTTCA
TGGGTTACATTCTTTTCCTTCTAGGAAACTGCGTTACCATTGAACTATAGGGTTAGAATACTTTGTTTATGATGAATCTTAAGAGT
TTTCCTATTCCTGCTGATTTTGTTTTATTTTTGAATGGTGGCATGTATCGGCCTCAATTACCTTCCATTTGTGTGAGATAAACTGG
ACGATTTGGGAACTATCATAAAGTCGGTTTGCTTTATTACCTTTTAATCAGAGAGAGGAACAAAGAGTTCTGGATGGTTATTCTG
GAATGTGAACCCAGTAAGCTCACGTTGTCACATGGGGTGTTCCATTTCTATATTTGGAGCCCTCTTAGTCTTCCTCACAGTCACAG
AGTACACACATATATGGTTCTCGCACATAGGCTTGGTGCTGGCTCTGCGGTCACAGACACGAATGAGTTTATTCCTCTGCCCCGAG
GTTACAGAGAGTAGCCAAGCAGCAGACACGTATGAGATGATACTGGGGTGTTTCCTTGGAATTTGGGGAGGATGCTCTGCAGTGGG

GTGCATATGTGTATGTGTGTGTATGTTCCTGAAGTGGGTGGCCTTCCCTGAATGCATTCCTACTGTGTAGTCATTCCAAACTTCTG
ATGGTGGAATGCCAGGATCACAGAGCACTTTTCATACTGGATGTCAGAGGCATGGCCCCTTCACAACACACGTGCTTCACTGTCAG
AGCGGTGGGCCTGTCACACTGCGTGTGCCTCAGAGATAACATAATCTTCCAAGCTGGTGATGTCATCCTGGAGAGACAGAGCCAAC
GATGAGCCCCAGGGGATTGTTCCTAACCATTTTAACAAGTCTCAGCCCTAAAGTCTTTATGGTTCTTATCAAGTTTTTTGACAAGA
AGTGGTAGATAACAAATACTTTAAATTCAAATTAATCTTGAAAACTGGAGCGAAGTGCCAAACTCTTCAGCCTTGATGTTGAACAG
AGACACACACATCCAGAAAAAAAACAATGAATCCACAAAAGTACAGAGCAGAGGGGTGAGTCAGCAGCAGTACATGCGGAGAGAGT
AGAGTTGACGTGCCTAGCAAGAGCTAGCAAGGCCTGCAGCTGCCAAAGAAGTCGCTCCTCACTAGGCAACAGTAATGGAAATATGA
TGTATAAGAGCCAAGAGGTGATAGTCTCATGGCCACCCTGCAGGAAGATCACACTAGATGCGTGCAGCCTGCTGGGCAGAGCACAG
TAGTCGGCTGTGGGCGGGGCGGGGCCAGATCAGGATGGTTGTGTGACCACACTGGAATGGCTTACAGGATGGTCCTGGCTGTCCTC
GGTGTTTGAATAGCTATTATAAGAAAATGGAAATGGACCCCACCATCACAAGACAGGGGAAAGAGGGAGCGGTGAGCTGAAGTCCC
AGAAGACACCAGCTTCGTGGGAATCACAGCCACCACAGGACGGGGTGACTTTGTCGTCACTGCTCTGAACTAACCTGAAAGACGGT
AGAGGAAGAAAGGGAGGCTGCGTGGGCTTCAGCTTCACAGCCTCGGAGTCTCTGGCTCTCCATCCCTGTTTTATATGGCATTAGAC
AGAGTTTTGCTTAGTAAATCATACCTTTGTAACAAGGGCTGATCAAGATTTGGGTACAAGGAAGTTATAATTTCATAATTTCATGA
TAGATAAGAAAATATAACAATAGATGCTAGTGACTTGCTGGCCATTTTCTCCATGGGTCAAAGTGCATGACTTATTTTTTTACACT
AGTATGTCAGTTTATTAACACAGGCCGAACAGTTTAGTCACAGCTTGAAAGGGTTTGGTTTGGTTTGGTCCTGAAAGACCAGGTAC
AGAGTGATACCAGATTTTTCCAACAACTTGCTGGCATTATCCTAGAAATCAGTAGAAGTTTTGGTGTTTACCCCTCCATGCCTTGG
TCTACATCATGCTATGCAGATGGCACACTGCTTTGGTTAACCTCAGTCTGACACGCAGATCGTGAGTTCTCTTCTGCCATGCTCCT
GTCTGGGGGCGTGATGTGACCGATGAAGTGTCACCAAGAAATAAGACAAAATTTCCCAGGTCTACTCACTGGCAATTCTCTTTATT
TAGTAGGGGTTTTCCACAGGAAATTGCAGGAGAAGAGTTGAAGTGCTCTTTGTCTTGTGTACTTTCCTCTGACTTTTCTCCTTCCC
AGAGTGGCCCACATTGCCTTGTAAAGGAGCTGTGTTGACCATGATACTGATCAAAGTCAGAGTGCGTTCTGCCAGTCAGCTAAAGC
CTGATTGCTCCTCTGCAGTGGACTTCTGTAGTTAAACTCTGTTTTCCAAACATGAAAACACATAAACAAGGACTGGAGATATGGCT
CAGCAGCTAAGATCACATACTTCTTCTGCAGAAGACCCAGGTTCCGTTCTAAGCACCCACATCGGGCAACTCACAACTGCCTATAA
CTCCAGCTCCAGGGGACCCAACACACTCCTCTGGCCTCTATGTGCACCTGCACTCATGGGCACATATTCACACATATATACATAAT
TTAAAATAAAAATCTTTTAAAAATTAAGGAACATATACCCAGAGAGTAAGTATTTGTCTCTAAGAAGAAATAAATCGGTGATATTA
ATCTGAAACAAATTTGGACATCACAGAATCAAAAATATTTCTGATTATCCTAAATTAAGATCAAAATCATTTTAAGGGAATTCTGG
AGAGGTGGCTCATTGGCTAAGCTCTTCTCTGCTCTTGCAAAAGACCCAGGTTTAGCACCCAGGACCCACATGGCAGCTTGGCAACT
CACAGCCATCTGTTGTCCCAGTGCCAGGAGACCTGACACCCTCTTCTGGCCTCTGCAAGCACCTGCACACACATGGTGCACGTATT
TCAGGGAAAACAATTATAGACATAAAATTCAAATTAATTTTAAAATGTCATTGTAAGATCATGCTAGATAAATTTTGTATAAATCA
TGTCTGTTTTCAGACAGGGATTCTGTACAAATCTAACATGCATTTTAGACGACTTAGTTTTGAAGTTTACACAAACTTTTTTTAAA
ACTTTGCTTAAAATAGCTAATTATGACTTCATTTGCCTGTTTTGGCCCTGCCCCCCATGGCCAAAGCCTGCTGACATCCCCAGGAC
CAATAGTCCCTTATTCTCAGTACTATAATAAGTACTGAGCTCCCAGGGCAGAGCCTGCACTGCAGTCACTGAAAAGCACTGTGTTG
AGTACCCAGACCCAGCCTGGATGGGAAAAGTTAATATCATTGCCTAACATGAAGGTTTTCTTTGTCTCTAAATTTTGATTTTTCCA
TGCTATCAGAGGCCCCCTTGCAACAGGTAATCTGAGTGTTGTCTGTTTTCTCCGTCCGTCATCGTGGTTAAGTGTATTTTAGATGC
TCACAATTGTAAAGCTAAATTGACTTGACAAGGTTCTTTCCGACCCAAATTCTGTGATGTCCGACTCTTTTTTTCTTCTTTTATTT
TGGATTTGTGATTGTTGACCATTGTCCTGTATGTCTTACATATATACTTTAGGGAACCATAAGCATTTTAGAGCTGGAAGCCATGC
TAGCTGTGGGCTAGGACTAGACATTTTGCAAAGAAAGCATTAGGAGACCAAAGTGGTTAGTGATTCAAACAAATTCATAGCAGTAG
CTAAAGACTAAAATTCTGACCATCTTCAGAGGACTAAAAATAATCAAAGTGTTATCTGAAAATTTGTAACTCCAAGCCAGGTATAT
ATATATATATATATATATATATATATATATATATATGTATGTATATATATTTACCCCAAACAAATTAAAGCTTCCTATAATTCTGT
ACTCATATATGTATATGGGGTGTGTGTGGAGAGAGAGAGAGACAGGGAAAGAGAAGGGGAATTAAATATTTTTGATAAACGTAA
GCTTTGATAAGATCTGCCTGACACAAAATACAAAATGCATTTGGTATAAACAGCATCATTCTCCCTTTATACCAAAAACGGAAATT
TCTTCGCATTCTATACATTTGAGAATGACACGTCGGGNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNCCGTGTTTGCTGCTCTGTACT
GGCTATAACCTGCACAAGTAATGGCCTCAATACAACTGTTTTCTAATAAAAGCATGCGTGATGTTATACCTCTAAAGTAAGATTGG
TTTATGTAGGTCTGCCATTGAGAGTGGTTATGGAGGATTCCTGTCCAGCATTGGGACTGTATCTAAAAACAGCAGTCTAGCTAGA
AAAAAACAGCACCTGTACAAAAAAACAAAAACAAAAACAAAAAAAGCAGCAGACTCAAAATAGGAATCTCACAGGTCTAATGGGAA
TCCTGAGTACTTCTGCTGCAGAAAAGTATCCTTAAGAGAGCCCAGGTGAAATAGTGTTCGAAAGAAAAACAGGGACATGTTGTGCA
AAAAAGATAGACTATTGCTTTGTTTGTAAGGAGATGGGTCAGCCGTACGCGTTTTAATAGAGTGGTAATGAGCATCACTAATCCCT
GAGCTTCAGAGCACAGACTCGGCGTCTGTCTGCAAAGGGGGCTGAGTTGGAGCACTGTTGTGATTGATTCCTCTTCAGTTAGACTT
AGAGTTCAAGCTAAGATGGATAGGCAAGGCAGTGAGATCAGAGGAAAGGGCTGAGCACGGGGGGAGGGGCAGGGGACGAGGAGGGA
AGTGCCCTGGCTCTGAAATTTGGCAGTTAAGTGGAGATAAGAGGCAGCGCACTCCCACACACTAGGACGGTTTTGGTACAGTGGCT
CTGTTCAACAGGAGGCAACGTTAGGTTTAAGAAAAAAGGTAAGCTGGGCGTGGTGGTGCACGCCTTTAATCCTAGCACTCGGGAAG
CAGAGGCAGGCGGATTTCTGAGTTCAAGACCAGCCTGGTCTTCAGAGTGAGTTCCAGGACAGCCAGGGCTACACAGAGAAACCCTG
TCTCGAAAAACCAAAAAAAAACAAAACAAAAAAAAAAGAAGGAAGGTAATTAGTTAGGCTTGGGCACATGGAGTGATAAACTGCTGT
GAACAGAAGGGCTTCCTGCTAAGGGCTAGAAATGTCACACATGCTTATCTGAGAGATGAAAACACTGGGTTATTGGAGGGGGCTGT
GAGAGCCGAGCGAGCACAGCCCGGGCATGTGTTTGAGGACAGGAGCCTAAGGAGAGCCAGAGGTATCAGGTCACAGTGGTGCTGGGG
CTGCAGGCGGTTGTGAGCCTCTCCAAACAACGCGGCCATGAACAACCAAATTCAGATCCACTGCAAGAGCAGCAGGCTCTTAACCA
ACAAGCATCTGTCCAGCCTGTAGACTCTAAGTTAGTATCATATGGAGTAGATGACACAACTCCATCTTGTGGATGGATATCAGATA
ATGTGATGACTTCCAGAAAGAGACTAAGAGTCGTGGACCGGCGCTTGTGAGCTCTTTGAGAAGGGGAATGTAGCCAAGCAGCTGGT
GATGGGGGAGAAGGGAGGTCAGAGGGACCACGTTTCAGGGATTGTCATAGGTCAAACCATAAGTGACTCATGGGTTTGGAAACTTA
AGGCAGTCAGAACCGTTAAGCAACCTTTATTTGAAGACTAGTAGAAGTACTTAACTTTCAGAAGACTTCGATGTGAGAAGCTGTTT
CTCCCTGGTGACTAGAGTTGTTCTTGAACGCTTGGGTGTAGAGAAAGGAATTCAAGCAAGAGTGGGTAGACTCTTTTAAGAACCTT
TCTCACAGACTTAGAAGAAGAAAAAAAAAAAAAAAAACGTCAAATGAAACAAACAACCCCCCACCCCAAGCCAGCCCTGTTTTCTTG
GAGTGGCACCTGCCGCCATGTGTTTGAATAAATGATAGCCTTTACCATCATCAAGAAAGGGCTGCTATTTTTAGAATAAGAAAGCA
AAGGAACTATGTGGATAGCTAAGGGGAAGGGTCATTTAGATGCTTTGTAGGGGTGGAGAGTAATTAGGGGATCTAGAATAAATATT
GTGGGTAAAACAAGATGGCTTTTCCTGGAAAAGGGAAGAGACACAGTTCTCTGAATTAGTGACAAGAACAGGTCCTGAGCTTCAGT
AACAGAAAGGAGAGACATTTGTGGAGGACCATTGTGGAGGCTTCTCGTTAGATCTCAGGAAATCCCAGCAGCAGATGTCTGTGAGG
GCAAGAAGAGATAAAGGAGAATTATTTTGCATCAAGAACTGTTCTAAGCAATTTAAATTATCTCTTCTTAATCCTTGTGAAAACTC
AGAGACTATGACATTGAAAGTTAAACAACTTTCCCAGAGCCTGATATCAGCTTGTTATGGAGGGTCTAGGATTCAGAGCTATTCTG

TCTGACTCCAACGCCTGTGTTCTTATGGTGAGAGGACTCACTAGTTTCAGGTCTTGGAAGTTCAGCCCTATATCATAGCAGGATGA
GAACTTTGAGGGAGGTCCATCTGTCCATCAGAGAAGGATGCTCTAAIATATTGGTTCTCAACCTACCTAATGCTGTGACTCTTTAA
TACAGTTCCTCATGTTGTGGTGACCCCCAACTATAAACTTATCTTTGTTGCTACTTCATTGCTGTAATTCTGCTACTGTTATGAAT
CATAATGTAAATATCTGATAGCAGGATGATCTTGGGTAACCCCTGTAAAAGGGTGGTTCAGCCCCCCAAAGAGTCTCTACCACGGG
TTGAAAACTGCTGCTCTCGTGAGAAATTGTGAGGCTGAGAAATTGGGGTGGAACAAAACTGTAGACCCTGTAAGTCTGACAGAAGT
TAAGATAAATGGACAGAAGACAATGTGAGGCAGATGCAGGAGACAAGAGGCCTCAGGTCAGCAGCCAACTGGAAACAGGAGGGAAG
GCAAAGGAGGGTTCTGACTTCATGGGGACAGCTGTTGGCTTGACTTGACTTTCTGGTGGTGATGACTGAAGAGTCTATTTCCACAG
CTGCTGTGATTTCTTTGGAGGTTTCGGTACCAATTCTGGTCTAGAGACAAGACCAGGGTCCTAGACACACAGTTAAGCTTAGGTTG
TTCTCTGGTATTTAATAAACAAGCTGTAACTCAAATAGCTTGTTGAGAGGCTGTGTTGGGATCCAGGTATCTTTAAAGGCAAAAGA
AAAAGCATATATCACTTTAATTTCTTCCTACCTAATATAACTGTTTACACTTTAGTAGTTTGCCTTCGATAAAGAACATTTAGTGC
TATCTCAATTGTTAGTCACGCCCACGTTAACAAGGTAAAGTTACAGGAAGTACTGTGAGCACAAGGAATAGGAAGGACGGGTGAAA
GGAACGCTTCTGTTCTTATAGTCCTTCCATTCTCACATAGAAGATAGGACATTATTGATACAGGGGACATGTTACATACTTAACAG
TTATTAACATACAGAGAAAAGATGAAGCAGGGCAGAGAGCAGAAGATGGGGTGGTCGGAGCTTAGGTGGAGGAAGATTCGAAAAAC
ACTTGTCTGAGAAGAGAGCTGTGCACAGACTGGAGCTGGACAGAAGACAGCAGACATCCTGGAACAATGTCCCAGCTAAGCACGCA
AGTCCAAAAGCTCTCTGGTGGGAGCCCTCTTGGCAAGTTCTGGAACAGCAGGGTCTACTATAGATGCATTGGAAATACAAGAATCA
AGGTTGAATCAGGACCCCACTCCCCCAAAACCCCCGAGGCTCCCTCAGCCGCTGTCGAGGCTTTTTAAGAATAGAGACATTAAAAT
GTTTTATTTACTTCTGTTCTTTAACATGGGAAATTGTAACCATGTATTCCCAGATCTGTGTGTTGAGGCCCTTGTGCTAATCACTC
AATTAACATGGGAAATTGTCATGTATTCACACAACTGTGTGTTGAGCCCCTGTGCTAATCACCCAGTTCCAAGTGTTTTCATTTTT
GGAATTTTGAACTGAGGAATGATGACTTAGCATGTCCTGACTTGAACAAAACTCACTCAGAACAGGTTCTGAGGAGGCAGGCAGGG
AGCCAGGGAGTAGCCTGAGAAGTGGTTATTTGGGGCTTCGTTTGAAGATCTGGCTTGATTTTATTTATTTTCTAATTTTTTTCAAA
AGAAGAACTGAAAAGGCAAATAGAACAGTGAAAAGAGAAAGGTAATAAAGAGGACCCAGCCCAGGGAGCACCCCTTCACACTTCAC
CTTCTACCTAGACGTGTACCTGCCTGGCAAAGGGCTGGCAGAGGGAATATTTCTAACACCATCCTATAAATGACAAGCCACATTCC
TATGCTCTTATGGAATCATGGAGTATTAAGCCCCCACACAAAGGTGAGGATTTGTCACATGTCACCAAAGCATTGAGACCCAATTG
TACCTGAGGTACCTAGGGGCGCTTGACCTCTAGATATTTATGATGCATGTGATAAGTTTGAAAATTAAAAGGAAACCAAAAAGAAA
ACAAGGAAGCCCTGCATCAAGGCTGATGAGGTGAAACTTTCAGAACAGGGGTACATGCAGGTAGAGGTGTTTCTCTCTTCTCTTCT
CTTCTCTTCTCTTCTCTTCTCTTCTCTTCTCTTCTCTTCTTTTTTCTCTTCTCTCTTCTCTTCTCTTCTCTTCTCTTCTCTTCGTG
TGTGTGTGTGTGTGTGTGTGTGTGTGTGTGTGTGTGTGTGTGTGTATAGACTAGAGTTTATTCAGGGCCTGGGGAGGGGAGTTAAG
AGGGTAGTGGAGTCAGAGAAAGGCAGAGAGAGGGAGAGAGTAGAGAAGTAGTTGCTGGCCATGACCATGTGGAGAGAGAGGAGAAG
GGAATGGGGAGAGAAGGGGAGCAAGAGGGAGAGGCAAGAGAGCAAGGCAAGAGTGAGGTGTTACCCTCTTTCAGAGCAGGATGTCT
GCAGGTAGAGGTATTACCTTCTTTCAGAGCAGAGGTGTGGGCAGTTAGAGGTGTTACCCTCTTTCAGGGCAGGGGTGTAGGCAGAT
AGAGGTTTTACTTGCCTGCTCTATATGAAACCCTCCTATCTGAGTGGTGGTTTGCCATTGTAGTTGCAGCCTGACTCTTGAGATGT
TGATCACACTAGTAGGGAGTGTCAGGAACAGTGCAAATCCATGAACCCTGGTGAAGGTCCAAGTGTGCCAGCAATAGTGGTGAATC
AAAGAGGTTTTCATGTATCCTGTCCCTGCTTTGCATTCCCACACCAATATTCAGAGGGCTAAGAGTGAACTCCACAGTGTTCTGCA
TCTTGGCTGTCTCTCCAAAGCTTACATCACTTGCTCAGGCTGTCTAAACTTGCCTTCTTATCTGCAAGTTAGGGTACTACTAGTGC
CAACCCTGCCTATCTCAGGGTCATCAGAAAGCCCAGATGTTCTCCATAGATAAGAGGGAAGGACAAANNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNCAAGACGAGACCAGTGTCATGAGTCCTTCCTATGCCATTAGCTATAATCCCGTTTAGCAG
GCTTGATCTGTGCTTCCTGGTTGCACAAATAAATAATAAAGAACAGTAAACTGACTCCCTGTCATCCTTGAGAGGACATACATTAT
TAAACCAATATACTATTTCGTCTCTCTCCCTCCCTCCCTCCTTTCCTTCTTTATAATTTGACTTGACTCAAGAAGAGTAAGCAAGT
AGCCTTTTTTTTTTTTTTTCTTTAAATGGAAAAGACTTTCAGGATGACAAGGGGACTCTTGCTCCTCCTTTTCACACCTAGCATGA
GGCCTTTCTCACAATATGCCTCACCAGTGCCAACAAACAAGCAGGAGTCTCTGTAGCTGTACCTGTGCTCAGATCGGTGATTACAG
CCATGGCGCATTAAAGCCAGTTAGAGAGAGAAAAATAACCGAGCCGAGTGTTGACATGGTGCCGTGGAAATGTGAATAGACTTTGG
GGAGGCAATGGCAGACCCCGAGTTCAGGGCACCACCACCTTACCCCAAACCATTGGAACCAGGTGGTGCATGTTACTTGAGCTTGC
AAAGGGCTGGTTCTTTCCCCGGTGATATGGGGTGCCACTACTGACCCCACATGGCTATTGTGGGGATTTCTCCCTCCTCGGCTGTA
GAGACAGAATATGCACATAGACTTAGATGAGCACGTGCTCTGGGAAATGAACAAGCATGCGAAGTAAGGCGTTATTTAACTCTTAC
TAGCCAGTCACATATCACTTTCCTACCCTATGTCCTCCAGTCATTCTCGAAAAGGGCACAGTCGTGGAGCCATGATGGGCTGACAT
TGTGAGGAAGGTGGGAAACACAAGAGACACGGGCTATTGGTGCAGCTGCTTGAAGGAACTCCTCCTGTGCGCTGTTAATGAGCAGG
ATGAGGGCTCGAGAGCACTGAGCACCCTGCGAGCATGACTGCGCTCGACCTGCTGAGAATGCCTTTCGAGCATGCGCTCTACCCGC
CCGCAGAGAACGCCTGCCAAGCTGCCTCATCCTCCACTTGCTCCTTGCTTACTCAGCTCAACTCTGCCTTCAAATCTCAGTCCGCA
TGAGTTAAACACACGGAAGCTTTCTCTCACTTCCCAACCGTGCCAGACACTGTTATTAGACATTCTGTTTGATGAGTTCTCCAGTT
AGGTTTTACCTGTATTGTTGAAGTTATCCAGTTAGCGTCAGTCCCACTGTTTAATTCCGTGAGAGAAAGAAATTGGGCTTGTTCTG
AACGAGGTGTAGCCCCCACTCTCTGACCTTCTACAGTGTGGGGCTCTTAGAAAGGGGCGTAACAAACTAAACTGGTAAACTTTAGT
ACTCAGTGAAATAATGGGCTAGATAAGAAACAATTTTCTGAGATTGTTGATATCATCAGGAACCAAGTGTTGTCTTAACTCCTTTA
ATCTTAAAACAGTCCGGTGATACTTCGTCATTAATTTGATTTTTGAAGGTGGAGAAGCTGACACACGTCACTCCACGACAGCTGGG
GGATTTGAGCCTGGCTTTGAGAGATCTTAACCTAACCATGACCTGCTGTGGTAAAGAGCAAGGTTGGGTTCACAAGCAGAGCCAGG
CCCTGTTAGAACCCTGAGGATGTTCGAAAGAAAAAATCAGCAGAAAAGAATGATTTTAGGTATGATGTCTTACATTTATGCTGCTT
CTGCTTTCTGTCAGAGTACGTTAGCCGAATTCACCCAGAGAGCAGCCTGGCTTTTGAAACTGTGATTCTGAGGAAGTTCTAGGCCA
CTCATCTCTTGTTACCCCTTTGAAGAAACAGCAAATGCTTTTCCACAGTAGGTCAGGGCTATCTGAGGTTAAAAAAAAAAAAAAGT
ACGTGGCTGACTAAGAGGGCTATACCTGAGCCCATGACTTGTAGTTTTTATTCTAAAAGGAGCTCTGACCCATGGACCCAGAATTC
TAGAAGAGTTTTCCTTCATCAGGGAAGGCCAAGGAAGTCTGATTGTCCCCAAGAAGGGCAGCTGACACTAGAATAACATAGTATTT
TGTAGAAAAATAAAACATAGCAAAAGAAAGAAAATAAATGTATTTGTAGAAAACTAACAAAACACTGCAATGTAGGAAAAAAGAAA
AACCTTCCCCTTGGAATTGATGTGGGCCAAGTATCTTCCAGCCACTTGCTTCAAATGGACCTATTAGACATCCTCTAAGTGAGGGA
GCATCTGACAGTCTGAGATAGCCCCTGAGGGTTGTCCTCTCCTCTGCTGAGTACTGTTGTGAAGTGTTTGTGTACTTGAATGTATA
TATTCCTCATTAACAGCCCCTTGAACATTACTGAACAGGTGAAGAGACCAAGCAAGGTCACACAGACTGGAACTGCCTCTCGCCCC
CAGCCAGCCTAGCCTGTCTCTGAAGTCCACACTCTTCCCATTCCGAGCAGCTGACTATGCAGGATGGCTCCTTTGGCTTCTTACCT
GTGCTTGAGTGCTTACAATTAAGAGTCTCTGTTCAGATTCTACTCTCAGAAAGACCTTCCTAGTAGACAGTTGCCCTCTATAAACT
TTATCTTCAAGAGACGTAATACTTCCATTCCCTTCCCAGAATAAGTTTCTACATTGGTGTTGAATACCTCTTGGCCACTATGGCAT
GATAAATGATGGTTCCAAGAAATCGTTGTCAAGGTTTGCCTGTGCCGCACAGCTGTCCCCACAAGTAAGGCTTTCTTTCTCTCAGA
TGGCATCTTGGAAAGTACAGAAAGCCCAGGGCCCTCCCTGTTTTCATTCTGTGGTCTGTGTAAAAACCAAATGCTACCATCCCGAC
TACACTCTTTCAGATGGATCAAACAGCACATTGGTCACTGGCAGATCTTTCAGGTCCCTCACCTCAGAGTCCTCCCAGTTCCTCAT
TCTTGCCGCCCTTCAACCATGAAGTTAGTCGTAAGAAAAACAGTAAGTGCTTCTTGAATGCACCTCCCACCTCTTTCCATCAGCGC
CAGTGACTAACTAGCCTGTCAGCGCAGCTTCAGAAGGCTCGGATTCCAAACTCTCAGGCCTTCTCCCATGAGGCCTTTCTGCTTAA
GTGGAAAGCCTGCCTGATTTCACCCAAGTGAAGCGCTTCCTTGAGCCTTCCTGTGCTGTGTTCCTGTGCACACTGTCAGTTAAAAG
TGGGATCACGTGACTCGCTGCCTCTGCCGATCATTAGCAGACCGATGTGATCAGCCATCCAGAGCTGGAATCAGCCATCCAGGGAC
TTTGTAGGAAGAGCATCATCAGCCTTGGAGCAAAACCAGAACTTGGTATTCATTGTCCTACACTTAAATGGGGAAGTATTTGGCCT
GTGAAGAAAGACGACTTGCTAAATTCACTTCATTTTAATATCAGAGAGAGTTTTAAGTTCTGTGCCTAAGAAAGCAACTCATGCTT

TGGGACAGTCAAGGTTAAAGTTAGTACAAAACCTTTCTCTGAATTAGGTCACTCTGTAACTATGGGAAGGCAGTCCTGCTGATGGC
CTCGCCGCTCTGGAAGAAGTATGCCCTGGTCTGTTTTATGTTGTGTTGGTTGACAAGCAAATTACACAGGATTAAAGTTTGCTTGG
GCTGGGGTTTGAAGCCTGGGAGGGTTCAGGTTGTTGGTCAGCCATATCTGACAGGAACTTTGCAGCCCAGGACAAGACAGGAAATT
ACATAACAAGAGAGGCCATCTTGGCTGAAGTATCTCTTCCTCTTTGTATAGCTCACAGCTCCGTAGATGAGGGCTGGCTCTTATGG
TGTATTCGATTTTACCTCCTAAAGATCAACTTCCAGACACCATCACTGGATTACATTTCTAGGCTCAAAACGTCTCATGAGTTTCG
GGGTGGGTTCAGAAGCAACTCATTATAATGTTGTATCAGGGCTCAGGCAGGCAGGCCTCCTTGGGGAGTCTAGAGAGCTGGCTCGC
TGGCCAGAAGGGAGACCGTTCGTTGTTCAGGGCAATGGTATCTTTTCTCCATACTCTCTGGGGAGTTACGCGAGCAGTGATCAGCT
TCCCGGCTCTGACCATCAAAGGTTACACTGGAGAAAATCCATATTGAGAGCAAACTCTGTAGAGCCCTGCCTCCGTGGAGTCTGGA
CCCCGCTGATAGTAGTGATATTATAAGTGAGCAGGCTGAGGAAGGCTGTGTGTCTTTACAGGAAGCTCCTTTAGCAGATGCAGGCT
CTTCACGTTCACGTTGACATTTATCCAGAGAGGCGACGTACCTCATGCATTTCTAACAATAGGTGGGATTTATGGCAGGTTCTGAC
TTAATCGAATTCAGAGGGGAGTAATGCCTCCGAGCTACTCATGGTGTGAGTCTCATGGTGTGATTTCCACGGTGTGATTCTCATGG
TGTGGTTCTCATGTTGGGCCATCTTGACGAAAAGTTCTACACTGTAGAGGTCGGGATGGTCAACTAGACAAGAAAGACTTGGTCAT
CTCCTCTCTCCTGTCCTGTCCATTGCTGTTCTTACCACGTTACAAATACACAAGTGAAATGATCGCCTCACATGGAAAACTTAACT
TTGTAAAACAAGAGCTAAACAACCAAATGTGAAACCTACAAATTTTTAGTCAGAAAGTTTGCTTTTTTTATTTAAGAAACTCACAG
ACTCCTGAACATATACAGTATAATGTCAGAACCCATGCCTGGGGAAACTACTGAAGAGTAGTGGGTGTCCTGTTAGGTGATGTGGA
CCAAGCCATGGGCTTATGTCATTAAAAACAGCCTCCAGAGCCAAGCCCCTGAACAAGACTACCTTTCTGGTAATCACATGACAAGG
AGTTAGGACTCAGTCTTTGTTTGTCTCTCTTACTTCATCAGTCACCCATTCCCTTCTCGTGCATGGTTTGCCCAGTTTTCATCACT
GAAAATCGTGACAAGGTTTCAGAGAAGAAACAGTGTTGGTTAGTAAGGACCCTTGTGACAGTCCTACCTAATGAGGCATACTCTGT
TTTCAGTGACTTGTTTCCTGGACATATAAACAACCCCCACTACCATTTTGATTTATTTCCATCCTGTCATGTGTTCATTGTATATT
TTTTAAGTTGAGCTATAGTCAAATATGTATATATCTGCAAAATGTTGTATTTATGCTGATTGGTTCCATGTTACAGTCAGGTCAGA
TGTACTAACTGTTGAAAATGTGCTTCTCATATAGATTTGCAACTATGTGGGGCCTGCAAAGGTTATCGTTCAGTTGGTCACAAATG
GAAAAAACATCCACCTGCACGCCCACAGCCTGGTGGGCAAGCACTGTGAGGACGGGGTATGCACCGTAACAGCAGGACCCAAGGAC
ATGGTGGTTGGGTAAGTGGGCTGTGTGGTATGGAGGGAGCGGGAACACGCGGGTGTGAAATCCATAGGCCCCTTTCCTTGTGGACC
TGTGCAAAGCCTGAGAGGGTCTCACACACGCGCAAGCAGATAAAGCCAGGGGAATGTGTGTCCTCTCCTTTGAACTGCTAAGGAAA
TAGAGGTGACCAGATATCTGTCCTGCCTGAGACAATCCTTGCATCTGTAGTCTTGGCCTCTCAAGGATGGGTTTGGACAATGTTAT
AATTATAAGCCCTTGACCTAAGAGGCATTTGTGCCACTTTCAATTATATTTTTCTTCTCCTGGAATTAAAGATGAAGAAAAGAATG
AAACAACTACCTTAACTATATAGAAATGATTACCATGGAGATGCTTTTTGCTTCCTTGAAACAGCATCTCCACTATAGTTTAAGCT
TACCTTGAACTCACATAGATCCTGTTGTCTCTACCTCCCAAGTGTTTGGGTGCTGGGATTAGAGATGTGTACCCCCCCACACACAC
ACAGAGCTCTGATTTTTGCCTATTTGGGATTTTCTTAATAGAGTTCTAGTGGGAACATTTGAGACATGAAGCAGATAAGTAAATAT
TGTTTCAACTCTGCGCTACTTATAAAATGTGTGCTTTTTAAGTCAACAAGACATGCAAATTGGTTTGGGCACTCATTTGATCTGGA
ACATAAGTAGAAATTAGTGTATTCTTTCAAAGTCAGTCAAATACAACAATTTTTAGTGAATGAAATTTTATAGAAGTAATCTTACT
TCAAAAGAATCACAAAATATTTAATAAGACATAATTATGGGCTGAGAGAGGATGAGTTTAGTTTGCAAGTATGATGGCCCAAGTTC
AAACCCCTGGCCTCTCATAGCAGCGCTTTTCAGAGCACAGGCATTTATCCTCCCAGCTCCTGCAGGGGAGCAAGAGGCAAAGACAG
GAGAATCTGGAATCTCATGCAGTAGCTGGCCTGGTGTGGTGGACAGACACCAAACAGATCCCATGTCAAACCTGAAGTTCTCCTCA
GACCTCCATATGCACAGCACATCACATAGTCAGCTGCGTTCGCACACATAAAATAAGGGCACTCACTATGCTAAGGTTTTATATTT
CATTATCCTGAAACACTAAAATAGCAGACAATGTAGTAACTTAAACAGCAAAAAATGATGTAAGCATTCAGTCATCTCAAATACAG
ATAACTCCTATTTTCCAGACTCTTAAAAAGCAAAGGGGATGATTCAGTGGGTAAGTGCACTTTCCACCAAATCTGAGAGCCTGAGT
CCAACTCCTGTAAGCCACATGGCAGGAGAGAACCAACTTCCACAAATTATTCTCCAACCTCCACATGCAACAAAGGTATAAGTGCA
CACACACACCATGTAATATAATTTTTAAATAAAAAAAAGTTAACTAAAATATTTTTAAAGCCAGAAGGTAAGAACTGGGGAGACAG
GTCAGTGGGTAAAGTATTTGCAATGCAAACTTCAGGGCCTGAGTTCTGAGCTCCAGCGCCCACATGAAAATCCAGGTGTTAAAAAA
ACCAAACTGATAAATAAAATAAAACCGGGAAAGGGCAGAGCACAGCGGTGCATGCCTTTAATCCTAGCACTCAGGAGGCAGAGTCT
AAGGCCAGCCTGGTCTACAGAGTGAGTTCCAGGACCGCCAGGGCTACACAGAGAGAGTCTGTCTCACTGCCCCCCAAAGCCAGGTG
TAGTGATGCACCTGGAACCCCAATGTCTGGCCACAGGGATGGAGCAGATCCGCGGGGCTCACAAGCCAGTCAGAAAGCACGGCCCA
GTCCATGACCCTGTCTCAAAAACCCGAGAAGTGACTGAGGAAAATTCCCTGCCTGTGTTCTCTGTGGTGCATGCAGATACATTGTT
GTTAATTTTATTTAATTGTGTACATCATCATTCAGAGTTCTCCAGTCTTAGATCAAGTAGTGTCTGGGAAGACCCCAGTGAATGTG
TGCTTTTTTTAGGAGATTGCTTAGCCAGCCTTGATGAAGTCTGACTCTGTTGCCCTGCAATCTTGGGAGGTTCAGCTTTCAAAGTC
CCAGGCATCATTTCTTCATTTGACGACGAAGATGCTCAAGTGTGAGAAGCTCCCAGAGAGAGGGCCCTTCCCTTTAAGTCATTCAT
CTCTGGTGCTTTTTGACGTTAGGGAGAAAAGTATCCGAATTGGAGGAAGCTGAAGTTAGTTCTCGTGGACTTCAGCTTGTCTCGGC
ATCTCCTGGCTGCCCTGCTTCTCAATCTGCCGAAACTGACATGCAGGGTGACTGTCAGTTTTAGACAACTGATCGGTTAGTGTATT
CCCTGATTGATTGGCCACATCATAGCCAATCGTTTTGAAGCATTTCCGTTCTTTTCGTCTTGCTTAATCTGTCTTACAATCATATG
TTCTTAAGGAAATCACAGGTGAACTTTTCTATCAGTATTTCTGTCAGTAATATTTATTATTATAAAACTTGCAGAGTGTTGCTTCT
CTGTTGGGTTTAATAATCTTTCATGATTTAGAAGAAACTAATGGTGGCTGTGTCTGCTGCTCTGAGGTAGACACAAGGCCTTGGGT
TGCTCTCCCCTGCAGCACTACCGCCACTAAAAACACTAATGGCTGTTTATGCTGGAGCCTAGGTTGCAGCGATGGAGGTAAATTCA
GTACCTGTAAGCAGAGCGGAGAGGTCCGTCGGTGTCTTTTAGTGTAGTGGGGCACGCGGCTCTAGAGTTGGGAGTGTGCATTACTC
ATGATTTAGCAAACATTTTCGACATCCGATTCCTGACTAGGCTCCCTGTGGTTTGATCTAGTCATAGCTGGGTGGATTTTCTAAG
GTGAGGAAACTTTCTCGGAGAGATGCAGTCGAGGAGATGCCGCCTGAGTGATAGGGGGTAACCACAGTAGCTTTTCCCACCCAAGA
ATCTCTCACTGTAGAATGTCTGCAGTCGCTTTCCCGGTTGGCATTTAGCTGAGAGAATTCTGCTCATGGTATTTTCCCCAGACACC
ATTTTCTTCGTGATTCCCTTGTCTCTGTGTCTCTGAGTTTCTAACACGGTCCTACACCTTTTGCCTTTTCTTCTGTTTCTCTTGGA
TGGAAAAGCAGCGTGCTAGCAGCCGGGAAGTCAGAGCTCCTGTATGCTTCTGTCACTCTGCACGGCAGAAGGGATGGAGGCCAAGT
TGCATGACTTTCTAAAGACTTGAATTCCTTTCCTTACAAAATCAGAAAGACACAGGCTCTGTCTATCTCCAGGCACTCAGTGTTTA
ACCCTCTAGACCAAGCCCACATTAGCTAATGTTATAAAACAGCAGGAGGAAACTTGCCACTATCTCCTCATGGTATGACATCGGTT
ATTTTATTTAGTTCTGACTTGTAGGCCAGAAGTTTAGGAAGGCAGCCAAAGGTGTTTCTTAGACTTGTCAATACTAGAAATAGAAC
CAAGATAACATTTTCCAATACTACAGATTAGACTGAATATAGATTCACAAACAAGGCACCAGTCCAAACTATCAACATATGTAAAA
ATGACCCCAAGAAGACCAAGCTTTGAACACATCAAAACCAAACGGACCCCCTTTGTTTACAGTGTCTTTCACACTTTCTTCTCTGT
TTGAATTCTTAATAAGTTTAGTTTCTATTTTTCCAAATTATTTTATCAGTATTAGAACTATCATCTCTTGGGGTTTGATTATGGAT
TTGATTGTTTTAATATAGGGTCATTAACTTCAGTTAGCTTTCATTAGACCTCTTGTCTCTTATGCACTTAGTTAGACTTCACTGCC
TTTCTTAATAAAAAAAAGAAGAAGAATCCTGTTTAGTATTCTATTTTGACCAGAATGTTAATATTTTTAGCAACACTCTAGTTCTC
TGAAATTTTTTGTTCAGTGGAAAATGTCTGTGCATTTCTACTTTGCTGTCGGCCACGTCCACCAAAAGTCACCACACCCTGTTTGC
TTACTGGCAACCTGGGTTGAAAGAGATGTACAGTTCTTTTTATTTCCCCCTCCCAACTAATTTATGTAGCTTATTGCAGTTGGTAA
GCAGGAGAGCAGAGATTCTCAACCTGTCGGTTGCAACCCCTTTGGGCGTCAAATGGTCTTTTCACAGAGCATGCCTATGGAGATTT
ATATTAAGCTTTATAACAGTGGCAAAATTACAGTTATGAAGCAGCAATGGAATAATTTTATGGTTGGAGGTCACCACAGCATGAGG
AACTGTTAAAGTGTTGAAGCATGAGGAAGGCTGAGAGCCACTGCCCTGGATGATAGTAGATATGAGAACACACAGGAGAGCCTTTG
GGCTCCCTCAGGAGAACTGCTACCTTAAATTACTGCTGGACAGATCCAGTGTAATTGTGTAGCTAAACACTAGCCCACAGGAGAGG
CACTGCCTAGAGAAGGTTGGACAAATATGGACTTAGGTTCAAGGTGTGCCGTACCAAGTTGGTGTATGCACTGTGAGACGAGAGCT
GCTTTTGAACACTGAAATCAAGGTCTGAAACAACAAAGGCCAAAGAGGGGCCACTGTGAGCCCCAACCCAGTGTTAGACACATCAA
AATTTGAGCAACAGCTTCCAAATGATGATGAAGATGAAACATCTGACCTACTTCAGCATGTTTATTGATGGCTACAGGTTTGAGTC

AGCCTGGGTCACTTCATAGAAAACTGATGCCAGCCTCCATCCCATAGAGTGGGATTTCACAGAGCAGTGAAAAGCCAGGCCCTCCA
GCCTCAGCATTCCTCTTGAGTTAATGGAAAGGTTTGTTTGAAAAGTCCTGATTCCGTAGGGTTGGATGGAGCCCCAGATTTTTAGT
AAATTCTAAAACAACACTGATGTTGCTAGTTAAAGACTCCCATGTTGTGAATATGAGAGGAAGAAGCAAGGTTGTGGTTTCAGCCC
TTTTATTAGTGGTTGTAGCCTTTCTGTTTTTAAAATGTCATTGCTGCAACACTCCTCCTCTCCAGTGTTCATTTCTGTGAATTTTG
ATAATCATGTGGCTACTCCTGATCTGAGGCTGATGAGTTCTCTTTGGTTTTATGTCTGAAAGTCGTCCCTTCATCTTCTCTGTTCA
AAGGTGTTTTTTGAAGAGCAGAGGATTCTAAGTTGACAATATGTACTTTCATGTGCTGGTTTTATGTTGATTCACCGTCTCAGGTC
TTGACCTGTTTCCAGCAAGAGCTCTCTGTAGGTCTGCCTCGGCTCTACTGTTCCCAGAGTGTGATTGTTTCTCTAGCAGCGTCTTG
ACTTCTCTTTAGCACTCGTAAGCAGTGTGAGTTTTGATGGGTTTGAGGGTAGCTTTCTTTTTAAGCTTGAGCTTCTTAGTCGTCAT
AGATCCTTGGATTTACATTTCTTATTAAATCTTAAAGCTTTTCAACTGTTAAGTTTTCTGTCACTGTCACTGCCCCTGCTCCCCAT
TCCTGTTCTCTGTCACTTTCTCTCTTCCAGACTAGGGTTGAGTTGGCACAGTGACTAAGAGAATACCTTCTGAATAGTCAATCTGA
TGCCTCATGTATTCCGAGGCTTCCTGTTTATGAGAATAGTAGCTCTTTCCCTTAGCCTAACAGGGCTTTCTCACAAATGTTTCCTT
TGGATTTTTGAAAATCTGTGATGCTCTGGATATAAATCCTAATCTTCTTAACCTCAAGTCTTTGCAACTGCAGAGCCCTAGCTGGC
TGCCTGGGTTCTCCTTCCCATCCTGCAACCTAAGCATTAAGCCAGGTAATTGTAAGAGTACCTTTCTATTTTTTTTTCTCGGATGA
CTGTTGTGTGTCACCTATCTCCAGAGCATGACAATCGTTTTCTCGTGTGTTTTGGCTATTTAATTCTAGAGGAACCGTCTAGGTCT
ATATTACTCCACCATGGCCTGTGGTGGAACACTGGTGTGTTGTTTACTAGGGTAAACCCTTCCTTCATGATTCATATAATAAATAA
GCATGATGCCTTTCTCCAGATGTGAAGCTGGGTTACGTGTAAGAAGTGCAGCCGAGGTGATGGTCACTGATGTTCCCAGGAGATGA
GCCTGGGCTCCTAGCGGGGAGCAGTCTGCACAAGAGTGAGACCCAAAAGGGCAGACACAAGATCTGTTAGACACAAGGACCTCCA
AGCACATTAGCAGGTTGGGTGCATAATGTTTGTGATGCTCCCTCCGTTCAAGCTTTCCTGTGGTCAGCAGGCACCTGCTGAGGACC
CACATGTCAGCCTGCATTCTAAGACCTGGGGATCTCTGTCCTGAAGGAGCTGTGGAAACACAGCATGTTGACTTCTCTGTGTCCCT
GTAACTCACTCCACATCTGGCCTTCTGTGTGTGTCTGTTTCCTTCTGCCTTCACCTCTTCTCCTCCCTTTCTCCAGTTTATATGAC
CTTTGCACAGTAGCTTGTTCTCATGTGTGCAGGCAAGTTGTCAGATCTCTTTTATTGTCATTTTATATTACATTTACCGTGTGTGT
GTGTGTGTGTGTGTGTGTGTGTGTGTATGTGTATTTCTGCTTGTCGGGGGAGGCTATCTGTTCTCTCTCTCTCTCTCTCTCTCT
CTCTCTCTCTCTCTCTCTCTGTGTGTGTGTGTGTGTGTGTGTGTGTGTGTGTGTGTGTGTGTCAGTGTCAAAGCCCAGAA
CAACTTGTGGAAGCCGCTTCTCTTCCCCTCCTATGTGGGTTCTAGGGATTGAACTCATGTCACCAAGCTTGGCAGCTAGTGTTCTT
ACTGACTTAGCCATCTCACTGACCTGTATCATTTTCTTCCTTCTACTAATATTTATTTTACTTTTAGAGTAAAAGTCCTTACAAAC
ATCTATATGGCTCTGAGAGACCCAGGCCCTGCACTTTTCTCTTTGAGTTCATCTGTCCCCTCTGACTCTGCTCCTTACCCTCAGCC
CATGTGCCACCTTTCTGCCACTGTCAAAAACAGCAGAGTGGCCCTGCCATGCGGTCTTCACATTCACCAGAGTGGTCCTGCCATGA
GGTCCTCACACTCACCTGTCTGCAGCTCTGATACTTAGAATCCACTTCTTCTGACCTCCCTCAGGACTGGCTCAGCTGTGATCTCA
GGGTCCTGTCTGGGTTCTCCCTGTCTGGCTCTCCTACCCTCTCACCCCTCCCAGGCCTGTCTTCTTTTTCTTGTTTACATGTATTT
CTCTTTTAAGTTACACTGTGAAATCTATCATCGTGTACGCTAGCATGTGCATTGTGTGAGAGCACAGTGAAGCCACACAAGCCTTC
CCTAGTAAAGCATACGTACAAGGGCACCCGTACTCTGTGACTCCTGAGTTGACATCTAATATGTTCTGAGACGTAAGGTGATTCAA
GTTTTCCTCCAACTCTTTTAGTAACCCAGTTTAACCTGGTCTGACAAATCCACTTCTGTGATGAGTCTGGTTAGAGGCCTCTCTGC
AGAAATGTCAATGAGCTGAGTCCATGGTGCACCCCAGCCCTGCCAACAGTGTCTAGTGACGTACAGCATATGCCATATATCACCCT
CATAAAGTCCCAAATTCTGATGTTCATCTGACTTTGGTCTGTGTAAGATGCTGAGTGCTTGTGTACTTTTTTCGGTGTGCTGTCTG
CTGACCTGAAGCCCGCCCCCCGGCCATGACCTGCCTGACTTCTGCTCTCACTGTTTTTCTCCAGCTTTGCAAACCTGGGAATACTT
CATGTGACTAAGAAAAAGGTATTTGAAACACTGGAAGCACGGATGACAGAGGCGTGTATTAGGGGCTATAATCCTGGACTTCTGGT
GCATTCTGACCTTGCCTATCTACAAGCAGAAGGCGGAGGAGACCGGCAACTCACAGGTGAGCCAGCTGACCCAGCCTTAATTAAGG
CTTCTATGTTCCAGGATGACTGCAGCCATCAGCGTGCCAGGCTATCAGAGGGAAGGGGATCAGTGCAGCCCGGTCTTTCCTTTCTG
TGCTGGGCACCGTGGAGAAGCGGAGCCCACACACAAAGACTCTGCATGCTTGTTCTCACCTATGTCTGCGCAGCCGGCGGTGCAGA
GACGTGCATTTCCCTGTACTCCTCGCATTCTATGTGTGCCTTAACTCTTAGCAAGCGTAGGAGAGTAGATGGGTGAGCATGGGAGA
AGACAATATTTACAATACTCCAGAGAAAACGCAGCTTACCTTTGATGGGTTTTTAGGCAATTTAATAAGACCATTGTGTAAAATTT
TACTAACAGTTTAAAGAATTTCAGGCCAAAGTAACCAAAAGAAAAAAGAAGACTCAGTTATTAAAATTAAAGGTGAAAATCAGGGA
AACATTACCAGAGGTAATGGTCAAATCCAAAGAATCATTAGGGCTTTGAAAATATGTATTACAGCAAATTAGAATATCTCACAGAA
ATGGGTAAGTTCACAGGTACATCTGACCTGCCAGTGTTAAACTAAGAAGATAGAAACAACTTACACAGACCTAAGACAAGCAATAA
AATATCTCCCAACTGAAAAATACCCGGTCCTAAATGGAATCATGGCTGAATTCTGTCAGATATTTGAAGAGATAACGTAAATCCTC
CTTAAACTGTTCCATAAAGTAGAAAGGGGAAGAATATCAACAAACTTATTTTCCAAAGCCAGCATTACTTTTATACCCAAACCAGG
TAAAAGAAGAAAAGAAAAGAAAAGAAAAGAAAAGAAAAGAAAAGAAAAGAAAAGAAAAGAGAAAAGGAAGGAAAGGATAAGCCGAT
TCATACATTCCCAATAAAATAATAGAATGAAATGAAAAATATACAAACGGGAAAGGAAGAGATGAAAGAGCCCTATCCACAGACAA
TATTGTCCCACACACAAGAGCCCAGGGCTGCACTGGAGAGGCTTAGCTCTGATTGAGGATCAGCCCTGCGTCATCACCACACAGAC
CCCGTGGTTTTCCTATATCGATAGTGAGTGTGTCGAGAAAGAAGTCAGCAAACGCGACGGCCGAGCACAGTCACAGGCATCCCCGA
TAGCTTCAACATGAGCAAATAAAACTCCTGGCAATAAACTAACCATGTGAAAAGTCCTTTACAGTGAACATGTTAAGACACGGAAG
AAGAAAGAGATGTCAATCAATGATGGAAAGGCCTCCCATGTTCCTGGGAGTTAACAGAGTTACTACCACAAAAATATGGCCAAGTG
TGGTCTGTAGATTGAATGCAATCCACAATGCCAATACCGTTCCTCCTAGAAAATAAAGTTCACTTGGGACGACAAAAGGTCTCAGG
TAGCCAAAGCTATAGGATAGTAAGAATATTTCAGAGGTATCACAGCTGCTGATATCAGAGGTATCATAGCTGCTGATTCCAGGTCA
CACTACAGAGCCATGGTAACAAAAAAAGCATGGAAGTGGGGTCAAAACAGACGTGTAAATCAGCAGAAGAGGAGAGTCAGAAAGGA
ACACAATGTAGCCACCTGATACCCGAGAAAGATGCCAAAATGTGCATTAGAGAAAAGACGGGCTGGAGAAGAAGTGATGCCGGTGG
GGAACCGGCTAGCACATATAAGGAAGGGAACCAGATCCCAGGCTCCTACCCTACCAACCAATTCAACACGGAGCAGAGATCTTAAT
GGAAAAGGTAATGGTCTGAAACAGCTAAAGGAAAACATGCAGAAAACGCTTCGTGATGTAGGCAAGCCCTTTCCGAAAAGGGCTCC
AGCAACTCAGGAAATAAAATGGAGAACTAGCAACTGAGATTCAAAGGCGTCACAGCAGAGGAAACAGTTTCCTGAGTTGCAGAGAC
GACAGCCTTCAGTGGGGGAAAGAATTGTTGCTAGCTATGTAGCTGACAGATTAGTATCTAGGTTCTATAAAAACTCAAAAAAAATT
AAATTCTGGAAACAAACAACCCAGTAAATAAATGCGCTCAGAAGAAAGAGAGGGTTCTCCAACAATGAGTGCAAACAGCCAACAAA
TGTGAAAGCATGCTTCACACCCTTAGTCTTTAAAGTTCCTCTGAGGTCCCACCTCACCCTGGTCAGAAGTCATTAAGAAAACAAGT
CCAGGAGCCTGCCAAGTAGCTCAGGTGGTACAGTACTTACCTCTTGTGTAAGAAGTCCTGGGTTTGATTCCCAGCACTCCCTACAA
ACGGCTGCACTCCCAGATGAGTTCAAGGTCGTCCCCAGATGTGTGTAGCAGATGTCTTAGCAGGTAACCCATACAAAGCCTGCTGC
CCTGAATTCAGTCTCCAGGACCCACATGGTAGAAGGAGAGAACCCCTCCCCTCAGGTGGTCCTCTACCCTAAACACACGTGTGTTA
TGACCAACAAATGCAAGAATGTAAATAAGAAAATCAAAATGGAGATTTAGAATAACATGAAGTAAAACTGCATAGACACAGCTATC
CACTCCTGCGGATATTTACAAAACTCTAACTCCACGTACTATAACATACATATTTCCCTGCACATATGTGTTTATTTCAGCCATAT
TCAAGAGCTACATCAAACAACCACCGTAGCTCTCCATTAGCAGATGATGGATTAAGAAAGCATGATATACATGAGTAATGGAGTTT
TTTTTTCAGCCATTAAAAAAAAAAAAAAGAAAAAAGAAAGAAGGGCTGGAGAGATGGCTCAGCAGTTAAGAGCACTGACTGCTCTTC
CAGAGGTCCTGAGTTCAAATCCCAGCAACCACATGGTGGCTCACAACCATCTGTCATGGAACTCGATACCCTCTTCTCTTGTGTCT
GAAGACAGCTATAGTCTACTCACATACATAAAATAAAAATAAATCTTTATTTTTAAAAAAGAAGGGAAGAAAAAGAAAAGAAACAT
TTGCAGGGCAGTGTATGCAACTGGAGATCTCTGTATCAAGAGAAATAAGCCAGAACAAAGAAGATGAACATCACCTACCGTCTCTC
TCGGTTCTAGATCCTAGGTTTTATATAGATGTACATAAGATATTTTGCATATGCGTGCATAGGATATGAAAGTAAGAGGAAGGTAC
TCCGTTGGTAGGGAGGGGACGAGTGGATATGAGGAGGGGAGAAGGGGCCTTAGGTACGGTCACAGTGCTTGTTCTAGGATGAAAAT
GTTTTCAGGGAGTTATCCCGATGAGCACTGAGTATATGCCAAGAAAGACTTTAAGGAAGTGTTTCTAGAGGAAAAGAATTGGTCTT
TGAGGCCATTTGCTGTCAAATGTTCACACTCTGACCTGCCTGGGCATGAGCAGACTGTTCTCTGTGACCCTAGACAGAGAGAAGGA

GATCATCCGCCAGGCAGCCGTGCAGCAGACCAAGGAGATGGACCTGAGCGTGGTGCGCCTCATGTTCACAGCCTTCCTCCCTGACA
GCACTGGCAGCTTCACTCGGAGACTGGAGCCTGTGGTGTCAGACGCCATCTATGATAGCAGTGAGTACTCGTTCCCAAGATGGGAC
AGTTTCATCTAGCTGCATGGAGGGCAGGGCCACCATGTCACTTACTCCCCAGAGCTGAGGCCTGCAGACTGGGACCTAGTCACATA
GAGCCTCTAGTTTATTGTCTACAAAGTTAATACTGCAGTCTAGGGTAAAGGGTCAATATCCAGTTAAGCGTCTTAAGAACATGTCT
GCCAAGGTAACACGTCTTCCTCAGAGAGTGCATTATATTCCTTACATGTAACACCAGTTTCTTACTATGAACACATGACTGTAAGG
ATAGATTGCTGGTGCTCTAAAAAAAATTACCTTAATTTTTGCACCTGGCAGCACGGAAGAAAAATTGATAGAAGACAAGTATCCCT
CTTGTCTCTCTTTATCCCCAGTTTGAAATACATTTATTAAGACAGGAACGTCAAGGTAACTAAATAAATGAATAGTATGTCAAAAT
CATCTTTCAGGCTGCAGATAATAGAATGTGCAGGGTTTCCAGTAGCTTGTAGAATTTACTGTGTGGCTCTATTGTTCGATATGCAT
TGGGCTTAACAAACAGTTGTTTCAGGAACCCTGCAAGCCTCACTGAATGGTCATAGAATACGATGCACAAGGAAATCCCATGTTCT
GCATCCAGCCACGCATGGGTCTGTCTGATTTAAACAAGTGTGAAGGGAAGTGAAAAGTTTATTGAATGGAAAAGAGTCTTACTGAA
CAGAGATGAGCTACAGCTGTTGGAGTTTTTAAAGGGGGAAGCTTTGCCCCACTCACCACTATGGGCAGCTTTGCCCCAAACCACAC
AGAATGCTAATTTAGGTTGGGGGAAAAAAAACCAGAACATCTTCACTTTTCTTTGAAGAATATGTGTGTGTGTGTGTGTGTGTGTA
AATGTGTGTGTATATATATATATATATATATATATATATATATATATATTAGCTTAAAATGTCATTTTCTTAAAATTTTTAAGAAT
TTTACAAATGACATAAAGTTAGCATGAAACAAGCCATTGGTCCAGAAACATCAGACAGCTCTCTCCTTCCTCATCAGCCTGTGTGT
TAGAGAAGCCATATGCCACCATCCCTCCCTGACCTGCTCCTTTCTGGGGAACCATGAGTGCCCCAGACCCAAGAACTGCTCCTAGT
ACCTTGGAAGTCTTTCTTGCTCTATTTGTGTCTTGTTAAAGGCAGTAAGAGTGAGCACCATCATGGAATCTTTGTGAGGCTAATGC
TAAGCCAGGGTATGCATTCTCTTCTCCCTTTGAACAGAAGCCCCGAATGCATCCAACCTGAAAATCGTGAGAATGGACAGAACAGC
AGGATGTGTGACGGGAGGGGAGGAGATTTACCTTCTCTGTGACAAGGTTCAGAAAGGTACGTACACACCTGGTCTCTAGATGTAGG
GTGTGGCTGTCAGAGTTCTCTGGAAACAGCGGACCAGGGGTGTTCTCCTTTTGCTGTGGGAGTCTCAAAAAGACAGCGTTCTTCAA
CACCATTACACGCATTGCAATCACATGCTTGAATACGCCCGTTTGCAGATGACATCCAGATTCGGTTTTATGAAGAGGAAGAAAAT
GGCGGAGTTTGGGAAGGATTTGGGGACTTTTCCCCCACGGATGTTCATAGACAGGTAGGTGGGTTATTATTGCGGGTTTTATTTTA
ATGCACTTCTGCCTTGGTGAAATTCAGTGCTCAGTTTCCACATATTAACAAAGAAACCGTCATAAGTCTCTTTGACATCCATACAG
CAATGCTATGTTTATGGCTCTTCCAGTACTCTGACATAGAGGCTGAGAAGTCTAAAGGGCTGAAGCTGATGCTGTCCTGATGAGCT
GGTGTCTCTGGCTGATTGGTTACATGACCAGGGTCTCCCAGCTGATGACATGAGCCACCACAGTAATAGTCACTCTTTGACAGTGA
CTGAGAAATGGTGTTTCAAATAATTGGCAACAACCCAAATGTTAGCCAGTAAATGACTAATTAGATAGTGGTGTATCTATGTAATG
GAACATCATACAGTTACTAGAAAAGAAGGCTCTGTGAATGAACAAAGACCTCAAAATAGAATGCAAAGATTTGTAACAATCGATAT
GGAACACACTGTATGTAGAAACAATTCTATGTAGAAACAATTCTGTGTTCACGTGCACAGGAAGCCTGAGACAATAACAACATCAG
CTCCCTCTAGGATCTCAAGTTAGTGCATGAGTGGGAAGCGGGGAGGCAGGGAGAACTGTCAGCATATGCTACCTATTGCTCATCCT
ATATTTGATGACATCTTGATCCTTATAAACATAGAAAGACCGAAACTAAGTCTGTCCAGTTCCTTATTATCCCAGCACTGCCCAGG
ACTAGCATGTGACACCTCCTGCGCAGCCTCACTTAGCATGACTGTCTGCGGGCTGTCAGGGGCAGCCCTGGTTCTGTCTGCTTCCA
CATCCACTCCTTCTCTTGCTGCTGGCAGTGATCCTGGATGTTTTCAAGGCTAATCTGAAAGAGAAATGGCTGAGTCGCGTGATCCG
CCATCATGCTGTTGTGTTTTACTTTCACGGGGGCAGTATAAGTGTGGGTATAAGGTACTAACTGGGAACTTTCTCCAGGTGCTGGG
TTTGCACACACTTTATTTAAGTTCAGAGTATACATAGACGTGTATAGAATGTGCCTGATGCTCACCGACACTAGAAAACAAATGTC
CTACACTAATGTTTGGGTCTTTTGCACCCTGCAGTTTGCCATTGTCTTCAAAACGCCAAAGTATAAGGATGTCAACATTACAAAGC
CAGCTTCCGTGTTTGTTCAGCTTCGGAGGAAATCAGACCTGGAAACTAGTGAACCGAAACCCTTTCTCTACTACCCTGAAATCAAA
GGTACCTGGGTGGTTTAGACTCTTGTGCTTGCTCTGAAAGACCAGTGGGCCCCTCCTCTACTCAGCCTCCCAGAAGCTGCTGCCTG
GATGCCTGGATGCACGCTGCATAGTGTTAGGGGCAAGGAAGGTCATATGCCAAGGTCATGGAATTAGCCCCAGGAATTTATGTTAC
TATATTGCTGCCAAACTTATATGAGACTTAGACATTTGGCTATTGTTGGGTATTAGAATCTAGTATGGAGCCAGCAAGATGGGTAA
AAGCACTTGCTGTCAGGGCTGACCTGAGCCTGAGTCCCAAAGCTGTGTGCTGAAAGGAGAGACGTACCTGCCAGAAGATGTGGGTG
GTCCTCCACCAGGAGGGGGCTGGGCGGGGAAGAAAGGGAAAGTAAAAAAAAAAAAAAAAAAAGTAAGGATTTTTTCAGCTACTCAG
AAGCCCTTAAGAATTAACCAGAAGGCTTAGCCACTGTCCAGCCCAGAACTATAAAATGAGATGGTCACTATACTTTCCTCACAAAA
GACACCTCAGGAAAGGTGCACTATAGTTAAAATGCAGATTACGATTCTTTTTGACGTGCTAGACATCATATTCTCTGGGCCTTGTA
GACACTAGGCAGCTGCTCTACCATTGAGCTGTAACCCTGGTTCTCAAATGATAAGTACCCAAAGGGAAGCATCCACAAACTGTATG
AAATAGTTGAGGAGACAGTCCTGGATTCCTTGGTCTAAAGAATCAGCCAAAGAATTTCTCTTCAGTGGGAAGCCACTTACCACATT
ATGAAGGTGAATGGAAGTCAAGGCTCTGTTTATGAAAGAGAGATGTTCAGAGGCAGAGGTGTGGCAGCTGGTGTTTGTCCTGGGAG
AAATGATTACTGTGAATCTGTGTGGACTGAGGAAGGTTGATTTAGGCGAGGGAGGCCAGGTCCACGCAGTGAAGCGTTTCTGTGGT
AGCTGGAGGTTTTTGCTTTGACTCTGATGGCTAGGCCTGCCTGCTCACTGTCTGGAATGATGTGGTCAGAATGTTGCTAGAATTCA
GTGGTGCCTCAGCAGAGTTAGAAAGGGTTCTAGCAGCCAGAAGTTAGCATCCAGCAGATACTTCTATGTTGGTGCTGTGGAGACCT
GGACCTTGGGTGAAGAGAAAGGAAGAGAGACTCGAGAGTCTCAGATTGTTCTAGGGTTTCCAGACTAGAAGTGTATGTATGGAGTG
GTTAAGTGGATAAGAGGAACAAGAGAGAAGAGTCTGAGAATGAAAGAGAAGCTTAGTGTAAACATATTTCAGTTGGGGTCAAGTGC
AGGAAGGGGGCCAGTCAAAGGCATGACTCAAGTGGGTGGGGGCTCAGCACTGAAGAGAATCCCGGCCCCACAGAGTTTAAGTGTAG
GGACGCACAGTGCTGCCGTCAACCTCTCTGTACCTCTGTTTCGTAAGTTTTGTCTTGACGGCCATGGTTCTGAGAAGAGATCCATG
AACTTGGAGGCTACACAAGGCCTGGGATCTGATAAAATCAGAAATGTCTGGCTCAGAGAGAATGGGGCTGGAGAGGATGTCACCAG
GTACAGGGTCCTAGGTCCAGGGCCACCAAGGCCCCCAGGTATCGGTCCCTTGAAGGCAAAGCGTGATGGTGGCCAGGAGTTTCTGG
GAGGCCATGTCTCTTTTTGAGGTATGGCCTCCCCATTCCTGGGTCAGGCTTATGGTGAGCTCTGTGGCTGGCCTACTTCCTTCCTC
ACCAGCTTGAGGTCTTCTAGATCCTTCTGTCCAGACAAGGCAACATGAAATTACTGCTTTACCTGAAGCCTCTGCCCTCTGAAAAC
AGGAAAAAAAGATTATTTTTCTAAGAAAAAAAAAAAAAAAAAAAACAGTGTATCTCCTGAAATGTGCCTTTAAAAGAGCGTTGTCAT
TGCTGATGCAGAATCATGAGAAAAAGAAAAGAACTGCAGGTTCCCTGTGCAGGATGTGAGCCTTGAAACGCTGAGAGTTTGGTTGC
CAGGCAACCTCTCCTGCTAGTGTCTGCATCTGAAGAGTACAGTAGACAAGGCCCAGCTTCCTGGGCACTCCACGCCAGCAGAGTGG
GAGTTTTCTAATGCCTGGATTAGAGGATTGGGGTTCCCTGAAGTCCTGGTTACCTCACATCTTCTAGGTCATCATGAATACAAATG
GTATACTTGAAATACAAATGAGGCTCCACCTACACACAGGATGGCTGAGGTACCTGTTGTGTTCCGTGTTTCTACACTGGAAACAT
TTTCTCTGTGAGATCCACAGTTCATCCACTGACTAAATGAATATAGCTATTGGTTTTGGTATAACTGGTGGAAAACTTTATAAAT
ATTTCTCCGAGCCATCAGTTAGAAAGTAGACAGTCTCTTCACCTCAGCCTCACTCCTTTCTGGCCGAAAGCCGTTTTCTTCCTAAT
CATCCTGGTGTGCCCAGGGTTGGTTTCCCAGGGACTGGCAAAGTCCACCATGCTCTCCTGCTTTCCCCATCAGCTGCAGTAGACAG
TCCAACATAGTCCAAAAGTCCTTGTGCCACACCGTGGGGTGATGGAAACACCCACAGAACTAGAGAAGTAGGGATCTTTCTGAACA
TAGACCATGATGAAAGCCAGCATTAATCAGTCAGACACAGGACGATTGTTCAGTATTGGCCTATTTCCTGGATAGAAAGCACCATG
TTGACTATTTAATGTAAACACAAAACCACCTTCACTTCTCAGCCCCAAAGACAAAGAAAACAAACAAACAAATATCTGCTAGGATA
CTCCCAGACCACAAAGGAACAGGAGCACCATGTCAGATCTCCCACACAGTAACAGGACTGAGAACATGGTGGCTGAAGATCACACG
GGCCATGACAGAACTGAGGGCACATGGCTGTCGATCACATTGCAGTAACAGAATTAGGGGGGCTGTCTGGGTTAGTAAGGAACACA
GACAGTGAAAGACAAGAGGAACACAGAACTCTGTGAAGAGCACCAAGATAAGTCTCGGAGCACCTGGACCTGGGCAGGCCACTGTC
CCAGAGGTCACTCACTGTCCCAGAGGCCATCTTTATTTTGGAGTCCGAGATCTCAGGAGCCAAAACATCTCTGTACACCCTCTCTG
TGTTTCTAGGATCGACATTATATCTCTTACCACAATAACCAGTAAGGACTCGGTCTCCATTGCCTCATGCCTGCACTGTCTTCCTT
AGAGTTTGACTCTAGATGTCCTGTATACAGTGATAGATGTAGCTCCTAAAATGCTGTCCTCTATGTCATTCTCTGAGACATTCAGC
AAGTCCCTCACATTCCTATCCCTGAGGCTCCTCAGCATGGGTGACCCCAGTCCTCCTTTAACCTTTATTATCAAAAGCACCCACCC
ATGTTATCCCTCTGAGAAGCCCTGTGTCTTCCCAGAATATCATGTTCTTCGGCCATAGTTGTTAGCCTTTATAGAGGCTTGTCATG
CTGTAAATCTGTTTTCACGTGATTTCACATGATGGTATGATGCATACCACCATAATCCATCCCACTAAATCCCTCTTTGCCTTGAA

ATTGACGGTCCTTGACAGCAGAGATGTGTGTTATGTTTCTGTCCATCCCAGCACCTTGCTCAGTGTATGTACGGAATAAATGTAAG
CTGGTTATTATAGTTGTCTTAGGTGAACAAGGACAACTGTTCTGAACGTTAGCTGGGTGTGGTGGTGCACACCTCTAATCCTAGTG
TTCACGAGGCAAAGGCAGTTTTGAGTCTTGTTCAAAACCAAGCTAGTCTACATAGTGAGTTCCAGGCAGCTAGAGTTACATGGTGA
GACCCTATCTCAAAATAAAGAAGTAAAAGTGCTTTAAAAAGAAAAAAATATGTGTATCTTTGGGTTATCTGAATCTTGAGAAGCAA
TGAGATCTCTCACCATCTTGTTAGCACTGGGAACTTAGGAGGGGGCGCTCAGGAGAGTTGGGTGTGTGCCGCACTGTGGTCTCTGA
GGCGTGCTCTTGGTCTTCTCGTCCATTGGAATAAAAACCATGCTTCCTCCTCGGGTGCCTGTTATTTCCCTTTAATGCATTTAAAA
ATATCATGAGAATCAATCGTCTCTCTGCGGCTGCTGGTGGAACCCGATCAGTCTTTCCCCCCTGGTTTCCCTTCCTGTAAATACAG
ACAAAGAGGAAGTGCAAAGGAAACGCCAGAAGCTTATGCCGAACTTCTCGGACAGCTTCGGCGGCGGCAGTGGAGCGGGAGCCGGT
GGTGGAGGCATGTTCGGTAGTGGCGGTGGCGGAGGGAGTACCGGAAGCCCTGGCCCAGGTACGAGAAACATCTCTTCTATCTAAAG
CCTTTCATGAAGAGGAGGGGGTAGTTTTCTTTCCCAGGTTGCCAAACATGTCTGCCCATGGAGTGATGGTGTCATTACTAATTATT
AGATTTCAATTAATGGAAAATTTCCTACAGATGTAAAAGAATTTTTGTTCTCAAAGAAATGTATGTCCAACTCAAGGCACAGCCAC
TCCCCACCCACCCACCCATCAAGTTTCACTTTCTGTGGTTTCAGTTACACGCGGTCAGCTTGAGTCTAAAAATATTACCATTGTGT
TGACAGAGAGAGACCACACACACAAAACTGTCTTCACAGTCTGCTGTTGTGACTGCCCTATTACTCTGTGTTCTTGGTCTCCTTCT
GTCTTTTTACGTTGTGAACTTTACCCAGGTCTGTATGCATCTGCAGTTTTAGGGATACACTGGGGGTCTCGGACGTTCTCTTTCAG
GATAATATGTGTCTAGTCCCGAAAAGTGTAAGAAAAAAGTCAACTGAAGGGTTTCTGGAAGAAAACCTTTCTCAAATCATTTTTGA
GTGTTTCCCTCCTCCCCCTCCCCTTTGCTTTTGGAGAAGCAGTTAGTCTATTATTCTGAGAAAATATTAAAACAACTATCATACCC
ACCGCATGGTAGATGGGTATAATATGCCTACACAGCATACTGTGGGCTGGAGACACTGTACTGTAAACAAACTGAGTCACAGAAAA
TCATGCCAGGGAAGCACTTCAATGGTGTGTAAACTACTGCTCCTAACTGCCACGCATACGGCACAGTCAGTATTTATGTGTTCCTC
TCGGGTCCGGCTATGATCCCCGTGCGTGGATTTCCAGCCATCTAGTTACACTCAGTTTTTAAGCATCGTTGCATCATGCTTGTGGA
TGCTGAGTAACGCTGCTTTTGCCTCAGATCTAGCTAGCTTAAGCATTTCAATGGAAATACAGTTCTTATAGCAGCATAAGTGTGCT
GTTTAATGACTCACACCTGTAGCGTAAGTGACTTGAATGCTGTTTCCTGTCTCCTGTGTAAAATAACTGTGCTGAGCATGGCCTTT
CTTTATTCCAGATAACATGTAAATGCTTTAAATGTCAGAGAGTGTAGTGCTAAGTGCTGGCTGCCCCTCTAAATTTATCATTCATT
TCTCCACTAAAGTATCATTATATTACGTATTGGGTTCTTCTCACCAGGGGTCTTCATATTTAACTATTTTTTTCTGTAGGTCTAAG
TCTTGGTGTGAGTACTTACCGTTAGCTTTTCCCTTCACCTGGAAGAAAGGGACCTGCTCAGCCTTAAGTCCTCCAAGCTGCTAGTC
TGGGCACTGCCTGTTTGTTCAGCTGTCTGTTTCCCACAGTGATTCTGCTAACTCTCGGGCAGAGCCAGCCTGTGTAGGCTTGTGCC
TCCCCATGCCACAGCCTGCCTCCCTGAGTGGGTCCCCATACTGTTAACAATCTCCCTGATGTTCCCTTTGTCTCATTGCTGGTTCA
GGGTATGGCTACTCGAACTACGGATTTCCTCCCTACGGTGGGATTACATTCCATCCCGGAGTCACGAAATCCAACGCAGGGGTCAC
CCATGGTAAGTCGGATCGGAATACCCAGAGCACTGCTCGGGCAACAGGATCATCTCTAAAAGCTTTGGAAAATTACTTTCCAATAA
ATTAATATTCTCAGGTTATTCTAGGACAGCATAAGGGACACCCCTGACACGTATTTGCTCATCACATTGTGTGGAAGCAGGACAGA
GGCTCTAGACAAAAGATGAGACTTGACTACGCAAACACACAAATACCACCTTCACATCTGTCCCTTTACCCCAAGTCCTATCCTTC
TGAAATCAGTTTTCATTTTGTAGGGTTGGCCTTTTCCCACTCTTGGGCATCCTCTGAGTAGCTGTGGCTACAGGCTGCTCGTGGTT
TATATAAAGCTGCATTCTTCTGTACCATGGAAATTATTCTATAAAGTCTTTCCATATATAATGCCAATTCATGCCAGATGCGTACA
TCACTGTAATCTTTTTTAAAGATTTTTAACTGGCATTGTCGTTTATCCTATAATCGGGTAACCATTCATCCCATAAAATAGAACTA
TTCTTTCCATTACCTATAACCTCATGACCCCTGTGCAAGGCCAGTGCTAGGCTTGGCACCTTCTGGACAGAGGTGAGGTTCAGAAA
CTTGACTTTCAGAAGACACTGTAGCAGGATTGGGTTTTGCTCCAGGTTTGCCCAGGAGTCTTACTGTCACTCTGTCACATAACGAG
GTTCCTACAGCAACCACAGTCAGTCTAGTGATGGTGCCCTAGAGCCCTCCTGGCCACACAGAATGGAAATGAGCTCTGCTCGGCTC
CCTTTCCCTTGCCACTCAGGATTAGGAAAGAAAATAACTGCTTTCGAGAACTTTCCAACTGGCTATCAGCGCACAGAACTTTATCT
CATTTTTCTTCTTCACAGATAAACAAAATGAGATCCATGCCTTGTTTCTGTTTGGTTTTGTTTGAGAGAGAGAGAGAGAGAGAGAA
TATCACTGTGTAACCTTGACCTTCTGGGACATTGTGGGTGGAGCTCACTACAGCTGGCTACAGAATGAGTTTTGGGAAGGCAGGCT
TTTAAATTGTTTCTGAATATTTAAAGTATTTCCGAAACATCCCAGGTTTTTTTGGTACACACCTTTAATCCCAGCACTTTTTGTGA
GTTTAAGGCCAACCAAAGCTACACAGCAAGACCCTGTCTCAAAAAGTAAATAGAGAAGGAAATATAAACTATGTCCAGAACATATT
ATTTGTGTTATAAACATTTATAGTTTTCCATTTCCTGCAGATAAGCCTTCTTCCTATTTTCCTTTTTTCTGCCCTATGTTGAGACA
GTGAGCTATAGCTTAATAGATTTTCTACGTTTTTTCCCTAGGCACCATAAACACCAAATTTAAAAATGGCCCTAAAGATTGTGCC
AAGAGTGATGACGAGGAGAGTCTGACTCTCCCTGAGAAGGAAACTGAAGGTGAAGGGCCCAGCCTGCCCATGGCCTGCACCAAGAC
GGAACCCATCGCCTTGGCATCCACCATGGAAGACAAGGAGCAGGACATGGGATTTCAGGGTAGGTGAGCTTGCCCACGAGGGCTTC
CAGGGACAGCTGGGGTGGGGTTTCAGGGTAGGTGAGCAAGCCCACAAGGGCTTCCAGGGACAGCTGGGTAGGGGCAATTCTAGGTA
CAGGAAGCTGTCTCCAGAAGACAGGTTTCCTTCCTGTTAATCTAAAGCTGATGAGTCAGCATCTCCTCTGAGTGCTCTGCACCGTG
CCCTGTGCCCTGCAAGAGAGGCGTCCTGTGGATCCAGTCATCTTTGCAGCCTCAGCTCTGATTATAAAGTGCAGAAACTTGCATGT
ACATTGCCTAGTCTGTGCAGTTCTGGGTATGAGAAGAGGCAACTCACTTTGTCCCTGGCCCAGGTAACAAGTCTCCATGTCCTTCG
CGCACCTCCCTCCCAGCAGCCGGCTCAGCTGCCACACTGAAGACGTTTGCCTGAAAATGGGAGTTTTCCTTTATTCTCTTGTCCTC
CTTGAAGAAGTAGAATTGAAAGGGAACGTTCCATCTGAGCAGGCCTCATTGGCATATGAGTCATGATTCTCGATCAGACGCCATCT
GTAAAGAGATAGTAACTATTTACACATAGTGGCCGTACACATGGTTACTATCACATATTTTTTTCCCTTTTTTCTGTTTTTAAAAT
ACTTCTTCTGAGATGCACAGTATATGTGTACAGAGCCATATGGAAACAGGGTAGATTTGCCCATTTGGGTTGAGGTTAGTCTGCTG
TCCCTTGACCTTAAGATAATAAAATTTCCAAAACCCCTGGGGTTTCTGAGACTGCCTGGCACCCAAGGGTGTGTTTCCTCACTTTG
ACTCAGACAGGATTCCAGAAGAGACTTTGTTTGTAAAGTGATTGGCTATTTAAAATTATGGCAAAAGCCAAGTACCCTGAGATTGG
TTGCTCCCCTTCAATCATAGACACTGATTGTTCTTCTCAAAATAGCAGATGTTTCTAGATTGTTCCTCACAGACACTTTAAAATCA
ACCTCTTAACTGTATTATCTGTCAGTGAGACAATGCCCGAAATGCCCGATTGTGCTTGTTCTAGTCAACACAATCCAGAGGCAGCA
AAAGGAGCATTTTCAGCTAAAATGGTGTGCCATGCCGCACACGCGGGGCGAGCAAATGACACTCAGCTGTGCTTGTGGGCATTACC
TTGCAGATAACCTCTTTCTCGAGAAGGCTCTGCAGCTCGCCAGGCGACACGCCAACGCCCTTTTCGACTACGCAGTGACGGGGGAT
GTGAAGATGTTGCTGGCCGTGCAACGCCATCTCACCGCCGTGCAGGATGAGAATGGGGACAGGTGAGTTGAGTGGGGACTGTTGCA
CAGCAAGCTCTGTTTCAGGACGGGGAAAGGCAAGGATACTGCCCCCACAAACTGCCTTTGAAAGGGAGCTGCCCTCATCTGACAAG
ACCTCGAGTATGCTGTCTCCCTAGGCAAGAAGGCTCAGATGTGTGTAATCATGCTAATTTTACACCCCTGCTTTGTGTACTAAAAT
AGTAATTGAGAAAATTATTGACAGCCCTGCAAAGTCCCTGAAGTGTTATTGTCATAGTGTACCCAAGAAAGCCAGGTAAGAACAAC
ATTAGTAGAGTAACTAAGAGGAAATGCCATTCTCTCCTAGCTACTAGCTGCTTCTCTGTGGCCTCTGTTAAGGGTCACGAGGATGT
GAGTGGGTCAGGCTCTGCCTCAGCTCCAGGCCAGGCTGTGTGGGAGTCTGACATTCCAGATGCCATGCCTGCTGTCTCTTCCTGAG
GTTCTGTGCACATAGCTACCTGGCTCCTTCCTAAATGGTTCTCTTAGTCCTTCCATTGAGACTTCTGAAATGATCTAAAAGCCTAT
AAATATAGCCAGAGACAGCAGTCTTGAGATTCAAGTGCTTTATCTAAGTGATCCTAAAATAGCGTTAACACTGAAAAGAAGCCCTT
TGCTGTGGAAGCATTCTGGAGTGCTGTGTGCTGAGTCTCAGTTTAAAAACAAAGCGCTTTAGGAGAATGCCAACCTCAGGGGTTTC
GTACAAGGACCTTTGTCCTCGTCACTTGCAGTGTAACTAAGGATGTGGTATAACCGCTGGTCTTATCCTACACAGTACAAGAGGAA
CCCAGACGACCTTCTGTGAAAAAAGCTCTGAATAACAGAAGGCGCCTTTAGAAGGAAATCATCCAGTTAGCATTCAATAGCAAATA
ACCACAAAACAGAGCAGATAACCAGAGCAGGACACCGGTGCTCCAAGGGTCATCTTTAAGGACTGGCGAAATTCATGAGTAGTGAG
GGCGAGTGGATTAAGCAATTAGGAAGTGAGATGCTGCTGTCTGAAGGAGAGGCTGTGGAAAGGGATGCCAGGCACATGCAGTCCAG
CAAGTGTGTGCAGAGGAATGCAGGAACATGTGTGTAAGGACCTGGGTGTTCATGGGGCAGCTGAGACACTGGCCTTCATTCCCAAG
TGGGTACAATACCCAGAGCCTCGTCAAGCCAAGACTTAAACAAGCAGGCTCCGTGTAAGGTTAGGGGTATGAGACCTGGGAGACAC
AGAGGACCCACTCAGGAGTATACATAAGGGACTATTCTGGTTTCTCTTCTTTATTCATCTTGTCATATTTGGCTTATAATCTGGAC
CCTGTCACCATTAGTCATCATAGAAATACAATAAAGAGAAACTATAGAACCCTCTGGTATCGGGGAACTTCCCCCAGGGGTATGAA

GATCTGAAGGGGAGGGGACGTGCTTCTGTGAAGTGCTGAGCTGCACATGGAAATGTTGAAGTAGCTTTTGAAGCCCTTTTACTCGA
CGTGTGTCTGTCCGCATCACCTCTCACAAGCCTCCTGTCAGGGTCCCATCAGGAGCACCACAGGAGCCTGAGGTGTGGCTGCGGTG
CTTCTCGTCAAAGCGTGCGTGCTTCACTTGCAGTTAACACAGGCCTGGGGTCATCTAAAGAGGCCACCATGATCTTCCCTGAGAAG
TATTGCTAAGTGAAATATCTCAGGGATAAATAACAAGACCTTGTTTAGAAATAGAAGTCACTTCTCATTGTTACGGAGGAAGAGAG
CTCGGCAGCTGAGTTTTCCCAAGCTGCTCCTAAGCCTAGGCATTACCTTTCTTCATGGTCTTGTATGTCAGGGAGAGACCAGGGAG
AGCTGCCAGGGACAGAGGCATGGAGACAGAGCTGGCTTGGAGCTTAATGCCATTTGGTTTCCGACCCGGCAGGTGCCTTTGCACTT
CATGTGTCTGGTCAGATGTTAACACTCCGGGGGCAGGTGGCACGATCAGTGTGTAGTAGCCTGCGATAGGCAGTGGAGAGGAAGT
TCTCCAGAGAAATAATGTCACCTCTGATCAGTACATTCAGTACATGGTAGGGCCAAGGAAAGCTACACATAGTAGCTAGTCTTCAG
CTCCAGTCATTTCTTGACATGTTTTTGTTTCTGTCGCTTGCTACATCCCCAGTTAATTATTGCCACTGTGTGTCATTCCAGTGTCT
TACACTTAGCCATCATCCACCTCCACGCTCAGCTTGTGAGGGATCTGCTGGAAGTCACATCTGGTTTGATCTCTGATGACATCATC
AACATGAGAAATGACCTGTATCAGGTAAGCAGCCACCACAAGGCCGTCGAGAAGAGAAAAGGTTGCCTTAGTAACACGTGCGTGAG
CTGTTCTCAGTGATGGGAAACAGGAAAACAGCATCTTCTCAGAAGTGGCATCTTAAAAGCATCCTTAGTTGGGAATGTCATTTTAC
AGAGAGATGGGTTGGGATCATGGGTTTGATGGCAATCACACTCCATGGAAATTCACCATCTTCTAGAAAAGGCAGACTTTAAGACC
TAGAAGTCAGAGTTGATAAAGTTTTGAGGGCAAACCTCAGAGGGGAAAAAGTCACAGCTGTCAGGAGAGACATGTTAAAAAGAAAT
CCAAGTTTCAAAAGCCAGAGTTACAGAGACAAAGTTTGGAGCTGAGATGAAAGGATGGACCATCCAGAGACTGTCCCAGCCGGGGA
TCCATCCCATAATCAGCCACAAAACGCAGACACTATTGCATATGCCTGCAAGATTTTGCGGAAAGGACCCTGATATAGCTGTTTCT
TGTGAGGCTATGCTGGGGCCTAGCAAACACACAAGTGGATGCTCACATTCAGCTATTGGATGGATCACAGGGCTCCAAATGGAGGA
GCTAGAGAAAACACCCAAGGAGCTGAAGGGGTCTGCAACCCTATAGGTGGAACAACAATATGAACTAACCAATACCCCCAGAGCTC
ATGTCTCTAGCTGCATATGTAGCAGAAAATGGCATAGTCGACCATCACTGGAAAGAGAGGCCCCTTGGTCTTACAAACTTTATATG
CCCCAGTACAGGGGAACGCCAGGGCCAAGAAGCAGGAGTGGGTAGGTAGGGGAACAGGGCGGGAGGAGGGTATAGGGGACTTTCGG
GATAGCATTTGAAATGCAAATGAAGAAAATACCTAATAAAAAATTGGAAAAAAAGAATATTTTCAAATTAAAAAAAAAAAAAGAAT
TTTGAGGTCTGCGGAAGGAAGGGTATTCGCACAGCAACTGGATCAAGTGTATGTCAAAGAAAGGCTCTGAGGTCAGCTCCTGGAGA
GGTGAAAGGCTGTAAGAACAGGAAGAGGCACCTCAGAGTCCCATCCAGCTCAGGTGTGAGAAGGTAAGGGAGCCCCGAAAACTCCA
GAGGAAACCACCTGGAGATGGCACAGTGAGCTGAGGACAGAGGCAGATGGTAGGGGAAAGCAAACCAGAAACTCCACTGGCAGACA
CAGTCTGGGAGCTGTGGCCTGGCCCAGGATGGTGGCCTGCTCTACGGCTCCTTCCTCTCTCACCTCTATTTCTCTCCAACCCTTTT
GTGCCCCTACCCCTCTCTCTGGGCCTGTTGGTCTTATCCTCTTGTTGGCTTCTAAGTTCCTCTGCACTCTCTTGCCCAGAGTGTCA
TTCTGATCCCCACAGCCAGGCCCATGCTGAGACCTCTGCTTCCAGGTCATATTGCTTTCATTGAAAAACTGTGGCCAGCAACCGTG
AATAATTTGTTATTTAGAAGAGATGAGATGAGAACTAGTGGAATTCTTACTTGTTTGTTTGTTTGTTTGTGATGTTTTTTGTTGTT
GTTGTTGGTTTTTTTGGTTTGGTTTGGTGCTTGGGACTGTACCCAAAGCTGAGCACAAACCCTTCCAGCAATCTACAACCATGTTA
CTCCATTTTTAAAACAAGTTATTCAAGTTGTTATCTCTTCTTATCCATGCTTCTCTCTGTCTCCCTCTGTGTCTCTCTCTGTCTCT
CTCTTGTCTGTCTGTCTGTCTCTGTGTGTGTGTGTATCCCTCTCTATCAATCTCTCTCTGTCTCTCTGTCTCTCTCTTTCTCTCCC
TGTGTCTGTGTATCTCTCTCTATCAATCTCTCTCTGTCTCTCTGTCTCTCTCTTTCTCTCCCTGTGTCTGTGTATCTCTCTCTATC
AATCTCTCTCTGTCATTCTGTCTCCGTCTCTCCCCCCCCCCCAATGTGTGTGTATCTCTCTCTGTTGATCTCTCTCTAGCGTACCC
AGGCTGGCACTGAGCCATCAGTGTCCTACCTCAGCCTCCCAAATCATAGGCTTGACAAGCATGTGCCTGGCATGTTATCTCATTTT
TAATTTAAAAAAAAATCATTATTTTAGAAGAAATTAAAAAGAAACAAAGAGATGACTCAGTGGACGAAGTATCTGCTGTACAAGTG
TGAAGACCATGTTCAGATCCCCAGACCATCGCCAGCCAGATTTAGTACCACACCCCCCCACACCCCGCCCCCGCCCCCCCCTCCCC
AGTCAGGGCAGGAGACAAGCATCTTGCATATGCGGGGGGGGGGGGGGGACAAAAGACCCTGCCTTATAACCTTGCACAAGGCAGGG
GGCAGTACACACGTTGCCCTCTGACCTCATGCACGTCCCTGCACCCACACGCTCAGAGAAGGAAAGAAGGGAAGGAAGAAAGGAGG
GAGGGAGGGAGGGAGGGAGGGAGGAAAGGAAGGAAACTAACTGCACATACACACTAAAACAAAAGAGCTAAAAAATTTTTATTTTA
AAGGAAATCATTAAATTCTTATTTTTTAATACAATATTTTAAAGTTACTTTGAAAATATGAATTGGAGGCTGGAAGTGGCTCACAT
GCTTAAAGCCTTGAGTTCGTTCGCTCCCCACACCAAAGAAAAGGCTGAAGCTGTTAGTTCGTTGGCCTGTAGCTCTCAGACTTTTC
CGTTTTCAGAGGAGCACAGCTGCTTTCTGCACAGCACTCTGCACTTCTCTGTCTGCAGTGATTCCTATCCAGGGGAGATGACTAGG
CTAAGGTGTTATTTTAGATGTGTTCTTCAAGATCTGTCTTCCTCGGGCTTCCATGCCATCTTGGGATACTATTGGTATTTGGAAAG
TCATTGGGCCCCGAAAGTTGAATTCTCCAAACTTTTAGGCAGATGGCACTTTTTAAAAACTTTGTATTGATTCTCTGTGAGTTTTG
CATCCTTTTTTTTTTTTTTTTTTTTTTTTTTTGGTTTTTCGAGACAGGGTTTCTCTGTGTAGCCCTGGCTGTCCTGGCACTCAC
TTTGTAGACTAGGCATCATGCACCCCAGTCCCACTCATCTCCCTGTCCCCTCATATCTGCCCTCTGCCCTTGCAGCCTCCCTCCCA
CAAAATAAACACTCAAGTAACAGGAAAGACAAAAACAAAAAAAGCATAGAGAACATCTCGATGTGGGAGCGATAGTGTGTCACCGG
GCATCCCACAGTGTATCCCTCTGTCCACACATCTTCACTTGCAATGAGTGTTTGGTTCTCTGTGACACGGTAATTCTGAGTTCCCC
AAAGTATAAGCTTTTATGTTATTTCAAGATTATGCTTATACTGAATTTACAATAATAACAAACATGTGGCAAAAGTGAGAGCCTGA
GAACTAACGACATAAAAGGCCGTGTCACATGCCGGAGAATGCAAGAAGAGAACGCAGTTTAAAGACTCACACTTGGGCTAGTCTAG
AGAGTGGGTGGGTTCCAACAGGGATCTGATAGTGACAGATGGTCACAGAAATGTACTATGGTTAGTGGAAGATGTTTGGGGAGAAT
GTTTGAGATAATTTACAAATAATCCAAATTACTTAGAGAACTGTATACTTTTCTGCCAAGAATAGTTATTATGTCATGCATGATGG
TGCATATCTATAATTCCAGCCCAGAGGAGTCTGAGGCAGGAGGATTGTTGTGAGTTTTGGGCCATTCTGGCCTATACAGTGGGTTC
CAATTTTGTCTGTATTATATTATAGGGTCTTGTTTAAACAACAAGAACAACAACAACACAAAACCAAACAAGCAAAGAAAAAAAAC
CCTCCAAATTAAAAAGAAACTAGTTCCCAAGCTGGGTGTGGGGCACACATTTGTAGTTCTAACACCCAAGAAGACACAGGAGGACT
GACAGCTTCATGTCAATCTGGGCTACACAGCAAGTTCTGGCCAGCCTCAAACACAGGAAGACCCTCTCTCAAACAAAACAAAGCA
AAACAACTCCACAAAGCCAAACAAAGAAACCCAAAAGTTATGAGAATCACAAACGCTGCCTGTCTATGTCCTTGAGGCTAAGAAGC
TGGTATGGCAGGGAATGTTTGTGCCCATTAACTGAGTGAAGCCAAGGTGGGAAAAACTGGGGAGAAAGGTCTGAGGGCTCCAAATC
AGAGCAGCTTTGCCTTGAGCTCAGGGGAGAGGTTCCATCCACAAAGAGCTGGGTCGCACACAATAGAGCCTGCTTCCGCAGATCCT
ATCACCAACAGCCAGCAGGGCTTCCTGAATCCAACCTCCAGCAGCCGAGGGAAGCAGGTTTTGCAAAGAGGATTGGGAAGTAGCAA
CTTCCTCTTCTTCAGTTTATTCTTATACTTGTGATGGTTAAAAATGCAATCTGCCACTTGTCTGCTATGAGGTAGCCTGGGTGCAG
GGGTGATGCCTTCCTCCACCTCGCCTCTCACTACCTGTACAGGCAGTCAGGAGAGCTGGCCTGGGGTCATGCAAGGGGGTGAGCTA
GCCCTGTCCTTCACAGGCTTTCGAGAGCAGGCCTTGCATCTAGCCTGTGCTGCAGAGTAGAGAGGACCCTCGTGGAGGGAGCACAG
TGAGCAGCCCTGAGGGTGTGAGAGCAGGAGAACTGGCCCTGCCCCTCACTCACAGGCTGTAGTGTATGGGAGAGTGGGTCCTGCCC
TTTGGGCAGCACAGTGGAGCCAGCTCTGGAGGCATAGGGAGATCCGACAGCTCCCTATTTCCAGATGTGACTGTTGCATGTATACA
GGAAGAGTGCTCTTCCAGTGTTGGCTACAGACAGCTGCTCTGGGAAGACATGTATTCTTTAGTGTGCTCACATGCACTCCACACGG
TTTTATACATACATTTCCTATCCCACATCAGCCTTTCCCTTTAAGTTTCAATTTAAAATACTTTCTCAATTCATAATTTTTGCAAA
ATTCAACTGAGAGGACCTGTATATTTCCTGAGATTGAACCAGACGCTTACCTCCTAAGTTCCCCCAGCAGTTACATCCTACTTAAC
ACAATAATACAATTAGGAATTGCAACAGATTCTAGTCAGATCTCAATATTTGTATGTACTCATATGCATGGATAGCTCTGTAAGAT
TTTTTTTTCCTAAGCTGAACATGGTGGTATTGTGTTTTAGTTCCTGTTATTCAGGTAGCTAAGGCAGGAAGAGTCCTTGAATATAG
ATGTTCCGATGTAGCCTGGGCAGAAAGCAAGACTGTTTCTAAGCAGAGTGAATGAATGAATGAATGAATGGTCAATCCATGTGCTT
GTGGATGTGTATGTAGCATTGTACTGTGTCCCTGTCTTGGGTCTTCACGCTGTTTATCCTTTAGTTTCATAGACAGCTGGTTTGTT
CTCATTGCTCCTGAAGAGAATGCTGTCATGTCCCCTAACTAGCTGCTCACTGTCCACAGGCTGTGCACTGCAGTGAGCACCTGCAC
TGTACCCATGGGTGCCACAAGTGGGTTTCAAGGGCAGTGTGAGCCCTGAGCTCCACAGATGGATTCTAGGGATTCCTTGAGGCACG
CATTTTATGCGTGTGTAAAATGTAACCCTTGGGTGTTGGTGGCCCACCAGCCACTGCTTCCCAGCAGGAAGAACTCCCTGAATGGG
AGGTCGGGCAAGTCCTCACTACACAGCCTGGGCTTCAGTGTAAAGCGCCTACAACACAGACTGGCTGGCATTGAGAGTTGGTGCTA

CTCTCTACCAGTGATCTCAGCTTTTTGACAGACACTTTTGTGATTGGGTTAGCCCCACTTGTTTCTGAAAACAGTTTCCATGCGT
CCTACTTACAACCATGGAAGCACAGCTTTGAGGTCAGGGTAGTCAGAAAGACCTCCCAGAGCCTATTATGCACAGTGCCTGGGATT
GTTTTCTGCCTGAACTTTCTTAGGGAGAGTGCTGTATTGGCTAACTATGCCCTGAGGATCTGGTAGCACCTGTGATAGTATTACAG
TGGAGCTTGTATCTGTGCGTCTCTTGAAGCCAGCCTGCAGCAGTGTGTTCTGGTTTAGCCTATGTACTTCCAGACCATTGTGGAAG
GATGACATCTTTCTTGATTGCTTTCTTTTAATAGTCTATTTATGTTTTGTTGTTAAAAACACAGCATTGGTTCGATTTGCAAATGT
ATAGAATTACCAGCTATAGATAGATACCTATCAGAAATTCAGAGAAATTAAATTTTCTTCTACAAGAGTTAAAAAAAATCATGTAG
CAAACGTATCCCATAACCCCGACTGTTGAATGCTGGTTCCCTCCGTGGGCCTTTGAGCCTGGCATGCAGAACAGGAAGCTCCTTGA
GTGACAAATGTCCCTACAGTATGACTCCATAACGTAGCCTGAAGCCAGGGGCTACAGACATCCCTAAGCAATGGCTCTCCCTCTCC
TTCTCTGTGTAGACACCTCTGCACTTGGCCGTGATCACCAAGCAGGAAGATGTAGTAGAGGATTTGCTGAGGGTTGGGGCTGACCT
GAGCCTTCTGGACCGCTGGGGCAACTCTGTCCTGCACCTAGCTGCCAAAGAAGGACACGACAGAATCCTCAGCATCCTGCTCAAGA
GCAGAAAAGCAGCGCCCCTTATCGACCACCCCAATGGGGAAGGTAAGAGCAGTCGCCTTGCTGGCTAACCGTCCCCTCTCGGCTGC
AGCATAAGGGCTTGGCATTTGACAAATGCGTCTGACATGACTTTCACCATGAATACTCTCCTCGGCCAGCTGGAGTTTGTCATATC
TTGTGGTTATTTCTGCTGTTTATTACAGCAAACGCCCTTAAAGAGTATTTGTGAGTATTGCTTAGTAAGTGGAAAAGCAAGCCCGA
GCAAACATTAGTCTTTATAAAGCATCGTGTGTGTGTGTGTGTGTGTGTGTGTGTGTGTGTGTGTGTGGAGAACGTTTACATAGT
AGCTCAGTGTCTCCCGAGAACACAGTGGGAGCTTGTGGCTTCTGAAAGTAGAACAGTGGACCGTCTGAGGCTTTGAAGCACGCCCT
GAACAGAAACCGGAAGTGCTGCGACCTGGTGGTGTCCCTCCTCCCCGCAGCCCAGCTCTGTCAGTCTCACCCACATCTCTGCCTTT
GTTCGGCCTTAGGTCTAAATGCCATCCACATAGCTGTGATGAGCAATAGCCTGCCATGTCTGCTGCTGCTGGTGGCTGCCGGGGCA
GAAGTCAATGCTCAGGAGCAGAAGTCTGGGCGCACAGCGCTGCACCTGGCCGTGGAGTACGACAACATCTCCTTGGCTGGCTGCCT
GCTTCTGGAGGTAAAGGCGTACTTGTGATCTTGACCTAAATCCCCCTGGAAGTTTTAGGGAAGTCTTTTCAAAGAATGACTCTAGG
GCCAGCTCGCTGTCAGTATTCCACTGTCTGCTTTCTGCATCCCCAGCTCCAGCCCAGCTCTCAACCTGGGGATGCCAGTGACAGAG
CCAGCCCAGTATAGCAGTTGGGTGAATGATGTAACCTGAGGTGCAACGGAGATGTGGAGGTAGACAGTGGAGGAGAGAGCCCCTGC
TAACTCTTTCTTTGTGGGCGGATTTAGGGTGATGCCCACGTGGACAGTACCACCTATGATGGGACTACACCTCTGCATATAGCGGC
CGGAAGAGGGTCCACCAGACTGGCAGCTCTTCTCAAAGCAGCAGGTAAGACGCTGTGGTCACTGATACCAGCAAAGCAAACTGCCT
GCAAGTTCAAAGCTGCCTGCGGAAGCTGCGGTTCTGCCCTGGGAAAGTGCCGGTAAAGGAGACCTGCACCCGGGCCACTCTGCAGC
GGCTGCACTGGAGGCTCTGCTTGGCCTCGGGTTTACCTTGATGCTCACGCTAGTTCCCACTCTCTGGTTATCTACGTTGCCTTTGG
GTTGTTGAGGTTTACTCGGTACCTATAAAATCTTGAGCTGTGTCAGCTTCTGGTCATATTACAAGTCTCCCAGAAGGAAAATATCT
AAAGACTTCCTTTTGCAAAAAAAAAAAAAAAAAAAAGTCTCTTAGAAGCTGAAAGCATGGGAAAAGCAAATGAGCCCTGTATTTGTG
TTACTGTGCACGAGGCACTAAGACTTGCTGGCCTCCACTGCCTTTCCTCGGCTCCAGGCACCATGCTGGCATCCCATATTCACAGT
CTCCTTTACCACATCTACTCCTCAGTAACTCTCTGAGATAAGAACTGTTGCTATTAATCCCATCTGAGCACCAAGAAGGCAGACTA
CAAAGATGATCAAGCCCCACTCCCAGTGTCCCGGAAAGCTGAGTCACAGCACCGCAGCCTGACTGCAGACTCTTATTCTTCACGCT
GTCTTTCTTTCTGCTCTGCCTGGGTTCTAGTCCTTCCTGTAAAGCACTCAGAAACATTCCAGCTCATCTCCTGTCTCCGGCGCTT
TGTGTGTTCAGTGTTAACACATGCTTGATGAGGTTGGCTGGCCTTCCGGGCCTGCCCAGACACACCTGATTTACTTCAGAGGTTGA
TAAGGGAGGATTTGTGAACGACACATTCCTTATAGATTCAGGCAAAGAGGCCTTGGTTGTAATGTGTTGAGTCATTTTGGAAGTCA
CTGATTATACTGGGGGAAAAAAAAAAGGTTGTCTCTTAGTCAGACACCTGAAATCTCAACATTCAGAAGGCTGAGGCAGGAGAATG
GCCACAAGCTTAGGCTACCTTGGTCCACATAGTTCTAGGCAGAACAAAGATACATAGTGAGATACTGTCTCGACAGTAAATATATA
AATGTAAACACTTTTACTTGAATTCACCATGAAAAGGTAGCAAATAATATCTTGTGTGACATAAAGAAAACCAGACAAAAAGCCTA
GCCCTGACCTCCTCGGAGTCACAAAGGTCACTTCAGGGACATGTGTAGCCCTCGGGTCTTCAAACTCATTTCCCAGTCATGTTCCT
GGCCATGTTTTGCTCACGTTATAAAAGAGCAAGTGAGGCAGCAGAGAGTGGCAAGGCAGCAGTGTTGGCCCAAGTCAGAGGTTCTG
TCGGCAGAACTGAAGCAAACACTTCATTCCAAAAGGCTGTGCTGAGCCTGCCTGCCTGCCTGCCACTGTTGGCCAACCCGGAGCCG
GACACCCATAGGTAGAATAGCCAGGAAGGCATGACAGGTAACTCCACTAACATTAGGGTTACGTTTTATGTTGTCTTGCTGGGAGC
TGAAGAACAGGCAATATCCCCAAGATTTGTTAGCACATTTTTTCCCCATAACAACACATCCTGCCTAGCATAGCGGCCTTGTGGTG
TCCATTTCTCTGTTGCTTAGGAACTAGAACTAGGGCCCCAACAGTACAGCACTCAAGGACAGAGCTGACCAAGCAAATCTGCTCCA
CACAAGTAAGCGAGAGTGTGAGAAGTGTGTATTGGTAGATGTAAATGTGAAGGTTCAGGACTTTACCCCTGTGGGTGCATAAATTG
CCACCTACTAGTCCTTAGGTTAAAATGTATCCTTTGTAGTATAATAATAATAAGAATAATAATCCTGCAGAGTTACAAATCCCAGG
AGTACTTGAAGTCTTTCATGTATTTACTTTACATCCTGATCACGTCCTCCCCTCCTCCCAGTGCCCTCCACATGGCTCCTCCTCCC
CACCCCCTCCCTCTCTCTTCACAGGGTCCTTGGCTACCTACTTCCATAGAAGTGAATTAAAATTTTTTAAAAAGTTGTTTTAAATT
TCACTAGAAATGAAAAGTTATCCTGTAATTGCCTTAATGAGCTAGAGGTAACCTGTCCCTCGACATGGGAATCCTGTGTGTGATTG
GAGGCGGACAAGCTTTTTCTTTTACACAACACTGTATTTCCCTGACTCCATAGGAGCAGACCCCCTGGTGGAGAACTTTGAGCCTC
TCTATGACCTGGACGACTCTTGGGAGAAGGCTGGAGAAGATGAGGGAGTGGTGCCAGGTACCACACCCCTGGACATGGCTGCCAAC
TGGCAGGTGAGCTCTGGCCAGCCCATCTCATGGGTATTTACGGGGGAGCCAGTGGCTCAGGAATATAAAACAGATGGTCTTCTTGG
CATGCTCCTCCCTAAAGAACATGCCTTTGTTTTTATGAGGTCTTGTTACCGAGAGGGTCTTAATCTTGAAAGATGGTCTTCATCCA
TGAAAGATGAAGGTCCGGTGAGCCAGAGTGTAAAGCTGTTTGCGTAACTGGATTAGAGTTCAGATATGAGAGGTTAGAAAAGCACA
AAATGCACAGAAGAGTTACCCTGTCAGCACACGACCAGAGTTCTTGCCATTTCTGTCAGAAGCTAGCTTGCTCAGAGCTTAAGTAA
GAGGTTTCTGAACATAAAAATAAACTAGAAATTCTCTTGTGGATTTCCATAAACATATTAAAAACCTTCCTTAAAACACAATAGGG
CTTACACTTAAAGGAGAAAACGCTAGGTGTTTGGACCAAGAGAAAAGAGCCAGAAGGCAAAGTAGGCAGTGTACGGGTCCAACACA
GTCGGGTTTGTAGGAATCTAGACTCTCTGTGCACGTCTAGGAGGCTGTTTGCTGGCTGGGTTCCTACAGGGAACACAGAAGACTGA
CCCCTGACCTGATTCCTACCCTGTGTACCATCAGCAAATTCACCTCAGAGAACACCAGGACTTTTAAAAATCATTTCTGTGAATAT
TTTCTCATGTTTTTCTATCCGACAGGTATTTGACATACTAAATGGGAAACCGTATGAGCCTGTGTTCACATCTGATGATATACTAC
CACAAGGTGAGGTGTGGCAGGACAGATTCTCTCAGCCAGTGTTGCCCCATGCTGTGGTTTACAGGAGGAGAGGAGAGGCCAGCCTG
GGCGAGGGTGTACTGCAAACCGTCTAGATCTTATCTCAGAATCTGAGATCCCTGGAGGTGTGGCCCTGAGCTGCTAGAAGCACCTA
CTGCTGCTGCTGAGCAGGGCCTCTGGACTTGGCAGAAGCTTGGTGGCCTCCATCAGAAGTTGGACAATGACCTGTCCTCTCAGATT
GATTGTCCCTGGAAGGTTAAAGCAGTCATGTTTCTTCCTTAGGGGACATGAAGCAGCTGACAGAAGACACGAGGCTACAACTCTGC
AAACTGCTGGAAATTCCTGATCCAGACAAAAACTGGGCCACTCTGGCACAGAAGTTGGGTCTGGGGATATTGAACAATGCCTTCCG
GCTGAGTCCTGCTCCTTCTAAAACTCTCATGGACAACTATGAGGTAACGCGCCGCCGTACCATTTAGAGTTGTCTGTGTAACTATT
GGTAGTTGACCGTGTCAGCTCCACTGAGCAGCACAGCCTACCCCCTTACATAGTAACTTTATGGAAGGAAACCAGTCATTGCCTGG
AAGGCAAAGGGAGCCAGGCCAGGATGGTGCCTTTTTCTTGAGAAGACTGGTTACCCGTCTCTCGGCATTCCCTGTTTTCAAATTCT
CAGAGCCTTCCTGTGAAAATACTACTGCCCACAGGATGGGGACCCCTCCAAGCTGTGATCGGTGTGTGCATCCCTGTTTGCAAGAG
GGAGCCCCCCAGGGCCATGGCTTCATTGTTCTGTCGGGTGACTCCTATTAGAGCAATTCCAAACTAAAAAATAGACAAACATGGGC
GAGCTACTCCGAATGAGGCTGTCATTGCTAGCTGCTGTCTTCCCTGGATTTCTGTGCGTAGACATCACCGTAGGCAGTCTCCTGTC
TCCTGTCTCCATGAACTCACAGGCTTCTTTCCCTTCCTTCCCCTCTTTTTTCTTATCTCTCTCCCCNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNCTACACAGAGGCCATTGAAGTGATC
CAGGCAGCCTTCCGCACCCCGGCAACCACAGCCTCCAGCCCCGTGACCACTGCTCAGGTCCACTGTCTGCCTCTCTCGTCTTCCTC
CACGAGGCAGCACATAGGTAAAGAGGCTGAGAGAGTTCCTTTCCTCGCTGGCAAGGTCTTTGTGCTCTGGGACGTGGCACTACACT

CCCTTTCCCTAGTCTTTTTCCTGACATGTGTTCCTCTGTGGCATGCCACCCGCTCCCTCCCCACAGTGCTCCTCTGCGCACACAGG
AACAGGCAGTTCCCACAGACACTCACATTGGCATATAGGTCACTTAGAATCCCAGTAGACACTGGACATGGCGGGGAGGGTTAAAG
AAACACTACTTCTCATATTTGTTAAATTTGGAGAAAGATTCTAAGAAAGGTGCACAGCCCTGGGCCACAGGACAAGAAGTAGGACC
CAGGAAATATGCATACTGTATGACCCTTCCTTTCATGCCACCACCCTTCAGGTCCCCCACCTCCTCCCTCCCTCGGCACTCCCCGT
CCTTCAGCTCCCACCCTGCTCATGGTCCTGCCTTTGTCTTCCCACACTAACATCCACCTGTCACCCCCAACACATGCATTTGTACG
AATGGATCTCGGTACAGGCTTGAATTATGTCATTGTTCCCGGCCCGTGCTTTCTCTAACCTCTGCACCCTTTCTTCTTGTTCATTT
TCTACCCTAGCCACAAACTCCACTGAGACCAGGAACAACTAGCATTCTCCCATCTTTGTATTATGCCAGCACCCCGGTGGCAGGCC
TTCCATCTGGGCTCAGTAGGTTATGTCTGCCTTCCACCTGGGGCAGTCCTCCCCTGGGAGCCTGCCTGTCACCTTCAGCAGGTCCT
GGACACCTGATTCTTGCCTCTTTGTCCTGTACTGTCCTTCCTTCCCCACCTGCTGTGGAGAACCTCTTGCTAGGATGTAACTTCTC
ATCTTTAAACACTGGCCCGCCTGGCCACAGGCTGCTTAGCCCCGCTTTGATTAATGTTACATCTCAATGAATGGCTTTGTCCATTG
ATAGCGCTTTGGTTAGCTCATGTTTTCACGTTTGTATGATACATGCTGTTATTTTTGTTCGCATTTCTCATATGGGGAATCTACA
GGTAAACACAGCCTCCAAGTTCAGTAGAAAAATGTCATCATTAAGAGTATTATATCCAATAAACAGTAAACAGATTTTTTAAAAGA
GAAAGAAATTTAGAGAAAAACGAGACATCACTTCCTTGTTACACCCTTTAGTTGAGTTCAAACTTCTCAGAAGAATATGATAACTG
CTGGTCAGCTCACCACCTCTGTCTGCCATCTGCTTCTGTCCCCTCCTCTCCTANNNNNNNNNNNNNNNNNNNNTCCCATTTTCACA
TGTGTTCCCTACCCTTTCCATCCCTTTAAAGCCTGTTTTCCGCCCCCTGGAGGCCTTTGCTAGTTTTCTGGCCTCTGCTCACGCTC
ACTTCCACATGAATATACATACGTGACTGTTGGAAGCTAGGATCCACACGTGAGAGAGAACGAATGGTATTTATGTCTCTGGGCTG
CCTTGATTAGTATTCTCCATTAACCTGCAAGTTTCATCTCTCTTTACGGCCGAATAAACTTTTATCATCTTCACACAACTCTTGGC
TCTTGCACAAACTCATACTGGCCATGACTGTCAAAAGTATAGCCAGGCTGTAACAAAATCTAAGAGTGGCAATGAATACTTTCTGG
CATTCAGGTCCTCATGCTTAACCCTGAAAGGGCATCTCTTCTTGGCAGATGCCAAGTTCCGCCCATCCTCACTGCTTGCCTGAACT
CCAACAGTGGCTCCTAGTCAGCCTCTTTGACTCTTTGCTTGCCCCTCCATCAGTTCTCTGCACCACAGTGATGTGACATGATAGGG
CTCCAGTCACAATTAAAAACTAAATACTTAGCAGCTAGGAACACGGCAAGTGTCCCCTCCTCTACCTTTGGGAAAGCCAGGTAGGG
TGACAAATATGTACAACCCGATAGCCCACTCATAGAGGAAAGGACACCGAGAGAGGTGGATCCACGGGACTCACTGGCCAGTCTCT
AAAAACAAGATGGAGAAGGAACAAGGAAGTCACACAATGTTGACCTCCCCACACCCCACACCTGCACATGAGCACACATCCCACAA
ACACAAAGCAAATACTTAAATACTGTATTAAATTTCCATCATTGTGACCAACTTAGGAGAGATTTGGTTGGGCACACAGTTTTTAT
AGGTCCCATGGTCCACTGCCTTCGGGGTATAGCAAAACCCTTCATCACGGAGGAAGCACATGGTAGAGGAATCCTACTCACTTCAA
GGTGTTTGGAAGTACACAGAGATGGGGAGGAGCCCCAGTACAGATCTCTTTCAAAGCCACGCCCTCCATGACCTAACTTCCTTCTG
TGAGGCCCCATAGTCAAAGAGTTGTATCACCTCCAGATAATGTCACAGGCTAGAAACCAAGCCTTTACTTAGCACACAGCCTTTGT
GGGACATTTTAAGATCCGAACCATAATACACAGTAAGCCTTTCAAATGCTGCCCAGTCCCTGTTACACAACAGACAGTTGACTGGA
TGAGGTAACACCAGGACACCGCTCTGGCATGGTCATTGAGTCTCAGTGTTCTGACGTTAAGATTCAATGATGAGAGTCAAACATTT
GCTTAGTAAGTGTTATGCTTCTGTTTCTTTAGTCACTCTGTACAGAAATAATCTGCTCTGGTTCCCAGGAATGCTTTTCAGGTGAT
AGAATTTAACTTAAGCTTTTGGTATAGACACATTTAAAAAAAAAAAAAAAAAAGCCTCATTTTCTTGCACCAAAAGAAAATTTTCTA
GGCAATGTGTATTCTTTAAAAGCTCATACAGCTCAGAGAAAGGTGCCATTTTTAGTTCCTGCTTCTGTTATGAGTTTATTTTGCCT
TGTATTCAGCAGTTTGCCCAGCCGTTAAAAAAAAAAAAAAAAAAAAAAAAAGACAAAAGGTAAAATGATAGAGCAGTTAGAAAAATA
TGCCTCACTTTACAAAGGAAAACATGTTTATCCTGAACCAAGAGAATTCAGAACAGATTCTGTTCAACAGAAAGACTGATGTACCC
TGTAGCACTCTCTAATGTCATTTGATAACCCTGCCATTCACACAGCCAAGCAGCACCGGTGGCATGATGGGGAGGTGGGATGCCGT
GCTGCGCAGGAGCTAGCAAGCACAACCCTCCATACCATAGTCCACACGGAGCCTGGGAGGGAGCCCACTGCTGTGGATGGTGCTCT
AGGGCACTGTGACAAAGCTCTCTGGGTTTATGTCAGAAGGGATTCAGGGCAGGAACCCAAAGACTTTGAATCTGAAACTGCTGAGC
ACCCCGGCTTGCTGCCTCATGGGCTCATGCTCCGCTAGCTTTCTTTTCCTTTTTTTGTTTTGTTTTTTTTTTTTTTTTTTTCGAGA
CAGGGTTTCTCTACATAGCCCTGGCTGTCCTGGAGCTCACTTTGTAGACCAGGCTGGCCTCGAACTCAGAAATCCGCCTGCCTCTG
CCTCCCGAGTGCTGGGATTAAAGGCGTGCGCCACCATGCCCGTCTTCCGCTAGCTTTCTTACACAGCCCTGGACTGCCTCTCAGGG
AGTGGTGCTGTCTACAGAGGATAGGCCCTCCTCCCGATCAGTTAACTGTCAGGACACGCCCACAGACCAGTCTCATCTGGCTGGGC
CCTCCCTCATCTGCACCTCCCTCCTCAAGTGATTCTAGACGTGTGAAGTTGACAGTTGAAGGTGACAGGGACAGCAGGCAGAACAA
AGCCCTTGCCATGGCCTAACTCAGCCCCTCATGAGTCTGTGCTTTCTCCGCAGATGAACTCCGGGATAGTGACAGCGTCTGTGACA
GTGGTGTGGAGACATCCTTCCGCAAACTCAGCTTTACAGAGTCTCTTACTGGAGACAGCCCACTGCTATCTCTGAACAAAATGCCC
CACGGTTATGGGCAGGAAGGACCTATTGAAGGCAAAATTTAGCCTGCTGGCCGTTCCCCCACACTGTGTAAACCAAAGCCCTGACA
GTCCATTGCATCGTCCCAAAGGAGGAAGGCAAAGCGAATCCAAAGGTGCTGGAGAATCGCCGGCCTGCAGGGTCACTCGATTTCAT
TCAAGGCCTTCCGAATTTGGCGTCCTTTCTTGGTTCTGAAATGAAATGTAGTTGCCACGCACAGACGGTGTCTAGCAATCATGGCG
CTCGCTCGCTCAGCTGCACTCTATGGCTCAGGTGCAGTGTCTTGAGCTTTCTCTGCTGCTACTGGATCACATTTGCTTTGTGTTGT
TACTGCTGTCCCTCCGCTGGGTTCCTGCTGTCATTAAAAGGTGTCGCTGTCCCCACCCGGTGTCCTTTCTAGCCATCTACTGTAAG
TTGTGCATTCAAATTAAGATTAAGGAAAAACATATTTTTAAATGAGTACCTTGATGCGCAATAAAAAAAAGACATTTCTTTTTTTA
ATGTGGTTTATCTGTGATTTAAAAATAAAAAACACATGAACTTACAATATTTAAAACATGCTACAATCAGTGCTGAAAATAGTATT
TTCCCCGTTTTATGCATTTTACTATTGTAAATATGTTTTCTAAATCAAATACTTTAAAAGAAGAAATGTTGAATTTATAAATGCTA
TTTACTTTTTTATTTTACTTTTATAATAAAAGTACAAGCACATTGTTGACCTAATCTTGTCTGATCTCTGCAAATGCTTTCTCAGA
GGAGTCCCTGTGACTGTGGGAACAGCCCCACAGCCCGTTTGTCACATCCACATTCTCCTTTAGACACATTTCTTTGAAGCTAGAAA
TGTTCAAACTGTGGAAATAAAAGTGTTCTAACCCAAGACTGAGGTTCTGACCCAGGCAACATCATCTGGACGCCTAGCATCAACAT
TCCAATCTCTACACCGTTCCTCTCCAGCGCATGCTGTCTGTGTCTATGAAGGATAACAGACCCCAGCTTCAGCACTTCATCTTCTG
GATACGATGTGGCCGTTGCGTTCATAGCTAACTACACAAAACCTGTACAAGATGTGGTCCTTCATCATGTATAGAGAGGGACTGAT
GAAATACACACACACACACACACACACACACAGACACCACAGCAACTGGCTGTTAATAGTTGCTAGGGGAATGGGAGTCCTTTCTT
CAAATGGTATAGCACATACTCCAGTAACTATTCCCTCCCCATGTTCATGCAGGCAGCCCTAATTAAACTCACTCATACACACAAGC
ACAGAAGCAGGAAAGTAGGAGGCGGAAACGAGAAGGCAGCGGGGTTTGTGGATATGATCTCAGTACATCCTATACATGTTTAAGAT
CATGAGAAAATATACATTTTAATTAAAAACTAAATTCCAACATATGTAAAAGATTTTCCAGTATGGAGTTTAGTAAGTCTGCATGG
CTGAAAGTAGAAAGAGGGAAGATTAATGGCTTGAAAGAGGTCTTAGTTTGGACTCTGGTGTGTGACCAAGGTCATTTAGTGTGTAG
TGTAAAGATCAAATCCAAAACCTTCTGGTGAAGTGGCTTGGCAGTTAAGAGCACTGATCTTCCAGAGGACCAGTCAGTAGGTTTAA
TTCCTAGAACCCCTCACAGCTCACAACCACCTGTAACTTCGGTCCCAGGGAATATGATGCCCTCTTCAGGCTTCTGTGGGCACCAG
GCATATAAATGGTGCACAGATATACATGCAGGCAAAACATCCATACACACAGAAAGAAAAAACCAAAAACCTAAGGCCTTGTCATG
CCACACAAGTGATCTACTACTGATCCCACGGAAGGGCCTGGAAACCCACCAGCTATTACCTTTGGGAACTGCCTTCACTACAAAGA
GCTCTCACCCCCATTTCCATGGTAGTCATATGAATGCCTTTCCCATTGCTGGGACCTTCAGATCCTGGGAGACAGGCTAAAGTGAT
CTGGGCTATCTCCCTCACACAGCTCAAGGTACCCCAAATAGACATCTTGTATGTCAGTGTCCATATGACAAAATATTCCAAACTTA
CAATGAAGAAAATGGAGGTCCAGAGAGCGCAACTCAGTTGTGGCTAAGGTGCATCTGTGTTTATATCCAGATACTTAGCGCCTAGG
CTTGTGAGTTAGAGAATGATTTTATTGGACAAAAAAGATGATAATGACTGAATCTATTAGTAGGGATTTGGTATAAGATTAAGTA
AAATATCATTAATGAAGAAATGATTAATTATTTAAATATATAATACAAGCTACTTAATAATACTTAAATTATTTAATAAATATTCT
AAGTGCCTAATCATCAATTAATCGGAATCCCTTTCTACCAAAAATTCCATATGTATGAAAATTTAAACCATACGAATTTTAGACAT
ACCACCAATCTTAGACAAACACCCTTGACATCGAGAAGGCCCTAATCGAGACTTCATTCTGTGATGTAAAAGCAGAGCAGATTCTA
CCTCTTAGAATGCATCACAAATGAACAGAAACTGGTTAAACAGCAAATTCTGTTTTCCATTACAGTACCCTCTCTCCACCCTAACA
CACCCTTATTCTGCGTTGGAAACTTTAAGACAAAACTGTCTTGCTTTGGTGTTTCGTAATACTTCCTGGGACAGCCATGCCACCAT
GCCCCAGCCAGGACTTTCTATGGCTGTTAGCTGTGCGTCTTAAAACATGGTGCTGAAGCCCTTCCCACAGAGAGACACCATTGTTT

CCTGGGTTTAGGCTCCTGAGTCCAGGCCACCTGTGGCAAGGCAGGAACGATGCTGAGTGACTTCCGAAACTAGGTCCGAATTTCTG
CCAACCTTCCCGGAACTACCTTCTCCTGGAACTCAGTCACCCTGTTCAGAGGGTGCCAAGCAGCCATGAAGAGAAGGCCAGTACAG
GTGTTCAGCCAACAGCCTTAACCGAGGTCCTAGCCAAAGCTTGCCAAGCCGACGGCTGCCAGGTGACCCTGCCTAGCCTTTAGGCT
GTCTCAGCAGATGCTGACCACAATAAACATGGGCTGTTTTGCTGTTCTCAAAGGACAGATTTGAAAGCAAATTAAATACTAGTGGG
GGTTTTAAGCCCTGGGTGTAGAGTGGACACCACCATTCTCCAGGCATTTGAAAAGACAGCTTGCCTACCATAAGTTCAGCAGAAAA
GATACTGACTGGAGAGACGC

MOUSE SEQUENCE - mRNA (SEQ ID NO: 26)
AGCGGCCGCCGCGGGCGCGCTCTAGCAGCGCAGGCCGGAGCTCAGGGCCCCGCGCGCCCGGCCCGCCCCGCGCTTCTCCGCCCGCG
CCGCAGCCATGGCGCGCCGCTGAGCCGCCCGCCCGCCCGCCCGCGCCCCGACCCGGCTCGGCTCCCGCCGGTCCGCGCCGCTCCGC
AGCGGAGCCCGCAGGCGAGGAGAGGCCGCGCGCATCTCCAGGGTACCCTCAGAGGCCAGAAGAGGGTGTCAGAGCCCTTGTAACTG
GAGTTTGACGGTCGTGAGCTGCGCATCTTCACCATGGCAGACGATGATCCCTACGGAACTGGGCAAATGTTTCATTTGAACACTGC
TTTGACTCACTCAATATTTAATGCAGAATTATATTCACCAGAAATACCACTGTCAACAGATGGCCCATACCTTCAAATATTAGAGC
AACCAAAACAGAGGGGATTTCGATTCCGCTATGTGTGTGAAGGCCCATCACACGGAGGGCTTCCGGGAGCCTCTAGTGAGAAGAAC
AAGAAATCCTACCCACAGGTCAAAATTTGCAACTATGTGGGGCCTGCAAAGGTTATCGTTCAGTTGGTCACAAATGGAAAAAACAT
CCACCTGCACGCCCACAGCCTGGTGGGCAAGCACTGTGAGGACGGGGTATGCACCGTAACAGCAGGACCCAAGGACATGGTGGTTG
GCTTTGCAAACCTGGGAATACTTCATGTGACTAAGAAAAAGGTATTTGAAACACTGGAAGCACGGATGACAGAGGCGTGTATTAGG
GGCTATAATCCTGGACTTCTGGTGCATTCTGACCTTGCCTATCTACAAGCAGAAGGCGGAGGAGACCGGCAACTCACAGACAGAGA
GAAGGAGATCATCCGCCAGGCAGCCGTGCAGCAGACCAAGGAGATGGACCTGAGCGTGGTGCGCCTCATGTTCACAGCCTTCCTCC
CTGACAGCACTGGCAGCTTCACTCGGAGACTGGAGCCTGTGGTGTCAGACGCCATCTATGATAGCAAAGCCCCGAATGCATCCAAC
CTGAAAATCGTGAGAATGGACAGAACAGCAGGATGTGTGACGGGAGGGGAGGAGATTTACCTTCTCTGTGACAAGGTTCAGAAAGA
TGACATCCAGATTCGGTTTTATGAAGAGGAAGAAAATGGCGGAGTTTGGGAAGGATTTGGGGACTTTTCCCCCACGGATGTTCATA
GACAGTTTGCCATTGTCTTCAAAACGCCAAAGTATAAGGATGTCAACATTACAAAGCCAGCTTCCGTGTTTGTTCAGCTTCGGAGG
AAATCAGACCTGGAAACTAGTGAACCGAAACCCTTTCTCTACTACCCTGAAATCAAAGACAAAGAGGAAGTGCAAAGGAAACGCCA
GAAGCTTATGCCGAACTTCTCGGACAGCTTCGGCGGCGGCAGTGGAGCGGGAGCCGGTGGTGGAGGCATGTTCGGTAGTGGCGGTG
GCGGAGGGAGTACCGGAAGCCCTGGCCCAGGGTATGGCTACTCGAACTACGGATTTCCTCCCTACGGTGGGATTACATTCCATCCC
GGAGTCACGAAATCCAACGCAGGGGTCACCCATGGCACCATAAACACCAAATTTAAAAATGGCCCTAAAGATTGTGCCAAGAGTGA
TGACGAGGAGAGTCTGACTCTCCCTGAGAAGGAAACTGAAGGTGAAGGGCCCAGCCTGCCCATGGCCTGCACCAAGACGGAACCCA
TCGCCTTGGCATCCACCATGGAAGACAAGGAGCAGGACATGGGATTTCAGGATAACCTCTTTCTCGAGAAGGCTCTGCAGCTCGCC
AGGCGACACGCCAACGCCCTTTTCGACTACGCAGTGACGGGGGATGTGAAGATGTTGCTGGCCGTGCAACGCCATCTCACCGCCGT
GCAGGATGAGAATGGGGACAGTGTCTTACACTTAGCCATCATCCACCTCCACGCTCAGCTCGTGAGGGATCTGCTGGAAGTCACAT
CTGGTTTGATCTCTGATGACATCATCAACATGAGAAATGACCTGTATCAGACACCTCTGCACTTGGCCGTGATCACCAAGCAGGAA
GATGTAGTAGAGGATTTGCTGAGGGTTGGGGCTGACCTGAGCCTTCTGGACCGCTGGGGCAACTCTGTCCTGCACCTAGCTGCCAA
AGAAGGACACGACAGAATCCTCAGCATCCTGCTCAAGAGCAGAAAAGCAGCGCCCCTTATCGACCACCCCAATGGGGAAGGTCTAA
ATGCCATCCACATAGCTGTGATGAGCAATAGCCTGCCATGTCTGCTGCTGCTGGTGGCTGCCGGGGCAGAAGTCAATGCTCAGGAG
CAGAAGTCTGGGCGCACGCCGCTGCACCTGGCCGTGGAGTACGACAACATCTCCTTGGCTGGCTGCCTGCTTCTGGAGGGTGATGC
CCACGTGGACAGTACCACCTATGATGGGACTACACCTCTGCATATAGCGGCCGGAAGAGGGTCCACCAGACTGGCAGCTCTTCTCA
AAGCAGCAGGAGCAGACCCCCTGGTGGAGAACTTTGAGCCTCTCTATGACCTGGACGACTCTTGGGAGAAGGCTGGAGAAGATGAG
GGAGTGGTGCCAGGTACCACACCCCTGGACATGGCTGCCAACTGGCAGGTATTTGACATACTAAATGGGAAACCGTATGAGCCTGT
GTTCACATCTGATGATATACTACCACAAGGGGACATGAAGCAGCTGACAGAAGACACGAGGCTACAACTCTGCAAACTGCTGGAAA
TTCCTGATCCAGACAAAAACTGGGCCACTCTGGCACAGAAGTTGGGTCTGGGGATATTGAACAATGCCTTCCGGCTGAGTCCTGCT
CCTTCTAAAACTCTCATGGACAACTATGAGGTCTCTGGGGGTACCATCAAAGAGCTGATGGAGGCCCTGCAACAGATGGGCTACAC
AGAGGCCATTGAAGTGATCCAGGCAGCCTTCCGCACCCCGGCAACCACAGCCTCCAGCCCCGTGACCACTGCTCAGGTCCACTGTC
TGCCTCTCTCGTCTTCCTCCACGAGGCAGCACATAGATGAACTCCGGGATAGTGACAGCGTCTGTGACAGTGGTGTGGAGACATCC
TTCCGCAAACTCAGCTTTACAGAGTCTCTTACTGGAGACAGCCCACTGCTATCTCTGAACAAAATGCCCCACGGTTATGGCAGGA
AGGACCTATTGAAGGCAAAATTTAGCCTGCTGGCCGTTCCCCCACACTGTGTAAACCAAAGCCCTGACAGTCCATTGCATCGTCCC
AAAGGAGGAAGGCAAAGCGAATCCAAAGGTGCTGGAGAATCGCCGGCCTGCAGGGTCACTCGATTTCATTCAAGGCCTTCCGAATT
TGGCGTCCTTCTTGGTTCTGAAATGAAATGTAGTTGCCACGCACAGACGGTGTCTAGCAATCATGGCGCTCGCTCGCTCAGCTGCA
CTCTATGGCTCAGGTGCAGTGTCTTGAGCTTTCTCTGCTGCTACTGGATCACATTTGCTTTGTGTTGTTACTGCTGTCCCTCCGCT
GGGTTCCTGCTGTCATTAAAAGGTGTCGCTGTCCCCACCCGGTGTCCTTTCTAGCCATCTACTGTAAGTTGTGCATTCAAATTAAG
ATTAAGGAAAAACATATTTTTAAATGAGTACCTTGATGCGCAATAAAAAAAAAGACATTTCTTTTTTTAATGTGGTTTATCTGTGA
TTTAAAAATAAAAAACACATGAACTTATCAATATTTAAAACATGCTACAATCAGTGNTGAAAATAGTATTTTCCCCGTTTTATGCA
TTTTACATTTGTAAATATGTTTTCTAATCAATACTTTAAAAGAAGAATGTTGAATTTATAAAATGCTATTTACTTTTTTATTTATA
ATAAAGTACAGCACATGTGACT

MOUSE SEQUENCE - CODING (SEQ ID NO: 27)
ATGGCAGACGATGATCCCTACGGAACTGGGCAAATGTTTCATTTGAACACTGCTTTGACTCACTCAATATTTAATGCAGAATTATA
TTCACCAGAAATACCACTGTCAACAGATGGCCCATACCTTCAAATATTAGAGCAACCAAAACAGAGGGGATTTCGATTCCGCTATG
TGTGTGAAGGCCCATCACACGGAGGGCTTCCGGGAGCCTCTAGTGAGAAGAACAAGAAATCCTACCCACAGGTCAAAATTTGCAAC
TATGTGGGGCCTGCAAAGGTTATCGTTCAGTTGGTCACAAATGGAAAAAACATCCACCTGCACGCCCACAGCCTGGTGGGCAAGCA
CTGTGAGGACGGGGTATGCACCGTAACAGCAGGACCCAAGGACATGGTGGTTGGCTTTGCAAACCTGGGAATACTTCATGTGACTA
AGAAAAAGGTATTTGAAACACTGGAAGCACGGATGACAGAGGCGTGTATTAGGGGCTATAATCCTGGACTTCTGGTGCATTCTGAC
CTTGCCTATCTACAAGCAGAAGGCGGAGGAGACCGGCAACTCACAGACAGAGAGAAGGAGATCATCCGCCAGGCAGCCGTGCAGCA
GACCAAGGAGATGGACCTGAGCGTGGTGCGCCTCATGTTCACAGCCTTCCTCCCTGACAGCACTGGCAGCTTCACTCGGAGACTGG
AGCCTGTGGTGTCAGACGCCATCTATGATAGCAAAGCCCCGAATGCATCCAACCTGAAAATCGTGAGAATGGACAGAACAGCAGGA
TGTGTGACGGGAGGGGAGGAGATTTACCTTCTCTGTGACAAGGTTCAGAAAGATGACATCCAGATTCGGTTTTATGAAGAGGAAGA
AAATGGCGGAGTTTGGGAAGGATTTGGGGACTTTTCCCCCACGGATGTTCATAGACAGTTTGCCATTGTCTTCAAAACGCCAAAGT
ATAAGGATGTCAACATTACAAAGCCAGCTTCCGTGTTTGTTCAGCTTCGGAGGAAATCAGACCTGGAAACTAGTGAACCGAAACCC
TTTCTCTACTACCCTGAAATCAAAGACAAAGAGGAAGTGCAAAGGAAACGCCAGAAGCTTATGCCGAACTTCTCGGACAGCTTCGG
CGGCGGCAGTGGAGCGGGAGCCGGTGGTGGAGGCATGTTCGGTAGTGGCGGTGGCGGAGGGAGTACCGGAAGCCCTGGCCCAGGGT
ATGGCTACTCGAACTACGGATTTCCTCCCTACGGTGGGATTACATTCCATCCCGGAGTCACGAAATCCAACGCAGGGGTCACCCAT
GGCACCATAAACACCAAATTTAAAAATGGCCCTAAAGATTGTGCCAAGAGTGATGACGAGGAGAGTCTGACTCTCCCTGAGAAGGA
AACTGAAGGTGAAGGGCCCAGCCTGCCCATGGCCTGCACCAAGACGGAACCCATCGCCTTGGCATCCACCATGGAAGACAAGGAGC
AGGACATGGGATTTCAGGATAACCTCTTTCTCGAGAAGGCTCTGCAGCTCGCCAGGCGACACGCCAACGCCCTTTTCGACTACGCA
GTGACGGGGGATGTGAAGATGTTGCTGGCCGTGCAACGCCATCTCACCGCCGTGCAGGATGAGAATGGGGACAGTGTCTTACACTT
AGCCATCATCCACCTCCACGCTCAGCTCGTGAGGGATCTGCTGGAAGTCACATCTGGTTTGATCTCTGATGACATCATCAACATGA

GAAATGACCTGTATCAGACACCTCTGCACTTGGCCGTGATCACCAAGCAGGAAGATGTAGTAGAGGATTTGCTGAGGGTTGGGGCT
GACCTGAGCCTTCTGGACCGCTGGGGCAACTCTGTCCTGCACCTAGCTGCCAAAGAAGGACACGACAGAATCCTCAGCATCCTGCT
CAAGAGCAGAAAAGCAGCGCCCCTTATCGACCACCCCAATGGGGAAGGTCTAAATGCCATCCACATAGCTGTGATGAGCAATAGCC
TGCCATGTCTGCTGCTGCTGGTGGCTGCCGGGGCAGAAGTCAATGCTCAGGAGCAGAAGTCTGGGCGCACGCCGCTGCACCTGGCC
GTGGAGTACGACAACATCTCCTTGGCTGGCTGCCTGCTTCTGGAGGGTGATGCCCACGTGGACAGTACCACCTATGATGGGACTAC
ACCTCTGCATATAGCGGCCGGAAGAGGGTCCACCAGACTGGCAGCTCTTCTCAAAGCAGCAGGAGCAGACCCCCTGGTGGAGAACT
TTGAGCCTCTCTATGACCTGGACGACTCTTGGGAGAAGGCTGGAGAAGATGAGGGAGTGGTGCCAGGTACCACACCCCTGGACATG
GCTGCCAACTGGCAGGTATTTGACATACTAAATGGGAAACCGTATGAGCCTGTGTTCACATCTGATGATATACTACCACAAGGGGA
CATGAAGCAGCTGACAGAAGACACGAGGCTACAACTCTGCAAACTGCTGGAAATTCCTGATCCAGACAAAAACTGGGCCACTCTGG
CACAGAAGTTGGGTCTGGGGATATTGAACAATGCCTTCCGGCTGAGTCCTGCTCCTTCTAAAACTCTCATGGACAACTATGAGGTC
TCTGGGGGTACCATCAAAGAGCTGATGGAGGCCCTGCAACAGATGGGCTACACAGAGGCCATTGAAGTGATCCAGGCAGCCTTCCG
CACCCCGGCAACCACAGCCTCCAGCCCCGTGACCACTGCTCAGGTCCACTGTCTGCCTCTCTCGTCTTCCTCCACGAGGCAGCACA
TAGATGAACTCCGGGATAGTGACAGCGTCTGTGACAGTGGTGTGGAGACATCCTTCCGCAAACTCAGCTTTACAGAGTCTCTTACT
GGAGACAGCCCACTGCTATCTCTGAACAAAATGCCCCACGGTTATGGGCAGGAAGGACCTATTGAAGGCAAAATTTAG

HUMAN SEQUENCE - GENOMIC (SEQ ID NO: 28)

TTGACAAGCTGGTTCTAAAATGAATTCAGAAATGCAAAGGACCTAGAATAGCCAAAACAACCTTGAAAAAGTGAACAAAGTTGGAG
GACTAACACTTCCTGATTTCAAGACTTATTGTAAAGCTATAAAGTAATCAAAAAGGTGTGGTATTATCATATAGATAGAGAAAAGA
TCAATGGAACAAAATAGAGTCCAGACATACAGATACAACTGATTTTTAACAATTTGCAAAGGCAATTTAGTGAAGAAAGGATAGTT
TTTTTTTTCAACAAATGCTGCAGAATCAGTTGGAAATCAATAATCTAAAAAGAAAAAACTTCAATCCATATCTTGTACCACTATAT
GCAAAACTTAACTCAAAATGTATCATAGACCTGAATTTAAGTCCTCAAACTACAAAAGTTCTAGAAGAAAATAGAGGAGAAAATCT
TAGTGAGTTTGAGTCAGGCAAAGAGTATGTGGATACAACACTGAAACCACAATGCAGTAAAAAACTGACAAATTATACTTCATCAA
ATTTAAAATCTTCTCTGCAAAAGAGGATGAAAAGACAAGTCACAGGCTAAAAAATATGTATTTGCAAATCATATATCTGGTAAAAT
ACTTTTATCCAGAATATAAAATGAACTCTCAAAATTCAATAATAAGAAAAAAAATACACCAAAAGTAAACAAAACATTTGAACATG
CATTTCACCAAATGGCAAATAAGCACATAAACAGATGCCCATCATCATTAGTCATTAGGGTAAAGCAAAGTAAAGCACAATGAGAT
ACCACTAAACATATCAATTGAGACGATTAAAATAAAAAAGACTGACCATACCAAGGATTGCAAATATATGAAGGAGCTAGAACTCT
CATTTCCTGTAGTGGGAATATAAAATAATATACTCTTTGGGAGAACAGTCTGCAGTTTCTTAAAAAGTTACATGTATGCTAACTAT
ACGCTCTAGCCATTTCACTTGTAGGTATTTCCCATGACAAAAGAAAGTATATGTCTATATAAACAGTTAAATAGTTACACACAAAT
GTTTTAGTGGTTTTATTAATATATCCCAAAACTGGAAACAACCTAAATGTCTATGAACAGGAAAATGGATAAATAAATTGTGACTA
TCTATACAATGGAATGCTACTCAACAATAAAAAGGAATTAACTACTGATACAACAAGGTGGATGAATTTTAAGATAATCATGCTCA
ATGAAAGAAGTCAGATAAAAAATAGTACCTACTGTATGATTCCATTTATATGAAACTCTAGAAAATGCAAACTAATCTATGACAGA
AAGCAAATCAGTGGTTGCTTAGGAATGTGGAGGAGTGAGGAATGGAAATGAGGAACTGCAGAGGGCCATGAGAAAACTTTTCAGCT
GATGCACATGGTCACTGTCTTTATTATGGTGATGGTCTCATGGGTATATATATATATCAAAAACAACTCTCTCTCTCTCTCTCT
CCAATCCCCCAACCCCATCTCTTACAACAAGAAAGTATGTCCCTAGTATATCTGGAAGCCATCTTTTGACCAAGAGAAAAGCCAGC
ATGAGGAAACTGTTAACACAGGGACAGAAGAAAAGAAAATGTCTCTGAAGGGAAGAGAGCCAGAATTGTGATTACATTAAACCTAA
AGATTTTCAACGATGTGACCTGATGAAATTCCATAATGGTTAAGTCATTTTGAGTCATAATTTTACATCTTTTCACCTTAGGTGAA
AAGCATCCTAACCAAGATAAGTATATGCTCAGCATTATATTGATAATTTATAATACGCCATTGGAAAAAAACTCTCTTCCTGGCAT
TTAATTATATTACTTATTTCAAAGAAAATGCAGTAAAAAACTGATCTGTATATCTACTGATATGTCTATCAGTAAAACAGATAAGT
ATATCTATGTATACCTACTATGCATAAGCCTTACAGGTGCTACATAGAGGTGATATCTGTTGTTTATACTCTTTGTCTTCCCTTCT
TCCTTTTTTTGATAACAGTGCCCCAATTTACGCTTAGCTAACCATAATCTAGCATTACTAATTCATGTCTTCCAGATGAGATAGAC
CCTCCTTGTCACTAAAAGTAGACATGTGACTCACAGCATTGTAAGAAGATAGTAATGGAACACAGGTCCCCAAATTTACCCTCCCA
ATCTCTTCTCAATTACTTTCCCCAACAAGAAAAAATGCTACTGCAGGTAAGCAGGGGCATCTAAAGCAGGGTCACTTGAGGTCACA
GGCTCCAGGGTCTCTAAAAGGAAAGAGATACTCTCACTCTTCTCTGTTTTAGGTAGTCTGGAGGGCCTTGTTATAGAAAGACTTAA
AACAAAGGTCTCAATTTTATGGCTTCCAGTGAAAGTTAGCCTGAGGAAACCTATGAATGCCTTCAGGAAAGCATTACATGGGCTTA
TTTGTGTGTGTTCTTGGGAAGGTCAGACTAATCTGGGTAGTGACAGGTCATATGGAGGAACGCTGCACTCATGGAAGAGTTGCTTG
TAGCCCCTGAGGTTAGGCAAGCATTCTCCCTTGCACCCAGGGAGCTCTGGGAGAAGAAAGACTTTTTTTAAAGCTTCAGCTGGGTG
TGGACAGATTGCCAGTTGGTTTACTGCCAGCCCACAAAAGCAGAAACCTTTCCCCTGGCAGGTTTGCCACTGCATAAAAGACCCTA
ACTACTTGTCCTGTAATTATAGGTCAGTGTATACAGAGCCTGAGGTCTGAACAGGAAATGGAAAATAATAACTTGCCCTAGTAGTA
CTAGCAAGCCAGAGAGGAAAACTAAGATAAACACTTCAAACAGGTGTTTTGTTGAAATAGAACTGTTAGAAGAGCCAGGTGAAAAC
TTTTCTAAAAAGTAATAAATTCACTCAAGAAGATATAATTAAAGATTAGGAGTTAAATATATGATAGATGCTAGAATGATAAAATA
TACATATTTTAAAAATAGGTAATGACTCCATGTGTTTCTGACAGTATACCCTTCCTGGTTGAAATAACTAAAATGAAATAACTAAT
GGAAATAACTGAAATGCTACATAACATATTTTTAAAAATTAATACTGCTTAGCCAGCATGAAATTCAGGAATCTAAATATTTCCAA
AACTGAAAAACTGGAGGGAATAGCTGAGAAATTGACTTAGTCTGTTTTGTGTTGCTATCACAGAAAACCACAGACTGGGTAATTTA
TAATGAAAAGAAATTTGTATCTCACAGTTCTGGAGGCTGGGAAGTCCAATATCAAGGTGCCAGCATCTTGTGAAAGCTTTCTTGCT
ACATCATCCCATAGTGAAAGGCAGAAGAGCAAAAGAGCAGACACTCAAGAGAGAAAGTAACCTACTCCTACAATAATGGCATTCAT
CCATTTATGGGGGTAGAGACCTCATGACCCAATCAGCTCTCAAAGGTCTCACCTCTCAACACTGTTGAATGGGGAGTTAAGTCCCA
ACACAGGAACTTTGGGGGACATGTTCAAACGATAACACTGCCTATCTAGTCCTTGTCACTGGACAGAAAAAGAAGAAATTCTCACC
TGTGAATTCATAACCACTTACACTATATGTGATGATCAAAAGCACACAAGTAAAAAATTTTATTTAAGGTTTTTCCAAGTTAATAG
TATACCAGATAACAGGCAAAAAGAAGCACAAATATTCCTTACTCAATTTTAAGCTAAGCTTCAAATAATTTCCAGAGATTGTTTCA
AGAAACGTAAGCAATTTATTATTTATAATTCAGTTGACATATTAAACAGCATATGAAATATAGCTGAAAAGAGAATTGATAAACTA
GAAGGCAAATATAAAGAAATTATCCAGAATGAAATCCAAAGAGATAAGAGATATTAAAAGACACAGAGATTAGATTGAGAAGATCT
AACATATACCTAATTTAAATTCCAGAAAACAAGTCTAGGGAAAATGAAAGAGAGGAAATGTTACAAAAGATAATGGACTAGGAATG
TAACTGTTGAAACATCAAATCTTGAGACCCAGGGAGCCATATAACATGCTTCCAAAATCCAAGCTTATTATATTAATATACAAAAA
CATTAATATTAATATTATATATTAATGTTATTGTTAATATAATCATTATATTATTAAACATATAATTTATACATAAACAATATATT
ATATATAAATGTATATGATATATAATATACAATAGTTATAATATATAATAAATTATATATTGACTTCTATATAATATAGAATTATA
TAACTTATAAATTATATATAGCTTTATATATAAATCAATAATTATATATTGATGTGTACAATTATTATTTTATATTATTATTAATA
ATTATATAATTGGTATTATATATAAATATTAATTTTATATTGCAATAGTTATAAAGTAAATATCATATAACATTATTAATATAATAAA
TATAGTATATTAATATTAATAACTTGTATTTATTAACAATATATTAATAAATGACATAATTTCACATTTCTTTCAGATTTCTGTAA
AAGCTTATCAGGAGTTATATATATGCAAAACCATATTTATCTTATTAAAGAGGCATGTATAAGTGTCAGACCATCACTTATGAATA
GATTTGACAGCCAGATATACAACCTTTCAGAAAACAGAAGCAAACCTCGTCCATAAAAACAAGTAATTGGCAGCTGTCTCAATGAT
GTCCAGTAACTAGCCTTATTTCACTCAGAACAGTTTTGTCGGCTTCATATAGCATGACCAATCACCCTGGTTTACTAGAGCCTGAG
GGTTTCCTGCGCAAGGGACTCTCTGTGCTAAAAGCAAGATAGTCCTGGATCAACCGGGATGAGTTAAGTCTCTATTTCCATGCCTT
CATAGAAATCATTAAAAGATATCTCTCTGTTATGGGCTGAATAATGTCACCCACCCTGAAATTCCTATGTTGAAGTCCTAACCCCC
AGTACCTCAGAATGTAACTGGATTTTGAGATAGGGTTTTTAAAGAGGTATATAAGTTAAAATGAGGACAGTGGGATGGCCCTAAT
CTAATATGACTGATGTCCTTATAAGACGAAATGATTTGGACGTGGACACAGAGGGACAACCACATGAGGACAGGGAGAAGATGGCC
ATCTGCAAGCCAAGGAGAGAAGCCCTCAAGTAAACCAATGCTGCCAACACATTGCTGTTGAACTTCCAGTCTCTAGAACGATAAAA

AATATTTCTGTTGTTTAAGCCACCCAGTCTGAGATATTTTGTTTTGGCAGCCCTAACAAACACATTCTCTAAGGGTTTATTCTCAT
TTTTGGTTTAGTGTATTTCAGCTGATGATTATAGCCTAATCCTCCCCCTCTTCTTGGGGCCTCTCTTCTCTGTACTCTTCCTCCTC
ATATCTCACACAGGCAGATTTGTTCCTGCCTCCTCACCTACATTACGAGCTCCGCACAGTCTCCTTTCTTCTGGCTCCGGCTCCCT
CATTAATCTTTCTGTTATTAACGCTATTAACTCGTTAAACTATCTCTCCACCTTTCCAAACTTCCATTAACTCTGGCACTCCATAA
GTGTTAGTCAAATAAATGAAGAATCACACTAACTCCTCTTTCCTTCCCTCTCACTCCAAACTCTTGGTGAGATTCTTTTGCACTTA
TGCCCTCGTTTCCCCATTTCCCACTCACTTGTCAGTCTCCTCATTCTTGGTTTTCCTCATTATATTGAAACACATCTGAAAAAGTC
ATGTAAGACCACAGCCACTGCGTATCAGTGCTATTTGAGAGTTAATAAAGTTCCCTTTTCCTCCAAGACAGCGAGCAGAGTGAACA
GAAGAAAGACTGGTTTCTGCCCTTGGCTGCACAGCCATGCCCCGAGGCCGGGCTTAGGATTTTATAATTCACAAAGTCAACTGTCT
CTTCCCCTCTCCATCCTTTAGGAACTGTCTGCAGTATCTGACACTGTTGAGAGCTCTCATACCCTTTAAACTCTCTCTTCTCTGGC
TTTCTGAGCTTTCCTCCCACTTTTGATGGCACTGCAGGAGTTTTTAAGCATCTCTCTCTCATACCAACCCCAGAAATAAAGTCATT
CCTCGAATTCCTGTACTTAGCCCCTTGTCTCCTAACTCTTTGACCACTAATGTTTTAGAAACACTCTCCTGACAACTATCGCATCT
TTGCAGTTGTTTCCATTTCCAACGCCATGTCTCTCTGAGCAGCAGCCTTGAGTTTTCAATTGCATATTTATTAGTCATTTCCATAT
GGACATCTCACTAAATCTGTCATACTAAACATGTCTAAAATATGTATGTTTACATGTTACATGTATGTTTGGTATGTGGGAATGCA
TTACATTCCCACAGATTTTTTCCCAGTTACAGTGTTACTATTCTAATCACTCAGACTAGAACACTCAGAGCTATCTATGAGTCTTC
CTCTCACTCAAACTGCATATATAATCTATAAACAGGTCCTGTTGACCTCATCTTCTAAAAATCTCTTACATGCTCCTTTTTATATC
ATTGGATAATAGTTTAGTGTGGTGGTTAATATCATGCACACTGGAGCCAAATCTGTTAGGTTCAAATCCTGACTCTCCTTCTATTT
ACTAATTATGAAACCTTAGGAAAATTATTCAACTTTTCATCTGTAAATATGGGGATTATAATGGTGATCCACTTTATATGACTGAC
AACCAAATAAGTGAATACGTGTATAGCAGGTATTAACCATATGCTTAGCCGACCTATTTCAACCTCCTAACCAACCAGAAGAACTG
TAGTTCCCTGCTCTCTTGCATCTAAGTGGGGCCATGGAATGTGGGCAGAAGTGTTGTGTCACCTTTACTCAGAGGCAATTAAAAAG
AAGACATACTTTTCCTATGTTCTCATTGCCAATGAACACACTACACAGATCTCAACCTCAGAATTCTCTCCACACTGGTATTTCCA
AAATAGCTTCATGAGAAGGGAAATTTGTGTAGTTTCAATGATTTAGTGATGCTATGGTTTGGGTATTTGTCTCCTCTAAACCTCAT
GTTGAAATGTGACCCCAATTTTGGAGGTGGGGCCTAATGGGAGATGTTTCGGTCATGGAGGTGGATCCCTCACAAATAGATTGATG
CTATTCCCTGCAAGGAGGAGAGGATGAGTGAGTTCTCACTGCATTAGTTCCTATGAGAGCTGGTTGCTAAAAAGTTTGGCATCTCT
CCCCTCTCTCTCGCCATACGATCTCTCTGCACACACCACCCGCCTTTGCCTTCCATTGAGTGGAAGCAGCCTGAGGCCCTCACCAG
ACGCATATGCTGGTGCCATGCTTCTTGTACAGTCTGAAGACTGTGAGCCTAATAAACCTCTTTTCATTATAAGCTACCTAGTCTTG
TGTATTCTGCCATAGCAACACAAATGAACTAAGACAAGTGACAAACACAAAATAACTTGTCTTTTAGACAATATTGATGTCTGAAA
TTCCACATTGAGGCATTTTTTTCCTCTTCGTAGCCTGATAATGGCAAATACCTTCTGACACCTTAGGATGGATGAATTTGTAAGAG
ATATTCCTGGAAGCAGGTTTGTACAGTGTAGAGAAAATGTTGGGTAACAAAGCAGGAATGGAGGAGGGTTAATCAGCAAGCCAAAA
AGCTTTTATAGTTTTGCCTGCTGTGCTGCATGGAATTACATCCCCCCAAAACATATGTTCAAGTCCTAACCACAGGTACCTGTGAA
CATGAGTTTATTTGGAACTAGGATCTTTGCAAATGTTATCAAGTTAAGATGAGGTAATACTGGATTGGGGTGGGCTCTAAATCCAA
TGACTGGTGTCTTCATAAAAGAAAGGTGAGGGAGAGTTGAATATAGCGACACAAAGGAAACACGGGGAAGGAGACCATGTGACAAT
GTAGGCACAGAATGGAGTGTTGCAGCCAAGCCAAGAATGGCAAGGATTGCTGGAGCTACCAGAAGCTAGGAGGAGGCAAAGGATTC
TTCCCTAGAGCCTTCAGAGGGATCATGGCCTTAATGGCACCTTGAGTTCAGAATTTTAGGCTCCAGGACTGTGACAGAATAAATTC
CCGTTGTAAGCTGTTCAGTCGGTGGTAAATTTTTACAGCAGCCCCAGGAAAATATGCCTGCTCAACACCCTACCCTGAATTTTAAG
TTCATTTTATGGAATCATTTTGAGTTTGGAGTTTGATTTAATCCTTTTCAGTAATTAACGCTCTTCATTTTTTACTCCCAAATATG
TCACACTGGCTCTACTCTTATAACAATTGTATTTATCTATCTTTTGGTAATGGGGCTGCCACCCCTTGCAGAATGAAAAGTAGAGT
GTGTATGCTCACACCTCCCTCCCTCCCTCCCTCTCTCCCTCCCTCTCTCTTTCTTTCTTTCTCAGAGTATCGCTCTGTCTCGCCCA
GGCTGGAGTGCAGTGGCGCAATCTTGGCTCACTGCAACCTCTGCCTCCAGGGTTCAAGCGATTCTCCTGCCTCAGCCTCTGGAGTA
GCTGGGATTACAGGCGCCCGCCACCACGCCCGGCTAATTTTTTTGTATTGTTAGTAGAGATGGGGTTTCACCATATTGGTCAGGCT
GGTCTCAAACTCCTGACCTCAGGGGATCCACCCGCCTTGGCCTCCCAAAGTGCTGGGATTACAGGTGTGAGCCACCGCACCCGGCT
CACAGAATTTCTTAAAAAGTCATTGTGTTCAGTCTTTATGGATCCTCTAAATTCCAGCTGCTCAGCCTCCCGGAGCTAAATGAGAT
CACTTAAAGGTCGACTGTTGGGGCCTCCAAGGTCGCCTCGGAGACTCACCTCATAGGCAATCAAATGGATCGCTGGGTTTTTGTGT
GTGTGGGTTTTTTTTGTTTATTTTTTTTTTCCAGAAAAACACTCCACCAAGAAGGTTTTTATAACCTACTGGAGGAGGAGGATGGA
GAGTGGGCACGAAAGGACTAGGATGAACATGCCATAAGCAATCTAAATGCCACTGCAATATTGCCTGGTGAGGACCTGATTACTGA
TGTTTTAAAGCTAAGCTTTCAGTTGTCACTCCACCCAGTAGTGAAACAATGAGCTCTAAAATATATATTTCGGCTCAAGCTTTCTT
ATGTGGGGAGGTAATCCACCCGAAGGTATCCCCAGCCTGTACCTAATACAGTGCCCAGCACTAAAGCAGCTCAGATGCCAGTGAAT
GGTGGCCACTGGGAGGCCTGTCAGTGGGTGCCAGTAGCGGTCTCTTCAGAGAAAAAGAAAACTCCCCTCTGCCAGATCAGTATTTT
ATGAGCTGTGAACCAAAACCATTGCTACCACCATCACTATAATTCTATCCACAGTAATTATCATAAAGGCCTAACAATGCCTTGTA
GATGAACATTCTGAGTAACTGCTCTATAACCAGGAGATTTAAGACCGCACCAAAAACCAGTAGAGGGTTATACTTTACTGGGCACA
AGTCGTTTATGATAACGAAATTGTAGTTTAATCTGTGAAGAGATGTGAATGTAACTGAGACACGCTTAAATGGAATATACAGATGA
GCTTTATTTTTATATCTGGCATGCTTGGATCCATGCCGACCCTCCAGCTGCTCGGGCCTGCCCTTAGGGGCTATGGACCGCATGAC
TCTATCAGCGGCACTGCCACCGCCGCCGCCTCCGTGCTGCCTGCGTTCCCCGACCATTGATTGGGCCCGGCAGGCGCTTCCTGGGG
GCTTCCCTACCGGCTCCAGCCCTTGGGATTCGGGAGCGCCCTGCTAGGAAGCCAGAGCCCCGCAGGGGCCGCGGCGTCCAGGCCGC
CTAACGCGCGCCCCTCGCCCGGCGCCCCGAAGCGGCCCCGAGGGGCGGGAGCCGAGGCGAGCGGCAAGGCCGGGCCGGGGGCGCAC
AGCGCCCCTAGAAGTGCGGGCTTCCCCCACCCCCGGCAGCGACCCTACCTCCCGCCCCCGCTGCGTGCGCGCGTGTGTCCGTCTGT
CTGTATGCTCTCTCGACGTCAGTGGGAATTTCCAGCCAGGAAGTGAGAGAGTGAGCGAGACAGAAAGAGAGAGAAGTGCACCAGCG
AGCCGGGGCAGGAAGAGGAGGTTTCGCCACCGGAGCGGCCCGGCGACGCGCTGACAGCTTCCCCTGCCCTTCCCGTCGGTCGGGCC
GCCAGCCGCCGCAGCCCTCGGCCTGCACGCAGCCACCGGCCCCGCTCCCGGAGCCCAGCGCCGCCGAGGCCGCAGCCGCCCGGCCA
GTAAGGCGGCGCCGCCGCCCGGCCACCGCGCGCCCTGCGCTTCCCTCCGCCCGCGCTGCGGCCATGGCGCGGCGCTGACTGGCCTG
GCCCGGCCCCGCCGCGCTCCCGCTCGCCCCGACCCGCACTCGGGCCCGCCCGGGCTCCGGCCTGCCGCCGCCTCTTCCTTCTCCAG
CCGGCAGGCCCGCGCCGCTTAGGAGGGAGAGCCCACCCGCGCCAGGAGGCCGAACGCGGACTCGCCACCCGGGTAAGCCAAACTCG
GGCGAGTGGGGGCCCGGCAGGGGACGCGTGGCCCAAGTCCTGCCGCCCAGCCCTCCGCACCCCCTCCAGCCCCACTCGGACTTCCT
CATTCCTGCGCTAACCGCTGGGCTAGACCGTGGGAGGAGGTTGACAGTAGCTGAGAGGCACATGGATTAGCGACAGCGGGAAAG
ACACATCCGGACCTCGCAGGGGCTAGTCGGCCGAAGGGCCGCGGCCGCCCGGCGGTCATTTCTCTTCACGTCCCTCCGCGGGGCGG
GAACGCGAACCGGAGGGGAACTCTTACAAACTTTTAAAATCCCCAACCCCAGCCCCACCTGGGGGATGGTGAGAAGGTTGGAGTGC
GCTGCGGCGCGGAGGAGAGAGGGAAGGTGGTTGGTGCAGTGCAGAACCAGATTTCACCTAAGGGCGTTCCATGAAATGAAAGCAGA
AGTGCTTGTCAGTCCTCTTGGCTGGGAAAGCCCTCCCCAGCTCCCCAGAATTGAATAGGAGAGTCTATTCATCCCTCTCCTACTTC
TAAAAGAAACCTGGCTCGCTCTCTGTCTCTCTCTCTCTCCCCCCTCCCCCCCTCCGTGCGCGCGCGCGCGCACACACACACACA
CACACACACACACACACACGATATAGTCACCTGCTATAGGACTTGATTCTGATCCTCCGGGGCCTGGCATTTGAGAGAGAAAATAA
ATTACCTACCTGGATACTTAGAGCACTTTTTAGACTTGATTATGTAAAACTCTTGGACAGTGCCACCATGCATTATGCATGACCGC
TGAAACAAAAATAATGTAAAAACAAGGCCTATCCTAAATGCAAGTTTTTCTATGGTCCTTAAAAACAGACACCAGGGAATGTTTGC
CCTGACTTGCTGGTTTATCCTGAAACTCAAATGGACTTAATGATACAGGATTTAATTATCAAGATTGACATGTTCATGGACTTTG
TTAAGTAGAGGTTTTCAGCATTACAGTTTATGAAATAACCATATTTAACTCAAAGATTTTTTTATAAAGCTCATAATCTGAAAAAA
TGCTTATGGTATTACTAAAACATTTAATAGCTCTGGCATCAAAATATTCTACATACTGGTGATCTTTTAGTAAATAGTAAAGGTTA
GGGTCAAGATATTCAATAATTTTTTTCTAGCAGAATCCCACAACTGAATATTGTCAAGCAGTTTAAACTGCATTCGTGTTTGTTAA
AACTTTAAAAGGGAAACTTAAAACTAAAGTATGTTGTTTTTCTGATTTTAATATTGTGCTTTCTAGAGATGAATCCTTTTACTGTT

TGAGGCACTATAGGAATTATGTATTTAAATGTATGTATTTAAATTTGAAGCAATGTACTTTTTTGAGTTTATAAACTTGGGAAGAC
AGGAAATAAAAAGATTTGTTTCCTGTAGAATTTTACATTCATGATTTAATTGAATTGCTAAAATGGAAAGAACATGTACGATTAAT
GGAGACTTGGAATCTGAGATTATTCATCCATATTGATACACCTGCAATTCCTAAATGCCCTCCCCTGCTTGTTTAGATAAAATGTC
TTCCTGGGCTGCAGCCTACTGGACAAACTTGAACACAAAGGAACACACGTTATATGTACATTTAATACTGGAACCGAAAAGCTGCT
TGCAGTGAAAAGCAAACCACTTCTAAACTCTTTGAGACTTTTTAAAGAAGGTAGTATAATCCTTTTAGGGGTTGGTGGTGATGTGA
AAATCTATTATCTTTTTGCTGAAATTTCATTCTTATGTTAGGCATTGGCACTCACTTGTGCTGAGTAAATGCCCGTGTTTTTCAAA
CCCAGATAGTAAATACTGGGGGTATACATACAAAAATAGTCCTTCCCCTTTGTAAGCATTGCTTTGATCTTTGCACTTCCTTTTTA
CTCTACCTCTTAAACAAGATCTGTGTTGATTGAGTTGATTAAAGCACAATTAATCTGAAATAGGCAGAATTTTAGATTTAGTGATT
ATATTCCTTACATTTCTGTTGTTACTGTTCCTGGAAACAAATTGAAGTAGTTTGAGAAACTAATATTTATGCAGGTTGTTTTAACT
ACACTTTTAGACTTGCAGATAATTGATTAAGTAAGTTACTACCTTGACTTTCAGGGATTGCTCATTGTGGTAGATACGAGGTCTAG
ATAGGGAATTGGCAGCTACATGAATGTTGGACTATCATTCCATCAGCAAGACCTTATTTTTACCTAATTTATGAGAGGTATTTCTC
TGTTCAGAAGAGGTGAAAGTTCTGGCTTCTGGGGGGAAGTGGTTACTTCATAACCTTCAATTGGTTTGAACTTGGGAGAAGTAAGA
AAAGTAGTCGATATTTTCAAACAGTAAAATAATTGTATCTGAGTTGCTGTATGGATTTTTGATGAAGTACTCAAAATGTCATCTTT
TGCATCTTCGGGTCTTAGTAGATGTGCTATTCTGAGTATAGAATTGACTGCAGTAGGAAGTGTATCTGGAAATGCACAGTACTGTT
GTATAACCAATAGAGTAAATTCTCAGATTATGCTCTTCTGGTCCACAGATTGATCAGAAATCACATTAACAGGAATGGATGTCTAT
GCAAAATTTCTCATTAATTTTCTTACTTTTAGTAGGAAGTTTGTTTTATTCGGTTGGTCTGTTGGTTTTCTGTTTACAAGGTGAAT
TGTCTACAGTAGTGGGATTGTTATATTAGAAGAGAAGGAGAAGGAAAAATAGAAGTTAGTGCTAGAGGATATGTTAAGATGTTCTG
CTCCTACATCTACATTTGTGTCTAATCAGCATTAATTGATGACTACGTCTTGGTAAACAGTAGACCATACTGGAGCAATTTACTCT
GCTTTTTGGGAATTAGTTCTCATTTGCACTCTTGCACCTCCCATAAATACTGAACTCACTTGTAGTGTTCTTAGAGGTCAGCAATA
AACCATCAAGAGCATATGATCTCTCTTTGAATTTTGTCACTACTTTGGAGACAGACAGTTTGGAAGAACTGAAGGTACTCAGCCTC
AGGGAGAAAGGTTTGAACGTTTTGGCATAGTCCTGCAAGTCAGTTTGGCTCCTTGACTGTGAATTTTTTTAATCAAAAAAGATTAA
TATTTGAAACCACATAATTTGGACAGTTAATAGTTCTGGTAACATAACCAAGCCTTTTATCTTTGTAGTATTTCATTAATTAGTAT
CCAGTGCCTCACTTTTAGAAGAAAAAAAGAGAAAAAAGTAGTAACATTCTTTTAATGATGATGAAATTGCTTCATACCTTTGGTCT
CTGCTGTATTAGGGGTTGTTCCTGGGTGCAAATTAATATACTGTCTTTTACATTTCCGGAGGTTGTTGACCTGGCCTTGAAGATCA
GCCAACAGTTTAGAAAATAAGTTGTGCAAATTGCATTGCTACACCTCAAATAATTTAAAGCCTGACAGTTCTGTACAGAATAAGAG
TCACATAAGATGTGATTTGTTAAAGATTATAGAAATCTCAGAAGATAGATTATGGATGAAAATATTTGTTTAATTTAGAGTACTAA
TAATAAAAATAGTGCATTTTACATTTTAAAAAGAAAACAATTTTTATTGTGTCATATTTTCTGTAGTAACAAACAGAATTAATTAT
CAGTAATACAAATTTCATCTGCTTTAATTTATTGCCTGTTTTTGAAATTAGAATAATGATATATTTGGTTTAATTATCCATTACAA
ATTGCCTTATTATTTTTCATTCATCTTTCACCATTAATCCAGAAACCTTAAAATAGCTTTTATTTTGACCCATGCCTTCAGTAGAT
ATCAACAGACTTGAGTTCATCTATGGATCCTATTAGTTTAAACTGTGACAAATTTTGACTGCTTAAGCTTTAGAAAAGAATTTATT
CTTTAAAATAGCCAAAATTATAAAAAATGAAATAAAGAAAAAGCTGTTTGAGAGAATCAAATACTCTAGTTTAACTAAAAATAAAT
AATATCTAATCAATACTGACGAAAGAAATATCTGATGCTTAGCATTGTACTTGTTGGTACATAGTAAGCAATCATTAACTGTTAAA
TGAATGAATGATATGAAGTTGTTGCAAAATTTTTTTTAAATGGACAGCCATCTTCTATAAGGAACAAAAATTACATTATTTTGCT
TTGCCTTCTGGAAAGAAAGGAGCACAAAATAGTCCTTGTGTCTTAGATATTATGGAAATCTTATCTAAAGACCACTTGAAGTTTAC
TAGAACAAATAATTTATTAAAAAGTATCTGGCGCCTTCCAAAAGGGGAACATTTCATTCTGAAAGAGGCATTTATGAACTAGAAAA
AAAATGCCTTTTAGTTTCAAAGTTTTAGAGGCACCAGAGCAAGATTATAAAGTAATTGGAATGATAAAAGGTGCTTGTAAAATTGA
AAAATGTAATTTTTTGGTGAAGTTAGAATTTCTGTACACTCCATTGAAACTGTAATATCAAATACTAATACCTCCAAAAAAGAAAC
AATGCTACAAGCACTGCTTGGTAATCTAAGAATTGTTAACACTTATCTAAATTAATCAACCAAAAAGTTAAAACAAAATATGTTAT
TTTGTTATGTTACCTCTAGAATTAAACACCTATCACCCATAAATCTGTGACTGTTAAAAGTTACTAGATGGCAAACCTCAGGAACA
AGCAGGCAGTTAGCAGGGTTTACTGTACTTTCCACTTTCATACTTTAATGATTTTTCCAACACTATAGAAGCTGGGGCCTCATCCT
AGCACAGTTTGTTCATTATTTTTCAGTTGTAGCATTTTTTAAAAGTTGGCCTTGAATTTCTGAATTGAACCATTTATTTTTTTGTG
GACACAGTTTTTTAAATGAAATATACCATTTAGACTAGAAAAGAGCAAAATGCCAATACTCCCTGTCCTAGTGTCATTGTGCCCCC
TGGGAACCCTAATGCAGTCCTGTGGGAAAACAACCATTTTATTGAGCGCTGCTCACTTCAGTTCATTCTGCTTTGCTGCCACTTAG
TTCTGGTTCTGACCCTGATCTCCCCTCACATCACTCACAACAAACATTCAAGTTTAAAAATTAAGTTAAAAGTTAGTTACATATAT
ATGTAACTAATGTGTATATTAAATATACTGATATATATTAGTTACATATACAGATATGGCAACAACAAAAAAGGTTATATATATAC
ATATTTGTAACTAATATGTATATTAAATATAATGATATATGTATAAATACATATTTGTAACTAATATAATTATATTTAATATATAT
TAGTTACAAATATGTATATATAATATTTTTTGTTGTTGCCAGCAAAATGAGAAAGAAATTAAATGTTAACATCTATAACAATTGCA
CTGTAAAGCCGTCATTGTTGAAAAGTACTCCGAAGATTAATAGCGGCAGTGTTTGTTAAAATAATAAAATAATCCAGGCACTCTGC
CTGTGACTTCCCCATGAGATGGCTTCCAGAAAAAGATAAATTTTTGTGTGTTCCCTTACGAGTTCAGTCTGCAGCTTCTGGGCATG
AAGAGAAGCATAAACAAAACAAACAAGGCTCTAACTTTGTTATAATCCTTACGCTACTAGTAGAAATGTATGGACAATTCATGCAT
TTCATATTGGAAAATAAAGCTATTAGATTTTTCTTTGAGATGAACCTTTTTTTTGTAATTTATTTTTTTATTTTTTTATTTTTTTTCA
GTAGAGATGGGATTTTGCTCTGTTGGCCAGGCTGGTCTCAAACTCCTGACCTCAAGTGATCCACCCGCCTCAGCCTCCCAAAGTGC
CGGGATTACAGGCGTGAGCCACCTAGCTTGGCCAAGGTGAACCTTTTTGATAAATGAGGCTCACTAATATTTCTAGGATTTACTTT
TCACTAACTTGAACTAAAGAAAGTATAAGACCTGCATAAAGTTGAGCATACCTCTATAATGTCCCATTTTAAAAAGCAATTTGTTT
TTAATGACTTTTATTTGTTAAAAGCAGTTATTTCAAAAAATTATTTTTATTATTTATCATGTCATTTGGACTGAAAATACTATACC
ATATTTCAGTGTTAGGGCACACCTATCCCTCTTTATCCCAGACGTCAATTCTACCATATAACAAGTATGTAAAAAAGAAAACATAA
AAGTGATGACAGACTCCCTAATCAACCTATTTATTGGTTCTAAGCCCAAGCCTGCAGTTCATTTAAATAGTGAAAATAGATTACTT
AATTGTCTTAGAAAATATGTCTTCCATGACAAGAATTTTAGGAATGAGAAAATAACTATTTTTGGATGTTTTAACACAAATTCCCA
AATCATGTTTTATTGATATTTGAAGAACCTCTTTGTCTACCAGTTCTTAAGTATAGGCTTATTTTGTAAGGCTTAAACATGTATTG
GTGTTTGACTGTTAAGACTTGATCTATAACCTTCAGTGTCTTTTTCTTTTAAATGGGGTATGGACTTGGTAATGAGGCCAGAGGAG
AATACCTTACAATTTATAGCATCTGCATATAATACAAGAACCTTCATGATTTATAAAATTAAAATGATGATGATGTGGAATGATGG
AAGAAGCATGTGTGGGTGGCTATTTATGTAGAGGAAATTAGAAGTTGAAAGCAATGTTAAGAATTAAAATTAAATTACACTTAATT
TTTTGCTCAGTTGGATAGTCAGGAAAGTACTTAAATCAGCAATTGATTTACGTAAAATCAAAACTGAAAACTGAATATACAATCTA
GGAAATAGGAAAAAAATTTTTAGTGTGTTCATACTGCTAATTTCAAACCAAGCAAAAAACCTGGGGAACTACTTTTCAGAGGAGAT
GGCTTAAAATGAACCAAAAATATAGCCATTTCTCTACCCTATTCATTTAATGCATTCATTTATTATACTGCTAATACATGATAAGG
TGAATAGGTTAAGCATCGTTTGTATATGTGTATGTGATTTTAAACCTGAGTCTTTTGAAAGATTGGTCAAAGCAAAGGATAATGAA
AGCATAAAGATTTAGGGGGAAGAGTCCTCTATAAAACTAAGACTTAAACTTTGTTATCAACTTGAATGTGAAGATAGAATAATCTA
GAAAGTGAATGAGAATTATAAATAAAAGGACAAATCATATATCTCATGGTTGAGAAATACAATGAGATAAATTGTATATCTCATCT
CATGGAGGGTCATAATGAAAGAAGTAAGAGAGATTGCCCTTGGATAGAACCTTTCGGCAGACATTTCATAATGGATTATCATATTT
TTGCTAAACTGGTAAGAATGGACTTTTCACCACATTATGAAAAAACACTTTAGAAGTCAGTTGCGATCAAGCAAATATAAGTACCT
GGGAAATTAAAATCAAAAGCACAGCAGTCCAACAATAGTCAAATGCATTTACTCACTCTCATACCATATCATAGCGTTAATTTGAG
ATAATGTCTTTATGGTGTCTCTTTATATATTTACTCGATTTCCCATTTAAGACATTTAACCAAATTTAAAAAGCAATCCTTAGAAA
CTTTAAAAATATTTCCTATTTTAAACCATTACATAATCATTTACCCTGCAGTTACAATTCTTTTTTTGTGATGGGTGGGGAAGTAT
CCCATTTCTCTTAGAAAGTCTTCTGTAATAGCCAAATAGGAGTCAATCCAAGTTGCTTAGAGAGGTAGTTCAATCCTGTGTTGAAA
TAGATGACTTTTGGAGTAATTGTGCTATATTTACTGAGTATCTGTTGAACTGTAGGAGTCCAGTCATTCAAGGCAAGCTACAGAAA
TGATTTTATTGCTGAGGCACTTAAGTATCTTCACATCTATATGCCAATATTAAATTTCTCATCATCCTTGATCCCCACCCCTTTGC

ACAGATTGGTATCCCCAGCACATTAGGGAATGCGGTTGTTAGCCTGTTAGGGTCCATTCAGTAGATTTCTCATGCCAGAAATCTAG
AAGCCCTTCTTATACATTCAGCTTTCTTCTTCAGTCAGTCACCAAGTTCTATCAAGGCAGCCTCCTCATCTCCAGAATCCATCCCT
TCCTCGTTGAATTTCTAGATTTTATTTTTGCATTGTCTCCTGCTTCTAACCCATCGTTCATTTCGCATAATGGTCAGCTTTCTAAA
GTGTAAATCGGATCATGTCTCTGCAGTGAATCCCAGTTGTCTTCACAAGCTGATTCTCGCACACCTCACCCCCTTACTCCCTCCGG
TGTTCCCCAACCATCCCCTAATCTCAAATCCTCCACACCGACTTTCAGTTTCCTATCCACAGTTTCTGACTTTGAATGCCTTTGCT
TATGCCCTCCCTGTATGAGAAGTGCCTTTTCTTTCCTAGCCATCCTGCTAACTTCTAAACCCTTAGGCCTGGTTTGAGCATCACCA
TTTCTCTCACTTGCCTTTCTGTTTTCTCCCTCCCCTTTCCATAAAGAGTTATGTGCCCCCATTCTGTGTTCTCCTGAATTCCTGTG
GGTCCTAGAAAAGAAACATCTTGAGGGCAAGGAGTTCATCTTATTTACTTTTTATTTGCACAGCACCTGGTGTGTTTGCTGAATAA
GTAAATACCGAACAAGATATCAAGAGACAGTGCTGCCAATTAACTGTGTGGCTCTGAGCAAGTTAATTAACTTCTCTGAGTCTTAT
TTTTGTTCTGTAAAAAAAATGAGGTTTGACAAAATGAGCATCTCTCTCTAAAATTATGTGTTTCTTTGATGTAATTGTATTAATTG
GAAGACTTCAGATTTTCTTGCCATATCAAGATAGGTCAACTTAAGAAAAATGCATGTGAGTAAGTACCAAGCCAATTCTTGATTAT
CAGTATGATAATATGAGGAACTGCCCATTTTGTCGATAATAAGTTATAATTTTTGGTCTCAGTGTAGGTTCTTTCTCAAATCCAGA
TGTTTCTGGTGTTGCATTTTTTCATTATTATTCAGTCTCTCGCATTCATATTTCTCCAAAGAAATTTATGGAAAACTTGAGACAAA
AATTGAATGTAAAGTTTAATATTTAAATGCTAGATTGATTCAAGGTCTCACTAATTATAACTATATTTGGAGGGAGAAGAAGGGGA
GGTGGTGGTAGTATGAGCTGAATCCTCCTCTGTTCCTTCCTAGAAGGAACACAACGTCCTACATCAAGAAAAAAGACAGAAGCATA
TCTTGGAGATATGAAAAGAACCACCAGGTAAACTAAAAAGAAAAAGAGTGTTAAAAAGTCAGCCTTTAAAAAGCAGGATTGGAGAT
GAGGAGGGGTGGAGTAGGGGAAGCATTGCTTGTTTATAGAAGCTCTTCCATCTTCGTATAGTGTGATTTTTATTTTTTTATTATTT
TTTCCTTCCTTTTTTTTGACCTCCAGCTTTAGTATAGTACAGTTGACAGAAATTATATATGTTTATGGTACACAAGATGATGTTTT
GATATACATATGCATTGTGAAATGATTAAATCAAGCTGATTAACATAAGTAGGGTTTTATTTCTTTAACAGTGATTTATTTGAATA
TATGTTGAGTCCTTTCTATGTGCCAGACACAGTTCTAAGGACAAGGCACACAGTGCCAAACAAGACAGAAATCCAGAAATTCATGG
AGCTTAAATCATGTAGTCTTTTCAGTTTTTAAAATTTTCTTGCTGGGCGTAGTGGCTCATATCTGTAATCCCAGTACTTCAGGAGG
CCGAGAAGGGAGGATTGCTTAAACCCAGGAATTTGAGACCAGCATGGACAAAATAGCGAGACCCCAGTCTCTACAAAAAATTTAAA
AATTAGCTGGGCATGGTGGCACATGCCTGTGGTCCCAGCTCTTCAGGAGGCCGAGGTAGGAGGTTCACTTGAGCCTGGGAGGTTGA
GGCTTCAGTGAGCAGCGATTGTGCCACTGCAGTCCAGTCTAGGCAACAAAGCAAGACTGTCTCAAAAAAGGAAAAAAAAAAATAGAA
AGAGAAACTTTATTTTTACATTAGCCTTTTTTAAGTTGAAGAATCCCATAGCTAAAAAAGTACACAAATATTAATGCTGCAACTTT
AGTGATTTATCTCAAGGTGAACACGTTTAACCTAGTGGGTGGTCAAGAAATAGAACACTGGCAGCACTGCAAAAGCATCCTATCTG
CCCTTTCTTAGTTAGGACCCCTTCCCTCCACTGTCATTTAAAAGATTTCACAAACAACTTTTGGATTATATGTATGTCAATTCTGT
GACTCATATATATCCACATAGACAATGGAGCGTTGACTGAATGAATTCATTATTCCAAGGTGCTAATAATGAGCTTCACACCAGCG
GGAACTCTGCTGTGTCAGTTGGGCTCTAAGCTCAGTTCATTTCAGATATTCCTCACTAAACAAAATACTGTTTTTCCTCAGTTGTT
AGTAAAAAATAGAAAACAGATTACATTCTCATTTACTTATTACTTATGAATGTAAAAGTAAATATTTTGCTTAATACCAAGTAGCC
AGGGTCACTGACAGAGACATGGAGAAAGAAGTATTCACATCTGATGTTATCAGACATAGAAGAGAGAGGTATAGTTCACCTATTT
CTCCCCACTCAGTTTATACATTGACATCTGTATGTGTGTTGTTTTGTTTTGTTTTGTTTGTTTTGTTTTGAGATGTGGTGTCGCTCT
GTCACACAGGCTGGAATGCAGTGGCGCAATCATGGCTCACTGCAGCCTCGACTTCCTGGTCCCAAGCTATCCTCCCACCTCAGCCA
CCCAAGTAGGTGGGACTACAGGCATGCACCACCACACCTGGCAAAATTTTAATTTTATTTTACTTTTTAAAATTTTTGTAGAGATG
GGGTCTCCCTATGTTGCCAAGGCTTGTCCCCAAATACCTGGACCTCCCAAAGTGCTGGGATTATAGGTGTGAGCCACCACACCCAG
CCATGTATATGTTAAAGCGTTTATCTGTATGAGTGGTCAGTGTAGAAAGGGCTTCATGGAGCCTGATTTTGATGGCTTGCAAATTA
TTATATTTGTGTATGTGTAATCGGAAGGCCTTCGCCGTGATTATTTCTTCAAAGGAACCCTTTGTCCCTCCAGGGTGATTGTACCC
ATGTTCATAGGACCAAACATTGCTGGTTCACATAATGACTCTTTTTCTTTTAAAACATAAAGGACATATTTTTATGGATTTTTGAG
GATTAATAGAATAAAAATCTAAAGTCATATTCAGATTCAAGAGAACTGGATCAGTTTCTCTGTATTTGAACAAAAGAATACTAATC
TCTAATTCATAATGCATGAGGTCTACTTCTTCCTCAGTCTGAAAATATGAATATTCCAAAATGGTACTCAAGGCAGAGGCTATATA
TCAAAATTAAAATTCTGTGATACTATGTTTTTAGGGAAGAAGAAAACTGTAGCATTAGAAATGTAGACGCAAAAGGTCAGAACAAA
GTCCAGTATGTCAAGTCCCTTTATAATGCCATCTGAATGAAAGACCTCACACCATGAACAGAGGTATTGAAGCAGTTTTTATGTGG
AAATGGTAGGCAGCAATATCTCAGGAGTGACTTTAATTATAACATTTAAGGTTTTATCTAACTTATTATAATCCTTAGAGAACTTC
TCTAGCTCTACAAGTTATTCTTGGCAGAATTTGTCCTAACATAGTATTAATAAAAATAAATTTTACTTGAGTATGATATAAATTAC
TCTTAATTACAATATAATTTTAAAATGCCCAAAACATCTTTTGATCTTATGAATTTCAGTCAGCTATGATTAAGAAGACAACCATA
GGCAAAAATTTCTTTCTTATGGAACACGTTGGAATGCATTCTAATCACTTCTCTAAGAAAATCCCTGTCCCCCATGATTATAGTGA
ATATGATACAGCAATAATTTAGATGCTTCTTTCAGGGGGGTGAGGTTATGTATTCTACAGTCTATTGATTCTAAGAGGCACTTTTT
TCTCCCCCAATTTAACAGTTTTGTTTAATTATATACATTTCAGGATTTTTTGGGGATATTTTTCTATCACTGGTTTCTGATTTAAT
TCCACTGTATTTAGATAACATATTCCGCATGCTTTCAATTCTTTGAAATTTATTGAGACTTACTTTATGGCTCGGGACGTGGTCTA
TCTTCGTGAATATTCCGTGTGTATTTAAAAATAATGTGGACTCTGGGCCGGGCGCGGTGGCTCACGGCTGTAATCCTAGCACTTTG
GGAGTCCGAGGCAGGCAAATCATGAGGTCAGGAGATCGAGACCATCCTGGCTCACACGGTGAAACCCCATCTCTACTAAAAAATA
CAAAAACTTAGCCAGGCGTTGTGGTGGGCGCCTGTAGTCCCAGCTACTCAGGAGGCTGAGGCAGGAGAACAGCGTGAACCCAGGAG
GCAGAGCTTGCAGTGAGCCGAGATGGCGCCACTGCACTCCAGCCTGGGTGACAGAGCGAGACTCCATCTCAAAAAAAAAAAAGAAAA
TAATAATAATAATAATGTGGACTCTAAAATCATTCAGTGGGGTGTTGTAGACACACTAGGTTCTTTTGATTGACAGTTTTGTTCCT
AAGCCTTCTATATCTTTCCTACCATGGTTTGAATGTGTCCCCACCAAATTCAGGTGTTGAAACAGGATGACCAATGTGATGGTAGG
AAAAGGTAGAGCCTGTAAGAAGTGATGGGATTAGGTGCCCTTTTAAAAGGGCTTGCTGGAGGAAATTTGTCTCCTCTTGCCTTTCC
GCCCTCTGCCAGTTGCGGACACAGCATTCCTCCCCTCTAGAGGATGCATCCCTCACCAGATACCAAATGCTGGTGCCTTGATCTTG
GGCTTCCCGTTGCCAGAACTGTGAGAAATAAACCTGGGCTCTTTATAAATTACCCAGTCTCAGCTATTCTGTTATAGCAGCATTGA
CAGACTAAGACATTTCCTGACACTCTGTTGATTTGCTTTGATCAATTACCGAGAGAGGAATGTTAAAAATCTTAACTGTAATTGTG
GATTTGTCATTTTTTCCATTAGTTCTGCCACGTTTTTGCTTCATGTATTTTGAAGCTTTGTTAATAGGTGCGTACACAATAAGATC
ATTGTTTTTCTTTGATGAGTTGGCCCTTTGATTCTTATATGCAATGTTCCTCTTTGTCCCTGGTAATGTTTATTCTCTTGAAGTCT
ACTTTATCTGATATTTTAGAATGTAGCCTGCTTTTCTTATATTTAGCATTTGCATGATATAACTAGCCCAGTGTACCATCTTCATA
CATTTAAACTGTGTCTTTAATATTAAAGTACAATTCTTATGAAAAGCGTATACTTTAATCTTGTTTTTTAAAAATCCAGTCTGATC
ATCTCTGTCTTCTAGATGAGTTCAGTCCAGTTCCATGTAATATTATTATTATTATTATTTTTTTTTTTTTTTTTTTTTTTGAGACGGA
GTCTCGCTCTGTCGCCCAGGCTGGAGTGCAGTGGCGGGATCTCGGCTCACTGCAAGCTCCGCCTCCCGGGTTCACGCCATTCTCCT
GCCTCAGCCTCCCAAGTAGCTGGGACTACAGGCGCCCGCCACTACGCCCGGCTAATTTTTTGTATTTTTAGTAGAGACGGGGTTTC
ACCGTTTTAGCCGGGATGGTCTCGATCTCCTGACCTCGTGATCCGCCCGCCTCGGCCTCCCAAAGTGCTGGGATTACAGGCGTGAG
CCACCGCGCCCGGCCCCATGTAATATTATTACTGGTATGTTGGGTTTAAGTCTCTCATCTTGGTAATTAATTATCTATTTGTGCTA
TCTCTTCTTTATTCTTTTTTTTCTCCTTTCCTGCCATCCTTTGAATAAATCAAGTTTAGTGTTTTAAATTCCATTTTATCTCCTTC
ATTGGTTACTTTCTAAAGTGGTTACTTTACCACTTTAGAAAAGTATTTGTTTATTACAATATGCATCTTTGAGTCTCTAATTAATG
TGAGAACTTGACAATGTTATACTTCTGTTTGTCCTTCTGTGCTGTTATTGTTATACATTTTACTTCTTTATATGTTATAAACTCCA
AAATATATTGTCTTGTTTTGCTGTGAACAGTCAATCGTCTGTTTAGGAAATTAAGAAAGAAGAAATAAAAGTCTTCTGCTTACTCA
CATATTTATCATTATTGATGCTTTTTATTCTTTCATACAGGTCCAAGTGTCTATCATTTCCCTTCAGCATGAAGAACTTCTTTTTA
GCATGTCTTTTAGCAGGTCTGCTGGTGATGAATACTCTTAGCTCTTGTTTACTTGAAAATGTCAATTTCACCTTTATTTTTAAAAG
ATTTATTTGCTGAATATTCTGGACCAATCTTTTTTTTTCTTTTATCACTGTAAAGGTGCCTTTACATTGCCTTCTAGCTTGTACTG
TATCTAATGATCATCAGAAACATCATGCTTATCATGCTTATCAATTGTTCCCCCATATATAATATGTCCTTTCTTGTCCTTTGCTA

TTTTGGGATTTTTTTTTTCTCTAGCATTAGTTTTTAACAGTTTGGCTGTAATATGCCTAACCATGGTTTTATTTGTCTTTATCCAG
CAGAGAGATCTTTGAGATTCTTGGATCTGTGGGTTGATTTTTTAAAAATCAAATTTATAAAAATTCTCAACTGTTATTTTTTTGAA
TATTTTTTCTGTCTTTTTTCTGTTTCTGGGATTCTATGTTACAAATACGTAAGACTGCTTAATATTGTCCTGTAGGTCACTGAAGC
TGTGTTCACTTCTTTCTGCCTTTTTTTCTCTGTAATTCAGTTTGGGTAGTTTTTATTGACCTTTCTTCTAGTTTATTGGTACTTTC
TTCTATAATGTTAAATATGCTAAGTCCATCTAGTGAAATTTCATTTCAGTTCTAAAATTTCCATTCTTTTTTTTAAAAGAAAGGTA
TGCATTTCTTTGCTGATATTGCCTATCTGTTCTCTCATTATGTTCATCTTTTCCTTTACATTATTGACCATATCCTATTAGCTATT
TTTGTCATTTGTGTCATTTTTTTTCTCAGTCTTATTTTCCTATTTCTTTGCAGGCTTCATTATGTTTTATTGTACATTGTGGAGAG
TTTGGGTTTTTTGTCTTCCTTTAAAACGTGTTGAGCTTTGTTGTAACAGGCAGTGAATTTACTGGTGGATCACCTAGTTTTATTAA
GATAGGTTTAGAGTAGCCCTTATTTTAGGATGTTTCTAACTCCCATCGTGTGGTCTTTCTGAAATCACAACTGAATGCTCACAATG
TTCAGTGATGTCTTTCCACCCTGGATTATTACAAATTCAGTGTCTCTCAGCACTATATGATGTCTGGAATTTCCATTTAGCTCTTA
GATGCCCAGAAACTGTTTTCTATTAGGTTTTGCAGGGTCTTGTCCTACACTATCACAGCTTGGTATTTGGCCAGTGACCAGAGATG
ACCCCTATGTAGATTTCTGAGGCTCTTGCTTTTTGTGTAAAGCCTTTCTTTGTGATATTCTGCCCATTGGTGTTCTCTCTTTGGGC
TCTCCCTGTGTCATGATTTGGAAAGTGTCCTCAGTCAAAAAGTGAGGGCGAATTTAGAATTTACCTAAATCACCTTATAATATATA
GCCCTATGAAGTCCAATGTCTTAAATTACATCTTTAGGTATTTTGTATGGTTTCACAGTTGAGTATAGCAAGGAGGGAGGTTCAGA
TAACTGTTAATCTGTGATAACTAAAACCAGAATTTGACATTCTGACAAACTTGAAATTGCAGTGCTTCTTAAAATTAATACAGTAT
TACTTAAAAAACAAAAACAAAAAAACCAGTGTTTTCCAATTTGCCAATACAAAAAAGCAAAACTCCCATCTCTACTTTTTAGGAAC
TTCTATCCATTTGAGAAGATGATAAATATATAGGTAAAGTTAGATTGCAGTAAAACTATGCTGTCTAGTGTTAAATCACCAGTATA
TATAAGTTTTATAGATATTATGTGACTACAGAGCTATAGGTTATAGGAAAAATATAGGATTTCTTGGCAGAAATTGGATTTAACTA
GAGCCTTGAAGAGTGGATGCTATTGAGGTAGAAAAGATGGCATTTCAGGATGCAGGGCATATTGTGAACAAATGTACAAGATGGAA
ATGAGCAAGTCATTCTGAATGATTAATCTGTCTATCATAGAGGGTTTTGATTAGGACATAGTAGTAGTTGATGCTTATAATATGAT
TTAATATAATAATGTAATGTGCTGAGTGGTTGAAGTCTTTGGACTTTATCTTATAAGTAATATGGCAATAATGTGAAAGATTCTTG
AAGAGGAAGAAACCCTGCAGTGAGGGAGAACAGTACAGAGCCTATTACTGTCATTCAGAGGTGAAGCAATAAAGGTCTCTCCTAGG
ATGTTGGAAAGAAGGGGCAGATCCAAGAAACAACATGAAGAAAATAATCAGCAGAAATTGGTTATTGACTAGATATGTGTGAAAAT
GATATATTGGCAGAAATTGCAAAGTCAAAAATGGGGCTCATTGCAGCAGGAATGATGAAATTGCTTTTAGATAGAGAGGTGAAGGG
ATTTAAAACATTAACTTGTTTTAAAGGTATCACTGCTTAAAGCTAACAGTGATTTATTAATATTTGCACTCTGAAAGTTTTAACAG
TTTTAATTTTATGTATACACCACAGTGTTTTGGCTACTATACTTGCCTCTTTTGTTCAGTTGGGAATTGAGTAAACTAATAGAGTA
AAAAAATGAGTACAAAGTGGGCTTTGACTAGAACTCTAACAAAGTGTGATCACTGAATTACCAAGATAATTAACCCATACCCCACA
TATACTCCATTTCCCATGACACATATTATATTGCATTGTTTTTGCCTATTTGCCTTCCTACTTCCTAAACTGTTGAATTAGTTTGC
TTTTGTTGCATTAAAAAAAAGGAGAATCACCACAAAGCAAATTAAAAGATACTGATTACTTCTTCCAATTCTGTGGGCTGCCTAGT
TCATCTGGCCTGGCCTGCTTTGGCTAATCTCTGAGGTCTTTTGGCTACTTGACTGGTACTGTTTGTAGCTACTCAGCTGGTGCCCT
ACTGCTGTTTGTCTGAAGTTGTTTTGGCTACTCAGCTGGTGCTAGGGAGCCTCAGCTAGGACTGCCTGTCTGTGCTCCACATGGTC
TTTGATTTTCCAGTGGCTGGTGCAGGCTTGTTTGCATGGTAACAATGTTCCAAGAAAGCAAGAATGATCATTGCAAGGCCTCTTAT
AAGCTGGGTTTAGAACTTGTACTTCTACCTCTTCCTATTGGTCAGAGTATGTCCGTAGGTCTGCCAAGACTCAAAGCACAGAGAAA
TAGATGTTTCCTCTTGTTGAGTGGCATATAAAAAAAAGTGTGGCCATTGATTTCAATCTGCCACAACCGTAAGCTCTGTGAGTACA
GGAACTGCATCACTCTTTTGCACTTTGGTGTTGTTGGCATAGTAGGCATAAATATTTTTAGGTGAATGACTAAATTAATGAAGGAA
GGAAAACATTCAGGTAGTATTTCAATATACTGTAGTTTTAAATTATATATATTATATACATTTGACATAGTTTAATATATAAGTAT
ATACACATATAGTTTTCATATATAAGTATATTATATATTATATAAAATATTTATATGTTATATATAAAGTATATATATAAAATATA
TAGTTTTAATATGTAAGTATATGTATATAATTATATATAATATTTATAAGTATATATATACTTTTATCATACCATCAGTGAGTATAGAC
ATGGTTCAGGGAGAATCTCTTTATTCTCCTTCTATCTTTTGAGATAGACCAAAGAAAATTTTTTTTAAAAAAAGAACTATCACTT
GAGTTAACATTGTTTGGTATTTTTATCTTCATAGGTGATTGCCTTGTGAATAACTCATTTTGTGTATTGTCAGAATCCTATTCTGC
TTTTCAGGCTGTGCTATGGGTAGACTAGCTCTAACCAGAACACTTGGGAGACCTGAAGCAAAGATACTATATTAAAATAAGTAATG
TTATATGCCAAAGAGGAAAACCACTTCTGCCTCCTAAACAACTGAAAGAACCAGAGACTGTGGGGTCAGATTAGCTAGCATTACTA
TATGAACCTGAGCAAATGACTTACTCTCTTTCAGCTTCAGTGTCTTATCTAGGACATTGGTTCTCAGGATTGGTGTGAGAATTAAG
AGAAATATGTGAAGTTCTAGCTACAGTTCTTTACCCATGAGGTAGTCAGTAAAGTTTACTTCTTTCTCCATGTGAACCAAAGCACT
GTCATGATGTGATGATGTTCATTAGGGTTTCTCATGCTCAGCGTTATTGATATTTAGGGTTGCATAATGCTTTATTGTGAGGGGCC
ATTCTGTGCAGTATAGGATGTGTAGCAGCATCTGTAGCCTCTATACACTAGATGCCAGTAGGACCACCTATCCAGCTGTGACAGGC
AAAAATGTCTTCAAACGTTGCCAAATGACCCCTGGGGGCAAAATGCAAACAGTTTATCTAGCAGTGACACATAGCCAGCCACCTTA
GAAAGGGAATTCTCAGCGTACCCATAAGTGTAGAATGGGAAACAAAATACTTGAGTCTTCTCAGAATGTCTAAACCTTTTGCTGTC
TATATTCCTCTTTTTATAATTTACCACAAACATATATTGCTTATTATGCCATCTAATAAAATGGCTAACATTTGCTGTGTATTATA
CTCGGTGTTAATAAGCTTTGTATGAACTCACTAGCTGAATGTTGTATATGTATCTGCCATACCTGTTGGATTGACATTCCACTGTA
AACAGGTACCTTATCTTTACCAGCTCCAAGCACAATGTATTTGGTATATCAGGTAGCTATTCTACATGTTAATTGATCACATTGCA
CCCAAATGAAACATGGATGTGGAAGTGGAAGGAGGAGCTTTAAAACATGGAAAATTAGTTTATACAAATCTAAATGTTGTAGTGCA
CCAAATTGATTCCTTTTTAAAAACTTTCAGAAATCAGCTTTTAAAAAAAAAAAGTAACTAAGCCATGATCAACTTTTCTTTCATCA
CCACTAAGCCAATATCAGAAAGTTGTCAATCAACAGCTCTTTTAAAGTTGAAATTTTTCTATTGTATTTCTTATGAAGATTTCAGT
AGTGCTTAGAAGTTTTTGGCTCATTTATCATGTTTTATTCACTGTATGTCATTAATTGTTAAAGATGTCAGAGTCAAACCTTAAAT
TCAGGAGAGATTTAAATTTTTCCTTTTTCCTATTTAAATATATGTTTCTAATTACATAATTCAGTTAGAAAATTACAATATAGAAA
CATATAACTTAGTAAAAGTTTCCAGTTACTGTATACAGATTATATTGTATACTGTATTATTTTTAACAGTTGCATATTTAACTTAT
AAATGTGGATAATTATAATTAAACAATCTGTTATCAAAGGACATACAGATTATTTCCAGTTCCTTGCTGCAGTGAATATCCTTGTA
CTTATTTTTTTACACACTTGCTCAGATAATTTTTAAGTACAAATTCCTGAGGTAGAATTGCAGAATCAAAAGATACTCTCATTTGA
GTGTTTCACATTTTATGCAAAGTATTTTGTTTCCTCCAGAAAATTCCTCTGTGGAAGAGGCTTCCTCACATTCTTTATCAGTTTAT
TAGCCAGCAGAGAAATGATGAAAAGCCACCCCTTTTCTTGTAGTTTCATCCTCATTGCTATCCAAGATGAGGTATTAAAATCACAC
CTAAGTGGTGTCTGTTGTGGCTGAATATAATCTTGAAAGTAGAGACACTTCTCTCTGATTCCCAACTGTACTCTCTGGATGGCACC
TAAAATTTTATTTTTTCAAACTGAAAATCATTTTAATGAGTTCGAAAAGGTTTTATTTTTTAAAATGAAATCCTATAGAAAGAATA
CATTTTCTTACAGTGGGTCATGGTCAAAAGTGTGAATATCTCTGATCCTAGAAAGTTTCCCCAGTTACAAGGACAGGCGTGCCCCC
AACTCCATCCTTAATGGCTCAATTCTGTGGCTTTCAAGTTAATCCACTCAGTCCTACAGCCTTCACCAAGAAGCTCATGTGCTACA
CTTTGGTGTTAGTGGGGGCATTGTCACACAGGTTCAGAAAATAACCCATTAGGCATTTTATAGAACTCATCACATGTTCAGAACTA
AGCTAGTTGTAACATTATGTTTTTCATGTGAAAAAACACAAGCAATTCACTGGCTATTTTATTTGTTGCTAGCTAGCTATACTTTC
TATTTCTTTTTCCATGGTGTGAAAATCTTCCTAGAAACCTCACTACTGGGAAAAGAGATGCAGAGATTAGTCCCATCTTCTCCCCT
GGAAATTTACTATCGCCCTTTTGGCTTCAAGGTCCTTCCTCAAAAGCAAAGAATTGAACAAGGGAAAGTGAGGGGAAGGGCTGGGG
GAACAGGGAGGTAGAAGGGGTTTTCCCTGGTCTTTTCTAATCCTTTAGTTCCAGACGCTTAGTTGGTATTTCAACATTTTAGAAAT
AAAACCTGCTTTTATAATTAATGCAAATATTACTTAGTGATTCATCCTTTCCAAATGATTCTTGGTTTCTTTTGACATTTCATTTT
TAAATTGTGAATTAACATTGACATCACCATAGCGTTACTGTTACCTCAGCTCTTTACAGTGAACTGTATTGCAAAGGCTATAAAAG
TTCAAATTTTAACTATCACCCACGTGAGCCTTGGACTGCTGTTAGGCTTTTGAAACTGTGATCCTTTGGCAAGAATTGGTGTATAA
ATCATGGGGAGGATATTGAAGGGGAAGATCATGTTTAATTTGGCAGTTGGGTGCCTGCAATTGACAGATTTTCATTAAGGTTTTAG
CTATAGTAGTGATCATGCATCAGTTCTAATAAAGTAGGTTTCGTTTGAATTTTAAAAATTAGAGTGAGGACTGCTTTATGTTAACT
GTTTACATTCAGAAGTCTGGAAAACCATCCACAGGTTTAAATGTATATAATCAGTATAATTAACACGTCAACAGCCTCACCTCTGT

TTTTGAGGACCATAGGCTAATATTATTTATGAAGGTTCAGGAGATCAGTCTAGATCGACAGAAAGCTTAAGGGCAAAGATGGTTTT
AATATTTTTCTTGGAAGTTTGAGCTTCTGTGGTATTCTAAGGATAGGGATTTTGACATTGATTGTTCTAGCTGGTATTCAGTACTG
TATTTAAGTGTAATGTTTTTACTAGTATTCTTCTTATAACTGAAAACTATGCTAATGATTCTTTACTATAGATATATACTCATAAA
TACGTGTAAAAATCCAAGTTTACCAAAAAATATTAAGACTTAGGGGATATGTTCTTTGAATAGGCATTTATTTGCTAATTAACATA
TTCTAATCTCAACAAGGTTATATTCTTTTCAAAAATCCATTCCATCAAAATCTGTCATTTTTACAGATGAAGAAACAAAGGCCACA
GTGAGTTTAGTTAATATCCAAGATCACACAGCCAGCATGATAGAACTCTTAAATTTCAAGCGACTTACCCTCTTTACTCTATCAGG
TCAAATTTCCCTTTTTTAACTGCTTGTCTTAATTGAGATTTGTTTAATTGTGGCTGTCTGTATATCTTCAATCAATACTTTCTTGT
TTTAGACAAATGTTTCCCTGCAAATCTGCATGAACTTCATGTTCTGAAGGACTCAGAGCTGCCCTTTCCTTTTTGTTAAAGTTGTA
CACTGCTCTGCATCCCCCTAGAATGCTCATCAGCTTTCTCACTCTTCCTCACTCACACAGTGCTCCAGCAGGAGAGGATTTCTTTG
AAAATCAGATTCTTTTTTGGAGGGCTTTGTGCTTATTGGAGTGTTATGTGATATATTCATTACGGTGAGGGCTAAACTCTTTGAAC
AAAGAGACCTGACAGTGCATTTGGGTTAAAGAACAAAGTTTATTTCTTTCTCTCATCACCACCAGCATGAGCAGCCTGGATGGTCA
GAGCAGATCTGTCTCATGGCATTGTTCAGGATCCTCATTCCTTCCCTCTCATTTATCTGCCATCCCCTAGGCTATTAATCATCAT
GTTTTTGGTAGAAGCCAGGTCTTCTCCATGTCTGAGTTTTTATGGAGGAAGCATGTCAAGGGCCTTAAGCCCAGACCTGGAAGTGG
TATGCAAGAGTTCTGACATTGCACTGGTGAGCATTTGGTCAGATGGCCATCTCTAACTCCAACGAAGTGTAGGGTATACACTAGGT
AAGCAGTCACATTTCCAGCTACAATACTAATTCTATGGAAAAGGAGACAACATATTTTGATGGGTAGCTGACATTCTCATCTGCAG
TCCTAAGTACTCATGACTGCTAGATAGACTTTATGAAAAAAAAAAAGAAAAATAACCCCAGGAATTAATATTTACCCTCAGCCTTG
TGTTTCTTTAATTTTCTCATTTTTCATGTCACATATCTAAAATTTGATCTAAATAAATTTAAATAAAATTAACCTTTATTTGAATA
ATAATAAGGACATTTCTCAAGGAAGTAAATCTCAAATGATATGTGATTTTAGATACTATCTTTATCTGACCATGTGGTTCCTTGCT
TCTATCAGGGAGGGCCTCTGGGTAATAGAAGATTGCTAGGGCCTGCAGTATATTAATGTTTAGCTGGAGTTGATTAAATCTGAGAC
CTATAGCCTTGATAATAAGGACTTTTATTTTTGTATTTTTATTGCACTTTTAATATTACAGAGTTTCTGTACCAGCTTGACAACAG
CAGAAACATGGCTTGCTTCTGTGGAACACGCTAGAGCAGCAGTCCCCAACCTTTTTGGCATCAGGGACCAGTTTCGTGGAAGACAG
CGTTTCCACAGACTGGGGGCTGGGGGTGATTTTGTGATGATTCAAGCACATTACATATATTGTGCACTTTATTTCTATTATTATTA
ACATTGTAATATGTAATTAAATAATTATACAACTCACCATAATGTAGAATCAGTAGGAGACCTGAGCTGCTTTTCCCACAACTAGA
CAGTCCCATCTGGGAGTGACGGGAGACAGTGACCCATCTGGAGACAGTGACAGTGATCATCAGGCGTTAGATTCTCATAAGGAGCG
GGCAATCTAGATCCCTTGCATGCACAGTTCACAATAGGGTTTATGCTCCTATGAGAATCTAATGCCACTGCTGATCTGACAGGACA
CGGAGCTCAGGCAGTAATGCAAGCGATGGGGAGCAGCTGTATATACAGATGAAGCTTGGCTGGCTTGCCTACTGCTCACCTCTTTT
TGTGCTGCGTTATCCATAGCCCAGGGTTTGGGGACGCCTGCACTAGAGGACAGAGCCTGGTACACTATAGCAGTCCTCCCCTTGTG
TTATGCAGAGTACTGATCAGTCCCTTTTGAGCTTCTCAACCTTGTATAAAACTTTGAGACAGTTCTGGGACCATCACAAGGTTTAT
GAAGGGTTAGAAAACAATACACTTAAGGAACTAAGACTGTAAATCGTTGAAGTTCAGAGGGCGATAAAAAGAAATGGCTTCTAAGA
ATCCCAGTATGTATGATTTTTTATACAAAGCATAAAGATGATTTGTACCAAACTCAGGACACTGTGGCGAGCTTAACACGAGGGTA
TTGAATCATACGTAAGTGGTAATTCCTAGATTATGCAATCTCCTTGCTGTTAGATTTAGATACCAAAGAGGAACTACATCATGTCC
TCCTCCTATGGTCTTCAAAAAAGGATTATATTTGGTCTTACTGGTATAATACAGTTTATTCCTGCATGAATTCCATGGTGATAGAA
TTTTTAAAGTTCATTCTAGTGTTACAGTTTTGTTTTGTTTTGTTTTAATACACAGCTTCAGAATGGCAGAAGATGATCCATATTTG
GGAAGGCCTGAACAAGTAAGTGTCATAATCTCACTGATAACTTTATTTAAATATATTCATATTTCAAAAATATGCAAAGGCAACAT
TAGTAAGTTAGCATTAGGAAAATTCTAAAATTAGTAAATTTTTTTTTAATTTCAGTTTCTCTGTCCTTTCATGTGAAGTTGGAGAG
GATATGTCATGTGAAATGAGTAACTTACACCCGCTTTCACACATATTGTTGCACAAAAATTGTATATGCTACAGGATAGCTATTGT
ATTAGCTTTCTAGAGCTACCATAACAGATCACCACAAACCTGATGGCTTAAAACAACAAAATACATTATTTCACAGTTCTGGGGGC
TAGAAGTATGAAATCAAGGTGTTGGCAGGGTTGGTTCCTTCTGGAGGCTCTCAGGAAGAATCTGTTCCATGCCTCTCCCCTAGCTT
CTGGTTGGTTGCTGGGAATCCTTGGCTTGTAGCTTTATCGCTCCAATCTCTTCCTCCATTGTCACGTGCGCTTCTTCCCGGCGTGT
CTCTACTGTGTCTCTAAACCTAAATCTCCTCTTTTTTTCTTCCAAAGGCATCATTGGATTTAGGGTACACCCTTATCCAATATGAC
TTCATCTTAATTTGTTTATATCTGCGAAGACCCTATTTCCAAATAAAGTCACATTCACAGGTACCGGGGATTAGGAGTTAAAGGTG
TCTTTTTGGGAGGGAACACAGTTTAACCCACTACAGCCCCTTGACATATTTATTTAATGTGTACACATGGATGTAGAGAGTGGAAT
GACAGACCATGGAGATTCAGAAAGGTGAGGGATGGGAGGGCAGTGGGATGGGGGTGGATGATAAGAAGTTACTTAATGGGTACAAT
GTATGTTATTTGAGTGTTGGATACCCTAAAAACCCTGTTTTGACTACTATACAATCTATGCATGTAACAAAATTGCACTTGTACCC
CATCAATTTATACCAAAAAAAAATTTTATTTAAAACTCATCTAGTAAGGAGACAGATTGTTGTAGTGAAAAGATCTTTGAACTCAG
AATTTGAAGATCTTTATATAAGTACTGACTACTACAGATCAGCTCCTCAGACTTGAAGAATCACTTAGTCCCCGTAATCCTCACTT
TTCTCATTTGTAAAATAGAAATGCTAGCACCACACTAACAGGGATGAGTCAAAAATATTCAATGAATGTGCTTTGTAAGCATAAAA
CTGTTCCTATCATTATATTAGTAGTTATGAAAGGATAGCAGTGTCCTTTTTTCTTTTTCTCCTATATTTTATTTGAAAAATGTTG
GAAAAAATAAATAGAAATGTTGGAATAATCTAACAAATCTACATTGGTTCTTAAAACCGACATTTTCGCTTTTCTCTTTGCTCTCT
TTTTCGTGTGTGTGTGTGTGTGTATATATGTATATATGTATATGTATATACATAATCATTTGGAAGCAATTTTCAGACTCTGGAGC
AATTATAATGGCAATATTAAATTATTTATGTAATGATTCCAAGAAGTATTTAAAACATGGAGATTTGTTCATTCATCAGCTATTTA
TTGAGGTATTACTACGTGCCAGAACATTGGCTTAGTGGGGAAAAAATAAGTGATATATACACTCTAATTTCAGGAACCTACTATTT
TAATAGGAAGAACAGAGACATTGTTTTCATATGGTGATATATGCTGTAGTAGTAGTCTGCGCAGAGCGGGATAGAAATGTTAATAA
GCAGCATCACTAGGTGCTACCTTGGAGGAAGAGCAGAGTTACGGAGGACTTTCCGAAGGAAGTCTTCGTAAGCTGTGTTTTGAAAG
ATGTAGTTTGCTAGACTAGAAAAGGTGAAGGAACACTAGGTGCCAGGATAGCCTGGTCACATTTAAATTTTAGTGGCAATCATTTA
AAATAGTGGATTTCTGAAATTACACGTGCAAAGAATCAGCCAGAAAAATAAAAACAGAAGTGGAATATGTTTTAAAGGCTCTGGTA
TCATTTCTATAAATAAAATGGACATATATAGCATTTATCACTTCGGGTGATATTCTGATTGATTTAGAAAGAATGACTGCATTGCT
TTTCACATTAAGATGCTCCTTTCTAATTTTATTTTTAAAATGGTGATTTAGGATAAATCTAGGAACATTTTCTTTTATTGCAGCAA
AGACATTTGGCTAATTGTATTATCAAAACCGTTTGTTTTACCTTTCGCTTTTAATGTGCTTTCTCTAACAATTAAGGGCGAACTAA
CCAGCATGAGGATTGTGTCTGCTTGATTTTAAACCATCCTTTCCTGTCTGTACACAGGAAATCTTATCAACAAGAGATGATTCTTT
ATGCCACAGAAGAGTAAAACCTGCTGAAAACATTCATTTTGAAAGTTTTTCTTATGTGGGCATCTTTGCGATCATCAGTAACAGGG
GTGGGGCATATTCTGACATTTCCATTTTTGAGGCAAGCGGTCTTGAAGTTTTTTTCTTTCTTGGCTTGAGCTTGGTGGTTTATATT
TCCTAAAGTTTGCATGTCCTCAGTGTTTTAGTGGAGTCTGGAATTTGCTCTCCACCCTGACCTAATAAACAATGCAAGCTGCAGCA
CAACACAACTTCTCTCTTAGTCTGAAGTGAAGAGTATATCTCTCTCCTAAGGTGAGCCCTTTTGCCCACTCGCCAGTCATTTAGCC
ACCACAGGAAAGAGTTTTCCTGCTAGGTTATCACGTTTGCTCTGAGACTGTTAAAATCCTGTATGAAATGAGGCCCATTTTTGCAG
AACATGGTTTTTCATTCCTGTATTTCCTTTTCAGCAAACAATGAATAGCATCAGTGAGCTTTTAATCTTATAAGCATTGAAGTGAA
GCGCATTCTTTGTTCCTCAGTTGAAAATTTGCATCCCTCAAATGGATCTGAAGTACTGTTTATTTAAACGAGATTTTTCTTACTCT
TAATTTAACTAGGCATACGAAGATCTTCAGTACCCCAGAACTTTCAATAATTTACTACTAAGGCAGGAGACAAACTGTAATACTG
GCTACTAAAAGAAACGAAAAGCTAGTTCAGCACACTTACTAGTCTCTCATAAAAAGTAAAATATGGCTGGTTTCCATGCTGTCCAC
AGACATTTCAAACATATAACTTCTTTAGGCTAAAATAAATAGCAAGCTCAGAAATACAGTTTTAAAGTCCTAAATGAGCCAGCGGT
TACAATGTTTTTTGATAAGTAATGAATAGTAATTTATTAAGATAAAATGTACTTTCTTCAAAATATTCTGCATGCCCACTCTGTTG
CTGCCCTAACACATTACCACATACTTGGGGCTTAAAACAACACAAATTTATACTTCTGAGTCAGGAGCTCAGAAATCTAGAATCAA
GGTGTTGGCAGGGCTGCTTTCCTTCTTGCGGCTCTCATCTCTTTGCCTTTTGAGCTTCTAGTGGCTGCCTGCTTTCCCTGTGCACC
CTCTCATCACTCCAGCCTCTTGCTTCCATTGTCATACTTCCTACTATTCAGTGTGATTTCCTGAACCCCTCTCATCAGGCCCACCT
GGACAATTCAAGACACTCTCTTCATCTTATTATCATTACCTTAATGACATACGTAAATTTCCTTTTGTCATATAAGGTAGCACTCA
CAGTTTCTGAGGATTAGGATGCAGACATGTTTAGGGGGTCGTTATTCAGCTTAACACACCCACTAAATTCCAGGTATTTTGTGTTA

TGCTAGGAAACTGAAGGTAAAAGAAATGAGTGAGCTCAGTGCTTAGTTGTCTACTCCTTGTACTGTCAGTTATATTTTCAGTTATT
CTGTAAATAGTCCCAGAGGTGCTCTCTAGCATTCTTTCAGGCCTTGACATTGGATAGCTAGATGAGGGGTATATTTACCACCATAG
AGGAAATACTGATTTCTAGGATTATATTATTTTGTTTATACTTCTAAGAGAGTACAAGCTTCAAATCATTCATTCAACAACTATTT
AAGTATCTGTTCTGTGATAAGCATGTACTTGGCCCTGAAGATATACCAGTAAGCAAGCTAGACCAGGTCCCTGCCTCTGGGGACTT
TATAGCCAAGTTATGCTTCTCAAATCCCTTTGTTACGTAAACGTTGTCCAACCCTATGTAAAGTTTTGTACACATGAGAACTACCT
AGGTCTCATGAAGATCTTTTAAAACCCTGATTTAAAATATTTTCAGATTTTTGTTTTTGGCTTTAGTTTCATTCCTATTATTACAT
TTTAGGTGTCCCAACTTCAAATTTTTCATATAATATAGGAAAAATAATGATTGAAACATTTAAATGTTCTTCTTTACAGATGTTTC
ATTTGGATCCTTCTTTGACTCATACAATATTTAATCCAGAAGTATTTCAACCACAGATGGCACTGCCAACAGGTAAGAAAACTCAT
CCCTGTTACCCTGTTGTTCTGCTTTCAGTCTTAGTAAAATGCAGGATTTGTTAATAGTCTGTCCTAGAAACTCAGTTGTGTACCTA
GAAAGGAAATGGTGATTTGTTTTAAAAACCAATCTTTTAACATACTTTCTTGAAATATATTTGCACAAAAATATTTCATCTCAATT
ACCCCTTGTATGGTTTACTGCATTTCATATCTGCTAGGACTACTCAGAAGGAATTTCTTTAGTCAACATAAGGATTTTCTTTATTA
TCTCTGTATTCCTTTTAAGTCCTTTCTCCCAGATTCACACGAGGCAGTCTCCACTCTTCATTTCTGTTTCAAACATTTAAGAGGGC
CTGTTGTGTACTACCAGTGTGCCAGTCACTGGGGAATCAAAGTGTACAGAAATCATAAAAAATCATCTCATTAGAAAAATCATCTTT
ATAAAAGCTTATAACTTTGCACTCTGACAAGTTGGATAAAACTCATAATCCTTCCTTCTACCATGACTGTATTAATTGTGCCGATT
GGCAACTGTTTAATTTCGTAAGAGTAATAAAAAAAAAACTCTCTTTCTTAAAAAAAGTGATTTATTTGTATGTAAGCTTGGTGTGA
TGGCTCTTTTTATGTTCTGTTTCTCTCCTCACTTCACCCGTCACCAGTGGGACTTAACAGATGTTCAAAAGCAGTGTGAGTGAATT
ATGGAAGTTATGTGTAAAACAGAGTTTGGTAATCGCTGAAGGAGGCATCAGCTCTTTTATTTACCAGGGACTTGCTTGTCTTCATT
GGGGGACTTTATCCCATTTGTATTATTCTCATTTAAAATTATCATGGTTACTACAGTACTTACTTTTCTTTTGTTTTAGATCAGAA
AACTTTTTCTGCATTTATATCTTTAGTATTTCTAGGAGTTCATGGGAGGGAAAAAAGTAGATCTACTATACTTTCACCTTCACTAG
TAAAGGTGTATTTCAGAGGTTTTTCTGTCTTTACCCCTACAAAACATTATTTTTAGGCTGCCATGTCTGAGATCTTGTGAAGTGCT
GTCCAATTTGAGCTCTAGGTCAGCTTCCTGTGAACAAGTAAATGTGTTGGTAGTGTGTGTGTTTCTTGGGAACTTTTTAATTCTGG
AGTTCAAAGTATTTTTCTTAGTATTAATGTATTACTGAAAATATAAGAACTTGGTTCTGTCCACAGGAAATACACACATTTTCACT
TAATGTCTTGTGAATACAAAACAGTATCCTGAGGCAGACGTTTTCTTAATTTTATTCCTTCGGATTTATGGGCATGAAAATAAGAT
TATCAAGATAACCATCTCAGTAAATATTCTGTGGTCACAGCTTTATGTTGTAGAAATTATTTTGCCATAATATTAGAAAAACTATT
CAAATTACACCTAAAAATCTAATTTGTTAGTTGTTGTTACACCATCATTTTTACCTGTATGTTTTTCCCTCTTCATTGCAGTGTCA
TAGTACAATTTTTATAGTCCAAGATATATAGGATGTTGTATTCAAATTTTTAAAGAGGTAGAGAGGTTGATTATCTAAACCAATAT
TAACTTGTAACTCTAAAATGGATTATTTTAGTCTTCTAGATTTTTTGAAATCTAAAACCTTTAGTAGATCTAGTACTTGTTGAAGA
CTATGGGATGCGACATAACTCTTAGGTCTAAAGTTAAATTTCTCTGCACAGTGGAATGTCCTTTTCATGGCACAGAAATGTGGCCA
AAAGAATGTTGGCCTTAAAACAACAAATACATGCATATCAAATCTAATTTTCTAGTTGATTTAACTTTAATGTGTTGCCTGAAAGA
GGAAAAATGGCTCTATTTTACATGAATTTTCACATATTTAAAGGAAATTAAATCCTACAGGAAAAATAATAGCAAATTTGCAGTTG
TTTTTAGCAGAGTTAATAAAAAGTGAGAGACTTGAAGTTTAATGTAAACATGCTTCCAACTCCTCTGTATTTTTCATGACATCCAA
ATATTGCTCTGTTAGAGGACTGTCTTGATATGTTCTTTCACCATTTTAACAGGGAATGTTTTTATAAGTGTTACATTGTCATACAG
GCTGTATCTTAGTGTGAAAATATTTCTATTTCTAATCAATTTTATTTCCATATCCAAAATTAAATTCTAGCCATTTGGTGGGGGAA
TTTTTAAATTATGATTATGTTTCTAAAAGTAATTTAAAATGTCCAGGGGTGAATTTCATACAGACAAAAGATGGTAATTTAATACA
TTGACCAGTGACTCCCCCTTTTTTTTCTTGTGTATGCTTCATTTCAGATGCATTCATTTTTCAACTCGCTTCCTTGTAAATTTCTT
GAATAATAACAGGAAGTAATTATACCCTTGTTTTCTCCAGTTCTCTAGCCTTATCCTGATTTATCATAATATTGAAAAGATAAAAA
TAAATTCTCTGGCCGGGTGAGGTGGCTCACGCCTGTAATCCCAGCACTTTGGGAGGCCAAGGCAGGTAGATCACGAGCTCAGGAGT
TCAAGACCAGCCTGGCCAAGATGGTGAAACCCCGTCTCTACTAAAAAAATACAAAAATTAGCTGGGCATGGTGGTAGGCGCCTGTA
ATTCCAGCTCCTCTGGAGGCTGAGGCAGAGAATTGCTTGAACCCAGGAAGCAGAGATTGTGGTGAGCCGAGATTGTGCCATGGCAC
TCCAGCCTGGGTGACAGAGTGAGACTCTGTCTCAAAAAAAAATAAAAAAAATTTTGAGAAAATTCTCTTTTGTGTTATAGTTTATT
TTTAATTTCAACCTGAAGGTTTATCTCCTTACAAATTTATTTAAAATAAGAGGTCTTGAGCTTCTTTCTGAGAGAAAATGAAGATT
AAAATGGACATACAAGCATTCTCCTAAAATATTTTTAGCACCAAACATCTTAATTTACATTCAAATAAATGAGAACCACCATATGC
CTTTATTTATTGCAAAGCTATGGATTATTGTACTATTGACATAAAAATCAGCTGGGAAGCTTAAGGACTTTTTCATATTGAACAGT
TTGTACTTATGTTGTCAGAGATTCTGATTTTCCTTTGAATTTTCAAGTCAGATACCATTAGGGTATGGATTAAGCCTCTAGTTTTC
TTTGCGTTTTTACAATAACTAATTTGCATGTACCTATCTTCAATTAAATTATTTTCAGTATTTTATAAAATGCCTGAGATGTTTTA
GAACACAGTTTATATGATAGCACAATATTCAAAATTGCTTATAAACATCAAAAGTTATGGATTAATATGAAAATTTTACCATGTCT
GATAGACCAGATTACATTGGGAGTGTCGGGGAGGAAAGGGGAAACAAACTAACCAGTTAAGGAATGAAGGCAGTACTTTCCCTTCA
AGTAAAGACGGGAACAGAAAACCTAGTGTAAGGAAAAAGAGATAGCCTGAGATCATTATCTGTCTATCAGACGTATACATACAGGT
TGCTAGATCAGTATCTTTTAATTTCACATTAAGCACACTCTCTTTGAGGAAGGAAAGTCAGTGTCTAATGATGTGAAGGCCAAAGA
AAATGTCATAGTGCCCAGAAGTGGTTGCTGCTTTTAATTGGTATTTGTCATTTTAAAATGCTTTGTTAGGAGACAAGATTATGAAG
GCTGTTGACATCAATTCTTGCCGGCCATATGCCTTTTATTGGGTTAGGAAATGTGCCATCACCTTTCAACAAACATTTACTTATTG
TGGTTCGCTAAACTCGTAAAACTATTGTTAAAATAAGGGTTAATTTAATATTTCAGACAAGGAAGAGAAATTATATCCTTGTGGAA
TTTTAACCAAAAATTGATTTGAAATTTCTACATAAAAACATAATTTCAGTTTTTTACTTGCTTTACGTTTTATACCAAATTTGAGA
AGCCTCACAGTTTCTTTTGGTTTCTGTTTGTTGTTTTTGTTTTTAGCAGATGGCCCATACCTTCAAATATTAGAGCAACCTAAACA
GGTAAGATTAAAGGGGTGGGACTTTAAATGTTAGATTCCAGTGTCTAATATTGAGATCATAAGCACTGAAGAATAGTAAATGAGTT
CTATGAAGGAAGTAGTTTATCTGAAAGATCAACAACCTGACAAATTATGTAACAGCTTTATTCATTCACTTTACATTTCTCTTACT
CATTGTTCACACTGTCCTGAGCCTCAGTATGTAGTTCTGTAAAACTGCAGTTAATTACAGTATTAGAATTACAGTTAATTACAGTA
TTCTGCTGTCTCTACCAGCTTAACTTACCAGCCACTCCCAGGAAAGGCAGAGGTGATTTCCATTCATTTTCATTTATAAGGAAATA
CGTATGCATACAGTTCACATGCACTGAGCGATATCCATCTATTATCCTTGCACATGTAGTACCTCTTAGCCTTCCTCCTTCCTTTG
GTAGCCCCAGCCTTTTCTGTGCTGCCTTTGGAACATGTAACCTAACATACTGAAAGTACCAGTGGGAAGTAAAATGTTAGAATGAA
CTATTGAATTCTCTTCCCTCTGGTGTCAGCCTCTTTGGAGTCAGGAGAGGAACATTTTTTCAGGTGCTTCCCCTTACCACTAGAGT
CTGTAGAGAATAGCTCAGAACTGAAATGGCCTCCTCACTGTATCCAACTCTGTGGATATTTATAGGAAATTGGATGGGACCAATGC
TGTGAAAGAGAAAGAAAGGCAATCTTTAATGTCTGCCTATAGGAGGTTTCAGTTATTAGGGAATGGAAATAATGGTGTAGTTCCCC
ACAGAATGAGGACAGTCAGTAAATCTTTGGATGGATAAACTAATCACATTTGTGTGTGACTGAGGATATGGATACCTGTCCTCTTG
CAGCTGTATTACCCAATACATTTTCTAAGTATTCTTTTCATTAGCTCAGGTGAGTGAAACACTTTTGCATTTTGTAACATTTAACA
CATGCTAACTAGGTCACAAGCTTAAATAAAGCATCCACTGCGTTGTGGTTTCCAGAATCGTGACATTATATATGTGTTATATTTGG
TCAGCATTTGATATAAATGTCTTAATGATTGCTGAACAGGCTATCCTTATAAGGTTGAAATTAGATGAAAGTTTTTGCTGTGGGGG
CAAGAATGCCACATGAAACTTTGGGGCATGGTTCTAGTTCTTGCAGGTGTCACATCCTGACTCCTCAGGTTGCCAGTAACACTCCA
TTGCTCTACCTCCATGTTCAACTCTTTTAAGCAGTTATTTCTCAGAGAAGGTCATCAATTCCCAGTGAAATTTTATCCCTTAAAGT
TCTGTGGGCTCTGGCATCCTGGAAAATTTCCCAGCTTTTAAGATCTAGGTAGTCTATGAAACAGAAATCAACTGAAATTTGTCTGC
ATATGCCAAAGTATTTTTTCCAATATCATTTTCATAAGCATAGCACTACAATAAGAATTTTTAAATGTAATTCCTTATTATGGATC
CAACAGTTATAAGGAAAAATTGGCATTTATGATTTACCTGAAGGGTTAATGTAGTTCCAAAATTCAAAATTTAATTCATATAAAAG
CTTATGTGAGTAAAACAATGTGTTTACCAAAGTGATGCTATTTTGATATCTGAATTCAGTGAAAGTAAGAAGGTTGTATTCAAGTC
AGACTTTCTGACTGAATGGATGTAGCCTTGCCTTTGAGGTTGATGACTCATTTTAAGCAAATGGAGTTACTGAGATGAGTGAATGT
GGATTAAAGGCAAAATTTTGATCTCAGAAATTTAGAATCAGAAATGCATCCAGACAATGCATTTGACGACATCTTCCTGAAAACAG
TTGTAAATTTTCATCCTCAGATTAAAAACTTCTGAGGATTTAGATACTCGTATGTAACCATGAAAAATATCTATTAAGTATTGTCT

ATTTGACACCACTCCCAATAAGATATAAACACATACGTGTCTATATTTTTCCAACTGTCCCAAAGGAATTTTGTGATTAAAGATAA
TGAATTGTGTGTGTGTGGTTTTGGGGTTTTTTTTGTTTTTTTTTTTTAAGTTAGAAAAAGATTCTTTTTTCTTTGGGACCTCTTAG
CCACAGATTTTCCCCAGCTCTGACGGAGATGAGTCATAGCATAGACTACTAATTTTTAGAACATCCAGTTCTGTTGCATATTAATC
AGTGTTAATTAACTAATACTGTTCAAAAACTCAAGTTGTGTTTAAATTAGCAGTCAAGTAAATCTCTATTCTTATGGTTAGCTAAT
TCTGCAGGCTATTATACATGTGTTGTTTTTTTCATGGTTTTAGAAATTTCATTTAATTATTTAAAAAACCAACAGTGTTTGCTTAC
AAAAGGCATAGGGCTGAAATTGTAAATTATTTTTAAATATGAATTGTGTGAGAACCAAAACAAAGGAATTACCTCTGAAACTTCAT
CTCCAAAGGCTTCCCTTGTGTTTAGATCTGGAATCTCCCAAGGAATTGTTTTAGGTCTCACTGTGATGAGTTGATCATAAACTTTA
GACATTTGTGTGCATTAAGGAAATTTCCCAGACCGGGGAATTTACTTTATGCCTTACTTATCTGGTAAATGTGTTTGAGGACTGAT
CTTTGGAAATCAGGAAGTTTTTGGATAATATAGGAACAGTTCCAGATGCTTCCCAGAGATTTAAATCAGTTCAAGGCACCTGGACT
GCTCAGAGACTGCAAGACCCTACTTGGCATAATGAAATGTAGTATAGTCTAAGTAGTGCATACGTTTTACATTTGTTCATGAGCTC
AAAGCTGTCAAAATCTGCATGTTTGCTGGGGTTCCACAATTTAATGAGTAAGGGAGTGAACCCCCACCAGCAGCTAGTGGGTGTGA
TGAGCTGATGGGCAGATGATCTAGTGCTGTATATGCAGAGTTGGTCCCACAATAGAAACAAAAGGTGAGAATGTTCCTTAAAAAAT
CTTAACGTATTTTCTTTTCACCAAAAAGTGAATCTGTAAAAAGAAAATCATCTGCTTAAGCGACTATGTGGTTATAAACTACTAAC
ACTACCACAAATTGATTGACATCATTATAAAGTTGAATCAGGAAATACAGCGAAACCTCCCTTAATATGATGATGAGGTCATAATT
ATCCTTGTTTTATTCTTTGTATACAAATATGAATAAGTTAGCTTTATAAATCATAAAAAGTTAGAACTGGAGTAGACCTTAAAGAT
GGTCTGGTATAATCTTCCCTTTTGTACAAATGAAAAAATATGACACTTCTTAGGTGTTTATCAGCCCACAGTAGTTTATATAGTGA
AGGCTTCCAGCTAACTTCATTAATTAACTATATCTATAAAATTTCTGGGTTAGATAGTATTTAAGGGAACTACCTAGTTTTGGAAT
CATTCTGGTAGGTTAATATTCAATCTTGAGCTTGACCATATTAATAATCATAAAAACAAAAATCTTATCCTCTGAAATGCTGAGAG
AAGCTTAACAGATGCAGGGTCTAGCACAGGGTGTGTTCTACAACGCTGAAACAGTATATCTAAATAAACATGTCTGTAGTCCCTGC
TGAACCAGCTGTAATCAGAGTAGATAAAGGAATGTCTTTAAGTAAGAGTCAAGGAAGCATAACTTTTATTAATATGGTACAGTTCA
GAGCTTGGCAGCAGCAATTTAAGACAAGGAAGCTCTGACTAGAACAAGCCGTAACATGTTAAGTCTAAAGCCTAAACTCTTCAGCA
GATTACTCTCCACACATGCATAGCATGAGAGGTTCCATGGGCTTAGGTACCTGGCTTTTTAGCCATATCTTAGTGTACAAATATCG
ATTAATACCATTTTTCGTAGTAAGATTACGGAAAAGTGATTCTTGTTTACAGAGCCCTCTTTCAGTTTCATGTTTTTCTTCTCTC
ATTTAGTAGACATAAGATTTAAAAATTTGTATGTACCTTTGTTGCCGTAATTTTTAATAAGTATTTCTCAAACTTAATTGGCTTAA
CGTTCACCTTTGCAGAGAGGATTTCGTTTCCGTTATGTATGTGAAGGCCCATCCCATGGTGGACTACCTGGTGCCTCTAGTGAAAA
GAACAAGAAGTCTTACCCTCAGGTCAAAGTAAGTTTGTGGTAGCTCTCCTTCTATTTGAATTCTGGAAATTTTGATTTCCTACGAT
TTCCAAGGAATTGCTTTAAATGAGTACGGGTTGCCTTCGCTCCTAAGCTGAAGTGTTCACATGATTATTAAAATTTTTAAATAGAA
ATTTGTCTCCTAGCAATAGAAGTGACAGATACTAAAACTTTGTTAACATTTCAATTTAGTAGAAATGTCTTCAGCATTAGCTAACA
AACTTACTATTTCTTGATCATCTTAAATATTTTTAAAAATTGGATACTTCCTGAAACTTTAGTAAGTCTTTGAAAGAAATGGTTGA
TTTTGACTCTTTGGAGATTTAGTGAAAAACCAAACAAGTGACTGAGTGTATGGATTATATAGTATATTATTTAAGATTATGAATTT
GGATCCAGACTTTGTTATTAGCTGTGTAGCTTTGAATAGGTTTAACCAATACTTCTAAACTTCAGTCTCTTAATGTATAAAATACA
AAAATATTAATAGTATATACTTCATAAGGTCCTTTAGAGATTAAATGAGAAAATGTGTACATGTTCCATAGAGAGCCTGAAAGTAC
CCTAGGGCCTTGGCCTGAGCAGATGTGTGGCCCTCTTGGAATGAACTGCTACGTGACATGCTGGTGGTGTATTTGGTCATACCTTT
TACCATGTTCAGGCTGTTTGTTTATTTGAGAATATTTTTCTTTAAGTTAATATGAGACTTTTAATTTCATAATATGCTATGTTTCA
TAGACTGACTGATGATAAACATAGACTGATAATCATTTTGCAGATAAACGCAGTCAACAATTGTAATTTAGTTTTTTATAAAAGTT
ATTATTTAGTCAGTAATTCAAGGCTTGGACAAAATAGTTCTCACTATGTGTGTGTGCCCCTCAGGGTGTAACATACTTTTGGACTC
AGTCTTTTTATTGGTTTGTTTTTGTAAGTAGATGAAGCTCAGTAGAGCAGGCCCTCAGCAAAGTTGTGATGTAAAGAAATCTGGCC
GGGCTCGTTGACTCATGTCTGTAATCCCAGCACTTTGGGAGGCCGAGGCTAGCAGATCACAAGGTCAGGAGTTCGAGACCAGCCTG
GCCAATATGGTGAAACCCCATCTCTACTAAAAATACAAAAATTAGCTGGGTGTGGTGGTGTACACCTGTAGTCCCAGCTAGTCAGG
AGGCTGAGGCACAAGAATCGCTTGAACCTGGGAAGCGGAGGTTGCAGTGAGCCGAGACTGTGCCACTGGACTCCATCCTGGACAAC
AGAGCGAGACTCTGTCTCAAAAAAAAAAAAAAAAAAAAAAAAGAAAGAAATCAGTCTTTGGTTTTGCCAGCCTTGCATTTTGTTTCTT
CCTTTTCCTCTTATCCATCTTCTAGGCCCAGGCCTGTTAAATTTCTTATCCTTATTCCCAAACAAAAGACAAAATGTTGTGACGTC
GTTCTTTACCTTTCAAGTGAAACTCACCCATTATTTAGTATGGGTACTACTAGTCATAGTTGCATTTTAAGTCTCTTAGCTCCAAT
TGTGGAATGAAAGATGTTTTTTCTATTAAAGGTAATTAGGCGGGTAAATATGATGGCTTAAGTCCACAGAAGCAGACCACGGTCTT
ATGGCCTCACATCCTTTAGAACCTTGATGAGGAATGAGGAGCAGCAATAAAGAACTAATTTGCACTTATTAAACACCAAAGACTTG
CCAAGTGGTTTGTATAACATTATCTCTTTTAATCTCTTAGAAATCTCAGTAGGTAAAAAGTGCAATGCCTGTGAACCCAAACAGAC
TCAGAGAATGGAATTATAATAACTGGCCTTTCTCACAAGACTAGTGGCTCTATATCCTGCAGCAGGCTATTGCTCACCGAACCTCT
CAGATATAATGTGTGCATTTGGGGTACTGGGGGCAGGGAGATAAGGGACAAATTATGGGCAGACAATGGTGTAAAAAGGCATGGTG
AATGATAACTCTTGAAAACAAGGGAGAGAGAGGCTCACTGGTCAATGGGTACTCAGGTATTGGGGAAGCCAGGCCCAGCACTTACC
TTTTTAACACATGTGTCTGTGTGTCACATGTATAAATATTGTGCCTTTATGTATGTGTATATTTGTGAGAAGGGGATTGAAAGACA
CATGTTATCAGCATTGACACTGACTATAATGACTGAAGAATTAACTTTAAGCGAAAAACTGGGAGTTTTTTCTGACTGGCTTGGCA
CAAAAAATATAAATTATGGTATTTTGTTTACTTCTATTGCTCTAGTATTTTGTGACTAAGTAACAATTCTTGATACTAACAAACAC
ATGGCATTAACTTTCTTATAAATTTATTGGCTAAGAAAAAACTTTATACATCCATTTATTCCCAGTCTTCATACATCTTTCTCATG
TAGAGGTTTCATCTACTCAAAAACATATTGCTGCCTCAGTCACAAAATAATTTACAAATGAAACAACTTAGTGGGCATTAGATGTT
CTTGTAGAATGAAAGTTTCTATATCAAGGAGTTTAGTACTTACTGGGAAATATAGATATGTAAACGACTAATAATAAAGCAGGTTC
AACTACAGTCACATTATTTCACCAAATCATTTATTTTTTATTCACTAATTCAGTATCCATTTATTAAGCATTTAATCAACTTGTAA
GAGGAGGAGGAACTTAGAGGTTAGAGAATATGACTATACAAGCTGTCATTGTTCCCTTAGCTTGTCATTGTTCACTGAAAATTGAA
ATGGTTCTGCTGTTCCTAACAGTTATAGGAAGTAATTTTATTTATCCAAGAAATTCTTAATTGGAAGGATGGGTAGGAGAGAAAGA
AAGAGACAACGGCCAGGTATTTTTTTTTTCCAGAGCTCTTCTCCAAATATCCCTTTTAATAGTTATTTCTGCTCAGGATGACCTTT
AAGTTTACAAAGGAATTTTAAATCCCCTTTTCCTCTAATGTTTAAGCAGAGGAAAAAATTGTTCTCCATGCATCTTTCTAATCAGG
AAGACCTTTAAAATTGTGCATGTCTTTTCTGTGGTACTTTACTAAGCACCTGCTCGGCGAGAGACTGTAAGGGAAACAGTGGGATA
GATACATCTAAAGTTTCTCAAACTTGGCATTATTGACATTTGGGGCTTGAAAATTCTGTGTTGTGGGAGGCTGTCTGACACGTTAT
AAAATATTTAGCAGCCTCCCTGACCTTGACTCACTAGATGCTAATAGGATTCCTATAATTGTGACATCCAAAAAGTCTCCACATTG
ACAAATGTCCCCTGGGGGGCAAAATCACCCCTCTTTGAGAACCACTGATGTGGATGCAACAAAGAATCAACCTTAACCTTACCCAT
TTGCATACTTTTCTACTATAAAGTTGTCTCGCATAGATTACGTAGAATTTCCAAGTGTATTATTTTATAAACGTGCCTTTCTTGGT
TATTAGTAGATATTTCTTATTTTCAAGATGGTGATCATGGTGTCTGTATAATTTCTGTTAGAATATCAGAGTTAGTGTACCTGGAT
ACTAAGGAGAAAGATAGAAAAGATAAAATATGTATCTTCAACATTTTGTATTCTGTGATGACCCTAAAAGCTAATGTGGGAGTTCT
ATTGACTGTTATAAACTATTACAGAAGAAAAAGTGAAAGTGGAGAGTTAGGTCCACTGTGTGTGAACAATGGGAGGGTCTATGGAA
TGATGCTTTCAGGTGGGCAGAGGCCTCGTGGAGGGTGGCCCCACACTCACCCCCAGAATCCAGAAGCCATTCCCACAGGAACTTCA
GAGGGAGAGCATTTACATTTACAGGAAAGAAATGATACTTCTGAGCCTACATTTGCCACATATTCATTAAGTGCATTTGTTCAGGC
ATTACAGTAAAATGCCATGGGCTGTGCTTTTCCTCAGCTACAGCCTCTCTCCCTCCAACCTCACACCCTGTCTTCCTGTCATGCCT
TTGTGAGAGCGCCACCACTCTGCGGTGCCTGGAGAGGAACCTGGAAACGCTGATAGTTCAGTGCATACTCGGTCATATGGAGACAG
CAGGAAAGAGTCAGGGGCCAACTTGAAAGATTTGGGGCCTGGCTGTGGTTTGCAGGCTGTAGACACCAAACTATACTTTATAGAAC
ATGTGAAAATATAGCCTGTATGGGTTACTCTCTTACTGATTGTCTAGATTTGATGCCTTACATTAAATCATTTTAAAACCAACTCT
AGAAGTCTACTCAAATCCTGCATTTTCTAGTGGAATAGTGTCCCATGCATAAAACCCTGTTTCGCATAGGTGTAGGCTAGCTTACA
GACCCTCTTACCATGTTGAGATTATTTCTAGAACTCAGGCTACTGAACCATCTGGTGCTCTGCCTGTGTCTTTCACATAGCTAAGA

AGAAAATTTCCATTTAGCTTTCTGATATCTTGCTGTGCAAGTCTTCATGCTCTTTTTTTAAAAAAAATTTAATTATAAATTCTTT
CAAGCATATAGAAAGTTTAAAAATTGACATAACAGACACTATGTACTACAACACAAATTTAATAGGTATTAGCATCTTACCTTATT
TGCTTTTGATAGCTCTCTCTCTCTCTCTGTCCTCTCTCTCTCTCAAATAAAATAGTTACAGCTAAAACCCCACTTATCCATCAC
TTTGTCCTCCTTCCTTCTCTTAGGTAAGCTGCTACTCTGAACTTTCACTCCCCAAGATGTTTTTATATTTTTACTACATAAGAATG
CATCTATATAAATACTGGCATTTTCAAGAAAGTTAATTTTTACTAACTTCAACCCAAATTTGCCATTGTATAGGATGGCTCCTTTTCA
GGGCAAACATAGGTTCAAACTGGACGCCATTGAAGATTAACTGGTACTCACTTTGATCCTATTGATTCCTCTTTGCTGTCTAGATA
AAAATGTATTTGTCACAATGTAGTTTGTACTAACTCTTATAGCAAAAAATAATATTCAGAGAAAAATCTAGGGGAAGTAAGTATGC
AGACTAACTCTTATATCTTGTCCTTTCATAAGAAAAATTTCCCATCATTAAACATTTAATAATCTATTATACTGCCTATAAAATTT
CCCTTTAAAGTTTTTTCCATTCTAATTTCATCACGGAGCTCCTAATAGGAGAGGCTGGCAGAAAAGCACAGGAACCTTCCTCTAGG
CCAAGCTTGTCCCTGCCTGCTTTTGACTCAGCAAGGAGTGCCAGGGAAGGCTCACTTCCCTATTTTTCCCGAAAGCCACGCGTGAA
GTTTTAAAAATCAAACTGTGTTCCCTTTCACAGAGGTGGGTGACAAGAGAAGCAGCCCCATGGGACTTCAGAAACTCTCACCAATC
CTCCTCTAGAGTGACCAGAAAGTACCCCTTCCTATCCTAACTAATGTGACACGTCCTTCTGTGTGGTCCACTCTATGCAAAACCAG
AAATTGACAAGAATGACTTTGATTGTAAAGTACAAATAGCTTACCAGTTAGTATTCTTAGTTTCTCTTTAGTTATAAATATCAAAT
GCAAGGGACTTGAAACCCAGCTGATAGAAGTGTAACTATTTTTAATCAAACATTTTCTGAAAGCTGGTTCTCACATATGTTGTCTT
TCCTGAGTGTTTTATGTTGAGTGGAAGACGTGTTGAAATGTTTAGGTCAACAACAGCTTTTTTGTTGTCCCCTTCCACTACTGATT
TAAAAAAAAAAAAAAAAGGCTCCACTCTTCGTGCCTTTCCTTAAAAATCCTAAATGTTAAGTTTCTGTCGAGGTCGAATAGAGACT
ACCCGATCTGCTGTGGCATTTGAGGATATCACATATCCTTGCATAATCTCCCTTTCCTTTGTATAGACTTGTCTGATTCACGTGGT
TTGGATCTATTTGGAGTACTAATTTAAAATAATTTAACCTGACTCAATTACTTAAAACAACAGGGGAGAAAATCACCAATAATCAG
CTATGTGTGCGTATAGAACCTAGTTCATATCATTTATTAGGATTTCCATTGAACCCCTAAGCTGTTCACTGCGCAAGTATTTAATT
AGGGAAAATAACCATACCCCTATGTACATTATACTGTGTGTTTTGTAAACCATGAGAAAAGCTGCTTATTGAACAGAAGGGCTTTG
TAACCTGGGGTCTATGGATCCCTGTTGGTGGTTTCAGACGCTTTTTAGATTCCTAAATTTATATGCAGACTTACACATGTATGTAT
GTGCATTTTTTTCTTGGGAGATGAGTATAACTTTTTATCATATTTTCAAAGGGAAAGATTTAACATGGTTAAGAATCATGCTATAGC
TTGTATCACTTTAGCTTTTTGATAGCAGCTATCTTTTTAAATGATCTATGTAATATTTTTTAAGATTTTTTCTTTTTTTATTCTTG
CATTTTTAATCAATTTTAAATGAAGTGAAATGCTTTATGTTGCTCAACACATAGCCCAGAAAGCCTCCCTTCAGTTTTCCAGTAGG
TGGAAAAAAGATGAAACTGAATTGATGGCCAAAAGAGTTGAATGGCTTCACTGCTTTTTCTTTCTTGCTCTTCCATACATTTGCA
GATTAAGCATGAAGCTCACCATTAGTGATTGAAGCCAAAAGAGAGAAGGCAATTCATTGAACAAATATTGATTGAGCACTGTGTGT
CAGGTACTGCTCTAGACAAGCAGACAAAACTCCCTCCCATATGTAACCACAATTGAGACTTATAGGTAATTTTTGTTGCAAGTAGT
TCAGAAGTTTTGTATTTTGTCCTAAAACAAAATATTACTTTGTAAAATATTTTTTTAAATAGCCCTCTAAATCCGCTGTATATATT
CAAAGTCACTATTATAAAGTGGAGAAAGCTACTCATTAGAGAAAACCTATCACACAGATAATAGCTGGGAAGCTTAACCCTTAGAA
ATTATTAGGAGAGATATACACTCCTTGAATTTAAGAACTGTCTGAATTTTAAATTTTGTTTTGTCAGTTCTGGCTTGGTGACCTTC
CTCTATTGGCCATTCCCATTTTTCTGCCCTCCACTCTTCCTTGACACATGATTATTTCTGGGACCACTGTTCAATCCGAAACAAAT
CTCTGGTGGCTTCTTTTAAACCCAGTGGTCTCAGTCTGAAGGTATAGTGGGGCAACTCTCGGCATTCCATTCTGGAGTCTTGACCT
CATAAAGTCACATATTGGCAATTCGCCTTGATAAGGTCAAGTGAACACACAGAAGCACTAAGATCCCCACATGTGTTCCACTGCCT
CCTCCACAGACAAGTTGTTTTTCAACCCATCTGCAGGTCCAGAGGTGAAGCTGGAGGCTACCCTGCAGTCTCAGTCTTTTCTCCCC
ACCCTCCAGATGGATTCCAAATAAGAAGCCTCCAGACTACCTTCACCTCCCTGGGGTCTCATGGACATGGGCTGGAAAGTGCTTGC
AGACTTTCACAGACAATGCTTTTTGCCTTTAGGTCCCCTTCTACCCAACAGAATGATTATAATAATGGCTTGATGTGCTTACTTAC
ATCATCTCATTTAATCATCACAGTAATCCACTGATGCAGGTTCTGTGATCCGCATTTTATAAATGAGGAAATTGAAGCCTAGAGAA
ATTAAGAAGCTTAACTTGGGTCTCACAGGCTTTTAGAGATGGAGCCCAAGATTTCAGTTCATGCCATATGGTTTAAGGGCCTGCAC
TCTTAACCATCGTGGAATACCATTCATTATCATAATGTTAAGGATCTGTTTCTACTTTGCAGGAATCTTTTAAATCTATGGTTCAC
AATCATGGGTGGGCTTCAGAATCGTCCAAAAATGTTTCAACCACAGAGACGCCTGGGAACCACTGCACAATTGCTAAATCACAGTG
CATCATCTAGCCAATCTTGGATTCTACAATAATTCTATGACAGTTTCTAAGAATACATGCCATTATATAATGGCAACATATTACAA
CAGATAATTGAATAGATATTCAATGTATACCGTGGAATAGAATATTTATGCCACTGTGTTTTAAAAAGATAATTAAGAGGATACGGC
ACAACGTGGAAAACTGCTTATAACATAATATTAAGGATAAACCTCAGCTGGGCATAGTGGCTCACGCCTATAATCCCAACACTTTT
GGGAGGCCAAGGCAGGAGGATTGCTTGAGCCCAGGATTTTGAGGCCAGCATGGGCAACATAGTGAGACCCTGTCTCTATAAAAAAA
ATTTTTTAAAGAAAAAAAACAGGATTTCAAATTATTGCTAATATTTGTAGTGATTACAAATATTAGCTTCTTTTTATAAAGCCTGG
GTGGAAGCATATAGATGTATCTTTAGCTTTATTTATTCATCCATGACTTAAAGAAGATTGGTTAACACCTGACTCCACCTTGAGTA
GCAGAACATTGGACTGAAGGAAAAAAAAAAAATGTGAAGCTGTCTTACTTAGCTACAGTTTCCAGCATGTTTTCTCAGGAGCTCTAT
GGGAGACAGTGCAGTGTGGCTCTCAAACCTTACTACCCATAAAAATCTGCTTCAAAACCTACTTAAAATATCGATTTTATTCCATT
TCCAAAGCCTGGTAGAGTAGGTAATTCTCCCACTACAATACACTTAGTAACACTAGAAAAAAAATATAACATTTTCTTTAATGCAT
AGCTGAGTTTATTAGAAAGTATGAAAACTACTGGGGCGCCGTAAGACAAAAGAAAACTTTGGGAAAACACAATAGGATATGGGTGG
ACTTTGGGGCCACAGCTGCCCTGGAAATATTTGCTGACCTAGATAATCAAGAAGCTTGGTTTTTAACAGCTTCCTAGAGACAAAAG
ATAAGGCCTTGGGCCTTTCCAATCTGAGGAATTGAAACTGCAATCACCCACTTAATTCAGGACTCTCTCAGGGCTATAGTTTCTGT
TGATGGGTGATCCTCAGGCAGAGAGGTGATGAGGAAGTTGCCTGTCTCAGCCTAGGGGGAATCAGAAGATAAAATGCCTCTCTTGA
GAATCAATAACTACAAGCCCAATTCTTACAGATTCAGAGTTAAGTGTATGCTCCCTGAATAGAGTAGGAAACCCCAAGTCTAGAAA
TGAATATGAAGTTGTTCCACATTGGGGATACCCTCAGAGCCTTGCAGAAAAGCATAAAAATGTATGTGGCTCAAAGAATCATGAGC
TCCTGATCAAAAATTAGAAAATATGTATGCAAATAAGCCACTCTGTTTAAGAACTGGCAGAACAATGAAAAGCAGAATTAAATCCT
CAGAATCTTCAGATAATTGAATTACCAATTGTTTGATATATAAATACAAAATAAGTTTGGAATAGAAAACAGACCAGGCAGAATGA
AAATAAAACCAAGTCGAATTTTTAAAATTAAATGTGCGACACTGAAAATGATTCAACATAGTTAACATGGATAATTTGAAAGAAAG
GATGAGCTGGCAGGTACAAAAGAAGGAGTAAGAGGTGTCTAGTTGAAGTCCTAGAAAGAAGTATTACAACTGCAGATTTCTGGCTC
TACATTTACAGTTTCTAGGTCTGGGGTGAGGTTTAGGTGGTTGTGGACCACACTTTTAGGTTTTAAAGCATTGGCTCTGAAGTCAT
TTGGTTCAGGGCTGGGCATGATGTTGGTTCCTAGTTTTGCGACTTGCAGTGTTACTTAACTTGGCTAAGCATCAGCTTCCTTATCT
ATGAAACTGAAAAAATAATACCTATGTCATAGGATATATGTGTAAATCAAGTTATTCAGTACATGCCACACAGTAAGCACTTAAAA
ATGACAGTTGCTTCTATTTTTATATAACCAAATACACTACTACAAATATTGAGTGGCAGCTTTGCTGGTTCATTTATGGATTCATG
GAGCTACACAAAACCTTCACCAGTCTATTAATTTATTTTAGTACAGTCTCTCTAATCAGTATTGCTTCCACTGTATATTGACCCC
ATATCCTTTCTTCTGTCTGGGGTTAAGATAGGAGACTGAGATCAGCTGTGTCAAAAGCTTTCTTTTAGATGTAACACATGATTTCT
TATGAAAATCTCATAGGTAGGATACCAGAAAGGAATTTATTAAATTCTTGTTAGGCTCCTTAGTTGTATTAGTTTTGTAGGGCAGT
GTTTTAATTTAGGATACTTATGATAGAGAGCACCCCTTCCATAATCAGGAGAAACACAAGATGGAAATAAAGATTTTATTACTTAG
TGGTCCTGGGGAGCACACAGCACTCCAGGGAAGGCCACACACCAAGATCAGGGAGCATGTGGAGAAAGAGAGAGAAAGGAACTCAC
GGGCCATTGCCTTTACTGGGTCCAGGACATTATCCAAACAGGTTTCCCACAGGGAGTTTTAATAGGTGGGTTTCAAGCAAGCAGGT
ATGAGTTCCAGGAGGTCACAGTGTGATGAAGAAGAAGTCACTGTGGCATATCTGCACAGTCCATGCGAGGTGTGAGCATCAGTGAA
GACCAGTCAGGTAGGCTATATGTAGCTGGCCCATGGGGAGGCGATCACCAGGTGGCAATTGTATAATGCAGATATCTAGATTGGCC
ACATAGAGGAACCGGGAGGAGGTGTAGTACTGGAAACTGCGTCGACGGTCACTGAGCCCTGCTTTAGGTATGAGAAAGTCCAGCTT
ATATTCAGAATGGATGCAAAGGCAGCATAAAATTAAAAGCATTCACTGCCAGCTGCCATAACAAGTTACCACAAACTAGGTGGTTT
AAAGCAACCGAAATTTATTCTTTCACAGTTCTGGATGCTAGAAGTCCAATATCATGGTCTTCGCAGGGCCGTGCCCCCTTTGAAGG
CTCTAGGCAATAATCCTTCCTTGCCTGTTTCTAGCTTCTTGTGGTTGCTGACAATTCATGGCAGTTTGTGACTTGCAGCTGCATCA
TTCCAATTTCTGTCTCCATCTTCACATGAATACTGTCTTCTCTCTGTGTCCCTGTGTCCAAATCTCCCTCTTCTTTCTCTTAGAGA

CCTGTCATTGTGTTTACGGCATCCATAACCCTATGTGACCTCATCTTAACTAATCATAATTGCAACGACCCCATTTCCAGATAATA
CAATATTCTGAGGCTCTGGGTAGATGTGAATTCTGGGGGTACACCATTCAGCCCACTACAAACATATTGTGGCATTGAGGTTATAA
GCCAGTAGCCCTACAAGTATAGGATGATGCCCATTTCAGCCTGATAATACACTCCGAAAGAGATCACATGGTAACATTGTTGCCTC
AGTTAATCATAGAAATACTCAATTAGTATAGAAATAACAAGAAAAATGCTTTGTTATTCTCATGTGTCATTTATCGAGTTCTTACT
ATGTATCTGTTATTGTTATCCTTATACACTTCTGCACAAAAGATGGTTTTATCCCAAGTTTTAGCTTAGAAACTGAGGCTTAATGA
GTAACTTGACCAAGAATGGCTTCTCTTTCTCCATTTCCTTCCTCTCATGGTGTTGTTTTATATACATAAAATAATATATATTATTC
TATTAATATAGATTAATATATGTATTTATATATGTACATTTTATATATGTATAAAACTTATGTTGTAAGTTTTATATACATATAAT
ATATATTCTGTTTATATATTGAATTTATATATATATAAAACTTAACACTATTAAGTCTTAACACTATTAAGTTTCATGGTGTTGAG
TTATATAGTTCTATTAATTATATATATATGGGGGGAGGGGTGTTTCTCCAGCCTAGACTTCCACTTTGAAATGTAGTCTCCAGGCT
CAGCCACTTGTTTGACATTGCCACTTGGATGTCTGGTAGACTTAACATATTCACAACTGAATTTCAAATCTTCCCCACCAATCTGC
TCTGCCTCATAGCTTCCCCTTTCCGGAATGTGGTTGCTCCATCCTTCCAGTTGTACAGGCCCCAAATCTTGGAGTTTTCCTTGACT
CCTCTCTTCTTCTCAAATCCCACATGTAATCCATCAGGTAGTCTTATTGGCTGTGCCTTCACACTTTTTTCAGAAACTAATTATTC
TTCAGCACCTCCAATGCTGCCACCCTGGTATAAGCTACCATCCTCAGCATCTTTCTTCCAGTGGATTTTAGCATCCATGAATGATT
ATTTCCTAAATCAATTATCAATAATTGTTATAAATTTGTGATTTTTCTATTTCTATCCTTCCTGTCACTTTTCCTGGTTTTTTTAA
AAGAGCTTTCTTCATTCTTCTTTAAAAAAAATTAAAATTAACATCAGTATGAACTCGTGGATTCTTTTGTTATTCATTGTACTATA
AGCCATTCCTGTCATTATTCATTTTGATTCTTAAATTGTCCTATTCTTGGCCAGTGGGAGTCTTTCATGCTAGCTCCTATGTCTTT
TTGATATGTCCCTATCTTTTTTTTCCTAACACTTTCCTTATTTCTGAAATTATAAGCTATTCTAGGTTCACCCTATACCTTCACTA
CCCCAGTCCAGGCATCGTCTGTCTCTCTAAGAAGTCCTAGTTCCTTTTAGCAGGAGAAGTTTTTCTAGACTCTTTCATTAGGCAGA
GCTACAAAATTGATTATTTTAAAAATCTCGAGTTCATACACTAATTCATAATTCTAATTCCAATCCAACACTGCAGGCTTCTTCCC
TCCCTCCCCTCATCCCATACTTGTATTTGGATTTGAGATATGAAATGTGTATCTCATATCCCATATTTCTTCAGTAACTGAAAACC
CTCATCATCAATGATAGCAGCATGCTTACTCATTTTGCTCAGTCCAATAATTCACACACATTTGTAGAAAAATAGAATAGTTTTAG
AATGGCTATACCAATATCTGCAAGCTTACTATATTCGGTTCAAAATTTCTTTGTAGTTCTTTTGACCAAATGTATTCAACAGAGAG
CATTCAGTCAGAGTAATGTGTACAAAAGTTACTTGGATTAAATTTTTTCCTTCTGTGTATTTATGTTTCCATTGAGCTATAGAGTT
AAGCTATTTTTTTATTTCTATTCCATTTTAGGGGTTTTCCCATCCCTGTTGATTTAGTAATTTTATTTTTTGAATATCAACATTTG
TCAGTTTCACATCTTTCATTTATATGAGATTAACTAGACCCTTTAGAAATCATTATAAAGTCAGGTTGTTCTATTAATTTTTAAAA
TAGGTGTATAAAAACCAAAGGGATCAATCAGAGCCTTGATTTAAAGTACGTTCTGAATTATTTCAGACACCCGAATCCATCTAAAC
CCAGAACATTTCATTTGTAGATTTCAAAGACTTCTTGGCCTTCCTACCTTCCTTACATTTATGTAGCATGTGTTTATCCAAGGCAC
ACCACCTGCCAGGCACAGTGCTGGGCTCTGTGTTGACACACATGAATGTGATCATCTTTCTGTAGCCCCCAGGTTACATGGCACCA
CAGCCAAGATACAGACATGTGTGAGAGAGTAATCAGGGTATTACTCAGGATTTGCTGGAGGAATCCCTCTTCAAAGGATATTCTAA
ATTGGTGTGTGTGTGTGTGTGTGTGCGCGCGCGCGCGCATGTGTGTGTGTGTGTGTGTTGAGGTGGACTGGGAGAAAATATACCCTCC
TTGAGATCACCTCCTGGCTAGATAGTCTCTCCCTTCTCTGTTTGTGATAGAATGCAAGTGGAATTTGACAGGTGTACTTCTCAAAC
TAGAAACTAGAGAGCTGCTATCTCAGTCACCGTAAGAAGAACGAATCACACCAAACCTACATCATTGTCTTCTATGACAGGATTTC
TACAAGGGTGAGATAGTGAGATGCTGGAGATCCCTGTTTTGGTAGTTCAGGGATTCACAGCATCTCCCATGCTGGTGATGTCAACC
TGAAGAGATATTTCTAGTGATAGGTTCCAGGGATCTTTCACCGAACACTTTAATATATTTTATCATTGTCCTGAATACAGCCCTAT
AAGGTTCTTACTAAATTTGCAGGTGACATGAAGTGATAGACAACAAACAATTTGAATTCAAATTAATTTTGAAAGCTGAGACAATG
GACCCAATTCTTCAGCAATTATATTTAAAAGATATAAATACATGCAGAAAACAAATTACAAGTACAGAGTGGAAGAGAGATGATTT
AGCAGCAACGCATGCAAAGAGATCTAGGAGTTTTAGTTAATAACAGACTCAACATGAACCAGCAATGCAATGTGACTACGAAACAA
GTAATTCCTCATTAGGCTGCATTAATGGAAACACGATACATAAGACCAAGGAAGTGGTGGTCTCATTTGCACTCTGTGATGAAAGG
CCACACCAGGAATACAGTGTAGTACTGCTGGGCACCACATTTGTAGAGAAATAGAAACCAACTTGAGTGTCTTCAGAGCAGAATGG
TGAGAGAACTCAAACTTCATTCAAATGAGGAGTAGTTACTGAGCCTGAGAAATTTATAGCCTGGAGGATTTGGTTGTCTTCAAATA
TATGAATGGCTGTGATGGGAAAAAAGGATTAAATGTGTCCTGTTGTCACAAGAGGATGAAATAAGGAGCGGTGAATGGAAGCCCC
AGGCGATAGAGGTTTCAGTTGCTGCTGACAGTCAGGGCTGTCTGAGGATGGAATGTATTGCCTCAGGACAAAGTGAGTTCATTGTC
ACTGAATTGGTTCAAAAATGACCAGGAAGACTACAGGGCAGGGAAGCTAACTTGTATTACAATAAGTAATTTTTTTCTTTTTTATT
TGTATGCCCTTAGAAAGATGTTTACTTAACTTGATAAATCTGTAACAAGGGATAATCAAGATTTGGTTATAGGACAATTACAATTT
TCTGACAGATCCCCCAAAATAATAGAAATTAATAGCTTACACACTACTTTATCCAAGCTGTAGAATGTATAACTTACTTCATATAC
TTTGTGTTTAGTCAGTTTATACAGGCTGAACAACATAAAGTTAAGACATAAAAGGGTTTTTTTTGCAGGTTTAAAGTACTACCATT
TTTCCAAATTACTGGCTGATCCTAGAACTTTGAGAAACTTTGGTGTTTCCCCCCTTCCTACTTTTGTTTACATCATAGTACGTAGA
TGGTACAATATTTTGGTTAACCTCAGTCTGACACTTAGATTGTGTTCTCTTTTGCCCTATTAATGTCTAGAGTGTAAAAAGACCAG
TAAAGTAGCACCAAGAAATCAAAGAAAATTTCCCAGTTCTACTCACTTGTAATTCTCTTAATTTAGTAGGGGCTTTCCACAGGAAA
TCACAATAGAATAGTTGAAATGCTCTTTATCTTATGTACTTTCCTTCTGAAGAAAGGGACAGTTAATTGGCTTTGTTCCCAACATG
GTCCAAATTTTTATACAGGGCAGCTGTATTTACTGTAGTACAAAGGAGATTCTACACCGAATGATGTCTCTAAGTATTTTCAATAA
TTAAGAACCTAATTGCTTCTCTAGCCAGTGGGTTTCAGTGAAAGTGATAAAATAGATTTTCTAAGCAAGAAAAATACATAATGAGG
GTGGTTGTATACCTTTAAGAAAAAAGAAATCAGTGATATTCAATCCCAAGTAAATTTGGATATCACAGAGTCAGAGATATTCTTAA
TTATTTTAAATTAAATTTGAGTCAAAAACAATGTGTTTAAAGACTTTAAAATCATTTTAAAAGAATGTTAGAAATTTACATTAATA
ATTTCTATTTTCAATGAATAAATGAGAAACCTAGAATTTTTGTTTTGGACACACTTTGATCATATCTAATGTGTTCTACTTCCTCT
AATTATTTTTATGTTATTAGGATTTTTAAAAAACCTCTTAGATTAAAATTTCTCAGTGTATCTTTGTTTGCGTGTCTTTTTTTCTC
TCTCCACTTATGTAATTGAAGCATTTCTGACATATCCTCAGAACACACAGATTCTTATCAGGACTTAACTACCCAGGGCAGGGCCC
TCCTTGGATTTACTGAAAATAAACACTTTGGGTACTCAGTCTCAGTTTTAATGACAAAAGTTGAAATCTTTGCCTAACATGAAGGT
TTTTCTTTGTCTCTAAATTTTGATTTTCCATGCTATCAGAGGTCCCATTGCAACAGGTAATTTGAGTGTTGATTGTATGTTTTCTC
AGTTTGTAATTGTGGTGAAATGTATTTAAAATGTTCACAATTTTAAAGTTAAGTTTACCTATAAGGTTCTTTCCAACTCAGAAATT
CTGTGATGGTAAACTTTTTTTTGTAGTTTGAAAATGTGATCTTTGGATGTTGTCTTCTGTGTCCTATGCATATGACTAGGAATCA
TAGAATTTTAGAGCTGGAAAATGAGTGAGACATTATCTAGTGATTCTGCAAATGAAGAATTAAAAGGCCCAAAGAGGTTAGGTGAC
TCACGCAGTATCATAGCAGTACCTGGTGACTAGAACTCTGGCCACCTACTGCAGCACTAAAAATTAACCCAAATGTTTTTATCTGA
AAATTTGCAGACTTCAGGCTAGGCCTGTAAATAAATAAATAAGAAGTTAAAACATATTTGAGAAAGGTAAGCTTAATATGCCACAT
TTGGGAAAAGATGTGCCTGACACAAATGTATTTGGTGTAATAGAGAACATTCTTCCATTATACCGAAATGAAAATTTTGAACATTC
TCAAAATTCTTGAATTTTGAGACATTTATGTTAAGCACAGTTTATTCAGACTCTCTATAAACTGTGCTGTCATCCCTGTTGTTTTC
TCCAAGCGGAATCATACTTAAATTACAACTTTAGGGAGTTTAATGTTTCCTATTTTTGCACTCCTGGGATATTTTTCAGTGACTCA
GAAGCATAAACTGACACTGTGAAGTTAAGGCTGTGTTCCAGTGGGAATGACAGCATGTCTCTAGGACGCTGATGGAGAAAGCCCTC
CTCATGCAGCTGTCTTGCGGAGTCTCCCTGCTGGTGGTGAATGTGAGATCACACAGGGCCAGCAGCCAGGGCAGTACATCTCTGAG
TCTCGCTTCTGGTTTTACCTAGAGCCAACCCTTGGTGATTGTTTGCCTCCTTGGCCAAGCCCCCACACAATAGGAAAACTGTGTCT
CAAGGTCGAATTCTATTAATACCTGAGAGGTGAATTTATAAACATTTAAGATGTTTTAGCATCTCTGCAGCCAGTGTCTCTGTAAA
TGAAAATTGCTTCTCGAAGTAGAAAAGCATAAGGGAGTACAAGGGCTTCAAAAAAAAATCCTTTTAGGAATAAGAATATTGCCAGG
GTTAGGTGTTCCTAACTGGTTTCAAGCAGAGGAGTTAGTAAAATTCCAAAAGTTAATCAGCTTCATGTACTCAACTTTTGCTAGGA
CTTTGCAGAAACTCCTCAGGGATCCCTCAGAGTTAGATTAGGGAGATGAACCCTGCCAAAGGACAGTAACCATTCAACAGCTGGAT
AGCCTGTTGGGCCCCACTGTCCTCAGCCTTTCCTAATGTTGCCTGGGAACACCACTTTTCTGCTCCCTCCTTTTTCAGTGTAGCCT
GGAGACAATTATTTATTTATTCACTAGCTCTTTTAACAAATATTGATTAAGTAGGTACTGGTTCTGTTCTAGGGGTTGGAAAAACA

GAGAAAATTAAATAGTGCCTTTGTCCTCAGGAAACAATCCATTTAATAGAGAGACGAAGATATGCCCTTAAATACCTGAATCACCA
GCATAACCAGCACCTGCGGCTGCCATGTGGGAATTGTTGAACAAGATAATTTTTGAATGAAAGAGTAAATGACCTAATTGCTCTAA
TGTAAAGATAAATTTCTAAGAGTCGGCTAGTTGGGATGATTATAGAAGGATTCAAGGGAGAGATGTATGAAAGCATAGATCTAACG
AGAGAAAACAGCACAGCAGAAAAAGCAGCAAAGGCTCAAAAGTAGAAATCTCTGAGATCGAATGGAGAATAGTAAATAATTTGCTG
GAATGAAGAGCACGTGGAAGGCAAGGGTGGTAAGAAGGCTGGAGATAGTAGATAAGGCCAAATCATGCAAGGATTTAAACTGTATG
TTTTTAGAAAATGGGTAAGCTATAGAAATTTTATATAGGAATGAGAAATTATCAGCAGCATTGTGCTTTAGGACTACAAATCTGGT
AGCAATCTGCTAACTGATATTGGAAGGGAAAGGCCCAGAGACACTAGTTGGAAGAATTGCAGTGATTCTTCATGAGCTAAACTAAG
AGTTCAAGCTAAGATAGATAAACTAAGGAATGAGAATGGAAGAAAGGGACAAACTGAGATTTTTGGGGTGGTACTCAGCTGAAGTT
TGGCAACTGGTTAAACATAAAAGACAAGAGACAGAGAGGAATCCAAGATGAAAAGACGATTTTGCTGCACTTTTTTAGAAGGCAGC
ATTTGTATTTCCATTTCCAGGAGGTGGAGTAGCTTTGAGAAGAAAGGTGATTAGTTCAGTTTTGGGCACATTGAGCTGTAACGGTA
TTATGGACAGGAAGATACCCTACAAACAGCTGGAAATTTTGCACATGCTTAAAAAGAAGATGAGAACATGAGATTTCAATTTAGCC
ATCATCTGCATGGAGTAGACAATTCAATCCAGGAGACTTGATAAGAATGTCAAGGGAGTGATCACGATAAAGGTGAGAAATGGACT
GAGCATCGATCTCTGCTGAAACATCTCTTTGAGCAGGAAGAAGAGTGCCTTAGCAAAAGCAGCCAGTGAAATGGGGAGTTGGGGGA
GACTTTCAAGGAGGGAATGGTAACATATCCCATTCCCAAAGAGGTCAAGTAGGGTTGAGGAATAATAAATGGCTAGTGGGTTTGGA
AACATGGAATCTGTCATGACCTTTACACACTTTTTCATTTGATGGATCGGGGGATTACTTACATTTGCAAAGGGCCTCAGATTAAG
TGGTAATGGTTCAGCATTAGTAATTAGAGATTTTCTTATGTACTTTGTAGGGAGAAATAGAATGGCATTCAAACAGGTTTAAGTTC
CTCTTAAGAACCTTTCTCATTGGATTGTAGAGAAGAGCAAATGAAAGAATCGTTCAGTATTTCTTGGTATCATGCCCAGCCCATAG
GTTTCAATTAATGTTAACCTTTACAGTTGTCATCATCATTATCCAGAAGGGGCTTTTTTTTAGAATAAGGAAGAAAAGCAGTGCTC
ATCTGTAGACAAGGGAAAAAATCCTTTGGAAGAGAGAAAGAGAAGGGTAATTCAAGAAGATCAGGAATAAACATTAAGGGTAAAAA
CAGAATGGTTAGCCCTGGAAAAGGGAACAAACATGTTTCTCTGAATTAGGAGAGGATATGTGAAGTAACAGGGATGTTTTGAGCCT
CAGTAACAGTAAGGAGAAGAGAGAGTGAAGGAACTTGCACGCAGTGACTTCTACCTTCTCATTAAAGTCAAGAGAGAATCTCAACT
GCAGATATCTGCTTAAGGTAGGAAGAGAGAAATAAGAATTGCTGTCATTTATTTTGTGGTTTTGATATAGCAAAAACTGTACTAGA
CACTTTACCTCCTTTAATCCTCAGAAAACTTAGAGATTATATATTTGTATTCACATTCTACAAATGAGAAAGCAAGTAAAAAAGTT
AAGTAACTCTCCCAGAGTCCCCCAGATGGTCATGGGAGCCCAGATTCAGAGCCAGCTATGTCTTACTCCAAAGCCTGTGCTCTTTA
TTGAAACATACTCCACTAGTTTGAGATCTTGGAGGTGTACCCCTGCACTACTATCAGGACTACCCCTGATAAAGTCCTGTGGGGTA
GTTGTTCCTATCCATCACCACAGTGAATATCAAAGAAGGGCCCTCTCATTTGAGGAATAACAGACAGTGAAATTGAGATGGACCAG
AAATGCAAATCCTGGATCCCTGAGGGAAACAACAAAATGAAGCATAGAGGAAATATCTGAGTCTGGTGCCAAGACTCATCAGTACA
AGAGAGAATGCCTAATGGGCAATAGCAGTTAGGGTGGGAAAAGAGAAGTACAAGGGGGTGTCTTGGAATTTGAGGGAAGAACTGCT
GCCTTAGTTTTCGAAGGATGATTATTCGAGAGTTGCCCTCCCTTCACTTTCTCACAGCTGGCAGAATTCCTACCTCTTAGTGGTTA
GATTCCTTTTTACCAGATACTGTAGGGCCAGTGCAGCAACCAGAGCCCCCAGCCAGAAACCCTGCCTCCAATTTAGACCAGTGTTC
TGCTCTGGTGTGTAATAATCATATTCTAATTTAAATAGCCTATTGAGATGCTACATTGATATCTAGCCTTTTTTTTTTTTTTTTTT
ACTTTTAAGTTTTATGTGCAGGATGTGCAGGTTTGTTACATAGGTAGACGTGTCATGGGGGTTTGTTGTACTGATTATTTCATCAC
TCAGGTATTAAGCCTAATATCTGTTAGTTCTTTTTCCTGGTCCTGTCTGTCCTCCCACCCTCCACCCTTTGATAGGCCCCAGTGTG
TGTTGTTTCCCTCTATGTGGCAATGTGTTCTCATCATTTAGCTCCCACTTATAAGTGAGAATATGCAGTATCTGTAATAATGGGTT
TTCTGTTCTCGTGTTAGTTTGCTAAGGATAATGACCTCCAGCTCCATCCATGTCCCTGCAAAGGACATGATCTTGTTCTTTTTCAT
TTCTGCATAGTATTCAATTGGCCATTTTTTAAAAGACAAAATAACAAAAAAAAGAGTACAGTTATCTCCACTCCTCACCCACTACC
TGCCACCTAACCCAACCCTTTATATTTTAGTAATTGCCTTCCATAAATAACATTTTATCCTGTCTTAATTATGCATTCATTAGTGT
ATTAATTATTGAATGCCCACAATATGACAAGCATTATTTCAAGCACTGAGGATACAGCAGAGAATAAATCAATATCCTCACTGTTT
TTATTCCATTTTTTTAAGAGACAGGGTCTCACCCTGTTGCCCAGGCTGGAGTGCAGTGGTGCGATCATAGCCCAATGCCACCTTGA
ACTTCTGGGCTCAAAGGACACTCCTACCTCAGTCTCCCAAGTAGCCGGAACTACAGGTGCACACTACCACACCCAGCTTCCCCACT
CTGAGCCTCCATCCTAATGGAGAAGAGAGGCAGTAATCAAAATACATGAAATGTGTAGTTATTAGATGGTTATTAATGTATAGAGA
AAAATTAAGCAGAGAAAGGAGCAAGGGATTAGGGGGTCAAAGTTTTGGTAGGGGTAGAGTGGTCAGTGAAGGCCACCTTGAGAGAT
GGCAATTATGTAAAGCCTGAAAGCAGGTAAGAAATATACAGGTATACAGATGTCCCTGGAAAGAGTGTTCTAGGCAGAAGAAATAG
AAAGTGCAAAAGCTAAGATTGCTCACCTGGCAAGTTCAAGAACAGTGAGAAACTGATGTGGCTGCATAGGAGTAAGAAGAAAAAGG
ACGGAGAGGACATGAGATGGTCAGATAGCCTAGGGCCTTGTAAGCCATTATAACGATTTTGTCTTTTAATATAGAGAAATATTAAA
GGTTTTTGTTTTAACTTTTTAACATAGAAAAATGTAATCATATACAAACACAGAATAGTATAATGAGCCCCCTTGCACTAATCACT
CAGCTTTAACAGCTTTCACTTTGGGAGGATTTTGAGTGAAGGAGTGATAAGACATGGCATGGCATGACATGGCTTGAACAGGATCA
CTCAGGAAAATAAGCCATGGGGAGACAGGACAAGCAGAGGAAACACAGGAGCAAAAGCTTAGGAGACAGATGATGGTGGCTGGAAA
CCAAGGGAGTAGCAGTGAAGGTGGTGAGAAGTGATCAGATAGAGGATATTTTTTTGAAGATTTGAATTAATATTATATATATTATA
CTTTAAGAAGAATTTGAAAATGCATGTAGTGTAGTAAAGATGGTAAGAAATACAGAAAAGAGGCCAGGCACAGTGGCTCATGCCA
GTAATCCTAGCACTTTAGGAGGCTTAGGTGGGAAGCTCCCTTGAGGCCATGAGTTCAAGGCCAGCCCGGGCAACATAGTGAGACTC
TGTCTATATGGAAAAAAAAATTAACCAGGTGTGGTGGCATGCACCTGTAGTCCTAGCTACCTAGGAGGCTGAGGCAGGAAGATTGC
CTGAGCCAGGAGGTTGAGTCTTCAGTGAGCCATGATCATACCACTGTACTCCAGCCTGAATGGCAGAGCAAGACCCTGTCCCTTTA
AAAAAAAAAAAAAAGAAAGAAAGAAAAAGAAATATAAGAAATGGCACTAGAATATCAGTATTATATACATTATTTTCTGTTTAAAA
ATCCACACAACTGGCAAAAAAAAGTGACATACATGAACATTTATAAGACAGTGCATTTATAAATTACAAACTCTCTGCTCCTCCCA
TATTATTTTGCAGTGATGAATGATTAGGCCATCCAAACGTAAAGATTTATTTGTAATTTGCTAATGATGGAATGACACCTTTTGAG
GTACAAGTTCCCAGCAATTCTTGGAATTAGCAATAGTGTGTTACTTCTTTCTCGAAAGAAGCATGATTTGTAAGCAGCTATATCTG
ATGTAGTAAAACTGAAAGTTAAAATAAAAAAGGAAACCGTGGGTCAAAGTATGAGGGAAAAGATAAAACTTTCAGAGCAAAGTTAT
ACATAGAGATTTTATTCATCGTCTCTGTGTAACTCACGAGAACTTCTTAGCTTATTTATTATGTCAAATGGCAGTTTGCTCTTCTA
ATCCCAGCCTGATATCATGAGCCATATATGCTGTTGGTCATTCAAAAAGGGCACTAAACAAGGTGAAAGAATGTCAGTGAATCAAT
GCAACTCCATGAGTACTACCAGGAAAAGAAAAACAAAAAAACATGCCATTAATAGTGGGTCAAAGAGGATTTTTTTCATGCCATCC
CCTACCTTACTGAGAATACCACATTTATATTCAGAAGACTCATAATGAATTCCAGAGTCCTCTGTGTCTTGGCTGTATGACTTTGG
GTTATGTCACTTTGTTAATCTGTTTGAACTTCTGTTTTCTTATCTACAAAATAGGGGTAATAGAGTCAACTCTGCCTCCCTCACAG
GGACTTTCGGAGGCCCAAGGGTCTTTACAAATAATAGAGAAGGAAAGAAACTGGCACTTTAATAGTACCTACCATGTACCAGGGAC
TGCGTAGTCCCCACGGCAGCCCTCTGAAGTCATGTGATTGGCCCATTGTGTAGATGTAGAGCTTAAGACTCAGAGAGGTTATTTAA
TCTGTGTAAGGCCACAGAGCTAGGAGGGGGAAAAGCTGGGATTGGATCACTGACTAGATCAACTTGAAATCTCCTGCCTGACAGAG
AAAGAAGAGACTAACTTTACTAAGCATCTTTTTATGCCAGAAACTGTGTTGGACATAATTCTGTACTATCTTATTTGGTAAAGGTC
ATTCTGTCCTTCCTGGTTGCCCTGGTTGCTCAACAAGTAATAAACAATAAAGCCTGACTGTTTTTCATCCCTGAGAGAAGAAATAT
TATTAAAATAGCGTTATTCTCTTTCTTTCTTTCCTTTTTTTTTTTTTTTGTGTGTGTGTGTGTGCGCCTGATAATCTGACTTGCA
ACCTCACAGAAAAGAATGAATGCATGGTTTCATGTCTGTGGCATGACTCTGTGAAGGAGCCTTGCAAGGGGACAGATACACCTCTA
CCACTTCTTATAGCCATTCCTGAGACCTCTCTTCTCACAACACATACCAGCAAAGCCAAACAAACAAGGTGTTTAGGAATAACTGT
CATCTATCAACACTCTCTAGGCAAAGTAGAACTATGAAACACTGAGGAGACTGCACACACACAGAAGAAAAAATGATCATGTCACA
TGGTAGCACAGAGCAATGGGAAATGTGAATAGTTTGTTGAGGTGGACAGACCCAAGCTCAAGGATTGCCTGATCGCTCCATTTATA
GGAACCATGGCAACCTACCTTATCTTACTGAGCCTCAGTTCCTCCAGCTGTGAAATGGGGTACAACCTCTGACCCCACAGGGCTGT
TGTGGGGATTATCTGAGTTAGCATGTTCCATAAAACACAGTAAGTTAGAAAGTATAGCTGTATAGGCTCTGGGAGTTTAACAAGCA
AGAGAAATTTGACAGTAATCATTTAGTTACTGAACCCTGTCTTCATATTGCGCTAGGTTGCATATGGTTTTCCTGCCTGTCCCTTC

CAATCACATTTCAGAAAAAAGGAGGCAGTAGTGGAGCCTTGTGGGCTAAAGTGGTGAGGAGGGTGTCATGGAAGATTCAGGTTCAG
GTCTTCCTCTTGAAGAATGGGTGCTTCTCAATAGGGAAGTGGAGGAACAACCTGGGAGTTGTCAGCTTGAAGAAAATCCTGAGACG
TGATAATGGGTAGGTCCAAGGAGGTCCTGAGTAGGACTCTGAGCACAGAGCCTTTCTTTGCATGTGCTGTTTTTCCTGCCAGCAAG
ACCCTCTAAACCCTTTGGCCTCACCCACCCCCTTGCCTGGTTATACTCCTAGCCTTCTTTCAGATCTCCACCCTCAGTACTCCCTC
AGGAAGCTTTCCCTGACCTCCTGACCCAGTCAAGTGCTTCTTATATATCTCCTTTGTAAGCTCTTCTCAGTTACACTTTTACATTT
ATTTGTGAGATTATTTGATTAGTGTCAGTCCCACTCTTTAATTCTCCACAAGAACAGAGATTGTGTCTGTTCTGCATTCACCATGC
ATCGCTTCATCCCCTAGTTTACTGCAGTGTGTGGAACATAGGACGGGCTTTAAATACTCGCTGAATAAATAGAGAAATGTTGGTTA
TTAGTGAGGTATTACCTAGGTGGGAGAGAGGATTTATATTTGGGATTGCTAATCCCATTAAGTGCCAAGCACTGCATTAACTCATT
TAATCCTAAAAAAAAGTCCTATGAAATCAAGTGTATTATTCCTTTTAACTTTAAAGACAGAGAAACTGAAATCCAGAGATGTTAAC
CATCTTTCCCCGTTGACACAGCTAAGTGGTAACAGTCGGTCTTGAGCCGGGCAGCCTGGCTTCAGACTTCCTAGACTTTACCACAT
TGTTCTGCTGCTTAGTGCAGTAGAGGACAAAGTGAGGTGCACACGTGAAGTCAGGACAGATTTCCGTTTGAAAATTGTGTATTTTC
CCCTCCTCTTCTTCATTACCATTGTCATCATCATCATCATCAATCACTTCACAAGCAAAGCTAAGCAGAAAAGAACTACTTCATGT
ATCACCTCTTACATTCATGTTAACTCTACATTCTGCCAAAGTGATTTGGCTGAATTTACCCAAAGAGCAAAGTGGACATTTCAAAT
CCTGATTCCTAGAAAATTATAGACTACATCCTGTAACCACCTTTGAAGAACCAGCATGTACTTTACCCCAACGTGACAAAATTGTT
TTAAATAAAAAGAGGTAATTTGACTAAGATTGGTATGATCTGAGACCCATGGTTTCTACATCTTATTCAAAAAGCAGATCCAGCAT
GTGGTCATCAGTAGGTATTGAGTCATAAAGCTCCTGGAAGAAAATCTTAAGTTTCATCAAGAATGCCAAGAAGTCCTGGTGTCTC
CAAGAATGATAGCTGTTACTATAATAACATAATTACTACTGGAAAATTAGCAAAGCCCAATAAAATATGGCATGCAAAAAAATATT
TACCCTGGAAATAGAAGTGACCTAAATCAGTCGTTTATTCCAGCCACCTGCTTTTAGATAGTACTTAAAAAAAAAACTTTCAGGAT
GGTCATATTGTTTAAAGTTCTCTAGGTGGAAGAGTACTTTACAGTAATAAATTAATAAATAAGACAAGTTAATGCTTTTAAACTAC
TATTATTTGTTGAGCATTGTTGTAAAGGCTTTACATGCATTAATGTATTTAGTCCTCACAACAACCCTTTGAGATACTATTATCAT
CATCTCCCTTTTACAGATGGGGAAACTGAGCAGTGCCCAAAGTCACACAACCAGGAAATGGGGAACTATCTTCAAGCCCAGCTGGT
TTGCGCCTAAAGTCCATGCTCTTACAGTGTCCCTGGGTAGCTGAGTACTCAGTTTTGTCACCTTTGTCTCCTTGACTTTAACTGAA
AGCTTAACTCTTTTTTTTTTTTTTTTTTTTTAAACGGAGTCTTGCTCTGTCACCCAGGCTGGAGGGGCAGTGTCTTGATCTTGGCT
CACTGCAACCTCCACCTCCTGGGTTCAAGTGATTCTCCTGCCGCCTGCCGAATAGCTGGGATTACAGGTGCACACCACCATGCCCG
GCTAATATTTTGTATTTTTAGTAAAGACAGGGCTTCACCATGTTGGCCAGGCTGGTCTCAAACTCCTGACCTCAGGTGATCTACCC
GTCTCGGCCTCCCAAAGTGCTGGGATTACAGGCATGAGCCACCACGCCCGGCCCGAAAGCTTAACTCTTAATAAGAGCCTGCAATG
TTCAGACGCTTTGAAAAAAACCTAACAGACACAGTTGCTCTCTATAAACTTAATTTTCAAAGGAACCACTATTCAAAGAAGTAAAA
GGGAGACTGAATAAAAAGAACTAAGTTCTCCGTAGAGCAAATGAATTTCTGAATCGATTGCAAACAGTTCTCCGTCATCATGACAT
CCTATGAAGTATGGTCTCCCCCATGTTAGAGTGCCAGTGTCTTCATGTACCATATAACTTGCAAATAGTATCACTGTCTTCTCACA
CTTCAGCTTTCTTCTTTCATCAGTATTTTGGAAAGTCCACAAAACCCAGGGGGCTCCCTGTCTGTTGCCCTGTGGTCTCTGTGAAA
ACCAGATGATGCGATGCTGACTTGGACTCTTTCAAATGGATCCAACAGCACATGAGTCACTGGCAGCATGTTCAGCTCCTTTACCT
CAGGAATTACCCAGTTCCTCATTCTTGCCACCCTTCAGCCGTGAAGTAGTAATATGAAAAATACATGAGTACTTTTTGAGGATACC
TCGGACCTCTTTCCTTCAGTACCAGAGACTAATTGCCTCATAATGGTAACATTGAAGAGCCCAGTTATCTGACGTGATTGGGCTTT
AATTATGGATTCCTTCTGGTGTCCTATTAAATTGGAAAGCCTAGCTGATTTCACCCAAATGAAGCATTACCTCATGCCTTGCTCTC
TTGCATTCCTGTGCTGGTGTCACTTAAAAGTGTAATTACATGGTTTATTTCGTGTTCTTTCACTCTTGATTTATTGCTGAAGAAGC
CCCTGTGGGATATATCATCATAACAAAGACTGATTTCAGATGTCAACACCAAGAAACACAACTGGCATCAGCAATCTATAGAGAGT
ATCTAGGGACAGCAGTCAGCTTTGGAGAAAACCCAGAACTTCTCATTCCCCATCCCACATTTAAGGGCAAAGACTTTCTCCTTGTT
AAAGGGAAAGCACTGAGTTCATAATTAGCTTTATACCCAGAGTTTTTTTTTTTTTAAATGTTATACCTGTTTAAGAAATTTTTTGTG
CTTTGAGGTGGTCAAGATCAAATTTAATTAGCAACCCTCTTCGTGATTGGCTTGCTCTGGGGACCCTGCTCTTTGAGAAGGAGGTT
AGAAGGTTTAGAAGCAAACCTTTGCAGCAGTTCTAGCCAAGTGCATGCATACAGGCCTTGAGGTGGAGCTGCACAGTTGACTGTAG
GGGAGTCTGTCCTTTGTTGACGAGTGAAACTTCTCTCCCTGCAAACCCTCTGGGGAATAATGCCAACTCTGATCAGCTTTTCGACT
CTTGGATGAAATATTGGGTTGGGAAAAAGTCCCTACTAAGAAAATTGTACCAAAACCCTGCTTCAATAAAATATGAATCCATTTAA
TGAAGTTTATAGTAGAAGTAGGCTAGTCGAAAAGGATTCTGTCTCTTTCCAAGTATTATTGGCAGGTTTAGGGTCTTCCTCAAGTT
TATATCATTAGGACTTCCATCAAAGAGTTATCTTTAGGTGTGATTCCCATCCTCCCTCCCTCTCTCCCTCCCTCCCTCCGTCCCAT
CGTTCCTTCCTCCCTTTCCTCCTTCCTTTTCTACCTTTCTTTTTCTCTCTGTCTCTCTTTCTTTCAAGCTGTGGGCTGCTTGTACA
GATTTATGCATTTTGAATTACTGCATAGTTGAGAGATTTGGGGCTTAGGTTATTCAGTTCTTCAAAAAGTACAGTATGAGCTAAGG
AGATAGGGTGACCAACTAATCTAGGTTTTGGCTCCAAAAACCCTGTGTCCCAGAAAACCCCTCAGTCCTAGGCAAACCAGGATAGT
AGTTCATCTTGTGAGGAGATTATAGTGCATTTCCAAGTATTTAGCTGAATTTATCACAGGGCTCTGACTTGATAGGATTTAAGAGG
GTTAAAATAAGGTCTTTTCTGTTATGTTAGTGTGACTTTAGTCTTGGCACTTTCTCAAGGAAGGAATCCAGTCTACTACAGGGGCT
AGGTGACTAAGGAGAAGAAAGAGGATATCTAAGTGTTTTTTGCTAAAAATGTCATTATCTCCATGCTATTTCCTTTTTATTGCTAT
TGTCATCATTTTAAAGACATACCAATAAGAAATAAATTACTTTAAATAGAAAACTCTAATTTTATCAAGCAACAGCAAACAGGGGT
GAAATCTAAGGATTTGTACTTAAGGAGTACACTTTTTTGAATTGAAGAAACTTACTATGACCTGAGCATATGTAACTCAATTATCA
GAAGTCAGAGAGGGCTACTGAAGGCTCAGGTTTTCTAAGAGATGACTGGAGGCCAAGTCCTCAGGCTTGAGTGTTTATAGCCAAAC
TTCAGAACCAGGTCCCTGGCAAGATTCTCCTGTCTAATCACATGGCACAGAGTTAGGATGGACTGTTTCTGCTTACCTGCCTTCTT
CCTTTGATTTCCATCAACCACCAGTTTCTTTCTCATCAATCATTTACCCAGCTTTTGTGACCAAGAATTTTGATGAGGATGCAGAG
GAAGGCATGTTCTTGGTTAATAGGAAAGGAGGCTTCATGACAACACAGCATCCTCTAAGATGTGTTGCTCCAACTTTCACAATACA
TATTGTGTTTTAAGTGGCCTGCTTCTTGGAGTGTATCTTCCCACTACACAAAACAACCACCATTAACATTTTCATTTACTTCCACC
CTGTCATTTGTTCATCACATACTTTTAAAAACAGGGCTGTAATCATGTATATACATATATGAATAACTTTATATTGTACTTTTTAT
ACTTACTGGCTTATCATAAACATGTTTCCATGTTGCTGGAGAGTCAGATATGCTAACTTTTGGAATGTGCTTCTTATATAGATCTG
CAACTATGTGGGACCAGCAAAGGTTATTGTTCAGTTGGTCACAAATGGAAAAAATATCCACCTGCATGCCCACAGCCTGGTGGGAA
AACACTGTGAGGATGGGATCTGCACTGTAACTGCTGGAGCCAAGGACATGGTGGTCGGGTAAGTAGGGGTATATGATGCTGTGGAA
GGTAGGAACAGATAGAATATGGGATGCAGGAACTTAGAATGAACAGGCCCCTTTCATTAGGGACCTTTCTGAGACCCTCAGATGAC
CTCAAAAAACTGTGTAAACTTGTGCTTTCATTTGGACTCCAGGAAGTAAGGTGACCACATGTCTGGATTTGCTTGAGACAGCCCAG
TTTGCCCCTGACCTAATTGTTTATAGCACCCTCTTCTACTCTTAAAGGTGGCCCAATTTGGATGATCACTTATATGTGGTCACTTG
ACCTACAAGGTATTTGTGCCACTATTAATTTTTTTTCTCCTAGAAATAAAAATAAGGACAAATTTAAAGAAACATAACTATAGCAG
AAATCATCACTTTATGTATATTTTTACATTCTTGATGATTTCTTCAGTGGATTTATGGTGTGAATATGTAGGGCTTTAAGTGAATG
CATAAATATCATTTCAGCTCTCTGCTGCTTAGAAAGTAATGCACAGATAAGAAGCCAAGGAATGCAAATTTATTTTGGCATTCATT
AGGCTTGAAATGTAAATAAAAAATGACGTATTCATTTAAGGGTTGATCAAATAAAACAAGCAATTTTTAATGAATGAAGTTTTATA
GAAGTAATTTCAATCCAAAAGAATTAAGCTTTTAAATAAGACATATTTATTGTGCTAAAAAACTTGTATTTTCATCTTCCTAATAC
ACTAAAATGGCACACAGAATAATAGTGTAGCACACAATACAAACTGATATGTGCAATCAATAATTCTAAAAATTGTTGTTTTAAGT
CTTTCTTTGGAAGTCCTTAAATGCAAAAGAAGAAATTTTTAATTTTGTTTAATTTTTAAAAACATTTTTCAGGGTTTTCTAATCCT
GGTTTAAATAGTTTATGCTGAAATCCCAGTTAATCTGGCTTATTCACTCAAAAGAAAGGAATTGTTTAAATCAACTATAATTAAGC
TGATGGTTGCATTTCAGGCTTTGAATTCCGATCAAGTTTCAAATTGGTTATTATTTTGGAGGTTGTGCTTTCATGTTCCTAGGTAT
TTCTTTGTTTGACTGTGAAAATATTTAAGTGTGAAAAACCTAGAGAGAGAGCTTTCTTTTAAGTCACTAAAATCGGATTCTTTTGA
TGTTAGAAAAAAAAAAGACTCTAGATTGAAGGAAGAAGCTTAAATTGATTTGAAAAATTAAAGTGTGCTTTATATGAACTTGTTTC
AACATCCTCTTGAAAGACTGGCGTGTCTCCTGTTGTATGTCACAGTTTGTTCATCATGTGTTGTGGAATACCCTTCCAGTTCCTGA

TTGACCTAGGAGAGCTTTGAAATGCAGGCTAATTATCACAGTTTTAGACTGATTGATCACTTAGTGTTTCTGCAGATTTATTCCAG
TAGGGTTCTGTCAGTTTGAAGTGTTTATTTTCTTTGGTCTTGTTTAATCCCTATTATAATCATATCCTTTAGAGGAAATTTTAGAT
GAAGTTTTCTACTAGTATTTATTATTATAAAACCTCCAGTATGGTGTCTATAATTTGGTGTTTCTGTGGTTTGCTTTAGTAATCTT
TCAGGATTTTAGAAAGAACTAATGGTTATTTAAATTTGAGACCTTAAAGCGGGAATAGAAGTAAATTGTTTCTAAGGAAAGTGAGG
GATTCTTTCACTCTTGTAGTGCAGTGGGGTGTGGGCTCAGTAGTTGGGAGAACTCATCAGTCATTCACTCATTCAACAAACATTTT
GAGCATCTCAGCTCCTGAATTTGATCATTATGATGTCTGGTTGTAGAGCAGTGAGATTTTCTAACCCTATATAATTTCAGTGAGAA
ATTTAATCAAGATGCCACTTGAGAGTTACAATAGACTCAGTAGTCTTAACGTTTTTAGTGTTTCCTATGAAAAGTCAGTACTCACT
TTCTAATTTGATTTTTAGCTGAGAAATTTTTGCTTACAATGTTTCCCAAAATACACTATTTCTTTCATAATTCATTTGTTTCTATC
TCTCTCTATATATACATTTTTAAGATTTTCAATGTCAAAACTTATTGCATTTATGTAGAAAGAACATCGTGCTAGCATTCTGGAAA
CCAGTATTTTATTCCCAGTACTGTTGCTCTCAACAGAGGACTGGGGGCAAGTTACATGACTTCTTGGGGACCTTAATGTCTTCCCT
CATAAAATGAGAGAGTTTCACTACAGAATCTTATCTTCAGGCTGTTTACATCTCCAGAATTCTGGACTGAACTAACATTACGTAAT
ATTATGAAATAACATGAGCAAACTTGCCACACACATATTTATGGTATGACATTGATTATTAGTATTTAGTTCTGACTTGTAGATAA
GAGTATCACATAGAGAAACTTAGGAAGATAGCCAAAATTATGTCTTAGACTTGTCAATACTAGAAATGAAGCTAAGATAACTTGTT
TTCAATAATACAGATTAGGCAAAGAGTATAGATTCACAAGCAAGACACTAATCCAACATACCAAAAAAGATATCCAAAAAATGGCA
ATGAGAAGACAAATAACTTTTAAATTGTCAAAACCAAAAGAATTACTTTCTTTGGAGTGTTTATCTTCATCTTTCCCTCAGTATGA
ATTCTTAATAAACTTAGTTTCTACTTTCAAAAGGATTTTATCTATGTTATAAATATTCATGTTTTAAATAGCCAAGTCTTGATTCT
GGTTTGATTGCTGTAATGTAGGGGATTTGTCATTTTAATATTTTTTAAGTTAGCTTTAATTTTGTCTTTTTTAAAATTCATTTATA
TAATTAGCCTTTACTGCCTTTCTTGGTAAAAAATAATAATAATAATCTTAGTTTGGTGTTTTATTTTAAACCCTGAATATCAACAA
TTTGTTTCAACATATTTTAGTTCGTTGACATTTTTATTCCATGGAAAATATCTGTGTTCCTTGACTTTGCTTTAGGCCAGTTCACT
TACCAGTAATCACCACACTCTCTCTGCTTTCTGGCAGTTTAGAGACATGGAATTTTTTTCCAATTAATACATATAGCATATACCAA
CTGGTAAGCACTAGGAGTAGATATATAAATATCAAAATACAGAGGAGAAACAAGGCCTTCTTCAGTGTCTGTTGTTCCCCTTTTTG
TCCATGAGTTCTCATCATTTAGCTCCCACTTACAAGTGAGAACATGCAGTATTTGTTTTTCTGTTCCTGCATTAGTTTGCTAAGGA
CAGTGGCCTCTAGCTCCATCCATGTTCCTGCAAAACATATGATCTTATTCTTTTTTATGGCTGCATAGTATTCCATGGTATATATG
TACCACATTTTCTTTATTTATTTATGTTATTGATAGGGAATTTAGGTTGATTCCATGTCTTTGCTATTGTGGATACTGCCGCAGTG
AACATTAACATGCATGTGTCTTTATGGTAGAATGATTTATATTCCTTTGGTTGTGTACCCAACAGTGGGATTGCTGGGTCAAATGG
TAGTTCTATTTTTAGCTCTTTGAAAAATCACCACACTGCTACAAAATCAATGTGCAAAAATCACAAGCATTCCTATACACCAATAA
CAGACAAACAGAGAGCCAAATCATGAGTGAACTCCCATTCACAATTGCTTCAAAGAGAATAAAATACCTAGGAATCCAACTTACAG
GGGATGTGAAGGACCTCTTCAAGGAGAACTACAAACCCCTGCTTAATGAAATAAAAGAGGACACAAACTAATGGAAGAACATTCCA
TGCTCATGGATAGGAAGAATCAATATCGTGAAAATGGCCATACTGCCCAAGGTAATTTATAGATTCAATGCCATCCCCATCAAGCT
ACCAATGACTTTCTTCACAGAATTGGAAAAAACTACTTTCAAGTTCATATGGAACCAAAAAAGAGCCTGCATTGCCAAGACAATCC
TAAGCCAAAAGAACAAAGCTGGAGGCGTCACACTACCTGACTTCAAACTATACTACAAGGCTACAGTAACCAAAACAGCATGGTAC
TGGTACCAAAACAGAGATATAGACCAATGGAACAGAACAGAGCCCTCAGAAATAATACCGTACATCTACAACTATCTGATCTTTGA
CAAACCTGACAAAAACAAGAAATGGGGAAAGGATTCCTATTTAATAAATGGTGCTGGGAAAACTGGCTAGCCATATGTAGAAAGCT
GAAACTGGATCCCTTCCTTACACCTTATACAAAAATTAATTCAAGATGGATTAAAGACTTAAATGTTATATCTAAAACCATAAAAA
CCCTAGAAGAAGACCTAGGCAATACCATTCAGCACATGGGCATGGGCAAAGACTTCATGACTAAAACACCAAAAGCAATGGCAACC
AAAGCCAAAATTGACAAATGGGATCTAATTAAACTAAAGAGCTTCTCCGCAGCAAAAGAAACTACCATCAGAGTGAACAGGCAACC
TACAGAATGGGAGAAAATTTTTACAATGTATCCATCTGACAAAGGGCTAATATCCAGAATCTATAAAGAACTTAAATAAATTTACA
AGAAAAAATCAAACAACCCCATCAAAAAGTGGGCAAAGGATATGAACAGACACTTCTCAAAAGAAGACATTTATGCAGCCAAAAGA
CACATGAAAAAATGCTCTCATTATCACTGGCCATCAGAGAAATGCCAATCAAAACCACAATGAGATACCATCTCACACCAGTTAGA
ATGGCAATCATTAAAAAGTCGAAACAACAGGTGCTGGAGAGGTTGTGGAGAAATAGGAACACTTTTACACTGTTGGTGGGACTGTA
AACTAGTTCAGCCATTGTGGAAGACAGTGTGGCGATTCCTCAAGGATCTAGAACTAGAAATGCCATTTGACCCAGCCATCCCATTA
CTGGGTATATACCCAAAGGTTTATAAATCATGCTGCTATAAAGACACATGCACACTTATGTTTATTGCAGCATTATTCACAATAGC
AAAGACTTGGAACCAACCCAAATGTCCATCAGTGATAAACTGGATTAAGAAAATGTGGCATATATACATCATGGAATACTATGCAG
CCATAAGAAAGGATGAGCTCATGTCCTTTGTAGGGACGTGGGTGAAGCTGGAAACCATCATTCTGAGCAAACTATCGCAAGGACAG
AAAACCAAACACTGCATGTTCTCACTCATAGGTGGAAATTGAACAATGAGAACACTTGGACACAGGGTGGGGAATATCACACCCCT
GGGCCTGTGGTGGGGTGGGGGGAGGGATAGCATTAGGAGATATACCTAATGTAAATGCTGAGTTACTGGGTGCAGCACACCAACAT
GGCACGTGTATACATATGTAACAAACCTGCACATTGTGCACATGTACCCTAGAACTTAAAGTATAATAAAAAAATAAGCAAGTTTAC
AAGGGCAAAAATAAAACAAAAAAGAAAAAGCACCACACTGCTTCCACAGTGGCTGAACTAATTTGCACTCCCACCAGCAGTATATA
AGTGTACCCTCTTCTCCACAGCCGTGCCAGCATCTGTTATCTTTTGACTTTTTAATAAAAGCCATTCTGACAGGTGTGAGATCATA
TCTCATTGTGTTTTAATTTGCGTTTCTCTAGTGAGCTTTTTCCATATGTTTGTTGGTGGCATGTGTGTCTTCTCTTGAAAAGTATC
TAAAACAGTCACTTATCTTTTAAAGAAACTTTTTAAATCAGAAAAAAGGTGTTTATATTTAACCACGTATTTATCATTTGCAGTGC
TGTTCATTCTGTTTCTTAGGTCAAAATTTCTATCTGGTATCATTTTCTTCTGCCTCAGGCACTTCCTTTTATTATACTGCTGATCT
GATGCTGATTAATTCTTTCAGTAGGTGTATGTTTTCATAGCTTTTTATTTTATCTTTGTTTTTCAAAGATATTTTGAAGAGTATAG
AATTTTAGGTAGACAGGCCGGGCGCAGTGGCTCACGCCTGAAATCCCAGCACTTTGGAAGGTCGAGGCGGGCAGGTCACCTGAGGT
CAGGAGTTCAAGACCAACCTGACCAACATGGAGAAACCCCGTCTCTACTAAAAATACAAAATTAGCCAGGCATGGTGGTGCATGCC
TGTAATCCCAGCTACTCGAGAGGCCGAGGCAGGAGAATCACTTGAACCTGGGAGGCAGAGGTTGCGGTGAGCCGAGATCGCACAAT
TGCACTCCGGCCTGCGCAACAAGAAAGAAACTCCGTCTCAAAAAAAAAGAATTATAGGTTGACAGTATTATTCTTTCACATCCTTA
AACTATGTTGTTCCACTGTCTTCTGATTTGCCTTGTTTCCAAGAAGTCACCTGTCAATCTAATCTTTGTTCCTCTGTATATAATTT
TTTTTTCTCTCTAGCAGCTTTTCAGATTTTCTCTTCCTCACTCGTTTTAAGCAATTTGATTATATGGATATTAGCATAGTTTCCTT
CATGTTGCTTGTGCTTGGGGTTCATCGAGATCCTTAGATCTCTGGGTTTATATATTTAGTACGTTTTAAAACTTTTTGGCCATTAT
TTTTTCAAATATATATTTTCTGTCCACTCCTGTTCATCTTCTTCTGGAACGCCAGTTGCACATATATTTGGCTATGTGAAATTTCCTA
CAGCTCACTGATGACCTGTTTTTTAAAAAATCTTTTTTTTCTCTTTCATTTTGGAAAGTTTTATTACTGTGTCTGCACGTTCACCA
ATATTTTTGTCTGTAGTGTCTAATATGTTCTTAATTCCATCTAGTGTATTTTTTTCCTCTTAGACATTGTAATTTGCAATTTCTATA
GATTTGTTTGGGGTCTTTTTTTTTCATATCTGCCATGTTACTCCTTAACATACCAATGCTTTCTTCTTTTTCTGATCATATGGAAT
ATATAATAGCTATTCAATGTACTTGTGTACAAATTCTGTCTTCTGGATCTATTTTTGTTTAGTTTTTGTCCTAATTATGAATTATA
TTTTTCTATTTCTTTCCATGCCTGTTAGTTTTTTATTGGATGACAGGTATTTTGAATTTTGTATCGTTAAGTCTTGGATTCCTTTT
TTTTTTTTTTTTTTTTGATGTGCTTTTAAATACGTTTGAGGATTGGGATGAAGTTAAATTTGGGAACAGTTTGATTTTTTTGATTC
TTTCTAAACTTACTTTTAAGCTTTATCAGAGAGACCAGAGAAACCTTTACTCTAGGGTTAATTTGACATCATTGCTAAGGCAGTAC
CTTTCTGAATTTTCAACCTGATGCTCCATGTATTACAAGATTTCTTGAACTATTCCAGCCCCATATGTGTTACAATAATTTTTCTG
CCTCCACCTCTGTGGTGGTTCTTTTCCCAGCTTTGACATATTTCCTCACACACAGCACTCAGCTGAAGACTCCAGTGATCTCTGAG
TGTATCTCTGTTTGCGGTTTCTTCCTTTCCGGTACTCTGCTTGTGAGTTTTAGCCTCCTTGGCTTCCTCCAATATTTAACTGTGTC
TCCTCAACCTCAGAGATTGCCAGGCTCTGTTGGGGTTCCTCCTTCCTGTGCTGCAGCCTGGGACTCTTTAGGCATTAAGCCAGAGT
AATTACAGGGTTCACCTTATTTATTTCCCTTTTCTTAAAGATTACTGTTCTGTGCTGCCTGTTTTCTAGTGTCTAAAAACCATTTC
TTCATGTATTTTAGATATTTAAGGTTCAAACCAGTCTGTTTTACTCTATCGTTACCTGAAGCAGAAGACTGACTTCTGTACTGTTT
CATTTGTTAACCAGAGTAAATCCTTCATTATTCACATAATAAATTAATAAGGATGATGTTTTTCTCACAGGGACTAGATTAGGCAA
TATACATGAAAAGCATATTATTGACAGTAAAGTGTAAGATGCTACACAGATGTTTATCATTGCTATTACAAAGGAGATAACCCCGT

TTTCCTGCAGTTAGGGAAGTTCTATATGGGAGTAAGGCTGAAAGGGCCAAAAGATATAGGTATTGTTTCTGAAAAACTGCCTATGC
TTCTATGCATATAAGTATGTCATGTTGCATATTTTTCTGTGCTGTATTAATTCATGCATTCCTTTATCAACAGATACTTATTAAAC
ACTCATATGTCAGGCATTGTTCTAGGGACTAGAGATCTCTGCCTTCAAGGAGCTTATTTTCTAGTGGTATATTTTCTGTTCTGTGT
CTTAGCTATCCACTTTTTTCATCTGCCTGGACACGTGACTTATTCTGTCTCTGGGCCTCTGGTATGAGTGCTCATTTCATTCTGCC
TTATAACTCCTATTTTCTTCCCTACTTTATCTGACCTTCCTACCTTAGCTTGTTCATTCTTTCCTTCAATCCAGTTGTCATGAAAT
CTCTTTCTTTCCTCTACTAATTTTTTTTTTCTTTCTTTCTTTCTGAGTAAAAGCCAGAGATCTGGCCCCCTGCTTACCTCTCTGAA
TTCTTTACTTACTTCTGACAACTTGCTCATTTCACTCCAGCTACATTGACCTCCTTGCCTTTGTTGTGTTTTGAAAACACCGGCGT
GGTCCTACCGCAGGAATTTTGTACTTACTGTTCCTTTTGCCACATTCATCATTCATATGTTCATGTCTTCCCCATCGCTTCCTTCA
AGTTTTTGCTCAGTTGTCATCTTTTTACTGAGTGTCCTTCCTGACCTCTCCCTACTTTAAAATGTTATGCTTTTTTCTACTACCTC
TCCTGTTCCTCATTCCTACCTTATTTTTCTGGATAACACTTATTGCCTTCTAAATTGTATCGTATAATTTACTTCTTTGTTTCTTC
TCCGTTGTCACACTAGCATATAAATTCAGTGAAGGTAGAGATTCTTTTACTGCTGACAAAAGCATCTAGGACATTTCCTGGCACTG
ATAAGGATCTGCATAAATATTTGTTGAGTGAATGAATCTCCTTGGTAAAGTCCTTTTTTGTTTGCCTGTTATATTTATTGAATAGA
CTTCTTTGTTTCATGTACTTTAATTTCAAAAGTGAATGGCTGGAACATTTTATATATTTTCTTATAAATCATTTTCATTGCTTTTT
AAGCTTATCACATATTTTGTTTTATAAATATGTAGCCTTCGTGAGATAAAAGATTCTCCATCCCTGTTTACGTGTATACTTAGATG
ACAACTCTAATGGTCATAAATAATTCCAACCTTATAGATAACTCAGGAGAAGATGAGATTATAAGTAGACTTTAAATCCCATTCAG
AAACCCAAGGACAATTCAAAAGGAAAATAATCATTCCAAATATAATATTCTTCTTATTCTTAAGAAGTTGTTAGTATTTTGAATTT
TGAATTTTTAATACAGCTCCCATAGAAAATACTTTAAAATGAACCCGGTAAGACTTCCTCATTAAGACATATAGACACATATGCTC
CCACTCCCGTAATCAAATTTGGAAGTCAAATAAGCTCTGAAAACCAAAACATTCTTCCAAGTTTATTGCAGACTCATTTGGTAGTA
AAAACCAATCTGACCTGATGTGTAGCTGTTTATAGTCTTTATTTTTCCATTTGGTGAGTCCAATTATATATTTCGCTGAGGAAATA
CTAACATGTTTGACTACATTGTGTGCCCCAGACCTGCTTAGAGTATTATGTAATATGCAGTATAGGCCATATATTGCCTTTTTAAC
ATCAAAAATACCCTAAATTCTGAAATGCATGTAGCCCCAAGAGATTTGTATAAAGCATTGAGGGCCTGTGTATTTTTTTTTAATTT
ACAAGTGAAGCCAGATTCCCTAGACATTAAGTGCCTAACTTTTGGTTGTTGTTGCTGCTGCTGTTACTGTTTTTTCTCCAGCTTCG
CAAACCTGGGTATACTTCATGTGACAAAGAAAAAAGTATTTGAAACACTGGAAGCACGAATGACAGAGGCGTGTATAAGGGGCTAT
AATCCTGGACTCTTGGTGCACCCTGACCTTGCCTATTTGCAAGCAGAAGGTGGAGGGGACCGGCAGCTGGGAGGTAAGCATCATTT
TCCTGGCCTTGATCCTCCAAGGGGTCCAGGCTTTGGTTTTCATCTGTATGAATTATATGTTCATCTGCATCCCTTCCAGTCTCTAC
CCCACACTGCTGTCACCTTTTCCTTGCTCAGAAACCTTTGATGGCATCCTGCTACTTCTAGGATAAAAACTATAGCTCCATAGCCT
GTCATACAAAGCCCTGCTTCTCTGGCCCCAGCAACTTTCAGCCTCATCTCCAGCCCACCTTGCATCCTCCTCTCGCCATCTCTGCT
CTCTGAATAACTGAGGCATATTTATACCTTTGTATGTGTCTTCCTTTACCAAAAACACCCCACCGTTTCCATTTCACCCTCTCGAA
AACCAATGCAGAAATCAAGACTTTGTCTAAAGAACACCTCTGTTTTACCTTCCCCAACTCACCTTATTATAAGGCATTTTTCTTTC
TTTGGGCTCTGCAGACACATTATAAACTATTATCTTTATCATAAAAATTGTATGATTTTCCATTTTCTTGCCTTTTCTCAAATTTT
TTTTTCAACCCCATTCCCTGTCCCACCACATGCCCCATTTCTACTTTTGTATTTTGTCCTTTACTCTGGCCGTCCAGTCGTCATCT
GATACTCATGTGATTCAAAACCGATTATCAGTTTTCCACTAAATATACTCCATCTTCTGATACCCAGACTCAAAACCTTCCCATCC
TCACTGACTCCCCCTCCTCGCCTGTGCTGCGAGGCTGTGGTCCAGCCATTCCGTGCTCTCTGGCATTCCACACTACATTCCATTTT
CATTGTTCCACTTATAGTAGTCTTCTCCCTGGTCTCTGTCCCTCCCCACTTCTAATCAAGTTTACATCTTGTAGCCAGATAGATAT
TACTGAAGTTCTGTATCTTGTTGCTGCTCCGTGTCTCCGTTTTGCCTACAAAATCCAAATTCTGTAAGTTGGCTTTTTGGATCTCC
CAAGAGCTGGCCTCAGGCTGCATTTCCAATCTATTTCCCGTTTTGCCCCTTCAGGTCAAAGTCTACTACTTGGTAGGAGCACCATG
ATTTCTCACTTTCCTGCCTTGGCACATTCTTTCTATTTTTGCTCTCTCCTCTTCCTGGAATGCCTTTATCCACTCTCTTATATGAA
TGTGTTCAAATTCTGCCACCTTTTCAATATTCAGAGCAAATACCTCTTCTGCTCCAAAGCCCTCTGAACTCTCTCCTTCTTTGAAA
TCCCACATCACTTCATTTGTAGCTCTCTTGTAGTACTTGTCTCTTGAATCACTACCTTTTATGATATTATAGCTTAATTATGACTA
TATCCATCAGCGTGAAAACTCCAGCAGGAGGAAGGAAACAGATGTCCCTGAGTCATTCAGTTCCTTCAGAAGTTGTTTTTGAGTA
CCTACTATGTGCCAGGCTGTCTGCTTGGCACTGTGGAATGAGCTCACTCCCTGTGCCCAAGCAGCTCTTGGAATAATTTGCACACT
TCTCAATACAGCTGGTGCCTAGTAGGGTTATTCAATTCCATTCCATCACATCCTGTGTGCCTTTAACTCTTACTGTAGAGAGGAAA
GGGAAAGGTGGGGGAAGGACAGGAGAGGAGGGAGGCGATCTGATACACGGAAGAGAGTTTTTACTGTAATCCGGAGAAAATGCAGG
TCTTCTTTGATGCCTTTCATATTGGATGACATAAATGAGACTGTTTTTAACACTTTATTAGCAATATGAAGAGTTTCAAAAGAGGA
AAAATGGGTTTTTATTGTAAGTTTACATTATTTGGGCTTTATAAAAGCATGGTCTTTTAAATGTTCACACTTCCCTGGGCATGAAT
GGACTGTGCTGTATGGCCCTAGATCGGGAAAAAGAGCTAATCCGCCAAGCAGCTCTGCAGCAGACCAAGGAGATGGACCTCAGCGT
GGTGCGGCTCATGTTTACAGCTTTTCTTCCGGATAGCACTGGCAGCTTCACAAGGCGCCTGGAACCCGTGGTATCAGACGCCATCT
ATGACAGTAGTGAGTACTTCACTTCCAACAGGGGGCACACCAAGAATAGACTTCCAGCCCTGCCCTGCCATTTACTTGCTAGCTGA
GTCCTGGGGAAGGTAACTTAATCACTTTAAGCCTCAGCTTTATCATCTGTAAAATGTGAATAGGAAAATCTAGCTTGAAAGGTTGA
TGTCCAGGTTAAATGAGGTATTTTAAGAGGGGCTCAATACATGTAATTCTTTTTCCTTTGATGTACATTTTTTAGTCTTTTACATG
AAATGTCACATCTCATCTTATTTATGAATGTCTTGCTATAAAGATATATGGTTGATACTTTTAGAAGGGAGAATAATCCCAATTTT
CTTTGGTGGTGGGGGGACTCTGGAAAGGAGTTTATAAAAGACAACTATTATTCTTACCCATTTTCTTTCCCATATGTTACTAGCTT
TATCAAGAGAGAAACTTGAAGTTAAATGAATGAGTAGCATAGACAGCATGTGACTTCAAAATCCTATTTCAAGCCGGGTTTGGCGT
CATGCCCTGTAGTCTCACCTACTTGGGAGGCTGAGGTGGGAGGATATGGAGTTTCAGCCCAGCATGAGCAACATAATGAGACCCTA
TCTCAAAAAAAAAAAATATATATATATATATGTATATATATATTAATTTATAGTTCCTAGGACTTTTTAAGAGTTTTACAGTAACC
TTCAGCATTTACCATGCAACTTTCTTGTTTGATTATGCATAGGATTGATTAGACAGGTATTTTCAAAAAGCCTGCAAGCATAGTAC
TCAGACTCATTGAAAGTTTATAGAATTTGGCCTGTGTGGAAAACTCTGTGCTCCAAGTACAACAACTAACTGAATTCTCTAATTTA
AACAAGTTTGAAGGGAAGTGAAAGGTTATAAAATGATACAGACTCATACCGCAGAATCACCTTAAAATGCAGCTGTTGGAGAAAAA
AAAAAAGGAAGCTTTATGCTATTACCACAAGAAAGTTTTGCCTCTTCACCACACAGAATCCTAATTTAGTTTGGAAAAAGAAACA
TAACATCCCCATTTTCCTTACCTGTAAAATAAAAGGTAGTAAGTAGTTTAAGAAAACTGTACTTTTCCTAAAGTTTTTAGGACTCT
TGTAAATAGAATAATAGCAGAATCATGGAAACAAATTTGGAATTGAAGTCTTGACCTCATTGAAAGCCCAGAAATCAATTAGCTTG
GTACTTCCCAATACATCAGAAAAGCTCTTTCTCTTTTTGTGTCAGTCTCTCTGTGTTAGAGGGAGAAACAAATACCTGTTCCTTTC
TACCTCATTTGTTTTGGTGATCATAAATGACCCATGCCCAAGAAGTGTTCCGAGAATTTCAGAAGCCATTCTTGTTTTATTTACTT
TGTTAGTGAAAGCATGTATTTAAGCTTTGTTTTGGTCATGTGTGCTAAGGGGAGGGTCCTACCTAAAGGACTGGCTTGTTGAGCTG
AGGATTAATCATGTTATTTGTTGTTTTTCCCCTGTGAACAGAAGCCCCCAATGCATCCAACTTGAAAATTGTAAGAATGGACAGGA
CAGCTGGATGTGTGACTGGAGGGGAGGAAATTTATCTTCTTTGTGACAAAGTTCAGAAAGGTAAATACATTCTGTGATCTCTGATC
TCAAGAGGTGTGATCTTGACACACTACAGTTCTGAGTGTGTCTGTGAGTCACATTTCAGCAGTGGACAAGAAACATCCCTCTGCTG
CCACAGAAAGTCTTAAAAAGCATTTACGTTTTACCTCTTCCAAATGTAAATTGTCTGTGTTATTTTTTCCTAAGTCAACTAAATCA
CTTTTAGATTTCCCATGGAAGTAAAAAGAAATAAGAAAACCGTGATAATTATAATCAGTGACTTTCAGTATCTTTATACATTAAAA
TTAATATCTGTACTTTGTTAAACAAAAATAAATAATAGCCCATTCTTGACACACATACAAACACACACACAGAATAATGTTTTACA
TAGTATTAAATTGTTTTTATTTCTTTTGCAATGCTTTGATATAACACTGAGAGAACTTCTCTGAACACTTCCACAGATGTGATGTT
TAAGAAAAGGAAAGAAAACAGGGAATACAGGGAAATTCTCAGAGCACATTTCTCTCACAATTTTCCCTTTGAGGAAAGGTTCTTGG
CGTTTATCTTTCATTAGATATATTACAACATTGATAGATTAGTAAAAGCTGAGGAAAATGTGTTATTTTCTCAAACTGCATTCTTT
TTTTAAATAAAATGATAGTTGGAAAGTGTAGTATAATATCTATTTACTACAGGGTGAGCATTTTCAAAGTAAGCAGATACCTAACT
GAAAATATAAATAAGTAAAATAAATCAATAAAAGACATGTGGCTTTTAAAAAAATTATCATATGAGATTAAATTCTCTGAGGTATA
TAGTCAGAAAAAAATGGGATCAGATTTTAAAGTAAACACATATATACTTTATCCAAAAAAATTTACAGGCCAGGCATGGTAACTCA

TGCCTGTAATCCCAGCACTTTGGGAGGCTGAGGTGGGAGGATTGCTTGAACCCAGGAGTTTGAGGCTGTAGTGAGCTATTATCATG
CTGCTGTACTCCAGCCTAGATGACAGAGCGAGACCCTGTCTCAAAAAAAAGAAGTTTACACTTTCTCTGCCTACACTAGTGTCAGC
CTTCTTCCAATCCACTCTGTCCAAGTAATACTCATTTCCAGATTCCTGGGTGTACATACTGTGAGTAAACACAATCTGAGTACCTT
TATTTGTAAATGAGATCCTTAGTCATTATCATTACATTGGAATGGCTTAGTAAGAAATGTCTTGTACTTGGCTTAAAGAATCTCAA
TAACTAGGTCCAGTGTAGTCCTACCTGAAGGCAAAGGAGATTGTAAGATGTTTTATCCATTCCTAGTATTTCTAAAATTCTACTTA
CATAGAGATAAAAACTTTGAACAAAGTCAATAGTGTGGCCCTTTCTATGAAACGGAAAGTGAAGCCAGGAAGATGCCAACCTAAGT
TACATTGTCACATTTCTGTAGAAGTGAATAGAGTATTTATAAACATGCTGAACATGGAGTATTTATAAACACACTCCATGACTAGA
GTGTGCAATTTAAGCATTGCATTTTAATTAGAAGGGACCTCATTATTATAGTATTTTGACCAATTACTCAATATGAACTAAGAGAC
TATTATAGTGAGACAGTTTAATAGTGTGTTAAGAGCCTGGACTCTGAAATTATACTGTCTATGTTAAAATCCTGGTTCCTCCAAGT
ACTAGCTGTTCAGCCTTGGATAAGTACTTAACCTCTTTGGACCCCAGTTTCTCTTTTGCAAAGTGAGGATAATAATAGTACCCATC
TACATCACAAGGTTGTGGTGAGGATGAAATCAGTTAATATGTGTATATATGAAGCACTTAGAATAGCATCTGCCATACATTAATAG
TAAAACTTATATAAGTTAATTATTTTTATGTACATCTATATATTGAATTGCACTGCCTCGTTGGATTCTAATATTCTTCCACCATC
GTTCGTGTTTCTCAGGATTGGCTTAAGGATGGAGAAGCAGTAAGGTCAAAGATTAAAAGAAAATTCAAGTTAACTAGGTAAATCTA
GTATTCTGTCATTGTGAATGAAAGTTGGGGCGCATTACTGTGGTAAATAATGAAAAAAGTCAGTGATTTGTGAAGATTTTAAAAGA
CTGAACCTTTTGATCTTGTTTTTTAAAAGTGTAAATAGATAGTAGGTAGAATATTAAACCAGTTTTATTTTTCAGCATGTTTATAA
ATACTATTTACACTATGTGAAATTACACACTTCAATGTGATTGTTTGCAGATGACATCCAGATTCGATTTTATGAAGAGGAAGAAA
ATGGTGGAGTCTGGGAAGGATTTGGAGATTTTTCCCCCACAGATGTTCATAGACAAGTAAGTGATTTATTATTATTATTAATCCTT
ATTATTTTTAGAGATGGGATCTCACTCTGACACCCAGGCTGCAGTGCAGTGGTACAATCACAGCTCACTGTATCCCCCAACTGTTG
GGCTTGAGGGATCCTCCTGTCTCAACCTACCAAATATCTGGGACTACAGGCATGCTACCATGCCCAGCTAGTTTCTTCAGTTTTAT
TTTTTTTGTAGAGATGATGTCTTGCCATCTTGCTCAGGCTCGTCTTGAACTCCTGGACTTAAGCGATTATCCCACATTGGCCTCCC
AAAATGCTGGGATTATAGGCATGATCCATATTACTATCATCATTTTATACTTTCTGCCTTATTGAAATTTAGTACTTAGCTTCCAT
CTATAAAAAAGAAAAATCATAAATATTTAACTTCCATAAAACAGTACTATTTTTAAGACTCATGCAGGTCATCCAAAAAAGTTACC
TACTGTAGAGTTATGGGCAAGGTTCTGTTAGATCGTTTGCTCTGAGATGTTGGCAAGATATTTAATTGCTGCCTAATCCCCTAACC
CATAAGACAGTGCCCCACAGGTCCTTTAATGAAAATGTTTTTAAGTGTTCTACCAAAGTAATTATGATGAGACCTATTTTATGATA
GCACCTAATTATGATAACACCTTAAAAGGGTGTTCTAGGAGTCATCTTCTAATTGAAGCTGATGTCATCCTGTTAAATGAAGATTT
CCCAATGGCAACTGATTAAATGATCAGTTTAATCATTTAATAACATGATAATGCTGAGCCCCCACCTTAATTATTATTCTCCTATT
TACAAGTAATTGAAGAGTATTCTCCTATTTACAAGTAATTATCTGGATTGGAAACAATCCAGATATTCATCAGTAAGTGGCCAGTT
AAATTATGGTCTGTCCTTTTGATGAAATATCATGTAGTTATTCAAAAGAATGAGTACTCTGCGAACTAATATAGAAAGATCTCAGT
TATACAGTGTTAAAATATTTTAAAAGATGGGTGTAGAACAAAGTGTGTGTAGAAATGATTCTATGTTCATGCATAAAGAAATTCTG
GAAGGATACCTAAGAAATTAATAACAGTATTTACCACTGGCTGGTACACTGTAGGAACTGCATGGATGGGGAACAAGAATGTCAAA
GAGAATACCACTGTATGCTTTTTTATATTTTATAAGATCTTTATCCCTGTGAATATATTACATAGTCAAAAAATCAAACTGAAAAA
TGCTTAAATCCCTAACATACCACCAAGAACTAATGTGTTATGATGCCAATGTAAGCAATGTTTAGTTTCTTCTCTTCATCACTGGC
CTGTAGCCATCTCTCTCTTTCCATACCCAGTCTTTCTGATGTTGCTCTAAATAATCTTGGAGGCTTTTAAGGCTGCTTTGGAAGAG
AAGAAAGTATATGCAGTGAATTTTATAAGCATGATATTTACAACTAGAAGACATTTGAAATAGACAAATGAAATAATAAAGCTTCG
TAGTACATTAGCATAGCATTGTATTTTGATTTTACAGTAGCAATTTCATAAAAATGTATCGGTATAGAATTCTGAGTTTGGAACTT
TCTCCAGGACACGGTGTTTTTTAGTACACTGTGTCTAAACATTGGGTATAAAGAAAAGCATAAGGAATGTGTTTAATGAGTAGCAT
TACTGCAAAAAAAAAAAAAATGTAGTCCTACACCAACATGTGGTTCTTCGTATCCTGCAGTTTGCCATTGTCTTCAAAACTCCAAA
GTATAAAGATATTAATATTACAAAACCAGCCTCTGTGTTTGTCCAGCTTCGGAGGAAATCTGACTTGGAAACTAGTGAACCAAAAC
CTTTCCTCTACTATCCTGAAATCAAAGGTAAGTCAGTTGTTTAAAATCTTATGCTCATATTTTATTTTATTTTATTTTTGGTTTTT
GTTTTGTTTTGTTTTGTTTTGAGACAGAGTCTGACTCTGTTGCCCAGGCTGGAGTGCAATGGTGCAATCATAGCTCACTGCAACTT
CGAACTCCTGGCCTCAAACAGTTCTCCTGCCCCAGCATCTCAAATAGCTGGGACTACAGGTGTGCGCCACCATGCCGGGCTAATTT
TTTTTTTTTTTTACTTTTAATAGAAATGAGGACTTCCTACATTGTCCAGGCTGGCCTTAAACTCCTGGCCTTAAGCAGTCCTCCGG
CCTTGGCCTCCCAAAGTGCTGGGATTACAGGCTTGAGCTGCCATGTCTTGCCATTGTGCTTGTTCTGAGAAGAGGACAAGCTAATT
AGAAAAGATCAGTTAGTCACCTCCTTTGAACAGCTTTCTAGTAACAGGTCCCTGGATCCATGGTGCTTATTTTTAGAAGAGACAGT
AGTATATTATTTTGAGGTCATGGAATTAGTCTAGCTTTTTAAAATAATGTTATTTCCACCAAGCTTATATGAGATTTAGACATTGA
GAATTTGTCTTTGAATTTGAGAATCTAACATTTATTGACTAACTCAGAGTTCAAGCAGCTGAGATGAAAAAATCAGGCTTGCTGTT
TAGGAGAATCAGCCAGTTAGTCAACCAGCCTCAGGCGGTGGAACAGAAAACTTGGCCAACATGCACTGTGAAACTATGAAAATGAG
TAAGGCATCATTCTTTCTCTCACAAAAGATGCTTTAAGAAAGGGCACTCATAGGTTTCTTAAATAGTTATAATGTAAATTATGATT
TTAATAAGTACCGAAAGTGAAGCATCCATAAAAATATAAGAGAATGTTGAAGAGAGAGCCCTTGGTCCCTTTATTCTAAAGCAGCC
TCCAAAAAGAAGGCTTCGTCAAGGAGATATCATTTGGCCCTCAGCATTTGGGGTATATTTCAGCAGTTGGGTGTTAAACAGAGAAG
GAGAGGGTTCTCTAGGTTTAAGTTACTTGAGGAGCTTAAAGAGAGAGGTAAACACAGCGTTTATGCAGAGGGAAAAAAGTGTTCTG
AGCGCTCGGATGGACATGAAGGCCAATGTGGGTAAGGAAGGCCAGGGCCAGGCTAAAGAGGGCTGTGAATGGCAGCTTAAGAATTT
TGCATTAATTCTGGAGAGGGATAGCCGGGCCTGCCTAGGTTTGGAATGATGGCTCTGACTTCTTTATGTCACCACTGAGGGTACCA
TTTAGGAGGCAGATGATGAGTCAGAAGAGTAATGCAAGAATCCAAGTGAGAAACAGTGCAGTTAGTAAAGGTCCTAGTGCCAGGAA
TGAAAGCAGATGGTGGACTTAACAGAGAGTGCAGTGATACTGTGATACAGTGATACTGTCAAGAGGGCCTAGAGGTCGGAGGTGTA
GGAGAGACGGGGAGTCATAGATGGGTCTAAGACTCCCTGACCAGGAAGCTTGTGATGTGGTTGACTTGATGAAAGGAACAAGGGAG
CAAAAGTAGATTTGGGTGGGAAAAGAAGGAGCTCAGAGTAAAACATGTTATATTTTAGGTCCCTGTGGGAAAATCAAGTGTAAGTT
CTTAGTAGGCAGATCAAAAGGCAGAACTGGAACTCAAGAGAGGACAGGGTGAGAAATTTAGCATTAGAATCATCTCAAAACGAGAT
ATAACAGGCACCAACTGAGTCATCATTCAAAGGTATTTCTTAACCTAGAGTCCACTGACCCCCAAAGAGTCTGTGGATAGAAATGT
ACTTTGATATCATCAGCTTCTTTTATAATCCTGTTTTGTATTTTACATACTGAAAACCATGATTCTGAGAAGAGATCCATAGAGTT
CACCAGACTGCAGAAGGGGCTGTGGCACAAACAAAATTAGAAACCCCTCAGTTGGAGGGAAGTGAGACTGGGAAGGGAGGTCAGTA
AAATGATTGCCACGGGGCACCCAGATCCTTGCTGAGGTTGGAAACCGCAAAGGTGCAGCAGCAACAAGCCACATGGGTGGGTGTTG
TCTCCTGTGAGAGCAAGATGTGATAGGTAATCTTTGAAGAGGGCTTTCCTTTCCTAGAATTCGTGCTGCCACAGGAGTGTTTAGAA
AACGGTTCTACAAGTTCTTGTTTCTTTTCTGAGGTTGTGGCCTCTCCATTGTTTGGTCAGATTTGTAGTGCACTCTATATCCTGGT
GGTTTGAAAACATTGTCAGCACAATAACTTTTCTTTCTTCACATGAGATTTCATACAAAATCCCAGTGTATGGACATGGTAGGCTT
TGATTAAAATAGGGGTGAAGGCCCTGCCAGCTCGGCCTTCCGAGAGGCTGTCCCACCTCTCCTTACTTCCCTATCAGGAGCTGCT
GGAGGTCTTTTGAATGCTTCTGCCTACACAAAATGATGTGAAAATCACTGCTTTAATTTAAACACCCTTCTTTAAAAGTAGACACA
GAAGAAAAATATGGATAATTTTTTTAGACTTTCTGAGGAAAAAAATAGATTTCTTCTCCAGAAATATGTCTTCAAATAATGCCAGT
TTTTGCTAACACAGAGAATCATGAAGAAAGAAAACCCAGGTTACCTGTATGTGTATGAACCTAGAAACATTCAAAGCTCAGCTTGG
TATCTAGGCTGCCTGTCTCCCTTTCCCAGTGGTATCTATGCCTCAGAAAAGTATGTTATAGTGGGGTATATTCAGGTGATTACTT
TAATGCCTCGTTATCATAGTAGGAACTATCCAATGCAGGGATTAGAGAATGGGGTCACCTGAAAGTTCAAAATTGCACCTGTATGC
TTCATGCACCTACCTTCCAGGCTGTCATTGATGAAAAGCTTCATACAATACAAAGTTTAAGAACTGTCTGGAGAAATACTTACCCA
TTGAGCTCTGAAGGAAAGAATGGACAACATATATATATATGAGGACAACAAATTACTTGATTCTATAGAAACCTTCTGGATTATAA
AAACTCAAGATAATAGGACCAAGGAGAAATAATCTGACCAGGCAATATAACTGGTCAGAAAAGTAGTAGGCCATTTAGCATTAGAG
TAAGTAGATAGAAATGGATCCAAACTGTACAACCCAGGGAAACATTGCATACATTTCTCAGAAGTCATTGAACACACTTTTCCTTA
CCCCCAGTCTCTGT

HUMAN SEQUENCE - mRNA (SEQ ID NO: 29)
GGCCACCGGAGCGGCCCGGCGACGATCGCTGACAGCTTCCCCTGCCCTTCCCGTCGGTCGGGCCGCCAGCCGCCGCAGCCCTCGGC
CTGCACGCAGCCACCGGCCCCGCTCCCGGAGCCCAGCGCCGCCGAGGCCGCAGCCGCCCGGCCAGTAAGGCGGCGCCGCCCGCGGC
CACCGCGGGCCCTGCCGTTCCCTCCGCCGCGCTGCGCCATGGCGCGGCGCTGACTGGCCTGGCCCGGCCCCGCCGCGCTCCCGCTC
GCCCCGACCCGCACTCGGGCCCGCCCGGGCTCCGGCCTGCCGCCGCCTCTTCCTTCTCCAGCCGGCAGGCCCCGCCGCTTAGGAGG
GAGAGCCCACCCGCGCCAGGAGGCCGAACGCGGACTCGCCACCCGGCTTCAGAATGGCAGAAGATGATCCATATTTGGGAAGGCCT
GAACAAATGTTTCATTTGGATCCTTCTTTGACTCATACAATATTTAATCCAGAAGTATTTCAACCACAGATGGCACTGCCAACAGA
TGGCCCATACCTTCAAATATTAGAGCAACCTAAACAGAGAGGATTTCGTTTCCGTTATGTATGTGAAGGCCCATCCCATGGTGGAC
TACCTGGTGCCTCTAGTGAAAAGAACAAGAAGTCTTACCCTCAGGTCAAAATCTGCAACTATGTGGACCAGCAAAGGTTATTGTT
CAGTTGGTCACAAATGGAAAAAATATCCACCTGCATGCCCACAGCCTGGTGGGAAAACACTGTGAGGATGGGATCTGCACTGTAAC
TGCTGGACCCAAGGACATGGTGGTCGGCTTCGCAAACCTGGGTATACTTCATGTGACAAAGAAAAAAGTATTTGAAACACTGGAAG
CACGAATGACAGAGGCGTGTATAAGGGGCTATAATCCTGGACTCTTGGTGCACCCTGACCTTGCCTATTTGCAAGCAGAAGGTGGA
GGGGACCGGCAGCTGGGAGATCGGGAAAAAGAGCTAATCCGCCAAGCAGCTCTGCAGCAGACCAAGGAGATGGACCTCAGCGTGGT
GCGGCTCATGTTTACAGCTTTTCTTCCGGATAGCACTGGCAGCTTCACAAGGCGCCTGGAACCCGTGGTATCAGACGCCATCTATG
ACAGTAAAGCCCCCAATGCATCCAACTTGAAAATTGTAAGAATGGACAGGACAGCTGGATGTGTGACTGGAGGGGAGGAAATTTAT
CTTCTTTGTGACAAAGTTCAGAAAGATGACATCCAGATTCGATTTTATGAAGAGGAAGAAAATGGTGGAGTCTGGGAAGGATTTGG
AGATTTTTCCCCCACAGATGTTCATAGACAATTTGCCATTGTCTTCAAAACTCCAAAGTATAAAGATATTAATATTACAAAACCAG
CCTCTGTGTTTGTCCAGCTTCGGAGGAAATCTGACTTGGAAACTAGTGAACCAAAACCTTTCCTCTACTATCCTGAAATCAAAGAT
AAAGAAGAAGTGCAGAGGAAACGTCAGAAGCTCATGCCCAATTTTTCGGATAGTTTCGGCGGTGGTAGTGGTGCCGGAGCTGGAGG
CGGAGGCATGTTTGGTAGTGGCGGTGGAGGAGGGGGCACTGGAAGTACAGGTCCAGGGTATAGCTTCCCACACTATGGATTTCCTA
CTTATGGTGGGATTACTTTCCATCCTGGAACTACTAAATCTAATGCTGGGATGAAGCATGGAACCATGGACACTGAATCTAAAAAG
GACCCTGAAGGTTGTGACAAAAGTGATGACAAAAACACTGTAAACCTCTTTGGGAAAGTTATTGAAACCACAGAGCAAGATCAGGA
GCCCAGCGAGGCCACCGTTGGGAATGGTGAGGTCACTCTAACGTATGCAACAGGAACAAAAGAAGAGAGTGCTGGAGTTCAGGATA
ACCTCTTTCTAGAGAAGGCTATGCAGCTTGCAAAGAGGCATGCCAATGCCCTTTTCGACTACGCGGTGACAGGAGACGTGAAGATG
CTGCTGGCCGTCCAGCGCCATCTCACTGCTGTGCAGGATGAGAATGGGGACAGTGTCTTACACTTAGCAATCATCCACCTTCATTC
TCAACTTGTGAGGGATCTACTAGAAGTCACATCTGGTTTGATTTCTGATGACATTATCAACATGAGAAATGATCTGTACCAGACGC
CCTTGCACTTGGCAGTGATCACTAAGCAGGAAGATGTGGTGGAGGATTTGCTGAGGGCTGGGGCCGACCTGAGCCTTCTGGACCGC
TTGGGTAACTCTGTTTTGCACCTAGCTGCCAAAGAAGGACATGATAAAGTTCTCAGTATCTTACTCAAGCACAAAAAGGCAGCACT
ACTTCTTGACCACCCCAACGGGGACGGTCTGAATGCCATTCATCTAGCCATGATGAGCAATAGCCTGCCATGTTTGCTGCTGCTGG
TGGCCGCTGGGGCTGACGTCAATGCTCAGGAGCAGAAGTCCGGGCGCACAGCACTGCACCTGGCTGTGGAGCACGACAACATCTCA
TTGGCAGGCTGCCTGCTCCTGGAGGGTGATGCCCATGTGGACAGTACTACCTACGATGGAACCACACCCCTGCATATAGCAGCTGG
GAGAGGGTCCACCAGGCTGGCAGCTCTTCTCAAAGCAGCAGGAGCAGATCCCCTGGTGGAGAACTTTGAGCCTCTCTATGACCTGG
ATGACTCTTGGGAAAATGCAGGAGAGGATGAAGGAGTTGTGCCTGGAACCACGCCTCTAGATATGGCCACCAGCTGGCAGGTATTT
GACATATTAAATGGGAAACCATATGAGCCAGAGTTTACATCTGATGATTTACTAGCACAAGGAGACATGAAACAGCTGGCTGAAGA
TGTGAAGCTGCAGCTGTATAAGTTACTAGAAATTCCTGATCCAGACAAAAACTGGGCTACTCTGGCGCAGAAATTAGGTCTGGGGA
TACTTAATAATGCCTTCCGGCTGAGTCCTGCTCCTTCCAAAACACTTATGGACAACTATGAGGTCTCTGGGGGTACAGTCAGAGAG
CTGGTGGAGGCCCTGAGACAAATGGGCTACACCGAAGCAATTGAAGTGATCCAGGCAGCCTCCAGCCCAGTGAAGACCACCTCTCA
GGCCCACTCGCTGCCTCTCTCGCCTGCCTCCACAAGGCAGCAAATAGACGAGCTCCGAGACAGTGACAGTGTCTGCGACACGGGCG
TGGAGACATCCTTCCGCAAACTCAGCTTTACCGAGTCTCTGACCAGTGGTGCCTCACTGCTAACTCTCAACAAAATGCCCCATGAT
TATGGGCAGGAAGGACCTCTAGAAGGCAAAATTTAGCCTGCTGACAATTTCCCACACCGTGTAAACCAAAGCCCTAAAATTCCACT
GCGTTGTCCACAAGACAGAAGCTGAAGTGCATCCAAAGGTGCTCAGAGAGCCGGCCCGCCTGAATCATTCTCGATTTAACTCGAGA
CCTTTTCAACTTGGCTTCCTTTCTTGGTTCATAAATGAATTTTAGTTTGGTTCACTTACAGATAGTATCTAGCAATCACAACACTG
GCTGAGCGGATGCATCTGGGGATGAGGTTGCTTACTAAGCTTTGCCAGCTGCTGCTGGATCACAGCTGCTTTCTGTTGTCATTGCT
GTTGTCCCTCTGC

HUMAN SEQUENCE - CODING (SEQ ID NO: 30)
ATGGCAGAAGATGATCCATATTTGGGAAGGCCTGAACAAATGTTTCATTTGGATCCTTCTTTGACTCATACAATATTTAATCCAGA
AGTATTTCAACCACAGATGGCACTGCCAACAGATGGCCCATACCTTCAAATATTAGAGCAACCTAAACAGAGAGGATTTCGTTTCC
GTTATGTATGTGAAGGCCCATCCCATGGTGGACTACCTGGTGCCTCTAGTGAAAAGAACAAGAAGTCTTACCCTCAGGTCAAAATC
TGCAACTATGTGGACCAGCAAAGGTTATTGTTCAGTTGGTCACAAATGGAAAAAATATCCACCTGCATGCCCACAGCCTGGTGGG
AAAACACTGTGAGGATGGGATCTGCACTGTAACTGCTGGACCCAAGGACATGGTGGTCGGCTTCGCAAACCTGGGTATACTTCATG
TGACAAAGAAAAAAGTATTTGAAACACTGGAAGCACGAATGACAGAGGCGTGTATAAGGGGCTATAATCCTGGACTCTTGGTGCAC
CCTGACCTTGCCTATTTGCAAGCAGAAGGTGGAGGGGACCGGCAGCTGGGAGATCGGGAAAAAGAGCTAATCCGCCAAGCAGCTCT
GCAGCAGACCAAGGAGATGGACCTCAGCGTGGTGCGGCTCATGTTTACAGCTTTTCTTCCGGATAGCACTGGCAGCTTCACAAGGC
GCCTGGAACCCGTGGTATCAGACGCCATCTATGACAGTAAAGCCCCCAATGCATCCAACTTGAAAATTGTAAGAATGGACAGGACA
GCTGGATGTGTGACTGGAGGGGAGGAAATTTATCTTCTTTGTGACAAAGTTCAGAAAGATGACATCCAGATTCGATTTTATGAAGA
GGAAGAAAATGGTGGAGTCTGGGAAGGATTTGGAGATTTTTCCCCCACAGATGTTCATAGACAATTTGCCATTGTCTTCAAAACTC
CAAAGTATAAAGATATTAATATTACAAAACCAGCCTCTGTGTTTGTCCAGCTTCGGAGGAAATCTGACTTGGAAACTAGTGAACCA
AAACCTTTCCTCTACTATCCTGAAATCAAAGATAAAGAAGAAGTGCAGAGGAAACGTCAGAAGCTCATGCCCAATTTTTCGGATAG
TTTCGGCGGTGGTAGTGGTGCCGGAGCTGGAGGCGGAGGCATGTTTGGTAGTGGCGGTGGAGGAGGGGGCACTGGAAGTACAGGTC
CAGGGTATAGCTTCCCACACTATGGATTTCCTACTTATGGTGGGATTACTTTCCATCCTGGAACTACTAAATCTAATGCTGGGATG
AAGCATGGAACCATGGACACTGAATCTAAAAAGGACCCTGAAGGTTGTGACAAAAGTGATGACAAAAACACTGTAAACCTCTTTGG
GAAAGTTATTGAAACCACAGAGCAAGATCAGGAGCCCAGCGAGGCCACCGTTGGGAATGGTGAGGTCACTCTAACGTATGCAACAG
GAACAAAAGAAGAGAGTGCTGGAGTTCAGGATAACCTCTTTCTAGAGAAGGCTATGCAGCTTGCAAAGAGGCATGCCAATGCCCTT
TTCGACTACGCGGTGACAGGAGACGTGAAGATGCTGCTGGCCGTCCAGCGCCATCTCACTGCTGTGCAGGATGAGAATGGGGACAG
TGTCTTACACTTAGCAATCATCCACCTTCATTCTCAACTTGTGAGGGATCTACTAGAAGTCACATCTGGTTTGATTTCTGATGACA
TTATCAACATGAGAAATGATCTGTACCAGACGCCCTTGCACTTGGCAGTGATCACTAAGCAGGAAGATGTGGTGGAGGATTTGCTG
AGGGCTGGGGCCGACCTGAGCCTTCTGGACCGCTTGGGTAACTCTGTTTTGCACCTAGCTGCCAAAGAAGGACATGATAAAGTTCT
CAGTATCTTACTCAAGCACAAAAAGGCAGCACTACTTCTTGACCACCCCAACGGGGACGGTCTGAATGCCATTCATCTAGCCATGA
TGAGCAATAGCCTGCCATGTTTGCTGCTGCTGGTGGCCGCTGGGGCTGACGTCAATGCTCAGGAGCAGAAGTCCGGGCGCACAGCA
CTGCACCTGGCTGTGGAGCACGACAACATCTCATTGGCAGGCTGCCTGCTCCTGGAGGGTGATGCCCATGTGGACAGTACTACCTA
CGATGGAACCACACCCCTGCATATAGCAGCTGGGAGAGGGTCCACCAGGCTGGCAGCTCTTCTCAAAGCAGCAGGAGCAGATCCCC
TGGTGGAGAACTTTGAGCCTCTCTATGACCTGGATGACTCTTGGGAAAATGCAGGAGAGGATGAAGGAGTTGTGCCTGGAACCACG
CCTCTAGATATGGCCACCAGCTGGCAGGTATTTGACATATTAAATGGGAAACCATATGAGCCAGAGTTTACATCTGATGATTTACT
AGCACAAGGAGACATGAAACAGCTGGCTGAAGATGTGAAGCTGCAGCTGTATAAGTTACTAGAAATTCCTGATCCAGACAAAAACT

GGGCTACTCTGGCGCAGAAATTAGGTCTGGGGATACTTAATAATGCCTTCCGGCTGAGTCCTGCTCCTTCCAAAACACTTATGGAC
AACTATGAGGTCTCTGGGGGTACAGTCAGAGAGCTGGTGGAGGCCCTGAGACAAATGGGCTACACCGAAGCAATTGAAGTGATCCA
GGCAGCCTCCAGCCCAGTGAAGACCACCTCTCAGGCCCACTCGCTGCCTCTCTCGCCTGCCTCCACAAGGCAGCAAATAGACGAGC
TCCGAGACAGTGACAGTGTCTGCGACACGGGCGTGGAGACATCCTTCCGCAAACTCAGCTTTACCGAGTCTCTGACCAGTGGTGCC
TCACTGCTAACTCTCAACAAAATGCCCCATGATTATGGGCAGGAAGGACCTCTAGAAGGCAAAATTTAG

Table 6 (human PVT1)

Human transcript (SEQ ID NO: 31)

Human genomic (SEQ ID NO: 32)

Human protein (SEQ ID NO: 33)

Human PVT1 Genomic (SEQ ID NO: 32)

```
GAGTTGTCAGCAACCTAGAGCCCTGAATGACTGCCTGGAAGAGCTGCCTCACCACCTGAGCATCTGTGTTAGACCAGCGGGACATT
CGCTAAAGCAATTAAAACATATTTTATTCAATACTACTCACAGTCAGGGAAAGACCTGAGCTCCATTCTAATTTGTGCCCAGGTAT
CTGGGCATTTTAAAGGGAGAAGGAGGGATGGGGATGGCAGGGCTTGAAATGAGTTAGGGAAGTGAAAAATTACAAAGAGCAGGAA
GGGAAAAGGGCTTGGCCCATGTGACTGAGACTAGCACAGGACCCTATCTTCAGGTGTTGGCTGGAACAAACAGTAAGTTCTTTGAA
TTTTGTAGGCAGGCACTTTCAGGGTGGGCTCAAATCGTGCTGGGGAGGCATCCTCGTGCTGTTAGAACCATGTTAGTGTGTGTCCA
AGGCTTTTTTTTTTTCTTTTTTTCAGAGACAGGGTCTCACTCTGTTGCCCAGGCTGGAGTGCAGTGGTGCAATCACAGCTCACTAC
AGCCTTGAATTCTTAAGCTTAAGTGATCCTCCCACCTCAACCTCCTGAGTACCTGACACCACAAGTGCACACCACCATGTCTGACT
AGTTTTTTTAAAAATATTTTTGTGAGATGGGGTCTCCCTCTGTTTCTTGGGCTGGTCTTGAACTGCTGGGCTCAAGTAATCCTACT
TCCTCGGCCTTCCAAAGTGCTGGGATTACAGGCGTGCACCACCATGCCCAGCCTGTCCAAGTCTTTTTAGGCTGAGGTTAAGACCT
GGTAAGAGATTGGTCAAGAAGAGAATCTTTGCTATCACCCTATTGGGCTTATTCCATTAGTTTACAAATGAAGCCCAGGAAATACT
AATGTAGATGGGTCCCAGCAAGAATTTAAGTACTGGTAATTCTTAACAAATGTTTATGAAGAGTTTATTATGTACCAAACTCCCTA
CCACACCTACATCCACAATCTTCACACCTACATCCACAATCTTCACACCTACATCCACAATACCACATCTACATCCACAATCTTCA
TTTGCTCCTCTATTTGCAAGATCTTGTCTTTACAAAAAAAATTTTAAAAATCATTAGCCAGGTATGGTGGTGTGTTCCTGTGGTCC
CAGCTACTCAGGAGGTTGAGGTGGGAGCATCGCTTGAGCCCAGGAGTTTGAGGCTGCAGTGAGCTAGGATTGCACTATGCACTCCA
GTCCGGGTGACAGACTGAGACCCTGTCTCAAAATATAATAATAAATTAAACAAACAAACAAACAAACAAACAAACAAATAAGTAAT
CTGGCCGACGCAGATACTGAACGTTGGTTAACACAATCCCAATTCCTAATCCCTTTCCTCCTTGACTGCTTTTGCTACAGAGGTTG
GAAAACTCCAACACTTGCTATCCCAGCTTTACTTGCAGCTAGCAGAGGCCATATGAGAGCTCTAGCCAATGAGGCTGAATGTTTTA
CCACGGGTTGGTTGGAAGGTTTTTGCTTTTTCTGGCAAACAGGACATACTTGGTTTAGGCTGCTTCTTTTACTTATTCCTGCTTTA
ACCATGGATTTGATGCCCAAGACCAAGAATATGCTGCTCTGACAATACTAAGCTCAAACAACAAAGAGTATTGTTTTTTACATAAG
AAGAGGAAAAATGATCTCTTCTTTTATCTCTCACTTACAATTGTCTAAGTTTTCCCAGAAGCTCCCCAATAGCTTTCCTTTCACAT
GCCATTGGCCAGAATTGGATCAGCTGAGCCCATTCCTGAATTGATCACTAAGAAAGGAAGTAGAATCACATGCTAGGCTTAGACTG
ATCACTGGGTAGAATGGATACTGGAGCATCAATCAAAGCATGCCCTATAGCAGGTAAATGTGATCACAAGACAAATAATGACTTTC
TGAATATAAAAACCATAGGAATAAATAACAAATTAAATGACTGGTGAATATGAGCTTATTGACATTAAAGCAAATTTATAAAACCA
CTCATAAACAAAAGGAAAAGGCAAATAAGACATCAGGGATATATTTTTGCAATTTACATGGCAAATAAAGTGTTAATATCTCATAA
GTCACTTCTAAAAATTCACCTTAAGGAAATCATTAAGGTCATATGCAATGATTTAGTCACAAAGATGTTTGTAACAGAACTGTGTT
TAATAACGGAAGTTTGCAAGCAAGATGAATTCCTAGCAATAAGGTACTGGTTAGGTAAAATATGATACAAACCTACAATGGAATGC
AACAGGACTATTGAAAAACAACGTTGCAGAAATATATGTACCGTTATGAAAAGATGATTGTGATGTGTTATTAAGTGGCAAAATCA
GAATACAAATAATGCACATGGTTTGTTTCTATATACCTGAAGAAAAATGTAGGAGTATGTGTGTGTGTATTTGTGTGTAATTATGT
AGAAGGATGTGTATTCCAGAGTTAACAATGGCCATTTCTGATGGATTTGTTGACTTTTCTTTTTCTTTCTTTCTTTTTTTTTTTT
TTTTTGAGACAGAGTCTCGCTCTGTCACCCAGGCTGGAGTGCAATGGCACAATCTCGGCTCATTGCAACCTCCGCCTCCCAGGTTC
AAGCGATTCTCCTGCCCTCAGTCTCTCAAGTAGCTAGGATTATAGGCACCCACCACCACGCCCAGCTAATTTTTGTATTTTTAGTA
GAGATAGGGTTTCACCAAGTTAGTCAGGCTGATCTTGAACTCCTGACCTCAGGCGATCCACCCACCTCAGCCTCCCAAAGTGCTGG
GATTACAAGCACCGCTCCCGGATATATTTGTTGATTTTTTAAACTAGTGAGCATTCAGGTTGCATTTGTAATAAAATAAACAAGTA
AAGGAACTTAATTTAGGGAACATGTATCAAAAAGTAAATGAGCATCTCAACAGCAAAGTGGTCAATGGGCCTAAGGAAAATATGTT
CATAAAATAAAGTACAATGAGCCAATGATCAGGTGTGTAAGAATTTATTCTTGGTAATAAATCAGTATGTAATTAAAACAAGAATG
TAATGTCTCTCTTTTTATTCCCAATCCATTGAACTGGAATGCTTTCCCCGCATGAAAAGAATAGAGCCTGGTTGGGAGTACAGGGA
AATGTACAAGTTCACAGGTAAAACTGTGCTGAAGAACAATTTGTCAATAGGTAGACATTCAAAAATGCCAATTTTCTTTTGTCTGA
GTTATTATTATTATTATTATTATTTTTATTTTTGAGATGGAGTCTCACTCTGCTGCCCAGGCTAGAATGCAGGGGTGCCGTCTCGG
CTCACTGCAACCTCTGCCTCCCAGGTTCATGCAGTTCTCCTGCCTCACCTCCTGAGTAGCTGGGATTACAGGCACAGGCCACCACA
CCAAGCTAATTTTTTTTTCTTTTTTTTTTTTTTTTTAGTAGAGATGGGATTTCACCATGTTGTCCAGGCTGGTCTCAAACTTCTGA
CCTCAGGTGATCAGACTGCCTCGGCCTCCCAAAGCGCTGGGATTATAGGTGTGAGCCACTGCACCTGGCCCTGGTCTAGGCAATTA
TTTTTAAATTATTTTTATTTTTATTTTTTGAGACAGGATCTCACTCTGTCACCCAGGCTGTAGTGCAGTAGTGTGATCATAGCTCA
CTGCAGCCTTGACCTCCTTGGGCTCAGGTGATCCTCCTACTCAGACTCCCCAGTAGCTGGGACTACAGGCTTGCACCAGCATGCCT
GGCTAATTTTTGTACTTTTTGTAGAGATGGGGTTTTGCCATGTTGCCCAGTCTGGTTTCAAAATCCTGGCTCAGGTGATCTTCCCG
CCTCACCTTCTCAAAGTGCTGGGATTACAAGCATGAGCCATCATGCCTGGTCTGATTTTTTTTTTTTTTTTTTTTTTTTTGAGA
CTGAGTCTCTATCTGTTGCCCAGGCTAAAGTGCACTGGTGCGATATCAGCTCACTGCAACCTTCACCTCCCGGGTTCAAGCGATTC
TCCTGCCTCAGCCTCATGAGTAGCTGGGATTACAGGCTTGCACCACCAAGCCCAGATGGTTTTTTGTGTTTTTAGTGGAGACGGGG
TTGCACCATGTTAGCCAGGCTGGTCTTACACTCCTAACCTCAGGAGATCCACCTGCCTCAATCTTACGAAGTGTTGGGATGGTGGG
AGAATAAAAATGATCAATGTGGTTCCAGGAAGCGCTGATTCATTAGGAGCTGGCTTTCTTTGTCTCTGTTGCTTGAAATAAAACTA
GTCTTGGCTTCCTGTCACCTCAGGGGCAGCTGTGCTGTAGAAGACACAGGTGACTTGTTCCTGGTTCTGCCACTTGCTGATTTGCT
TAGACAAGACTTCTTTGGCCTCGGCTTGCCTGTGTGACTTGACAAGTGAGAATTATAACAATGTCCCTGCCCGGGAGCTGTTCAG
AGGCTGAAGAGGAAGACCAGACTCAGTAAAGCCTGGAGGCTGCCAGGTAGGGCTGGGGCCAGCATGACCTGAGTCCTAGCAGGAGA
AGAAGAGGCAGCCTAGAGTCTTCTGTGAAGTGCACATAGAAGAGAGACTGGGGCCAAGCCACAAAAGATAGAATGCACAGCTGGGC
CCTAAAGAAGGATTTTCTAAGAGAAAAGAAAAAGCCAATGAGACAGGACAGCTAGATACTGTGTGGTTAGAGTGAGACAAGAAAAA
TCTGCATCTTCATAAGATAAATCCGGAGAGACTATTGAGAGTCTATTAGTGGCAGAGAATTGAATTCAGAGGGGCCAGTTGTGCTG
CTGCAGAATCCAGAAAAAAAAAGACAGAAGCCGACAAGGCACAGTGGCTCACACCTGTAATACCAGAACTTTGGGAGGCCCAGGTG
GGAGGATTGCTTGAGCCCAGGAGTTTGAGGCTGCAGTGAGCCACAGTTGCACCACTGCACTCCAGCCTAGGGTGACAGAGTGAGAC
TCTGTCCCGAAGAGAGAGAGAGAGAGATTGATTGAAGAGATGGAAGCTTATTCCAGAGCAATGACCGTGAGCATGAAGAAAGGTGG
GTGGGTGAATCTATAGGACTAATCAGTAGACAGACATTGGGAAGAGGAGAACTCAGGTTCATGGCCTCAGCAAGTGGTGGTACCTG
```

TCATAACCACTCTTGAAGCCTTGAAGCCTGTTATGTCAGGCAGAAAGTAGGGGACAAACTTTCAAATCATACATCTACAAAGAACA
TTCGTCATGCACTCCAGCCATCCTGAATTGCTAGCAGTACATCAACCTATGCACATACACACTGTTCCCTTTGCTAAAATGCTCCC
CATTCTCTTGGACAAATGGCAAACTCCAACCTATTTTTTGAGTTTCAGCTCTGGCCCCACCTCCCTGGGGCAGTCTTTTCAGCCTT
TGCCAAATAGATACCTCTCTGTGCTTCCACCACACCTGGGGTTACCCCCTCTCATTCTGTTCTCACAAGATCTAGGATGTCTATCC
CTTCGTTCACTCATTGGTTCTATATGGAGCTACTGATATGTGATTCCTGACCCAATAGAAAATGATGAGGGTGGCTGTTTGTCTGA
CTCAAGCCTTTGTGAAATGACTGATGGTTCTCTAGGCCTTTTTCTGCTGTTCTCTCTGCCTGGAACACTCTAACACTCTATCTGGT
TTTTATGGCTGGCTCCTTCTTTTGTTTTTTTTTTTTTTTTTTTTCCTTTGAGATCGAGCCTCTACCTGTCACCCGGTGGAGTGGAGT
GGTATGATCTCAGCTTACTGCAACCTCCACCTTCCAGGCTCAAGTGATTCTCCAACCTCTGCCTCCCAAGTAGCTGGGACTACAAG
GTGCGTTGCCACCACACTCGGCTAATTTTTGTATTTTTTGTAGAGATGGGGTTTGGCTACGTTGGCCAGGCTGGTCTCGAACTCCT
GGCCTCAAGTGATCTGCCTGCCTTGGGCTTCCAAAGTGCTGGGATTATAGGCATGAGCCACCACACCTGGACGGCTGGCTTCTTCT
TGTTGGTCAAATTATCTAGTATCAATTACCTTCTTGCCCCATTTTCTCTTTAGCTCTCTGCCGAGATTTACTGTCTCAATAGCATT
GACCATTGTTGAAATTACCTTGCTTATTGGCTTGTCTGCTTCTTCTGACTAGAATGTCAACCTCACAAAAGCAGGGACTTGGGCTG
TCTTATTGCACTCATAGTCCTGAGGACTAGCACAGTGCCTGGCACATAAGAGGAATTTGATAATGTTTGATAAAGGAATGAATGGG
TTTTGTAGATAAGGATGCAGATATAGGGCCAGGCATGGTGGTTCATGCCTGTAATCCCAGCACTTTGGGAGGCTGACATGGCCAGA
TTACTTGAGGTCAGGAGTTCAAGACCAGCCTGGTCAACATAGTGAAACCCCTGTCTACTGAAAATACAAAAAGTAGCTGGGTGTGG
TGGCAGGTGCCTGTAATCCCCACTCAGGAGGCTGAGGCAAGAGAATCGCTTGAAGCCAGGAGGCGGAGATTGCAGTGAGCCCAGAT
CGTGCCTGGGTGACAGAGCTAGACTCCATCTCAAAAAAAAAAAAAAAAAAAAAAAAAAAAGAGGATGCAGATATAGAGAAGGTAAAGA
CTGGTACTGAGCCTAAAATTATTTAATTATAGAACCAATACTAAACAACCATGAGAACTGCATTTACAGAATGGAAGTGGTAATCG
AAAGACAATAGCACAACTAGGCTAGCGCAGTGGCTCATGCCTGTAATCTCAACACTTTGGGAGGCCGAGGCGGGTGAATCACTTGA
GCCCGGGAGTAAGCCAGGCATGGTGGCACATACCTACTCAGGAGGCTGAGGTGGAGGACTGCTTGAGCCTGAGAGGTGGAGGCTG
CTGTGAGTCGAGTTTGCACCACTGCTCTTCAGTCTGGATGACAGAGTGAGATCCTGTCTCAAAACAAAAACAAAAACAAAAACAGA
AAACAACGAACAAATACAGGTGAGGTAAAGGGAGATGTGAGAAAATGTGTGAATGTAATAGCAAAAAGTTGCCTGGACTTTACAAT
GATGAGGCCTACAAGTAAAACATCATCATGCCACTCTTTCCTTGCTTTTCATTATCTTTTCTGAACCTTTGTAGCCTTATTTAGAA
AGCAATGCTTCTCATGTGGTAAATAAATATTTAAAACACAACAATTCCTTTAGTTTTATGTTAGCTTCCTTTTCTTCTACTAAAAA
AATTAGATTTCTTAAAAATTGCAACTATGAGACATTAACATTGTAAAATATTTTTCCTATCTATTTGTCAATCCTGCATCCATCTA
TATATCTATATCAAGATCTATGCCTATATGTATCCATCTGTGCCCAAAAAGGCCTCATCTGATATTCAGAAAAATTATTTCTGGCT
GATAAAATTTGTTTTTCTTCTCTATTTTTTTTTCTGCAGAGTTTTAATGTTTTACAACGTGTATGTATGTGTGTGTGTGTATATAT
ATATATAATATCACTTTTGCAAGAAGAAAAGTGACTTTTAAAAAATGAAAGCAGGCCTGGGCACGGTGGCTCACGCCTGTAATCCC
AGCACTTTGGGAAGCTCAAGCGGGTGGATCACGAGGTTAGGAGATCGAGACCATCCTGGCCAACATGGTGAAACCCCGTCTCTATT
AAAATACAAAAAATTAGCAGGGCGTGGTGGCGCACGCCTGTAGTCCCAGCTACATGGGAGGCTGAGGCAGGGGAATCGCTTAAACC
CAGGAGGTGGAGGTTGCAGTGAGCCGAGATTGAGCTGCTGCATTCAAGCCCGGTAACACAGCAAGACTCCATTTAAAAACAAACAA
ACAAACAAACAAACAAACAAAACAAAACAAAAGCAGATATACCAAAATGTTTATTTTGGATGGTAGGAATATTAAAATATTATTTT
TTCTTTGTATTTTTTACTGAAAAAATGTTTTAATTCTAAAAAAGAAATTAATAAAAAGGAAAATGTGAATCTAAGATTAGTGCATGA
GAGCTGGAGACCCAGAGAGGGCAAACAGCATCAAGGTCAAAGTTGAGTGAGTCCTGGGTGAAAGTGTCTTTTCAGCCAGGGGAGAT
TTTCCATGCAGAAGGGACTGGGCATCAACTGTCAGGACATGGACAGAGACGTCTGCCATTAGAAGCTGGTCCTCTCATAGCCCACC
AGCATCCCCCAGGAGGACACTGGCTTCCTCAAGGTCACAGCCTTTGGTTTCTGGAAAAAATGATTAAGCAGTGAAAGGATTAAGGT
CTCACAGCTCCTATCGCATGATGTATAGATGCAGAGGTTTCTCATCATCTTCTTTTTATATTTTAAAAGATGGATGACAACTAGAT
GCCAACTGCAGCTCAGGCACCTGCCCAGTCCCTCTCAGAGCCGGGCACAAAACAGACACCTGTCTAGCCCCTCACAGAGAGCCAGG
CACAAAACAGACACCTGTCCAGCCCCTCACAGAGAGCCAGGCACAAAGCGGAGACTTGTCCAGCTCCTGGCAAGGAGCCAGGCACA
AAAGAGATGCCCATGAGTGTAAATGAATCTGTGAAACAGGCAGAACAGGAGCATCAACTGAGTAATTGTTTTTTCTTTTTCAAATA
AGAACATTTGCGTTGAGAAGCCGCCACGGAAGAGATGTGATGAAACACTACACAGAAAGAGGGAGGAGCCTCCGGGTAGGTTGGA
GTGAGTAGGATGTAGGGTAGCCGGCAGGGGAGAAGAGGGGCAACCTTCTGTTCCATAATATTGATACCACCCATTTGTGTGTCCAA
TTTTTGCTGGTTTGTGACGTCCTTCTAGGGTCCATGAGTAACCCCCACCCCATTGCCTAGCTGGGTGCCTGACTCCCCTTAAGTGC
TCAGTAGACGTTGAACTGGACGGGCGCTGGCGTGGTGGCTCACGCCTGTAATCCCAGCAATTTGGGAGGCCGAAGCGAGTGGATCA
TCTCAGGTTCGGAGTTCGAGACCAGCCTGGCCAACATGGTGAAACCTTGTCTCTGCTAAAATTACAAAAATTAGCTGGGCGTGGTG
GCGCGCGCCTGTGATCCCAGCTACTCCGGAGGCTGAGGCAGGAGAATCGCTTGAACCAGGCAGGCGGAGGTTGCAGTGAGCCATGA
TCGGGCCACTGCACTCCAGCCTGGGTGACAGAGTGAAACTTCATCTCTAAATAAATAAATAAATAAATAAATAAAATAAACTTTGA
ACTGAACAGGGGCAGCTCTTACATCTGTCCAGTCTCGGGTTTATGCAGATAGAACAAGATAACCACATCCCACATGGGAGGCCAGT
GTCCACTTGCTCCTTTGGAGGAACTACTGGGCATTTAAGGGACTCGTTGAACACACTCTGTAGGTCAACTCTGCCCGGAGTGGAGG
GAGCTGCGCAGACCGCTGGCCCTCTGCACGGGTTTGCAACTCGGCTTCTGGAAACAGCGGGAGACGTCGAAGGAGGCGAGAAGGTG
CGCGCGCCCTTCTCACCAGGCCGGCGGATGCTGGGTGGTACCCAAAGAGGCGCTCAGATCACTGGAGCTGGAGCTCAGTCGGCGGT
CTTCTGCGGGAACTGCACCTATCCCGGAGCATCTCTGGCCCTCCTATTTCACTGAACTCGCCTTCCTCAGCAGGAAAGTGGGAAGA
CCGCTTGCTTCCAGGACGGTGTGGGGAGGGTAAGAGGGCTCAGGGAAAGACCGTGGAAAGCTCGGCGCACAAGCGAGCTGGCATCA
CCGGGGATTCCCTCCGGCCCGGTGGACTCCGGCGCGCTTGTAGCGAGGACCCCCAGCCCAGGGCAGAGCCTACCCTCCGCTCCCC
CAGTCTGGGCCCTTGGCGGCCGCAGTCGCCCAAGCCCGCCCCGCCTCCGCGCGCGGCCCTCTCCGGCTCAGTGCCCTGCGCTGCCG
GGAAGCAGGCTGAGGGGCGCACCGGGCGGCGGGCGGGGACCTGGGGAAGGCCGGGAGCGCCGGGACCGAGGACGCACGCGGCGGGC
CGGGCCGCGCGCGCCTCCCCCCTCCCCCGAGGCCCGAGCGCGAGCCGCCGTGACGTCACGGGCAACCCGCCAGCCCCGCGCTCCTC
CGGGCAGAGCGCGTGTGGCGGCCGAGCACATGGGCCCGCGGGCCGGGCGGGCTCGGGGCGGCCGGGACGAGGAGGGGCGACGACGA
GCTGCGAGCAAAGATGTGCCCCGGGACCCCCGGCACCTTCCAGTGGATTTCCTTGCGGAAAGGATGTTGGCGGTCCCTGTGACCTG
TGGAGACACGGCCAGATCTGCCCTCCAGTAAGTTCCAATTTTGTCCCCTGCGTTTCTGGAAACTCTCGAGACTTGGCCCTGAGTAG
GGATGCGCTGTGAGTAGTCGGACGGAGGAAAGGGGCTTTCGGAATCCAATATGTGTGTGCTAGGCGTGCACTGTCCTCATCACCAG
CCTCAAGACTGGTGGCGAGTTTGGGCGTTTGGGGAGGGGGAGGGGAGGGGGATGACACCGAATGGCCGGTGCATAGAGGCAATTCT
GGATTTATGAACTTGATCAAAGGCATATTAACTAATTTCTAAAAATAGACCCCTCTGCAGTCAGAACAGATTTGGAGCCATGATGT
GGTTTGGAGGCCCCTCTGGCGAAACTAGAGGTCTGTGAATGTGTGATCCTCCCTCCATCCTTTGACCATCACTCCCCGCTTCCTCT
CACACCCCCTCAGGGCTGGGCGAAGGTGATTTCCAAAAACTCAGTAACTCCGTGTTTTGAGACTTTCTCAGTTTTTGCATCGCATG
TGAGGGTTTGTTGGGTGCTCCTAAACCCTCATCATGGTCATTCTTCTTGGCTGGGCTGTTGATTTAATTAGGTTTGCCTTCTCTTG
CTAACGTGCTCTTATTTATGCCAGTGTTTGTGGTTCTGGGTGTAGACTTTCAGGAAGCACAGCCATGAAGCTGCATGCGTCTGTGT
GTGGATGCCTCATGCCTTTTCCTGGTGGTTTGCAGGCATGGAAGATTCTTCTGTTCATCTAAGCTCCTGACTTTAGGCCCTTCTAG
AACATTAGAGAGGAGAAGAAATCACGTGCAAGGATTTAGGGACAAGAGGATGAGGTTTTGGTGTTTCCTTTCTGGTATTAGGTTTT
CCAGAGAGAGGCTGTTAGCCTGTCTCTCGCCCACTCCACCACTGTGAATCCTGGCTACTGGGGCTAGTTCCTTGCTAGGAAAAAAA
AACTCTAGAGAAATGTGTTTGTGTGTGGTGGGTGGGCGGGGGCCCTGAGGATGCAGGGACTGTGTCTGCTGTGTTGTCTGTTATAA
GATGTTTCAGACCTATTTTGCATACTGGCAGCGACAAGTTGAGACTTGTTCAACTTGACACAGTCCTGTGGTCATAGCGAATCTTT
CTAAAGCTCTGATCAGTCAAGAAGGGGGTTGTATCAATCCTCAGAACCCTGAGTGGAACTTTCTACAGGATTTATTAGGAGAAAAA
CCTTCCCGGAAGCTGCAGAAGGACAAATACAGAATCCGTGTCTGGGAGAAACCTCGTGGCCTGGTCTCCATTATTTGAGATGAGTT
ACATCTTGGAGGTGAGGACGTGCCTCGTGGTCTAAAGCTTCGGCACAAGGGCCCAACTGGAATTCCACTTACGGGTATGACTGTGG
GGTATATGCTGTACCCATTGAATTCCCAGAGGGATTTGTTAAGTGGTTGTGTTGCTGTTTTGCCTGGTTAAGAGTGGCTTATTCTT

GTTGCCTCATCTATCTTGAGTGCAGAATTTTTGCATAAACGGCTTCTCTTGAAAAATAGAAGTCCGTTTTTGTTTTGTTTTGTTTT
TTTTCTGATTAACTTTCCTTTTCCTCTGTCTTAAACACCTTCCCCCGCCCCTCGCCACCTCCATGCTGTGTTTCTGTGGCTGGAGC
TTTTCTGCACTGGAAAGGAGGAGTTCTCCCCTCTGTTCTGACATATCTGATCCCCACTAGTTTATATGATTGCTGGTCTGAAAACC
TGGTGAATTCTCACTGCCCACATCAAATCAATTCCCTGTTCTTTAGTTGTTTTTTTTTTCCGCCTCCTGAGCGGTAGGCAGGCACT
CTGGCTGGATTCTGGAGCCTGTGTTTGCCATGCCTGGGCCTTCTGCCTGCCCAAGATGGACACGTTTCTCTGGCAGCTTCGTCAAG
AGATCTCCTGGACTATAAGACACCCTGTTCTTTCCTCCTGTTTGTGTGAGTGGCCCTGCATTTGGGCAGAGTATTGGCAGAATATC
TGGGGGAAGTGGAAGACTTTGGATAGTTGCCGGTCCAGGTCGTCTGGAGAGGCAGTGGCAGGCCTGCTGATGGCTGCCCCTTTGCT
ATTTGTTTTATATCAGGTGGCCCTTCTGGATGTGTGGCCTTTTTATTTTGCATTTCCTTTCATAGTTGGCTGCAGACATAAGATTC
TTCTTTTTCTCTTGTTTTGCTTTTTTTTTTTTTTTTTGAGACAGAGTTTCACTCTTGTTGCCCAGGCTGGAGTAAAATGGCTGGA
TTTCAGCTCACTGCAACTTCCGCCTCCCAGGTTCAAGCGATTCTCCTGCCTCAGCCTCCCAAGTAGCTAGGATTACAGGCATGCAT
CACCACACCCAGCTAATTTTGTATTTTTAGTAGAGACGGAGTTTCTCCAGATTGGTCAGGCTGGTCTTGAACTCCCAGCCTCAGGT
GATCCGCCCGCCTTGGCCTCCCAAAATGCTGGGAGGCCTCCCAAGTTGCTGGGATTACAGGCATGGACCACCATGCCTGGCTAATT
TTGTATTTTTAGTAGAGACGGAGTTTCTCCAGATTGGTCAAGCTGGTCTCGAACATCTGACCTCAGGTGATCTGCCCTCCTCGGCC
TCCCAAAGTACTGGGATTACAGGAGCCACAGTACCCGGCCTTGTTTTGCTTTTTATTGTTTCCTGATGCAGGTTTGTGATCTCTTT
GAGGGATCAGGACCACACCCCCCACCCCAATTTATATTCCCAGTGTCCATGGTGGGACCCTGACTGAGGATGGGAACAGAGCAACA
ATCTGGAATCATTTGGACCATTGAGTCCTGTGGACAGTCACTTCAATATCATCCCTAGAGAGTCTCAGAAATTCAAGGCCTTTTGG
AATGCTGCCAAAATGGGTCCTTTGGCAGGAGCCCCCTTTGTCTCTTCTGTTTTCTGACCTCAAATAATCTAAGGAACTAATAACGG
TAATAAATAACAATTAATAATAAGAACAATAATAGTGCATGTATAACATGGACATTGTTTATCACGCAAAGCATGTTCTTCACCTT
CTACTCCCTACAACTCCATGAGGCATTTATAATGAAATGTCTAGCAATGGTAGACTTCTTGTTGTGGGGCAGGGTTGGTTCTAACA
CTTCAAATTATATGATCTCATTTAACTTTCCTATAAAATAGGATGATTATTGTTGTGGTTTTGCAGATGAGGAACCCACAGCCCAG
AGAGGTTAAAAATATCCTCCCAGGTCACACAGCTTGGGAGAGGCAGAGCTGTAGAGATTTGGACCACAGTCTTCGACTTCTTAGTG
TGACTCTCAGAAGTTAGAGGATAATGAGGTTAAGAACAAGCAAAGGGCCGGGCGCGGTGGCTCACACCTGTAATCCCAGCACTTTG
GGAGGCTGAGGCAGGTGGATCACCTGAGGTCAGGAGTTCGAGACCAGCCTGGCCAACATGGTGAAACCCGGTCTCTACTACAAATA
CAAAAAATTAGCCGGGCATGGTGGCGCACACTTGTAATTCCAGCTACTTGGGAGTCTGAGGCAGGAGAATTGCTTGAACCCGGGAG
GCGGAGGTTGCCGGGAGCCGAGATCGCACCATTGTATTCCAGCCTGGGCAACAAGAGCAAAACTCAGTCTCAAAAAAAAAACAACA
AGAAAAGGGTATACCCCTTGGCCTTGAGCAGGAGAACACAGTGTGTCCCCCACCCTCACCGTGCACCTTAGGCACAAACAGAAAGC
TGAACATCTTGCATGAAGATACCTCATGCTCTGCCTGCCGACCTTTTCTGCAGCCACGGAGCAGTCAGAGACAAAGGGGCCAAGAG
GCCTTAGGTGAGGGCTGCCATGCTGGAAAGTTTGGGCTGTGGGTGTGGGCAGGAGTCATGGGTAGAGGTCAGAACCCTTAGTCTTG
GCTGGGTGCAGTAGCTCACACCCGTAATCCCAGCACTTTGGGAGGCTGAGGCCGGTAGATTACCTGAGGTCAGGAGTTCAAGACCA
GCCTGGCCAGCATGGTGAAACCCTGTCTCTACTAAAAATCCAAAAAAAAAAAAAAAAAAAAAAATTAGCCCGGCGTGGTGGCACA
CGCCTGTAGTCCTAGCTACTCGGGAGGCTGAGGTAGGAAGATCTCTTGAACCCGGGAGACGGAGTTTGCAGTGAGCTGAGATTGCC
AGTGCACTCCTGCCTGGGAGATAGAGTAAGACTTTGTCTCAAAAAACAAACAACCGCCTCGGTCTTTGTGCAAAATCTATAATCAT
TTGCAAGAGGCTGCAATCAGGTATAGCACTGGATTTTGTGACCAAAACTAGGTTTCAGGATTTAATTAAAAACAAAAACAATGTCA
GGTCATAAAACACAAGACATATACATGATGTGGAAATGGTCTTATTCATTTTTAATTGCGAAATTCCGAACTATCTGTTTTTTTT
TTCCTTTATCTTTTCCTTTTTGTGTGTGTGGAGTTAGGTGGGGGGGAGGTATGGCATATTGTTTTGTTTATCTGCCTCTTTAT
TATCACTGACTGTGAAATTCTTGAGAACAGGGGCGTCTAAGTGCCCAACACAGCCCTGGTACTTTGTTGAATCAGGTGGCTGTCAA
GAGCTGCTGCTTCAGCCCGGCATGGTGGCTTATGCCTATAATTTTTGCACTTTGAGAGGCTGAGGCAGGAGGATTGCTTGAGCTGA
GAAGTTTGAGACCAGCCTGGGCAGCAGAGTGAGACTCTGTCTCTGTAAAAAATAATAAAATAAAAATTAAAAAATAAAGAAAGAAC
TGCTTTGTCTTCAAGGAAACAAGATACACAGTCAACAGTCATTATCTTTTTAGATATGAGACAGCTTGACATGGTGTGGAAGTGGG
GGGCTTGCCTGTAGGGAAAGTATGCTGATGAAAAAGAAGGAAAACAGACAGTAGCAGGAGGCAGTGGGGATGTGTCAGGGAGCTGA
GGAGTGTAGATCTGGTTACTTTTGGGCAAGTTACTTCTCTAAGCTTCAGTTTCCTCATCTATAAAATGGTTTGCTGTGATGACGAA
AGAGATGATGAACAGAATGTGTCTGGCAGAGGACCTGGGCTCATAAGTGAGCACACAGTAAAAGGTAGCTAATATCATTACTGTTC
CAATTATTGAGCTTTATTACTTCCTGTGGCTGCATGAAGTAGTGGTTTCTGAATAGAAAGGCAACATTCTAGTTGCCTAGATCTTA
AGATGGCAGTGACTTCACTGTAGTTCTCTGCAAGCATCAGAAACAAGCCCTGGCTTATGTAAGTTAGAAACAGATGAGAAAGTAAA
TAAAGCTGCAGAAGTGGGTCTCAGGAAGGACAGGAATCAAAGGGCTGTCCCTCGGATGTCAGACTGCCTTTGCAAGTTACTTCAGG
TCACTGTGCCTCCATTTCCTCAGCTGCTGAGCAGGGGAGGAGAATCACTTCTCCCTCCCTGGGATGGTGCAGAGCACTTAGCAGAG
CACTTAGCACAGAGGAATAAGCCCTTCAGTAAAACATTTGAGTAAAGCAATTATTATTTATTATTCTTCAGGGCTCTTTTTTTTTC
TGGTGCATTAGCCCTAACATTGACTACTTGGTTCAAGAGTGATGGATGCATTCATTTCCTGAGCAGTTGCTGTATGCCTGGCACCG
TTTTAGCTGCTGGCGATCCAGCAGGGAGCAGAAGAGACAAAAATCCCTGCCCTCCTGAAGCTTCTGTCCTAGTGGATGTCACACAG
TAGTGGGGCAGGCTGGTCCCCAGAGACAGCTGGGGGACAGATGGAGGCAGTGCAGGCAAAAGCTAACTCCTGTGCAAAGTCTCCCC
GGCCCCACTGTGTTAACAGTAAAGTGCTTAGCAGAGGCTCCCACACATAGTAGTTACTATGTAGTGGTAGTTGCTATGTTTATTAT
TATTATATTATTCTCCTCCCTCATAAAATGGCAGCCTCCCTTTCTTCCATTCCCCTTCCTGAGTTATTTTTTCTCGATAGGGCTTA
TCTTCCTAGTATACCATATGTCTCTCTTCTCCCACTAAATTGTAACCCATTCATTTTTCCAGGATATATTTGTTGGGTGTCTACTC
TGTGCCTGGCATTGCTGTAGATGTTGGGGATGCAGAAAGGAAGAAAAAGGACAGATTTCCATGCTAGAGCTTAGGTTACAGCTGGG
TTAGACAGATAAGGAACAGCAGACATAATAAAGAAGGACATGAATTGTGTATTCAAGATTATGAAGTGCTGTGGAACAGGCCTAG
CAGGGTCAGTGTCTGGAGGGCTGGGGCGTGGTGCTGTGTGGTTAAGTTGTCCGGTCAGAGTCAGGGCAGGCTCCATGGAGGAGCTG
ACGTTAACCTCCACCTGAAGTAGGGGAAGGACAGGCGTTTCAGGCTCTGGGAAGAGCTAGGACAGAGGTGGAGGCAGAAGTGAGCC
TGTGGGGGTGAGGAGCAGCCAGGAGAAGCGTGAGATGAGAATGGCGTGAGCAAGGGAGGTAGGAGAACCCGAGCAGCATGCAGTGT
GCAGCCTCACAGAGAGCCTGCAGGGCATTCCAAAGATTCTGTCTTTTACTCTGTAAAATGGGGAGCCCTTGCGGTGTTTTTTGTTT
TTGTTTTTTGTTTGTTATGAGGCAGAGTCTTGCTCTGTTGCCCAGGCTGGAGTGCAGTGGGGAGATCTTGGTTCACTGCAACCTCT
GCCTCCTGGGTTTAAGCAATCCTTACACCTCAGCCTCCTGAGTAGCTGGGATTACAGGTGCGTGCCACCACGCCTGGCTAATGTTT
TTATTTTTGTAGAGATGGGGTTTCACCATGTTGGCCAGGCAGGTCTCAAACTCCTGAACTCAAGTGATCCACCCACCTCAGCCTCC
CAAAGTGTTGGGATTACAGGTGTGAACCACTGTGACTGGCTTCCTTGCAGTGTTCTAAAAAGGAAAGTAATTTGATCTGACTTGGC
TTTTGAAACGGTCAGTCTGGCTGCTGTGTTGAGAAGAAACTAAATCAGGGTCAGGAGCTAGAGCACCAGGACAAACATGCACAGAG
CGTTAAGTGCCAGGTCCAGGCTGGCAGTGGTGGATGGGGCCACAGGAATGGACTTCTAGGCTGTTCACTGTTGTATCCCCAGCATC
TTAAACTGTGCTCTCCATATGGTAGCTGTGAGCCCTATGTGGGTATTGAGCACTTGAAAGGAAATTGATATGTACTGTGTGTTAAA
CGCACATTGGATTTCAGAGACTTAGTGAGAAGAGTAAGATATCTCATTATTTATTTATTTATTTATTTATTTATTTACTGAG
ACAGAGTCTCACCTGTTGCCCAGCCTGGAGTGCAGTGGCATGATCTCGGCTCATTGCAACTTCCGCCTCCCGGGTTCAAGCGATTC
TCCTGCCTCAGCCTCCCAAGTAGCTGGGATTACAGGTGCCCACCACCACACCTGGCTAATTTTGTATTTTTAGTAGATATGGGGTT
TCACTATGTTGGCCAGGCTGGTCTTGAACTTCTGACCTCAGGTGATCCATCCCACTCAGCTTTCCAAAGTGCTGGAATTACAGGTG
TGAGCCACTGCACCAGGCCTATTTGTTTATTTATATGAGACACGGTCTCACTCTGTCACCCAGGCTGGAATGCAGTGATGTGTTCA
TAGATCATTATAGCCTCGACCTCCCAGGCTCAGGTGATCCTCCCACCTCAGCCTCCTGGGCAGCTGGGACCACAGGTCTGTGTCAC
CACGCCCAGCTAATTTTTTTGTATTTTTGGTAAAGCTGGGGTCTCCCTGTGTTGCCCAGGCTGGTCTTGAGCTCCTGGGCTCCAGC
AATCTGCCTTCCTCAGCCTCCTATAGTTCTAGGGTTACAGGCGTGAGCCACTGCACCCTGCCAAGTTATCTCATGAACCAATTCTT
TGATGTTAATTACATATTGAACTGATAATGTTTTGGATATATTGGGTTAAATTATTAAAATTAATTTCATCTGTTTTTTCTAACTT
TATGGCTACTAGAAAATCTAAAATTACATGTGGGTTGAATCATATTTTCGCTGGATGGAACAATGCCTTGGACGAGGTCAGCATTT

AGTGAATCAAATGIGTGGGTGAGGCACAGGTGTTCTGCCGTGAATGAGAAACCGTTGCCATCTTCGGGAAGCTTCCAGTGTGGGAG
GGAGGCACACTGCAGATGTAACTGCTAGGAGAGAAAGAAGGCAGGTTGAAATGGGACCACAGCCAACCAGGCCTGCTGTAGCCCAG
AAGCTGGAAGAGACTCGCCCAGGAGGGAAGGAAGACACTGGGGTGTTGGGTCTCAGTCTGGGTCTTGGTGACAGCAGTGGGCTGGC
AGTGCAGAGGTTCAAGGCCTTGGACTGCAAGTCCACTAAGGTGGCTGGTGGTGTGGGTGGAGGTGGAAAAGGCAGGTGGGGCGAGG
GTGAGGGGCAGGACATCCTGGAGAGCCTGAATAACCAGGTATAAGTGATTTCTTTTTCTTTCTTTCTTTCTTTTTTTTTTTTTTTT
TTTTTTTTAAGACGGAGTCTCCTTCTGTCGCCCAGGCTGGAGTGCAGTGGCGAGATCTCGGCTCACTGCAAGCTCCGCCTCCCGGG
TTCACGCCATTCTCCTGCCTCAGCCTCCCGAGTAGCTGGGACTACAGGCACCCGCCCACCACGCCCGGCTAAGTTTTTGTATTTTT
AGTAGAGACGAGGTTTCACCATGTTAGCCAGGATGGTTTCGATCTCCTGACCTCGTGATCCGCCCGCCTTGGCCTCCCAAAGTGCT
GGGATTACAGGCGTGAGCCGCCGCGCCCAGCCCCAGGCGGAAATTATTTCAACTGGGTCCTGAGAACTGGGCACTTTTCAAGGATT
GTCAGCAAGGAGCAACTTCCCAAATATACATCTAAAAAGATGACAAACTCATGTGGAATGTGGATTTAAGGAGGAAGGTACTTGAC
TTAAGAATTCCAGGATTTTGGTGGAAGAAGGAAATATGGGCCAGGCACTGTGGCTTATGCCTGTAATCCCATCACTTTGGGAGATA
GAGGTGGGAGGATTAGTTGAGCCCATCTGAGACCAGCCTGGGCAACATAGTGAGATCCTGTCTCTACAGTTTTTTTTTTTTTTGA
GACGGAATCTTGCTCTGTCATCAAGGCTGGAGTGCAGTGGTGAGATCTCAGCTCACTGCAACCTCCGCCTCCTGGGTTCAAGCGAT
TCTCCTGCCTCAGCCTTCCGAGTAGCTGGAGTTACAGCCTTGTGCCACCATGCTTGGCTAATTTTTGTATTTTTAGCAGAGACGGG
GTTTCACCATGTTGGTCAGGCTGATCTCGAACTCCTGACCTTGTGATCTGCCTGCCTCGGCCTCCCAAAGTGCTGGGATTACAGGC
ATGAGCCACTGCACCTGGCCTCAAAAATTCTTATTTTTAATTAACTGGGCTTGGTGGCATGTACCTGTAGTTCCATCTTTGGTGGC
ACGCACCTGTATTCCCATCTACTTGGGAGGCTGAGGCAGGAAGCTTGCTCGAACCCAGAAGTTTGAGGCTGCAGTGAGTTATGATT
ACACCACTGTACTCCAGTCTGGGTGACAGAATGAGACCCTGTCTCIAAAATAAATAAATAAATTTTTAAAAGCTGGGAGGTGGGGT
TGGTAAATTCATAAAAAATAATATTTCTTGAGTTGAATACAAACAATACAATCACTGTTTTTTCTTTGCCTTGTTTTATTTTTACT
TATTTATTTATTTTTGCAGACATIGGGCCTCACTCCTAGGCTGGTCTTTAACTCCCGGCTTCAAGTGACCCTCCCACTTTGGCCTC
CCAAAGIGTTGAGATTACAGGTGTGAGCCACTGTGCCCAGGCTGTGGTTTTTTATTTTTTAATTTTTTATTTTTTTGTATTATAAT
CATAGTGTATATGTTGCATCCTGATTTTTTTTTTTTTTTTTTTTGAGACAGCATCTCATTCTGITATCCAGGCTGGAGTGCAGTGG
CACAATCTCAGCTCACTGCAACCTCTACCTCCCAGGITCAAGCGATTCTTCTGCCTTGGCCTCCGAGTAGCTGGGATGCCACCGTG
CCCGGCTAAATTTTTTTCTTTCTTTCTTTTCTTTTTTTTTTTGTATTITTAGTTCAGACAGGGTTTCACCATGTTGGCCAGGCT
GGTCTTGAACTCCTGACCTCAAGTGATCCCACCTCGGCTTCCCAAAGTGCTGGGATTACAGCGTGAGCCACCACATCTGGACACAT
CCTGATTTTTTTCTTAGTTGCTAITAGGACACAGGCATTTTTGAATATTATATGCACTTTGTCCATGTCTTTTTTTTTTTTTTTTT
TTTGAGACGGAGTTTCACICTTGTTGCCCAGGCTGGAGGGCAATGGCGCGATCTCGGCTCACTGCAACCTCTGCCTCCCGGGTTCA
AGTGATTCTCCTGCCTCAGCCTCCIGGGTAGCTGGCATTACAGGCGCTCACCACCACGCCCGGCTAATTTTGTGTTTTTAGTAGAG
ACGGGGTTTCTCCACGTTGGTCAGGCTGGTCTCGAACTCCTGACCTCAGGTGATCCACCCGCCTTGGCCTCCCAAAGTGCTGGGAT
TACAGGAGTGAGCCACTGTGCCCAGCCGTCCATGTCATTTTTATTGGGTAGATATTTCTGTTCAGTGACTGTTTCGTTGCITACTT
TATCATTCCTTTGATTTGGATGCTTAGCTTCATAACAGCCTGTTTATATAGATTATGTTTICTATITTTCAGTTAATTAAAAAAAA
AACAAATTCTCAGTCCTAGGATTAITGTGCATAATGTCTGAATTTTTTAGAATTTCAGGTAAATATTGTGGTTCATGAGATTTGCA
CAGAAGCTGAATTTGCTGCTTTCTGGTGAGGCACGTGATGTTTTTTGCTGTCCGTGATGCACTAACATGTCATTAACATGTAIGTA
CAGTGTTATATGCTCGCACATGTTTATGTGCAGCCTATGCACCTGIATTGGATTTTTCTTCACTCTCATITATTGGCAGGTATTTG
TGGAATGCCTGTGGTGTGCCAAGTTCTGCTTGAGGCCTTGGGTATTCAGTAGIGAICAAAGTAGATCAACIAGIACCAGTTCTTAA
GAGGCTTATTGTTAAGTGGGGAAACAAGCAAATAGACAAAGAAAAAATAACATTATTGTAAGGAATATGAAATAATATGTACIAG
AATAATATGAAGGGATGGTTATTGTGAGGTAAGGGGGTTCATACTGAAATACTGTCTGAGGAATCAGGATTTGAGTGAGACACGAA
GACTCAGTAGAAGTTAACCAGAAGAACAAAGTGTTAGCAGAAAAGACAAGATTGATCAATATGACTACAIATTAAAGAAAAAGGCA
CATCTAAGCAACAAAAACTGCAATGGTATTAAAAGCCAAATACAAGACTTAGAAGATATITGCAATATTTATAACACATTACAAAA
CACTAATATCTAGAATGTATGCAGAATTCCCAAAAGTCAACAACAAAAAGACAAACAGCCITATAGCAAAGTGGGTGAAAGATAGG
ACCATGCAGTGCTCATAGAAAGCAAAGTAGATGGCTGGGCACCGTGGCTCATGCCTATAATCCCAGCACTTTGGAAGGCTGAGGCG
GGCGGATCACAAGGTCAGGCGTTCGAGACCATCCTGGCCAACATGGTAAAACCTCATTTCTACTAAAAATACAAAAATTAGCTGGG
TGTGGTGGCGTGTGCCTGTAGICCCAGCTACITGGGAGGCTGAGGCAGGAGAAICGCTTGAACCTGGGAGGCATAGGTTGCAGTGA
ACCCAGATCTTGCCACTGCATGCCAGCCTGGACACAGAGAACGACTCCGTCTAAAAAATTAAAAAAAAAAGCAAAGTGGATGATAA
ATGTGAAAAGATTCTGAATTTTGCTAACAAAGTCAGGTTTTGGAGATCTAAITTCTAGTCAGTAAAATTCATCCTTICTAGGATAC
AATGCTAAGAGTITTGATAAAIGCATAGAACTGTGTTACACTGTTGTGIAATTACCACAATCATGGCTTAGAACAITTCCATCGTT
CACCCAGATTCCCTCCTGTCCCTTCACAGCCAGTCTCCTTCTCCACCCCCAGCTTTAACCAACCCCTGAGTTGTTTGCTGACCTTA
CAGTGTTGCCTTTTTCAGAGTGTCCTATAAATGGAITCATACAGTGTGTAGTCTCICGGATTTGTCTTCTTTCACCTGGCATAAIG
CATTCGGACCCTTCCATGTTGTTCTATGCATCAGGGGTTTGGTAGITTTTATIGCTGACTGGGACTCTGTTGTATGGATATACCA
CTGTTTGTTTATCTGTTCTCCATTTGAAGAGCATCTGGGTGTTTCCACAAGATACATGTTTCATGCTTTACAACGIGCCAGAAATA
GGATGTCTGACTCAAGTGTTGACAAGGCACAGGGTGTTCAGCAGCTCCGCTAGTGAGTGTGTGAGGTCACTTTAGAGACCACCTTG
CCAGCAGGTAGTGAAATTGGAAACATGTGCAGCCCCGTTCCTAGGIAAGATTGAGAATTCACAGTTCGTGCIGTATGTTACTTAAG
GATATTTAGAGATGAAAGTGAAGGTTTAAAAAATGGGCTGGAAAGATCTATATCAAACTCACTGTCATCATCTGGGGAGAGAAGGG
GAGTGGGCTGATTGTGAAGGTCAAAGCCTACTTTAACTTTGCCTTTATTGCTTTTTTTAAATTCAATTAATTAATTTACTTGTGGG
GACAGAATCTCACTCTGTCAAGGTTGCTGGAGTGTGAGTGGTGIGATCGTGGCTCACTGCAGCCTCTACTTTCCAGGCTCAAGTGA
TTCTCCTGCCTCAGCCTCCGGAGTAGCTGGGACTACAGGTGTGAGCCACCATGCCTGTATTTTTTGTAGAGATGAGATCTCACTAT
ATTGCCCAGGCTGGTCTTGAACTCCTGGCCTCAAGCCATCCTCCTGCCCAGTTCTCCCAAAGTGCTGGGATTACAGGGAGGACCCA
GTGTCTGGCCTATCTTTAATGCTTTTTTTGAAAAGAAGGTGGATACCTACACATATCACTTACACAGTTTGAAAATATGTAGAAAA
GAAAAAAAGTTTGAAAATACATATGACAAGNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNCGTGGTGGCTCATGC
CTGTAATCCCAGCACTTTGGCAGGCTGAGGCGGGCAGATCACTTGAGGTCAGGAGTTCAGGACCAGCCTGGCCAACATGGTGAAAC
CCCATCTCTACTAAAAATACAAAAATTAGCCAGGAGTGGTGGCAGGTGCCTGTAATCCGAGCTACTTGGGAAGCTGAGGCAGGAAA
ATAGCTTGAACCCGGGAGGCGGAGGTTGCAGTGAGCTGAGATTGAGCCATTGTATTCCAGCCTGGGCAACAAAGTGAGATTCCATC
TCAAAAAAAAAAAAAAAAAAAAAAAAAGAAAGAAAGAAAAGAAAAAGAAAAGGACTTCTTGGAGGGCAAAGTTTGAGGGAAGAGT

TAGCTAGCTAGAGACCTTCCAAACTGTGCTCAGGCATTTGGATTTTACCCTGTGGGTGATAGGGAACTATTGAAAGTTTTCATTTT
TTTCTTTTTGGAGACAGAGTCTTGCTCTGTTGCTTCAGGCAGGAGTGCAGTGAGCCATAGTGGCTCATTGCAGCCTGGACCCCCTG
TGCTCAAGCAATCCTCCCACCTCAGCCTCCAGAGTAGCTGGGACTACAGGTGCATGGCACCACCCCCAGCTAATGTTTTGTTTTTA
ATTTTTTATAAAGATAGGGTCTCACTTTGTTGTCCAGGCTTAAAACTCCTGGCTTTAAGCGATCCTCCCATCTTGACCTCCCAAAG
TGTTGGAATTACAGGAATGAGCCATCATGCTCGGCTCTATGGAAGGTTTTGAGGTGGGGCAGGGATGAGATTAGAGTGATGTGCCT
TTGCCAGAGAGATTTGTCCAGAGGAAGGGTGACCAGCCTGGAGTCTCTTTGGAATGTTCTAGGTAATGTAGCAGAGATTGTGTCAG
TTACCCTCTGGTGGGATGCTTGGCTTTTCCCATTTTTTCAAAAGGAAATGGAAAAGGAAAAGGTCCTCTGTATGGAAAGTCCCCA
GGAATTTATAGGAGAGTGGCTAACGAAGACAGAGTTCCCTTCCGGCATTTATTGTGACTGAGTTTGTTCCATAGTTTTAGTCCCAC
TTAGGTCATGATTCATCCCACTGGGCCAGCAGCAGCATTGGGATCACGTCAGCCAATCTTCGCACTTAGAGATTTTGAAGAGAGAT
ATCTCCTATCCATGTGGTACATTGCTTTTCACAAAACAAAAGAGTTCATTTTGTCGGTGAAAAGCCCTGGCCAGACCCAGCCGGTG
GAGACCAGTAGCTTCCTCTGGCAGGATGGGCTTCCTATGGTCATTGCAGTCCCTCCCCATTTATAAGGCTGCCTTCCCTGAGAACA
CATGGGACAGATGCCCCCCAGAAGCATGCCCTCTGGAAAGGGAGAGGAGCAGGAGGGGGCTTCAGGCATGCTGGGCCTGGGGGGCC
ACCCCCCTGGCCTTTGATAAGTTCCTGGCTGGTATGAGCCATCTTCTTTCCTCTCCCCAGGCCTTGCAAAGGCCATATCTCTTCCT
TTTCATGACTCTGGAAATTCCATACATACTTTCTGCATTAGGAAGACATTCTCTGCATGCTGATCTCGTTAAAAAGGAAAAGCCAA
GGAGCATCCTGCACCTCCCTCCTGTAAGCGAGCTTTGCAAATGGTAAACTAGATCCTGAAAGGCAACCACTTAAAAGCTTTTAATG
GCTTCCCATTATTAGCTTGAGTGCCTGGTGCGTCAGAGGCTCTCAGTGAGCGTGTATTGAATTGCTGAATGCATGGTGGCCCTGGG
CGAGGAATCTGGCCCTCACTGTCCCAAGGCTGAGTGCCTTTGCCCTGTGGAGGAAAGCACTGCCTTGGGGTCAGAAATAAGGATTG
GCAGGATTGCCTGGGGTTAGAAATAGTGATCATGGGATCAGAGGCAGCAGGTGAATGTGGGGTGTGAATATTTTTGAAGGCTTGGA
ATGAAATGATTCTTCCAGACTTTAGAGGATTTGTGTCTGCAGATTCTTCTCTAAGGTTTGTGTCTCACATAGTGAGAGAATCAGCC
AGCCAGGGAAATGGGACAGGATTTTGTCTCTTTCCCCATTTCCTCTTCTAATGAACTCCTACCTATGCTTTATGACTGCATTAAGG
GGTCACCTCCTCCAGGAAGCATTCCCTCATCTCCTTACGCTGGTTCTGAGTGTGTCGTTCCTGCTTGATCCCCTCCCAGCACCATG
TGTGTGAGTTCTCACAGCTCACACCCCATAGTATTACTATGTTGTTTCTACAGTGGGTTCCTGTCAGGGGTTCCAAACTTAGCTGT
TTAGGAGGATGAGTAGATTGTACAACCACACCAGGCGGGCCTGATCAAATACAGTAAGAAGTGTCCGGATTGGCAAACCAGCGGGT
CCCTCTTCTAGATGTTATCAGAGAGATACTGTTAGAGAGAGGTTGCTAGATGTTAATAGAGGCTGCTGTTAGATTCCAGCTCCCTC
CTCCCCTGGTCCCTTTTCTCTCCTCATGCCAAAAGATAGACCCAGTGTTGCCAGAGCTTCCACTTTTAAAAGAGAAGCCTTTTAAA
ATATAAACTCTCCTGATTTTTAAATGTTTTCAGTTAAGTTTTTAAAGGAACATTCATAGGCCAAACCAAATACCTGTGGGCCGTAT
GTGGCCCCCGGGGCCAGCAGTTTGCAGCTCCTTGGTTATAGGTGCCTCTAACCCATTGCACAAGCTCCCTCAGGCATTTCATGTCT
TTCCTTTCCATTCCCCCTGGGCCTAGCAGGGAGCTGGCATGTCACAGTGGGTGCTCCCTAATTATTGGTTATTGACTTGAATGCTA
GACTGAGCAAGCACCTCCCTGCTCACAGGGTGTTAACATTTTTCCCTCAGAACTGTTTGTATGTAATGCTATTCTGAATTGCTTCA
GTTCCTTACATACTTTAGTGATAGTCTGTATGTGCCTGGAAAAGTCATCATTCCTTAGATTCTAGACAACTCTGTTCTTCTGTTCT
TTTCCTTTTTCCTTTCTCTCTCCGTCCCCTCAACATATGTTTTTGTTTTTTGTTTTTTGCCAGTTCTTTTGTATCTGGCCGGTTAA
CACAGGGCTTCATAACATGGGGTCGGCTGTTATTAAGCAACAGTGGTTTCCGGTAGCTCACTGACTTTGCCCTCTCTGCTGGTAAG
CCATTGCTGATTTCCATCAGGGTAACAAGCATGATTTTCAGATTTACCTTCATTTGCAGTGAACAATTATAGCAAAATTGCTTAAA
GAAATGAACCTTCAGGTGTGGCGGCTCGTGCCTGTAATCCCAGCACTTTGGGAGGCCGAGGCGGGAGGATCACTTGAGCCCAGGAG
GTCTCTAGGCTGCAGTAAGCTGTAGTTGCACCACTGCACTCCAGCCTGGGTGACAGGGTGAGACATCGTCTCAAAAGAAAAGAAAG
GAAAAGAAAAGAAAAGAAAAGGAGGGGAGGAGAGGGGAGGGGAGGGGAAGAGGGAGAGAGGGAGGGAGGGAGGGACGGAGGAAGGA
GGAAAGGCAGGAAGGCAGGAAGGCAGGAAGGAAGGAAGGCAGGAAGGCAGGAAGGCAGGAAGGCAGGAAGGAAGGAAGGAAGGAAG
AGAATGAAAGGCTGGGCGTGGTAGCTCTTGCCTGTTGTAGTCCTGGCACTTTGGGAGGCTGAGATGGGAGGAATTGGAAACCAGCC
TGGGCAACATAATGAGACTTCATCTCTACAAAACAAATTTTTTTTTAATTAGCCAGTGGCATGCACCTGTGGTCCCAGGAGGCCAA
AGCTGGAGGATTGCTTGAGGATGGGAAGTTGAGGCTGCAGTGACCTATGTTCCCACCACTGTACTCCAGCCAGAGTCACAGAACAA
GACCTTGTCAAAAAAAGAAAAGAAAGAAAAGGAGAGAGGGAGGGAAGGAAGGAAGAAAAAAAAGAAGGAAAGGGAGAGAGGAAGGA
AGGAAGGAAGGGAAGGGAAGGGAAGGAAGGGAGGGAAGGGAGGAAGGTAGGAAAATGGAAGGAAGGGAGGGAAGGAGGGAAGAAAA
GAAAGAAGGAAGGAAAAAACCGGTCGGCTATCTTGACGAAAAAAGTCTCAGTCCACTCCATTTTGTGCAGCATTTCCCGTCTTGTT
ATCTTTCTGGAAAAACTCAGTAAGTCTCTGGCTGGAATTATAGAAGCTTAATATGCACTTGTAACTGTGTCAGGCTCTCACAGGG
AACAAACAAGAGGAACTATAGATTTTTATAGTCTGCAAGAGCCCAGTCCATGCATTTAGGAGGCACAGAGTGAGAGAAAGAGCATT
GACAATGATAATATAGCATTAATATAGGTACAATCTGGAGTAATGTTATCTAGTACTACTAATAGCGATATTAAAATGGCATTATA
TAGATATTAAGCCCTGACTTCGTGCCGGTTTGAACTTTTCCTGTATTAATTCATGTGAATCAATTTTGAGACTCTGTCTCAAAAAA
AATATATGTATATAATATATACAAATATATATAATATATACAAATATATATAATATACAAATATATATATATAATATATACAAATA
TATAATATACAAATATATATAATATATACAAATATATATATATATAATATATAGATGTCATGGTGATGGGAAGACTGGGGAGGAGAG
GAGCAGTTTAATCCCAGGATCCCATGGAGAGGAAACTTGTAGGTCTCTTACCCAGTGTCTTCTCCTGCTCTCTGGCTCTGATTTCT
GAACTAAGAAAAGTGTGCAAACATCATCTGCTCTCCTGGTTGTATTTGTAAATGCCTCCCCTAGACCCAGCTCTACTTTAGGCCAC
GTGAGTGACAGTGACCACATCAGTCAGGGATTCTCCTACTGTCCCTGACCCATGTCCGCTTTTGTCTCCTCCCACTCTAATAACAG
ACCGTGTGGCTTGAACCTCAGCCCTGCTGCCAAGTAGCTGTGTGAGCTTCAGAGCCTGGTGCCTCAGTTCCCTCATCTGTAAAATG
GGAATACAAATGGTCCCTGTGTGTTTGGGTATTCAAGAAAATTAAGTGAGTTGCTGCACACCTCACCTCTGGCTCCTTCAACAAAA
ACATGGTCACCCTCAAACCAAACGTGAACAGGAGGGTGGTGACGCAAACCACATGGAAACGAGAGGCAGACCCAGGCTGGAGTGCA
ATTGCACGATCTCGGCTCACTGCACCCTCTGCCTTCCAGGTTCAAGCAATTCTTCTGCCTCAGCCTTCTGAGTAGCTGGGATTACA
GGCACCTGCTGCCAAGTCTGGCTAATTTTTGTATTTTTAATAGAGACGGGGTTTCGCTATGTTGGCCAGGCTGGTCTCGAACTCCT
GATCTCAGGTGATCCACCCGCCTTGACCTCCCAAAGTGCTGGGATTACAGGCATGAACCACCGCCCCCGGCCGCAGATGGGAAATT
ATTTTTGCACTTTCAGTTGTCCTAAATCTAGAGTTTTCCTTTATCCAGACAGCTGGTGAAAGTGGACGTGAGAATAGAGAGATTTC
CTGGTGGGGAAGACCTCCATCCTCCTTCTCCAGCCCCTTCCTCATTATTCCCAAGAAAAGAGCTTCTGGGTTTCTGGAGAGGCTG
AGCAGGTGGAGACACCTGGGCCATATCTGGGTCTCTCCAGAATGCAGGAGCCTGCCTGACGCGTCAGCCGGGGAGGGTTTCTGTGT
GAAGTCGGCTTGAGTGAGCCAGCCTTTCATGCATCCTTGTCTTTATTTCTTCCCCAGCTCAGGAGGGGCTGAGTTGTGTCTTAATG
AGCAGTTCCAGCTGCATGCTGGGCATCTCCTAATTTACTCTCATTTATTTAGCTCCTGCTGCATGCTGGGCAATGCTCTAAGTGCT
CTGCAGGTTTTGGCTAATTTAATCTCCCTAGCAAACCCTTTGAGGGGACTGACAGGATGACCATCCTCATTTTACAGATGTCGGGA
TTGAGGCACAGACAGTTTATTTTTAATTTTTATGTGTAAAATGTTTTTACTGTGGTAAAATTTACATAATAAAATTTGCCATTGTA
ACCATTTCTAACTGTACAATTCAGTGGCATTAAGGCCATTGGTATTGTTGTGTAACCATCACCGCTAATCTAACCATCTCCGGAAC
ATTTTTCATCATTCCAAACTGAAACTCTGCACTCATTAAACACTTACTCTCCTCTCCCCCTCCCCTCATCCCCTGGTATCCACAGT
TTCTATGAATCTAACTACCCTAAGCATCCGTATAGGTGGAATCATAGTATTCATCCTTTTGTGACTGGCTCATTACACTTGGTGTA
AACTTTTTTTTATTTTTTTATTTTTTTTTGAGACAGAATCTTGTTCTGTTGCCCAGGCTGGAGTGCAGTGGCACAATTTCAGCTCAC
CACAACCTCTGCCTCCCAATTCAAGAGATTCTCCTGCCTCAGCCTCCCTAGTAGCTGGGATTATAGTCGTGCACCACCACGCGTGG
CTAATTTTTGTATTTTTTAGTAGAGACGGGGCTTCGCCATGTTGGCCAGGCTGGTCTCAAACTCCTGACCTCAGGTGATCCACCCT
CCTCGGCCTCCCTAAGTGCTGGGATTACAGGCATGAGCCACCGTGCCCCACCTTAGTGTAATCTTTCAAAGGTATATCTCCGTTGT
AGCCTGTGTCACAATTTTATGCCCTTTTAAGACTGAATGAGTTTTTAAATTCATTCCCATTTAACTCTTTGTTTTGTTGCCTGTGC
TTTTGGTGTGAGGCACAGACATTTTAAGAAACTTAGCCAAGGCCATTCAACCAGAAGTGGCAGAGCCTGGATCTTGACCACATGGC
TTTGCTGCTCCCAGAGTCTTGACCCTGTGCTGGGGCTGACTTCTGCTTTTACATGAGTTCTTGTCAGTGCTTTCGGCAGCTTGATC
AGGTAGATGCTGCTTTTCCACTTTGCAAATATGGAAGGGAGCACCCAGCTCTCTTGGGCATGGCAGAAATTCATCAGGGGCGGAGG

AGGCACAGGGTACCCACCCAAAAAATCCCAACATATTGAATCACCTTCCAATAATCTATAGCATCATTTATGTTATGCTGCCCACA
ACATAAATGGCTAGCAAATAATTTGGATGCGACAGAATTGGTGAATGAATTTAATGTAGCTTCAACCTGAAGACCATTATACACTA
TTAATACCTACAAAATTATAGATGTAAGGCTGGGTGTGGTGGCTCATGCCTGTAATTCCAGCACTTTGGGAGGCCAAGGTGGGCAG
ATAATGAGTTCAGGAGTTCGAGACCAGCCTGGCCAACATAGTGAAACCCCGTCTCTACTAAAAATACAAAAATTAGCTGGGCTTGG
TGGCACATGCCTGTAGTCCCAGCTACTCAGCAGGCTGAGGGAGGAGAATCTCTTGAACCCGGGAGGCGGAGGTTATGGTGAGCTGA
GATCACGCCACTGCACTCCAGCCTAGGCAACAGAGTGAGACTCCGTCTCAACACAAACAAAATTATAGATGTCACCTACAGGCCAG
TTGGGAGGCATATTGGTAAGTAAATACCCATGACCTCTCCACCCTGCCTAGGATCCAAACTATGAACACTGATGGTTGACTCTAAC
TCCATATTCTTTTCAGTTTTCTCCCATGCCTACCCCCTGCCCCCGAGCATTCTCCCTCATTGGTGGCCCTGATTTTCTTTTTGTTG
AGATAGGATCTCACTATGTTGCCCAGGCTGGTCCCAAACTCCTGGGCTCGAGAGATCCTCTTATCTCGGCCTCTCAAAGTGTTGAG
ATTACAGGCGTGAGCCGCTATGCCAGGCCAACTCTTATCTTGAATGTTGATGTTTATCATGTCATGGATGAAAAAAAAAAAACCCA
TGCGTATTCTTTTTTTTTTCTTTTTTTTTTGAGATGGTGTTTCGCTCTTGTCACCCAGGATGGGGATGGAGTGCAATAGCATGATC
TCGGCTAACTGTAACCTCCACCTCCTGGGTTCAAGCGATTCTCCTGCCTCAGTCTCCTGAGTAGCTGGGATTACGGGCACTCGCCA
CCATGCCTGGCTAATTTTTGTATTTTTAGTAGAGATGAGGTTTCACCATGTTGGCCAGGCTGGTCTTGAACACCTGACCTCAGGTG
ATCCACCCGCCTCGGCCTCCCGTTTGTATTCTTAAATAGTGTATTGTTTCCTTTACTTCCTTTAGAGCTTTACAGAAATTCCATGC
TGCCTTCAGCTTCCTTTGCCGCTCTGCATCATGTTGGTAAGTCTCTGCTGATGCCTGCTGCTGTAGCTTATTTTTCACTGTGATAA
TCTGTTCCATTGTGTGAACTGACCACAGTTGATTGATCTGTTCTCCAAGGACATGTGAGTTGTTCGCAGGTGTCCCCCCACCCCCT
CCCGATACAAACAGTGCTGCTGTGGACATTTCATACATCTGGTTGCCTATGTCAGAATTTCCAGGGTATACAGCTAGGACGCAGTA
CTGCTGTTGGATTTTCCTGGGATTGTAGACTCTCAAATTTGGAATGGACTTCTATGGCCAAGTAGTTCACCTTCAGCAATCATCTC
AAAAGAAATGCTCTTGGTGTGATGGTTGAGAGCGCTGTTCTGGAATAGGAGAGACACAGACCTGCTGGGCCTTTGCCTCATTTTAT
TACTTAGTTCATTATGCAATGAACTTAACATCTGTCTACCTCAGTAGTTTCCCTGTCTGTGAATTGAGTCTGTTACATATATATAT
ATCTATTTAAATATATATATCTATTTAATATATATTAAATAATATATATTTAAATAGATATATATATATTACAGACTCAATTTAAC
CCTTAGGACCCCAGGAGCGGATGAATCGTAACCCAGTTCNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNCTTCCAGGCTTCCCATTTTAGACTTGAAATGTAGGCCTGTTGGGGCCTCCTCCAGTAGA
TGTGTGCCCTTGAGCTTGGATACCCCCTGCCAAGATACGCCTTTCAAAAACAGCATAAACTTATCGCAACTGAATAGAAGGGGCT
CCCAAGGGGTCCCACCCTGCTCCGGAAGTCTCAGTGAGGAATCAATAGCTGTTAATTCCTGTGCTGCCCCCTGCCTGACATTCCAT
CCACACTTGAGGCCCAGATTGTAAGAAGGGAGAGCAGGCAGGTCTCTCTTCCAGGAGAGGGACCCGGTTCATCAGCAGGATTTATT
CATTCATGTATTACGTCAGGCTCAGCGCCTGGGCTTTTTATAAATGAGGGCTCTGCTGGCGTTCTGTGTTATTCAGATATTCGGTG
AGAGACCACCAGAAGCCACAAAAAATTGCTGACATCTGTTGGAAGGCAATCATGCATAATGGATAAGGGTGCACTCTGCAGTTCCA
GCAGCATTGATTTGAATTCCCCCTCTGCCGCTTCAAAACTCTGAGATCTCAGCGAGTTGCTTACCCTCTAGAGCCTCCACGTCCTC
ATCTATAAAACTAGAAAATAAGTGGTACTCACTAGATCCTTACCTTGTTGTGAGAGTAGAATGACGTGCATGTAGAGCTTTCCCAG
AGCTGGCATGTGGTAGGTGCTCAATAAACGCTGGTTATTCTCTCTCTCTCTCACTCTGTTGCCTAGGCTGGAGTGCAGTGGCGT
GATCATAGCTCACAACAGCCTCTGTCTGCCAGGCTCAAGGGATACTCCTGCCTCAGCCTCCTGAGTAGCTGGGACTTGCAGGCAAG
TGCTACCGCTCCTAGCTAATTTGTTTTATTCTTAGTACAGATGGGGTCTTGCTATGTGGTCCCTGCTGGAGTGCTGGTTGCTCTCT
AACTGGCTGCGAGCCCCGGGGTAGGCACACTGGTACTGGGGCTATAGCAAAATCCGTGCTCGGTAAATCATCATTGAAGGAGCAAG
GGTGCTTTACAGTTGTAGAATCTCATAGTACACGTTACTTTGGTTTTTCTCTTCCCAGCTCTGGAGTCCTGGGATCCAAGTGTGAG
CCCCATGTCTGCAAAATGAGGCTAGTCACTTGAATCCTCTTGGATCTTGCTTTCCTCATCTGTTAGTGAGCTTGTAGATAGCTCTG
ACTGTCACCAGGCTTTGCTCTGAGCACTCTGCATCTGTATTCAACCTGTATACTTAATCTTCACAACAGTTGAGGTGGTTGATCCA
TAGTAATTTCTCGTTGTACAAACGGAGAAACTGAGGCAGAGAGATAAGTGTCTTCCCAAGGTTAACAGCTAACCAGTAGCAGGGCA
CGCTGGGATTTGAAAGACTCTCTATGTGTCCTAACTGGTATGCTCTCCTGTCTAGAGGTGATCTCTTCCTCCAGGGTTCCAGGTAG
ATTGGTTGAGATGTGCAATGCGGAAGAGCCTCGGGATAAGTCGGCTAATGACACCCTCAGGCTACCCAGCCCTCGGGAGCTCACAG
CAGAGCAGTTGGGGATGCCTTGCAAGGTTACCTGTGCCAGAGTTCCTGGGGTACATATTAAATAGGTAGGACCCAATACTACTTTG
CATTTTCCTGGAATCCTAGACTCTTAACATTGGAAAGGATTTCTTTGCCAAGTAATTCGCCCTCATCATTAACCTCTAAGAAATGC
AGAGGTGGAATTAGAAGGTGCGTGTCTCTTTCTTTTGCACCACAGATTCTATCGAGTCTCATCTGGCAGCTTCAAGGATCATCCGC
ATTCTTTCATCTTCATGGAATGTCACTAGATTGAGATGATTCTAAGGGTGAGGTCAAGCATTTGGAGTTTGGAACTCTGCCTTTTG
TAGAGATGCTCCCGTGGGACTTGCCCTGCACCCAGTTCAGCTTGCTTGGGATGCATCTGCTGAGCCTGGAAGAGAAGTATGGGTTC
AAGGCCAGCGGTCCAGCACTCCCACAGACCTGATCTGTAATAGGAAGGGTTGCCTCTGTGTGGGTTGGGAGTTTAATCAGCAAGA
GTGGATTAGCAGCAGGCTCATTCTGTTTACTCCAGTAGGTCACGGGGCCAAGGGCAAACATACACCCCTCCCAGCCCAGAAACTGA
TAGCTGCAAAGTTGCACACCCTTCTCCCCAAATTGACAAATATGGTTTTCATTGTTGGAGTAATAAATTATGCTTTTTAAGGATAA
AAATGTTGCCCAGCTGTTGAGTGGTTTAAACTTCCAGATGTTCCTCTGCTCAGTAATCAGAGCAGGATGCATGATGGGATCTCTTT
AAAAGTTGATGAACAGAATGGTTCATTAAATTTCAACAATGTGCCTGGAATGCCAAGGGGAGGTGTAGCATCTCCCTCGAGGGAAG
GAGGTACACATTTCCTAGGGTGCTTGGGTGAAGAATTAGCAAGCAGTAACTGAAGACCAGTTACTGGTCAGGCAGGGAAGCCCAAG
TTCTGGCCCCTGCTGACTGTGTGACTTTGGGCTGAGCAGTTGGCCTCTCTGAGTCTTAGGTTTTGTTTTTTTTTTTTCTTTTGTGT
TTTTGAGATAGAGTCTCGCTTTGTCGCCCAGGCTGGAATGCAGTGGCGCGATCTTGGCTCACTGCAACCTCTGCCTCCCAGGTTCA
AGTGGTTCTCATGCTTCAGCCTCCCGAGTAGCTGGATTACAGACATGCACCACCATGCCTAACTAATTTTTGTATTTTTAGTAGAG
TTGGGGTTTTACCATGTTGGCCAGGCTGGTCTTAAAGTCCTGGCCTCAAGTGATCCGCCCGCCTTGGTCTCCCAAAGTGTTGGGAT
TACAGACATGAGCCACTGCGCCGAGCCTGAGCCTCAGTTTCTGATACTTGCAACTGGGAGCATGTTTTGAGAGCTAGAGAAAGTAT
TGTTGTCTTGTGTAACATGAAAAAAGCTGTGCGGATACTAGCTAAGATCAATGAAAGGTTATCAAACTCAATCTGATGGTGAAAGA
AGATACAGAGATCAAGCCCGTCCGCATTTTTAACGTGGGGACACTGAGGCCCAGGAAACTTGGGGCTTCTTCAAGTAGTAGCTAGG
CCTGTGCTGTCCCATATGGTAGCCGCTAGCCACTAGCCGCATGTGGCTATTGAGCAGTCGAAATGTGCGAAAATTCAAATTAAAAT
ATGTTGTATATGTAAATTGCACAATGGATTATAAGACTCAGTTTAAAAAAAAGAAAAAATTCATAACAACTTTTGAAATATTGATT
ACATGTTGAGATGATAAGATTTCGATATACTAAGTTAAATAAAACATTAAAAATGAATTTCACCTTTGGTTTCTGCTTAGTATTTT
TTAATGTAGCAACTAGAAAATTAAAAATTACTGCCGGGTGCGGTGGCTCACGCCTGTAATCCCAGCACTTTGGGAGGCTGAGGTGG
GCTGATCACGAGGTCAGAAGATCGAGACCATCCTGGCTAACACGGTGAAACCTCATCTCTACTAAAAATACACAAAAAATTAGCTG
GGCATGGTGGTGGGCGCCTGTAGTCCCAGCTACTGGGGAGGCTGAGGCAGGAGAATGGCGTGAATCCGGGAGGCGGAGCTTGCAGT
GAGCTGAGATCGCACCACTGAACTCCAGCCTGGGCGACAGAGGGTCTCTGTCTCAAAAAAAAAAAGAAAGAAAATTAACAATTACA
TGTGTAGTTTGCATTATATTTCCACTGGGCAGTGCTATGCTAAACTGTGACACAAACGGTGGACTTTAGAGTTGTGCAGGGTACAA
TCTGCACGGCCATATGTGGCAGGCCTACCAGGTGTGGATGAGGCTGTCTGGGTTCGAATCCTGGCTCTACCAGTAATCAGTTGTGT
GGTATTGACTAAATTGTTTAAACTCTCAGAGCTTCAGTTGTTCTATCTGTAAAATGAGTATTGCAATAGTGCATGCTTCATAGAGC

TGTTGTAAGGATTAAATGAATTAGTGCATATAAAGTGCCTAGAATAGTGTCTGGCCCGTTTAAGAGCTCAGTGAGTCTTGGTTGT
TGTAGTTGTTACTTTAGTGGTTGTTTTTAGAACCTGAGGACACAAGGATGGAAAGACTCAGTCTGTCTGTGGCAAACCACGAAAG
GAAAGATTAAACAATTCAATGCAGGCTGGGTGCAGTGGGTCACACTTGTAATCCCAGCACTTTGGGAGGCCAAGACGAGCGGATAG
TTGAGATCAGAAGTTCTTGACCAGCCTGGCCAGCATGGTGAAATCCCATATCTACTAAAAATACAAAAATTAGCCAGACGTAGTGG
CGAGTGCCTGTAGTCCCAGCTACTCGGGAGACTGAGGCACAAGAATTGCTTGAACCTGCCAGGTGGAGGTTGCAGTGAGCTGAGAT
CACGCTACTGCACTCCAGCCTGGGCGACAGAGCAGGACTCTGTCTCAGAAAAGAAAGAAAGAAAAAATAAATTCAATGCAGTGATT
ATTAGAGGGGTAAGTCCATCGGGACAAATCAGGGGGCATTCCCACTGAGGCAGGACTTGAGATGAATCTCGGAAGCAGAAATGCTG
AGCTTGTTGGGCTGGCCAGAGATAAGAAGGACATTGTGGGCGGGAGAAACAGCACCTACAGGGCTGAGATGGATGAAAGCTCAGTT
CAGTCTGGGAACTGCAGAAACTCAGTTTGCCTGAGCAAACTGCAATGTGGCAAGTGGGAAATGAACCCAAGCTAAAAGAGGACAGC
AGCTGGGTGTAAATTTTGTCCTGTAACCCATGGAGAGCCAAGAAGGATTTTGAGCCAGGGAGTGACATGATTGGATTTCATGATCA
GATCATTCTAACTGCTTTGAGGAGGTTGTCTTGGAAGGAAAGCAAGAGTCTTTGGCAAGAAGATCAAGAGAAGAAAGATTGCTATT
TCCAAAGCTCAAGTAGTGTATTTTTCATTGTTGTTGTTAAAGTTCAAAAGTCTTTATATTGAAAGTGGCACGTATGCTATGATCTC
ACACCTGTAATCCCTTCCCAGTTTATCTGCATGAGAAAAGCGCTTGGAATGGTGCATATCTAAAGGTGGTTTCTGCTCAATGGGGA
TGTGGATTCTTGTGTCAGGTTGTTGGGGAGGCTTTAGGCTGTGGCTGCCAGCCCATCCCACTGACAGCCAACTTCATATGGATGCA
GAGCTTTTTAACCATATCAAAGAGAAGGATAGGAGCTTAAAATATGAAACTCCTGCTTTTGATATGCTTAAATTCTGGGTCTGATA
TCTTTTTGAAAGAATTAGGTTTTTAATTGAAATAGCACATGCATATATTTAACATCAAATGGGTAAGGCTTTTATGGAAAATCCAG
ATTCCCCTGCCCTTTTTCTGCCTGTCCTTAGCCCTGTTTCCCAGAGTGAATCAGGGACAGGTCAGTACCTCTAAAGAATAAGCTCA
TGTTGCTCCTTGTCTGTTGACTTAGACTTCTGGACCCTCAGCAATGTCCCGGGACTCACACTGGGGGAGGCAGGAGACCAGCATG
CCCCCTCTTGCCACTTGTACTTCCCATTTTCCCCCTGGCAAGGTGTGGAACATTTATACTCCCACGCCCACACTTAAGACTCCTGT
GCTTTATCTGCAAGGTTAATTCCAACAATTGATAAATGGATGAACAGTGTTTACATTAAGAGGCTTGTCAATGGCCTTCACTGCCC
GTCGAAGCAGTCGGCTTTGATCACATGTCCTTTGTGTTCAGCTCTCTCTTTGCCCTCAGCTTTCTAATCGCCCTTTAAGGGGCTAT
CTGAGGCTGGGTGAGGTGGCTCACGCCTGTAATCCCAGCAGTTCAGGAGGCTGAGGCGGGAGGATCTCTTGAGCCCAGGAGTTCAA
CACTAACCTGGGCAACAAAGCGAGAGCCTGTCTCTACAAAACATTAAAAAATTAGCCATACATGGTGGAGTGCGCCTGTGGTCCTA
GCTGAGGTGAGAGGATTGCTCGAGCCCAGGAGTTTGAGGCTGCAGTGAGCTGTAATTGCACCACTGCATTCCAGCCTGGGTGACAG
AGTGAGACCCTATCTCAAAAAAAAGGAACTATCTGGCTTCATCCTAAGAGTAGTTGCATTTTAAACTCTCAGTCAGCTTCTTTTAC
TGTAAAATGGAATAATAATTAACAGTCTAAATTCCTAAAGTGATTATGAAGGCTGAACAGGTGATGCCTGTTCCTCAGGCCCCTC
CCTTGGGGCCCCTTCAGCCCCATGTGTCCCAGGCTAATCTCAAACTCCTGGGCTCAAGTGATCTGCCTACTTTGGCCTCCCGAAGT
GTTGGGATTACAGACACGAGCCACCACTCCCAGCCAGGTTGCTATTAAATTTTACCTTTTTATCCAGGAGCGTGGGTCATCATTTC
TTTCAAATAATGCTTCTATTTATGAGATTTTTCTTTGTAGAAAATGTGGAAATGTTAAGAGTATAAGGAAGAAAATAAGTCGTGT
TAACGCAATTGCTGCATTTTTCTTTTTTTCTTTGCATATTTCTTTTAAACTTGAGATCATGTAATGTCAACAAGTTTATTGGATTT
TTCTCCTAATGTAGTATTAGTATTTCCATGTGTTGTAAAGAGCAAGCTTTTAGGTATCATTTTTAATGCAATATAATGTTGTAACA
TCCTATTGCAATGACAGCTTGTCTTTCTCCCAATTTTATCTGCACTATTAGGTATTAAGAATATTTAATGTGGCCGGGCATGAAGG
CTCCTGCCTGTAATCCCAGCACTTTGGGAGGCCGAGGTGGGTGGAACACTTGAGGTCAGGAGTTCGAGACCAGCTTGGGCAACATG
GTGAAACCGCATCTCCACTCAAAATACAAAAATTAGCTGGGTGTGGTGATGCATGCCTCTAATCCCAGCTACTCGGAAGGCTAAGG
CTGGAAAATCATTTGAACCCAGGAGGTGGAGGTTGCAGTGAGTCGAGATCATGCACTGCACTTCAGCCTGGGTAACAGAGCCAAAC
ACTAGCTAAAAAAAAAAAAAAAAAAAAAAGAACATTGTGTATTTTCCTCTTTTAAATAATGTTGAGATGAGTGTTTTGGTGTATA
TGTCTGTGCATGTGTGCACATATGTGTGCAGAAACCTATAGGATAGATTTCTGCAAGAATTATCAAGTCAAAGGTTCTAGACATTT
TTAAGGCTCTCAATGCATGTTGTTCAGTTGTTTCCCCATTTGGTTACACCAAGTTACAGTCACAAAATTACACCAAATATTTTTTC
CTCCATGGAGTTGCTTTCTCATGAAATTGGGTTTCTGAGGCCAGGAAGACTTGGGTCTTGTTGAGGTGAAGGGGATAATTCTTCAG
CATTTGTCACTATTTTTCACTATCATTTTGAGTTTGATTTAAATAGCACAGTTTGCTGCGTGGAAAATGAGAGTAGCTTTGAAGGG
GCACAGCTTTGAAGGGCACACAGTAGATGATGACATCTACTTCAGAACTCTGGCTGCTACGTGGGGTACGACCATGTTGCATTTTC
AGGATGCTTTAGTGGAAATCAAAAGCATGGGATGCGAATGCAAAACCAAAGGGCTTAGGTTCTGCATCTGACATATGTGGCTAGCT
GTATGTTCCTTGCCGCGTCACTCAACCTTGCTCAGTACATACCTTCTTCATATGGCGTGCATCTCTTCCTCAGCTATATGGGTGTC
CTCGAAAATGCTGAGCATAAGAAAAGTGCATTTGTATCCTCTTTATTTATTTATTTATTTATTTTTTGAGATGGGGTCTTACT
CTGTTGCCCAAGCTGGAGTGCAGTCGCACAATCATGGTTCATTGCAGCCTCAAATTCCTGGGCTCAATGGATTCTCTTGTCTTAGC
CTCCTCAGTTGCTGGGACTACAGGAGTGTGCCACCATGCCCAGCTTTTTTTTTTTTTTTTTTTTTTTGATAGTAGAGATGAGGTC
TTGTTATGTTGCCCAGGCCAGTTTCGAACTCCTTGGCTCAAACAATTCCCCCTCCTCTGCCTCCCAAAGTGCTGGGATTATAGGCA
TGAGCTACCGCTACACCCAGCCTATCCTGTTTACCTACAACTTGCCAGTAACCTTGGGCAAATTGCTTGGCCTCAGGATCTTGGTT
TTGTTTTCTGTAAAATGGAATAATAATAATAATAATAGTCCCTAATTCCTAAGGGGATTGGAAGGTTGAACAAAGTGCTTCCGC
TAGATTGCTTGACCTGGTGCCTAATCCTGGTGATTTCTCGTAAAAATCAGTTGTTCCCACTAGTGGAGTCCACCAGACCTGATGAT
TCCCCAGCGTTGAGTTCAGCCAACCTGCACTTTAGTTCTTCAGGCTGGTTGTATATGGCTGGCTAGATTCATCGTTTGGAATCTTC
AGAGTCAGAAGGGACCCATCCAAGTTTGCCACTCAATGGATGACAAAGTAAGGNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNN

NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNCCAGTACAGATGTCTGGCATTTTATT
GCAGACAACATCTGGGCAGATGACAGCATCTGGTATTTTTATGATTGTCACTTACCAGAGGTATGGATAGACATTTCTTGAAGAAG
ATAGGAGCAAGCCTGATTATTTGAGGAACACGAGGGCCAGATAGCAGAAGTGGAGGGAGAGGTGTGGTTGGCTTGGCAGGTGGAGA
GGGTCAGGGATGGAAGCAAGGAGCTTGCTATGTGGGGGTGTGGGAGTGGGGTTGGGGAAACCATATTGCTTTATTTCAGGAGAGAG
TGATGTGGCCTGTGTCTTGGTGGTAGCAAAGAAAGCGGAGAGAAAAACGTAGATTTTACATTTATCTGGGCAATTGTGCCAATAGG
ACTTGCACACGGATTGGATATGGAGGTGAGAAAATAAGGGAATCAAGAATGACTCCTACGAGTCAGGCTGGGTGGTTCATGCCTAC
TATAGTCCTAGCACGTTGCGGGGCTGTGATGGTTAATACTGAGTGTCAACTTGATTGGATTGAAGGATGCAAAGTGTTGTTTCCAG
GTGTGTCTGTGAGGGTGTTGCCAAAGGAGATTAACATTTGAGTCAGTGGGCTGGGGAAGGCAGACCCACCCTTAAGCTGGTGGGCA
CAATCTAATCAGCTGCCAGTGAATATAAAGCAGGCAGAAAAACATGAAAATGCAAGACTGGCCTAGCCTCTTAGCCTACATCTTTC
TCCCATACTGGATGCTTCCTGCCCTCAGACATCGGACTCCAAGTTCTTCAGTTTTGAGGCTTGGACTGGCTCTCCTTGCCCCTCAA
ACTTGCAGACAGACTATTGTGGGACCTTGTGATCGTATAAGTTAATATTTAATAAACTCCTCTTTATATACATACGTGTGTGTGTG
TGTGTGTGTGTATATATATATAGATAAAACTCCTCTTTATATACATACGTGTGTGTGTGTATGTATATATCTATATCTATATCTAT
CTCTCTCTCTCTCTATATATATATATATCTCCTATTAGTTCTGTCCCTCCAGGGAACCCTGACTAATACAGAGGCTGAGGCAGG
AGGGCCGTTTGAGGTCAGGAGTTCAAGACCAGTCTGGACAACTTAGAGAGACCTTGTCTCTACAAAAAATTAAAAAAAAAAAAGAA
TGACTCCTAGGTTTTTGGCATCAGCAACCTGGCAGATGCTGGTGTCTTCAATGGACACGGGGAAGATGGGCAGTAGATTGTGGGCG
CAGAGAGTTCTGTGCAGGCATGTTAAATTGCAGATGCCCATTAATGGCCACAGGAGATGCCAACTAGGTACTTGGCAAAGTGAGTC
TGGGAATTGAGACCATCATGGTAGGAGATAGCATTTGGAAGATTTTGGCAGAGGTGGGATCCAAATCCATGGGAGTGGAGTATAGA
TAGAAAAGTGGACATAGGACTAGACATGGCCACTAGTGGTAGGGGTAGAAGTTGGGGAGCTGGAAAAGGTGATTACAAGGAGCCAC
TGGTGAGGCAGAAAAACATGCCGGGAGCCTAGAGAAGGAAGACCTTCTTAAGGAAGGAGGAGAGTCATCACTTGCAATGTTGCTAA
GAGGTCGGGTAGAATGAGTCACACAGAAGTGACTGTTAGATTTGGCATCAAGGAGGTTGTTGACTAACTTGGCAAGAGCGCCTCAT
GGGAGCAATCAAGAGGAAGCCAGTGACCAATGAAGGTGACTGCTCTTGCGTTCTGCTCTCTCTTCATTGCCTTTTGCTCTTTGCTT
AGGCAAATCAGTGTCTTCAGTAGGTTCCTTGAAGGCTAATGTCACCATTAGTATTTACCTACCCATTAGCTCATTTGATTCTCAAA
ACAACATCTTTGCAGAAAAGGCTGGGCAGGGCAGGCAGGTATTATTCCCATTTTACAGATGAGAAAGCCACAACTTAAAAACGTCA
AGTGTTTTTTTTTCAGGCTGTTTCTTAAAAGTAAGAGTTATGTGTCAGGCACCCCTCCTCCACCCCCATCTCGGCATACATCCCAG
TGTTTTGTGGTTTGCTACATTTCTTGGTTTTCTTAGCTAGATTGTGAACCCAGTGAAGGGAGGATCTGAATGACATCTCTATATTC
CCACGCAGAGATAGCAAATGCTTGCCACACTTGTTACTTTCTCTTGGGTACCCATGGCAGGCATCACTAGCTGATCATGACCTTCT
CTTCCTTTGAGCCCAGACTCAGCTTAGAATTCTACCCAGCATTCCAGATAGCCTGTCAACCTGCTGACAGATAAGGTATCATCAAC
AGGCATTTTTACTGTAGTACTTAATGTCATGCCTGGTTCAGCAAGTGGTTTTGTGACTGATTTAGCAAATAAGCATTCTCTGAAAA
CACAGTGAGTGAGTTCTATGTCACTAGAGGTAATCAAGAAGAGATCAAATTCATGCACATGGCCGGGTGTGGTGGCTCACGCCTGT
AATCCCAGCACTTTGGGAGGCCAAGGCGGGCGGATCACGAGGTCAGGAGATAGAGACCATCCTGGCTAACATGGTGAAACCCCATC
TCTACTAAAAATACAAAAAATTAGCCGGGCGTGGTGGCGGGTGCCTGTAGTCCCAGCTACTTGGGAGGCTGAGGCAGGAGAATGGT
GTGAACCCGGGAGGCGGAGCTTGCAGTGAGCTGAGATCGCACCACTCCACTCCAGCCTGGGCGACAGAGTGAGACTCTGTTTCAAA
AGAAAAAAAAAAAATTCTTGCACAATTGGATAAGGTAAATAGAATTCAGGTATTGGGTAAAAGGTTATCCAACTCTGAGATTATCT
GATATTCATGGAGTTATTTAATGAACAGTAGTATCTCTTACCATTCTGGAACAAAACAAGAAACCTTTGAGAGGAATCTCCCCCCA
CCTCCCCAGATATATAGGAGGGGACAGTGGGCAACACTATTATGGCTCCTTTCTTGTTGTCTTGGCCACTTGGGGCTCTGGTCTCA
GCCTTTCTGGATTGCCATAATCTGTTGGGCCCTATGGAGCCAAAGGGAGTAAACAGAATAGTACTGTTCTGGGTTGTTGAGGGATT
CGGTGAGATTCCCTAGGTAGGTTTTGGTCAACAAGCAGGCATTTCATAATCCTCTCTACCTCCTGCCCTGCCCTGGGGATTGTGAG
GATTGTCTGCATTCCTCTGTGGAACTGGAATGTGGCTGCAGTGATGGTGTCTGCAGAGATGTTGAAGCTGCTCCTCTCTCCTGCCC
TCTATCTTGGCCTCGCCCGGTCCTAGCCGGGGTAAGTGAACATCAATCCCATGGACAGAGTCTTCTCTGAAAACTGCTTTATGGAT
TTTTTTTTTTTTCCCAGACAGGGTCTTGCTCTGTCGCCCAGGCTGGAGTGCAGTGGCACAATCTCACTGCAACCTCAGCTTCCTGG
GCTTAAGTAATCCTCCCACCTCAGCCACCCGAGTAGTTGGGACTACAGGTGCATGCTATGATGCCCAGCTAATTTTTGTATCTTTA
GTAGAGACAAGGTTTCACCATGTTGCCCAGGCTGGTCTTGAACTCCTGGACTCAAGCGATCTACCTGCCTCAGCCTCCCAAAGTGC
TGGGATTACAAGCCTGCCTTATGGATCTTAATGTCTTGGCCCGAGGGCATTGGCTCTGCCTGGCTCCAGAGGGATATGAAGCCATT
CCTGGGGGACACCATTGTTTTCTAGTTAATATTATCAGTCCTGTAGTCCAGTGATTTTCAAATTTTAGTGTAGTCAGAATCACCAG
AAGGGCTTGTTGAAACCCAGACAGCTGGACCCCTCCCTAGAGACTTAGGATGAGGCCTGAGAATTGGATCTAACAAGCTCACAGGT
GACACTGAAGTTGACGACCTTGTATCTCACTTTAAGCAAAGTCTTCCCATAGGAACCATTGTTCCTCCCTGCCCATAGGTAATATC
TTCTACTTGGGCCCTCTGCTTCCACTGTGGCCTGCTTACAACCTCACCTACACTTATTAACTAGCAGAGTTCACTGAGTCTGCTTA
AAACCTTAAACTTCAAACTATACCACAAGGCTACAGTAGCCAAAACAGCATGATACTAGTACCAAAACAGAGATATAGACCAATGG
AACAGAATGGAGGCCTCAGAAATAATACCACACATCTACAACCATCTGAACTTTGACAAACCTGACAAAAACAAGCAATGGGGAAA
GAATTCCCTATTTAATAATGGTGTCAGGAAAACCGGCTAGCCATATGTAGAAAACTGAAACTGGACCCCTTCCTTATACCTTATAC
AAAAATTAACTCAAGATGGATTAAAGGCTTAAACATAAGACCTAAAACCATAAAAACCCTAGATGAAAACCTAGGCAACACCATTC
AGGACATAGGCATGGGCAAAGACTTCATGACTAAAACACCAAAAGCAATGGCAACAAAAGCCAGAATTGACAAATGGGATCTAATT
AAACTAAAGGGCTTCTGCACAGCAAAAGAAACTACTATCAGAGTCAACAAGGAACCTACAGAATGGGAGAAAATTTTTGCAATCTA
TCCATCTGACCAATGGCTAATATTCAGAATCTACAAAGAACTTAAATTTACAGGGGAAAAAACAAACAAACAAACAAACAAAAACC
CCATCAAAAGTGGGCAAAGGATGTGAACAGACACTTCTCAAAAGAAGACATTTATATGGCCAACAAACAAATGAAAAACAGAAAA

CAAAAAACAACTCATCATTACTGGTCATTAGAGAAATGCAATCAAAACCACAATGAGATACCATCTCATGCCAGTTAGAATGGTGA
TCATTAAAAAGTCAGGGAACAACAGATGCTGGAGAGGATGTGGAGAAATAGGGACGCTTTTACACTGTTGGTGGGAATGTAAATTA
GTTCAACCATTGTGGAAGACAGTGGGGTGATTCCTCAAGGATCTAGAACCAGAAATACCATTTGACCCAGCAATCCAATTACTGGG
TATATACCCAAAAGATTATAAATCATTCTACTATAAAGACACATGCACATATATGTTTATAGCAGCACTTTTTACAATAACAAAGA
CCTGGAACCAACCCAAATGCCCATCACTGATAGACTGGATAAAGAAAATGTGGCACATATACACCATGGAATACTATGCAGCCATA
AAAAAGAATGCATTCATGTCCTTTGCAGGGACTTGGATGAAGCTGGAAACCATCATTCTCAGCAAATTAACACAGGAACGAAAAAC
CAAACACAGCATGTTCTCATTCAGAAGTGGGAGTTGAACAATGAGAACATATGGACACAGGGAGGGGAACATCACACACTGGGGCC
TGTTGGGGGATGGGGGGCAAGGGGAGGGATGGCATTAGGAGAAATACCCAGTGCATGCGGGGCTTAAAACCTAGAAGATGGGTTGA
TGGGTGCAGCAAACCACCATGGCACACGCATATCTATGTAACAAACCTGCGTGTTCTGTACATGTATCCCAGAACTTAAAGTACAA
ACAAACAAACAAAAAACCTCCACAAAAACCCTATCTCCAGGCTTCCCATTGTATCTCCTGACTTTTTTGAAATCCTGAGCAGCCTG
TTCCTGTTGATCGAGTGAATGAGCATTCTCTGAAATTGCAGTGAGTGAGTTCTCCATCACTAGAGGTAGTCAAGAAGAGGTTGGAA
TCACACACAATTGGATGAGGTAAATAGAATTCATTTGTTAGATAGACTATGCAGGCCTAAGATTGTTTAGTATTCACGGAGTTATT
TTGTGAACACTAGTGTCTTCCAGCCTCATCAAGTGGTCCCTCCCTCTGTAGCCCCACATCCTGGTCTTTGATCAGTTCCCTGACAC
CCTTCCCTGGGCACCACAGGACCTTTGCACTTGCTGGTCCCAGATCCTAGAATGCTTTTTCCCCAGCTTGTTGAAAAGCTGATTCT
CATCCTAACTTCAATATCACCTCCTCAGACCACCCCTTCTAATAGACTCATTCCCTAACCTCCATTTCTCATCACTGGATGCTGCT
GAATTTCACTGTGGCACCAGTCATAGTCTGTAAAAAAAATTTATTAAATTATTTGGCTCTTTGATTTTGGTGTGACTCCTCCCCTA
GAGAAAAGAAACTGACTTTGTCCTTGAACTCTGTGTTGTCTGACACTCAGTGGGTGGTTCATACATATTCTTTTTTTAAAATTAAT
TTTAATTTTTATTTTACATTCCGAGGTACATGTGCAGGATGTGCAGATTTGTTGCATAGGTAAACGTGTGCCATGGTGGTTTGCTG
CACCTATCAACCCATCACCTAGTTATTAAGCCCAGCATGCATTAGCTATTTTTTCTATGAAAGGTGAGCAAATGCTTGTATACAGG
CTGCAGAGGGTTAGGTCAAGGCTGAACCATGTAGTCTAAATTTTGGAGTGACTGCAGTGAAGCCCCTCCATGGTGGAAGAAAATGG
AATGTCCTTGTTGGAGCTGGCCAGAACTGGGCAGATCCAGAGGCTGGAGGTAATGGCAATAACTCTGACTGCTAATAATAAAATAA
GAACGTAAAAGAGTGCTTACAGTGTGTTACAGGCACTGCTTTAGTGCATTACGTCATTTAGTTTCCACAGTTGTCTCCATGTTAC
AGAGGAGAGGACTGAGGCACAGAGTATTACAATAAGAAACTTGGCCCACAGTCAGCGAGCAAGAAAGCCTAGATTCATACCTGGGC
ATATGGCTTCAGAGTCCACACGCTTGGCGCCTGCACTGCACCGAGAGGGGCTGCTGGGCCGGGAGTCCCCCACCTGCTCTCTGTTT
TCAAACTCCCCATTTCACACCCATGCCAGGTGTGATGAAATCCTCTAGGTGATGGGCAGTGGACATGAGTGGTTCTGGTTACAGTT
GGACTTCACTGGCCTGTCCCACACCTGAGGATATGGATTCTAGCCCATGGGACCATAAGCCAGGCCCCCTGAGGGTCTGTTATTTG
TCTGGGAAGTGGCAGCGCTCTTTGGAATCTTGTTCATGACTGACAAACACCCCCACGCCCCTTCAGCCATAATTATCACCTAGTCC
CCAGAGAAGGGCCATGGGTGGGCTGACTGTAGAGATAGACGCTTGGGTCTCTGGCTGGGAACACCACATGCAAGAGAAGAGGCCAT
GGGCTCGCCTTCCATCCAGCTGAAGTCTTTCTGGCCGGGCTGCAGGCTTCATGCAACACCTTTCAACCTGTAAAAGGAAAAAAGAA
GGCCGGGGGTGGGCCTCAGCTGCTGCCACTGGCATTGTATCACTGAAGCGTTTTTGTTGTTGTTTTTTTTTTTTTGAATACCGGA
AATTAAGGGGAAATTCGGAAACCTCATAATGGCTTCATGAACCATTGCACTTGGAAAATGTACTTTTGAAAAATTCCCTTGATTTA
TTGTTTTTCGGAGGCTGTAGATAACATTAGAGTATATTATTCCTATATAATTTCCTGAGTTTTCTATGTCAATTACTTTGTTTTAT
TAAAGAGAAAAAAGGAACTAATATTGATTGAGGCCCTCCTGTGTGCCAGGCTAAANNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNTTGCAAGGCTATGGACAGATACTGGTAATGGAACCTGTGCTCAGAATTGTTGGAATCTGTAGTTTAAAGGT
TTGTCATTTGGAGGAATATCAAGTAAACCCTATGGGGTCAAAAACAAGGTCTAGCCAGGTGCGGTGGTTCATGCCTGTAATTCCAG
CATTTTGGGAGGCTGAGGCGAGTGGATTACCTGAGGTCAGGAGTTCAAGACCAGCCTGGCCAACATGGTGAAACCCCGTTTCTACT
AAAAATACAAAACTTAGCTGGGTGTGCTGGCAGGCACCTGTAATCCCAGCTACTCAGGAAGCTGAAACAGGAGAATCACTTGAACT
CGGGAGGTGGAAGTTTCAGTGAGTCGAGATGGTGCCACTGGACTCCAACCTGGTCAACAGAGTGAGCCTGACTCTGATGATAGTAA
TAAATAATAATGGTAGCTTCTATTTATTGTTTACTACTATGTGCCAGACACTATTCTAGGTGCTTTCATCAATTAGCTCATTTCA
TCTTCGTTATAACCCTATGTTCTAAGTACAGTCATGTACCACATAATGATGTGTGGATCAGCAATGGACCGTATATACAACGGTGG
CCCTATGAGATTATAATACAGTATTTTTACTGTACTTCTTCTATGTTTAGATATGATTAGATACACAAATGCTCACCATTGTATTA
AGTTGACTCCGATGTTCAGTACAGTAACATGCCATTCAGGACTGCAGCCTAGAAGCAGTAGGCTGTACCATATAGTCCAGATGTGT
AGTGAGTAGGCTGTCATCTAGTTTTGTACAAACTCCCTCTGTAATGTTCGCACAACGAAATTGCCTAACGCCGCATTTCTCAGAAT
GTATCTCTACCCTTAAGCAACGTGTGACTGTCCTATTATCATCACCCCATTTTACAGATGAAGGAGCTGTGGCAAGAAATTGACTA
ACTTGGCCCAAGGTCACCCATATAATAAAAGGCGGGCTGGGTGCAGTGGCTCGTGCCTGTAATTCCAGCACTTTGGGAGTCCAAGG
GGAATAGGATCACTTCAGGAGAGGAGTTTGAGACCAGCCTGGGCAACAGAGGGAGACCCTACCTCTACCAAAATTATAAAAAAATT
AGCCGGGCATGGTGGCATAGACTTGTGGTCCCAGCTGCCCAGAAGGCTGAAGTGGAAGGATGGCTTGAGCCTGGGAGGTCAAGCCT
GCAGTGAGCCGTGATTGTGCCACTGCACTCCAGCCTGGGTGAGAGAGTGAGATCCTATCTCAAAAAAAAAAAAATAAAAAAAAAATG
AAAAAAAAAATAAAAAAAAATAAATAAAATAATTAAAAAAAGAGCTGGGACTTGAAGCCAGATAATAAGGCAGGCAGAGGGCATAC
GAAACACCCAGATGGGTCATGAGCCCGTGCATCAGTCAGGGTCTAATGGCTCAGCTGAGGAAGGGAGGTTTAAGGAAGAGACTGTG
GACAGAGGTGTTAGGGAAGGTGTCAGAGAAGGTTAAGGAGCCAACATGGATCATGGGGGTGGTACAGTGTTGCCAGGGCTGGGGAG
GATTGGCTGCAGTGTGGGGTACCCAGCCGCTGCCATGTGGAGAGGGACCTGTCACTCCTGCTGTGAACTCTCCCTTCTTCTGCCCT
CTGACCTCCTGCTGGTGCCTCCCATTGGCTAAACACAGTTGATGGCCAGTGCACTGGGGAGCTGTTCTTGGAGCCCACAGGCATCT
GCTTCTTGGCACAGAGCAGACAATGGATTGAGTCGGGAGGGAGGGGAACTAGAGAATACCCAAGTCCCAATGGCTGGAGAGGAGTG
AGCAGGTAAAGGGGGTAGAAGGTGAAGTCAGAGAGGCCGGGGTCCACACTACACAGGGCCTTTGGCTTTCTTCGGAAGGAGCAGGA
AGCCAGTGGAGGGTTTGAGCAGATGTGTGCATCACGGCCACTGCCATCAGGAGAACGTCTTTCTCTATGCTAGGTGTGAACAGTCA
CCCCTTCCTCTCCCTTTCTCAGGATGAGTGCTGAGGAGGAAAAGGCCCTTTCCTCCAATGTCTTCAGAGGAGACGTCTGCCCAGTC
CAGACTACGTGCCTCATGCCAGCCAGCCTGTAAATGACAGGAAAGAGAAAGGAGCAGTGTCTCAAAAGAGGATATTCTGGAACGTT
TGATAATCAGAGCCCATTGTAAAGTCATGTTTTGAGTTACAGCATTTAAATCTTTGTTCCAGTGGAAAACTTTTACAGGCCGTCCT
GGGCAAGGCTTGAATCCTGTTTGGATCTCCAAAGGCCTTAAAATAGTGTGGGATGCCGGGAGACTGGCTCCAAAAGGGAGGAGACC
TGTGCTGGCAGCCCTGGCCACACCTTGGTGGGACCGGCTCCTGGCCTTGGGGACCCTGCCACTCTCCTAGCTGTGCTTCCGCAGAG
CTGGACACACAAAAGCTTTCTTGTCTGGATTTGTTCCCCTAAGAGGGTGGCACCGAACTGGTCCTGCCCATGCAGGAAGCTGGTCC
TGTCCAACCAGGGAATGAAAAGTTGGCCAGTATCTTCTCAGGCCACGCTACAGCCAAGTCTCTTTTAATGAGCTGGTTCTCTTAGG
ATGCCCAGGGGAGGGAGAACACACTTGAGTGGGATGGCGAATGAAGAAGGAAGCGGCTGTTCAGACAGCTTCCAGTTCTGTCAGGT
TCTGCTGCTGTGACCAAGGCACCAAGTCATCTGGAAACCCACTGCAGGCAGCAACCTCTTTTTCACCTTTGTGTGCCCCAAAGCTT
GCTCCGGGAATCGATGGTTTGGGAAACAATGCTTGCTGTCTTTGAGTCCTCAGTCCTGTAAAATGGGGAAAGTATACATCAGAGGG
CATTTGAGAGTAAGGCAAGAAGCGCTTGGCATGTGCCTGGCACATAGTACCTGTTCAGCAGATTCCCTTTCAGCTCAGGGTTAGAT
CTTCATCCACCTCTTGTTGGCCTGAAGGAAGGGGAGGTAGGTACTATTAGCCCCATTTATTTTATTTTATTTTTTATTTTTCTTG
AGACAGTCTTGCTCTATCACCCAGGCTGGAGTGCAGTAGTGCCATCTTGGCTCACTGCAACCTTCACCTCCCAGGTTCAAGTGATC

TTCCCGCCTCAGCCTCTCAAGTAGCTGGGATTACAGGCATGCACCACCACGCTTGCCTAATTTTTGTATTTTTAGTAGAGAGGGGC
TTTTGCCATGTTGGCCAGTCTGGTCTTGAACTCCTGACCTCAAGTGATCTGCCCACTTCAGCGAGATTATAGGCGTGAGCCACCAC
ATGTGGCTTATTAGCTCCATTTTATAGGAGGTGAAATTAGACAGCAAGAGGCTAAATGCCATGGCTGAGGTAAGATCTGTGGTAGC
ATCCAAGTCTTGACCCCAGACTTGGTGTACTTCTCATCACAGATGCAATTTTGTGCCTCATCATCTGACCCAGGAGCTACACATCT
GCTTTTTTTTTTCTTCTTTCTGAGACAGTCTCACTCTGTCACCCAGGCTGGAGTGCAGTGGCACAATCTTGGCTCACTGCAACCTC
TGCCTCCCAGGTTCAAGTGATTCTCATGCCTCAGCCTCCCAAGTAGCTGGGACCACAGGTGTGCGCCACTACCCCCAGCTAATTTT
TGTCTTTTTAGTAGAGACGGGGTTTCACCGTGTTGGCCAGGCTGGTCTTGAACTCCTGACCTTGTGATCCACCCTCCTCGGCCTCC
CAAAGTGCTTGGATTACAGGCGTGAGCCACTGCGCCTGGCTAAAGTTGTTATTTTAAAATTATTATTTATTTATATTTGTTATTAT
ATTTTTTATTTATATATATTTTGTTATCTTATTTTTAAAAGTTGTATGGCCCATTTAAACTTTTGCAGAAATAGTTTTTTGAATCC
ATGGATTTAATGCTTTCCTCTTTAAATTTTTTTAAGTCAATTTAGAGTTTTTAAGACAAAAACTGGAAAACGGCTTTGCTCTTTT
GTGGAACTGCTCTGGATTTTTTTTTTTTTTAAGAAAGAATTTTGCTCCATCACCCAGGCTGGAGTGCAGAAGTACAATCATATCT
CACTGCAGGTTTCAACTCCTGGGATCCAGTGATCCTCTTGCCTCAGCTTCCTGAATAGCTGGGAATACAGGTGTGCGCTACCGCAC
CAGGCTATTTTTTTTTTAATTTAAATTTTTTTTGTAGAGATAGGGTCTCTCTTTGTTGCTCAGGCTGGTCTTGAACTCCTGGCTTC
AAGCAATCCTACCACCTCAGCCTCCCAGCGAGTTGAGATTAAAGGTGTGAGCCACTGTGCATGGCCTGTGTTGAATTTTTTGTCTC
CCCATTCCCAAGTAGCAACTCTGGACTCCACAGAAAGGCCGAGTCTCTCCAAGACCCAGCCGTCTTGGAGGGTCATCTGTCCACTC
CTCCAAAGAGGGTGAGACAGGGCCAATAATGAAGTGATCACTGTGCACCAACCCTCCCTCTTGCTTCAGAAGAGACCTTTTCATCC
CTGAGCCAATTAATCAATTTCAAGAGATAGCATCTGAGTGAGATGGGTCACCAAGTCACAGAGGCCCTCAGCTCCTGTGCCGGCTC
TCAAAGGGGGTTGAATGAGGTGGCCTTAACAGATTGCCTAGTACCCAACTTCCTAGAGTTCGTATAGTAGAGAGCAAGTAGCATAG
GGCTAGAAGGCCAGGTCACAATACCCTCTCTGGAGTCTTCTGAATGTATGACCAATAGGCAAGAGCCCCAGTTTCCTGGTCTACAG
AAGTGACAGATGGGACCCTACCTACCACACAGGTTCTGAGGATAAAAATAGTGATATGGGCTGGGCACGGTGGCTCGTGCCTGTAA
TCTCAGCACTTTGGGAGGCTGAGGCGGGTGGATCACTTGAGGTCAGGAGTTCGAGACCAACCTGGGCAACATGGCAAAACCCCATT
TCTACTAAAAATACAAAAATTAGCTGAGTGTGATGGTGCATGCCTGTAATCCCAGCTACTCAGGAGGGAACTGCTTGAACCCGGGA
GGTGCAGGTTGCGGTGAGCTGGGATCATGCCACTGCACTCCAGCTTGCGCAACAGAGCGAGACTCTGTCTCAAAAAACAAACAAAC
AACAAAACAGGTAATATGGTTGGAAGAGGTTTATCCTTGCCAAGTTTTTGTAAATCAAACAGCTCTTGAGAGATGCCTGTTTGGCT
GTTTTTATTTTTTTAGAGATGGAGTCTTGCTCAGTTGCCCAGGCTGGAGTGCAGTGGCGCGGTCTCGGCTCACTGTAAGCTCCGCT
TCCCGGGTTCACGCCATTCTCCTGCCTCAGCCTCTTGAGTAGCTGGGACTACAGGCGCCCGCCACCACGACCGGCTAATTTTTTTT
TGTATTTTTAGTGGAGACGCGGTTTCACCATGTTAGCCAGGAGGGTCTCGATCTCCTGACCTTGTGATCCACCCTCTCCGGCCTCC
CAAAGTGCTGGGATTACAGGCGTGAGCCACCACGCCTGGCCTGTTTGGCTGTTTTTTTAATAGGAGGTGGGAATCCCTTGTCTTCC
TGGGCAGACTAGATGTGATGTGTTCACAGGGAGCTTTGTACAGATTGCTTGAGAGAAGCACCTTTCTTTCCAGAGTGGCTGCTTGG
ACGATGCCTTCCAAAGCTGCTAAGTCCTGTGGCAGGGGATTGTTGATATTCTTCATGGTTTCTCTGGGGAGCCTCCTAGCCTCTGA
ATTATTGATGCATGTCTACATTATTATTATTTTTTTTTTGAGACGGAGTCTTGCTCTGTCACCCAGGCTGTAGTGCAGTGGCGCAA
TCTCGGCTCACTGCAAGCTCTGCCTCCCGGGTTCATGCCGTTCTCCTGCCTCAGCCTCCTGAGTAGCTGGGACTACAGGCGCCCAC
CACCATGCCCGGCTAATTTTTTTTGTATTTTTAGTAGAGTTGGGGTTTCACCGTGTTAGCCAGGATGGTCTTGATCTCCTGGCATG
TCTACATTATTAAAAGGGTCTCTTTGCATGAGTTAGCAGAATGTAGTGCCCTCTCGTGAATGAGAAAAGCCGCCCAGTCCTGATTG
CAGCCTCATAATGGGTCTGAAATGACAAGTGTGAAGCAGTGGGCAGAGCCAAGACCACCTTTATTTGTGCATCCGTGCTCTTGGGT
GGGTATGTTTCACACAGTTTGTTGTCCTTCTGTCCCTTGCCCATGAGGCCTTAGAAACTCAGCTTGTCTCCACCCATTCCTCTACA
ACCCACACAACAACTTGTAACCTTTAATCTTCTTTGCTTCCTAAAGATGCAACTCATGCCACCTCCCCCAGGAAGTTCTCCCTGA
CTTCCAATCTTTAGAATAGGCTGCCAGAGATCCCTGTGTTAACTGTGAACAGAGCCCTTTTCATCCTCTTGCATCAGAATTCCTGT
CTCATCCACTCGACTGAACTTCTTGGGTAGGAATGGAGGTGTGTTTTTTTTTTTGTTTTGTTTTTGTTTTTTGTTTGTTTTTAAGA
TGGAGTCTCACTCTGTTGCCCAGGCTGGAGTGCAGTGGCACGATCTCGGCTCACTGCAAGCTCCGCCTCCTGGGTTCATGCCATTC
TCCTGCCTCAGCCTCCCGAGTAGCTGGAACTACAGGCGCCCACCACCACGCCTGGTTAATTTTCTGTATTTTTAGTAGAGACGGGG
TTTCACTGTGTTAGCCAGGATGGTCTCGATCTCCTGACCTCGTGATCCACCTGCCTCGGCCTCCCAAAGTGCTGGGATTACAGGCG
TGAGCTACCACGCCCGGCCGGAGGTGTGTTTTACATGCATCTCACTGCCTGGGACAGCGCCTGGAACCTAGCAGGTGTTTTATGTG
TGGGGGGTGTTGGTTGTAGTAGTGGGAAAGGTTTCTTAAATGAGGTGTGTTTAGAATTTAGCCCTTTGCAGGGTATAAAATCTGA
GAAATAAGGTGTGTGTTGTGACGAGGGATGGCACTGGGGGCCTGCCTAAAGCACTGGCTTCATTGTGGGGGCTGAGGTGGCTTGTT
TCCTTGGAGCTAATGGTCAGAGTGAGGAGCTGGGGACTGAGCTTGGATCTAGATTATTGTTGCATGAATGGCCTAAATGAAGAGTG
TTTTCTCAAGTCCCTATAGAAGAGGGAGATCCTCATGGCTTTCAAGGAGTCATGCCCAAGATACAGGAATGGCTCTCTGGTGGACT
GGCTGGGACAGAGGGGAGAGGGTGAAAGTGGGGAGGAGGAGGAAGTTTAACAGTATTTGAAGCATTTGAAAGCCTAGACCCATCGT
ACGCTGAAACAGAGAAGAATGGCTAGCCAAAAATACTGTGTGAGTCTAGGAACTTAAGAATGCTAGTCCTTGACCCCAAACATACT
TATATAGCAGACTCAACATTATTGTCTTCCACCTACCGGATGGGATTATGCAAGTGATATGACCCAGAGCATTCTGGAAGCCGGCA
GGGCCATAAATAATACATTAGATATGCAGATGAACTGCCCAGGGTGTCTCTGGTCTTGGGAAGAATTCTTAGTAGACCTGAGATAA
TTGCAGAACAGAGTGTCCTGTTTTCCCATCCACAGCCTCCAGGCCTGGCGAGGATTTTGTGTTCAAAAGAGATTTCCAAGGTGGAC
ATCTCTCTGACACTGGCGATTTTTCTCTCTCACTTGAACAAAACTCGGTGGAAAGGTCACTCCTATTTGTCTTAGCTTCTCTGTCA
CATCCCACCCACACTTTGAAAGGTCTGTTCTTAGCGGGGTCCCAGGAAGGCTGTGCTGGCCCTGTCAGCCCTGAGCTGGGTGGTAG
TCCTTTATCAGAGATACCCATGAGAATCACTCACCCTGGAGNNNNNNNNNNNNNNNNNNNNNNGTGACTGGAGCTTTAAGAGCTACTG
AACTAAGTGGTAATAAAAGTGAACAAAGTAACCAGTCTGGAAATGTCAGTAGAATGGAAAGAAAGAACAGGTGGGAGAGTTCTCCA
TTTTTTCACGGTAAGACCCGGTTACTTGGCTTTTGTTGAGTCCCTGGTGGGTGTCCATGGTGTCTTTGGCCTTATCTCCCTACTAA
ACAGGATTACCTGTGTGGGGCACCTTTCCTGGTCTCCTCTCTCTCCAAGGAGCAGGTAGTACTTGGAGGCCGTGGTTAATCACATT
TCCTAAGTGAGCTTTTCCTTTCTAGAATAATCCTTGCTCTTACCCTTGCATTCCTACTGCATGTTCCCTCAGGCTGAAGCCTTCCT
TTACATGTGAGCATTCTCCTGGATCAACTCCCTGATGACTGGAGCCTGTGCCGTGTGTTTCCAAGTTGCACCCACCACCCACGGAG
ACTAGCTCATGGCCGAGTGGCAGCTGGTGTAGCTGATAGCTGTGTGGGAAATGGGTCTGCACAATATAAAGACAGCTCACAGGAAA
GGGGCCCCAAATGGTACTCTCTTTTTTTTTGAGATGTTGCCCAGGCTGGAGTGCAGTGGCGCAATCTCGGCTCACTGCAACCTCTG
CCTTCCGGATTCAAGCGATTCTCCTGCCTCACCCTCCCAAGTAGCTGGGATTACAGGTGCCTGCCACCACACCCAGCAAATTTTTT
TTTTTTTTGTATTTTTGGTAGAGATGGGGTTTCGCCATTTGGGACAGGCTGGTCTTGAACTCCTGTCCTCAGGTGATCCGCCCACC
TCGGCCTCCCAAAGTGCTGGGATTACAAGCGTGAGCCATTGCACATGGCCTTTTTTTTTTTTTTTTTTTTTTTTGTTGTTGTTGTT
GTTGAGAAGGAGTATTGCTTTGTTGCCCAGGCTGGAGTGCAGTGGTGAGATCTCAGCTCACTGCAACCTCCGCTTCCCAGGTTCAA
GCAATTCTCCTGCCTCAGCCTCTGGAGTGGCTAGGATTATAGGCGCCCACCACCACACCTGGCTAATTTTTGTACTTTTAGTAGAG
ACAGGGTTTCACCATGTTGGCCAGGATAGTCGAACTCTTGGCCACCGCGCCTGGCCCCAAATGACACTTTGATAAGTTGCTCTACC
CAATCCTAAAAAAGGAAATACAGATAAAAAATACCAACGTTGTATAATACACATGTTGTTGATGCGCTCTGTTGTTGGCAAAAGCT
GTGTTGCTGGACGGAATGCAAGTGGATGTGGCCCTTTGGAAGAATTATGGCCTTTTGGCATTCTCTATCCAAACTACAAAAGCAT
GTGCCCTTTGACCTGGCCATCTCATGTTGGGACATTGGGATATTTCCCCTACAGAGATAACCTATAGATATTTGTAGGAAAAGAAC
AGTGCCCAAGGTTATTCATGGAACATTTTTATAAAAGCAAAAGACTGGGAGTAACTCAAACATCTGTGAGTTGAAGACTGGCAAAT
GATGACACATTGTGCTAGGCAAGATGCGGAAAGGAATAAGGAAGTGCTGTGTGCACAAACACGGGGAGATCTCAGGATGTATTGTT
GACACAAAAAGCACAATGTGGAGCGGGGAGTATGGCATGCCATCTTTTCTGTAAAAAAAAAGCATGGGTGTAGAGGGGATGTAAAA
ATGTGTCTTTGCACAAACTCTGTTAGGATGCACAGGAAACCAACACAAGTGGTGGCCTGTGCGTGGTGGGAGGAGGGAGATGCAGG
GAGGGGAGACTGGGTTAGGGAGGAAGATGCATGAAGAAGATTTTTTTTTCTTTTCAGAGATAAGGTTTCACTCTGTGGCCCAGGC

TGGAGTGCAGTGGTGTGATCATAGCTTGCTGCAACCTCAGACTATGAAGGAGACTCTTGATTGCGTATCTTTGGGTATCATTTGAA
TTTTGAAGCATGAATATATTGAAATCGGGAGCAGCTCAAACATTGAGTATATTATCTATCATACATTTAATTTAAAAAGTTTATAA
CCTTGTCATGATTAGGCTGGGGTTATGCATTTTTAGGAGGGAGACCCCAGAGGTAAAACGTCAGTTCTTATCACATGTCAAGGTTA
TGTTCTATTCACGCGACTGATATAAATCCCGGTTGCATATCCCTATAATACACCTGACCAAAGCCAGGTGTGGTGGCATGTGCCTG
TAGTCTCAGCTACTGGGGAGGCTGAGGCAGGGGGTTGCTTGAGCCCAGGAATTCGAGGCCAGCCTGGGCAACATAGCAAGACCTCA
TCTCTCCAAAACCACAAATTTGGCTGGGCGCAGTGGCTCACGCCTGTAATCCCAGCACTTTGGGAGGCTGAGGTGGGCGGATCACC
TGAGGTCGGGAGTTCAAGACCAGCCTGACCAACATGGAGAAACCCCATCCCTACTAAAAATACAAAATTAGCCGGGCGTGGTGGCA
CATGCCTGTAATCCCAGCTACTTGGGAGGCTGAGGCAGGAGAATCGCTTGAACCCGGGAGGCAGAGGTTGCAGTGAGCTGAGATCG
CGCCATTGCACTCCAGCCTGGGCAACAAGACCGAAACTCCGTCTCAGATAAAAAACAAACAAACAAACAAAAAAGGCCAACAAATT
TGACCAATACTCCTCAAAACTGGAAAAGCTGGTGACCACCACCCCTAGTGCTCCAATAAAGGGCTGTGGGAACAGTTGTGCTCAGG
GTGGTACTGGTACAGCCTGGCAGAGGACATGATGCTTGCATAGTGTGGAAAGGCCCTCTGAGATATTGCTGGGCCTTAGGGGAGGA
GGTCCAGGCCTAGAGAGGACTGAAGCCCTCCCCAGGGCATGGTGTGGACCACGCTGAAGGGAGGTGGGGTTGGAGACACAGCTGGG
AGTCAGATCCCAGGGGCTCTCCTGTGTGATGCTGAATTGCTGGGAAGGGGATGCCAGCTAGTCTCTTTCTCTGTGCCAACCCTGAT
TGGTTGGGTGGCTGCTGAGAGCCTTATGGTGCCCTGGCTCAGTAGGAAGAGTGTGTGTTCATTTAGTGAGTTTGCCAAAGACAAGG
GAAGGGGGTTAGAGAGTAGGTGGCAGATAATGCCGGCCTGGGGATCCTGGAGGTTTTAGGCTGGCAGACCCTGGCTGAGGCTGTTT
TCTGTTCTTTTCCTTAGGCCTTGGGAGACCTTTACCTTTTCAGTGCCTCTAAGACAGGGTCAGAGCAGTTTGGTGACAAAGTGGAA
GTAAACCATGGGTGAAAAACAGCTCTGGGGACCCTCATAGGCTTCCACTTATTGAGGATTAGGCAGAACTTGCAGCTTGGCCCAGC
TTTCGAAACAAGGTGTTCCCAGGAGGTGGAAAGACTGGTTTTCAATCAGATAAAGGTAGGATCCTATGAAAAACTCCTAGGGCCTT
GCCGTTCCTATGAAGTGGTAGCTATCATTCACACTCCTAGTTATGCCATCGAGGTAAAAAGAGGTGGAATAAGAATCAGGGACACT
TGAAAATAAGAAAAGAGAAAGACTGCGTGCATGTGCATGGGTGCACAGCCTGTACATATGTGTGTGTGTGCCCCTGCGTGCACGTG
CATGGGTGCACAGCCTGTACATATGTGTGTGTGTGCCCCTGCGTGCACGTGCGTGGGTGCACAGCCTGTACATATGTGTGTGTGTG
CCCCTGCGTGCACGTGCGTGGGTGCACAGCCTGTACATATGTGTGTGTGTGCCCCTGCGTGCACGTGCGTGGGTGCACAGCCTGTA
CATATGTGTGTGTGTGCTCCTGCGTGCACGTGCGTGGGTGCACAGCCTGTACATATGTGTATTTGTGCTCCTGTGTGCACGTGCAT
GGGTGCACAGCCTGTACATATGTGTATGTGTGCTCCTGCTTGCATATGCATGGGTACACAGCCTGTAACTACATGTGTGTGAGCCT
GTGGGTATGTGAGGGGAGTGAGGGACCCTGAGGGACTAGAAACTTCCTTAAAGGCCATTTGAGAGAGTGCTGCTTTCTATACCACC
CACCCTAGGGCAGTTAGGCAAAGGTGTGCCTCCCTTTAAGAGGACCTAGCGGTCGGTCTGGTTTACAACATAGGTTATCAGGGAGT
AGTACTTTGGCCTGGAAAAGGAACCTTTATTTTGGAAAAGATTTGTAATCTCTGTGGCTTGTTTGATACACATTGGAAACCTGACT
GGAAACTCAGGCCACCAGCATCTGTTGGGGGCACATGTTTGCACTTTTGGTTATGATGCCACGGCCCTGGTTCAGGTATCCTAAAG
AAGATAATTAATGATGAAAATGATTGGTGTAGATTTCATACCACAGAGCACTGTGGTTTGTATAGTAAGACACAGTGATGGTGTGG
ACCCATGCACAGCCAGCTTTGCAGATGTTAAGAATAGCGACTTTGCCTGACATAATTGGTGTCTGCACATGGGATTAAACAAGTAA
GATTGATGTGAATTTTATTCTTTCATTTTTTATGTAGACTCACTGATTGGGTAACTGTGGAAATAATGTCACAGTGCCTTTTTAAG
TTAAGAACATGTAGTTGTGATCAGGAGCGGTGGCTCACGCCTGTAATCCCAGCAGTTTGGGAGGCTGAAGTGGGTGGATCACCTGA
GGTCAGGAGTTCAAGACCAGCCTGGCCAACATGGCAAAACCCCGCCTCTACTAAAAATACAAAAAGTAGGTGGGCGCCTGTAATTC
CAGCTACTGGGGAGGCTGAGGCAGGAGAATCACTTGAACCCAGGAGCTGGAGATTGCAATGAGCCAAGATCGTGCCATAGCACTCC
ACTTTGGGTGATAGAGGGAGACTCTGTCTCAAAAAAAAAAAATTAAAAAAAAATAAAAAAAGATAAAATGTAGTTATGAAAAGATTT
CTAAGCATTTTTAAGCATTTTATATTAAAACAGGAATTCAGAACAAATCAAAACGAAACAATAAAACTGGGGATGAAGGAAACTCA
ATTTTAAATATAAACTTTGGCAGGTGGGTGGTGAATCCGGGTGTTGTGAGTCATTTGCTGGCTTGTTTGTCACATGGCCTGCTCTA
TAAGTGTCACCCTAATTGATGTATTCGTTTGGAGTGGGTGCTCTTCTGAGGTTTTTGTTTCCCCCTTTCTTTGGGGTGCACTGTGT
GTGCATGGTAAGAACAAGGGGCCATGGAATTAGAGAATCTTGAGTTTCTAATGCAAATTCAGTTCCAAGCAGTGTGACCTGGTTGT
TTAACTCCTTTGAGCCACCCCCGCCCATGGCCCTCATCTGTACCCTGAGGATAATAGTGTGGGCTTTGCAGGCTTTTGGTGAGCAA
GTGAGATGATGTAGCAAAACACCCAGCCCAGAGCCTAGCACCAATTGGTCTGTAATCCATGCTGCACGGACACAGCCATTCTCTGG
ATGTGGCCTCTTCTGCCTCCACTGTGAGGTCAGAGACTGAGTCACTGCAGGAGTAACCTCTCCTTGGCAAGCAGCGGGAGTCATTT
CATCCCAGCCTTTCAGGAGGGTGAATCTGCACCTGGGGTCCAGAGTCTCAGAGATGAGACGTGAGCCAGGCGCTGATTCATCATGA
TGCAGGCTGTGGAGACTCTAGCCATGGTTTCTCCATGCAGGAGTGAGGTTGGGATAAGGGGTCTTTCTGGGGGCTCTGTGCTCTGT
GGCCCCTGCTGCTCCGGACTGGTTCATTGGAGAAACCTGTCACATTCTCTAGACCGGTTGCCACGCCATGCTCACAGTCTCTGTTC
TTGCCTTCCTAGGTGGGAAGTGAGTGATGACCCTGAAGTGAGGACTCATCTCTAGATCTCCAAGGGCTGCAGCTCAGCCAGCACTT
TACAAGGGTGATCTGGAGCCAAACTGGCCTGTTGGCTGACCATAGGTGACTCTGGGTAGCCCATACCCAGGCTCAGCAGCAGTTGG
GGAGCTGCCTCGATTTCTGGTTACAGAATTCCTGGAACTGAGTCACTGCAGTAATTGCTGTGATGAATTGTGTTTACTTTGTGTGG
GATTCCAAACTGTAGCAGCAGTGACTACAGCTGGAAGACAGCATGATCAGCAGCTTCCAAGGCAGAGCCTGGCGTCAGAAAGCTGC
ATTGCGCTAATGCTGAAGCCTGTGGGAGCCTGTTGGAGAGACACTTGGATGTTTAGCGAGCTGGTGACTCTCCTTGTCATGAGTAA
GCTTAGGACCTTGGGCAAGTCATCCAAACTCTTCTGGGCAAGTCATTCTCCTGCTTGGATGCCTTGAGGCAGAGAGGCAGTGAGGT
GAAGTGGTCAGTGCGTCGACTCTGCCTCTAGCCTGCTGGGGTTTGAATCCACCTGTGTGATGTTGTATGATATTGACCTTTCTGGC
TCTCAGCATCCTCTTGTGTGAAATAGGAGATTTTAACAGTATCTATTTCGTAGGGTTGGTGTTTGAATGAGTTAACATATGTAAAG
TGAATGGTACAGTGCCTGGCTTCCTGGCAAGATTGCTATCAGGATTAAGGCAGGTTAAGCCCTTGGCACACACTAAGAGCTCAATA
AATGTGAGCTGATGTTATTGGTCCTTTATTACTATTCAAGAAGCCTGCCCAGCCCTCCTCCCTCTCCATCCACACAGCAGCCTGGT
ACCCGCTGTTCTCTAGGTTCTGGACACACGTTATGACATGTTCTGATGATCTGGCTTAGACAGTGGGGCCCTCGAGGTAGGCCCAG
AGGACTTGGTCCTCACTGCCTCTGTGGCGCCTTGCACTGGGTCCAGCTGACGTGGAGAGAGACTCAGGAAACAGTGGCTGAGTGTG
ACTTTGGCTGGCATAGTGGTTGCTGAGAGAACAGACAAGGTTCTCTCTCACGACATACAGATTTCAGATCAGGGAAAGTCCCAGCT
GGCATAAGTTTATCGAGCATCTCCCATGGACAAGATCAGCTGTGGGTGGAGCCTTGAAGTACATGGTAGAAGGACAGCGAGTCTTC
CCAGGCCAGGGCTTCAAGTGAGGAGACAAGATATAGCCTCCCAGAGAATTCCTATAATGCAATCGTGAAAGAACCATACCCAGCAG
GAGGCCGGGGAAAGTGACTCCTGCAACTCTAGGAAGGCTTCCTGGAAGAGGTGGAACGTGAGCAGCATAGGATTTTGAGAGAAGAA
ATGGAATGGGCTGAGGGAGATTCTGCTGGTGGAGGTTCAGGTTGACCTAAGGGCTGGCAGCAGTGGAGGCCCCCCACGAGTGAGTT
TGAGGGGCCTCTTTAGCTCAGTCCAGTTGAGGCAGCAGAGCCTTTCCATAGGGGTGTGGTGTGACCTGAATGTTGGGCACGTGGTC
GTAACTGAGCTTTAAAAGTGAATGAGAGGAGCCATGCGTGATGGCTCGAGCCTGTAATCCCAGCACTTTGGGAGATCAAAGCTGGG
GGATCACCTGAGGTCAGGAGTTCGAGACCAACCTGGGCAACATGGTGAAACCCTGTCTGTACTAAAAATACAAAAATCAGTTGGGT
GTGGTGGTGGGTGCCTGTAATCCCAGCTACTCAGGAGGCTGAGGCAGGAGAATCGCTCCAACCTGGGAGGCAGAGACTGTAATGAG
CCAAGATTGTGCTGCTCTACTCTAGCCTGTCTCAAAACAAAAAACAAGAAACAAAAACAAAACAAAACAAAAAAACACTGTCTCAA
AAAAAAAAAGTGAATAAAGAGAGAAGAAAGGCGGGGCACGATGCTTTTTAAGTCTACGAGCATCTTATAGCATTGTTTACATCCCA
GCTTTTCACTGACCCTTCCTTTACCCCGCCCCCATCCCGTGGCCCTTGCTCTGTCCTGCCCTTTCTGTACGGCGTTTTCTCTTCC
CGGCTCCTCTGCCCCCAGCCCCACGTGCCAACTTGTTTCAGCCTGGCAAGGAGGCCTGCTGGTATCCCAGCCATTTCCTGCCCCAA
GGCCGCCCACCCTCCTCCGATCTAGCATGGCCTTGCCCTCCGTAGGCAGGCCTCACCCCTGCGGCAGCCGGGAAGCAGGGCTCCTG
TTTTGCCTGTTTACCCTGGCGCAGGACAGGTAGATCATGTAGCACTGCTGGGATGCCAGCTGCGTAAGTCTCCGGCAGTGGGCAC
AGGCGGAGGGCAGGGGGCATTCCTCTGGGCTGACTCGAGCCCGGCCTGCCTGGCACAAGACCCCCTGCTCCTGGAGGCAGCTCAGG
GGCTTGCTCATGGCTGCCCTGGCTCTGCGCTGGGCCGCTGAGTCATCGCTCAGCAGTGAGGCCTCTGAGTCACAGTGCAGCCCTGG
ACTGGACAGTGAGTGCCTTGTGGGACCCTCCTGGGCTTTCCTGTCCCTCAGAGCATTTAAAGGGGTCCCAAATTGTCATGTGACCC
TCTCTGATGCCAGAGAGACACTCTAGGTCTTTCGCGCCCTGGTCATTTTACAGAACTGTGGCTGGATGCCAGGCATTCAGAGATAC

ATTCCTCAATCTCGGTCCGCCTTACAGGATCCAGCATCGAAGAGTTCAGTTCAAGTCAGCAGCCGAGTTACAGGAAAGCAGCCAGG
GCAATGATTCAGCAAGTCCAGGCAGGAGTGAACCAGGCAAGATTTCGGGAGGAGAGGGCTGGGTCGCGTGTCCTGGCAGGGCCACA
GGAAGAGCATACACATCTCCTGCGTCTTCGTATTTTGTGAGTGTCTTGAAATGACCTTGAGAATCTACGCAATACCATGAAGAAGC
AGGAGCAGGAATACCATTTATCCATTGGCACAGGGTAGCATGCTCAGTGTGCACTGAGGTTGTGAATTCTTGTCCTGACCCAAAGA
TGCTGATGCTGTCCTTATACCCATTTCACGGTTGAAGAAACTGAAGGTGTAGTAGGAGAAGATACTGACTCAGAGGCACAGAGTTA
GAAAGCAGCATGTCTGGGATTTGACCCCAGACCTGTCTGACTCAGTTTACTATTTTAAACTACCCGATTCAAGTTAAGTTTCTCTC
CTCTCTCTCTGTCTCTCTTTCTCCCACGTGGCTGCTGGAATCTTCCCTAAGGACCATAGCTGTCTGCAGTGCAGGAAGCCAACTAT
TAAGGGGAAACAAAAGTGTTCTTAGGAGTCCTGCTGTCACTGTGGATTGAGCCGGTGAAGCCCTGAGGGATTTCATCGCTGAGGTG
GATGGAGAAGCAGCTGGGGGCCTTGGCTGTAAGGACCCCTAAGGGTTAGTGTCTGGAGCTTGGGGGATGGGAATGGGTCCCGGTGT
GCGGAAATTGGATGGCTCGCTGACCCCAAGGGGAAACCCTTGTTGACGCTGTAAAGTGTCTTCTGGCCTTAGAGAAACCCTCTAGG
GTTCAGGTTCCAGCTTCACCCTCCTTTAGTCAGGTGGCTTTGGGGGGGACATTTCTGCTGGCCCTGGCAGACACTGGACTAGAGCA
GTGTTTCTAAAGTGTAGCCCCTTCTCTCTCCCGGGAAGGGAGACAAGTGGGTGAGGGGATTGGAATCTGCATTCATAACACACTCC
CCAGGAGAGACTGGAGAGCACCTTTCCCGGTGAACTCCCGTCATGTCAGGCCTCAGCAGACTGAGCGGGAACCCCCACCAGCGAGG
TTGCTCAGGACAGCGTTTCTCTTGGGCCTGTGTTTGGAACAAGTATCCTTTGAACTAATCACTAACCTGTGAAAATGGATAGACCT
CGTGTGAAAATGAAAATGTCTGGCTTCCCTTCTGAGGAGCGGAGGCATGAAAGGTGTGGCCGGTAGGGGTGGGGGCCCCACACCTG
TGCAGCCGCCCTGGCCAGAGCCCACAAGCAGCGTCCCCTGGTCATGCCCCACTCCCTTCTCCATAATCTCGCCCCTCCCTGACCAG
GCCACCTGACGTTGCCATTTATATTCAAGTTCATTCCGTTGTTATTCTTTTAGAAAAGAGAGATGAGAAATATTTCTTGTGCCTAC
ATCTTTATCAAAAGTAGAGCAAGAAACTTGAGGACTCTGTTTCTAGAAAAATGAGAGTGGGCATCCCTCTTCTCTGAAAGGAAGAA
TTTCCATACCTGCCCTCTACCCAATGTGTAGACGAAAGGATGAGTGTCCTTTTGGTGTGTGGAGAGCTTTGATGGGGCATGTGATG
TGTAGGGCTTAGGGGGTATGCTGGGGTGGCTGGAAAGGTGGTTGGGTGTCCTGGGAAGGCTTTTATAGACACTGCGTGGCAGGCC
TAGGACTCTAAGTGCTTCCAGCCAGCTGTACATACGTATATATTTTTTTTTCTTTTTTTTTTTTGAGACGGAGTCTTGCTCTGGAG
TGCAATGGCATGAACTTGGCTCACTGTAACCTCCGCCTCCCAGGTTCAAGTGATTCTCCTGTCTCAGCCTCCCAAGTAGCTGGGAT
TACAGGCACCTGCCACCACGCCCAGCTAATTTTGTATTTTTGATAGAGACGGGGTTTCTCCATGTTGGTCAGGCTGGTCTCGAACT
CCCGACCTCAGGTGATCCTCCTGCCTCGGCCTTCCAAAGTGCTGGGATTACAGGCGTGAGCTACCACGCCCAGCCCCAGCCATCTA
TATTAACTCAATGGAATTCTTGTTGAGGACTAAAATGGAGTTTTTGTTGAAGACTAAATAAGTAAATGACAGTGGAGTATTGTCAT
TATCCCCATTCAATAGATAAGGAAATTAAGGTTTAGAGAGTCACAGTAACTGGTCCTGGCTACACAGCCAGTCAGGAGCAAGGCTA
ACATTTAGGCCTAGAGTGCTCTCCTAACCGCTATGCCTGGGGTGAGGAGGGAAACCTTCCATCCTTGTGTCATCCCATTTGATATA
TTAGTTACTGAAGCTGAAGCTAAAATAATCCGAGGGCTGGGCGAGGTGGCTCATGCCTGTAATTCCAGCACTTTGGGAGGCCGAGG
CAGGTGGATTGCTTGAGGTCAGAAGTTCGAGACCAGCCTGGCCAACATGGCGAAACCCTGTCTCTGCTAAAAATGCAAAAATTAGC
TGAGCGTGGTGGCAGGCACCTGTAATCCCAGCTACTCGAGATGCTGAGGCAGGAGAATTGCTTGAACATGGGAGGAAGAAGTTGCA
GTGAGCCAACATTGTGCCACTGTACTCCACCCTGGGATACGGAGCAGGACTCTGCCTAAAAAATAATAATAATAGTAATATAATAA
TAATCCGAGAGTGCTTACCATGTGCCTACATTGTTCTGGGATACTGAGATGAATAAGACCTCTTTTGGGCTCTTGATGACTTGGTT
ATAAGAAGAGGCAGCTCGGTTCCCCCAGGGACTCTCAACAGTTAGGGACAGGCTGGGGCATGGTGGCTCCTGCCTGTAATCCCAGC
ACTTTTAGAGGCTGAGGCAGGAGGATGGAGTATGTCCAGGAGTTTGAGACTAGCCTGGACAGCATAGTGAGGTCCCATCTCTACAA
AAAATAAAAAATTAGCTGGGCATGGTGGTGTGTGCCTATAGTCCCAGCTACTCAGTAGGTGAAAGAGGAGGAGATGGGAGAATTGG
TTGAGCTCTGGAGTTGGAAGCTGCAGTGTGCAGTGATCACACCACTGCACTCAGTCTAGGCCACAGAGTGAGACTGCCTCAAAAAA
CAAAACAAAACAAAGCAAAAACAATTAGGTACAGAAAGGGAGGAGCATCCACATTTTCTGACATTGTTTATTGCTTTTTTACCCTC
ATGTTCTCCCCTCTCCTGGCATTGCCTGGCACTTGGGAGAAGTTTCATAAATATTGGTCGAATGAATGAATGGATATTAAATCATT
TGTGTTTTCTTTTGTGAGTTGAAACCGTCTTCAAATGAGTCTTCAAAGTGCTCTGATTCGTATGGTGGAGTGTGATGAAGTTAAAC
TCTGGGTTGAGAAGAGAGGAAATGTGAGTTAAGGAAATAAAGAGAATGGAGTTGAATGTTCAGAATCTTTGGAGTGCTGTCAGGGA
TCCATGATTCTGAATTCAGTTATCATCTTTGGTGTGAGAAATCCCCAAAGATTCAAACTTGTCACCAGAAAGGTGGTCTTAGGCTG
GGTGTGGTGGCTCATGCCTATAATCACGGCACTTTGGGAGGCCAAGGCGGCTGGATCATTTGAGGTCAGGAGTTTAAGACCAGCCG
GTCCAACATGGTGAAATACAACATGGTGTATTTTGTGTCTCTATTGGAAATACAAAAATTAGCCGGGCATGGTGGCGGGTGCCTGT
AATCCCAGCTACCAGGGAGGCTGAGGCAGGAGAATCACTTGAACCTGGGAGGCGGAGGTTGCAGTGAGCGGATATTGTGCCATTGC
ACTCCAGCCTGGGTGACAGAGTGAGACTCTGTCTCAAAATAAATAAATAAATAAATAAATAAATAAATAAATAAATAAATAAAAGA
TGGTCTTGAGGTTTCGGTTTGGGCATAGGTTACCTTCTGCAGAATGCATGTGAGTGATATGGAGACAAGTTTTAACATGGGCACTT
TTTGAAAACAGACATCTTAAATATTGGCCAAAAGGCCAGATGTCAAAGTGGACTTTCTATTTTTGATGGAAAATTTCAAGCCTGTT
TTCTGTAAAGGAATGATTATTGTACAAGGATTTCTGGAACTGCAACTGGCCCATAGATGGTGGCCATTATAGTAGTTTCCTATGAA
ATCTACTAATGTACAGCAAGCAGTTCTGTGACTTTCAGTCACCACTTGGTTTCTTTTTTTTTCTTTTTTCTCTTGTGGACTACACTT
GAAGAATCCTTTTTTTTTTTTTTTTTTTTTTTTTTTTTGAGACAGGGTCTTGCTTAGGCCGGAGAGCAGTGGTGCTATCATAGCT
CATTGTAACCTTGAGCTTCTGGGTTCAAATGATTCTCCTGCCTCAGCCTCCTGAATAGCTGGGACTTTGGGTGTGTGCCACCACGC
TTGACTAATTTGTTTACTTTTTGTAGAGACAGGGTCTTGCTATGTTGCCCAGGCTGGTCTTCAACTCCAGAGCTCGAGTGATTCTC
CCGTCTTAGCTTCCCAAAGTGCTGGGATTACCAGCATAAGCCACCACACCTGGCCTGAAGGCTCGTTTGTAATGGCACTCTTAAGT
GAGATAAATTGAGTATTTTCTTCCTCACAACCTTCTTATGCTTGTCTGTAGTTCGCTGTCTCATTTTGGACAATTTTGATTGTCGG
CTTGGCTCTTCAACTGCCTGTGTTGTTGTATTCTTAGAACACACAGAACAACCAGGTCTGGGCTTCTTATCTTGCGAGGCAGGTGA
AGAAAGACTAGGAACAATGTATTTGGGTGCCCACCTCATACCGGCCATGACCTTCTATTGAGGAACAGCAGGTAGATGAAGCAGCT
TTCTCATATTACGCCGTGGCTGACTCCAAACTTGAACTCTGGTCTCTGTTTTTGGAGCTTGCTTCCTTCCTACGACCCTAGACTGC
TCCTCCTAAATAATTAAGTGGCAAACATGGCACAGACAGTAACCTCACATAAGAAGCTATGAGGTTAGGACAGAAATATCCACTGC
TGGCCTGCGAACTATTCACACAGGTCTGACTCCCCGCCAGAGTCCTGGCTGCCCTGGTCCTGTGGAATCCCGTCATGGCTTCTGTC
TAAACGGAGCAGGCTCAGGGCCATGCTGAACTTGGAGAAATCTGTGCAGGAGTCAGATTTGGGCTTTAGAGTCACCTGGGGTTGAA
TCTCGGGCCTGCTATTTATTGAACCTGTCCTAGCCTCAATCGCCTTATCTGTACCATGGGGATGATACCATCTAGGTCGAAGGTAG
TTGCAGGAGATTCTCATGCACGGAGGCCCTTGGCCGTCATTTGTTTCTTTGTCTCTACAAGCAGAGAGGCTGAGTCATACAGATCC
TGAGGGGAGATCACTTTCTCTTCCCTCCTTTCGTGTGTCATCTCCACCTGGGTGTGGTGAGCCCCAGGAATAATGCTGTGACATCA
AGCCCCGTGAACACCTCCTTTCACACGGACCCCGTGACTGGCTGCGGCTGCCAGCGGTGATGAGAACTGATGACACTCCAGGGCTA
CCAGGAACGGTGTGTGTGCCTCTGGGCAGGGCCACTTCCTAGAACCATGGGGCTGCTCTGTGAAGAGAACCGGCTCCCAGTGAGCT
CCAGGAGACGAGGAGCAGGTCCTGGTGGCGGAGGTGCTGGCGCAGGAGGCCGGCTCCTGGCAGAATCGGCTTCCAGCTCTGGGCAC
TGGGGTGCGGTGTGGGACCAGCAACAGGCTGCTCCTCTGCCAGGAACCGCCAGAACCAAAGTCTTCTCGAAGGCATCTTCAGAAGG
CAAGCACAGGGCTGAGCACTCCGGAGAGGAGCCTTTGGGGGAAAACTTTTTCTCGGAGCTTCGGAGCTCAGGGAGTACTATGGGGG
AGATTCACTAGGTTGATGCTTTGCTGATAGGCAGCCCTGTAGAGAAGACAAGGCAGCTGGGCATGGTGGCTCAGGCCTGTAATCCC
AGCACTTCGGGAGGCCAAGGTGGGCGGATCACAAGGTCAGGAGTTCAAGACTAGCCTGGTCAACATGGTGAAACCCCGTCTCTACT
AAAAATACAAAAAAATTAGCTGGGCGTGGTGGCACGCGCCTGTAATCCCAGCTACTCGGAAGGCTGAGGCAGAAGAATTGCTTGAA
CCCAGGAGGTGGAGGTTGTAGTAAGTCGAGATCACGCCACTGCACTCCAGCCTGGGTGACAGAGCAAGACCCAATCTCAAAAAAAA
AAAAAAAAAAAAGGGAGAAGACAAGGCGACAACTGAGCTTTCTGTCTCCTGTGGAGTTTAGCACTTGGTCAGGTGGCCATTTCTCT
CCCTCCTACTCGGTGACCTTCTCAAGGGCAAGGGGCTCCTTGAAGAGTGTCTTTCACATTGAAACATGCACAGAATGGTGGCTTAT
GACCCATGGTAGAGGCGTGAACTTGGGATGCCACTGTGGACGTGTCCCCAGCCTCCCTCCTCATTGGTGTACTGATATCGTGTTGC
CTTGGACCTATTACCTCACCTCTTCCTGGTGATTTCACCTCAATATCCCCTTTCTCCTATGCTTCTGTTTTTCCTTCTACCCTTCC

ATGTTTTTTTCTCCCAACCATTCTTCTGTATTTTATTCCACCCAGCCACCCTTTCATCTAACCAGCCTTCCACCTAGCTAGGCTTC
CAACTAACTATCCTTTTTTATTGAGACAGAGTTTCAAGTGGCACAGTTGGGCCACTTGTTTCTGATGGGAGCCACTTCTCAAACAG
GTCGATGACTAGGACTTTGGACAAAAGCATGGGTGCAGGCTGGGCGCCGTGGCTCACACCTGTAATCCCAGCACTTTGGGAGGCCA
AGGCGGGTGGATCACCTGAGGTCAGGAGTTTGAGACCAGCCTGGCCAACATGGCAAAACCCTGCCTCTACTAAAAATTGATCAAAT
ATAGATCACCCGTAATTATCACCCATCATTCTGACACTTAGCAATAGTCACCCTTAACATCTTGTTGTCTTGCTTTCAGATGGTTT
TGTTTTTGACATGTTTTTTTTTAACTTATATTATGACCATCTCCTCTATGTCTTTTAAAGTTTTAAAAACTTGTTTTGAATATCT
TTTCCATATTTCATTATGCACCTGTACAGGAAAGGTACTCCATAAGTAGGTTTTTCTTCTCTGTAAAATGGGGAAAATAAGAACCC
CTTCTACATGGGGTTGTTGCGAGGATGAGGTGAAGAAAGTTGTCAAAGCCCCTCGCTAGTACCAGGCACAGAGCAGGTACTGGGCG
CATGTTGCCTGCTACTGGTATTATTGTCATTGTCATTGCTAATCATCAGCCAAGTCAGTCCTGCGGGGCTTTCAGATTTCCTCTGC
TTTTACCTGTTGCAAGGTATAAACCTTTTCATGTGTCTCTGATTGTTTTCTTGGCAGAGCCACCCACACCCCAGGAATTTAATAGG
GTCTTGATGCATTCCCAGCTTGCTGTTGAGGAGTATTTAACCAGTCACCACTCCACAGGCAATATTAGGGGTTGCTGAGGCAGCCT
CCAATAGTGTGAGCATTCTAATTACTGCAGCAAACTCTTTTAAAAAAATTTTAACTTTTCTGGCTGGGCGCGTTGGCTCACGCCTG
TAATCCCAGCACTTTGGGAGGTCGAGGTGGGTGGATCACTTGAGGTCAGGAGTTCGAGACCAGCCTGGCCAACATGGTGAAACCCC
GTCTCTACTAAAAATACAAAAATTAGCCGGGCATAGTGGCACATGCCTGCAATCCCAGCTACTTGGGAGTCTGAGGGAGTAGAATC
GCTTGAGCCCGGGAGGCGTAGGTTGCAGTGAGCCAAGATCACGCCACTGCACCCCAGCCTGGGTGACAGAGTGAGACTCCATCTCA
AAAGCAAAACAAAACAAACAAACAAAAAACAAAGCACCCTGGAGTACTACTCAGCAACAAGAAGGAATGAACTACTGATAACACTC
AGTGACTCGGAGGAATCTCTAAAGGATGATGCTGAGGGAAAAACCCAGTCCCACAAGTTTACCTGCTCTATGATTCCAGTTACGTA
TAACATCGTTGAAATGAGAAAATTTCAGAATTGGAGGGCAGATTAGTGGGAGAAACAGGCAGAGTCTACAAGGGATCCCTCCGTTA
TTTCTTACAACTGCATGTCAATCTATAATGATCTCAAGAAATAAAAATTCAATTTGAGAAACCTGCAGTTCCAAGTCTGCATTGCA
GCTGGAGGTAGTCANNNNNNNNNNNNNNNNNNNNNNGGCGCGTTGGCTCACGCCTGTAATCCCAGCACTTTGGGAGGTCGAGGTGGGT
GGATCACTTGAGGTCAGGAGTTCGAGACCAGCCTGGCCAACATGGTGAAACCCCATCTTTATTAAATATACAAAGATTAGCCAGGC
GTGGTGGCAGGTGCCTATAATCCCAGCTATTCAGGAGGCTGAGACAGGAGAATTGCTTGAACCCAGGAGGCAGAGGTTGCAGTAAG
CTGAGATCATACCACTGCACTCCAGCCTGAAAGACAGAGTGAGACTCCATCTCAAAAAAAAAAAAATTAACTTTTAATTTTTATTTT
TTAGAGACAGGGTCTCGCTTTTTCACCCAGGCTGGATTGCAATGATACGTTCATAGCTCAGTTCATAGTTCATAGCTTAGACCTCC
CAAGCTCAAGAGATTCTTCCACCTCAGCCTCCCACAGGTGCACACCACTATGCCCAGCTAATTTTAAAATATTTTTTGTACACATG
AGGTCTCACTATGTTACCAGGCTGGTCTCAAACTCCTGGCCTCGGGCAATCCTGGACTCCCCAGATGCTGGAATTATGGGTTGAGC
CATAGCCTGCAGCAAACTCTTAAAACATGAGGGGAACTATCTTGTTATAAAGTTGCTTTTCTTTGACTACTGGTGTGGTTGGCCAG
CTTTTCATGTTTATGGCTTATTTGTGTTTGTTAACTGGTCATTTCCTTTGGGGTGTTAGCAATTTTTTCTCATTGATTTGTAAGA
TCTGTCTTGATTAACTTGTGAATTTTGCAGATGGTGGAACCGGGGTGCCCCACAGTAAGTCCCCTTGGTTAGGCATCCTGTCCCTG
GATTCGACACCTCCATTTCTATGGGGGACATTTCTTTACAAATCTGCATCTGATTGTGATACCAAACCATGCTTTGCAAGTGAATA
CAAGGATCTACCCCATTAATCTGGGCAGTTGCTAGTTTTATTTATTTATTTATTTATTTATTTATTTATTTATTTATTTATT
TTTCCGAGACCGTGTCTTGCTCTGTCACCCAGGCTGGAGTGCAGTGGCATGATCTCGGCTCACTGCAACCTCCGCCTCTTGGGTTC
AAGCAATTATCCTGCTTCAGCCTCCCGAGTTGCTGGGATTACAGGTGTCCGCCACTGCGCCAGCTAATTTTTTGCATTTTTAGTAG
AGATGGGGTTTCACTATGTTGGCCAGGCTGGTCTCGAACTCCTGATCTCATGATCTTTCTGCCTCAGCCTCCCAAAATGCTGGGAT
GACAGGCATGAGCCACCGTGCCTGGCCAGTTGCCAGTTTTTAATGTTCTCAGGTGAGTGGCATTTGCCAAGGAACTTCTCTCTTCC
CTTCTGTCTTCATAAACAAAAAATCCTACTAACTTCAGGTGTCAGTTTAAAAGTCATCTCTTCTGGGAGCCCCACCTTAAACCCCT
AAATCAGCTGTTGGCGTCGGGGGTGGACTTCTCCTTTGTTGCACCAATTGCAATTTCTTACTTAGGTGTGTGCTTGTGGGAACATT
TTTGTAATATCAGCTTCTCTGCTAAGGACAGCGATCATGTCTCTTTTATCATTTGGTATGTGTTGCTGCAGCACTTAGCACAGTGC
TTAGCTTGGAAGCCAAAGCCCAGAGGGGTAGAATGACTTGCTTGAGGCCCCTCAGCTTGGACCTGGCAGATCTGGGACCCGGTGTC
TGGGCCCACCTTCAAGCTGGATCCCTCAGGACCCTGTGTACCTGTGGCCAGGGCTTTGCTTTGGTGCCTGGATGAGAGTGGTAAGA
GTTGCTGAGGTGCCAGGCCTTTGCCCCCATGCCCGCTGGCATCACTGATTCCAGGTTATTTTGGCTCAGCGCGGATGGAGCGAGAT
GTAGGGGAAAGGTTGGGACCGGCTTCCAGGTGATTGCCACATCCCTGCCCTAAACCCTGCATGGCTTTCCTGGGCCTGCCATCCAA
GGGGAAAGAAGCCTCTTTCCTCACAAGCTGAAGCTTGAAACCAATCAAGCTCCAGGCCAGAGAAGCTGCAGGTGGGGTGTGGTTTG
CAGTGGGGGTGGGGCAGGAAAGAAAGGTTACGTGGGGTGAAGGGGTCAGGTCAATTCAGTGTGGGTTGGAATGCTGGGGTCCTGGG
TGGGCAGGGATGAAGGACACCTGGGGAGGGGAGAGAGCCTGCCGGCTGGAACTGGGGCCCTGCCAGTCCCCCGCTGCATGAGTCTG
GGCATGCTTCCAATCTCTGGGAGCCTCAGCCTCCTGTCTGTAAAATGGAGCTAATGAGGCCTGCCGTCAGGGTTTGTGGGCAAGAT
TCAGTAACTGGATGCCTGCTCCTGGCCCTTGGCAAGCATTCAATAAATGTTGATTTCTCTCCCACCCCTCGCTCCTTCTCTTATTC
TTTTCCTGTTGCTTTTCACACGGACATGTGTTGAATGTGGCAGGATATTTCTTTCTTTTTTTTTGAGACAGAGTCTTGCTCTGTC
ACCCAGGCTGGAGTGCAGTGGCGCGATCTCGGCTCACTGCCAGCTCCGCCTCCCGGGTTCACGCCATTCTCCTGCCTCAGCCCCCT
GAGTAGCTGGGACTACAGGCGCCCACCACCACGCCCGGCTATTTTTTTTGTATTTTTAGTACATATGGGGTTTCACCATGTTAGCC
AGGATGGTCTCGATCTCCTGACCTCGTGATCCGCCCGCCTCAGCCTCCCAAAGTGCTGGGATTCCAGGCATGAGTCACTGCGCCCA
GCCAGGATATTTCTAACACAGAGAAATTCTCAGTGGAGGCCGCGGAACTAGAGATAGAGGTCCTCTTGCCTACCAAAGCACCAGA
TGGAGGTGACAGAGGCCATCTTGTCCTAGAGGGACCTGGCTAAGAGGATCTAAGACCAGAACTAGTCTGAGTGCTTGGGATTCATA
TGGGGCTGTAGACGGCAGGCCTGCTTGAACACCAGTGTTCCAATCCCACATCTCATTCTTAGAGCTGTGTGGCCCTGGGCGAGTCT
CTTGCCACTATTTCTTCATCCTGAAGTGGGCACCACATTACTGCACCTCAATTTGGACGAGGATTCTCAGGATGGGGAGGTGGTTG
AAATCATCCAGGGGTCACTGGAATCAGGATGGTCCTGGCTTTTTCAGAAGGACAAGGTGGTAGGTGCCAATCTGGGGGCTTTGCAG
ATATTGAACGGCAGCCTGAGGATGGGGATAAACCTGGGTGTGGCAGACAGAATTATGCTTCCCCCAAAGAGGTTCACATCCTGATC
TCTGGAACATGGGGATGTCATATGCCATACAGAGAAGGGAAATTAAAGTCACAGGTGGAATTAAAGCTGACCTTAAAAGAGGGAGA
TCCTTTTAGATTATCCAGAGGGCCCAGTCTCATCACAGAGTCCTTACAAGTGGGCGAGAGAGGCAGAAGAAGGGTTCAGAAACATG
TGAGGTGACAAGTAACATTGCTGGCTTTGAGGATGGAGGAAGGAGCCATGAGACAAGGAATAACCGTCCCTAGAAGTTGGAAAAGG
CAAGGAAATAGGAACTCCCCCAGAGCCTCCAGGAAGGAACACAGTCCTGCTAACACTCAGTGAGACCCACTTTGAACTTTGTATGT
TCAGAACTGTATCATAATAAATGTGGGTGGATTTAAGCCTCTGAGTTTGTACTAGTTTGTTCCGGCAGCCCTAGGAAGTGAATGCA
ATGGGCGCAGCATTTGATGGCCAGCCTGGCATCCAGCCTGTGGGGAGTGGAGCTGAGGGCAGTCCAAGGGAAATGTCCTTTGAGT
GTGGCACCGGCAGGCAAACCCTTGTTTGGGTGTGATCATCAGTGAGTTGGGCCACTGAAGCTGCCTGGTGACCTGGCAGGGATGGC
TTAAGTAGGCAGGGTTTGCAGATCAGCGAGATAAAGGGGCTTGCATAAATTCACATGCCCATGGCTTCTCCCTCTGACAGCGCAGC
CCTGCCTCCGGCTGCCTGGCCAGGATGGCGTGTTGGTGCATTAGGGACCCCTTCCTCTCCTGCCCTGCTGTTTAAATTGTTGGTCT
GTTGGCGTTCTCAGGAGCCCTTTACCAGTACCCTTAGGCCCCAGACGACCTACTCCTCAAGCTGATCATATCTGCTGGCTGTGTCC
CTGCTCTGGGCCCAGCACCCTCATGGTTTTCCATAACACGTATCCCAACTCCTGCTGATGGGACCCTCGTGAGCTGTCTGTGTAGG
GAGCATGGCTGCCCCTGGGTCTGCTTTCCTGCCCCTGACCCACAGGAATAGGATGTGGCAGAGTGGGGGCCAGATGACAGAGATGC
TGGTGCTCTCTGAGCTGAGGCCCACTGCTGACCTGCTGGTGGCTTGTCCTGCTCTTGGATCATAGGCACTGGGACATCCTGCCTTC
CTTCAGTGTCCCCTCCCACTCCCTCTCCTTCAGCATCAGAGGAATGAAGGAGGCCTGGAGGAGTAGCCCCTATCTAATAGAGACAG
GAGTGGGCTGGGTGAGCATGGGGTGGTCGCAGGGGAAGGGGATAGGAGATGGAGAGAGGAACCTGCTATCTGGTGGGTTCCAGGA
TGGTGACAGACAGTGGCAGAAGCAGTGCAGAGACCTTAGTTTGTGCTGGAGGTTCAGTTAATCACTGGTGAAGGCAGACATCACTG
CAGGAGACCAGACTGTTATGTATTGTCTGTGCATTCATATTAGTACACAGGGAAGATCTTGGGCTCCGAGACCTAGGCAGATCTTG
CCTCCCATCTGCCGACCCTCCGCAGATGCTACACATTATGCCAGGTAAACACATTATCAAGGAGCCTTGGGCCGTGTTAGAGGAAG
GCTCCTTGTGCCTGTGAGGAGTTTGCACTAATGCTATGTGTGTTTGTGTGTAAGGCTTGCATTCACTGATTCACTGTAGAGGATGA

CTTGAAACCCAAGTCATCAGCTGGGCAGTGCATGGGCTGCTCGCCTGAATGGTGAAGCTCTACCAAGTGGTTTCCACCTAGGGCCT
GGCTTAGCTGGCCTCAGATGCCCTTCTCAGTTCTGCTCAAGACCTGCCTGCGGCCTTCCTCGGGCTTGCTTGCCCAGAATGCTAGG
AATGGCCAGGGATACTGCCTGGGCATGTGTTTGGCCTCAGCCAAGGTCAGGCTGTCAGGAAGGAAGAAGGGGAGGGGCACACTTG
CCTGGGAGGGTGGAGGAGGAGGGGTCCAAGCCGCCACAGCCTGGCAGTCCTTTTTTGTGCAAAGGAGACCCATCAGCTCCTCTGCC
TGGAACACCCCCATTTACTCCTCCGCAGTTAGAGGGGTGGAGGTGAGGAATTTTGTGAGGGGAATTATCTCTGATCTTGATGTGC
ACATTCCTCGGCAGTATCCCGGGCTTGGCCAGCTTGCTGACACCTGGCTGGAATAACAATGAGCGATGCTTCTGGAGGATGCTCCG
CTGGGGAGGGCTCTGCTGAACCAGCACTTACTCCCTGCTTTTCGATCCTCACTCTGCCTTCCTGTCTGACAAAGGCCGGGCTGTTG
GGATGGTAATGCCCTCTGGGCTGGCCTTGATTGAAGGTAAAAAATGAACTTCGAGGATAGCCCCTGTGGCTGTGCAGTCTCATTTT
TTATGCAGATATGTTGGGGGATGCAGAGACATGGTGGCGGGTGGGGTGGGGAGAAGGGGACTGGGAAATCTGAGACCTGAAGCTAA
GATCTGAGATGTCATCCTTTATGGAGCATCTCCGGATGAAAATGTCCCAGAGGCCCTAGGTCACTCACCCTCTGCCCCCTAATAAA
ATCACTGTTGCTGCCTCCTGCTGAATCTGAAGTATGCCTTCACTGAGGGCAGGACGTCCATCTCAACTATTATCAAAGGAAAACAT
AACAAGAGGCAACAGCACAGCCCCCGGGGCATGAGGCTCCTTCCTCTGTGATCCCTCTGTCCTTGTCTGCCAGTGTTTTTTTCCTT
GACTTTTAAAATAATCTTTTTCTTATTATGAAATAGATATTTATTTTGCTTAACAAAGAATTCTGTAGTCCTTCTAGAAGCTAGGT
AACTCTTCTAAGTCCTTTGCAAATATTAAGTCATTTAATTTAATCCTAGAGCAGTTCTGCGAGGTGGTGGGAAGACCTAGAGAGGT
TAGGGTCCACCTTAGCACTAGCTCTCTGAGCTATAAGGCCTCAGGGTCTGTGGCATTCCCTACTCACTGAAAAGAAGGGAATTTAA
AACATGAGTTCCACCACCCAGAAATGAAACAGTGTTCATTTCCAGAATCCAGGCATATGGTTGATTTTACCAATTTCCTATCACTT
GAATCACAGTTTTTTTTGTTTGTTTGTTTTTGTTTGAGATGGAGTCTCACTCTATTGCCCAGGTTGGAGTACAATGGTATGATCTT
GGCTCACTACAACCCCGACCTCCTGGGTTCAAGCAATTTTCGTGCCTCAGCTTCCAGAGTAGCTGGGGTTACGGGTGCATGCCACC
ACACCCAGCTAATTTTTTTGTATTTTTAGTAGAGATGGGTTTTCGCCATGTTGGCCAGGCTGGTCTCCAACTCCTGACCTCAGGTT
ATCCACCCTCAGATTATCCACCCTCCTACACCTCCCACAGTGCTGGGATTACAGGTGTGAGTCACTGCACCTGGCCCACAGGTTTT
TTTTAATAGTCCTGTGTGTATAATTTTGCATCCTATTATTTCTCCTTCCTTTTAACATATATATATATATATATATACGTATATAT
ATATGTATATACATATATATAATATATGTATATACATATATATATACATATATATGTATACATATATATACATATATATATATATA
TATGTATGTATTATCTCTTTACTTTTTAAAAGTTTCTCAGACTTTAAGAATTACTTAAAATTTCCCAAAACAAACCCACAGGCACT
GCTGTGATCTTCAGGCACACTTTGGTATTCAGGAGTTGCTTCTGCTGGACCAAAGTCAATAACAAGTAGCTTAAGAAGCTGTAGAT
GGCAGAGATAATATTGACTGGAGGCTTAGGGGTCTGGTTCAGGGTGGACTCAACAATTTTGCAAGTTCTATATTGTTAAAATTACA
ATGAATTATTTATAAGCAGTTGTTAGTAAATCATGAAGTTGGCATTATTATTCTTTATTTATTTATTTATTTTGAGATGGAGTCTC
GCTCTGTTGCCCAGGCTGAAGCGCAATGGCATGATTTCGGCTCACTGCAACCTCCACCTCCCAGGTTCCAGTGATTCTCCTGCCTC
AGCCTCCTGAGTAGCTGGGATTACAGGCATGCACCACCACACCCGGCTAATTTTTGTATTTTTAGTAGAGATGGAGTTTCATCATG
TTGCCCAGGCTGGTCTCGAACTCCTGACCTCAGGTGATGCACCCACCTCCTCCTCTCAAAGTGCTGGGATTACAGGTGTGAGCCAC
CACGCTTGGCCTATTTTTTATTATTAAAAAACAAAATGATGCTGATGCTTTCAAATATGTATTGCAGTCTTATTACTTAGTATTGA
GCTTTGCAGTGTTGGACTAATATCACAAGTCGAAGGGACAAAGAAGATGGTAGACAATACCAACTGTTTCCCATTAGGCTCTTTCC
CTCCTCTTGTTTTTCTGAGACCTTAAGGGCCTGGTTGTTTCTACTGTTCCCTGCTACTTCATAACCTGCAAGCTTGAGAATGTTTG
TCATTTACAAATTTTACCTTATAGTAGAAGAACATAGTAAATAATTGTACCAGATTGAGTAGTGTCCCCCCAAAATTCATGCCTGT
ATGAACTTCAGAACATGGCATTATTTGGACATAGGGTTTTTGTTTTTGTTTTTGTTTTGAGATGGAGTCTCGCTCTGTCACTCAGG
AAGGAGTGCAGTGGCGTGATCTCGGCTTACTGCAACCTCTGCCTCCTGGGTTCAAGCTATTCTCCTGCCTCAGCCGTCCGAGTAGC
TGAGATTACAGGCACCCGCCACCAGGCTTGGCTATTTTTTGTACTTTTAGTAGAGACAGGGTGTCACCATGTTGGCCAGGCTGGTC
TCGAACTCCTGACCTTCAGTGATCCTCCTGTCTCGGCTTCCCAAAGTGTTGGGGTTACAGGCACGAGCCACTGTGCCTGGTGGACA
TTGGGTCTTTTTAGATGTCATTACTTAAGATGATATCATATTGGATTAGGGTGGGGGCTAAAGCCAATGACCCATGTCCTCAGAGG
AGAAGAGAGGGACATAGGAAAGAAGGATTTGTAGAGATACAGGGAGAAGGCCATGTGACCACAGAGGCAGAGATTAGAGTGACACA
GCTACAAGCCAAGGAGCTCCAAGGATTGCAGGAAGCCACCAGATGCCAGAAAGAGGCAAGGAAGGATCTTTCATGGAGCCTTCAGA
GGGAGCACAGTCCCGCTGACACCTTGATCTTGGACATCTAGTCCCCAGAACTGTGAGAGAGTGCATCTGCTGATGTAAGCCACCCA
GTTTGTAGCAATTTGTTATGGCTGCCCTCAGAAACTAAGACAGTAGCCAACACAGAACCAAATACCCTTTGGGGATTGAGGGAAGT
GGAGCGGGAGAATGAGCGGCACTCCCTCCCTTTACATAGGACTACTTGGTAAATATTTACTGAATGAAGGAAAGCTGAAGTATCTG
TAGAACTGGCCATTTCATTCTGCATTTAGAGCTCTGGGAGCTCTTGAAATGTCTAGACAAACATCCTCTTCAGCTAGAGGAAGCTC
CTTCTCCCACTTACTAGCTATGAGATCTTGGGCAGATGGCTTAACATTTCTGGGCCTCAGTGACCTTACTTGCAAAGTTGTCTGGG
TCAAGGCAATGATTAAATGCGATGATGCATGTAAAGCCCCAGCATGCAGCCTGAAAATGTGAGCCCTTATTATTTATACTGTTTGC
CAAAGTGGTGAGTAGTGTTGCTGAATATGCCGTGTGTCCTATTAACTCTGTGACGGCCACAGTCACGTCTCCTTTCTGCCTCCTCC
TCCTCCTCCTCTCTGTCCAGTGACTAGGGAGGGCAGGAAGTTCAGAGGGTGAAATGCTGAATTAATTCCTGTCCTCCTGGTCTATA
TTCCAGATCACCCTGTGGGTGGGTTGAAAACCATGCCCTGTCTACCTCCTCTGCAAGGAAAAAATAAAGACAGAGAGCAAATCTAA
AACACGCAATGCAGAGGAATGGTTACTGCCTGCTGTGTGCCTCTCCAGGGAGCACCAGGAGGGGCTGTGTGGGACAGGGACACTGC
GTATAAGTGACACTCCTCCACATTTTGGCCTGGAGTTAGCACATACAACTGCCTTTTTGCTGACGCGATGCCCAGGAGCCCTCTCA
GGGGATGGCAAATCTCAGAATAAATGTGTTTTTTACTCATGGAATGCAGGGGAATGGATATGAAAACTTTTGTATCAAATAGACAT
TAGCGAGGAGAGGGATGCTTCACATCCTAGGTTTGTGTGATGTCGGCACAGCTGATGAGGTCTTTGCAAATTTTGCTGCTGAGGAA
GGGATGCGCTGGACTCCCAGTGTCCAGGGGCCCTCTGCTTGTCTGTCCTTAGGCAGAGTCCACCAGAATCGGGTAAAGTATGTGAC
TGCCTCTTAGTGTAGGGCATGACGTCAACTGTGAGGAGAAGAGATCAAGATACAGCTCTTTGGTGGTGTTTTTTGACCCATATCCA
GGAACTGTTCCCACACCGAGAGAGGAATATCTGCAAGTTCTGGTGCCAGATGTTGATTCAAAAACAAAACCCAGCGAAGCATCTGT
ACTTTTTTTTCTGATTGTATTAGAAGTACAGTCCACAATAATATATTGTACATTTAAAAATAACTGGCTGAGCGCAGTGGCTCACG
CCTGTAATCCCAGCATTTTGGGAGGCCAAGGCAGGCGGATCCCCTGAGGTCAGGAGTTGGAGACCAACCTGGCCAACATGGCAAAA
CCCCGTCTCCACTAAAAATACAAAAAAAATTAGCCAGACGTGGTGATGGGCACCTGTAATCCCAGCTATTCTGGAGGCTGAGGCAG
GAGAATCACTTGAAGCCGGGAGGCGGAGGTTGCAGTGAGCTGAGATCATGCCATTGCACTCCAGCCTGGGGGACAAGAGTGAGACT
CCGTCTCAAAAAAACAAAAACAAAAGAACCCTCAAAAAACTAAAAGAGGCCGGGTGTGGTGGCTCATGCCTGTAATCCCAGCACTT
GGAGAGGCCGAGGCAGGCAGATCACTGAGGTCAGGAGTTCAAGACCAGCCTGGGTAACATGGTGAAACCTTATCTCCACTAAAAAT
ACAAAAATAGCCGGGCTTGGTGGTGGGTGCCTGTGACCCAAGCTACTCTGGAGGCTGAGGTGGGAGAATCGCTTGAACCCAGGAGG
TGGAGATTGCAGTGAGCTGAGATTGCGCCACTGCACTCCAGCCTGGGTGGCAGAGAGAGTATCCGTCTCAAATAAATAAATAAGTA
AATAAGAGTTTAATTGGATTGTTCGTAACACAAAGAAAGGGTAAATGTTTGAGGTGATGGATGCCCCATTTACCCTGAAGGGATTA
TTATGCATTGTGTGCCTCTATCAAAATATCTCATGTACCCTATAAATATATATGCCTGTGTACCCACAAAAATTAAAAATTAAATA
AAAAGTTCATTAAAAATAACTTCAAACACTACAGAAATGTTAAAAGTAGAAGTGACCTTTCCGTAGAATGTCATGCCTGTACTTTG
GCAGAGAATTTCAATGGAAGCATCCAGAAAACCCTGGCTCGGAATCTGAGCTTGATTATTTCAGTGTTTCAGGTCTCACATGGGGG
ACAGTCTTGCCACTGTCAAAGGCCATCATGAGAGTTACAGCAGACTTTTGATGAAATTATGTCCACAGTGGTCCCTGGTCCTTTGT
GGTTTGAGATATATTCTTTGTCCCCACGCCTGCCCCTTGCTTCAGATCTTCTAGGAATCCAGATACATTAAAGCAAAGTGACGGGA
CAGCCCTGTCCATTTCCTGATCTGTTGCTGACTGTACCACTGAGCTGCTCTCTCCTACTGAGCCCCGCATGGTTCCTCCTGTTTTC
CTGCCCTCTCACTTGCTGGTGTTGGCCTGCTCTGGCTGTTCTGCTTGGGGTTAGGTGAGGATATGGGGCAGAAGGCTTCCAGCCTG
GAGCGCCTATGGTGTCCCAAGACAAGGGCCTGCCTCTGGCCTCTGGGTGTGTCCTCCTGCGAGTTTTAGAGAAATCTGGGCAGGCT
CAGACTGAATGCCTGCTTCTCCTTGCAGCCTTCTGAAGATTCCCATGACACACACCTGATTTACCTTCAGCTCTTTTACTTGCTGG
GTAAGCACAGCCCTGGATTACTTGGCATCTGGAATTAACTGAAGGGTAACACTTTCTTAACCTTATTTGGAAAATGAATAGGAAGG
AGACCCCTTCTAACTTCTGGAAGCACAAAAGTGTGTTTATCCTTCAGGAGCATTCTTGGCTGCTGAAAAATCATCATGGCTAGCTT

TCACTTTCACGTATTGAGCTAGTAGGTGGTATCAATAGTAGCTAATATCTATTTCATGTTTTTTTGTGTAGGAGGGACCCGGCGTG
CAATTTGCATGGATTGTCTCATTTTATCCTCCCAGCAGGTAAAAGAGCTGGGAGGGAGTAAAGGGAGTTGTTAATTTCTGAGAAGG
AAGGTGAAACTTAGGAATGTTAAATACCCCTCTTGGTGTACACAGTATGCAATGGAGGCAGGGTGCAAATGTGCTATGGAGATTTC
AGAGCCTGGGCTTCCCAGAGGAGGCCTTTGCCCCACAGAATCTTTGCTCCATTTCTCCACTTTGCTGCCTCTATGAATCAATGATT
CTGTGTTGTCCATTTTTCCTTTGCAATCTTTTGCCATCTCTTCATTAAAAATGGTCTCCATCATTCTGGCCAAGCACAGTGACTAA
CATACAATAGGTGTGCAGTAAATGTCTAGTGGATGAATACCCAGTGTGAACATTAATTGAGCACCACTAGCTGCCAAGCAGCCAAG
CACCCTATATCTGGGAGATGTAGAATTTAAGGCTGTGTAGTCACTGTCCTCAAGCTGCTCACAGCTTTGTGTAAGACACTCAAGGG
AACATGCAAAGCTCAGGGCAGCTCAATTTGGGGCGAGGGTTATGGAGAAAATACAGTCTGCATGCTTTTAGTACATGCAGTTCTGC
ATCTTGGGGTGCAGTGCTAGAGTGAATGACACAGTAGAGGTGACAGGCTTAGGATTTAAATTTGATAATGTCATGATGCCATGGGA
GCACTGGAGAAGGGGCAGCAGTGGATTCAGCTGAGAGTCGCAAGGGGTTAATGAGAAGATTGGGAAAGGCTGCACAGAGGAGGAGA
AGGGCTTTGTAGGATGAGTAGAGGTTCACCAAGGGTGGGTAACAGTTAGAGGAACAGCAGGTACAGAAGCCCAGAGGAGAATCAGT
GAATGACACATCCACCATGTCCAGAGATCAGGGATGGTTGCAGCTGAAGCTAAAAGTGTAAGAGCTTGGACTGTGGATCTTCAATC
CCAGTTCATCTTATTCCCACCCATATGGCTGGTGCTATGGTTTTGTCACCTGTAACACAAGTATGAGACTGGTACCTTCCTAGGAG
GGGTGTTGAGAGTCTGAGCCACACTCAAATTCTTGGTGTGCAGTGTCTCTGATCTGAAATGTGCACCGTGAATGTTAGCAGTGGGT
ATTACCTTTGGCCTGATTGTGAAGCGGGTTTGAGACAGAAGTGAAGCAGTTCAATTTGTTGTGTGATCTTCTTCTTCCCCAAAAGA
AGGTTTTCATCCTCCTTAAGGCTTTTGCCCACCCTTCCCTTCCTTCACATCCCTGGTGGGTACCCTTCCCACCTGCAGGAAGCCTA
CTGATCTTTGAGTTTTGGCCTGCCACCTGGCCCTTCCCTCGCTCTGCAGTCTTGGTTGGCCTCCAGGTGTGTGTGTGTGTGTGTGT
GTGTGTGTGTGTGGGTGTGTGGCCCTGGGCTGCACTGTGCCTGCTGGAGCAGGTGCAGTTGCCCTTTCACCATGCGTGCTTCTTTG
GATGTGACCCCTTGGGTGCCTGTGTATTTAGAGTGGAGGAACGTCCTCCACTATAATGATGAGAAGTGTCATTGCTAGTTGCACTC
AGTTTTCTATGGTGTTTGGGGGAGAAATTGGGGTGCTGGATTTTGGTTTTAACTCTTTCCCACCTTAACCAGTGACTGTGGAAAGT
TGCACATCGTCCCCTTCCTCACAGCTGGGGTCTGTTCTTCCTCCGGGTGTTACGGTTTGTGAGAGCCAGGAGGGTACACAGAGGTG
GCCTGATTTCTGGAGCCATAGCAAGAAGGGAGAGGTGTGTGTCCTCTCTGTCTCTGTCTCTGTCTCTGTCTCTGACTCTGTCTGTC
TCTGTCTCTGTCTCTCTCTCTCTCTCTCTTCTTTCTCCCTCTCCCTTTTTCACATGTTGTTTTCTGATTATAAGAGGAATACTT
CATCATTAGACATATTTCGAGCCTCCGTTCCTCATCTATGAAATAAGCATAGTAAAGGACTGAAGGATTTAAAGGGATGTGGCCAA
GTGTGCAAAGTCATATGTGCTCAATAATTTATTTTCCTTGTTTTCTTTTCCATTTCCTTCTTCTCCTCTAAGGCGCTCCACATCTC
CGTGATATAGCGTGTACTCCTTTCCTCATTTTCTTTTCTCTGAGACAAAGGGAAGTTCCTAGTTCTGCTATTTCCTAGTTCAGTGA
CCTTGGACAAGGCACCCTGGGGAGCCTTAGCTGCTCATATGCACAAGCTTCTAGCAACTGCTTCCAAGGGAAGGGTGGGGAGGAAG
GGAGACCCTGAGTGTTTGGACCCCGCCTGTGCTCAGCACAGAATGGAGCTCAGTAAGGGCAGGTCGTGGGCTCCTTCTTCTCCCTT
TCAGCAACCATCACTCGTATTCTTTTTTATCTCCAAGGCCTCATGCTGGAAAGAGATTGATTGCTCTCTGAGCAGTAAGTCCTCTG
AAACAGGGTTTCTCTGGGGAGTTCTGTGGCACCCCTGCCTTACCTGGGGGACCTGGCAAAGAGGCACCCTCCACCCCCCCTCACCC
TCAGCCAGGTTTACTGAACAGAGTTACGGGGGTGGGCCTCCGGATCTGCATTGGAGCAAGTCCACAGGTGGTGCTTAGGCACACTC
AGGTTTGAGAGCCACTGACCTAGAGGGGAAGACTGGGAGAAGCCTTTTAAGAGACCAGTACTGTGACTTGCACCATGGAGACACCT
GGAAAACATACAGTGATTCACACACACGCCCCAAACAGCAGTGTGTGTGTGTGTGTGTGTGTGTGTATGTGTGGTTTCACACTTGG
TTTGCTATGAATGCTGTAATGCCACCCAGTTCCCACAGAGCTCTGGGGACTCAGGTTCCCCAAGTCAGGGTTCTTTTCTGGAGTGC
TATTCTGACAGGTTTTTATTTTTATTTTTTTAATTATTTTATTTTATTTTTATTTTATTAGTATTATTATATCTGACAGGTTTTTA
ATACAGTGAATTTGTATGTCTCCTGTGTATTTATTTTAGCTGCTCCCTTCTTTCCCCTTCTCTCCCTTTCCTCTCATCTCCTCCCG
CTTTCGTCCTTTACCTTTCCCTGGATGATATTAGCATTACCCGTACACTCTTCCCATCTCCTGGCCCCTCCAGTTCTGAAGGGATG
GGGACTAAGTGGGATCTGTCCTCTAAGCAGGCGGAGGAGGGAACCATGTCAGCTCCTAGGTGCCCCCAGAGCACCTGCTGCCAGTT
TTCCACAACTCCTGTCCTCCGTGGCGCTTGGGTGACAGCAGAGCAGAGCCGATGTGCCTGCTCCTGGTCATGGGCTGTTCGTGCTG
TCATCGCTTTCATTTGCCCCATCCCCCCAATTTGGACTTACCCAGAATCCTTGGGCAAGCTAGATGGCTGCCAAAGTTTTACTTAGC
GAGATGTGCCTGCGTGACTTGAGTAGAGACCTGCTTCTGAGAAAGTCAGAAGTGCATTCCAAAGTCAGGAGCGTGACCTCCCCGGA
AGCCCAGCTAATCCAGGTTTTAAAGAGGAGTTGGAACAAAGGTGGTGCTTATGGGATGTATGGAGCTGTGGGATGTGGAGAGGAC
AGGTTTAGCAGAGTGGCGTGGTGGAAAGTTTCCTCTCGCCGAGGAGGACAACTCCCCAGCGCCTGAGACAGCGGGAGCTCAGAGCG
CCTAGAATGTGCCCAGATTCTAAAGTGAAGCAGGCTGTGGTGTGATGATGGCCCCCGAGGGGACTTGCAGTCTTATGGACCTGGGT
TCAAGGCCACCCTCTTCAGCCACACAAACTGGGCAAGGGGCCCGCCCTTTTCACGCTGCTGTTTCTGTAACTTTCAGATATGAGAC
CTGTCTGTGGGGTTGTGAGGATACATGGGGATTAAGTGGGAAATTAAAAGGGTTTTCTTTGTGACTGGTGACTGATAAATGCCATT
TCCTTTCATCCTCCTTATTTTGACATTAGCCCCAACCTCTACCCCGAGGGTCTCTCTCTCTCTCTTCCCAACTTAGATTCCTCTCA
GGGGACACATAGACAACCCAGAAAAGCAGCTCTGATCTCTCGTGGAGCTTCCCTGAACTCCACACCGCACATCTACTACTGTTTAG
GATAACATTTTGACTTAGTCCTTAAACGGACTATTTCTCATAGTCCTGTTTTGTAAATGAGATTTTAAGCTCTTTGCAGACAGGGA
CCAGTATAAACTCTTGAGTCTCTGTCAAACTTAGTGAACTTTCTTTCCAGCCTGGGCAACATGATGAAAACCTCTCTCTACAAAAA
AAATACAAAAATTAGCAGGGCATGGTGGCTCGCACCCGTAGTCCCAGCTACTCTGGAGGCTGAGGTGGGAGAATTGCTTGAGCCTG
GGAGTTTGAGGCTGCAGTGAGCCATGATCACGCCACTGCACTCCAGCCTGGGTGACAGAACCGGACTCTGTCTCAAAAAAACAACA
ACCCCCCTCCCCCACAAAAAACCTTACTGAACTTTCTTGGAGAACATGCACACTGTAGAACCTTCTGTATTTGGAATATTCAAGTG
CAGGTCCTGCTGTTCTGTAAAGAACTGTCACCTTGAGTCATTGTTTGACGGCTCAGGTCCTTGGCACTTTACCCCTTTGTTCATGA
TTGATTGACATTTGGACCTTTGCTGACCTTTCCCTGAGGGATTTTGTGGGCATAGTCAAACAAAAGGAAATTGAAAAAAAAAACCC
CACAAATTTTGAAGGTCTGTCACTTGGAGAAGAACAGTGTTCATGACTCTTCTTCGTCATTTCAGCATCTTAATCATCTTGAGCCT
AAAGATGAAGGAGAGTGGAGGTGAGCCTGATTAAATGGGAGCTTTTCCTATATTGAGGATCTACTAGGTGCAAGGACCTCACTTGG
GTTATCTCTAGCCTTCCCCCAACTCTGCAAGGCATTTCACAGATATGGATGCAGGTTCAAAGAACTGAAGCTTAAAGCTTCGCAGA
TTTGGAAGCCTCAGAGCTAGAATTCTCCCCCTAGCCTGTTTGTCTCTGAAGCGTTGGTTATTGCTTGACATTGCATCCCTCCCTCT
TTTTTCCCTCTCTTCCTCTTTCCCTTCCCTGCCACTGTCTGCCTGTCTTCCTGTCTGCCTCTCTTTCTGTCTTAAAATTACTGTTG
CTATTCCTTACTGGTCTGGTCTGGTCTAGTTAATTAAAAAGAAGGCTATCGTGAGGTAACCCTGGGATCCTTATTGGCACAGTCAT
ATTCCCATAACTTTTAGGTGCCTGAGTCCTAATGGATCTGTAAGACAGTGCCCATCAGCGAGAAATACCCACAGCCTCCCTGGATG
CTGCCCACAGGCATCTTGCAGATGAGGGTGTTTGCCTTCTCACCTGCAAGGGGGTGGGGAGAAGTGGCCAGGGGTGGCTGTTCTG
GGGACGCCTGCCTCCCCACTCCCTGAGCCTAGGGGGAATGATATTATGGCTTCAGGCCACAACTAGACACATTATCTACTCTGACA
GGGAGCCAGTCTGGAGAAGGATGTCATGGATGACGTGAAGAAATTACTGAGGGAAAAAGAGCAAGGCGTGAGCCCTATTGGGGCTG
TTTTGGTCAACTCCTGGCAGTAGATGATATTGATGCTTTGGAAATCTCTATTTTCCTTGCATTTCCCAGCAAAGTGCCATTCCCAT
CTCTTTCTTTTAGAAGCTTCTTATCCTATGGCTCTGCCTCCAGTCTTTCTCATCACATAATTCTTCAAAGGCAAGGCAGACTAGTG
GTTAAGAACAAAGACCCAGGGCTGGCCCGTGGTGGCTCCTGCCTGTAATCCCAGCACTTCGGGAGGCCAAGGCCGGGTATCAGTTG
AGGTCAGGAGTTGGAGGCCAGCTTTGCCAACATGGTGAAACCCTGTCTCTGCTAAAAATACAAAAATTAGCCGGGCGTGGTGGCAG
GTGCCTGTAATCCTAGCTACTTGGGAGGCTGAGGCAGGAGAATCACTTGAACCCAGGAGGTGGAGGTTGTAGTGAGCTGAGATCAC
GCCACTGCACTCCAGCCTGGGTGACAGAGCAAGACTCTATCTCAATTAAACAAAACAAAACAAAACAAACAAACAAAAAAGAACAA
AGTCTCCGGAGGCAGTGCCTGGGCTCGTATCCTAGCTCTGTCTCTTGCTGACTCCTGCATGGCTTGGGCATACTTGGGCTTTGCTG
TGCACCAGTGTTCTCGTTTCTTAGTGAGTTCGTATCTCACAGCATTGTTGGAGATTACAGGAACACAACCAATGCTCTCAGAACCA
GCACTCAGGAAGTGTGGTAATTACATGCTCTTCTGGGCTTCTGGTCATGCCACCTTCGACGGACAATGCGCCAGCACCCACCAGTT
TAAGGACCAGTTCCTCCTCTAGGCATTGTGCCCAGCGCACCCTTCTCATGGAGATTTGCTTCTCACTCTGCATCTGCAGGTCTTCG
CTGGGTGATCTTGGGCAAGTCACTTTACTTCTCCAGGCCTTACTTCTCAGCTCTTCAGTGAGGACATTGAACGAGATTATTTCCAA

GGTTTCTTGTGTGCTGACGTACTGGGGCCTCTCACATTCTATGTGGTCCAACCTTTCCTCCTGTTCCTCAGCTTCTGCCAGCCCGC
CAGGGCAACCTGGTTATCAACAAATGTCCTCGCTTATGGACATGTCCTGCTTCTCTGGAAATCTTAGTACAGCCTGAAAGCTCCAT
GAAGTCAGGATTGTGTCTTTCATGTTTGATATGATTTCTGGCTTCTAGCACTGTGCTAGGAGCTAGCACGTGAACACTGTCTGTGT
AGCTGGAATTAATCACCCACTATTCCAGTCAACAATTCCTTTTTTTTTTGAGACGGAGTCTTGCTCTGTCCCCCAGGCTGGAGTGC
AGTGGCATGATCTTGGCTCACTGCAACCTCCACTTCCGGGTTCACGCCATTCTCCTGCCTCAGCCTCCTGAGTAGCTGGGACTACA
GGCGCTCGCCACCACGCCCGGCTAATTTTTTTTTTTTTGTATTTTTAGTAGAGGTGGGGTTTCACTGTGTTAGCCAGGATGGTCTC
GATCTCCTGACCTCATGATCCGCCTGTCTCGGTCTCCCAAAGTGCTGGGATTATAGGCATGAGCCATCGCCCCCGGCCCTTTTTTT
TTTTTTTTTTTCCGAGATGGAGTCTCACTCTGTCTCCCAGGCTGGAGTGCAGTGGCATGATCACTGCTCATTGCAACTTCCGCCT
GCTGGGTTCAAGCAATTATCCTGCCTTAGCCTCCCGAGTAGCTGCGACTATAGGCGTGCACCACCATGACTGGCTAATTTTTTGTA
CTTTAGTAGAGACGGGGTTTTGCCATGCTGGCCAGGCTGGTCTTGAACTCCTGACCTCGTGATCCACACGCCTCAGCCTCCCAACG
TGCTGGGATTACAGGCGTGAGCCACTGCGCTCAGCTTTCCAGTCATTTCTTAAGCGATGTTGAGTTCCCAAGCACTGTGCCGGACA
CGCATGGGTTGCTGGAGGCTGGCAGAACCCGAATGTCAGTATTCCTGAAGAATGGCTCTAAGCAATGTACCTTGCAGAGCTCAAGC
AGGGCTCTGCTGCATCATGCCCATGAAGGTGCCTGCTGGTAGAAGGCTGGTTCTATACTGGCTGCTTGCCTCCTCCTTGTGGAGAC
CCACATTGTGTCTGGCAAAGTGCAGAAGGAAATCAAGCCCTTAATAACATTAGGGAACTGGACTGGGTTCGGTTTGCATCTCCCGA
GTCTGACTCGTGGGAGTAACCACCATAATAGCAGCGCTGTGCTGTTGAGGGTGACTTTGACTATCTGCTTGTGTTGTGAGCCCACT
GTGTGCCGGAGCACTCTCTATACATTCAGTCTTGTCCGCAGATGAACTCCAGTGGACACATGTTTCTATTCCTTATTATTATTATT
ATCATTTGTTTTTCTGAGATAATAATAATAATATTATTATTAATATTATTGTTATTATATTATTATTATTATTATTATTATTATTA
TTTAGATGGAGTTTCACTCTTGTCGCCCAGGTTGGAGTGCAGTGGCACAATCACAATCTCAGCTCACTGCAACCTCCGCCTTCTGG
GTTCAAGTGATTCTCCTGCCTCAGCCTCCCGAGTAGCTGAGATTACAGGTGCCCGCCACCACACTCAGCTAATTTTTGTATTTTTG
GTAGCGATGGAGTTTCAACATGTTAGTTAGGCCGGTCTCGAACTTCTGACCTTAGGTGATCCACTTGCCTTGGCCTCCCAAAGTGC
TGGGATCACAGGTGTGAGCCACCACACCCGGCCTATTATTATTATTTTTTGAGACAGGGTCTTACTTTGTCACCTAGGCTGTTGGC
TCACTGCAACCTCCACTGACTTCCTGGACTCAAGGAATCCTCTTACCTCAGCCTCCTGAGTAGCTGGGACTACAGGTGCGCCACCA
CTGCCAGCTAATTTTTGTATTTTTGTAGAGATATGGTTTCACCATGTTGTCCAGGCTGGTCTCAAACTCCTGGGTTCAAGTGATC
TGCCCGCCTCCACCTTCCAAAGTGCTAGGATTACAGGCATGAGCCGCTGTGCCCGGCTTATTCTTTGTTTTATTGAACAGATGAGG
AAACACTTTCAATGAGCTTAAGGGATGTGCCCAAGGCCACACGGTTGTGACCACAGCATGAGGATTCACACCTTGGTTCCTGTGGC
TCCCATCCCCCCTCCCCTGAATGAGGAATCGGGTGAGTGTGACGGGAGCAGGCACTCGAAGGCTTGGGAATTCCAAAGCAGCTGAT
CCATGTCATCCCAGCACACGTTAGGGAGGAAGTGAAACGAAGCAAAAATAGAGCCTGTCAGCGGGGGGACAGCTGCCGCTTTCTCT
GGGAACAGATCTCCCCGATTTATCTTCCAGGTTAGGAAAGCCCCAGCTCCTGGGGTACCACAGAGAAATAATGTGAGCACAGCCAG
AGAAATTGCCACCTCTCCCTTCCTCAGAAGAAGATAAAGGAGACATCGTCTTTTTGGCATCAGTTTTGAAGAAATCTTTTTTTTTT
TTTGAGATGGAGTCTCGCTCCTGTTGCCCAGCCTGGAGTGCAATGGCACAATCTCGGCTCACTGCAACCTCCGCCTCCCAGGTTCA
AGCGATTCTCCTGACTCAGCCTCCCAGGTAGCTGGGATTACAGGTGCCCGCCACCACGCCCAGCTAATTTTTGTATTTTTAGTAGA
GAGGGGGTTTCGCCATGTTGGCCAGTCTGGTCTCGAACTCATGACCTCAGGTGATCCACCTCCTCGGCCTCCCAAAGTGCTGGGAT
TACAGGCGTGAGCCACCACACCCAGCCAGTTTTGAAGAAATCTTACTTCACAATCCTGAGAGAAAGAGAAATTGAGGCACACTTAG
CATGTGAGACTGATTTCTCACTTCTGCTGCTACAACTGCTGCCCCATTGACCGGGTCTCAGAGGTGTTATAATTAACATCCTCTGA
CTCTTGCTGGTACTAAAATTAACAACATGACTGGTCCCTTCTGCAAGAGAGGGAAAGAGATACACACATGTGCACATACACGCACA
CACATGCCTGCACACACACACTTGCACGCACACATGCATGCACACACATCACACACACACACACACACACACACACAGACACATGC
AGAGTCCAGAAGGCTCAGAGACAGAGAGAGAGAGAGGAAGGCACAGCCACACACACACTGGGAGACACTTATTATGGCTCCCCCTG
CAAACGGTGCTCCCCTCAAAGCTTTAGGACCAGGGACGCCTTCCCTCACTTTCTGTGTTCTGTACCTCATACCCCATTTACCAGCC
TGTGCCAACCCTGTATACTTTAAGTCTTCAGTTACCAAAGCATCCATCTGCCCGTCCTATATTGAGGGGGAGTGGATGCGCAGAGG
TGGTCTGTTGGTGTTGGGTAGGGAATAGACGCAGCCTGTTTTTCGTTTTTCTTCCAAATGTCTTCGGCAGGGCCTCAGCTAGTGGA
AATACCTGGTGGGGAGGGTCAAGAAGGAAATGACAGTGGGGGGAATGGGGAGGGATAGCATTAGGAGATATACCTAATGTTAAATG
ACGAGTTAATGGGTGTAGCACACAAGCATGGCACATGTATACATATGTAACAAACCTGCACGTTGTGCACATGTACCCTAAAACTT
AAAGTATAATAAAAAAAAATAAAATTAAAAAAATAAAATAAAAAAAGAATTCCTATTTTAACTTTGGAAAAAAAGAAAAAAAGAAG
GAAATGACAGATTTGGTCCTGACAGCCTGGGAGGCAGAGAGGAGAGAGATGGAGCCAAGAGAATGGGGCTGGGGCCACGTTTTCTT
GTGGGGCAATTCCGTGTCACTGGGCCAGAGCATGCTTATTGTGAAGACTTCCCCTCAGTGTCCTCTGGGGAAGGGTCAGCTGTGAG
GCAGTGGGGCGTGAGAATACGATGAAACAGGGAAACCTGGAAGCTGCCGATTCCCAGGCCTTGAAGGGGTGTTCTGATGTCTTTGA
CATTCCAGCAAACATATTTTCACAGGAAAACTCAAAAAATATAAACTCCCTTTTTATAGAAATAATGTGCATGGAGAAAAGTGCAC
ATGTCATATGTGTATCGATAGCTTGACGAATGTTCTCAAACTGAACCTGAGTGTGGAGCCAGCACCTGGATCAATAGAACATTTCC
AGCATTTTGGAAGCCCCCTCCCAGTCAGTCACTCAGGAGAACTTTGTCCTGACTTCTAACCACACAGATTAGTTTAACCTGTTTAT
GTACTTAATCTAAATAACATCATCACACACTCCCCTGTGTCTTGCTTCTTTCACTCAAAATTATGTTTGTGGATATATTCATGTG
GTACGTGCATTTTCAGATCATTCTCACTGGTCGCTAGATTGTGTGCATATTTTAAAAAATCCATTCTACTGTTGATGGCATTTGA
GTAGTTTCCAGTGTTTGGCTGTTATGAATAGAGCTGCTGTGATATCCTTAGACTTGTCTTCTGGTGAACATATGCACACATATCTG
TGCATTGATACCACGGAGTGGAGCTGCAGGGTCATAGGGTTTGGGTATATTCAGCATTAGTAAATAGTGCAGAACAGTTTCCCAAA
GTGGTTGTTTATCCTTTTTCAGAGTGTCTGTTCACCAGCACGTATGAATTCCAGTTCCACATCAAAGCCAATTATTGGCATTGTGT
GGTTTTTAAATTTTAGCCAACCCATTACAGAAAGAGCAAATGAAAGGAATCTAACAGCAAGTAATCAGGAGAGAAAAGAAAATGCT
CCCTGAGAAAGCATTATGATTTCATGATACGGGAAATCCCGGTGTACAATGCCACACATTGTGTACTTTTGCAGGCGGAGGGCGAG
GTCCAGCCACCTTCTTGTGAGCATCAGGGCAGTCCTTGCCATTGGTCAGGGACCACCACTTGACTGCAGTAATACCCAACAGACCA
TGTCATAATTCTTCCTCAGGTGACAGAGCTTTAATGGATCCGACCCCAACAGAACAAACTTGGCTTCAGACTTGCCTGTGGGCCTC
AGTAAATATTCAACAGACTGTGTCCCCCTGTTCACTCCCCTGTCATTAGCTGAGGCTGGTACCATAAATCACAGCTCCCCTTCCTG
TTGTAAGTGGCCCCTCTCAGCTGACGGTACTCCAGTCCTGACATTCAGGAGCAGTGCCTCTCCTGGGCTGCTTCCCAGGGCTGGCA
GGTCTTGGTGGTCACTTGTAGGAGCCGAGGAGTGAGTGTGTGCAGTCCTGGAGTCACTGCTGTGGGGTGACTTGCAGGAGCCTCTT
CCATTTGATGTGAGAATGATAGATCCGTCAAGCTCTTAATATGTGCCCAGCAACATGCTAAGTCCTTTATGTTGTTTATTGCAACC
TGTCACAGTCCATTCAGGCTGGTATAACAAACCACCTTAGACTGGTAGCTTGTAAACAACAGAACTTTATTTGTCATGGTTCTGGA
GGTGGGGAAGTCCAAGATTAAGGCTCAGACATATTTGGTGTCTGGTGAGGGCCTGTTTCCTGGTTTAGCAATGATGCCTTCTTGTT
GGTCTTTATATGGTGAAAGGATCAAATAAGGTATTTTATACCTCTTTTATAAGGGCACTAAGCCCTCCAGCCTCATGACCGAATC
ACTTTTCAAAGGCCTCCCCTTTTGAGATCATCACCTTGGCAGTTAAGAGTTCAACGTATGAATTTAGGGGTCGGGGGTGTAGGTAA
CAAACATTCAAAACATCATACAACCTCACAGTAGTTCTATGAAATGAGTAGATGTTGTTTACCACCATTTTCAGATGAGGAAATTA
AGGGTTAAAAACATTTCCCCAGAGTAAGAAACGTAGTAAATTTGGGAGTCTAACTTCAAACTGTAATTTTTAATTACTGTACTTCC
TTCTTCTTTTCTCTTAGGGGCTGTTTAGATACTTGAGCTTCCTTTTGAAGCCATCTTCCTCTTGGGTCTAAAAGCTTTCAGGTAGA
TTTAAATGGGACAAGAAGGCCAGATGCAGTGGCTAACACCTCTAATCCCAGCTATTTGGGAGGCTGAGGCAGGAGAATCACTTGAA
CCTGGGAGGTGGAAGTTGCAGTGAGCTGAGATGGCACCGTTGCACTCCAGCCTGGGCAACAGAGCAAGACTCTGTCTCAAGGAAAA
AATAAATAAAATAAATAAATAAATAAATGGGACAAGAGGAAGTGACCTGCAGAGCACATATTGGGTTCAGATGTAATTATCTGCTT
TTTCAATGATTTTAAGAAATTAGAACCACTGTTTTTCTCTGGCTCTTTTCATGACTTTTTTCAAATGTTGGTTTTCAGCAGTTCAA
CTATTATATACTTAGGTGTGATTTAAAAAAATATATATCCTGCATGGGATTTGCTGATATTCTTATATCTATAAATTAATGTCTTT
CACTGAATTTAGAACACTTTCATCCCTTCCCACCCCCGATATTGATCTTGCTCCATTCTCTTCTCTCCTTTTGGGACTAAAATTAC
ATGTCTGTTAGGCTTTTTGATATTGTCCTACAGGTCTCTGAGGCTCTCTTCAATTTTTCAAATTTTTTTCTGTTATTCAGATTAAA

TAATTTCTATTGGCCTCTCCTCAAATTGTACTGACTCTTGGTTCCTCTTCAGTCTGCCGTTAAATGGATCCAATTGATTGTTTCAG
ATTTTTTTTTTAATTGTAGAATTTCCATTTGGCTCTTTCTTTGCTAAGCTTTCTTATCTTTTTATTTACTAAGAATACATTTTCCT
TTGAGTCTTTGAGCATAGTTATAATAGCTGCTTAAAAAACCCTTGTCTGCTAATTCCAGAAACTGGGTCATCTCAGGATCATGGGG
ACGGTCCCCATAACTATCTTTTTTCTTAAGAATGGATTACATTTCCTGTTTTTGTTTTCTTTTTAGTTGTTTGTTATATTGAGCTA
TTATAAATTATATCACAGACATTGTGAGTGATACTTGGTAAGGACTGATTCTGTTTTGTTCCTCCAAAGAGTAGTGCTTTTTTGTT
TCAGTAGGTAGTTAATTCAGTAATCTAAGACTTCAAATTCTGACTCCCTAGTATTGGGCAGCAATATAAATTGCTGTTTAGTTCTT
TTGGCTTTAACTGGGCTACTCCATACAAACTTGGCTCAGGAGTCACTTAGTGACTTAGGGAACATTTGTACACAGAATTTGAACTC
CCCTTTTTGGAAACTCTCTTTTTACCAGGATTTCTTCTTTCACTTTCACCTATTGTGTTGCCTTGATCTCTGTCCTGATGTTTCAG
TGCAGCAACACCATCTGAGTGTTAGCCACTCACATGTTGCAGAACCGGGCCTACTCTCAGACTAAAATTGACCAAAAGCAGGAACT
CACTGGCTTGCTGAATGTCCAGGTCTTGATCACACTCCAGAATGTCCTACTTTTGGTCATCAGGTGCCTAGATTTTTTAGAAATCT
TTGTTCTAATTTTTAGAAATATTTGTTCAGAGTTTATAGTTGCTATCTACCAGGGAACTGCTTCAATAGGAACTACTCAACCATTG
CCAGAACCTTCTTGAACTTTCTAGAAACACTGTCTTATTTATTTTATTTTATTTTTTAGACAAAATCTCACTCTGTGCTCTGCCAC
CCAGGCTGGGGTGCAATGGTGTAATCTTGGTTCACTGCAACCTCCACCTCCCGAGTTCAAGTGATTCTCGTTCCTCAGCCTCCCAG
GTAGCTGGGATTACAGGCACCTGCCACCACACCTGGCTAATTGTTTGTATTTTTAGTAGAGACGGGGTTTCACCACGTTGGCCAGT
CTGGTCTCAAACTCCTGACTTCAGGCGATCCACCCATCTCAGCCTCCCAAAGTGCTGGGATTTACAGGCATGAGCCACCACGCTTG
GCCTAGAAACACTGTCTTATTTAAATAAGAAAGAGTGTCTCTCAGAATGGAAAGAAAACAGCTTTGGAGTCAAACCCAACTTGGAT
GCAGAATCTCACTCTATCTTGGTTTTTTTTTTTTTTTTTTTTTTTTTTTGAGACTGAATCTCACTCTGTCACCTAGGCTAGAGTGC
AGTGGCATGATCTTGGCTCACTACAACCTCGGCCTCCTGGGTTCAAGCCATTCTTCTGCCTTAGCCTCCTGAGTAGCTGTGATTAC
AGGTGTGTGCCACCACGCCTGGCTGATTTTTTGTAATTTTAGTAGAGACGGGGTTTCACCGTGTTAGCCAGGATGATCTCGATCTC
CTGACCTCGTGATCCACCCGCCTCGGCCTCCCAAAGTGCTGGGATTACAGGCGTGAGCCACCGCACTGGGCTGGATATGTTTTTA
AGAAGAAAACGTACCTTACTGTTTCCTTCGTGATTTCTTCACTAGCCGCTGCAAGTGTGTGTGGCTCTGTGCATTGTGGGCAGGGT
GAGGTAGCTAGCTTGATTTCCATTTTGGGAATGAGTGGTGCCTCCTCATGGAAGACTTCTTAAAACAGGAGATGGGTGCCCTCTGT
TATTCTTGTATGGCAGGCAGAATAACGTCCCCCTAAATGTCCACATCCGAATCCCTAGAACCTGCGAATATGTTGCATGTCAAAAG
GGACTTTGCAGATGCTAGTAAGGCTAAGGACCCTGAGGTGGACGGATGATTTTGGATCATCTGGGTGAGCCCAATATAATCACATG
CGTCCTTAGGAGTGGAAGAGGGAGGCAGAGAGTTATTCAGAGACAGAGATATGGCCACAGAAGCAGAGTCAGAGATGCCTTGGTGT
TGTATTTGAAGATGGAGGAAGAAGCCACAGCCAAGGCCTATGAAGCAGCCTTTAGAAGCTTGGAAAAGCAAGGAAACACATTCCCT
CTCAGCCACCAGAAAGAAATGCAGCCCTCCCAACAGCCTGATTTTAGCTCAGTGAGACCCATGTCAGACTTCCAGAAATGTAAGAT
CATAAGTGTGTGCTGTTTTATTTTTTATTTCTTTTTCTGTTTTAAACCACTACGTTTGTAAACATTTCTTTGGCAGTAATCGTTGC
TTTTGATGGAAGTTGGAGCAGTGGTCAAGCTCGTGGGCTTTGGAGCCAGTCTGCCAGTCCGCCTTGTTTCAAACCCCTTTTCCTTT
CTCTCTTTCTTCCTTCCTTCCTTCCTTCCTTCCTTCCTTCCTTCCTTCCTTCCCTCCTTCCTTCCCTCTCTCTCTTTCTTTC
TTTCTTCTTTTTTTTTTTGAGATAGAGTCTCATTCTGTGGCCCAGGCTGGAATGCAGTGGTGTGATCTTGGCTCACTGCAACCTCC
GTCTCCTTGGTTTAAGCGATTCTCATTCCTCGGCCTCCCAAATAGCTGGAATTACAGGCATGCACCACCACACCCAGCTAATTTTT
GTATTTTTAGTAGAGACAGGGTTTCACCATGTTGGCCAGGCTGGTCTTGAATTCCTGGCCTCAAGGGATCCACCAGCCTCGGCATC
ACAAAGTGCTAGGATTACAGGCGTGAGCCACCGCGCCCAGCCTCAAACCCTTTTCCTGTGATTTTTTTTTTTTTTTTTAAAGCTGT
GTGACCTTGTGGAAGTTGCTTCACTTCTCTGGGCCTCAAATTTCTCATCTCTAAAGTCGGTTAACAATAATCTACAACTCATAGTT
TTTTGTGAGGATGAAATGAGGGTTTCATACATGCAGAAAGCCTGGAAGTAAGTTTTAGCTGTGGCCATTCTGGGCAGGAATCCCTC
ACTAGGTAACTTAGAGCGGCGGATCAGGGCACAGACTTGGGAGACAGACTGTCTAGCTTCAGATTTAGGCTTCTTACTAGTCTGAA
GTAGAATGGTTGTCTGTTCCTCCACTGCTTCATCTGGAAAACAGGGGCAGGCATCCCAGGGTGTTTGAGAGATCATATCAGATGGA
GAGGCCAGAGTTTTTAGCCGATACATCATAGACATTCCCCAAAGGACAGTTATTGTTATCATTGGGTGGATGGGGAATATTGAGCC
TCCAGCCCTAGGCATTGTGGATCAATTTGTTCTTCAGTTATCTTTGGAATAATTTAAATACACTGGATTGGGAAGACAAAGCTATC
ACCCTCCTGATGGGAGAGTGCTGGGTACCTAGCAGGTGCTCAGTAGTTAGTGAATGAATGAGTAAGAACAGAACAGGTCCTAGGCC
CTGCCCTGAAGAGGCTCACAGTGCAGGACAAGGCCTGGAGTCAGGCCAGGGTTTTGTGGTTGTCTTTAAAAAGAAACAAAACAAAG
AACTACTTCCTACCAGCTGACCCTAAACCCCTCAGCTCCCTGCAGGGACCTCTCAGCCCTGCAGGGGAGCCCAGGCTATGTGCCAG
GCCTGGCCTTGCTGTCAGCTTTCTAAGCCAGTCACAAACATTCTCCTCTCAACTTTATAATGGATGCCCCCCAGCCTCCTCTCCCC
TCTCAGGGTGCTCCTGAGGCTATGAGCAGCTGGCAGTTCCCTGTCTGAGAGGTCCTTCGTACCGCAGGAGAGGCAGCTCTCACCTG
AAGGCCTCCCTGGACACCGGTCTCAACTTTGTGTGTTACCAGTTATGGAGAATTATTTTTAGTGAGTTACACTCTGTTAGGGCCAA
ACACAAGGAGGAAAGAGTGACTGAATACTGCTCTGAGTCAGTGACTGTGACAAGACTGTTTTTTTCTTCTTCCCTGTTGCAGGCCT
GATCTTTTGGCCAGAAGGAGATTAAAAAGATGCCCCTCAAGATGGCTGTGCCTGTCAGCTGCATGGAGCTTCGTTCAAGTATTTTC
TGAGCCTGATGGATTTACAGTGATCTTCAGTGGTCTGGGGAATAACGCTGGTGGAACCATGCACTGGAATGACACACGCCCGGCAC
ATTTCAGGATACTAAAAGTGGTTTTAAGGGAGGCTGTGGCTGAATGCCTCATGGATTCTTACAGCTTGGATGTCCATGGGGGACGA
AGGACTGCAGCTGGCTGAGAGGGTTGAGATCTCTGTTTACTTAGATCTCTGCCAACTTCCTTTGGGTCTCCCTATGGAATGTAAGA
CCCCGACTCTTCCTGGTGAAGCATCTGATGCACGTTCCATCCGGCGCTCAGCTGGGCTTGAGGTGAGGCTGTCCTGGCTGGTCACG
GAGGGTTCACTGGGATGTGGCTGGTGTTCTCAACGAGGTGGCATTTAATGAGCACCCTCTGTGTGGTTGGCGTGATGCTGGGTGCC
TGGGGGAGACAGATGGAACTAAAGAGGTCCTTCCTGGAAGCCGTTACTGTTCCCTGCCCTGACGTGTGTTATTGTACCTGTCACCA
TTTTCTGATGTTCCCAGTTTACGTATCTGTCACCATCTACCTCCCTATAAAGTGCCTCTGCTGTTGGTTCTGTGTCCTTAAGACAT
TGTGTGGCATTTGGCACAGTCTCTCATGCTGCAGAAAGGGTAAATTAAGACAGGATTGTTAATGAATGAAGACATGGGAGATTCAA
TGGAAAGATATGCATGGGGGGATAATTTCTTGGCTCTGTGCTCAGAGCTAGGAACAACATAAGGCCATATGGCTGGTGCTGTGAGG
GTTGTATGTGGGCAGATGCATGTGGAGGCAGACAGGCCTGGGTATGACTCTTTAACACATACACACTGCATGCCTCCAGGCAGACT
GATGCATTCCTTTCCCTGCAGTGAAGGGTTAAGTATTATCACACGTCACAGGACTGGAATTGAAGATCGCCAAGTAGACATTGAAA
TGCTGACCACATGACAGACACTCAGCATGTGGTGGGTGATGTTGGCTGGGGGTAGCTTTTGGGGATGGTGGACATTTCTTGGCCCT
GATCTTTCTGAGCAGAGCCAGGGAGGCAGTGCTGGACTCTGACCTGGGCACAGCTGGTTGGCGTGGACAGTTGATTAAAGGTCACA
GTTTCCCTGCAGCCCATGGGAAGGCAGAGTGACAAGTCAGTGGCAGATGAGGAAATGGAGTCTCATGCCAGCTGGAGGGAGGGAGC
AGCTGGGTTGGCCACAAAGGCCTGTTGCAGTTCTGCAGCTGACAAGTGGAAGGACTGGCCTCTCAGGTCCAGGCCTGGTGGCCTGG
GAGGCTGCTAGCTCTGAGCGATGAGTGGTGCCAAGGTGCCTGAAGGAGAGTTGCAGCTCAAACAGCAAGTGGTCCAGCTCTCAGAC
CACTTGAGGCCACCTGGCATGTGGCGGCCAAGACCACAGTTGGGGCGGGGCCTTGAGTCTGATTTTGATTGGATTCCTAGTTCGGC
TCATGCCTGACAGCACTTTCTCAGACAGGCTTCCCTGACCTCCTAAGGTAAATAATGTCCCTCTGGGTGTTACATGCTGTTCTCAC
CCATAGCTCTTAGATGTGTTCTAATTGTATATTCATATGTGCAATTTACTGGGTTTCCTGTCTTTCTCTCACACTAGAAAGTAATC
TTTCTGAGTTGAGGAAATGCATTTGCCTTGCACACAGGCTAGACACATACATAGTAGGCATTTTATTTGTTGCATCACAGTTGCCT
TGATCAAATATGGTGACTCTCCGTGGTTTTCTCTTGGTTTCTTTGAAAAGGTCAGACAGAAGGGAAACACTCTTTTCTTGAGTATA
TATATGACTTAGCCCAGCAATCCCACTAGGAGAGCAATGGTTTATCCATTATACAGTTGAGCGCCTGGAGGCTCTGAGGGGCCATG
TACCTTCCCCAAGGTTATTTATCCAGGAGAAGACAGAGCCAGAAGCTGAGAACTGCCTCGTGTTTTATACCAAACAAGGCCCCTTC
AGCTGAAGACTTTTCCTTGCGGTCTTGGGAAGGGTGTGTGTGACGGTGAGCTGCAGCAGGTGCTGGGCTGTGCTTGACTGTGGTCA
CGTAGGGGCAGGCAGAGGAGCCATTTCGGGGGGCTGCTGAGGGTCTGGAGAGAGGCTCACAGTGGTGAGCAAAGAGGTTCTGGGAT
GGGAGGGGATCAGTTGGGAAAGACAGAGGCAGATACTCCCCACTCCTTTGTGCCTTCCCAGTCCTCTCTCACCCCTTCCTGCCCTC
TCCCCACACCCTATCGTGGTGGCAGCTGTGCCTCACTTATCACATGGTCCATAGCTCTGTTTTTTCCCCTTTTATCAGTTACTGCC
TAGAACAACCCAATTGTGGTGGTCTGGGTTATCTCCCACTCTTCACAGTAGGGCCAGAGTGGGCTCAGACACCAAAGGACACAGAA

TGGTTATGGCAATGAATAAGATTCCTTCTCTGTGTCAGCAAGGATGCTGATGTAGTAATAATCTCCATTTATTAAGCCATGCTGTC
TACAGGGCACATAGCACATGTTTTTCCATTCAAACCTCAAAACAGAGCTATGAGATGCGCACCATTATGATGATTATCTGTTTTAC
TGTTTTTTGTTTTGTTTTGTTTTTGTTGTTTTTTTTGAGACTGAGTCTTACTCTATCATCCAGGCTGGAGTGCAGTGGCGCAATCT
CGGCTCACTGCAACCTCCACCTCCTTGGTTCAAGTGATTCTTGTGCGTCAGCCTCCCAAGTAGCTGGGACTACAGGCGTGCGCCAC
CATGCCTGGCTAATTTTTGTTGGCCAGGCTGGTCTTGAACTCCTGACTTCAAGTGATCTGCCTGCCTCGGCCTCCTAAAGAGCTGC
GATTACAAGCGTGACCCACCTTGCCCGGTCTGTTTTACTGTTGAAGTAACTGGAGCACCCAGAGGGAAATCAAGTGTCCCTAGAGA
ATACAAGCAGCATTTGGACAGCCCTGTCTGACTCCAAAGCCACTGCACAACATTGCCTGGAGCTGAGAGATGCACATTCTCCCTTC
TTGGCCACTGGTGACTGTGACCTTGGGCAAACTACCTCTGCTTTTTGGGCCTTAGTACATTCATTCATAGAGAAGCTGGCTAATTT
CTCTTCTCAGCCTGGAGACTTGAGAGTGAAAGGGGCACTGTATCACTTACTCTGGGATGACAGGTGTATACGAGGATGTAGCTGGG
CAAGCCAGGCCTAGTGGCTCGCACACTCATCAGTTAGATGGTGGGTGCTGGTGCTGCTGGTGCATCATGCATACAAGGCTGGCTCT
CCCAGTTTTCCCTGGACTTTTGCCTGAACAGTTTGCATTTCTACTCGTGGTGCAGGAGCTTGCTGTTCTGATTTCATGTCACAAGC
CTGCTTTCAGCCACTTAGAAGAGCCCTGTTATCCCTGACTGCCGCTACAGCAGTCCCAACCTGTGTTGTTTTCACTTGATGGGCTC
CTGGAATTATTGACAGCACACTCTCTAGTCGTACGGGCTGCCCTGCCACCTACACTGACATCCTGGCCAGGTTCCTGGAGATGGCC
TGGCATCTTGGACCTGTTCCCCACGGGGTGCCTGGAGCCTCTGATGGTCTACCCAGGGTGGCAATGAGGGAAAGGCTGTGACTTGA
TCATCTCCAGGGGTTCTTCGGTGAACAGCCCAAGGTTGCTTAACAGCTGGTGTGTATGAATACCTGGAATGTGCCAGTTCACCTTC
TAATCTGGCAAGGAGAATGTGTCTCCTCCTTATGCATATGAACAAACTGAGGTTCAGTGAGATCCAGAAAGCTGCCACGGTTACTG
ATGGAGAGAGCAGAGAAGCTGGTGTTTGCAGTCCCATCTGTCAGCCTTGACACCCCTACTCCTGTCCAGCCAGTGTTTCTCAAAGC
GTGCTGATGAGCAATGCAAGATGATTTCATGTTATAGATAAGAATAAAAAAATTGTTTTGTGTTTAACTCAAATTAGAAAAAGGCA
ACAATTGGTATGTGCGACCTGTGGTTTTGCAGATGATACTGCTTAGGATGTTGGTACTTAAGAAAAGGTCAACTTTTCAAAAATAC
TATTAGTGACATGTGGACCTAGTCCTCCTGAAGACGACTACATTGGGGCACCGGTAATTGTTTCTATTTGCGGTACTCTGGCTGTG
TGGCTCTGGCCACGCCACTGGAGGCAGTGTCTGAGCCTGTGACTTGAGTAGTAGCTCTGTGTCATGTCTGCTGATTCTCCCCAAAT
CCTGAAGATTCATGATGAAGTGACTGGCCGGCTTGGTCTGAAGCTAGATTGAAAACAATAAGGATCCCAGAACGATAGCACTTTAC
AATCCTATAATTTGGCTCAAATTGCCTGCAGTTACTATCTCAGCCCTGCCTGTTATGTTCATTGAGCACCCAAAGTTTTTCAGTCA
ATTCCTGAGTTAATTATTCTCTGGATTGAATTATGAAATAGTAAATATTTCCACTATGCAATCAATTGGTGACTTATTCATGTAT
TCATTTCATTCATTTAGTCTCAATAAATTGAAGATAAGGGCTGAGCACAGTGGCTCACACATGTAATCCCAGCACTTTGGGAGGCC
GAGGTGGGCAGATCACCTGAGGTCAGGAGTTCAAGACCAGCCTAACCAACATAGCGAAACCCCATCACTACTAAAAATAGAAAAAT
TAGCTGGGTGTGGTGGCATGCGCCTGTAATCCCAGCTACTCAGGAGGCTGAGGCAGGAGAATCATTGAATCTGGGAGGCGGAGGT
TGCAGTGAGCCGAGATTGCACCACTGCACTCCAGCCTGCGTGACAAACTGAGACTCCATCTCCCAAATAAATAAATAAATAAATAA
AAGATAATATTACCTACCCCATGAGTTTATTATGAGAATTAAATAAGAGAACATATTAAAAGGTTTCATTCAGTGCCAGGCATATA
ATATGTACTCAGGGAATACTAGTTTTTTTTAAATAAAATTTTAAAATGGGATTAGAAGGTCAAAGCATATAGGCATAAAGGTATAA
AAAATATTGAAGATGAGTGAGTTACTAAAATTTAACATTACGTTTAGCTCTGAGCTTCCTAATTAGCACAACATGCTAAGTAGGTT
ATATTTGTATCTAAAGTAAAGATTGGCAAACTTTTTATATCAAGGCTTAGGTGACAAATGTTTTCAGCTTTGCAGGCCACACAGCT
CTGTACTGCTTGTCAGTTCTGCCTTTGTATCTGGAAAGTAGCCATAGATAATATGTAAATGAGTGGGTGTGGCTGTGTGCCAATAA
AACTTTATTTACAAGAACTGGCAATAGGCCTTTAGGCTGTAGTTTGTCCTTGGCCTAAATAAAGGAAACATGTTTGTCTTTCAAAG
GCAGAAACTCCTCCTGGATCATAAACATTGAAAAAAAAATTGTTACAAGATGCAATATTTCTGTGAGACTTGTTAAGCAGTATATG
CGCAGTGCTTTCATTAGGATTTTACAGAAAACTTAGAAGATGGCCTTCACATGGCTAGTTTCCTTACTGTTCATTCAGCAGACATT
TACTGAGGGCTTACTATGTCCTAGGCTCTGATATCCATACATGGTAAAACGTAAAGGCATGTGCATTTTGGCAGGGGGTGCTGCAT
TGACAAGGAGTGGTCTCGGTGACAGTTTTGGGAAGTCAGTTTAGAACAGTATTGGACACACTGTTCCATCCCTGAATTACACACAG
GCCTCAATGCTAACTTGAGTGGGCTTTTGGTCCAGCAGGCCTGGGCTCCAGGGGCAGTCACTCAGCGTCCTGCCTCAAGAGTGGTT
GTGTCATGAAATCTTGGCCCTCTTTGACTGATAGCTACAATTATTTGAAGAGTGTGGTTTTTAGAACAAAACTAAATCTGTTTTGA
AGCTCTGCTGTTTTATACCTGCTGTGTGATCTTGCTCAAGTTTTTATTTTTATTTTTTTCTGTATAAGGGATTTTATTTTATTTTT
ATTTATTATGTATGTATTTATTTTATTTTAAGTTCTGGGGTACATGTGCAGGATGTGCAGGTTTGTTACATGGTAGACGTGTGCCA
TGTGATTTGCTGCATCTATCACCCCATCACCTAGATATTAAGCCCAGCATGCATTAGCTATTTTTCCTGATGCCTTTCCTCCCCCC
CCCCGCCCCCGACCCACAGGCCCCAATGTGCTCAAGTGTTTTAATCTCTCTGAGCCTCACTTTCTTCATCTGCAAACCATAACACC
TCTGAATCTATTTGTTACTCTAATGCTAGCTATCTAGTGTTTGCTCTCTAAATGTTAGTTCCTCCACCTGGGGGCTGGGCGGGCGG
TGGCTGACCTCACCCCATGAAGCTGTCTCCTGGCTTTCCCAGCTAGAGCCTGTGGGTATGAAGATTGCAGCCCCCTCCTCCTGGGG
GAGTATTCAGTGTAACAAGCCTTGAGAAAACACTGGCCTGTGGAATTCCAAAGTTAATGAAGGGAACAGATTCCAAGAGGGTCTCT
GTTTGACCTTCCACTCTTTCCCCAACCCCGTTACCCCTGAGCGAGTTTGGGGGCAAGCATAGGTTTGTAGGCACTGCAAAGAAGAA
CTCTCACGCTGTGGTGCAGGACCGTGGCCTTGGCCTCTGGATATCTGGGCCAGGTGGGTGCATTTGGGCCTCTTCCCTACAGCGCC
TGGTCAGACTGCAAGAGACATAGGAAGCAGCCAGGGCAGGCTATCTTAAGAGGAGGGAGCATATTTGATAAAGTTAGAAAATGATT
CTCAGCTGTCCATAGGAAGAGCAGAGTAAGGGGCTATGTGAGAAGTTTGGAAGATGGCCCAGTTCCAAAAATGAAGAAAGAAAGTA
AGAAAGAAAGAGAGAAAGAGAGAAAGAAAGGAAGGAAGGAAGGAAGGAAAGAAAGAGGAAAGAAAGAAAGAGGAAAGAAAGA
AGGAAAGAAAGAAGGAAGGAAAGAAAGAAAGAAGAGAGGGAGAGGAAGGAAGGAAGAAAGGAAAGAAAGAAAGAAAGACAGACAAA
GAAAGAAAGACAGGAAGGAAGGAAGAAAGGAAAGAAAGAAAGAAAAAGGAAGGAAGGAAAGAAGGAAGGAGGGGAAGAAGGAAGAA
AGAAGGAAAGAAAGATTCATTATTCTGTCCTCTGAACCTGAGTGAAGAAAAATACCCTGTCCTTTGTACCTGCGTGAAGAGAGAAA
AGAAAAGAAAAGAAAAGAAAGAAAGAAAGAAAGAAAAGAAAGACAGGAAGGAAGAAAGGAAAGAAAGAAAGAAAGAAAGAAAGAAA
AAAAGACAGACAGGAAGGAAGAAAGGAAAGAAAGAAAGAAAAGGGAAGGAAGGAAGGAAAGAAGGAAGGAAGGAAGAAAGAAGGAA
AGAAAGAAGATTCATTATTCTGTCCTCTGAACCTGAGTGAAGAAATATACTCTGTCCTTTGTACCTGCGTGAAGAAAGAAAAGAAA
AGAAAGAAAAGGGAAGGAAGGAAGGAAAGAAGGAAGGAAGGAAGAAAGAAGGAAAGAAAGAAGATTCATTATTCTGTCCTCTGAAC
CTGAGTGAAGAAATATACTCTGTCCTTTGTACCTGCGTGAAGAAAGAAGAGAAAAGAAAGAAAAGAAAGAAAGAAAGAAAGAAACG
AAGGAAGGAAGAAAGAAAGAGTGTAAAGAAATAAAAGGCTGTAAAGAAATAAAATAATGTAAAGAAAGAAAATAATGTAAAGAAAG
AAAAGAAAGGAAAGAAAGATTCATTACTATCCTTTGGACCTGAGTGGTGTTTTCAGTTAAAATGAGTGGAGTAGAACCCAGAGATC
AGGCTTTTGAGGACGGTAAAGCCTCAAGATACATTCTGTTTCTGGCTCAGGGAACCTTCAGGGAAAAGGCTTTGGAGACCAAGCTA
GGCAGGGAAGCGCCTTTGCTTATCATGCTGGAGACCAGACTGAGGCAGTGAAGTCACCATAATTGATGGGCCACACTAGACTGGAG
CCAACCATGGCCCATTGCTGAGACTCTGGGCCTCAGGGTGGCCTTGGCTGGAACCTGTATTCCAGTGGCCCTGGTGGCTCTCTTCC
CTCTCACCTTCCCAGGAGGCAGATTATTTCCCTGTGGTGGGGGTGAGGCGGTGGCGTGGGGGCTTCTTCCCTCTCTCTCTGTCTTG
TAGCCTTGTTAAAATAGTGGCCACACGCTGATAGCTGGATTTCCAGTGCAGTTGCTGGGGATGGGGGTCTTTCCCTCTCATCTGTC
TGTCACCCTTTATCACTCCACAAGGGGTGGGGGTACTGGACAGAAAGAGTGTGTCCCACCTGATTACTGGGAAGTGCAATGGACAC
CCCCACCCCCCGTTGACTTATCTGGCTCCAACCGAAGAACAGCTGTTGGTGGTCAGGTCTCTGAGTCACTCAGTAGCTTGGGACTG
GGTTCAGTGGGAGATGATGGCATAAACAAATAAACACTGGCTGGCCCCCAGCTCTCAGCTTTCTGCTCACTGGGGGGCCCCAGAAG
CTTCCTGTTGCGTCTGCAGGGAAAATAAGGATACCCATAATGAAAATAGTTTTCCTGATGGAGAAATGCTCTGGGGAGGTTGTTGA
GGCATGTTAGAAAATCCCTGAGGCTTTCTGGCTCTGTGAGCTAAGGGAGGACACTTGGCCTCTCTGGGCTTTCGCTTGCTCCTTTG
GGAAGCCTGTTATTGTGAAAGTGAGATGATCTGGGAGGTAAAACTGGCCAGTTGTCCATCTCCCCTCTCTGAACATGATGCATAGT
AAGCCCTGAATCTCATTTAAATTTGGAAGTCAGACTGACTTTGTGACTGTCCTAGTTTCCGGCTGTTGAGTCAGCTCAGGCAGTTC
TTGGGACTTCTGTGAGTCTCTGGCTCCCCGTCTCTCAATGGGGTGACAGTCCTCGTCTTGTGCCCCTCACGCGCTTGTTGAGATGA
CAGATGAGTCACCTCTGGGAAACTGCCTGAGAGCTGGATGACACCACTCTGTGGGATTGCATTTCATCCACAGAGACATCTGGAAA

AGGAGGCAGAGCAGAGCCTGACTTTTGATTATTTGGGGAGGGAGCCTGGAGCTGGTGGAAGTGAGGGCATAGAGCACATTCCTTTC
AGGCCAGGCAGTGCTTGGCTGCTGAGGCACCGGTGGAGACCTCATGATCTGGTGGTCCGCCTCCCTCTTGTGCTGTGTGTGCCTGG
AGCTCTCTGGCTTCCTTGGCTTTCCACTGTCTTTCCCAAAGCCTCCATGTATTCTAGAATCTGCTATGGAGTTTCAGGGGGATCTC
TGTATAGTGTGAATATCTAGCTCTGAAACACAAAAGAGAAAGCTACAAAGGGCAACGTAACAAAAGCCGTATTCCCACCCAAGGAT
TAGCACATGCTCATGAGGTGGCATGCTTTGCTTTGGATCATTGTCTTTATTTTATTTTATTTTGTTTATTTATTTATTTATTATTT
ATTTTTTGAGATGCAGTCTCGCTCTGTTGCCCAGGCTGGAATGCAGTGGTGCAATCTCAGCTCACTGCAGCCTCTGCCTCCCAGGT
TCAAGCGATTCTCCTGCCTCAGCCTCCTGAGTAGCTGGGACTGCAGGCGCGTACCACCACACCTGGCTAATTTTTTATATATTTAG
TAGAGATGGGGTTTCACTGTGTTAGCCAGGATGGTCTTGATCTTCTGACCTCATGATCCCCCCACCTCGGCCTCCCAAAGTGCTGG
GATTACAGGCATGAGCCACCACACCCAGCCTGGATCATTGTCTTTAAAGAAATTTAAAAAAAAAAATAGATAAATGGGAAGTCTAT
GAGCCTTGTCTCCCATTCCCCATCCTTCTCTTCATACAAGTACCTCTCCTGAACTTGGGGTATAACAGGTCATGCTCTTACAATTT
CATTACAAATCTATTTGAAGATCACTGGTAGCACCATTTTGTGTGTTTTTAAATTCTTAGGTAGGTGACATAACGTATAAGATGTC
TGCACTTTCCTCTTTTCTCCCGGCGCTATGCTGGGCAGATGATGCTACTATGCTTGTTCATTCATTCCCTGGGCCACAGAGTCTTG
GGTTGTTTCCAGAATTTTGCTTTTGTAAACAATGCTGTCATGAGCATCCTAGCACAGGGCTGCAGGTGCATGTCTGGGTGTGTCCC
AGGCAGTACAGGGCACAGAGCATGCACATTTGCAGCCTGACTGGAGGTTGCCACATCACTCTCCAGTTGGCACTATTTCCTAGGTT
CCTTCCCTGAGAGCCTGGGAACTTCTGGAAGGCAGGAGTGGGTGGTGTTCACCACTATATACCTCTGCCAGTCAGCATGCCCCACA
CCATGTACCAGCTCATGACACGCCTGAATGGGGTGAATTCTGGAAGGGGACAAGGCAAGTGCCAGGGCAGGTCCAGAGTCTGGGTG
TGGAAGGAGCAACATAATGGGGGCCCCTTGCAGAAGCAGCAGAAAGAACAAAGAAATAGGGGCCTGGAGTGGGGGTGTGAATGAAG
ACATTGCTGTTGAGAAGATACTTGTGAAGAAGCCAACAGCGTTGTCTGGGCCTGGCTGGATATCATGAGGCTGGACATGGAGGTCC
CAGGCACCATTGCTGTGGGCTTTTGGGGAGTTGGATGCACAGTGGAGAGCTGAGGAGGAAGTTGGGCTTGCTGCAGAGGGCTGCTG
GAGGGGCTGGCTGGAGTCTGGCTCTGGCCCTCACTCACTGAGGGTTATCCAAGTGACCCATTGCAGGAAGAGGGGTGGGGGACCCA
GAGTTACTGTAACCACTTTATGAGTCTGTCTCAGCCAGGATCTGGGGCTTCTAGCAAAGCCCACAGCACAGGCACATTTTAGGAAA
GGCAAGATTGAGATGCCCTCGGTGGCCCAGAAGGACACTTCAGGGCAGCAGGGCTGTAAAGGTCAGGAGGGGGAAACTTCTGGAAG
GAAGTTGAATGTTGTGGGAAAAAGCTGACATAAATGAACAAGGAATGATTTTTTTCTTTTCTTTTTTTTATTTTTCTTTTTAGAGA
GACAGTCTTGATATGTTGCCTAGGCTGGTCTCAAACTTTTGAACTTGAGTGATCCTCCCACCTTGGCCTCCCAAAGTGCTGGAATT
ATAGGCATGAACCACCATTGTACAGCCACAAGGGAAGATTAAACATAGACAGTTTGAGAAGAGTTTTTTTTTTTTTAAATTTCTAA
TTTTATATTTAATAGAGATGGAGTCTCACTATGTTGCTCAGGCTAGTCTTAAAGTCCTGAGCTCAAGCGATCCTCCTGCCTTGTTC
TCCCAAAGTGCTGGGATTACAGGCTTGAGCCACTGCACCTGGCCAAGGGTACTTTTTTTTTTTTTAAGAAAAATATCGTTAGAAAT
AGAAAATTTGGATTTGGTAATGGATAGTGCATACTATGAAATAACCTTTTTACTTCTTCAACACTTCCCATGATAATAACACTAAT
ACAAGATCTTTGGTGAAAACTTAGAATGCGTAGAAATGTGTTGGTGGTTGAAAGAGAGCCCCATAACCCTCCACTGAGGAGTAACC
TCTGTTTGACCTTAGAAATAATACCTGTGAACAGAATGGCCAACTTGTCCTGGTTTGCCAGAGACTTTCCTGGTTTTGGCACGGAA
AGTCCAACACTCTGGGAACCTCCTCAGTCCTGTGCAAACTGGGATGGTCGTTCACCCTACCTGTGAATTGAATGATACCACTGAGC
ATCAAGCACTTTGACAGTTCACAAGTCATTTCTAGTGCTGTCATTGTGTATACTTGGTTCCGGCTGTGTGCAAGGCATTGTGCGAG
GCTGCATGTTCTGGAGCTACACCAGAGCTCCAGAGCTGAAGTTCTATTTTCTTTTTTTTTTTTTTTCACTTTTTATTTTAGAATCA
AGAGATGCATATGCAGGTTTGTTACAGAGGTATATGGATGCGGTTGAAGTTTGGAGTATGATTGAACTCATCACGCAGGTACTGA
GCATAGTATCCAGTAGTTTTTCAATCCTTGTCCCCCTCTTTTCCTCCCCTTTCTAGTAGCCCTCAGCATCTATTGTTCCCATCTTT
ATGCCCATGTGTACCCAATGTTTAGTTCCCACTTATAAGTGAGAACATGCGGTATTTTGTTTTCTGTTTCAGCATTAGTTTGCTTA
TGATAATGGCCTCCAGCTGCATCCATGTTGCTGCAGAGAGCGTGATTCCATTCTTTTATATGGTTGCATAGTATTTCTTGGTGTAT
ATGTGCCACATTTTCTTTATCCAGTCCACCACTGACGGGCATTTATGTTGATTCCATGTCTTTGCTATTGTGAATAGTGCTGCCAT
GAACACATGGGTGCGTGTGTACTTTTGGTAGAACAATTTATTTTCCTTTGAGTATACACCCAGGTATGAGATTGGTGGGTTAAATG
GTAGTTCAACTTGGAGTTTTCTGAGAAATCTCTAAACTGCTCCTCACAGTGGCTGGACTAATTTACATTCCCACCAACAGTGTGTC
AGTGTCTCCTTTTCTACACAGGCCCACCAACATCTGTTATTTTTTGACTTTTTAATGAAAACCATTCTGATTGGCATGAAATGTAT
CTCATTGTGGTTTTGATTTGAATTTTTCCAATGACTAGTGATGTTGTGCATTTTTTCATGTTTGGTGGCTGCATGAATGTCTTCTT
TTGAGCAGTGTCCGTCCTTGTGCTTTGCCCACTTTTTAATGGGATTATTTGCTTTCTTTGCTTTTTGATTTAAGTTCCTTATCGATT
CTGGATATTGGAACTTTGTCAAATGCATACTTTGTGAATATTTTCTCCCATTCTGTAGCTTGTCTGTGTACTCCCTTGATAGTTTC
TTTTGCTCTGCAGAAACTCTTTAGTTTAATTAGGCCCCAATTGTCAATTTTTGTTTTTGTTGCAGTTGTTTTTGAGGACTTAGCCA
TAAATTCCTGTCAAGGCCTATATTGAGAGGGGTATTTCCTCGGTTTTCTTCTAGGATTTTTATAGTTTGAAGTCTTAAGTCTTTAA
TCCATGTTGAGTTATTTTTTGTGTGGTGATAAATAGGGATTCTGTTTCATTTTCTTCTGCATATGGATAGCCATTTATCCCAGCAC
CATTTATTGAATAGGAAGTCTTTTCCCCATTGCCTCTTTTTGTCAAGTTTGTTGAAGATCAGATGGTTGTAGATAAGTGGCTTTAT
TTCTGGATTCTCTATTCTGTTCCGTTGGTCTGTGTATTTGTTTTTGCACCAGTACCATGCTGTTTGGGTTACTGTAGCCTTGTAGT
ATAATTTGAAGTCAGGTAATGTGAGGTCTCTAGCTTTGTTCTTATTTCTTAGAATTGCTTTGGTCATTCAGGCTCTTTTTTGGTTC
CATGTGAATTTTAGAATAGTTTTTTCTACTTCTGTGAAAAATGATGTTGATAGTTTGAAAGAAATAACATTGAATCTATAAATTGC
TTTGGACAGTATGGCCATTTTAATGATTTTGATTCTTCCAATCAATGAGCATGAAATGTTTTTCCATTTATTTGTGTCATGTCTGA
TTTCTTTCAACAATGTTTTGTAGTCCTTGTAGAGATCTTTCACCTCCTTGGTTAGAAAGTATTACTAGATATTTTATTTATGTGTG
TATGAAGCCCTGTTTTCTATCCTTAAGGAGCTTACAACTTGTCTTGGTATGCAGTAGGGGCATTTCACTTGTGAGAGTGGGGGTC
TTACTGTAGCCTGGGCAATACCCTCTTTTATTATTGGGTACTAAGGATTTAAGGATATTTTTGTTCTGGTGGGTTATCCAGTTTTG
CAGGTTGGTACCAGGAGAGAGGATGCATGTTGGTACCAATGCTGAATTTGACTGCCCCTGGCCCTGCTGTGCAGCACTTTCAGACC
CCTAAATGATGTGACCCATTCTGGGGCCAGCTCAGTGTTTTACAGTGATGAAGATGATGTTGATGATGATGATGATGATGTCAACA
TCCATCATTCTGAGGTGTGTGTCTATCTCTGGCCTCGTTATACCTGCAGAACATTCATGTGGTAGCCAATACCTGGGAAAATAATC
CCACTTGCCAGGCAGCAAACCGTGGCCCTCCGTTAGGGGGATGGCATCTCACACTCATGAAGTGTTGAGTGTATGGGTCCCTGGCC
AGCTTTGGCAACTGTGAGGCAGGCATTATGCTTCCATTTTACCACTGACAGACTGAGGCCGGTGATGGGGAGCACCTGGGCCTGTT
GGCTTGTCTGTGGTAGGCCAGGCTGGCTACATAATATGTGGGGCTCAGTGCAAAATGAAATATGGGCTCTCTTGTGTAAAAATTAG
GAAGAATTTCTAGATGCTGACAGCAGAGCTTTAAACCAAGCTCGGGCCCCTCTGAGCCAGGAGCCCTGTGTGCCTGCACAGGCTGT
ATGTCCAAGGAGCTGGTCCTAGGGCAGGACTAAGGTTCAGGCTTCGTATTGACTTCCATGACCAAGCATCCTGGGTCCAGGCTGTG
TTTTGTGTGTCTGCAGAGCTCTGGTCTATCTGCTGATAAGTCCCTGTGGTGCTCAAAGGTACCTGGCTTCCTTGGAGAAAAGTTTC
TCTCTCCCCTGCCCTTCCCCAGCCTCAAAATACACAAATGCTCAGATGAATACTTAAGAATCCAACTGGACCAGCTTCTGTCTGTG
ACTTGATGACTTAATGTGATTTTGGGGGATGCAACATTGGGGGTGATGCTTTAGCATGCCTTCTCTCTCCCACTCAGGAACATGGG
CAAATGCCATGGGCTGGAGCTCTCACCTGCTCTTTTGTGCTGAGCTTCACTATCTCTTCTGGGCACAGATTGGTTACAGGGATAT
AGACCACCTGTGTGGTGCCCTGGGGGTTATGTCCACGGGGAGCCTCACATGAAGAAGAGAATTTCCTTTGGGATGTGTATTTCTTG
TGGTCTTAGCATGCTGGCAATTCTTATTTTTGGCATCATTTAATCCTTACAACAACTTTGTAAGGTTTTCACATCCATTGGTCATT
TCTGAGTTGAGCACCTCAACTCAGGTGAATGTGTTTGTATGAATGTTTGTTGAGCACCTACTATGTGCAGGGCAGTGTAGTAAAAA
ATTGAGGACCTGTGGGTTTTTTTTTGAGTATATATAATTGTGTTCTTCTGTCATTTTACATGACTTTCACCTCATTTCATTGCTGT
AGCAACACTGTGAGGTTGTTGCTGTCACCCCCATTTCTCAGGCGAGGAAATAGAGGTTGAGTCAATCTCCCATCTTGGATTTACCT
GACATCACAGTCTGAGTCTTTTGTGGCATGTGCAAGAGTGCACTGGCTTTCGGGGCCTGAGCTGGCTGGCCTGGATTGAATCCTGG
CTTTGCCATTTGCAGCTGTGGTCTGAGGCAAGTTATTTAACTTCTCTTGACCTCGGTTTCCCCATCTGTAAAATGGGGCTATACTA
GTTGCTCTGTGTAGGAAGGGTCTGAGGTTTCAGTAAGATACATTTTGCACAAACCCCACACACACCAAGTATCCATGAAGCCTGGT
TATCCTTATAACCTCAGAGCGGTTTGGAGAGGGTTAGGAACTTGCAGATGGTCACATAATCGGTGTATGGCAGATCTTGGGCAGAG

CTGGACTGGTCTTCCTAATGAGAGCTCCTTTCCCCACTCTGCTTTCATGGACAGACCCTAATGACCACCTGGGTTATGTTTTTGTC
CCTGTCAATTCCTTTGTCCTTGCTGATAACCTAAGGGAACCTTGAGAGCCAGAACGAGACAGGAAGAAAATGCCTGGCTGCTGCTT
GCGGGGTTAATTAAACCATTCCTGGGGTTTCATTTGCTCTCTAGTTAGCACCAAAATAAATCCTCCAAATGGCTTTGAGCCTTCCA
GTTGACATTGCATTTTGAATTCTGACAGCACCAGCATTTTACATCTTCCTGAGGATGATAAATGTTTAGATAGCACCGAAGATAAT
GGTAAAAGGAGAAACTGGGGGTAGTTCAGGCTGTGGCTGAGCAGATTGTAACTCTTTGGTGTGACGAATGGGTGAGAAGTTGCTTA
AAGGACACATCTGATGTCCCCTGCTCCTAGGGGTTGAGAGGGGGATGGGGGGTGGACTCTGTTTTGCCATCCGGAGAATCCATTGC
CTTCCCGATGCTTCTGGGGTCTCATGCTTGAGTCTTGTTTCCATGCGTTCTGTCAGATGTTAAGGTCTTGTGCATAATGGTTGAGG
TGTCAATGTGCAGGCGTTAATGTCACCATTCTTATGTAATTGTTACCCTTTATTGAGTGCCTACTATGTGCCAGGATTTAAATATT
TCTCTCAGCAACCCAAAGATGTAAATGTCATATCACCTTCATTTTACAGATGTGAAAACAGGCTCGGAGAGGCTGAGACACCCAGC
CATTAAGGACTCAGCTGGGATTCCCAGCCTTCCCTGGCACTCTCTCTTTTTTTTTTTTTTTTTTTCTGTTTTGAGACAGAGTCTTGC
TCTGTCAGCCAGGCTGGAGTGCAGTGGCACAATATCAGCTCACTTCAACCTCCCTCTCCCGGGCTCAAGCAATTCTCCTGTCTCAG
CCTCCCGAGTAGCTGGATTTGCAGGCATGTGCCACCGCACCCGGCTAATTTTTATTTTTAATTTTTTTGGTAGAGACGGGGTTTC
ACCATGTTGGCCAGGCTAGTCTTGAACTCCTGACCTCAGGTAATCTGCCAGCCTCGGCCTCCCAAAGTGCTGGGATTACAGGTGTG
AGCCACCATGCCCGGCCTTCCCTGGCACTCTTGAGCATGTTTCCAGACCGCCTGCTTCCTACCCCAGACATGTCAAAATGCGGTGC
TGTAAATGACAGGGCTCTGGTCCCAGATGGACAGCCCGGGCCCTTTGAAGACACTCTGGCCTTCAGGAGTCTGGATCTTTATGCCT
GATGGAACTGACACGGGCCTGCTATGTTTACAGTGCCTGTTCTCCTGCAGGGTCTCCGGAAGTCATCAGCCGTGCTGGCCAGCACT
CCGGAGGATGACAGGAAATTCTGCTGCAGCTCTGTGGGCTGGCTGGGGTGCATGCTGGACAGGGAGCGGGCGGACAGGGCTGGTAG
GGAGGAAAACACCACTGAAATAGTTCAGACCGTATTGTCAGTGAATTCCTGGATTAGTATGCAGTGCTGCCAATGATCAGATTTGG
GTCAGGAAGCCCAGATGGCAGAAAGATTGTGTTAAATCTTTATATTTTTCCACGAAATCCTCACCGTTTCAGACCCTGTTTTCTAA
GCTATCACCCTAATAATTGTGGATTTTGGATTTCCAAGGCACGTTTTATAGTGGTTGTAAGGAGGGGATGGTTGGGTTGGGGATTG
GGATGGGAGGGGGGTAGCTGAAGAAATAAGAAAGTGCTGCAGAAAGGAGCACATTCCACACCCAGCTTCTTCCTGATTGTGTTTCC
CTCCAAGCAGCGAACAAGAGGCTGATTCAAGTGGCTCAGTGGCCTCCCTGCCTGGAGTTGGGTTCCTATGCCTTCCAGTGACTAAG
GAGGAAATTCCATTTCAGTTAATCTGTGGCCGCGCATCACCCATGGCATTCTCTATCTTAATTTTATGGCTGCCTCATCTTTTTGG
AATTCGCCTTCTTTTTGTGTGTTGGGTGATAAGTCAAGGAGACATATGGCACAGCTCCACAAAATACAACCACATTTTAGTATTCC
GTACACAGTCCTTTCTGGGCATCCCAGGCTTTGTATTTTCACGTTGGTCGGCCGTGAGGAGGGTGGATCTTCATTATCTGTGCATG
CATGTGCATCCCTTCATCCCTTCCTCTTCAGTTTCTTTTCTTTTTTTTTCTTTTTTCTTTCTTTCTTTTTTTTTTTGAGATGGAAT
CTTGCTCTGTCACCCAGGCTGGAGTACAGTGGCGTGATATTGACTCGCTGCAACCTCCGCCTCCTGGGTTCAAGCGATTCTCCTGC
CTCAGCCTCCTGAGTAGCTGGGATTACAGGTTGTGCCACCACGCCCGGCTAATTTTTGTATTTTTAGTAAAGACGGGGTTTCACC
ATATTGACCAGGCTGGTCTCAAACTCCTGACCTTGTGATCCGCCCACCTCAGCCTCCCAAAGTGTTGGGATTACAGGCGTGAGCCA
CCACATCCAGCCTCCTGTTCAGTTTCTTAACCCTTGATGGGACTCCCGGCTAAACACCCCCATTGTCTGGGACCTTGAGATTAGAT
GCAGTAGCACTGCTGGTTTCATGGTGGACTTTGAGTCGGATGGAGGAACTCCTTGGAGTGCGGGAGGAAGGGCTGCGGAAGGTGTC
TGAACTATCGTAGCAGTAGGGTCCCTGCCACAGGGAGGTGAGGCAGCCATCATCACCAGTCCTAAGAGGTACGGTCACCCTCCGCC
TCTGTCTCCCTCTCCAGTCCCCATCCAATTTCAGCTTTGAACCCAGCTGCCGGGTCTTCAAAGCCTGTTCTGACTTACCCAGCAGA
GTTGGTTGCTCTGTCCTCTTAGCTTATAGACGCTGCCCGTTTTTTCCCCACGTGGCCCCTGCCATTTAGGTCCATGTCTCTCGTGA
TCCTGTGTGTATTTCCAGTGGAAGGACAAGGTTTGGCCCACTGTATATTTGAAGGCCCTCAATATGGCGTTAGTTCGTATTTACTG
ATGGATGAAGAACTCTGAGGAGATCTGGTTGAAAGAGACTTTTAAAAATTAAAGGGAAATGGTCACGCAGGCCGCGTTTTTGTCC
TTGCCTATTGGCTACTTTTGCTATTCAGATCATCTGTGAATGTAAGTGACTGTCTGCTGTGTTCGCAGTGTGGTTACTGGGAAAAC
AGAGCTCTCTCTCCCCAAATCAGGTGAAACTACAGGAAAACAGTAGGCAAGAGTCTTCAAAGTGATGCATCTCGAGAGAGAGAGGG
CCTGGGTCTGAATCATGGCTTTGTGCCTTACCAGCCTGTGATTCTAGTCACTTTACCTTCTGAGCCTTGTTTTTCCCAGCTGCAAA
ATGAGTGTGATAATAGCGCTCGCTTCAGGGGATTGTTTCAAGGATCAGTTGAAATAGTGCACACCGAATGTTTGGCATATGTGCGG
AGCTCTTTGGGGGTGACTCATGGGCAACTGTTTTTCATTTAAGAACTGAGTGGATTGGACCTTTAAGAACTGCTTTGGTCCCACTG
TCATGGGAGGTTAGTGCTGTCTGGAGCAGGCATGGGGATGTGGTGGTACAGGGTAGGGTGTAGGGGTCCCCAGAATTGGGGGCCTC
CGTGTTGGAAAGGGACAGTGGGCAGAGTCCATGCAGGCGCTCTGGGGCTGACAGGGACCTGGGACTTCTTTCTGAGAGGCAGTGAC
CAGCCAACTGGAGTGGAGGGTTGTGGGGAAAATGTTTGGCCAAAGGATTTAGGAACCAGAGAGAAGCAGGCACATGATGCTCCTTT
TCTTGGTGGGGTGTTCATCAGGCCTGTAGCTGCCACAGTCATCACTGTGGCTGAGATTCATACCTGTAGCACCAGAGTGGGAGGCA
ATTTTTCCAACATGGGGCAGAGGCTGGGTGTGTGGGGATTGGTTGCAAGGTGCTGGGAGTGGGGTGCAGGGAAGGGCCTCTGCACA
CCACGTCCGTCCTCATCCTGCTATGAGGTCAGCTCTGTACATGTGCTGGAAGAGCAATTAGCGGGGTGCCTAAGCAGTGTCCATCA
TGGACGTGGGTCTCTGCTGGCCACGCAAGGGGAAGATGGACTGGTGGGAAGATTGGGGAGATGGAAGCGGGGGCAGTTGTGTGCG
AAAGGTACAGGGTCGTGGAAACATGCACAAGAAAGTGACATTTTAGAAGGTGGTTTTTGTTCCATAATAAATTTATTAGCTTTGGA
ATCTGACTCTATTGCCTTGGCTTCTCCAAACTTTGGTGTCCTCATCTGCAAACTGGGGATCACAATCTCAGTTTTGCAGGGAATGT
GAAATCCTGACCTGAAATGATCACATGTGTAGGAAGCTGACAAAGAATGCTAATTTTCTTCCTTCTTTTCTTTATGGCCTTTCCTT
GGTAAATCTCTGTGTGATTGATAACTCATGAAGAACCTACCAGTCAAATCAGTTAAATTAAATGACTTCCTAATGATAATAATTAG
ATTAGTAACTAATAATCATAGCTGACATTTATCTAGTGCTTATTAACTTCTGGGCAGTGCGAATTTGGTCTCATCTGTTAAATAAT
TTAATTCCTGTAATAATCCTAGGAGGATGGTGCTATAATTAGCCCCATTTTACAAACAAGAAAACTGGAGGATCAGAGAGGCTAAG
TATAATGTCTAATGTCACACTGCTGAAAGTGGCTTGCTGGTGTTCCAGCCCCAACAGTCTGGCTCCAGAGTTTGTGCTATTAACCA
TTATATCAGTCTGCTGTGTGTGTGTGTGTTTGTGTGTGTGTGTGTGTGAGAGAGAGAGAGAGAGAGATTGTGCATTGTACGGTG
AAGAGCAATAGAAATCATTATAAGGCATTCCTGTGCTACAGTCTTGGTGGGTCAGGCTTACTTATAGAAGCTGTGAGAAGGATGGG
GCTATGGGCTTTAACTCAGTTCTGACAATCTGTCACTCCCAGAACCTGAGGCAGGCTGGACCTATAGACTTCCACCTCCTCTTCCT
CCTGCCCTGAACCGTCCAGGGACTCAGAGGAGGTCAGACAGGTGGGACTCCCAGAATAGTGACCTCCTCTGCCACTTGCAGCCTAT
GTCGGGCTCCTCTTATAGAGCCACCCCCTTGCTTATGCTGTAGTGTTCCTTTGTTCTGGCCATAGTTTTCCTGGGCCTGTGTCAGT
TGAGTGCGTCCAGATGCAGCTGGGTTGAGACTCAAAGCCCTGAAGATACCGCAAGGGGAGTGCTCCTGTGTGCAGTTGCTCTGACC
TGCACAGCAGGTCGGAGTGGCCGCCGGTCCCCGGCTGCCAGCTGTTTACCTGGCCGGCCTTGCAAAGGCTCTTCAGGGCCAGGCCC
CAGCCGGTGAATCACTGAAACTGATCCCCGTGGCCACTGTGAAGTACGGGACTGGAGGTCTCCGGAGGAGATGGCAGCTCTAATAA
AAACAGGCTGCCAAACCGCAGAAACTGTATAAGCCTCTGTTTCTCATCCTCGCTACCACCCCTGCGTAGTCCCTGTTTTACAGTTG
AGGAAACCGTGGCCCAGGGAAGGTCGGGAACTTGTCCAAAGGCACAGTCATAGAACTAGGGCTTACACCAGCTGTGTCTAATAGCA
TGCGTGCCCCCTCTGCTGTGCTTTTATTTGCTTCTTGGAGTGCTGTTTAGAGAAATTGATGGTTCTTTTGTTGTGACTAGGGGTCC
GCACTATGGGTGACCTCCCTGCTCCCTTTGGCCTCAGGCCCTTATCTTAGGCGATGGCCAAAAGAACAGCTTTACATTCAGTAAAC
TGTTTTAACTGAACCTCACCACAGGTTTACGGGTGGTGCTCGTTTCATAATGGATAAAGAATGGGTGTGGCTCAGTGTGGGGAAGT
TGGTGGATGGGGAGAGGGCAGCAGAGTAACCAGCTTTTAGTGGAGACCTGGATTTGAGTGCCCTGAGTTAGGTGAGCCTTTGTACC
TGTTTCTTCACTTCTTGGAGCAGAACATTAGTTTCTCCAGATCCCACATGTGATAAAGTTCTGTGATCCTGCAGTTTGAACATCCT
GGCATTCAGCTCAGCCCACTGTGCAGAAATCTATACTAAACATGTAGACAGTTTAGGAAGAGGGATTGTTTATTGTGTTGAATGTT
ATAGAATAATAGCTACTTAAATTTTGGAGCACTTGCCATTCATCTTCCAAGTGATTTCTACGTGTTGACTCATGTAACACAGGCAA
CCACCCTATAATAAGTACTATGTTTACACACCCACTCTACAGATGAGGAAACTGAGGCTCAGAGGAATTAAGTAATCTGCCAAAGA
TCCCTTGGCAAGTAAGTGGCTAAGGGGGGATTGGAACCCAGGGAGAGAGCATAATCCTAGGAGGAGATTCTGCCAAGGTACAGCCC
TCCACGATGGAAGGGCAGCAGCCTCCTGTGAGCTGACTCACTTGAGTTATTCCACACCATCCCAGTGGAGAAGTTGGATTATCCCT
ATTGTGATAGAGGAAGAAAGGTGAAGGAATTTGCCAGAAATTATAAATGGTGACATTCGCATTGAACACATGTCCACTGACCCCAA

ATACTGGGTCATTTTATATCTTTCCCTCCCTTCTTTTTTTTTTTTTTAGACTAGTCTCACTCTGTCACCCAGGCTGGAGTGCCATG
GCACGATCTTGGCTCACTGCAACCTCCACCTCCCGGGTTCAAGCAATTCTTCTGCCTCAGCCTTCCAAGTAGCTGGGATTACAGAC
ACCTGCCACCAGGCCAGACGAATTTTTTGGATTTTTAGTAGAAATGGGGTTTCACCATGTTGGCCAGGCTGGTCTTGAACTCCTGA
CCTCAGGTAATCCACCTGCCTTCACCTCCCAAAGTGTTGGGATTACAGGCGTGAGCCACGCCCACCCACCCCCTTCACACCCCACC
TTGTTTTTTTCTTTTTAGTAGAGATGGGGTTTTACCGTGTTGGCCAGGCTGGTCTTGAACTCCTGATCTCAAGTGATCCGCTTGCC
TCGGCCTCCCAAAGTGCTGGGATTACTGGTGTGAGCCACCACGCTTGGCCTCTTCCCTCCCTTCTTGCTAACTTTGAAGCTCAGCT
CCTTGTCCAAGAGCCTTTCTGTCCAGGTGTTTTCATGGTGCCTGTGCCTTCTGCTGGTTTGGAGTGAGCGGTTTCCACAGCATCCT
CCCATCAGCCATGCATTCCTCAGGGTCAGGCGCAACATGCTCTTCATATCTGCTCCCTGGCACTTGGCCTTCAAACCAGGAATTCA
CTGCACCTAGCCTGATCCATTGACCTGGGCCCAGTATAGGCCCTCCCATGTTGGGAGGCCATGGGGTGTTAAGGTTAATGCTGGGC
CACACATGACTGTGATGAGTCCCTTTCTCTCTGCTGAAGAACGTGTTTCCATGGGGGACAGGGGCAGTTAAGCCTGGGTTCTGTTG
GGCGCTGCTGCTTGGGTGGGTCCAGTCTGCCCCATGTGGGGTCTGCATTCTATGCCAAGTGTTGGCAGGTTCTGCCAGGTAGAGCT
CTTCAGTATGGAAGCACTTCCCTGTGCTCAGCCTCTCCCCGACTGTGCACATTTCCTTCTCTTCTGCTGGGGAAATGGAAAACAGC
TGACCACTGATTCCTTGAAATCACCTACTCAGACACTCCAGGGTCTGTGTGGGATTAAGTCGTCCGCAGTCTTTCCCTCCCTTAAT
CTCTTCTAACCTTCCTCGAAAGTGCCGTTTCCCACCCCATTTTAATCATGCCCTTTGCTCTCGCCTGGACATCTCCAGGGGCTCCA
GTGCCTCTCTAAGTGAAGGGTTTGGGGCCACACACATGGCTGTCTGTTCTCTCCAGGCCCACATGCAGAATCCAGCAGCCCCCTGG
CCTCACACCTGCTTTTCAGTAGTGGCTGGGACTGCCAGATGAATAGGCTTGAGGCAGCATACGAGTTTGCTATACACTTGCTCAAA
ACATTAGTGGACTCAAAACCAAAAAGACAACCCAGTTTCAGAATGGGCAGAAGACTTGAACACACATTTCTCCAGAGAAGTTATAT
AAATGGCCAATAAACACATGAAAAGATGCTCAGTAGCCATGGTTGTTAGGGAGATGCAAATTAAAACCATGAGATACCCCTTCATA
CCTACTAGGATGGCTGTAATTAAACACCACCACCACCAACAGCAAAAAAAAAAAAAAAAAAAAAAAGGCAGAAAATAGCAAGTGTT
GGTGAGGATGTAGAGAAATTGGAACCCTTGTACATTGCTGGTAGGAATGTAAGATGATACAGTCTCTGTGGAAGACAGTTTGGTGG
TTCCTCAAAAAGTCAAGTATAGAATTACATATGACCCAGCAATTCCACTCCTAGGTGTATATCCAAGAGAGTTGAAGGCGTATGTC
CACAAAAAGCTTTTACAGGCATGTGCACTACTCACAACAGCCAGAAGGTGGAACAACCCAGATGTCCAATGTCTGTCCGCGGAAG
AATAGCAAACAGTATTCAACTGTAAAATGGAATGAGACATTGATACATGTTACAACATGGATGAACCTCAAAAATGCTGTTCTAGG
TGACAGAAGCTAGGCACAAAAAGTCACATATTGTCTGATTCCATTTTTATGAATTTTCAAGAATAAGCAAAATTATATAGACAGAA
AGCAGATTAATGGTTGCTGGGGGCCATGGGGAGTAGCTGCTTAATGGGTACAAAGTTGTTGTTGGTTTTTTTTTTTTTTTTTTTGA
GACCGAGTTTCCCTCTTGTTTCCCAGGCTGGAGTGCTGGAGTGCAGTGGTGCAATCTCAGCTCACTGCAACCTCCACCTCCTGTGT
TCAAGTGATTCTCCTGCCTCAGCCTCCTGAGCGGTTAGGGTTACAGTCATGTGCCATCACACCCGGCTAATTTTGTATTTTTGGTA
GAGATGGGGTTTCACCATGTTGGCCAGGCTGGTCTCAAACTCCTGACTTCAGGTGATCCACCCACCTCGGCCTCCCAAAGTGTTGG
GATTACAGGTGTGAGCCACCACACCTGGCCAATGAGTACAAAGTTTTATTTGAGGGTGATGAAAATGTTTTGGAACCAGATAGACA
TGGTGATTATACCACATTGTGAATGTACTAGACAAACGTCACTGATTTTGTACACTTTAAAATGGTTAATATTACTTTATGCAGAT
TTTACCTCCATTAAAACAGNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNC
CTGTAATCCCAGCACTTTGGGTAGGCCGAGGCGGGCGGCAGTATCACCTGAAGTCGGGAGTTTGAGACCAGCCTGACCAACATGGT
AGAAATCCTTTCCCTACTAAAAATACGAAATTAGCCAGGCGTGGTGGCACGTGCCTGTAATCCCAGCTACTTGGGAGGCTGAGGCA
GGAGAATTGCTTGAATCTGGGAGGCAGAGGTTGTGGTGAGCCGAGATTGCATCATTGCATTTCAGCCTGGGCAACAAGAGCGAAAC
TCCATCTCAAAAAAAAAAAAAAAAAAAAAAAAGAGGATATGTGGACACTATGTCTGGCACTTAAACTGGTGGATTCTATAGAGATG
GGATGGGTTCATTAGCCCCATTCTACAGATGAGAAAGTAGAGGCTCCATTAGAGCACACAGTGTAGAAGTGGAACCAGGATTGGTC
TGACTCTAGAATGCTGCTTTTGATCACTTTTCTATGTTCCTCTTCCTCTACAGTGGACAGGTTTTGTTTTGTTTCGCTTTGCTTCA
GATTCTTGGCTCATGCCAAGTGCTGTGTGCCATGGGTACATCACCTGACCCTGAAGAGCCCAGATCTATGTCTTTTCATGCAACCT
TGCCCTGTGCTTTCCACATATTCACATCCTTCACAGTCAATCCCACCCTGGCCACTAGGCCAATAGGGACTCCCCACATCCCACTT
ACAGGGGGGCAAATATTTATAGCCAAAAGAAAACAGAAGATGTGGGTTTGAGTCACAGTTCCACTATGTGGTCATGGATGAGTGCC
TTCAACCCTCAGTACCTCAGTTTCCCTAAATGTGCAGTGGGAGAGCATGAGAACACTTATCTGATGTCTTCCCTGGGGTTTTGTGA
GGATTAAACACACCATGTAGGGGCAAGTGCTTTGAGAATGGAAAAGCACTGTGCAGACATGAGTGATGTGCATGATGGTTATTGTT
AGATGTTCTCTCCTGGAATCAAGTTCAAGCTTCTTGCATACTCAGCAGAGAACAGACCCAAACTCCCGAATCTAGTGCTGTGGCAT
GTGGTTGGTGCTCAATAATGATTTGTTGTGTGAACAAGTGCATGAATGAGTAGTAGGTCAATGACCTCAATGTGGGCCTGGTCAGG
ATTTAGATTTCTTGTCTTTGTGTGTGCTCAGCCCAGGACTGAATGCACAGCAGGCTGTCAGGAAGTAGTCATGGATTGATGTGTCC
GTATTCGCCATAACCAAGCAGGAGATAGATGATTTCGTTCTGGTCTCTGGATTTTGGCTGCATTTTCTCTCATAGTAGGAATTATT
CCCGGAAGATCCAAATGCTCAGCTTTGTCCTCCTCTCATCCTGGTGAGGCGTGTGATCCAGGTGAGAAGCAGGGAGAACTGGCCTT
CCTGGTGGGAAGTTGTGGGCCATCACCTTAAGCTTGGCTTGTAGTGGTGGGAGACCTGTGTTCCTTCCTTCCTGAGCCCCACTTCC
TCTTGATTTTATTGTATTTTTAGCAGCACTGAGCTGAAGCTTTCTGGGTTGCATGTTAGAGGCAGCACTTCTTGCACAAGCTAGGG
GAGCGTGAGTATCGTTTCGGTTCTGATAGCAGGATGGATTGTTATGTATCCTTTCTGATGGAGGCTACCAAAAGCTTTAGATGTTA
GGATGGATTATCTCACCTTGCACAAGACAGTGAGTGCTAGAGTTTAAGTTTTCTCCTCTGCAAAATAAGGAGTGGCAGTGAGACTT
AGAGCATCTCTCAAGCCCACTTTGGCCCAAAAGTTCCATGACTGTGGCAGTCTAGAGTAAATGTGTTTGATAGCCCCTAGTTACAA
CTCTCTTCTGTCTCTTCCCCTTTCTGCCTGCGCTACCATCTAATCCATCCACGCAACCACCCATCCACCCACCCACATTTCCTAGC
CAGCTCTCTGGACCAGGCCTTGTGTTAAGTCCTGAAGACACAGAAAAGGCTAAAACAGCCCCTGGTCTTAAGGACTCACAGATCAT
GAGGAGGCGATAGATACTGGAATAGAAGGCAAAAGTCAAATGTCATGAGTGCTGGGATTCAGAAAGGTGCAGGCTGCTCTGGTAGC
AATAAAGGAAACCAGCTCTCCCTTGAGATCCCTTTCATGAGGGTTATAGCCTGGATCCTGTGATGACCCAGGACAGACTGGTCTTG
ATCCAGGGGAACCATATCAGTGACCTCAAGAGGCCCCTTTTAGCCACAGTAATCTATGATGTGGAGTTCTCAATTGCTGGCTTTGC
TGGAGAAAGAGAGAAGGAAGAAATAGAGGGGCCGGTCAGCCCTCCTGTACATTTAGTTTGCGGTCTCAAACCTCTGTTTCCTGGAG
CTGCAGGCTGTATGTGTGGGAGGTGCACAGCAGTGGCTTGGCCGCCCCAACGCGCCTGCAGCCCGCTCTGGGGACAGCCGTCTTTT
GGCAGAGTCTGCCTCTGCAGTGGTGGCAGCTGGCCCAGGCAGACCTCCTCTCTGTCCGGTTAGTCAGCGTCTGGTGGCCAAGGGCA
GAGTGGAGCCTGTGTTTCCGACAGGCCAGCTGCCCCTCAGAGTGAGGCAGCGCCGTGATTCTGAGGGTGATGCTGGCGTTCGCCCC
TCAACCCTGTCCCCCACAAGGAGATTACGGACAAGGCAGCATTTCTAATAAGCAGCCTCTGCTTGCCCCGTAGCAGATGAAAGACT
GCGTGTAAGAGTGGGTAATTTTGGGTTTTCCTGATGAAAGTAGTTGACATTTGTGCTGAGACCTCATGCGCTCTGCAGCGCTGCCT
TTCATCTCTGCTCTCAAAGCCCTGCTCATCTCTGCCATGACTCGGGGGCGGCCTGCGCCACCGGAATTCAGTTGGCCAGTGGTGA
AATATAGAGGAAATGGTGGAATGACCCCTGAAGGCAGTGGATGGGGGAGTGGAGACAAGTCCTGACTTTCCTGGTGGAGGGGATGT
TCCTGGGGGTGTGGAACTGTGTATCGGCCCATGCAGCACAGCCAGGGAAAGAGTGTGGATCTTGGAGCTTGGTATGGCTGGCTAGA
TTGGAAAGGCAGCTCCTCCACGGACTTGCTCGGTGAATGGAGGGCATCCTTTCTCTTCCCTGATCTGTGTCCTCATTTGTCAGTGG
GAATAATAGAATAACATTACCACCAACGACGATGGCTTTCACAACTGGAACCACTAGGCCCTCAGGAATGTTTACTGGCTTCTAGT
CTCATTGATAAACTGAAGCCATTGTGCCATAGCTCTAGAGAACTGGCACTAGGGGCTTGCATGATTCATCTGAGTTCCCCAGACAA
TGCTGGCACATAGTAGGCCCTCCATAAAGTAAGAAGGGACACTTTGCTCCTGACATCCATACAGCTCCTCTAGCTTTGCAGAGAAA
GCCCTAGAGAGAAAGAATGTGAAGGAACAAAGGGTCAGGTTAGGTTGTGCAACCTTTCTGATATGCATTTGTAATGCAGTGAGCAA
ATGCACAAGCCTTGAGTCGCTGCATTCTTTTCTAACCACCCAAGAGATAAGTTGAACCCCCACTCGTAGCAACCTGGCCACTTGTC
TGTTTAGTCAGCCCTGGAATACCTCCACTGACGGGGCACTCACTACCACTGAAAGATGGTGTGCAGAATTCTAATGTTCTTCCTCT
CGCGGGGTCGTGGGGGCTTTCTTCCAGTTCTCTGGGTTCTGATCGTTGGGGCTGTCCTGACACCCTTCTTCAGATGACCGCTGTCA
GGTTGGTGAAGGCAGTGCGCGTTGGGCCTTCCCTCCCTAGAGATGTGATTGCACGTCGGGTACCAGGAGCCTGGCCAGCCCTCAGC

TTCTGCTAACCAGGGAGCAGTGAAGCCCGGTTTACTTTTGTTGGCTTGTTTATTTTCTAATGTCACCTGCCTCTGCACTTTAAGTA
GTATTTGGGCTGCTATGGAGTGTGCACATTCTAACACAGCACCTTGTCAGCTGGCTGAGAAGCTGAAAATTCTCGGCATTCCTCCT
TGATGTCAATTACTTCAAGGAGCAGGAAGGTGACTTCTTTTAACAAGTGGAAAGGATTCAGGTAACAAAGCCATTTCCTCTTCTTA
ACCCTCTACTGTGGAAAGTTATTGACTTAGCGACACAGTCTGTTTATCTGGGCTTCAGGAGCAGGGCTTTGGAGACTGAAGGCTTC
TCCCCACTTTTGTGCAATGTTATATATTTTTTCTCACTCTTCATCCAGTCTGAGTGGGGTCTGCCCCCTCCCCTGAGCAGTCACCA
TCCTGCTACCCACTCCGGCTCTATTGGCGACTGTGGCATAGGGTGGTGGAAAGGACGCTGGACTGAGAGCCCAGAGATCAGATGTC
TGACCTTCCCTCTGCCTGGAAGAGCTGCCTGGCCTCTGATCAGGGTCCCTACCTTCGTTTTCTGGCCTCACTCACTGTGGCCCACC
ATCCAGCAGTGCAGGTGTCCTGGGGACTCGGGGAAGGTCAGAAGTGGAAGGAAAGCCTCCAAGCCCTCGCATGCAGGCCCACCATT
CCACTGTCAGGTTTTCTTCCCTTTCCTTCTGCTCTACATTTTGTGCCAGCAGTTTGGGGGTCTCCTGAATCTTATCCCTTCGCAGC
GGTAGGCAGGTGCAATTTCTGGCCAGTCACTGAACATGAAGAAGCATCTATAGGCGGAGTTAAATCCAAAACACTTGGCCTGACAC
ACCTGGCCTTGAAGTCTCCCCCAGCCTCTCCTCTTGTCCCCAACAGTGGTTCATGTTCTAGCCACACAGAAATTGAGGGGGTCCTG
TGTTATCTGTGTTCCTGCAGACAGAGCTTGCCTAGGACAGCAAAAGTCATCCTAACATAAAAATATGCATGAATAAACTCACTGCC
AACATTCAGCCCACCCACATCCAATGATAATCAGTCATTTACTGATGGCCAGACGTTGTAAACATCTGTCAGTCCACTGCAAGAAC
AGCAGAAACTCGTAGCTGACCTCCATTTGGCCTACTCACTGAATTAAGTGATGTTACTCATTCGCTTTGTAAAAGCAAACTGAGTG
AGGCCCCAGGCTCATTGAAGACTCTGTGGGTTACCCAGAAGCCCAACTTTCCAGATTCAATATTTTTTCTATTTCTTAGCTGTGTT
ACCTTGGGCAAGTTAATTAACCTCTCTGTGCCTGCATGTTGCATCTGTAAATGAGACTAATACTAGTACCCACCTTCTAAAGTGAT
TATGAGCATTAAATGAATTAGTACGTTTAAAGGCTTAGAACAGTGTTTTATGATACGATAAACACTCAATAAATGTTAGCTATTGA
TATTGGTGTGCCCAGAAGGCTTGTTACTACTAGTTGATTTATGTGCTTGCCAAAAGTTGTTGTGTTGGTAATTAAGTACGACATAA
ACTAATGAAAATTGAGTTTATAAAGAGTTGTTTTATGAAAGTTTAGTACTTTGGATAGAATGGATAATATTGATTTGCTAAAAAA
AAATTGTCACCAAATAACTTAAAGGCAAAACAGCTGTAAAAAATTGTAAAACCATAGTTTGTATTCCATATGCTTTACAGGTGATT
AACTTTGGCACTGCTTTAAAGAAACTGAAACCAGGAATTGTAGATGTTGACTTGTGAGTGTGTATTTTGCATGGAAGATGATTTGC
ATCTCTAGTCAGCGTGCCAAAAAAAGATCCTACCCTTTGATTGGTTAATAAATATGCAACTATAATAAAATATTTAAGTGTTGGTC
CTTAATGATTGCCTACATTTTCAATGAAATGACAGTGGCAGAACTGAGGAACAGGGGTTTTTCTCTCCAAGGCAGATCAGCTGCCA
GAAATTGCACGTTTTGTACCAGCTGCAGGGTTTCCTGGAATCAGCAAAGGCCTGTGAGAGGCTGCATGAACCACACACGCTTACTG
ACACCTCCTGTGTGCCGAGCGCTGAGTAGTGCTGAGGATATAGCAATTGGGGAGCACTAGCAGAATCAGGGTTGTTAACCGTGGAG
AAGAGAAAGGAAAGATGATGTCTCTCTATGCAAATCAAGGGAAAGTGATTTAAAAATGTTAAAAAAAAAAAGTTAAGAGAGGTCTT
CGAAAGGTGTTTTGCAAAATGATTCCTTGGATTAATGATGAAGGAGTGGAGTGAAGGAAGTTTATATTTTGTGAGAAAACGTGTGC
TAAGGAAGGCCTCGACATTAGATTCATTCTGTACACAACTCAGCAAATTAGATCATAGTTTAAAATGATAATTTCAGGGGCCGGGC
ACAGTGGCTCCTGCCTGTAATCCCAACACTTTGGGAGGCCGAGGAGGGAGGATCATTTGAGGCCAGGAGTTTGAGACCAGTCTGGC
CAACATATCGAAACCCCATCTCTATTAAAAAATACAGAAAATTAGCTGGGCTTTGTGGTGCATGCCTATCTTCCTGTCTACTTGGG
AGGCTGTGCATGAGAATCANNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNACTGCAA
CCTCTGCCTCCTGGGTTCAAGCAATTTACCTGCCTCTGCCTCCCGAGTAGCTAGGACTACAAGCATGTGCCACCACGCCCGGTTAA
TTTTTGTATTTTTAGTAGAGACGGTGTTTCACCATATTGGTCAGACTGGTCTTGAACTCTTGACCTCGTGATCTGCCCGCCTTGGC
CTCCCAAAATGCAGGGATTACAGGTGTGAGCCACTGCACCTGGCCAGTTCAGGTTTCAAAGTATAAGATCAATCTTATTTTATGTA
TAGTTTATGTGTAATTATAAATTGTATACTAATAAAGTCATTTTTATAATTCTTTATTTCTTTTTCCAAGATACCCACCCCCCCCC
CACCAAGGAGGGCTAGGACAAGTGACAAAAGTCACCCCAGATCACAGCCAAATGGGTAGAGCTAGGAATCCGAAGTAGGTGATGAA
GCTTGATTTTTCAAGATGCTGCAACCCCTCTCCCCCATATGCTACATTAAATATCTGGACCTATTTTAAGTGGGTTCTTTTCTGGG
GTAATGAGGTTCTCTTCTGCCTCAGTTTTCCATGGCTGCAGAGCTCCTGCTGTTCCTTCTGCCTAGTTCACTCTTCTTTGCCTTGA
CTGCACCTCTCTGAGCTACCTTCCTACCTCCATCCTTGCATTCATTAGGGGTTTCTTTATGTGTCCAGCTCACTGTGGGACCCAGG
ACTCCTTGAAGAGGGAGATCTTGCTCATCCATCTGTGTCCCCCACTCTACTGCCAACACAGTGGTAGGAAGGGAAGGCGGCAGGCT
TAGGCGAGGTCCTGCAGGCTGGACAGGATCTGTCACCATTACTTAGCCCAGGCCCATCTCTTTACTTCCAGACCAACTGGTGTCAC
CATAATGCCTTCTGGCAATTTGAAGATTCTGGCAACCTCTTACCGGATGCAGCTGGCAGGATAATCCCTGTGCATTAGCCACTTTC
AGAGGTATCTGGGCCCCCAAAAGAGTCACGGGAGTCCCAGACATGAAGGAGCATTAGTGGCCATCTCCCCACCTTGCTGTATCAGC
AAAACCGCTAAGCCCTGACCCATATTTTGGGCAGTGCTCACCCTATCTCATTTTATCTTACTGTATATCTCGTTTGTATCATTTTA
TTAGGTATCTTATTTTATCTCATTATATGGTAATCTCATTTGCTGGGTAGACCAGTGAGGAGGGCTGGGACAAGTGACTGGAATTG
CCCAAGGTCACAGACAAGTGAGGTTTAGAGATAGGACTCCGAAGCAGGTGGCGGAGTCACCGTTGAGTTCATCATAGCGATGCTGG
CTGCCTCAAAGTACTGTTTTAGTTAATGCTCACAAGCAACTCTGCGAAGCAGACGGGCCATTCATTTGTGCTTCACAGTGTGGGAA
ACTGAGTCAGAGCAGGTTCATGTCACACAGCTGATAAATGACAAAGCCAGATGTGAAGCCTGTGAGTTGGATTCCAACATTCATCT
TTGATGAAGCGTTCAGGTTGGGGCCTGGTGTGGGACATGCTCAGAGGAGGACGAGGAGGATGATTTGGGGGAGGCCTCAGAAGGCG
TTCCTGGTCTGAGTGTGCTGCTCTCACTGCCTTGGTTTGGGTCCCCGCTTCTGAGCAGGATCTTCTATTGGCAGAGGGTTCAATGC
AGTGGGTGCCATGAAGGAAGATGTCTCCCCAACAGCATCACAGTCTCAGGGACTGTTCACATCACATTAAAAGCGCTGAGACTCTG
GCTTTTGGTGAAAACGGCTATTTTGTCTTGTTCCACGGGAGCTGGGAAGAGGAGACTAAACACAGGAGCCTCCCCCTAACCCAGGG
GCACATGGAGAAGGAGGAAGGCAGCCCATGCTGGGGAGCAAAAGGGAGAGAAGGCGAGGGCCAGAGCCCTGGGAAGTGTCAGATCC
TGGTGTGCCGCGCTTCTCCCAGGTTGTATAATCAGCTGTCACTTCAGCATTTCCCTGCATCAGCCTTCCCAAGTACAGATTGAACA
TTAAACAAACAGTGACGGCTTTATAAATATTTAACTCCCACTGTTTAGGAATTGCTCGGGGCAGGAGACAAAATAAGAGTTCCTGG
CTGTCTGCTCCATGCCCCCTCTGGCATGGTGTTGGAGGCCGGGGTCAGAGATTTGGGGAGTGGTGGGCAGCAGCTATTCTCTTCT
TCCTTTCCAGGGGGATGTGTTTGGGGACACATGTCTGTGTGTTCATGGAGCTGTTAGGGCCTCTTCTAGAAGCCACCAGTTTGCGT
TTTCATACAGTGGCTGGGATCCATCATTGCTGGTGTTATTCCTTCACATCAGAGCAAACTTCTTCCTTTCTTTTTTATATATAGCA
GCTTTATTGAGATATAATTCACATATAATTCACCTGAAACTCACCCATTTAAGGTATACAACACAATTTTTTTTGTTTGTTTGTTT
CTTTGAGACAGAGTCTTGCTCTGTGACCCAGGCTGGAGTGCAGTGGTGCCATCTTGGCTCACTGCAACCTCCGCCTCCCGGGTTCA
GGCTATTCTGCTTCAGCCTCCCGAGTAGCTGAGACTACAGGCACACACCACCATGTCTGGCTAACTTTTGTATTTTTTTTTTTTTT
TTTTTTGAGACAGAGTCTCACTCTGTTGCCCAGGCTGGAGTGCAGTGGCTCGATGTCGGCTCACTGTAAGCTCCGCCTCCCGTGTT
CATGCCATTCTCCTGCCTCAGCCTCCTGAGTAGCTGGGACTACAGGCGCCCACCACCACGCCCGACTAATTTTTTGTATTTTTAGT
GGAGATGGGGTTTCGCCGTGTTAGCCAGGATGGTCTTGATCTCCTGACCTCGTGATCCGCCCACCTCGGCCTCCCAAAGTGCTGGG
ATTACAGGTGTGAACCACCGCACCAGACCCCAACTTTTGTATTTTTAGTAGAGATGGGGTTTTGCCACGTTGCCCAGACTGGTCTT
GAACTCCTGGCCTTAAGCGATCCACCCACCTCGGCCTCCCTTAGTGCTGGGATTATAGGCAGGAGCCACGGTGCTCGGCCAACACA
ATTGTTCTTAATATATTCATGGAGTTGTGCAACCATCACCACAATCAATTTGAGAGCATTTTGATCACCCCAAAAAGAAATCGTTT
TTATTAGCAGTCACCTCTTTATTCCCACAATTCTCTGCGTGCTAAGAAATTGTTGAGCTACTTGTACTACAAATATACCTATCCTG
GAATGGTCTTTTGTGCCTGGCTTCTTTCACTTGGTGTAATGTTTTCAAGGTTTATCTGTGTTGTAGCATAGATCAGCATGTCATTT
CTCCTTAGGGCTGAATACTGTTCCCTAGCACAAGTATATTGCATTTTTAAAAATCTACTCATCTGTTGATGAACATTTGGGGACAG
CAAAGCTCTAGTTGAAAAATCAAGATGAAAAATCAAAAAAATATAGTTGAAAAAATCAAAAAAATCTAGTTGAAAAATCCAGCATC
ATGTGATTAAGGCCAAACTCAACTTCACAAAAGAGAGCCTCTACTATTTTGGGAAAGATCTAGAAGGTTATCTGTAAGCATATGGT
GGATGTTACAATGTGTTCAGTGGACACGCCATTCAGCTTTTATTCAGCACTTACCATATGGTGGGCACTATGGTAGGATTCATTAA
TTAATTTATTGAAAATAATTATTGAGATTATATAGTCTTGTGAAAAGATTTATCAGAGGCTGTTCTGTGAATTTTAGAGGTTTTTT

GTTTTTGTTTTTTTTTTGAAATGGAGTTTTACTTTTGTTGCCCAGGCTGGAGTGCAGTGGTGCCATCTCGGCTCACCGCAACCTCTG
CCTCCTGGGTTCAAGCGATTCTCCTGCCTCAGCCTCCCAAGTAGCTGGGACTATAGGCATACACCACCATGCCTGGCTAATTTTGT
ATTTTTAGTAGAGATGGGGTTTCACCATGTTGGCCGGGCTGCTCTCGAACTCCTGACCTCAGGTGAGCCACCCACCTCGGCTCCC
AAAGTGCTGGGATTACAGGCATGAGCCACCGTGCCCAGGTGAGTTTTAGACTTTTTTGGGGTGTAAGAATGGAGCATGAGTTTGT
GTTCTCAGCTCGTTTCATTTGATTTCAGATGTCAGCTCTCTTTAGGAGAGAGGCGTGGAGAAAAGGTGGGACTCTCTGCAGTCCTG
TTCTTGTTCTGCCCACTTCCCTCCAGTCATGGTCTCTGGCCGGCTCTCTTCTCCCCAGAGTGGTTCTTTTCCTGCCCCTGGTGTCC
CTCTAAGACCCAGATGTTTCCAGCACCCATTTGAGAGGCTGATTCAGCCCAGGCAGTGTCTGGCCTAGGGAAGGTGACTGCAGGAA
ATGTTAAAAAATGGGGGCTGTTATCATGCCTGAGCATCGCACAGTCCCTCCGTTGTGACTAATTGTAGTGGTTGTTGTTGAAGGGC
TGCCCCAGGTCCCCCTCCCTGGACCGGTACACTCACCCCAGCTGCCGAGACGGCGGCTGCTAATGTCCACTGCTGCTAATGTCCAC
AGCACCACTTCTCTGGAGATTAGCCTTCCTCCAACTGGAGCTGCTTCATCCGGGAAGTTGCTCACCCTGCACCAGCCCTGGCTGAC
TCAGGGGTGCAAAGGGCTGCTCCTCCTTGTTTCCATGTGGGTAAACTCCATGGTGCGGTTCATGTTCCAGGGCATCCTGTGGATG
GCCTGAGTTGGTCCCCAGCCGAGACCACCTCCTTCCCTTGCCTGGCTCTGCCACCTTCCCTTCCTTTCTCCTGAGGTTGCTCCCTG
TTAAATCACACAGACAGACATTCCCTCCTCAGGATCTGTCCCCGGGTGCCCACCCTCAGACACTATCCGGCAACACGTTCCCTTTC
CTGAGTCCCCTCTGATGATGTATCTGACTTGGAGATCCTTGGCCTGCTGGCTCTCTTTTCAGTTCTGTTCTGGAGAATAGAAGCTT
CCTGGGCTGGCCTGTTGAGTGTTTTGTTTGGTTGGTTTTGATTTCCAAGTCTTTTCAGGCTAATCGGACACGAGGAAAGGTCTGCA
TTGAATCACAGCCTTGGCACTGTTCCCCCCTTCCTTCCCTCCTCCGTTCATTCCCCCGAACATCCTTTTCTGTGCCGACAATGACC
TCAGCAGAAAGGGGAAAAGGACGTGGGAAGGCTGCATGGGAAACTCATGGAACTTGGTAGCAAACAGCTCTGGGTTCAAGTCCGGG
CCCTGTTGCTCAGGAGCCAGGCACTCAGTTGTGGTGGCTTCTCTAACCGGGAACTTCTTCTATGAAAATGGGACTATTCGTCTCTA
CTCCTTGCATTATTGTGGGGATCGGAAATAATCTGGGTCAATGATGTGGTGCAGTGACTAGCATCGAGGGGACACCCTCTCCACAC
AGGCTGGAAAGACATCCAGAAGGAGTCCTGGGCTCCCTCACCACGCAGGGGCTCAGCCTCGCCAGATAGTTGTCATCACCCATCCA
GGCCCGTTTCTGGTCTCATTGCTTTTGTTCATGTACTTTCCTCCTGGGTTGCTCTTCCTTTTCTCTCTGACCTGTCAAAAAGCCCC
ATTGTAAGCAACATTTAAATGCTACCTCTTCCATGAAGCCCTTTTGGATGCCTCTTAGGGGGATGAGTTCTTTCCATCCTAGCACT
GCTTGCTGCATGGTCCAGGATCTGTGGGGCCTGTCTTCTTTACCACTTTGCTCACTTCATGAATGTGGGGTGTATGAAGAGTGTG
ATGAAATTAGAGGCTATGTTCTGGAGTCAGACTGATCAGTACTGGGATTCTGGCCATACCCCCTCCTTAGCTGGGTGACTTCAGGC
AACTGACTGTACCATCCTGAAAGTCATCTGTAAGCCGTGCTGACCCCCCAACCACAGGACACTTGTGCCCGCTGCAGGTGCTCTGT
CAGCAGTGACTTCTCACTACCTGGCATCCTCTGAATTCACGAGCAGTTTGAAGAATGAGCAAATACTGAGCAAAGCTCATAGGATT
AAATACACACAGACCTGTGTAAGTTAACACCAAGCTAAGGGTTAAATACTTTACAAAAATTCTCAGTGCGGTTGGTGGGTGAGAAG
GGCTGTCGTCCCCTCTGGCTGCCTTGCCAAGCTGGTAGAGGGGCCATCTCCCACTACCTACTGACAAAGATTCTTTGCTTGACCCA
ATTACCCAATTTAATCAGACACTTAAGTCTTTTCCTAGGCCCATCTGTGTACTTCCTGTAAAATCTAGTTTTAGCAAAAAACTCTG
CTGAGTCAGTTTGGTAAGGACCACCGGCCGTCAATATCTTCCATATCTGATAGATAGGGTTCCTCATCTTCTACCACCCTCCAGAT
GATGTCTGGTCACCCTGGCCTGCCTTTAGCAAGAATCTTGATAGGTTGATTTAACCAGAATCCCCCTTACTCCTGATGTTGCCTGT
TAGTAATTTTCTATCCTCTGACCCCCCACCCTGCTTCTTGGCTGTGAATTTCCACTTGCTCAGGCTGTATTGAGTTGAGCCCTATC
TTTCTCCTACACTGCAAATTCCCATTGCCGTGGTCCCTATACCTATCAGGATGGTCCTGAATTGTCTTCTTTATCATCTGCTCTGC
TCTGCCAGAGCTTAAGCCCAGGCAGCTGAGTTAAAAGTGTGACCAAATGGACCATCTTGTCCCTGAAAATTGGGACCTAAGCTCGA
TTCCGAGATTTAATCATGGCAGAGGAGTTTACTCTGCTAACCACCATCACCCCACCCTATCCCCCAGAGGGGTGCGGTGAGTAAGG
GGGTGGCAACTGGTTGTTTCCAATCTCCCATCTCTCTCTGGCCCTCCTCTGTCTCTGGCCTCCCTTCTCCCTTTGAAAGGGACACA
GTGCTGCTCACAGCCTGTCCTGCTGCGGTGGCTGCAGAGAGGGGCTGAGAATAAGCCTGTGTCCGAGGCAGCCCTCCAAATACAGC
AGGGCTGGGTTGACCTTTAAATAGATGGGCTCTGGGCCACTCAGTTCCTGGAAGTTTGAAGAAAGTCAGCCTTTCAAGTGATGCGA
CTTCCAACCTCAGGAAAGATCGCTGTGTTTGTAAAGGGTATAATTTACTGTTGCTTTTGCACTTCATGATATGCGGCTGCACAGCC
AGTGGGAGCCAGCATGCCAGAATTAGGAGGGGAAAATCCTCAAGTATTTTAATATACCCCTACTTAGAAGCAAGATAAACATATGT
ATTGTACTTTGGGACAGAAGCAAGGGGGGTTTAGCGGGTGGGAAAGGGAGAGTCCTGGCATTGCATGAAAGGTCTTTTTAAGCTGA
ACTCTGAGGGTTTTCAGTTTTTCATTTTTCTGTTTTTTTTTTTTTTTTATTTGTAAGCAAAGCTGGCATGAGCAAATGATGCCAGG
ATCTACTTGGCTGTGTAAAAGCTGTAGTCCCTCCTCAGACAGGCAGACTGCTAGAACCCTCCCTAAGCCATAAACTAGTACACCGT
TTTGCCTCGCCCAATTAAAAATGATTGGGTGTGGGTTGTCATCACAGTTGACTTAGATGATTTACTTTCTTCCAGGAAATCTTCCT
CCCAACTCTTGCTTTGCAATTAGGCAAAAGAAAAATAAATGTTAATATGTTCTGTGCTCCCAGGGAAAATTTTTTTAAAAAGAAAA
TAAAACACTTTGGGAGGCCGAGGCAGGCGGATCACGAGGTCAGGAGATCGAGACCATCCTGGCTAACACGGTGAAACCCCGTCTCT
ACTGAAAATACAAAAAAAATTAGCCGGGCATGGTGGCGGGCGCCTGTAGTCCCAGCTACTCGGGAGGCTGAGGCAGGAGAATGGCG
TGAACCCGGGAGGCGGAGCTTGCAGTGAGCCGAGATCGCACCACTGGACTCCAGCCTGGGTGACAGAGCGAGACTCCGTCTCAAAA
AAAAAAAAGAAGTGTCAAGGTCATGAGTGTCAGGGAAAGACTGAGAAATGGTTCCAGACTGAAAGAGACTAAAGAGATGTGAGAAT
TAAATGCAAAGCTTGATTCTGGACAATTGTCAAACCTTGGATGGCTGGGAGCGTTAGATGTTAGTTACGTATCTGTGTCAGCTTCC
TGATCTTCCTATAGAACATGGTCCTAAGGGAGAATGTCTTTGCTTTAGAAAAATACACATGGAAGTACTCTGTAGTGATAGGGCTT
CAGGTCCACTACTCCCTCTCACATGCTGTAGGGGAAAAGGATTTGCTGTGTGTTCCCAACACGTTTGTGATTATTTCACAAATAAC
AATAATTAAAAAAAACCAACCTCTGGTGGGGCGGTGCGGAATTATTATTATTTTTTTTTGGGACAGAGTCTCACTCTGTTGCTCAG
GTTGGAGGGCAGTGGCATGATCTTAGCTCACTACAACCCTCAACTCCTGAGTTCAAGCGATTCTTGTGCCTCAGCCTCCCAATTAG
CTGGGATTACAGGCATGCGCCACCAGGTCTGGCTAATTTTTTTGCATTTTTAGTAGAGACGAAGTTTTGCCATGTTGGCCAGGCTG
GTCTCAAACTCCTGACCTCAGGTGATCCAACCACCTCGGCCTCCCAAAGTGCTGAGATTAGAGGCGTGAGTCATCATGCCTGGCAG
TAATCTTTTCAGTGGAGAATAAAATAAACCCGGGTTTGAATTCTGACCCTTTAAATTTACTCAGGGTTTGACTTCAGCCAGGGAAC
ATAACCTTTCTGTGCCTCAGTTTCCTCATTTTTCAAAACAAAGATGGTGTGTGTGTGCATCTCCATCTATGAGTTGTTGTGAATAG
GAATGTAGGGGCCCCAGCAGGGTGTGTAAAGGTACCAGTTCCTGGGGCGTCAAAGGTTTTTCTTTCCCTTTGTACCCCCTCACTGA
CATGCCCAGGTCTGTCTGTGTAGAGCAGTGGGTGGATTTACAGACTCCGCTGAGAACCCCCTTATAGCATATTTTCAGGTTCGTGA
TGATATCATGGGCTGTGACCCTGGGAGTTTAATTGGAATGAATTTCAAGGTTTTGTTAAACTTGGCTAGAATCTTCCTATATCTCC
AGCAAGATATTGATGGGGTTTTTGTTGTTGCTGTTGTTTTTGAGACAGGGTCTTGCTCTGTCACCCAGGCTGGAGTTCAGTGGTGC
CGTCACTGCAGCCTCAACCTCCCAGGCTCAAGGATCCTCCCCTTCAGCGTCCCTAGTAGCTGGGACTACAGGTGCGCTCCACCAC
ACCCAGCTAGTTTTTGAATTTTTTTGTAGTGATGGGGTTTCACTATGTTGCCCACGCTGGTCTCAAACTCCTGGGCTCAAGCCATC
TGCCTACCTTAGCCTCATAAAGTGCTGGAATTGCAGGTGTGAGCCACTGCACCTGGCCAAGAACTTGGTGTTTCTTATTTCCAAGC
TTATCTCCTCTGTTATTTGCTCCCAGGGAGACCCCTTTTCCATTTATGTTTCCAGGGTGCTGGGGGGAAGTGATTGCCCTCATCTC
TTTTGCCCAGCCACCAAACCCAGTGCAGACTCTGCTGAATGCAGGCCCCATCTCTGTATTTTACTTATTCCATTTCCTGTCTTGAA
GGTGGGGGCACTATGTTTATCTCTAGGACCCTGACTGGCTCATCATTGAAAGCTTATTTTGTGCAAAGCATTGTTCTGAGCACTGT
GTGTGTATGATTCTACTTCATCCTCTGTAACCCTAGCAGTCCGGTCAAATAGTATCATTTTATAGACAAGGCAACTGAGGCACAGA
GCAGCTCAGCAGCTTGCCCAGATCACACAGCTGGAAAGTGGTAGAGCTGGAATCTGGATGGGTGCTGGCAGCCTGGCCTCAGAGTC
GGTGCTAACCACTGAGCGGCACTGCCACCCAACGGATGGGAGCCTGCAGGATGAGGCCATGGGAATTGATTTCACTCAGCGCGAGC
CAAGCAGAACTCCCGTCCAGACCTCTGTCCACCACCCTAATAAAGCCAGTGTTTACACAGGCTGGAGGTGCTCATCAGATGTTTTT
GCCAATCCCCAAATACGGCCAGGGCCAGAAAGTGAGAGGGGTGCCTATTATGTAAGGCCCTGCGGCTGGTCCCCAGACAATGCCAC
TTCCTCGCTTGGCGCTGTGTGACTGCATAACAACGGGTTACTCATTTCCTCTGGAAAACTTCACAGGGCCTCTTTGCCCCATTTCC
TCCTCCCACCCCATCCTCAGGCTTCCTCTGTTGAATTTTCTCCAGATTCCTGGGAACACCACCATGGAGGGCCTGGCCTTTGGCGG
TGGACAGTCTGTGGCTGGGTGGGAACGTCAGGAGTGGGGCCTGCCTCTTTGCTGGGAATGCCGTGCACTCCCAGCCCACTTGCCCC

ATGCCAGCACTCGAGGCAGGTGAGGCCCAGCCATGGGTATAAGGTGCTCCCAGGGCGGGATTAGCCTCAAGGAGGCAGGGGTTCCA
TGTTTCTGCTCCTCTCCCATTACTAACGGGTGTCTGCTTTAGGTTTGATCTCCCTCCCTTCTGCCCCTCTCCTGGTAAAGGGATGA
ATAATGAAGGCCTTCCTTGGGAAGTCTGAGATGGATTACCCCAAGGAGGCTCGAACATGGGAGGACATGCGAGAACTATTGTCACA
TGAGTTTTCTTTCTATCCTGTCTGCAGGACTTCGCAGGTGAGCAGTAACGGAAATCAGCCCCGTTTATTCCTCCCAGCACCTGCCT
TATCCAACTCCCCACGCTGTGGCTGAGTCCCAGCCTGCTATGGAAGCATCACTGGACTCCCATTGAACTCTGTGCAGATTCGCTGT
TCGTAGACATGGTACCTGATGGACACCAAGCTACGTACAGCTTCAAGGCCCCTTCTTTCTTTGCTAATAAAAAATAAAAAATAAAA
ACATAATACACGTTATATTTCTATCTTATAATACACATTTCTTATTTTTATCTCAATAAGCTGATAGATATATAGTAGATATACAC
ATCTCTGACTGTACATACATACACAAACACACACACACGTACATGCATGTATATGCGTATCTATCTCCAATGCATGTGGAGTGTTG
TGCCAGGTCATCCTCACATTTAATTTTTCTGTTCTATTTATCATTCTTAAGGATTGTCAGGCATTAGGGATTTATCTATAGCAATT
TGGATCTTAGCAGTTCTTTTCATGGGTTATCTTATTTTTTCCTTCCAACAACCTTGGAGGCTATAAAGGGCATATTATTGCCCTT
TTATAGATGGGAATTAATTTATTAGCAAACTCAGTTTCATGATTTTTTTTTTCTTTTGAGATGGAGTTTCGCTCTTGTTGCCCAAG
CTGGAGTGCAATGGCACGATCTCGGCTCACTGCAACCTCTGCCTCCCGGGTTCAAGCGATTCTCCTGCCTCAGCCACTGGAGTAGC
TGGGATTACAGGCATGCGCCACCATATCTGGCTAATTTTATATGTTTAGTAGAGATGGGGTTTCTCCATGTTGGTCAGGCTGGTCT
CGAACTCCCAACCTCAGGTGATCCACCTGCCTTGGCCTCCCAAAGTGCTGGGGTTACAGGCGCGAGCCATTGTGCCTGGCCAGTAT
CATGGATATTTTTAAAGTGCTTGTTGTGTGTTGGGCACTACTATGTCCTGCATTTAGAAATAGAACTCATTCCTATTCTCTGCTTT
CCCATGGAGAGAGGCACAAACCTGCGGGTATAGGTGGGTATAGTTTCTTAATGTGCACACCATCGTAAAGTGGAGACCCTGTGCGT
GAGATGTGTGCTGTGTACAGGGTGGGCAGGAGAGAGACTGGTGAGGGGATGATGCCCTTATGCTGGAAGGGGCAAGATGAAGCCTC
TCTGGGTTTGGTGGACCTTGGAGACATCTGCCTAGTGTGTGGTCACCCAGAGGCCAAGGATGTGCTGTTTCTACTTGCTTGCTGC
TTTCTCTGGGAGTGGCCATTTCTTCCAAGGCAGTGTAAGAGCCTGGGCATAGGAGCCAGGCAGAACTGTGTGGGGATGTTGCCTCC
ATAGCTTTCTAGATGAGCAACCTTAGGAAAGTTTGAACAGTTTCTCTACATCCTGGGTTCTTCATCCATGAAATGGGAATTATTGT
TTGAGCCTCTAAAGTGAGACAGTCAAATGAGATGGGCATTTGAAGCCTTCAGCAGGGTTCCAGCCTCACAGCTAACTCTCAACTAG
CTGTGATGGTTACAGAGCTGTAGGTTGCTCATAAGTCAACATTGTAGTTGCTGGAGCCATCAACTCAGCTATAGGATACAACACTG
AGCCCTTTTCACTATCCTTTGATGGGTCTCCTTTTCCCTAGTATGGGCCATCGGCTAAGGTGATTGAAAAAGGGCCTTTCATGTTT
TCAAAGGGGCAAAGAAACTTCAGTAGTTGGTTGGGCATTTTATTGAGTAGAGGTGACTCCATGCAGTTCTTTTTGGGGGAAGGATG
CTGCACATTCCAGGGTTTAACAAAAATTTATTGAATGCTTACTTTGCCCCAGATACCTGTCTTGGTAAAGGTGATAAATAAGACAG
CCTCATCCTCATCTTCATCAAGCCACCAGTCTGGTGGTGGTGACTGGGGTGGGGACTGCTGAGGCATTGGGCCCCTTGAAGCCTTC
CCTCTGGGTGTCTTCCTCCCCAGGATGGTCTGGCTCTCTCGGGATGGGAGGAAGTTGGTCTGAAAGTGCCTCAGTGATCGCTGAA
ATAGGTTTGCCAAAGATAGAGGGGAAAGAGATGGCCAGCCTTTGTTTAGGGACTCCCAGAATGGTCTTAGGTCAGGAAAGAGCGAA
TGGGTACATTTCTCCCAATTTCTGGGCTGGAGATTCCAAACTGACTCCTTCATGGCAGGCTTCCTTATCTCTGCAACATTTGTTTA
CATGTTCCCTTCATAGATTTCTAACAGGATGAAGACTTTGTTCCTTGGGGTAAGGGTCTACCTCCAGGTGTGTGGGGAAAAGTTGA
GAAGCTGCTAAATTCTTGGCTTTGACTTATCAATGGTTTTTCGGAGGGCCTGGAGCTGGGCTGGGACTGATCAAATTTGGTAATTG
CTTAGGCAGCCTCCACTTTTGTTGCTCCTGGGGCCAGATGGCGTTGGGCTAGAAACAATATCGGGGAGTTCTGTTTGGTTTTAACT
GCTGTACCTGGGTGCTCTGTTATCCAACCAGGCCCTCTCCTAAGTTCTCTTCTTTTTTTTTGAGACAGAGTTTCACTCTTGTTGCC
CAGGCTGGAGTGTAATGGCGCTATCTCACAGCAACCTCTGCCTCCCAGGTTCAAGCCATTCTCCTGCCTCAGCCGCCGTAGTAGCT
GGGATTACAGGCATGCACCACCACACCCAGCTAATTTTGTATTTTTAGTAGAGACGGGGTTTCTCCATGTTGGTCAGGCTGGTCTC
GAGCTCCGGACCTCAGGTGATCTGCCCGCCTCGGCCTCCCAAAGTGCTGGGATTACAGGCGTGAGCCCTGTAATCCCGGTGCCCAG
CCAGTTCTCTTCTTTTTTTAAGCCTCTTCCTTTAGAAGGAAGAAAACATAATCTATATGCTGGCTGGCTCTACACAGCAGCAGGCA
TCACAAGGGGTTTTTGTCTGCTTTTGCTTCTGAAGGTGAACCCACTATCTATTTAGAGAGGCCAGGTCAGGGTGTGTGTGTGTGTA
TGTGTCTTTTTCCCTAGGGTGGGTATTTTCATTCANNNNNNNNNNNNNNNNNNNNNNNTGTTGTTATGATCCCCTGTTGTAGAAGAAGC
AGTGAAGTTTTAGAGAGATGAAGTGCTTTGTCTGATGTCACACAACTGATAGATGACAGAATCACATATGTATTCAGGGGTCTGGT
CCAAAGCCTAGAAGAAGGTGAGCCATGCATGGCCCGATGGCACAGAGCATGAGCTCTGGTTGCACAATCGTGGGAGGTTACTTAAT
CTTGAGAAACCTCAGTTTTCTTATCTGTAAAATGGGACAGTATGTATCCCAAAGGGCCATTTTGACCATTTATCAAAGTCAGTTTC
TCCTCCGTATGGAGCGGAGAACAGTGTCCAGCATGCAGTACGTGCTCTGATGATCTTGGCTGTAATGACCATCCTGGTTAGGGTGG
ATTTGGCCAAAAGCTGGAGTAACTGGTAGCTTCCTGATCTGAGAGGTGTCACTGGGTGGTGTGGCCCAAAGATTGAGAATCTTGTC
AGGGGTGGCCACGTGAGGGTGGAAGTGGTTTGAGTGAGGAATGGTCTTGAGGTAAGAGTTGGACAAACAGTGTCTTCTCTCTCTCT
CTCTTTTTTTTTTTTTTTTTTTTTTTTTACTTTTTTTTGAGATGGAGTTTTGCTCTTTTTGCCCAGGCCAGAGTGCAATGGCGCGAT
CTTGGCTCACCACAACCTCCGCGTCCCAGGTTCAAGTGATTCTCTTGCCTCAGCCTCCCGAGTAGCTGGGATTACAGACATGCACC
ACCCACGCCCGGCTAATTTTGTATTTTTAGGAGAGATGGGGTTTCTCCATGTTGGTCGGGCTGGTCTCCAACTCCCGACCTCAGGT
GTTCTGCCTGCCTTGGCCTCCCAAAGTGCTGGGATTATAGGCGTGAGCCACCCCACCTGGTCGACAGTGTCTTCTCTAAGCAGAAC
TGTAGCCCTGAAGCGCCCAGGCTAGAACTCTCTATTGAATGAGGATCACTCCATTGTGCCAGCTGCTCTCATATTGCTTTGATGGC
CTTTCTCAGAAAGGAGAAAGGTGGTGCTGGGTGTGGCTTTGGCTTCCTATGGCCGCCCCAATGCTGGTCTGACACTTTCTGAATCT
GGTTTGTGGTTGGATCTGTGCATCCTACCGCTCCACTCCATGTCCTGGCCTTGGAGAAATCATTTCCCTCCCGCCGTGATTGTTCA
CCTTCAAAGGTCAACAAAGGCAGTAGGCACTGCTGAGCTCTTGCAGCCTTCAGGGCTGAGCTGCTTCCTCTCCTGCTTCCAGTGAG
GGGTTGCTTGAGGGTGCCTTTAGGAAGGAACCTCACCCCTAATGTAACTTGAGCAGGTCTTTGCTCTAAACTCTTCGCTGGGAACA
TACCCTCCCGTGGGACTTGGCATGGCACCCACCTCTGCAGAGCCACCTGTGCCACAGAATAACCTCTTCTCCCTAGACACACTGTG
TGTCCTTGGTTGGATTCTCTAGATGGGAGGGACCGGCTGAAGCATAACTGTGTATGTTCACATATGTGCCTTGGAAGAGTTTTATG
CAGGAAAGGATTTGTAAATCAGAAAAATGAATGCTGGTATCTAATTTCCTGTCCCCGTTTTAAGTCCAGACCAAGTCACCTAACCC
CTCGGGCTTGCAGCCCGCATCATCCACTGGGTGATTACCTTGAGTTATCTGGTTCAGTCCCTTTGCTGTGGGCACCCTCTTTACCT
CTACAAGTAAACTGCAATTAATCTGACAGCAGGGGCAAGGGAACTGACGAGTCTTGGGTGCAAATCTTGAGATGCTTTGGCACATT
ATCTAATTCATTGTTTTTCTTTTCTTTTTTCTTTTCTTTTCTTTTTTTTTTTAGACAGGCTGGAGTGCAGTGGCACGATCTTGGCTC
ACTGCAACCTCTGCCTCCTGGGTTCAAGTGATTTTTGTGCCTCTAGCCTCCTGAGTAGCTGGGATTCCAGGTGTGTGCCATCACGC
CCAGCTAATTTTTGTATTTTTAGTAGAGACAGGGTTTTGCCATGTAGGTCAGGCTAGTCTAGAAGTCTGGCCTTAAGTGATCCACC
CTCCTTGGGCAGGGTGCCACCTGCCTGAAAGAAAATGCCCACCCTAGGGAAAAAGACACACACACACACACACACACACACACACA
CAGAGAGAGAGAGAGAGAGAGAGAGAGAGAGACCAACGTGGCCTCTCTAAATAGGTAGTGGGTTAACCTTCAGAAGCAAAGGCAGA
CAAAAACCACTTGTGATACCTGCTGCTGCGTAGAGCCAGCCAGCATATAGATTATGCTTTCTTCCTTCTGAAGGAAGACTCTAGGA
TTACAGGAGTGAGCCTGGCCCCAATTCAATTCAATCTTTATGGGCATCGAACAAGGGCATTATTTATTACCCATGGTTTACAGAAG
TCAAACCCAAGTCTCAGAGAGGGAAGACATTTTTTAGTCTCACTGCGAAAGTGACAGAGCTAGAATTTAGTCTCTGTCACGCTAAA
TCGTGCTACATGCTCCGTCTAGTTCAGAGCCTGTGTGGAGGTCGACACTGGAGTCCCTCAGTTTTCCTCATCAGGCTCCTGGTCTG
ATTCTTCCAAGATCAGTCTCTTTCCCTCAGCGGAAGCCAGCTGGGAATCACTGGCCTTGTAACCTCTTCAGGCGACCTCCTTGGGA
TGTTCACAAGGAGCATAGACATCTGGAGAATGATACTGTTTTTTCCTAGGGCTGGGATGGGGGTGGTATCCCTCACATCATTCCAG
AAACAGCGCCAGCCCTGCTTTTATCAGCGGGGGCCACAAAGAGGCGACAAGCCCTGATCAAATGTGAGCGAGATTGACAATCTCAG
CACTTTCCAGGGAAAGGATGCCGTCCCACATGGCTTCAGGCCTCCAAGGAAGCCCCATCCTGAGACAGAGAGACCCCAAATCACAG
TGTGTTTCCTTTTGGCCAGAGATGAGGCTTACTCATCTCAGACAGCTGTGTCCACGGCATGTTCCTCAAGGACAGCTGCTCCAGAT
GAAAAGAAGAGATGCTCCCAGAGCCTTGAGGGGCTGCTGATAGGAACCCAAGGCCTCACCCTGGGGCTGTATCCTGTGGCTTCAAA
AATATATGTATTTGAAAGCTTCTAGCAAAACAGATTACACAAGAACGCAAATGTAAGATCCCTTTGGGATTCTAACTTTTTTTCTT
AAGGTGTTTAAAGAAAAATGACACCCTTAAAAAGAAATGCCATTTGTTTTCAAAGCTGCAGCACCAGAGGACATGGAAGAGAAAGG

CTGAAGTGTCCCTGAAAATGAGTGAAGATTCCAGTCTGTTTTTACTAATGTCCAAAATCTTAGGTTTATTGCCTTTGCGTTCCTCT
GCCCCAGGGTCTCTGTGTGGCAGGCGAGTGGGTCTCGTTCGATGGTGCAGTCATCGTGTGGCGGAATGCATGCTCACACAGGGAAG
TTGGTTGAGCGTCTCCTTTGAGCAGTCCTTCCTCTGGTGAGGTGCACAGGCGGGCATCATGCCCATTTCATAGATGAGGAAACTGA
GGCTGAGAGGGATGCTGCAATTTAGGACTTCTGGGTGATATCACAGAAGCCTCATTCTCTTTTGTGGCATGTACCTTCTCTATTTG
GGAAGTTAAATTGGACTATGGGGATTGGACCAAGGGGTCTTCTTGCTTCCGCTTTCTCTAGGCCAAGGCTTTGCAGTTATGGAGGA
AGAAAGAGCTGGGGTCTGTCTTGGCAACCCTGTGACTCTTCTCCCCTATCCTCACCTGCCTTCTCACTTGACCAACATATTTTAAG
GGCTCCATGTACAAAGGTAGGGCTGTGGCCCTTTCCCAACCAGCACCTTGGCCCTGTACCCTGATATGGGATGGTTAAGGCACACA
GGCTTCATGGATGGGGGCCTACGACCGCAGATGGAGCAGAGGTGGACAGGGTATGGTCCGGCAGGTGGAGGTGCTGGAGAAAAGCC
GCGTTAGGCAGCACGGGAGCCAGGTGCGGGGTCTCTCAAAGCCTCGAGTCCCTGGCAGTGCTGTGCTCTGTGAGGACTGCTTTTGA
CTGCAGGGCACTGACACTCATACCTTTTGCTTCTGCAGCTGACCATACTCCCTGGAGCCTTCTCCCGAGGTGCGCGGGTGACCTTG
GCACATACAGCCATCATGATGGTACTTTAAGTGGAGGCTGAATCATCTCCCCTTTGAGCTGCTTGGCACGTGGCTCCCTTGGTGTT
CCCCTTTTACTGCCAGGACACTGAGATTTGGAGAGGTAAGTGGCTTACCTGAGGCCATGTGCTAACAGAGAAGATGAAGAGATGAT
TGAAACAGGCCTAAGACCAGACCTAAGGGTCTGTACATTTTCCACATACTTTCCATATCTTTAGAGGCCTGACCAAAGCAGATCTT
TTCCTTTCTTCTAGGTAAGTCCAAAGGCACCTGCCTGCTGGGCCCACTGTTTTCTAACTTTCCTAACTTTCTGATCCCTTGGAGGT
GATAATCAAATATTCTAGTCTGAGGCATTGGGATACATGGTGCTAGGTTCTGAGACTCTGCGTCAGGCCTGAACCCTGCATTTTGT
GGAGGTGGGTGGAGAATGTTCCCCTGGGGAACATGCCTAGACACGGGGGACAACAGTTGCCCTCATGGGGAGGTACCTGTTTACT
CGCTGTTATGGGACCGCTTTCACAAAACCACTGCAGGTGAGTGAGTTCCTGCTGAATATCAGGCCTGGTGTCTCTAGACTCATTAT
TTCCCCCACCCAACCCCTATGTTAGTTCATCTCGAGCCACATTTTTATTGCCATAATCCAGGCCTGGACAGGCCAAGATCTTTTAA
CAATTTTAATTACTGAAAATAATAACTGCATTTTTTTTAAAGCCCAACTTTTGGTAGAGTCAGCCCAAAATACAGTCTTTGTGTTG
CCATCTGGGAACTGGATTTGGAATTGTTCTTCCATGAGACTGCAGAGCAGAACGGCAGGGCCAGAGGTCCCACGAGCTGGTCAGAC
CCGGTTCTGCTCCTTGCTGGCTGAGTGACCTTGGGCATTGTGATTCATATTCTCATCTGCCTTCAAGTGTGGTTACGAATGCTTTT
TATCCTGCTTATCAATTTCTATGATTGCCATAAAATATTAACACAAATTTAGCATTTATATAACATAATATATTAATGCATAGTTT
TTTTGTGGGGGGGGGCGGTGGAGGGGCAGTGTTGAGACAGAGTCTTGCTCTGTCACCCAGGCTGGAGTGCATGGTGTGATCCTGGC
CCACTGAACCTTGACCTCCAGGGCTCAAGGGGTCCTCCCACCTCAGCCTCCAGAGTAGCTGGGACTTCAGGCATGAGCTATCACAC
CTGGCTAATTTTTTGTATTTTTTGTAGAGACGAGGTCTCACTATCTTGCCCAGGCAGGCCTGGAACTCCTGGACTCAAGCGATCCT
CCCACCTCGGCCTTCTAAAGTGCTGTGATTACAGGTGTGAGCCACTATGTCCGGCTCACGTCCAGTTATCTAGGGCAGAGGTCAGA
CATGGATCTCTCCAGGCTAACATCAAGGAACCTGTAGGCTGCCTTCCATTCTGGAGACTGCAGGGGAGAGTCTGTTCCTTGCTCAT
TCTGGATGTTGGCAGAATTTGTTTCCTTGAGGTTGTAGGGCTGAGATCCCGTTTTCTTGCTGGCTGTCCAGATGCATTGAGCTTCT
GGAAGCCTCCTCGCTAGGCTTGTGGCCTCCTCTCTCCATTTTCAAGTCCAGCAATGGTGGGTCCAGTTCCTTTTAGGATTTAAATC
TTTTCTCCCTCTTTCTTTTCACCTCTGTGATCTCAGCTAGAAAAGATTATCTAATTTTAAGGATTCATGTGATTGGACTGGGGCCA
CCCTGATAATCCAGAATAATCCCTACTTCAAGGTCCTTAGGTTTAATCACATCTAGGAAGTTCTATTGCCATGGAGGCAACATAT
TCACAGGTTCCAGGAAATTCCGACATGGGCATCTCTTTGATTGGAATGAGGGGTGGCAGGTATTCTGACACAGTACCTCAGAAGGG
TTGCTGTGATGATGAAGTGAATGGAGACACGTAAAGCGCTTAAAATATTATGTAGAGCCATGTGAATTTGGCAGCATTGAATTGTT
GACCTATAAAAGGTCAACTTAATAGTATCTGGTCTATAGTAAATCTTCAGTAAATGTTTGCTGTCATTACTCTAGTGGTAGAAATG
TCATGCTTTATTTAGCCAGTTTGCTATTGTTGGGCATTACTTTTTAAAGAGTTATTATTTTGTTATTATAGTATATATGTGAACAC
CGTGATCATCATGCCTTTGCAAATTTTATTCTGATTTAGGGCAATGCATGTCTTTCCAGGCCACACTCAGTCTCCTGCTCTGGTGC
CAGTGTCTCTGTGGCCTGAGGGGCCCCTCTTTCTTGCAGAGCCCCTCATCCAGCACCAGCTCAGCTCTGGGCCATGTGTGTGGGGT
GGGACTTTTCTCTTTTGGGAAGTGGGCCATGCCTCTCCTTCCTGAGACCATCCCCTTTGGCTTCCTCGGGGCCCTTGGCTCTTTGA
ACCAGCCTGCAGGGCACCACACCTGTGGGCCCATTTTTGTCAGGAAACGACTCCCTTCCCAAGGGCACCCTGAGTCCCAGGTCCCC
AGGCCCACAGATGTTGGGCCCTCAGAGTGTGATCCCATGTCCCCAGCTCTGTAACTCCCTGCATGTGGCTCTTAAGACTCACAAGC
CCATGTTTGCCTTGGCCAGACACTTTCGCCTGTCAGTTCTGCCAGTTGGGAAGTGTCATCCCCACTGTGTGAATGTGGAAGGGATA
ATGAAGAATTTCCACGGCTTGCCTAGGGTCGTTGAGCAATGTGGAAGGGATCATGGAGAATTTCCATGGCTTGCCTAAGGTCATCA
AGCTGGCATCTCAACCCAGGTTCATCTGCCTGTGATGACAGGAAAGAAATCAGGTGTGATTCTGTTCTTGTTGACCCATAGCCACC
TCTCTCATTAAGGCTTCTCCTGCAGGCAACTACACAGCCAGTGGTGGGAAAGCACCTAATTTACATCTTCATGCATTGAAACTGAT
GTCACTCACCTCTCCCCCTTGACCTGGAAGAGCCCCTGAAGGCTGCCATAGGGGAAATGATAACCCATTCTACAGCTGCTGAAACT
GAGGCCCAGGGAGTGTCCAGAGCTTTCTGAGGTCCACCATGTACTGTTGACCAGGATGAGTGATAGCCACCTGGTGACAATGTTGA
AGGTTCTCATTCAGTAGAGCTGGGTTCAATTCTGACTTGCCTGACCAGCTGGTGCCTCATTTTGCTCTAAGTATTGGAGCCTCCAG
CTTTATTTTCTTTCAGTGGGAAAGACTGTGAGATTCAGTCCCTCCTTGAAGCTGCGCATCGACTCTCCTGTCCTGGGTACTCTTTA
GGTAGTGGCTGTTCTGAAGTGGCTTTGGAAACCCTGGAATGAAACTGTCATGTCCAATGCTGAGATTTGTATAAACTGTCATTTTA
ACTGTGATTATTATTCCTTAGGATTTGAGAAATAAAATCTGAGGAGGGAGCAGGACCTGCCTCAGGGCCTCCTCTCCAAGTTGAGT
GACCCATCTTCCCAGGTCTCCTTTTGTTCTGTCTTGAGCCTCTCTCCTGAGGTCCTGAAAGAAGAGAATGTGCTGTTGTAGGGGGC
CTGGAAATGTCCCATGGACCATCTCGTGACTCAGTAAGATGATGTGTGTCCTCTGGGGACAGAAGGAGGTCCTGCACAGAGTGTTC
CTGCCCGTTGACGCCCTCCCCAGATCCTAACGCGAAGTCTGACACATAGTAGCCTCCCGGTAATCTTAATAAGCAAAAGGACACAT
TTCTCAAGTGATCTTAGCACCATTTCATGCAATCAATTCCAGCCCAGCCCTTTATTCTGGCCAGCCCTGGTGGGTCCATTAGCTGC
AAAAATTAGTCGTGCTCAGTTTTAATGCAAATGACTAAGTATTACTTTATTGAATTGATTAATAAAGAAGCTCTCTTTGTATGATA
TCAAGACCATGAAGAAATTGCAAAGACATAGGTAGGGTCTACAGCAGAGTTTCTCAGCCTTGGCGCTGTTCATATTTTGGGTCAGA
TGATTTGTTGTGGGGGCTGTCCTATGTATTGTAGGATGTTGCTCGGCATCTCTGGCCTCTACCCACTGGATGCCAGTAGCTCTGTC
AGTCGCAACCCCAGCTTCCAGTTGGGACAACCTAAAGTGTTTCCAGACATTGCCACATGTTCTTTTGTGGTGGTGTTGGTGGTGGT
GCGTACATATGTGTGTGTAAAAATCTCCCTTTGTTGAGAACCACTAATCTAGAGACCACCTAATTAGGGCTGACGTGGGCTCTTCC
ACAGAGCCTTCTCCTTAAAGACAGATAGGTTGATGGACAGTTGGATTTAGGGAAAAGAAAACTGAGTTGAGGGTTTAAAGTGAATT
TCATCCCCAATGTGGAAGATGAGCGTTCCGTTCCTGGCTGTAGGATGTGGAGCATGTTACTCCACATGTGGGAGAAAAAAATCATC
CCATCTACTTCAGGACTTTTGTGTGATTAAAGAGGCAAGATAGTCTAGTGCTTTTCATTCCATGCGCACTCAATAAAAAATAGGGG
TTATATTTATGAATAGTAACAAAAAGCCTCTTAAGGAAAATTATTTTGGCCCTTGTTCAAGGTTCAGTTGACCATTTATTTTTAA
AGAGATGGGGGTGGCCCAGGCTGGAGTAGAGTGGTGCCATCCTAGTTCACTGCATCCTCAAACTCCTGGGCTCAATCGATCCTCCT
GCTTCAACATCCCAAAGTGCTAGTATTATAGGCATGAGCCATTACACCTGGCCCATTTGACTATTTTTTCTGCTTGATTGTAGAGA
AGGGAATGTAAGTCATGAAGATCTAAACTATAAAACCTGCGTGGCTTAGAGGCTGAGCATGGATTGTGATTTAAGGAAGCCCCAGG
TTCAATTTCTGGTTCTGCCACTTGCTAGCCATGGTGAGACCTTGCCCAGCTTTCATACTTTCAGGTTCTTTGTCTGTAAAATGGGA
ACAATAAGAGTACTGACCTCACCGGGCTGTTGTGCTACTGGCTGTGCTGCCTGAATGGGCCCTCCTTTATTTGAATCCCTCTGCCA
CAGTTGTCCCATTTTTTTTTTTTTTTAAATGGTGACCCAAGTTCCATTATGGACGATCAGGCATTCAGGGGAAGGAGGGATCCAAA
GCAGAGACAAAAGCAGCCAGCCAGCTCATCCCCTCTGCAGCTGATACCGAGGAGGAGGATGCCAGTGGGAAGAAGCGTGGCCATAT
GGCTGGCAGGATGCCTCACTGGTAACAGTCAGCCCGGCCCCTTTGAAGTGGTTTTAAGACCAATTTGGTAACTTGATTTGGAAAGT
GGTATCGATGACTCCGAGGGCGCAGGCTGCTCATTAGCTGCTGTCTGGGCCCGGCAAATTGGTTTAAGTGGAATGAAATTGGTTCC
TTCCCTCCCCGTGCTCATGGCTGAGGTCCAGGCCCGATTGTAATGGGAACCTCTCCTGGGCAAAGGACATAGAAAAGCTTTCCGGG
GAGTCTGACCCTCTTTTTTCATATGTGAATTCTACTTTCCCCTGGGGGTCAAGGCAATAGGCTGATAAAACATAAAAATGAACCTG
AAGTGGGTGGGGTGAGTGTGTGTGTGGGAAGTTGGATCAGAGGAGAATGAACAATGAACAGTGGCCGAGTGTTTTTCAAGGGCCAG
TTAACCATTCCCCTGTTGAGACACTGTGCTTGATGCATTTTTTTTCTTTTCTTTCTTTCTTTTTTATTCTCCCATATTACAAACAC

TGCTGGGAGGAACATCCGCACATGCTTTCTTGGTCACTTATGTGAGAGCTCATCCAGGATTTATACCCTGAGGCGACTTCACTGCC
GTGGAGGCTAAGGAACATTTTCTGTTTCATAAGACGCGATCACATTGCCCTCCAGAACAATCATGCCAGTTTACACTCCCACTGAC
TGTACACAAGAGCCCTGTTTTCCCCCATCTGGTCACCCACATTTGATAGGTCAGAATTTTGCTTTTTGCCAGTCTCATGGGGGTGA
TGCCATTTCTGGTAGTTCCCCTGATTGCCAGGGGGATGAGCAGCCTGCCCTCAACTGCCCCATGCTGAAGGCCCTCCAAAGGCAGC
ATGAATCCTCACAGTTCCCAGCCTGTGCCCTGACAACCTAGGAGGACACGCTGGTCTGGTCCATTGCTTATCTGAAACGATTCCCT
TTCCCTGTTCCTTTTGTTGCCTTGAGTGTCAGTTTTGGACCTCCCACTGAGCAGTTTTTTCTGTCACGTAGCCATAGACAGGACTG
AGTTCTTTGGAAAATAATCACATCCCACTGTTGTTCCCATTCGTTCATTTTTTCCTTCAACTAACCAACATTATTCAGCACTTTAC
TCAGCACAGGATGTGGAACCGAAGGCCTGGCTTTTGGGACACTCACCGTCCCCAGTCAGCTCTGTGTCCTAGGGAGAGACCCATGA
GCTCTCTGGGCCTCCTTTTGTTTCTCTGTAATAAAGGACTAAAGCTACCAGCCTCGCTGCGGGTTGGAAAAAAGAAAGAGTTAGTG
TAACACATGTGAATGTGTACGAAGGGGTTTAGAGACAGACAAAACATTGCACCAAGGCAGGGCTCGCTGGAGTGCAGACCCAGGAA
AAACACTGCAGGCCAAATTGCTTCTGGTCTGTCAGGGTACCAGCCACACATCCAAGGCAGGGAACTTAGGGCGATTGGCCTTTCTG
CTCATGCTTACTCTCCCAAGCTTGTTGGGTTATTATCATGCACAAGCCATTCAATCGATTGTATTGGGTGCCTAATATATCAGACA
CTCTTAGGTGCTAGGGATGTAAGAGCAAGCAAAACAGACATGGTTCTGCCCTTGTGGGCTGTATAGTCTAGTGGAGGAAGGGGGAA
GAGGTTAAGTATTTGTAGGAAGGTGAACCCCATGTTAAGAAAGCATCTTCCATCTTTCAAATGTTTCCAAGTAGCTGGTTATTCAT
GATAGCGAGGAAATGGTGGTCATCTCCATTGCTCTGGTAACTAGTTTGTGTCTGAGAAAGTGCTGATTTCCAAGAAAAGGAAAGAG
ACCACGGATGAATGATAGCAAGACTATTAAACAGGAATACAAAGAAGAGCTCACATTTATTGAGCACCTACTATATGTGCTGGACC
CTCTTGTAAGAGCTTCATACAAATTAGGCAGACAGGCAATATTATTATCCCCATTTGATCTACAAGGAAACTGAGTACAAAGTGAT
AGCTTTTCTGCAGAGCTTGTGCACAGGGGCCAAAGTTTGGGAACAATGCCTTTCTTTAATTTGAAAGTATCTGTTGTTGGTGTCAC
TTGTTGGAATTCCTAGAATCCCAGAAAAGTAAGTTTAAGTTTTCTTCTGCCAGCATCGGTCAGCCTACCTTTGACCCAAGGTCACG
ACTCCCTCCCTCCTCCCTTCCCTTCCCTGCTCTGACATTCTTACCTTGTTTTTGAGTTCCTTATTCCCTAGCTTCCTCCAAGCAGC
AGGCTCTGGGAACAGTCCCGTTCATTTATATAACAAATATTTATTGAAATGCAGACTTGTTGTTTTCATAATACCTGTGTACAAGT
TAAACCCGTGGTCACTTGTTCATGTGCCTCTTGTTTATGACAAGAGGTGGTCCCTTGTTGAAAGCCTGTCACATGGCAGGTACTCA
GTCTTTTCTTTCCTTCTAATAAGTAGGTGAGTATTTTCCTTGGTTTGCACACGAGGAAATGGAGGCTCAGAGAGCTTAGGTAGCTT
ACTCAAAGCCACACAGCTGGGTTAAATGGGTTCAGCACCATGAAAATCTGACCTTTAATGTCTGCACTCTTATTCTCTGGCTTATT
GGGAAAGAATTGGACAGGGATGGTAAATGGAGTTGATGGAGGCAAAGAGATAATTACTGTTGCTCAAAGGTGGATCGAGAGAGTTT
GGGCAAAAACGGAACCACGAACAAGCCGTTGAAAGTCCCCTCATCCTGGACCTGTGGTCTGTCCTTTGTAACTGCTCTTTTTTTGG
CATTCCCTCTGTCTCCTCTGCCCATCCCAATGCCAGGCACACTGAGCCTGTAGGCATTTACAAGGTGGGCCTCAGGTTTCTCCCCT
CCCAGGTGGGCATAGGAATCACTGCTTAGCTTAGATTTGGCAGAATGAAAGAGTCAGGCAGAGTCTCACCCGGCCCTGTTTCTTAG
CTGCTTTGACACCTGGGGCAGGTTTTTGGACCTCTCTGGACATGGCCAGCTGTGTTGTGACTGCCCACCAGAGGCTGGGTCTGTGA
ATGGAAGACTGAAGAAAAGGCGATGATCACAGTCCAGGGACAAGGTTTCTGAATCTTGTGAAATAGTTACAAATACTATGAAACAG
GAATACAAAGAAAAGCTCACATTTATTAAGCACTTACTTTGTGCTATACCCTCTTGTAAGAGCCTTGTACAAATTAGGCAGACAGA
CAATATTATTATCCCCATTTTACCTATAAGGAAACTGAGTGCAAAGTGACAGCTTTTCTGCAGAGCCTGTGCACAGGGGTCAAAGT
TTGGGAACACATTGCCTTTCTTTAATCTGAAACTATCTGCTGTTGGTCTCATTTGTTGAAATTCCTAGAATCCCAGAGAAGTAAGT
TTTAGCTTTCTTCTGCCAGCCCCGGTCAGCCTGCCTTTGACCCAAGGCCATAGTAAGTTCCAGAGTGACTTCCCGTCTGTTTTATT
ATCCCTGACAAAGGGATACAGTATTGTGCGGTGGCTGTGGATGGCGTGTAGTAGTGTTTTCCTTCTCTTGAAAGCAGCTGCTGAG
TGGTGGAGCACAGAGGTATTCCCTGAGCCCCAGCTTGTGCCCATGGTAGGTCCAGGGCGTTTGGGGGAAGGAGACCGACTCTAGGT
GGTCCCGTCTGGTTTCTCAGGGGAGAAAATTCATAGATCCATCTCCTAGCTGTCATCCAATTCTGTGTAGAAGGGGAAAGCCAAGG
TTGCCCTCAGATTTTGATCTTGGTTCACTCTGATTTCTCTATGCCAGACTAGTAGAGCTTCAGGGAAGCTTGGTGACTATGACTGG
ATGAATTCTGTCCCCCTTAAAACCATAGGTTGAAGTCCTACTCACCCATACCTCAGAACGTGACTGTGTTCGGAAATAGGGTCTCT
AGAGAGGTACAGTTAACTGAGCTCACGTGGCGGGGCTCCAATATGGTCAGACTGGTGGGTGTCCTTATCAGAAGAAGAGATTAAGA
TGACAAAGGAGGAGTGAAGACCACGTGTAGACACAGGGAGGAGGTGGCCATCTACAAGCCCAGGAGAGAGGCCTCAGAAGAAACAA
CTCTGACCACACCTTGATCCTGGACTTCTGGCTTTCAGAATTGTGAGGAAATAAACCTCTGTTGTCTGAGCAACCCAGCCTGTGAA
AATTTGTTGTAGCAGCCCTGGCGGATCAATACAGTGACCATGAGCAAGTCACTCACCCTCTCTGAACCCCCTTGGTCTCAGCTCTA
AAAAGAAGCCTGAGAATTTCTGCCTCACAGGCTGTGGTAAGAGGCATCAGAAATAGCCGACTTTCATCGGAGGCTCACTGCGAGCC
AGGGCCTGTTTAAGCCTTTTCCATGTCTGAACACACTTCATCTTCATGTGTAGCCTTTGGCTGGGGAGGGGAGATACTATTGTTCC
CATTTTACAGGTGAGGAGAGTGGAGCACAGAGCTGTTAATTAACCTGTCCCATGTTAGTCAGCAAGGAAGGGGCTGGTCCATATGT
GGAACTTCGTAGTTGTACTCTGGTGCCCACACACCAGATCACTATGCCGTCCCAGCTGTTGTTACAATAAGACTATGTAGGTGAAG
GGCCCACCCAGTAGTGCCTGGCCTATGGGACACATTGAAGACCAGCCCCCTGTGACCAGAACCTCCCATTTGCTGCATTGAGTTTG
GAATGTTTGAACTCCAGGAGCTGTGTGTGTGTGTGTGTGTGTGTGTGTGTGTGTGTGTGTGTGTATCTGTCTGTCTCCCTCT
CTCTCCTCTCTCCCCTACCTTCCTTTTCTTCCCTCCTGTTTTTGGGAGGGAGCTCAGCTTCCCTTCACTGCCACTGCCCATCAGCA
TCCTCTCCCTATTCAGCCACCCCCACCCCCCATCCCTGGTATTAATTGACTTTGGCTCCTGTCCTGGAGCTCAGCCTGGCACCCAG
ACCTGCCGTGCTATACTAACACCTCTCACATGGTTAAACCAATGGGGGTGGCATTTAGCAGATGACGCTGAAACCCGTGAATCTGC
CCTTTCTCACTGGGCCCTCACTGCTCTAGAGTCCCCGCCTCTTTTACGAAGCGGCTGTCATGGTTGATGGTGAGTGTCAGGCCCC
ACCCAGGGACAGGGAGGCCCAGTGCCGGCTGTGGAGTTACCCAGACCTTACGGTAAAAACGCATCACCCTGAATGGGGGCCAGGCC
AGGCATCGGCCCAAGCCGGCCCTGCCCAGCGGCTGGATTCTGACTCGCTGTGCCTTAGTCACAGGCCAGAGACTCTTCTGCTTCCA
GCTGAGGCTGTCCCCTGCCTTCCCTGGCGGCCCGGGTTCCTGGAAAAGCTGTGTGCCAGAGGACGGGAGTTTCCCTCCAACTTAAG
CCATTAGAAAGCTCATTTATTGAGCACTTACTAAGTGTCAGAGACTGTGCTAGGCACTTTACATGAATCTTCTCATTTAATCCTTT
TAATAAACTTCCGGGGCACATGCTCTTCTAATCTCCCTTTTACTGATGATGCAGGCTTGACAGTGGGAATGCTAACCGCAGAAAGC
AGCTCACCTCACTTGGGTGCTACTGTGTGCCAAGTCCTGGGGTTAGCACCCAATAGCCCCGTGGCGGCATGCTGTTATTACTCTCC
CTGCATAGGAGTGGGAGGCTGAGACCTGGGTGTTTCTGGGTGTCCCAGCTCCTGTATGCCATCTGCCTGTCCAGCCCTTAGCCACA
AAGCTGTTGTGCCATGTGGGGAGATGCGGCCAGTTTCCCAGGTGAGGCTGGTCCTGCTTCAAGCG

Human PVT1 Transcript(SEQ ID NO: 31)

AGCACATGGCCCGCGGGCCGGGCGGGCTCGGGGCGGCCGGGACGAGGAGGGGCGACGACGAGCTGCGAGCAAAGATGTGCCCCGG
GACCCCCGGCACCTTCCAGTGGATTTCCTTGCGGAAAGGATGTTGGCGGTCCCTGTGACCTGTGGAGACACGGCCAGATCTGCCCT
CCACAGGCCTGATCTTTTGGCCAGAAGGAGATTAAAAAGATGCCCCTCAAGATGGCTGTGCCTGTCAGCTGCATGGAGCTTCGTTC
AAGTATTTTCTGAGCCTGATGGATTTACAGTGATCTTCAGTGGTCTGGGGAATAACGCTGGTGGAACCATGCACTGGAATGACACA
CGCCCGGCACATTTCAGGATACTAAAAGTGGTTTTAAGGGAGGCTGTGGCTGAATGCCTCATGGATTCTTACAGCTTGGATGTCCA
TGGGGACGAAGGACTGCAGCTGGCTGAGAGGGTTGAGATCTCTGTTTACTTAGATCTCTGCCAACTTCCTTTGGGTCTCCCTATG
GAATGTAAGACCCCGACTCTTCCTGGTGAAGCATCTGATGCACGTTCCATCCGGCGCTCAGCTGGGCTTGAGCTGACCATACTCCC
TGGAGCCTTCTCCCGAGGTGCGCGGGTGACCTTGGCACATACAGCCATCATGATGGTACTTTAAGTGGAGGCTGAATCATCTCCCC
TTTGAGCTGCTTGGCACGTGGCTCCCTTGGTGTTCCCCTTTTACTGCCAGGACACTGAGATTTGGAGAGGTAAGTGGCTTACCTGA
GGCCATGTGCTAACAGAGAAGATGAAGAGATGATTGAAACAGGCCTAAGACCAGACCTAAGGGTCTGTACATTTTCCACATACTTT
CCATATCTTTAGAGGCCTGACCAAAGCAGATCTTTTCCTTTCTTCTAGGTAAGTCCAAAGGCACCTGCCTGCTGGGCCCACTGTTT

TCIAACTTTCCTAACTTTCTGATCCCTTGGAGGTGATAATCAAATATTCIAGICTGAGGCATTGGGATACATGGTGCTAGGITCTG
AGACTCTGCGTCAGGCCTGAACCCTGCATTTTGTGGAGGTGGGTGGGAGAATGTTCCCCTGGGGAACATGCCTAGACACGGGGGAC
AACAGTTGCCCTCATGGGGAGGTACCTGTTTACTCGCTGITATGGGACCGCITTCACAAAACCACTGCAGGTGAGTGAGTTCCTGC
TGAATATCAGGCCTGGTGTCTCTAGACTCATTATTTCCCCCACCCAACCCCTATGTTAGTTCATCTCGAGCCACATTTTTATTGCC
ATAATCCAGGCCTGGACAGGCCAAGATCTTTTAACAATTTTAATTACTGAAAATAATAACTGCATTTTTTTTAAAGCCCAACITTT
GGTAGAGTCAGCCCAAAATACAGTCTTTGTGTTGCCATCTGGGAACTGGATTTGGAATTGTTCTTCCATGAGACTGCAGAGCAGAA
CGGCAGGGCCAGAGGTCCCACGAGCTGGTCAGACCCGGTTCTGCTCCTTGCIGGCTGAGTGACCTIGGGCATTGT

Human PVT1 Protein (SEQ ID NO: 33)

MGPRAGRARGGRDEEGRRRAASKDVPRDPRHLPVDFLAERMLAVPVICGDTARSALHRPDLLARRRLKRCPSRWLCLSAAWSFVQV
FSEPDGFTVIFSGLGNNAGGTMHWNDTRPAHFRILKVVLREAVAECLMDSYSLDVHGGRRTAAG

Certain aspects of the present invention are described in greater detail in the non-limiting examples that follow.

EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all and only experiments performed Efforts have been made to ensure accuracy with respect to numbers used (e.g. amounts, temperature, etc.) but some experimental errors and deviations should be accounted for Unless indicated otherwise, parts are parts by weight, molecular weight is weight average molecular weight, temperature is in degrees Celsius, and pressure is at or near atmospheric.

**Example 1: Insertion site analysis following tumor induction in mice**

Tumors are induced in mice using either mouse mammary tumor virus (MMTV) or murine leukemia virus (MLV). MMTV causes mammary adenocarcinomas and MLV causes a variety of different hematopoetic malignancies (primarily T- or B-cell lymphomas) Three routes of infection are used: (1) injection of neonates with purified virus preparations, (2) infection by milk-borne virus during nursing, and (3) genetic transmission of pathogenic proviruses via the germ-line *(Akvr1* and/or *Mtv2*). The type of malignancy present in each affected mouse is determined by histological analysis of H&E-stained thin sections of formalin-fixed, paraffin-embedded biopsy samples, Host DNA sequences flanking all clonally-integrated proviruses in each tumor are recovered by nested anchored-PCR using two virus-specific primers and two primers specific for a 40 bp double stranded DNA anchor ligated to restriction enzyme digested tumor DNA, Amplified bands representing host/virus junction fragments are cloned and sequenced. Then the host sequences (called "tags") are used to BLAST analyze the Celera mouse genomic sequence. For each individual tag, three parameters are recorded: (1) the mouse chromosome assignment, (2) base pair coordinates at which the integration occurred, and (3) provirus orientation Using this information, all available tags from all analyzed tumors are mapped to the mouse genome. To identify the protooncogene targets of provirus insertion mutation, the provirus integration pattern at each cluster of integrants is analyzed relative to the locations of all known genes in the transcriptome. The presence of provirus at the same locus in two or more independent tumors is *prima facie* evidence that a protooncogene is present at or very near the proviral integration sites. This is because the genome is too large for random integrations to result in observable clustering. Any clustering that is detected is unequivocal evidence for biological selection during tumorigenesis. In order to identify the human orthologs of the protooncogene targets of provirus insertion mutation, a comparative analysis of syntenic regions of the mouse and human genomes is performed.

An example of PCR amplification of host/virus junction fragments is presented in Figure 1. Lane 1 contains the amplification products from normal control DNA and lane 2 contains the amplification products from tumor DNA. The bands result from 5' host/virus junction fragments present in the DNA samples. Lane 1 has bands from the *env*/3' LTR junctions from all proviruses (upper) and the host / 5' LTR from the pathogenic endogenous *Mtv2* provirus present in this particular mouse strain. This endogenous provirus is detected because its sequence is identical to the new clonally integrated proviruses in the tumor. All four new clonally integrated proviruses known to be in this tumor are readily detected

**Example 2: Analysis of Quantitative RT-PCR: Comparative $C_I$ Method.**

The expression level of target genes is quantified using the ABI PRISM 7900HT Sequence Detection System (Applied Biosystems, California). The method is based on the quantitation of the initial copy number of target template in comparison to that of'a reference (normalizer) housekeeper gene (Pre-Developed IaqMan® Assay Reagents Gene Expression Quantification Protocol, Applied Biosystems, 2001). Accumulation of DNA product with each PCR cycle is related to amplicon efficiency and the initial template concentration. Therefore the amplification efficiency of'both the target and the normalizer must be approximately equal. The threshold cycle ($C_l$), which is dependent on the starting template copy number and the DNA amplification efficiency, is a PCR cycle during which PCR product growth is exponential. With a similar dynamic range for the target and normalizer, the comparative $C_l$ method is applicable.

An example of the comparative $C_l$ method of gene expression for quantitative RT-PCR is shown in Figure 2. In the first step, assays are performed in quadruplicate on a normal tissue and several sample tissues In these tissues, the means and standard deviations of $C_l$ values are determined for housekeeper genes (chosen as controls if shown to be biologically stable among various samples, irrespective of disease state) and for the target gene. Figure 2 shows an example of average $C_l$ values for a housekeeper gene and target gene. These values can fall within a range from upper teens to 40 depending on the intrinsic expression level of the gene in the particular tissue. The coefficient of variance of all replicate sets cannot exceed 15%

An assessment of how the $\Delta C_l$ changes with template dilution verifies that the efficiencies of the target and housekeeper amplicons are approximately equal if the log input amount of template RNA versus $\Delta C_l$ plot has a slope < 0.10. With the relative efficiencies verified for target and housekeeper, the $\Delta\Delta C_l$ comparative calculation becomes valid, as mentioned above. An example of the calculated difference between the $C_l$ values of target and housekeeper genes ($\Delta C_l$) for various samples is shown in Figure 3. The $\Delta\Delta C_l$ is calculated for each sample by subtracting its $\Delta C_l$ value from the $\Delta C_l$ value of the baseline (calibrator) sample. If the expression is increased in some samples and decreased in others, $\Delta\Delta C_l$ will be a mixture of negative and positive values The final step in the calculation is to transform these values to absolute values. The formula for this is:

$$\text{Comparative expression level} = 2^{-\Delta\Delta Cl}$$

The final value for the calibrator should always be one. Figure 4 shows the $\Delta\Delta C_l$ and comparative expression level for each sample from Figure 3.

**Example 3: Analysis of mRNA Expression of Various Genes in Cancer Samples**

mRNA was prepared from various cancer samples as by standard procedures as are known in the art. Gene expression was measured as described in Example 2 above and as further described below The 5'-nuclease (TaqMan) chemistry differs from standard PCR by the addition of a dual-labeled (reporter and quencher) fluorescent probe which anneals between the two PCR primers. The fluorescence of' the reporter dye is quenched by the quencher being in close proximity. During thermal cycling, the 5' nuclease activity of Taq DNA polymerase cleaves the annealed probe and liberates the reporter and quencher dyes. An increase in fluorescence is seen, and the cycle number in which the fluorescence increases above background is related to the starting template concentration in a log-linear fashion.

For data analysis, expression level of the target gene was normalized with the expression level of a house keeping gene. The mean level of expression of the housekeeping gene was subtracted from the mean expression level of the target gene. Standard deviation was then determined In addition, the expression level of'the target gene in cancer tissue is compared with the expression level of the target gene in normal tissue. In Figures 5-24, bars represent the mean of expression level and error bars represent standard deviation.

(i) **SNL1**

Figure 5 depicts mRNA expression of SNL1 in breast cancer tissue compared with expression in normal tissue Samples 1-50 are breast cancer samples Samples 51 and 52 are normal tissues. SNL1 was up-regulated in approximately 20% of breast cancer samples examined

(ii) **FOSB**

Figure 6 depicts mRNA expression of FOSB in colon cancer tissue compared with expression in normal tissue Samples 1-11 are normal samples. Samples 12-31 are colon cancer tissues.

Figure 7 depicts mRNA expression of FOSB in lung cancer tissue compared with expression in normal tissue. Samples 1-9 are normal samples. Samples 10-43 are lung cancer tissues.

Figure 8 depicts mRNA expression of FOSB in pancreas cancer tissue compared with expression in normal tissue. Samples 1-10 are normal samples. Samples 11-31 are pancreas cancer tissues.

Figure 9 depicts mRNA expression of FOSB in ovary cancer tissue compared with expression in normal tissue.

Samples 1-16 are normal samples. Samples 17-44 are ovary cancer tissues.

Figure 10 depicts mRNA expression of FOSB in stomach cancer tissue compared with expression in normal tissue. Samples 1-10 are normal samples Samples 11-39 are stomach cancer tissues.

Figure 11 depicts mRNA expression of FOSB in breast cancer tissue compared with expression in normal tissues. Samples 1-9 are normal samples. Samples 10-30 are breast cancer tissues. FOSB was up-regulated in approximately 26% of breast cancer samples examined.

Figure 12 depicts mRNA expression of FOSB in prostate cancer tissue compared with expression in normal tissue. Samples 1-7 are normal samples. Samples 8-37 are prostate cancer tissues.

(iii) **MYC**

Figure 13 depicts mRNA expression of MYC in breast cancer tissue compared with expression in normal tissue. Samples 1-50 are breast cancer samples. MYC was up-regulated in approximately 8% of breast cancer samples examined.

Figure 14 depicts DNA amplification of MYC in breast cancer tissue compared with expression in normal tissue. Samples 1-49 are breast cancer samples. MYC DNA was amplified in approximately 21% of breast cancer samples examined.

(iv) **CCND1**

Figure 15 depicts mRNA expression of CCND1 in breast cancer tissue compared with expression in normal tissue. Samples 1-50 are breast cancer samples. Samples 51 and 52 are normal tissues. CCND1 was up-regulated in approximately 26% of breast cancer samples examined.

Figure 16 depicts mRNA expression of CCND1 in colon cancer (sigmoid) tissue compared with expression in normal tissue. Samples 1-12 are normal samples Samples 13-29 are colon cancer tissues

Figure 17 depicts mRNA expression of CCND1 in colon cancer (transverse) tissue compared with expression in normal tissue. Samples 1-12 are normal samples. Samples 13-30 are colon cancer tissues

Figure 18 depicts mRNA expression of CCND 1 in lung tissue compared with expression in normal tissue. Samples 1-9 are normal samples Samples 10-43 are lung cancer tissues.

Figure 19 depicts mRNA expression of CCND1 in ovary tissue compared with expression in normal tissue Samples 1-14 are normal sample. Samples 15-41 are ovary cancer tissues.

**(v) NFKB1**

Figure 20 depicts mRNA expression of NFKB1 in breast cancer tissue compared with expression in normal tissue. Samples 1-50 are breast cancer samples. Samples 51 and 52 are normal tissues. NFKB1 was up-regulated in approximately 25% of breast cancer samples examined

Figure 21 depicts mRNA expression of NFKB1 in lung tissue compared with expression in normal tissue. Samples 1-9 are normal samples. Samples 10-44 are lung cancer tissues.

Figure 22 depicts mRNA expression of NFKB1 in skin tissue compared with expression in normal tissue. Samples 1-9 are normal samples. Samples 10-46 are skin cancer tissues.

**(vi) PVT1**

Figure 23 depicts mRNA expression of PVT1 in breast cancer tissue compared with expression in normal tissue. Samples 1-50 are breast cancer samples. PVT1 was up-regulated in approximately 10% of breast cancer samples examined.

Figure 24 depicts DNA amplification of PVI1 in breast cancer tissue compared with expression in normal tissue Samples 1-50 are breast cancer samples.

**Example 4: Detection of elevated levels of cDNA associated with cancer using arrays.**

cDNA sequences representing a variety of candidate CA genes to be screened for differential expression in cancer are assayed by hybridization on polynucleotide arrays The cDNA sequences include cDNA clones isolated from cell lines or tissues of interest. The cDNA sequences analyzed also include polynucleotides comprising sequence overlap with sequences in the Unigene database, and which encode a variety of gene products of various origins, functionality, and levels of characterization cDNAs are spotted onto reflective slides (Amersham) according to methods well known in the art at a density of 9,216 spots per slide representing 4,068 sequences (including controls) spotted in duplicate, with approximately 0 8 μl of an approximately 200ng/μl solution of cDNA.

PCR products of selected cDNA clones corresponding to the gene products of interest are prepared in a 50% DMSO solution. These PCR products are spotted onto Amersham aluminum microarray slides at a density of 9216 clones per array using a Molecular Dynamics Generation III spotting robot. Clones are spotted in duplicate, for a total of 4608 different sequences per chip.

cDNA probes are prepared from total RNA obtained by laser capture microdissection (LCM, Arcturus Enginering Inc , Mountain View, CA) of tumor tissue samples and normal tissue samples isolated from patients.

Total RNA is first reverse transcribed into cDNA using a primer containing a T7 RNA polymerase promoter, followed by second strand DNA synthesis cDNA is then transcribed *in vitro* to produce antisense RNA using the T7 promoter-mediated expression (see, *e.g.,* Luo et al. (1999) Nature Med 5:117-122), and the antisense RNA is then converted into cDNA. The second set of'cDNAs are again transcribed *in vitro,* using the T7 promoter, to provide antisense RNA. This antisense RNA is then fluorescently labeled, or the RNA is again converted into cDNA, allowing for a third round of T7-mediated amplification to produce more antisense RNA Thus the procedure provides for two or three rounds of *in vitro* transcription to produce the final RNA used for fluorescent labeling. Probes are labeled by making fluorescently labeled cDNA from the RNA starting material. Fluorescently labeled cDNAs prepared from the tumor RNA sample are compared to fluorescently labeled cDNAs prepared from normal cell RNA sample. For example, the cDNA probes from the normal cells are labeled with Cy3 fluorescent dye (green) and the cDNA probes prepared from suspected cancer cells are labeled with Cy5 fluorescent dye (red)

The differential expression assay is performed by mixing equal amounts of probes from tumor cells and normal cells of the same patient The arrays are prehybridized by incubation for about 2 hrs at 60°C in 5x SSC, 0.2% SDS, 1 mM EDTA, and then washing three times in water and twice in isopropanol. Following prehybridization of the array, the probe mixture is then hybridized to the array under conditions of high stringency (overnight at 42°C in 50% formamide, 5X SSC, and 0.2% SDS. After hybridization, the array is washed at 55°C three times as follows: 1) first wash in 1X SSC/ 0.2% SDS; 2) second wash in 0.1X SSC/0.2% SDS; and 3) third wash in 0.1X SSC.

The arrays are then scanned for green and red fluorescence using a Molecular Dynamics Generation III dual color laser-scanner/detector. The images are processed using BioDiscovery Autogene software, and the data from each scan set normalized The experiment is repeated, this time labeling the two probes with the opposite color in order to perform the assay in both "color directions." Each experiment is sometimes repeated with two more slides (one in each color direction). The data from each scan is normalized, and the level of fluorescence for each sequence on the array expressed as a ratio of the geometric mean of 8 replicate spots/genes from the four arrays or 4 replicate spots/gene from 2 arrays or some other permutation.

<u>Normalization:</u> The objective of normalization is to generate a cDNA library in which all transcripts expressed in a particular cell type or tissue are equally represented (S.M. Weissman, Mol Biol. Med. 4(3):133-143 (1987); Patanjali, et al., Proc, Natl. Acad Sci USA 88(5):1943-1947 (1991)), and therefore isolation of as few as 30,000 recombinant clones in an optimally normalized library may represent the entire gene expression repertoire of a cell, estimated to number 10,000 per cell

Total RNA is extracted from harvested cells using RNeasy™ Protect Kit (Qiagen, Valencia, CA), following manufacturer's recommended procedures RNA is quantified using RiboGreen™ RNA quantification kit (Molecular Probes, Inc. Eugene, OR). One μg of total RNA is reverse transcribed and PCR amplified using SMART™ PCR cDNA synthesis kit (ClonTech, Palo Alto, CA). The cDNA products are size-selected by agarose gel electrophoresis using standard procedures (Sambrook, J.I., et al. Molecular Cloning: A Laboratory Manual, 2d ed., Cold Spring Harbor Laboratory Press, NY). The cDNA is extracted using Bio 101 Geneclean® II kit (Qbiogene, Carlsbad, CA). Normalization of the cDNA is carried out using kinetics of hybridization principles: 1.0 μg of cDNA is denatured by heat at 100°C for 10 minutes, then incubated at 42° C for 42 hours in the presence of 120 mM NaCl, 10 mM Tris HCl (pH=8.0), 5 mM EDTA Na+ and 50% formamide. Single-stranded cDNA ("normalized") is purified by hydroxyapatite chromatography (#130-0520, BioRad, Hercules, CA) following the manufacturer's recommended procedures, amplified and converted to double-stranded cDNA by three cycles of PCR amplification, and cloned into plasmid vectors using standard procedures (Sambrook, J.T., et al Molecular Cloning: A Laboratory Manual, 2d ed., Cold Spring Harbor Laboratory Press, NY). All primers/adaptors used in the normalization and cloning process are provided by the manufacturer in the SMART™ PCR cDNA synthesis kit (ClonTech, Palo Alto, CA). Supercompetent cells (XL-2 Blue Ultracompetent Cells, Stratagene, California) are transfected with the normalized cDNA libraries, plated on solid media and grown overnight at 36° C.

The sequences of 10,000 recombinants per normalized library are analyzed by capillary sequencing using the ABI PRISM 3700 DNA Analyzer (Applied Biosystems, California). To determine the representation of transcripts in a library, BLAST analysis is performed on the clone sequences to assign transcript identity to each isolated clone, i.e., the sequences of the isolated polynucleotides are first masked to eliminate low complexity sequences using the XBLAST masking program (Claverie "Effective Large-Scale Sequence Similarity Searches," Computer Methods for Macromolecular Sequence Analysis, Doolittle, ed., Meth. Enzymol 266:212-227 Academic Press, NY, NY (1996); see particularly Claverie, in "Automated DNA Sequencing and Analysis Techniques" Adams et al., eds., Chap. 36, p. 267 Academic Press, San Diego, 1994 and Claverie et al. Comput. Chem. (1993) 17:191). Generally, masking does not influence the final search results, except to eliminate sequences of relative little interest due to their low complexity, and to eliminate multiple "hits" based on similarity to repetitive regions common to multiple sequences, e.g., Alu repeats. The remaining sequences are then used in a BLASIN vs. GenBank search. The sequences are also used as query sequence in a BLASIX vs. NRP (non-redundant proteins) database search.

Automated sequencing reactions are performed using a Perkin-Elmer PRISM Dye Terminator Cycle Sequencing Ready Reaction Kit containing AmpliTaq DNA Polymerase, FS, according to the manufacturer's directions. The reactions are cycled on a GeneAmp PCR System 9600 as per manufacturer's instructions, except that they are annealed at 20° C. or 30° C for one minute. Sequencing reactions are ethanol precipitated, pellets are resuspended in 8 microliters of loading buffer, 15 microliters is loaded on a sequencing gel, and the data is collected by an ABI PRISM 3700 DNA Sequencer (Applied Biosystems, Foster City, CA).

The number of times a sequence is represented in a library is determined by performing sequence identity analysis on the cloned cDNA sequences and assigning transcript identity to each isolated clone. First, each sequence is checked to determine if it is a bacterial, ribosomal, or mitochondrial contaminant Such sequences are excluded from the subsequent analysis Second, sequence artifacts, such as vector and repetitive elements, are masked and/or removed from each sequence.

The remaining sequences are compared via BLASI (Altschul et al, J. Mol., Biol., 215:40, 1990) to GenBank and EST databases for gene identification and are compared with each other via FastA (Pearson & Lipman, PNAS, 85: 2444, 1988) to calculate the frequency of cDNA appearance in the normalized cDNA library. The sequences are also searched against the GenBank and GeneSeq nucleotide databases using the BLASIN program (BLASIN 1.3MP: Altschul et al., J Mol. Bio. 215:403, 1990). Fourth, the sequences are analyzed against a non-redundant protein (NRP) database with the BLASIX program (BLASIX 1.3MP: Altschul et al., supra). This protein database is a combination of the Swiss-Prot, PIR, and NCBI GenPept protein databases. The BLASTX program is run using the default BLOSUM-62 substitution matrix with the filter parameter: "xnu+seg". The score cutoff utilized is 75. Assembly of overlapping clones into contigs is done using the program Sequencher (Gene Codes Corp.; Ann Arbor, Mich.). The assembled contigs are analyzed using the programs in the GCG package (Genetic Computer Group, University Research Park, 575 Science Drive, Madison, Wis.. 53711) Suite Version 10.1.

**Example 5: Detection of CA -Sequences in Human Cancer Cells and Tissues.**

DNA from prostate and breast cancer tissues and other human cancer tissues, human colon, normal human tissues including non-cancerous prostate, and from other human cell lines are extracted following the procedure of Delli Bovi et al., (1986, Cancer Res. 46:6333-6338). The DNA is resuspended in a solution containing 0.05 M Tiis HCl buffer, pH 7,8, and 0,1 mM EDTA, and the amount of DNA recovered is determined by microfluorometry using Hoechst 33258 dye. Cesarone, C. et al., Anal Biochem 100:188-197 (1979).

Polymerase chain reaction (PCR) is performed using Taq polymerase following the conditions recommended by the manufacturer (Perkin Elmer Cetus) with regard to buffer, $Mg^{2+}$, and nucleotide concentrations. Thermocycling is performed in a DNA cycler by denaturation at 94° C. for min. followed by either 35 or 50 cycles of 94° C, for 1.5 min., 50° C. for 2 min. and 72° C. for 3 min. The ability of the PCR to amplify the selected regions of the CA gene is tested by using a cloned CA polynucleotide(s) as a positive template(s). Optimal $Mg^{2+}$, primer concentrations and requirements for the different cycling temperatures are determined with these templates. The master mix recommended by the manufacturer is used. To detect possible contamination of the master mix components, reactions without template are routinely tested.

Southern blotting and hybridization are performed as described by Southern, E. M., (J. Mol. Biol. 98:503-517, 1975), using the cloned sequences labeled by the random primer procedure (Feinberg, A. P., et al, 1983, Anal. Biochem. 132:6-13). Prehybridization and hybridization are performed in a solution containing 6xSSPE, 5% Denhardt's, 0.5% SDS, 50% formamide, 100 μg/ml denaturated salmon testis DNA, incubated for 18 hrs at 42° C, followed by washings with 2xSSC and 0.5% SDS at room temperature and at 37° C and finally in 0.1xSSC with 0.5% SDS at 68° C, for 30 min (Sambrook et al., 1989, in "Molecular Cloning: A Laboratory Manual", Cold Spring Harbor Lab. Press). For paraffin-embedded tissue sections the conditions described by Wright and Manos (1990, in "PCR Protocols", Innis et al, eds., Academic Press, pp. 153-158) are followed using primers designed to detect a 250 bp sequence.

**Example 6: Detection of CA Sequences in Human Cancer Cells and Tissues.**

DNA from human cancer tissues, normal human tissues and from other human cell lines is extracted following the procedure of Delli Bovi et al. (1986, Cancer Res 46:6333-6338). The DNA is resuspended in a solution containing 0.05 M Tris HCl buffer, pH 7.8, and 0.1 mM EDTA, and the amount of DNA recovered is determined by microfluorometry using Hoechst :33258 dye Cesarone, C. et al., Anal Biochem 100:188-197 (1979).

Polymerase chain reaction (PCR) is performed using Taq polymerase following the conditions recommended by the manufacturer (Perkin Elmer Cetus) with regard to buffer, $Mg^{2+}$, and nucleotide concentrations Thermocycling is performed in a DNA cycler by denaturation at 94° C, for 3 min, followed by either 35 or 50 cycles of 94° C for 15 min., 50° C for 2 min and 72° C. for 3 min. The ability of the PCR to amplify the selected regions of CA genes is tested by using a cloned CA polynucleotide(s) as a positive template(s). Optimal $Mg^{2+}$, primer concentrations and requirements for the different cycling temperatures are determined with these templates. The master mix recommended by the manufacturer is used To detect possible contamination of the master mix components, reactions without template are routinely tested.

Southern blotting and hybridization are performed as described by Southern, E. M., (J. Mol. Biol. 98:503-517, 1975), using the cloned sequences labeled by the random primer procedure (Feinberg, A. P., et al., 1983, Anal. Biochem 132:6-13). Prehybridization and hybridization are performed in a solution containing 6x SSPE, 5% Denhardt's, 0.5% SDS, 50% formamide, 100 μg/ml denaturated salmon testis DNA, incubated for 18 hrs at 42° C, followed by washings with 2x SSC and 0.5% SDS at room temperature and at 37° C, and finally in 0.1xSSC with 0.5% SDS at 68° C for 30 min (Sambrook et al., 1989, in "Molecular Cloning: A Laboratory Manual", Cold Spring Harbor Lab. Press). For paraffin-embedded tissue sections the conditions described by Wright and Manos (1990, in "PCR Protocols", Innis et al., eds., Academic Press, pp. 153-158) are followed using primers designed to detect a 250 bp sequence.

**Example 7: Expression of cloned polynucleotides in host cells.**

To study the protein products of' CA genes, restriction fragments from CA DNA are cloned into the expression vector pMT2 (Sambrook, et al., Molecular Cloning: A Laboratory Manual, Cold Spring Harbor Laboratory Press pp 16.17-16.22 (1989)) and transfected into COS cells grown in DMEM supplemented with 10% FCS Transfections are performed employing calcium phosphate techniques (Sambrook, et al (1989) pp. 16.32-16.40, supra) and cell lysates are prepared forty-eight hours after transfection from both transfected and untransfected COS cells. Lysates are subjected to analysis by immunoblotting using anti-peptide antibody..

In immunoblotting experiments, preparation of cell lysates and electrophoresis are performed according to standard procedures. Protein concentration is determined using BioRad protein assay solutions. After semi-dry electrophoretic transfer to nitrocellulose, the membranes are blocked in 500 mM NaCl, 20 mM Tris, pH 7.5, 0.05% Tween-20 (TTBS) with 5% dry milk. After washing in TTBS and incubation with secondary antibodies (Amersham), enhanced chemiluminescence (ECL) protocols (Amersham) are performed as described by the manufacturer to facilitate detection.

**Example 8: Generation of antibodies against polypeptides.**

Polypeptides, unique to CA genes are synthesized or isolated from bacterial or other (e.g., yeast, baculovirus) expression systems and conjugated to rabbit serum albumin (RSA) with m-maleimido benzoic acid N-hydroxysuccinimide ester (MBS) (Pierce, Rockford, III) Immunization protocols with these peptides are performed according to standard methods. Initially, a pre-bleed of the rabbits is performed prior to immunization. The first immunization includes Freund's complete adjuvant and 500 μg conjugated peptide or 100 μg purified peptide. All subsequent immunizations, performed four weeks after the previous injection, include Freund's incomplete adjuvant with the same amount of protein Bleeds are conducted seven to ten days after the immunizations.

For affinity purification of the antibodies, the corresponding CA polypeptide is conjugated to RSA with MBS, and coupled to CNBr-activated Sepharose (Pharmacia, Uppsala, Sweden). Antiserum is diluted 10-fold in 10 mM Tris-HCl, pH 7.5, and incubated overnight with the affinity matrix. After washing, bound antibodies are eluted from the resin with 100 mM glycine, pH 2.5.

**Example 9: Generation of monoclonal antibodies against a CA polypeptide**

A non-denaturing adjuvant (Ribi, R730, Corixa, Hamilton MT) is rehydrated to 4ml in phosphate buffered saline 100μl of this rehydrated adjuvant is then diluted with 400μl of Hank's Balanced Salt Solution and this is then gently mixed with the cell pellet used for immunization Approximately 500 μg conjugated peptide or 100 μg purifies peptide and Freund's complete are injected into Balb/c mice via foot-pad, once a week After 6 weeks of weekly injection, a drop of blood is drawn from the tail of each immunized animal to test the titer of antibodies against CA polypeptides using FACS analysis When the titer reaches at least 1:2000, the mice are sacrificed in a $CO_2$ chamber followed by cervical dislocation. Lymph nodes are harvested for hybridoma preparation Lymphocytes from mice with the highest liter are fused with the mouse myeloma line X63-Ag8, 653 using 35% polyethylene glycol 4000. On day 10 following the fusion, the hybridoma supernatants are screened for the presence of' CAP-specific monoclonal antibodies by fluorescence activated cell sorting (FACS) Conditioned medium from each hybridoma is incubated for 30 minutes with a combined aliquot of PC3, Colo-205, LnCap, or Panc-1 cells After incubation, the cell samples are washed, resuspended in 0.1 ml diluent and incubated with 1 μg/ml of FITC conjugated F(ab')2 fragment of goat anti-mouse IgG for 30 min at 4°C, The cells are washed, resuspended in 0.5 ml FACS diluent and analyzed using a FACScan cell analyzer (Becton Dickinson; San Jose, CA) Hybridoma clones are selected for further expansion, cloning, and characterization based on their binding to the surface of one or more of cell lines which express the CA polypeptide as assessed by FACS A hybridoma making a monoclonal antibody designated mAbCA which binds an antigen designated Ag-CA.x and an epitope on that antigen designated Ag-CA.x.1 is selected

**Example 10: ELISA assay for Detecting CA related antigens.**

To test blood samples for antibodies that bind specifically to recombinantly produced CA antigens, the following procedure is employed. After a recombinant CA related protein is purified, the recombinant protein is diluted in PBS to a concentration of 5 μg/ml (500 ng/100 μl) 100 microliters of the diluted antigen solution is added to each well of a 96-well Immulon 1 plate (Dynatech Laboratories, Chantilly, Va), and the plate is then incubated for 1 hour at room temperature, or overnight at 4°C, and washed 3 times with 0.05% Tween 20 in PBS. Blocking to reduce nonspecific binding of antibodies is accomplished by adding to each well 200 μl of a 1% solution of bovine serum albumin in PBS/Tween 20 and incubation for 1 hour. After aspiration of the blocking solution, 100 μl of the primary antibody solution (anticoagulated whole blood, plasma, or serum), diluted in the range of 1/16 to 1/2048 in blocking solution, is added and incubated for 1 hour at room temperature or overnight at 4° C. The wells are then washed 3 times, and 100 μl of goat anti-human IgG antibody conjugated to horseradish peroxidase (Organon Teknika, Durham, N.C), diluted 1/500 or 1/1000 in PBS/Tween 20, 100 μl of o-phenylenediamine dihydrochloride (OPD, Sigma) solution is added to each well and incubated for 5-15 minutes. The OPD solution is prepared by dissolving a 5 mg OPD tablet in 50 ml 1% methanol in $H_2O$ and adding 50 μl 30% $H_2O2$ immediately before use The reaction is stopped by adding 251 of 4M $H_2SO_4$. Absorbances are read at 490 nm in a microplate reader (Bio-Rad).

**Example 11: Identification and characterization of CA antigen on cancer cell surface**

A cell pellet of proximately 25 ul packed cell volume of a cancer cell preparation is lysed by first diluting the cells to 0.5 ml in water followed by freezing and thawing three times. The solution is centrifuged at 14,000 rpm. The resulting pellet, containing the cell membrane fragments, is resuspended in 50 μl of SDS sample buffer (Invitrogen, Carlsbad, CA), The sample is heated at 80°C for 5 minutes and then centrifuged for 2 minutes at 14,000 rpm to remove any insoluble materials

The samples are analyzed by Western blot using a 4 to 20% polyacrylamide gradient gel in Tris-Glycine SDS (Invitrogen; Carlsbad CA) following the manufacturer's directions. Ten microliters of membrane sample are applied to one lane on the polyacrylamide gel, A separate 10 μL sample is reduced first by the addition of 2 μL of dithiothreitol (100 mM) with heating at 80°C for 2 minutes and then loaded into another lane. Pre-stained molecular weight markers SeeBlue Plus2 (Invitrogen; Carlsbad, CA) are used to assess molecular weight on the gel. The gel proteins are transferred to a nitrocellulose membrane using a transfer buffer of 14.4 g/l glycine, 3 g/l of Tris Base, 10% methanol, and 0.05% SDS. The membranes are blocked, probed with a CAP-specific monoclonal antibody (at a concentration of 0.5 ug/ml), and developed using the Invitrogen WesteinBreeze Chromogenic Kit-AntiMouse according to the manufacturer's directions, In the reduced sample of the tumor cell membrane samples, a prominent band is observed migrating at a molecular weight within about 10% of the predicted molecular weight of the corresponding CA protein.

**Example 12: Preparation of vaccines.**

The present invention also relates to a method of stimulating an immune response against cells that express CA polypeptides in a patient using CA polypeptides of the invention that act as an antigen produced by or associated with a malignant cell This aspect of the invention provides a method of stimulating an immune response in a human against cancer cells or cells that express CA polynucleotides and polypeptides The method comprises the step of administering to a human an immunogenic amount of a polypeptide comprising: (a) the amino acid sequence of a huma CA protein or (b) a mutein or variant of a polypeptide comprising the amino acid sequence of a human endogenous retrovirus CA protein.

**Example 13: Generation ot transgenic animals expressing polypeptides as a means for testing therapeutics.**

CA nucleic acids are used to generate genetically modified non-human animals, or site specific gene modifications thereof, in cell lines, for the study of function or regulation of prostate tumor-related genes, or to create animal models of' diseases, including prostate cancer, The term "transgenic" is intended to encompass genetically modified animals having an exogenous CA gene(s) that is stably transmitter in the host cells where the gene(s) may be altered in sequence to produce a modified protein, or having an exogenous CA LTR promoter operably linked to a reported gene. Transgenic animals may be made through a nucleic acid construct randomly integrated into the genome Vectors for stable integration include plasmids, retro-viruses and other animal viruses, YACs, and the like Of interest are transgenic mammals, e.g. cows, pigs, goats, horses, etc., and particularly rodents, e.g. rats, mice, etc,

The modified cells or animals are useful in the study of CA gene function and regulation For example, a series of small deletions and/or substitutions may be made in the CA genes to determine the role of different genes in tumorigenesis. Specific constructs of interest include, but are not limited to, antisense constructs to block CA gene expression, expression of dominant negative CA gene mutations, and overexpression of a CA gene. Expression of a CA gene or variants thereof in cells or tissues where it is not normally expressed or at abnormal times of development is provided. In addition, by providing expression of proteins derived from CA in cells in which it is otherwise not normally produced, changes in cellular behavior can be induced.

DNA constructs for random integration need not include regions of homology to mediate recombination Conveniently, markers for positive and negative selection are included For various techniques for transfecting mammalian cells, see Keown et al., Methods in Enzymology 185:527-537 (1990)

For embryonic stem (ES) cells, an ES cell line is employed, or embryonic cells are obtained freshly from a host, e.g. mouse, rat, guinea pig, etc Such cells are grown on an appropriate fibroblast-feeder layer or grown in the presence of appropriate growth factors, such as leukemia inhibiting factor (LIF). When ES cells are transformed, they may be used to produce transgenic animals After transformation, the cells are plated onto a feeder layer in an appropriate medium. Cells containing the construct may be detected by employing a selective medium. After sufficient time for colonies to glow, they are picked and analyzed for the occurrence of integration of the construct, Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting chimeric animals screened for cells bearing the construct. By providing for a different phenotype of the blastocyst and the ES cells, chimeric progeny can be readily detected.

The chimeric animals are screened for the presence of the modified gene and males and females having the modification are mated to produce homozygous progeny. If the gene alterations cause lethality at some point in development, tissues or organs are maintained as allogeneic or congenic grafts or transplants, or in in vitro culture. The transgenic animals may be any non-human mammal, such as Laboratory animals, domestic animals, etc. The transgenic animals are used in functional studies, drug screening, etc., e.g. to determine the effect of'a candidate drug on prostate cancer, to test potential therapeutics or treatment regimens, etc.

**Example 14: Diagnostic Imaging Using CA Specific Antibodies**

The present invention encompasses the use of antibodies to CA polypeptides to accurately stage cancer patients at initial presentation and for early detection of metastatic spread of cancer. Radioimmunoscintigraphy using monoclonal antibodies specific for CA polypeptides can provides an additional cancer-specific diagnostic test The monoclonal antibodies of the instant invention are used for histopathological diagnosis of carcinomas

Subcutaneous human xenografts of cancer cells in nude mice is used to test whether a technetium-99rn ($^{99m}$Tc )-labeled monoclonal antibody of'the invention can successfully image the xenografted cancer by external gamma scintography as described for seminoma cells by Marks, et al, But J Urol 75:225 (1995). Each monoclonal antibody specific for a CA polypeptide is purified from ascitic fluid of BALB/c mice bearing hybridoma tumors by affinity chromatography on protein A-Sepharose. Purified antibodies, including control monoclonal antibodies such as an avidin-specific monoclonal antibody (Skea, et al.., J Immunol. 151:3557 (1993)) are labeled with $^{99m}$Tc following reduction, using the methods of Mather, et al., J. Nucl Med. 31:692 (1990) and Zhang et al., Nucl. Med. Biol. 19:607 (1992), Nude mice bearing human cancer cells are injected intraperitoneally with 200-500 μCi of $^{99m}$Tc-labeled antibody. Twenty-four hours after injection, images of the mice are obtained using a Siemens ZLC3700 gamma camera equipped with a 6 mm pinhole collimator set approximately 8 cm from the animal. To determine monoclonal antibody biodistribution following imaging, the normal organs and tumors are removed, weighed, and the radioactivity of the tissues and a sample of the injectate are measured Additionally, CA-specific antibodies conjugated to antitumor compounds are used for cancer-specific chemotherapy.

**Example 15: Immunohistochemical methods**

Froze tissue samples from cancer patients are embedded in an optimum cutting temperature (OCT) compound and quick-frozen in isopentane with dry ice. Cryosections are cut with a Leica 3050 CM mictrotome at thickness of 5 μm and thaw-mounted on vectabound-coated slides The sections are fixed with ethanol at-20°C and allowed to air dry overnight at room temperature. The fixed sections are stored at -80°C until use. For immunohistochemistry, the tissue sections are retrieved and first incubated in blocking buffer (PBS, 5% normal goat serum, 0.1% Tween 20) for 30 minutes at room temperature, and then incubated with the CA protein-specific monoclonal antibody and control monoclonal antibodies diluted in blocking buffer (1 μg/ml) for 120 minutes. The sections are then washed three times with the blocking buffer. The bound monoclonal antibodies are detected with a goat anti-mouse IgG + IgM (H+L) F(ab')$^2$-peroxidase conjugates and the peroxidase substrate diaminobenzidine (1 mg/ml, Sigma Catalog No. D 5637) in 0.1 M sodium acetate buffer pH 5.05 and 0.003% hydrogen peroxide (Sigma cat No. H1009). The stained slides are counter-stained with hematoxylin and examined under Nikon microscope.

Monoclonal antibody against a CA protein (antigen) is used to test reactivity with various cell lines from different types of tissues. Cells from different established cell lines are removed from the growth surface without using proteases, packed and embedded in OCT compound The cells are frozen and sectioned, then stained using a standard IHC protocol The CellArray™ technology is described in WO 01/43869. Normal tissue (human) obtained by surgical resection are frozen and mounted Cryosections are cut with a Leica 3050 CM mictrotome at thickness of 5μm and thaw-mounted on vectabound-coated slides. The sections are fixed with ethanol at - 20°C and allowed to air dry overnight at room temperature. PolyMICA™ Detection kit is used to determine binding of a CA-specific monoclonal antibody to normal tissue. Primary monoclonal antibody is used at a final concentration of 1 μg/ml.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims

The following clauses represent preferred embodiments of the invention.

1. An isolated nucleic acid comprising at least 10 contiguous nucleotides of a sequence selected from the group consisting of the human polynucleotide mRNA sequences of SEQ ID NOS: 5, 11, 17, 23, 29 and 32 shown in Tables 1-6, or its complement.

2. A host cell comprising a recombinant nucleic acid of clause 1.

3. An expression vector comprising the isolated nucleic acid according to clause 1.

4. A host cell comprising the expression vector of clause 3.

5. The polynucleotide according to clause 1, wherein said polynucleotide, or its complement or a fragment thereof, further comprises a detectable label.

6. The polynucleotides according to clause 1, wherein said polynucleotide, or its complement or a fragment thereof, is attached to a solid support.

7. The polynucleotide according to clause 1, wherein said polynucleotide, or its complement or a fragment thereof, is prepared at least in part by chemical synthesis.

8. The polynucleotide according to clause 1, wherein said polynucleotides, or its complement or a fragment thereof, is an antisense fragment.

9. The polynucleotide according to clause 1, wherein said polynucleotide, or its complement or a fragment thereof, is single stranded.

10. The polynucleotide according to clause 1, wherein said polynucleotide, or its complement or a fragment thereof, is double stranded.

11. The polynucleotide according to clause 1, comprising at least 15 contiguous nucleotides.

12. The polynucleotide according to clause 1, comprising at least 20 contiguous nucleotides.

13. A microarray for detecting a cancer associated (CA) nucleic acid comprising: at least one probe comprising at least 10 contiguous nucleotides of a sequence selected from the group consisting of the human polynucleotide sequences of SEQ ID NOS: 5, 11, 17, 23, 29 and 32 shown in Tables 1-6, or its complement.

14. The microarray according to clause 13, comprising at least 15 contiguous nucleotides.

15. The microarray according to clause 13, comprising at least 20 contiguous nucleotide.

16. An isolated polypeptide, encoded within an open reading frame of a CA sequence selected from the group consisting of the human genomic polynucleotide sequences of SEQ ID NOS: 4, 10, 16, 22, 28 and 31 shown in Tables 1-6, or its complement.

17. The polypeptide of clause 16, wherein said polypeptide comprises the amino acid sequence encoded by a human polynucleotides selected from the group consisting of SEQ ID NOS: 5, 11, 17, 23, 29 and 32 shown in Tables 1-6.

18. The polypeptide of clause 16, wherein said polypeptide comprises the amino acid sequence encoded by a human coding sequence selected from the group consisting of SEQ ID NOS: 6, 12, 18, 24, 30 and 33 shown in Tables 1-6.

19. The polypeptide of clause 16, wherein said polypeptide comprises the amino acid sequence of an epitope of the amino acid sequence of a CA polypeptide selected from the group consisting of SEQ ID NOS: 6, 12, 18, 24, 30 and 33 shown in Tables 1-6.

20. The polypeptide of clause 16, wherein said polypeptide or fragment thereof is attached to a solid support.

21. An isolated antibody or antigen binding fragment thereof, that binds to a polypeptide according to anyone of clauses 16-20.

22. The isolated antibody or antigen binding fragment thereof according the clause 21, wherein said antibody or fragment thereof is attached to a solid support.

23. The isolated antibody or antigen binding fragment thereof according the clause 21, wherein said antibody is a monoclonal antibody.

24. The isolated antibody or antigen binding fragment thereof according the clause 21, wherein said antibody is a polyclonal antibody.

25. The isolated antibody or antigen binding fragment thereof according the clause 21, wherein said antibody or fragment thereof further comprises a detectable label.

26. An isolated antibody that binds to a polypeptides, or antigen binding fragment thereof, according to any of clauses 16-20, prepared by a method comprising the following steps of: (i) immunizing a host animal with a composition comprising said polypeptide, or antigen binding fragment thereof, and ii) collecting cells from said host expressing antibodies against the antigen or antigen binding fragment thereof.

27. A kit for diagnosing the presence of cancer in a test samples, said kit comprising at least one polynucleotide that selectively hybridizes to a CA polynucleotide sequence selected from the group consisting of the polynucleotide sequences of SEQ ID NOS: 4, 10, 16, 22, 28 and 31 shown in Tables 1-6, a fragment thereof, or their complement.

28. A kit for diagnosing the presence of cancer in a test sample, said kit comprising at least one polynucleotide that selectively hybridizes to the sequence of a polynucleotide sequence selected from the group consisting of the polynucleotide sequences of SEQ ID NOS: 5, 11, 17, 23, 29 and 32 shown in Tables 1-6, a fragment thereof, or their complement.

29. An electronic library comprising a polynucleotide, or fragment thereof, comprising a CA polynucleotide sequence selected from the group consisting of the polynucleotide sequences of SEQ ID NOS: 4, 10, 16, 22, 28 and 31 shown in Tables 1-6.

30. An electronic library comprising a polynucleotide, or fragment thereof, comprising a CA polynucleotide sequence selected from the group consisting of the polynucleotide sequences of SEQ ID NOS: 5, 11, 17, 23, 29 and 32 shown in Tables 1-6.

31. An electronic library comprising a polypeptide, or fragment thereof, comprising a CA polypeptide encoded by a polynucleotide of a sequence selected from the group consisting of the polynucleotide sequences of SEQ ID NOS: 6, 12, 18, 24, 30 and 33 shown in Tables 1-6.

32. A method for screening for anticancer activity in a potential drug, the method comprising: (a) providing a cell that expresses a cancer associated (CA) gene encoded by a nucleic acid sequence selected from the group consisting of the sequences of SEQ ID NOS: 4, 10, 16, 22, 28 and 31 shown in Tables 1-6 or fragment thereof; (b) contacting a tissue sample derived from a cancer cell with an anticancer drug candidate; and (c) monitoring an effect of the anticancer drug candidate on an expression of the CA gene in the tissue sample.

33. The method of screening for anticancer activity according to clause 32, wherein the CA gene comprises at least one nucleic acid sequence selected from the group consisting of the sequences of SEQ ID NOS: 5, 11, 17, 23, 29 and 32 shown in Tables 1-6.

34. The method of screening for anticancer activity according to clause 32, further comprising: (d) comparing the level of expression of the in the absence of said drug candidate to the level of expression in the presence of the drug candidate.

35. The method of screening for anticancer activity according to clause 33, wherein the drug candidate modulates the activity of a CAP sequence selected from the group consisting of SNL, FOSB, CCND1, MYC, NFKB1, and PVT1.

36. A method for detecting cancer associated with expression of a polypeptide in a test cell sample, comprising the steps of: (i) detecting a level of expression of at least one polypeptide having an amino acid sequence encoded by a human coding sequence selected from the group consisting of SEQ ID NOS: 6, 12, 18, 24, 30 and 33 shown in Tables 1-6, or a fragment thereof; and (ii) comparing the level of expression of the polypeptide in the test sample with a level of expression of polypeptide in a normal cell sample, wherein an altered level of expression of the polypeptide in the test cell sample relative to the level of polypeptide expression in the normal cell sample is indicative of the presence of cancer in the test cell sample"

37. A method for detecting cancer associated with expression of a polypeptide in a test cell sample, comprising the steps of: (i) detecting a level of activity of at least one polypeptide having an amino acid sequence encoded by a human coding sequence selected from the group consisting of SEQ ID NOS: 6, 12, 18, 24, 30 and 33 shown in Tables 1-6, or a fragment thereof, wherein said activity corresponds to at least one activity for the polypeptide listed in Table 130; and (ii) comparing the level of activity of the polypeptide in the test sample with a level of activity of polypeptide in a normal cell sample, wherein an altered level of activity of the polypeptide in the test cell sample relative to the level of polypeptide activity in the normal cell sample is indicative of the presence of cancer in the test cell sample.

38. A method for detecting cancer associated with the presence of an antibody in a test serum sample, comprising the steps of: (i) detecting a level of an antibody against an antigenic polypeptide having an amino acid sequence encoded by a human coding sequence selected from the group consisting of SEQ ID NOS: 6, 12, 18, 24, 30 and 33 shown in Tables 1-6, or antigenic fragment thereof; and (ii) comparing said level of said antibody in the test sample with a level of said antibody in the control sample, wherein an altered level of antibody in said test sample relative to the level of antibody in the control sample is indicative of the presence of cancer in the test serum sample.

39. A method for screening for a bioactive agent capable of modulating the activity of a CA protein (CAP), wherein said CAP is encoded by a nucleic acid comprising a nucleic acid sequence selected from the group consisting of the polynucleotide sequences of SEQ ID NOS: 5, 11, 17, 23, 29 and 32 shown in Tables 1-6, said method comprising: a) combining said CAP and a candidate bioactive agent; and b) determining the effect of the candidate agent on the bioactivity of said CAP.

40. The method of screening for the bioactive agent according to clause 39, wherein the bioactive agent affects the expression of the CA protein (CAP).

41. The method of screening for the bioactive agent according to clause 39, wherein the bioactive agent affects the activity of the CA protein (CAP), wherein the CAP is selected from the group consisting of SNL, FOSB, CCND1, MYC, NFKB1, and PVT1.

42. A method for diagnosing cancer comprising: a) determining the expression of one or more genes comprising a nucleic acid sequence selected from the group consisting of the human sequences outlined in Tables 1-6, in a first tissue type of a first individual; and b) comparing said expression of said gene (s) from a second normal tissue type from said first individual or a second unaffected individual; wherein a difference in said expression indicates that the first individual has cancer.

43. The method for diagnosing cancers according to clause 42, wherein the difference in said expression indicates that the first individual has a propensity towards cancer.

44. The method for diagnosing cancers according to clause 42, wherein the gene comprises SNL1 sequences corresponding to SEQ ID NOS: 4, 5 and 6 and the tissue is breast cancer tissue.

45. The method for diagnosing cancers according to clause 42, wherein the gene comprises FOSB sequences corresponding to SEQ ID NOS: 10-12 and the tissue is selected from the group consisting of colon cancer, lung cancer, pancreatic cancer, ovarian cancer, stomach cancer, breast cancer and prostate cancer tissue.

46. The method for diagnosing cancers according to clause 42, wherein the gene comprises MYC sequences corresponding to SEQ ID NOS: 22-24 and the tissue is breast cancer tissue.

47. The method for diagnosing cancers according to clause 42, wherein the gene comprises an CCND1 sequences corresponding to SEQ ID NOS: 16-18 and the tissue is selected from the group consisting of colon cancer (sigmoid), colon cancer (transverse), lung cancer, ovarian cancer, and breast cancer tissue.

48. The method for diagnosing cancers according to clause 42, wherein the gene comprises an NFKB1 sequences corresponding to SEQ ID NOS: 28-30 and the tissue is selected from the group consisting of lung cancer, skin cancer, and breast cancer tissue.

49. The method for diagnosing cancers according to clause 42, wherein the gene comprises PVT1 sequences corresponding to SEQ ID NOS: 31-33 and the tissue is breast cancer tissue.

50. The method for diagnosing cancers according to clause 42, wherein the gene expression in the cancer tissue is up-regulated relative to the gene expression in the normal tissue.

51. The method for diagnosing cancers according to clause 50, wherein the difference in said expression indicates that the first individual has a propensity towards cancer.

52. A method for diagnosing cancer or a propensity towards cancer comprising determining the amplification of one or more genes comprising a DNA sequence selected from the group consisting of the human sequences outlined in Tables 1-6, in a first tissue type of a first individual relative to a second normal tissue type from said first individual or a second unaffected individual, wherein an amplification of the DNA indicates that the first individual has cancer or a propensity towards cancer.

53. The method for diagnosing cancer or a propensity towards cancer according to clause 52, wherein the gene comprises MYC and the DNA sequence is SEQ ID NO: 22, or a fragment thereof.

54. The method for diagnosing cancer or a propensity towards cancer according to clause 52, wherein the gene comprises PVT1 and the DNA sequence is SEQ ID NO: 31, or a fragment thereof.

55. A method for treating cancers comprising administering to a patient an inhibitor of a CA protein (CAP), wherein said CAP is encoded by a nucleic acid comprising a human nucleic acid sequence selected from the group consisting of the sequences outlined in Tables 1-6.

56. The method for treating cancers according to clause 55, wherein the inhibitor of a CA protein (CAP) binds to the CA protein.

57. The method for treating cancers according to clause 55, wherein the inhibitor of a CA protein (CAP) modulates the activity of a CAP sequence selected from the group consisting of SNL, FOSB, CCND1, MYC, NFKB1, and PVT1,

## Claims

1. A method of detecting a cancerous cell in a sample comprising detecting the presence of increased expression of a FOSB gene in a patient sample relative to expression of the FOSB gene in a control sample, wherein the presence of increased expression of the FOSB gene in said patient sample is indicative of a cancerous cell, said patient sample comprising colon, pancreas, ovary, stomach, breast, or prostate cells.

2. A method of detecting a cancerous cell comprising:

    a) measuring a level of FOSB gene expression in a first sample, said first sample comprising colon, pancreas, ovary, stomach, breast, or prostate tissue; and
    b) comparing the level of FOSB gene expression in (a) to the level of FOSB gene expression in a second sample, said second sample comprising a normal tissue type,

    wherein an increase in the level of FOSB gene expression of at least 50% in (a) relative to the level of FOSB gene expression in the second sample is indicative of a cancerous cell.

3. The method of claim 1 or 2 wherein FOSB gene expression is determined by measuring mRNA levels.

4. The method of claim 1 or 2 wherein gene expression is determined by measuring FOSB protein levels, said FOSB protein encoded by a polypeptide at least 95% identical to a sequence comprising SEQ ID NO : 11.

5. The method of claim 1 or 2 wherein the FOSB gene encodes nucleic acid sequence comprising SEQ ID NO : 11.

6. The method of claim 1 or 2 wherein the FOSB gene encodes a nucleotide sequence at least 98% identical to SEQ ID NO : 11, or a complement thereof.

7. The method of claim 1 or 2, wherein the FOSB gene encodes a nucleotide sequence at least 99% identical to SEQ ID NO:11, or a complement thereof.

8. The method of claim 1 or 2, wherein the FOSB gene encodes a nucleotide sequence comprising SEQ ID NO : 11, or a complement thereof.

9. A method for detecting colon cancer, breast cancer, ovarian cancer, pancreatic cancer, prostate cancer, or stomach cancer associated with expression of a polypeptide in a test cell sample, the method comprising:

    a) detecting a level of expression of a polypeptide encoded by a nucleotide sequence at least 95% identical to SEQ ID NO:11; and
    b) comparing the level of expression of the polypeptide in the test cell sample with a level of expression of the polypeptide in a normal cell sample;

wherein an increased level of expression of the polypeptide in the test cell sample relative to the level of polypeptide expression in the normal cell sample is indicative of the presence of colon cancer, breast cancer, ovarian cancer, pancreatic cancer, prostate cancer, or stomach cancer in the test cell sample.

**10.** A method for detecting colon cancer, breast cancer, ovarian cancer, pancreatic cancer, prostate cancer, or stomach cancer associated with expression of a polypeptide in a test cell sample, the method comprising:

a) detecting a level of activity of a polypeptide encoded by a nucleotide sequence at least 95% identical to SEQ ID NO : 11, or a fragment thereof; and
b) comparing the level of activity of the polypeptide in the test cell sample with a level of activity of the polypeptide in a normal cell sample;

wherein an increased level of activity of the polypeptide in the test cell sample relative to the level of polypeptide activity in the normal cell sample is indicative of the presence of colon cancer, breast cancer, ovarian cancer, pancreatic cancer, prostate cancer, or stomach cancer in the test cell sample.

**11.** A method for detecting colon cancer, breast cancer, ovarian cancer, pancreatic cancer, prostate cancer, or stomach cancer associated with the presence of an antibody in a test serum sample, the method comprising:

a) detecting a level of an antibody against an antigenic polypeptide encoded by a nucleotide sequence at least 95% identical to SEQ ID NO:11, or an antigenic fragment thereof; and
b) comparing said level of said antibody in the test sample with a level of said antibody in a control sample;

wherein an increased level of antibody in said test sample relative to the level of antibody in the control sample is indicative of the presence of colon cancer, breast cancer, ovarian cancer, pancreatic cancer, prostate cancer, or stomach cancer in the test serum sample.

**12.** A method for detecting a presence or an absence of colon cancer, breast cancer, ovarian cancer, pancreatic cancer, prostate cancer, or stomach cancer cells in a cell sample, the method comprising:

a) contacting cells in the sample with the antibody or antigen binding fragment thereof according to any one of claims 18 to 22 ex vivo; and
b) detecting a complex of the FOSB polypeptide from the cells and the antibody;

wherein detection of the complex correlates with the presence of colon cancer, breast cancer, ovarian cancer, pancreatic cancer, prostate cancer, or stomach cancer cells in the sample.

**13.** A method for diagnosing colon cancer, breast cancer, ovarian cancer, pancreatic cancer, prostate cancer, or stomach cancer ex vivo, the method comprising:

a) determining the expression of a gene encoding a nucleic acid sequence at least 95% identical to SEQ ID NO : 11 in a first tissue type of a first individual; and
b) comparing said expression of said gene from a second normal tissue type from said first individual or a second unaffected individual;

wherein an increase in said expression in (a) relative to the level of expression in the second normal tissue type indicates that the first individual has colon cancer, breast cancer, ovarian cancer, pancreatic cancer, prostate cancer, or stomach cancer.

**14.** A method for screening for a bioactive agent capable of modulating the activity of a FOSB protein, wherein said FOSB protein is encoded by a nucleic acid comprising a nucleic acid sequence at least 95% identical to SEQ ID NO : 11, said method comprising:

a) combining said FOSB protein and a candidate bioactive agent; and
b) determining the effect of the candidate agent on the bioactivity of said FOSB protein;

wherein a candidate bioactive agent which modulates the bioactivity of the FOSB protein is identified as a bioactive agent.

**15.** The method of screening for a bioactive agent according to claim 14, wherein the bioactive agent increases the expression of the FOSB protein.

**16.** A method of screening a candidate anti-cancer agent for anti-cancer activity comprising:

a) contacting a cell that expresses a FOSB gene with a candidate anti-cancer agent; and
b) detecting a difference of at least 50% between the level of FOSB gene expression in the cell in the presence and in the absence of the candidate anti-cancer agent,

wherein a decrease in the level of FOSB gene expression in the cell in the presence of the candidate anti-cancer agent of at least 50% relative to the level of FOSB gene expression in the cell in the absence of the candidate anti-cancer agent indicates that the candidate anti-cancer agent has anti-cancer activity; wherein the cancer is colon cancer, breast cancer, ovarian cancer, pancreatic cancer, prostate cancer, or stomach cancer.

**17.** The method of claim 16 wherein a decrease in the level of FOSB gene expression in the cell in the presence of the candidate anti-cancer agent of at least 100% relative to the level of FOSB gene expression in the cell in the absence of the candidate anti-cancer agent indicates that the candidate anti-cancer agent has anti-cancer activity.

**18.** The method of claim 16 wherein the candidate anti-cancer agent is an antibody, small organic compound, small inorganic compound, or polynucleotide.

**19.** The method of claim 16 wherein the candidate anti-cancer agent is a monoclonal antibody.

**20.** The method of claim 16 wherein the candidate anti-cancer agent is a human or humanized antibody.

**21.** The method of claim 18 wherein the polynucleotide is an antisense oligonucleotide.

**22.** A method for treating colon cancer, breast cancer, ovarian cancer, pancreatic cancer, prostate cancer, or stomach cancer, comprising administering to a patient an inhibitor of a FOSB protein, wherein said FOSB protein is encoded by a FOSB gene.

**23.** The method for treating cancer according to claim 22,
wherein the inhibitor of the FOSB protein binds to the FOSB protein.

**24.** Use of an inhibitor of a FOSB protein wherein said FOSB protein is encoded by a nucleic acid comprising a nucleic acid sequence at least 95% identical to SEQ ID NO:11 in the manufacture of a medicament for treating colon cancer, breast cancer, ovarian cancer, pancreatic cancer, prostate cancer, or stomach cancers.

**25.** The use of claim 24, wherein the inhibitor of the FOSB protein binds to the FOSB protein.

**26.** An isolated antibody or antigen binding fragment thereof, that specifically binds to a polypeptide comprising an amino acid encoded by a nucleotide sequence at least 95% identical to SEQ ID NO : 11.

**27.** The isolated antibody or antigen binding fragment thereof according to claim 26, wherein said antibody is a monoclonal antibody.

**28.** The isolated antibody or antigen binding fragment thereof according to claim 26, wherein said antibody is a polyclonal antibody.

**29.** The isolated antibody or antigen binding fragment thereof according to claim 26, wherein said antibody or fragment thereof further comprises a detectable label.

**30.** The isolated antibody or antigen binding fragment thereof according to claim 26, wherein said antibody or fragment thereof is linked to a therapeutic agent.

**31.** A hybridoma that produces the monoclonal antibody according to claim 27.

**32.** A pharmaceutical composition comprising the antibody or antigen binding fragment thereof according to any one of claims 26 to 30, and a pharmaceutically acceptable excipient.

**33.** A kit for detecting cancer cells comprising the antibody or antigen binding fragment thereof according to any one of claims 26 to 30, wherein the cancer is colon cancer, breast cancer, ovarian cancer, pancreatic cancer, prostate cancer, or stomach cancer.

**34.** A kit for diagnosing the presence of colon cancer, breast cancer, ovarian cancer, pancreatic cancer, prostate cancer, or stomach cancer in a test sample, said kit comprising at least one polynucleotide that selectively hybridizes to the sequence of a polynucleotide sequence comprising SEQ ID NO : 11, a fragment thereof, or its complement.

**35.** Use of the antibody or antigen binding fragment thereof according to any one of claims 26 to 30 in the manufacture of a medicament for detecting a presence or an absence of colon cancer, breast cancer, ovarian cancer, pancreatic cancer, prostate cancer, or stomach cancer cells in an individual.

**36.** Use of the antibody or antigen binding fragment thereof according to any one of claims 26 to 30 in the manufacture of a medicament for inhibiting growth of colon cancer, breast cancer, ovarian cancer, pancreatic cancer, prostate cancer, or stomach cancer cells in an individual.

**37.** Use of the antibody or antigen binding fragment thereof according to any one of claims 26 to 30 in the manufacture of a medicament for delivering a therapeutic agent to colon cancer, breast cancer, ovarian cancer, pancreatic cancer, prostate cancer, or stomach cancer cells in an individual.

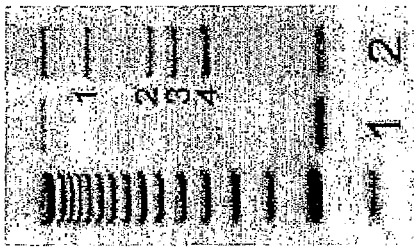


Figure 1

**Average Ct**

| Sample | Housekeeper | Target |
|---|---|---|
| Human Genomic_clontech | 23.40229767 | 24.718297 |
| Malignant mammary adenocarcinoma pleural effusion | 24.57459367 | 25.62105175 |
| Mammary ductal carcinoma piueral effusion | 23.935565 | 25.064095 |
| Mammary ductal carcinoma_human _45537 | 24.06496275 | 24.8361145 |
| Mammary ductal carcinoma_human_45523 | 24.286542 | 25.59857167 |
| Mammary ductal carcinoma_human_45535 | 24.271219 | 25.62112767 |
| transformed primary embryonal kidney_human | 24.136573 | 25.86051867 |

Figure 2.

**Target Normalized with Housekeeper**

| **Sample** | ΔCt | Stdev (ΔCt) |
|---|---|---|
| Human Genomic_clontech (**Calibrator**) | 1.69 | 0.22 |
| Malignant mammary adenocarcinoma pleural effusion | -1.85 | 0.14 |
| Mammary ductal carcinoma pluerai effusion | 1.12 | 0.24 |
| Mammary ductal carcinoma_human_45537 | 1.02 | 0.16 |
| Mammary ductal carcinoma_human_45523 | 0.49 | 0.26 |
| Mammary ductal carcinoma_human_45535 | 1.03 | 0.18 |
| transformed primary embryonal kidney_human | 1.81 | 0.15 |

**Figure 3.**

**Target Normalized with Housekeeper**

| **Sample** | ΔCt | Stdev (ΔCt) | ΔΔCt | Stdev (ΔΔCt) | Comparative expression level |
|---|---|---|---|---|---|
| Human Genomic_clontech (**Calibrator**) | 1.69 | 0.22 | 0 | 0.22 | 1 |
| Malignant mammary adenocarcinoma pleural effusion | -1.85 | 0.14 | -3.54 | 0.14 | 11.60 |
| Mammary ductal carcinoma pluerai effusion | 1.12 | 0.24 | -0.57 | 0.24 | 1.48 |
| Mammary ductal carcinoma_human _45537 | 1.02 | 0.16 | -0.67 | 0.16 | 1.59 |
| Mammary ductal carcinoma_human_45523 | 0.49 | 0.26 | -1.20 | 0.26 | 2.30 |
| Mammary ductal carcinoma_human_45535 | 1.03 | 0.18 | -0.66 | 0.18 | 1.58 |
| transformed primary embryonal kidney_human | 1.81 | 0.15 | 0.12 | 0.15 | 0.92 |

**Figure 4.**

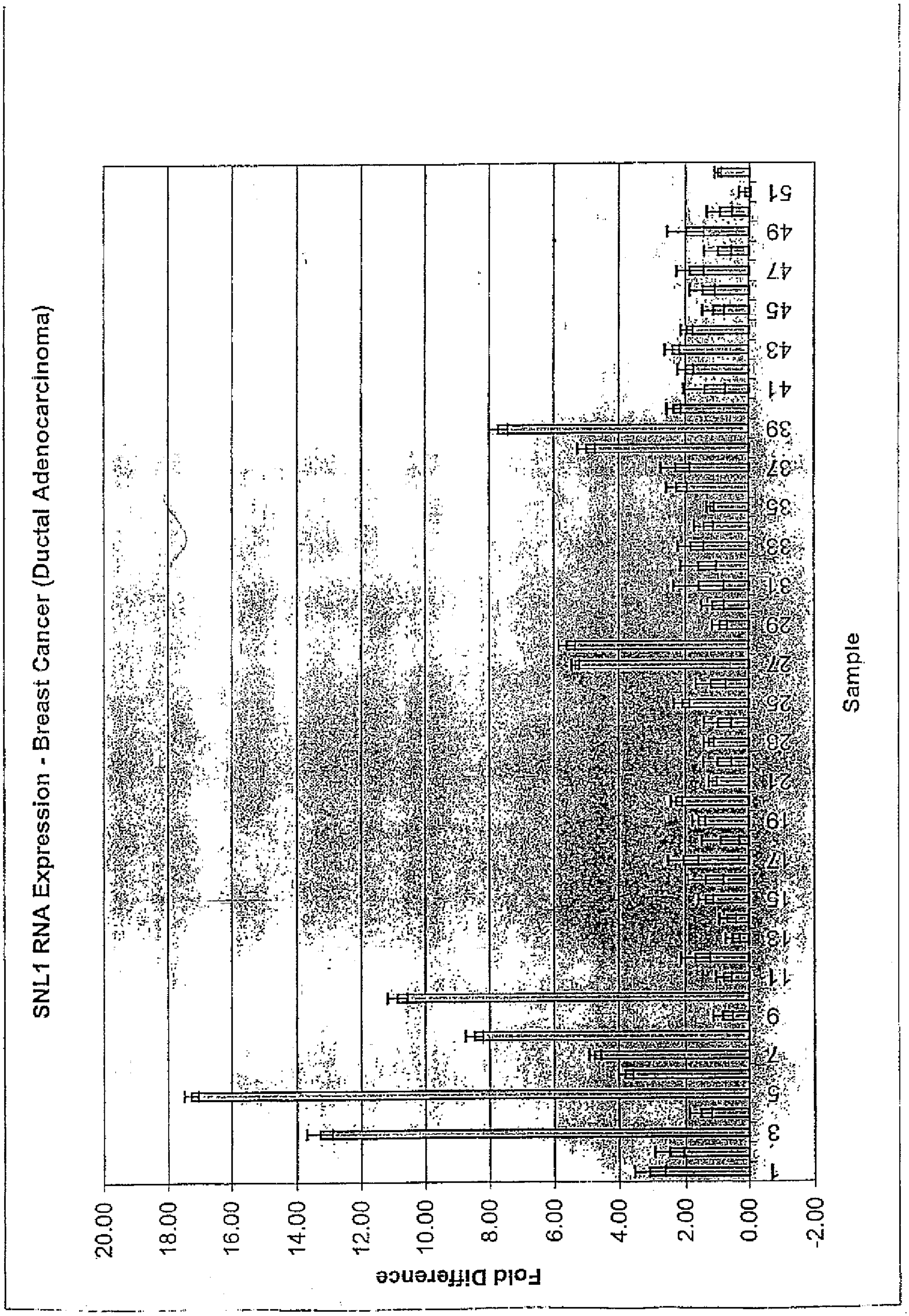
SNL1 RNA Expression - Breast Cancer (Ductal Adenocarcinoma)
Fold Difference
20.00
18.00
16.00
14.00
12.00
10.00
8.00
6.00
4.00
2.00
0.00
-2.00
Sample
1
3
5
7
9
11
13
15
17
19
21
23
25
27
29
31
33
35
37
39
41
43
45
47
49
51

FIGURE 5

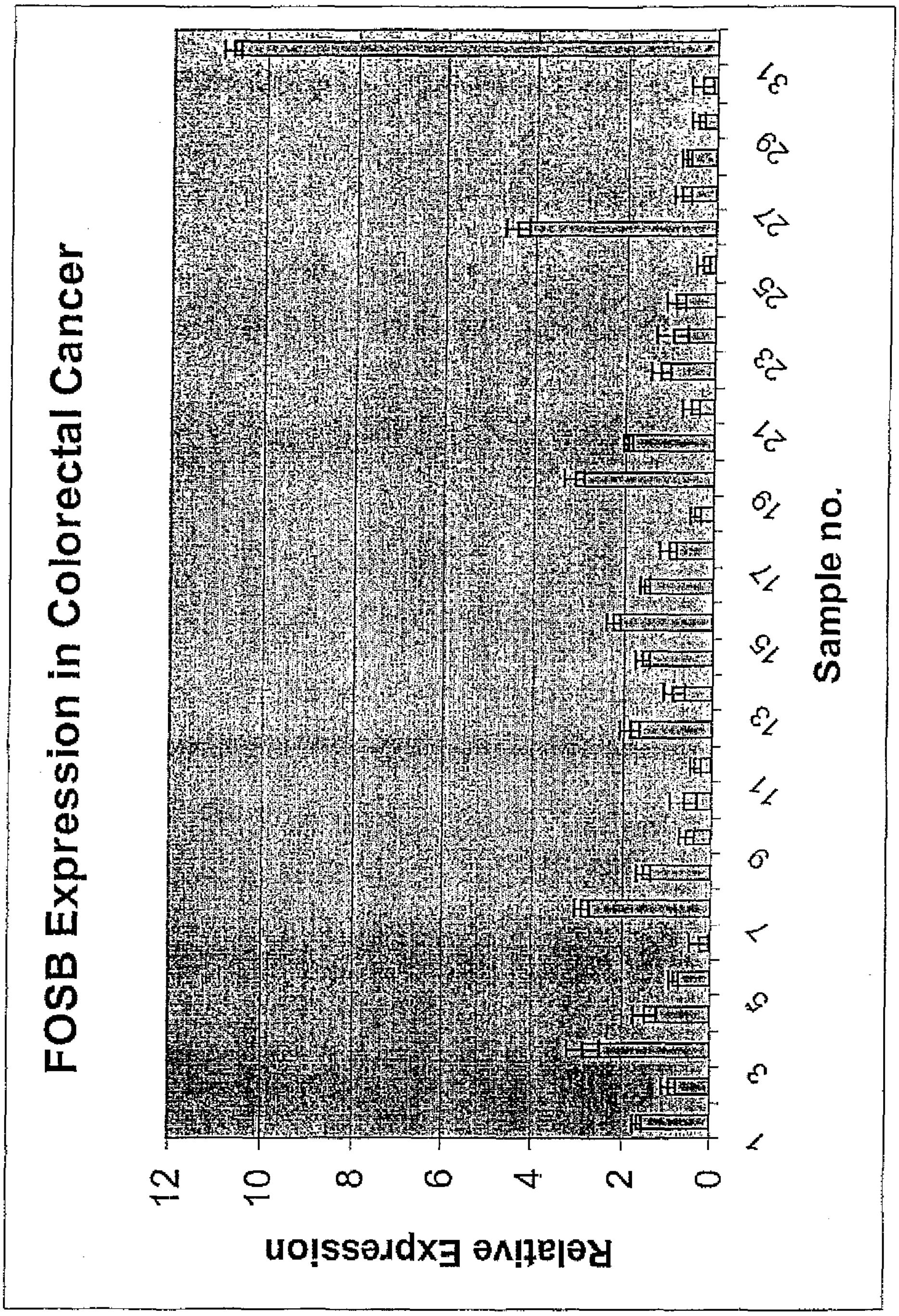
FOSB Expression in Colorectal Cancer
Relative Expression
12
10
8
6
4
2
0
1
3
5
7
9
11
13
15
17
19
21
23
25
27
29
31
Sample no.

Figure 6

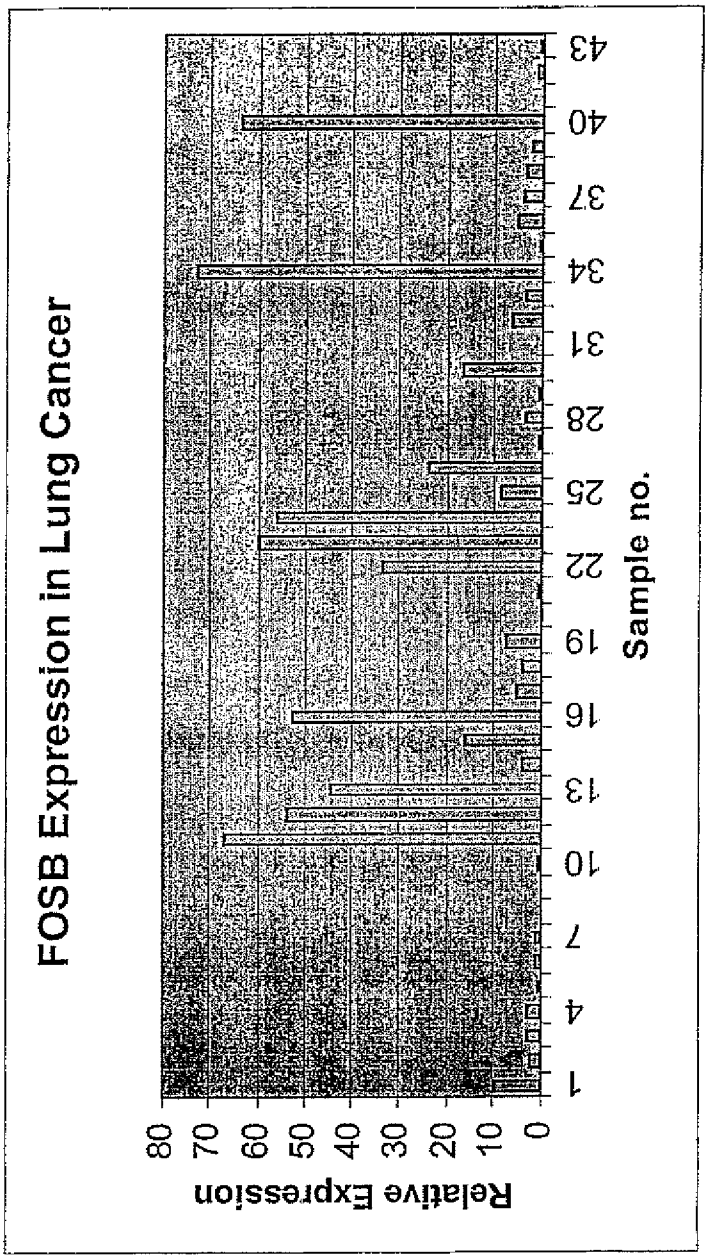
FOSB Expression in Lung Cancer
Relative Expression
0
10
20
30
40
50
60
70
80
1
4
7
10
13
16
19
22
25
28
31
34
37
40
43
Sample no.

Figure 7

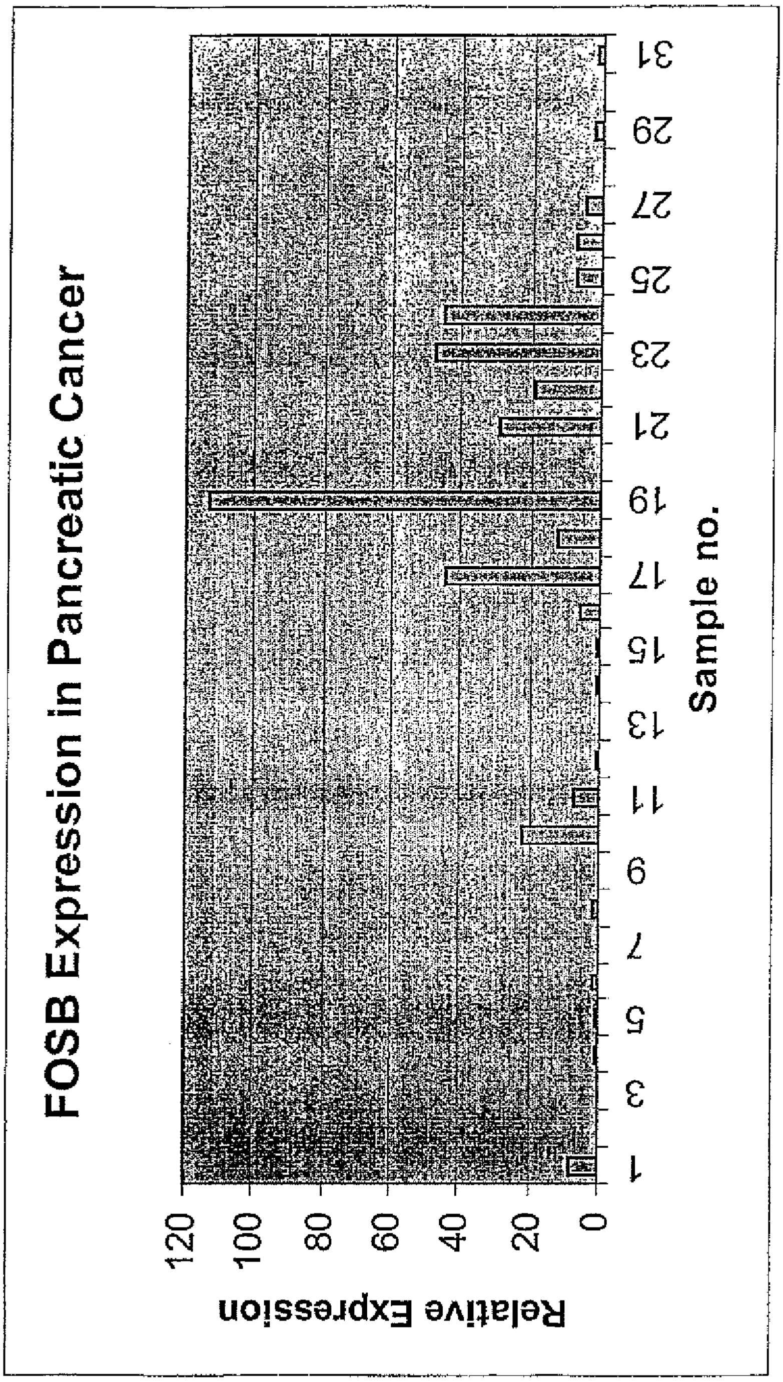
FOSB Expression in Pancreatic Cancer
Relative Expression
0
20
40
60
80
100
120
Sample no.
1
3
5
7
9
11
13
15
17
19
21
23
25
27
29
31

Figure 8

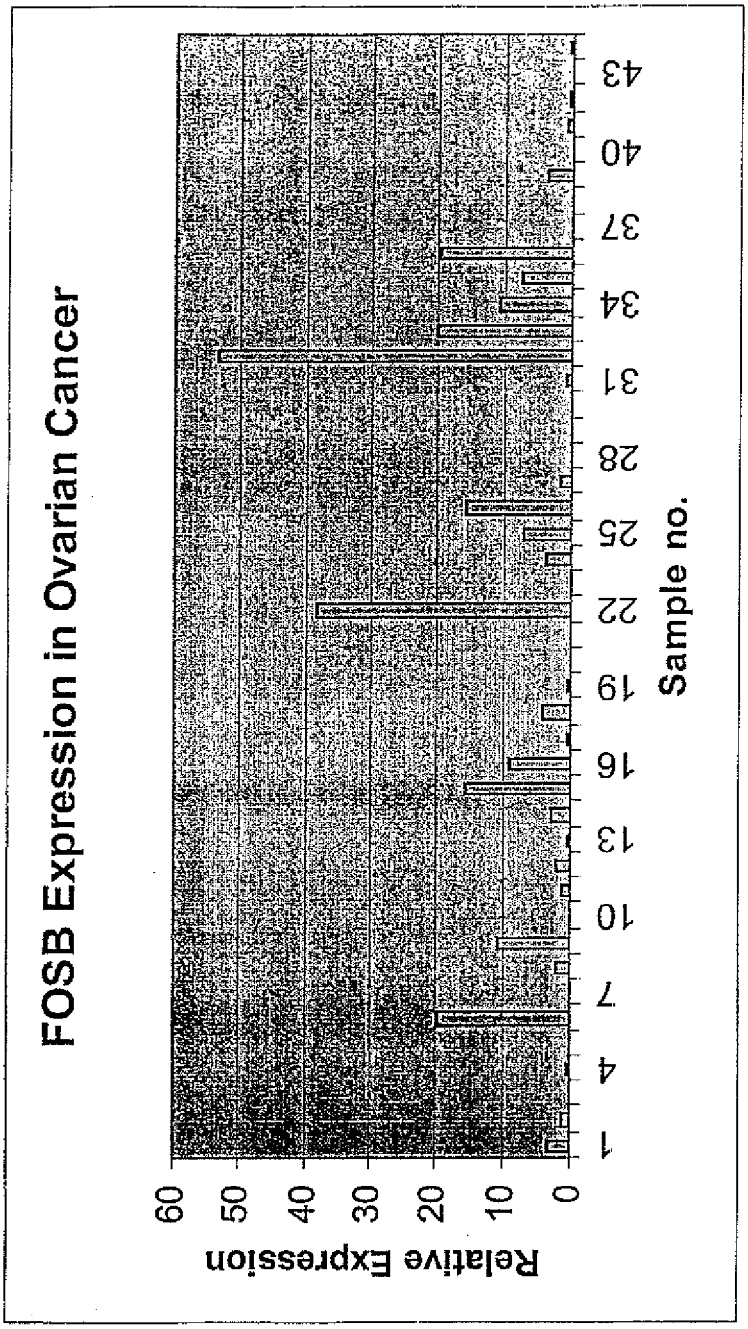
FOSB Expression in Ovarian Cancer
Relative Expression
60
50
40
30
20
10
0
1
4
7
10
13
16
19
22
25
28
31
34
37
40
43
Sample no.

Figure 9

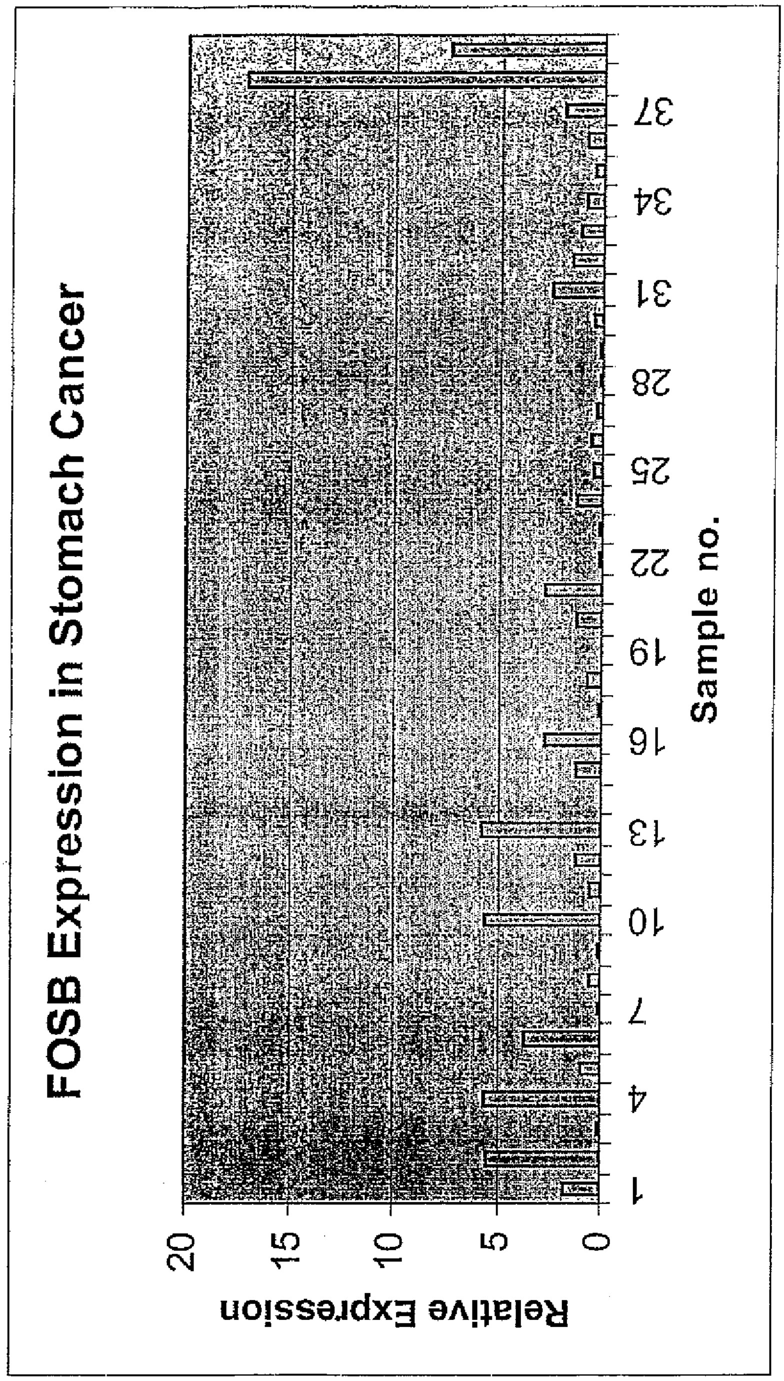
FOSB Expression in Stomach Cancer
Relative Expression
20
15
10
5
0
1
4
7
10
13
16
19
22
25
28
31
34
37
Sample no.

Figure 10

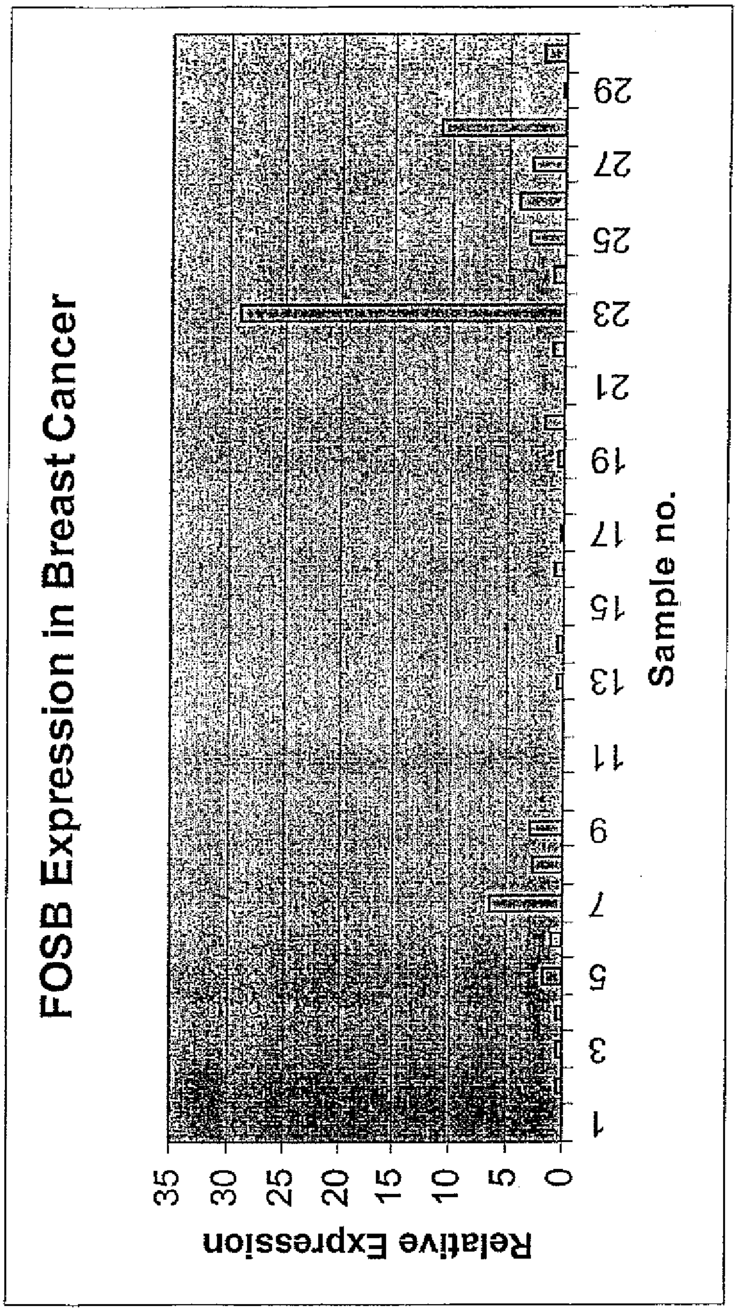
FOSB Expression in Breast Cancer
Relative Expression
35
30
25
20
15
10
5
0
1
3
5
7
9
11
13
15
17
19
21
23
25
27
29
Sample no.

Figure 11

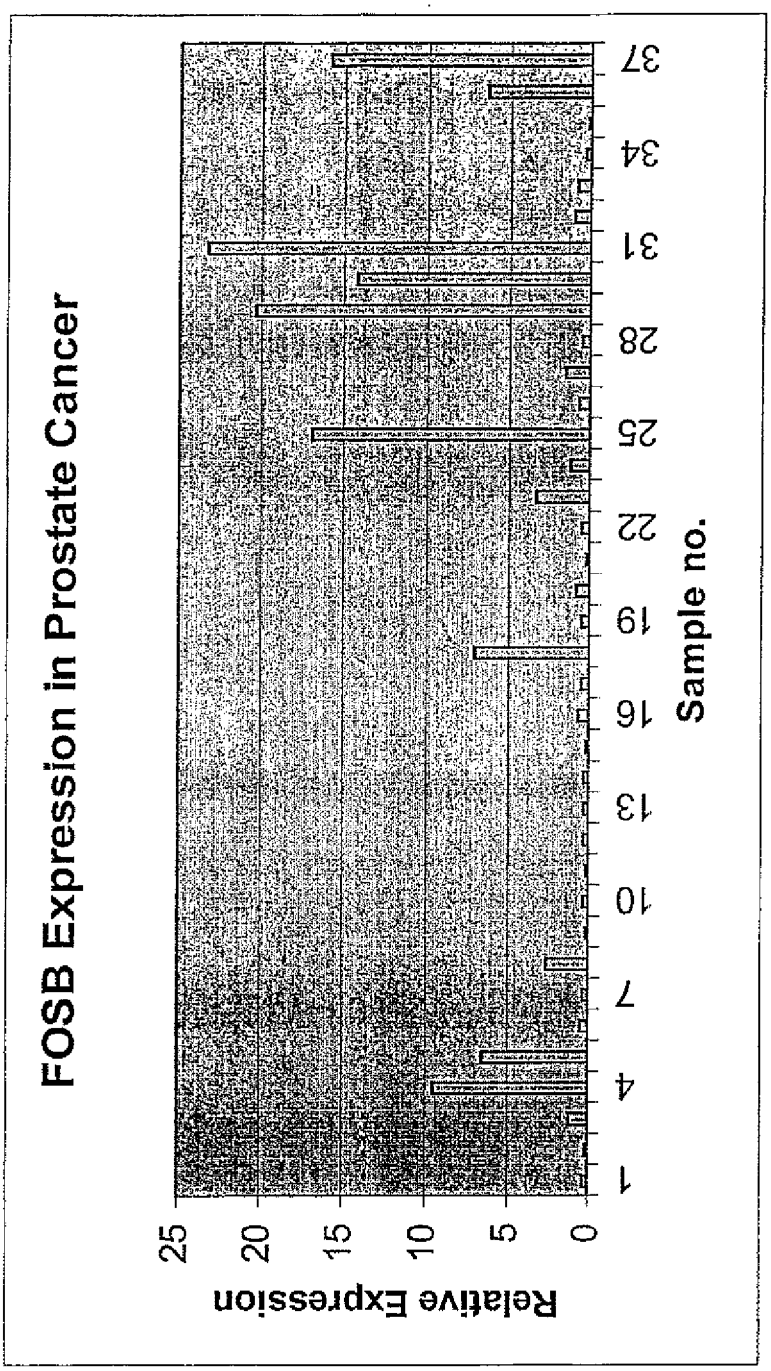
FOSB Expression in Prostate Cancer
Relative Expression
25
20
15
10
5
0
1
4
7
10
13
16
19
22
25
28
31
34
37
Sample no.

Figure 12

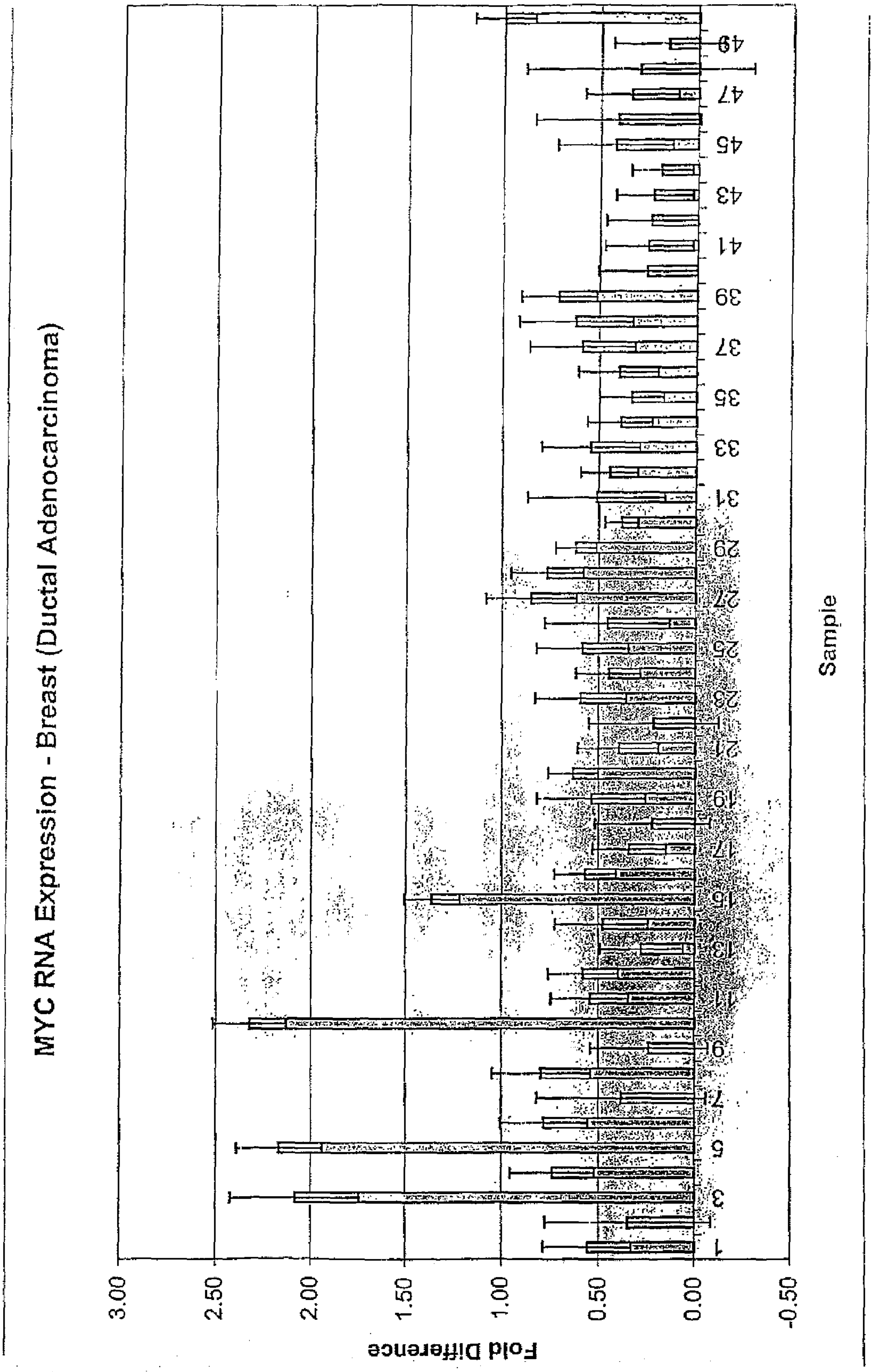
MYC RNA Expression - Breast (Ductal Adenocarcinoma)
Fold Difference
Sample
3.00
2.50
2.00
1.50
1.00
0.50
0.00
-0.50
1
3
5
7
9
11
13
15
17
19
21
23
25
27
29
31
33
35
37
39
41
43
45
47
49

FIGURE 13

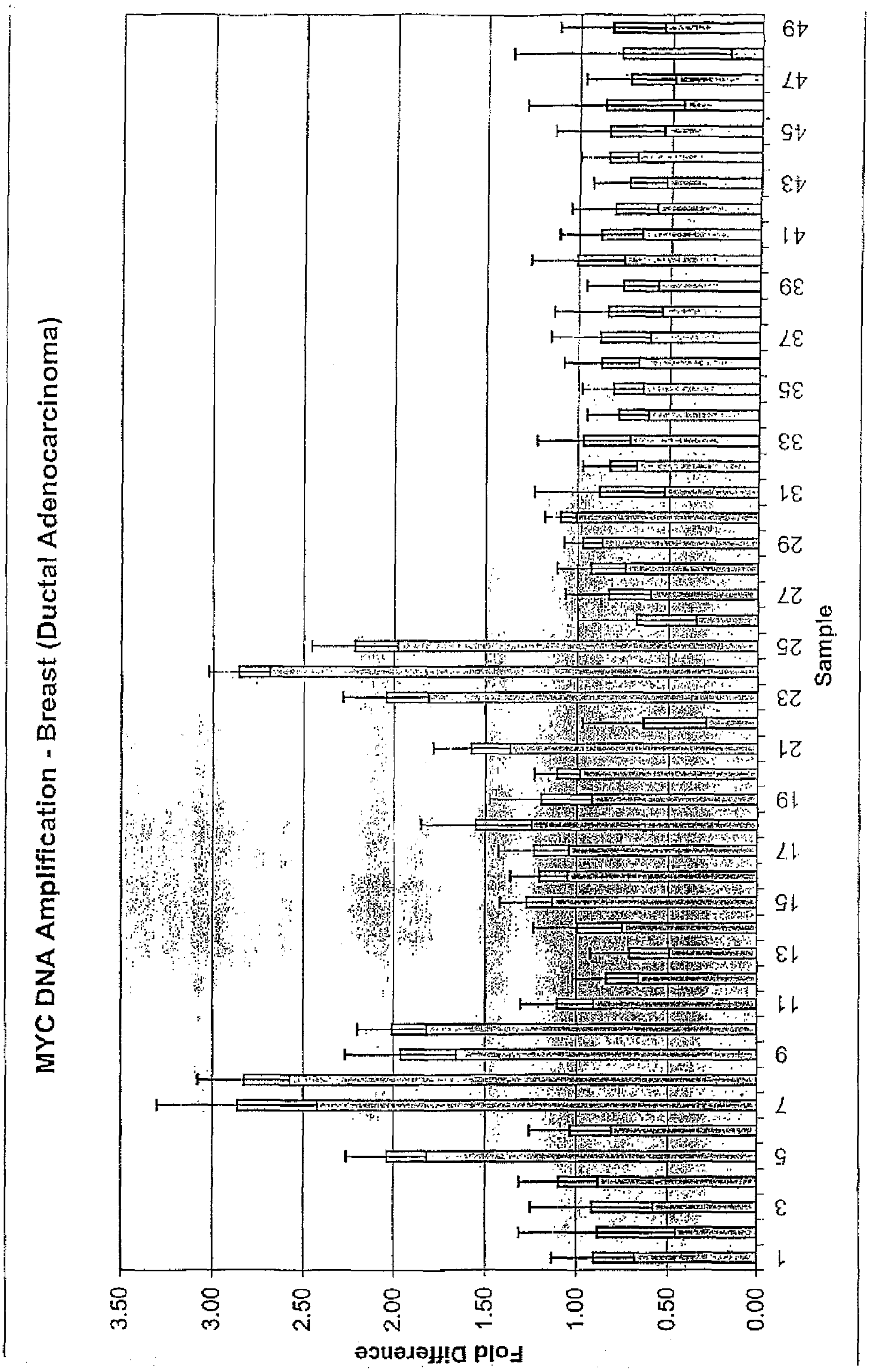
MYC DNA Amplification - Breast (Ductal Adenocarcinoma)
Fold Difference
0.00
0.50
1.00
1.50
2.00
2.50
3.00
3.50
Sample
1
3
5
7
9
11
13
15
17
19
21
23
25
27
29
31
33
35
37
39
41
43
45
47
49

FIGURE 14

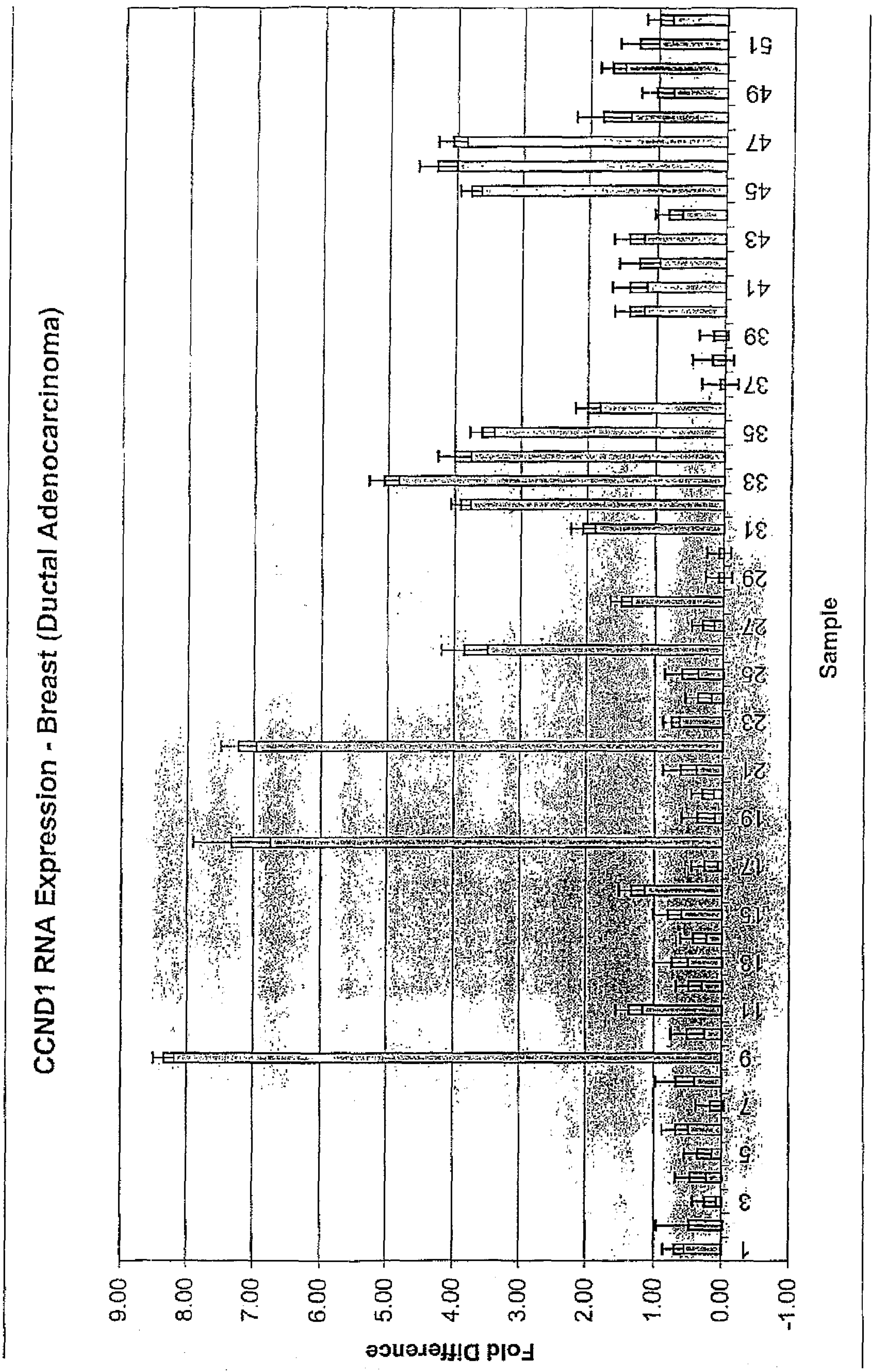
CCND1 RNA Expression - Breast (Ductal Adenocarcinoma)
Fold Difference
9.00
8.00
7.00
6.00
5.00
4.00
3.00
2.00
1.00
0.00
-1.00
1
3
5
7
9
11
13
15
17
19
21
23
25
27
29
31
33
35
37
39
41
43
45
47
49
51
Sample

FIGURE 15

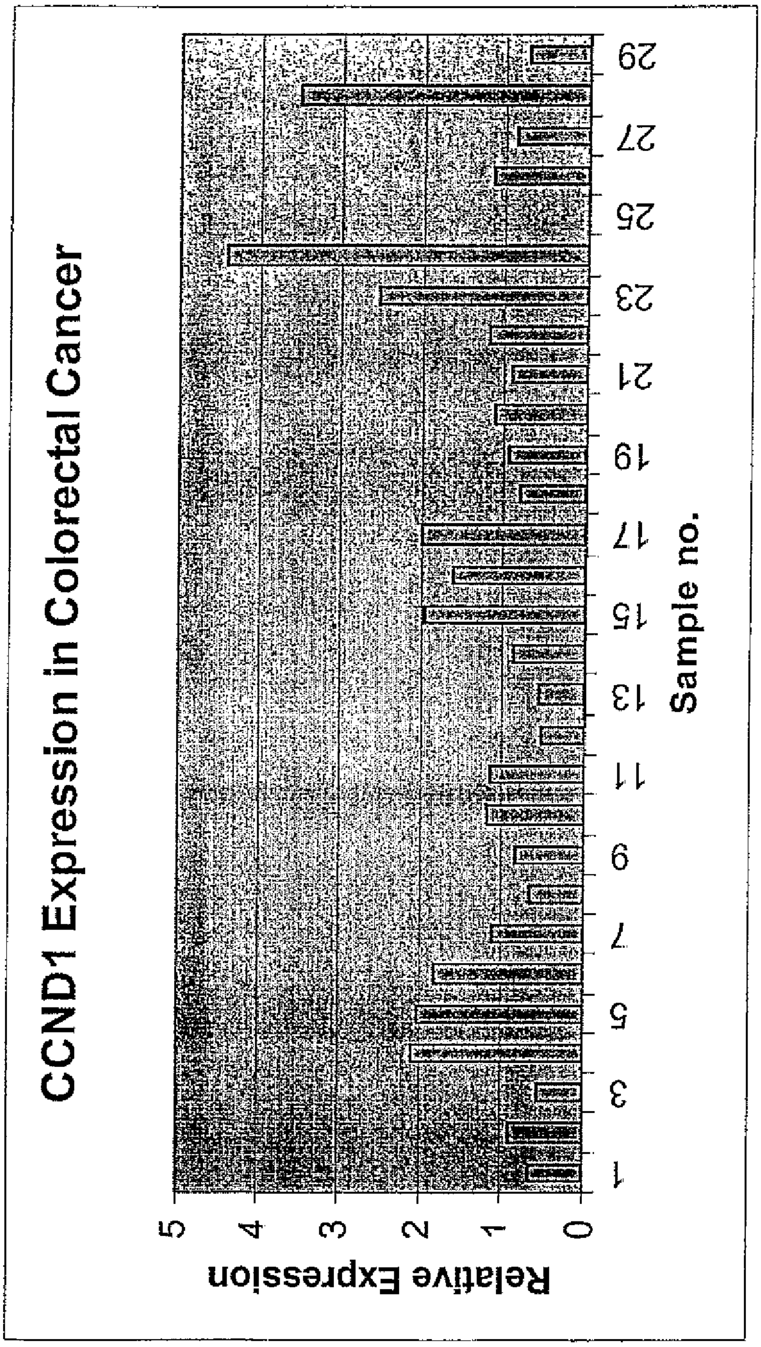
CCND1 Expression in Colorectal Cancer
Relative Expression
0
1
2
3
4
5
Sample no.
1
3
5
7
9
11
13
15
17
19
21
23
25
27
29

Figure 16

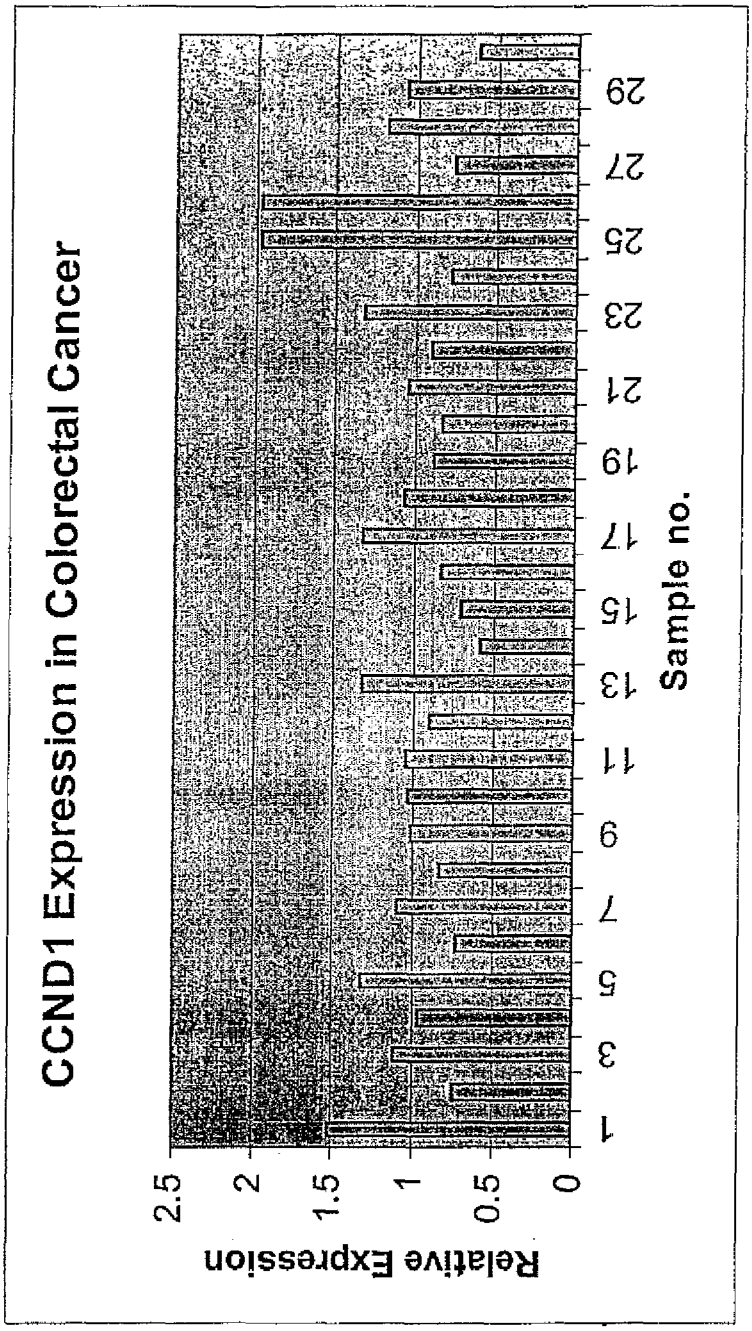
CCND1 Expression in Colorectal Cancer
Relative Expression
2.5
2
1.5
1
0.5
0
1
3
5
7
9
11
13
15
17
19
21
23
25
27
29
Sample no.

Figure 17

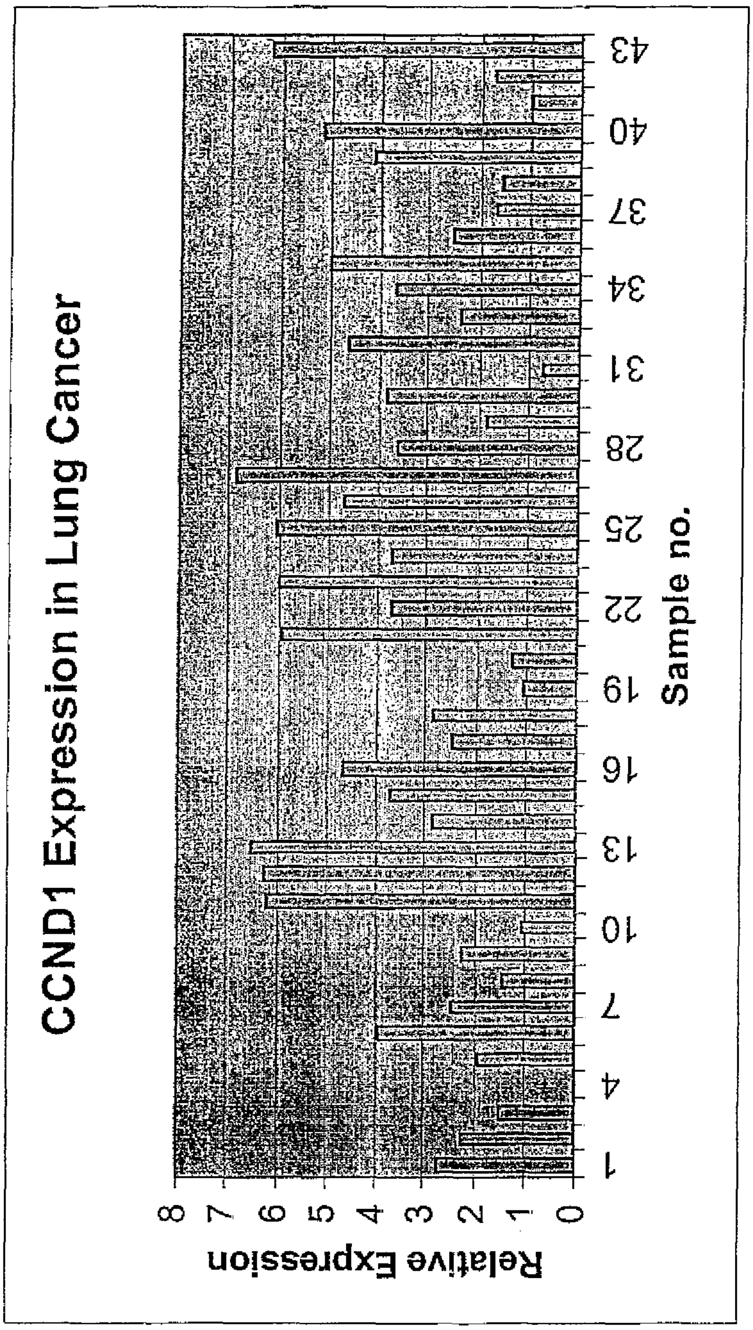
CCND1 Expression in Lung Cancer
Relative Expression
0 1 2 3 4 5 6 7 8
Sample no.
1 4 7 10 13 16 19 22 25 28 31 34 37 40 43

Figure18

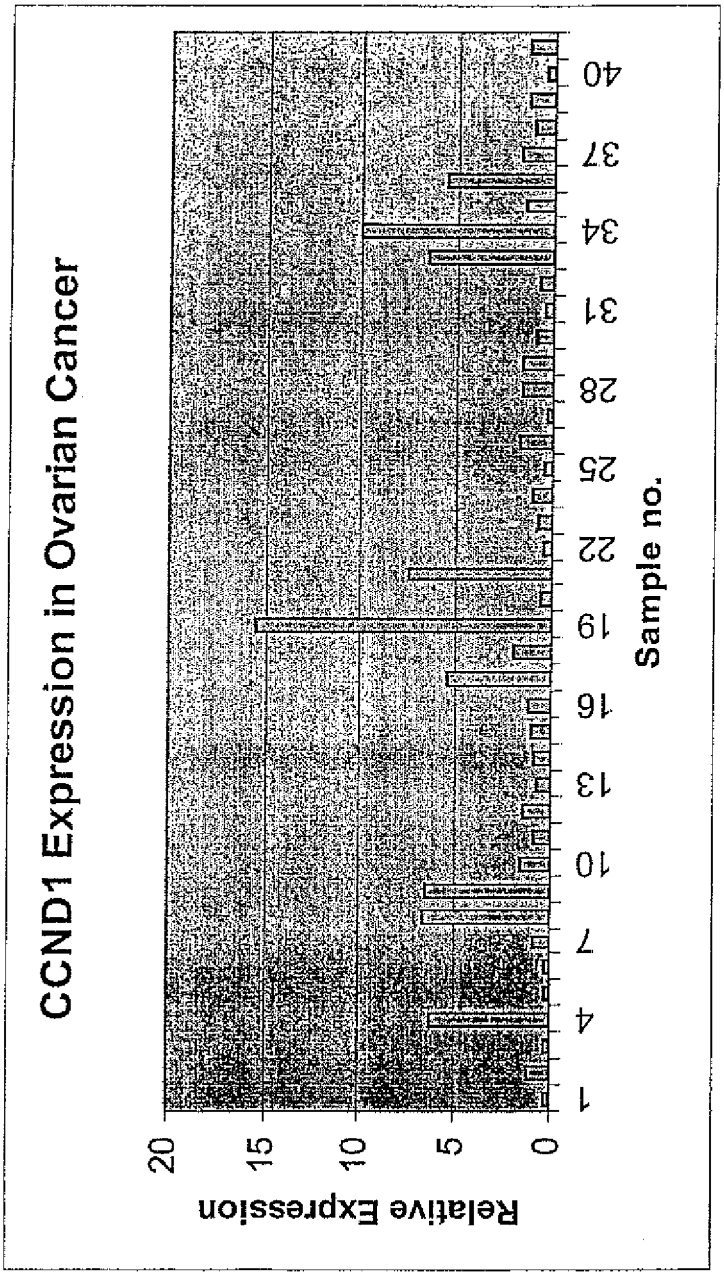
CCND1 Expression in Ovarian Cancer
Relative Expression
20
15
10
5
0
1
4
7
10
13
16
19
22
25
28
31
34
37
40
Sample no.

Figure 19

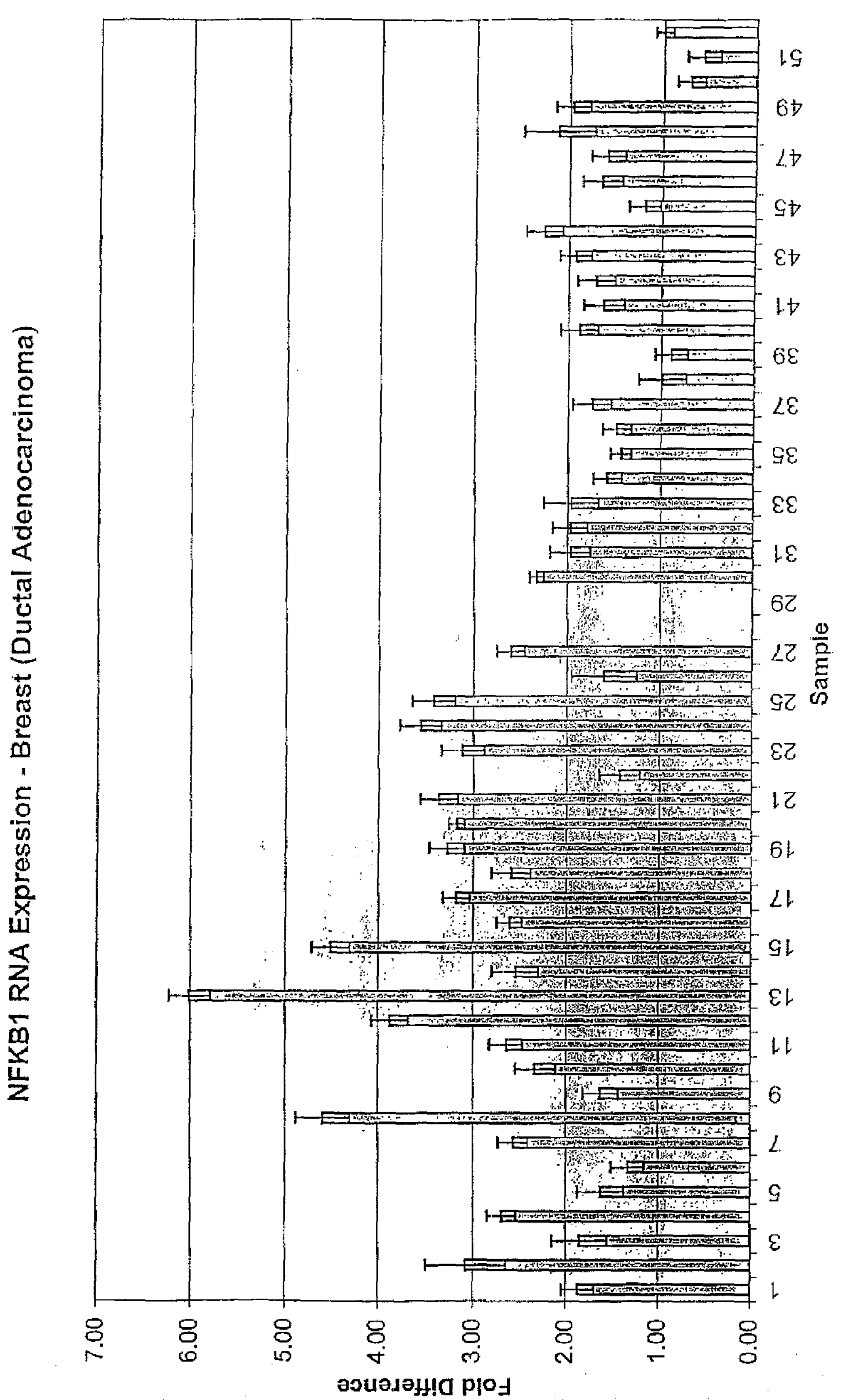
NFKB1 RNA Expression - Breast (Ductal Adenocarcinoma)
Fold Difference
0.00
1.00
2.00
3.00
4.00
5.00
6.00
7.00
Sample
1
3
5
7
9
11
13
15
17
19
21
23
25
27
29
31
33
35
37
39
41
43
45
47
49
51

FIGURE 20

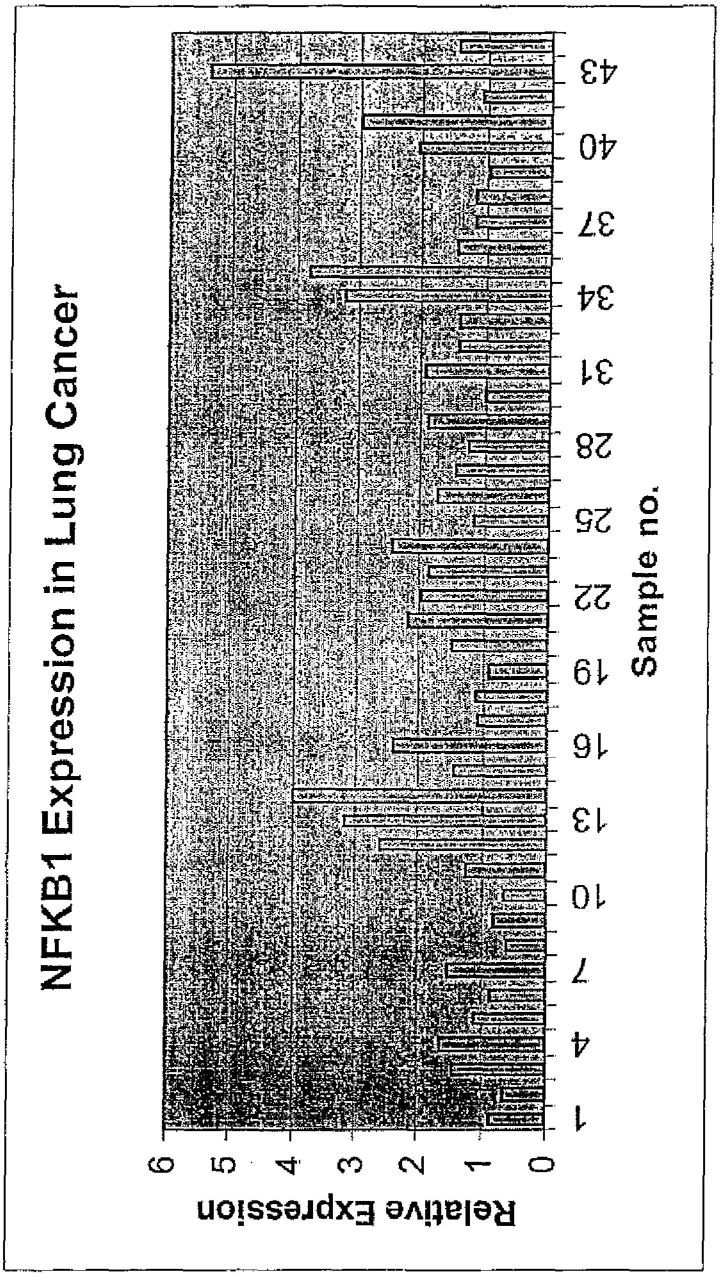
NFKB1 Expression in Lung Cancer
Relative Expression
0
1
2
3
4
5
6
Sample no.
1
4
7
10
13
16
19
22
25
28
31
34
37
40
43

Figure 21

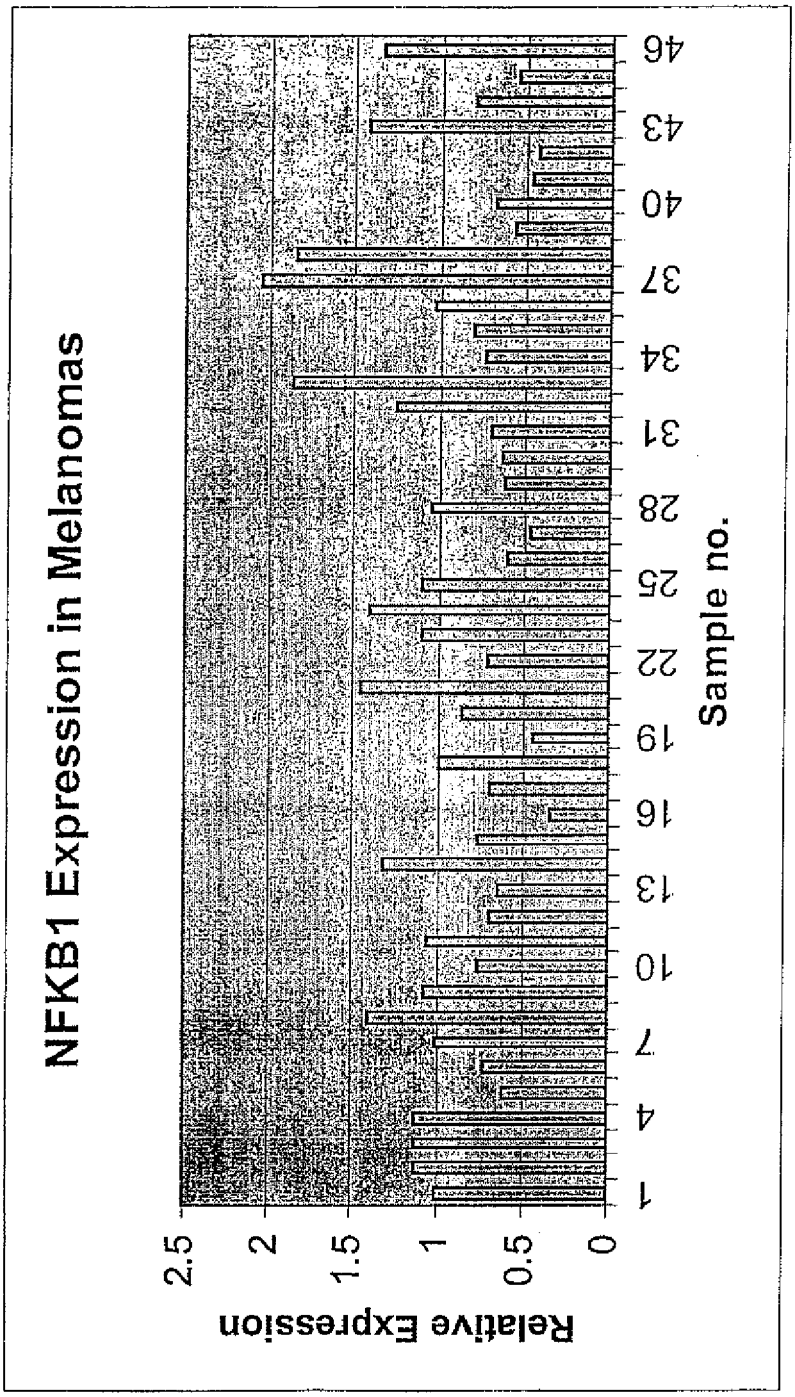
NFKB1 Expression in Melanomas
Relative Expression
2.5
2
1.5
1
0.5
0
1
4
7
10
13
16
19
22
25
28
31
34
37
40
43
46
Sample no.

Figure 22

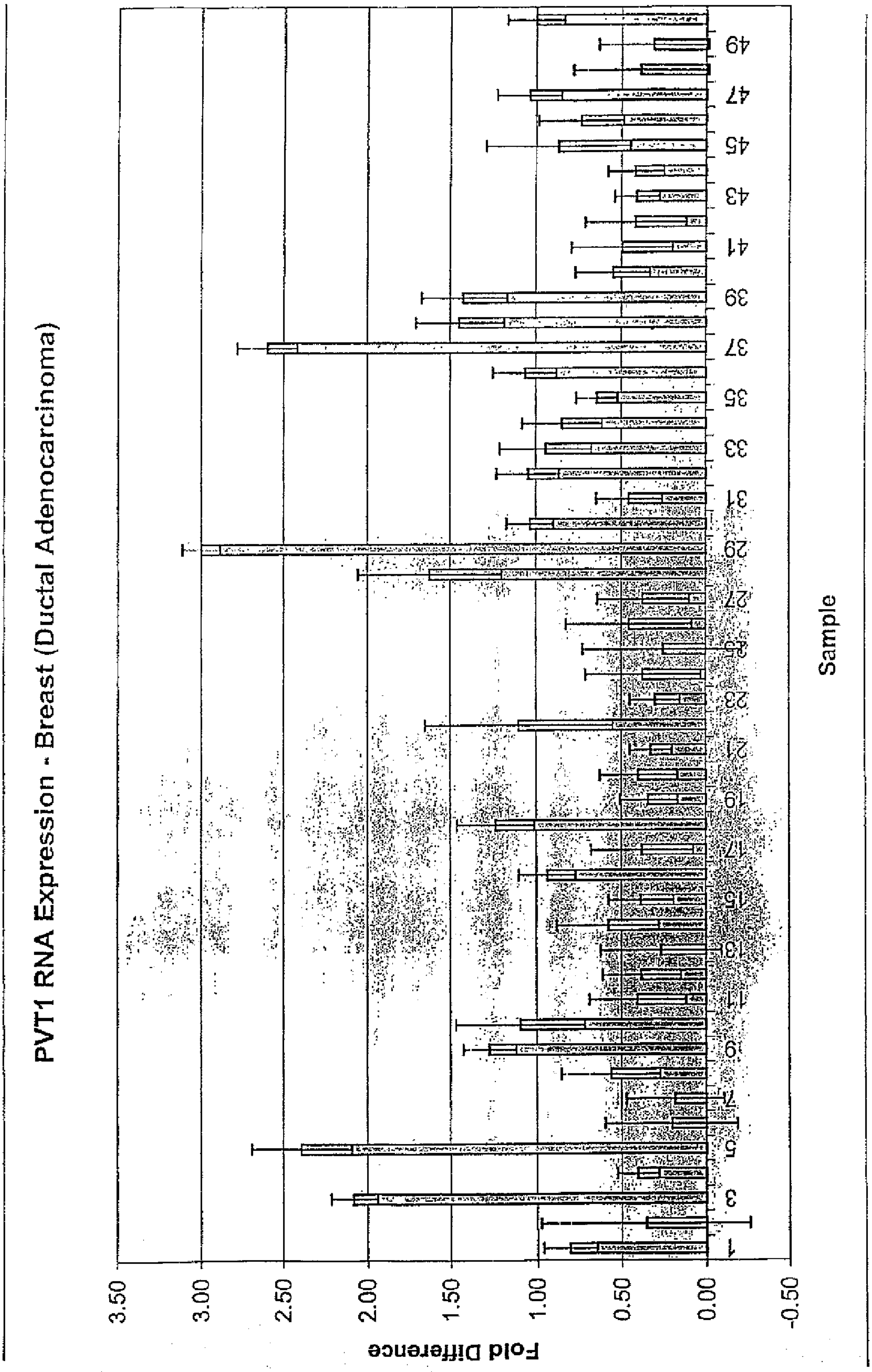
PVT1 RNA Expression - Breast (Ductal Adenocarcinoma)
Fold Difference
3.50
3.00
2.50
2.00
1.50
1.00
0.50
0.00
-0.50
Sample
1
3
5
7
9
11
13
15
17
19
21
23
25
27
29
31
33
35
37
39
41
43
45
47
49

FIGURE 23

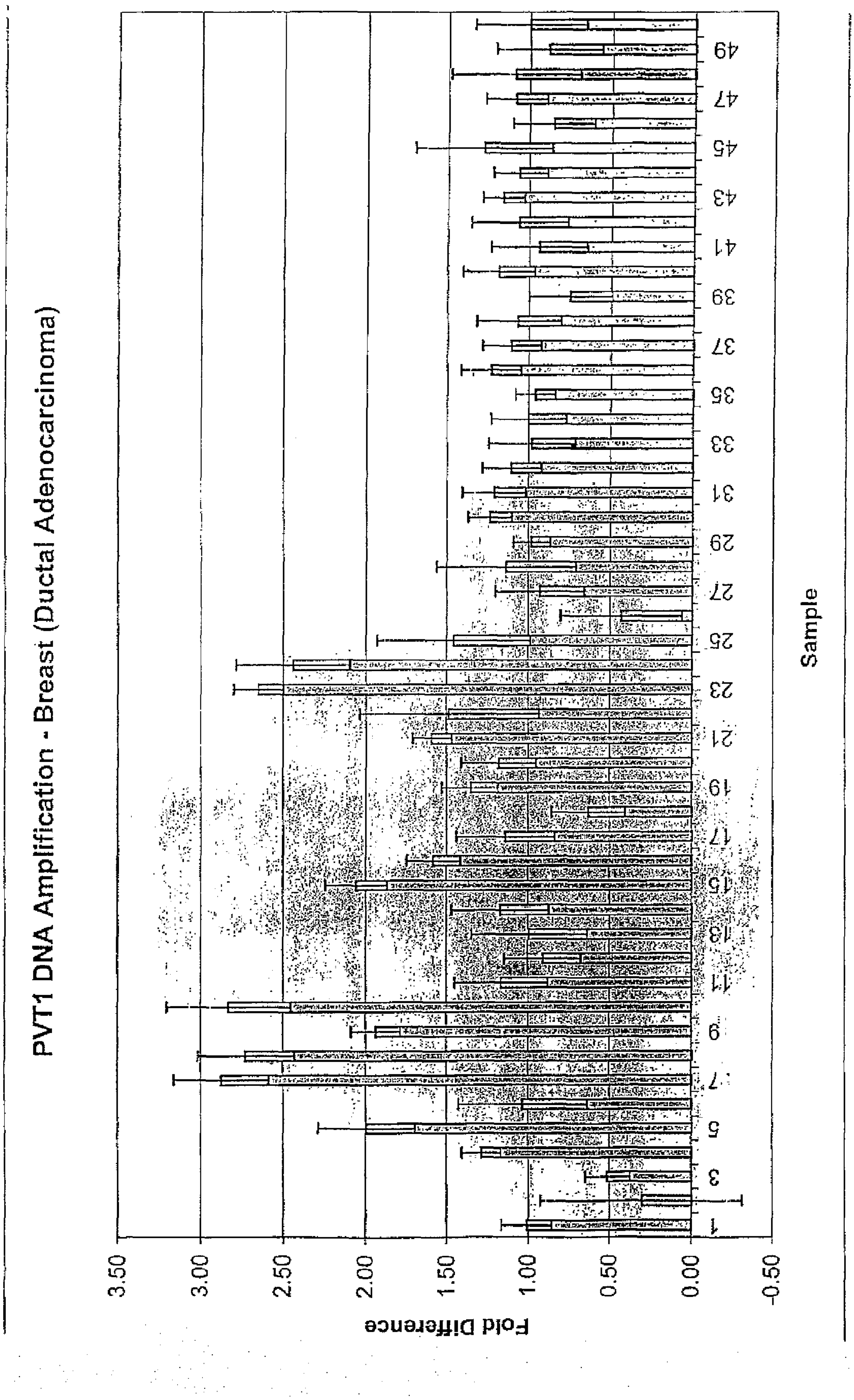
PVT1 DNA Amplification - Breast (Ductal Adenocarcinoma)
Fold Difference
3.50
3.00
2.50
2.00
1.50
1.00
0.50
0.00
-0.50
Sample
1
3
5
7
9
11
13
15
17
19
21
23
25
27
29
31
33
35
37
39
41
43
45
47
49

FIGURE 24

Europäisches Patentamt
European Patent Office
Office européen des brevets

# EUROPEAN SEARCH REPORT

Application Number
EP 08 17 0786

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | BAMBERGER ANA-MARIA ET AL: "Expression pattern of the AP-1 family in breast cancer: Association of fosB expression with a well-differentiated, receptor-positive tumor phenotype" INTERNATIONAL JOURNAL OF CANCER, vol. 84, no. 5, 22 October 1999 (1999-10-22), pages 533-538, XP002348444 ISSN: 0020-7136 * abstract * ----- | 1-13 | INV. C07H21/04 C12N15/63 C07H15/00 C12Q1/68 |
| A | "Santa Cruz Biotechnology, Inc Product Catalogue: Fos B (C-11): sc-8013" 2000, SANTA CRUZ BIOTECHNOLOGY, INC , XP002521662 * page 107 * -& ANONYMOUS: "Fos B (C-11): sc-8013" SANTA CRUZ BIOTECHNOLOGY CATALOGUE DATASHEET, [Online] 2009, XP002525552 Retrieved from the Internet: URL:http://datasheets.scbt.com/sc-8013.pdf > [retrieved on 2009-03-03] * the whole document * ----- | 1-13 | TECHNICAL FIELDS SEARCHED (IPC) C07K |
| A | ADAMKIEWICZ J ET AL: "MAPPING OF FUNCTIONAL DOMAINS IN FOS AND JUN PROTEINS USING EPITOPE-SPECIFIC ANTIBODIES" ONCOGENE, vol. 5, no. 4, 1990, pages 525-534, XP009114365 ISSN: 0950-9232 * abstract; table 1 * * page 526, left-hand column, paragraph 2 * ----- | 1-13 | |

~~The present search report has been drawn up for all claims~~

| Place of search | Date of completion of the search | Examiner |
|---|---|---|
| Munich | 28 April 2009 | Strobel, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

6

EPO FORM 1503 03.82 (P04C01)

Europäisches Patentamt
European Patent Office
Office européen des brevets

Application Number
EP 08 17 0786

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

see annex

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION**
**SHEET B**

Application Number
EP 08 17 0786

The Search Division considers that the present European patent application does not comply with the requirements of unity of invention and relates to several inventions or groups of inventions, namely:

1. claims: 1-13

A method for detecting a cancerous cell/for detecting colon cancer, breast cancer, pancreatic cancer, prostate cancer, ovarian cancer or stomach cancer comprising the detection of FosB expression/activity or fosb gene expression in a sample.

---

2. claims: 14-25,33-37

methods for screening/treatment of colon cancer, stomach cancer, ovarian cancer, pancreatic cancer, breast cancer or prostate cancer involving detecting expression/activity of FosB or fosb gene product, or involving modulating FosB protein bioactivity; second medical uses of an anti-FosB antibody

---

3. claims: 26-32

an anti-FosB antibody

---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5FLY357025 A
- US 09747377 B
- US 09798586 B
- US 10004113 B
- US 10052482 B
- US 09997722 B
- US 5644048 A
- US 5386023 A
- US 5637684 A
- US 5602240 A
- US 5216141 A
- US 4469863 A
- US 5235033 A
- US 5034506 A
- US 5837832 A, Chee
- US 5470967 A
- US 5714331 A
- EP 0225807 A
- US 5419966 A
- WO 9525116 A
- WO 9535505 A
- US 5445934 A
- US 5384261 A
- US 5700637 A
- US 9701019 W
- US 9701048 W
- US PN4959314 A
- WO 8705330 A
- US 4640835 A
- US 4496689 A
- US 4301144 A
- US 4670417 A
- US 4791192 A
- US 4179337 A
- US 4753894 A, Frankel
- US 4938948 A, Ring
- US 4956453 A, Bjorn
- EP 519596 A
- US 4816567 A
- US 815SG7 A
- US 5545807 A
- US 5545806 A
- US 5569825 A
- US 5625126 A
- US 5633425 A
- US 5661016 A
- US 913242 A, Kabat
- US 5530101 A
- US 5585089 A
- WO 9824893 A
- WO 9110741 A
- WO 9630498 A
- WO 9402602 A
- US 5939598 A
- WO 9633735 A
- US 5681702 A
- US 5597909 A
- US 5545730 A
- US 5594117 A
- US 5591584 A
- US 5571670 A
- US 5580731 A
- US 5624802 A
- US 5635352 A
- US 5594118 A
- US 5359100 A
- US 5124246 A
- US 5681697 A
- US 9701019 B
- WO 9104753 A
- WO 9010448 A
- WO 9314778 A
- WO 9007936 A
- WO 9403622 A
- WO 9325698 A
- WO 9325234 A
- US PN5219740 A
- WO 9311230 A
- WO 9310218 A
- US PN4777127 A
- GB 2200651 A
- EP 0345242 A
- WO 9102805 A
- WO 9412649 A
- WO 9303769 A
- WO 9319191 A
- WO 9428938 A
- WO 9511984 A
- WO 9500655 A
- US PN5814482 A
- WO 9507994 A
- WO 9617072 A
- WO 9530763 A
- WO 9742338 A
- WO 9011092 A
- US PN5580859 A
- US PN5422120 A
- WO 9513796 A

- WO 9423697 A
- WO 9114445 A
- EP 0524968 A
- US PN5206152 A
- WO 9211033 A
- US PN5149655 A
- US 9303868 B
- WO 9202526 A
- US PN5124246 A
- US PN4683195 A
- US PN4683202 A
- WO 0143869 A

**Non-patent literature cited in the description**


- **SORENSEN et al.** *J of Virology,* 2000, vol. 74, 2161
- **HANSEN et al.** *Genome Res.,* 2000, vol. 10 (2), 237-43
- **SORENSEN et al.** *J. Virology,* 1996, vol. 70, 4063
- **SORENSEN et al.** *J. Virology,* 1993, vol. 67, 7118
- **JOOSTEN et al.** *Virology,* 2000, vol. 265, 308
- **LI et al.** *Nature Genetics,* 1999, vol. 23, 348
- Mammary Tumors in the Mouse. Elsevier/North- Holland Biomedical Press
- **MARSHALL.** *Cell,* 1991, vol. 64, 313-326
- **WEINBERG.** *Science,* 1991, vol. 254, 1138-1146
- **KANE.** *J. Cell Biol,* 1975, vol. 66, 305-315
- **OTTO et al.** *Cell Motil,* 1980, vol. 1, 31-40
- **OTTO ; BRYAN.** *Cell Motil,* 1981, vol. 1, 179-192
- The FOS and TUN Families of Proteins. CRC Press, 1994
- **NABEL et al.** *Nature,* 1987, vol. 326, 711
- **KAUFMAN et al.** *Mol. Cell. Biol.,* 1987, vol. 7, 3759
- **SAMBUCETTI et al.** *EMBO J,* 1989, vol. 8, 4251
- Cancer Principles and Practice of Oncology. 2001, 495-508
- **BEAUCAGE et al.** *Tetrahedron,* 1993, vol. 49 (10), 1925
- **LETSINGER.** *J. Org. Chem.,* 1970, vol. 35, 3800
- **SPRINZL et al.** *Eur. J. Biochem.,* 1977, vol. 81, 579
- **LETSINGER et al.** *Nucl. Acids Res,* 1986, vol. 14, 3487
- **SAWAI et al.** *Chem Lett.,* 1984, vol. 805
- **LETSINGER et al.** *J. Am. Chem, Soc.,* 1988, vol. 110, 4470
- **PAUWELS et al.** *Chemica Scripta,* vol. 26 (141), 91986
- **MAG et al.** *Nucleic Acids Res.,* 1991, vol. 19, 1437
- **BRIU et al.** *J. Am Chem. Soc,* 1989, vol. 111, 232.1
- **ECKSTEIN.** Oligonucleotides and Analogues: A Practical Approach. Oxford University Press
- **EGHOLM.** *J. Am Chem. Soc.,* 1992, vol. 114, 1895
- **MEIER et al.** *Chem, Int., Ed. Engl.,* 1992, vol. 31, 1008
- **NIELSEN.** *Nature,* 1993, vol. 365, 566
- **CARLSSON et al.** *Nature,* 1996, vol. 380, 207
- **DENPCY et al.** *Proc. Natl. Acad Sci. USA,* 1995, vol. 92, 6097
- **KIEDROWSHI et al.** *Angew Chem. Intl Ed. English,* 1991, vol. 30, 423
- **LETSINGER et al.** *J. Am. Chem Soc.,* 1988, vol. 110, 4470
- **LETSINGER et al.** *Nucleoside & Nucleotide,* 1994, vol. 13, 1597
- Carbohydrate Modifications in Antisense Research
- **MESMAEKER et al.** *Bioorganic & Medicinal Chem Lett,* 1994, vol. 4, 395
- **JEFFS et al.** *J. Biomolecular NMR,* 1994, vol. 34, 17
- *Tetrahedron Lett,* 1996, vol. 37, 743
- **JENKINS et al.** *Chem. Soc. Rev.,* 1995, 169-176
- *Rawls, C & E News,* 02 June 1997, 35
- **SMITH ; WATERMAN.** *Adv. Appl Math.,* 1981, vol. 2, 482
- **NEEDLEMAN ; WUNSCH.** *J. Mol. Biol.,* 1970, vol. 48, 443
- **PEARSON ; LIPMAN.** *PNAS USA,* 1988, vol. 85, 2444
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *PNAS USA,* 1993, vol. 90, 5873-5787
- **ALTSCHUL et al.** *Methods in Enzymology,* 1996, vol. 266, 460-480
- **MANIATIS et al.** Molecular Cloning: A Laboratory Manual. 1989
- Short Protocols in Molecular Biology

- Overview of principles of hybridization and the strategy of nucleic acid assays. **TIJSSEN.** Techniques in Biochemistry and Molecular Biology-Hybridization with Nucleic Acid Probes. 1993
- **SCHENA et al.** *Science,* 1995, vol. 270, 467-470
- **DERISI et al.** *Nature Genetics,* 1996, vol. 14, 457-460
- **NIELSEN et al.** *Science,* 1991, vol. 254, 1497-1500
- **NIELSEN.** *Curr. Opin. Biotechnol.,* 1999, vol. 10, 71-75
- **URDEA et al.** *Nucleic Acids Symp. Ser.,* 1991, vol. 24, 197-200
- **PEVZNER et al.** *J. Biomol. Struc. & Dyn.,* 1991, vol. 9, 399-410
- **MASKOS ; SOUTHERN.** *Nuc. Acids Res.,* 1991, vol. 20, 1679-84
- **R DRMANAC et al.** *J. Biomol. Struc. & Dyn.,* 1991, vol. 5, 1085-1102
- **P. A. PEVZNER.** *J. Biomol. Struc. & Dyn.,* 1989, vol. 7, 63-73
- **T. SANO ; C. R CANTOR.** *Bio/Technology,* 1991, vol. 9, 1378-81
- Pierce Chemical Company catalog, technical section on cross-linkers. 155-200
- **SMITH et al.** Direct Mechanical Measurements of'the Elasticity of Single DNA Molecules by Using Magnetic Beads. *Science,* 1992, vol. 258, 1122-1126
- **PEASE et al.** *Proc. Natl. Acad. Sci USA,* 1994, vol. 91 (11), 5022-5026
- **GUO et al.** *Nucleic Acids Res.,* 1994, vol. 22, 5456-5465
- **MASKOS ; SOUTHERN.** *Nucleic Acids Research,* 1992, vol. 20, 1679-1684
- **GERMER, S et al.** *Genome Res.,* 2000, vol. 10, 258-266
- **HEID, C. A et al.** *Genome Res.,* 1996, vol. 6, 986-994
- **HUNTER et al.** *Nature,* 1962, vol. 144, 945
- **DAVID et al.** *Biochemistry,* 1974, vol. 13, 1014
- **PAIN et al.** *J. Immunol. Meth,* 1981, vol. 40, 219
- **NYGREN, J.** *Histochem. and Cytochem,* 1982, vol. 30, 407
- **SMITH ; WATERMAN.** *Adv Appl. Math.,* 1981, vol. 2, 482-489
- **GO et al.** *Int, J. Peptide Protein Res.,* 1980, vol. 15, 211
- **QUEROL et al.** *Prot. Eng.,* 1996, vol. 9, 265
- **OLSEN ; THOMSEN.** *J. Gen. Microbiol.,* 1991, vol. 137, 579
- **CLARKE et al.** *Biochemistry,* 1993, vol. 32, 4322
- **WAKARCHUK et al.** *Protein Eng.,* 1994, vol. 7, 1379
- **IOMA et al.** *Biochemistry,* 1991, vol. 30, 97
- **HAEZERBROUCK et al.** *Protein Eng.,* 1993, vol. 6, 643
- **MASUL et al.** *Appl. Env. Microbiol.,* 1994, vol. 60, 3579
- **CREIGHTON.** Proteins: Structure and Molecular Properties. W.H Freeman & Co, 1983, 79-86
- **APLIN ; WRISTON.** *LA Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol,* 1987, vol. 138, 350
- **FIELD et al.** *Mol Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **FLOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci., USA,* 1990, vol. 87, 6393-6397
- **GARNIER et al.** *J. Mol. Biol.,* 1978, vol. 120, 97
- *Adv in Enzymol.,* 1978, vol. 47, 45-148
- *J Mol. Biol.,* 1982, vol. 157, 105-132
- *J. Virol.,* 1985, vol. 55 (3), 836-839
- *CABIOS,* 1988, vol. 4 (1), 181-186
- **WELLING, G.W. et al.** *FEBS Lett.,* 1985, vol. 188, 215-218
- **KOHLER ; MILSTEIN.** *Nature,* 1975, vol. 256, 495
- **GODING.** Monoclonal Antibodies: Principles and Practice. Academic Press, 1986, 59-103
- **WALDMANN, T. A.** *Science,* 1991, vol. 252, 1657-1662
- **WINTER et al.** *Nature,* 1991, vol. 349, 293-299
- **LOBUGLIO et al.** *Proc. Nat. Acad. Sci USA,* 1989, vol. 86, 4220-4224
- **SHAW et al.** *J Immunol,* 1987, vol. 138, 4534-4538
- **BROWN et al.** *Cancer Res.,* 1987, vol. 47, 3577-3583
- **RIECHMANN et al.** *Nature,* 1988, vol. 332, 323-327
- **VERHOEYEN et al.** *Science,* vol. 239, 1534-1536
- **JONES et al.** *Nature,* 1986, vol. 321, 522-525
- **RIECHMANN et al.** *Nature,* 1988, vol. 332, 323-329

- **PRESTA.** *Curr. Op. Struct. Biol.,* 1992, vol. 2, 593-596
- **JONES et al.** *Nature,* 1986, vol. 321, 522-52.5
- **VERHOEYEN et al.** *Science,* 1988, vol. 239, 1534-1536
- **HOOGENBOOM ; WINTER.** *J. Mol. Biol.,* 1991, vol. 227, 381
- **MARKS et al.** *J. Mol Biol,* 1991, vol. 222, 581
- **COLE et al.** Monoclonal Antibodies and Cancer Therapy. Alan R.. Liss, 1985, 77
- **BOERNER et al.** *J. Immunol.,* 1991, vol. 147 (1), 86-95
- **MARKS et al.** Bio/Technology. scientific publications, 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern Rev Immunol.,* 1995, vol. 13, 65-93
- **MORRISON et al.** *Proc. Natl. Acad. Sci, USA,* 1984, vol. 81, 6851-6855
- **MORRISON ; OI.** *Adv. Immunol,* 1988, vol. 44, 55-92
- **VERHOEYER et al.** *Science,* 1988, vol. 239, 1534-1536
- **PADLAN.** *Molec Immun.,* 1991, vol. 28, 489-498
- **PADLAN.** *Molec Immunol.,* 1994, vol. 31 (3), 169-217
- **KETTLEBOROUGH, C.A et al.** *Protein Eng.,* 1991, vol. 4 (7), 773.83
- **CHOTHIA et al.** *J. Mol. Biol.,* 1987, vol. 196, 901-917
- **MERRIFELD.** *J. Am Chem. Soc,* 1963, vol. 85, 2149
- **CAPRINO ; HAN.** *J. Org Chem.,* 1972, vol. 37, 3404
- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **NEVELLE et al.** *Immunol Rev,* 1982, vol. 62, 75-91
- **ROSS et al.** *Eur. J Biochem,* 1980, vol. 104
- **VITTETA et al.** *Immunol Rev,* 1982, vol. 62, 158-183
- **RASO et al.** *Cancer Res,* 1982, vol. 42, 457-464
- **TROWBRIDGE.** *Nature,* 1981, vol. 294, 171-173
- **SCOPES, R.** Protein Purification. Springer-Verlag, 1982
- **LOCKHART.** *Nature Biotechnology,* 1996, vol. 14, 1675-1680
- **ZLOKARNIK et al.** *Science,* 1998, vol. 279, 84-8
- **HEID et al.** *Genome Res,* 1996, vol. 6, 986 994
- **STEIN ; COHEN.** *Cancer Res,* 1988, vol. 48, 2659
- **KROL et al.** *BioTechniques,* 1988, vol. 6, 958
- Remington. The Science and Practice of Pharmacy. Lippincott, Williams, & Wilkins, 1995
- **FINDEIS et al.** *Trends Biotechnol.,* 1993, vol. 11, 202
- **CHIOU et al.** Gene Therapeutics: Methods And Applications Of Direct Gene Transfer. 1994
- **WU et al.** *J. Biol Chem,* 1988, vol. 263, 621
- **WU et al.** *J Biol. Chem.,* 1994, vol. 269, 542
- **ZENKE et al.** *Proc. Natl. Acad. Sci. (USA),* 1990, vol. 87, 3655
- **WU et al.** *J. Biol Chem,* 1991, vol. 266, 338
- **JOLLY.** *Cancer Gene Therapy,* 1994, vol. 1, 51
- **KIMURA.** *Human Gene Therapy,* 1994, vol. 5, 845
- **CONNELLY.** *Human Gene Therapy,* 1995, vol. 1, 185
- **KAPLITT.** *Nature Genetics,* 1994, vol. 6, 148
- **CURIEL.** *Hum. Gene Ther.,* 1992, vol. 3, 147
- **WU.** *J. Biol. Chem.,* 1989, vol. 264, 16985
- **PHILIP.** *Mol. Cell Biol.,* 1994, vol. 14, 2411
- **WOFFENDIN.** *Proc Natl. Acad. Sci,* 1994, vol. 91, 1581
- **WOFFENDIN et al.** *Proc Natl. Acad. Sci USA,* 1994, vol. 91 (24), 11581
- **BROWER.** *Nature Biotechnology,* 1998, vol. 16, 1304-1305
- **MULLIS et al.** *Meth. Enzymol,* 1987, vol. 155, 335
- **SAMBROOK et al.** Molecular Cloning: A Laboratory Manual. Cold Spring Harbor Laboratory, 1989
- **SAIKI et al.** *Science,* 1955, vol. 239, 487
- **SAMBROOK et al.** Molecular Cloning: A Laboratory Manual. CSH Press, 1989, 14.2-14.33
- **HANAHAN et al.** *Cell,* 2000, vol. 100, 57-70
- **LUO et al.** *Nature Med,* 1999, vol. 5, 117-122
- **S.M. WEISSMAN.** *Mol Biol. Med.,* 1987, vol. 4 (3), 133-143
- **PATANJALI et al.** *Proc, Natl. Acad Sci USA,* 1991, vol. 88 (5), 1943-1947
- **SAMBROOK, J.I. et al.** Molecular Cloning: A Laboratory Manual. Cold Spring Harbor Laboratory Press
- **SAMBROOK, J.T. et al.** Molecular Cloning: A Laboratory Manual. Cold Spring Harbor Laboratory Press

- Effective Large-Scale Sequence Similarity Searches. **CLAVERIE.** Computer Methods for Macromolecular Sequence Analysis, Doolittle, ed., Meth. Enzymol. Academic Press, 1996, vol. 266, 212-227
- **CLAVERIE et al.** Automated DNA Sequencing and Analysis Techniques. Academic Press, 1994, 267
- **CLAVERIE et al.** *Comput. Chem.,* 1993, vol. 17, 191
- **ALTSCHUL et al.** *J. Mol., Biol.,* 1990, vol. 215, 40
- **PEARSON ; LIPMAN.** *PNAS,* 1988, vol. 85, 2444
- **ALTSCHUL et al.** *J Mol. Bio.,* 1990, vol. 215, 403
- **DELLI BOVI et al.** *Cancer Res.,* 1986, vol. 46, 6333-6338
- **CESARONE, C. et al.** *Anal Biochem,* 1979, vol. 100, 188-197
- **SOUTHERN, E. M.** *J. Mol. Biol.,* 1975, vol. 98, 503-517
- **FEINBERG, A. P. et al.** *Anal. Biochem.,* 1983, vol. 132, 6-13
- **SAMBROOK et al.** Molecular Cloning: A Laboratory Manual. Cold Spring Harbor Lab. Press, 1989
- **WRIGHT ; MANOS et al.** PCR Protocols. Academic Press, 1990, 153-158
- **DELLI BOVI et al.** *Cancer Res,* 1986, vol. 46, 6333-6338
- **FEINBERG, A. P. et al.** *Anal. Biochem,* 1983, vol. 132, 6-13
- **SAMBROOK et al.** Molecular Cloning: A Laboratory Manual. Cold Spring Harbor Laboratory Press, 1989, 16.17-16.22
- **KEOWN et al.** *Methods in Enzymology,* 1990, vol. 185, 527-537
- **MARKS et al.** *But J Urol,* 1995, vol. 75, 225
- **SKEA et al.** *J Immunol.,* 1993, vol. 151, 3557
- **MATHER et al.** *J. Nucl Med.,* 1990, vol. 31, 692
- **ZHANG et al.** *Nucl. Med. Biol.,* 1992, vol. 19, 607

SEQUENCE LISTING

<110> Sagres Discovery
Morris, David W.
Engelhard, Eric K.

<120> NOVEL COMPOSITIONS AND METHODS FOR CANCER

<130> 529452000940

<140> PCT/US03/08919
<141> 2003-03-21

<150> US 60/367,025
<151> 2002-03-21

<160> 34

<170> FastSEQ for Windows Version 4.0

<210> 1
<211> 32767
<212> DNA
<213> Mus musculus

<220>
<221> misc_feature
<222> (1)...(32767)
<223> n = A,T,C or G

<400> 1
cctctcaaac tcttggtaat tctcctgctt cggcacgctg gggactggga ctacacctgt 60
gtgccaccat ggctggctat ttctctctct tgaacactgg aagagtgctc agagtttact 120
ggatctggga gaagctcgag gctgggttat ggaagccggc tctgcttgct ccagctcagg 180
gaggagttgc ctgagggcca ggcacttcga aggacagatg ggacccaagg ccagacactg 240
ggccccagct caccagaagt ggagccctga ctgtctctga gagggtaagc tggggtggct 300
gccaggcggg caaggccaaa gcctggcagc agccggtggc ccctcttctg gcagagatat 360
cttggcatga gcctggctct gcccatgaca ctaaagtgcc ctcttaatta gccaggctct 420
tgccaagcac tggccacgat tgccttgttt cacagaattc accttggact ggcacagatg 480
gggagggtgg atagcccggt gttttgtctt tcttctaggg gagctgggct caagggcagg 540
actcctgggc cagcccaggt ttttccctca gaaaccacag catgaatact ggcatgatgg 600
agcacacctg taatcagcag aaattgagac aggaagattg ttgagaattt gaggccaacc 660
taggctaaat agggagaccc tatctctaca cactccccct ccccccgccc ctggaaatgc 720
tggagggtct ggggaagatg gctcagtggt taagggcact tgctgctctt gcaggggacc 780
caagtttgat tcccagcatc tataacttgg tggctcacaa ttccagttcc aggcactctg 840
acaccttcct gtggtctgca aggcacctgc attcattctc acgtgcatgc gcacacacat 900
gcgcgcgcgc acacacacac acacacacac acacacatct cataaataca aataaaataa 960
atcttgaaaa caacagtgac aacaacacat gctgaacatg gtggtgctcc tgacatggtg 1020
gtgctcctga catggtggtg ctcctgacat ggtgatgctc ctgacatggt ggtgctcctg 1080
acatgatggt aagcctgaca tggtggtgct cctgacatgg tggtgctcct gacatggtga 1140
tgctcctgac atggtggtgc tcctgacatg gtggtaagcc tgacatggtg gtaagcctga 1200
catggtggtg ctcctgacat ggtggtgctc cctgcactga cagttctgag gaggtggaga 1260
taggaggatc cagacttgga agcttgcctg agatagaccc tgtctcagaa taggctggag 1320
gctgcctctg cccttttgct tctatctctt tttccatatg tgtgtgtgcg tgtgtgcata 1380
tgtgtatgca tatttgtaag tatatgtata taaataaaca cacacacaca caaacttact 1440
ctgtatacta ggctggcctt gaactcagag acctgcctgt ttctgccttc ccagtgctgg 1500
gattaaaagc gtgtgccatg ggctggtgag atggctcagt gggtaagagt acctgactgc 1560
tcttccaaag gtccagtgtt caagtcccag caaccacatg gtggctcaca accatccgta 1620
acaagatctg acgccctctt ctggtgtgtc tgaagacagc tacagtgtac ttaaataaat 1680
aaatcttaaa aaaaaaaaaa aaaaaaaaaa aaggcgtgca ccaccactgc ccggctagtt 1740
agctttatct tccgtggatg ttttgtctgc agtatgtctg agtgagggtg tcagatcctc 1800
tgtaactgga gttatagaca gttgtgagca gccatgtaag tgctgggaat tgaatccggg 1860
ttctctggaa gagcagttca tgctcttaag cactgcgcca tctctccagc tgtccttatt 1920
tatttttaag ggtctcttgt agccatacag gccagaaacc caccattagc cacggacctt 1980
gagctccaag tgctgggtga caggcccgca tctctcctgg tttgtgatct gctagggatg 2040
gggtctagga cctcacacag gctgcataag cattcccctc acccaccctc atccccaccc 2100
agtcctggac aggctctggc tgtgtaggct ggtctcctcc tttctatccc cctgcctcag 2160

cctcctgagt gctgaggnnn nnnnnnnnnn nnnnnnnnnn nnnnnnnnnn nnnnnnnnnn 2220
nnnnnnnnnn nnnnnnnnnn nnnnnnnnnn nnnnnnnnnn nnctgtggtg gctcccgagt 2280
gctgtcatcc cagcactggg gaggttgaag caggaaggag aatcacaggt tcaaggccag 2340
cttggtttac ttgagactct gtctcaaaaa gaccaaacca gggactggga ggatggccag 2400
taaaatgctt ttccagctat cgagggaacc tgagtttcat cctggaactc atacaaaaag 2460
ctggctgtgg tcaggtggcg catgccttta atcccatcac tcaggagaca gaggcaggca 2520
gattcttgag ttcgaggcca gcctggtcta cagactgagt tccaggatag ccaggtctac 2580
acagagaaac cctatcttgg aaaacaaaac aaacaaaaaa aactaggtgt ggctagcctg 2640
taaccccagt gctgggtaaa caggggcagg tgaatacatc tccgggattc gccagcctgt 2700
cctatctatg agctctaggt ctggggagac agcctgtcaa atcaatgcaa gcaggtgaca 2760
accgaggaaa gacacccaag gttgatgcac gggcaagtgt gcacacacac acaccacagc 2820
aaacaaaaac taacaaaacc caaaccatat tttgctgtgg tggtggttgt taaattttat 2880
ttattctgtg tgtatgggta tttcacctgc atgaatatct acctgtgtag catatacatg 2940
ccgggtactt tcggaggtca gaagaggaca tcaaatcccg tagaactgga gttacagaaa 3000
atgaacttgt ggcttctggg aatctaactt ggatcctcta aaggacaggc aggactctga 3060
attcttgagc cacagataca gctttccaaa cccatgtttt gtaagagcca ccaggcctga 3120
aaactctggg ccatgtcccg tttgttgtgt ctaggcctca gtttcctgga aggcagagaa 3180
tgcgtaggtc cgttgttagt ggagacagca gtggtggtaa cagcctggtc taccctcttg 3240
gtcagaggga ggcaggcgga gagtaactca gggcctgggg gcaacaccca gctgggcctg 3300
gggctagtgg gggctcagcc aggcaggacg tgggtcctcc tacttcctaa gctctgatgg 3360
gcaagtggtg ccagtggtac caggctgccc tgaggggccc ttagagcagg tgaccacaga 3420
gccacaaaga ggctattcat ggcctccggt tcacgaggct gcccctttat tgagggcttg 3480
aaccacagaa agctgtgtgg tctgcaggga agctagttct gagctgtctg gccagctaca 3540
gtagtgtgtg tgtgtgtgtg tgtgtgtata cacatggtac ttttttctat ctatctatct 3600
atctatctat ctatctatct atctatctat ctatctatct ttgtgtctat gtatctaata 3660
ataaattgat ctatttaact atgtagatat caatccattg atctatgtat ctatcaactg 3720
atgtgtatgt atgtatggat gtacctacat gcgtacttga ggcaggactt aaagtagccc 3780
aggctagcct taccacattt catagcagag gatggtcttg aacttcaggt cctcctgtct 3840
ctacctcgtg agtgctggga ttacaggttt gtgccaccac gcctggctta ggaggttctg 3900
tggttagaat ccaggatttc actatcaact gggccacatc gtcagccata ttcaatatgc 3960
tttcttttac attcttactt atttactaca aatgggtccc aggaaccaag gtcggcaagc 4020
ctgatggtga gtgctcttcc ctgctggctg acttatttgc tctttttttt tttttttttt 4080
ttttctagac agggtttctc tgtgtagccc tggctgtcct ggaactcact ttgtagacca 4140
ggctggcctc gaactcagaa atctgcctgc ctctgtcccc aaccccaccc ccagtactgg 4200
gattaaaggc atgcgccaca cgcccagctc ttatttgctc ctttgaatta aggtctcatg 4260
tacacaaggc tgaccgttca tgcacaatac agctaaggct tgctttgaat ttctcagttt 4320
ctttcttcta cccaacagtt tctgagattt caggtccgtc ctatgtcatg cccaggtttt 4380
ttgtttgttt gtttgtttgt tttttggttt tgttttgctg tttttcaaca cagagtttct 4440
ctgtggaacc ctggctatcc tggaactctg tataccaggc tgtccttgaa ctcagagacc 4500
ctctgcctcc ctagtgctgg gttaaaggtc cctgccatca ccgtgaaact taagcccagc 4560
ttctcatttg gtggctggaa atttgcctgc ctctgcctct ctccagctgc ctagcactga 4620
tgtctggttg agagcagggg aaactgaggt gccgctagga caagatctca tgcaaccgtg 4680
aacagcccta ctgagccatc ctcctgggac agcctctcag gccaggcctt ctgtgtcttc 4740
tgctatagta gcagtacaaa gttgacacaa ccttgctctt cccagcagct gggagttcct 4800
gagaagtgta aatgaggttt ccaggaaagc caagaggtga gagactgaaa agttggaggg 4860
ataagggggt gcccatcaag ctgcagtgga gacttgttat ctgttgcgca tccctgacat 4920
ctcagcagga ggatcaggag ttcaagggca tcctcagcaa catagtaagt ctatggagct 4980
agcctgggct acacagatgc cattacaaaa aaaaaaaaaa aagtaaaaaa aaaatcttgg 5040
gatatggggg atgggtgggt gaactgtgta gcccagggag tcactactgg ttcctttgga 5100
agtccctgtt ctgcatggag atcatggaga ttctggggac actccaggag tcctccttca 5160
gggaactaag ttggggagca gggcagccta tcctgtggcc ttatttcctg tctggtgcaa 5220
gcgccaggca aggcatccag ccaggagcga ctcaggcctg acctccgcta actgttctgt 5280
tttgtgctgc accctcatct cagcagggat caggtggcac ttgggtcccc aggggatcca 5340
gggacacagt gtccatgtcc ccatctttcc ccacagggac ctggggcagg gccgaagagg 5400
agacctcgtg atgtgtacag gtgtagctca tccctccgag gactcagggg acacttgggg 5460
aaggtgtgac acagagcagg ctggagctct gtgcggctct ttattaagtg tgcctacaac 5520
aggggcagag acacagcagc gacaatgata acagcccact cccaaccgaa gctcaatggt 5580
taccagactc agccaactgc agaattccag tccataacgg cacctccttt cctttctcag 5640
gagcttacca gagaggtctc accccaagcc ttcaccccat ctagagcaaa tgcttatatc 5700
ccccatgttt gctcagagca gagcatggag ggaccttcct gtcaccagag gcagaggcat 5760
tctgtatacg tgcatacacg cctctgtgtg tgtgtgtgtg tgtgtgtgtg tgtatttggt 5820
acagatctaa gcatagaaaa ttgaaaagca ttgaggttgg tgattgaaag gctcactgat 5880
gggcaagtcg ggttcgagtg cgctaactgt atgactctat ggatccgaat atcatgctgt 5940
tttggaaagg aatgagggag ctactgtttc taaggaagag aagaatgggc accaagacat 6000
agggagggaa ggtgggacag taagcactta cagccgaagc ccaagacctg gaacccaagg 6060
ggaaaaacag gtgtggccag gcttggtggc acgcgccttt agtctctgag ttcaagtcca 6120
gccaggatta catagcaaaa ctttccctca agaaaaaagg taggggctgg agagatggct 6180
cagcagttaa gagctcttcc gaaggtcctg agttcaaatc ccagcaacca catggtggct 6240

cacaaccatc tgtaatgaga tctgacgccc tcttctggtg tgtctggaga cagctacagt 6300
gtacttacat ataataaata aataaatcct aaaaaaaaaa aaaaaagaaa aagaaaaaga 6360
aaaaaggtag aggaggagga agaggaggag gagacggcaa gacaaaacta actacatagc 6420
tgggggaggt ggtgcttgcc tataatccca gcacttggaa ggctgaaaga ggaaatcagg 6480
agttcctatg cagctttggc ttatgtgagc ctttctcata aaaccaagag caaaaaagca 6540
aaacatgagg tggtaactca gctggagagt gcttgtctag attccctcag tgagggctg 6600
gggtgtgact cagtgcctag aatcccccag ggagggctg ggggcgtggc tcagtggtag 6660
agcccctgcc tagaatcccc cattgacggg ctggggtgtg gctcagtaac agactgctca 6720
cctaagatgt tcaaggccat gactttcatt gaaaacatca caaaacaaac aaaaccatca 6780
gtgaggttga atgtgtatcc atgtttgctt gtctccattt aaagaacacc tggaagatct 6840
ctagctgctg gtggaggcgg gagggtgaag gctgcttcat cctttgtatt tgaactgtgt 6900
ggctgtgggt gtattatctg ttcccaagga ctgcacatga aatgagaaca atgaaggctt 6960
cggttctgct gagatgaacc tcaattctcc acagatgatc tcacccagag ccagatggaa 7020
gtcccccaac atttaagcct ctgttctgag tgctggaccc aaactcatct taagggaaaa 7080
cccaaagagc ctttctgtgg tttccttcca caactgtctt tcaatgtgtg tatgtgtgca 7140
cgtgtgtttg cttgtgtgtg tgtgtgtgta ctatgtgcat gtgtgtgagc atgtgtgtat 7200
atgtatgtgc atgagtgtgt gtgtgtgtgt gtatacatgt gagcatgtat gtataggcca 7260
gaggttagtg atgggtaact ttctccagag ctctccacct tagttttatt gtctgtgtgt 7320
gagcggctgt gggcactgta caccacagca catgtgtgga ggtcagagaa caacttgtgg 7380
ctgtcacttc tctccctctg tgtgggtcta agacctggat ccagcgcttt cgcttgcttg 7440
agacatctcg ttgactagct cctccgttgt taacacaggg tctcactatg taggtccagt 7500
tatcttggaa tttgctctgt agaccaggcc ggcatcaaac tcagagatag acctgccttt 7560
gcctccagag tactgggatt aaaggtgtgc accactacca cccagtttaa aattctaaaa 7620
agatttgtgg gtgcagggat ttgaggaggc caggagagac tgttggatct ctgggaactg 7680
gagttagagg tggttgtgag cccccacggg tgctgagaac tgaactaggg tgcttgagag 7740
gagctcactc actgctgagc ccccacttta gatttgagtc cctcgtactg aagctgagtt 7800
cagtgatttg gctgagctga ccgctcaggg tgggcttttc gatgtgcacc acaggcctgg 7860
ctttataggt gaggtcatgc ttgcacaact ggccttttgg tgactcagcc aactccccag 7920
acctttttc ttttgcattt acgtgtgtgt gtgtgtgtgt gtgtgtgtgt gtgtgtgtgt 7980
gtgtacatga agtgagaggg ggcagtctgg gaagggtcag tggacaactt aagggagttg 8040
gttttgcctt ccatcatgat ggtctagaga ctaaattcag cccctcagcc tgggagcgtg 8100
cctttaccct ccgagctccc agctttgggt ttttgaggca aggtttcata tagcttaggc 8160
tggcctggca ttcaaggtca tctcagaggc ctaggatgac cttgtgttgt cggttctccg 8220
gcctttacct ctggtgtgct ggcgtttgcc ttaccacacc caggcatccc ccatagctta 8280
agcctaaagc tctcggatgg ccagcacgcc catgccctga cgtgaagttc agtgactaat 8340
caatacagag cgttcacgga gccctgacac atagcagcat tgacttaacc ttaacagcct 8400
ggtgtgtaat gaggccaact tgtccgattc tgcccacgat gcgatgctct gggttaaaca 8460
ggtttgaaca gagtaccgga tggcagggag ggcaccctca gtacagggtt ttggctgtta 8520
ctgcttccag tctcctgacc tgctccccca aggctggtct taaactcaaa gcaatcctcc 8580
tgtccctgct tctcaagttc tggaattaca agtgtgactc actgtaccca ggaatatgtg 8640
tctttattac agaggaagga gagctcagag agtgagagta acctgtgcaa agtcccacag 8700
caggagaggg tggctggcta tcggccgtga acttggtgac ctttcctgtg acctctaagg 8760
gaggaactag atgtcccagc aaggattcta gggacttgtc aggactgacc tttgatgatg 8820
ggaaagccat gccatctccc cttccccttt gatggtcgct ggaggggttt gcctgggaca 8880
gagatgtgtt tggacacaga tcaccgtgaa gactggtcag tttcagtacc ctgcccagcc 8940
tgtggctggg aagggccagg ggcccagatc ttgtgggcag cctggggtag ggccagcatt 9000
ccaggggcct gcctggcatg tggggaatgt cccaggagaa gtctcagtcc tccagaaact 9060
caaagggaag tgttcttacg tggggtggtg tgtggtggga ggtgggaggt ggtgtttgtg 9120
ggaatgtccc tctggtctgg aaccctgagg ggctgcagtt aggaggtaca ttggggctgt 9180
gccattctag ggagctgacc tgcttttctg atgtcttcat aaacagagac cactattcct 9240
gccttaccta ccctgcttcc tggtagacca gcaccccggg gcttgagcat caccacagta 9300
gggacttttg tgggccactg acttgggcca cagttaccac aatttattta gagtcagcaa 9360
ggtggctgta ctctatgctg acccgggaat agaaatctcg gtttctgctc aggaggccca 9420
ggctacaagg ccttgaaaaa ccctgaggcg ggcggggcag gtagggctct gcatgcgcct 9480
ctctagaaat taacctcctt tctaccccag acccttctgg cgtctctggc ttcttcaagg 9540
accggctcta aggctacttc ctggtacacc ctggaccctc cctccatcct gaagggatca 9600
ttatcttaaa cctggtcctt cccatcgact gggtttgggg gtaggggtgt ttcaggatgg 9660
cctcagagag caggagaaag ttggagatca gccaactact gcagagcacg actgtggagg 9720
aacagttaaa tggactagca aacacgaacg ggccagaggc agaaggactg ctatgagttc 9780
gagtccagcc tgagccacac agatacttca agcctagtct gagttacaga gtgagacccg 9840
gtcttaaacc ccagaaaaac actccgggga agcgcagggg accgggcgcg cgctgcagag 9900
ccccctcccc ggcaggcccg ggtaggggcg tggccacggt gacgtcatcc tcctataaaa 9960
ccctgggcgc cgccgggctg gctttgtgga gaactgcagc gggctaagcc gtgttgaaca 10020
aaggaggtcg ggcacagcta tccaagctcc cggggccacc gggccgccct ccgccaccat 10080
gaccgccaac ggcacggcag aggctgtgca gattcagttc gggctcatca gctgcggcaa 10140
caagtacctg acagccgagg cgttcgggtt caaggtgaac gcatccgcta gtagcttgaa 10200
aaagaagcag atctggacgc tggagcaacc tcccgatgag gcgggcagcg cggccgtgtg 10260
tctgcgcagc cacctgggtc gctacctggc cgccgacaag gacggcaacg tgacctgcga 10320

```
gcgcgaggtg cccgacggcg actgccgctt tctcgtcgtg gcgcacgacg acggccgctg 10380
gtcgctgcag tccgaggctc accggcgcta ctttggcggc accgaggacc gcctgtcctg 10440
cttcgcgcag agcgtgtcgc cggccgagaa gtggagcgtg cacatcgcca tgcacccgca 10500
ggttaacatc tacagcgtta cccgcaagcg ctacgcgcat ctgagcgcgc ggccggccga 10560
cgagatcgcg gtagaccgcg acgtgccttg ggcgtcgac tcgctcatca ccttggcctt 10620
ccaggaccaa cgctacagtg tgcagacgtc cgaccaccgc ttcctgcgcc acgacgggcg 10680
ccttgtggca cggccggagc ccgccacggg cttcacgctg gagttccgct ccggcaaggt 10740
ggcctttcgc gactgcgaag gtcgctacct ggctccgtcc gggcccagcg gcaccctcaa 10800
ggctggcaag gccaccaagg tgggcaaaga tgagctcttc gccctggaac agagctgcgc 10860
tcaggtggtg ctgcaggcgg ccaacgagag gaacgtgtcc acgcgccagg gtgagttggg 10920
gacatgtccc ctcccttggg tcctgaccca gtgcacaaaa gcatctctgc actccttcta 10980
tttccctatt tcctcctggg ctccctttag gctcacaggc ctctgggctc cgccatgggg 11040
aggtgggctt ctattttgaa agacctgatc tcaccagata gccttggcta tcctggaact 11100
cgctatgtag actgggctgg ccccgaactc gcagaaatct tccttcctgt ctcttgtgct 11160
gggaataaag gcatgtacca cccctctgtt caggacgtag gcttcttctg ctgggagaag 11220
gtgacgtggg gaagtgggta aggcctaaca cattctaaag caatactgtc caccaccacc 11280
cccccacccc cccacccccc cggctggggg cgtgggactc catagaccta gcttagatct 11340
tctccgtttc ctcttccttc ccagtctgag ccatccccat tcatgagccg attctttagg 11400
ataggctgtc ccaggtcttg gagaggaagg gttcccccta caccccccct tgttcatttc 11460
ctcgtgcgtc ctctcccgtt tcgcgggcct ttggctggag ggccagggga cagtcacccc 11520
tgccctcccc gccttaaatg gcgagataga ggaggttgga ggggcttttg atcccggcgg 11580
ggtgtgctgg gcggggtcgg ccctgcgact cttggtggtg gtggggttgt ccgtaggggc 11640
ttcccctccc ccactccatc tcccctcgct aaggcctggc gctccgggcc gcggcctttg 11700
tgagcagggg ggcgggtcgc cacggctggg ccctccctcg tccccttttgt cctgctaagg 11760
ctctgcagat gggaaaacca gatgcggcgg agctgggggga ggggatcggc ttaggcgggt 11820
ccctctgcgg aagagccacc cccaaccctc caggcgttgt ccctggtcca gggttcattg 11880
agaaggcgcg gagtgaagcc gcctggctct tccttcgcac cccggctggc aggcggcgtg 11940
gcgcgggtgg ctgcgcgcca ggcgccctcc ctgccctcct atgcacgcct tgcggtccgg 12000
cactcggcgg agtgtccctc caaaagcctc tactctcgag tgaaccggtc cccaaggcct 12060
ctctggctgg gaacaaccgt ttgcagctct atttgtcccc atccagggcc tccagcaggc 12120
tggcaggcca tttccgaccg gcatgggcat ggtacaagta ggcgcctcct ttctggcgta 12180
cccccgactc ttcaaagggt ctttgctgcc tgctggtagg ggcttgtgag atctcgcttc 12240
cttggttggg aaactgaacc ccaggctttg gagatagctg gatgggagcc agtctgtggg 12300
gaggaagacc cccctccccc aatgcagagg gcaaagcctt gggtggggcc tgcttgagtt 12360
tggctgaata acgctcagaa aagtgcgacc atttgcttac tgttacacag taagtcagca 12420
ccatgctgaa ctgggtgtac ttgatgccag aatgttctct cattatacac acacacacac 12480
acacacacac acacacacac acacaccaat gaaccatggc ctctcttgtt tcacagtgct 12540
gggcgagggg gtcagagttc ccacctgttt agcaaacatt gagatacgag ctgcggtaaa 12600
cgtctcttgg gaatgtgact ggtatgtaaa gcagaagatt gggggtacag gatcggggta 12660
gtagattata aagatctggg gtcccttctg aggacccagg agctgcccca ggagagtgaa 12720
tagaaatgag catgcaccaa gtttatggac tggttttgca ggcaggggcc tcagcaaagg 12780
cccggaggca gggagttgca aatagagaag ccctgcatag ctaggaggaa ggctgggatt 12840
ggagggaatc ctgcagatga cgggtactcc agagcctaga gagaccagct ctctgcttac 12900
agagctgtgg ccgaggcttg tgccctgggg aaggttaggc aaggcacagg tgacttgggc 12960
ccttggggcc tgaaaggagc tagagggtgt gttttgcgag cctggacagg taccctttttc 13020
tgagggtgcc atgattgtgc atgattcaca ctgatgcggc aggaggggc gggaattgaa 13080
ggggtgagaa tctgacggga acttggggtc acacacagtc agacctcttc tgccctccta 13140
tgtctgggtc caagtctcat tcccgagtca gccaactcct cccccggctt tttgagggtg 13200
agctgttggg tgggtgtgcc ggggaggcca ttttgggcag gcttgcgtgg cagggaggcg 13260
gggctgggct tcggggaaac acgtgttgaa ctgctagcca ggagtaggga gagggaaggc 13320
tcaatctggt ctggggttgt gaatcttcca tgcagatttg ctgcttccgg cttcctggcc 13380
tcagtttccc catctgaaag cacagaggta cattgagcca ggatcttcct gggaggtgga 13440
gatcctatga gagtaggatg gggggtagtg ggtagggtct tttcctcctt gcttctcatg 13500
accctcccaa gcccctgctg gctcaaggag accccgtgct atgtctcccc acactgccca 13560
ccctggctta cttgttgggt tctaatgctt tcaaagaatt ttgtccccat catttctagg 13620
gcggctggct aagaatgagt gacaggtgag gctgggcttt gtagggtatc cttttgacgg 13680
cttccctctt gggtcgagga atggcttagt tttgagcact gtacgacttt gggcggtgaa 13740
gtcctggagg gttctaaact aagccgcctt ctgggttgtg aggattgctt atttcgaaca 13800
acaacgctag ggagtctggc aaacagctaa gcccaggaag ggggttgcgg gttagaggac 13860
cctctgagtc accgcagaga cgcctaggaa gctggaagac agccacgccc cctccgtggg 13920
ctcctaggag ccagtgactc aggttttctg tgctgctgct gctgaaaaaa aaaaaaatcc 13980
cacagaaaac ctgttaccat agcaacgcag ctccaatatg attccaaggc caagtggggc 14040
ctttccccct ggctgagcct tagagggccc attctggaag gggggctaact tccctttccg 14100
gctgaggtgg gagggcccac catctccttg gcaacaggcc ctcacgtcct ttcacggtcc 14160
caacagaatt gagaggcgat tctttttggc aagggaggga aactgaggca gagagcggag 14220
aagacattcc ccgaagggtg cctggagtcc acctttggtg ccttggtggc ctctttatta 14280
aaaagtggtt gcacaggccc ctttgttcca gtacctggag atgagaccca catggtgagg 14340
cccttttttgt tctctgtctg gacgtctcat ttttcagagg tactgggagg ttgaaggttg 14400
```

gtgtatgtcc acagcactgt gctgtcttga actcagtgcg catgacatct tgggccaggg 14460
cctctccttt gtgtgaggga agctagcatg ctgggaaact ccgactcctt tagtccaccc 14520
actttagttg tagtttttgt cttgtagccc atctggcttc caactctcca tcctcctgcc 14580
tcagtctcca gggtgcggcg atgacaggca taggtcacca caggcttctg gcctccactt 14640
gatggttgcc ggagtggctg ggagtggctt ttctttttctt ttctttttctt ttctttactt 14700
tcaggcaggg tttatctgtg tagtccggct gttctggaac tatgtnnnnn nnnnnnnnnn 14760
nnnnnnnnnn nnnnnnnnnn nnnnnnnnnn nnnnnnnnnn nnnnnnnnnn nnnnnnnnnn 14820
nnnnnnnnnn nnnnnnnnnn nnnnnnnnnn nnnnnnnnnn nnnnnnnnnn nnnnnnnnnn 14880
nnnnnnnnnn nnnnnnnnnn nnnnnnnnnn nnnnnnnnnn nnnnnnnnnn nnnnnnnnnn 14940
nnnnnnnnnn nnnnnnnnnn nnnnnnnnnn nnnnnnnnnn nnnnnnnnnn nnnnnnnnnn 15000
nnnnnnnnnn nnnnnnnnnn nnnnnnnnnn nnnnnnnnnn nnnnnnnnnn nnnnnnnnnn 15060
nnnnnnnnnn nnnnnnnnnn nnnnnnnnnn nnnnnnnnnn nnnnnnnnnn nnnnnnnnnn 15120
nnnnnnnnnn nnnnnnnnnn nnnnnnnnnn nnnnnnnnnn nnnnnnnnnn nnnnnnnnnn 15180
nnnnnnnnnn nnnnnnnnnn nnnnnnnnnn nnnnnnnnnn nnnnnnnnnn nnnnnnnnnn 15240
nnnnnnnnnn nnnnnnnnnn nnnnnnnnnn nnnnnnnnnn nnnnnnnnnn nnnnnnnnnn 15300
nnnnnnnnnn nnnnnnnnnn nnnnnnnnnn nnnnnnnnnn nnnnnnnnnn nnnnnnnnnn 15360
nnnnnnnnnn nnnnnnnnnn nnnagagaga gagattggga ctgactggtc ccagttctgg 15420
ctttccctga ctgttccatt ccccgcagtg ggcacggtta gatcaggtgg ggtcacttag 15480
gctcgctagc attgggtgtg gctcttttct gcccagtatg ctgtgctaac tcagctgtcc 15540
ctgcacgtgg caggtcaatt acaggtaggc tgaggggaca gtgggtgaca ccctgtatat 15600
gtgtagccaa ttgttctctt aatatttagc catccctagt gtgggccagg ggcaggagag 15660
ccatttctgc ctgagaaggt tgaacagact acagcccaga gctgtggcct tggggtggct 15720
gtctagcctg ggatggccct cctactctcc tctgtgcttc agtggtagat cctgtggagg 15780
cactgggcca gggtccttgg ggacccatca tggaagccat gtctgctgat ccatatgtct 15840
agaaggggag gcatctgtcc atccccactc ctgaagtcaa gggacccctt ggtcttgcag 15900
ggtgaccttg ggccaaggcc agccacctct gagctccatc ttctgctttg tgaggtagag 15960
gtaagagcct ttggcattga caacacagat agagactcgg ttgggtcagg gtggcattgg 16020
ctggcatact cttgggtgta gtgtcaacta aggatgagtt cctaggggca aggctgaagg 16080
tggcatctac catcccatac cacgttctgc tgcctaagga cgcctgaaga caactgtgag 16140
actggcagtg tacactgctt ccaagctgag gacattgtta gaatcccaga tccttgaaac 16200
aatgagttta tcttgttagg acccaagatc ccactcctag gtggagtcaa agccttggga 16260
gcggcacctt attggggctc agtgaggtca tgtgacatgg aaaccatgta gtgttactat 16320
gtagcaagaa gttagaaagc catgcaacaa acctgtaatt ccaactcctg aggcagaggc 16380
aggtggatct ctgatttcaa aggtagctcc agatacactg tgagaccttc tcaaaacaag 16440
acaagcagac tggagagata gctcagaggt taagagcact ggctactctt ctagaggtcc 16500
tgagttcaat tcccagcaac cagttggtgg tcacaaccat ctgtaatggg attcgatacc 16560
ctcttctggg gtgtctgagg agaatgacag tgtagtcaca tacgtaaaat aaataaatct 16620
ttaaaaaaag aaaagaaaaa tgaaaaggga gggggtgat ggagacatgg ctcagacagt 16680
tttttgtttg ttttggcttt ttggtgtctt ttcttctttc tttctttctt tctttctttc 16740
tctctctctc tctctctctc tctctctctc tctctctctc tctctctttc tctatctttt 16800
ctcttcttgt cttgtcttgt atctgtgtag cagccctggc taccctggaa ctcacctata 16860
ggtcaggcta gccttgaact cagagatcag cctgcctttg ccttccaatg ctgggttaaa 16920
ggtgtgctcc agcactgtcc actctggacc acttgttctt gtattcacct agttctcaca 16980
gggtggctca tgacagttcc aggcgacctg acctcttagg gcaccaggca catgtgcgca 17040
tattcataca cacaggcaag acgttcacac acgatacata caatacaaga cgtatctttc 17100
taaaaattaa agaaaataaa tgagaagcga gagggaaagg agggagagcc actgtttgga 17160
ctggaccacc actggcaggg aaatgccaga aggttcagac agcattcagg gtttgtggct 17220
ggtgggggca gggataaagg aggagttttc taaaggaaca tgtggtctgt gcgtgtgtgt 17280
gtgtgtgtgt gtgtgtgtgt gtgtgtgtgt gtgttactag gggtagactc cagggccttc 17340
caccatgctg ggcaaacaat ttacactgag ctatattttc gaccccctct ttattttatt 17400
tttattttat tttatatata gctctgactg gcctagaact cattatgtaa actaaggtga 17460
cctcaaactc acccatactc aaatgagatt cacctgcctc tgcctcctga gtgctagaga 17520
cagtcatgaa gcaccacgcc caccttctct ttaaaaaaaa aaaaaagcct attgcttttg 17580
tttttatttt tgtctgtgga gggtggtgcc cacacccagt ggtgccagtt tggaatcagt 17640
tctcctgccc tattctacct agggatcgaa cgcaggtggg taggtttggc agcagctgct 17700
tttaccccac gagccatctc gctggcctgt agccctttgt gtgtctgtct gtctgtctgt 17760
ctgtctgcat ggagtggagt ctcatgtagc tcaggctggc ctgcgagtta tgatcttcct 17820
gaccctggta tccaagtgcg gagactgcag gctcactctg ccacgccctt ttacacagtt 17880
ctgggatgga ctctgcatgt caagtgagtt atagccacgg ccctatttta ttttattatt 17940
tttgagatcg ggccccactc atcatggagg ctaaggtcgg cctcctgagt ggtgggatga 18000
caggcgtggg tcgatgtgcc tggctcctac tctattcttt tactttcaca catacccact 18060
acaggaagcg agtagcgagc ccccttttac cagcaccccg ccatgtgaca ttccgcagaa 18120
ccagcgccat ctgtgtgttt attcaatggt tttggttgta tttaaagcaa attccccaca 18180
ctggtgcttc ttacccggaa acgcctccat ctgtagctac aaaaccaggc cattccttac 18240
ataacggcag cgttgcctaa ctggaaccga tctttaagac ccaatccagg cccacgggag 18300
agttctgcta ggccttagaa catctttgtg ggtggttcgt ttgaagtttg atggcttcct 18360
tcctcccctg ctccccttttc tccttgccat tttgggcact agagtttaaa gaaacccgcc 18420
tgccactgaa ctaaatcccc agattttttt tttttaagt tttttttcga gacagggttt 18480

ctctgtgtag ccctggctgt cctggaactc actctgtaga ccaggctggc ctcgaactca 18540
gaaatccacc tgcctctgcc tcccgagtgc tgggattaat gtcttatgta tcccagactg 18600
gtcttgaact tgtggtcatg cctggagtat cctattttga ttgtttatgt gtatacccct 18660
aatatggatg tcagagattc atatcatatg tgccgtcttt ggtcatcctc catacttttg 18720
ggggatgtat gggggcaggg atggagtcta gcctgggctg tggtaacaca cgcttttcat 18780
tccagcatta aagcagagat aggtcgatct ctgagttcta ggacagcctg ggctacacag 18840
ggaaattctg tctcaaaaaa aaaaaaaaac aaaaagaaag aaagaaagga aaagaaataa 18900
gagtctccct atatagccct ggctgtccta gaacttatta tgtagaccac gctggtcttg 18960
gactcacaac agagatccat ctgcctctgc ctcctgaatg tccactctct gcttggagac 19020
aagaatcgct cagtggcccg gattcaggga ttcagctaag ctggctggtc cctgagctcc 19080
aggcctccat ctgtcttggc ttccctgtga caggattatc ggctcatgcc accgcgcctg 19140
gctcttacac cattcctagc gatctgaatt taatactcta tggtcaagcc atccttcaat 19200
gctccctccc ctcctgttga gacagagtct tactcactcg gtagcctggg gtcccagaac 19260
tcacagttag tctcccgttt gtttcctgag tggtgagatc gttcctggct agccttggct 19320
gctgatttgg tagcctggtc ccgcccactg cttaggatta tcactgagga gtttaagcca 19380
cacttcagga agccctgtgg atcctggaat ctgagtgaga caagggtcac acagtggcct 19440
gaccggctct ggtctctctc tgcaggaatg gacctgtcag ccaatcagga tgaagagacc 19500
gatcaggaga ccttccagct ggagatcgac cgcgacacaa gaaagtgtgc ctttcgcacc 19560
cacacgggca agtactggac actgacggcg accggaggtg tgcaatccac tgcgtccacc 19620
aagtgagtga caccctacac cccttcatca cctggcctgg ctcttccccc aggactgggc 19680
agcctgctcg atgcccccac gttgccagcc cctcttctct tccccaggaa cgccagctgc 19740
tactttgaca tcgagtggtg tgaccgccgg atcactctga gagcctccaa cggcaagttt 19800
gtgaccgcca agaaaaatgg ccagctggcc gcctcggtgg agacagcagg tagtcacttg 19860
ggggcacgta ccctaagcct gtttccctag taccccgtgg tcaaccatca gtcccacctg 19920
gacctctctg tgtgttcagg gaatccctgt aagctggtgt accctcaggc cagcagcctg 19980
tgacccttag cttcttggta tccctctctg ctgagccatt ccctgactgg cccatctttg 20040
ttgctgtgaa gctcactggc tccctttccc tgtggcaggg gactcggaac tcttcctcat 20100
gaagctgatt aaccgcccca tcatcgtgtt ccggggggaa cacgggttca ttggctgccg 20160
caaggtcacg ggcactctgg atgccaaccg ttccagttac gatgtcttcc agttggaatt 20220
caatgacggc gcctacaaca tcaaaggtgg gttcactggg tgaggatgca cctggccatt 20280
caaagccgac attagggaac gggtgtccta tgaccgcctg ggtagcccc tcccccttg 20340
tcttagaact ttcctggccc aacctgggca gacagcgaag gtgggaagca gctagggaga 20400
gtggtcgtgg ctccaatctg ggaatcggag acaggagaat tatctttttt tttttttgg 20460
tgttgaggac agaacccagg gctttgagct tattaggcaa gcattctact gctgagttaa 20520
atccccaacc ccaagaatca tctcaaaaag aaagaaagaa aagggaaaaa gaaggtggag 20580
cctggtggtg gtggtggcgg cggcggcggc acacgccttt agtcccagca ctcaagaggc 20640
agaggcaggt gggtctctga gttcttgcca gcctgctcta cagaatacat tctaagacag 20700
ccagagctat gcagagaaac cctgtctcaa aaacaaacaa aaaaaggggg tggggagaga 20760
agatagaaga taaggatatt ccttcttagc tagctgtggg accaaaacca gtgagaggac 20820
acagcaatcc agaggtgtca gtcagaatca gagccctgtg ctgtgtgtgg ccttagcaag 20880
gcaaacggga ctcctttctt acagtgctga ggactggact cagagctaag atccagcccc 20940
tccctgggg attctaggca ggggctctac cactgagcca cgcccccagc ccctcactgg 21000
gggattctag gcaggtgttc taccactgag ccacgccccc agcccctcac tgggggattc 21060
taggcagggg ctctaccact gagccacacc cccagcccct cactggggga ttctaggcag 21120
gggctctgct gctgatatat gtcctttttg tttgtttact tgtttgtttg tttgtcttat 21180
tgtggcccta gctgtcctag aacttgcttt gtagaccaga gatatacctg tctctgcctc 21240
ccaaatgttg gatttaaagg catgtgccac catgccccac cctagcctag ccctcttatg 21300
ttttgtagtt tgagatggca tgtcagtaga tttcccagga tggccttgaa tctaggtaat 21360
cctcctgcct cagtttcttg agtatttgaa ttatgggact gtaccttaca tccagctgag 21420
cccactcagt tctggaagct gacagcggga gggagtgtta atcctttggg gactgacaag 21480
gtggggttaa catgtagtct ccctcctcag actccacggg caagtactgg acggtgggta 21540
gtgattcctc ggtcaccagc agcagcgaca cccctgtgga tttcttcctt gagttctgtg 21600
actacaataa ggtggctctc aaggtgggcg gccgctacct gaagggggac cacgctgggg 21660
tcctgaaggc ctgcgcggag actatcgacc ccgcctcact ctgggagtac tagggccacc 21720
tgccctctgc aggccgctct cgtcagtccc tcctgttatc cttactcatc gggtggccct 21780
gcagcaggtg gcaaacccct tgcctttcaa actggaaacc caagagaaaa cggtgccctt 21840
gctgtcaccc tctgtggacc ccttttccct aactcactgc tccccatggg tcggtggctg 21900
cagactgtcc ccaggaggga ctctggttcc ctctgtcccc ttctttccat ggggaactct 21960
ggcacctttc ttctgacctc agtcaactct gagccttatt tccccccagg aagtggccta 22020
ggagaagcta caggcctag ggacttaccc tgagcttgta actggaagac cccgtcccta 22080
tccccgctcc cgccccacc ccaccccacc cctgctctgg ccccagcctc tggaggccag 22140
ccttttggcg ggactgaagc cgggcatggc caacccttgcc cacaagtgtt tttctggatc 22200
ttggctggaa ggcagtctgt cccatcctgc agtgtttggg cctggctctt tgactcaaag 22260
ctagctaggt ggcactccgt gtcgctcctg cacattctgg aaggggcggg cctctcaccc 22320
acctcattcc ttttcccct ggcctgactg gaagcagaaa aatgaccaaa tcagtatttt 22380
tttttttttt ctttaaggaa atgttactgt tgaaaggccc taggcaagcc tgccctgttg 22440
gttgtagtcg tgagtggtct tggggggaga tgcttggctc ctgtccctgc ctccccagcg 22500
ggttccctcc ctccctcctg cctgaccacc ccagctctgg ctctgtgatt ggtgctccac 22560

gtcttcccag acacctcggg gctcctgggc gggagaaagc cggatgtgcc cctccctggg 22620
agccctggag taaacctcag ggggcccttt cccaatcacc cccttccacc gacccctcaa 22680
caccatgcat ctcactctgg gtgtctcgct cctttatttt tttgtaactg tcatttctat 22740
aactctgaag acccatgata gtaagctttg aactggaaaa taaagtaaaa tcaagtctgc 22800
ggcccgtgtg tgtctcaggg gaagtggatg ctggagtggg cagagggccg gctgggaggg 22860
aggcagcgct gattgattgg cagcccgcag actcttgttt cggtctcgga ccctagcggc 22920
ttcagcatat gccttagtaa ctctggactg agaaatggct gacacgaggt tcccgagcct 22980
tacaagctgg gcaggctaga gggttcagca ggtaaagttg tgtgctgtca agcttgatca 23040
gactgagtcc ccagcaccgt gtgaaaagtt gggcacaacg gtgtacaccc accccagggc 23100
ctctggcaag cacagagaca agaggatcct tggagcctgc tggcggccca accttgccca 23160
atctgtgatt cccattgagt gagccataag gtggagatgg gttttggaag ataccccaca 23220
taggcctgcg gcccccccaca cccggaaata aaaacaagcc aggcttgtca tcccagttat 23280
tcggggagct gaggcaggag gatcacagat ttaagactgc ccaggttgaa gagtaagttc 23340
aagggcagcc tggacatctt gctagattgt ttttttgttg gttggttggt ttttgttttg 23400
ttcttttttt cgagacaggg tttctctgtg tagccctggc tgtcctggaa ctcactctgt 23460
tgaccaggct ggcctcgaac tcagaaatcc gcctgccttt gcctcccgag tgctaggatt 23520
aaaggtgtgc gccaccatgc caggcttaga tgttttaaat gaagggctgt ggagtgtggt 23580
tcacgggtag cctcgctcag acaggctggg gcaggagaaa tggatggctc agcattggct 23640
gctgccttag ggtttccatt gctgtgaaca gacaccatga cctggcagct cttctgaagg 23700
acattggact ggggctggct tacaggttca tcaaggcagg aagcaaagca caggcatggt 23760
gcaggagggg ctcaagtcta cgacccgggt ctcaaagccc acccccacag tgacagactt 23820
tctccaacaa ggccacaccc actccatcaa ggccacacct cctagtagtg ccactccttg 23880
agccaagctt attcaaacca ccaatcacaa ctgcttttct agaggaccca ggttcaattc 23940
ccagcatcca catgacagct tacaattgta actacaattc caggggatcc aacaccctta 24000
cacaggcaca cttacaggaa aagcaccaat cagtgcacat aaaaatacat aaatcgttag 24060
agagaagaga ccattagcta ccttcaccct taacaggcaa gctgaggtcc tgggagtgtg 24120
ttagaggtcc gcagcttgga gtctataggg tagtggcatc gtctatacgt tccttccttg 24180
acaggtaggt acttagctga gacctctggg gtatcccaga gacccatttt acagagggag 24240
gcactgaggc ggagggaaaa gacaaccttg tttgctggct cagaagagtg ccagtggggt 24300
acccacccac aaggctcagc atcccgaggc actgtggagg cagttacaaa gccgcattga 24360
ttggctgctg atgacgaaat gaagagcccc gcggtgtgat tggcggtccc agagggaagg 24420
catgtgggga gattgacagg ggcagatggc cacaaagggt ccagtgactt cccgcagggt 24480
ggaagctctg ggcccaagcc agtaaagtgt caatgacatg gagagacaga tgaatccttc 24540
ccagttgaat tctcaggctc tggtggggcc gcgctgggga gaaaagacac cctgtccgtt 24600
acaaccacca agcgcagcca ggctacgtga tgactggggg aagtcccaga cgtgacttcc 24660
tgcagcactg gaggggaggg tccgacggag gaggctgaga gtctgttgga ggcagccgag 24720
gatcggaggg aagaggcaca ggtcttacat gggaggcata aagtctggtg ggagtccaag 24780
ggaggacgga tgtagggtga tgggaagaaa ggacctaatc cagaaaaagg aacagaagag 24840
ccacctacct ggttagggga cgctggccct ccgcagggaa agctgtcaaa gaaaggcctg 24900
gctaatgaga ccatgtcgtg gggggcccag gcaactgtgg aggcatcaga caccttgggg 24960
caaggagggc acccatgagc tagtacacac cactaagtat ctcagagtcc tgcacacaca 25020
ctcattgaca caggtgggtg agcagcaccc acgggcagct gaagctgagt atcctgcccg 25080
ccccttttgtt tcttccaact gagcagggac aagaaggatc cctatgaata tggctttttt 25140
tgtttgtttg tttggttggt tgggtttttg acttggtttg gtttttccag acagggagta 25200
aagacataca ccaccataac ccagctttgt aaatctggtt tttgtgtgtg aacctatgaa 25260
ttccactcct gttgcactga tgggaaattg aggcaaagtc tcaaagaagc tgacagcaca 25320
ggctagtgtg ttcattttct ctctacaaaa agaggaaaaa ttggggctgg tgagatggct 25380
cagcggttaa gagcattgac tgctcttcca aagcaggtcc cgagttcaaa tcccagcaac 25440
cacatggtgg ctcacaacca tctataatga gatctgagac cctcttctgg ggtatctgaa 25500
gacagctaca gtgtacttag atataataaa taaataaatc tttaaaaaaa ggaaaaatta 25560
tttattattt tattattaaa ttaattgttg tatttattta ttttattaat ctatatttaa 25620
tttatttttt tattttgtgt gcatgagtgt tttgcctgca tgtgtgtgca ctacctgtgt 25680
gtccactgcc tatggaggtc agaagaaggc gttgggtccg ggaactgggg ttgtatggtt 25740
gttagccgcc gcgttcatgc tggttctctg cgaggcggca cttagtcctg agccatcttc 25800
tgtcagctcc tctactttct tttagacagg gtctcctgta gctcaggctg ggcttcagct 25860
tgcctggtgg ttaagggtga ctaagttaaa tcatcttaca tttctgcctc acttccacct 25920
cctgagtgct gggcttgcag gcatgtgcca ctgggatggg ttttgtttcc tgctggcttc 25980
tcaccccgta gctccgtgtg ttgggtcagg gccagccctg tgcccactga gccattaacc 26040
ctactgagcc cactgtaggg aaaaaccata gaggagcttc tatgaccctc atgggtccct 26100
ccaggtctgt cagagttcag gaggcatgga tctctctggc agggagctgt tgactttagc 26160
cttggaattt cccaaccaag tctacactga gcaccctgtc gacgacctgc ttctgactcc 26220
tcctgtgagg ctcttttgga gtgatcttag tccccacagg gcccccatct tctccagagc 26280
tgagtggcct ctcctactgg acagagaaga gctgtcctga cttggttaaa atgttgaatg 26340
caaggagcgg aggaaacggc ccagctggtg aagtgtttgg ctcgatcccc agagctgtgg 26400
agttcccaac aggatggggc tcactggctg ccctgatcag caagctcaga aaccaagatg 26460
gatagctcct aaggactgga attggaggat gacccctggt gacagacaca cttgcatatg 26520
catctgtctg cacacataca catatgcatg taaaaccctg atccacgtgg tagtgcctga 26580
ctgtaatccc agtacttggg agacaggaac agagaatcac cttcagtttg aggccaacct 26640

cgtctacata tccagcacct taggagactc tgtttccaac tcttcagggt ggctcacatc 26700
tctaatctca accctcaaga agccaagact ggaagatgcc tagagaggaa ggtttgcttg 26760
ggttcagtgc cagcctccag aggcagaggc aagcagattt ctgagttcga ggccagcctc 26820
tacagagtga gttccaggac agccaggcag ccagggctat acagagcaac cctgtctcaa 26880
acaacaacaa taacaacaac aacacaacaa gaaaacttga gtgtgcgtgt gcgtgtgcgt 26940
gtgcgtgtgt gtgtgtgtgt gtgtgtgtgt gtgtttacta tttacagtaa acgagtcttt 27000
ggggaggcag ccaaagtagg cgaggaactc cttgtcccct gctgggctct ctgggttgag 27060
atcttgtctt gatggggaga taggaaggga ttctctgtcc ctcagggtgc tatcagcagt 27120
gggaacagat ggggtcggga gagagactgg gggaggggag aatcagggtg gaggagggct 27180
gtgatttagg tttaccatga aaccggagtc atcaccggtg ggagaggctg gtcagagttc 27240
ctctttttc ctcttctttc cctaggaccc aggcctgatc cggtggggag gggaatgatt 27300
ggaaaatgct gccttctaga gtccctagcg agagccacac cccaggcctc agccatcaat 27360
gacaaccttt ctgttctcac tgaaagcaat taaagatctg taagatgagt cagtgttacc 27420
cattgtaagc tgctgactgc cttggtccca tccctgggac ccacatggtg gaaggaaaga 27480
atggactgat gcccttggac ccccacgtgt gtgctgtggc acacacacac acgcatgcac 27540
acaaaaataa gtaatgtagt tcaaaattta gaataaatca ggcgtggtgg tgcacacctt 27600
taatcccagt actcgggagg cagaagcaag tagatctctg tgagtttgag accagcctgg 27660
tctacagagt gagtttcaag ccagccatag atagctacat agccagatct tgtgttagcc 27720
tcctccttct accaacagaa attaagatca caaataaaca ggaaagaggg tggaagagac 27780
aactcagtgg ctatgaacat ttgctgttct tgcagagaaa ccaggattga gtcccggcac 27840
ccacatagtg ggtaaacatt atctctagct caaattccag aggacctggc ttctgagagc 27900
acggtacaca tggggtgttc tctctctctc tctctctctc acacacacac acacacacac 27960
acacacacac acacacacac gcaggaaaaa cagaaaagca cataaaataa aaacaaatat 28020
ataaaaatag aatccaaggg gattcaacga tgtcatgagt ccacagaaat cctatcctcc 28080
cctttttcgg ggtgttacac gggcggatca aacccagggn nnnnnnnnnn nnnnnnnnnn 28140
nnnnnnnnnn nnnnnnnnnn nnnnnnnnnn nnnnnnnnnn nnnnnnnnnn nnnnnnnnnn 28200
nnnnnnnnnn nnnnnnnnnn nnnnnnnnnn nnnnnnnnnn nnnnnnnnnn nnnnnnnnnn 28260
nnnnnnnnnn nnnnnnnnnn nnnnnnnnnn nnnnnnnnnn nnnnnnnnnn nnnnnnnnnn 28320
nnnnnnnnnn nnnnnnnnnn nnnnnnnnnn nnnnnnnnnn nnnnnnnnnn nnnnnnnnnn 28380
nnnnnnnnnn nnnnnnnnnn nnnnnnnnnn nnnnnnnnnn nnnnnnnnnn nnnnnnnnnn 28440
nnnnnnnnnn nnnnnnnnnn nnnnnnnnnn nnnnnnnnnn nnnnnnnnnn nnnnnnnnnn 28500
nnnnnnnnnn nnnnnnnnnn nnnnnnnnnn nnnnnnnnnn nnnnnnnnnn nnnnnnnnnn 28560
nnnnnnnnnn nnnnnnnnnn nnnnnnnnnn nnnnnnnnnn nnnnnnnnnn nnnnnnnnnn 28620
nnnnnnnnnn nnnnnnnnnn nnnnnnnnnn nnnnnnnnnn nnnnnnnnnn nnnnnnnnnn 28680
nnnnnnnnnn nnnnnnnnnn nnnnnnnnnn nnnnnnnnnn nnnnnnnnnn nnnnnnnnnn 28740
nnnnnnnnnn nnnnnnnnnn nnnnnnnnnn nnnnnnnnnn nnnnnnnnnn nnnnnnnnnn 28800
nnnnnnnnnn nnnnnnnnnn nnnnnnnnnn nnnnnnnnnn nnnnnnnnnn nnnnnnnnnn 28860
nnnnnnnnnn nnnnnnnnnn nnnnnnnnnn nnnnnnnnnn nnnnnnnnnn nnnnnnnnnn 28920
nnnnnnnnnn nnnnnnnnnn nnnnnnnnnn nnnnnnnnnn nnnnnnnnnn nnnnnnnnnn 28980
nnnnnnnnnn nnnnnnnnnn nnnnnnnnnn nnnnnnnnnn nnnnnnnnnn nnnnnnnnnn 29040
nnnnnnnnnn nnnnnnnnnn nnnnnnnnnn nnnnnnnnnn nnnnnnnnnn nnnnnnnnnn 29100
nnnnnnnnnn nnnnnnnnnn nnnnnnnnnn nnnnnnnnnn nnnnnnnnnn nnnnnnnnnn 29160
nnnnnnnnnn nnnnnnnnnn nnnnnnnnnn nnnnnnnnnn nnnnnnnnnn nnnnnnnnnn 29220
nnnnnnnnnn nnnnnnnnnn nnnnnnnnnn nnnnnnnnnn nnnnnnnnnn nnnnnnnnnn 29280
nnnnnnnnnn nnnnnnnnnn nnnnnnnnnn nnnnnnnnnn nnnnnnnnnn nnnnnnnnnn 29340
nnnnnnnnnn nnnnnnnnnn nnnnnnnnnn nnnnnnnnnn nnnnnnnnnn nnnnnnnnnn 29400
nnnnnnnnnn nnnnnnnnnn nnnnnnnnnn nnnnnnnnnn nnnnnnnnnn nnnnnnnnnn 29460
nnnnnnnnnn nnnnnnnnnn nnnnnnnnnn nnnnnnnnnn nnnnnnnnnn nnnnnnnnnn 29520
nnnnnnnnnn nnnnnnnnnn nnnnnnnnnn nnnnnnnnnn nnnnnnnnnn nnnnnnnnnn 29580
nnnnnnnnnn nnnnnnnnnn nnnnnnnnnn nnnnnnnnnn nnnnnnnnnn nnnnnnnnnn 29640
nnnnnnnnnn nnnnnnnnnn nnnnnnnnnn nnnnnnnnnn nnnnnnnnnn nnnnnnnnnn 29700
nnnnnnnnnn nnnnnnnnnn nnnnnnnnnn nnnnnnnnnn nnnnnnnnnn nnnnnnnnnn 29760
nnnnnnnnnn nnnnnnnnnn nnnnnnnnnn nnnnnnnnnn nnnnnnnnnn nnnnnnnnnn 29820
nnnnnnnnnn nnnnnnnnnn nnnnnnnnnn nnnnnnnnnn nnnnnnnnnn nnnnnnnnnn 29880
nnnnnnnnnn nnnnnnnnnn nnnnnnnnnn nnnnnnnnnn nnnnnnnnnn nnnnnnnnnn 29940
nnnnnnnnnn nnnnnnnnnn nnnnnnnnnn nnnnnnnnnn nnnnnnnnnn nnnnnnnnnn 30000
nnnnnnnnnn nnnnnnnnnn nnnnnnnnnn nnnnnnnnnn nnnnnnnnnn nnnnnnnnnn 30060
nnnnnnnnnn nnnnnnnnnn nnnnnnnnnn nnnnnnnnnn nnnnnnnnnn nnnnnnnnnn 30120
nnnnnnnnnn nnnnnnnnnn nnnnnnnnnn nnnnnnnnnn nnnnnnnnnn nnnnnnnnnn 30180
nnnnnnnnnn nnnnnnnnnn nnnnnnnnnn nnnnnnnnnn nnnnnnnnnn nnnnnnnnnn 30240
nnnnnnnnnn nnnnnnnnnn nnnnnnnnnn nnnnnnnnnn nnnnnnnnnn nnnnnnnnnn 30300
nnnnnnnnnn nnnnnnnnnn nnnnnnnnnn nnnnnnnnnn nnnnnnnnnn nnnnnnnnnn 30360
nnnnnnnnnn nnnnnnnnnn nnnnnnnnnn nnnnnnnnnn nnnnnnnnnn nnnnnnnnnn 30420
nnnnnnnnnn nnnnnnnnnn nnnnnnnnnn nnnnnnnnnn nnnnnnnnnn nnnnnnnnnn 30480
nnnnnnnnnn nnnnnnnnnn nnnnnnnnnn nnnnnnnnnn nnnnnnnnnn nnnnnnnnnn 30540
nnnnnnnnnn nnnnnnnnnn nnnnnnnnnn nnnnnnnnnn nnnnnnnnnn nnnnnnnntg 30600
actgctcttc caaggtcttg agttcaatcc cagcaaccac atggtggctc acaaccatcc 30660
gtaatgagat ctgacgccct cttctggtgt gtccgaagac agctacagtg tacttacata 30720

taataaataa ataaatctta aaaaaaaaag aaagaaagaa agaaagaaag agagagagaa 30780
agaaagaaag aggtgagggc tgggtgtggt ggcgcacacc tttaatccca gcacttggga 30840
ggcagaggca ggtggatctc tgtgagtctg aggccagctt ggtctacaga gtgagttcca 30900
ggacagccag ggctacacag agaaaccctg tcttgaaaga aagaaagaaa gaaagaaaga 30960
aagaaagaaa gaaagaaaga aagaaagaaa gaaagaaaga aagaaaggaa ggaaggaagg 31020
aaggaagagg tgaataggat aaaatgaggt ccaataggtt gggggcatgg ctcagtgtta 31080
gagggcttgc ctagcaagtg agaggcctgg gttcaacctc tggcactgaa gagggatggt 31140
aggtaggtag gtaggtaggt aggtaggtat ctagatagat agatgataaa taggtagatg 31200
gatggtagat atatagataa tagatgtggg atagatagat gatagagata tacatagatg 31260
atagattata gatagataga tagatagata gatagataga tagatagata gatagacaga 31320
caaatggtag gtatatagat gatagaaaga tgagggataa atagatggta gataagtaga 31380
tgatagtagg tagatgagag ggagagagag agagagcgag agagagcgag aaaggatata 31440
ataaggtcat taggctaatt tgatccaaag acagattggg gttcttataa gacaagtgat 31500
caagcaggcc agatatgggg ggaactgatc tgtaattcta gcacctgggg tgggagatgg 31560
aggcaggagg aaggggaggt cgagggtatc cgcagatact tagtgagttt gaggctatcc 31620
ccagccacgt gagactctgg caaatccttc cgcactaaca acaaaaattc tgaaaggcaa 31680
acctccctta actcataaag catgaattgg ggcgtatgcg ggccttcaga gccatgtggt 31740
gtgtttggga acagacgaca ggaaacactg ttgagaggac tcagacccag agaggaaggg 31800
acagcagagt caccatgtgg acaggcgttg gggcttgctg ggccgctcac aggaagcagc 31860
gcacaccaga gccatccgcc taggagctgg gccacagttt cctgcagagt gggccctgac 31920
acacccggcc aaagcaagca ctggcctgtg gacagaaagc agacacagga cggagcccgt 31980
tccggggtca gctcactgta cttgaagatg tgagcccaaa gaagtggggg gtgccacggc 32040
gtcttttgca gatgttacta tgccagttgg tgttagacac ttagatatat ttttaagaag 32100
agcagtcggt actcttaact actgaaccat ctctccagcc ccacaatgac attcttcaaa 32160
gagcagagct tagaggcaca ggcacttgtc accaagcagc aacatgagtc agatccctga 32220
aaatccacag gcagaaggag agagctgact cctgtccgtt gaccttcatg tgaatacagt 32280
ggtgtgtggt ggtgtgtgtg tgtgtgtgtg tgtgtgtgtg tgtgtgtgtg catttatgcc 32340
atgtgtgtca agagtgtgag gaggacccaa gagagttcct agacttaaag ctggttnnnn 32400
nnnnnnnnnn nnnnnnnnnn nnnnnnnnnn nnnnnnnnnn nnnnnnnnnn nnnnnnnnnn 32460
nnnnnnnnnn nnnnnnnnnn nnnnnnnnnn nnnnnnnnnn nnnnnnnnnn nnnnnnnnnn 32520
nnnnnnnnnn nnnnnnnnnn nnnnnnnnnn nnnnnnnnnn nnnnnnnnnn nnnnnnnnnn 32580
nnnnnnnnnn nnnnnnnnnn nnnnnnnnnn nnnnnnnnnn nnnnnnnnnn nnnnnnnnnn 32640
nnnnnnnnnn nnnnnnnnnn nnnnnnnnnn nnnnnnnnnn nnnnnnnnnn nnnnnnnnnn 32700
nnnnnnnnnn nnnnnnnnnn nnnnnnnnnn nnnnnnnnnn nnnnnnnnnn nnnnnnnnnn 32760
nnnnnnn 32767

<210> 2
<211> 2634
<212> DNA
<213> Mus musculus

<400> 2
gagctaagcc gtgttgaaca aaggaggtcg ggcacagcta tccaagctcc cggggccacc 60
gggccgccct ccgccaccat gaccgccaac ggcacggcag aggctgtgca gattcagttc 120
gggctcatca gctgcggcaa caagtacctg acagccgagg cgttcgggtt caaggtgaac 180
gcatccgcta gtagcttgaa aaagaagcag atctggacgc tggagcaacc tcccgatgag 240
gcgggcagcg cggccgtgtg tctgcgcacg cacctgggtc gctacctggc cgccgacaag 300
gacggcaacg tgacctgcga gcgcgaggtg cccgacggcg actgccgctt tctcgtcgtg 360
gcgcacgacg acggccgctg gtcgctgcag tccgaggctc accggcgcta ctttggcggc 420
accgaggacc gcctgtcctg cttcgcgcag agcgtgtcgc cggccgagaa gtggagcgtg 480
cacatcgcca tgcacccgca ggttaacatc tacagcgtta cccgcaagcg ctacgcgcat 540
ctgagcgcgc ggccggccga cgagatcgcg gtagaccgcg acgtgccttg gggcgtcgac 600
tcgctcatca ccttggcctt ccaggaccaa cgctacagtg tgcagacgtc cgaccaccgc 660
ttcctgcgcc acgacgggcg ccttgtggca cggccggagc ccgcccacggg cttcacgctg 720
gagttccgct ccggcaaggt ggcctttcgc gactgcgaag gtcgctacct ggctccgtcc 780
gggcccagcg gcaccctcaa ggctggcaag gccaccaagg tgggcaaaga tgagctcttc 840
gccctggaac agagctgcgc tgaggtggtg ctgcaggcgg ccaacgaggg gaacgtgtcc 900
acgcgccagg gaatggacct gtcagccaat caggatgaag agaccgatca ggagaccttc 960
cagctggaga tcgaccgcga cacaagaaag tgtgcctttc gcacccacac gggcaagtac 1020
tggacactga cggcgaccgg aggtgtgcaa tccactgcgt ccaccaagaa cgccagctgc 1080
tactttgaca tcgagtggtg tgaccgccgg atcactctga gagcctccaa cggcaagttt 1140
gtgaccgcca agaaaaatgg ccacgtggcc gcctcggtgg agacagcagg ggactcggaa 1200
ctcttcctca tgaagctgat taaccgcccc atcattgcgt tccgggggga acacgggttc 1260
attgcgtgcc gcaaggtcac gggcactctg gatgccaacc gttccagtta cgatgtcttc 1320
cagttggaat tcaatgacgg cgcctacaac atcaaagact ccacgggcaa gtactggacg 1380
gtgggtagtg attcctcggt caccagcagc agcgacaccc ctgtggattt cttccttgag 1440

ttctgtgact acaataaggt ggctctcaag gtgggcggcc gctacctgaa gggggaccac 1500
gctggggtcc tgaaggcctg cgcggagact atcgaccccg cctcactctg ggagtactag 1560
ggccacctgc ctctgcagcc gctctcgtca gtcctcctgt tatccttact catcgggtgg 1620
cctgcagcag gtggcaaacc ccttgccttt caaactggaa acccaagaga aaacggtgcc 1680
cttgctgtca ccctctgtgg acccctttc cctaactcac tgctccccat gggtcggtgg 1740
ctgcagactg tccccaggag gactctggtt ccctctgtcc ccttctttcc atggggaact 1800
ctggcacctt tcttctgacc tcagtcaact ctgagcctta tttcccccca ggaagtggcc 1860
taggagaagc tacagggcct agggacttac cctgagcttg taactggaag accccgtccc 1920
tatccccgct cccgccccca ccccacccca cccctgctct ggccccagcc tctggaggcc 1980
agccttttgg cgggactgaa gccgggcatg gccaaccttg cccacaagtg tttttctgga 2040
tcttggctgg aaggcagtct gtcccatcct gcagtgtttg ggcctggctc tttgactcaa 2100
agctagctag gtggcactcc gtgtcgctcc tgcacattct ggaagggggcg ggcctctcac 2160
ccacctcatt ccttttcccc ctggcctgac tggaagcaga aaaatgacca aatcagtatt 2220
ttttttttt ctttaaggaa atgttactgt tgaaaggccc taggcaagcc tgccctgttg 2280
gttgtagtcg tgagtggtct tggggggaga tgcttggctc ctgtccctgc ctccccagcg 2340
gttccctccc tccctcctgc ctgaccaccc cagctctggc tctgtgattg gtgctccacg 2400
tctccagaca cctcggggct cctgggcgga gaaagccgat gtgcccctcc ctgggagccc 2460
tgagtaaacc tcaggggcc ctttcccaat cacccctcca ccgacccctc aacaccatgc 2520
atctcactct gggtgtactc gctcacattt attttttgt aactgtcatt tctataactc 2580
tgaagaccca tgatagtaag ctttgaactg gaaaataaag taaaatcaag tctg 2634

<210> 3
<211> 1482
<212> DNA
<213> Mus musculus

<400> 3
atgaccgcca acggcacggc agaggctgtg cagattcagt tcgggctcat cagctgcggc 60
aacaagtacc tgacagccga ggcgttcggg ttcaaggtga acgcatccgc tagtagcttg 120
aaaaagaagc agatctggac gctggagcaa cctcccgatg aggcgggcag cgcggccgtg 180
tgtctgcgca cgcacctggg tcgctacctg gccgccgaca aggacggcaa cgtgacctgc 240
gagcgcgagg tgcccgacgg cgactgccgc tttctcgtcg tggcgcacga cgacggccgc 300
tggtcgctgc agtccgaggc tcaccggcgc tactttggcg gcaccgagga ccgcctgtcc 360
tgcttcgcgc agagcgtgtc gccggccgag aagtggagcg tgcacatcgc catgcacccg 420
caggttaaca tctacagcgt tacccgcaag cgctacgcgc atctgagcgc gcggccggcc 480
gacgagatcg cggtagaccg cgacgtgcct tggggcgtcg actcgctcat caccttggcc 540
ttccaggacc aacgctacag tgtgcagacg tccgaccacc gcttcctgcg ccacgacggg 600
cgccttgtgg cacggccgga gcccgccacg ggcttcacgc tggagttccg ctccggcaag 660
gtggcctttc gcgactgcga aggtcgctac ctggctccgt ccgggcccag cggcaccctc 720
aaggctggca aggccaccaa ggtgggcaaa gatgagctct tcgccctgga acagagctgc 780
gctgaggtgg tgctgcaggc ggccaacgag gggaacgtgt ccacgcgcca gggaatggac 840
ctgtcagcca atcaggatga agagaccgat caggagacct tccagctgga gatcgaccgc 900
gacacaagaa agtgtgcctt tcgcacccac acgggcaagt actggacact gacggcgacc 960
ggaggtgtgc aatccactgc gtccaccaag aacgccagct gctactttga catcgagtgg 1020
tgtgaccgcc ggatcactct gagagcctcc aacggcaagt ttgtgaccgc caagaaaaat 1080
ggccacgtgg ccgcctcggt ggagacagca ggggactcgg aactcttcct catgaagctg 1140
attaaccgcc ccatcattgc gttccggggg gaacacgggt tcattgcgtg ccgcaaggtc 1200
acgggcactc tggatgccaa ccgttccagt tacgatgtct tccagttgga attcaatgac 1260
ggcgcctaca acatcaaaga ctccacgggc aagtactgga cggtgggtag tgattcctcg 1320
gtcaccagca gcagcgacac ccctgtggat ttcttccttg agttctgtga ctacaataag 1380
gtggctctca aggtgggcgg ccgctacctg aagggggacc acgctggggt cctgaaggcc 1440
tgcgcggaga ctatcgaccc cgcctcactc tgggagtact ag 1482

<210> 4
<211> 32767
<212> DNA
<213> Homo sapiens

<400> 4
ttgcatttga agctaccatg actgcaagag ggagttcccg aaggggtctg ggaatggccc 60
agggaaatag gggtgaggtg agactccact gccccgacag tgcccatgta tgaattagaa 120
aaagtgggcc aggcgtggtg gctcacacct gtaatcccag cattttggga ggctgagtgg 180
gcagatcatg aggtcaggag ttcgaaacca gcctggccaa catggtgaaa ccccatctct 240
actaaaaata caaaaattag ctgggcttgg tggcacacat ctgtagtccc agctactcag 300
gaggctgagg cagaagaatc gcttgaacct gggaggtgga ggttgcagtg agccgaggtc 360
atgccactgc actccagcct gggtgacaga gtgagacttt gtctcaaaaa aaaaaaaaaa 420
aaaaaaaaga aaagaaagaa agaaaaagaa aaaaagtggg ccaggtgtgg tggctcatac 480
ctgtaatccc agcactttgg gaggctgagg tgggaggatt gcttacagcc aggagtttga 540

```
gaccaggttg gacaacatgg tgagaccttg tctttacaaa aaaatacaaa aacctatctg 600
ggcatggtgt tgcattcctt tagtcccagc tacttaggag gttgaggtgg aaggatcact 660
tgagttaagg gaggagacca cccctcatat tgtcttatgc ccaatttctg cctccaaaga 720
aagaaaaagt aaaaactaaa aggcagaaat gaaaaccaca ggcagacagc ccagcgccac 780
accctgggcc tcgtagttaa agatcgaccc ctgatctaat cggtgatgtt atctatagac 840
tacagacatt gtatagaaat gcactgtgaa aatccctatc tggttttgtt ctgatctaat 900
taccggtgca tgcagccccc agtcacgtac cccctgcttg ctcaatcacg accctctcac 960
gtgcaccccc ttagagttgt gagcccttaa aagggacagg aattgctcac tcggggagct 1020
tggctcttga gacaggagtc ttgctgatgc ccctggccaa ataaacccct tccttcttta 1080
actcggtgtc tgagttttgt ctgcggctca tcctgctaca gagtctagga ggcagaggtt 1140
gcagtaagcc aaattcacgc cactgcactc cagcctgggt gacagagcaa gaccccacca 1200
aaaaaagaaa agaggccagg cgcagtggct cacgcccagc taatttttgt acttttagta 1260
gagacggggt ttcaccatgt tggccaggct gatctcaaac tcctgtcctc aggtgatccg 1320
cccaccttgg cctcccaaag tgctgcgata acaggagtgg aggctcaact ttttaaagaa 1380
gaaaaggacg aatcaggaca ggagacaatt acaagctctg ttcattcgga attctcattg 1440
gcttacagaa ataactttgg ctagtgattg gttatatgtt gtagacagac ccacagggtg 1500
gatggcgtct gtggccactt gggcattagc tggtccagag cccagagtcc atgtagcaag 1560
tagcttcagg acgtaattat ttagctcaat agaaagtgag atgtgactgc tgttactttt 1620
tttttttttt gagacggagt ttcactcttg ttgcccaagc tggagtgcaa tggcgcgatc 1680
tcggctcact gcaacctctg tctcccaggt tcaagtgatt ctcctgcctc agcctcccga 1740
gtagctggga ttacaggcgt gtgccaccaa gcccggctaa tttttgtatt tttagtagag 1800
acagggtttc actatgttag ccaggatggt ctcgaactcc tgaccttgtc atccaccggc 1860
ctcggcctct caaggtgcta ggattacagg catgagccac cacgcccggc cttttaactg 1920
ctgttacttt ttttttttta atcattttta ttctgctagt tccataaaag caatgtaaac 1980
accagcattc tatacatctt gctggtgaac tcacataatg cttagttccc tgatcctttg 2040
acctccttgt cttctccagt tattttctgt ttggaccact ggccacagga atggagtggg 2100
gttggggctt aggagagagc aatgtggctt tttggtatga cttgtttcat tgactgctgt 2160
tacattttaa atgcctttct gggcctgata atttaagggg gctggcattt ctcacatcaa 2220
agggcaaagg gtttttttgt ttgtttctca ctattgctca aaagccacat gtgtcaagga 2280
cagctcttgt taacgggcag agaagcttat agaacctttc cagggctggc gcggttgttc 2340
atgtctgggg gacaagagcg aaactccatc tcaaaaagaa aaaaaaaaaa aagaacattt 2400
ccaggccagg cacagtggct cacacctgta agtcccagca ctttgggaga ttgaggtggg 2460
aggatcactt gaggccagga gtttgagacc agcataggcc acatggcaag aacccagttt 2520
ctacaaaaac attttttttt taaattagct gggctcggtg gtgcacactt gtggtcccag 2580
ctactcggga ggctgaggtg ggagaatcgc ttgagcctgg gaggcggagg ttgcagtgag 2640
ctgtgatcgc accactgcat ttcagcctgg gcaacagagg gagactctgt ctcaaaaaaa 2700
atcaaataaa gaaaaaagaa catttccatt attgcagaat gttctattgg acggcgctgg 2760
gccagatgcc tgccccaagc cctgggacac ccaaacctgg tgaaagtgcc aagctcccat 2820
ctgggggtgc tcacggaagg ggccgggagc tgggattaca agcgtggagg caggcgccac 2880
cccagaggag gtgggtgatg tctgagccag agtctttggg atgagcaggg ctttggcagg 2940
cagctttgtt gggcaggcaa caggcacagc aggtgcaatg gcatcgaggt tggagaggtg 3000
gactggcggg gagaagagaa gggagggtgg caggagagat gggcagaggc cccccaggag 3060
cccagagcct tcagggcttt gggccctctt ggggcactgg ggagccacgg gagaagtgtg 3120
gggtggagag gggcgccctg gatttgacca ccttcaggaa cctaccttgg ctgcccgggt 3180
gggtgggatg gaggtgatga aggtggaggt cgtagcattg tcactgagag cgatgcttat 3240
tctgattttt gcccccgtga gcctcagaaa tcggtcagaa cagtgctcgg gctgggcgtg 3300
gtggctcacg cctggaatcc cagcactttg ggaggccgag gcgggcggat cacctgaggt 3360
caggagtttg agaccagcct ggccaacatg gcaaaacccc gtctctacta aaaacacaaa 3420
aattagctgg gtgtagtggt gcatgcctgt aatccagcta ctccagaggc tgaggcctcc 3480
ctgggaggcg gagtgcagtg agccaagatt gtaccaccgc ccctccagcc tgggtaacag 3540
agtgagactc tgtctcaaaa caaacaaaaa agaacaaacc aaaaccagtg ctccaattca 3600
ccttgctgaa ttggagaaga acaggtgcct gggtcctccc ggctgacggt gacgtcacta 3660
tgagccacca cgcctggccc ggtgggtgtt tacagcagag gtggttcagg gaggagtgag 3720
tggcagatgg tgcaggctca gggaggagtg agtggcaggt ggggcctgag gccagaggca 3780
ggaggaccgt tcaagactga ggtgtcttgg gcactggtgc cggggccggg gagagccctg 3840
gcatgccggg ccccggccct cagtgaaagc aggatgaggt ggcggccaag cagggaggcc 3900
aaggcctggc agcagccggc agcccctctc ctggcaggga catcttggca ggagccgggc 3960
tctgcccagg gcgctaatgt cctcttaatt agccaggcac ttgccaagca cttgccgctg 4020
cggcctcact ttgcagaacg cacttgggtt tggagcagga ccagcatcgg ccaccgggtg 4080
agtagggggg atggccaggt gagtaccatg gccagaaggt gcggcctctg agtccccact 4140
gaagccaatc caggccccga cctctggtca tcatcaacag aagagtgaca gaaacagaaa 4200
agatatcaag gaacacgttt tcagggccag attccaggtc cccacctat tttcctttgc 4260
tcctgccttg cctggctctg ccccttgaa ctcttattca cccagtgtag gcatcacctc 4320
ctcctgaagg ccctcctgga ctgcacagtt aactctggag cctcccctgc acagggcagc 4380
tcatgtatgg atccacaggt gagttcctgt ctttgaaggg ttacagttta ggtcctggag 4440
cagtggtggg gaggtcacac ctccacccac atccctcttt gcaaatccca tgggaactat 4500
gagcctgggt cccccaaact gcatgttccc cacttaaagc tgggccagat cctgtttgct 4560
gtatccaggc ctcagtttcc ctgcttgtaa ccagaagtcc atgcagccag aaattgtcag 4620
```

ggagctgggc acggtggctc atgcctgtaa tcccagcact ttcagaggct gaggcaggag 4680
gttcacttgt gatcaggagt ttgggacccc agcctgggca atacaatgag atccctgcct 4740
ctaccaaaaa attaaaaaca attagccagg cctggaggca tacacttgta gtcctagcta 4800
ttcaagggag gctgaggtgg gaggatcact tgagtccagg agtttgtggc tggggtgagc 4860
tctgattgta ccactgcact ccagcctggg taagagaatg agaccttgtt ttatttttatt 4920
ttatttttat ttattttgag atggagtctc actctgtcac ccaggctgga gtgcagtggt 4980
gcaatgtcgg ttcactgcaa cctctgcttc ccggcttcaa gagattctcc cgcctcagcc 5040
tcccaagtag ctgggattac aggcgtgtgc caccacgcct ggctaatttt ttgtgttttt 5100
agtagagaca gggtttcacc atgtttgcca ggctggactt gaactcctga ccttaggtga 5160
tccatgcccc cttggcctcc taaattgctg ggattatagg catgagccac cgggcctggc 5220
cgagaccctg ttttaaaaaa attaatcagg tccagtgcgg tggctcacgc ctgtaatccc 5280
agcactttgg gaggccgagg agggcagatc acctgagatc gggagttcga gaccagcctg 5340
accaacatgc agaaaccccg tctctactaa aaatacaaaa ttagcagggc gtggtggcgc 5400
acacctgtaa tcccagctac ttgggaggct gaggcaggag aatcacttgg acctgggaag 5460
cagagcttgt ggtgagccga gattgcacca ttccactcca gcctgggcaa aaagagtgaa 5520
actccatctc aaataataat aataataata ataataataa taataataat aattggtaga 5580
gatgttggta gaggcaggac agtgggagtg acagcctggt cctgtcctcc cgggctctgg 5640
ctgaccatgc tgtagtgtgg ctcagggccc tggagggcac agctgtcctg tggccctgtg 5700
gtcagccggg aagccaggcc tggcaggact cctgccctag aatcagctcc tcccactccc 5760
cagggtccca tgcagggcgc tggccgtacc aggcctcctc gcgggtgcca cagagccagc 5820
ggctgcacag caacagagac gccattcaca ggctctcggc cacgaggccg ctcatttaca 5880
gagggctgag ccgctgacat ctgtgcgttt gtaacatgga cagcatttct ggtcccatac 5940
agacgcctgg gccagccaca tttatctccc cttgagagaa gggaaacgag gctcgggagg 6000
gaatgaggga ttggcttggg gtcacacaac tttaatgatg accctgggca ggaagctccc 6060
aggatggcaa cagagactca gggcaggctg ggcatggtgg ctcacacttg tcatcccagc 6120
cctttgggag gcagaggtgc aaggatcact cgagcccagg agctcaaggc cagcctgggg 6180
aatatagtga gaacccatct ctaaaaaaaa aaaaaatagg ccggtcgcgg tgactcacgc 6240
ctgtaatccc agcactttgg gaggctgagg cgggtggatc acgaggtcag gagattgaga 6300
ccatcctggc taacacggtg aaaccccgtc tctactaaaa atacaaaaaa taggccgggc 6360
gtggtggctc atgcctgtaa tcccagcact ttgggaggct gaggccggcg gatcacgagg 6420
tcaggagttc gagatcagcc tgacaaacat gacgaaaccc tgtctctact aaaaatacaa 6480
aaattagccg ggcgtggtga cacgcgcctg tagtcccagc tactcaggag gctgaggcag 6540
gagaatcgct tgaaccatcg aggtggaggt tgcggtgagc cgagatcgca ctactgcact 6600
ccagtctggg agacagagtg agactccgtc tcaaaaaaaa aaaaaaaaat tagccgggcg 6660
tggtggcggg cgcctgtagt cccagctact cgggaggctg aggtaggaga atggcgtgaa 6720
cccgggaggc ggagcttgca gtgagccgag atcgcgccac tgcactaaag cctgggcaac 6780
agagcgagac tctatctcaa aaaaaaaaaa aaaaatttag ccgggcatgg tggtgcatgt 6840
ctgtaccccc agcaatttgg gaagctgagg tgggaggatt gcttaagccc aggagtttga 6900
ggcgggagtg agctgtgatg gcaccactgc actccagcct gggtgacaga gtgagacccc 6960
gtctctccct atcccccgca aaaaaaaaaa aacaactcag ggctttccca cccctgtcct 7020
tgcacagatg aagaaccgaa gcttctagaa ggggtatatt ttgcccccag gccccaaatc 7080
ctggtctttg gactacagtc aagacctagc acagggctca ggcctaaaaa aaagctgttg 7140
aggagggtgt gaggctgcaa cgttgcggtg agaagggggt ccccagggag ggcgcagcag 7200
gaagccccag ggaagtgcct gggagaggga ggttgtgcag ccagaaggag cagcccgcag 7260
cctttggttg ttgactcctg ctttgtaagt ggcagtctgg ttgggtagga cagggtccga 7320
gtcctcactc agggaactga gtccagggtg agctgagcag cccttcctgg tggcctcact 7380
ttcccctgca gagcctgctg catggtgtcc agtggccagc ctgggaggag ctcaggactg 7440
gcctcacctc gtgccacgcc ctcgtaccaa ggtggctcag atggcacctg ggttcccgaa 7500
gggcccagga acacagcgtc catgtcccca tccttccccg gagggacttg ggtggggcc 7560
tgcaggaagg atcatgtgac ttgttcaagg gcagcttgtc ctgccccgga cacagaggtg 7620
cccatcgtaa gcgagatgca ggcaaggtga ggacagggca tggtgccgag caggaatgat 7680
tttcgaaaat gctactctgt ggtcgggcac ggtggctcac tcctgtaatc ccagtacttt 7740
gggaggccca ggcgggcaga tcatgaggtc aggagttcaa gaccagcctg gccaacatgg 7800
tgaaacccct tctctactga aaatacaaaa aactacccgg gcgtggtggt ggatgcctgt 7860
aatcccagct actcaggagg ctgaggcagg agaatcgctt gaaccctgga ggtgaaggtt 7920
gcaatgagcc gagattgcac cactgcactc cgacctgggc gacagagaaa gactccgtct 7980
gaaaaacaaa caaacaaaac cccgagattc tcttttcccc cgtccggagc tctatggcca 8040
tctggagctc gttctgtcca caaggacaca tttcctggca gcagctgtgg accagggctc 8100
actcactcac atctgtaccc aatctagagc aggacaaaca cctatcacct gcactggcaa 8160
tggacagagg acagtggcag ccccatcacc agaggcattc aagccaaggc attttctatc 8220
gtctatttat ctatctatct atctatctat ctatctatct atctatctat ctagatgaga 8280
tcttgctatg ttgcccaggt tgaactcctg gcctcaagcg atcctcctgc ttcagcctcc 8340
caaagtgttg ggatttcagg tgtgaaccac tgtacctggc tggagtgcag tggcactgtc 8400
atagctcact gcagcctcca cctcctgggc tcaaggaatc ctctttcctc agcctcctga 8460
gtagctggga ccacaggcat gcaccatcac acccagctaa accttgattt tttccatagt 8520
atatggctca gggcagagca aagaagcaca aaaacatgtt ggcttgcggt tgagggctga 8580
tgggaggggg ttctggccaa agcccaaaat taacagccac aacgtcagtg tctgtgggag 8640
gggttgccag gggcgtgcgg gttctggggc tcaaggccct gccggtaaac ccatttgaag 8700

caggatggca agaaggtgac accatcttcc cccgcgcact gaaagcccct ggctgggatt 8760
gcctggggca gacacaggct cggaccagcc ccagcaatcc cagtttatca gcgagccggc 8820
tgagggcccg gagttatctc agtgcccggc ctgagacctt gtgggcagcc tgtggtcatg 8880
ctggcattcc aggggccttt tggcatgtgg ggaatgtcca ggaaaagcct cagccttcgg 8940
tgaggcgcag aaaagggaag tgtccctaga gggggtgggt gagggcgtgg gaggtggtgt 9000
ctgcagggaa tgtccccttt gggggaggag gatggagggt tgggattctg aggatggggg 9060
ggggggctgt agccagcacc atgtccctcc tgtgtgacca gctcagagtc ccatgaaatt 9120
ggggcttggg aggggaaggg acactggcct gggaaccaga gacctgggct ggtctggctc 9180
acagttgctg gacctctgtg atccgtgtca aaaaacgaga acaccaattc ctgtcctgcc 9240
cactcaccac caggtgggac ctgaaccctg gcatcgccag cattgggaat gtcggccact 9300
gactcaacca ctctcccgga gacctatttg ggccacccga ggcgggtgcc tgggccacac 9360
ggaggggtcc tggtggtctt cagggcagcg gctgtggggc tgaagcctca aggaaccaca 9420
tctctgcata ggagggccag gctgcagggc ctcggagaca acctagttgg gcgtgttggg 9480
gtctgtgggt cccaggtcct ggcctcaccg ggtccccacc gcgctgtcag ctcccagcct 9540
ctttccctcg tctgcctctg ggcttctgta aggctatgtg ctccaaggcc acctcctcca 9600
ggcagccctc agacccccac cttcgcccag taccgatctg caccgtggtc tctgaagtct 9660
ccatcgtgac ctcaaacctc gctcgtcctt gctcctagcg aggcttgggg tcggggtgtc 9720
cgaggtgggg gacatccggg ggggttaggt ggctggcgcg gggagccggg gttgtgaggg 9780
gtgatgtcct caggcggcgg cgctgcgggg tgcggcgagg acaccggtgg ggtgagagca 9840
ccggcggggc agcagcgggg gccgcagcgc cgggtccctc ggcccggggc ccctcccgcg 9900
cggagccagg ggcgggacag gggggcgtgg cctggtggcg ctgacgtcac ctcgcctata 9960
aaatgtccgg ggcgccgcta gctgggcttt gtggagcgct gcggagggtg cgtgcgggcc 10020
gcggcagccg aacaaaggag caggggcgcc gccgcaggga cccgccaccc acctcccggg 10080
gccgcgcagc ggcctctcgt ctactgccac catgaccgcc aacggcacag ccgaggcggt 10140
gcagatccag ttcggcctca tcaactgcgg caacaagtac ctgacggccg aggcgttcgg 10200
gttcaaggtg aacgcgtccg ccagcagcct gaagaagaag cagatctgga cgctggagca 10260
gccccctgac gaggcgggca gcgcggccgt gtgcctgcgc agccacctgg gccgctacct 10320
ggcggcggac aaggacggca acgtgacctg cgagcgcgag gtgcccggtc ccgactgccg 10380
tttcctcatc gtggcgcacg acgacggtcg ctggtcgctg cagtccgagg cgcaccggcg 10440
ctacttcggc ggcaccgagg accgcctgtc ctgcttcgcg cagacggtgt cccccgccga 10500
gaagtggagc gtgcacatcg ccatgcaccc tcaggtcaac atctacagcg tcacccgtaa 10560
gcgctacgcg cacctgagcg cgcggccggc cgacgagatc gccgtggacc gcgacgtgcc 10620
ctggggcgtc gactcgctca tcaccctcgc cttccaggac cagcgctaca gcgtgcagac 10680
cgccgaccac cgcttcctgc gccacgacgg gcgcctggtg gcgcgccccg agccggccac 10740
tggctacacg ctggagttcc gctccggcaa ggtggccttc cgcgactgcg agggccgtta 10800
cctggcgccg tcggggccca gcggcacgct caaggcgggc aaggccacca aggtgggcaa 10860
ggacgagctc tttgctctgg agcagagctg cgcccaggtc gtgctgcagg cggccaacga 10920
gaggaacgtg tccacgcgcc agggtgagtg gggacgctgc ccccgcctct cctggtccgt 10980
gcacaaagcg caccccaccc gcgcccctcc agcctcccgc cctttctcgc tcgcggcgcc 11040
gctgcggtcc ggagcactgc ccattgcgcc cccgctaggc acgcgggcta ccccgcctgg 11100
agggggcgag gagtggggct ttgcccatcc tcgggtgccg ctgcccacct cccaccccgg 11160
gctgggatca tgggctcccc taggccccgc ggagtcgcaa ccgtacgtgc accctcctaa 11220
cccccccccc cgcccaatct tggctctccc cacgcgcgct gatccctcgg atcagctgtc 11280
ccagctcttg cggagtggga gcccctcacc catttcctcg tgcccctccc cccgccggct 11340
gtcctggagc tcgggggtcc gaggcaaggg tcgccacccg caagggcgcg cctccacccc 11400
caccggcagc ctttcgcggg cgagatgggg gaggttagcc aggcctttga tcccggcggg 11460
gcgcgcctcc acctccccgt ctgcccggcc ttcctccacc tcctcccgct gccgggcggg 11520
gtcggcctcc gctggtgggg gggggcgcgg ggtgtcagcc cttcccccca gcccctcctc 11580
ccgcgtctgc cccgcgctcg aggccgcggc ctttgtgagc agggggggcgg gtcgcctcga 11640
ctgggtcctc cctcgccccc tttgtcctgc tccatctctg cagatgggaa aaccagatgc 11700
ggcggggcgg gggaggggat cggcttttgc ggttcacccc tgcagaggag ccccccgcgc 11760
cgcccccggg cccagggctc gggtcccgag gttccccagg aggcggtctc cctcctcgcg 11820
ccgcggcccg ggaacggcgt ggcgcggatg gcggccctcc aggcaccccg cccttcgccc 11880
gccgcgcgcg tctccaggcc ggtgcgctga gctccgctcc gcgggcgacc gagggcggct 11940
tcagcgcgag ccgggagacc ccaggccagc tccctcggga agcccctacc ctctggtgaa 12000
cccatcccct agggcgctcg ccgggaacaa ttgactgcaa ctcgatccgt cccctccggg 12060
gcctcctgca ggcacggcta tttgcgaccg gcctgtgcgc cactctttcc cttctttctc 12120
agattctccc cgagggggctt tctcttcctt ttggcgtcct cttccactcc tggcggagaa 12180
gctgttcctt gcttgcagac agaggccccg ctggagggag gggcgtgggg ggatcttttt 12240
ccctggatag gaagtcggac cccaggcctt ggagatggct ggacgggagc ccagtttgtg 12300
tcctcatccc tcctcttgga aggagccctc ctgacggccc ccctcttggc tgtgggctct 12360
gcaggagggg gaaagacccc ccatggcagt gggggtgagg gtggaggcct gggtggggta 12420
atggccattt tgtgcctgaa gtttgctacc ttaagtccca gagaggtgaa gctgcttgtc 12480
atgggtcaca cagcaagtga ccacaaggaa gcagcatgca gaagtgggtg catttggctc 12540
cagaaacccc gtttcctgtt tcccacagcg catggcgggc agagctcctc acctgatcag 12600
caggcattga gccctactaa ggggacctgc tgagccatga gtgaaccagt ggggttcacg 12660
ttctcttggg gacattacga agtgtcaagg aaaaggcagc gggtacaggt tattagatgg 12720
ctgagtcctt tctgagcagg acatttgagc tggcctgaag gatgaataga aggtggcagg 12780

```
tatggggggg gtgctgtgtg gaccagcgtt gtaggctgtg ggtgtctcct gtgcaaaggt 12840
cttatggcag gaaggtacag agaaagccca gtgtggctgg gaggaagctt gtgaggtctg 12900
ggccacggga ccttgggtga gagcttagtg cagggctatg gtccttgcct tggggttgca 12960
gccacagcct cccgtgggga agaagtcgca gaagtaggtg gccttggccc tgggggccag 13020
gaagttgcag agagcctgag ggtgtttttg gcgagcctgg gcagataact cccctctcct 13080
gagaagcttg ctgggggcgt ctggtgtgtg attcaggctg atagggtggg aggaaccaga 13140
aattgcagcc aggagaggtg gtggtgatgg ctcagcttgg agttgaaagg gatgtgggac 13200
ggcgcggggg tgggggtact tgggggccga ggttggggct cctctggcct cagatcttgg 13260
ggtccttatc tcgtttctat gtcagccaac tcccgggggg cttttgggaa ctggagtctg 13320
caggggtggg cgtgggggggc gtgtctgcag aggccatttt ggacgggcct gcgtggcaag 13380
ggggaggcgt ctgcttccga cacacatgct ggggaacgcc agccaggaag gggaggatcc 13440
ggacttagct ggcagggggg atgtgaatca tctctgcagg cctgcacggg cgggcccggt 13500
gggggcaggg tgctcccctt tgaaaaacgc tgcgccctgc tcttcagcct cagtttcccc 13560
atctgtaaag tgtgcattcc aggcctcctt gggctggccc agagctgccc tgggcagggg 13620
ctttcagccc ttcacaccag aggctgggtc agctgagtga gtgctccttt gtcctcccgc 13680
catgccccag gctcctccct gctccccagg atccacacct accctggccc cgaccctggg 13740
ccatgcctca ccacacttcc tgtcttctct cacatctctc acatctggga gtcctctccc 13800
gccagcctgt gcttgcatct ggggatccat gccagggata ggacctgtcc tccctgctgg 13860
cttgggacat gccctctccc agccactctg aggagggctg actgaggaag ggctcgtcaa 13920
gctgggcttt gcaggatgtg taagagttct ccccacgagg cgcagggcat tctgagccca 13980
gggaatggct tgtataagga tgcagaggca tttgaaatgg ccaagtagtt gcaggaatcg 14040
actggaaacc ggggtggtaa ggtgaagcca tagaacattc caggccccct cccctaaatg 14100
agatggaagg agtgcctgtt ttgaacaagc caggggcatc tggggaccgt taaggcctgg 14160
gggtggtgat ggggactgga gggtgtgagg caaccagggg gtgcccctct gagccaccac 14220
agacagaatg gttcagaagg ccaggcacac tggctctcgc ctgtaatcca agcactttgg 14280
gaggccaagg agggaagatg gcttcggcca aggagggaag atggcttcag cccaggagtt 14340
caaggccagc ctggaaaaca tggcaaaacc tctgtctaca aaaaatacaa aaaattagcc 14400
aggcatggtg atgcacgcct gtcgtcccag ctactcagga ggcttagatg ggaggatcac 14460
ttgagccggg gagtttgagg ctgcagtgag ccgagatcgt gccactgcac tccagcctgg 14520
gcaacagaac gagaccctgt cttaaaaaaa ttttttttgg cctggtgcgg tggctcacgc 14580
ctgtaatgcc agcactttgg gagtccaagg tgggtggatc acctggaggt cgggagtttg 14640
agaccagtct gaccaacatg gagaaacccc atctctacta aaaacacaaa aattagccgg 14700
gcgtggtggc gcatgcctgt aatcccagct actctggagg ctgaggcagg agaatccctt 14760
gaacccggga ggcggaagtt gcagtgagcc gagatcgcac cattgcactc cagcctgggc 14820
aacaagagtg aaactccatc tcaaaaaaca aaacaaaaca aaaaaaacaa ccaaaacaaa 14880
caaacaaaaa actgtaatta aaataattaa aagaaaaaag ttaaaaaaaa aaaaaagaga 14940
ggatggctca aaggctggaa acagggaagg ccactgtgag gggaggacag gcagggctag 15000
acctctctgg aaggaggagg gagaggtacc cgtgggcccg gcacaggaga ctcttaatct 15060
cctggctccc gggggcttct gtgggcctgt gactcaggat ttctgtgcct ctgttgatga 15120
aaagaaaaat cctgaagaaa acactgttcc catagtaaca cagctctaat tagagactcc 15180
aaggccaagc gagggccttg gagccaggag gggccctgct ctactggggg gacgacaccc 15240
ctttccccta tccctccctg ctggggtgtg gcctgatgtc tacgtggcac caggccccta 15300
aatcctttca acatcctggc ggagcggaga ggctgggccc ttttgacagg tggggaaact 15360
gaggaaggtt gcagggaagg cactcatcca agggtgcctg gccccccgcg gtgggtgcct 15420
cactggctgg cctaaacctt tgcatcaaac cagttccagg attgaacgca acaggctgat 15480
ggggactgag tcgtggtaca gatggcggca gttgtgtggc cctgggccca ggaatggctg 15540
ggaagctccc tttcttctct ggcttggggg atgaggttag tgtaaatttc atggagttgc 15600
caggaggatt aaggccgacg tgcattcaca cgtggccccg tggctgccgc cagttaagcc 15660
ctgcgcagcc attggaagtc tcattgagaa ggagcctccc ggcttcctgc atttgttcca 15720
ggcgggctgg taagcgccct gcttatttgc aaggctgtgt gaggacgagc cctgtaaacc 15780
ctaggacgag gctgtgctaa tgtggtttct gttccactgt ctccccttct ccttttgccc 15840
ctctttctgg ggaacctgga gcccctgatg ccctctgttt tttcctgctg ctggggtgga 15900
cctagaacct ctccctccat cctttctgct gctggggtgg gcctgcagga ggctgtcctc 15960
ttacctggtc cctaacttcc ctccgatcag cggggcttca gcggagcccc agcgcattgg 16020
acaggtggtg ctctgtggcc ggcacaggcc agggcccagg gtgcggcccc tgcctgtagg 16080
tagacatgca ggcccttctg agcagaatga gggggcggtg agggcagctg tgcgggtgtg 16140
gctggcctga cggctccctg cagccctgcg gcttctgtgc agtgggggtg gggcgggcca 16200
gacctttgca gggcctcttg gctggtggag ggctgagtga gcagaggccc cagccctcgt 16260
gttccctggg gtgtggcctt aggatggtcc cggcaccctg gggacccagc ccctgctcac 16320
cttatcctcc tcccgtgctt ggctggggtg tggtggctga gccagcccac ccagtgcggt 16380
ggatgctcgt cggagggcag agggtgggct accggcttaa ggggccccag ggaacctggg 16440
gggtggagcc caaggggttc caagaagggg cggggccagg aacccataga caagagggtg 16500
gggcagggaa agggcgtggc aagagggcca cccacctccg gtccagagag ccagccagac 16560
ccttactttc tcttgctgtg tgaccttagg caaggacatg ccaccaccgg ccttagtctc 16620
cctctttgtg aagtaggatg aagatcccca cctgtggagc agatgtgggg ctgcatggtt 16680
gggtcaggat ggaacaggat ggggaggccg ggcgtggtgg cttacccctg taatcccagc 16740
actttgggag gcaaaggtgg gaggactgct tgagtcctgg agtttgggca acatagcgag 16800
accacccccca tctctacaaa ataatgttaa agttagccag gtgtagtggt gagtgcctgt 16860
```

ggtcccacct actggggaga ctgaggcaag aggatccttt gagcccagga ggtggaggct 16920
gtagagagcc atgcttgggc cacttgcact ccagcctggg caacagagca agaccctatc 16980
tctaaaaaaa aaatggaatt gaggatgtgt cctctgggtg tgggctctac cacccaccag 17040
cctccctctc ctgtgggtgc tgcctgccac ccaccgctga ggtccctgca gcatcaaggt 17100
gcccaaacga agacactctc cagctctgag ccaggcaccc atacagagcc cggcagacgc 17160
ctctgctgct gcagttctta gaatccagag cgcgtgggga atgtgaattt gtgctgctgg 17220
agccaaacat cccacctcca ggttttagct ggaggaatcc atgtggatat gcaaagcaat 17280
gtagttataa tgaatagcaa ttgtgggctg ggcgcagtgg ctcatacctg taatcccagc 17340
actttgggag gctgaggtgg gtggatcacc tgaggtcagg agtttgagac cggcctggcc 17400
aacataatga aactccgtct ctactaaaaa tacaaaaaaa ttagccgggc atagtggcgg 17460
gtgcctgtaa tcccagctac tcaggaggct gaggcaggag aatcacttga acccaggagg 17520
cagaggtttc agtgaggcga gatcgtgcca ttgcactcca gcccgggaga cacagcgaga 17580
ctctctttct caaaaaaaga atagcagctt tgctatagct taggggagca aaacccgaaa 17640
gccaccttaa gtttcccgaa gatagagatc cctggggtcc tattctgaga cagagtcttg 17700
ctctgtcgcc caggctggag tgcagtggcg gatctcagct cactgcagcc tccgcctcct 17760
gggttcaagc aattctcctg cgtcagcctc ccgagcagct gggattacag gcgcccggca 17820
ccaccccccag ctaatttttt tttttttgag acagagtctc gcattgtcgc ccaggctgga 17880
gtgcagtggc gcgatcttgg ctcactgcaa gctccacctc ccgggttcac gccattctcc 17940
tgcctcagcc tcccgagtag cagggactac aggcgcccgc caccgtgccc ggctaatttt 18000
tttttttgt attattaata gagacgggtt tcaccgtgtt aggatgatct cgatctcctg 18060
acctcgtgat ccgcctgcct cggcctccca aagtgctggg attacaggca tgagccacct 18120
tgcctggtcc ttttttttgt atttttagta gagatgaggt ttcaccatgt tggccaggct 18180
ggtctcaaac tcctgacctc atgatccgcc cgcctcggcc tcccaaagtg ctgggattac 18240
aggtgtgggc cactgtgccc ggccagtccc attctacttt ttagggacat ggaatcatgt 18300
aaagatgcca tacagaaaac agtatgcaaa tccagagaca gaggacatct gtgcatgttt 18360
ctttctttct tcctttttttt tttttttttt ttttttgaga tggattcttg ctctgtcgcc 18420
caggctggag tgcagtggtg caatctcagc tcactgcaac ctctgcctcc caggttcaag 18480
tgattctcct gcctcagact tccaagtagc tgggattaca ggcatgcccc accatgccca 18540
gctaattttt gtatttttag tagaggcggg gtttcaccat gttggcaagg ctggtcttga 18600
actcatgacc tcaggtgatc cacccgcctc agcttcccaa agtgctggga ttacaggcgt 18660
gagccgccgt ggcaacccgc atgcttctta tgtataagaa aaaaacagct agaaagttgt 18720
gggttcactg gctgggcaca tggtggcttg tgtctgtaac ctcagcactt tgggaggctg 18780
agttaggagg atcatgtgag gccgggagtt caagaccatc ctaggcaaca tagccagacc 18840
ctgtctgtac caaatagaaa aaaaaattaa cttgatgtgg tggtgtgtgc ctgtagtctc 18900
agctatttgg gaggctgagg tgggaggatc acttgagccc aggaagtcga ggctacagtg 18960
agctatgatt gtgccactgc actccaacct gggcaacaga gcaagaccct gaatcaaaaa 19020
gaaagagatg tatttgccaa cacagggggtg ttctgcagtg gggtgggaag gggacgtgta 19080
ggacttcata gtttcctttt ttttgtttta gagacagggt ttaaaaaaaa attttttttt 19140
ttttttgag gcggaatttc attcttgttg cccaggctgg agtgatctcg gctcactgcc 19200
atctccgcct cccaggttga agtgattctc ctgcctcagc ctcaggcaat tttgtatttt 19260
tagtagagac ggggtgactc catgttggtc aggctggtct caaactcccg acctcgggtg 19320
atctgcctgc cttggcctcc caaactgctg ggattacagg tgtgagccac tgtgcctggc 19380
cccttttttt tttttttaag tagggtcttg ctaagttgtc caggctggtc ttgaactcct 19440
ggtctcaaat gatcctcctg acttggcctc ccaaaaggct gggattgcag gagatatgag 19500
ccaccgcacc cggccctttt ataagttttc taaaggaaag gaacatgtac catcgtgttt 19560
tattttaatt tttacataga agcaggaaca atagcatgaa cccccagaca cggcccccca 19620
agatgcgctg ttccgctgtg ctgcagcccc atttgtcctt ttctatacca tttttggttg 19680
tatttgaagc aaatccctga catcagggca tttcatccca aaatacttcc atctgtgttt 19740
ctaaaaaaac gaggccattt tcttatgtaa ccacaacgtg atcaacagta atcaatgctt 19800
aacatctaat tctgggccac gttaagaccc tcctgattgg ctcaagaatg tcttttgtgc 19860
agttggtttc ttgattcttt ttgttttttt tgagacaggg tctcactgtg tcacccaggc 19920
tggagtgcag tggtgcaatc acggctcact gcagcctcag cctcctgcct cagcgatcct 19980
cctgcctcag cctcccaagt agctgggacc acaggtttgc accaccactc ccagctaatt 20040
aaaaaaagta atttgtagag acgggggtgg ggtgggggtc ttgctatgtt gcccaggctg 20100
gtctcaaact cctagactca aacaatcctc ctgccttggc ctcccaaagt gttgcgatca 20160
caggcatgag cccctgtccc tggcccctat tatttctcta actggggaaa gtcatgtggg 20220
aacagatgta gcttgccttg gcctctgacc ggccctgcct gcgttcctgg gtgctctctg 20280
ctgcttctca tgtgtgccac tgtggggact cggccgccca ccccaccccg tggtgttacc 20340
ttgcgtgtgt agttctgtga gctcagggct atggtctgcc agaactaggg ggcgtggggc 20400
cccagtacca gcccaaggcc tcctctctgc aggtatggac ctgtctgcca atcaggacga 20460
ggagaccgac caggagacct tccagctgga gatcgaccgc gacaccaaaa agtgtgcctt 20520
ccgtacccac acgggcaagt actggacgct gacggccacc gggggcgtgc agtccaccgc 20580
ctccagcaag tgagtgcctc gctcccacct gtcaccgccc ccaccacctt gcctgggcta 20640
ccccgcctga ccctgtcccg ccatccccca ggaatgccag ctgctacttt gacatcgagt 20700
ggcgtgaccg gcgcatcaca ctgagggcgt ccaatggcaa gtttgtgacc tccaagaaga 20760
atgggcagct ggccgcctcg gtggagacag caggtaacac taaagcccca gttccctgga 20820
gccgtcctgg agtcctggag ggtctggcca tgccgtggtc acttggtagc cccagccaag 20880
gcctgctctg tgctgggcat cccccccggac tggccccgca ctgtcctacc ctggggctga 20940

ctgctgtgtg accccagctc ctggccctcc ctctctggtc accccagcct ccacccccact 21000
ccctgccagg aggctcactg actcccctct ttctgggaca ggggactcag agctcttcct 21060
catgaagctc atcaaccgcc ccatcatcgt gttccgcggg gagcatggct tcatcggctg 21120
ccgcaaggtc acgggcaccc tggacgccaa ccgctccagc tatgacgtct tccagctgga 21180
gttcaacgat ggcgcctaca acatcaaagg caggttctcc tgtgggcagc tgctgggcag 21240
ggaacccctc ggtcggggct ggggtcagtg ctgcggggag cgccctctgc atccacactg 21300
gaccctggct tggctcaggg ccattccagg ccctaaaggg acaggtgtct gatggccacc 21360
aggggctctg ggatgcaagc agcccctttc cctcttgtct gtgtggttgg ggggacttac 21420
cttgcccacc tgacagagag gtgtgtggag gggagagcag ggaggggaag gagagcaggg 21480
aaggggaggg aggagagcag gggaggggag agccgggaag gagaggagag caggggtggg 21540
gaggttctgg aaagggtgtg caggggagga cgcgcctcgg ttatggact ggagcccctt 21600
cccaggagga cccccaacaa tccagaggtg cctgttagga ttcagaacat ggttttttg 21660
tttgtttttt tgagactcac tccctcaccc tggctggact gcagtggcgt tatctcggct 21720
cactgcaacc tctgcctcct gggttcaagt gattctcctg cctcagcctc ccaagtagct 21780
gggattacag gtgtgcacca ccacgcctgg ctaattttta tatttttaaa atttattatt 21840
tatttatttt gagacccagt ctggagtgca gtggcgttat ctcggctctc tgcaacctct 21900
gcctcctggg ttcaagcgat tctcctgcct cagcctcccg agtagctggg actatgtgtg 21960
ggagccacca tgcctggcta attttttgt atttttcata gagacgggtt tcaccatgtt 22020
gtccaggctg gtcttgaatt cgtggcctca agtgatccgc ccacctcagc ctcccacagt 22080
gctgggttta taggtgtgag ccaccacacc cggctaattg ttttgtattt ttagtagaga 22140
cggagcttca ctatgttggc aaggctggct cgaactcctg acctcaagtg atccgcccac 22200
ctcagcctcc caaagtgctg ggattacagg cgtgagccac tgcggccgag cagaacacgt 22260
tctaggaccc ttgttcatgt gtccatcatg gacaggagga cgtgcgggcc atagggaccc 22320
tggctcattc cggagccggg actggagggt ggggcgtcac ccttgggaac acccgtgccc 22380
accctccgct gcccagggta ggggtgggga gccaggcttt gggccccact tgataaagtc 22440
ccctccccag actccacagg caaatactgg acggtgggca gtgactccgc ggtcaccagc 22500
agcggcgaca ctcctgtgga cttcttcttc gagttctgcg actataacaa ggtggccatc 22560
aaggtgggcg ggcgctacct gaagggcgac cacgcaggcg tcctgaaggc ctcggcggaa 22620
accgtggacc ccgcctcgct ctgggagtac tagggccggc ccgtccttcc ccgcccctgc 22680
ccacatggcg gctcctgcca accctccctg ctaacccctt ctccgccagg tgggctccag 22740
ggcgggaggc aagccccctt gcctttcaaa ctggaaaccc cagagaaaac ggtgccccca 22800
cctgtcgccc ctatggactc cccactctcc cctccgcccg ggttccctac tcccctcggg 22860
tcagcggctg cggcctggcc ctgggaggga tttcagatgc ccctgccctc ttgtctgcca 22920
cggggcgagt ctggcacctc tttcttctga cctcagacgg ctctgagcct tatttctctg 22980
gaagcggcta agggacggtt gggggctggg agccctgggc gtgtagtgta actggaatct 23040
tttgcctctc ccagccacct cctcccagcc ccccaggaga gctgggcaca tgtcccaagc 23100
ctgtcagtgg ccctccctgg tgcactgtcc ccgaaacccc tgcttgggaa gggaagctgt 23160
cgggtgggct aggactgacc cttgtggtgt tttttgggt ggtggctgga aacagcccct 23220
ctcccacgtg gcagaggctc agcctggctc ccttccctgg agcggcaggg cgtgacggcc 23280
acagggtctg cccgctgcac gttctgccaa ggtggtggtg gcgggcgggt aggggtgtgg 23340
gggccgtctt cctcctgtct ctttcctttc accctagcct gactggaagc agaaaatgac 23400
caaatcagta tttttttaa tgaaatatta ttgctggagg cgtcccaggc aagcctggct 23460
gtagtagcga gtgatctggc ggggggcgtc tcagcacccct ccccaggggg tgcatctcag 23520
ccccctcttt ccgtccttcc cgtccagccc cagccctggg cctgggctgc cgacacctgg 23580
gccagagccc ctgctgtgat tggtgctccc tgggcctccc gggtggatga agccaggcgt 23640
cgccccctcc gggagccctg gggtgagccg ccggggcccc cctgctgcca gcctcccccg 23700
tccccaacat gcatctcact ctgggtgtct tggtctttta tttttgtaa gtgtcatttg 23760
tataactcta aacgcccatg atagtagctt caaactggaa atagcgaaat aaaataactc 23820
agtctgcagc cccaggccgg cctgtgtgtg tcttggggct gaggtgggtg ggggggctga 23880
ggtgggtggg agggctggcg ggacaggtag gcgccctggc tccccagctc agtgctggga 23940
gtgtgcagtg ggagggaggc cgtggctcca gtgggtgctc cggagctcgt gggcccagca 24000
cacctcctta agcgggggat ggagcgctgg gaggggtgg actgtggccc atgcgacccc 24060
cagagccatt aggaggagtt ctgtggtgag aagtggctgt ggctcctcgt aggctacgtc 24120
caccatgcgg gggacctcgg gggtgtctgg cggtggcacg ctggatgttg agaaggcgca 24180
gcccagggaa cactcaaacc aggagacccc acattatcct ctagttgaca tgtgcccttc 24240
gactagggga cttggtggta ggggcagctc cgcccccata ctgcgaggat ccgggccttc 24300
cacttcccca gacacagcag gttggggtgc ccctggggct gggagatgct gccctgggcc 24360
cctcaggggg cggccgtggt aggatggacc gggccgagcg tgcagacccct gggcaggtcc 24420
ctacatcgga accagcagcc ctggggatat ctggcatatc agaggctgag gactgtttcc 24480
agactttacc aaggccacag tcagggcgac ctgggttctg agcctccctt tgcatcctag 24540
gctgcaggga caggggctgg gcagaggctc gcggacctcg tgcagcttca ttcactccca 24600
ggtgccccta aggatagcag ctgcgagaag actgagggga ggagcagtaa ggcgcagcct 24660
ggagcggagg gacctggcgt ccagtcattg tgcgctggga aggcggtgat ggcgacggaa 24720
tttgggacac gcggggcgtc ccgggggcag ccagggcact gcaggccgga gccccctgtt 24780
cccccgcatc ctccccgccg tcggcagcag agcaagcgct gattggctgc tgatgacgca 24840
actgggaggg ctgcgctgtg ataggtggtc cctcgggggc gtggggcgca agtcttgaga 24900
ttggcagggg caggtggctg ctgcagaggg aagtcggggt cacctcagcg aggttccggc 24960
ggccacccct ctcccctccc ccatgaaagc caggctggaa gccagaaaaa tcccagtgac 25020

```
tggaagggac agattaatcc tccggaacca aactctttga gacctgggga ggagggtggg 25080
aggcactggg aagggggatt gggggagcc tggctcattt ccacccatgg agctgaccta 25140
gaatgaccct aaggtccccg gagccaccag ctgattccga attccattca ttctgcaaaa 25200
tttggcgcct accacgtggc cgagattccc gaggctgcag ccaatgagca gcatagtccg 25260
atgggggagg ctggaagggc cgttcacgag atctctcggg aggaatcggt aggagctgcc 25320
caactgaaag ggacggggga ataacccggg gcgagggacc tgcccaggcc ctgccctctg 25380
gggggaagcc aggccagggg ccgggggcat ctctggggtc ccaggtgaga tgcgggtcga 25440
gggagccagg ccacggttct ccagactcgc gtggaggcca cgagcgtctt ctggaggagc 25500
gggcactgcg cggaccggca gactctgggg cgctcgtgct ccccatctcc tgaccttttc 25560
ctaccttcag ttccctcctg tcaggacagg ttctggggct cgccggggca gggagccagg 25620
gcagagggtg cagtttccgc tcctgcctga gtcaccttcg ctttctcagc gattcttcat 25680
tcacaagggc gtggcccatc acacgcactc acacacttgt gaacttgcac gcacacacac 25740
atatggacct gtgcaaagag ccccatgcac aggcaggtac acgtgcatgt aggcgtgcac 25800
acagacacgc acagatgcag gcagaccaac tcccaggaat ctgtctccag cctacaggga 25860
ttcagcactc agggtaccag cctctgtccc gtgcaccccc attcccggca gttctcccag 25920
gtgggtcggg ggccactagg gaccccatac cccgagggtg gctcctccat taagcctgcc 25980
atggggttgg gagtgagagt cccacctccc acttgagagt ccccagtgag tcaaggcaag 26040
tgcagccagg gctccaagat ccttgttggg attcactgtg atccccaaag atcacgactt 26100
aagggaaaac attcaattaa acttgttaga gcaaagaaag gagagactgg gctgggagca 26160
gtggctaacg cctatatagt tcagctactc aggaggccca ggtgtgagga ttgcttgagt 26220
ccaggaggct gaggctgcag tgagctatga tggcaccacc cactgcactc cagcctgggt 26280
gacaaagcaa gaccctgtct caaaaaaaaa aaaaaaaaaa aaaaaaaagg aaaaagaggc 26340
cgggcacagt ggctcacacc tgtaatctac taaaaataag aaaattagcc agacatggtg 26400
gtgggtgcct gtaatctcag ctacttggga ggctggggca ggagaatcgc ttgaacccag 26460
gaggcggagg ttgtgtgagc cgagattaca ccactgcact ccagcctggg cgacagagtg 26520
agactctgtc tccaaataat aataataata ataataaaat aaaaagaaaa aagaaagaaa 26580
gaaaggagag actgagctat attgcccagt ccaggcatgt gggcagtctg ggggacagtg 26640
gggcatactc ggtgtctcag ggtcccagcc ctgggtgccc ccactgctgt ctgctgtgac 26700
ctcctcagga ggcctcattg ggagggaccc tgagtcctgc ggggggctgt ggagctgtca 26760
ccagccccag gaggactcag gcaagtccct ctggtggtcg aggcctctga gcttcccagt 26820
gtgtctccac gggacacccc ccctccagac cttctgcccc agctcagcct cttccaattc 26880
attatcaggg ggtgggggag gggacaggaa gccccagacc cagctgggcc ccatttcctc 26940
cagggccaca gtggcctctc ccagagtcac gaggatgggg gctgatctca ctcttttttt 27000
tttttgagac ggactctcac tctgtcgctt tttttttttt tttttttttt gagacggagt 27060
ctcactctgt cgcccaggct ggagcgcagt ggcgtgatct cggctcactg caatctccac 27120
ctcccacgtt caagcgattc tcctgcctca gcctcctgag tagctgggac tacaggtacg 27180
tgccaccaca cctaactaat ttttgtattt tttagtagag atggggcttc accatattag 27240
cctcccaaag tgctgggatt acaggcatga gccaccatgc ccggccttga cttcactctt 27300
ttaaaaaagt caaatacttg gtgcttgaaa tcccagcact ttgggaggct gaggcaggag 27360
aactgcttga gctcaggagt ttgagaccag cctgggcaac atggtgagac tcctgtctct 27420
gtaaaaacta cgaaaattag tcaggcatgg tggtgcgtgt ctgtcgtccc agctgctcag 27480
gaggctgagg tgggaggatg gtttgatcct ggaaggttga gactgcagtg agctgtgatt 27540
ccaccactgc agtccagcct gggccgcaga gcaagactct ctctcaaaaa taaataaaat 27600
aaaataaaaa gtctaaaact tttctttcaa acgacggctt ttggggctca taaggtgcgt 27660
gaagatggaa agggggcccg gcagtgccca tttcggctcc cttgggaccc cggcttccct 27720
gaagacccct gagcaatggt tggctgggtg cctggtctcc ctgaacgggc tgtgggcaac 27780
aaggttgtct ggatgggggga ggggctctga gacccttggg ggcagcagag ggagaaggga 27840
acagatgggg tctcccaggc agagaggcct ggaggggtgg ggcggggcag ggcatttggc 27900
gtgaaaccca agccccttaa cggggcccta gcttaaaccg ggaggagggg cctctgtgat 27960
gtgcaaataa ttaacacaga agccttcact gtgctcccct cctcatccca ggacccaggt 28020
ctgaccctca ggagggggagg aggtgagcaa gggggaacc caccttccag ggaaccccca 28080
gatagtgcca ggcaccaggc aagtgctcta aaaggcttta ctaatattat ttgaagaaac 28140
taagctaata aataaatacg gtataaaatc aaagggtacc cacagatata aacaaaaatc 28200
cacagctaac ccggccccag ccctctttcc tttggaggta actgtgttaa tggtttttta 28260
caccccctctg gaattttttt tttttttttt tttttttttt tttttttggg aaggagtctg 28320
gctctgtcgc ccatgcagcg cagtagcgtg atctcggctc actgcaactt ccacatcccg 28380
ggttaaaacg attctcctgc ctcaaccgcc tgagtagctg ggactacagg ggcgcaccac 28440
catgcccggc taattttttt tttttttttt tttttttttg agctggagtc ttgctctgtc 28500
gcccaggcgg gagtgcagtg gcgcaatctc ggctcacttg caagctctgc ctcccgggtt 28560
cacgccattc tcctgcctca gcctcctgag tgggtgggac tacaggctcc agccaccacg 28620
cccggctaat tttttgtatt tatttttttaa tagagatggg gtttcactgt gttcgccagg 28680
atggtctcga tctccctttt tttttttttt tttttagacg gagtctcgct ctgtcgccca 28740
ggctggagtg cagtggcatg atcgtgactc actgcaaact ccacctcccg ggttcacgcc 28800
attctcccgc ctcagcctcc cgagtagctg ggactacagg cgcccaccac cacacccggc 28860
tattttttttg tatttttagt agagacaggg gtttcaccat gttagccagg atggtctcga 28920
tctcctgacc ttgtgatcct cctgcctcgg cctcccgaag tgctgggatt acaggcgtga 28980
gccaccgcgc ccggcctctt gttttttttt tttttttttt ttatagactt ctaaggaaag 29040
caacactaag aaaatatatt tttaagaaat tattcagcca ggcgtggtgg ctcacgcctg 29100
```

taatcccaac actttgggag gctgaggcgg gtggatcact tgaggccagg agtttgagac 29160
cagcctggct cacatggtga aaccctgact ctactaaaaa tacaaaaaat tagccgggca 29220
cagtggcatg ctcctgcaat gccagctact tgggaggctg aggcaggaga atcccttgaa 29280
ctggggaggc agaggtttca gtgaaccaag atcgagccac tgcactccag cgtgggtgac 29340
agactgaaac tgtgtctcaa aaaaaaattt ttgtttttgg agatggggtc ttgctctgtc 29400
acccaggctg gagtacaatg gggtgatcta ggctcactgc aacctcaaac tcctgggctc 29460
aggtgatctt cctgcctcag cccctggagt aactggcact acaggtgcat gtcagaacac 29520
ctggctagtt tttaactttt ttgtgtgtag acagggtctt gatatgttgc ccaggctggt 29580
ctccatcacc ttctgggctc agtgatcctc ctgcttcagc ctcccaaaat gctgggatta 29640
caggtgtgag ccaccatgcc cgaccctgag ggatacctta atgtttattt ctcacatttc 29700
ttctagaaca aacattgcca gcgcatccca gactctgaac tgtggctggg gactgcagca 29760
tggaggtctt tgcaggcact ctgggaaaca tgccacaagc agggtattaa cccaagacag 29820
tcagtccctc caatgagaaa aaggagccga gggactgtgc atgcctcaga gccagatgca 29880
gcagaactgg tgggttgagg gggcactgag cgcctggtgc ctcctgctgc cccccaccag 29940
ctccgacagt cctcgtcctt gtcctcccca tcagaaggga agagtgaatt gtctgtggga 30000
tgtgccagtg tccccggttc tgaggttggg caggctgggg agccccagac atggcttgct 30060
gcccactgtg gagcctcctg gacttagttc caggctctgc ccgatcctct gagggtgtgg 30120
cttcaaagcc ctgcttgggc ctaaggccat cccagggcag tgactgaggg tagactgaga 30180
tgtcccttgg gcaccagtcc tagggtacct gggctggggg tcttcagctc aggcccccta 30240
gtgcccgtcc tttgggctgt gggcaggttt ccacagcact ggccctttct gaccttacac 30300
ccctgtgcat cctcactggg gctctgtgaa ggtgaaagtc acactaagga ctgtgcctat 30360
ggtggccagg gcaccccacc ggcagcacac ctcagaggcc tcatcctagg cgctgctcac 30420
tttttttct tttttttcct ttttttttt ttttttttt ccgagacaga gtcttgctct 30480
gtcacctagg ctggagtgca gtggcatgat ctcggctcac tgcgacctcc tcctcctcct 30540
gggtttaagc aattctcttg cctcagcctc ctgagtagct ggcattacag gtgcccgcca 30600
ccacgcccgg ctatttttgt agttttagta gagacggggt ttcaccatgt tggccaggct 30660
ggtctcaaac tcctgacctt gtgctctgcc cgccttggcc tcccaaactg ctgggattac 30720
aggcgtgagc cactgtgcgt ggccctgctc attttcttac cacagctttc cagaaataaa 30780
gttaggatgg aaagagagag agagagattg agagagagag agagcaccac attcagatag 30840
agacaggaga gttctcagca agaagagaac tcagctcaga gttgagaatg tctcaactca 30900
gctgtctccc tgctgagacg tcctgggctg aagtgtcccc tcacccctgc ggggctgtga 30960
ctgcacactg cacacatggg tcacagcagg gcttggcgtt tggagcaggc cgggttggtg 31020
gcttgtcctc atctcttgcc tggagctgtg ctggcctgtg gtttgctttg gctgctacca 31080
tgtggaggga gaggtgcttg tgtccatgcc tggcctcttg gaaggccacc actgtgatgg 31140
agagaggaag gccaccactg tgatggagag aagccggggc cagaccgatg agggctgaga 31200
gcccatgggc aggggttgtg gtctcggcca aaccccagct ggcagctgat ggcagccacc 31260
ccggggtcct cagatatggc acagctgcct ggctgagcct cggcaaaggg aagccttgtg 31320
gaaaatagca actcagggat gttgtgagta gtgggctgag ctgtggttta ttttttagag 31380
acagggtctt gctctgtcac ccaggctaga gtccagtggt gtaatcatag ctcactgcag 31440
ccttgacccc ccaggctcaa gtgatccttg tgtcttggcc tcctgagtag ctaggactat 31500
aggtgcatac caccatgcct cgcttgtttt ttgttttgat agagatgggg gtcttgctag 31560
gttgcccagg ctggtctgta actcctgggc tcaagtgatc ctcctgcttt ggcctcccaa 31620
agtgctggga ttacaggcgt gagccactgt gctggccttg ggtgtggttt ttatgtggcc 31680
atagatgact gagatgggtg agaagcggga cctaggggaa tatacagggc agggggatcc 31740
gacaggggga caggagggca gacagctcct ggaaggcctg ggaaagagat atcagggctg 31800
gaccctggag aagtgaggct ttgagggagg ggaggagatg gggcctggcg gggtacgggc 31860
gctgctccta ggtctgaggg catcggcggt ctgagagctt gcgggaggga gggctgcctc 31920
ccggtgctcg gtgccctggc tctcctcctt gctgctggct ggcctcctcc tcctcatgca 31980
tctgtgtgtc ctggaacaca gggcactgtc cctctgcctg tccagaaaag cacgtgctcc 32040
ttggggcctg gcctgctcag ccctatgtcc cacccgctcc ctgctgctat tcccaggcct 32100
tcgggcctca ctgcctgggg tggccgtgct cagccccacc ctgtggtgcc atggcctccc 32160
cacccctgcc ccgaactgcc cctgggacca gttccttcag ctcccaggac agggcctggc 32220
catccctgtg gattctccct gttcgtggac ccccattggt gcccagtctc cttggactgt 32280
cttctgagcc tctctcagat gtacccccaa ctgtaccctt tcacccagag gccatttggc 32340
agcgtcctcc aggactcctg ccgtggggga agtctccacc aaggccaacg cgtgactgca 32400
tcccgaggag ctgatttaag atctggttct caggaacacc tggaactggc cagtcagcat 32460
ccccggccct gcggtctgca cttggacaag ggtcaggcaa tagatgagg cagcaggcgg 32520
ggcttctcct ggaagccttc ccagcctcct gggccactcc agtgggtccc ctccacagac 32580
tcagcctgtt ccctgccacc tggtgccggt ttctctgtgg cacctactag gggccgagtg 32640
ccccgggact gcccagctga gcagcgccgt ccaggtctgc acccagctac actcagggca 32700
cgtgcacagg tgagcagccg ggcacgcagg cacctgccag gtgtgggtgt ggactaggtg 32760
ctcccgg 32767

<210> 5
<211> 2767
<212> DNA

<213> Homo sapiens

<400> 5
gcggagggtg cgtgcgggcc gcggcagccg aacaaaggag caggggcgcc gccgcaggga 60
cccgccaccc acctcccggg gccgcgcagc ggcctctcgt ctactgccac catgaccgcc 120
aacggcacag ccgaggcggt gcagatccag ttcggcctca tcaactgcgg caacaagtac 180
ctgacggccg aggcgttcgg gttcaaggtg aacgcgtccg ccagcagcct gaagaagaag 240
cagatctgga cgctggagca gccccctgac gaggcgggca gcgcggccgt gtgcctgcgc 300
agccacctgg gccgctacct ggcggcggac aaggacggca acgtgacctg cgagcgcgag 360
gtgcccggtc ccgactgccg tttcctcatc gtggcgcacg acgacggtcg ctggtcgctg 420
cagtccgagg cgcaccggcg ctacttcggc ggcaccgagg accgcctgtc ctgcttcgcg 480
cagacggtgt cccccgccga gaagtggagc gtgcacatcg ccatgcaccc tcaggtcaac 540
atctacagtg tcacccgtaa gcgctacgcg cacctgagcg cgcggccggc cgacgagatc 600
gccgtggacc gcgacgtgcc ctggggcgtc gactcgctca tcaccctcgc cttccaggac 660
cagcgctaca gcgtgcagac cgccgaccac cgcttcctgc gccacgacgg gcgcctggtg 720
gcgcgccccg agccggccac tggctacacg ctggagttcc gctccggcaa ggtggccttc 780
cgcgactgcg agggccgtta cctggcgccg tcggggccca gcggcacgct caaggcgggc 840
aaggccacca aggtgggcaa ggacgagctc tttgctctgg agcagagctg cgcccaggtc 900
gtgctgcagg cggccaacga gaggaacgtg tccacgcgcc agggtatgga cctgtctgcc 960
aatcaggacg aggagaccga ccaggagacc ttccagctgg agatcgaccg cgacaccaaa 1020
aagtgtgcct tccgtaccca cacgggcaag tactggacgc tgacggccac cgggggcgtg 1080
cagtccaccg cctccagcaa gaatgccagc tgctactttg acatcgagtg gcgtgaccgg 1140
cgcatcacac tgagggcgtc caatggcaag tttgtgacct ccaagaagaa tgggcagctg 1200
gccgcctcgg tggagacagc aggggactca gagctcttcc tcatgaagct catcaaccgc 1260
cccatcatcg tgttccgcgg ggagcatggc ttcatcggct gccgcaaggt cacgggcacc 1320
ctggacgcca accgctccag ctatgacgtc ttccagctgg agttcaacga tggcgcctac 1380
aacatcaaag actccacagg caaatactgg acggtgggca gtgactccgc ggtcaccagc 1440
agcggcgaca ctcctgtgga cttcttcttc gagttctgcg actataacaa ggtggccatc 1500
aaggtgggcg ggcgctacct gaagggcgac cacgcaggcg tcctgaaggc ctcggcggaa 1560
accgtggacc ccgcctcgct ctgggagtac tagggccggc ccgtccttcc ccgcccctgc 1620
ccacatggcg gctcctgcca accctccctg ctaacccctt ctccgccagg tgggctccag 1680
ggcgggaggc aagcccccctt gcctttcaaa ctggaaaccc cagagaaaac ggtgccccca 1740
cctgtcgccc ctatggactc cccactctcc cctccgcccg ggttccctac tcccctcggg 1800
tcagcggctg cggcctggcc ctgggaggga tttcagatgc ccctgccctc ttgtctgcca 1860
cggggcgagt ctggcacctc tttcttctga cctcagacgg ctctgagcct tatttctctg 1920
gaagcggcta agggacggtt gggggctggg agccctgggc gtgtagtgta actggaatct 1980
tttgcctctc ccagccacct cctcccagcc ccccaggaga gctgggcaca tgtcccaagc 2040
ctgtcagtgg ccctccctgg tgcactgtcc ccgaaacccc tgcttgggaa gggaagctgt 2100
cgggagggct aggactgacc cttgtggtgt ttttttgggt ggtggctgga aacagcccct 2160
ctcccacgtg ggagaggctc agcctggctc ccttccctgg agcggcaggg cgtgacggcc 2220
acagggtctg cccgctgcac gttctgccaa ggtggtggtg gcgggcgggt aggggtgtgg 2280
gggccgtctt cctcctgtct ctttcctttc accctagcct gactggaagc agaaaatgac 2340
caaatcagta tttttttttaa tgaaatatta ttgctggagg cgtcccaggc aagcctggct 2400
gtagtagcga gtgatctggc ggggggcgtc tcagcaccct ccccaggggg tgcatctcag 2460
ccccctcttt ccgtccttcc cgtccagccc cagccctggg cctgggctgc cgacacctgg 2520
gccagagccc ctgctgtgat tggtgctccc tgggcctccc gggtggatga agccaggcgt 2580
cgccccctcc gggagccctg gggtgagccg ccggggcccc cctgctgcca gcctcccccg 2640
tccccaacat gcatctcact ctgggtgtct tggtctttta ttttttgtaa gtgtcatttg 2700
tataactcta aacgcccatg atagtagctt caaactggaa atagcgaaat aaaataactc 2760
agtctgc 2767

<210> 6
<211> 1482
<212> DNA
<213> Homo sapiens

<400> 6
atgaccgcca acggcacagc cgaggcggtg cagatccagt tcggcctcat caactgcggc 60
aacaagtacc tgacggccga ggcgttcggg ttcaaggtga acgcgtccgc cagcagcctg 120
aagaagaagc agatctggac gctggagcag ccccctgacg aggcgggcag cgcggccgtg 180
tgcctgcgca gccacctggg ccgctacctg gcggcggaca aggacggcaa cgtgacctgc 240
gagcgcgagg tgcccggtcc cgactgccgt ttcctcatcg tggcgcacga cgacggtcgc 300
tggtcgctgc agtccgaggc gcaccggcgc tacttcggcg gcaccgagga ccgcctgtcc 360
tgcttcgcgc agacggtgtc ccccgccgag aagtggagcg tgcacatcgc catgcaccct 420
caggtcaaca tctacagtgt cacccgtaag cgctacgcgc acctgagcgc gcggccggcc 480
gacgagatcg ccgtggaccg cgacgtgccc tggggcgtcg actcgctcat caccctcgcc 540
ttccaggacc agcgctacag cgtgcagacc gccgaccacc gcttcctgcg ccacgacggg 600
cgcctggtgg cgcgccccga gccggccact ggctacacgc tggagttccg ctccggcaag 660

```
gtggccttcc gcgactgcga gggccgttac ctggcgccgt cggggcccag cggcacgctc 720
aaggcgggca aggccaccaa ggtgggcaag gacgagctct ttgctctgga gcagagctgc 780
gcccaggtcg tgctgcaggc ggccaacgag aggaacgtgt ccacgcgcca gggtatggac 840
ctgtctgcca atcaggacga ggagaccgac caggagacct tccagctgga gatcgaccgc 900
gacaccaaaa agtgtgcctt ccgtacccac acgggcaagt actggacgct gacggccacc 960
gggggcgtgc agtccaccgc ctccagcaag aatgccagct gctactttga catcgagtgg 1020
cgtgaccggc gcatcacact gagggcgtcc aatggcaagt ttgtgacctc caagaagaat 1080
gggcagctgg ccgcctcggt ggagacagca ggggactcag agctcttcct catgaagctc 1140
atcaaccgcc ccatcatcgt gttccgcggg gagcatggct tcatcggctg ccgcaaggtc 1200
acgggcaccc tggacgccaa ccgctccagc tatgacgtct tccagctgga gttcaacgat 1260
ggcgcctaca acatcaaaga ctccacaggc aaatactgga cggtgggcag tgactccgcg 1320
gtcaccagca gcggcgacac tcctgtggac ttcttcttcg agttctgcga ctataacaag 1380
gtggccatca aggtgggcgg gcgctacctg aagggcgacc acgcaggcgt cctgaaggcc 1440
tcggcggaaa ccgtggaccc cgcctcgctc tgggagtact ag                    1482
```

<210> 7
<211> 26993
<212> DNA
<213> Mus musculus

<220>
<221> misc_feature
<222> (1)...(26993)
<223> n = A,T,C or G

<400> 7

```
ctcactccgc cagtctgaag catccgggct ctgtctgtga aacagtccgc gaactgggag 60
ggtctaccca ctggccccag cacccctcct cccaaggccc cccatgctgc tgcttcatgt 120
gaccagggct gggccgaatc tctgagcaga cagagagaag catggtgtga ccgcatgagt 180
ggagtgagtt ctggggacag tgcccagttg tgcgatagac agcagattcc ggaggaccac 240
tgtgcatgtg tgtgtgttgt tgtatttggt ttgtgtttgt gagtatgtct gtgtgtgtgt 300
gtgtgtgtga gagagagaga gagagagaga gagagagaca gacagacaga cagagacaga 360
gacagagaca aagagagttg tgtgaggacc actgtgcatg tgtgtgtgtt gtgtgaatgt 420
ttgtgagtgt gtgtgttgtt gtatttggtt tgtgtttgtg agtgtatctg tgtgtgtgag 480
agagagacag agagacagag agagagagag agagagagag acagacagac agacagacag 540
acagagagac agacagagag agacagagag agacagagag agagagagag ttgtgtgagg 600
gcctccccagg tgatctggga agcttgtgaa tgtgaaagtg cctgtgggtg gcttcacctc 660
tggcagaggc attctgcctc ctgtatatta actatgtgtg ctcagacaat ccactcaaag 720
atgctctggg gctgcagctg atagagcagc ttacttagtg tgcacaaggc cgtgggtgca 780
aacaccccag aactacaatc ccagaactct ctctgcgcac tgaactcgac ttcctcattt 840
gtttcttgtt tctttttttt gacggagtct aacattgtcc ggattgcttc aaactttata 900
gcggaggaag atcgatggca attcttctgc ctccaccttc tgagtgctag gattgcaggc 960
atgtaccctc atgcccagtt gagacagtat cagtgattga acccagggcc tcacgcctcc 1020
tggagaagcc ctgtaccatg gagctacaag tctagttcct ttcttgtcc ttaaaaaaaa 1080
taaaaagctg ggtcttctta tagcccaggc tggctttgta ctccccatcc tcctgcctca 1140
gcctcccaag tgctgggatg ataggcttat ggcacggcca tgatagcacc tacaatttcc 1200
agagttgcct gtcctttgca gtgtactatg ccttgtgagg gacaggctct gagggtctgc 1260
ttgtgatcac acacctgagg acctgtttgc tgggagttct gtccccattt ctttgccagg 1320
ctggaagaca ctgacacacc caccatggtg acagtaccca tggacttaaa tggtgtgact 1380
ccctctgctc taatgggagg gtctcagtgc cccgtatctg ctgtttctca aatggggtag 1440
gtaatacaga gataggaggg ctgacacaaa acatatcaaa caccaagata gcaaaggcta 1500
gaatgagacc ctgtctcaag ctttcccata gagaagggga gtgtataatt gcacccactc 1560
acaagactgt tggccataaa caagtttgta tggaaaatgt ttacaaagtg cttggcacat 1620
ccaggggtgg tggcgcacac ctggattctc atacttggag agaagagcca ggaagattag 1680
ggacggttcc agtcaggcct acatattgag accttatctc aaaaacaaaa caacacaaca 1740
cacacacaca cacacacaca cacacacacc tcaaaaacct ctggaagttc acattttggg 1800
aaaggcatct gaatggtaaa gtgtagggat taagagtaga gactagagtc cctccatggg 1860
accgagatgt agaaaagaaa gaaaagagag cttgctaaag caggccagcc taagggcaga 1920
ctcctgaaag gacttgctcc aaatgattgt tgagggtgga ggactctaag aaactggaat 1980
tgtgggaaaa actctcgggg aatgacaaaa gccggtttgc aggttggtga gattagaaag 2040
taatggccac aatatagggc acaatataca ggctggtctt gaacttgtaa tcctcctgcc 2100
tcaacctccc aactattata ggtatatgtc accagctgga tgcttttgcc tgtcttgttt 2160
ctttctgtgc cccagggctt cagtgaatag ggatggctgc cgattaaata actgaattgc 2220
tagcagggtg ggtgagtcag ggctttaatc ccagccttca ggaggcagag gcacacagat 2280
ctttgtgagt tcgaggctaa cctggttaac agagagagtt ccaggacagt cagagctaca 2340
cagagaaacc ttggctcaaa aaacaacaaa atatctgaat ggctgattga atgactgggc 2400
agatggacag atagacagat ggacatgtac gatccacaga catgtacgat ctttgggatg 2460
gtggggaagg cttggtagct aacacaggct ggctcaagac ctgcttctgg gaacccttc 2520
```

```
tttcctttgt atcctcagct ctgggtcatg aagggtgtgg agtgggccgg gaagggagtg 2580
tcaggctcag tggctgcttg ccttggattc tggcccagag tgtctggagg aagaagaggg 2640
tatctcggca tctgagtcac cttagagaaa ccctagccac atacctgact tctgacatca 2700
cacaaccagg gcaaccctgg tggccgagac aaggagactg aatcatggac tgaaatcagt 2760
caccttgggc atgggcacgt ggccgctagt gaccccctga gacacccccg agtggctgtc 2820
tggctgtgtg ggcatgcaaa ccacatcaag gtcagtaaag gctctccagc ctgagtcagg 2880
gacagggtgg ccactgccag agaccaccga ccacagcatc tgcaggacag gaagagaaag 2940
ttgagggaga aaaggcagag ggtaggcccc agtcagggag agggaagtgc ctgtgggaga 3000
aaggggaagc tgtggcttgc ggccagtagc ctggtgacct gtggctggga tgtggaatgg 3060
ggtcttagtg tttgcgcaca gaatggcgaa gttgcaagcg aggctcagag gacaatggag 3120
tcagcagaca atgggctctt acacttgcac acgcaggcac aggcgcacaa aagccagcgt 3180
gggagcccta ccctccagcc accccagagc tacagcttgc cacacacaca caagactgtc 3240
cccacaagac tcgcttcctt taccctatta agtgacatcc gtaattctca ttgtgaagat 3300
tgcaactctc ggtattttga tgatctcatt tgatatgaat aacttagaca tttgcagtgc 3360
caggcactgt tgtagactat ttagctgtgc tagggattga acccagggct tcttgcattc 3420
tgggcaaaca tcctgccact gagcttgctc ccagacccac gtgcccatgg tttgtcccca 3480
gacccctgtt ttatatgtaa ttctgagaca aggtctcact gtaatgccca gagacccttg 3540
agctcactct attacccagc cttgaactca ctgatccttc tgcagggatt gtaggcatga 3600
ctagctagac acagctaagt atctgttaga gcgtgtatgt gtaccttggg gcagacatct 3660
gtccaactgt atatgtctat acattgttag gtttcaaaca aatggctgag gtgcctattt 3720
aacatatcaa gaggacctgg cccccaattt ctccctgcaa taactcagct cctatatgcc 3780
ctcctggctg gcatacccca cccgctatgc tgaacttctc cagcccaggg gctgggctgt 3840
tcatccctat gtaatccaac tattttagct ctcccctctc tttgtacctt aaggctgcac 3900
ctggctccca tggctcccct ccctctctcc ccacacggct cagggttaag tcgactctgg 3960
actatcctag aggtccctgc ctctggctat gctctcccac atatctataa taaactctcc 4020
tctgcatacc taggaacagt catgttccct tcctttctct ttcttaattt ccttttcttt 4080
tcattcatac atacgtttct ttggattctg tattctattc tccctcctta aaaatataca 4140
cagctagcca ggcatggtga tgcacacctt taatcccagc actcgagagg cagacacagg 4200
tggatctctg tgagttcgag accagcctag tctacagatt gagttccagg cctgtctggc 4260
ctatacagtg agatcctcaa aaagaaaaat gtacacacac taccccccttc aagtcagaag 4320
accctgagac acaagcccat ccatcgccgg gcagcatggg aaaagcaggt tgttacaaat 4380
gcctggagtc aggtctgaca ctcacacaca aggtccctgc caccagttga tccagcagct 4440
acattcgaag gatcagagag gtccagataa ctgtgctgcc ctgtcagacc ttatgaatga 4500
agacctgaat ggaggctgat gtctgtaatt ccagcactcg ggaggcactc aggaggcaag 4560
gaggattgga gggagttagc tggaggccag cctatgctat agagtaagac tgcttcaaac 4620
aagagaaatg agaactgaaa agattgctca gtgggtagag gcgcttgcct gcaagtctaa 4680
ggaactgagt tcaattcctg acatttgcat gatggaagaa aagaaactcc ttcaaggtca 4740
cacacacaca cacacacaca ctcaaagaag taaaaatact ttttaattta aaagaacaga 4800
gaaacttgaa cacatggtcg gtctgcgatg tggctctgca gtaatgcctg ccttggatgt 4860
atgtagtcct ggttttgacc ccaccattat acagaccagg tgtggtgata caaacctgta 4920
attccagcac tgggggctgg agccaagacg atgggagaag ttctaggtgg ctcatccttg 4980
gctaaaaaaa catagcctga gctacctgac actggttgct agtgtgagct atctgagact 5040
ctgcctcaca aataattaag taaaaatatg gtagattcat gacgacagca cctacagatt 5100
gcctgtcctg tgctgggaca cagggcagct tcacactaac cctgatggga acacctggga 5160
gacgacatgc ttgttgcaag gcgctggctc atctgagacc aaagcgactt caggagccaa 5220
gtagaccaga atccgtgtgc ttcagacagt gggaagggca tacagaaacc tcaggaactg 5280
aggcacagca gggagatggg aggctgggac aagggaccac tgacagacac catacatcat 5340
accatcttgt ccctctgaat ctccatgggc accccctctg ggaggactcc aggactgcaa 5400
gcaaaggtta actgagtcag ccaattcaac cctggcctag gggtggagg caggggggtct 5460
atcagcgcga ggagacagga caaggacact catgtattgc catagggatg gctctcttgt 5520
caaaccatca gaacacccac aaacatcccg gctcacaggg gtctgcaggg gtagggctgg 5580
gatgtggccc gagacttgtg ggaactgagg aatgtagtgg caaaagtttg gccaagggaa 5640
gaccagagga ctctgtatgc atgaaggcca gggtgatgac atttcaggaa agaaaagcac 5700
agaatcaagc atggtaggta gttggaaagt ggaagcgggn nnnnnnnnnn nnnnnnnnnn 5760
nnnnnnnnnn nnnnnnnnnn nnnnnnnnnn nnnnnnnnnn nnnnnnnnnn nnnnnnnnnn 5820
nnnnnnnnnn nnnnnnnnnn nnnnnnnnnn nnnnnnnnnn nnnnnnnnnn nnnnnnnnnn 5880
nnnnnnnnnn nnnnnnnnnn nnnnnnnnnn nnnnnnnnnn nnnnnnnnnn nnnnnnnnnn 5940
nnnnnnnnnn nnnnnnnnnn nnnnnnnnnn nnnnnnnnnn nnnnnnnnnn nnnnnnnnnn 6000
nnnnnnnnnn nnnnnnnnnn nnnnnnnnnn nnnnnnnnnn nnnnnnnnnn nnnnnnnnnn 6060
nnnnnnnnnn nnnnnnnnnn nnnnnnnnnn nnnnnnnnnn nnnnnnnnnn nnnnnnnnnn 6120
nnnnnnnnnn nnnnnnnnnn nnnnnnnnnn nnnnnnnnnn nnnnnnnnnn nnnnnnnnnn 6180
nnnnnnnnnn nnnnnnnnnn nnnnnnnnnn nnnnnnnnnn nnnnnnnnnn nnnnnnnnnn 6240
nnnnnnnnnn nnnnnnnnnn nnnnnnnnnn nnnnnnnnnn nnnnnnnnnn nnnnnnnnnn 6300
nnnnnnnnnn nnnnnnnnnn nnnnnnnnnn nnnnnnnnnn nnnnnnnnnn nnnnnnnnnn 6360
nnnnnnnnnn nnnnnnnnnn nnnnnnnnnn nnnnnnnnnn nnnnnnnnnn nnnnnnnnnn 6420
nnnnnnnnnn nnnnnnnnnn nnnnnnnnnn nnnnnnnnnn nnnnnnnnnn nnnnnnnnnn 6480
nnnnnnnnnn nnnnnnnnnn nnnnnnnnnn nnnnnnnnnn nnnnnnnnnn nnnnnnnnnn 6540
nnnnnnnnnn nnnnnnnnnn nnnnnnnnnn nnnnnnnnnn nnnnnnnnnn nnnnnnnnnn 6600
```

nnnnnnnnnn nnnnnnnnnn nnnnnnnnnn nnnnnnnnnn nnnnnnnnnn nnnnnnnnnn 6660
nnnnnnnnnn nnnnnnnnnn nnnnnnnnnn nnnnnnnnnn nnnnnnnnnn nnnnnnnnnn 6720
nnnnnnnnnn nnnnnnnnnn nnnnnnnnnn nnnnnnnnnn nnnnnnnnnn nnnnnnnnnn 6780
nnnnnnnnnn nnnnnnnnnn nnnnnnnnnn nnnnnnnnnn nnnnnnnnnn nnnnnnnnnn 6840
nnnnnnnnnn nnnnnnnnnn nnnnnnnnnn nnnnnnnnnn nnnnnnnnnn nnnnnnnnnn 6900
nnnnnnnnnn nnnnnnnnnn nnnnnnnnnn nnnnnnnnnn nnnnnnnnnn nnnnnnnnnn 6960
nnnnnnnnnn nnnnnnnnnn nnnnnnnnnn nnnnnnnnnn nnnnnnnnnn nnnnnnnnnn 7020
nnnnnnnnnn nnnnnnnnnn nnnnnnnnnn nnnnnnnnnn nnnnnnnnnn nnnnnnnnnn 7080
nnnnnnnnnn nnnnnnnnnn nnnnnnnnnn nnnnnnnnnn nnnnnnnnnn nnnnnnnnnn 7140
nnnnnnnnnn nnnnnnnnnn nnnnnnnnnn nnnnnnnnnn nnnnnnnnnn nnnnnnnnnn 7200
nnnnnnnnnn nnnnnnnnnn nnnnnnnnnn nnnnnnnnnn nnnnnnnnnn nnnnnnnnnn 7260
nnnnnnnacc acatttacta agcccctccc tgccaaagta agcaataaat tatggctaag 7320
agtctcccca ctcctaagat ggaaagatgc caagctgcaa aggagatcct gacaacagac 7380
cagctacttg gaagactcag aacctagcca agctgcctgc aagaggttta gaccaactaa 7440
gacatctgga gagagcaccc tccaatctgt ggagcagtct tcaggctctg cagggtgctc 7500
caggctccca gctttgtaag ctgtcactca tgctggggcg ggcttgggtg acgcagctgt 7560
ctttgagtga cttctcccgt aagtcacccc tcacccatgc acactcctgt aagtaactct 7620
aatagaaaat tcatcggttc agcaaattgg actttggtct cttctgagct ggctccctgt 7680
ctagggtgaa tggacatgtg tgtgtggcat ctcccctgga aaagttttct ttccctaggc 7740
ttggcctggg gtctcccagg ctttttgcag ttgctggtgg catccttctt agtgggtact 7800
taagaatcag gaccctggcc agcgatgcag cagccgttgg cagagggctc ccctagcatg 7860
caggaggccc ctggattaca tccccagtaa taaaccaggc ctggcgacaa tatagaggtg 7920
agagcaagat gttcattgtc atccttgggt acatagtaaa ttctagtctt tgttttaaaa 7980
acaaacaaac aaacaaacaa gcaggacata agatcagata tgcctggcat ggtggaaggt 8040
gcctttaact ccaacactca agaggtagag gtcatcctgg cctacagaat gaattccagg 8100
ccaaaggtgg tacataatga gaccccatct caaaaacaga cagagaaagg aggagggcaa 8160
agggtagaaa gaggagggga tgttcacaat agggggaggg gatgttcaca atacgtgtat 8220
atatattctt tccttctgga tgtggataga acccaggact tcacacatgc tagacattca 8280
ttccacttta agctacattc ccgccctcac atcccctttt gtttaaatca ttaatattga 8340
tattattatt attattatta ttattattat tactacatat gagtactggt tgttttctgt 8400
ctttgcatgc atgtggagat cagagaacaa tttttaggag tcatttttct tcttccaccg 8460
tgagtcccag ggatgggact caggttgtca ggtatgtgta gcaagctcct ttacctgtga 8520
accatcttgc tggggctgat acctgctgat tgctaaacac atgccaccga ttatattaat 8580
gcactagtat tttatttatt tatttttcga gacagggttt ctctgtgtag ccctggctgt 8640
cctggaactc actctgtaga acaagctggt ttcgaactca cagagatctc cctgcttctg 8700
ctgggattaa agatgtgtgc tgctgccgca gctgccacca ccaccagaca ccaccaatta 8760
ttattgttca attattattg tatacacact ttcttttctg tctcctgagc taagtgggaa 8820
ggagaggctt ccaacaccat caagcctgaa gaactcaccg ggtgtgggtt cctacctgct 8880
ccaattctgc aggtccccca gattcaggga cccggaaaag ccacctatcc tttgcttagc 8940
tatgagtact gaagttttag atcagggagg gagcacattt tccaagctca ccgagtcaca 9000
cagaatccgt gacaaagcta gtggggaggg gactgcatcc ttacccatcc ctacacctgg 9060
ggatatcaca gactctccaa ttccccctga gcctcagttt ccccacctag actgattata 9120
cccctctcgc cagaatctgc actggggacc ttgctctttt tcaccagcag ggcgctgca 9180
ccggtttggg gagggtgggg tcccaggggt ataagcagac ctgggatctg gagttgcacc 9240
ttctccaacc cgggtcagca ggggccttct gagggagttt aggcgcctgt caatctcagc 9300
ctccgggaca gcgtggaact gcgcaggcgc gggcgggttc cgcacagcgc aacagcgggc 9360
gcgcgcggga gcgagggatt ccctctgacg taattgctag gataccaaac aaacactggg 9420
ccgcgctggc cgagctcctt atatggctaa ttgcgtcaca ggaactccgg ggagggcggg 9480
gcgggatccc ctcccgcgaa gcccctcaga acgcagcctt ggggaccccc gagaccccca 9540
gggtcacact atgggcaggt ggcaggtgca cgaggccctg gagggcgcca caaggagctc 9600
agtcggcggg aagggagagt ttgggaggtt tgtgcatgga gttgcgggtg acgcaagcgc 9660
ggggggcggg tcccggaggc ataaattcag cccggcagct cccggtttca ttcataagac 9720
tggagcggct acgccgggga cacgcggagc cggggcttgc tggacttgac tactggtgac 9780
tctttctttt tcttcttctt ggaggccgtg aaaaatttat atatatatat ttatatatat 9840
ttttaaactg gagagaaagt tttgtgggtt tcctttttgc aagactcttt ctagatcgtt 9900
cttccgattc gtccggagga tagactttct ttctgtgggc ttctggatcg cgcccctctg 9960
ctctccctca tttcttggga cgcttcgaca ggattggaac tgcatctgaa gcgcacgctg 10020
caccgcggca ctgcccggcg ggtttctggg cggggagcga tccccgcgtc gccccccgtg 10080
aaaccgacag agcctggact ttcaggaggt acagcggcgg tctgaagggg atctgggatc 10140
ttgcagaggg aacttgcatc gaaacttggg cagttctccg aaccggagac taagcttccc 10200
cgagcagcgc actttggaga cgtgtccggt ctactccgga ctcgcatctc attccactcg 10260
gccatagcct tggcttcccg gcgacctcag cgtggtcaca ggggcccccc tgtgcccagg 10320
gaaatgtttc aagcttttcc cggagactac gactccggct cccggtgtag ctcatcaccc 10380
tccgccgagt ctcagtacct gtcttcggtg gactccttcg gcagtccacc caccgccgcc 10440
gcctcccagg taagttctag atcgtaaaga ttctacttta gtggttgggg gggggtgttt 10500
ctttcaggct aaagtgtaga ttgagcatcc tctcaaatag agacgcgcca aaacccagga 10560
tctgggattg caatagttcg gtttgcactt tggtttttttg ttgtttgcaa actgcaaaga 10620
atggagtgat gtttgcaaaa ggttatttgc gcggagcgcg ggaaaggaat gcagctgggc 10680

aaacgttggc gatgcccggt gcaaagtata tacccggtgg ttagcagaag ctgagaactt 10740
ttagccgaaa gccggctccc taagccgaag ctaggcaagt aggggaagaa aaagaaacaa 10800
aaaattccag agaagcttcc aggagcctcc tcctcttccc tcttccttca aaacgcggac 10860
tgcaagtccg cagtcaccct ccacccagca agagttaggg cctcgaaccc cggtcacgct 10920
gcctccgcct cctgcgcgga gacgtaacgg gggacccgtg cgtaaaggct gacgcgctgg 10980
aatcctccgt ctgacgcggg gcacgcacag cgcgcagcgc cccctccgcc cgccccgccc 11040
ctgacgtccc gggcacgttc tattttggaa cgccgaggcc acgttgctaa gggagggggc 11100
agcgtggctt tgtgattggc tgtcgcggcg cgagctttag ccaatcagcg ttcccttcct 11160
atttgtagag cgtagctccc ttccttgctt tttgtggttc ttcccgtgct gggggtctcc 11220
aagagggaga gctaggggat tcttgtcgcg atcgggggac tcgttgtcac cccatgggtc 11280
tgcgaggacc ttgtgtggac ctggtcctgt tgtcataagc tagaggcttt tggctgagtg 11340
ttagcgcctc taagggggaa ctgaaggcct catcccttct ccaggcacac atatacgtgc 11400
tccctgagct ctagacactc agtccttccg aggtgttcaa acactagatg agctagccta 11460
cggagaggca gccaggtggt ctctaaaagg tccgccttcc cttagttccc agggctctga 11520
ttggccaggg attcagccct tccctcgcca cgccccctag agtagttaag cctctaggat 11580
tccacttgcg ggaagggggg ggggggcgtg atggacgctt cttgggttcg gggacgcaga 11640
tcctatgtca ccccatcccc tgcaagacag tctgagagat tctcgctgtc acttttctct 11700
gcctatcagt tcactgaaac ctgtcagtct cactggggaa gagacagaca ctcggaaggg 11760
atgctctcaa ctcttaggcc ggtcccccaa caccgttgga actgggatct ccgcccctgc 11820
gggagccctc atgcagtggg gggtgtgttt gtgtgtgagt ggaggagagg aaggcttggg 11880
ctaaggcctc tccctctccc tacctactgt ggtgggggtg gggtgttttg gctgtatgtg 11940
tgtgtgannn nnnnnnnnnn nnnnnnnctg acttgaaggg ggtagtgtgt gtacattttt 12000
ctttttgaga caggatctca ctgtataggc cagacaggcc tggaactcaa tctgtagact 12060
aggctggtct cgaactcaca gagatccacc tgtgtctgtg gctccagtcc cgggattaaa 12120
ggtttgcatc accaggttct gtccagcctc cgtctcccac ccaccccccc acacctaaga 12180
gtcaccaacc cggggtgtga ttcaccaccc gctggaaccg tgcaaccttt ccccgaggaa 12240
gaaggaggag gtagaaggca gttgaacaga atctctcatt aaccactgcg tcacggtgta 12300
gtggaagggt gggtgttgtg gctttttgcc tgtgacacac acatccacac ccgctcaccc 12360
tgtgctcact cacaggggtc ggtgtgtgtt atgtgtgttg ggcgtgtgtg tgtcggtggc 12420
tttgtttgtg tgtctacgcc tgtgtgtgta tgtctcaccc cgtaggagtg cgccggtctc 12480
ggggaaatgc ccggctcctt cgtgccaacg gtcaccgcaa tcacaaccag ccaggatctt 12540
cagtggctcg tgcaacccac cctcatctct tccatggccc agtcccaggg gcagccactg 12600
gcctcccagc ctccagctgt tgacccttat gacatgccag gaaccagcta ctcaacccca 12660
ggcctgagtg cctacagcac tggcggggca agcggaagtg gtgggccttc aaccagcaca 12720
accaccagtg gacctgtgtc tgcccgtcca gccagagcca ggcctagaag accccgagaa 12780
gagacagtaa gtatgaggcc tcaggagttg ggatggagga gcctagctag ggatgtgggc 12840
tcagtttgta cagtgcttgc tgccatgcat gaagatccct agcacagcat aagccaggag 12900
tggttatgca gacctgtaac cccagctctc agaaggtgga ggcaggagga gcaggagttc 12960
gaggccagcc tgtgctactt atggagtcca gcctgcactg caagagatca ttattttcaa 13020
aagttggcct tggggggagg tgggtgaggg aagtaagaga aagtgacagt aattttatca 13080
cttaatagtt ggaggttcct ctgaggcctc aagtctgaag gaactttacc attctggcca 13140
gtgaggagta ggggttatta tttggggttc aggaggaagg agttttctta gggctgatag 13200
aggtaccccc agatctcatg gtccttatct ctgactcagc ttaccccaga agaagaagaa 13260
aagcgaaggg ttcgcagaga gcggaacaag ctggctgcag ctaagtgcag gaaccgtcgg 13320
agggagctga cagatcgact tcaggcggta aggaggagtc tgggggtgtc ttgaggccgt 13380
gctgggagca ctctgccttg ttcttccccc gtttctcact gtgcctgtgt cctaaacgag 13440
gaaacccccct cttagggaac aggggtcagt ataggctgat ggagtggctc catatgcatg 13500
ctcagaccca tgcccactta ctttcgactg ttccccactt tccctgaata tgtccccaca 13560
tgtcaccctc ctggctttct ctcagcctaa ggagacaagc tggaggaggt aattctctca 13620
ccttctttcc ttcactaaat aataatccat tttgccttcc tgcctccatt tttttttcct 13680
gagctgggga tctacctgtg tagttcagcc ctcctccccc aacttgatag cctcaagttt 13740
cagcccttgg ctgagatgcc atcatcctga ctggctctgg ctggaaacta ttttgtgcta 13800
agtcaattcc ttacctgcta ctccagctat ctacagttct gccgaacttg agctggtggc 13860
gcccaccaag cccacttctt tctctcttct acctcagtgc aaccccccaca cacacacaca 13920
cacctcatgc ctgccccttg aaaccagggt gtgtctctga tttcccgtcg ggaggctgaa 13980
ggagatgggt aacagaacct cattaaaaac aacacataag cattacctac tgactcaaca 14040
aactgtagtg tttttctttt ttcctctcaa aaaattattt cgtttgttta tttattattt 14100
gcttatgttt gagtgagtgc tggtgcacca cagcacacat acgaggtcag agggaaattt 14160
tcatagtttg ttctctcctt ccgtgttgtg ggtgcttgct ggcaatctcc ttcactcagt 14220
gagctacaat gccccccttct gccctttaag gcagagtact ccttagtaca gggggacccct 14280
ttcctcggcc tctcaaagtt gagattacaa atgttcacca tcacaccagg cttggagttc 14340
ttgcctatca gtgacgtcca ctcctgccta gcttcttccc aaccatctct tagtctgatg 14400
gggaaaccga ggcacgagta gcatggtcta ccaggatttc ctcttagggg acggtcccct 14460
cagttgggag ggagctgtcc agccccctgg atcagcagca agaatgtatg agtgtggggt 14520
tgggcgggtg aagctactct gtgtggtcgc tgaccagcaa ttctcctttc tctgtctcct 14580
atgacctggc cctgctggga tccattagga aactgatcag cttgaagagg aaaaggcaga 14640
gctggagtcg gagatcgccg agctgcaaaa agagaaggaa cgcctggagt ttgtcctggt 14700
ggcccacaaa ccgggctgca agatccccta cgaagagggg ccggggccag gcccgctggc 14760

cgaggtgaga gatttgccag ggtcaacatc cgctaaggaa gacggcttcg gctggctgct 14820
gccgcccccct ccaccaccgc ccctgccctt ccagagcagc cgagacgcac cccccaacct 14880
gacggcttct ctctttacac acagtgaagt tcaagtcctc ggcgacccct tccccgttgt 14940
tagcccttcg tacacttcct cgtttgtcct cacctgcccg gaggtctccg cgttcgccgg 15000
cgcccaacgc accagcggca gcgagcagcc gtccgacccg ctgaactcgc cctcccttct 15060
tgctctgtaa actctttaga caaacaaaac aaacaaaccc gcaaggaaca aggaggagga 15120
agatgaggag gagaggggag gaagcagtcc gggggtgtgt gtgtggaccc tttgactctt 15180
ctgtctgacc acctgccgcc tctgccatcg gacatgacgg aaggacctcc tttgtgtttt 15240
gtgctctgtc tctggttttc tgtgccccgg cgagaccgga gagctggtga ctttggggac 15300
agggggtggg gcggggatgg acacccctcc tgcatatctt tgtcctgtta cttcaaccca 15360
acttctgggg atagatggct ggctgggtgg gtagggtggg gtgcaacgcc cacctttggc 15420
gtcttgcgtg aggctggagg ggaaagggtg ctgagtgtgg ggtgcagggt gggttgaggt 15480
cgagctggca tgcacctcca gagagaccca acgaggaaat gacagcaccg tcctgtcctt 15540
cttttccccc acccacccat ccaccctcaa gggtgcaggg tgaccaagat agctctgttt 15600
tgctccctcg ggccttagct gattaactta acatttccaa gaggttacaa cctcctccgg 15660
gacgaattga gcccccgact gagggaagtc gatgccccct ttgggagtct gctaacccca 15720
cttcccgctg attccaaaat gtgaacccct atctgattgc tcagtcttgc cctcctggga 15780
aaactggctc aggttggatt tttttcctca tctgctacag agccccctcc caactcaggc 15840
ccgctcccac ccctgtgcag tattatgcta cgtccctctc accctcaccc ccacccccagg 15900
cgcccttggc cgtcctcgtt gggccttact ggttttgggc agcagggggc gctgcgacgc 15960
ccatcttgct ggagcgcttt atactgtgaa tgagtggtcg gattgctggg tgcgccggat 16020
gggattgacc cccagccctc caaaactttc cctgggcctc cccttcttcc acttgcttcc 16080
tccctcccct tgacagggag ttagactcga aaggatgacc acgacgcatc ccggtggcct 16140
tcttgctcag gccccagact ttttctcttt aagtccttcg ccttccccag cctaggacgc 16200
caacttctcc ccaccctggg agccccgcat cctctcacag aggtcgaggc aattttcaga 16260
gaagttttca gggctgaggc tttggctccc ctatcctcga tatttgaatc cccaaatatt 16320
tttggactag catacttaag agggggctga gttcccacta tcccactcca tccaattcct 16380
tcagtcccaa agacgagttc tgtcccttcc ctccagcttt cacctcgtga gaatcccacg 16440
agtcagattt ctatttttta atattgggga gatgggccct accgcccgtc ccccgtgctg 16500
catggaacat tccataccct gtcctgggcc ctaggttcca aacctaatcc caaaccccac 16560
ccccagctat ttatcccttt cctggttccc aaaaagcact tatatctatt atgtataaat 16620
aaatatatta tatatgagtg tgcgtgtgtg tgcgtgtgcg tgcgtgcgtg cgtgcgagct 16680
tccttgtttt caagtgtgct gtggagttca aaatcgcttc tggggatttg agtcagactt 16740
tctggctgtc ccttttttgtc actttttttgt tgttgtctcg gctcctctgg ctgttggaga 16800
cagtcccggc ctctcccttt atcctttctc aagtctgtct cgctcagacc acttccaaca 16860
tgtctccact ctcaatgact ctgatctccg gtctgtctgt taattctgga tttgtcgggg 16920
acatgcaatt ttacttctgt aagtaagtgt gactgggtgg tagatttttt acaatctata 16980
tcgttgagaa ttctgggtgg aaatgtctga tcaggagaag ggcctgccac tgccgaccac 17040
aattcattga ctccatagcc ctcacccagg ctgtatttgt gatttttttc attttgtttt 17100
tttgtatttt gcacctgacc ccgggggtgc tggggcagtc tagcactggg cagctcccct 17160
ccccccccttg gttctgcact gtcgccaata aaaagctttt aaaaaactgt attcttcagg 17220
tcaaagtgtc tgttttccct ggacatctac tacatggctt cctttcagaa aaacggagtt 17280
tggattgcta gggaggtctt gctggcactt agtgggacgc ctaatgaatc agaacctaca 17340
acgggactaa aaggaagtgg agacttgcta ggttttccca tgttcccagg ctgggccacc 17400
tacttgaaaa aataaggggc ggaaaagtgt aaggtacaaa tttggtgaag ggtctggaag 17460
acttcatgat cggaaaagaa tttattcacc ttgggtgtgc aatgacttca gcaacagcta 17520
agggcaaggt gtaaaagctg ggcacacttg taaatcctag catttgagag gtggaggcaa 17580
ggggatcact ggtggagttc agtgtcatgt ggatcgtaga taccaagcgc aaagatctgc 17640
tatggggaga gggcttggta caccagggga gccagaagtt cgtggtgagg gtagtggagg 17700
gcaggtggag agtgagagtt agcctcaggg agattctaca ggcaatgatg cagagttcag 17760
acgctccttt gaaagcacta gagagccgca gcaggttttg agcagagaag gttagcgtta 17820
ggtggtctct tctagcccat cccaggctga ggaggacgct gagggtttca agaaggatcg 17880
agaatggaaa gcagaggaga aaaaggatcc aagaggcatg gaggaggcag aacacatttc 17940
tcttctttaa tagcaagcct ggaaaggata acttgctgca ggaggagatg ctcaccagtc 18000
gggtggtcta gggggttctt ggaaaagaga aggcatttgc tcaagcctcg gttcccccat 18060
tctcgctctt ctgtcagctt gtcttccatt aagtgtgtgt ctcaaggcca ccctgctcag 18120
gactccttgt gagacgacct tctatgctcg agttcattaa aaacacaatt gcctggtgcc 18180
ctgctctctc cactggctca gttacctcaa aagaccaggg ctaaaggtgt gatcacaact 18240
ctatccccat tactgctcca acgcagagac aggactgagc cggagtgaac aaatgaacaa 18300
aaatgactaa taatgcatgc gtgattaaat acataaaaga gcagatgact ggatgagcaa 18360
atcgtttaag gagagacagc aagatcctag aattttggag actaatttaa atccatcttt 18420
gagatgtatt tggtcggaaa ttcctgggag gaaaaaaagt gtaagtatga agagagaata 18480
aatgagaata ggggtggctt cagagaggtt aactgcgcgc tggtcgcttt tgtacaagaa 18540
tgtgaattgc agggagcaaa atgggataga tactcccgcc cgaaaggtgg aattgaacca 18600
ctctgtcgct aaacagctac aggtttgaag cctgcacccc agaccactga ggatcatccg 18660
ggcgaaagga gctattttcg gttagttata taaaggccag atactactac tttttcact 18720
tatggtcatt atttgtggta tacagtagat aattaatttc aatggtttcg aacatttttt 18780
ttcacttttt cttgtgaaca tgtgtttcct cagtaaagtg ttccgtgaat gactctacta 18840

```
actaaaaagt aagtagcttc atttgcatag cgccttgcat tctgggaagc agcgcctaaa 18900
gtgcctgtct ccctaactaa aagcagaatt ttttgcaaag tgaaaagtca gttttatttt 18960
tgtttgtttg tttgtttgtt tgtttttaat ggaaaaactt ctcacgcggc ccattcgtag 19020
cagaattcga gattttctgc aagcgagacc gtagggtctg acggcacgcg gccgcagagc 19080
gacacctgcc gttgctttat agaactgcaa gtatgtaggg aatctactga gtccctaagg 19140
tgatggagtt gacaaccaac tccccttgcg tttagacgct aaaaaccatc cctttttata 19200
tctatgtgat tagcccaggg aaactaaggc tcagacatgg ataataccac agccgagttc 19260
ctgtagccca actccctagg ggaaatgaaa cctacagttg tggttttaat atgcttggcc 19320
caggggcagt ggccctattg gcaggagtgg ccttattaga ggaggtgtac cttgttagag 19380
gaagtgtgtc acttggaggc gaggttttga ggtacgtatg ctcaagtctg gccagtgtga 19440
tcctggctgt ctgcagaacg cggtctcctg gctgccttcg gatcaaggtg tagaactctc 19500
agctccttct ccagcaccat gtctgcctgc ttaatgcttt gcttctttcc atgacgataa 19560
tgaactgtgc ctctgaaact gtaagtcaac cccaattaca caattacatg ttttctttta 19620
taagagttgc atatatatat atatatgtca cactctttag ctctggctgg cctgaaattc 19680
actatgtagc ccaggattgc ctgaactttg aagcaatctt cctgcctcag cctcccaatg 19740
gtattacagg catgagtcac aacaagccat ttaaatctta tgatgactta taagaagaca 19800
gaaaatcaga gttcctttac ctagttcaca gatccctaca atctaacctc gttcgctcca 19860
taaacagccc taccccaccc tcctggaact gctttgagga atgctgcagg ctctcacagg 19920
cacactcctc cttggttaat ctcttcagcc tggttgcctt ccctccccat gtccatgtgg 19980
cccaaagcct ctcatcctgt tctcaaatac cactagctag taaggcctcc ccgacctgac 20040
cccggtttaa atattagaaa agggtcactt tctcccctgc cacagacaac caaaccacca 20100
tatgcttgtc acttactacc tgactatgaa ggtgaataga tgtcttcaca acctttctct 20160
gagcctcagt ttccccacct gcataatgca tctgagacac agaattccct agagctgtgg 20220
ttctcctcat tcctagtgct gggacccttt aatacatttc ctcatgttgt ggtgactcca 20280
ccaccaccat aaaattattt tcattgatac ttcataactg taattttttc tattgttatg 20340
aatagtaatg taagcatttg tgtttcccag tgatcttaga tgaccctgtg gaagagtcat 20400
tccaccccca aaggggtccc caccacaagt taagaattcc tgccatagag gaatcacaag 20460
gaaccaatga attacacttt gggtcgactt ttgggctggc ctctgggagg cgnnnnnnnn 20520
nnnnnnnnnn nnnnnnnnnn nnnnnnnnnn nnnnnnnnnn nnnnnnnnnn nnnnnnnnnn 20580
nnnnnnnnnn nnnnnnnnnn nnnnnnnnnn nnnnnnnnnn nnnnnnnnnn nnnnnnnnnn 20640
nnnnnnnnnn nnnnnnnnnn nnnnnnnnnn nnnnnnnnnn nnnnnnnnnn nnnnnnnnnn 20700
nnnnnnnnnn nnnnnnnnnn nnnnnnnnnn nnnnnnnnnn nnnnnnnnnn nnnnnnnnnn 20760
nnnnnnnnnn nnnnnnnnnn nnnnnnnnnn nnnnnnnnnn nnnnnnnnnn nnnnnnnnnn 20820
nnnnnnnnnn nnnnnnnnnn nnnnnnnnnn nnnnnnnnnn nnnnnnnnnn nnnnnnnnnn 20880
nnnnnnnnnn nnnnnnnnnn nnnnnnnnnn nnnnnnnnnn nnnnnnnnnn nnnnnnnnnn 20940
nnnnnnnnnn nnnnnnnnnn nnnnnnnnnn nnnnnnnnnn nnnnnnnnnn nnnnnnnnnn 21000
nnnnnnnnnn nnnnnnnnnn nnnnnnnnnn nnnnnnnnnn nnnnnnnnnn nnnnnnnnnn 21060
nnnnnnnnnn nnnnnnnnnn nnnnnnnnnn nnnnnnnnnn nnnnnnnnnn nnnnnnnnnn 21120
nnnnnnnnnn nnnnnnnnnn nnnnnnnnnn nnnnnnnnnn nnnnnnnnnn nnnnnnnnnn 21180
nnnnnnnnnn nnnnnnnnnn nnnnnnnnnn nnnnnnnnnn nnnnnnnnnn nnnnnnnnnn 21240
nnnnnnnnnn nnnnnnnnnn nnnnnnnnnn nnnnnnnnnn nnnnnnnnnn nnnnnnnnnn 21300
nnnnnnnnnn nnnnnnnnnn nnnnnnnnnn nnnnnnnnnn nnnnnnnnnn nnnnnnnnnn 21360
nnnnnnnnnn nnnnnnnnnn nnnnnnnnnn nnnnnnnnnn nnnnnnnnnn nnnnnnnnnn 21420
nnnnnnnnnn nnnnnnnnnn nnnnnnnnnn nnnnnnnnnn nnnnnnnnnn nnnnnnnnnn 21480
nnnnnnnnnn nnnnnnnnnn nnnnnnnnnn nnnnnnnnnn nnnnnnnnnn nnnnnnnnnn 21540
nnnnnnnnnn nnnnnnnnnn nnnnnnnnnn nnnnnnnnnn nnnnnnnnnn nnnnnnnnnn 21600
nnnnnnnnnn nnnnnnnnnn nnnnnnnnnn nnnnnnnnnn nnnnnnnnnn nnnnnnnnnn 21660
nnnnnnnnnn nnnnnnnnnn nnnnnnnnnn nnnnnnnnnn nnnnnnnnnn nnnnnnnnnn 21720
nnnnnnnnnn nnnnnnnnnn nnnnnnnnnn nnnnnnnnnn nnnnnnnnnn nnnnnnnnnn 21780
nnnnnnnnnn nnnnnnnnnn nnnnnnnnnn nnnnnnnnnn nnnnnnnnnn nnnnnnnnnn 21840
nnnnnnnnnn nnnnnnnnnn nnnnnnnnnn nnnnnnnnnn nnnnnnnnnn nnnnnnnnnn 21900
nnnnnnnnnn nnnnnnnnnn nnnnnnnnnn nnnnnnnnnn nnnnnnnnnn nnnnnnnnnn 21960
nnnnnnnnnn nnnnnnnnnn nnnnnnnnnn nnnnnnnnnn nnnnnnnnnn nnnnnnnnnn 22020
nnnnnnnnnn nnnnnnnnnn nnnnnnnnnn nnnnnnnnnn nnnnnnnnnn nnnnnnnnnn 22080
nnnnnnnnnn nnnnnnnnnn nnnnnnnnnn nnnnnnnnnn nnnnnnnnnn nnnnnnnnnn 22140
nnnnnnnnnn nnnnnnnnnn nnnnnnnnnn nnnnnnnnnn nnnnnnnnnn nnnnnnnnnn 22200
nnnnnnnnnn nnnnnnnnnn nnnnnnnnnn nnnnnnnnnn nnnnnnnnnn nnnnnnnnnn 22260
nnnnnnnnnn nnnnnnnnnn nnnnnnnnnn nnnnnnnnnn nnnnnnnnnn nnnnnnnnnn 22320
nnnnnnnnnn nnnnnnnnnn nnnnnnnnnn nnnnnnnnnn nnnnnnnnnn nnnnnnnnnn 22380
nnnnnnnnnn nnnnnnnnnn nnnnnnnnnn nnnnnnnnnn nnnnnnnnnn nnnnnnnnnn 22440
nnnnnnnnnn nnnnnnnnnn nnnnnnnnnn nnnnnnnnnn nnnnnnnnnn nnnnnnnnnn 22500
nnnnnnnnnn nnnnnnnnnn nnnnnnnnnn nnnnnnnnnn nnnnnnnnnn nnnnnnnnnn 22560
nnnnnnnnnn nnnnnnnnnn nnnnnnnnnn nnnnnnnnnn nnnnnnnnnn nnnnnnnnnn 22620
nnnnnnnnnn nnnnnnnnnn nnnnnnnnnn nnnnnnnnnn nnnnnnnnnn nnnnnnnnnn 22680
nnnnnnnnnn nnnnnnnnnn nnnnnnnnnn nnnnnnnnnn nnnnnnnnnn nnnnnnnnnn 22740
nnnnnnnnnn nnnnnnnnnn nnnnnnnnnn nnnnnnnnnn nnnnnnnnnn nnnnnnnnnn 22800
nnnnnnnnnn nnnnnnnnnn nnnnnnnnnn nnnnnnnnnn nnnnnnnnnn nnnnnnnnnn 22860
nnnnnnnnnn nnnnnnnnnn nnnnnnnnnn nnnnnnnnnn nnnnnnnnnn nnnnnnnnnn 22920
```

nnnnnnnnnn nnnnnnnnnn nnnnnnnnnn nnnnnnnnnn nnnnnnnnnn nnnnnnnnnn 22980
nnnnnnnnnn nnnnnnnnnn nnnnnnnnnn nnnnnnnnnn nnnnnnnnnn nnnnnnnnnn 23040
nnnnnnnnnn nnnnnnnnnn nnnnnnnnnn nnnnnnnnnn nnnnnnnnnn nnnnnnnnnn 23100
nnnnnnnnnn nnnnnnnnnn nnnnnnnnnn nnnnnnnnnn nnnnnnnnnn nnnnnnnnnn 23160
nnnnnnnnnn nnnnnnnnnn nnnnnnnnnn nnnnnnnnnn nnnnnnnnnn nnnnnnnnnn 23220
nnnnncttgg attacaaaca tgtgccacca agtctggctg cccattatgc atttctgtgg 23280
caggcgttga tggctattaa acccaagcct tgtatatact aggcaagtac ccatccactg 23340
aactacagcc ctagcatgga cctcacaggg caatggcctg acatctggga aacactatca 23400
cagagtataa taataaaacc gtagcatgct tttcagattg agcatttctg ctgggtcaca 23460
cttggaaaac cttcttattg ttatcatgag tatgtgtgca tacatgtgga ggtgagagga 23520
tgacgtcata gagacagctc tgtctcccat tctttattgc caatattatt ttatgttttg 23580
tctgcgtgta tgtttgtata ccaaagtgca tgcagtgcct gtagagacca aaagggggca 23640
tccctgagac tggagttaga agtggctgtg aaccactcac tctgtgggtg ctgggaatca 23700
aaccccggtt ctctggcaga gcaactggtg ctcttaacca ccgaatcaac ttctccagcc 23760
ctgcattgct ttaatctttt ggtcgattcc cccaatccaa ctcataccgc caagtctgag 23820
tagcaagtgc ctttatctgg gagactcact gatcttattt ttttttcctt tctttttctt 23880
tctttttttc tttttttttt tgagtcaggt atgattattt agatggcctc gaactcacag 23940
agatccacct ccctctgtta tgccaggctt ttaaaaaaca acatttattt ttgaatgcat 24000
atatagggct gtgcaaacac agcctgaagg agtcgtttct gattgctcac aacgtggttc 24060
cagggatgga acgcgggtga ccagcttgaa ggtaggtgct ttcaccagcc ccaagaattt 24120
atttttttaac tttatttttt taattaaatg attaaatgac acatgttcat tgtgggtagt 24180
gcaggtatgc gatggagcac atgtagaggt gacattactg caggcggtct ctctaccatg 24240
gggattcaac tcagaccact gcttccttta ccagatcatc attcttgcat cccccaatca 24300
tttcttttttg ttgatggttt tgtttgtttg ttttagtttt ggggggtttt taaagttttt 24360
ttcgagacag nnnnnnnnnn nnnnnnnnnn nnnnnnnnnn nnnnnnnnnn nnnnnnnnnn 24420
nnnnnnnnnn nnnnnnnnnn nnnnnnnnnn nnnnnnnnnn nnnnnnnnnn nnnnnnnnnn 24480
nnnnnnnnnn nnnnnnnnnn nnnnnnnnnn nnnnnnnnnn nnnnnnnnnn nnnnnnnnnn 24540
nnnnnnnnnn nnnnnnnnnn nnnnnnnnnn nnnnnnnnnn nnnnnnnnnn nnnnnnnnnn 24600
nnnnnnnnnn nnnnnnnnnn nnnnnnnnnn nnnnnnnnnn nnnnnnnnnn nnnnnnnnnn 24660
nnnnnnnnnn nnnnnnnnnn nnnnnnnnnn nnnnnnnnnn nnnnnnnnnn nnnnnnnnnn 24720
nnnnnnnnnn nnnnnnnnnn nnnnnnnnnn nnnnnnnnnn nnnnnnnnnn nnnnnnnnnn 24780
nnnnnnnnnn nnnnnnnnnn attaaaggcg tgtactacca ctgcctgggc ataaccggcc 24840
tttctgcctt atttgtttcg aaacggagtc tcatgtagcc tgaggtggcc tcaaacatgc 24900
aatgtagctg cagctgggat tacatatgtt caccaccaca acctgtttta tgcactgcta 24960
agagattaaa cctgggcctt cgtgctaggt aaggactcta ttaacagagc tacaacctct 25020
gcctggtttt tagtttttca gacagggctt tgcgacatag ccctaactgg ccttgaacat 25080
gcaatgatcc tcctgcctcg gcctcctggg tcctcagatt gttgatgcta cacccacata 25140
tctcagacct gagggttggc agcttgggcc atgctctgat ttgggagtga catattaaca 25200
gtagtttaat acgaatttga atccacctta ctttttttggt ggcactgaca ttgaactcat 25260
ggccttccac aggcttttac gtgagaaatg ttttacctcc cttccttgag ccatgagcga 25320
gcccctcact ggtggagtct atgcagctgg tctgaaactc ataccatctt cctgccccac 25380
tctccgagtg ctgagtgtct acagagctcc tgactctgcc tgggacgtga tgtctgtggg 25440
accttggcta tagaagggag gcagtcacac cacccatttt tggcttccat gtcggcaatg 25500
gagagcttgc catactcacc tgatgttgtc tgtacagaag ggggacggta aacaaagcaa 25560
ccactcctac agatgcagag agagagatgg gggatggggg gtgtcaggtt ctcctggcaa 25620
agggtcagct ccgttgaggg cttttccact cccgaccaaa ttcccaaaat aacgactctg 25680
agactcaata tttcatgaac aaatgcttgg gctagcttgg gcttgttccc tgacaagccc 25740
ctatctcatt taacccgttt attctattct atgaatgcca catggctggt tacctcgtct 25800
cagtttcatg agaccctctc atcagcacta gggcaaaccc tgattctatt actctcttcc 25860
tgcaggagct cgggccccct atttcctccc taagctaata gcccaacagc ttttgattga 25920
caggtgatgc ttccacacag tgcacacgaa gattctcttt accctccatg tgnnnnnnnn 25980
nnnnnnnnnn nnnnnnnnnn nnnnnnnnnn nnnnnnnnnn nnnnnnnnnn nnnnnnnnnn 26040
nnnnnnnnnn nnnnnnnnnn nnnnnnnnnn nnnnnnnnnn nnnnnnnnnn nnnnnnnnnn 26100
nnnnnnnnnn nnnnnnnnnn nnnnnnnnnn nnnnnnnnnn nnnnnnnnnn nnnnnnnnnn 26160
nnnnnnnnnn nnnnnnnnnn nnnnnnnnnn nnnnnnnnnn nnnnnnnnnn nnnnnnnnnn 26220
nnnnnnnnnn nnnnnnnnnn nnnnnnnnnn nnnnnnnnnn nnnnnnnnnn nnattttttgt 26280
agagagggct gaggtcaagt ttaagattag gaaagagaat caagaattat caaggataaa 26340
gggagaggtc aggatttaca ggggagcagg gttcatggtc agaatgaaaa tggatgagag 26400
agggcaatgc gtgagctgag gtcaagggtc acaatggacg gtccgtcggc gggggctggg 26460
cagggccaag cattctcact ggaaagggtt ggtgccgtcg ccccggtgca cggcctgcag 26520
cactttgcgg taaaccctga gagagatggt ggcgcagaga cccaacaggg ccaggtgcgc 26580
agccacggac acgatgctaa agtgcaggag gcagaggaga gaggccatga ggcccgtgaa 26640
gaccgctccc gacgtcctgg tgtccttcca gtacaacagg tctgccactg tggaaaagaa 26700
gacaatgttg ggacccgacc tgaccaagct cactcccttt ctgtttgttt tttagagata 26760
aggtctcact atgaagctaa ggctgacttc aaactatgaa accctggctg gtcttgaact 26820
cctgattatt ctaattcatc ctttcttttt cttagactga gttaagtttg accggcttca 26880
aactcaagac aaatactcct gcctctaccc tttagtgctt caaggaggtg ctaaggatca 26940
aatttagggc ttcatgcatg ctaggtaaaa cccaactgag ctacttccct gac 26993

<210> 8
<211> 4145
<212> DNA
<213> Mus musculus

<220>
<221> misc_feature
<222> (1)...(4145)
<223> n = A,T,C or G

<400> 8
```
ataaattctt attttgacac tcaccaaaat agtcacctgg aaaacccgct ttttgtgaca 60
aagtacagaa ggcttggtca catttaaatc actgagaact agagagaaat actatcgcaa 120
actgtaatag acattacatc cataaaagtt tccccagtcc ttattgtaat attgcacagt 180
gcaattgcta catggcaaac tagtgtagca tagaagtcaa agcaaaaaca aaccaaagaa 240
aggagccaca agagtaaaac tgttcaacag ttaatagttc aaactaagcc attgaatcta 300
tcattgggat cgttaaaatg aatcttccta caccttgcag tgtatgattt aacttttaca 360
gaacacaagc caagtttaaa atcagcagta gagatattaa aatgaaaagg tttgctaata 420
gagtaacatt aaataccctg aaggaaaaaa aacctaaata tcaaaataac tgattaaaat 480
tcacttgcaa attagcacac gaatatgcaa cttggaaatc atgcagtgtt ttatttaaga 540
aaacataaaa caaaactatt aaaatagttt tagagggggt aaaatccagg tcctctgcca 600
ggatgctaaa attagacttc aggggaattt tgaagtcttc aattttgaaa cctattaaaa 660
agcccatgat tacagttaat taagagcagt gcacgcaaca gtgacacgcc tttagagagc 720
attactgtgt atgaacatgt tggctgctac cagccacagt caatttaaca aggctgctca 780
gtcatgaact taatacagag agagcacgcc taggcagcaa gcacagcttg ctgggccact 840
ttcctccctg tcgtgacaca atcaatccgt gtacttggtg tatctgaagc gcacgctgca 900
ccgcggcact gcccggcggg tttctgggcg gggagcgatc cccgcgtcgc cccccgtgaa 960
accgacagag cctggacttt caggaggtac agcggcggtc tgaaggggat ctgggatctt 1020
gcagagggaa cttgcatcga aacttgggca gttctccgaa ccggagacta agcttccccg 1080
agcagcgcac tttggagacg tgtccggtct actccggact cgcatctcat tccactcggc 1140
catagccttg gcttcccggc gacctcagcg tggtcacagg ggcccccctg tgcccaggga 1200
aatgtttcaa gcttttcccg gagactacga ctccggctcc cggtgtagct catcaccctc 1260
cgccgagtct cagtacctgt cttcggtgga ctccttcggc agtccaccca ccgccgccgc 1320
ctcccaggag tgcgccggtc tcggggaaat gcccggctcc ttcgtgccaa cggtcaccgc 1380
aatcacaacc agccaggatc ttcagtggct cgtgcaaccc accctcatct cttccatggc 1440
ccagtcccag gggcagccac tggcctccca gcctccagct gttgaccctt atgacatgcc 1500
aggaaccagc tactcaaccc caggcctgag tgcctacagc actggcgggg caagcggaag 1560
tggtgggcct tcaaccagca caaccaccag tggacctgtg tctgcccgtc cagccagagc 1620
caggcctaga agaccccgag aagagacact taccccagaa gaagaagaaa agcgaagggt 1680
tcgcagagag cggaacaagc tggctgcagc taagtgcagg aaccgtcgga gggagctgac 1740
agatcgactt caggcggaaa ctgatcagct tgaagaggaa aaggcagagc tggagtcgga 1800
gatcgccgag ctgcaaaaag agaaggaacg cctggagttt gtcctggtgg cccacaaacc 1860
gggctgcaag atcccctacg aagaggggcc ggggccaggc ccgctggccg aggtgagaga 1920
tttgccaggg tcaacatccg ctaaggaaga cggcttcggc tggctgctgc cgccccctcc 1980
accacccccc ctgcccttcc agagcagccg agacgcaccc cccaacctga cggcttctct 2040
ctttacacac agtgaagttc aagtcctcgg cgaccccttc cccgttgtta gcccttcgta 2100
cacttcctcg tttgtcctca cctgcccgga ggtctccgcg ttcgccggcg cccaacgcac 2160
cagcggcagc gagcagccgt ccgacccgct gaactcgccc tcccttcttg ctctgtaaac 2220
tctttagaca aacaaaacaa acaaacccgc aaggaacaag gaggaggaag atgaggagga 2280
gaggggagga agcagtccgg gggtgtgtgt gtggaccctt tgactcttct gtctgaccac 2340
ctgccgcctc tgccatcgga catgacggaa ggacctcctt tgtgttttgt gctccgtctc 2400
tggttttctg tgccccggcg agaccggaga gctggtgact ttggggacag gggtggggc 2460
ggggatggac acccctcctg catatctttg tcctgttact tcaacccaac ttctggggat 2520
agatggctgg ctgggtgggt agggtggggt gcaacgccca cctttggcgt cttgcgtgag 2580
gctggagggg aaagggtgct gagtgtgggg tgcagggtgg gttgaggtcg agctggcatg 2640
cacctccaga gagacccaac gaggaaatga cagcaccgtc ctgtccttct tttcccccac 2700
ccacccatcc accctcaagg gtgcagggtg accaagatag ctctgttttg ctccctcggg 2760
ccttagctga ttaacttaac atttccaaga ggttacaacc tcctcctgga cgaattgagc 2820
ccccgactga gggaagtcga tgcccccttt gggagtctgc taaccccact tcccgctgat 2880
tccaaaatgt gaacccctat ctgactgctc agtctttccc tcctgggaaa actggctcag 2940
gttggatttt tttcctcgtc tgctacagag ccccctccca actcaggccc gctcccaccc 3000
ctgtgcagta ttatgctatg tccctctcac cctcaccccc accccaggcg cccttggccg 3060
tcctcgttgg gccttactgg ttttgggcag cagggggcgc tgcgacgccc atcttgctgg 3120
agcgctttat actgtgaatg agtggtcgga ttgctgggtg cgccggatgg gattgacccc 3180
cagccctcca aaactttccc tgggcctccc cttcttccac ttgcttcctc cctccccttg 3240
```

acagggagtt agactcgaaa ggatgaccac gacgcatccc ggtggccttc ttgctcaggc 3300
cccagacttt ttctctttaa gtccttcgcc ttccccagcc taggacgcca acttctcccc 3360
accctgggag ccccgcatcc tctcacagag gtcgaggcaa ttttcagaga agttttcagg 3420
gctgaggctt tggctcccct atcctcgata tttgaatccc caaatatttt tggactagca 3480
tacttaagag gggctgagt tcccactatc ccactccatc caattccttc agtcccaaag 3540
acgagttctg tcccttccct ccagctttca cctcgtgaga atcccacgag tcagatttct 3600
attttttaat attggggaga tgggccctac cgcccgtccc ccgtgctgca tggaacattc 3660
catacccctgt cctgggccct aggttccaaa cctaatccca aaccccaccc ccagctattt 3720
atccctttcc tggttcccaa aaagcactta tatctattat gtataaataa atatattata 3780
tatgagtgtg cgtgtgtgtg cgtgtgcgtg cgtgcgtgcg tgcgtgcgag cttccttgtt 3840
ttcaagtgtg ctgtggagtt caaaatcgct tctggggatt tgagtcagac tttctggctg 3900
tcccttttg tcaccttttt gttgttgtct cggctcctct ggctgttgga gacagtcccg 3960
gcctctccct ttatcctttc tcaagtctgt ctcgctcaga ccacttccaa catgtctcca 4020
ctctcaatga ctctgatctc cggtntgtct gttaattctg gatttgtcgg ggacatgcaa 4080
ttttacttct gtaagtaagt gtgactgggt ggtagatttt ttacaatcta tatcgttgag 4140
aattc 4145

<210> 9
<211> 1017
<212> DNA
<213> Mus musculus

<400> 9
atgtttcaag cttttcccgg agactacgac tccggctccc ggtgtagctc atcaccctcc 60
gccgagtctc agtacctgtc ttcggtggac tccttcggca gtccacccac cgccgccgcc 120
tcccaggagt gcgccggtct cggggaaatg cccggctcct tcgtgccaac ggtcaccgca 180
atcacaacca gccaggatct tcagtggctc gtgcaaccca ccctcatctc ttccatggcc 240
cagtcccagg ggcagccact ggcctcccag cctccagctg ttgaccctta tgacatgcca 300
ggaaccagct actcaacccc aggcctgagt gcctacagca ctggcggggc aagcggaagt 360
ggtgggcctt caaccagcac aaccaccagt ggacctgtgt ctgcccgtcc agccagagcc 420
aggcctagaa gacccccgaga agagacactt accccagaag aagaagaaaa gcgaagggtt 480
cgcagagagc ggaacaagct ggctgcagct aagtgcagga accgtcggag ggagctgaca 540
gatcgacttc aggcggaaac tgatcagctt gaagaggaaa aggcagagct ggagtcggag 600
atcgccgagc tgcaaaaaga gaaggaacgc ctggagtttg tcctggtggc ccacaaaccg 660
ggctgcaaga tcccctacga agaggggccg gggccaggcc cgctggccga ggtgagagat 720
ttgccagggt caacatccgc taaggaagac ggcttcggct ggctgctgcc gccccctcca 780
ccacccccccc tgcccttcca gagcagccga gacgcacccc ccaacctgac ggcttctctc 840
tttacacaca gtgaagttca agtcctcggc gaccccttcc ccgttgttag cccttcgtac 900
acttcctcgt ttgtcctcac ctgcccggag gtctccgcgt tcgccggcgc ccaacgcacc 960
agcggcagcg agcagccgtc cgacccgctg aactcgccct cccttcttgc tctgtaa 1017

<210> 10
<211> 27184
<212> DNA
<213> Homo sapiens

<400> 10
cactaagcac tctcaccacc caatgcctgg agtggttgta gtcagtgagt gacacattgc 60
acagtgtgtg ccccctggat tgggggtggg tgagagacag cccccacagt gagtggcacc 120
cctggccagg gcctgggaca agtctgtatc caagggtggc tctctgctta ggtctgtgtt 180
tgtacctggg tgtgtctgtg tctgccctac tctgtgcata ctcatatatg tgaacccgtg 240
tgtgtgtgtg tagttgtgag tgtgtgtgga agaggctgca tggcagtgga agctaggtgt 300
gtgattcgtg tatctgtgtg cctagagtgc cttgttctat agatttggat gccactccaa 360
gcaagtcagt gggtcttttt gtttttttgtt ttttgagatg gaatctcact ctgttgccca 420
ggctggagtg cagtggcacg atcttggctc actgcaacct ctacctcctg ggttcaagtg 480
attctcctgc ctcggccccc cgagtagctg ggattacagg tgcccaccaa tacactcagc 540
taatttgttg tatttttagt aaagatgggg tttcaccatg ttggccaggc tggtctcaaa 600
ctcctgacct catgattctc ctgcctcggc ctcccaaagt gctgggatta caggcatgag 660
ccaccgcgcc cggccaagtc agtgggtctt taggagctgt ttcgtacggt gtgactgtga 720
gtgaaccttc gcacacgtgt ctgtacggat atctaagaaa ttcttcaagt aggccgggca 780
cagtggttca ggcctgtagc cttagcactt tgggaggccc aggtgagtgg atcgcttgag 840
ctcaggagtt cgagaacagc ctgggcaaca tagtgagact tcgtatctat aaaaaatatg 900
aaaattagcc aggcatgaca gtgggcacct gtagtcccag ctactacttg gggggctgag 960
gcaggaggat cccttgagcc tgggaggtgg aggctgcagt gagctgagat cgagccactg 1020
cactccagcc tgggcgacag agggagaccc tgtctcaaaa aaaaagaaaa gaaaagaaaa 1080
aaagaagaca ttcttcaagt ccacaactct gagggtatca ctgtgagagc aggggtccta 1140
gctctaccca tttgtgtgtg tgtggagatg tgtaagtcta tgtagacaaa agtgtgtgtc 1200
atttactgtg tttggggtgt gaacacctat gtgatgtgtt tgcacaactg cacgtgtttt 1260

gttctgtgtg tgtgatcgtg tgttcaaata agtcatcttg tcgccgggcg tggtcgctca 1320
tgcctataat cccagcactt tgggaggcct aggcaggagg accattgagc ccaggaggtc 1380
cagactgcag tgagccgaga ttgcgccact gcactccagc ctgggcgaca gcaagacctt 1440
gtctcaaaac aaaagcaaaa acaaaaataa acaaatagtc atcaggtgcc tgcacagaca 1500
aaggtgaaag gtgtctgcct gttgagatct gtggataggg tgtatatatg gacatctcag 1560
cctgtctacg tgtgtatctg tctctgtcct cacggcaaaa gagaggttgg ccgggtgtgt 1620
ggtggctcac gcctgtaatc ccagcacttt gggaggctga ggcgggcgga tcacctgagg 1680
tgaggagttc aaaaccagcc tggccaacat ggcgaaactc catctcttct aaaaatacaa 1740
aaaaattaac cgggcgtggt ggcacacgct tgtaatccca gctactcagg gaggctgagg 1800
cattagaatt gcttgaaccc aggaggtgga ggttgcagtg agccaagatt gcgccactgg 1860
actccagcct gggcaacaga acaagactcc gtctcaaaaa aaaaaaaaaa aaaaaaaaga 1920
ggctcagata tgtttctgtc tgagagtctg tcagagttta ggaattagta aatgaatgaa 1980
tggtgaagat ccatttactc aacaaacatt tatttattta tttatttatt gagacagagt 2040
ctcgctctgt cacccaggat ggagtgcagt ggcgcaatct cggctcactg aagcctctgc 2100
ctcctgggtt taagagagtc ttgtgcctca gccaccaagt agctgggatt acaggtgtgt 2160
gccactgcgc ccagctaatt tttgtatatt tagtagagat ggggtttcac catgttgccc 2220
aggctggtct cgaactcctg gcctgaagtg atctgctcac ctcagtctcc caatatgctg 2280
ggattacagg catgagccac cagtcctgcc tcatttattc ttatattata ttatattgtt 2340
tttattttaa attttttttgt agtgacaggg tcttactatg ttgactgggc tggcctcaaa 2400
ctggcctcaa gtgattctcc tgcctcagcc tcccaaagtg ctgggattac aggcatgagc 2460
cactgtgcct ggcttgaaca aatatttaat aaccacctat tgggtaccac gtgctatgct 2520
ggggacaagg cagtgaccgg gatggttttg gccgagcttt cacccagctc acaacccaat 2580
gagggagaca aacctatccc cagacaatga tgagcccaga ttggcagagt tggagggagg 2640
gaccccagga gaggggggcct tgactcagcc tggagatcag ggagggcttc ctggaggaga 2700
ggttatggga gttaagactt ggaggaaaag actatgagcc acaggaagcg aagggaagag 2760
tgacccaggc agagggaaaa gcacctgcaa aggcctgaag tccagggaga gaggccaggc 2820
atctggaaca caacggggag aggagagacg agactagggc accctcggag caggtcgttc 2880
agggcctcag gagccatggg gaggagtata atgcaggatg caggggtact gggagggtca 2940
agctaacagt gtccaatctg ggttactctg cttccctgaa actccaggga catctcctcc 3000
aggaagccct agggattgct cagtgtggag cggctcagcc acccagtcac actcactatt 3060
caatgctgag cactgaggag gcagccgtta ctaaatcagc ctggatttgc cctcccagag 3120
gtcacagtca caacagtcaa acacacacct gcacgtgcac actcatgaac gtgcccctcc 3180
cctcctctgg agcctcagaa acccacagac cttgttcctc ccaggggggtg tccctggggc 3240
caaagcttct gcactaccag gggcttgtt ggaaatgcaa aagatggctg ggcacagtgg 3300
ctcacacctg taatcccagc agtttgggaa gctgaggcag gcagatcatt tgaggtcagg 3360
agttcatttg aggttgggag ttcaagacca gcttggccaa catagcgaaa ctctgtctct 3420
actaaaaata caaaaaaatt agccgggcgt ggtagcacac gcctgtaatc ccagctactt 3480
gggaggctga ggcaggagaa tcttttgaac ccgagaggtg aaggctgcag tgagccaaga 3540
tggcaccact gcactccagc ctgggtgaca gagtgagtct taaaaaaata ataataaaat 3600
aggctgggcg cggtggctca cgcctgtaat cccagcactt tgggaggctg aggcaggcgg 3660
atcacctgag gtcaggagtt cgagaccagc ctgaccaaaa tagtgaaacc ccgtctctac 3720
taaaaataca aaaaaattta gctgggcgta gtggctaacg cctgtaatcc cagctacagg 3780
ctgaggcagg aaaattgctt gaacccaggg gtgcagaggt tgcagtgagc caagatggca 3840
ccattgcact ccagcctggg caacaacagt gaaactccgt ctcaaaaaac aaaatacaaa 3900
aattagccag gcatggtggc acacacctgt aatcccagct actcgggagg ctgaggcagg 3960
agaatcactt gaacccggga ggcagaggtt gcagtgagct gagatcgtgc cactgcactc 4020
aagcctgtgt aacaagagtg aaattccgtc tcaaaataaa ataaaataat aaaaataaaa 4080
agatgaccac agcagatctg ctggctctcc aggcaattcg tgatcaccac gaattgagaa 4140
gcgctgctct caggcttaac ccccctgtac ctcagtttcc ttttctgtac aaggagatca 4200
aaacagaatc catgtagagg actgacgtgg agtgaggctt aactgagata atgagtgtga 4260
aagtgctaag cacagcttcc taagtgcttg ttttcagcag ctcctcttcc tcttttctat 4320
tttttatttt ttattttttt tggggggggga acagggtctc gctctgttgc ccaggctgga 4380
gtatagtggc agagtcatag ctcactgcaa cctcaatctc tcagggctca agtgatgccc 4440
gtgcctcagc ctcctgagtg gctgggacta caggtgtgtg ccaccatgcc cagctaattt 4500
ttaaatttct tgtagaggtt gggggtgggt ctcactatgt tgcccaggct ggtcttgtac 4560
tcctggcctc tggcgatcct cttacctagg cctcccaaag tgctgggatt acaggcgtga 4620
gccaccacac ccagccctcc tcctcctctg tattattaaa tttcagagct ggaaagaaat 4680
ttcgaagacc gccatcactc aaccttcttg ctctacagat gggggaactg aggaacagag 4740
gagcagaggt ttggccagag gaaggtggga aggacgtggt gtggctggcc ttcacagact 4800
ctgccggggg ctgtttacag gatactctga ggaaagccag gatgggcact gatacccacc 4860
tccaccctgc gggaggcaga aggctaaggc tcccccagcc gtctccccca agcagagctg 4920
agcccttggg agccagggca gagatgcaac cctctccccc atgctcaggt cctcctcctc 4980
ctggaagctc tccttccaag aggagcctct tattttattt tttattatta ttatttttga 5040
gacaaggtct cactttgtca cccaggctgg aagtgccgtg gcatgatcat ggctcactga 5100
agccttgccc tccccaagct caagtgatcc tctcacctca gcctcccaaa tagctgagac 5160
tacaggcaca cgccaccacc cctggctagt ttttgtattt ttagtagaga tggggtcttg 5220
ccattgtcca ggctggtctt gaactcccgg gctcaagtga tctgcccacc ttggcctccc 5280
aaagtgctgg gattacaggc atgagacact gagcctggca aggaatgagc cttatggaga 5340

```
agaacttgat ttacccactc catgctctgc aggcaggtgt gtcatgtctg tgagtgcagg 5400
tgtgtgtgtg tgtgtgtatg tgtgtgtgtg tgtgtctcca gggccaattt ctcccttagg 5460
cccaggggca cagtgcctag ggcccatgat aaatttaacc gacccacgga tatgggtcag 5520
gagggtatac atgtataaag ttcaggaaga ctttgtttta gtaacaaaac gagcatgtac 5580
aatctgtcag gagccttatg atatcttccc accagtctca tgagtgggca ctacatcttg 5640
ttctcctttg tcgggtaggg aaactgaggc cccggatgtc ctagctcaag ttcaccaagc 5700
tacttggtga tagaagcgga acataagcct aggccaccta gcttctgggt ctgcgatcgt 5760
gtgaggataa atgcccagtg tgttcgtgaa catgagcatc cctgtgtgaa taaattgaca 5820
tacataaacc catttaataa atttcaaagc caggcgccgt ggcttactcc tgcaatctca 5880
gcgctctggg aggctgaggt gggaggatcg cttgaagcca ggagtttgag atcagcctgc 5940
aacaaagtga gaccctgact ctaaaaacat ttttttgaat aaaaaaatta ggctggacac 6000
agtggctcac gcctgtaatc ccagcacttt gggaggccaa ggtgggtgga tcacttgagg 6060
tcaggagttt gagaccagcc tggtcaacag ggtgaaaccc tttctctact aaaaatagaa 6120
aaattagcca ggcgtggtgg cacatgcctg taatcccacc tactggggag gctgaggccg 6180
gaaaattgct tgaatctggg aggtagaggt tgcagtgagc caagatcatg ccattgcact 6240
ccagcctggg cgaagaaacg agactctgtc tcaaaaaaaa aaaaaaaaaa ttagtcaagc 6300
atggtggtac gcacctgtag ttgttagtta cttgggaggc tgaagcagga ggattacttg 6360
aggccaggag ttcgaggtta cagtgaacta tgattgcatc actgcactct agcctggatg 6420
acagagcaag atcctatctc aaaataatac taatagttca aggaacagac tttgaagcct 6480
gacaccctgc atggtgttat tccagctttg ctacttactt gctgtgtgac tctgggtgaa 6540
taacttaacc tctctgggct tctgtttccc ttcctgtaaa atgatcattt gtacctcaca 6600
ggagtgttgt gagaattaaa taagttaata taagctcttg ggaagaatta gctcttgtta 6660
tggttgtgtg aagagcttta cacgagtccc tctgcaggat gcatggttgt tggtctgtgt 6720
gtgtgtatat atatatatgt gtgtgtgtat gtgtgtatct atgtgtatat gtgtatatgt 6780
gtgtgttgct ctcttgatct ctgagtgttg tgtgtgtgtc aatgtgtgag atcatatatg 6840
cacctttagt aaagatctag ggtctctgt gtgttttggg ggtctgtacc tgtccttggc 6900
tgtacacctg aaccctacgt cttcttgtgt gtgtagggat gtgtgtcatg tgtgttgata 6960
accatatgac agtgtgtgtt tccacatgcc agtgtgtgtc tgtgaagctg tttctatgtg 7020
gctgtgggtg attgcccacg cgtgacctgg tgagcggctg tgtggaagtc tgtgttgcct 7080
gtgtcgggtg tggagtctct ggccagccag ggtaggtctt tgcatgtttc ctttattcca 7140
tggaggagga gatgaggggc ttggatgaga cagaagaatg aggggtcact ccctgtctga 7200
tgctggggcc gagtcactgc ctactagtgg ctgctgtgtc atctcccagt ctctgtccct 7260
ccctcctggc tggtggcaga cagagggggc gtggaggaac agggcagggg gtgggcttga 7320
ggctggactt ttgtctggaa acttgaacct ccacctatgc ccccccacgtc cctctagtct 7380
tccacatctc cctacaccct ccctactatt ggggcaggga atcaggactc cctgaaaatt 7440
aagaaccatc gccttggctc tgccacaggc ttgctgtgta gcccggagac aggccctgcc 7500
cctctctcaa cctgtgaaat tcaatgtttt aaaagtgctt cttcctttgt gcccagggct 7560
gggccaggct gtgcactggg agggaagtgc tgggttctct gaggtttgtg aaatagctgg 7620
tagcaccctt cccagtgggt gcttaagagt tgggggctgt agcatctgaa atacccaagt 7680
cagtgcaggc atagtggctc atgcctgtaa tcccagcact ttgggagacc gaggcaggcg 7740
gatcacctga ggttgggagt tcgaaaccag cctggccaac atggtgaaac cccatctct 7800
attaaaaata caaaagttag gccgggcaca gtggcttacg cctgtaatcc cagcatttaa 7860
gaaggccaag gcgggaggat cacctgagtt tgggagtttg agagcagcct gaccaatatg 7920
gagaaactct gtctctacta aaaatacaaa aaaactagcc aggcatggtg gcacatgcct 7980
gtaatcccag ctactcggga ggctgaggca ggagaagcac ttcaacccgg gaggcggagg 8040
ttgcggtgag ccgagatcgt gccattgcac tccagcctgg gcaacaagag caaaactctg 8100
tctcaaaaaa caaaacagaa caaaacaaaa attagccagg tgtggtggcg cacacctgta 8160
atcccagcta ctcgggaggc tgaggcagga gaatcacttg aacccaggag atgtaggctg 8220
cagtgagccg agattgtggc actgcactcc agcctgggca acagagtgag actctgtcaa 8280
aaaaaaaaaa aaaaaacgtc agattcatga accccatttg ctaagaagat ttgctcaggt 8340
agtcacctgt accccctgtga gccaagattg caacagtaca tgctaagagc agtaagaagg 8400
agtttgtaaa gcttagaaag acctgacagg ccaggcgctg tggctcacgc ctgtaatctc 8460
gacacttcgg gaggccgagg caggcggatc accaggtcag gagttcaaga ccatcctgac 8520
caatatggtg aaaccccgtc tgtactaaac atataaaaat tagcttgacg tggtggcccg 8580
cgcctgtagt ccagcaactc gagaggctaa ggtgaggcgg aggttgcagt gagccgagat 8640
cgccactgca ctccagcctg gcggcagagc aagactccgt ctcaaaaaaa aaaaaaaaaa 8700
aaaaaaaaaa agacctgaca agtgaaagct actaatattg ccattatcat ttaaaaaaac 8760
cccagacaca ggtttttcag ggagtttcat ccaaccaggc aggtctcaga aatcagaaaa 8820
gaaatggaaa gggtaggaaa tctggagttt gacaattctg agtttgaatt tcttgtgatg 8880
ggatcttggg caagtcattt aacctccctg agtatcattt ttttctttta taaaatgaag 8940
atttttctct cttaaccttc cagagctgtt ttaaggatta caaatcttct actgaaaggc 9000
gtagcacagg agctgtgctt ggcaagtgcc aaatacaagg cattaattat tattattaga 9060
attaataata atatcccctc cctcttacac attctttgtc tccgggtgga ttaaaaggtg 9120
gaaggagagg ctaccaacac catcagaaga gaggcttctc tctaagtttc atttcccatc 9180
tcctccaaat ccgcatccct cccaaacgcc ggacctgtaa ggccagcagg gtccaagaca 9240
cacatccttt gcccagcggg gaagattaaa gctaaagctc agagagggaa aacatttcct 9300
aagctcgcac agcgaatcag gacagaaacc aggacgagcc tcggaatccc tccattacct 9360
ccactttcac ctgagcatca cagcccgctc gggactcagt ttccccacct acgtgaccac 9420
```

accacactaa tcagggtctc cttttggaga tctgctcttc ttctcgaatg ggggcgctgc 9480
accatcggta gaacagggta ggtgggggcg ccagaggtga aggggacctg caggctgggg 9540
tcttccccgc ccgggtcagc ggggtccctg cggggctagt ctaagcgcct attattacca 9600
gcccccgggg cggcgttgca ctgcgcaggc gcgggcgggg cgcgggcgcg cgcgcgagcg 9660
agcgagggat tccctctgac gtcattgcta ggataccaaa caaacactcc gccgcgccgg 9720
ccgagctcct tatatggcta attgcgtcac aggaactccg ggaaggcggg gccgggatcc 9780
cctcccgccg agtggcccgg aacgcaaccc ccgagacccc cagggccccg agggtcatgc 9840
aagtgaccag atcgagtcta gaacagacct cttgctggac agtgcgggac tcgatttggc 9900
ggggccggag atttggggaa gtttgtccag caaggggcgg gtgacgtaag caggggggcg 9960
ggtcccgggc atataaatac aggctggcgg gtctgtgctt cattcataag actcagagct 10020
acggccacgg cagggacacg cggaaccaag acttggaaac ttgattgttg tggttcttct 10080
tgggggttat gaaatttcat taatcttttt ttttccgggg agaaagtttt tggaaagatt 10140
cttccagata tttcttcatt ttcttttgga ggaccgactt actttttttg gtcttcttta 10200
ttactcccct ccccccgtgg gacccgccgg acgcgtggag gagaccgtag ctgaagctga 10260
ttctgtacag cgggacagcg ctttctgccc ctgggggagc aacccctccc tcgcccctgg 10320
gtcctacgga gcctgcactt tcaagaggta cagcggcatc ctgtgggggc ctgggcaccg 10380
caggaagact gcacagaaac tttgccattg ttggaacggg acgttgctcc ttccccgagc 10440
ttccccggac agcgtacttt gaggactcgc tcagctcacc ggggactccc acggctcacc 10500
ccggacttgc accttacttc cccaacccgg ccatagcctt ggcttcccgg cgacctcagc 10560
gtggtcacag ggccccccct gtgcccaggg aaatgtttca ggctttcccc ggagactacg 10620
actccggctc ccggtgcagc tcctcaccct ctgccgagtc tcaatatctg tcttcggtgg 10680
actccttcgg cagtccaccc accgccgccg cctcccaggt aagtttttga tagtaggggt 10740
gctgctttgt aggttttatt ttttaagtca agggtgaaaa gaataaaccc caaccccccac 10800
aaaaaggcgc atcagaaccc tagatctgag atggaaaagg ctcacagcgc actttgcaaa 10860
ctgcaaagag tcgggagatg tttgcaattg gttgcgtgcg tggagcgcaa ggagggaacg 10920
cggcagggag ggtaggcttt ggggcgaggt gggggtgggg tgggtaatgc gctgctcaat 10980
gcaacgtgta tgcggtagcg gggctgagaa ctttgagccg gccccgggac tgccccctgc 11040
tcgggtccca gacctgaagc tagcgcagtt aggcaggtgg gggaaatccc ggggaagctt 11100
ccagcagtct cttttccttt cctcctcctt cggagcgccc acttcggtgc cgggtcgccc 11160
tccacccatc gggaagaggg gcctcgaacc ctcagccgcg ctgcctccgc ctcctgcgcg 11220
gagacgtaac gggggacccg tgcgtaacgg ctgacgcgct ggaatcctcc gtctgacgcg 11280
gggcacgcac ggcgcgcggc gcccccttcg tccgccccgc ccctgacgtc ccgggagcgt 11340
tctattttgg aacgccgggg ccacgttgct aagggagggg gcagcccggc gtttcgattg 11400
gccgccgggg cgcacgcctt ggccaatcag ctttcccttc ctatttgtag ggtgcatttt 11460
ccttcccccc tctctgtccc cggaacccgt ggttccttgg cggctgggtc tcttttcggc 11520
gcctctagag gcagagggag gggatccctg tcgtgacaag agcgcctgtc tgcgacccaa 11580
tggatctgcg aggcccttgc ggggatctag tccctgggct ctcaggagaa gggggtgtct 11640
gcttgtgtgc tggcgtttct tggagagata cggtggctgt caccctcttc tccaggcaca 11700
cacagacaca ttcccactcc ctctgctcct catgcccggt tcctccggtg tgtcccaaga 11760
caggactaga accgcgaacc gaagggcaac cagccaggtg gtctccagga gctccgcccc 11820
ctctgggttc ccaggactct gattggtcgg cgagccagcc cttccctcac cacgcccccc 11880
gagagagtag ttaagccttc agagcagttc caggagtcca tttacgggag gggggagatg 11940
agcgctgctg aggcttgggg gctcaggtcc cgcaccattc cccctccgcg acaatctgag 12000
agagctccag tggttacttt tatctacctg tccgttcacc ctaaactgtc actcgtcagt 12060
ctcactctga gaagagacag taacttgaaa cgttgttcta aactcctagg cccgtccccc 12120
aaacaccctt ttgactggga cccccgcccc tgcatgggac ctcgcgcaga ggggggtgtg 12180
tatgtgtgtg agtgtagagg aaggcttggc ctaaggcctc tccttctccc tccccttgcc 12240
tctggggtgg gggtggggtg ttgtggctgt gtgtgtggct gtggctccgt cccgggggtt 12300
ctgtcacccg gctgtgtcca gcctcctctc caccccccat acctaagagt caccaacccg 12360
gggtgtgatt caccacccgc tggaaccgtg caacctttcc ccgaggaaga aggaggaggt 12420
agaagccagt tgagcagaaa tcctctcatt aaccactgcg tcacggtgta gtggaagggt 12480
gggtgttgtg gctttttccc tgtgacacac acatccacac tcgctcaccc tgtgctcact 12540
cacggggtcg gtgtgtgtta tgtgtgttgg gtgtgtgtgt gtcggtgtct ttgtttgtgt 12600
gtctacgcct gtgtgtgtat gtgtcacccc gtaggagtgc gccggtctcg gggaaatgcc 12660
cggttccttc gtgcccacgg tcaccgcgat cacaaccagc caggacctcc agtggcttgt 12720
gcaacccacc ctcatctctt ccatggccca gtcccagggg cagccactgg cctcccagcc 12780
cccggtcgtc gacccctacg acatgccggg aaccagctac tccacaccag gcatgagtgg 12840
ctacagcagt ggcggagcga gtggcagtgg tgggccttcc accagcggaa ctaccagtgg 12900
gcctgggcct gcccgcccag cccgagcccg gcctaggaga ccccgagagg agacggtgag 12960
taagggacat cagaacttgg cctgggtagg gggaagcaag agaggcagga agtttcttat 13020
gaatggaggg ggctccact aaggcctcag tgttacagaa accccaagat ccttgctaca 13080
cgagcgagga ccggaggctt gttttttggc tcttgggggt tctgaaagaa gtagaggttc 13140
gggatgggtg gaggagtgct gtatccccaa atctcatggc ctctatctcc ctgactcagc 13200
tcaccccaga ggaagaggag aagcgaaggg tgcgccggga acgaaataaa ctagcagcag 13260
ctaaatgcag gaaccggcgg agggagctga ccgaccgact ccaggcggtg aggacaggcc 13320
ctggggtggg agaggggatg ttgaggggag ctctctcccc attctctgcc ccctctccac 13380
ctgtaccctt atcctgggtt gagaactaga cgttccacac atggaactag gtactgctgt 13440
ggccagactg ggtagcccag ggcacaaaca cagaccccca tggacttaag tcaactcctg 13500

gtcccccccc catttcctga ccccacggac tactctccta gccttcattt catcccaagg 13560
ggccacatgg ggcccttgag gagaggggcg cccccatcat ttctcccatc tggtcttcag 13620
caagtaacaa cccattttgc ctcagtttct ccatctctgc aaaccctcat caaatctccc 13680
gggctctttc tacccttaac actctggaaa gcctgtgaaa tgaaattatt ccacctcctg 13740
ccctagccac ccacagctct cctggtgctg gtggcatccc ccaaaaccca ctcccttcct 13800
acgtcctccc ttggtctgag agttccctgc tgtatgcctg cagggtgagc tgttactcct 13860
tgagggaaca agggaattgt caactttcct tctctacttt ttctcttccc cgggaggtag 13920
agagggaggg gtaatagaag ggaacacatt aaaaacacat aacagtggct catgcctgtg 13980
atcccagcac tttgagaggc caaggcagga ggattgcttg agcccaggag tttgagacca 14040
gcctgggaaa catagggagg acttgtctct accaagaaaa aaaaaaatta gttgggcatg 14100
gtggtgcaca ccactgtggt cccagctact atggaggctt tagtgggagg atcgcttgag 14160
ccgaggaggt ccaggctgca gtgagccatg attgcactat tgcactccag cctgggggac 14220
agagcgagac tctggctcaa aagcaaaaca aaaccaacca cataacgatt gttcattcat 14280
tcaacaaacc tcctgcacac cagaaacttc cccataaacc tggccccttc tgtaccatcc 14340
tttggacaga tgggggaaac tgaggttcct ggaaggcaag acccttgccc caactcccat 14400
ggccaccagg actccatctc aggaggtgtt ttttctcagc ccggggctgc cttccagcag 14460
caagtccaag ggagccatga cccgagtttc ccggtcactg acatgctttt ttctccttcc 14520
tctctctctt ctgtgacctg gcctccctgg cctcaggaga cagatcagtt ggaggaagaa 14580
aaagcagagc tggagtcgga gatcgccgag ctccaaaagg agaaggaacg tctggagttt 14640
gtgctggtgg cccacaaacc gggctgcaag atcccctacg aagaggggcc cgggccgggc 14700
ccgctggcgg aggtgagaga tttgccgggc tcagcaccgg ctaaggaaga tggcttcagc 14760
tggctgctgc cgcccccgcc accaccgccc ctgcccttcc agaccagcca agacgcaccc 14820
cccaacctga cggcttctct ctttacacac agtgaagttc aagtcctcgg cgacccccttc 14880
cccgttgtta acccttcgta cacttcttcg tttgtcctca cctgcccgga ggtctccgcg 14940
ttcgccggcg cccaacgcac cagcggcagt gaccagcctt ccgatcccct gaactcgccc 15000
tccctcctcg ctctgtgaac tctttagaca cacaaaacaa acaaacacat gggggagaga 15060
gacttggaag aggaggagga ggaggagaag gaggagagag aggggaagag acaaagtggg 15120
tgtgtggcct ccctggctcc tccgtctgac cctctgcggc cactgcgcca ctgccatcgg 15180
acaggaggat tccttgtgtt ttgtcctgcc tcttgtttct gtgccccggc gaggccggag 15240
agctggtgac tttggggaca gggggtggga aggggatgga cacccccagc tgactgttgg 15300
ctctctgacg tcaacccaag ctctggggat gggtggggag gggggcgggt gacgcccacc 15360
ttcgggcagt cctgtgtgag gattaaggga cgggggtggg aggtaggctg tggggtgggc 15420
tggagtcctc tccagagagg ctcaacaagg aaaaatgcca ctccctaccc aatgtctccc 15480
acacccaccc ttttttttggg gtgcctaggt tggtttcccc tgcactcccg accttagctt 15540
attgatccca catttccatg gtgtgagatc ctctttactc tgggcagaag tgagcccccc 15600
ccttaaaggg aattcgatgc ccccctagaa taatctcatc cccccacccg acttcttttg 15660
aaatgtgaac gtccttcctt gactgtctag ccactccctc ccagaaaaac tggctctgat 15720
tggaatttct ggcctcctaa ggctccccac cccgaaatca gcccccagcc ttgtttctga 15780
tgacagtgtt atcccaagac cctgccccct gccagccgac cctcctggcc ttcctcgttg 15840
ggccgctctg atttcaggca gcaggggctg ctgtgatgcc gtcctgctgg agtgatttat 15900
actgtgaaat gagttggcca gattgtgggg tgcagctggg tggggcagca cacctctggg 15960
gggataatgt ccccactccc gaaagccttt cctcggtctc ccttccgtcc atccccccttc 16020
ttcctcccct caacagtgag ttagactcaa gggggtgaca gaaccgagaa gggggtgaca 16080
gtcctccatc cacgtggcct ctctctctct cctcaggacc ctcagccctg gccttttttct 16140
ttaaggtccc ccgaccaatc cccagcctag gacgccaact tctcccaccc cttggcccct 16200
cacatcctct ccaggaaggg agtgaggggc tgtgacattt ttccggagaa gatttcagag 16260
ctgaggcttt ggtaccccca aacccccaat atttttggac tggcagactc aaggggctgg 16320
aatctcatga ttccatgccc gagtccgccc atccctgacc atggttttgg ctctcccacc 16380
ccgccgttcc ctgcgcttca tctcatgagg atttctttat gaggcaaatt tatatttttt 16440
aatatcgggg ggtggaccac gccgccctcc atccgtgctg catgaaaaac attccacgtg 16500
ccccttgtcg cgcgtctccc atcctgatcc cagacccatt ccttagctat ttatcccttt 16560
cctggtttcc gaaaggcaat tatatctatt atgtataagt aaatatatta tatatggatg 16620
tgtgtgtgtg cgtgcgcgtg agtgtgtgag cgcttctgca gcctcggcct aggtcacgtt 16680
ggccctcaaa gcgagccgtt gaattggaaa ctgcttctag aaactctggc tcagcctgtc 16740
tcgggctgac ccttttctga tcgtctcggc ccctctgatt gttcccgatg gtctctctcc 16800
ctctgtcttt tctcctccgc ctgtgtccat ctgaccgttt tcacttgtct cctttctgac 16860
tgtccctgcc aatgctccag ctgtcgtctg actctgggtt cgttggggac atgagatttt 16920
attttttgtg agtgagactg agggatcgta gatttttaca atctgtatct ttgacaattc 16980
tgggtgcgag tgtgagagtg tgagcagggc ttgctcctgc caaccacaat tcaatgaatc 17040
cccgaccccc ctaccccatg ctgtacttgt ggttctcttt ttgtattttg catctgaccc 17100
cgggggggctg ggacagattg gcaatgggcc gtcccctctc cccttggttc tgcactgttg 17160
ccaataaaaa gctcttaaaa acgcattcgc caggctacag tgtttatttc ctcctaacac 17220
ccattgcctg gcttcctttt agtaagagag gatgaggtta gattgtcgaa ggactacaca 17280
cacacagaga tcatacaccc atgcacacac acacacagac acacacctat gcaggaactc 17340
cttctagtca atgggtctac agcaggataa aaggaaggca gacagtcagg aggacttcct 17400
gtactcgcag gcagtgcctc ctactagaaa aagtaagtgg ctacagctgg gcgcagtggc 17460
ttgcgcctgt aatcccagca ttttgggagg ccaagatggg cggatcactt gaggtcagga 17520
gtttgagacc agccttgcca agatggtgaa accctgtctc tacaaaaaat acaaaaatta 17580

gcctggtgtg gtggtgcaca cctgtaatcc caggctactt gagaggttga ggcaggagaa 17640
tcactttaac ttaggaggca gaggttgcag tgacacaaga ttgcaccact gcactccagc 17700
ctgggtgaca aagtgaaact gtcaaaaaaa aaaaaaaaag taaggggtca gaaggttagc 17760
ctgcaggtgt gggatcacag aggtctccta gtgatggagc ttgtatgctc tatgggttaa 17820
aaacagacgc taaggagata aactatacac agaagaagct taatgggctg tgcacagtgg 17880
cttgcacttg caatcccagc tctttcggag gccgaggtag gaggctagga gttcgagaac 17940
agcctggggc aacatagtga gacacccccc accccaaccc atctcattat gttgagaaaa 18000
aaaaaaaaaa gaggatcttg agaaacttgc acagcaaact actaaagacc actcaggcta 18060
ggaatgaggc gtcatcttgg attttttaca aactacaact acaaaaaaca attattttac 18120
actgaagatg tgtttccttt tgttttccat atgaccacta ttggggccaa cccaagactg 18180
actgaagcaa atatatgacc attttttttt tttacacata gagtggagcc atccccattg 18240
caaaatccta gccgtctctt atttgcataa cagcaagtcc tctgggaatt gaggtgaggg 18300
tccaggggtc gctcagacgc tttggtaaat caataagcaa gggaatttgt catggtggaa 18360
tccatttatt aattcactca aaaaaatgtg ctaagcacct acccaactgt gctgggagtt 18420
ggggacacta tagggactga gcctcaggtc ttgccctcct ggggctcaca gtccattggg 18480
ggacacacac cgttgccagt cagggacaac ctaaagtgga ttgggtttgg gaagcccatg 18540
gggctaggga agcacagcag ggacacctag cttggactgg gagtcgggaa aaacttcctg 18600
gcggagggga cagctgagct gagtcctaga ggaggataga gagtatagaa agaaatagaa 18660
aaaggccaag gccaagaaaa tagcacagcc atagatctgt tggtggggag aaggcttgat 18720
acattgagaa cgcttagagg gtcgggtgcg atggctcatg cctgtaatcc cagcactttg 18780
ggaggccgag gtgggtggat cacctgaggt caggcattca agaccagtct ggccaacgtg 18840
gcgaaacccc gtctctacta aaaaatatat atatatatat acaaaaaatt agctaggcat 18900
ggtggtgggc acctgtgatc ccagctactc gggaggctga ggcaggagaa tcgcttgaac 18960
ctggaaagtg gacattgcag tgagctgaga ttgtgccact gcactccagc ctgggcaaca 19020
cagcgagact ctgtctcaaa aaaaaaaaaa aaagaaagaa aaaaaagaga aaactcagag 19080
attcgtggag actggaacca cgggtgtgga gagaggggtt agtagagacc agattctgca 19140
ggtactataa tgacattccc aggctaagga gtttagatct tttcttcagg gcactgggga 19200
gctattgcag gttttgaaca cgagaggggc aggggcaggt ttgtgattta ggaaagaccc 19260
ctctggcccc agactgagcg tgactggaag ggagaggagt gagactgagc aggagaccag 19320
ggaggaagtg gggtggggac cctgcagggt ggggtgagga ggagtccaga acagggccag 19380
ggacatggga cagagaggag gggaaggatt caggagacac tcgggaggca ggacagttgg 19440
gcttggtgag cagccggctt gggaaggaaa atacagggca gtaataaata gcatgggtca 19500
agtggtctga gtgaggctgg gagtggtggc acgcacctat ggtcccagct actcaggagg 19560
ctgaggtggg aggattgctt gagcctggaa gtttgaggct gcagtgagct atgatcgtac 19620
cgctgcactc cactttcggt gacagagtaa gaccctgtct caaaaaaaaa aaaaaaaagg 19680
aaaaaaaaaa aaagacactg cctatgagtt aaccctgctc tacaaggagc agtttttaaa 19740
gtaaaattaa aaaaaaaaaa aaaaaagaaa gaaagaaaga aagaaagaga gagaaaaaag 19800
aaaaaaggct gggttgcctt ggacaacaga cttcatctcc ctgagcctcc atttcctcat 19860
ctgtagaatg ggggctgtta agaggagttg caaggcttgt gcatgccagc agtaagtgca 19920
gagtgacggt gcaattatca ttacccccat catctttatt ggggtcagcc tgaaccctcg 19980
atatcccata atattcaccc ccatccttca agggtctgcc ctaatgttcc catgacaccc 20040
gaccagctca gctctcctta tgagagggcc tcacttttct attccctttg cagccgttat 20100
ccccttttgta gttgttaatt aagtgtgtaa ttacatgatg gcttaatgtt tgtctccccc 20160
actgggctga ctgcaccagg agaccaaggc cagggctgtc acccaccgct gcgtcctcag 20220
caaatgcttg tttgatgagt gaatgacaga tgaacaaatg ggcaagtatt tgactgatta 20280
atcacggcat gcgtgattaa ataaatgagt gagcaaacga ctgaatgagt aagcaattga 20340
aggggagaga tctaggataa tttccaaact gccaatatcc cagaactggg tagatgactt 20400
tttctccgtc ttttggagtg ggtttttata ctccggaggg ggaaataagt aacgacaatc 20460
agagatcgga gtgacagaga atgagtttgc gatgcggaag gccgcagcga cctctccagg 20520
tgcaggctgg aggagcgcct cccgggcaac aagcaaaata agtccggttc gataagtaag 20580
attcaaggcg cttagttact accgcccgaa aggtggaatt gaaccactct gtcgctagac 20640
agctacaggt ttgaagcctg caccccagac cactgaggat catccgggca ggacaacctt 20700
cttcccgcgc agctatataa ggatcgcaaa agcttacctt ttagagttat ggtcgcgtcc 20760
tttggaaagg tgtgagatgg ttgacttcaa gggctataag ttctccccac tcatttatcg 20820
tgagagatgt ccttgtaaaa cgttgccttc acttcatata gtatgaatca tcctcgctcc 20880
ccaaaccgtc tcatttacat agcgccacgc attctgggaa gcagcgtttg agcacctggc 20940
aagctgttaa agggcccagg accccctttt tccaaaatga aaagtcagct ttttaagagg 21000
agagattcat gattcggcat tcagaagcgc cttctcggaa aattccgaat tctctttctg 21060
cggaggagtg gtgagtgggt tcctgttaga gtctgattca ccctggcccc aaaggcgctg 21120
agcaggcggc tatagggctg gtgcgacacc tgctgttctt ttatggaatt gcgtaggccg 21180
atggagctcc gcgttcattc aaacgtttat tgagcgtcta ctgagtccca ggaactgtct 21240
cggtgctggt gacgacagac atcctgtccc gattgagttt aaacactaga cataaccaat 21300
ttctgcactt gcgttttatg gttggagaaa ctgagacaga ctcagaaacg gatagtacca 21360
tattccggtt aaaacagccc atccacttag ggaaatcgaa cttgtgttac aatttttatt 21420
acaaaaaagg tgattctttt ttcttaaatt tttacatatt taggaggtac aggtgccgat 21480
ttctacatgc atatgttgat tgcatcgtgg tgaaatctgg gcttttaggt gtactcatca 21540
cgaaaaatta ttcaatattc taagtcagtg attctttgac aataccattc tcctgcatga 21600
aactctccca tgacttccat ttcagagtaa aagtcaaagt actcaccctg ctttacaaat 21660

ccctgtaatc cagcccttt ggtccctctg accctgtctc acatactcct cttcccttgg 21720
atattattgg atttaggcac acagatgtct tgctgcacac atggatcatg ctaccaccac 21780
agggactttg tgcttgccga tcccctagcc tggatctcct cttcacatgt tcccatggct 21840
catcgcctcc cttcactctg tcatctgatc aaatgtcaat accaaaaact cactgagtga 21900
ccagcactca gtggggcctt ccctgatccc agtttaaaat agcgagtggc ggccgggcgc 21960
ggtagctcac gcctgtaacc ccagcacttt gggatgccaa agcgggtgga tcacgagatc 22020
aggagatgga gaccatcctg gctaacacgg tgaaaccttg tctctactaa aaatacaaaa 22080
cattagctgg gcgtggtggc gggcgcctgt agtcccagct actcgggagc ctgaggcagg 22140
agaatggagt gaacccggga ggcggagctt gcagtgagct gagattgctc cactgcactc 22200
cagcctgggg gacagagcga ggctccgtct caataaataa taaataaata aaatagctag 22260
tggctgggat cggtggctca gggcctgtaa tcccagaact ttgggaggcc gaggcgggtg 22320
gatcacctga ggtcaggagt tcgaggccag cctggccaac atggtgaaac cccgtctcca 22380
cgaatgatac aaaaattagt caggcatagt ggctcacgcc tgtaatccca gctactcggg 22440
agtttgaggc aggagaaccg cttgaaccgg ggaggcagag gttgcagtga gctgagatgg 22500
tgccattgta ctccagcctt ggtgaaaaga gcaaaactcc atctccaaat aaaagtaata 22560
ataataataa ataaaataaa aattttttaaa aagcgagctc tgtcactcta tccccttacc 22620
ctgctagaca tttccccacc actctgatca ccacctgcca ttttctgtgt ctatttgctt 22680
accgtctgtc tgcttctgta gaatatcagc accatgaaac tatgcacttt atttttgatc 22740
attgctgtat ctctagtgcc taaaaagtgc ttgagaacat agcagatgtt cagtaaatgt 22800
ttgtggaatg aataaaagaa tatcatcact gtcttttttt cattcttctt ccagacagga 22860
tcttactttg tcacccaggc tggaatgcag tggcgcaaaa acggctcact gcagcctcga 22920
cctcccaagc tcaagtgatc ctcttgcctc agcctccctg ggatgacagg catataccat 22980
cacgcccagc taattttaat tttttttgta gaaacagggg cctcactttg ttgcccagac 23040
tggcctcgaa ctcctggcct caagtgatgc ttctgccttg ggctcctaaa gtgctggaat 23100
ttcacgcgtg agccacagca cctggcctca ctatccttct ttcagcctca gttttctcat 23160
ttgtataacc agactagtac aactgatctc actggagaaa tcatgatata aaattctgac 23220
actggctgag gcaactggga ggagctcagt aaaggctgtt tctgctgggc acggtggctc 23280
acacatgtaa tcccagcact ttgggaggcc gaggtgggtg gatcacagga gattagaagt 23340
tccagaccat ctggcaagca tggtaaaacc ccatctctac taacaattca aaaagtagcc 23400
aggcatggtg gctcacacct gtgatcccag ctactcggga ggctaaggca ggagaatccc 23460
ttgaacccag gaggctgagg ttgcagtgag ccaagattgt gccactgcac tccatcctgg 23520
gcgacagagc aagactctgt caaaaaaaaa aaaaaagttt tttttttttg gctggaatta 23580
caggcgcctg cccccacacc tggctaattt ttgtttttttg tttttttagg agagacgggg 23640
tttcaccatg ttcaccagac tggtcttgaa ctcctgacct caggtaatcc aactgcctca 23700
gcttcccaaa gtgctgagat tacaggcggg agccactaca cctggccaat aaaggccgtt 23760
tcagtcttca atctgttttg agcttggagg ctttagtcat tcccagaccc aaaatctcaa 23820
tcagaccctc ttccaccact ttttgtgata gatcaataaa cattttgtct tatgggaagt 23880
ttaactaaga gtatctttag aaagttttgg acaggcgctg taatcccagc actttgggag 23940
gccgagatga gcggatagct tgagcccagg agttagagac aagcctgggc aacatagtga 24000
gactctgtct caaaaaaaaa aaaaagaaag aaaggaaaag aaaaaaagaa aaaaaaagta 24060
ttccctctgc ttggcaaatc cagattcaag atatatcccc taaaccctct ttgttttaat 24120
tagatagttg ctgctaggca cctctgtata caaattctga ggatgtaagg actccttgga 24180
acaccgttat ctgtctccta atatgtgacg tgtgtatgac gaacaagtga gttttttttt 24240
tttttgtttc ttttttttgg ttttttgttt tgagacgcag tctcgctctg tcgcccaggc 24300
tggagtgtag tggcgcgatc ttggctcact gcaagctctg cctcccaggt tcacgccatt 24360
ctcctgcctc agcctcccta gtagctggaa ctacaggcgc ccgccaccac tcccggctaa 24420
ttttttttgta tttttagtag agacggggtt tcaccgtgtt agccaggatg gtctcgatct 24480
cctgacctca tgatctgccc gcctcggcct cccaaagtgc tgggattaca ggcgtaagcc 24540
accgagcccg gctttttttt gagatggagt ttccctcttg ttgctcaggc tggagtgcag 24600
tggtgtgatc tcggctcact gcaacttccg cctcccgggt tcaagcgatt ttcctgcctc 24660
agcctcccaa gtagctggga ttacaggcat gcaccaccac gcccggccaa ttttgtattt 24720
ttagtagaga catggtttct ccatgttggt caggctggtc tccaactccc gacctcaggt 24780
gatccacctg ccttggcctc ctaaagtgct gagattacag gcgtgagcca ctgcgcctgg 24840
ctgcacaaac attttaaacg tcaatatttt tgtgtttatt tttactattg tcttctattt 24900
ctggcaagca atactggttt ctggtggcaa ggtaaccttt cttcttaaaa tacatttgtt 24960
attatattat taaatattaa accaattttc aagaaaaata ttagataaat agcagaacag 25020
gttgtagaaa gatgaaggtc atgtggggaa tgactgaggt ttgggaaaca atattataac 25080
tccttactgt gcatttatta tatgccaggc ccaatgccaa gggctttaca tgtaggtata 25140
gttatggttg acagttttct tctttttctt ttcttttttt ttgaaacagg gtcttgctct 25200
gtcgccctgt ctggagtgca gtgacgcaat ctcagctcac tacaacctcc acctccttag 25260
ttcaagtgat tctcctgcct cagcctcccg tgtagctggt cttacaggtg cccaccacca 25320
catccggtat ttttagtagg gacgaggttt taccatgttg gcgaggcttg tcttgaactc 25380
ctgacctcag gtgatccacc ttccttggcc tcccaaaggc tgggattaca ggtgtgagcc 25440
actgcacctg gcctcttttt ttcttactta attttttgt agagatgagg tctatgttgc 25500
ccaaacttgt ctcgaactcc tggccttaag tgatcctccc tccttggcct tccaaagtgc 25560
tgggattgca gacgtgagcc atcgtgcccg gcctaataag ttttccattt gaggaaactg 25620
aggctttgag agatgaagtc atatgctcaa ggtcatacag cgaaggggta gtagagccag 25680
gattcaaacc caagtctgtg gatctttaga cctcagtgtt ctcccagtct ccccacgggt 25740

cccttttgctt catcctttcc gagtttagtt gtcttttttg ttgttgttct tgttgttgct 25800
gttttttgat agagtctcgc cctgttgccc aggctggagt gcagcggcat gatcctggct 25860
cactgcaacc tctgcctcct gggttcaagt gattctcctg cctcaggctc ccgagtagct 25920
gggattacag gtatgcgccg ccacacccag ctaattttg tatttttagt agagatggag 25980
tttcatcatg ttggtcaggc tggtctggaa ctcctgacct cagtgatctg cccaccttgg 26040
cctcccaaag tgctgggatt acaggcgtga gccactacgc ccagtatgta gtttttttgt 26100
gttttgtttt tgtttttgtt tagagacagg gtctccctct gtaactcagg ctgaagtgca 26160
gtggtgtgat catagctctc tgtaacctcg aactcctggc ctcaagcaat tttcctgtct 26220
tagcctccca agtacctggg actacaggca tgcaccacca cgcccggata aattttttttt 26280
tttttagag acgggggtct tgttatgttg ctcagggtgg tctcgaactt ttggcctcaa 26340
gtgatcttcc cacattggcc tcccgaagtg ttgggatcac agatgtgagt catcatgcca 26400
ggcctcagat tagtgttgaa actggaagtc cagggaggcc ctgtatttgt tcccgaggca 26460
atcagggtgg ggagacgtgg gtggatgaga gaagtttgtg aggcaggata gacacagcat 26520
tgtgactgat tgtttgtggg aggtgagtgt gtagaagtcc agagccatac ctgggtgtct 26580
ggcctggtga caccgtgggc agtgaaaaca tctctgagtt ggaaatcagg taggatgaag 26640
aagacgtttg gttagattta gggtggaagg ggcatccaga ggccgttgga cacgtgggtt 26700
tgggctcagt tgaggtggct gcacagtagg cagggtgtgg gatccattag tgatgtctgc 26760
cttgagcaca ggcatgggaa gtggctgcca aggcttggct aaccctggaa tgggtggtct 26820
gtcagcatct tcccctcaat catttctttc tttttttttt ttttgagaca gaatctcgct 26880
ctgtcgccga ggctggagtg cagtggcgca atcttggctc actacaacct ccatctccca 26940
gattcaagcg actctcatgc ctcagcctcc cgagtagctg tgattacagg catgcaccac 27000
cacacccggc taaattttgt atttttagta gagatgaggt ttcaccatgt tggccaggct 27060
ggtcttgaac ttctggtctc aagtgatccg ccctcctcgg cctcccaaag tgctgggatt 27120
acaggcgtga gccactgcgc ccggcctccc ctcaatcatt tcataaccta cacagatggc 27180
agag 27184

<210> 11
<211> 3775
<212> DNA
<213> Homo sapiens

<400> 11
cattcataag actcagagct acggccacgg cagggacacg cggaaccaag acttggaaac 60
ttgattgttg tggttcttct tgggggttat gaaatttcat taatcttttt tttttccggg 120
gagaaagttt ttggaaagat tcttccagat atttcttcat tttcttttgg aggaccgact 180
tacttttttt ggtcttcttt attactcccc tcccccccgtg ggacccgccg gacgcgtgga 240
ggagaccgta gctgaagctg attctgtaca gcgggacagc gctttctgcc cctgggggag 300
caacccctcc ctcgcccctg ggtcctacgg agcctgcact ttcaagaggt acagcggcat 360
cctgtggggg cctgggcacc gcaggaagac tgcacagaaa ctttgccatt gttggaacgg 420
gacgttgctc cttccccgag cttccccgga cagcgtactt tgaggactcg ctcagctcac 480
cggggactcc cacggctcac cccggacttg caccttactt ccccaacccg gccatagcct 540
tggcttcccg gcgacctcag cgtggtcaca ggggcccccc tgtgcccagg gaaatgtttc 600
aggctttccc cggagactac gactccggct cccggtgcag ctcctcaccc tctgccgagt 660
ctcaatatct gtcttcggtg gactccttcg gcagtccacc caccgccgcg gcctcccagg 720
agtgcgccgg tctcggggaa atgcccggtt ccttcgtgcc cacggtcacc gcgatcacaa 780
ccagccagga cctccagtgg cttgtgcaac ccaccctcat ctcttccatg gcccagtccc 840
aggggcagcc actggcctcc cagcccccgg tcgtcgaccc ctacgacatg ccgggaacca 900
gctactccac accaggcatg agtggctaca gcagtggcgg agcgagtggc agtggtgggc 960
cttccaccag cggaactacc agtgggcctg ggcctgcccg cccagcccga gcccggccta 1020
ggagaccccg agaggagacg ctcaccccag aggaagagga gaagcgaagg gtgcgccggg 1080
aacgaaataa actagcagca gctaaatgca ggaaccggcg gagggagctg accgaccgac 1140
tccaggcgga gacagatcag ttggaggaag aaaaagcaga gctggagtcg gagatcgccg 1200
agctccaaaa ggagaaggaa cgtctggagt ttgtgctggt ggcccacaaa ccgggctgca 1260
agatccccta cgaagagggg cccgggccgg gcccgctggc ggaggtgaga gatttgccgg 1320
gctcagcacc ggctaaggaa gatggcttca gctggctgct gccgcccccg ccaccaccgc 1380
ccctgccctt ccagaccagc caagacgcac cccccaacct gacggcttct ctctttacac 1440
acagtgaagt tcaagtcctc ggcgacccct tccccgttgt taacccttcg tacacttctt 1500
cgtttgtcct cacctgcccg gaggtctccg cgttcgccgg cgcccaacgc accagcggca 1560
gtgaccagcc ttccgatccc ctgaactcgc cctccctcct cgctcggtga actctttaga 1620
cacacaaaac aaacaaacac atgggggaga gagacttgga agaggaggag gaggaggaga 1680
aggaggagag agaggggaag agacaaagtg ggtgtgtggc ctccctggct cctccgtctg 1740
accctctgcg gccactgcgc cactgccatc ggacaggagg attccttgtg ttttgtcctg 1800
cctcttgttt ctgtgccccg gcgaggccgg agagctggtg actttgggga cagggggtgg 1860
gaaggggatg gacaccccca gctgactgtt ggctctctga cgtcaaccca agctctgggg 1920
atgggtgggg agggggcgg gtgacgccca ccttcgggca gtcctgtgtg aggatgaagg 1980
gacgggggtg ggaggtaggc tgtggggtgg gctggagtcc tctccagaga ggctcaacaa 2040

```
ggaaaaatgc cactccctac ccaatgtctc ccacacccac cctttttttg gggtgcccag 2100
gttggtttcc cctgcactcc cgaccttagc ttattgatcc cacatttcca tggtgtgaga 2160
tcctctttac tctgggcaga agtgagcccc cccttaaagg gaattcgatg ccccccctaga 2220
ataatctcat ccccccaccc gacttctttt gaaatgtgaa cgtccttcct tgactgtcta 2280
gccactccct cccagaaaaa ctggctctga ttggaatttc tggcctccta aggctcccca 2340
ccccgaaatc agcccccagc cttgtttctg atgacagtgt tatcccaaga ccctgccccc 2400
tgccagccga ccctcctggc cttcctcgtt gggccgctct gatttcaggc agcaggggct 2460
gctgtgatgc cgtcctgctg gagtgattta tactgtgaaa tgagttggcc agattgtggg 2520
gtgcagctgg gtggggcagc acacctctgg ggggataatg tcccccactcc cgaaagcctt 2580
tcctcggtct cccttccgtc catccccctt cttcctcccc tcaacagtga gttagactca 2640
agggggtgac agaaccgaga agggggtgac agtcctccat ccacgtggcc tctctctctc 2700
tcctcaggac cctcagccct ggccttttttc tttaaggtcc cccgaccaat ccccagccta 2760
ggacgccaac ttctcccacc ccttggcccc tcacatcctc tccaggaagg cagtgagggg 2820
ctgtgacatt tttccggaga agatttcaga gctgaggctt tggtaccccc aaacccccaa 2880
tatttttgga ctggcagact caaggggctg gaatctcatg attccatgcc cgagtccgcc 2940
catccctgac catggttttg gctctcccac cccgccgttc cctgcgcttc atctcatgag 3000
gatttcttta tgaggcaaat ttatattttt taatatcggg gggtggacca cgccgccctc 3060
catccgtgct gcatgaaaaa cattccacgt gccccttgtc gcgcgtctcc catcctgatc 3120
ccagacccat tccttagcta tttatccctt tcctggtttc cgaaaggcaa ttatatctat 3180
tatgtataag taaatatatt atatatggat gtgtgtgtgt gcgtgcgcgt gagtgtgtga 3240
gcgcttctgc agcctcggcc taggtcacgt tggccctcaa agcgagccgt tgaattggaa 3300
actgcttcta gaaactctgg ctcagcctgt ctcgggctga cccttttctg atcgtctcgg 3360
cccctctgat tgttcccgat ggtctctctc cctctgtctt ttctcctccg cctgtgtcca 3420
tctgaccgtt ttcacttgtc tcctttctga ctgtccctgc caatgctcca gctgtcgtct 3480
gactctgggt tcgttgggga catgagattt tattttttgt gagtgagact gagggatcgt 3540
agatttttac aatctgtatc tttgacaatt ctgggtgcga gtgtgagagt gtgagcaggg 3600
cttgctcctg ccaaccacaa ttcaatgaat ccccgacccc cctaccccat gctgtacttg 3660
tggttctctt tttgtatttt gcatctgacc ccggggggct gggacagatt ggcaatgggc 3720
cgtcccctct cccccttggtt ctgcactgtt gccaataaaa agctcttaaa aacgc      3775
```

<210> 12
<211> 1017
<212> DNA
<213> Homo sapiens

<400> 12

```
atgtttcagg ctttccccgg agactacgac tccggctccc ggtgcagctc ctcaccctct 60
gccgagtctc aatatctgtc ttcggtggac tccttcggca gtccacccac cgccgcggcc 120
tcccaggagt gcgccggtct cggggaaatg cccggttcct tcgtgcccac ggtcaccgcg 180
atcacaacca gccaggacct ccagtggctt gtgcaaccca ccctcatctc ttccatggcc 240
cagtcccagg ggcagccact ggcctcccag cccccggtcg tcgaccccta cgacatgccg 300
ggaaccagct actccacacc aggcatgagt ggctacagca gtggcggagc gagtggcagt 360
ggtgggcctt ccaccagcgg aactaccagt gggcctgggc ctgcccgccc agcccgagcc 420
cggcctagga gaccccgaga ggagacgctc accccagagg aagaggagaa gcgaagggtg 480
cgccgggaac gaaataaact agcagcagct aaatgcagga accggcggag ggagctgacc 540
gaccgactcc aggcggagac agatcagttg gaggaagaaa aagcagagct ggagtcggag 600
atcgccgagc tccaaaagga gaaggaacgt ctggagtttg tgctggtggc ccacaaaccg 660
ggctgcaaga tcccctacga agaggggccc gggccgggcc cgctggcgga ggtgagagat 720
ttgccgggct cagcaccggc taaggaagat ggcttcagct ggctgctgcc gcccccgcca 780
ccaccgcccc tgcccttcca gaccagccaa gacgcacccc ccaacctgac ggcttctctc 840
tttacacaca gtgaagttca agtcctcggc gaccccttcc ccgttgttaa cccttcgtac 900
acttcttcgt ttgtcctcac ctgcccggag gtctccgcgt tcgccggcgc ccaacgcacc 960
agcggcagtg accagccttc cgatcccctg aactcgccct ccctcctcgc tcggtga    1017
```

<210> 13
<211> 28486
<212> DNA
<213> Mus musculus

<220>
<221> misc_feature
<222> (1)...(28486)
<223> n = A,T,C or G

<400> 13

```
cacgggatcc aaaaccctct cctgacttct gcaagcgctg cacacacatg gtacatagac 60
atgcacacgc acgtgcgtaa acatgctggc tttctgatcc tacggcttct aatttagcac 120
taacacttag actttttatt ccctgttgct gccatgactg accccagacc ctgcctttca 180
```

ggcttctcct tgcttttcag acatgggaca gttctggggt ctcttttgca aattgagacc 240
tgtgctcacc tagcacacat gtggccttgg gttccatccc cagaactgag tgaatgaggc 300
atggtggctc accctgtgat tccaatattc agaatgtgga gacagtagaa tgaagagttc 360
cagtttcagg tacaccaaac cttgtaccag aaagaagagg gggaagagaa ggaagaggag 420
ggagaaatct agcaaaagat ggaggaagca tacagtgact ttgaagatgt gttaactttc 480
ccttgctgta aggaaatacc tgcggtaatt aacttataaa gaacagaggt tatgtgaact 540
cacagtttgg ggattttagt ccagaacagg actctattct acttcgagtc tctaatgcag 600
atgatgagag cgaaatgctg agcaaacaga actgaactgc cctgtactac atggcaaatc 660
agaggaagag tagtcccatc gtcctctctg aggccacctt cagtaaccta aggactgcca 720
ggctgtgctg tttgtttgtt ctctgtctct agggtgtttg ttttcgggtt tgttttggtt 780
ttgttggtgg taggttttgt tcatttgctt gtttgttttt gatatagaat catgctatgc 840
agcccaggct gttctcaaac ttgtaatcct cctgtcttcg ccccctgact actaggatca 900
cagtatgcgc caccacacct ggctctagaa tccatctctt ggaagttcta ccacctccca 960
ataaagccac cctgagaatg aagccttgga caaccagaga atattgctga gccaaactac 1020
agcaagaagg ctgaccagaa ggctctgcca aattcacaag ccactggccc atctgcaata 1080
gatgccatct gtaaaggagt cacttctccc cagggtgaca gattcaatgc aacctcaatc 1140
aaaaccctag ctgtggtggg tttgtgtgtg tgtgtgtgtc tgtgtgtgtt tatgtgtgtg 1200
tctctctatg catatctgtg tgtgtgttgg gggtgctaac ctgtgtctgt gcatgcatgc 1260
acagtgtctg agaaagaggg tccaagatgc cggccactgt aggccatgtt actgttcgca 1320
tgggacaggc cttaaagaag agctgcaaaa gagccttgta cccacctcct ccaatgagac 1380
ccaccccagt ctgctttctc agttaatctc ttcctctctc aacttgaagc ctgtctggga 1440
atttgctgtt gagtagatta taggcttggc ctcagctggt ccactaaaag caggaggtga 1500
gggcccaggc ctgaggaagc attggggagg cagatcccag gcctaagttt gttggagaca 1560
cattctccag gaccacaaat agctgacatg ctgttaccag attgtcatcc cagtacctgc 1620
aaagtgggag aaccagaatc aggtgttcaa ggccagcctc tcaaaaaaca aaactgaagt 1680
aagtacatca actctactct gccttttaag agttaaaaag gggcctggag agatgactca 1740
gcagttaaga gcactggctg ttcatccaga agaacctagg ctcaattccc agcacccata 1800
tggcagctca caactgtcca tgacttcagt tccataattc caggggattt gtcatgttca 1860
cacaaacata catgcaggca aaacagcaat gcacataaaa aaaaataaat cataaaacaa 1920
acaacaaaga gttaaaagga caggagaaga ggaacaagag aaggttcatg tggccaggtt 1980
ttcttttccc caatttaaaa gtttcttctg aggtttacat ctcacaccca taaagagctc 2040
agaagtcaac aaagcgagga ggcttgattc atcatgaagt catttcaaaa tttctctttc 2100
tgaatataaa ccaaaatatt taattaattc cacataccac aggaagtaca ttacagcctt 2160
caaattcact ctgcttaaca gtaaactcgt gaacgaatgc acagaacaga ccccagtcac 2220
ccaccagcac tatgctaaat gtcctatgca cattacctga attacttctg gccatggcct 2280
ggcagaggaa atcatcccac tgcccagcag agatgagggc atctttgccc acagcctctc 2340
caatgccaga gagcacagct ctgtacgttg cctcctgtag ttacagaaat gttttccaac 2400
ttcctggcat cctgaaaaga agtggcttgc cttaaagtga cttttctttt tccttttcac 2460
aaacagtgca ggatggcctc ctgtgccaat gacaggtatg tgctacctca tctagttctg 2520
gtagtacgtg gattttctct ctctctctct ctctctctct ctctctctct ctgtgtgtgt 2580
gtgtgtgaga gagagagaga gagagagaga gagagaaaga gagagagtga gagagagagt 2640
gtttgtgtac ctgttctttc ttgtgtatgt gtgtacctgc tctttcttgt gtgcacatat 2700
gtgcacctgt tctttcctct gtgtgtatgt atcagctctt tcttgggggg gggtgtatgt 2760
ccccctgctt ctgccttaca agggctagta tctcaagcaa tgagttggtg gagttacaga 2820
tgcctgtgtc cagctctcta tggattgtta agctgctaac ctcagcacac tggttttctg 2880
ccctgtcaaa tgcctgcgat gacagcatcc acctggtaag ctggaaggtg gggtctgctc 2940
ctccatccca cctgagaggg aatgatttcc ttagataggc tgcggctgga actcacctga 3000
tctagaagta cactgggggc acacaggagc catggagccc ctaaggccca ccagcagctt 3060
ctgtcctatc ccttctggca aaggggccat ctgtcaaagg aggggttgaa agatggccca 3120
gaagaggtct cctcaaatct ctgaccctta caggactctg atgataaatg atacagtctc 3180
agtaggccta tcctggcttc atttcttctg ctgtgataaa atgtcattgg gggaaaaaaa 3240
aaaaagcagc ttagggagaa agggcttatt ctggctcgga actccaggtt tccagtccat 3300
tactacatga aaggcagtgt ggcaggaact tgaagcacat ggtcacatca tatcctccca 3360
gagcagtgag atatgaatcc aggggtgggg actggaatga tggctcaggg gttaagagca 3420
tcggctactc ttccagagga tgccgattaa aactgtctgt aactccagtt ccaggggatc 3480
tgacaccctc acgcagacat acttcaaata gacatacatg tgggcaaaca ccaatgtact 3540
taaaataaaa ataaataaat tataataaat aaataggtgc ttagttactt ttcccacctt 3600
taatcagtcc aagacccaag cctagggaat gtgtcaccca ctcccacact gggtcctctc 3660
acatcagcta agatagccaa gacactaccc acagccgtac ccatagggct tcttgagact 3720
ctctagcaac gtgaatcgag gttgtgtcaa gttgacaatt gaaactatta tggtgcttaa 3780
ctgctccctg atgaacagga tgtggcagag acaggcatcc cattggttaa tgtcagaagt 3840
gtcatccctc cgtggactga atttttgcct ttaacttata tttatttgct tgtttgttta 3900
ttagtgtttg tatctatgtg catgtgtgag tgcggcttcc ctcagagtcc agaagaatgt 3960
gtagaatccc tctagagctg gagtaggggg ctgccgggta tgggtgctgg aaatcaaacc 4020
cggtcctctg gaaaaatcag caagcactct gacctgttaa atcatctacc taacctcatc 4080
cttgctcatg ttttaaaaat agttatagtg gccaggtggt ggtggcgctc gcctttaatc 4140
ccagcacttg ggaggcattg gcaggaggat ttctgagttc gaggtcagcc aaagtgagtt 4200
ccaagacagc cagcagggct atacagagaa accctgtctc aaagaaccaa aaataaatga 4260

```
aataattata gtgggttttt ggacattctc tgtatgctaa gcaccttcca gatgggcttc 4320
ctgacaagag atccgtggcc tgacccacac tgatttgaaa taatgatgaa aattcaactt 4380
tcagaagcaa aggctgtagt tcctctctgg cttctaccac agtcagtaac cagtccagtt 4440
ccttagagag tcagtcacct tagtacacat tccagcggag ccaagacagt atagtgctcc 4500
ttataaacat ggaagggcag ttcctaccca gcgagtcact gcgggcttgg tgcctctact 4560
gaagcgcagg ctcaaccacc gttcaccgcg gggagcgtcc tcaggctctc gcgagccact 4620
gctgcgcgtc gcgtccaagg tttacggtaa gcgcggcgct caggacgacc acgtggcggc 4680
gaaacaccgg ccggcgggaa tgcgcggaat gcgcggtgcg gcctcgcgcg ctccccaagc 4740
cgtgaccccg gagttcccgg cgcgcgtttg tgggaccgcg gaggctagtg ccatgggtcc 4800
gcccgcgcgt ccaagccacc aagcaccaca gggagcaggg gccccatgga gggctcagca 4860
cgtgaggcta gacaaccttt tccaaaacct ggggctaggt ttgccctccc tgtttgggct 4920
ccatcacaga tctggaaccc gcttagtccc cattctaaag cccccactga tctggattaa 4980
gaatggagat gcctggtttt gcagggaagc aggtcaaagg gctctgcgcc ctgaccgccc 5040
ttcggagtgc tgagggacca gtgccagggg ctgaaagacc ccagttccag cagctgcgcc 5100
ctgggatcgc atgagggttc gctcaccacg attgagccac gtggtcctgc ctggccactg 5160
gtggctcacc ttggcccgag tgcccgctcc ccaccattta gtctgggaag ggccgagggc 5220
caaagcccaa aaggacccta gggttcttca gagcacctgt gattcgttca gagagctgga 5280
gaccgagcag acatccctcc ttcaaattgc atctctaccc ttgggggtcc ctggagccag 5340
gcttgccgct ccagagctca ttgcacggcg cgcgtctatt cgctctgggt ggtgggattt 5400
agggactgaa acttatttgg taaggcacgc ttgggaagat aggatgcgac ggtcccaggt 5460
gtgcacaggg gatgagctga gcatacacac cttagccgaa gggtgcctga aatccgctca 5520
gggtaaccta ggcggagcag ccgtgtagca cgtgggctgc cacgcgcgcc ccaaaacgcc 5580
ttctgggtga ggagagggaa gccgtaatgc ctccggaacc ttggggtcca tattcggaag 5640
tgttttctct gggcgacttg aaggctaaat taacaatggc tagctggagc agaaacagcc 5700
aagtcctttc aagttgccgc caggtatgcg gctgcaggtg accccaccta ggtgcgtccg 5760
ctcttctcca ggaggggcta cagccagagg ctcagtattg ccgcccagcc ccgcacccct 5820
aacccgagcc ccgcgcttag tcgcgagggt tcctcaatga acgcgctccc tcccacttct 5880
caatgaagtt cccacagcca gggatagtgg caaacgcaag actaaatctc cgctctcttt 5940
ggaaccttgg ctccagtcag gtgggggtag gggtagggtt cgaggaggca ggaaatccga 6000
aaggggatta aagttacttt gagattttct ttccaaataa cgtggttccc tctcggctgg 6060
gcaagtggcc ttttgtctcc aaaaggtcac tgacaaataa gcgcagaaca ctttaagccc 6120
agagcggaca gttcctgcca cgagattttt tttaaagcca atgtaaagtt aaaattgcaa 6180
aagaaagtgg gtgtttgtgt ttaagttact atttcgctgg aaaataaaaa tccaagttct 6240
ttctctgaga ttcctaaagc agttgtcccg gaaagaactc tgaattgcag cccacctcct 6300
gggctcggga ttcggacaag cagggtttcc aggatggagg atgggcgctc cccgccgggt 6360
cccaaattcc tgcgcctccc ctacgggttc ctggacggct ctacacgccc gccaaccgga 6420
cttgcacttt tgctcctctc cctgagcgca gagctcaacg aagttcctcg tggagatctg 6480
cccggtccgc ctagtaacag cactgagtcc ggattggctc atgcaaattt cagtttcctg 6540
gtctttcccg cggtggcggt ggtggggggt ggctgcgggt cgctagctcg tgctgcaggg 6600
tgggcgagcc cctttatgcc ccgctgccct tggtggcgca ccctggctag gctagcctgc 6660
tcagttcgca ggcactcagc cgcgggtgat agcgcgcagc cctataaatc atgcttactg 6720
ccgcccggta gggattttat gaatgaaaag gcagcctggc cgccctcgtg cttaggctaa 6780
ggctcccagg cttggcgggg cctgtggcca gagcaagcgt gggcactctg cccgggccac 6840
ccaccacctt cggagctaca gtggaatcac tgtcccgaag ggtccggact ttagggaccc 6900
caatgtcggg ggagggggag cggaaggcat gagctcggaa ggtgaggggt ttgcgtcttg 6960
tgtgccgcct ggacgccgtg cgctccttta ccagtttctt ctgggacacg aaagtattcc 7020
gtgggaaatg tgtgtgaata gttcgcctag cttgaagtcg ggtattgtga aaacgtttcc 7080
aaaactaggg aactaaataa tggacgaagg tggtggaacc gctttattct aaaaatattt 7140
tatttgaatc tataaattat aaacttagga gtccattcag ttaaccacga ccatctatag 7200
attctcttta aatatcacct tatcggctca caagtttatc ttgattctca ccagccgccc 7260
cccccccca actcaagact gcaattctaa aggtggagaa acaccaccac cctcaacgaa 7320
gccaatcaag aagcttccgg tggtctggtt cctggaaggg cgactaataa cttgcagcga 7380
tttacttttc ttaattaaaa aataaattag gaaggagcct atcgtgtctc aacctttttct 7440
tcaacggttt atttttcttg ggcaaaccgc ctgcagtgga ggagggggat actgggggac 7500
cctggccagg ataaaccggt cactgtatga agacaaatct cagatcccac cccacccccc 7560
agcgaggagg aatagatgaa ataatggcca ccatcttgag ctgttgctgg aattttcggg 7620
gttttatttt atttttgagc gagcgcatgc taggctgggg atcctttaaa gttcagatac 7680
ccctctggcc ctttgcaacc accccagtgc gccaggatgg agcctgcacg agagcttagg 7740
gctcgtctgg catcttcggg tgttacacag ttcctgaatt ttacacgtgt tgatgaaatt 7800
gaaagaagac agggacgctg ggatttctaa gcaatgggtc cgcctgtggg tgcctcgtgg 7860
cgtcctcgga aacgcaccca ttctcccggt ttaagaacag ggtgtccttg cacccccagg 7920
ctccccttcc atacattctt ccttggcttg cgtgtggcct ggcctccctc ctagctgtcc 7980
tcctgtccag agccgccact accccacctc cacaggtctc ggaggaccct cttagggaaa 8040
gaagcccccc tccccccttgc cccgctcttt cccagtttgg agagaagcag tccgagcgat 8100
ttgcatatct acgaaggctg agggggaagg gnnnnnnnnn nnnnnnnnnn nnnnnnnnnn 8160
nnnnnnnnnn nnnnnnnnnn nnnnnnnnnn nnnnnnnnnn nnnnnnnnnn nnnnnnnnnn 8220
nnnnnnnnnn nnnnnnnnnn nnnnnnnnnn nnnnnnnnnn nnnnnnnnnn nnnnnnnnnn 8280
nnnnnnnnnn nnnnnnnnnn nnnnnnnnnn nnnnnnnnnn nnnnnnnnnn nnnnnnnnnn 8340
```

nnnnnnnnnn nnnnnnnnnn nnnnnnnnnn nnnnnnnnnn nnnnnnnnnn nnnnnnnnnn 8400
nnnnnnnnnn nnnnnnnnnn nnnnnnnnnn nnnnnnnnnn nnnnnnnnnn nnnnnnnnnn 8460
nnnnnnnnnn nnnnnnnnnn nnnnnnnnnn nnnnnnnnnn nnnnnnnnnn nnnnnnnnnn 8520
nnnnnnnnnn nnnnnnnnnn nnnnnnnnnn nnnnnnnnnn nnnnnnnnnn nnnnnnnnnn 8580
nnnnnnnnnn nnnnnnnnnn nnnnnnnnnn nnnnnnnnnn nnnnnnnnnn nnnnnnnnnn 8640
nnnnnnnnnn nnnnnnnnnn nnnnnnnnnn nnnnnnnnnn nnnnnnnnnn nnnnnnnnnn 8700
nnnnnnnnnn nnnnnnnnnn nnnnnnnnnn nnnnnnnnnn nnnnnnnnnn nnnnnnnnnn 8760
nnnnnnnnnn nnnnnnnnnn nnnnnnnnnn nnnnnnnnnn nnnnnnnnnn nnnnnnnnnn 8820
nnnnnnnnnn nnnnnnnnnn nnnnnnnnnn nnnnnnnnnn nnnnnnnnnn nnnnnnnnnn 8880
nnnnnnnnnn nnnnnnnnnn nnnnnnnnnn nnnnnnnnnn nnnnnnnnnn nnnnnnnnnn 8940
nnnnnnnnnn nnnnnnnnnn nnnnnnnnnn nnnnnnnnnn nnnnnnnnnn nnnnnnnnnn 9000
nnnnnnnnnn nnnnnnnnnn nnnnnnnnnn nnnnnnnnnn nnnnnnnnnn nnnnnnnnnn 9060
ctttattgca aagcaaaaag tgtttattaa taattggggg cagggcaggg agcggcagta 9120
ggggcgctgg gacaggcacg aggggcctcg tggcggccgg gaatcactaa ataagacagt 9180
gtgaggaata tttggggtgg gggattcttt gttcaaacag cggacctggg taccccgcac 9240
acgctgcctg gcccagagaa aacgccaggc tgcaaggtcg cgtggccccc tagtcaatga 9300
gactgactgt tgtgcgtgcg gttctggatt tggaggaacc tgtctaggtc aaactggggg 9360
cggaggggga caaaagcaca ttttcagatg ttcagcagga gactcagggg tcccaaaata 9420
ttttaaaata atttttaaag tgaggcataa tgcccttgta gaggcaaaca gcgcccgcac 9480
cctgcaaaag gggggccccc cggagtttga gttgctggag ctcacccccc aatgtccact 9540
gagctcctga ccctcggctt ggatcaaggg tcgtccgatg gttattttca ggtcgttttt 9600
aggcacctag ttattttta aatattttta aatatttttt gaaaagatga cgtctgggaa 9660
atgcagcgcg gcggcctggg acgccaccat tgtgtctcgc gtgcgctcga gcggcgccca 9720
gcccccggcg ccccgccagc cccgcggtgg ggttctctgg cttgtggctt cctaatttca 9780
acaggggacc ggggtctccg tggccaccat tgcttctacc ggccccgcca gctgcccaaa 9840
gactttccct ttcagtttca gggtgggtgg gcactgggaa ccctcggagc aggagcgcat 9900
cccagggagt tggccaccgg aatccctctc tgtgcaatct aggaaaaggg aatggggtcg 9960
tatcgagcga cgccccccctc ccacgttgac ggtcagggtc cggatccccg caggtctgtg 10020
aggagcagaa gtgcgaagag gaggtcttcc cgctggccat gaactacctg gaccgcttcc 10080
tgtccctgga gcccctgaag aagagccgcc tgcagctgct gggggccacc tgcatgttcg 10140
tggcctctaa gatgaaggag accattccct tgactgccga gaagttgtgc atctacactg 10200
acaactctat ccggcccgag gagctgctgg taaccacgta acccaccacc gacaatccct 10260
ctttatgctg ggtggtggga gctgcaaatg gcaggagatc cagccatcca tggacagatc 10320
tcctgcccca cgaaaggcac ccgcaccgca ccgcacgaca ccgaaatggc agtgagcaga 10380
atgccaaccc attctacctc tgtgcccgcc ctctttccgt actggactcc ccaccatgtc 10440
tgtggtgaaa ccactctgaa atgtgggcgg tgtgcgcccc ctagcgacgt gcaggccacg 10500
gcgctggcgg tgggagattc tttttgtccg cagtctgtcc ccttagtatt tctacaggca 10560
cattttgttg tttacctgta tggggtcccg ggttgaccca actctttaga ccttctcgaa 10620
tcagcctgcc ttccttttga ccgtgttcct cattctctgc agcaaatgga actgcttctg 10680
gtgaacaagc tcaagtggaa cctggccgcc atgactcccc acgatttcat cgaacacttc 10740
ctctccaaaa tgccagaggc ggatgagaac aagcagacca tccgcaagca tgcacagacc 10800
tttgtggccc tctgtgccac aggtaaggct ccacccccat tcttcccagg aagagcgggc 10860
tccttgccac tccccctggg gttaaggcct ctttccttgc tatctattct gagcccaggg 10920
tgcagatagt ttgttggaga gatagatgat actgggtggc acattctctt ggctagtatc 10980
ttggagacct aacagaatga cacagcttcc ctgtgccaac ctttccactg ccaggaagtg 11040
gtacggccac agcatgcttt ctgaagaggt ttttgcctct tttcctaggc ttcctgcctt 11100
gcctctgttc ttaaggagct cgagctatag agcacacaca cacacaccag cttctcccag 11160
gctgagccac ctgctagggg ttcctacatg gatgggacaa atggtccagc ctcagcccca 11220
cgagctacat acagtacagc cccagagcag gaacttaaga ggccccacca ggggtactgc 11280
aggccagtac tttttggctg agtcctgcct gggagggttg gctggaaaca gagaaactag 11340
aacctcccta tgactcctcc aagggaccct ctctggcccc agaccccagg tagattaggc 11400
tgacataagt catggtagac agccatgcag gaacccacac acatagacaa ccctgctact 11460
tctgagtggt ttcagtacta cttctgacag ctgctcctca cctctaactg agaaccctac 11520
ccaggaggct tagcaatatt gagggggtgt ggtgttctcc cacctgttga ggaccccctt 11580
cctctgtggc cctgatcaga cctcagttgc tatagctgtt tggcctgtga tgtcagaagg 11640
gccggtagag tatctccccc cacccccaac ctccacctca cccccgctgt ctcagtgaca 11700
tcttcttatg tctgacagcc ccatagccag tgtttctaag ctgctggtgt gagaatttgg 11760
aaattctagc ttgccaacta cctactgaga gtgctgagcc cgccccaagg gtaccaaaca 11820
agggggtggc agatgggagt agcaaggcca tcggtaggct ggaaggatct aaggggtggg 11880
ggtggccctg ctggcagtca gggaggcagc tggctggggt tcctctgagg agagcggagc 11940
ctttggaagc tccaggcagc cttttatgga gctgaaagtg ccttcagaga ggcagcagag 12000
tttcccagag ataacttggg gatggagagg cacaacctcc cccaggatag ttctggcagt 12060
agtgtgcccc tcccgggacc cctagatgcc agcagagtaa tcagtaagcg ctatatctgg 12120
gtacagctgg actcagctct tccagctctc cttactgcct ttgtctgtgc ccctggaata 12180
caggtggggg taaaggacag cttggtgacc tgcggtaggc cagtgttctg tggtctggag 12240
catttgtttc tctgtaggag caaccagctc ctttgtcaca cgggaaccac tttgggaaga 12300
ggccagtgtg acatcaccgt gaatggaaag atgctaacat caaggccagg agatctgtcg 12360
tctgtgtgac aggggttccc cctataggag atgggaaggc ataacttccc ttctgcaaac 12420

cgcccccccc ccaacctgga ttcctgactg cagttgaaca atgtgctagc tagggttggg 12480
gttgttaaca caggtggcca gagcgctgtg tggatggata cagacagtgg gaaactgaac 12540
ttaatgaagt atgtaggtgt cagacatgcc actaagccag ggtacaccta tgtcatcagc 12600
aaccagtgtt agtagctagg gatccttata ggacataaca gatcttgcta ctgcccgtgg 12660
gagggagaaa aacaggctct ggaaggggct ggggactcct ggtcctgctg gctggagtct 12720
cactccctgt aaaagggctg tttgtgattt gaacttgact ccatagccag gttttggttc 12780
actggactca ctagcatgaa ctgtccttgg tgacacctgg aggtattgct tgtctgctgg 12840
atccctttaa cagccaaagc tgtatgttct ctcagggtgg tccccagagg gccacctcgc 12900
tacagcaact ctctgccttc ctgatcccat tccttccagc ctccttgatc ccaggctctg 12960
ggtgacacac aagatgattg atgggagctg acatctgttc attcttgtgt cctcctctgg 13020
cctggaagcc ctgattcgca ggccaagtga ccagctgtcc ctgtcactat ttctttgttg 13080
gacgtgacag ggaagcagtt ttcagactca tctttggtgg gtttatgcgg agaggcattc 13140
catcggtgtg acatacaact gcaggatgct gagtccagtg gacctgagag gtgtggagtt 13200
ggtgacccca gatgaagtcc taaaatgaca ccccactact tcccagttgg gacatgtaca 13260
tatgagagtc cctaagactc cagggcccct catgagggac cttggcctgg agatctgagg 13320
tggctattcc cagctccctc tgagtctatt cctactgggt tttcctggac tggggtcaca 13380
tgtcttcccc aacctgggtc agtaagccgg gaaggctaac tcccttctgt ggagtctagc 13440
ctggcctctg aggaacctgg gcctgtcatc tctacagctt tctcctgtgt ctgagatgtg 13500
tctctagtcc ttggggtgct gccaggctga ctctggaaga gacagagatc cctgcctccc 13560
acctcagccg cgcaaatact gggatgcaga tgtgaaagca ccacaccttc atagattcca 13620
tcctcttagg gcgacatagc caacccctcc ttgatgtttg ctggaagtgc tggggattga 13680
acccggtgtg tatgccaggc agcattttaa agtttgagcc aaggccttat tgacaggtag 13740
catcaaggct ctggcctgga caccgtaatc ctccgatgtg ctggggtcta gagatgcagc 13800
actgtgtgca cctagtgcca cttaggtgtc tccaagggag ctagagttga tgtgggcatc 13860
cattggaagg gcttgaagac cagccccgag gctgtgggtg ggagctgaat cataggcatg 13920
tctctgtccc tctcgctgta gatgtgaagt tcatttccaa cccaccctcc atggtagctg 13980
ctgggagcgt ggtggctgcg atgcaaggcc tgaacctggg cagccccaac aacttcctct 14040
cctgctaccg cacaacgcac tttctttcca gagtcatcaa gtgtgacccg gtgagtaagt 14100
ctcatcaaag gaggctacag ctggcaaggg cctgggaacc ttggtgggca gtgccaaggc 14160
actgtgtgac tgggctggag ctgagaggtg ccaaatctgg gtccccacga gcctcttccc 14220
agagtcatgg gtcccaaagg catagagggc tgtggtgtgg tgacagtgcc cctgcaatgt 14280
gtatgggtag tggcatgtga agcccggtct ggcccagcca gcataggacc atatctccct 14340
cagtgcagac gttggggatt gaatccataa aggactctgg tatagaccta cacattctag 14400
aactttcttt gtgtgagaga ggggtttgca tcaggcagac tgtgctacgt ctaactgtac 14460
ctttctgcca aggagtacag ctgatcatgc ttggggctct ggccgtgaat gttttctcaa 14520
gggcatcaca agttggcata gacaggcaaa agtccagttc tggcttgaac ttcagttggg 14580
ggttcaatgg aggtcagagc cactggcatg tggaaggctt ccaggtgtga cagctgttct 14640
gccctgcctg gcatcagcca cagggacacc ccgtgcccca ttcctacata tctcaattgt 14700
agaacatgca gcccccttcc cctaagtgtc tggggctcct gagaactgag aatagggtat 14760
gtgggcaggt tggagcccac aggcttcaga gagggaaggg tgtctgtggg tccatactag 14820
gctacttggg cctcactttc tccatctgtg aaatggggcc attgcccttc catcctctag 14880
ccatggtgtt catcaaacaa agggggaaca atacacaagt tttgaatgtg ggggcttccc 14940
aggtgttccc caggaatcct cctggcttca ggaatataga cagtgcccta gcttcagcct 15000
gcctaggcag ggcttcagtt catgagtctt attcctgggc aaatgctttc cagaccctac 15060
ctgtgctgga gctgtcctct ctcagaggcc acccaaggca aaaagttatg agagatagac 15120
gctccttgtg ggaagttcca gggtacagcc tgggaggagg aacctagaga gtagcagtac 15180
ctttctagtc tctggcagct ctcaccggat ttaggggtgg cttggggagt ttcctcaagc 15240
cttgctctta gcaaagcctg cctcgcaggt caaggccctg gccaccggcc tctggctaaa 15300
caaggacccc ctccatctcc gcccgcctct ccaggactgc ctccgtgcct gccaggaaca 15360
gattgaagcc cttctggagt caagcctgcg ccaggcccag cagaacgtcg accccaaggc 15420
cactgaggag gagggggaag tggaggaaga ggctggtctg gcctgcacgc ccaccgacgt 15480
gcgagatgtg gacatctgag ggccaccggg caggcgggag ccaccaagta gtggcacccg 15540
caaagaggaa ggagccagcc cgggtgctcc tgacgacgtc cccccttgggg acatgttgtt 15600
accagaagag gaagttttgt tctctttgtt ggttgttttt ccttaatctt tctcctttct 15660
atctgattta agcaaaagag aaaaaaatat ctgaaagctg tcttaaagag agagagagag 15720
agagatagaa tctgcatcac cctgagagta gggagccagg gggtgctaca aaaatagaat 15780
tctgtacccc agtaatcaac tagttttcta ttaatgtgct tgtctgttct aagagtagga 15840
ttaacacagg ggaagtcttg agaaggagtt ttgattcttt tatatgtttt aaaaaaaaaa 15900
gcttaagaaa cattgcttta aaaaggaagg aaaaaaaata cagcaaacca ttgttaaagt 15960
agaagagttt ttaggttgag aaatgtactc tgctttgctg aaaagccaca gcttaggccc 16020
tcagcctcac tccctggctt gctcagtgcc tacagccctg ttacctgata cctgtgcttt 16080
atcccagggg tgggcagacc tcttaacctt atagatggtc agtgcgacct ctagtggtct 16140
catggcgtgt ggcacaaccc ccctccccag ggctcagctt aatgtgccct ctcccccaa 16200
caacctgcag gttcacagcg ccagccacac agcggtaggg atgaaatagt gacataatat 16260
attctatttt tgtaaccttc ctatttgta gctctgttta gagagatgct ggtttttgcc 16320
tgaaggccct gcagcctgcc cacatcaggt taaacccaca gcttttgtgt gtggtttgtt 16380
ttgttgtgtt ttctttctct atgttccaaa accattccat ttcaaagcac ttttggtcag 16440
ctagctggag gcagtgttgc tggtgtgtgt tgggggagg ggttctaatg gaatggatgg 16500

ggatgtccac acacgcattc agatggctgt acaacaggtt gtagggctgg tagtatgagg 16560
tgcttgggaa gttttgttgg gtcaagaaga gagaactctg ttctcgcacc accgggatct 16620
gtcctgcaaa gttgaaggga tcctttggtg ccagctggtg tttggaagta ggaaccatga 16680
tggcattacc tggacaagga gattggggac aactcttaag tctcacacag gaggctttta 16740
aacactaaaa tgtctaattt atacttaagg ctacagaaga gtatttatgg gaaaggctgc 16800
ccatgaccag tgtgactcaa agcaatgtga tctcccttga ttcaaacgca cacctctgcc 16860
ctgctggaga aggtttaggg ccatgtctga gagattggtc ttttattggg caacgggggg 16920
gggggggggg tccttaaaaa aaaaaaccac aaagacagag atttggtctg cttgactttc 16980
ccaacccaat tggccccatt ggagagccat ccaaactgag gaaaattagg ggactccaaa 17040
agagtttgat tctggcacat tcttgccgct gcccccaagt taacaacagt aggtaatttg 17100
cacacctctg gctctgtgcc tttctattag gactttttgg caaaaggtgg agagcgggag 17160
gcttaagagg ggatgtgagg gaagaggtga aggtgggacc acatgggaca ggccacggct 17220
cctctcatgg cgctgctacc gatgactccc aggatcccag gcgttcagaa ccagattctc 17280
attgctttgt atctttcacg ttgttttcgc tgctattgga gggtcagttt tgttttgttt 17340
tgttttgcaa tgtcagactg ccatgttcaa gttttaattt cctcatagag tgtatttaca 17400
gatgcccttt tttgtacttt tttttttttaa ttgtgatcta ttttggctta atgtgattac 17460
cgctgtattc caaaaaaaaa aaaaaaaaaa aaaaaacagg ttcctgttca caatacctca 17520
tgtatcatct agccatgcac gagcctggca ggcaggtggg cggtctgcct ccagggatcc 17580
tgggaccctg atggcgatcg tcctgtcatg ctgggccctt catttgatct gggacatagc 17640
atcacagcag tcagggcacc tggattgttc tgttatcgat attgttactt gtagcggcct 17700
gttgtgcatg ccaccatgct gctggcccgg agggatttgc tctgagtctc cggtgcatca 17760
tttaatctgt taggttctag tgttccgtct tgttttgtgt taattacagc attgtgctaa 17820
tgtaaagact ctgcctttgc gaagccagct gcagtgctgt aggcccccaa gttccctagc 17880
aagctgccaa accaaaacgg gcaccaccag ctcagctgag gcatcccagc caggcaggac 17940
ccttgagggc cgctgtgtcc atggtgatgg ggtgaggttt tggccaaaag gccaaagact 18000
ggtggtgggt ccacggaatc tgccctgtga catgaaaggc tttgagggac tctggctggt 18060
ggccaggttg gctttttgta tttctggttg acacaccatg gcgcttccca gcacagacat 18120
gtgaccagca tggtccagga aaaaaaaaag acaaaaaatc tagaaaataa aattggtaaa 18180
atctcagctc actgttgtct gtgttttctg ggaacaggag tggggtgatg ccacagccat 18240
gtggggtggc atctctggcc ctggcaccaa cctgggattc ccaaggggaa gggtgtatgc 18300
aagtgacagc caagagcaaa gtgggagcct ctggcgtcag ggaggccagc gacagccact 18360
gatcagaagc ggctctgggt ccatcactgc cagctcaccc tcctgcttat ttgcagatgg 18420
aagatcaaca gaacccgcgt tcactgcccc ttgtctggcg caaatgggtg tttgctctgg 18480
gtgaatcacc gtttcttagg tgctgccaag cgcccagaga gccatgctta gtgttagggc 18540
caagacccaa ggtggccttt cacatagttc tgcagacctg agtggcctgg caagactcct 18600
gtgatgtctt cggggtggca gttactcatg actagtattg tttccaaacc ctgtggggac 18660
aggtggcttt tagacatcat cttctacaag gtagcttttg ggtctcagct tggctgtttt 18720
ggtgcctggt gggcccctgc ccttctggaa agtggagggt gtagaggaca cctggtctga 18780
gtggtgccac ccaatgatga aagcagaaag agatggacgg agggaagccc acagtgacct 18840
tgccaggcga cctactcaat gagagtatat gggaacacca gagccagagg tggcttatga 18900
agacacagta ctcatcttgt accaaattgt ctgatggcag gatccagaag gtattttgtc 18960
agcccagctc catataagca tccgagccgg gtccacagtc acatggcatc caggagaatg 19020
ggcatggggc cgtcagcatt ccaggaaatg gccaaagggc attgaggtgg aagagtgctt 19080
tccccccttgg ttcttagaag cactggggag cgagcttatc gtgtccaggt aggctgacat 19140
gaggaaatcc gctggggtgc agcagccagg cagccagtgc agcagagtgg tactaggaat 19200
gcagacacaa atagcaaggg gctaagcatc cactcaaagg ctgctgctgc tgctgcagct 19260
gctgctttcc accactgcct atcaaaggtg ctcatggaag ggcgcccaac tggaatgggc 19320
aagcatcacc atcaactgca cagggctcac acatggtgcc gtctcttaac ggtgcaattg 19380
gcttgtctcg tcatccagtg gtctgtgatt cccagctgtg gctgatagcc aagcctcttt 19440
tcttggtttt agcagtgaaa ccccagatac cactggccac tccaggctcc aaccctcaag 19500
tttaactggt aacactggag gctagatcct ctgctgggtg tagagatact cctgttctaa 19560
gggtgatggt aaaatagctt tttgagggcc ctcagagata aggactcagg gagccagcac 19620
acccaactgt ctcctctcag aagctcaatg gctggttctc tctctgcctc taccagctcc 19680
atggtcccct gccctgtgtc ctggccaggc tctccttttg caggcggcct ccatcctgct 19740
gagccatcct acctctctca gccaagccaa gctcttgaac ctgcctggcc tctctgagcc 19800
tcagctccct gaactggaaa gattgcccct ggctccccat tctgtgttgc cccactgcct 19860
ggacctgctc cagccttcct gtttgtccta caggactcac agtgaatgtg gcccctgtgg 19920
agcatgtgga gcatgacatt gcacaggtga ctctgtgtag cctctcccct cctgactcca 19980
catcctagaa ccaagtgtga ggaagttagc ctgttgccca tgggtcagct caactgctgc 20040
actttgtccc agaaagcagg ccaacttcac caaccctaca gaggatctgg gctgctgggg 20100
cccacaaaat gccagcagtg ggagctgagg actccaaggg gtgggacaca ggcagaagag 20160
taggactgtg tgttctccct gcagtgcccc aaagccctgg ccacaacatc ccaggagaca 20220
gtcaatgcgg gttgggctgt gtgtttggtg gcaaggctat gcaggccaca taaggccagt 20280
gctgggagct ggcactgcca agtgtgcttg ccaagcaaac ctgtacttga gctgataaag 20340
cagtctgttg agtgtgtggg gtggggtgta taggtaacat gaggcgggca tgttgtatac 20400
agggccccac gtgggcacag cacagtgctg gtgggatgca gtacccctag ttctgagctg 20460
atctggcctc tcttactcag ggatgtcatc ccaggaatgt atttgatcaa acagttgtgc 20520
aagaggacaa agcaagcaca ggannnnnnn nnnnnnnnnn nnnnnnnnnn nnnnnnnnnn 20580

```
nnnnnnnnnn nnnnnnnnnn nnnnnnnnnn nnnnnnnnnn nnnnnnnnnn nnnnnnnnnn 20640
nnnnnnnnnn nnnnnnnnnn nnnnnnnnnn nnnnnnnnnn nnnnnnnnnn nnnnnnnnnn 20700
nnnnnnnnnn nnnnnnnnnn nnnnnnnnnn nnnnnnnnnn nnnnnnnnnn nnnnnnnnnn 20760
nnnnnnnnnn nnnnnnnnnn nnnnnnnnnn nnnnnnnnnn nnnnnnnnnn nnnnnnnnnn 20820
nnnnnnnnnn nnnnnnnnnn nnnnnnnnnn nnnnnnnnnn nnnnnnnnnn nnnnnnnnnn 20880
nnnnnnnnnn nnnnnnnnnn nnnnnnnnnn nnnnnnnnnn nnnnnnnnnn nnnnnnnnnn 20940
nnnnnnnnnn nnnnnnnnnn nnnnnnnnnn nnnnnnnnnn nnnnnnnnnn nnnnnnnnnn 21000
nnnnnnnnnn nnnnnnnnnn nnnnnnnnnn nnnnnnnnnn nnnnnnnnnn nnnnnnnnnn 21060
nnnnnnnnnn nnnnnnnnnn nnnnnnnnnn nnnnnnnnnn nnnnnnnnnn nnnnnnnnnn 21120
nnnnnnnnnn nnnnnnnnnn nnnnnnnnnn nnnnnnnnnn nnnnnnnnnn nnnnnnnnnn 21180
nnnnnnnnnn nnnnnnnnnn nnnnnnnnnn nnnnnnnnnn nnnnnnnnnn nnnnnnnnnn 21240
nnnnnnnnnn nnnnnnnnnn nnnnnnnnnn nnnnnnnnnn nnnnnnnnnn nnnnnnnnnn 21300
nnnnnnnnnn nnnnnnnnnn nnnnnnnnnn nnnnnnnnnn nnnnnnnnnn nnnnnnnnnn 21360
nnnnnnnnnn nnnnnnnnnn nnnnnnnnnn nnnnnnnnnn nnnnnnnnnn nnnnnnnnnn 21420
nnnnnnnnnn nnnnnnnnnn nnnnnnnnnn nnnnnnnnnn nnnnnnnnnn nnnnnnnnnn 21480
nnnnnnnnnn nnnnnnnnnn nnnnnnnnnn nnnnnnnnnn nnnnactaag taactgctag 21540
atccttggac ttccattcac agctgctact gaaccattgt tgggaattgg actgtagact 21600
gtaagtcatc aataaattcc tttactatat agagcctacc cataagttct gtgactctag 21660
agaaccctga ctaatacaac atgcatacac ataaaattaa aatgtgccag gcagtggtag 21720
cacatgcctt taatcccaac acttgggagg aagaggcagg tggatttctg agtttgaggc 21780
cagcctggtc tacagagtga gttccaggac agccagggct acacagagaa accctgtctc 21840
gaaaaaccaa aaaaataaaa attaaaaaaa taagtaggta aattaaatta aattaaaatg 21900
tttaaaaaga agaaatcttg gctgaatatt ctacagtttt ttaagagttt gtcaaacatt 21960
ttgatgttac aatggccaat gatgacagta gcccctcaca taaatacata gtacagaggg 22020
caaaagtgtg cacagggcca gttcagaaat tgctggaaaa ttctgtcctg accctaagac 22080
aagtttcact gaacaatttt tttatgaaaa tggtcgggct ggagagatgg ttaagagcac 22140
tgactactct tccgaaggtc ctgagttcag gtcccagcaa ccacatggtg gctcacaact 22200
atccatattg agacctgacg ccccctcctg gtgtgtctga agatagctac ggtgtactta 22260
catataataa ataaataaat catcaaaaaa aaagaaaatg gtcaattttt aaaatcagac 22320
attctaatat aatacaaacc atagctatac taatttgaca cctgttgagg cctgagagca 22380
caacacatta aagacttggc ttgccataag gaacccgtca tgcctctgtg agagcagaga 22440
gctgtgtaca agatggatgg acgttgtact tcttacaatg ctggtctggg ttgagggggt 22500
ttcattcggg tgctgtgtgt tggtgcagat ggaatgacct cgtgcagtac agagcacaga 22560
accgagactg cattgaagtc ttgggatgca atataggtga gcgtctagaa gcacccttag 22620
atctgattat gcatttgatg tacttcattt ttagacagga cctcactatg tagctttggg 22680
tggcctggct gagtagacca agctggcctc aaattccaga ggatgcatct gcctctgcct 22740
tctgggtgct tggaatcaag gtatgcgtgc caccaggccc agtaaattta attttttcaa 22800
attaattctt ttctcatgtt tagaaatctt ttcctggttt ttgttgttgt ttgtttgttt 22860
gtttgtgact cctacattgg gtttttgtga ctcccataca gaattgggat ccacagtttt 22920
taagaaactg ggcactaaat taccccctgcc ttatgcaaag cccacaagga caatctggag 22980
agaccgctta tgtgaacagc aggagctgac tctgttcagg gagctaaagg gtaaaactgt 23040
gtatgctgga ccactccatt tctgggggtt cctggcaagc cttaatgtta ctcatctgga 23100
cctccctcta ttttcaggct gtcttgggtc tttacgttgt agcttctcat ggcctcctcc 23160
tcagggtcca ctaccaccca ccttaaacaa tgccagttcc agcctttcaa gctgaaatca 23220
agtgtaaaag gcccagccat gcttgagcag gccttccctg cacaagttca accacaagca 23280
aacttctgag tgaactgggt ggctaggacc cttccagagc agacttggca gcaggcctct 23340
gagcttgaga gcaggttcca aaactcttgg tttcaagggt ccgcacccac tggcaacagt 23400
aaggaacctt ctgggagaat gggcaagcac ttcaagtctt caatcctgca gacacttggt 23460
ctttaggcac tgatgcaacc tctccaatag aggactaaag gacccaccat ccatgaagca 23520
tggggacagg cactccagcc ctgcaaccca ggaatggcca gggtcttatg gaaagtgcct 23580
ctgggaagct gaccacagaa aggcaaggag tgacttccct tcttagaata ttaacctgag 23640
gtcaccctaa cttctaggaa atgtgagcaa agccctattc accccagatg ggctccagt 23700
gacagaccaa agtatacttc tgccacagcc caatttggtg aaccaatgag tttctaagtg 23760
gttagttggt tataggagtg tgggagacac cccagctaca gataatgatc ttgtgggagt 23820
taaaaaaaaa gggtccccac ttctagtata ctcctgaatc ccctaagagc tctgctgctg 23880
gggacaacca ggtgaagtag tggctggaat cccagggtcc tccctgcctt ctccctgtct 23940
gtcacccccct taccatgtgg ttatctctgt aactagttgc cacagcaacc taccctacat 24000
catcattaag atggtggtag agtgaactga actacaacac agagccacag gctgctgacc 24060
tttgagacag gttcctggtt gtcagggctc tggggtccca tgcctcattt tctctgtcag 24120
ccctgtgact tcagtttcct gagatacaca gcaatttcta gctctgctca taaagctggg 24180
agccactaag gtcagtgggg caatctttct agtcagcaca ctttggtcat ctccaggtgg 24240
aacctcaaag tgaacatggg agtctccctc ccaggaagtc tccctgccac ctcctcaggt 24300
aactgagtgc tcccctacct ctgggctccc agaaggtggc agccattatc agagagccca 24360
cacggggtct gaagcatctg acagcccttc aacactgctc cttctctata aatccatgct 24420
ttcctagacc tgctagattc aagacatgcc ataggctcaa atgagcaaga aactaggctg 24480
gagcagcagg cacaactctc accccactat gctttgtccc gcctggaatt ggccctttac 24540
tgtgacctcc tgcagtctct ccaccagccc acgttgctga ctacagtctg gtacatgtag 24600
acttagtggt atggggaagt cagggcttag caaacagaac tgtggcccct tcttccagtt 24660
```

tcctcatctc actaccaggc agaggaagaa aaggtttggg gtaaatagaa aataaggagg 24720
gagtgagcca gagtttctgg gtgtttggtc tcaacacaac tatcctacaa acatcaccat 24780
cagcagggct gggggatgga gaggtggtta agggtgcttg atgatgctct tgaagaggac 24840
cctgcttcag tcctgaccac catatgacag ctcacaagca catggaactc cagttnnnnn 24900
nnnnnnnnnn nnnnnnnnnn nnnnnnnnnn nnnnnnnnnn nnnnnnnnnn nnnnnnnnnn 24960
nnnnnnnnnn nnnnnnnnnn nnnnnnnnnn nnnnnnnnnn nnnnnnnnnn nnnnnnnnnn 25020
nnnnnnnnnn nnnnnnnnnn nnnnnnnnnn nnnnnnnnnn nnnnnnnnnn nnnnnnnnnn 25080
nnnnnnnnnn nnnnnnnnnn nnnnnnnnnn nnnnnnnnnn nnnnnnnnnn nnnnnnnnnn 25140
nnnnnnnnnn nnnnnnnnnn nnnnnnnnnn nnnnnnnnnn nnnnnnnnnn nnnnnnnnnn 25200
nnnnnnnnnn nnnnnnnnnn nnnnnnnnnn nnnnnnnnnn nnnnnnnnag gcaggcgaat 25260
ttctgagttc aaggccagcc tggtctacag attgagttcc aggacagcca gggctacaca 25320
gagaaaccct gtctcgaaaa accaaaaaac aaaacaaaac aaaacaaaac tcctcactaa 25380
cactagcttt atacagaact tcaatctgta tttagctcca ggctacaatt ggtcctgcag 25440
tcacagttag ggattacata gtgtctctgc aggcccaaaa ggccttctgg atcacagttt 25500
agacagactg caggaccatt ctgccaggcc ctctctctgt ttagttactt ttctgtgttg 25560
acaaaaaaaa aaaaaaaaaa aaaaaaaaaa aggcaatttg cgggatgaag gagtttattc 25620
tgtccagttt ataggtggca atcattatgg tgggaggcat gacagaagga gcctgaggca 25680
gatgctcata tcgcatccac ggccggtgaa cactgttgct cagctcactt tctcctttct 25740
tttcagtcca ggaccccact tcatgggaca cagcgcattc aaggtgggtc ttcccttctc 25800
agttaagcct ccctgagaag accctcccag gcacacccag aggtatctac gaggtgattc 25860
taaattgtct caaggtgact atcaagcagg cagcaagctg agcagaaaga cttggggaaa 25920
gccagcccag aaacctctgc ctcagacctc cagcatgtga caagtgattg ctcataaggg 25980
atgacatcta agctagagtc acaggaagcc agaacagggt gggatgagtg atagaaaaca 26040
gaccggtagt ggggacacat agctcacagg acatggtctg agaaggcagg ggattgacca 26100
gcagctcagt gattatcaag gacacaggtt ccactggtac ctgactctgc caccttcagc 26160
gacagcttgg ccacagctcc cacagttgtc tatggcagtc caagtaacac cttcatggta 26220
cagcatccaa aagcagagca ttcttctgac gacctatgcc ctcccctttc acagcaggtg 26280
tgactcagtg gtgggaggag gcggcatgcc cacgtggcag aatggtcctg tagtctgtct 26340
aaactgtgat ccagaaggcc ttttggggta accaaagcat tctggggact tgatggggac 26400
tttgagaaag ggtcaagtgg gcagaggtca aggcttgtac aggaacatgg aaagttatca 26460
ggcaagcggc tgtggtgatg ctgatgggtg ggagatctga cacctgatgt acacaaggtg 26520
aggggatagg aggtagagat gaagggagaa gagaggggaa gagacaagag aggaactagc 26580
aactgattga tacctgtagt gaatgtgaca ggcacagtag gcccatatct gtgaacctac 26640
atcttgacat gcaccatctt ggcactgaag gactgctgca gctgtcagta gaaatgatcc 26700
cagacagact actgctttaa aatacactat ttattctttt ttctgaatat aataggaaag 26760
taaaatatgg tgataacagc agctcagttc tcacaccgaa gagtccctga tcctgggcac 26820
ctgagagagt ggtgctgaat acctgagagg tagccgccag gcccaggagg ccaggtgcat 26880
ggtgccccaa gcctcagact caaaacatat ttacaaatcc ttcggacata cagctaggct 26940
ctattctgac tagattataa ctgaagataa cccatttatt ttaacctgca ttctgccagc 27000
tggctggtta cctgtgctca ggtaccatgt gtccatctcc tcacatcctc cctggtagat 27060
ctcccacctg gctctatccc agaatgcttt ctctctccca gatgttccgc ctatttcctg 27120
cctaagccat aggccatagg ctttttgcca caggtgaggc atccatacaa cacaaagata 27180
ttctctctac gaaatgcctg agagcactgt gccactcctg ccagctcgtc ttatatctgc 27240
ctgtaaatac catgccagac acacaagctg ttgctaaagg aagtacagtg tcaccaggca 27300
agccctacac attcacagtg gcggccacca attagtcctg ctttggtttc caatagttcc 27360
cctaatgcca ctttttgttt gtttggtttt gaattcactt tgtagactag gctgacctca 27420
aactgagaga tctgcctact atctcttgag cactgggatt aaaggcgtgc accaccagca 27480
cccagctcca ttgccactcc ttaatgtata gagctcaccc cacacctgtt aggagcagca 27540
tgaaggccct gcttcagctt cctccccttgt ctcgtcaccc ttgtcttctc agctgggcat 27600
ccagaggcaa accaagtcca gtggagcctg agtcctgtgc agatagcagg aatcaggtcc 27660
tccagtggtg ttggcaatga cagcgcatgt tggaggcaga ggcaccatgt ggtacttcag 27720
ggcatggacc tggggtgggg gccagctctg ccaccaggcc cttagactag atgggtctat 27780
tttatcactt ctaagccata aggtatcctc cacctctggg gctgccagag ggatggagtg 27840
aaacaaggag gcccaagcat ggccagcaga ggctcagcag gcagcaaaga tcctgtcacg 27900
gcttcccatg acccatttca catcggcctg cctctgtgag tcccaccctc agggtaaccc 27960
tacccagggc cactcactct ggccagtgac aagcggtctt ttgagtgtgt tgcccctgtg 28020
cagaaggcag ggtggtcagg taagccacct aggcagagac tcagggaaga ggccaagctg 28080
gtcctcccac agaacaatgg attcctctca ggcttgcctt taatcactgt gacacttaat 28140
gtgcagacat ctgctcttct gtgggtcctc aaaacgcaag tccagagcct cctggagtca 28200
ccttgaagtc tggctgcacg ttgagcagtg agcacagctg aaagtaccca agagcccatg 28260
ccttacacct cctgcacttc tgctcagctt tccctatccc agcacaagcc caaaggctat 28320
gtcgccttaa cgtccccaac ccactttcag tcattctaca agggccaagg aaacaagttc 28380
ctctgggtct atatctgggc acacattgcc caacacaacc atcacatgaa ccctagggcc 28440
cacgccaacc tggccttcaa agccccctca ggcacacccc atagac 28486

<210> 14

<211> 3737
<212> DNA
<213> Mus musculus

<400> 14
gagggctgtc ggcgcagtag cagagagcta cagactccgc gcgctccgga gaccggcagt 60
acagcgcgag gcagcgcgcg tcagcagccg ccaccggagc ccaaccgaga ccacagccct 120
ccccagacgg ccgcgccatg gaacaccagc tcctgtgctg cgaagtggag accatccgcc 180
gcgcgtaccc tgacaccaat ctcctcaacg accgggtgct gcgagccatg ctcaagacgg 240
aggagacctg tgcgccctcc gtatcttact tcaagtgcgt gcagaaggag attgtgccat 300
ccatgcggaa aatcgtggcc acctggatgc tggaggtctg tgaggagcag aagtgcgaag 360
aggaggtctt cccgctggcc atgaactacc tggaccgctt cctgtccctg gagcccctga 420
agaagagccg cctgcagctg ctgggggcca cctgcatgtt cgtggcctct aagatgaagg 480
agaccattcc cttgactgcc gagaagttgt gcatctacac tgacaactct atccggcccg 540
aggagctgct gcaaatggaa ctgcttctgg tgaacaagct caagtggaac ctggccgcca 600
tgactcccca cgatttcatc gaacacttcc tctccaaaat gccagaggcg gatgagaaca 660
agcagaccat ccgcaagcat gcacagacct ttgtggccct ctgtgccaca gatgtgaagt 720
tcatttccaa cccaccctcc atggtagctg ctgggagcgt ggtggctgcg atgcaaggcc 780
tgaacctggg cagcccaac aacttcctct cctgctaccg cacaacgcac tttctttcca 840
gagtcatcaa gtgtgacccg gactgcctcc gtgcctgcca ggaacagatt gaagcccttc 900
tggagtcaag cctgcgccag gcccagcaga acgtcgaccc caaggccact gaggaggagg 960
gggaagtgga ggaagaggct ggtctggcct gcacgcccac cgacgtgcga gatgtggaca 1020
tctgagggcc accgggcagg cgggagccac caagtagtgg cacccgcaaa gaggaaggag 1080
ccagcccggg tgctcctgac gacgtccccc ttggggacat gttgttacca gaagaggaag 1140
ttttgttctc tttgttggtt gtttttcctt aatctttctc ctttctatct gatttaagca 1200
aaagagaaaa aaatatctga aagctgtctt aaagagagag agagagagag atagaatctg 1260
catcaccctg agagtaggga gccagggggt gctacaaaaa tagaattctg taccccagta 1320
atcaactagt tttctattaa tgtgcttgtc tgttctaaga gtaggattaa cacaggggaa 1380
gtcttgagaa ggagttttga ttcttttata tgttttaaaa aaaaaagctt aagaaacatt 1440
gctttaaaaa ggaaggaaaa aaaatacagc aaaccattgt taaagtagaa gagtttttag 1500
gttgagaaat gtactctgct ttgctgaaaa gccacagctt aggccctcag cctcactccc 1560
tggcttgctc agtgcctaca gccctgttac ctgatacctg tgctttatcc caggggtggg 1620
cagacctctt aaccttatag atggtcagtg cgacctctag tggtctcatg gcgtgtggca 1680
caacccccct ccccagggct cagcttaatg tgccctctcc ccccaacaac ctgcaggttc 1740
acagcgccag ccacacagcg gtagggatga aatagtgaca taatatattc tatttttgta 1800
accttcctat tttgtagctc tgtttagaga gatgctggtt tttgcctgaa ggccctgcag 1860
cctgcccaca tcaggttaaa ccccacagctt tgtgtgtggt ttgttttgtt gtgttttctt 1920
tctctatgtt ccaaaaccat tccatttcaa agcacttttg gtcagctagc tggaggcagt 1980
gttgctggtg tgtgttgggg ggaggggttc taatggaatg gatggggatg tccacacacg 2040
cattcagatg gctgtacaac aggttgtagg gctggtagta tgaggtgctt gggaagtttt 2100
gttgggtcaa gaagagagaa ctctgttctc gcaccaccgg gatctgtcct gcaaagttga 2160
agggatcctt tggtgccagc tggtgtttgg aagtaggaac catgatggca ttacctggac 2220
aaggagattg gggacaactc ttaagtctca cacaggaggc ttttaaacac taaaatgtct 2280
aatttatact taaggctaca gaagagtatt tatgggaaag gctgcccatg accagtgtga 2340
ctcaaagcaa tgtgatctcc cttgattcaa acgcacacct ctgccctgct ggagaaggtt 2400
tagggccatg tctgagagat tggtctttta ttgggcaacg gggggggggg gggggggtc 2460
cttaaaaaaa aaaaccacaa agacagagat ttggtctgct tgacttttcc caacccaacc 2520
caattggccc cattggagag ccatccaaac tgaggaaaat taggggactc caaaagagtt 2580
tgattctggc acattcttgc cgctgccccc aagttaacaa cagtaggtaa tttgcacacc 2640
tctggctctg tgcctttcta ttaggacttt ttggcaaaag gtggagagcg ggaggcttaa 2700
gaggggatgt gagggaagag gtgaaggtgg gaccacatgg gacaggccac ggctcctctc 2760
atggcgctgc taccgatgac tcccaggatc ccaggcgttc agaaccagat tctcattgct 2820
ttgtatcttt cacgttgttt tcgctgctat tggagggtca gttttgtttt gttttgtttt 2880
acaatgtcag actgccatgt tcaagtttta atttcctcat agagtgtatt tacagatgcc 2940
ctttttgta cttttttttt ttaattgtga tctattttgg cttaatgtga ttaccgctgt 3000
attccaaaaa aaaaaaaaaa aaaaaaaaag aggttcctgt tcacaatacc tcatgtatca 3060
tctagccatg cacgagcctg gcaggcaggt gggcggtctg cctccaggga tcctgggacc 3120
ctgatggcga tcgtcctgtc atgctgggcc cttcatttga tctgggacat agcatcacag 3180
cggtcagggc acctggattg ttctgttatc gatattgtta cttgtagcgg cctgttgtgc 3240
atgccaccat gctgctggcc cggagggatt tgctctgagt ctccggtgca tcatttaatc 3300
tgttaggttc tagtgttccg tcttgtttg tgttaattac agcattgtgc taatgtaaag 3360
actctgcctt tgcgaacgca gctgcagtgc tgtaggcccc caagttccct agcaagctgc 3420
caaaccaaaa cgggcaccac cagctcagct gaggcatccc agccaggcag gacccttgag 3480
ggccgctgtg tccatggtga tggggtgagg ttttggccaa aaggccatag actggtggtg 3540
ggtccacgga atctgccctg tgacatgaaa ggctttgagg actctggctg gtggccaggt 3600
tggcttttttg tatttctggt tgacacacca tggcgcttcc cagcacagac atgtgaccag 3660
catggtccag gaaaaaaaaa agacaaaaaa tctagaaaat aaaattggta aaatctcaaa 3720
aaaaaaaaaa aaaaaaa 3737

<210> 15
<211> 888
<212> DNA
<213> Mus musculus

<400> 15
atggaacacc agctcctgtg ctgcgaagtg gagaccatcc gccgcgcgta ccctgacacc 60
aatctcctca acgaccgggt gctgcgagcc atgctcaaga cggaggagac ctgtgcgccc 120
tccgtatctt acttcaagtg cgtgcagaag gagattgtgc catccatgcg gaaaatcgtg 180
gccacctgga tgctggaggt ctgtgaggag cagaagtgcg aagaggaggt cttcccgctg 240
gccatgaact acctggaccg cttcctgtcc ctggagcccc tgaagaagag ccgcctgcag 300
ctgctggggg ccacctgcat gttcgtggcc tctaagatga aggagaccat tcccttgact 360
gccgagaagt tgtgcatcta cactgacaac tctatccggc ccgaggagct gctgcaaatg 420
gaactgcttc tggtgaacaa gctcaagtgg aacctggccg ccatgactcc ccacgatttc 480
atcgaacact tcctctccaa aatgccagag gcggatgaga acaagcagac catccgcaag 540
catgcacaga cctttgtggc cctctgtgcc acagatgtga agttcatttc caacccaccc 600
tccatggtag ctgctgggag cgtggtggct gcgatgcaag gcctgaacct gggcagcccc 660
aacaacttcc tctcctgcta ccgcacaacg cactttcttt ccagagtcat caagtgtgac 720
ccggactgcc tccgtgcctg ccaggaacag attgaagccc ttctggagtc aagcctgcgc 780
caggcccagc agaacgtcga ccccaaggcc actgaggagg agggggaagt ggaggaagag 840
gctggtctgg cctgcacgcc caccgacgtg cgagatgtgg acatctga 888

<210> 16
<211> 30346
<212> DNA
<213> Homo sapiens

<400> 16
catgcctgta atcccagcac tttgggaggc caaggcgggc agatcacctg aggtcaggag 60
ttcgagacca gcctggccaa catggtgaaa cccgtctcta ctaaaaatac aaaaaaaaaa 120
aagaaaaaaa agaaagaaaa gaaaaatagc tgggcatggt ggcgcatgcc tctaatccca 180
actacttggg aggctgaggc aggagaatcg tttgaaccca ggaggtggag tttgcagtga 240
gctgagaccg cgccactgca ctccagcctg ggagacagag caagcctctg tctcaacaaa 300
aaaaaaaaaa aaaaaaaaaa aaaaaggga aaaatcactc caatcatccc aagtctcagc 360
tcctactctt ttgctatatg tccttcaaac cagctatgta tttattttgc tgaatttatg 420
ctggttttat cttttgtatc ttgctttttt tcatttaatg tttttccatc cagtgaacac 480
accctaattt gccattcccc tcttcttgaa catgagattg tctctagggt atctcttgca 540
aataggataa ttgtcaatga tgtaacgggt ctctatgcaa cttgcaaaag tgatcagtct 600
tggtgtcctc ctcatcctag tgtttggttc caagcaacag aaaccaatgc tggcagattt 660
cagcagaaaa ggaaaatcag atgcagaaga taggcaggaa caagggaggc aagacagcac 720
gaatcacaat ggaagcatgc tgcagatctt ctctgatgag aagccagggc tacccctacc 780
catcccaaca ccagaccctc cagtaaaact gcagcggccc acaccacctt gtcaccagag 840
cccacagctc tatgatgggc agcacagata ctgctgctca taaacttgaa tgcctcaccc 900
agttctacac tagaaaacat ttcctacctt ttctgattct tcccaccacc agctcccaat 960
tcaaaatctt gatccaaaaa gatgaaatgt ataaataata ctatttataa tgacatcaaa 1020
atatcaagta cttaagaaga aatctaataa aagatgcaca acacccaaca cacacacaca 1080
cactcacaca cacacacctg caattaaatt aaggacaatc taaataaaca gaggtctata 1140
tcatgcccat gaattgaatg actctgttag taaaagtgtc aattccctca aaattgatct 1200
atagatccaa tacaatccca atcaaaattc cagcattgtg tgtgtgcaca tgtgtaagtt 1260
gattctgaaa tgtttatgga gccccaaggg ccaggagtag ccagggcaac cataaaagag 1320
aagtgggttg gaggatttag cccgccagat atcaagaatt cgtagcgtat agcttccatg 1380
atgcttctgg ggtaaggact gaaaaagaga ccagtggaac gggatagaaa gtccagaaac 1440
aagagatttg tgttaagtga gcagatttta gctgctctgt tcacaaaaaa aagtaactat 1500
gtgagatgac aggtatgtta cactaattca ctatggtaac cattttacta tgtatatgta 1560
tcttataaca tcatgctgtc aagtttattt caaaaataaa ttttacataa aatcttagca 1620
gtgttattca taatagacaa aaagtgggaa aattctattt ttttaaagta cagaaacttt 1680
attcctcaaa ggggaaatag gttaataata ataagaagaa gaattaaggc caggtgtggt 1740
ggctcatgcc tataatccca gcactttggg aggccaaggc cagaggatta cttgagccca 1800
ggagtttgag accagcctgg gcaacatagt gagaccccat gtttacaaaa attaaaaaat 1860
tagctgggtg acgtgacaag tgcctatagt cccagctact tgggaggcga aggcaggagg 1920
attgcttgag accaggaggt caaggctaca gtaaggtatg atcgtgccac tgcactccag 1980
catgggcaac agagcaagac cctgtatcta gggggaaaaa aatgataagc cttgtgaaag 2040
agaaatgaaa acagcatcca cacataaact tgtacctctg tgttcatcgg cagcattgtc 2100
cacagtaacc aaaggtagaa accatcctaa ggcccattga ctgatgaatg gataagcaaa 2160
atgtggtcta tcaatacgat tgatagtatt caaccataaa gaggaatgaa ccatggacac 2220
atgccacatg tgggccttga aaacgtgatg ctaagtggaa aaagccagat gcaagaggct 2280
gcatagcata tgattccatc tgttggaaat gtccagaaaa ggcagatcca cagagaaaga 2340

```
ttagtggttg ccaggggctg gggaaaaggg ggaagaaaag ggtgaataac tacaaatggg 2400
tacagggttt ctttggcgga tgatgaaaat gttctcagat ggtgatggtg gctgtccaac 2460
ataacaaaat ccatcgaact atatactcta aacgagtgaa ctttacggta tgtaagttat 2520
ttttcaataa agtggtttca aaaaaataaa aaggtgaacc ctaccatttc taggtgcttt 2580
ggagtttacc gtttttctcc ttgcattctg gagtcagagt gagaaggcat ctcagctcta 2640
ccacagctga atgatgtgtt gggcttcggt ttcctcatct gtaaaatggg gataaagggc 2700
ctctccttgg agctgccatg aagattgaat tagatgccct ctgtgaagat gcagggcagg 2760
tggagagcac agtaagcgtc tcggggggcc acgccatcac cgccagcagg gctcaccagt 2820
atctttgccc agagaatagc cggggtctag ggagcagcca gggtctaggg agaagggctg 2880
cttccctgtg gaggctccta actttcccat ctcctccctg tttctcccct gcccacccct 2940
tactcctgga agtgtgactc gctgcctcct cccgtgactc ccctacattg catctcccca 3000
gttactgtcg ttatctctca tctcttcttt ccaaacagcc tctgtctagc accttggagc 3060
tcgcatggga attcatgggt gggtggattc caagcttggc ttcatcagat gacaactaaa 3120
aggcagaggc ctgggggggc aagggagctc caggatctgt ctccctttgg agaagcccag 3180
gttcaccttc ccagcaagct gctccggccc cacgcccctg gccctcaata accttcagtg 3240
gctcccttct tgcagctata attcccctct tgggatgcta caaaagggat tccagagatt 3300
tttttttcaa taactcaaaa gggatggagt aaacgatata tgtgcccagg acaaggcagc 3360
taagggaagg cctcaggctt cctttgcctt tgcctggaag tataccaacc cgcagcccgg 3420
agggccagca gagaaccaat gctcagagcc tggggcccca aggaatgctt tccaggctgg 3480
cggtcccagg agccccctct cccttcccta tagcaagata ctaagagcag aggctggcct 3540
ggcaggccgt gtcgaggccc tgagctcaca tggccttggc agcttcttga cagtcgtgtc 3600
tttttgtttc cagcagtgac actcactgac ccacaccttt ggccagcgtc ccttgtgact 3660
gccccctccc gcttgagcag aatggctcct attacacaat gggtgccagg aacctggcca 3720
gtccatccag agacagggca gagccctggg ctaccgaccc tctggggcca gcagaaaccc 3780
tccctatggc tctgcctctc atcctggcat tgacacttgt gcccttaatt aaagtgcttc 3840
tttttaaat caacatttct actcctttg cagagtaaga gaaagaagag ttaggggaga 3900
agaaagaagg aaggattcat ggggttattt ttttttttc atgtaaaagt ttgttccgtg 3960
atttaaatct catgtccaca aacagctcag gagaagtcag catagagaag caagctgaca 4020
gattataaag tcatttgtaa aatcctcttt ctgcatataa actgaaatgt gtaattaatt 4080
ccacatactg cgtgtggcac atggccttca tcctgcataa gaacaaatac atgaatgaat 4140
aaatcgagca ggccttgaca ctcaccaggc cctgtgctaa atgctttcta catattacct 4200
taattactcc tcaccgtggc cttgcgaaga aaacgatcac agcccgtttt acagatgggg 4260
acagagattt gtccaaggcc acacaactag tggcagatgt aggtctgtat gtgacattct 4320
tgttcagttt cataaatgtg gctgccacct gcctgaaatt ttgagggaaa aaaaagtggt 4380
ttggctttgc atgaattctt gaaaaagtag atttttatct ttaccagctt ctaagcaagc 4440
tggatttatt ctgacagtgt ccatgaaatg gctacagacc atcaccgttg ccaatgttgt 4500
gaacctgaac aaggcacctg gtttctctgt gcctcggttt ccttaccatt aaacgcattt 4560
ccctgagatt aagtgtgtga aactataagt tggctggagg aaacacctca tagaagtgag 4620
aagatgagga tgggtggtga gtgaaggctt tttctccaaa ccctacagga gatggacctc 4680
aaagcaaatg gccatgcttc ccttagactg ggtgaactgg tgctgctcag ggctagaagt 4740
gtgtctagaa gcatggggac caatgcagcc ccctccaccc tagggcacat aacctgtctc 4800
tctcccatag gatagcagag acagctctct aagtcaggat tcagcgagat ggtccaggag 4860
gggcctaagg attcctctga cttatccaag cctggggagg caacacaacc tagcctgtgt 4920
ctcagggggc cccaatcatg ccctgaacct catgagccca ggcaggcatc ctgttggtta 4980
aggtcggcct cagggtccag ggatgcacta gattctttat cttaaagtga ccctcaagaa 5040
aaaaaaaata aaatgtagct ataggcagtt tctggacgtt ttcctatttg ttagcacct 5100
tcttgctccg agctttccag ccacagtgca ctggacgcca tcggaaggct gcagctgcta 5160
tttggtgctt taccaccgct gagaaggtgg aatggtcacg gcccggttct gtcactttct 5220
tcagccggac agtcgcctta ttacggattc cagtagggcc gagcacactc cttcctgccc 5280
gtctttacag atgaacatgc tatcgctcca gcagtacaac ccgccttatt acgtaaaaaa 5340
gcagccccta tctacccgca gggaaggagt atgttcggca ccacagctgg actggtgctc 5400
gagttaagcg tcctgggagg tccgcatgcg ctccggaacc gtaatgcgcg ctttttctaa 5460
gccttacggt aaacgcggac gcagggcaac cacgtggcgg tggaaccgag gcccggcggg 5520
aatgcgcgga atgcgcggcg cggcctcgcg cggttcccga gccacggccc agggtccggc 5580
ggcgcgcgct ctcgcctcct cccctcacct ctcccagccg caccccggcc ctggccctgc 5640
cacccagaac tcgctgggca agtcgtgccc cgcgtgaaca cacagaaggg gcttggggac 5700
cgagcgcggc ccatcagtcc ctcagaccct gaggacccag aattccctaa ggggtccgaa 5760
tccgagtcct gcccccagcc cttaaggcac gggctccagg gaccccaggg gaagggcgcg 5820
gggcattagg tacgcaaccc gtttccccgc acctggaaaa aaactccctt tccctcccct 5880
cccctgcttg ttgagtgtgc ggataaccag aactctaagg cgccccgtaa taacgacccc 5940
gctgtccctc cacccacccc caagtgccaa agcgagggat ggaagcgctt tcaagcgttc 6000
caagggcatt gaggagcgag ctggagaggc gcggggatgc ggggtcctcc ccgcagtctt 6060
ccggaaaggg cgggggaggg cgcggcaagt tccggagtgg ggcatgccgt gggagcccac 6120
gagggcctca gcgcggatcc tccgccggaa aaccggctcc cgcgagccgc cgccgcaggt 6180
ttcctaggcc ccgcgagtcc cgcagcgaag ccctgcgtct ccgtccgacg cggggtctg 6240
ctcagcctcg ggtgggccgc ggccaggcct gactgcgggg gagagggccg aacgtgacct 6300
ccgaggtcac ccccagccag ctttctctcc tgtggtcgga agtggttttc ttctcgatct 6360
gggcgcctac tccccaccac ttggtctgag aggggctggg gccggaaggc cagggaatct 6420
```

```
ctggtggatt tgggggttca tattgctcag ggtaccagcc gatgcgtttt gaggggcggg 6480
agtcgaggaa ttagaatcgc ctttaaccct caagagttgc gccttcagcc tcgggatccc 6540
agatgcgtcg ttggagccag ggccgccccc ctacctgttg ggtttgcgtt ttaactccag 6600
cgcacacctt gccggcagcc ctcggagcta ggggaggggt ctcgtttccc cgcagcccgc 6660
cggacagacg actggggcac gggagggggcg gtggcagggt ggtctgtgtg tggctgaaac 6720
taattgatct ggagcggaaa cgcacgtctg cggttggggc gatgggggg gcggtgcggc 6780
tgtccatgtg ccgagcgtgt ggctgtctcg ggtgggcact ggggccggag ttcgccccgg 6840
cccacctcgc agttttgggg cgcctgggat cggcgctacg taagcgaagc agagctgcca 6900
tagcacgtgg gccgccacgc gcaccccaaa agcaagcagt gtgggggggaa ggggagctcg 6960
agcgccttcg gagcccaggg gccggctttc ggaagcgttt tcccgggcga cttaagggct 7020
taacaatgga aaactcgcgg agcctgagcc aagtcctttc aagtcgccgc caggtatgcg 7080
gctgcaggtg accccacctg ggtgcgcccg cccgccagcc gccctggtgg aaaagcgggt 7140
gcgggaggtc gctggcgaaa ggtcgggact ggtccctgca ccacccgccc ccaacccaag 7200
ccccgagccc cgcggcgcgc agccgcgctg agtcccgggg tctgcgtcgc ggcgcgccgg 7260
ttcctgaatg aacgcgctcc cttcccccgc ctgaatgaag gttcccacag ccagggacgg 7320
tggcgaacac gcgcctgcag cggaattcgc tttctcctga ccgaccatcc gcccaggccg 7380
cggtcaccgg ggcgggggcc agggggcgag gaaagcgtga aggtgatttc agttaatttt 7440
ggattttctt tcaaacaacg tggttaccct cccgactggg ccacttgccc tttgtctcca 7500
aatggtcacc aagaaataag aacagagcac tttaaatgag cccagaatcc gcagttcctg 7560
cttcgtggtg ggttttaaga agacagtgta aagtaaaact gcaaccgaaa agttttttaa 7620
agttgctttt ctctttggaa aaaataaaat caaaatgctt tctctgcgct tcttgaagca 7680
atgaccctca aaagcccaga ggtattggcc ccctcggggg acccgggggc cgccaagcag 7740
ggttccccca ggtgggggct gggcagctgg cgctccccgc cgggccccaa attccagcgc 7800
cgggcccgca aattccagcg cctcccccgc gggttcctgg acggctcttt acgctcgcta 7860
accgggcttg caattttgcg ctcgtccctg agccgggaaa tcaacgaagt tcctagtcga 7920
gatctgcccg gtccgcctag taacagcgcc gcgcccccat tggctcatgc taattccagt 7980
ttcctctgtc ttgcgcccgg gatgggggg tgaagctccc tcctggaccc agagccggtt 8040
gtgccggagt gggcgagcct ctttatgccc tgctgcccct agccgacttc ggcccgcttc 8100
gcgcctcggg ctgggccagg gcgcacgcgg ggctcggggc ccctcgcccc acgggatggg 8160
agaggccggg tgatagctcc gggccccata aatcatccag gcggccgccg ggtcgggatt 8220
ttatgaatga aaaagcagct gggccgccct tgtgcgcggg ctgatgctct gaggcttggc 8280
tatgcggggg ccaacgcgat tgtgggtgct cggggagtgg gggggggcac gaccgtaggt 8340
gctccctgct ggggcaaccc atcgctcccc atgcggaatc cgggggtaat taccccccca 8400
ggacccggaa tattagtaat cctaattccc ggcgggggag ggggcgcggg aggaattcac 8460
cctgaaaggt gggggtgggg ggggtcgcat cttgctgtga gcaccctggc gaaggggaga 8520
gggctttttc tatcagtttt ctttgagctt ttactgttaa gagggtacgg tggtttgatg 8580
acactgaact atattcaaaa ggaagtaaat gaacagtttt cttaatttgg ggcaggtact 8640
gtaaaaataa aaacaaaagt taagacagta aaatgtcctt ttattttttta atgcaccaaa 8700
gagacagaac ctgtaatttt aaaaactgtg tattttaatt tacatctgct taagtttgcg 8760
ataatattgg ggaccctctc atgtaaccac gaacacctat cgattttgct aaaaatcaga 8820
tcagtacact cgtttgttta attgataatt gttctgaatt atgccggctc ctgccagccc 8880
cctcacgctc acgaattcag tcccagggca aattctaaag gtgaagggac gtctacaccc 8940
ccaacaaaac caattaggaa ccttcggtgg tcttgtccca ggcagagggg actaatattt 9000
ccagcaattt aatttctttt ttaattaaaa aaaatgagtc agaatggaga tcactgtttc 9060
tcagctttcc attcagaggt gtgtttctcc cggttaaatt gccggcacgg gaagggaggg 9120
ggtgcagttg gggacccccg caaggaccga ctggtcaagg taggaaggca gcccgaagag 9180
tctccaggct agaaggacaa gatgaaggaa atgctggcca ccatcttggg ctgctgctgg 9240
aattttcggg catttatttt attttatttt ttgagcgagc gcatgctaag ctgaaatccc 9300
tttaactttt agggttaccc ccttgggcat ttgcaacgac gcccctgtgc gccggaatga 9360
aacttgcaca ggggttgtgt gcccggtcct ccccgtcctt gcatgctaaa ttagttcttg 9420
caatttacac gtgttaatga aaatgaaaga agatgcagtc gctgagattc tttggccgtc 9480
tgtccgcccg tgggtgccct cgtggcgttc ttggaaatgc gcccattctg ccggcttgga 9540
tatggggtgt cgccgcgccc cagtcacccc ttctcgtggt ctccccaggc tgcgtgtggc 9600
ctgccggcct tcctagttgt cccctactgc agagccacct ccacctcacc ccctaaatcc 9660
cgggggaccc actcgaggcg gacggggccc cctgcacccc tcttccctgg cggggagaaa 9720
ggctgcagcg gggcgatttg catttctatg aaaaccggac tacaggggca actcccgccgc 9780
agggcaggcg cggcgcctca gggatggctt ttgggctctg cccctcgctg ctcccggcgt 9840
ttggcgcccg cgccccctcc ccctgcgccc gcccccgccc ccctcccgct cccattctct 9900
gccgggcttt gatctttgct taacaacagt aacgtcacac ggactacagg ggagttttgt 9960
tgaagttgca aagtcctgga gcctccagag ggctgtcggc gcagtagcag cgagcagcag 10020
agtccgcacg ctccggcgag gggcagaaga gcgcgaggga gcgcggggca gcagaagcga 10080
gagccgagcg cggacccagc caggacccac agccctcccc agctgcccag gaagagcccc 10140
agccatggaa caccagctcc tgtgctgcga agtggaaacc atccgccgcg cgtaccccga 10200
tgccaacctc ctcaacgacc gggtgctgcg ggccatgctg aaggcggagg agacctgcgc 10260
gccctcggtg tcctacttca aatgtgtgca gaaggaggtc ctgccgtcca tgcggaagat 10320
cgtcgccacc tggatgctgg aggtgcgggg cttcgggcgg ctctcttaag acttccctgc 10380
aacttgttgc ccagacccac gtttctttgc tactcacccc cctcccttct ctcccgctag 10440
aactttgaag tttgccgtgg tgtttctagg gatccgtatt ttcaaaataa aaattgcggg 10500
```

tattttctga aggaggaagg ggtgggggtg ggggtgctag aagtagcgtt tcgtgggagg 10560
ggagaagggg gtccgggagg ggtgccttcg ggagaagcca gtgccagggg caccccaatg 10620
ggcccgaggg tgcgggctgg caggctgggt gcgctttgtg tcccccgcct gcgccccagc 10680
ccggctgcgc ctcagcggcc gggagccgcc aactccgggg ggaggggca tagatttgat 10740
ttttaaatta atatccatgg acacgtatgc aagggccgct cgtgccagta ttatgcgcca 10800
tctttgctct tttattgcaa agcaaaagtg tttattaata attgggggca gggtgggggc 10860
ggggagcggc cgccgggcgc tggggccgca gctaagggcc gcgcggctgc cgggagcccg 10920
cgggaggggc gcagggacgc ggcatgggta gttttggggg gaccccgcta gggaaggggg 10980
ggcctttgtt caagcagcga gtcccggggc gccccgaacg ggcagcctgg gccggagagc 11040
acggcgagct gcaaggtcgc gtggccccca agacgccagg gcttgatccc cgtctgcagg 11100
gatatcggct tggaggacct tctccgagcg agccgggggc ctgggagcac attttcagac 11160
cttcggtggg cgcctgaggg gcccgcaagt attttaaaat aatttttgaa agtgcggcgt 11220
ggtgcccttg cgagagggaa acgccgcccg cgcccagggg gaaggggggg ccccggagtt 11280
tgaattcctg ggctcccccc cggagcctgt aacgaactcc caaccccccgg cctgggtaaa 11340
gggtcgcccg agggtcattt tcagggtttt tttatgcact tagttatttt tttaatattt 11400
ttaaatattt tttgaaaaga tgacgtctgg ggaaatgcgg cgcggcggcc tgggacgcca 11460
cctttgtgtc tcgcaggcgc ggcgcccaac cccgcggccc gttccgcggc cccgcacccc 11520
agttggtgtc gaccccccagt cagagggacc acggagctcc agggcgggcc agggtcccgg 11580
gggccggcag cccgcgccgc cgcgcacgcc gcccagctgt gcccgctccc gcccccaccg 11640
tgccagcctc gcggggactt tccctttcag tttcggggag ggtgggtact ggggacgcgc 11700
gggggagggg gcgcatcacg ggaagctcct gccgccccca gccccgaccc ctcggcgccc 11760
tccagacctg gcggccctgc caagcgcgat gggggtgcg ggggcgtgcg ggggggcggc 11820
gcgacctggc ggcggcggtc acgggccccg tgcctccgta ggtctgcgag gaacagaagt 11880
gcgaggagga ggtcttcccg ctggccatga actacctgga ccgcttcctg tcgctggagc 11940
ccgtgaaaaa gagccgcctg cagctgctgg gggccacttg catgttcgtg gcctctaaga 12000
tgaaggagac catccccctg acggccgaga agctgtgcat ctacaccgac aactccatcc 12060
ggcccgagga gctgctggta accactggac cccgccgccc cccgcccccc gcgagccgca 12120
cgcaggacca cggggccggg gaaggtgcag gcggtggcgg ccggcccgcc tctgacatat 12180
ctgctcctcc gagggagggc ggccccgccg ccgggcgtcc ctgtccgggg agcgggcggg 12240
atcctagccg ccctcgtccc gccgccctgt gtgcgcttgc ctgcgactcc caccgcgttc 12300
gcgccccgcg gtgtggccga aaagtgggcg gcgcgcgccc tccagcggct gcacgaggag 12360
cgccgcgctc ggcgctgagc ctccagttcc aggtggtggg aggtcttttt gtttccactt 12420
gcagagtctt ttcacgcggc gggcgccttt tctgttttga tctgggattg cgtgttgccc 12480
cagctccctt gagtccccag cattcgccag ccctcccctc caacatccag gaccgcacga 12540
gacgcagggg ccagtgctct gagccggagg tgcggcgtgg cccggccccc gtgctgccgg 12600
cttccccgcg cccccgggct ggcccgcacc tcccctgatg gccgctcacc ctgtgttcgc 12660
agcaaatgga gctgctcctg gtgaacaagc tcaagtggaa cctggccgca atgacccccgc 12720
acgatttcat tgaacacttc ctctccaaaa tgccagaggc ggaggagaac aaacagatca 12780
tccgcaaaca cgcgcagacc ttcgttgccc tctgtgccac aggtagggca ggcccggcag 12840
cccccggcct cccccttgaga gccggctcct taggtgaccc tggccggctt cttgctctcc 12900
acctgggtgc tgtctgggaa gatgtcccca gaccccctcc tgcgctggag agcgctcttc 12960
cagctctggt gagcagaggc cctggattgt ttgtcgcgct ggatggaggg agatttgctc 13020
cctcacggcc accatgcagt accttgggca ttggtgtgga cggctcagcc tgcctgtgtc 13080
ccgttactct ggcctcgtcc ttcaggccag gcagcctgtg gccactccat gctgaaaggg 13140
gtttaccttg gccacagggc cgcctccttt ctccacccac ctccagccct tcttgtgtcc 13200
ttaaggagcc tgagctgcag aggcccccctc ctggcctctc ccaggctggg ccacctgcca 13260
gaggcgcctc caggggcggg gagagctgtc ggcctgcctg caccacgtgc tctgggcagc 13320
cgagtgcagg ggtgtccagc agaggagctc ggctgcctga ggccctgcca ggggtgccgg 13380
cagccagccg ggctcagctg agccctgagg gggcgcttca gagcactctc agcttgggcc 13440
gccaccgtgg gcagcagaag cacccagtcc tcacttcccc tggcatggcc ccagaggccc 13500
ctccctgaca tggccttggc cccagaaccc agtggggaca gactcgcaca tacacagggt 13560
gccgcctcct gctgtcccca gccctgcctc tgacccccct gtgaccgcct ccttccctgg 13620
cccaggaggc ctggttacct tcatgggggga gcatggcccc atcccaccca gctctgctgt 13680
ggcccacctt tggtcaagcc tcagttgtca catctgtttg ggggctcact ctgggtgacc 13740
taggccacaa ggcccacggg gcatcaaaga ggcagtagca tcttctcccc tccccagagg 13800
gcagagcccc ccaagcctac ttcagagctc ccttctgaca ccggtagccc gcagccggta 13860
ttccagaatg ggttctggtt taggcgtgag gcctccccca cctcctccac ctgcttgggg 13920
catgaacccc tcccccacgt ttccaagcga gtccccaagg tgggcagatg aagatgccaa 13980
ggatgtcgac cagtctggat gggtctgggg tgggggggca tgcggcagac agggaggcat 14040
tctctggctg gtgctcctca gaggagagag gcctccggag actccagaca gccttttatg 14100
gagctgaaag tggcttcaga gaaatgcaaa gtttcctgga gagaacgtgg ggcgtggttc 14160
ttgcacagcc tccctacagg gtggctccag cagtggagct cccctcccag gacccctggg 14220
tgctagtggg aggcagtggg caggtgcaga ttctcgtcct tcccactact gcacaccctt 14280
tgtctgcgaa ggcgccccca gcggtgggtg aaggaggagg gacacttggg gacccagctg 14340
tgcacgtgct ctcagtgact gtggagtcca ctccagggtg ggtcccgagg gaggggcagg 14400
agaccagggg acccaccccct gcaaagtgct ccgggtcctg acccgtggcc accccatgga 14460
acgtaactga gcagccagtg ccttgttcct gctggacatc tgtggagaca agagtgactt 14520
acggctgctt aaagtcagaa acaggttgaa ggaggtggag gcgtgggaaa gagtctagga 14580

aggtgttttt gccctccacg tggcaaaggt tacatttaaa ggtgatgctg ggtgttctcc 14640
ctgcactagg cattcctggc cccaggtccc cagcaggtgt gcacatgctg catacactca 14700
cgcatggggg tttcagggca ggtgcgccct tggctccgtg ggaggccagg tgaggaacgt 14760
ccagtgccaa ggagcttccg ggacagctgt cacttccctt tacaaccagg cagcggatag 14820
ggtcaaatcc tggagctttg gtgtctaatt ctgggtggct cctaatctaa gcacagacag 14880
caccacacac tggggtgggg gcacgagctt ctgaaacaac gtggccccag tgactccacg 14940
ctgtgtgtgc ccctggagac gggggggtgc acaaggtgcg gagccagcta gaacctgtcg 15000
ctccctgcag aagcggtttc tgtgtgcggt tctgatttgc ctcaatgaga aggttttcat 15060
tcatggctcc cggctctcag actgggtgga actgctccca tttaaagggg aaaagaggtg 15120
gctcggctcg ttaaggattt ctttttctaa gttgttacgg cgcccagcag ccggctttgt 15180
ctccccttca gggtggctgc ctttcttccc ggcccctcgc cggcggccct ctctttaaca 15240
aggccgaagt tgtttattct ctcgggatga agtctcggat gggccgccac acccctggcg 15300
gcccgtgggg gcccctctcc ctttgtgcct gggtcggctc ccattcagct cccccgaccc 15360
cccttgttcc cgggcgctca gtggcgcgag atgaggcgat ggggccgaca aagatgccac 15420
actcatccct gccgacctcc ggctcccagc ccagggcccc tggttcctgt gcagaattcc 15480
tcgtgggtgt gacaaaaggc tgcccccagg ctccgctggg gtgggggcca ggccaagagg 15540
cacatcccac actggcccac ctgtccacgg taggcgcatg actgccctga ggaggggagg 15600
ccggcattcc ccgccacaaa ccaggacgta attggtggca gggctctctg tggaaagagc 15660
cagtctgctg tttgtctagg aggtcagtca cagaggcccc gagacgccca ctactgcagc 15720
ctggcaggcg gatgagccca gtatctggca gtgaccagag ggagttttgt gcagaccaca 15780
aaggctgatg ggctgcccta gattggtgtc cctcttggaa gtgggcccag atgtgcggga 15840
cagtccccag gaagccccag gtgagggcac tggtgccctc ttgggaaagc tgctccctcc 15900
tggggcccgg ctcccggccc agtcctccag gggtgtccca tggtgactgg tgctaggaac 15960
cccacacctc ttcccttact tgggaagtca ctggaattgt tgggctacat cagacggccc 16020
agaaaagtgt ttttgtcatc ggccagaaat aggagagttg tgagtagagg gcccgggtgg 16080
agttggggtg tacttggtct gtgctctgaa ggtcactgtg acagtcatgg tcccatggta 16140
aggggcatgg gttgctggaa gagctcttcc ttcccgagtg agccaagccg ggctctcctg 16200
gcgccagggc ctgagccgca gccacaccac agccgccctg aaggctgccg gccagggctt 16260
accccctcaag ggacacggaa tggcttcatc agtaccctgc agccccgtgg cctggcccgg 16320
gtggaggcct aggcttcagc catgcgatgt cccttcagaa tatgacttgt ctgcaatccc 16380
tgctgctggg gggtggcagg tacttggggt gagggttagg gtcatagaag cgacatctct 16440
acgtcctcat atttgcgtca tctaattttg tttttgtgaa tacgtgataa cattcacaag 16500
gctcaagatg ctaaaaggat gagaaggcag tgatgtcccc atcacctgtc ctgtgtcttc 16560
ccgtggcttt ctctttcctt ggttatgttt gagtcaacag tggggctgac gttccaggag 16620
ggtccgtggg ccaggctctt gctctccgag tgcccaggga tggctggagg ctgaggaggg 16680
cctggatgtg gagcctcaga taccaagtgc ttcccttcag gccgggccgc ttgctcagag 16740
ccagcacaca gggatgcccg gatcacgggg gccctgagag ggtcccctgc tcacagcctc 16800
cttccctctc tccttctgcc tcagatgtga agttcatttc caatccgccc tccatggtgg 16860
cagcggggag cgtggtggcc gcagtgcaag gcctgaacct gaggagcccc aacaacttcc 16920
tgtcctacta ccgcctcaca cgcttcctct ccagagtgat caagtgtgac ccagtaagtg 16980
agggtgatgt cccaggcagc cttgccgggg cttacagggg gagacaccta gtgccacgga 17040
aatgccgagg ctggtgccaa ggcccccaag ggtgacaagg ttggggctgg ggctgggccc 17100
ctcggacccc aggccacaga ctgacagggc accggcttct tccactgctc ctagaactta 17160
ctgactggct gggaggtcct cacagccttc tcacgtcccc tggggcttcc aggagccgta 17220
gagtttctgg gcgaagcgtc cgggacggag gccccaggcg gccccagcca atggtctgtg 17280
tggtgatggt gtgtggggtt aggcccaggc gagctttgtt tgggccacaa tgtgcgtggc 17340
caataaatag atgcttgaaa agggctcctg tgaggtccga gacaccggac aacgggcgga 17400
tagagacagc cttgttgttt acggcctctt tgagaggctg ctgctgttaa accctgggat 17460
gactgtgtct ttcttcttaa aaatgccatt gttttattcc cgagtctttt cttaaagaaa 17520
gaattaaaat gacaatcaaa agggtttgtg gcatttacca aattagacca gagaggtggc 17580
cgggtcagcc gccggccccg cggtgtgtga gggagtgacc gcctgacccc agcttggggc 17640
tgggtgggcc tgcaagaccc gttttggctc tggcctgggc cgcctcttgg tggtctgccc 17700
tcgagcctcc cggggactcc gcacgggtct cagcagatgc tatctagggt ccacctgcct 17760
gtcccctgcc tagtggtgcc tctgtcccgg ggacactggg agtagcggct gcccagccca 17820
tgtgtgtctc ggaagaggaa gaagcttttt tgccgtggga caccgaagtt ggcaggggcc 17880
tcccttctgt gttctcggcc atggcctccc ttgcaccctg ccccgtgtta tcctttgggg 17940
gtggtgaggt gtcctcaccc gctgtagggt ggaggccagc agcccgcagc tctctcagga 18000
aaatggctca gaaacaccat cgaggcctcc agaagcccag caaagagaaa gcccctccat 18060
caaaatgaaa ctcgcgtctg cacttttcat ttcgaactcc acgccctgag tgaaaaccgc 18120
ttccccgcca ggggtgactg ccctgggatg ttgctgtctt cgggcagttg tgggaagttg 18180
ggcgctggcc cttatttgag tagagaccat cttaactaga ttggaggcac acgtctcaca 18240
gctgacagac acacggggtg aagttacccg aggcggagtc cactctgcct gatcagctag 18300
tgaccaacgt agctgagccc agactcagaa aaaccgtcca cagcagaggc ccctgcattt 18360
tctagggcgt gttctagaat tttctttggt gggtggaatg tccatctgtg caaatcgggt 18420
gcgcagtgcc acacaccagt gacttttcgc ggaggagcgt gctgcctttt tggagcttct 18480
ggctgtggga gaacagcttt gtccaccggg gtagccttgc aggcagctgt ggggccagag 18540
gaatgaagga aggtcctgga gtctagctgc atgtgtgacc ctggagtggg tcatgggcga 18600
gggacgggcc gcaggtgaag aatccctgga tggagctgcc aggcccctgg ggctgagaat 18660

tgaagctggc tggtgtttta ggttgaacgt caggagtctt gtatctcacc ccaggcctct 18720
ggcctcagtt tccccatctg tacagtggga ctgtttgtgc agccagcccg gccagcttca 18780
tttgccatga tgagaattta tctgaggggc gggagaggaa agccctccct ataaaggtac 18840
aggcgctaaa atgtcatgac ctcagtggtc cacctaaaag tcgttctggc ctgggtcatc 18900
gcctgtcgtg ctatgccttt gtccagcccc ttctggttgg gagttaagtg gcacctgtgc 18960
ggcacgtggt ggggctgtgg cccagccctg ctccttgtgg aaggtctgtt tcctgggctg 19020
cctagagact tggcttgaag ccctagcgtg gcttcctggc agttgggaca cacacagccc 19080
caacacatgg agccggttct ccatccagaa gcccccgggc agtaagcagc cacttcaggc 19140
tgcgtgggac ttgcccgtgg tggagcctag gagaggcccc tggctgggcg tggcgttcca 19200
gatttcacgg ctgctctttc ccactgacag tgtggtgtgg acgctgccaa gggagtctgg 19260
agccccagag ggtggaggtg caggacttcc aggagcgtcc gtcgcactcc acccgagggc 19320
gagcacctca gtggccgcag tgggtggatg catgctgtgc caggctgatg gctggccccg 19380
gggcacaggc ctgagcggga gaggatggag gggagggatc aatggtccag gtccccctgg 19440
ccacccagca ttcatcctca gtcatgcacg gcccaaggct tcgacagcca ttgatcatgg 19500
aaggccaggt tcacctcaag ggctgccaca tggagaggtt aagtctgaaa aggctgaaaa 19560
ggcagggtta aaagggcctc ctgtccagat cagatggcac tgaattcccc agggagctgg 19620
cacggccagt gggaacaggc ggtgaaggcg ctgttggaca tggggacggg caggggggtgt 19680
gcagggtggg cgggcaagca tctggtgtct tgtggctcca gagaccaggt gggaggtgga 19740
ggcgtttggt cctgagtgtc ctgacaggtg atggcagctc ccacatctcg ctcaggttca 19800
gaggaggcag catgggccga gggacagttt ttggcttagt cttgctctta taaaggcttc 19860
cgggtcatgg cacctgggaa ggggccctcg ctgcaggccc cttctaagga ccccctcttc 19920
ccacctctcc ccaccctctc tctctcagga ctgcctccgg gcctgccagg agcagatcga 19980
agccctgctg gagtcaagcc tgcgccaggc ccagcagaac atggacccca aggccgccga 20040
ggaggaggaa gaggaggagg aggaggtgga cctggcttgc acacccaccg acgtgcggga 20100
cgtggacatc tgagggcgcc aggcaggcgg gcgccaccgc cacccgcagc gagggcggag 20160
ccggccccag gtgctccact gacagtccct cctctccgga gcattttgat accagaaggg 20220
aaagcttcat tctccttgtt gttggttgtt ttttcctttg ctctttcccc cttccatctc 20280
tgacttaagc aaaagaaaaa gattacccaa aaactgtctt taaaagagag agagagaaaa 20340
aaaaaatagt atttgcataa ccctgagcgg tgggggagga gggttgtgct acagatgata 20400
gaggatttta taccccaata atcaactcgt ttttatatta atgtacttgt ttctctgttg 20460
taagaatagg cattaacaca aaggaggcgt ctcgggagag gattaggttc catcctttac 20520
gtgtttaaaa aaaagcataa aaacatttta aaaacataga aaaattcagc aaaccatttt 20580
taaagtagaa gagggtttta ggtagaaaaa catattcttg tgctttttcct gataaagcac 20640
agctgtagtg gggttctagg catctctgta ctttgcttgc tcatatgcat gtagtcactt 20700
tataagtcat tgtatgttat tatattccgt aggtagatgt gtaacctctt caccttattc 20760
atggctgaag tcacctcttg gttacagtag cgtagcgtgg ccgtgtgcat gtcctttgcg 20820
cctgtgacca ccaccccaac aaaccatcca gtgacaaacc atccagtgga ggtttgtcgg 20880
gcaccagcca gcgtagcagg gtcgggaaag gccacctgtc ccactcctac gatacgctac 20940
tataaagaga agacgaaata gtgacataat atattctatt tttatactct tcctattttt 21000
gtagtgacct gtttatgaga tgctggtttt ctacccaacg gccctgcagc cagctcacgt 21060
ccaggttcaa ccccacagcta cttggtttgt gttcttcttc atattctaaa accattccat 21120
ttccaagcac tttcagtcca ataggtgtag gaaatagcgc tgtttttgtt gtgtgtgcag 21180
ggagggcagt tttctaatgg aatggtttgg gaatatccat gtacttgttt gcaagcagga 21240
ctttgaggca agtgtgggcc actgtggtgg cagtggaggt ggggtgtttg ggaggctgcg 21300
tgccagtcaa gaagaaaaag gtttgcattc tcacattgcc aggatgataa gttcctttcc 21360
ttttctttaa agaagttgaa gtttaggaat cctttggtgc caactggtgt ttgaaagtag 21420
ggacctcaga ggtttaccta gagaacaggt ggtttttaag ggttatctta gatgtttcac 21480
accggaaggt ttttaaacac taaaatatat aatttatagt taaggctaaa aagtatattt 21540
attgcagagg atgttcataa ggccagtatg atttataaat gcaatctccc cttgatttaa 21600
acacacagat acacacacac acacacacac acacacaaac cttctgcctt tgatgttaca 21660
gatttaatac agtttatttt taaagataga tccttttata ggtgagaaaa aaacaatctg 21720
gaagaaaaaa accacacaaa gacattgatt cagcctgttt ggcgtttccc agagtcatct 21780
gattggacag gcatgggtgc aaggaaaatt agggtactca acctaagttc ggttccgatg 21840
aattcttatc ccctgcccct tcctttaaaa aacttagtga caaaatagac aatttgcaca 21900
tcttggctat gtaattcttg taatttttat ttaggaagtg ttgaagggag gtggcaagag 21960
tgtggaggct gacgtgtgag gggaggacagg cgggaggagg tgtgaggagg aggctcccga 22020
ggggaagggg cggtgcccac accggggaca ggccgcagct ccatttcttt attgcgctgc 22080
taccgttgac ttccaggcac ggtttggaaa tattcacatc gcttctgtgt atctctttca 22140
cattgtttgc tgctattgga ggatcagttt tttgttttac aatgtcatat actgccatgt 22200
actagtttta gttttctctt agaacattgt attacagatg ccttttttgt agttttttt 22260
tttttatgt gatcaatttt gacttaatgt gattactgct ctattccaaa aaggttgctg 22320
tttcacaata cctcatgctt cacttagcca tggtggaccc agcgggcagg ttctgcctgc 22380
tttggcgggc agacacgcgg gcgcgatccc acacaggctg gcgggggccg gccccgaggc 22440
cgcgtgcgtg agaaccgcgc cggtgtcccc agagaccagg ctgtgtccct cttctcttcc 22500
ctgcgcctgt gatgctgggc acttcatctg atcgggggcg tagcatcata gtagttttta 22560
cagctgtgtt attctttgcg tgtagctatg gaagttgcat aattattatt attattatta 22620
taacaagtgt gtcttacgtg ccaccacggc gttgtacctg taggactctc attcgggatg 22680
attggaatag cttctggaat ttgttcaagt tttgggtatg tttaatctgt tatgtactag 22740

```
tgttctgttt gttattgttt tgttaattac accataatgc taatttaaag agactccaaa 22800
tctcaatgaa gccagctcac agtgctgtgt gccccggtca cctagcaagc tgccgaacca 22860
aaagaatttg caccccgctg cgggcccacg tggttggggc cctgccctgg cagggtcatc 22920
ctgtgctcgg aggccatctc gggcacaggc ccaccccgcc ccacccctcc agaacacggc 22980
tcacgcttac ctcaaccatc ctggctgcgg cgtctgtctg aaccacgcgg gggccttgag 23040
ggacgctttg tctgtcgtga tggggcaagg gcacaagtcc tggatgttgt gtgtatcgag 23100
aggccaaagg ctggtggcaa gtgcacgggg cacagcggag tctgtcctgt gacgcgcaag 23160
tctgagggtc tgggcggcgg gcggctgggt ctgtgcattt ctggttgcac cgcggcgctt 23220
cccagcacca acatgtaacc ggcatgtttc cagcagaaga caaaaagaca aacatgaaag 23280
tctagaaata aaactggtaa aaccccagcg tggtgcctgc ctctttgctt cctgggctgg 23340
ccgtgagcca gggacgcgtg tcctggtgcc ctagaaccag ggcagggtgg caggcttggc 23400
ggatgtggga ggccgcagcc tgtcctgtgc gctgtgggaa gttcagcagc atcctgacct 23460
ccatccccgg gatgacagtc acgccacccg ccgtgacaac caagaatgtc tcctgacact 23520
gccacatccc cgggggtggg gacagaatcc agccaggagc aggcacaccc ctcccaactg 23580
ggaggaagcc ctcagcacag gtgtgtgagg tgggaggcgg tgtcctgtcc ccgggaggct 23640
ccagagaata atttgcaggc tgcctggctg ggtgagccca cctccaacca cgcgagacaa 23700
cagctccggc ctgggtgacg tgagcggtgc ccattgatgg ggaacatctt ccccctcttc 23760
cttgccccac cagtttgtct tcccgggtta tttgcagata ggaaaataaa taaagccggc 23820
attcgttaac cctcttctgg cgcaaactgc tgtttgctct ggatgaatca tggtcctttg 23880
gcgacgccag gctccgggag agcaaagcac cgtgtcaggg ccatgatccg gggtggcctt 23940
tcactgggat cgtggggacc tggaggccgc cttataggac acccatgacg cccacctctg 24000
gatttcaggt gcacgtgact ggacttaact tcaaacccca gggtggaggc aggtagtggg 24060
agtgccctgg gaaggtgtcc tcggaccttg gtcactgctc ctgaacccat ctgtgaggct 24120
ggtttgtcct catcccaagc taagtggaag ctcaggtccc aagccaccga tgggtgctac 24180
ttgtcagctg caggttgaat ctccgtggcc tttatgaagc acctgctgtc tacccttcct 24240
gccttgtaga gcactcctcc cagggctcaa cagtggggcc ggggtggtcg gtgtgttggc 24300
tccacaggcg cctgccctgg gaggaaggtg gggtgtggag ggaaacgctt ggcccctgta 24360
ggtctccacc agcctctccc ctgagggtgg gggctccggg agccttcctc gagggagtcc 24420
tatattgagt gggtggggga gcctgcaagg tgcccctgac aggtcacatc agaaagagct 24480
caagggacag tcggagccag aggtgacact ggtggccact cgggtggctc acaaggccca 24540
gctcctcctt gctcctgggc aaattactct gaaggcaggg accaggtctg caccattgcg 24600
gctctccagt tccaggcaat ggccaggtcc tgtgtcaggg ctggggtcct agggaagcca 24660
tgtccccacc cccggcctgc agctgggttt acattcatcc cccgagagca catgggtgta 24720
gcaggaggcc tgtgcagaga gctccgacca tcgcacaggg cacctttggt tgtttcacgg 24780
agcaggcaag ggagccatcg gatcctgtta ggtttgagca aggatgtggg gaagaagctg 24840
gagagccact ttgccatgca gggagaggag cacatgggtc tagggatcta ctttagtgtt 24900
tggaaggttt tttaagatga aagagggatg tgtaggctga taggtctggc agagccaaaa 24960
ggcagcgaca tgtctactgg gagagatgga gctgagcgcg gggctcaggc agggtggcag 25020
ggcagggccg gggccctggg tgggtcaggt gggttcacag ccaagtgtgt agagagggct 25080
tgggcccaga gtgaagcagt tgcaagctct cccacaaccc attctctctg tctcggcatc 25140
tgtggcatcc cgtgatgggt gggtctgtac acacccccacc cctggctgtg ccacaatggg 25200
ggtgtctgta cacccctcac ccctggctgt gccacgatgg gggggtctgt acacccccca 25260
tccctggctg tgccacgatg gggggttctg tacatccccc agtgatgggt gggtctatac 25320
atcgtggtta tgccacgagc tccaaggctg tatcagtccg ttttcacact gctaacaaag 25380
acatacccga gactgggtaa tttataaaga aaaagaggtt taatagactc acagttctac 25440
gtagctggga ggcctcacca tcacggcgga aggcgaaagg cccgtcttcc atggcagcag 25500
gcaagagaga atgagactca agtgaaaggg gaaaccccta taaaaccatc agatctgctg 25560
agacttgttc accaccatgg gaacagtata ggggaaactg ccccatgatt caatcacctc 25620
ccaccgggtc cctcccacaa cacgtgggaa ttatgggagc tacaattcaa gatgagattt 25680
gggtggggac acagccaaac catatcaaag gcatcgtctg gccactctag gcccttgttc 25740
ccaactctca aactccactg acatcacctc tgtgcctgca acccctcctg ggattcagtg 25800
gagacctcgt cccttgttgg agtatctgcg ctacccagag ggtgatggta aaaactgacc 25860
tttgaggaac ttcagagaca aagccttccg cagtcagctc tccctgcagc cccttcgcag 25920
aagcctgtcc tcacacgccc gccaccccta cctgccctgc ctcctggccc atctccctgc 25980
agccagacag tcctcatcca ttcttcatcc tctgccccca acccccagcc ttcctatctc 26040
ccttcagctc cgtccaaggt ccctgggact cagccccatc tctccccagc cttgacccca 26100
ataagctcga tgtctttgcc caggagctcc tgacacacca gactgcatcc agcttcctgt 26160
tctgcgcttg gcccaccgcc tcggtccaca ggaaccttca tctctgctct gactgtaagg 26220
atgggctctc tgagggcttc cccaccccac agcaggcagg tggttctggg agtctgggat 26280
tgtcatgcct gggcaccttc ctgctctggc tgagcccctc caggacggaa atctcccaga 26340
tttctctctg cctccgtctc cccaccctgg catacagtgg gtcctaggaa gggggccgaa 26400
tggaagggag gtcgtgtcgg ctcctacagg gcagccccaa gctgctgggc tgaggtgcag 26460
agccatgggt ccagcctgac ccgcatgccg ggagctgatt ccatggctgg aagggtcggg 26520
ggttccccag gagcccagac agcacctaga gttgtcacaa ggagtgaaag gccctgaggg 26580
gacagaggaa ggaggctggg cagagtgggc acgtggactt cggaccgcgc ttgtgggata 26640
acagtggcta gggctgtgca gagggtggtg cactgcccat gggagtccag agaagccagg 26700
tccagtggca gtggcatgca tccggcaatc cctccggcta ccctgcatgc agcgtgaccc 26760
tccttgaagc accccaggaa gcagtcacag cctcgctctg cccagcattg ggggcctggc 26820
```

ctctgaatct ggtggggaag gctgtgttgc ctgctggcac ccgctctatc accagtccca 26880
ccataaggct agtccccaaa gtcagcactg cagccagggc cagcgagggc cagcaagggc 26940
aggcctgtgc aggggctgca gcgtggggtg tgcgtcccgc actctgggcc agcctcgcag 27000
ctcacagggc tctgtcatcc caggaatgtc tttgatcaag agtttatgca agacgaggaa 27060
agcaagccca ggacctctaa tccgttccca cagtcccctg cccgccaggg ccccacaagc 27120
ccagcgccac accgccaggc ctgagcagac aatagcaggc ggacgggtgg gcgggcagct 27180
ctggggccgc tggccttggg gcccacatcc gggagcattg tcaccgccac tcacgagggc 27240
acacaaggag ggctggggaa gagagaatgc agagccctct gctgaagggg cttcggtggg 27300
ccttgagcct ttcaaagacg gcggtgtcca ttaatccttt taatttgcac ctagcggcag 27360
ctgagacaag cctctcaacc ggcagccact ttaagagctg agtggtctaa cccgaggatc 27420
agctcctatt ccagaccctg ggccctctga gcctgtcgag gcagtcctcc ttgcctttgt 27480
ttaatcacgg gaggaatcgc agctggaggt tactgagcac gcactgtgtg ccttgtgctc 27540
aacaccgaac acagtttctc tctgagtttt gcaacgaccc catgggttgg tgacagggag 27600
gggacactct ggtggcagcc acagacacag ccctgccctc ccagagccta cactctcccg 27660
cacacaatca tcccatctgc taggtcaggg attggcaggt tttctggaaa gggccacaga 27720
gagaggcttc aggctttgtg ggccacaggt tctcttgccc tgtgcaaaag cagctgcagc 27780
cgctctgtga atgagtgagc atggctgtgt gccaataaag ctttatttac aaaaataggc 27840
agcagactgg attcaggctg agggtggtag tttgccgacc cctgttgtag atgagtaaac 27900
cgaggcccag agaggtaaag taactgagcc aggactgcac agctagtgtg tgaccaagcc 27960
agcctggtgg gtcagcctca tggagaagaa aaggctcaag cttcaaaaag ggaaatgcac 28020
ggaggcttca ttgggccatt tgcagccagg ccccacgcac acagctcagg gccctgcttc 28080
gccagctcca cctgtttcga ggctcccagt aggttgcccc tgcgggcggc actgtgtctt 28140
ggctgctggc ctggagaagg gcagccaaac aggccggcag tggacatctg agggtgacca 28200
gctgtttaaa aagaaaactc accggcatgt ataggcacac attaagcaca cacgttttgg 28260
agtaaaaggc cctggggttg gatctcggat ctgcagcttt ccagctgtgc ggcctcattt 28320
ctctgggcct caatgttcac acctataaca tggggaaatc ctgcaagaac acagcccatg 28380
atggggagtc gtctccatct cctactcacg acaacgacca gcacagaacg gggctccat 28440
aaatctctgt caataactga attctttctg ggtgctgag gattctatgg ctcaatgtgt 28500
gtgaagcact gctgagcacc gagcttgggg tgcagtgatc actcagggaa cgccatcatc 28560
ccctcggcgc atccgcataa tcaccatcag cattccatgc actgcccagg tctggaagca 28620
caggcctcca tgccacggcg gccgcctggg tggtggcacc aaagccgatc ttacttccct 28680
cctttggcac attaagtaga cagttctggg tcttcttgac accacggatg ccccctcctg 28740
gtgttgacag cacccagact ttgctccagg gaaccaccat tcccagatct gtggtttgag 28800
tgggaccgaa cccatcccca gctccaggcc aggtcttcat aggctgaaac ccaactgcac 28860
atccacaccc accagaagag cagcaagtta atggatgggt gagaaatcaa atagagccat 28920
tgagtcttga ggagacattt tctggagctt ctgaaaaaga taatgcttcc cccttcctta 28980
ggattcccag cagggaaaca caagcccagg ggtggcactg cccccctgca gccctaaggg 29040
aagactgagc tgctcccagg acctcacagg ggaggctgag cgtaaggcca gccctgaggg 29100
aaagcaggga atgggtctcc agacaaactg ctggatcaag cctctcctga agtcatcccc 29160
cttttgttcc agaagccaat cattgtcctt cattaagtca gtttgatatg ggcactgtgt 29220
catttggttt tcaaagacac ttaactatta cagcttgtgt gtgttttctt tttttttttt 29280
gagacggagt ctcactctcg cccaggctgg agtgcagtgg cctgctctcg gctcactgca 29340
agctctgcct cccggattca cgccattctc ctgcctcagc ctctcgagta gctgggacta 29400
caggcgcccg ctaccacgcc cagctaatta tttgtatttt agtagagacc gggtttcacc 29460
gtgttagcca ggatggtctc gatctcctga cctcgtgatc cacccgcctc ggcgtcccaa 29520
agtgctagga ttacaggcgt gagccaccgc acccggccgc ttgtgtgtgt tttctacctc 29580
ttctagatct aacatgttat gattgatata aacaacagga gcgactgaca aagttaccat 29640
gtgatcttgg aatttcaaca tgcctgtctt gcttagctgg gagttccaca gggtcaggga 29700
atgactcctc ttggggccac cagtgcccag aagtatgcct agaactgctc tgtctgaagg 29760
gacaggtccc tcttccatgg cacacactct gtgtgtaaca gagcacacgt gtgaccaaac 29820
tgacctggag aaagggcctg accacatcca tatcccaagt tctgaggccg gatagtgcct 29880
gcaggggctg agaccagggg agaggctcaa gccagccttc tctggtggac cctgactcag 29940
tcctctgtgc agggcagctg cgcctcccag aaggcagagg cctcttggag agtgagcctt 30000
gcggtcacag cgcctttcgg gggctcccgc cacttgccct cagtggcggc tgagacctgg 30060
gggagtccag gcaaatccct gaggaggccc cacatgcaag gccagaagtg tctggatggt 30120
gtggccggca cccttaccct ccagaccagc aaacgaagcc caggcaggga gaggggcctt 30180
gtagtttttct gtggctgctg taatgaattt tcacacactc agcagcttaa aacaaccaac 30240
atttctctca tagttccaga ggccagcagt gtctgcagtg cccgctctct cccagcttct 30300
ggtggtttcg ggcgaccttc ggctcttctc agctggtcca tgtgca 30346

<210> 17
<211> 1325
<212> DNA
<213> Homo sapiens

<400> 17

gcagtagcag cgagcagcag agtccgcacg ctccggcgag gggcagaaga gcgcgaggga 60
gcgcggggca gcagaagcga gagccgagcg cggacccagc caggacccac agccctcccc 120
agctgcccag gaagagcccc agccatggaa caccagctcc tgtgctgcga agtggaaacc 180
atccgccgcg cgtaccccga tgccaacctc ctcaacgacc gggtgctgcg ggccatgctg 240
aaggcggagg agacctgcgc gccctcggtg tcctacttca aatgtgtgca gaaggaggtc 300
ctgccgtcca tgcggaagat cgtcgccacc tggatgctgg aggtctgcga ggaacagaag 360
tgcgaggagg aggtcttccc gctggccatg aactacctgg accgcttcct gtcgctggag 420
cccgtgaaaa agagccgcct gcagctgctg gggccactt gcatgttcgt ggcctctaag 480
atgaaggaga ccatcccccct gacggccgag aagctgtgca tctacaccga cggctccatc 540
cggcccgagg agctgctgca aatggagctg ctcctggtga acaagctcaa gtggaacctg 600
gccgcaatga ccccgcacga tttcattgaa cacttcctct ccaaaatgcc agaggcggag 660
gagaacaaac agatcatccg caaacacgcg cagaccttcg ttgcctcttg tgccacagat 720
gtgaagttca tttccaatcc gccctccatg gtggcagcgg ggagcgtggt ggccgcagtg 780
caaggcctga acctgaggag ccccaacaac ttcctgtcct actaccgcct cacacgcttc 840
ctctccagag tgatcaagtg tgacccagac tgcctccggg cctgccagga gcagatcgaa 900
gccctgctgg agtcaagcct gcgccaggcc cagcagaaca tggaccccaa ggccgccgag 960
gaggaggaag aggaggagga ggaggtggac ctggcttgca cacccaccga cgtgcgggac 1020
gtggacatct gaggggccca ggcaggcggg cgccaccgcc acccgcagcg agggcggagc 1080
cggccccagg tgctccacat gacagtccct cctctccgga gcattttgat accagaaggg 1140
aaagcttcat tctccttgtt gttggttgtt ttttcctttg ctctttcccc cttccatctc 1200
tgacttaagc aaaagaaaaa gattacccaa aaactgtctt taaaagagag agagagaaaa 1260
aaaaaaaaaa aaaaaaaaaa aaaaaaaaaa aaaaaaaaaa aaaaaaaaaa aaaaaaaaaa 1320
aaaaa 1325

<210> 18
<211> 888
<212> DNA
<213> Homo sapiens

<400> 18
atggaacacc agctcctgtg ctgcgaagtg gaaaccatcc gccgcgcgta ccccgatgcc 60
aacctcctca acgaccgggt gctgcgggcc atgctgaagg cggaggagac ctgcgcgccc 120
tcggtgtcct acttcaaatg tgtgcagaag gaggtcctgc cgtccatgcg gaagatcgtc 180
gccacctgga tgctggaggt ctgcgaggaa cagaagtgcg aggaggaggt cttcccgctg 240
gccatgaact acctggaccg cttcctgtcg ctggagcccg tgaaaaagag ccgcctgcag 300
ctgctggggg ccacttgcat gttcgtggcc tctaagatga aggagaccat cccccctgacg 360
gccgagaagc tgtgcatcta caccgacggc tccatccggc ccgaggagct gctgcaaatg 420
gagctgctcc tggtgaacaa gctcaagtgg aacctggccg caatgacccc gcacgatttc 480
attgaacact tcctctccaa aatgccagag gcggaggaga acaaacagat catccgcaaa 540
cacgcgcaga ccttcgttgc ctcttgtgcc acagatgtga agttcatttc caatccgccc 600
tccatggtgg cagcggggag cgtggtggcc gcagtgcaag gcctgaacct gaggagcccc 660
aacaacttcc tgtcctacta ccgcctcaca cgcttcctct ccagagtgat caagtgtgac 720
ccagactgcc tccgggcctg ccaggagcag atcgaagccc tgctggagtc aagcctgcgc 780
caggcccagc agaacatgga ccccaaggcc gccgaggagg aggaagagga ggaggaggag 840
gtggacctgg cttgcacacc caccgacgtg cgggacgtgg acatctga 888

<210> 19
<211> 25032
<212> DNA
<213> Mus musculus

<220>
<221> misc_feature
<222> (1)...(25032)
<223> n = A,T,C or G

<400> 19
cttccttctc ccttcactga aactgaactg accttgggat ggggaaaccc ccacttcatg 60
gcacccactc taaaagatgg ctgttggtca aacagcctag caagctctaa accaggtact 120
ctgcacctgc cctctgacgc cctgggatta tatggcagag ggtaagaaca ggaatgacat 180
aattaaaacc agcctcagaa gctgctgaag gcattagaac taaagcccag aaaagcaaca 240
aactgttacc atcaaagcgt gaaagaggaa acggttaatc ttttcaaggg aagtgaagca 300
actcggaagg ccataggaat ccacatcacc tgaactgcag accaagcctt ggaactgggg 360
ctttcaagct acatctgcta ctcacacctt tgacctctct gggtgaattg ttttcttact 420
agtcaccaat gttttcaaac ctggattttc acactccctg gaatgcctgg aagggctgct 480
accagcggac ctatattgca gggccaccaa ctgccaggtt ctgagccaca gctcttagct 540
aggaccaagc aaatggtccc cagtgtgtta cagtctcctg tcctccaaag tgagttcctc 600
agatgcactc ctttccaatc ctnnnnnnnn nnnnnnnnnn nngattttcc tgtgggcaca 660

```
tcagcttcct atgctgaaaa aaacagagag tagtagaaag ggaaagccta atcaagcctg 720
cagctgagag attgccctgt gccacaaccc ccaagtccac ccccttatag gacagcacct 780
tagtcattca gggaaagcct taatggattg ccttgattcg ccttgattcc ataatgtaat 840
tgaccatgag tagagcaagc tgcaccaact cctatcgtaa atgaagacag ggtgagact 900
ggaagggtca gatactggaa aatctgagat tctctgcctt ggtttctcat cttgatcagt 960
ttcaaccaaa aaagaaggaa gctttaagca ggactccttg tttttgtcaa aagaatatgc 1020
taaagtacac attcccatta ttgctcatga gaccttgttc atatgtcacc aagaatattc 1080
agtggtatat gtttgttcat accttcttaa acataattta agatttcttt atttgatgta 1140
tgtgttttgc ttgcataatg tatatgtacc acgtgtgtgc ttgtacctgc acaggtcagg 1200
agaaggtttc agtttccctg gattgatgac tgtgaactat catgtgtatg ctgtgaactg 1260
aactaaggtc ctctgtggaa gcaacaagtg ttcttaactg atgatccatc tctccagctt 1320
ccgctcagaa attttcacac ttaaagaaat aagggctggc tggtgagatg gctcagcggt 1380
taagagcacc gactgctctt ccaaaggtcc tgcgttcaaa tcccagcaac cacaaagaaa 1440
taagggcagg ggctagagag atggctcagc agttaagagc actgactgct cttccagagg 1500
tcctgagctc aattcccagc aaccacatgg tggttcacaa ccatctgtaa tgtgatctga 1560
tgtccttttc tgatatgtct gaagacagct aaattttact catatacata aaataaagaa 1620
ttctttagaa agagagaaag gaaggaggga agggaggaag gaagggagag agagagagaa 1680
agaacggaag ggagggaggg agggagggag ggagggaggn nnnnnnnnnn nnnnnnnnnn 1740
nnnnnnnnnn nnnnnnnnnn nnnnnnnnnn nnnnnnnnnn nnnnnnnnnn nnnnnnnnnn 1800
nnnnnnnnnn nnnnnnnnnn nnnnnnnnnn nnnnnnnnnn nnnnnnnnnn nnnnnnnnnn 1860
nnnnnnnnnn nnnnnnnnnn nnnnnnnnnn nnnnnnnnnn nnnnnnnnnn nnnnnnnnnn 1920
nnnnnnnnnn nnnnnnnnnn nnnnnnnnnn nnnnnnnnnn nnnnnnnnnn nnnnnnnnnn 1980
nnnnnnnnnn nnnnnnnnnn nnnnnnnnnn nnnnnnnnnn nnnnnnnnnn nnnnnnnnnn 2040
nnnnnnnnnn nnnnnnnnnn nnnnnnnnnn nnnnnnnnnn nnnnnnnnnn nnnnnnnnnn 2100
nnnnnnnnnn nnnnnnnnnn nnnnnnnnnn nnnnnnnnnn nnnnnnnnnn nnnnnnnnnn 2160
nnnnnnnnnn nnnnnnnnnn nnnnnnnnnn nnnnnnnnnn nnnnnnnnnn nnnnnnnnnn 2220
nnnnnnnnnn nnnnnnnnnn nnnnnnnnnn nnnnnnnnnn nnnnnnnnnn nnnnnnnnnn 2280
nnnnnnnnnn nnnnnnnnnn nnnnnnnnnn nnnnnnnnnn nnnnnnnnnn nnnnnnnnnn 2340
nnnnnnnnnn nnnnnnnnnn nnnnnnnnnn nnnnnnnnnn nnnnnnnnnn nnnnnnnnnn 2400
nnnnnnnnnn nnnnnnnnnn nnnnnnnnnn nnnnnnnnnn nnnnnnnnnn nnnnnnnnnn 2460
nnnnnnnnnn nnnnnnnnnn nnnnnnnnnn nnnnnnnnnn nnnnnnnnnn nnnnnnnnnn 2520
nnnnnnnnnn nnnnnnnnnn nnnnnnnnnn nnnnnnnnnn nnnnnnnnnn nnnnnnnnnn 2580
nnnnnnnnnn nnnnnnnnnn nnnnnnnnnn nnnnnnnnnn nnnnnnnnnn nnnnnnnnnn 2640
nnnnnnnnnn nnnnnnnnnn nnnnnnnnnn nnnnnnnnnn nnnnnnnnnn nnnnnnnnnn 2700
nnnnnnnnnn nnnnnnnnnn nnnnnnnccc tcctcctagc tttctgaagc cagtcttctc 2760
cggtttgcct tcagaacaag atgtaaaact ctcagctcct ttagctccat gcccggactg 2820
gatgctgcca cacttcctgc cttgatgata atgaactgaa cctctgagcc tgtaatccag 2880
ccccaattaa acattgtcct ttataagagt tgccttggtc atgatgtctc ctcacagcaa 2940
tggaaaccct aactaagaca gcaagtttag cagtaaatgg ggtggcagag acagggtgat 3000
agcaggaaat actttgacat agctagtgag acagcaggta atgacagtag ccaaaccagc 3060
ctgataaaca aacagctagc ccagaatgca tagcacagca gcaaaaacat tggagaactg 3120
gctcaatgac aaagtggaag gaggaaaggc aattcctaaa tgttgtcatc tgatcaccat 3180
atctgcacac tgaagcacac acacacacaa ataaacaaca ctttaaagaa ataaatatta 3240
catgatgatt gcaacaacca caggccatcg gaaaagaaag aggaaaaaaa atcaatggag 3300
caaagagacc aggctgcaca cacaggagga acatcaggag acccaaattc acccaaatgt 3360
tctcaatgtc agcatgtttg catctaggtc ccaacactct aaggggattg gcaggatccc 3420
tgaccccgac tcactaaata ccaataggaa gccccaaccc ccaccaccta taacaacctc 3480
aaatatctcc tcctgttggt caatgtcccg tggaagcaac attacctcta gttgagaggt 3540
actgatctct agaatccaaa agtgtgagca tttccagaag ctttcccagc aagcaacaaa 3600
aaagagatgg tctcacggtc accggttgtg gggaagatag cttgagtgcc tttagggagg 3660
ttcattcaat gataaagtcc acatacagta gtcaggaggg aaatggagaa ggtgaaagtg 3720
agagcaagca aggctgagct gcactcaaaa ttctacctac agaagtcaag agctgtgacc 3780
catagtttgg ggtctgttct agccaataag aggcacaggc tactattgca ctggcttcta 3840
aatgaaagtc aaagattacg gacagaaaaa aaggaagaaa aaaaagtaaa aaagaaagca 3900
agacccgtca tgcacctcct ccccagtcac ctttaccccg actcagcaag ggtccatcat 3960
ggagacagat gccgcccaaa aggaccatca caaaggccgg ccagtgaaca aaagtgcaag 4020
attgacttgg gtttgccatt tacagaacaa taaacgaagc ctttgaagca ctgttaaaca 4080
aaagagccat ttgtctatgc tggtggctac aaaggagaac aggattgcag cagaggacta 4140
agaaaagaaa gagaaaatgc caaactgaga tgtgcctgga ctttaccggg tcctgcccag 4200
agaccttata tgccaggacc taagatctaa aagccactgg gtaagaaaac aatcaaatat 4260
gcttaatgca atacaggcaa tgtaggttct tgggtattgt gttaaaaata tctactaatt 4320
tttttccttt gtcagctgct ttaatttatg cagtatggga agggatcaca tcggaaaaga 4380
caagggaaga acagaaaaga aacaccgtac attttcaaat gcacaaagat ctcatgcact 4440
gccttctgtt tctgtgccta tgaattagtg atgctaaatt ttatcttatt ttcttgcctg 4500
gtgggggggg gggggagggc ggggagggtg actgtgttcc tctctgtggt ggcatagctg 4560
ccatttacat gcctgtgagg tcctcttttc ctttagcatg ccatggctag cttggttttt 4620
aatctaacag cgcttctttc caaatgatgt ttctgcggca gacaggtatt tatcattgtc 4680
tttgctctcg tcacttcccg acaggactcg tgagaactga agtaattttg aaatcgtagc 4740
```

ccgtccttag ggtctcctgc ttctctcccg agtaggagct aggtttttga tccagtttgt 4800
gtggatttag tgactgtttt tatacatgga aggttagctc agtggtgatg gttggggggg 4860
ggggctcttt gaagcatgca gaaaagagat tcaagaacat tgggggatgt tatctagtgg 4920
ttttctcctt gttgttacaa gagtgacttt ggagaagagg ggtatacttt ggctcagagt 4980
ttgacggaaa aatctgggat ggaagaagtc attgtgggag cagctggaag ctttgtcact 5040
tggggagcct tgtcatactc tagccaaagt taggaagcag gttggagggt ggggagacag 5100
attctggagt ttggcgcttc tgttttatac cttctctacc cacattgtga atagacctga 5160
cccctgggtt gacacagatc acattagtgt gagtaggtct tccttctaca catatacctc 5220
tctagaaata ccctcatagg taacaccaga tctgcatctc ctaagtgatc aagtcgacaa 5280
tcagaactaa ccatcatagg cagggctgca acacaccttg aatcccgtga cttaagaggc 5340
aaagacaggt ggctctctga gttcaaggcc tgcctggtct acagagtttc aggacagtca 5400
gggaaaccct accttgaaaa acaaaaaaca aaaaacaaaa aacaaaaaaa aagaaaagaa 5460
aagaaacacc caaattaagc gtctcaagga tgaccgttct tggtatgtta ttcacaccac 5520
atatttggac ctccccctctt gtgtctttgc aaagtaagat gttgggccga cttgttcatt 5580
ctagctggga atatacagtt cagtgactga atgaatgctg accacaaatg aggccctgtt 5640
tcaatactga gcacaccttc ctctccttcc cataacagcc ctttctttct ggaggtcttc 5700
cttttttct taaaaatgta agagcatcat gactgttagt atgagacagt gaggttctgg 5760
gcactgtgat tcttagtttt tcatactgta agagggaatg tactctctgc catcgggaca 5820
cccagtggaa ctgctcacct ggagtcttgc ctccacgaag actaggatct tctggaaagc 5880
tgtgtgcttt ggacatctaa ctcacatcca gcaatagctc ccagaaaagg acctcagagt 5940
tacaacgttc cagaggaaca aaacagtggg acatcttcca ccatcttcca aaaggaaacc 6000
tcagaacaga acaagcaacc tttgtcctta gtaagcttgt ccacagaggc acgcatggct 6060
gtcatccgaa cactgggaag tccaaggcaa gcgaacagca agttcaaaac tagcttgaac 6120
tggctgcata gtgaggccct atctcaaaac atcacgggct aactatgtag ctcagagaca 6180
aagcccttgt ccagtgtttg aaagactctg gatttgattc ccaacaataa aaacaacaac 6240
atacactcag atactcacac acacacacac acacacacac acacacacac acccaactcc 6300
ttaaacacta aaatttagtg taggtagagt ggcacaggaa actctaattc cagcactaca 6360
aaccctgaag cagtttttat tttagttaga agccatccta ggctaccttc tcaacaagaa 6420
cattccaatc actacacttt attgacaaga ttataaactt gtggctttcc tgtccttttt 6480
aaagtatttt ttaaaattta ttttacaatg aagtggtacc ctttcatttc ctaatatgta 6540
aaatggggat gtcccctacc ttcctgttac ttaaaagcaa caccccgggt gatgtcatca 6600
ggctggggta cagtacgggc aagtccccaa accacaggct attccgctac ctgggttccc 6660
acacctacac tggaccggca tgatttggac gtaagatctt aaaataatcc ataattacac 6720
cttttcaaaa aggacctttt gagggtcagc tctacccact gtcatatctg ttggtgctgt 6780
cctgtgttta gggagaggca gagagaaagc gggtgtgcgc aacctctaag agagtaatag 6840
taaagagaca agcctaggaa atcttcccca agaggccttt tggggaaggc tatccccccct 6900
tccgtggatg ttgaatacct tgcctgttgc tcagatgcag tcaagcatct gatgatgctt 6960
gaggcatgat gctgagacca ctgtgtaggt tggtacaaat tccctttcta ttatacccac 7020
ccttatgcag ttatttgggt ttaaattttc aacgttttaa aaaaaaaaaa aaaaagtatt 7080
gccgggcaat agtgacgcag gcctttaatc ccagcactcg aaagacagag gcaggcagat 7140
ctctgagttt gagaccaacc tggatggtct aaggccttga actcaagaga tctatccacc 7200
agcttgtatc ccatttaatt gagtgatttg tttgctctct ctctctcccg ctccccctct 7260
ctccccccctc tccctcccct ttttcagttc tttatttata tttgaatatt atctctctct 7320
cagatgtgta cttggtgacg gtcttttcta caaaggatta tctgtttcat gacacgccat 7380
ttattaatct gcttgtccct ggagcttctg tacagtagcg tgacctgatc ctctagcttc 7440
tccatcccag atgattggga ttcaaggcat tcatcaccac gcctacctaa aattccaacc 7500
ttttaataaa atggctctct aaaatatttt ggcaaaggaa tctcactctt tctcttcact 7560
aatatagttc aaccacccaa ttcgtaagca gtcgggctga gggtgagcat ccttaagaac 7620
ataaaagcaa aatttggagg ctcaagattc agtttagttg ctagagggct cacatagcat 7680
gccctcccca cccgggattc cattctcatt tatcgaggca taaggccagg tgtggtggga 7740
tatgtgctgg gatgcataag atcttttata aagaagagga agaggaaaaa cagttataca 7800
aaactaataa aactgtgtga gtttcaggct agcaaagaat agtcgtgaga atctctctca 7860
aaaaaaaaac cccaaaataa taataatgat aataatgata ataataataa taataataaa 7920
acaaggcaat aataagctaa tgctcctgcc ttgcattctg actccttttg cccagtaaaa 7980
ttcaatgctc tgctttgaca cgtccagctt acaacacgac aaaggtgtga gacatgggtg 8040
ctaaaacttg ttctattgcc tttccgtttc tgttagatct ccttcacttg tatacctgtg 8100
actcattcat tttccccatc cacaactagg gctctgtttt gggtgactgt cagaaagtag 8160
ggatgggttc atctactccc cctctttgca actgaatagc cacctctgaa ccattttttt 8220
ctctagtaat ttctcttcct ttgcctcctt tgcagcctaa aagagtcatt taaaggatga 8280
ccggaagctt gtcttaggca aggaagcatc ttcccagaac ctggaaaccc tgcagccctg 8340
cccccatccg acctccgccc tcgttggctt cgcaacgctg tggtctctgt ggccagtaga 8400
gggcacactt actttacttt cacaaatccg agagccacaa cccgggtggt gggggggtgag 8460
ggggcgggga aagagtctct gcagcaaaac gcagactagg gattggtggc tcttggtgtt 8520
tgaggcaaaa tcctagaggc tgtagtcatt ttgcaatcct taaagctgaa ttgtgcaatg 8580
agctcgatga aggaagatac tatcattcaa cagctgaatc ctaaattgca aactcagtgg 8640
ctaataacaa ctttgaacaa tgagcacctt atacacgcta ctgtattttc ttttctttct 8700
tttttttttt tttttttttt tttaaaccgg gtagcagtga gagaggtttc tttaagtgcc 8760
ttggggcgag gagtccggaa taagaagact tctttgggtt ttaaagtgta ggataagcaa 8820

atcccgaggg aatatgcatt atataataaa tctagaacca atgcacagag caaaagactc 8880
atgtttctgg ttggttaata agctagatta tcgtgtatat ataaagtgtg tatgtatacg 8940
tttggggatt gtacagaatg cacagcgtag tattcaggaa aaaggaaact gggaaattaa 9000
tgtataaatt aaaatcagct tttaattagc ttaacacaca catacgaagg caaaaatgta 9060
acgttacttt gatctgatca gggccgactt ttttttttaa gtgcataatt acgattccag 9120
taataaaagg ggaaagcttg ggtttgtcct gggaggaagg ggttaacggt tttctttatt 9180
ctagggtctc tgcaggctcc ccagatctgg gttggcaatt cactcctccc cctttctggg 9240
aagtccgggt tttccccaac cccccaattc atggcatatt ctcgcgtcta gcgccttgat 9300
tttccccacc ccagctccta aaccagagtc tgctgcaaac tggctccaca ggggcaaaga 9360
ggatttgcct cttgtgaaaa ccgactgtgg ccctggaact gtgtggaggt gtatgggggt 9420
gtagaccggc agatactcct cccggaggag ccgggtagag cgcacccgcc gccactttac 9480
tggactgcgc agggagacct acaggggaaa gagccgcctc cacaccaccc gccgggtgga 9540
agtccgaacc ggaggtgctg gagtgtnnnn nnnnnnnnnn nnnnnntgac gcggtccagg 9600
gtacatggcg tattgtgtgg agcgaggcag ctgttccacc tgcggtgact gatatacgca 9660
gggcaagaac acagttcagc cgagcgctgc gcccgaacaa ccgtacagaa agggaaagga 9720
ctagcgcgcg agcaagagaa aatggtcggg cgcgcagtta attcatgctg cgctattact 9780
gtttacaccc cggagccgga gtactgggct gcggggctga ggctcctcct cctctttccc 9840
cggctcccca ctagcccccc tcccgagttc ccaaagcaga gggcggggaa gcgagaggag 9900
gaaaaaaaaa tagagagagg tggggaaggg agaaagagag attctctggc taatccccgc 9960
ccacccgccc tttatattcc gggggtctgc gcggccgagg acccctgggc tgcgctgctc 10020
tcagctgccg ggtccgactc gcctcactca gctcccctcc tgcctcctga agggcagggc 10080
ttcgccgacg cttggcggga aaaagaaggg aggggaggga tcctgagtcg cagtataaaa 10140
gaagcttttc gggcgttttt ttctgactcg ctgtagtaat tccagcgaga gacagaggga 10200
gtgagcggac ggttggaaga gccgtgtgtg cagagccgcg ctccggggcg acctaagaag 10260
gcagctctgg agtgagaggg gctttgcctc cgagcctgcc gcccactctc cccaaccctg 10320
cgactgaccc aacatcagcg gccgcaaccc tcgccgccgc tgggaaactt tgcccattgc 10380
agcgggcaga cacttctcac tggaacttac aatctgcgag ccaggacagg actccccagg 10440
ctccggggag ggaatttttg tctatttggg gacagtgttc tctgcctctg cccgcgatca 10500
gctctcctga aaagagctcc tcgagctgtt tgaaggctgg atttcctttg ggcgttggaa 10560
accccggtaa gcacagatct ggtggtcttt ccctgtgttc tttctgcgtc ttgaatgtag 10620
cggccggtta ggacagtctt tcttccattc ctgtgctttt gacacttttc tcaagagtag 10680
ttgggggtagg ctggggtaga tctgagtcgg ggtagagcga cttgtcaaga tgacagagga 10740
aaggggaagg gaaaaaccgg gatgcatttt gaagcggggt tcccgaggtt actatgggct 10800
gacgctgacc cggccggttg gacattcttg ctttgctaca ttaattgata tgtgtccttt 10860
gaggggtcaa accgggaggt cgcttcgtgg tggccaaaga aagcccttgg aatcctgagg 10920
tctttggaga agggattacc ttttgcgttt gggagcgaga aggctccgta gcttctgact 10980
taccagtctc tgagagggca tttaaatttc agcttggtgc atttctgaca gcctgggacc 11040
gacacggagg tgcgtcccgc ccgccaatcc ccggcggcga tcgcaacccg tccctgagcc 11100
ttttaagaag ttgctatttt ggctttaaaa atagtgatcg tagtaaaatt taagcctgac 11160
ccccgcggca ctaggacttg atgttgggct agcgcagtga ggagaagcaa aattgggaca 11220
gggatgtgac cgattcgttg acttggggga aaccagaggg aatcctcaca ttcctacttg 11280
ggatccgcgg gtatccctcg cgcccctgaa ttgctaggaa gactgcggtg agtcgtgatc 11340
tgagcggttc cgtaacagct gctaccctcg gcggggagag ggaagacgcc ctgcacccag 11400
tgctgaatcg ctgcagggtc tctggtgcag tggcgtcgcg gtttagagtg tagaagggag 11460
gtgtctctta ttatttgaca ccccctcccc ttttatttcg agaggcttgt gatagccgga 11520
gactgagctc tctcctccaa gtcagcaatc ggaaagaaaa gccggcaaag gaaggaaggg 11580
ggcgcgctgg gggtggagaa agaggagggc ggagagggggc ggcggcgccg gctgggtagg 11640
agcgcggcga cggcgcgaat agggactcgg acccggtcgg cggcgcagag agccggcaca 11700
cgggaggggg ccgagcgacg cggcgcctct cgcctttctc cttcaggtgg cgcaaaactt 11760
tgcgcctcgg ctcttagcag actgtattcc ctacagtcgc ctccctcagc ctctgaagcc 11820
aaggccgatg gcgattcctg ggcgtctgca gggctaagtc cctgctcgaa ggaggcgggg 11880
actcggagca gctgctagtc cgacgagcgt cactgatagt agggagtaaa agagtgcatg 11940
cctccccccc aaccacacac acacacacac acacacacac acacacacac acacacacac 12000
ttggaagtac agcacgctga aaggggagtg gttcaggatt ggggtacgcg ctgcgccagg 12060
tttccgcacc aaccagagct ggataactct agacttgctt ccccttgctgt gccccctcca 12120
gcagacagcc acgacgatgc ccctcaacgt gaacttcacc aacaggaact atgacctcga 12180
ctacgactcc gtacagccct atttcatctg cgacgaggaa gagaatttct atcaccagca 12240
acagcagagc gagctgcagc cgcccgcgcc cagtgaggat atctggaaga aattcgagct 12300
gcttcccacc ccgcccctgt ccccgagccg ccgctccggg ctctgctctc catcctatgt 12360
tgcggtcgct acgtccttct ccccaaggga agacgatgac ggcggcggtg gcaacttctc 12420
caccgccgat cagctggaga tgatgaccga gttacttgga ggagacatgg tgaaccagag 12480
cttcatctgc gatcctgacg acgagacctt catcaagaac atcatcatcc aggactgtat 12540
gtggagcggt ttctcagccg ctgccaagct ggtctcggag aagctggcct cctaccaggc 12600
tgcgcgcaaa gacagcacca gcctgagccc cgcccgcggg cacagcgtct gctccacctc 12660
cagcctgtac ctgcaggacc tcaccgccgc cgcgtccgag tgcattgacc cctcagtggt 12720
ctttccctac ccgctcaacg acagcagctc gcccaaatcc tgtacctcgt ccgattccac 12780
ggccttctct ccttcctcgg actcgctgct gtcctccgag tcctccccac gggccagccc 12840
tgagccccta gtgctgcatg aggagacacc gcccaccacc agcagcgact ctggtaagct 12900

```
accccattca cagcagggta ggaagcgaga ggttggatgg acctccttct ccaccactca 12960
ttggcattaa ttcaattggc ctccggggct cccccttct ttcccttctg tctaagagct 13020
cttcatccct ggattcccgt gnnnnnnnnn nnnnnnnnnn nnnnnnnnnn nnnnnnnnnn 13080
nnnnnnnnnn nnnnnnnnnn nnnnnnnnnn nnnnnnnnnn nnnnnnnnnn nnnnnnnnnn 13140
nnnnnnnnnn nnnnnnnnnn nnnnnnnnnn nnnnnnnnnn nnnnnnnnnn nnnnnnnnnn 13200
nnnnnnnnnn nnnnnnnnnn nnnnnnnnnn nnnnnnnnnn nnnnnnnnnn nnnnnnnnnn 13260
nnnnnnnnnn nnnnnnnnnn nnnnnnnnnn nnnnntcagg aatttcattt ggtttttaa 13320
atcttctggc ttatctttca gctccatcca ctccctttac ccctcctaag cattttaatt 13380
accctgggaa gggtgtgaat gaggataaag gaactgatct ggaggggggt gaattacctg 13440
cttctttttct tgactgccag aagaatattt gaatttaatg gatacgtttg tctaagaccc 13500
aagagaagca atgacagaag ctgggacagc ctttatagcc ttagagccag gcactagtga 13560
aagttcctaa agaattgaag agctgggctc ttttgggtgt tttgttttgc tttgtttttt 13620
cgtcctcttt tgaacacttc aaagcaaatt ctgttcaatt tggacttctc cccccgtccc 13680
aacactcccc caacaccagg acgtttggca aagctgcaag actttttttt tttttttttt 13740
tttttaattg tgcttccagt aaaataggga gttgctaaag tcatagcaag agatttgcag 13800
ctatccctca cgggacctga aaggttctcg gtaaagtccc ttaaaaatag gaggtgcttg 13860
ggaaatgtgc tttgctttgg gtgtgtctga agcctcatta aatcttaggt aagaattggc 13920
aaggatacca tatcctggta catggtaatt ttctcacctg tgccctaacc ctgttctgcc 13980
tttctgggag aagggaagat ggtgtctgga tctgattctt actttcttcc ctttccaact 14040
tggtatttgg atagcatcgg tcaaatccta tgtatagcgt ccgggattca ggaggcgtgg 14100
ctaactgtga tctttccactt cctcccttac agaagaagag caagaagatg aggaagaaat 14160
tgatgtggtg tctgtggaga agaggcaaac ccctgccaag aggtcggagt cgggctcatc 14220
tccatcccga ggccacagca aacctccgca cagcccactg gtcctcaaga ggtgccacgt 14280
ctccactcac cagcacaact acgccgcacc cccctccaca aggaaggact atccagctgc 14340
caagagggcc aagttggaca gtggcagggt cctgaagcag atcagcaaca accgcaagtg 14400
ctccagcccc aggtcctcag acacggagga aaacgacaag aggcggacac acaacgtctt 14460
ggaacgtcag aggaggaacg agctgaagcg cagctttttt gccctgcgtg accagatccc 14520
tgaattggaa aacaacgaaa aggccccccaa ggtagtgatc ctcaaaaaag ccaccgccta 14580
catcctgtcc attcaagcag acgagcacaa gctcacctct gaaaaggact tattgaggaa 14640
acgacgagaa cagttgaaac acaaactcga acagcttcga aactctggtg cataaactga 14700
cctaactcga ggaggagctg gaatctctcg tgagagtaag gagaacggtt ccttctgaca 14760
gaactgatgc gctggaatta aaatgcatgc tcaaagccta acctcacaac cttggctggg 14820
gctttgggac tgtaagcttc agccataatt ttaactgcct caaacttaaa tagtataaaa 14880
gaactttttt ttatgcttcc catctttttt ctttttcctt ttaacagatt tgtatttaat 14940
tgttttttta aaaaaatctt aaaatctatc caattttccc atgtaaatag ggccttgaaa 15000
tgtaaataac tttaataaaa cgtttataac agttacaaaa gattttaaga catgtaccat 15060
aatttttttt atttaaagac attttcattt ttaaagttga ttttttttcta ttgtttttag 15120
aaaaaaataa aataattgga aaaaatacaa ttgggccaac tgtgttttc tttttcctct 15180
tcctcaaact tcctttcctc aattacagat taaaagaatt tgaccatttt cacagggtag 15240
gtttacaaat atgggaaggg gttatcattg ttaaaatggg gctgggggtc ctcaggattt 15300
ctaagttgtc tacaggatgc tttctgtgga tagtaataaa aaccagagct gttagttagg 15360
aatgggcaaa aggcaagtga gaaggctaga tgcagggaag ggaaaagcaa gaggttaaag 15420
ataacagcta aatatacagg aggaagagat ggcagaatct cctacagtta accgaagcca 15480
ttccctggtt cacctcaacc caaggactct gccctgccaa agaactggtg aggggaggga 15540
gagagaacca ccgtttgttc cttgcctctt gctcccaggt gatagtccct tcacatcagt 15600
atctcctatg cttctgaaaa aaacagagga agagcattac cactgctaag ttgatcctgg 15660
ttttccaaac aaggacatac aaaggttcag agggttgcag agcttaatgg gtacgaacac 15720
aacaaaaata acccagggcc cctgtcttga aaacaagcct tgctgccttg cttagttggg 15780
tgtccttccg ctggttaggg gtctgagaat gagcgctaca ggctccatta ggagctttga 15840
cagagtgcca cgaaagaggt actatgatct ctttatacct agtacaggta gaaacagaca 15900
tgaaataacc catccatgca cccaggggat tcaaaaccac tttatcctta gtgcatccag 15960
gaggaagatc ctagctacga aggagagggc aggagtcatt aaagcttcac tactcaagaa 16020
ctggtagact tcagttcccc tccttgtttg tggattgggg ggttggggtg gagggtgttg 16080
tgtgtactca gaggcacata gctcactcct accttaccca cttagttttt aacatcagat 16140
ccctgcttgc tcctgggaag aagccagttt agaagtgcta ctggtcagat cataaaataa 16200
aaccttgttt tacatgagtc attattttag aaattgcaag ctcgccttcc tcccaagcac 16260
tttcacacac tcatgacaca cgctcatcaa tggcccatga gaaagccttt tggaacaggt 16320
atcttattaa ttataagcct ctgaaaagcc acaatccaaa ccagaaactg aaacatgtag 16380
gtaaagtcca gggagaaagg taccctaaat gtcctaaatg accccattgt ctcagagaag 16440
gtctctagca tcctggagtg tctttgggac tttaattcac catcattcat aaaccaataa 16500
gtaattacta tctttgacac cccccccca tgatctattg aagcgtttaa catttagttt 16560
ttgatcttat ttttggctct tttagactca ccctcagctt caaactgtta caccctctat 16620
cctacattca aaagaaaaca caaaccccag tagtagtttg aaatactttg agtagttcaa 16680
aggattagca aaaggggggga gcagggggtg ctcttaaata gttccctctt tttcccctttt 16740
gtgagcctag gctggagtgc agccctgggg tgactcactt gagacctggg aaggtgttag 16800
gttgaatcac tcagtccagg caagcccaaa gaatagagga aagcattcct cattaggaaa 16860
acaactcctg ttccaaatga tcaggaaaca agtttagaga ttcagatttg gctgtgggga 16920
tggaatcgaa gtatcacagc tccctatctg ggcacttctc agctttacca agccagggaa 16980
```

tggtctgaaa acaggacatc ggccagcttc cttccagaaa gtcaggctga tcttgaccat 17040
aacacaggaa ggctctccca gccaatctgg agtcccaggg gtctgcaaca ttgtgcaatc 17100
aaatttatag ataacaatca cgatgagggg tgaagtggga ggagctttca gcttggtctg 17160
ggatatacaa gaattagcta gtctcttctg ttgttcaccc atgacactgg aactcagttt 17220
ccccgagagg atccctggga tggtgctctt caggataaac tgagtgagga ggaagtgtga 17280
ctttatgcta tcattcgggg acaacactaa aaagcaatca attacacttt aagttgaaca 17340
aaagtttcac aataccagga tagtgcttga gtcatccaaa gcttgaacac gtatgtagag 17400
tcttctgctg ttctttaaga tggactttgg cactaacctt ggaaaagagg gccaaaaaac 17460
ccaccccccca aaaacaaaca gaacaaaaaa cgtgaggggga ttttctgtgg tttgtttaca 17520
ataagagatg agtcacaata atgatttttt tttttttttt tagcatgact gctaaggaac 17580
cctgaagcat ttcttcccaa caaaaactaa tctttaggtt ataacatgct ggaagatgag 17640
cttcaagcct gtctataaat acatcactgg gtatcttcag ggaacaaact cttcacttaa 17700
ggttagagac catcctggat cgatttcaag aacagaggtt tattttaaac aatgaagcct 17760
gggctagaga tgtcgttcag tgataagaca cttgtctagc atgtataagg acccatgctt 17820
gacaccacag gaataaataa aaccaaaaat gaggttccta ctcattcccc aaactttagc 17880
aattttggtg gtggtggtgg tggtggttcg acgtctttcc agagaaatcc aacaaactgc 17940
caatttaggg aaaacatcta gctctagcga ctttgccaaa ctggtgtgag ttgactcaat 18000
gactgagtgg atttcctttt tgggttttac ttcactcaat attggtctac actaagctct 18060
ttaggaagac agggttgaga gaggaacaag tgttaatggg atgtagatcc tctgaggctg 18120
aaaggaatgt cctttgtctc taaacaatgc cgaggggctc aggaacactc tcttccagtc 18180
ttcacccgac catctcatta aaggctaaac tctccagttc gggtctatat ggctaggaaa 18240
gaggagcttt ggggaaagct ctgtcagaat atacctgctt tctgcagggg gcgggaggac 18300
ccccacaaac aactcagctg gagaacaatt gttagatgtg acaatactga agtttgtaca 18360
cagacatgtg aaattgctgg cctgaagtca cataagcact aactaacgat aatagcaatt 18420
aatgtttact gtgtgctaaa agtttgacat acattatctc cttttattcc caaagcaacc 18480
ccctatacac acacacacct cctttttggt tttggtttta ctttttaaat tttactatta 18540
aattttaaat atgttttttt tttaaattta ctgcactaga gagggaacct agaacctcct 18600
gcatgcaagg taagtgatca atgggtgagt cacatctcta tagagattgt aggttgtttc 18660
accaaaagta ttagggcatt tcctacagag tggtggaagt tcgagtcctg ggaacatgga 18720
ataaggatgg aatccagtaa gtcttgagaa agaagctaaa ggagaaaatg tcagcagcag 18780
atggcaggga agcaaagaag aaggagagct aaaaccaaca agctaaaacc aaaaacgctt 18840
gctatttgaa ctgtgcctaa ggtagcagga agtacagcaa ggatccactg gcttagtgag 18900
ggaatgcttc tctaggcctc tcctatccac ttccccttcc acccagtatc ctcctactcc 18960
ttcatctgct agagacaact gcccataggt agttaattag ctgtcccagc acccaactct 19020
cctgcagtac tttggttctg tttatttgtt ttggcaatag gatctcatta ggcaggccat 19080
actggcctca aactctggaa tttcttgcct cagcctctta agtgttgtgt tgggattacc 19140
agtttgggac acttattctc cagctttcat gcagggcttt attcaggaat ttccactgtg 19200
tgattctagg atgcaaatac tagttctcat tccctgaggg tttaattgtt gttgttgttt 19260
cgttagttgg ttggttttgg gttttgttca ttgggttggt ttgggttggt tttggttttt 19320
caaaacaggg tttctctgtg tctgagaacc attgtctttt ctaccactgc aaacccaacc 19380
atcttttcaa accactggta agccaagtcc tttctggatt aagtctgaag ccattctcca 19440
ctcctattcg atacacttac tacctaccac aaacacggca gcctggcagc ctcaatgcca 19500
gttttgggct tctccttgta tgcttggaga ccagttgtct tcatcatcag atcaacccct 19560
tgaagaatgg aagttcattg gtcttgctta atcatgcctg acaagaaagt gcaattcact 19620
aattctgtaa acatctaagt gaagggctct ttgatgggct caatggtgca tcagacctga 19680
gtaaggggtg gagtagaagc aaagttgttt caagttgcat agcgagaaag gtcaaggaaa 19740
gagatggggg ttcatcaggt gaagggaagg attttgatta gacttgcttt gcttgatttg 19800
gtttttgagc tgaaaagaca ggaagcctaa ttcatttcca tgatagtagg ctagacttga 19860
gagggaaaga actgctgaca gaaaaggagg ggaattgtac tgactggttt tgtgtgtgtg 19920
tgtgtgtgtg tgtgtgtgtg tgtgtgtgtg tgtgtgtgtg tgtgtgtgaa cttgacacaa 19980
gctggagtta tcacagagcc tcacttgggg aaaagcctcc gtgagatcca gctgtaaggc 20040
atttctcaa ttagtggtga aggagggcct atgtgggtgg tgccatccct gggctggtag 20100
tcttgagttc tctaagagag caagctgagc aagccagggg aagcaagcca gtaagtaaca 20160
tccttccaag acctctgcat cagctcctgc ttcctgacct gcttgagttc cagtcctgac 20220
ttcctttggt aatgaacagc agtgtgtaag tggacagtga ataatccttt tctttcccaa 20280
cttgcttttt ggacatgatg ttttgtgcag gaatataaac cctgactaag acaagaatca 20340
aagtgtctag tcaggtgagt tactggaccg gtaggtagct ttgttttgct tgcttgatgt 20400
ctggagccct ctcttagctg ggtatggctg aacaaatgaa aaaagaatat gttcccccta 20460
cttaggtaag tattcttcca ggtggtgatg tcactggcca agctgttctc ccttggcttc 20520
ccacagctct cttcctttac cagataccat gtccatcttt acacaaatga ctctaaacct 20580
cttctctcca cagacacctt caatccacat gagataaagc ctccacattt tgtagctctg 20640
gctgacctgg aactcactat gcagaccagc cttgcattga actttcagag atctgcctat 20700
atctttctta ggagtgctag gactaaaggc atgcaccaca tttagtcctg ctaaacttct 20760
ttttttgttt ttttggtttt ttcgagacag ggtttctctg tgtagccctg gctgtcctgg 20820
aactcactct gtagaccagg ctggcctcaa acttagaaat ccacctgtct ctgcctccca 20880
agtgctgaga ttaaagatgt gtggtactac tgcttagact gacacacaaa atattgttga 20940
agccagtgag ataaagctca ccttcaatct cagaggtagg ccaggcagac ttttgtgagt 21000
tctgggccag ccagagcttc atatgaaaag taataataat gataataata ataataattt 21060

```
taataaacaa atgggtgtat gaaagagtaa atgtgcacat atttctggca tctgttttaa 21120
gattctactt ccttaggctg tcagtcattc tcaccccatt caaccctttg ctctgcactg 21180
tgattgacaa atcttctctc cctctggtga ctacctaatt catcccagac agcatcagcc 21240
ttggcaacaa cttctctatg cagggactgt aagttccaga caacagaaac tctgttccaa 21300
ttcacaaaat caactaggaa aggagaagag acagaggaca gaggaaagga aggaagaagg 21360
gagaaaagga gggagggagg gagggaggga gggaaaatgt caatttatgc aactgtttca 21420
tgattcatcc aaggatcctc tctgattctt ttttggttgt tgttgttttt gttttttcga 21480
gacagggttt ctctgtatag ccctgactgt cctggaactc actttgtaga ccaggctggc 21540
ctcgaactca gaaacccacc tgcctctgcc tcccaagtgc tgggattaaa gatgtgtgcc 21600
accacgccca gctaaacctc ttccttctag ccagaagaac acagcagaaa gaactatgat 21660
cagtcttctg gaaccatgtg cttaccctta aaccagacaa ttcacttggt tcccaggacc 21720
acactcctcc tgcaaaagtt ggaaccctgt gattctcagc ttctgaggca aagggaagac 21780
ctatccaaga taaaaaacca acaacaacaa aaaagtcaat ataagaaaag aaaaaaatgt 21840
tctctagata cattctgggt agtgatggtt ctcactgttc agagatataa gaaaagagca 21900
gatgcttaaa accctgactc ttgagaacaa cttttggtaa gagagactcc ttttttttaaa 21960
attttattta tttatttatt tacttactta ttatatggta agtacactgg tagctgtctt 22020
cagacactcc agaagagagt atcagatctc attacagatg gttgtgagcc accatgtggt 22080
tgctgggatt tgaactcagg atctttggaa gaacagtcag tgctcttaac cactgagcta 22140
tcttacccag ccccccaccc acccccccaa ccccgttaag agagattcga atggtccatc 22200
ttagaggtat tgaaattgag tctgaagtgg gaggcctagg ggctgttttg agccaactca 22260
gcccaggtga tttgcagtga ggtgggaatt ccaagggcaa acaaggatca gcaaaagcta 22320
tacccctcta ccacagattt ggagagaatt cagatgggag gcctgttcca gccattgctc 22380
agtaagtaag caagagtcta gctacctctt catctccctc cttctaccac accattaagt 22440
ccctccaggc catcagcctc aatgattctt tcttttttttt tttttaatta ggtattttcc 22500
tcatttacat ttccaatgct atcccaaaag tcccccatac cctcccccctc cactccaact 22560
ttttggccct gttgttcccc gcctcaatgc ttcttggctc tatccatgtc tccttgccca 22620
ctttgcttct gccaaccgat attctttacc tgggactctc tagcagatgc caactgcctg 22680
tctctcatct ggtttcctca aaggtagaaa aatggtctcc caaataagcc cgatcagttt 22740
actccctggc gtaggagaac ttgatgattc atgtgctctg cttctaaaat acagatctgt 22800
gggcctggta gtgcagatgt taatcccaac tactctactt gggaggccta gggaatggac 22860
tccatgttca aggtctgcct ggacagcaag atgagtgctg gggaagccag agaaaatgag 22920
taaaactttc tgaaaagtaa agcattataa aaacaaacaa acaaaggtcc agagaactgg 22980
ggagatgatt ggctggttaa gagtacttgc tcttctgcat gggacccaag ttcagttcct 23040
agaagccgag tagcttgcac cttcaattat ggccccctct tctgttttct caggaccagg 23100
tgcacacagt gcatataaca tacatgtaag caaaatactc acacacataa aattaaaaat 23160
aaataaatct ttcaggcaaa actgacctgg gatatagctc aaggatagag gatttgcatg 23220
ggatgctagc agccctgact tccatctaca gtccagaaag acaagtaaat cagaaatgcc 23280
tttgagggac tgaagagatg attcagaggt taagagcact ggctgctctt ccagaagacc 23340
tgggttcaat tcctagtacc tgcatgagag ctgactccag ttccagggaa tctgacccta 23400
tcactcagac actgttcttg tcatctgcac acgggatgat cacacacata tcatttcaca 23460
tgtggggata ctgagaatga gagtcagcag gtaactgtct caacttcaac taagcagggc 23520
agtttgttag cagggatgga atctggattc ttattgcccc ccagcttctt cacttcctgc 23580
tgcctctcta caaccacacc atattgaaat taccttcttc cctctcttgc ttccgcactg 23640
aaactggggc tgaatctagc tggctacctg acctgattct ctcctatcag atggatgact 23700
tgctgtcctc ctgggtcatc tcccgtgttt agcaagacag ggtatagagt tttaaatgag 23760
tgtaggcaga gtgcatgaat catcacactt ttaggcagag tgcatgaatc atcagacttt 23820
cctacgccag ggagtaaact gagcacgcat attttggaga ccacttctct actcctaggg 23880
tcagaagagc ctgcagatga tctactcaaa gcaattttat ctgccattct gttgagtagt 23940
gaggcaagga tcatgttacc tgcaacgggg ccaatctttg cctctgtttt tgaagtctct 24000
ggagaagtag ccgtcttcct gcgctgggga tatgattaag cagtagaaca tgcacataat 24060
ttcatgaggc taacatacag ctcttcagag atcagcctac ttatttatga cacaattgtt 24120
ttaagacatc aggtattcta ggagataaga ttccttctct tgctttcctg gtacccagaa 24180
tgatctccta aaccacaagt aattggcaca gagatggatc ttccaaactg acccagatct 24240
acacagatct tatcttcata cccaagaact ccccagtcaa tggatatttg gggcatttgc 24300
tatgcacaca gacgtcagtg tggaataacc gtactccagc acttagtaga aaggatgtat 24360
actgaacgag tatttacaaa taattaacca gattattttc agggcacaaa ttagtggagc 24420
aaggattgca gagttacatg aacatctgat agcattgcgg ttcagggaaa gttcccagcc 24480
agggtacaga ggatggtgat gaagaaagca tgctcaatat cttgaggcgc tgctatgaga 24540
gagaatgaaa ctctctaggg gaactgaaag catacaagga tgtaaagaat aggaagagca 24600
aaaggacttc aacccttgga tgagacacag ggtgatccaa tacccggtga cctgcaggga 24660
ctcaggccca gtggttatga tggcaaccta cccacagtcg tgagtatgat gcagtgggaa 24720
gtcacagtac tgaaaaccaa cacacttaac ttgcctgtgc tgctgtgaac gtccattcaa 24780
tgagggtcag gaagctcaaa taagataatc gcaaatactg agtggttctg agaggaatgc 24840
aaccatgaaa aaaaaagtgc tgacacttgc agaatgttta tgtttctaat gctgggctaa 24900
gtacttcacc tggattattt caccaattcc tcctcctcct ctccctgttc tcccttctcc 24960
tcctcttctt cctgttcttc ctcctcctct tcctcttctt ccaaagattc atgtgtatca 25020
aggtattctc aa 25032
```

<210> 20
<211> 1799
<212> DNA
<213> Mus musculus

<400> 20
gattggggta cgcgctgcgc caggtttccg caccaaccag agctggataa ctctagactt 60
gcttcccttg ctgtgccccc tccagcagac agccacgacg atgcccctca acgtgaactt 120
caccaacagg aactatgacc tcgactacga ctccgtacag ccctatttca tctgcgacga 180
ggaagagaat ttctatcacc agcaacagca gagcgagctg cagccgcccg cgcccagtga 240
ggatatctgg aagaaattcg agctgcttcc caccccgccc ctgtccccga gccgccgctc 300
cgggctctgc tctccatcct atgttgcggt cgctacgtcc ttctccccaa gggaagacga 360
tgacggcggc ggtggcaact tctccaccgc cgatcagctg gagatgatga ccgagttact 420
tggaggagac atggtgaacc agagcttcat ctgcgatcct gacgacgaga ccttcatcaa 480
gaacatcatc atccaggact gtatgtggag cggtttctca gccgctgcca agctggtctc 540
ggagaagctg gcctcctacc aggctgcgcg caaagacagc accagcctga gccccgcccg 600
cgggcacagc gtctgctcca cctccagcct gtacctgcag gacctcaccg ccgccgcgtc 660
cgagtgcatt gacccctcag tggtctttcc ctacccgctc aacgacagca gctcgcccaa 720
atcctgtacc tcgtccgatt ccacggcctt ctctccttcc tcggactcgc tgctgtcctc 780
cgagtcctcc ccacgggcca gccctgagcc cctagtgctg catgaggaga caccgcccac 840
caccagcagc gactctgaag aagagcaaga agatgaggaa gaaattgatg tggtgtctgt 900
ggagaagagg caaacccctg ccaagaggtc ggagtcgggc tcatctccat cccgaggcca 960
cagcaaacct ccgcacagcc cactggtcct caagaggtgc cacgtctcca ctcaccagca 1020
caactacgcc gcacccccct ccacaaggaa ggactatcca gctgccaaga gggccaagtt 1080
ggacagtggc agggtcctga agcagatcag caacaaccgc aagtgctcca gccccaggtc 1140
ctcagacacg gaggaaaacg acaagaggcg gacacacaac gtcttggaac gtcagaggag 1200
gaacgagctg aagcgcagct tttttgccct gcgtgaccag atccctgaat tggaaaacaa 1260
cgaaaaggcc cccaaggtag tgatcctcaa aaaagccacc gcctacatcc tgtccattca 1320
agcagacgag cacaagctca cctctgaaaa ggacttattg aggaaacgac gagaacagtt 1380
gaaacacaaa ctcgaacagc ttcgaaactc tggtgcataa actgacctaa ctcgaggagg 1440
agctggaatc tctcgtgaga gtaaggagaa cggttccttc tgacagaact gatgcgctgg 1500
aattaaaatg catgctcaaa gcctaacctc acaaccttgg ctggggcttt gggactgtaa 1560
gcttcagcca taattttaac tgcctcaaac ttaaatagta taaaagaact ttttttatg 1620
cttcccatct tttttctttt tccttttaac agatttgtat ttaattgttt ttttaaaaaa 1680
atcttaaaat ctatccaatt ttcccatgta aatagggcct tgaaatgtaa ataactttaa 1740
taaaacgttt ataacagtta aaaaaaaaaa aaaaaaaaaa aaaaaaaaaa aaaaaaaaa 1799

<210> 21
<211> 1320
<212> DNA
<213> Mus musculus

<400> 21
atgcccctca acgtgaactt caccaacagg aactatgacc tcgactacga ctccgtacag 60
ccctatttca tctgcgacga ggaagagaat ttctatcacc agcaacagca gagcgagctg 120
cagccgcccg cgcccagtga ggatatctgg aagaaattcg agctgcttcc caccccgccc 180
ctgtccccga gccgccgctc cgggctctgc tctccatcct atgttgcggt cgctacgtcc 240
ttctccccaa gggaagacga tgacggcggc ggtggcaact tctccaccgc cgatcagctg 300
gagatgatga ccgagttact tggaggagac atggtgaacc agagcttcat ctgcgatcct 360
gacgacgaga ccttcatcaa gaacatcatc atccaggact gtatgtggag cggtttctca 420
gccgctgcca agctggtctc ggagaagctg gcctcctacc aggctgcgcg caaagacagc 480
accagcctga gccccgcccg cgggcacagc gtctgctcca cctccagcct gtacctgcag 540
gacctcaccg ccgccgcgtc cgagtgcatt gacccctcag tggtctttcc ctacccgctc 600
aacgacagca gctcgcccaa atcctgtacc tcgtccgatt ccacggcctt ctctccttcc 660
tcggactcgc tgctgtcctc cgagtcctcc ccacgggcca gccctgagcc cctagtgctg 720
catgaggaga caccgcccac caccagcagc gactctgaag aagagcaaga agatgaggaa 780
gaaattgatg tggtgtctgt ggagaagagg caaacccctg ccaagaggtc ggagtcgggc 840
tcatctccat cccgaggcca cagcaaacct ccgcacagcc cactggtcct caagaggtgc 900
cacgtctcca ctcaccagca caactacgcc gcacccccct ccacaaggaa ggactatcca 960
gctgccaaga gggccaagtt ggacagtggc agggtcctga agcagatcag caacaaccgc 1020
aagtgctcca gccccaggtc ctcagacacg gaggaaaacg acaagaggcg gacacacaac 1080
gtcttggaac gtcagaggag gaacgagctg aagcgcagct tttttgccct gcgtgaccag 1140
atccctgaat tggaaaacaa cgaaaaggcc cccaaggtag tgatcctcaa aaaagccacc 1200
gcctacatcc tgtccattca agcagacgag cacaagctca cctctgaaaa ggacttattg 1260
aggaaacgac gagaacagtt gaaacacaaa ctcgaacagc ttcgaaactc tggtgcataa 1320

<210> 22
<211> 37487
<212> DNA
<213> Homo sapiens

<400> 22
actcccacca cacctattca acatagtatt ggattccctg gccagaacaa ttaggcatga 60
gagagaaaca acaggcatcc aaacaagaag agagaaggtc aaattatccc tgtttgcaga 120
caatatgatt ctgcatctag aaaactccat agcctctgcc ccgaaactcc ttgagttgat 180
aatcaacttc agcaaagttt caggatacaa gatcaatgta caaaaattag tagcactcct 240
atacactaac atcacccaag ccaagaggca aatcaggaac acaatcccat tcacaattgc 300
cacaaaataa taaaaagctt aggaatacag ttaactagcg aggtgaaaga tctctactat 360
gagaattaaa aaacactctt caaagaaatc agagatgacc caaacaattg gaagaacatt 420
ccatgttcat ggaagggaag aatcaatatt ggtaaactgg ccatacacct caaaacaatt 480
tacagattca attttattcc tatcaaacta ccaatgacat tctacacaga aatggaaata 540
actactcaaa aattcatatg gagctaataa aagagtctaa atacacaagg caatcctagg 600
caaaaagagc aaagctgaag gaatcacatt gaccaacttc aaattgtact acaaggatgt 660
agtaatcaaa acagcatgga actgatacca aaacagacac atagaccaat ggaacaaaat 720
agtgagcaca gaaataaagc cacataccta caaccattgg atctttgaca aatctgacag 780
aaacaagcaa gagggaaaag actccctatt aaattaatgg tgctgtgatt actggttagc 840
catatgaaga aaactgaaac tagacttatg ccatatataa aatccatatg ccggaaagct 900
gaggcaggag aatcacttga acccgggagg tggaggttgc agtgagccga gatcacacta 960
ctgcactgca gcctagcgac agagtgagac tccgttgcaa aaataaataa ataaataaat 1020
aaataaataa ataaataaat atcaactcaa ggtggattaa agaccaaaat gtaaaactct 1080
aaaactataa aaaccctgga agatacccta ggaaatacca ttttggacat aggagctgac 1140
aacgattttt tgaaaaaaac accaaaagca aaagttgaca aataagacct aattaaacta 1200
aagaacttct gcacagcaaa agaaactatc aacagagtaa ataggcaacc tgcagaatgg 1260
aggaaaatat tgacaaacta tatatgtgac aaaggcctaa tatacaaaat ctacgaggaa 1320
cttaaacata caaggaaaaa tcaaacaacc cacctcacta aaaagtggga aaaggacata 1380
aaccgacacg tttcaaaaga agacatacaa gcatatacat acaagccaac aagcatatga 1440
aaaaatgctc atcatgacta atcattagag aaatgcaaat caaagcagca ataagatacc 1500
atctcacact agtcagaata gctattatta aaaagttaaa aaatcacaga tgctggtgag 1560
gttgtggaca aaagggaacg cttatacact gctggtggga gtgtaaatta gttcaaccat 1620
tgtggaaagc agtgtggtga ttcctcaagt aattaaaaac agaactacca tttgacccag 1680
caatcccact actggatata aacccaaata aatataaatc tttccaccac gaaaacacat 1740
gcaagcatgt gttcattgta gctctattca caatagcaaa ggcatgaaat cagcctaaat 1800
gctcaactac tgcagactgg ataaagaaaa tgtggtacat acacacgatg caatactaag 1860
catccattaa aaaaaaaaaa gaaatcatgt ccttttcagg aacatgaatg gaatcagagg 1920
ccattatcct tagcaaacta ccacaggaac agaaaaccaa acctatgttt tcacttataa 1980
ttgggagcta aataatgata acccatgggc agaaagaggc ctcacactgt gaggcctgcg 2040
tgaaggagga ggatgggagg agaaagaagt ccaggggaaa aaaacttcgg gtactgtgct 2100
tagtacccag acagcaaaat aatctgtaca cattgatggg acgtatctca aaataataag 2160
agctatctat gacaaacaca cagccaatat catactgaat gggcaaaaac tggaagcatt 2220
ccctttgaaa actggcacaa gacaaggatg ccttctctca ccactcctat tcaacatagt 2280
gttggaagtt ctggccaggg caatcaggca ggagaaagaa ataaagggta ttcaattagg 2340
aaaagaggaa gtcaaattgt ccttgtttgc agatgacatg attgtatatt tagaaaaccc 2400
catcgtctca gccccaaata tccttaagct gataagcaac ttcagcaaag tctcaggata 2460
caaaatcaat gtgcaaaaat cgcaagcatt cttatacaac aataacagac agagagccaa 2520
atcctgagtg aactcccatt cacaattgct tcaaagagaa taaaatacct aggaatccaa 2580
cttacaaggg atgtgaagga cctcttcaag gagaactaca aaacactgtt caatgaaata 2640
aaagaggaca caaacaaatg gaagaacatt ccatgctcgt gaataggaag aatcaatatc 2700
gtgaaaacgg ccatattgcc caaggtaatt tatagattca atgccatccc catcaagcta 2760
ccaatgactt tcttcacaga attggaaata actactttaa agttcatatg gaaccaaaag 2820
agagcccgca ttgccaagtc aatcctaagc caaaagaaca aagctggagg catcacgcta 2880
cctgacttca aactatacta caaggctaca gtaaccaaaa cagcatggta ctggtaccaa 2940
aacagagata tagaccaaag gaagagaaca gagccctcag aaataatgct gcatatctac 3000
aaccatctta cctttgacaa acctgacaaa accaagaaat ggggaaagga ttccctattt 3060
aataaatggt gttggaaaac tggctagcca tatgtacaaa gctgaaactg ggtcccttcc 3120
ttacacctta tacaaaaatt aattcaagat ggattaaaga cttaaatgtt agacctgaaa 3180
ccataaaaac cctagaagaa aacctaggca ataccactca ggacataggc atgagcaagg 3240
acttcatgtc aaaaacacca aaagcaatgg cgacaaaagc caaaattgac aaatgggatc 3300
taattaaact aaagagcttc tgcacagcaa aagaaactac catcagagtg aacaggcaac 3360
ctaaagaatg ggagaaaatt tttgcaatct actcatctga caaagggcta atatccagaa 3420
tctacaatga actcaaacaa atttacaaga aaaaaacaaa caaccccatc aaaaagtggg 3480
tgaaggatat gaacagacac ttctcagaag aagatattta tgcagccaaa agacacatga 3540
aaaaatgctc atcatcactg gccatcagag aaatgcaaat caaaaccaca atgagatacc 3600
atttcacacc agttagaatg gtgatcatta aaatgtcagg aaacaacagg tgctggagag 3660

```
gatgtggaga aacaggaaca cttttacgct gttggtggga ctgcaaacaa gttcaaccat 3720
tgtggaagtc agtgtggcga ttcctcaggg atctagaact agaaatacca tttgacccag 3780
ccatcccatt actgggtata tacccaaaag attgtaagtc atgctgctat aaagacatgt 3840
gcgcacgtat gtttattgcg gcactattca caatagcaaa gacttggaac caactgaaat 3900
gtccatcaat gatagactgg attaagaaaa tgtggcacat atacaccatg gaatactatg 3960
cagccataaa aaatgatgac ttcatgtcct ttgtagggac atggatgaag ctggaaacca 4020
tcattctcag cacactatct caaggacaaa aaaccataca ccacatgttc tcactcatag 4080
gtgagaatta aacaatgaga acagatggac acaggaaggg aaacatcaca caccggggcc 4140
tgttgtgggg taggggggagg ggggatggat aggattagga gatatatcta atattaaatg 4200
acgaattaat gagtgcggca caccaacatg gcacatgtat acatatgtaa caaacctgca 4260
cattgtgcac atatacccta aaacttaaag tataataaaa aaaatctgta cactaaaact 4320
ccaagtcatg agtttaccca tataaaaaac ctgaacctaa aataaaagtt aaaatattca 4380
aatataaata agtaaataaa taaaatgaca aacccatggc taacatcatg ttgaataagg 4440
aaaagttgaa tgcttttttt tctaaaatcc aggagaagac aaggatgtcc attctctctt 4500
ctactcaaca tggtactggg agtcttaggt agaacaatta cacaagagaa tgaaataaag 4560
gtcattcaaa ttggacagga ggaagtcctt ttctgcagta acatgatctt ataggtataa 4620
aaccctatag actcaaccaa agaaaaataa ctggtagtat tgataaatga attcagtaaa 4680
acttcaagtt acaaatttaa tgtacaataa ttagtagtgt ttctacacag caacagcaaa 4740
ctatctgaaa aagaaatcaa gaaatctatc tcttttacaa tagctacaaa aatttcaaaa 4800
aatttcaaaa aatacctaga aataaattta accaaggagg tgaaagatct ctacagtaaa 4860
aaataataaa atatggatga aagaaattga agaggatact aataaatgta aagttaaccc 4920
atgctcatag atttgtagag ttaatgttgt taaaatgtcc atactaccca aaccaatgta 4980
cagatacaat gcaatctcta tcaaaatact aaggactttc ctcacagaag taaaatatat 5040
atattttttta atatataaaa tgtatatata aatataatat ataatatgaa tattatatat 5100
gattataata ttatatgatt atatactatt aatattatta ataatatata ttattatata 5160
tattatatat attattatat attgtatata aatatatatt atatactata ttattatata 5220
ttgtatataa atatatatta tatatactat atatttatat actatatata tagtgtatat 5280
atatatttat atagtatata taatatatac actatatatt tatatagtat gtatatatat 5340
atactatata taatacataa aatatatata catatatagt atatatatga aatatatata 5400
tactaaattt gcacagaacc acatgattcc aaacagccaa agcaatctgg agcacaaaga 5460
acaaagctag atgcattaca ctacctggct tttaaatttt ctacaaagca atagtaaaca 5520
aaatagcata gtactaacat gaaaacagat ccatagactg atggaggaga atagagaccc 5580
caaaaatgaa tccatacaat tatagccaac tagtttttga catatgtacc aagaaaacac 5640
attggggaat ggaaagtctc tgcaataaat ggtgctggat aaactgaata tccacaagca 5700
gaagaaagaa actaggtgct tctctctccc aacatataaa aatcaactca aaatggatta 5760
aagacttgaa tataaaacat gaaactaaga agatccttga agaaaacaca ggggaaacaa 5820
ttcatgatat tggtctggga aagaattttt caaataagac ctcaaaagca caaacaacca 5880
aagctaaaat agatgaatgg gataatatca agccaaaaag cttttacatc ttaaaggaaa 5940
caacagaatg aagaaatgac ctacagaatg aagaaatatt cacatactat gcatctgaca 6000
agaggttaat atgaaactat gtaatcaatg caagcaactc aataggaaac acacaaataa 6060
tccaatcaaa acttgggcaa aggattcgaa tagatatttc ttaaaaggag tcacacaaat 6120
gggcagcaag tatatgaaaa aaggctcaac atcactaaga accagggaaa tgcaaatcaa 6180
caccacagtg agctatccac tcatcccagt tagaatagtt attatttaaa aaaataaaat 6240
aaaaagacaa gaaaatggcc agtcatggtg gctcatgcct gtaatcccag cattttggag 6300
gccaaggagg gcagatcact taatgccagg agttcaagaa cagcctggcc aacgtgacga 6360
aaccccatct ctactaaaaa tacaaaaaat tagctgggca tggtggcaca tgcctgtggt 6420
cccagctact caggaggctg aggcacgaga attgcttgaa ctggggaggc agaggttgca 6480
gtgagctgag ttcgcaccac tgcactccag cctgggtgcc agagcaagga tctgccaaaa 6540
aaaaaaaaaa aaaaaaaaaa aaaaagacaa gaaataacat atgctggtaa ggatgtggag 6600
aaaagtgaac tgttatacaa tgctatacac ttggtgtact actatattat ccagcaatcc 6660
cactgctgga tccccctccc ccccaaaaat gaaatcaaca tatcaaagag ttcagatccc 6720
catgtttatt gcaacactat ttgcaatagc caaaatatgg aatcaactta agtgctcatc 6780
aacagatgaa acaggaatga aaatatggta tatatacaca atggaatgct attcaatctt 6840
taaaaaagaa tgaaaccctc aaaaagtggg tgaaggacat gaacagacac ttctcaaaag 6900
aagacattta tgcagccaaa aaacacatga aaaaatgctc accatcactg gccatcagag 6960
aaatgcaaat caaaaccaca atgagatacc atctcacacc agttagaatg gcaatcatta 7020
aaaagtcagg aaacaacagg tgctggagag gatgtggaga aataggaaca cttttacact 7080
gttggaggga ctgtaaacta gttcaaccat tgtggaagtc agtgtggcga ttcctcaggg 7140
atctagaact agaaatacca tttgacacag ccatcccatt actgggtata tacccaaagg 7200
actataaatc atgctgctat aaagacacat gcacacgtat gtttattgcg gcactattca 7260
caatagcaaa gacttggaac caacccaaat gtccaacaat gatagaccgg attaagaaaa 7320
tgtgacacat atacaccatg gagtactatg cagccataaa aaatgatgag ttcatgtctt 7380
tggagggaca tggatgaaat tggaagtcat cattctcagt aaactatcgc aagaacaaaa 7440
aaccaaacac cacatgttct cactcatagg tgggaattga acaatgagaa cacatggaca 7500
caggaaggag aacatcacac tctggggact gttgtggggt ggggggaggg gggagggata 7560
gctttaggag atatacctaa tgctaaatga caagttaatg ggtgcagcac accagcatgg 7620
cacatgtata catatgtaac taacctgcac attgtgcaca tgtaccctaa aactaaaagt 7680
ataataataa taataaaaaa gtaaaaatga ttaaggacta taataggata agaactctag 7740
```

```
gaagatgata aagtatgccc aaaagaaagc aatgctataa taaaaacaaa ataaaacaaa 7800
acaacaacaa caaaaagaat gaaaccctgt catttgcaac aatgtggaca aacctagagg 7860
actttatgtt aagtgaaata aaccaagcac agaacgagaa ctaccacatg atctcaggca 7920
tatgtggcat ctaaaaaagt tgacctcaaa gagagtttag tagtggctat cggaaactga 7980
ggagagtagt tgcgaggggg aggggaacag gttgctcaac agctacaaag ttacacttat 8040
ataggacaaa taatttctga taatcgactg cacagtaggt tgagtgtagt caacaataat 8100
gtattgtgta ctttaaaata gttagacgag aggattttgg acgttctcac caccaagaaa 8160
tgacaaatgt ttcaggtgat agacatatgc taattatgct gatttgatca ttacacaata 8220
tatatgtatc aaaacatcac actgtatccc attaatatgt acaattatac atcaatttaa 8280
aacaaaataa aatgtatttt ataaaaattt taaaacttca accagcaagt ttgaatctcc 8340
tattcccagt tttggtcagc cctacagtgt ataccaacca ggctggcctg ggctgctgt 8400
tagaggaaag aaagaaacat ctggacttgg aatgaacaga tctggaagga atcctaaatt 8460
ttccaataaa gtctctgtat tcctttcatc ctggtcccta cccaggaagc agtctagagg 8520
agatgatcct tttctaagaa cagcaacatt cagtaaacaa acttccttac taatgtgtgt 8580
ggatcaagag atggtgagga aagtagagtc agtgatattt ttgaatcaat tatgagaagg 8640
ccaccatgct attgctcagt aatattagat gcccatggac agtaagattc atcgatggtc 8700
cgtctttatt attaggtcat tactgagtgg cttttgcaat aaaatacacc attggcagaa 8760
taaaaatatt ctctaatgca aaaacatagc atggattgtt tttaaggggc acaatagtgt 8820
gcttgatcag actggaatag caacattgta atctgaaaaa agctcaacag tggtgaggca 8880
tattcaaaag ccccaagagg agagtcaaca gtcagatcca tcagagttga gaggttgcca 8940
caacttatgt aatgacgtct ccacgccatg aaacacatgt gtcagctttt gtcaggagtc 9000
acagatctgg catgcagcag gagattctgc attagatata taaagtcagg cccaatcatc 9060
cccttgatta gttagtctca tcagagaatg agcccttgcc atgggtctgt atatgaggaa 9120
tccaggcagt tcagtaagta gtcaagattt gtttccacat gcggtcccaa agaagtcttt 9180
aatctgacag actagttttc caagtggcat accaaaaggc tggaccattg gtagtgtccc 9240
aagaattggg ttctgaccat tgagcagaga aaccaagtcc tctttttatt tctgcatagc 9300
tggttcagag gctgaacaga cacaggagcc agtgaacacc atcagttttc agcttagcca 9360
atccttcact gaggcaagcc cagatatttg gggaaacctc tgtaaacagg gtccaattga 9420
actagtagtg ttgtttcaag caggagaata aaaggttaag tttcccccag agagacagtt 9480
gccactctta catataaagc agagatgcag aagaaattcg agttgatgcc atcgcccacc 9540
caccaaccca cccccgcatt tccgccctga gactaggatc ccagcgccag ggacccagac 9600
cctgggattg atcgcacaga gccgtgatcc ggagagtgcg ccggggatga gacggaatct 9660
cggggccact cgagagcttg gggcaggaac catgcatcca ttgtccatcg tgactgcatg 9720
tggagcagct atttggcctg ggaacagctg tagagagcgg ggagcgactg gctcgccact 9780
ggcgcgccct gggggaaccc gcccaaggtg ccctcctccc cagactgccc tcccagcctc 9840
gaagacggca atccagcgcc cactgctccc tggactctgg gcaaggccaa gactcaggcc 9900
tgctccagat ttgagaaccc aaacaactca gagggtgagg aaattgacct tgtgacagca 9960
aagaaaaggc agattgggtt tacagaagtc gatcaccatc atggttcgag tagaccgtct 10020
gaacccctgc atgaaacact tccatacctc catccaccag caacaacaca actgcgctgc 10080
ccatatttct ccagaaagct gctcccaaga agaggctcca gagagaggtc tccaagaaga 10140
ggttctggag ggagatgctc caggggaaac ggaagatgag gaggatgagg ggattgtgag 10200
cccctcacct atagaaagca aggctgccca gtgctgccag ttctgacact gaggatgtga 10260
ccaagaggaa gggccacagc ttcctgcagc acaagaggtg gaatgacctg cgcgttctca 10320
gttcttggcc ctgagggacc aggtacccac cttggccagc tgctctaagg tccccacggt 10380
agtgatccta agcaaggcct tgggatactt gcaagacctg gcgtgaaccg agaaaaagat 10440
gtctatggag aaaacgcagc tccaacgcca gcaacagcaa ttgcagaaat gaattgcgta 10500
cctcagtggc tactaactga ccaaaaagcc tgactgttct atcttacaaa gacacaagtt 10560
tattttttga cctccccttc ccctttagta attttcacat tttggttatg acgggacagt 10620
ctttgcagta ggtcccagaa tgcattgcag ccagtacaca cagaataagg gcttgcattc 10680
ttggaaacct tgaaacccag ctctttctct cccctgactc atgctgtgtc ttctctggcg 10740
cctttggctt ctcagcagat agctgactga ggagatttgg ggtctgttta cctcactagc 10800
tccgaagaaa aggctgacag atactatgca acaggtggtg tatgttgtcg gggactccag 10860
cctgcatgaa atctcacact ccgcgtgagc cttagactag gaaagaatgc tccctggtat 10920
ctctggggtg atgctagaat gctccctggt atgtctgggg tcatgcaagg acagctgggc 10980
ctggacagca ctctcgctgt ggcttttttt tccaggagac acacaagctg tcttgggtga 11040
tgacaagctt gaagatttga tcaacatgac cattgcttca ctgtcagaca ctttacagta 11100
tctgaggagt tggaaacctt taacgtatat attgtgatat tagctgacac ctctccttcc 11160
agtttcaatg ctgagaccct gagaacattt aaagagcttg cactctaggt tcgttgtctc 11220
agagctctct gggccttctc tgatgaaggg acctttctgt cctcatgaga gacttttgtt 11280
tcattttgcc tttgttgtgc aatgggcttt acagcatcct ttcccacagg ttagaaatgt 11340
ttccccaagt tacagggaag tggggatcct agcctggggc ctgaggaaat cttggagtcc 11400
tggctcctga acttgttccc tgtccgagtg gcacttgagg ccacccatct tataatctct 11460
tctcaaggca gatgtaagtc acctcagaag ggagaactgt accgcttcct cttttccatg 11520
aagctctcat ctcaatcttt gactgatgag tgtgaaattc taccggaacc atgcaaacac 11580
gtccaccttg ggcatctcca aggaacttgt ggggtctgca gcatacttgg ctccctacca 11640
gcctgccatg agtcatattt cttttccaga aggtggactt gcttcctggt atgttttaaa 11700
ggagcctgca ggagctctgc ttagccaatc atgatggatt tttgccccag ctggactctg 11760
catgtccaag gagaatccag gtacagcctc cattccggga aagacatcca gcccagcagt 11820
```

```
tgtcatgtgg gtaacctcag gaacccctaa ccctgtcctg gaaaaaggac aagcccctcc 11880
agaactctgc ccaaaatagc aggtgcttga tggttctgaa tttggaaagg gatgggggt 11940
gataagtact atctgtggct ctggaaaacc agctgctaca ttcaaatcta ttttccataa 12000
tggtttcttt ctgaggttgc ttcgtggcct cagagaatcc cagaggatgt tttgaaatag 12060
cctctctacc cttcaggagc atggtagttt acaggaacca actgacttct ggaactgtct 12120
atggagggag aacaggccag gtgtaggtta ctaatgtcct acatgaatag cttggtttta 12180
taagctgctg ttgggtatta tgttggggca gtctttttaa tatattgtat ttttgtacgc 12240
atttgcaaa gtggagttaa ctgttttgta aaagaaaaaa aagaaaccct tgggtgcttc 12300
ttctgtttca agggtctgat ttatttggaa aggcaagttt atctgaaatt ttgtattatt 12360
gattgcccaa ttttaaaatg ttgccttctg ggacatcttg acaaaaaata tttctcaaac 12420
atgaagaaaa taaatataat aaaaaagaaa tgtctgtggg atcagatgac tgttcttgat 12480
tttaccgttt ccatttcata agccaagctt attgacccct tcctacctgc tagtcattga 12540
gtctgagttg accaactcaa agatggaaat atcgagccag atggtaacaa attctccatg 12600
aatcgattgt tcagttttga cagaatccca attgcaagcc attagctgct tttcaaaagg 12660
gatgtaccct gtagccatat tagggaggca acaagtccag ttccccaagg gatctctctg 12720
ggtgttgact gcttcccctt gccaggtgcc cccatcggca aaatcatcag ttacacagat 12780
ttgtcctgta tgtaatattc aaggagttga ttgaggttct gagcctcttt ttcggtggca 12840
ggttgtatga agagacgtca attttttcct gttagaggga tctaatgttg tgaatttgcc 12900
tgcttagtcc caagagatta cttggcaagc aggcccctgt atttgggagc ataaatcagc 12960
cacccttgct gacagatatg taataacact gcattgagga cccttgagac tgagacttct 13020
agccggccag ctgacacaat tattatgtat atagtgaaaa tattgaacat tagaaggtag 13080
agacactaat gctaaatcct gagccaacca ctgctgacaa atctcattgg agtttgttcc 13140
actaacactt ccctctacct ctgtctctct gtgatgggga tgactttata tgagacctgt 13200
agaatgagaa attacaagca tatgacccat caaatcctgc catttataag tagaagcaac 13260
aagaacagtt tattcaactt tcccaaaggc gtccctgtac aaagtcatat tggcttccaa 13320
accccactgt gaaccatgcc caaaccccaa caattgcttt tctggacttt tttcccttaa 13380
atgaactcag acctagagtt cactacagga atttcagggg ttacagcaag agctgttagg 13440
ggatttccct atgtccaggc cagaatgtag ggattgcagg ggagtcttgg gatgaggcga 13500
ggggtgggga ctgtaagcct tttttgtaat ccctctggct ctttgcccca gcttgtatgc 13560
taaatcctct cctgtgtacc tccatttgtg cagaactgta agggctcagg gattcttcca 13620
ttctgtccct tgatttcccc agcagctctg ctccacagtc ttggtgcttt catctacctc 13680
atacctatta ccccttccct ccactcccca gaaaatcaga taagatagtg tcatgggaga 13740
ttatatgtaa cagttctgtg aacatgtgag tccgtccctt tcctgaagta ccctaccatg 13800
catatgcctt ttagtctgtc ttgaggacag agagacttct ggctcaccac taatcatctt 13860
tcttcttata gcacctgaag tgagaaaagg aagagaacca ggagttagga aatgcagaga 13920
gagagagaga aaagactctg tctatactag taagcaaggc atgtttgcat ttggtttcca 13980
gtagccagca gtgtggttca accaaacaaa cttcagatgg tttttttttcc actgaaaact 14040
ctatatcttc actgctgacg ccacttattc aggtttggca agtcattgac tcagcaccat 14100
gacatcatcc cttcttcctc ccatgcagag gagagtgaac aacaaccaaa ccatgattca 14160
aacagcttgg tttaattctg catcctgccc ctcagcttca ttaataggta ggagagtgag 14220
tggggaaggg cctttaatgc acttagtgcc aatccattcg ggaaaaagca tttgctctcc 14280
aatcccgagg agtcttctca tatcccctaa ccaggccagt gaagcaccct tgttttatca 14340
ctttccagaa tgctatgttt catcttcatt accaaaattg gcaagaaatg tgaagagttt 14400
gagactgtac cctacttgca agctcacaaa ctagcatacc tcagtttcac agatgttggc 14460
agaatatatg agattcttgg gtcagagttt gttttggttc atgtttgcat taattcccct 14520
tgccctctgc cccaaatcac acaggagaaa tgaggagcag ccagtcaaat gttgacatac 14580
aagtattctt caaactatag ttagagacaa aagctgttac aataaatgta gaaaggccat 14640
ggacaattat ctcaacaata tctgaccatt gtttctccac tatcttggct tctgacaaag 14700
tttgccatta agtatgtgtg ttgattttct ggatatcagg gctgagcaag gaaaaagtca 14760
tgaccattag atggcagtag cacacaacat gctttatttg gttggccctt tgaaaggttt 14820
caggagagtg gaacttccct cacagcagga gaccttccag aggcagcagc aagaggccac 14880
cacccagaag aggaagagtt caaggcaact tccaaaggag agaagaattg gacaggaggc 14940
tcatgtgtct atgggatgtt actcagcagc caggtgtgga gtctctgtgt cagagagttc 15000
ccatgggcat cagaggcttg gggcttttgt ccagccccag agtatgtcct atctacggct 15060
agcagatggt gtgtctggtt ttacagaggg tgcaatgtgg gtgggttcta aagggccaaa 15120
aatgtgctta ctgggggcca tatttaaagc aattgggtgt gtaaacattt gattttagtt 15180
ccaatgagct ttgagctaat ggtcttagct gtctctgaat atagggaaca agatacaagg 15240
caccatctaa agatattaat ccacttattt aacagtccat gccttggccc catgttgttt 15300
tacaatctaa gttgtatgct atttcatgga gtctcagttg ggagccagcg atgaagcatc 15360
ctcaagaccc agaatgctat aaggctacaa attaggattt taaaagccag ttctcagcca 15420
atctgcccac ttgggcagcc agtagaataa atttgaaagg agacaatttt gttttgacca 15480
gtccgtttag tcaatagagt ggcatttata tccccctttgg tacatgaccc cagctatctg 15540
taaaacttgg tgttatctac gcacatgcag gaagataatt ttagcaagca cacagcccct 15600
gtgccctgtg acccctccag cagcctgacc tagggctatg cagttatctc acaccatggc 15660
cactaaagta cagtgagatt cagcaaagag tttcagtcct tgggctgttt atctaatttc 15720
tgtgggtaat tcctgcaaca tggctatgtg tttctcttaa ttgtggtgtg ctgttccaca 15780
catatgctgt tcctactgcc tccaaaatta gtgatcctag ggaagttagt accatgagga 15840
actatttgga aagctcattt gtgacattgg ggagtcacat ctagtgtggt ttggtggcgg 15900
```

aaagtacttg agggaggggg ttccactgtt gaataaaatt ctactggaat tttgcaccaa 15960
tcccaagtgc aggaaatatt cttcagggtt ggatgaggag aagattgtga tagggaagcc 16020
atctgattcc tttacaggac ctccaggctc cctctgctgc ctgtattctc tgtgttccac 16080
tgaagggaag ttcacatggc tttgtgaagt tccagaagtt ccaggttgtg ttaataggag 16140
cagaaaatgg tatagtcaaa caagcttgtg ttatccacct tgcattcagc acctgggtta 16200
actaaatttg tcgtgggctc agcccaccat tgtctataca tttatgtgag ataagcctct 16260
ggataaggtt gttgaatgtc aatggttagg ggaatcggct catctaaata agtgaggtat 16320
atgatgcagc gttgccagga aagaagtatt acactatgtt ttctaaaatc aggttggcag 16380
aagcaaaccc tgcattaggt tagatttacc atcttatatg ttgctgtggc taaggctata 16440
ggcacgtttg gtgtagtcgg caattgcatg ttcctccttg ctgtgactgt tgtgtgttgt 16500
gcttgagttt cctgttgatt gccaataata aatagcattg atgtgttccc atgagtgtgg 16560
gataggtttt gcaggtgtta ttggcagtcc aagaagaacg ttgttagtct agccacacac 16620
caaattagat tctgaattat gaggcactcg catatgagtg aacaaaacag gtaaaccaag 16680
tattttttaa aaaaatagat atcagctggg cacagtggct catgcctgaa atcccagcac 16740
tttgggaggc cgaagtgggc agatcacgag gtcaggagtt ccagaccagc ctggccaata 16800
tggtgaaacc ccgtctctac taaaaatacg aaaaaaaaaa aaagattacc cgggtgtggt 16860
ggcatgctcc tgtagtccca gctacttggg ggctgaggca ggagaatcac ttgaacccag 16920
gaggcagagg tttcagtgag ccgagatcac gttactgcac tccagcctgg gcgacagatc 16980
cagactctgt ctcaaaaaaa aaaaaaaaaa aagatatcaa ttgtatcaag gcataggtcc 17040
cttttatgtt cccttgtatt gcaacattat tgtgtttcag tgggggacca aaagctttat 17100
ggactccatt ttagcggaaa ctttcatagg gaagaggttt gtataccttt aataacctca 17160
ggagtggccc cttgggaggt ggcagtgtga ctacaccaat aaatttcctc ccaagtactc 17220
atggacataa agtgtctact gagacactgg agactactag agggggacgg gggaggggag 17280
caagggttga aaaacaaact gttgggtact atgctcaata cctgggtgat gggatcattt 17340
gtaccccaaa cctcagcatc atgcaatata cccaggtaac aaacctgcac atgtgcccaa 17400
tgaatctaaa ataaaagttg aaaaagaaat gacaaggcat aaaaatgttt taatgaagat 17460
gaaaacattc ctcccaagtt agtttcatta catgtcttct cattatcgtt tatgtcttct 17520
gtaggtttgg agtgtgtatg gatttttaag gagccctcag aaaagtaaca ttgggcaaca 17580
actgccctca aatagtttct aagtttttaa taactgtggt tttggacaaa tttcacaata 17640
gctataagca aatagttatc aatgtgggtg gagaaactcc catgcaaaca gtagttagat 17700
ttctccctga gatcaaatat gtttcttgta agaactgtct gtgctgtgac accacgagct 17760
atgagttgtt cactgtgggt agccagcatt gcaattgctg tagagttcac ttccattgag 17820
tgttatgtaa aacttgcagt tagtatatta agattaggtt agggaagaat tcccaagctg 17880
gttgttttgt gtgtgtgtgt gtgtgtgtga tttttttttt aagaaatgtc ttggcccagg 17940
taatgtcatg tagtatgcat tgccttagaa gccaataatt gtaaaggtat acactgtggg 18000
tgtcgattgc acttattata attccaggga taagaaaaag ctatcatttt atctatgcaa 18060
tcccgtaatg gcttgtgggt attgtatctg gattatagta ttaataacaa caccaatcac 18120
aagcatcctg gttgtaatat ttggtgcata ctatctctgg gcattcctta aacgaggctc 18180
aatagatagt ggcccattgc tccatttgtt taatggcaca tgagttaata atagtgggta 18240
tcatctgatg tgaaggaaaa ggcggcccac agagcttaga aggcatctat gagtcacaag 18300
tgaaattgta caatctgact tagctggaag gatgttacta agtggtagta gagagaggag 18360
gagaagtgct aacctcagtg ctgatgtctg tggactgacc cagtgaaatg ggagcatggt 18420
tccctttgtt cccagtctga cctatgttgg gtgtccctgt ggtgtgtatg aggcatgctg 18480
tttccataca tactctgcca gcagtagggt gtggattacc agggataggg tgatccacca 18540
atcgaggtat aattgacatt ggtgaaggta ggaactttct caggtaagga aagggcagaa 18600
caacagcata ggcataaatg ttggaggtca tttgcagcca gcagtaggga atctgattgt 18660
cctctgggag tcacccaaat ggtgttacca gatacaaggg ttgttggttc aatctcctgg 18720
gtgtccggag caggtcaagg tttgttccat agtgccctta gtggagcaaa gtacatgggc 18780
agaaaatgag gatgcttccc tctccagata gtggtgggtg gtttgctagt tttatgctgg 18840
gtaatgagca gactaagact gagaatgcac ccagagtgag tggtccatgc cagatccttg 18900
tcaggattga gaggggaaaa aaatggccag gctctggatt ttctatgagg gagaaggaag 18960
aggtgaaaga gccaagttcc tttgctgtgg gcagtcactt cagggccccc atgttcgtga 19020
atactggtaa atcatgttgt gtccagtggg tacatacatt accagaggtt gcagcatctc 19080
tgttggtggt ctcctcattt gctgtgaatt aaaatatggt gcatttatgc cagagaggta 19140
tggatttaat gtaagcatga ctatgggcag tattttggc tacggggaat gctattgtcc 19200
tgcatgttct ttcaatatcc cttgttttaa aaagcccatt gtgtcattct gtgacacctg 19260
ctactgtagg accagagaat aaatgagaaa tccaagcagt gttctttggt aatgcctatg 19320
gttgaatctt ctttgcagag aagtgagagt cttgatctga ctggataact ttgtgtaact 19380
tttgtactcc caaaggccat acaaactatt ttatcagttc ctcaataaca ttagcagagc 19440
ttggtgcctt tcgtgggtaa gccaagagta agcctgtgta ggaattacta cattgttgtg 19500
gcatatgtaa tagttcctgc tgcttcagag aggggcccaa taaaattgac tggatttgcc 19560
agctctgata tagcccttct cctgtgcagt aggtccaagt ctctagattt ctggtctatg 19620
gtacggtccc taacagcttc atgtaaatgt ctctctgctg ggtctatttc tcattaacag 19680
ttgattttca tggtctcatg atgcccaaat gccctgacct ctcctttata gggtgaaggg 19740
tagctcttac caaagttctg ctgtctccct tcttttcaaa aggattaact actttggtac 19800
tttagctagt ttgtcagttt ccaggttgta ttgtttgctg tgagctgagg tatggtgtac 19860
aaagatgtgc agagtggagg ccaatgtgct gatattttcc cccacatctc cttaccccaa 19920
atgtgtcagc cttgtatgtt ccagccctga ttttgtccaa gtcccaaccc aatagcctat 19980

tagccactga ccaggagttg ccaaaaatgt gaaacttagt aaaatgtttg tcgaaggcct 20040
tctctatttc taggggtgtt gcacacagcc tggcccattg ggcactttgg cccatcccca 20100
tatggaagtt ggagtatttg aataagggta gtaggctgtt gctgtccttt atctgactcc 20160
cttataccct gtgctcactc cattggtaaa gaaaaacatt aattttcttg ttatgaaagt 20220
gctggccata ctccctccat tccccaatga gcaagagcca ggggcatttc caggggtttt 20280
ggcaagttaa cctcactgac agagtatgtg gccatttcct cacacagtag tgacactcca 20340
ggagggccag gtgtagttca atcctgtaga taccattttc attttaataa agaagcttct 20400
gttgctgatc caattttatg ggcagctctg tccattaccc agggcataac aggaatctcg 20460
gtccataggg taattttctc tgccccacca gggcttcagt tttgataagt gccccatatg 20520
ctgccagtgg gtgtctgtct agtgaatact ttaggctgag gctgagagct acttacacca 20580
aaagcccatg ggtaggcatt tacctgtcca tttggtccag aggctccagg aagcaaattt 20640
gggagcagtg gaaacttata attgcaagtc ttgtccttgg gagcactaga gggagagcca 20700
aagtattagc ctcctttagc tcaaccagtg actgtagatg ttcaggtccc tactgaaaaa 20760
aggaagcttt acaggtgtct cgatagacaa ttcagcaagg gtcagcaaat attggaggtg 20820
aggaaggcat tgtgctataa ttcctaatga atgttggggg ttctttactg atgtgagcgg 20880
tttaagcatg aggagcttgt cttttatcag attggaaata gtctgaacac tatttctgtc 20940
ctgtcaaagt gtagagatct tattcctaga tttagactgc ccttaggaat cggctcagat 21000
aatgggccag cgtttgtggc tgacttggta cagaaaacag caaaggtatt gaggatcaca 21060
tggaaactgc atgctgccta ccgaccgcaa agttctggaa aggtgaccga gtgtggatca 21120
aagactgaaa catagcccct ttgcatgcac ggtgggaagg accccagccc atcatcctga 21180
ccactctcac tgctgtgaag gtagaaggaa tcccggcctg gatccacctc agcagtgtaa 21240
aacctgcagc gcctgaaact taggaggcaa gaccaagacc ggataactca ggccgtgctc 21300
cgcctgtcag atagtaaatc ccaggtgatg tacctgctgt cagcagatct aggctttctt 21360
cttggacacc ttatacccac agtcctccag gtgccaaagt agggcatccg ttcccttggc 21420
aaacccgact gctgtggggt gtcctagcag aaggtcctcg acgtactgga gcaacacgca 21480
ccctgggtct ctggcaggaa acttctggaa gtcccgaacc aatgtttcct cgaagagatg 21540
gtgggggagt tcttgaacct ttggggaagg cgggttcaac tgtactgagt ggtgacaccc 21600
gaccccagat cttcccactg aaaggcaaac agtttctggc tctcaggggc tagtctgatg 21660
ctaaagaaag agtctttcag gtccaagcag gtgaaccagc tgtcctcagc tgacagcaac 21720
cctaacaaca tgtacagtta ggcactgttg gatgtaaact cactgtagct tgattaacca 21780
agcacaagtc ctgtactggc ctgtagtcct tggtccttgg cttgggaaca ggcaggagga 21840
aagtgtagta ttctgaggtc tgaacaggca ggagtggaaa aacacccttc cgagttccta 21900
gtgtagcctt caaattttac agacttatac cttacatggt gtaattgcca atccactctc 21960
agtgagaggt ggtttactga gggttgtgat caactgtgaa acagagtctt ggtcaggttc 22020
tgcgaggagg atgtcaccga tataatgcca aagcatagtt tctggtgaaa ttgaggggca 22080
ggtgtctatg tcctgttggc aatgactgtg caattggagg gctattaagt atcccccaaa 22140
aagctagcat cacaatgaag atgaggggaa acaaataccc caaaagcaag tgggtaaagg 22200
cgtactattg actctcaaag gtggaagtga aatgtgtttg actcaactgt gaaattttta 22260
ctaaacagaa catgttagcc aagtctacaa ctccagattc aaattctggt ggctttagat 22320
gccctggatt aatataaact taccactgat gttttgaatg acacccatta aggcaacaat 22380
gtcctataca ggtgctctaa taaaggaaac agccctgctg aactctctat ggtctatagt 22440
ctccactgat tagtagtgtc tcttagggca ggccatatgg ggaattgata ggtgaggtag 22500
taggagtcag tgttccttgt ttatctatgt cacctgtgac aggccataat ccttcagtgt 22560
cctgcttcag cttctgctgt ggtatatgaa ccacctgaac caggaggggc aggttaactg 22620
gttccctccg ggaagtgccc accagtagca taaaaacctt agtcttatgt catgagttta 22680
tctgcggcct caggaggtct cttccaataa tggggaaatg gataatcttg gaggggaggc 22740
aagcaggaca gaagcatatt tttagcaaaa ctgagccaat tttaatagtt aagaggggaa 22800
ttatcccttt aattaatccc tttaattaac ccccttccaa gcaggtagcc tacttgccag 22860
gaggggggttc cttttaaagc atgtgtgtgt gttggcatta gggagatttg ggcacccctg 22920
actgacagag ttagccagtg ttgtttgtca gtgcccaagc cacagttaat atggtgaaag 22980
ggcaactgtc tgtcagaagg ggccttccga cactgcagga tccctgtgtg agagaagatt 23040
cttagccatt tggcaagcat cagagacagg tgtggaagat aaggttacct ccaaagggaa 23100
agccagctgg ggtgggaggg ggtcgtaatc taaccctagt aagtattgca attgctccaa 23160
acattcacca gagatcatcc aatttgggac cttcttgtgc cttagggtct aacataacct 23220
cttttcgtca tctcgtggtt gatgcataca tgggatcctg gccacaggtg gtttgtttgg 23280
cccaggaggg gcagcctgag tggtgatagt aactttgatt taggccaggt agggtattat 23340
agtgttagca ggcagcacct gactctgagt ttgtctttca tgagtttcta ttaaatccag 23400
tgcagctatg gtctcttgaa ttgttttagc agtagccacc atgcccagta aagaagattt 23460
tcccaggagc cccgcccccc gaggaacaat tgagtggact caggactcaa ttcagactcc 23520
tccagtcttg tcaagtcatc ctgccccacc tcatttgttg cctccactcc aacatgtcca 23580
actggtggag gagatgtttg gccttggcta accttttccc tggacagtct tcccaggcca 23640
ttatgaggtc taatggtggg cagtcttcta atccacaaaa atacacatca gcaataaaga 23700
tgtgaacaga ggtccaatgt ggcttcttcc caaattctgc tctaattata caggttcctt 23760
tagtggggac acaatttttc catcatgtac tatcgctcct aattgtttaa tttcattccc 23820
cattaggtgg aatcagttcc ctgccagatg cattctgtag agccaggcag ccccttgttt 23880
atttggttct tggcctgggt acttaaggag agactggggc tcgggtaaga gagaggatag 23940
gactcctcct ctgagcagga acagaccctt ccttcttggc tctccctttt tttcttttcc 24000
atttttatta ctttctgggt ggctacaggc aaagcagagg gtaagttttc ccagtcccta 24060

```
ccgtggtgac actgtgctgc cttttcttct ggagagcacc tattctcctc tccagggacc 24120
tcttgtccaa actgtctctc ccaagagttt gggctcttac ctcttctcca ggtggcatcc 24180
tggtgataaa ttgctgccag caaggtcagc acatgcctca tggttaggaa tccttcttcg 24240
acctgacagc aacctcctgt agggttccgg gaggggtagg aatataaatt tctttgcctt 24300
cagtcacttt aagtaccact ctagctagct cagtccaaag gttgagatat taatatccct 24360
ctggagaggg gatagcatga tgacaactgc aacatcataa ttgagtgtaa caacagtaga 24420
ggcatgctac ctgcttgcaa aggcctttcc accagctgcc cttaggagac cagccttggc 24480
ctgatgtctc tgccttattg gctcatttac ttgcctatct gtctccgggg aggaggaaat 24540
cctcacatct cagactaccc caaatcctca acccagccac agggagcact tcccaccata 24600
gcctgttatg cagtccaggg tggttcaccc ttattccaga ggcatgcact ccctctcctt 24660
ttctattcag gtacacaggt gcacaatgtc caatgtcaac tgaggggggg acccaggtaa 24720
gtgggctgac ctccttccca agtaccttgg catctaagac cccaaaagtc agttctagtg 24780
gtgtgggttc actcaactac tggtgctacc tctgggccag gtcaatgagg tatagaagga 24840
gactgcaaaa agtatattgt tgcggtacag caatgagcga tgctaagcaa aactgctaca 24900
gaggcattct aaatgtagag tctgagcact taaggctgcc taggactgat ttttcaattg 24960
attgtcctac tgactgggta gacgctatgc caatttcttg ttcacgaaaa tcccatcctc 25020
ctcaccactt gtggtgagtt ccaagatttc tgggctgatc aatgaagaag acatgaccac 25080
gaggtggcag tagcgcccaa aaacattttt tttttttttt gagaagggag aaggagtctc 25140
actctgttgt ccaagctgga atacagtggc gcaacctctg ctcactgcaa cctccgcctc 25200
ccgggctcaa gtgattctcc agcctcagcc tccgaagtag ctgcgattac aggctcccgc 25260
cacaatggcg ggctaatttt tgtatttttt agtagagacg aggtttcacc ctattggcca 25320
ggctggtctc aaacagctga cctcaggtga tccacccgcc tcggccttcc aaagtgctga 25380
gattacaggt gtgagccacc gcgcccagcc aagaagcttt ttatttcggc ggtgctttga 25440
caggttgcca gaaaggggag gtctctcaca tcagaccttc cagaggcagc agcagagggc 25500
cactgagcag aaggggaaga aggcaaggta actcctgggg tagaggagaa taggagaggg 25560
ccttatgtgt ctgggcgata tgactcagca gcaggagagg gagtctctgg gtcagagggt 25620
tctggaggca agcagtggca tggggtcttt tatagcccct gtgttttctt taaatgtggc 25680
tagcagatgt cagtgcagtt ttgtggggca tgcaaggcag gcaagcccta agtggctaca 25740
catatgctta cttgagctat atttaaagca attgcatatg tgaaaaattc gagtttggcg 25800
ttggcaggct ttctgcctgc tgtgaggaag tataatggca atataaaggc taatatatgg 25860
aggtcatctt taacccactg atataataac atgccactta gtcagccact ctgattaatc 25920
cgactagcat ggtctgagat aaaatccatt caatctgtac tgtcaacagt actctaagca 25980
gcaaggtgag atcaatctgc aacgttaacc cgaacttaaa acgttaagcg cacacgtaca 26040
atactactgc tcctgagttt ttatctaaga gaaatgaaag cataggtcca cataaagact 26100
tgtatgtgaa tgtttatagc agcttcaatt gtgctagtga aaaactggaa acaatccaaa 26160
ttaccataca atgaaatact atactatgac aaatagtaat gaactattga tagaaataac 26220
agcatgggtg aatctgaaaa taactacgct gattttaaga agacaaaaaa gagtatacag 26280
tgtataattc tatttatata aaattctata aagcacaaac taatctatag tgagagaaag 26340
tagatcagtg gttgcctagg gacagggatg ggatggggat gggtagggag ggaagcaaca 26400
taaaggggca tgagaaagcc ttaggaggtg atgaaaatga tcgtcatttt gaatgtggta 26460
agggcttatg attattggta aggagttatg cttataaaat ataaataatt tgtattttat 26520
aaattatata aattacatat aatttcattt agttttaaat ttaaggaatg gcaatatgtc 26580
ttgcatcaaa gaaccctcaa aacaacacct taagtactgc aaagtctttg acgatgtatt 26640
tgccagttaa cttatcttta aacatcaata ataaataaaa atggtcaaga cttatttgtt 26700
tctatgtgtt tattttagtg tgctattgct ttttcacatt ctgggtcata ttttcaaagt 26760
attatttcac attgtgtaaa tgtatttttt tttcactatt agtaaagagc atctcaagta 26820
aggtcctact ttctactttg atggtatagg agcatgtttg actcagatat gaaccacttg 26880
tggacagagt tatgggtgag aaatctgata tttaaggaac actctggatt agtttccact 26940
ggtgagaaaa taaaaaagtg atgagcactt ttttccaaata ccacgtgatg aatcaagtac 27000
tcaaacatgc taactgcatt tagactattc tattaccaaa atattccaat caatgcctgt 27060
ggacagtttg taaactgtca ttcccaatgc ctcctcattc ttcaagttcc cactgaaagg 27120
tctcatagct tatcagacct ttgggggaag tgcttttttt ttttaattaa aagttaagtc 27180
atgtgcaggg cagagtgagg caaaatgcag ccagtggtta ggacctaaag acctctcctt 27240
cacctgggcc ttgagagttt gagagaagct caggaagaaa agccaaccat ctgagtccag 27300
tgggctttga agcagtggtc ttgcatctgt acatgtggga taaatgatgt gaaaaccatt 27360
aactctgctg ctgatccagc tgcagggaaa agcatttcct ctgatcaagg tgaacatctg 27420
gctggtggac ctcattttgc accaggtcct gatcgatgag ctgagcctga gtccacagtt 27480
cacctgatgg agaactcaga gtcaaagccc aggcccaagt gaggctgtct gactgctgct 27540
tgagggccta gggaccacac ccctggagtc aggagggcag aggtgaggat gacagtggaa 27600
tggccagaag gtaaagatac atgtgaatat tctatctcca ttaggtcaca tggatgaaag 27660
gcaagagaaa agtagcatat acatttttga gaagttaaag ctgataggac acaaagatgg 27720
ttgattgttc tacagacgtg gaaggcaggg gcttaggtcc tgtaaagtcc cccactgagc 27780
aaataactga gacagtactt cctagtgatg cattctgctg tcaaacagtg tgaaattttc 27840
ctggagaagg ctcaggccat cagtccctgg tccatcttct ggccccacag agatgctgat 27900
cgaacaccct ccacagggag agcattgcac aagtgattcc ctttttagaa attcttctaa 27960
gccagtcaga agaagacaag cttgcagatg gaagggaatc agaacctggg caatatcttg 28020
tagtggccga gctatccctg tgtgtggttt ggagtgtttt cactaagaca aaacagtggg 28080
agaaagaaaa accctacacc catacaaact ttgagggaat actggagctt attgtaacag 28140
```

acactggtca tgctatgaaa ttttattacc ttttctggag gggtcgcagg gctttgtgtt 28200
tgggctgctt tccgtggtgc agtaattgtc aagcttgcca aaagtgtgac agaatcacgg 28260
aattaccctt ggcagaaacg cagaattact cctaacctct actctcatcc tccacctgga 28320
ccagacagag aaacaacttc ttgagttttc agaaactacc aaatcttggc tctagctccg 28380
catccttggc cagagcatgg aaggttgggg tgagggacac ggtgaacaaa aactgcatcc 28440
tcgagctgct cgcacttcct ctttccctct ttggtcttgt gtgtggggtc agaggttacc 28500
aggcaggggt cactaaggtt gtcagtggga ggatgtgagc cctagtgggt tcctcccagg 28560
cagctcaaca tgggaggctc ccaattcccc tttctcctgc aaggaaagat accacagctg 28620
tgtcattctc cttcagagac tgcagattgg aggtctgttt ttccacaaaa cctagttcta 28680
agacatttct agcaagtgag agtgactgat tggaagtcta gtggtgacag ctacccatgg 28740
ggcaggctta aatagcgact cctccttgta gtgaacgaat ttcagatttc acacaagaag 28800
ggcaaaacca tgggctattt tctcccatga atttttgcct attttgggcc ctaaatcaaa 28860
ttgagaaaag tgtgactaaa acactcaaac tgccaatata gttagagata tacactttgt 28920
gagactaact cgggaatcta ctgccctgct tctgcaagga taaggctctg taaggcatga 28980
cttgaaatga aatccaagca ccttttatct gtttaactct ggtgcaggag aacagagcag 29040
catttaaggt ataaattaaa ttagtctatt cctgaagaga acatttgcta ctcacattta 29100
tggtggaaga tgcaatctca caacacaaca aagctctcct tgtgagtgga gcaggccaat 29160
actgggtagg gctagactat agggctcaca ggctctatgc cctaagctac tgatgcccct 29220
ttgtgccaga ggctgggttt tgcaacaaac tcagttcagg tctgggaaca ctgggcccac 29280
cgagaatcct aaatagtcaa ctaaaatccc tgaaataccc aggcctgttt ctttaatcat 29340
tagtattgtt tgcatacctt taggaggtac aagcgcagtt ttgttacatg gttatattac 29400
ccactggtga agtgtaggct taccaaagca aaaacagggt ggtagataat ctcccttgtg 29460
caatcaacaa ctataaccct ggcaggtagg gtgcctacta ttcatactga ctacagagag 29520
cctggactcc tctaacctga taaaggatat gaagccagtt atctatttat tacctcttag 29580
ctccaaattc accagtcaat atatattctt cgataataga gggattcttt aagcatctct 29640
cctttaaagt agcatcaaag agcacgatgt tacacttact taatagaggg ctctggaggg 29700
acagacattg aaaaaggaag ggtacttcta tgatttctgg ctctcagagg tgtggaggta 29760
aggacattca ggatgctttg tcccaggcct gggcccagaa caactgtccc tcagtaaacc 29820
tgcagtcctg gtgtgccctg atgatcagct tcttgtggcc ctcttgatag ggacatcctg 29880
tactccaagt catctgccca ctgcttccct ctgctcactt gctccgcagc cacacacaca 29940
cacacacaca cacacacaca cacacacaca cgaccaggtt catgtagtcc tttcaatact 30000
gcagccctct caacaccgag cccccaccct gatcctgtgc aaggggcatt gtttactcca 30060
ggcctccaac tgagacagga ctcccaaccc ctacttcaca ccacatacta ggcaccagct 30120
atggcttgtc cacactacca tgctccaaag ggtgccttct tgcttcctct gcgaccgtag 30180
accagttctg cctctgtaga ccagcttagg tactggcaaa ccagtggaac ttctctgcca 30240
tccagtaggt tcttctccaa ttaggtataa aatccatcct tggggagaag ctttcattct 30300
aagcttgtct ttccttgggt actctctccc agccccagaa aaccccacat agagttgtct 30360
tgtatcctat acaagatctt ttatcatcgc ttaatgattc tttatattaa attttcccct 30420
tctagatcac tgtgtgcttt ctatctcctg attggatcca tattgctgca gaacacaaca 30480
tctacagcaa gcatcattct taatggagaa tcactgaaag ctttctcttc aagagctaca 30540
tcaagtaagg atatccacta tcaccacttc tatgcaatag tttactgggg gtagggggcgg 30600
ggtcccaggc agagcagtag ggcaagaaaa aggaataaga tatttggaaa ggaagaagaa 30660
agactggaac tattttcaga tgatatgaca gtgtatataa aattctacaa agtattacag 30720
acaaattatt agaatcgata gaagagttta tatgcttata ggataaaatc ttaacgaaca 30780
aaagccaatt gtatgctatt tagagctata aatttagaca ataaaaattt caaacatatg 30840
atttgcaaaa tattaaatgc ctaggataaa aaagttgcct tgtgggaaaa atataaaata 30900
atatttgggc aaattaaagg tgatcttatt aacagaagag atagaccata accacgaatc 30960
gaacattcaa tattgagaag tcaaatctaa aatatcagca caattcaaat tagaacccca 31020
aactagctgt tggttgaagt ttttacgatt agtttataat ttaaatggaa ttgaaatgga 31080
ccaaaagtag ccaagacact tttgaagagc aaggtagaat tgatttgcct aatccaaata 31140
tcagaattga ctatggatct gtagtaatta agacagtgtg gtatttcaaa cagacaagtg 31200
cttaatagag agtatagaac tagcccacat atgtgtagat acttgattta tggcactgta 31260
aatcaactgg caaaaggcag acttttcaat aagtggttca ctgagaaaac tggatattca 31320
cttacttaca tacatatata cataaatgga actctactta acacaataca caaacatcaa 31380
ttgcaggtgg attaagacct gactatgaag gacaatagca gacatatttt agaagaaaat 31440
atagagaatg ctttcatggt ccagggttcc ttaaaacaga aaagatttct taaaacaaaa 31500
aagcactaac catcaggaaa agaatgatga aattgactat attataatta atagcttctg 31560
ttcacaaacc ggagcacaaa gcaagtgaat atacaaacag agcaagggag tattttggcc 31620
gcaggtgtgg aagagtattt aaaatattta acagactcat taagtcagta tcagatgaac 31680
aataaaatag aaacgtgggt taaaagaggt aaatttgaat aggccttcct caaaatagaa 31740
actccaaaag gctcattaac acaaaaatgt gctaaagctt aatagtaatt tggatattgc 31800
ccaccagtag accggcaaaa tttaaattcg ggcaatactt ggtgtaggtg aacatgtgga 31860
tcagtaagga ctctctatgc tgctggtggg attgacaact ctcccaatca ctacagagaa 31920
caatttggca tcatctagta aagctgagga tatccattcc ctatggccct gtgattctat 31980
tcttggatat ataccctaga gagattttta cacgtgtccc attagaaatg tgcaagaatg 32040
ttcacaataa tcattgaaaa gtagaagcaa tcctaatctc catctgctgt agaatggagt 32100
ggaataatca tacaataatc actttgctat aagcatatgc tggaaagaca gtcgatggag 32160
acaaaggatc tacttactta tagctattat gaacaacatg gacgagtctc tcaagtataa 32220

```
tacagagtga aaaaagcaag ctataaaagg aagcatgccg tatgattcca cttatacaac 32280
ttcatatcaa ctctattgtt tagggatgcg tacaaaagtg ataaagctat aaaggaaagc 32340
agtgaaatga ttattacaaa gtcggagcaa ttactatatc tatataggag agtgtggttt 32400
gtgactggaa ggagtacagc agctgtattc tgggaggttg gcagtggtct attgcttgac 32460
cttggtcctg gttaagtagg tgtctgcttt tgtaaaataa tctaaactat acagatacgt 32520
tatatgcact cttctaattt cagatttgaa aagtgcttcc tccagaaaaa gtaatagaaa 32580
gaaaagtatg tggagtagaa attttgatga ggttcgaata gttatggttg aaaaacttga 32640
ttttcaagag accaagtgtc cactgttatt aaaagagggc gagagagctg ggagtattta 32700
aacaacaggt tacacccccc caccccccacc accaccaatc tgttcatcac tctaagcctt 32760
ctcatctcag atatttcacc ctatatttgt gtcaattcca aactcattcg tgacagacaa 32820
attggaggat tgtcgtcatc attaataagg acattaggca ccaaaggccc agcaagactg 32880
ggccaccaca cttaaatgtg agcaggtaca ccggggcgag tggacaagat cgtctgtgca 32940
cattacttgg cagagtccat tgagtgactg agggtttaac cttaacacat ttaactggaa 33000
ctcctgccca gagaatgtgc tggagaatgc agaataaact gagcaatgcc ctcattgtgc 33060
tatgagagtg gacatgaagt ttatcatggt acaaccgatc gtcgaagggc aaagagttgg 33120
tttaggttgt gcactgtata gggagaagcc aggggcgggt tgggtttttt tccagatttc 33180
agtataaatt gtttcatcct ccagactttg catttgtagg ctgaagagtg ctaatctgtt 33240
ttggccgcat ctctcactca ttctaatctc tgttatcagc agacaaaaat tgaactatgt 33300
ggcaaattgt accgagcact ttgctcaaga caattgtgag tatggaagtt gtcccgcgca 33360
gaggtgggaa ggggaagggc aatttccgca gaaatgccag cactatggtg gtttaatgct 33420
gacacagaga cagttttttc tctcaggaat tgtctttcat ccatcaatcc agccaggtag 33480
ccagccaata aacaaatttt aacacacaaa aagtatgaag caacatgtct aacactggga 33540
ggggctggcg tgtgtttatt tgggtatata caatgagtca cggattaaaa ttgcttttac 33600
cagatagata cctaggtgtt ccacagcatt attgaacaag atgtcgtcat ttccccatta 33660
cttcagaaga cctccttaat tacttatatt aattgtagaa tatatttatg cataccaggc 33720
tctacctctg ggctttctat tccgttccag agacccagta ccagaatatt taaatcactg 33780
tggcgttgaa tacattttaa tatctgatag ggccagtttc tgcgcattgc acattttttt 33840
ttccccttttc aggaacgtgt tcaggcccgc ggcaggggggc gggatcgcgc ctcctcctcg 33900
gctctggttc cagccgagcc tctcggacgc agagatggaa atcccgaagc tgctcccggc 33960
tcgcgggaca ctacagggcg gcggcggcgg cggtatcccc gcgggtggcg gccgagtcca 34020
ccgaggccct gactcgccgg ctggccaggt ccccacgcgc cgcctcctgc tgccccgggg 34080
cccccaagat ggcgggcccg ggcggcggcg cgaggaggcc agcacggcat cacggggccc 34140
tggcccaagc ctgttcgcgc cgaggcccca tcaacctagc ggcggcggcg acgacttctt 34200
cctggtgctg cttgacccgg tgggtggcga cgtggagacc gcgggctccg gtcaggccgc 34260
agggcctgtg ttgagggagg aggccaaggc gggcccgggg ctccaggggg acgagagcgg 34320
cgcgaacccc gcgggctgct ctgcgcaggg cccccactgc ctgtccgcgg ttcccactcc 34380
ggccccgatc tccgcccccg gccccgccgc ggccttcgcg ggcacagtca ctatccacaa 34440
ccaggacctg ctgttgcgct ttgagaacgg cgtcctcacc ctggccagcc ccccaccaca 34500
cgcctgggag ccagggggccg ctcctgccca gcagcccagg tgtctgatcg ccccccaagc 34560
tgggttcccg caagccgcgc acccgggtga ctgcccagag ctgcggtccg acctcctgct 34620
agccgagccc gcagaacccg cgcctgctcc ggcgccccag gaggaggcgg agggcctggc 34680
cgccgccctg ggcccccgcg gactgctggg ctctggtcca ggcgtggtgc tgtacctgtg 34740
ccccgaggcg ctgtgcgggc aaaccttcgc caagaagcac cagctgaaga tgcacctgct 34800
gacgcacagc agcagccagg gccagaggcc cttcaaatgc cccctgggtg gctgcggctg 34860
gaccttcacc acctcttaca agctcaagag gcacctgcag tcgcacgata aactgcggcc 34920
cttcggctgc cctgcggagg gctgtggcaa gagcttcacc accgtgtaca acctcaaggc 34980
gcacatgaag ggccatgagc aggagaactc gttcaaatgt gaggtgtgcg aggagagctt 35040
ccccacgcag gccaaactcg gcgcccacca gcgcagccac ttcgaacccg agaggcctta 35100
ccagtgcgcg ttttctggct gcaagaagac atttatcaca gtgagtgctc tgttttccca 35160
taaccgcgcc catttcaggg aacaggaact gttttcctgc tctttccctg gctgcagcaa 35220
gcaatatgac aaggcttgta ggctgaaaat tcacctgcgg agtcacaccg gcgagagacc 35280
tttcctttgt gactttgatg gctgtggctg gaacttcacc agcatgtcca aactcttaag 35340
gcacaaaagg aagcacgacg atgaccggag gttcatgtgc cctgtggaag gctgtgggaa 35400
atctttcacg agggccgaac atctgaaagg ccacagcatt acccacctgg gcacaaagcc 35460
tttcgtgtgt cctgtggcag gctgctgtgc caggttctct gctcgcagta gcctctacat 35520
tcactccaag aaacacctgc aggatgtgga cacttggaaa agccgttgcc cgatctcctc 35580
ttgtaataaa ctcttcacat ccaagcacag catgaagacg cacatggtta aaaggcataa 35640
ggtgggccag gatctcttag ctcagctaga agcagcaaat tctctcacac ccagcagtga 35700
acttaccagc cagagacaga atgatctcag tgatgcagag atagtgtctc tcttctctga 35760
tgtacctgac agtacttctg ctgcattgct ggacacagca ttggtgaact ctggaatctt 35820
gactattgat gtggcttctg tgagctcgac tctggcaggg cacctccctg ctaataataa 35880
taattccgta gggcaggctg tggaccctcc gtccttgatg gccaccagcg accctcctca 35940
aagtctggat acctctctct tttttggaac ggcggccact ggttttcagc agagctcctt 36000
aaatatggat gaggtctcaa gtgtaagtgt ggggccattg ggatctctgg actctttggc 36060
catgaaaaac tccagtccag agcctcaggc tttgacaccc agcagtaagc taacagtgga 36120
cacagatact ctgactcctt cgagcaccct ttgtgaaaac agtgtctcag aactactgac 36180
accaaccaaa gcggagtgga acgtacatcc taactctgac ttctttggac aggagggaga 36240
aacccagttt ggattcccca atgcagcagg aaaccatggt tctcagaaag aaagaaatct 36300
```

```
tatcactgtg actggcagct catttttggt atgaagcaac tctattcatt ccttgccatg 36360
tggctaactt ttattacagt caattttgag gatattctgg actaaatatt taagtgcagt 36420
catttctttt tggtttgcaa aaagagcaca gccctggact atgagtttgg agatctaaat 36480
tctgatcttg agtctggaac tgacaagttg tgtgaccctc agcaagtcac ttaacctatc 36540
tgagccttaa tttccttatt tataaattgt ggtggtttga acacattgct cataaggtct 36600
tttcagtttt gttttgtttt gttttgtgat tttgtgcttt ttcttgaaaa ttttcgggca 36660
ttttgcaatt attattgttt gtacttgtaa tcagaagcgg tttgagccct gtagcactaa 36720
ataatgaaag attgaggaac tggtgtttta cattaaaaat ttaatggaaa ttttacacag 36780
tacgaaaatc taatgataga gctcaaaaaa aaagagctaa aaataggagt tggttcttct 36840
tagctgttta tccttctaac cttttttttta aatgatgaag gtatgttttt gttgtggaaa 36900
atacaagggc ttttgttatc actcagaccc agagtgaaat gtttgctttg tggttttgaa 36960
taagttggcc tttaataagt tatttagtct ctcttagcct cagttttcta tctgtaaaat 37020
ggtcatagca atgtgaacat atgtgttaca tcctagactt atttttctac cccagtaggt 37080
tgtattgaaa ggaaaatgtt atatgtgttt cagcatgttt tggtgaatct tcattccctt 37140
tcctgcccct tgttttccct cctcaaaggg gagaaattag cacaaattag tatcaggatt 37200
gtgcaggaaa taaacatttg tgaaggttaa gaaagaggaa aaggaagtta tttcttcaac 37260
agattaagga tttttctaca cacagttctt ttgtggcatt ggccacatgt ccattagacc 37320
aatttgatag tatcttcggt ctgcattcaa agccagctca tgcaatgagt attcagccta 37380
ttcttccaag acaatctgga catagaccag agggagattt ttctcccctc tgtgccctag 37440
agagctcatt gctggcttac tcttagagtt gaaatgagag ggttttg               37487
```

<210> 23
<211> 2121
<212> DNA
<213> Homo sapiens

<400> 23

```
ctgctcgcgg ccgccaccgc cgggccccgg ccgtccctgg ctcccctcct gcctcgagaa 60
gggcagggct tctcagaggc ttggcgggaa aaaagaacgg agggagggat cgcgctgagt 120
ataaaagccg gttttcgggg ctttatctaa ctcgctgtag taattccagc gagaggcaga 180
gggagcgagc gggcggccgg ctagggtgga agagccgggc gagcagagct gcgctgcggg 240
cgtcctggga agggagatcc ggagcgaata gggggcttcg cctctggccc agccctcccg 300
cttgatcccc caggccagcg gtccgcaacc cttgccgcat ccacgaaact ttgcccatag 360
cagcgggcgg gcactttgca ctggaactta caacacccga gcaaggacgc gactctcccg 420
acgcggggag gctattctgc ccatttgggg acacttcccc gccgctgcca ggacccgctt 480
ctctgaaagg ctctccttgc agctgcttag acgctggatt tttttcgggt agtggaaaac 540
cagcagcctc ccgcgacgat gcccctcaac gttagcttca ccaacaggaa ctatgacctc 600
gactacgact cggtgcagcc gtatttctac tgcgacgagg aggagaactt ctaccagcag 660
cagcagcaga gcgagctgca gcccccggcg cccagcgagg atatctggaa gaaattcgag 720
ctgctgccca ccccgcccct gtcccctagc cgccgctccg ggctctgctc gccctcctac 780
gttgcggtca caccccttctc ccttcgggga gacaacgacg gcggtggcgg gagcttctcc 840
acggccgacc agctggagat ggtgaccgag ctgctgggag gagacatggt gaaccagagt 900
ttcatctgcg acccggacga cgagaccttc atcaaaaaca tcatcatcca ggactgtatg 960
tggagcggct tctcggccgc cgccaagctc gtctcagaga agctggcctc ctaccaggct 1020
gcgcgcaaag acagcggcag cccgaacccc gcccgcggcc acagcgtctg ctccacctcc 1080
agcttgtacc tgcaggatct gagcgccgcc gcctcagagt gcatcgaccc ctcggtggtc 1140
ttcccctacc ctctcaacga cagcagctcg cccaagtcct gcgcctcgca agactccagc 1200
gccttctctc cgtcctcgga ttctctgctc tcctcgacgg agtcctcccc gcagggcagc 1260
cccgagcccc tggtgctcca tgaggagaca ccgcccacca ccagcagcga ctctgaggag 1320
gaacaagaag atgaggaaga aatcgatgtt gtttctgtgg aaaagaggca ggctcctggc 1380
aaaaggtcag agtctggatc accttctgct ggaggccaca gcaaacctcc tcacagccca 1440
ctggtcctca agaggtgcca cgtctccaca catcagcaca actacgcagc gcctccctcc 1500
actcggaagg actatcctgc tgccaagagg gtcaagttgg acagtgtcag agtcctgaga 1560
cagatcagca acaaccgaaa atgcaccagc cccaggtcct cggacaccga ggagaatgtc 1620
aagaggcgaa cacacaacgt cttggagcgc cagaggagga acgagctaaa acggagcttt 1680
tttgccctgc gtgaccagat cccggagttg gaaaacaatg aaaaggcccc caaggtagtt 1740
atccttaaaa aagccacagc atacatcctg tccgtccaag cagaggagca aaagctcatt 1800
tctgaagagg acttgttgcg gaaacgacga gaacagttga aacacaaact tgaacagcta 1860
cggaactctt gtgcgtaagg aaaagtaagg aaaacgattc cttctaacag aaatgtcctg 1920
agcaatcacc tatgaacttg tttcaaatgc atgatcaaat gcaacctcac aaccttggct 1980
gagtcttgag actgaaagat ttagccataa tgtaaactgc ctcaaattgg actttgggca 2040
taaaagaact tttttatgct taccatcttt ttttttttctt taacagattt gtatttaaga 2100
attgttttta aaaaatttta a                                          2121
```

<210> 24
<211> 1320

<212> DNA
<213> Homo sapiens

<400> 24
atgcccctca acgttagctt caccaacagg aactatgacc tcgactacga ctcggtgcag 60
ccgtatttct actgcgacga ggaggagaac ttctaccagc agcagcagca gagcgagctg 120
cagcccccgg cgcccagcga ggatatctgg aagaaattcg agctgctgcc cacccccgccc 180
ctgtccccta gccgccgctc cgggctctgc tcgccctcct acgttgcggt cacacccttc 240
tcccttcggg gagacaacga cggcggtggc gggagcttct ccacggccga ccagctggag 300
atggtgaccg agctgctggg aggagacatg gtgaaccaga gtttcatctg cgacccggac 360
gacgagacct tcatcaaaaa catcatcatc caggactgta tgtggagcgg cttctcggcc 420
gccgccaagc tcgtctcaga gaagctggcc tcctaccagg ctgcgcgcaa agacagcggc 480
agcccgaacc ccgcccgcgg ccacagcgtc tgctccacct ccagcttgta cctgcaggat 540
ctgagcgccg ccgcctcaga gtgcatcgac ccctcggtgg tcttccccta ccctctcaac 600
gacagcagct cgcccaagtc ctgcgcctcg caagactcca gcgccttctc tccgtcctcg 660
gattctctgc tctcctcgac ggagtcctcc ccgcagggca gccccgagcc cctggtgctc 720
catgaggaga caccgcccac caccagcagc gactctgagg aggaacaaga agatgaggaa 780
gaaatcgatg ttgtttctgt ggaaaagagg caggctcctg gcaaaaggtc agagtctgga 840
tcaccttctg ctggaggcca cagcaaacct cctcacagcc cactggtcct caagaggtgc 900
cacgtctcca cacatcagca caactacgca gcgcctccct ccactcggaa ggactatcct 960
gctgccaaga gggtcaagtt ggacagtgtc agagtcctga gacagatcag caacaaccga 1020
aaatgcacca gccccaggtc ctcggacacc gaggagaatg tcaagaggcg aacacacaac 1080
gtcttggagc gccagaggag gaacgagcta aaacggagct tttttgccct gcgtgaccag 1140
atcccggagt tggaaaacaa tgaaaaggcc cccaaggtag ttatccttaa aaaagccaca 1200
gcatacatcc tgtccgtcca agcagaggag caaaagctca tttctgaaga ggacttgttg 1260
cggaaacgac gagaacagtt gaaacacaaa cttgaacagc tacggaactc ttgtgcgtaa 1320

<210> 25
<211> 96598
<212> DNA
<213> Mus musculus

<220>
<221> misc_feature
<222> (1)...(96598)
<223> n = A,T,C or G

<400> 25
tcttctgaat atcatgttta gattatgtaa gtattcaccg gctttctgtc actataaacc 60
aatacctgag ttgattaatg aataaagaaa aaaaggttta gttgatgagt tttgaaggtt 120
ccagtctacc agtctgttac tgcagacgtc caccgtgata gtatgtcatg gccagagcac 180
ctggcaaagc ataaactacc tgctcgtcaa tcaggaagca aatagaaaag agaactggtt 240
gcggtcccac aacatccttc caggtcatgt cttagtagag cacttctccc aggagaagca 300
ccctgttggc cttgccgtca acacatgaac agcttgggga ctcctactga ggtaacagta 360
cccattctgc atacattccc tgagattaac tgagacttga ttcatggccc gctgttttgt 420
ctgcctttgt gagtgttctg cttgcagccc tactaaacac agatggccaa tgtggtattc 480
agagtagtgg gcctttaaga ggttatgagg tcatagcaca taccctgccc ctgccatgaa 540
taggatttta aagggggctg atgaaggagg gttgtcccct ctgactcttc cacctttact 600
gtgtgacaat agagtgattg tttcctcgtg acattcctca tttgcgtgct ttgaccttgc 660
acttcttgcc tccagagtga taaaaaataa acttatgttt tttataaact acccagactc 720
aagtactgtt taacagtaca gacatatgaa gacagttggt cgtgtttgta gtcagcaccg 780
aaagaggaaa gttgaaaact tccactctac ttgtggattt ctgtctcttc cacaaatgtt 840
taggtctact actttgtact tcatgtattt tggagaccta ttaataagtg catgcaggtg 900
gaggtcatta tttttataag ggctcagtag tgcatacata taaggttcca acactcagca 960
tctagaggca ggagggttgc tgggagtttg agcccagcca gagctatcta tagagaccct 1020
gtcataaaac aacaaaatta ttactgccca ttgatgacct ggccccttta ttcttatata 1080
tatatccttc tttgccctta gtaatattcc accccctgat cttctctatt ggtggttatg 1140
atataaacta ctttgctgtg tttagtattt gtattattag cacagtatcc caggcttctg 1200
cttttaaaca ctttgcgtct ttcatgttaa aaataattca taggaaaagc attttcttgc 1260
gtcttgcttt ttctgtactg tttaccatga atgcttttta tagctgagtt tattttattt 1320
gcatttaata ttggacctct tctttatcca cattttgtct ttcattgtca tcttttaaat 1380
gaaataaacg tagcacttga agagttccat tttatctccc gtctggtagt tctgtaaaac 1440
ggtggctttc tgtagtagga ctttctgcat cctgcgtcta tagcgagtga agcgctatgt 1500
acctaatgtg gaagctttga tttgttctca cctctctgct agtgctggta gacttttttc 1560
cctccatggg ttcagaatac tccataatat attgtgttgt tcaatgtgtt tgttgtacag 1620
taagtttgaa catactgcaa agcgaaagcc ccatgtgccc acacctttat caccagtagt 1680
gttttccttg tttcttaaag gtccaattct atctcatttc ccttcaccac aaagatctgc 1740

```
ttttaggatg tcttctgtca cttctacgcg ttatgaattc tctcagctct gctttattag 1800
aaagtgtcag tttaccttta ttgtcaaacc attctgggac aaagctacct gcttctttgt 1860
ggcttcaatg tttttgttgt tcattataga tatcatgatg tgaagagttt gcatgttttt 1920
cttttattca gagttgagtt ttgtcctagt aggccttact aagatgcatt taaagtcgcc 1980
cttattttat gatgtgttct tgactcttat aacacggttt tcccctagac tgctataagc 2040
acacatgttc agaacgttta ctgatgtctc ttcagcccac gtgattgcac cctcagtgtc 2100
tctcagctct gtatggcgtc tgtaatctta tcagagcatt tatagaacaa cagtggcttt 2160
cactgagctt tttgggatct ctgttaggat ttcatcagag taaaggtggg ctgattatag 2220
atttttgagg ctttgcacat ataccatttc tctgaccaat gagatcactg ctctttattt 2280
gaactctgct tcctgtggcc tggattagaa agtgccctca gtcagaaggt gagcttggag 2340
ttcacttaaa ctgtgcagcc tgtatctctt gtttagtgtc tcaggctatt gtgtttcata 2400
attacatatg aatttggcaa gatcaaatag tggttgattt ataatgactg aaattagatt 2460
tgtgtggatt tttaaagatt tttttttca agacaggggtt tctctgtgta gccctggctg 2520
tcctggaacc cactctgtag accaggctga tcttgaactc agatctacct gcctctgcct 2580
ctggagtgct aggattaaag gcatgctcca ccactgccca gctgtagcat gctattaatg 2640
attttgaaag acattattcc agggcattta tatattcaat atgttggcta gctagcattt 2700
ctctacagct ttgttttgaa actttctcat catcccagaa gagtgccttg aatgtggtaa 2760
atgatggctc ccttcccagt cttcactcac tcccctggcc tctggcaagc aatgatgtgt 2820
tcctcttcct acgaatgtgc ttgttcttgg tattcaatgt gaagggaatt gagtataggt 2880
gagcgaccct ttctgtctgg tttcagtata aatcatttag tctactggtt ttgaaataca 2940
aaatcaaaac tcccattgct ggctgtaagg cacaccattt aaggagactg taaatatata 3000
atgtatgatt aagttgcata gaaacagtga accatagtgc aaaaccacca ggaaatgtta 3060
tagaccttat ggactgaggc gataacagtg gaagagaaaa tacaggtttt atggaagaag 3120
ttgattttga agtaccatgt tgtggaatac ataatatcta aacaggatag aatctcgagt 3180
gtgaagagat gtacacactg gaaagaaatg acatagagct gttacatgtg tgtgtgtaca 3240
catgcgtgtg tgcgtgtatg cagtgagcat tcacatgtgt agaggtcaaa ggacaatttt 3300
ggaagctggt tctttccttc gagcatgtaa gttccaagta aggacttagg tccttgggct 3360
tagcagcaag tgtttttacc tgcagagccc tcttgttggc caagcaatgc atctttaaat 3420
gattgatttc cctatagtaa agtgcttgtg ttggatcatg gtgatagttg tggtgcttat 3480
ataatatata atataatata atcatatgct gagtagttgc tctttggact tcactatgta 3540
aagcacctgg ctgtgatctg aaagatattt aatcaggaag ataccctgcc atgggggtga 3600
gcggtagaaa agctgtttct gtcagagtga ggtaataagg tctgccctag ggtgtggcta 3660
tggacataga aaggaagtgc acacacaaga aacagtgtga ggaaagaagt gagcgacaac 3720
aagactgttc actgcattgg ttggagaagg ctacatttgc aagaatgaga cagtcaggag 3780
tcgggcttgt tgtcgagtag taaactgtag acattagggt tgatgggttt aatgtatcat 3840
taacttgttt tctaatggca tcgttaccta aagctaacag ggctcattta ccaaatacac 3900
cttcagagcc ttaacagttc taatcatgta ctgatgtcac gggggttggg ctcgtatctt 3960
ggatagcttg ggttttttgt tttgtttttgt ttcttttttc tctttccttt ttttaaaaaa 4020
aattatttat tttttgttca attgagaact cagtagccga tggagaaaag ccagtgcaaa 4080
gtccagaccg tgactaagag tgtaattaaa gctgtgatac tgaattgcca gggtaattaa 4140
cccatgctcc acacgccctc tgttcccatg acactgacca agctgtatcg tgtaggctgc 4200
tgtctttcca tttactaacc gttctgattg gctagccttt gctgcggcga gataaatcat 4260
gaagaagcac atagaaacac agatatgcct catttcttgc gacctgtgac tgtgtgtggt 4320
gatttgtctg gctgctctgt ggaggtcctc agctgctctg ccaccgaggg ctcagcctgg 4380
gaagtcttgg ctgagctctg ctgcgtttcc actccacacg tgggttctca tcctccactg 4440
ggctggccag gcctttccct tccaggaaag aaaatccagt cattgaacgg cttcttgaaa 4500
gccatgttca tgactcatgt ctgcatcctc ctgttggtca gggcctgcca agggcctgac 4560
aacattcaag gggcagagat gtcaatttta ttgtccctgt gttgtgaggg atagaaaagg 4620
gcagtggcca ttgttttgag cctactacag ctataagccc tgagttcaga ctatgccact 4680
cttgtgtagt tttgtttctt tggaaactga cagggtaggc aaacctcttt ctgatgggtt 4740
gatgaagggc caaaaacatc caggtaacat ttcaaacatg gtttccctgt gatacagacc 4800
atggcataca caagtatcac aaatctgttc ctacagcact gtggatgaca cacacacaca 4860
ctctctctct ctttctctct ctctctctct ctctctctct ctctcacaca cacacacaca 4920
cacatgtttt aatttttagt ttagctaact gtggcttttg tgagatattc tctctctctc 4980
tctctctctc tctctctctc tctctctctc tctctctctc tctctctttc tctctctctc 5040
tttctctctc tcccttcacg tttggattgc cttgtaagta gctcatctga aattcatgca 5100
ctgtaaggtc ctattctgtt ctctgggtta ctctgtagaa gagttctatg ggctcaccag 5160
gtactagatg taggaacaga aagcacactc gggagaaggc atgttgcaga aacgagagaa 5220
acatgctgag actcctaatg tgcatctccc cagtggagac aacacaattt gactggacag 5280
tgtgtgcatg aaggctaaag agcgctcagg gaatatagag ggagcctctt cccgctccag 5340
tcaccgtgac agcatgctac ttcgtgtgtt ggcatgcaca aaattggcag aacgcctcaa 5400
gtgtggagaa ttcaccatta taacaagatt atagtaagca aaaaatcact ccacatcacg 5460
tagaagaggg agttggcaaa ggcggctggt ttaaagcagc tctttgcaat cttctctaga 5520
gggtcttatc tgaaagcaaa caaaaaacaa caggaacagc ttgaatgtta gcagttatcc 5580
tggcaggcag cactccaacc cagcccagcc cacccagccc ccagcacagg tgtgtaaccc 5640
tttagtagcc tacttaatat ggctgaagag atagatggct taactagtaa gagtgcttac 5700
tgctcttgca gaggactcag gctggttcct aactcccaca tgatgactga caacctcctg 5760
tcacgctggt tccaggggat ctggcactcc cctccttgcc tctgccagta cccttacatt 5820
```

```
tgtggtgcac agtcatgctt gcaggcaaaa cactcaagcc acataaaata aaaattaata 5880
aaaccttgaa aaaaagatcc tagctagcag ttaatatcat gagtagacta gctccagcca 5940
gaacttttga gaggcttgaa gcaaagatgt tttaaatga ataatacttc tgtcaagagg 6000
aaaatcacat cacccattac gctagtacaa gggccataag ctgtgcagtt agagaggcca 6060
gctctagtct gtcagcctga gcaaatgact cattctctct gaagcttggc tcttcaggtg 6120
gactctactt tctgggctca gcatagcaag agggcagcat acgtaagcat tacctccggt 6180
tccttagtta tgtggaagca gtctgtcaag cttagctttc cccatacaaa ggagagcact 6240
tgtcctgagg ccagagcact cactggcttc tcctcctcag cgtaactggc accagggatt 6300
aggtaaattg tccctgggtt aatcctgagc cttccaggat ctttggatca cttctagaag 6360
atggtaggac cagccatcca gtcctgtgag cccagaatac ttcagagaat aacttctaaa 6420
ctccccctca gggaaaaata caagttatag ggtaatggca tgcaaccact tcagactcaa 6480
atatgttaga ctgtcagtag ctattggcag aaaatttatc tgtctttgct acagttctat 6540
tgctgtgaag agacatcatg accaaggcaa ctcttctaaa agaaagcctt taaatgggcc 6600
tggcgtacaa ttgtagacgt ttagtccatt atcatgatgg cagagagcag aggacacagg 6660
caggcacaga atcaggagct gagagttctg catcatggtc tgtaggcaga gaaagacaga 6720
ctctggactt gggatgggct tttgaaacct cagtgctcct cctcagtgac agacttcctc 6780
taacaaggcc acacttcgta atccttttaa tcctttcaaa tagtgtcact ccctggtgac 6840
taagcacaca aatatatgag cctgtgtctt tctcaggaca acgaaaagtt tatcttgcat 6900
ctttcttcct ataatttatc atgagtgagc tattagactg tctggtgagt taacatatgt 6960
tctgtatctc ccactcacta ataagcttcg catggattag ctagctgagt gtttcccatg 7020
ggtctgccag cccattaggc tgacactcca tcatgaatgg gtttcttcgt cagctctcag 7080
atttgcattt gcagtgcgtg catgtattgt tctaggtcat tgtcacgatg ctcctggtga 7140
aataatattg aaaataaaag aagtgggaaa ccaggttctg taaatctcag tgtggtagca 7200
tgcacagaaa cattgattta tttttaaaag aaagctttta gaaatcagca ttactgaaaa 7260
aaaaaaagtc cattgccagc ctttatcatt agtgtgacat actgtcagac aggaggacac 7320
acagaaaggg ggacgcttct gaagagcagt caggaataat taggagtgtt gacgaattca 7380
tacttcatat tttatttaac actgtcaggg cattttaggt tcaatgattt aaatttagat 7440
tcagccccct ttttattcta atacatgtct aatgtgtatt agataaattt ccatgtcact 7500
gtacaaatct ccccattgtt atagcaattc cataatattt aacttacagt tgtagaacaa 7560
gaatgcaaac agttagatca gagcacatca caaactcctg aaaatcgact tgcagaattt 7620
acgccctctg aatattttac atggtataca ctttatgtgg ccatagccct tacattatgt 7680
tttcttcaaa aaattctcta tggaaaagaa actttaacat tttataattt aatctttagc 7740
aatttattgt gcgtgcaaga tacaaaacaa aaaatgttgt gaaaagtctc ttttcccagt 7800
atctttgccc cattgctaaa caaaacagta cattaaatga cacctaagtg acacctgtta 7860
tggttactag aattttaaac caccctgcca ccccaccccc tccctgattt ccaagtgcac 7920
cttctagctg gtgtctgacc gcctactttt catagtgcgc attgtggttc tctttggatt 7980
atggtatttt aatgggtttg gaaatttaga gagccttttc ccccaaagaa atcgaacaga 8040
acagaaagag ttgttggtgt tggtggtgtt gttgttggtg gtgcttacag tgagtcaggc 8100
ctcggctgcc tatatctctg aacctagatg ctatatagtt actgacagga attctcctga 8160
ctttgtcctt gatggcatac agccatgcct ttagaatttc tccactcagt ccagctgctt 8220
caccaagatg cccctgtgtc agattgagtt actaggaaga ttgccacagg aatgcaggaa 8280
acagttcgtt cagcattcat agaatatgtc acatactcag gacttggctg gttttgttac 8340
attgtgcttt gtatgaaaac cacaactccc cggatatttg ctgagaccta gctacatttt 8400
ctgcgtctcc ttatgtggtg agaagatccc taaagattcc tggttggggt agacaaatgc 8460
ataaatgaga cccaggcttg tgtctccctt tgtgtctcaa ggtccttcct caaaagcgca 8520
aaaggaaagt gggggaaggg ctgagaaagc aggggtacat aagggacttt cccactgctt 8580
tctcatcccc agctccaaag cttagtcgat atttcaacat tttaggtata aacttgcttt 8640
tgcaatcaat gcaaatatta ctcagtgatt catccttttcc aagtgactct cggtttcttt 8700
cttttgacat ttaatttaaa attagaaatt agcatttata tcatgctaga gctgcactta 8760
cctttcaacg cctcagtaat ggccaaggct ttatgcgtca agattctaag gcacccgagt 8820
gttccttgag cttctgtagt acctgacgga gttaagtttt tagccacata taatctagaa 8880
accgtaggag agggtgccat gatgacaagg tgcccagtat ggtcgtttgt cagacattta 8940
aagtggagcc gtcttcatta gggcctcatg tagggaatgg accaggagga taccettacg 9000
tctgtgaagt gcggttggag ttgaatcttc caagttggag tgagacttct ttatgttaaa 9060
ttcccccact caaacttcta caaatcatcc aattttaaag aatgattttt aagatgataa 9120
tatattatat catttcccct tcccctgcct ccaagttctt tcatgtacct ctctctcaaa 9180
acttcatgac ctctttttct ttaattgatg tatctttttc taagtacaaa aaaaaaaaaa 9240
tcaatctgct ccatccatct aatgttactt gtatgaatgt ttccattggc attgagtaac 9300
caatcagtgt acatttttgg gaaacacaat ttctcatgct ctcagctttc cctatgtgcc 9360
tgtagttctt agtctagggt tgagacctcc tgagcaatct ccctttcaca ttgcctgtct 9420
gttggtgcca tccttgttca ggtacacaca gaatcaatga cataaaggta ctaagtgggt 9480
ttggacagtt taataaggca ataatgtcac ctccacctta gaagattgtc tttggattgt 9540
taggaaagaa tatcatcacc caggttaatg gaaaacctag agtcataagg atagtgtaaa 9600
acattgtttg tggaaattaa ttagtatcca aagtcacata ccagacagtg gcagaaccct 9660
ggtattttca atgatgtcat accccttttac tgcatcaagt caaaattccc ctgttttaac 9720
ttttttgtct gaataaatat gtttagttgc agctgcttag agagttttgt tttgtttgtt 9780
tcattttgtt ttaagacaag aatcttactg tgtagccctg gaactcacca tgtagaccag 9840
gctaacctct accacaaaga catctacttg cctctgcctc ccaaatgctg agattaaagg 9900
```

cgtgtgccac tacacccagc tcgtgttttc tttttaagta tgtatgtaaa ttgtgtgttt 9960
gtgtgtgcct gcataagttt atgtatactt tatgtgtgca ggtaccctca gaggccagaa 10020
gagggtgtca gagcccttgt aactggagtt tgacggtcgt gagctgcgca tgtaagtact 10080
gggaactgaa tcagggcctt ctgcacgctt aactgttgag ccagctctcc agcttgtgtt 10140
taaacatttt caagtgtttc ttgttttaaa caaccccact tccttgcaca cacctttgtg 10200
aagttgtgcc aaaggactta gagctcctac ttttccttta gttggtgctg gccctgctcc 10260
atgtggccct gggagttctc cagagcctcc cgtgcatccc attagtcacg tggtgttata 10320
gcacagagcc tttcactgga aatgaagcct caagttatgg gaatgtcccg agatgttttt 10380
attagtgtaa gataggcttt tgatggaggc tagacagtac attggattga aaactaacta 10440
gcctccattc ttccatcagc ctatgttcaa gctccaaggt gggcacaaca gccccatcct 10500
ttgatacaga gtctgttccc tcatgtgcta gaagccaggg cttttctaca tttgggtttt 10560
attcagcatg gaacgcagtt ttttaaaaaa agatttattt atttatttaa tgtatgtgag 10620
tacactgtag ctgtacagat ggttgtgagc cttcatgtag ttgttgggaa ttgaattttt 10680
aggacctctg ctcactctgg tcggccctgc tcactccagc ccaaagattt actattatac 10740
ataagtacac tgtagctgac ttcagacgca tcagaagagg gcatcagatc tcattacagg 10800
tgggtgtgag ccaccatgtg gttgctggga tttgaactca gggccttcag aagagcagtc 10860
agtgctctta cgcgatgagc catctcacca gcccagaatg tgattttgag gtgcagatag 10920
atggttctat gtgtttatca gggtctctgg gtgcctgaag gccgctgagg ctgcttatat 10980
taatagcttg ctaagttcag taagtttgag actttcaggt ttgatgtttt cactggttat 11040
tttttagtg cactctttag agttacagaa ttttcattgc actttgacaa aggcagaatg 11100
actctcacca gtcctcacaa gagggcaggg ggtgtcgttc tatgcccact caagtgttat 11160
gtaaaatggt atcaggactt cctcagcctc tcaaccgagt ctagacagtc ctaggaccat 11220
ctcagagttc atgagaaaaa aaatgcacaa agtagaagtg gccacagggt tccatctgga 11280
cctgattttt acccagaggt aggaccagct gtatcaaacc gaggaccctg agcttagcca 11340
aagggtttag atcccgtggt ctccctacaa tgagaggtag atgccaagga ggtacagcac 11400
gcatgatgcc ctcctacaga ggtctttttt gcagacattt gtggtccctg tagaaataca 11460
gcctagccct gcatatgtgc cagagttgta ggatctttaa gattcactcc agtgctacag 11520
ctttcatttc ttgttctgtt ttgtacagct tcaccatggc agacgatgat ccctacggaa 11580
ctgggcaagt aagtcctcag ctcactagca actttgtttc aacgcgttct tatcccaaac 11640
acacagaggc aacgctagta actcagtatc aagcaaaccc taaattagta aacactactc 11700
ttaatttatt ttcagttttt ctgttcttcc agagttggag agggcccttt cttatgtatt 11760
tttgcataaa accgtatata ctacatgcca gaaagtagcc actgagtcag ttgtggtgtt 11820
gaaggtctga ccccagtatt tcatatggtg actggtatat taaaaaaatt gctattcagt 11880
gagatttcta gggctgctat taacatcgct acaaacctgg tgacttgaca caatagaagt 11940
gtatcctttc ataggtctgg aggcaagaac tctgaatttg gggctggcag aatagttccc 12000
acctcctacc actgctgggt gtgtttagag atccttggca tcacaccagc cacagtctgt 12060
tgttgtgtgg ctttcctcca cgtcttcaca cataccatct cctctgtctt aaaaacacat 12120
cactttttgg atgtaggatc catctctgtc tagtgtgaat tcatcttaac atatataaag 12180
gcaaaggctg ccgtgtccaa ataagtcaca tgaaggagga gcacttatga attctgaata 12240
tatttttttt caaaggtact gtagttaagc ccactgcagt ctgtgggagt gagtgtgctt 12300
tgagagcgga gcagtgttta tcatcacgtc attgtcgctg tcatcatagg tgtgacagca 12360
gccatgacac agactgacac gaggatatct gtatcttcac tttccttcta gtttttctc 12420
ttaaaaaatc taaacccata gaagtgttag acagtctgat gatacttcca tttctttctc 12480
ttctccccat agagaatata gggagcatag aactgattct tgaaagcagt ttctagctgc 12540
tagcatgatt acagtggcaa tattaaaagt ggactctaat ggtctattaa attatttaaa 12600
atactggtat ctctttgctc ctcagctatc tactgagact ttatagaaga ctttatgccc 12660
agaacattag catcctagag acgaagggac aggtgatatg ctcactgggc tcaggactgc 12720
atcccggctg gagtaacgga accacacttt acacagtctg tggatgttgc catggttcac 12780
tgagagacag aagcagttgg agggattgct gtgtgttcct ttagggaaag agcaggttaa 12840
ggatgacttt gcaaaggagg actaagctga gctttgaaag ctacaaccag tgcaggggca 12900
ctatttacca aggtagccct gccacaacat tcattactac ttcacattta aattttggtg 12960
gtaaacaccc gagactacca tttcccaaat ttatgctcgc aaagagtcag ccaaaaataa 13020
aactgaaaat gaaatatttg gggagacct ggtgtttcta tagtcacaca gccccagagc 13080
cgcagtcaga gctgccatgg cctgcaccgt agtgacagct gcagtggcct cagagctgca 13140
gtcagagccg cagtggccct cttcttctca cccagcgggc ggagtgagca tgcaccatcc 13200
gctcctgacc atgttataaa gatggtggta ctgggtaaac ctaaggatgt tttcttttat 13260
ggcagcataa aagacacctg gcaaccatac gctcaaaccc cttgtttaac ctttcacttt 13320
taatgggcct cctctaacac aggccagccc tcaccaccat gagagctgtc tgcttggttt 13380
caaaccatgc ttttctgtct gtacacagga aatcttagca accagagatg gtcttttatg 13440
ccacaaaaga gtaagacctg ccaaggacag tcattttgaa agtgtctcat gtgggcagca 13500
gttctgagct cattagcagg gtgggcatgt tctgatagtt cagttgtggg ggaaggggag 13560
gcccttggag gcttttaagt ttttgtcttt gggttggttt tttcttcctg tcttaagctt 13620
taagtctatc tcttctaaag tttgcgattt ttgagggagg tgtcctccgt gttttagcca 13680
cactggagtt tgttctccac ctgtcaccaa gcaaacacca catgccgcct tcaacaggac 13740
gtctgtctta gtgggcttct cccccagcca gatcactgca gacagaggcg gaaccacagc 13800
aaaggacttc tttgtgaggt cgttctcttt gctcagagat ggaggcctgg agcttattat 13860
aaaacctagt ttcccattca gtgtgtcttt catggttaat agcgaatggc acagatgcgc 13920
tttgctttta aggattgaaa tataacacac tctttgctcc tcagttgaaa atttgcatcc 13980

ctcaaaagtg gatttgaagt gactcgcatt taaacatcac attttagggg ccagtaagat 14040
ttttcagaga gcaaagtgct tactaagtta gcttgacttt gacttcctcc actacccaca 14100
cataagcaca ctcatgttac acacacacac atatccatct catgttaaac acacatacta 14160
cacatacata tgcactctta cactatgcac acatacacac cttacacaaa catatatgtg 14220
catacactca aacacacaca ctcacatact cccactacac atacatacac tttcacacta 14280
tacacacata catgcacaca cactcacact acatatacat acatactttc atactacaca 14340
tgcatacaca catactacgc atacatatac actctcatac cacacacaca tacactcaca 14400
ctacacacac acatacacat tctcacacta cacacacaaa tgtaataaac atgcaagcat 14460
tactcttctt attcttgatt taactagaca tagtaggatt tgaggtaccc aagaatatct 14520
agtgatttac taataaggac aaaaaatccc aaaaaccaaa aaccaaaaaa aaaaagaaag 14580
aaagaaaaaa accgtctagc ttgcaagctg accagagaaa gtaaaagaaa taatttggtt 14640
tctatgttgt ccataaacat ttcaacaagt cacttctggt gagtgagaca gatagtaagc 14700
tcacacatac aaatgtgatt ttctaaatga gctaactggg aaaattcctt ttggagtaat 14760
tattagtcat aacttaccaa ggtagagctt aattctatca aaacgttata tgttcacttt 14820
ttcagttttc ttagttgtgt gttgtttctg taacaagtta ccatagtatt tgaaacaaca 14880
caaatatctt ctaaagatta gaataaaaat gtcagagtcg aggcatcaac agggctacac 14940
tcctagcagc gtttatttct tgtcttccaa gctctagggg ctgcctgtaa gtcctgctat 15000
catccccag tcctgcctct gtaggcctgg gtgagtcctg ctagcatccc tgactcctgc 15060
ttctgtaggc acactgcact ttgatttctc actctcctaa gtagctcact tggatattcc 15120
aggataatcc tgtcatttaa ttgtcctaca ttgatggcac atgcaatata tctttttgcca 15180
cagaaagcat aggttctggg gaataggaag gttctgtgga gtagcatgta gacatcttta 15240
gagccattgt tcagtgtgcc ttcatctact agattcaggt atttatattg taatagaaaa 15300
tagagctggg cagtggtggc acatgccttt aatctcagca cttgggaggt agaggcaggt 15360
ggatttctga gttcgaggcc agcctggtct acaaagtgag ttccaggaca gccagggcta 15420
cacagagaga ccctgtctcg ataaaaaaaa aagaaagaaa gaaagaaaga aagaaagaaa 15480
gaaagaaaga aagaaagaaa gaaagaagac gacgacgacg acgacgacga cgacgaaaag 15540
aaaaagaaaa tagaggcggg agaaagtgag ccctctgctc catcatgttc tcagttgtat 15600
tgccagtcat tttgtaagtt gtgtaactcc tgcctgtaat tctttcaagg ccttgaagtt 15660
ctgtagctca gtaagtaatc tatttatcat cagaacagaa gcatacatat ctaagatcat 15720
gtgcacgtat gtgctgtctg tctgtacgtg tacattcaca agtgtgtgga ggctgaaggt 15780
ggctccccat ctttgatttt gagaaacggt gtctaaccta acctacagct cctggattca 15840
gctaagacct ccagccagca aatcttcatg tctccacctt cctgtagctg ggactgcaga 15900
caactgccgc cagcccagct tttcactggg gtactggggc tctggactca ggttcttgtg 15960
ctctacagca agcaccttat caactgggcc atctccccac cctctgggat gtaatgaact 16020
caagttccta agagggtgcg agattcagat cacttactct acacattttt ctgaagggtc 16080
cagcatgcct gaagctctct tggctccgaa gatgcacagc ttgcgggatc caccaggccc 16140
ttgtctttag ggaccccata gccattattc tcctcaaatt tctttgttac ataagttaca 16200
cacacagggg cctaagaagg tcccagaaag gcacgttaaa attttgattt caggaatcct 16260
atttttgcgt ttttatttct agttttcaat catgtaatta gttttctggc tttgtgtatt 16320
agtgcaagaa gagaaattaa aatatttgta ttttcttctt tacagatgtt tcatttgaac 16380
actgctttga ctcactcaat atttaatgca gaattatatt caccagaaat accactgtca 16440
acaggtaaga aaaacacccc cttttcctgt tgttttgctt tcccttaaaa atatttgggg 16500
tcaggagtca gggagatgtg gatcaaccaa actaagtata tatgaaatta ctataaggaa 16560
atctgttgct ttgtgggtgc ttgggaccta gcccaggccc tctggaagag ccacaagcac 16620
ttttaaccac agaaccatct ctttagccac aacagtctct tttaaaattc aacaatattc 16680
ataggaagga agtggtgatc cgtgttttaa aaccaccctt tcagtatgct ttctggaaat 16740
gtgtctcaca aaaatgctcc ggcgtgcgcg cacacacatc gttttagcaa gtgtggtttc 16800
tggtctctgt tagaataccc tgaagccctg tcttcacccc gtgtaaggac tttcctcttc 16860
tccgtatctc attacccatt agtgctcctt tttgctttac cgcagctcac agagagccag 16920
tcttagcccc tcagttctgt atctgagggc cagtcttgtc agccaggaca cgaggctctg 16980
gggaaacagt aggaaagtaa cctttatatt ctgaccagac caagcgtaca gaactaatgc 17040
tcctgacatg actgctaatt aacgaaccca caaactgttt aaggtccctt tatgctatag 17100
cgcacgggct tcctttagac tcattattga ctggcatgtg tgtagccttg gcctgatggc 17160
tcttttgctg ctttgatcac ctgttggagc taagccgtct tcagcagcca cttgttagtt 17220
ggtggagatg cgtatggaag tgattgtcca ttactgaggt ggacagcagg tgctttgtgt 17280
gctccggatt tctgtgtctc gtgatgtagt ttttatcatt ctcatttaaa atgactatgg 17340
ttttgccagc gcctgcctta cggaaagttt tctacagacg tgggaggaag gagagttagt 17400
atgtcaccct cctcactggt gcccagaagt tacatcacgg aggcctgtct gtccttaccc 17460
caaataacca ccacagttta tctgtaatct taagctgtcc aacacgctct aggtcccctt 17520
cttgtgacca agtaaatgca ggggaggcaa ccgtgtttcc tgtgaacttc taaactctga 17580
gaccccaaga gcttccttta gcatttctgc tttattgaaa atgttgcaga ctcggttctg 17640
ccctcaggga aagcacaggt ttctagaaaa tgcctctaat tagttagaat actaaacagc 17700
atcctgcagg cagatgatat tctaatgtgt tctccttttc ctttggttta tggacttaga 17760
aataaagtta tccaaagaat catctcagaa agtcgttggt gttcatatca ttgcgcttcg 17820
ggaatcttct tttccatagt gttagagaat ccttcagaat tgcatggaag atgcattctt 17880
ttcctgttgt aatggctcct tgcttgcact agctctccgg ctctgtttca gtgcgttgtc 17940
ctgtttttat agtccaagat ggatgggaca ttttgttcag attttaaagg actagaaagg 18000
ctatctgtga cagtgtttgc ttgtaatttt catgtggatt tttttcaat cttctcaact 18060

```
tcttaaaatc tcaacgtatt agtatttcta accctgtgtt gggaactctc taacaaggga 18120
cagctgatag gtctgtgtca ttttgctgtg atttaatttt ctttttacaa tggaagatca 18180
tttttttct ggcacagaaa tgtagccaac agaatattag ccttaaaaca agtatctgtg 18240
tatcagctcc agtgttctac ttctcttacc cttgggtgtg ctcttctaag ggtaatgatt 18300
gccccctctc cgcatggaac ttcacatatg taaggaaaac ctaaaaccaa tcctgtgagt 18360
gtttcagacg ggctgttaag aaggaagaga cttggagcct aacacgagga tgtctcctct 18420
gcctccatat ttcagcagca tcttatccaa atgctaccac cttcctggcc tggcttctca 18480
gtaggctctt ttggatgcct agaggaaatg tatattttta atattgttat acaagctgtc 18540
tgatatgcct agcatgaaat ctttttctaa tcagttttat ttcctcataa aagttaaatt 18600
cgagcaattt tctttttgaa atttaaagtt aacagttacc tttctaaaag tttaaagtgt 18660
caagagatga atcccatgag acagaagaag gtaatttgct acattactga ccccagtaac 18720
cacccatctg tctttcttat ttggcttcaa attaggtttc caattgcttt cctggtacag 18780
tgtttaggct tccgtgttat ctgatcagtc ataatattga gaaggtaaaa tgtattctct 18840
ttgaagttac agtttattaa aaattacaac ctgaactttt atctcacctc aaatttacat 18900
atgcaaaata aggcttaagc ttcagccgag aaagtgaatt ttcaaatgag cacagaagtg 18960
ttcttctaga ctgtcttcag cttcaagtct taattttcac tcaaacaact ataaaatgtc 19020
gaatatgttt gtgtattaca cagctattgg ttatcatact gggacataga agccatctga 19080
cgccccttatg gaatttttta tcataacatt tatatttatt tattttgaaa tttcaagtca 19140
gatatcattg gtatataaat tgagccttta ggtccttttc atagtaatta attggcatgc 19200
atttatcttc aattacatta tttttggtat tttatcataa ctctgggctg ttttagaaca 19260
gagttcttat gagcacacac tgttagatac tttattatat ctgcatcaaa agctgtgagt 19320
aagatgccag ggtagtggtc catgctttta atcccagcac ttggcaagaa aatctctgtg 19380
agtttgaggc caactttgtc tacatagtga gctccaggcg agccaaggat acatggtgag 19440
tctctgtcta acaaaacaaa acaagccatg gagaagcaag aaaagttctt caggtctcac 19500
agagggcaag gaagaagcca gttaggcaac agtggctgtc ccttggcttg aagtaaggga 19560
aagactacat ttcaggagca gtgttaggag aggcaggtgg cccaggatct cccttctgtg 19620
ggtttctagc ttcagttctt catttactcc tgggcagttc tctttgaggg aggaaagtag 19680
tctgcaggtg aaggccacct gacaccacag cactcagagg tgctccctgc actcagctgt 19740
attgtgccct aaatgtttgt tgacacatga gtctgaggac ggctcggatc agaatctctg 19800
tcagcattca gaccttttgt tcagtgccag tggtgtacca ttaccattca gaaaaagcca 19860
cttatttacc gttagtcact taaactataa aactannnnn nnnnnnnnnn nnnnnatcag 19920
acactctcat ctggcttcct tgggcactaa gcaaagcact tatgagcaaa aaataaatat 19980
tttttaaaac atttatttaa tatttcaaat gataacacag atcagtagaa cacacttcac 20040
ctataaaaat tgatttgaaa tctctgttta aagcataact tcagcatttg tcttgctttg 20100
tatttcatac aagattccag aagtctcaca gttttctttt ggttttcttg tgttttcctt 20160
gtttttaaca gatggcccat accttcaaat attagagcaa ccaaaacagg taagcgtaaa 20220
ggggtcgggt gtttggtaat attacatctc atggtaaggc tcggtctctg agtaatagtt 20280
aaatctgatg acagaggtaa gggttttatc tgaataatcg gcagcctgcc gtgttatgca 20340
ataacttcct gtgtctacat ttttcttaca aatggctttg aaacatcagt taattataaa 20400
tccttggtag tctgtccttt ttccagctta gctcatcagc tgcttccagg gaaagcacta 20460
aagagtctca ttcatgaaga aatactcaca cacacacagg ttcacagcca ctctgacgct 20520
ctattgctca tggccatgcc cacagggtac ccctcctgcc ttcataagct ctggcctttt 20580
ctgtgcatag aatgtgtagc ttaaggcagt aaacgcactg ggagacatta gagtggactg 20640
ggagatctcc cctccacccc tctggcttta atcccagcct ttttgaagca aggagaggat 20700
tttttttgttt ggttgttgtt gttgtttttg tttgtgtttt tgtctggctc tttcgctttc 20760
tactaaatat tgtgttcacc agctgaagat taaaatagcc tcaaaactgc aaatatgctc 20820
attagaagtt gcatggctct agtactgtaa gagagaagag aatcttcaat atctgccgac 20880
agaacatttc agtcattatg ggatggaagt tatggtatag cctcccacag agtgagaacc 20940
aagtcactag atctctgggt ggatatgctc atcacagccg tggcgctgaa ggactgtata 21000
cctggcctac cgagttgtgt cacccaatgt cttttctagg ctgttccttc ttgtaactca 21060
ggagagtggg ttaacacatt gtgttgtatg acactcaatg ctaagaagtg aatgctgaca 21120
gcattcggtg ctgtgctgta gaattctgat gtggtcagct gttaaatagg agaaacatct 21180
tagggatgtc catgctactt tgtaaagctg aaatcagatg gaaatgttaa ttataaagac 21240
aggaatgtca catggaactt tggggcattg tttggttgct tataggcggc ctcttaacac 21300
acacacacac acacacacac acacacacac acacacacac gcacacacac atacttatac 21360
acatgtacac cctccctggg ctgcagataa cacctgccct tgctttatct ccacgtccat 21420
ctttgtaaag atgatttctc acataaagtc gctgttctcg gagctcttca gtagagttgt 21480
gttctgtgaa attctgccca gtaatctgga atcctggaaa atgtcctagc ctggaagatt 21540
tggtgaattc ataaaactga tgagtccaaa atgtacctac acatcaaata gatttttcag 21600
atcctttaga aaattttaaa taatttattt aattttattt tgtgcgcatt ggtgtgatgg 21660
cgtcagagcc cttgcaactg gaattacaga cagttgtgag ctatgacgtg ggtgctggga 21720
attgagcgca ggttccttgg gagagcggac agtgctctgc tgaaacattt ctctagctcc 21780
ccttttttaac aatattaaaa ttgcattgat ttattttttta atgtgttgct ctctttgccc 21840
ccaccccgtg tgtgtgtgtg tgtgtgtgtg tgtgttatta tgaaccccaa agtcataggt 21900
atttatgatg tgtttgaaca gctagtatag ttccagaatt taaaatttaa ttcattttgt 21960
gtgaatatat aaaatcatat atgtataaaa catcatgttt gattgccagt ccgattttat 22020
tttggtatct ggctttagtg aaagtaagaa ggttgtatgt aagccaggct ttttgactgg 22080
atgtatggag tctctctgag gttaatgact catttgaagt aaatggcgtt cctgagatga 22140
```

```
acagcgatag atggtgaacg tttaatttca gaatactaga attagaaatg tgtccagagc 22200
gtgcatttta agacttccta attttaggtt gttatttaat ttaaaacatt agtttatttc 22260
tgagtcaaaa aatgtctgta gtcattgctg gcttcaaact ggtgccagtt ttcctacctc 22320
ccctctccaa gtgataagat gacaggtgtg agccaccatg cccagctcaa aggcatcgat 22380
gctccagccg aacagcgcaa tacacattac aaccttcaga ataaatgctg ccttggatag 22440
gacgttcaat attcttattc gaccgagaat aatacacagt gtcacacgct gtgcaataaa 22500
tcatgctggc tacagggtta aacgtcactc cccataaggt atgagaggta caggtacaga 22560
tttttccaat gccacaaaga aattttgtaa ttaaagataa ggaaccacta tgttaagttt 22620
ttgagttaga aatgttcttt ttcttggagg ccttttgggc acagatgatg aaaatgaacc 22680
aaaccacaga ctacatgtct ttagactgtc cagttccgtg gcatgtttct cactgttaac 22740
tgtttattca tgttgtgttg tgttgtgttg tgctgtgtga gttttgtact gctttgtttg 22800
gaggcagggt ctcagcagtg tagccttggc tggccccaaa ctcacagagc tccatttacg 22860
tgcctctgcc tcacaagcac tggggtaaga tgtgtggcag ttataatctg aaggtgtgtt 22920
tgaattttct cagttaagtg gatccccatc ctaacggcag cccatttggg gctcttggtg 22980
ttatacatgc tattattgta tagtttgggg gtgattgagt tcaagcgtta tccagactgg 23040
atcccagtaa tattaatttt tacaaacttt atgatcttat tccttgaagt gctcagaaga 23100
ctgctagagt cacagtgtcg tagcatacag catgtctgta acacagatgc acttaatcct 23160
ccagagactt gagaaggggc accaggagaa gggggatgtg gggggggagc accctctcag 23220
agtcgaggag gaggaggaat ggggtgagga acttgggggg gtgatgtaaa taaataaaat 23280
aattcttaaa aagacacctt tatggtctgt gatcagagca gataaaggaa ctcctttaag 23340
taattagcat aacttttact gatggcagag cggagagctt ggcagcagcc attggtaacg 23400
agaggctcca gttgaataaa gtgctaacgt atatccaaag gcttaaactc ttcatcactc 23460
ctgtgttgaa ggtgggtgcc tggcttctcc gccactgctc agtgcatcat tttcatagtg 23520
gctgttagaa agcatttctc cgtctgcaga gttgtcttcc acagttttgc atgtttagag 23580
acgtgtgtgt acatctgtca ttgttgcgtt cagtaaatac cgctgtgttc acctctgcag 23640
aggggatttc gattccgcta tgtgtgtgaa ggcccatcac acggagggct tccgggagcc 23700
tctagtgaga agaacaagaa atcctaccca caggtcaaag taagtttgaa acctctgctc 23760
tcactgccca ggagcaaagc caaaacgttc aagtgaatgt gactgggatg ttcctttgcc 23820
cctcagcggg agtgtttaca tggttcttag aatttttaaa tagaaattca cctcatgaaa 23880
atgacgaatc gtcaaaacac cattaacatc tccatttaag tgagatggtt ccaacatcaa 23940
ctaagaaaaa cttaactacc tctccagtga tcttaaagct gtcttttcat cttaaagtat 24000
ttttaaatat tctctgatgt tttagtaaaa cttggagcca ggtgtcatag tccatacctg 24060
tagtcactgg acttaagagg ctagcaacag gactgttaga tatcccaagc tagcctgagc 24120
tacatagcat gcttgagctc agcctgggct aggtgagacc cttatctcgt aagaaaacga 24180
agaaaataaa aaatgtcttt gaggcaaatt tcaaatactg ggtttataga gatttcatga 24240
caagacccctt gacagaatat atcggctatg gagtatatta attatttaag atcataagtt 24300
cagacccaag tgatggaata aacaggttgt aagcaggctt atctcactga ccaaacatat 24360
aaatacacaa ataccactag catgtacttc acaaggtgtt ttagagtttc aatgaggaaa 24420
tatgcttaag ttctctgtag agagcctgag agaaaccgtg tgcagctacc atgaatgagt 24480
tctgtagcat gggggggcaga ggggtggggg aagcttcact gtctttacct tggggtgtcg 24540
ggtgtagctt cgctgctctt cgctttttac gttgttgatc tagttgagac taccttcctc 24600
tagttcagag tttctacttt cctaatccag tgttgtgttt cagagactga cagtgatttt 24660
ccaaggaaac agttaaaaac tttcgagtgg tttaaaaggt tttatttatt tggtaatctg 24720
ggcttagact aaatgttacc agcagtaaac gttatggcac tccagcctct gtctttctgc 24780
tggcttgaga tctcagacgc tcttgccctc agctgagctg acactgggga agttatcttc 24840
agttttgcct cctaggttgc ttgtcccccct tttaatgttg tacttccagg ctcaggcctt 24900
ccaggttctt gccctctgtc taagacaaaa gagccatacc atcctttatc tttccagtga 24960
aaaaaatgtg aaaaagccat accatccttt atctttccag tgaaactcag ctcctggctt 25020
gggtcctgag agtcacattt tcattttaag gctcccagct ctagttgtgg aatgagccgt 25080
tttttttttc tcagtaaagg taactgaacg tggaagtgca gtggctttag tgcacagaag 25140
gccacagtta tattagacag tgtttttcag agaagtcgaa ccaggtcggt gaagacaggg 25200
agatagttag atggttgact gagcgatagc ggaggagatg agagtaatcg gcctcagctg 25260
tgacgattat gaacgacacc aagacccact ctgcagcgtc tgtaaaatgt gcgcagggac 25320
actgctagga ttccagcaca gaagcctggg cgccctgtaa ccaaggggac tcttcactaa 25380
atctgggcgt ggctatgctt ttcctctaaa ggcaggagag gttggactgc tgatgccctg 25440
gaggggcaag aaatgaagag tgtcctccct cccattcagg tggggcagta gccggaggga 25500
gggaaggaga ggattctctg gtgttttggt ccagcaggtc ttctagctga agggaaggtc 25560
ttccatcagt ccagcaaact ttccagtgtc ctctggaaac gtgcacacag ctaggaagca 25620
aatgctttat cagctttcag catctgccca gtgtagtcga ctgcacaccg gaaacttccc 25680
tcaggacagt ctgtggcttc cagtcctgca gaggccaggc aaggaatgca gagcagagca 25740
ccggcgaaga aaacggttat tccacacccc tggtggtcct cgtgtctccc atctgttccc 25800
tcctgtagcc ttattaacac tgaggagctc aggctgaggt taactggccc tcttcaaagg 25860
tgctgggctt ctttctcctg cagctcactg gccctcccat gtgagcgtgt gcagctgagg 25920
ggccaggggc caggggctga agaacaagtc aagaaggact gataaatcaa gtcagggagg 25980
atgagcattc tgaacagcaa agaagaaatg ggggcagaag tcatcagggg tcagatctag 26040
aatttcctac ctgtgtgtgt cctgtacaaa tattgtgtgc aattatgtgt ccttatctgc 26100
accagggtca ctgaacatcc cttgtcagca ctgatactga tcataactac ttaagaaccg 26160
atgttaggag aaaaaagtag ggttttttc tctattggcc ttctaccaaa aaaaaaagaa 26220
```

agaaaatgta cattgtgttc tatttatgaa ctctttcctt ctgaggcact agaattctgt 26280
ggtctgccat gcatgaaaga tgggagtgtc tgtagaaaga ggcttccagg ggtatcatgg 26340
cttactgcat acccacccat aattcatcgt agaggtgggt ccatgtactt cctgagggag 26400
cgactcttcc gtagccttgc acatgcaagg ctgtgacttg catgaagtat atgtcttcag 26460
actcaccttt tcttcacccc cactgacaat ctctgtctcc ctggactcag gccctctttc 26520
ctgccctgcc tcttcaagca cccctccctg ctgtccttag ggagcgcccc tgcaaggtta 26580
gtggttgatg tgatgtgaag attgcagtga agatctgaag gctcttgaaa gctttgggaa 26640
ctggtcatga cttaaggctg cagaaaccaa gctttgcttt atggagcttg taaacttaca 26700
tcttgtttgg tcgtctactt gttgactgtt tgaagcatca ggttgacttg caagctaact 26760
ctaggaatct cccaaaacca gcacttccta ttagaagtat ttccacgctg tggtagatgc 26820
tgggatgcaa gctcagtcgt cacccccctcc ccccactgag gtaccacctt ttctagaact 26880
caggcggctg taccatgtgt atgttcctct cgtgtagttg agaagaaaga acctctttgg 26940
ctctctagga tcttgttgta caagtcttca cgttcttatt tcctctctct cttttgatta 27000
atttctctgg gattactggt tcctatgtcc tcacaggtat catgaatggt ttcttcagat 27060
tgaagcattc cttccactgc cttcaggagc tcggggtggg tgaatagtct cttttaaacc 27120
agctacttca tatttcaaaa tattttaagc atacaagttg taaagttcta tataacaaat 27180
accatgtacc aaaattaata aatttaatag atggtaatat tttgcctatt tgctatctgt 27240
ctatctgttc tctctctctc tctctctctc tctctgattc tctctgaagt acaacatagt 27300
tacagtggga gcccctactc agccactgct ctgtcctctt atcctctttc tttgcccaaa 27360
ggaaaaccac tgttctgaac attctttatc tgagatgcat tcatatcata tactaaaatc 27420
actttcaaga aagttacttt ttactatctt taatccaaag ttttttatta tatatggtgg 27480
ctcttttca aactatatcc aggttaaaac tgggcccctg aaggcttaaa cattgattgc 27540
tttaatccag tctcttcctc tttattgttt gactaaaaat gtcttcagtc acagtgtgga 27600
atttatgctg acccttaaag caaaaaatca tattcatgag gaagctaggg aaaataaata 27660
tccagaattt aagaaagttt tccactttta aaaaatggag taatccactg tctagcctgt 27720
aagctctccc tccagtgttt gcttggttct gactttatct gcggctcctg ggagaacagg 27780
agagacagca gagctcctct gggccaagcc tgtcgctacc tgttcttgac tcagcaagga 27840
gtgccaggga aggctcactg ccctcctttt cccgaaagtg gtgcagaaaa attttaaatc 27900
aaagtgtctt ccttgagcaa atggaacagg gccaagagga gaagccacac gtggctttag 27960
gaatgcgcct gcagcccgtc ctcacttgct tgagtccttc tgtgctgggc accttgtgaa 28020
cattgcactt ggacatcagt gactttcatt tctaaaagac agtttgtttg taatcatcca 28080
atgccaggag gttggaacac agtgatgcta atatgacgat tctgatcagc tttgctggca 28140
gctgccctgc ctgccactct cctggggttt gatggttaaa gaacgggttt attggaatgt 28200
ttctgtgaac aacagctttg gctccgttct cccacctcct gtagtgtgca ctcttacgtg 28260
cctctcactg aacagtcctt aatgttacgt ttccgtcaag gttaataaag cccggctgat 28320
ctgctgtagg cttgaggata tcacatatct ttgcaaaagc gccctttccc ttgcacaggc 28380
ttgtctgcat ccggagactt gccatcctaa agatctagtt ggaatactaa ttttgaataa 28440
tttagcctga tccagttacc taacatgcca gggggaaag atcaccacaa cagattgggc 28500
attcagaacc tacctagttc atagcattta ttagaatgtc ctttgacact tccaagtgtt 28560
tactgtgctc aaataaaaga aaagagctat gttccctacc aatactatag tctgtgcttt 28620
gtaacacaca cacacacaca cacacacaca aacacagagt ttattaaaac aagggctttg 28680
taacccaagg ttcatggatc cttttgtggt ttcagaaagg tttgcaacct ctgactctac 28740
acatagagtt ttatgcatat ttgtatgcat ttttaaagac tcgaacatgt gtaaaagtaa 28800
tgctgaaggg cattggcttt tttaccacag ctgattaaat cgatctctac aattttggag 28860
ggatgggggc gggggcgggg ggagggcgac tggctctcac tgtgtagctt aggctggcct 28920
tgaactcatc ttcctacctc tgcctccaga gtattgagaa attccaggga tgcaactgtc 28980
ttgaatgttt ttcttctttc ttttttcatt tcaaaaattt acatttacac tttttagttt 29040
tacccatttt aggcaagttg aagcgctttg tgtttctcag catgtaattc aagctagttg 29100
ctctttagta ttccattgac tggaaaagat gtgagattta gcgcctcctc acacacacac 29160
acacaccaca ccccaccccc gcctttctgt tctctccttc ctcccctctc ctccctctct 29220
cccactccca ccgagtctct tggcactgac taaatacgat ggttcaaaac caaaaagaaa 29280
agagaatcat ttccctgata ttcactcagc acggtggcaa gtcctgcact acaaggaggt 29340
aacatcctct gcgtggccat aataatctag acttctgcat aacttttctt ctacgtatcc 29400
caggcgtttt gcgattgcct ccatgagtaa aatactgact tgttaaaaaa caaacaaaca 29460
aacaaaaaaa ccagcccttt atttcactgt tagcattaga agttagagaa agctgcttac 29520
tgaaggaaac tgttgagcag tgagaattga tcctgagagt taggagagcc aagctcctga 29580
gggttgcagg ttgtctgact ttccaggctt ggtatggtgg cctgcctcgc ctggcatttc 29640
ctcttcccct ctcctgtttc tctgcatact cattgctctt gggatgcttg ttcctagtgg 29700
cagtgttcag tctgaaatca tctctgatca aagtggtctc tgtttgaaga ggatcattac 29760
attcaggccg ggaatcccgg tttcgtaaag ccacatgtga agattggttt tgatgaggca 29820
gagtgagtaa ccagagccta tgatcctcat gtccccagcc accttctcca tagtcagggt 29880
ctagggtctt tggtgggtct agggatgttc tgctggctct cctggagtcc ccatcttccc 29940
cctcccactc taagtggatt ttaggtcagt cttccaaaca actttcactt tcttgaggtc 30000
taatggaggt gagtgggagg atatctgggc agacatgtga aacaatccag tctgaagacc 30060
aggggaatgc agagtaagtc cccagcatct tcagataacc gagttatcta tccattgctt 30120
gatagataag tacaaaacag gtttgcttgt attagagaag acttggaatt gaaaacaagc 30180
cagaagaagg taaggccaaa cacacatttt tcaaatcaca tatcattgaa atggtctaga 30240
cattggaaaa acaatgatca ggtgggagag tggataatga ggtttctcgg ggtggaacag 30300

agaaccgtgg aggctgggag aaagggagag ctgaaagcag agcagagggt gtctagctga 30360
agtcctggga gaaactaaca tggctgcatg tgtgtgtgtg tccttctcta cgcacagctc 30420
tcagattggc atgcagtact cagcttgtgg gcaacatatt cacggtgtta agcaccagct 30480
gtgaactgat ggccagcgtg ggtgtgaca ttacttccta gattctcatt ggctaagctt 30540
tgtgaactga tggccagcgt ggggtgtgac gtcacttcct agattctcat tggctaagct 30600
ttgtgaactg atggccagtg tggagagtct cactggctaa gctgcagctt cttcatctgt 30660
aaagctgacg tagagaacca actcaatgga acatgtgcgt acattcagtt gctcaatgcc 30720
ggcacacagt cagcctggaa acacaattac acccgttttc atataaaatt tcaggaaatt 30780
gggtactact ccaaaagctt gtgtttagag gtaacacatg atgtcatagg aaactcacag 30840
aaaacaagag gatagaaaca agtgttaggt tttgttaggt cccttcactg tgtcctggta 30900
ggccctatgt gtctaatttg cacaacgctg tattcattcc taggttgcca taacaaataa 30960
ctgcaacctg ggtcatatta aacaacagag tttattgttc caaagttctg gaggcccaga 31020
gtccacagtg aagttgccag tgtggtcttg cttcccacag aggcttcagg aaagagtcaa 31080
tcctggcctc ttccagtttc gggtacttgt tggcagtcct cagcaagctc tagctgcagc 31140
cacttctctg atctctgctt ctgtcttcac agggctcttc tccctgactt tgtatctgaa 31200
tctctccctc ctttccctcg taaagacacc agtcagcgtg gttagggacc accacgctgt 31260
gtagcaggac ctcttcttga ccaatcgcat gtgtaggaag cctgtgtgta gatgaaatca 31320
tgttcttagc tcctgcatag gcccgagtcc tgggagatgc tcttctcact tactacaggc 31380
acgatgtgca gtggttacag agtgttcccg agtgtgtgca tttaaggcca tgtcgttccg 31440
tgctgtgctc agaaagaagc ctgtgtgcac cacgtgctcc cctgtgtacc tttgttagat 31500
tcaggtagtg gaagaaaatc tctcaggtct gaggaaattg ctgtactagg gttttgaaca 31560
tttattctag tagatggaag gtgtgctaga aagtgtctgg catcagactc ttggatggca 31620
ctctgaattg tagcatctgc cagtttcctt cccatagtaa atttcaggct gctaacatga 31680
agtcaactgg cttgcaataa ataaataaat agataaataa ataaatattt taaaaaacag 31740
tgaaaattta actattctga aattaactcc atgaactagt caaccggttt ctaacctact 31800
aggtgttaag ccagcaaagt tagagcgaga gaaggtgata cattgtttcc tgtaggagct 31860
actttctatg cacatgttac ataatataaa gatgtgcagg ctatgaggga acccgatagg 31920
tgttacagct gggacttcat tgtgtgatag ttaaagagag ctgcggttga gtgggattac 31980
tgctgtttag aagcaaggct agacctcggt cccattctgc cactcgctcg caggtggggt 32040
gttcttcaga aagttgctaa agatcttcaa acttggtttt gtttatgaga tgaaagtaga 32100
aacaactcct tcacaggaac catgcgtgct tacgtaaaaa actgacctct tagaaaggca 32160
gctgccatag tcctggcaca tctgcgtcat taagtgctca ttcggacacg tgtgtagaaa 32220
gagccatgga gatgactgtt tttttcctga gagcagaagc ttgcaggtac tttgtcaaca 32280
gcacctagaa ggatgcccat gtatagcaga caggcagcag ttacttgtac agtgagcagg 32340
tggtcagtgc tcagaagctg gaaggatcat ctaaggaggc acttgtcctc ccatggtggc 32400
gctaagcaag tcactggagt ggccaccagg tctccacctg tcactgctgc tcactcccca 32460
ccagcctgct cctctttgct tctccagcct ggcatgactg ccagccagga cagccacatc 32520
ccagacctgg ctgtacactg tagtgtagct cttagacata gcaccaatct attctgggtt 32580
gaaactggct tttctgcctt agtttctaaa cctattttaa ctgtccaatt gcaaaataac 32640
acttaaagaa actaaaaagg tttaagcaaa atagacattt atttatttta aaaactgaaa 32700
attctaaggg aagaagtaga aaaaagaaac ccaaacaaac aaacaaattt gccaaatccg 32760
tagaaaaact cccattgggc tttttagcat ctcgacttct accttagagg ccactcagca 32820
gcagcaggct cactgtgaga agacctgcat tcccaaagct gacatcaaag cttatgagac 32880
acccaactgg cagttcctag gcctttggta ctctgagttg ttccactgcc tgcccctgca 32940
gcagtgagac aggggatagg gttgagccct gtgtccgtca gtcgcatact ctgccgctgg 33000
ttctacaagt gggattagtc actgactagg aacatgggga atgaactgag gttaggattc 33060
actgagcaaa gagctatgcg ctagtgacac gcggatgctc caagatcaca aggattagct 33120
cattcgtcca tcccgtgacc agcctacagt ttctgaactc tcattcctaa cagttggtaa 33180
acccgggttc ctctcttgag aatgactgtt ccatgctact cctaatatat gaaacttttg 33240
tttttaaaat aaaccaaaaa gtttttttgtt tatttacaga tatcacttgc atttaataaa 33300
atttcaagaa acacaaagtt ataggtagta agtccccttc tgtccaagat tcctttgttc 33360
cctggagagg agagctgtta ggggcttctc aagtgcactc ccaaccgtgc ctgccgtgtg 33420
tttaccatgt gtgtttatac ctgctggcac acttccactc acgcttcctc gtgtaagtaa 33480
gcattagtgt agcgcttgtg agcacggatg tagtctacta atctatgaac catctgctgc 33540
ttactttttt cacttactga gtctgaaatt tgctcagatt aacagcaaac tccagagctg 33600
gcaaatccag attcaagtcc agctctgtcc cctgagtaac tgtgtaacct tgagctaact 33660
gaccaccttc tctgtgcccc tatccaacta gcataagaac aactccatgt ctcatagtgt 33720
tgttgggagt actaattgaa ttagtgttga aagagcaatg tctgctccag ggaagagttc 33780
tgatgttgaa tttcctaccc atctactctg taaaggctgt gtgctttcca tggctgcttg 33840
gggcatgctg tcttctctaa gctccatctg tcttctatca caggccaacc ttagtaaccc 33900
tgcatgatag taacttctcc cttatcctgg ggtctttgtc actttgggat gttttgttat 33960
tgagatagct ttttaaaaat acttattcat ttttatttta tgtgtctgag tgtttttgca 34020
tacatgtatg tatgtgcacc atatgtatgc agtgcccacg gggggccagaa gagggcgttg 34080
gaatccctga aactggcgtt gcagttggtt ttgagcagcc atggggattt gctagaagag 34140
cagaaagtgt ttttaaccac agagccattt ctccagccca gagataccat tttaaaatta 34200
taatctgtga ctgttttgtt ttgttttgtt ttgttgaaac taggttttac catgaagccc 34260
cctagctggc caggaaccca ctgtatagac aagtctggaa tgggactcac agaggttggc 34320
ctgcctctgc ctcccaagtg ctgagattaa atgcatgtgc cactgtgtct aacccttttct 34380

taatgtagtg tgtttattct ttgaagagtc ataaactaga ttttcaccat attcacttcc 34440
cactgttctt tttagctctt ctgaggtcta cctcctacct cctctcagtt tcttccttcc 34500
ttcctccctc ccttccttcc ttcctccctt ccttccttcc ttccttaatt cattcattcc 34560
tttctttctg cctatgtact aatggatatg agactatcca ctgaccatgg tcattcctat 34620
aaacattttc ctttcataaa tgttactcta tgcatgtgga ggtcagacgc agctttctaa 34680
ggtcagttct cactttctgc ctgtctctct tgttccccac tgtgatgcat cctccagact 34740
tgatggccct ggagctccca gctggttctc ctgtctctgc ctcccatctc tctatggggt 34800
gttgggatta tagatgtgca tcacctaata gggcttttta cctgggtccc agggtctcaa 34860
actcaagttg ctgtgatagg catatataac aatgctttta ctcatgagcc tccctggatg 34920
ctgaattttt ttttatcata gctttatttt gactctttat tgtgccattt gcttcttata 34980
tgtttaatga tgtgtttttc aatgtgatga ccctttcaga gagtctgccc tgagttgttc 35040
ccaggcctcg cccacttgag tgtgttctgg ggatgccttc ctaatgagcc ccatgcggac 35100
tctgatggct gccgtactat gtcttactca gatgtgcccc tcctcatttc tagaccttat 35160
ttcatgtgca taagaactgt gtctttttag attccctaac actgtgctca gttaaattcc 35220
ttttcattct tgggtactag gtgattaccc cagttagcaa tagaaatatg caattagtat 35280
tagacataag aggaaacatg tctctcccgt tcatctctta ctgagctctt gctgtgtctc 35340
acagccaatc atattcagct ccaccaggga ggcgttggta tcctagtttt caattgggtt 35400
tgatgaacac ttggccaaaa acagcttgtc tgtctacctt tcttatgtac atgcacacac 35460
acatatctcc taattacttt ccccagccta gacttctcca gaaaaccagg cttcagatct 35520
ggctacttgg tcgactttac catttggata cctggtaggc ttgctgagct caaagttgaa 35580
tgcttgattc cctacttgaa gttgtccact cttagaaaat ggtaacccat cccccagttt 35640
ctcagaccaa gcggtcttga agttattctt gacgctcctc ttcttgatca ccacttataa 35700
tctaccctac tggctatttc ttcaaactaa ttagaatcta cttgcttgtt gccagctcca 35760
ccccatgagc cctggtataa accatccgcc tcctaatggc tgccatttgc atgggtgatg 35820
cctgtctcaa gcaggtattg ctaactgatt acaaatttag ggtttgctct atcatttttt 35880
ttgttagata gcatttatct ttttgtactt ttcaaagtag ttttctcttc ttcacactca 35940
ctaaaaaata tttcagacta gtttaaatgt ttctaatcta aaaacacaaa gtcaaaaaat 36000
gctctgaaag aaccagggga aaatgaatgg gggtgggtgg gggggtaaag tacttgtcat 36060
acaagcatga agagcttgag tcccccagaac ccacacgaca gctcaatgca gtatagtagt 36120
gcaaggctct ataatctcat tgctcctgta cagaaatggg aggtaggcac tggagactcc 36180
ctggaaattt acctgtggtc ctgagcgtgt cagctgagtg cagttctgga tcctcttctc 36240
atccgggagc cacgcatcac tcctgccatg atttactgtg atatttgaac ggtgcttgtc 36300
accaaccagc agaagtgttg catgccagct ctcgtgtccg ctagacctcg ctcccccact 36360
gctaactgac tgggctcatc ctccatctcc tgctctgtcc tgaaatggct catctgtcca 36420
agggacctga ttccttagca gagtcgcttt tcctaggttt tcctgaggct ccatcattga 36480
tctttttgt tgttgttgtt tttaagtctt gagttcagag cctatttcac acatctcatt 36540
gttgccatca tcacagactc tccttccctc cccatcctag gtttgtatct ccttttccca 36600
ataaaatgca aaccctcatt gccaataaca ctagaagtgc ctactcagtt gattggtcct 36660
acaatacttc caaacacatg ggagatagt atggtttcag gatgacgacg ccagtatcag 36720
ccctagcaac ttaacaaaga gttgcgtttt ctttttattt ctttaaccaa aattcagcag 36780
ggaataatca accagaatag cgttttcatg ggttacattc ttttccttct aggaaactgc 36840
gttaccattg aactataggg ttagaatact ttgtttatga tgaatcttaa gagttttcct 36900
attcctgctg attttgtttt atttttgaat ggtggcatgt atcggcctca attaccttcc 36960
atttgtgtga gataaactgg acgatttggg aactatcata aagtcggttt gctttattac 37020
cttttaatca gagagaggaa caaagagttc tgggatggtt attctggaat gtgaacccag 37080
taagctcacg ttgtcacatg ggtgttcca tttctatatt tggagccctc ttagtcttcc 37140
tcacagtcac agagtacaca catatatggt tctcgcacat aggcttggtg ctggctctgc 37200
ggtcacagac acgaatgagt ttattcctct gccccgaggt tacagagagt agccaagcag 37260
cagacacgta tgagatgata ctggggtgtt tcccttggaat ttggggagga tgctctgcag 37320
tggggtgcat atgtgtatgt gtgtgtatgt tcctgaagtg ggtggccttc cctgaatgca 37380
ttcctactgt gtagtcattc caaacttctg atggtggaat gccaggatca cagagcactt 37440
ttcatactgg atgtcagagg catggcccct tcacaacaca cgtgcttcac tgtcagagcg 37500
gtgggcctgt cacactgcgt gtgcctcaga gataacataa tcttccaagc tggtgatgtc 37560
atcctggaga gacagagcca acgatgagcc ccaggggatt gttcctaacc attttaacaa 37620
gtctcagccc taaagtcttt atggttctta tcaagttttt tgacaagaag tggtagataa 37680
caaatacttt aaattcaaat taatcttgaa aactggagcg aagtgccaaa ctcttcagcc 37740
ttgatgttga acagagacac acacatccag aaaaaaaaca atgaatccac aaaagtacag 37800
agcagagggg tgagtcagca gcagtacatg cggagagagt agagttgacg tgcctagcaa 37860
gagctagcaa ggcctgcagc tgccaaagaa gtcgctcctc actaggcaac agtaatggaa 37920
atatgatgta taagagccaa gaggtgatag tctcatggcc accctgcagg aagatcacac 37980
tagatgcgtg cagcctgctg ggcagagcac agtagtcggc tgtgggcggg gcggggccag 38040
atcaggatgg ttgtgtgacc acactggaat ggcttacagg atggtcctgg ctgtcctcgg 38100
tgtttgaata gctattataa gaaaatggaa atggacccca ccatcacaag acaggggaaa 38160
gagggagcgg tgagctgaag tcccagaaga caccagcttc gtgggaatca cagccaccac 38220
aggacggggt gactttgtcg tcactgctct gaactaacct gaaagacggt agaggaagaa 38280
agggaggctg cgtgggcttc agcttcacag cctcggagtc tctggctctc catccctgtt 38340
ttatatggca ttagacagag ttttgcttag taaatcatac ctttgtaaca agggctgatc 38400
aagatttggg tacaaggaag ttataatttc ataatttcat gatagataag aaaatataac 38460

aatagatgct agtgacttgc tggccatttt ctccatgggt caaagtgcat gacttatttt 38520
tttacactag tatgtcagtt tattaacaca ggccgaacag tttagtcaca gcttgaaagg 38580
gtttggtttg gtttggtcct gaaagaccag gtacagagtg ataccagatt tttccaacaa 38640
cttgctggca ttatcctaga aatcagtaga agttttggtg tttacccctc catgccttgg 38700
tctacatcat gctatgcaga tggcacactg ctttggttaa cctcagtctg acacgcagat 38760
cgtgagttct cttctgccat gctcctgtct gggggcgtga tgtgaccgat gaagtgtcac 38820
caagaaataa gacaaaattt cccaggtcta ctcactggca attctcttta tttagtaggg 38880
gttttccaca ggaaattgca ggagaagagt tgaagtgctc tttgtcttgt gtactttcct 38940
ctgacttttc tccttcccag agtggcccac attgccttgt aaaggagctg tgttgaccat 39000
gatactgatc aaagtcagag tgcgttctgc cagtcagcta aagcctgatt gctcctctgc 39060
agtggacttc tgtagttaaa ctctgttttc caaacatgaa aacacataaa caaggactgg 39120
agatatggct cagcagctaa gatcacatac ttcttctgca gaagacccag gttccgttct 39180
aagcacccac atcgggcaac tcacaactgc ctataactcc agctccaggg gacccaacac 39240
actcctctgg cctctatgtg cacctgcact catgggcaca tattcacaca tatatacata 39300
atttaaaata aaaatctttt aaaaattaag gaacatatac ccagagagta agtatttgtc 39360
tctaagaaga aataaatcgg tgatattaat ctgaaacaaa tttggacatc acagaatcaa 39420
aaatatttct gattatccta aattaagatc aaaatcattt taagggaatt ctggagaggt 39480
ggctcattgg ctaagctctt ctctgctctt gcaaaagacc caggtttagc acccaggacc 39540
cacatggcag cttggcaact cacagccatc tgttgtccca gtgccaggag acctgacacc 39600
ctcttctggc ctctgcaagc acctgcacac acatggtgca cgtatttcag ggaaaacaat 39660
tatagacata aaattcaaat taattttaaa atgtcattgt aagatcatgc tagataaatt 39720
ttgtataaat catgtctgtt ttcagacagg gattctgtac aaatctaaca tgcattttag 39780
acgacttagt tttgaagttt acacaaactt tttttaaaac tttgcttaaa atagctaatt 39840
atgacttcat ttgcctgttt tggccctgcc ccccatggcc aaagcctgct gacatcccca 39900
ggaccaatag tcccttattc tcagtactat aataagtact gagctcccag ggcagagcct 39960
gcactgcagt cactgaaaag cactgtgttg agtacccaga cccagcctgg atgggaaaag 40020
ttaatatcat tgcctaacat gaaggtttc tttgtctcta aattttgatt tttccatgct 40080
atcagaggcc cccttgcaac aggtaatctg agtgttgtct gttttctccg tccgtcatcg 40140
tggttaagtg tattttagat gctcacaatt gtaaagctaa attgacttga caaggttctt 40200
tccgacccaa attctgtgat gtccgactct ttttttcttc ttttattttg gatttgtgat 40260
tgttgaccat tgtcctgtat gtcttacata tatactttag ggaaccataa gcattttaga 40320
gctggaagcc atgctagctg tgggctagga ctagacattt tgcaaagaaa gcattaggag 40380
accaaagtgg ttagtgattc aaacaaattc atagcagtag ctaaagacta aaattctgac 40440
catcttcaga ggactaaaaa taatcaaagt gttatctgaa aatttgtaac tccaagccag 40500
gtatatatat atatatatat atatatatat atatatatat atgtatgtat atatatttac 40560
cccaaacaaa ttaaagcttc ctataattct gtactcatat atgtatatgg ggtgtgtgtg 40620
tggagagaga gagagacagg gaaagagaag gggaattaaa tatttttgat aaacgtaagc 40680
tttgataaga tctgcctgac acaaaataca aaatgcattt ggtataaaca gcatcattct 40740
ccctttatac caaaaacgga aatttcttcg cattctatac atttgagaat gacacgtcgg 40800
gnnnnnnnnn nnnnnnnnnn nnnnnnnnnn nnnnnnnnnn nnnnnnnnnn nnnnnnnnnn 40860
nnnnnnnnnn nnnnnnnnnn nnnnnnnnnn nnnnnnnnnn nnnnnnnnnn nnnnnnnnnn 40920
nnnnnnnnnn nnnnnnnnnn nnnnnnnnnn nnnnnnnnnn nnnnnnnnnn nnnnnnnnnn 40980
nnnnnnnnnn nnnnnnnnnn nnnnnnnnnn nnnnnnnnnn nnnnnnnnnn nnnnnnnnnn 41040
nnnnnnnnnn nnnnnnnnnn nnnnnnnnnn nnnnnnnnnn nnnnnnnnnn nnnnnnnnnn 41100
nnnnnnnnnn nnnnnnnnnn nnnnnnnnnn nnnnnnnnnn nnnnnnnnnn nnnnnnnnnn 41160
nnnnnnnnnn nnnnnnnnnn nnnnnnnnnn nnnnnnnnnn nnnnnnnnnn nnnnnnnnnn 41220
nnnnnnnnnn nnnnnnnnnn nnnnnnnnnn nnnnnnnnnn nnnnnnnnnn nnnnnnnnnn 41280
nnnnnnnnnn nnnnnnnnnn nnnnnnnnnn nnnnnnnnnn nnnnnnnnnn nnnnnnnnnn 41340
nnnnnnnnnn nnnnnnnnnn nnnnnnnnnn nnnnnnnnnn nnnnnnnnnn nnnnnnnnnn 41400
nnnnnnnnnn nnnnnnnnnn nnnnnnnnnn nnnnnnnnnn nnnnnnnnnn nnnnnnnnnn 41460
nnnnnnnnnn nnnnnnnnnn nnnnnnnnnn nnnnnnnnnn nnnnnnnnnn nnnnnnnnnn 41520
nnnnnnnnnn nnnnnnnnnn nnnnnnnnnn nnnnnnnnnn nnnnnnnnnn nnnnnnnnnn 41580
nnnnnnnnnn nnnnnnnnnn nnnccgtgtt tgctgctctg tactggctat aacctgcaca 41640
agtaatggcc tcaatacaac tgttttctaa taaaagcatg cgtgatgtta tacctctaaa 41700
gtaagattgg tttatgtagg tctgccattg agagtggtta tgggaggatt cctgtccagc 41760
attgggactg tatctaaaaa cagcagtcta gctagaaaaa aacagcacct gtacaaaaaa 41820
acaaaaacaa aaacaaaaaa agcagcagac tcaaaatagg aatctcacag gtctaatggg 41880
aatcctgagt acttctgctg cagaaaagta tccttaagag agcccaggtg aaatagtgtt 41940
cgaaagaaaa acagggacat gttgtgcaaa aaagatagac tattgctttg tttgtaagga 42000
gatgggtcag ccgtacgcgt tttaatagag tggtaatgag catcactaat ccctgagctt 42060
cagagcacag actcggcgtc tgtctgcaaa gggggctgag ttggagcact gttgtgattg 42120
attcctcttc agttagactt agagttcaag ctaagatgga taggcaaggc agtgagatca 42180
gaggaaaggg ctgagcacgg ggggaggggc aggggacgag gagggaagtg ccctggctct 42240
gaaatttggc agttaagtgg agataagagg cagcgcactc ccacacacta ggacggtttt 42300
ggtacagtgg ctctgttcaa caggaggcaa cgttaggttt aagaaaaaag gtaagctggg 42360
cgtggtggtg cacgccttta atcctagcac tcgggaagca gaggcaggcg gatttctgag 42420
ttcaagacca gcctggtctt cagagtgagt tccaggacag ccagggctac acagagaaac 42480
cctgtctcga aaaaccaaaa aaaaacaaaa caaaaaaaaa gaaggaaggt aattagttag 42540

gcttgggcac atggagtgat aaactgctgt gaacagaagg gcttcctgct aagggctaga 42600
aatgtcacac atgcttatct gagagatgaa aacactgggt tattggaggg ggctgtgaga 42660
gcgagcgagc acagcccggg catgtgtttg aggacaggag cctaaggaga gccagaggta 42720
tcaggtcaca gtggtgctgg ggctgcaggc ggttgtgagc ctctccaaac aacgcggcca 42780
tgaacaacca aattcagatc cactgcaaga gcagcaggct cttaaccaac aagcatctgt 42840
ccagcctgta gactctaagt tagtatcata tggagtagat gacacaactc catcttgtgg 42900
atggatatca gataatgtga tgacttccag aaagagacta agagtcgtgg accggcgctt 42960
gtgagctctt tgagaagggg aatgtagcca agcagctggt gatgggggag aagggaggtc 43020
agagggacca cgtttcaggg attgtcatag gtcaaaccat aagtgactca tgggtttgga 43080
aacttaaggc agtcagaacc gttaagcaac ctttatttga agactagtag aagtacttaa 43140
ctttcagaag acttcgatgt gagaagctgt ttctccctgg tgactagagt tgttcttgaa 43200
cgcttgggtg tagagaaagg aattcaagca agagtgggta gactctttta agaacctttc 43260
tcacagactt agaagaagaa aaaaaaaaaa aaaacgtcaa atgaaacaaa caacccccca 43320
ccccaagcca gccctgtttt cttggagtgg cacctgccgc catgtgtttg aataaatgat 43380
agcctttacc atcatcaaga aagggctgct atttttagaa taagaaagca aaggaactat 43440
gtggatagct aaggggaagg gtcatttaga tgctttgtag ggtggagag taattagggg 43500
atctagaata aatattgtgg gtaaaacaag atggcttttc ctggaaaagg gaagagacac 43560
agttctctga attagtgaca agaacaggtc ctgagcttca gtaacagaaa ggagagacat 43620
ttgtggagga ccattgtgga ggcttctcgt tagatctcag gaaatcccag cagcagatgt 43680
ctgtgagggc aagaagagat aaaggagaat tattttgcat caagaactgt tctaagcaat 43740
ttaaattatc tcttcttaat ccttgtgaaa actcagagac tatgacattg aaagttaaac 43800
aactttccca gagcctgata tcagcttgtt atggagggtc taggattcag agctattctg 43860
tctgactcca acgcctgtgt tcttatggtg agaggactca ctagtttcag gtcttggaag 43920
ttcagcccta tatcatagca ggatgagaac tttgagggag gtccatctgt ccatcagaga 43980
aggatgctct aatatattgg ttctcaacct acctaatgct gtgactcttt aatacagttc 44040
ctcatgttgt ggtgaccccc aactataaac ttatctttgt tgctacttca ttgctgtaat 44100
tctgctactg ttatgaatca taatgtaaat atctgatagc aggatgatct tgggtaaccc 44160
ctgtaaaagg gtggttcagc cccccaaaga gtctctacca cgggttgaaa actgctgctc 44220
tcgtgagaaa ttgtgaggct gagaaattgg ggtggaacaa aactgtagac cctgtaagtc 44280
tgacagaagt taagataaat ggacagaaga caatgtgagg cagatgcagg agacaagagg 44340
cctcaggtca gcagccaact ggaaacagga gggaaggcaa aggagggttc tgacttcatg 44400
gggacagctg ttggcttgac ttgactttct ggtggtgatg actgaagagt ctatttccac 44460
agctgctgtg atttctttgg aggtttcggt accaattctg gtctagagac aagaccaggg 44520
tcctagacac acagttaagc ttaggttgtt ctctggtatt taataaacaa gctgtaactc 44580
aaatagcttg ttgagaggct gtgttgggat ccaggtatct ttaaaggcaa aagaaaaagc 44640
atatatcact ttaatttctt cctacctaat ataactgttt acactttagt agtttgcctt 44700
cgataaagaa catttagtgc tatctcaatt gttagtcacg cccacgttaa caaggtaaag 44760
ttacaggaag tactgtgagc acaaggaata ggaaggacgg gtgaaaggaa cgcttctgtt 44820
cttatagtcc ttccattctc acatagaaga taggacatta ttgatacagg ggacatgtta 44880
catacttaac agttattaac atacagagaa aagatgaagc agggcagaga gcagaagatg 44940
gggtggtcgg agcttaggtg gaggaagatt cgaaaaacac ttgtctgaga agagagctgt 45000
gcacagactg gagctggaca gaagacagca gacatcctgg aacaatgtcc cagctaagca 45060
cgcaagtcca aaagctctct ggtgggagcc ctcttggcaa gttctggaac agcagggtct 45120
actatagatg cattggaaat acaagaatca aggttgaatc aggaccccac tcccccaaaa 45180
cccccgaggc tccctcagcc gctgtcgagg ctttttaaga atagagacat taaaatgttt 45240
tatttacttc tgttctttaa catgggaaat tgtaaccatg tattcccaga tctgtgtgtt 45300
gaggcccttg tgctaatcac tcaattaaca tgggaaattg tcatgtattc acacaactgt 45360
gtgttgagcc cctgtgctaa tcacccagtt ccaagtgttt tcatttttgg aattttgaac 45420
tgaggaatga tgacttagca tgtcctgact tgaacaaaac tcactcagaa caggttctga 45480
ggaggcaggc agggagccag ggagtagcct gagaagtggt tatttggggc ttcgtttgaa 45540
gatctggctt gattttattt attttctaat ttttttcaaa agaagaactg aaaaggcaaa 45600
tagaacagtg aaaagagaaa ggtaataaag aggacccagc ccagggagca ccccttcaca 45660
cttcaccttc tacctagacg tgtacctgcc tggcaaaggg ctggcagagg gaatatttct 45720
aacaccatcc tataaatgac aagccacatt cctatgctct tatggaatca tggagtatta 45780
agcccccaca caaaggtgag gatttgtcac atgtcaccaa agcattgaga cccaattgta 45840
cctgaggtac ctaggggcgc ttgacctcta gatatttatg atgcatgtga taagtttgaa 45900
aattaaaagg aaaccaaaaa gaaaacaagg aagccctgca tcaaggctga tgaggtgaaa 45960
ctttcagaac aggggtacat gcaggtagag gtgtttctct cttctcttct cttctcttct 46020
cttctcttct cttctcttct cttctcttct tttttctctt ctctcttctc ttctcttctc 46080
ttctcttctc ttcgtgtgtg tgtgtgtgtg tgtgtgtgtg tgtgtgtgtg tgtgtgtgtg 46140
tatagactag agtttattca gggcctgggg aggggagtta agagggtagt ggagtcagag 46200
aaaggcagag agagggagag agtagagaag tagttgctgg ccatgaccat gtggagagag 46260
aggagaaggg aatggggaga gaaggggagc aagagggaga ggcaagagag caaggcaaga 46320
gtgaggtgtt accctctttc agagcaggat gtctgcaggt agaggtatta ccttctttca 46380
gagcagaggt gtgggcagtt agaggtgtta ccctctttca gggcaggggt gtaggcagat 46440
agaggtttta cttgcctgct ctatatgaaa ccctcctatc tgagtggtgg tttgccattg 46500
tagttgcagc ctgactcttg agatgttgat cacactagta gggagtgtca ggaacagtgc 46560
aaatccatga accctggtga aggtccaagt gtgccagcaa tagtggtgaa tcaaagaggt 46620

tttcatgtat cctgtccctg ctttgcattc ccacaccaat attcagaggg ctaagagtga 46680
actccacagt gttctgcatc ttggctgtct ctccaaagct tacatcactt gctcaggctg 46740
tctaaacttg ccttcttatc tgcaagttag ggtactacta gtgccaaccc tgcctatctc 46800
agggtcatca gaaagcccag atgttctcca tagataagag ggaaggacaa annnnnnnnn 46860
nnnnnnnnnn nnnnnnnnnn nnnnnnnnnn nnnnnnnnnn nnnnnnnnnn nnnnnnnnnn 46920
nnnnnnnnnn nnnnnnnnnn nnnnnnnnnn nnnnnnnnnn nnnnnnnnnn nnnnnnnnnn 46980
nncaagacga gaccagtgtc atgagtcctt cctatgccat tagctataat cccgtttagc 47040
aggcttgatc tgtgcttcct ggttgcacaa ataaataata aagaacagta aactgactcc 47100
ctgtcatcct tgagaggaca tacattatta aaccaatata ctatttcgtc tctctccctc 47160
cctccctcct ttccttcttt ataatttgac ttgactcaag aagagtaagc aagtagcctt 47220
tttttttttt tttctttaaa tggaaaagac tttcaggatg acaaggggac tcttgctcct 47280
ccttttcaca cctagcatga ggcctttctc acaatatgcc tcaccagtgc caacaaacaa 47340
gcaggagtct ctgtagctgt acctgtgctc agatcggtga ttacagccat ggcgcattaa 47400
agccagttag agagagaaaa ataaccgagc cgagtgttga catggtgccg tggaaatgtg 47460
aatagacttt ggggaggcaa tggcagaccc cgagttcagg gcaccaccac cttaccccaa 47520
accattggaa ccaggtggtg catgttactt gagcttgcaa agggctggtt ctttccccgg 47580
tgatatgggg tgccactact gaccccacat ggctattgtg ggatttctc cctcctcggc 47640
tgtagagaca gaatatgcac atagacttag atgagcacgt gctctgggaa atgaacaagc 47700
atgcgaagta aggcgttatt taactcttac tagccagtca catatcactt tcctacccta 47760
tgtcctccag tcattctcga aaagggcaca gtcgtggagc catgatgggc tgacattgtg 47820
aggaaggtgg gaaacacaag agacacgggc tattggtgca gctgcttgaa ggaactcctc 47880
ctgtgcgctg ttaatgagca ggatgagggc tcgagagcac tgagcaccct gcgagcatga 47940
ctgcgctcga cctgctgaga atgcctttcg agcatgcgct ctacccgccc gcagagaacg 48000
cctgccaagc tgcctcatcc tccacttgct ccttgcttac tcagctcaac tctgccttca 48060
aatctcagtc cgcatgagtt aaacacacgg aagctttctc tcacttccca accgtgccag 48120
acactgttat tagacattct gtttgatgag ttctccagtt aggttttacc tgtattgttg 48180
aagttatcca gttagcgtca gtcccactgt ttaattccgt gagagaaaga aattgggctt 48240
gttctgaacg aggtgtagcc cccactctct gaccttctac agtgtggggc tcttagaaag 48300
gggcgtaaca aactaaactg gtaaacttta gtactcagtg aaataatggg ctagataaga 48360
aacaattttc tgagattgtt gatatcatca ggaaccaagt gttgtcttaa ctcctttaat 48420
cttaaaacag tccggtgata cttcgtcatt aatttgattt ttgaaggtgg agaagctgac 48480
acacgtcact ccacgacagc tgggggattt gagcctggct ttgagagatc ttaacctaac 48540
catgacctgc tgtggtaaag agcaaggttg ggttcacaag cagagccagg ccctgttaga 48600
accctgagga tgttcgaaag aaaaaatcag cagaaaagaa tgattttagg tatgatgtct 48660
tacatttatg ctgcttctgc tttctgtcag agtacgttag ccgaattcac ccagagagca 48720
gcctggcttt tgaaactgtg attctgagga agttctaggc cactcatctc ttgttacccc 48780
tttgaagaaa cagcaaatgc ttttccacag taggtcaggg ctatctgagg ttaaaaaaaa 48840
aaaaaagtac gtggctgact aagagggcta tacctgagcc catgacttgt agtttttatt 48900
ctaaaaggag ctctgaccca tggacccaga attctagaag agttttcctt catcagggaa 48960
ggccaaggaa gtctgattgt ccccaagaag ggcagctgac actagaataa catagtattt 49020
tgtagaaaaa taaaacatag caaaagaaag aaaataaatg tatttgtaga aaactaacaa 49080
aacactgcaa tgtaggaaaa aagaaaaacc ttccccttgg aattgatgtg ggccaagtat 49140
cttccagcca cttgcttcaa atggacctat tagacatcct ctaagtgagg gagcatctga 49200
cagtctgaga tagcccctga gggttgtcct ctcctctgct gagtactgtt gtgaagtgtt 49260
tgtgtacttg aatgtatata ttcctcatta acagcccctt gaacattact gaacaggtga 49320
agagaccaag caaggtcaca cagactggaa ctgcctctcg cccccagcca gcctagcctg 49380
tctctgaagt ccacactctt cccattccga gcagctgact atgcaggatg gctcctttgg 49440
cttcttacct gtgcttgagt gcttacaatt aagagtctct gttcagattc tactctcaga 49500
aagaccttcc tagtagacag ttgccctcta taaactttat cttcaagaga cgtaatactt 49560
ccattccctt cccagaataa gtttctacat tggtgttgaa tacctcttgg ccactatggc 49620
atgataaatg atggttccaa gaaatcgttg tcaaggtttg cctgtgccgc acagctgtcc 49680
ccacaagtaa ggctttcttt ctctcagatg gcatcttgga aagtacagaa agcccagggc 49740
cctccctgtt ttcattctgt ggtctgtgta aaaaccaaat gctaccatcc cgactacact 49800
ctttcagatg gatcaaacag cacattggtc actggcagat ctttcaggtc cctcacctca 49860
gagtcctccc agttcctcat tcttgccgcc cttcaaccat gaagttagtc gtaagaaaaa 49920
cagtaagtgc ttcttgaatg cacctcccac ctctttccat cagcgccagt gactaactag 49980
cctgtcagcg cagcttcaga aggctcggat tccaaactct caggccttct cccatgaggc 50040
ctttctgctt aagtggaaag cctgcctgat ttcacccaag tgaagcgctt ccttgagcct 50100
tcctgtgctg tgttcctgtg cacactgtca gttaaaagtg ggatcacgtg actcgctgcc 50160
tctgccgatc attagcagac cgatgtgatc agccatccag agctggaatc agccatccag 50220
ggactttgta ggaagagcat catcagcctt ggagcaaaac cagaacttgg tattcattgt 50280
cctacactta aatggggaag tatttggcct gtgaagaaag acgacttgct aaattcactt 50340
cattttaata tcagagagag ttttaagttc tgtgcctaag aaagcaactc atgctttggg 50400
acagtcaagg ttaaagttag tacaaaacct ttctctgaat taggtcactc tgtaactatg 50460
ggaaggcagt cctgctgatg gcctcgccgc tctggaagaa gtatgccctg gtctgtttta 50520
tgttgtgttg gttgacaagc aaattacaca ggattaaagt ttgcttgggc tggggtttga 50580
agcctgggag ggttcaggtt gttggtcagc catatctgac aggaactttg cagcccagga 50640
caagacagga aattacataa caagagaggc catcttggct gaagtatctc ttcctctttg 50700

tatagctcac agctccgtag atgagggctg gctcttatgg tgtattcgat tttacctcct 50760
aaagatcaac ttccagacac catcactgga ttacatttct aggctcaaaa cgtctcatga 50820
gtttcggggt gggttcagaa gcaactcatt ataatgttgt atcagggctc aggcaggcag 50880
gcctccttgg ggagtctaga gagctggctc gctggccaga agggagaccg ttcgttgttc 50940
agggcaatgg tatcttttct ccatactctc tggggagtta cgcgagcagt gatcagcttc 51000
ccggctctga ccatcaaagg ttacactgga gaaaatccat attgagagca aactctgtag 51060
agccctgcct ccgtggagtc tggaccccgc tgatagtagt gatattataa gtgagcaggc 51120
tgaggaaggc tgtgtgtctt tacaggaagc tcctttagca gatgcaggct cttcacgttc 51180
acgttgacat ttatccagag aggcgacgta cctcatgcat ttctaacaat aggtgggatt 51240
tatggcaggt tctgacttaa tcgaattcag aggggagtaa tgcctccgag ctactcatgg 51300
tgtgagtctc atggtgtgat ttccacggtg tgattctcat ggtgtggttc tcatgttggg 51360
ccatcttgac gaaaagttct acactgtaga ggtcgggatg gtcaactaga caagaaagac 51420
ttggtcatct cctctctcct gtcctgtcca ttgctgttct taccacgtta caaatacaca 51480
agtgaaatga tcgcctcaca tggaaaactt aactttgtaa aacaagagct aaacaaccaa 51540
atgtgaaacc tacaaatttt tagtcagaaa gtttgctttt tttatttaag aaactcacag 51600
actcctgaac atatacagta taatgtcaga acccatgcct ggggaaacta ctgaagagta 51660
gtgggtgtcc tgttaggtga tgtggaccaa gccatgggct tatgtcatta aaaacagcct 51720
ccagagccaa gcccctgaac aagactacct ttctggtaat cacatgacaa ggagttagga 51780
ctcagtcttt gtttgtctct cttacttcat cagtcaccca ttcccttctc gtgcatggtt 51840
tgcccagttt tcatcactga aaatcgtgac aaggtttcag agaagaaaca gtgttggtta 51900
gtaaggaccc ttgtgacagt cctacctaat gaggcatact ctgttttcag tgacttgttt 51960
cctggacata taaacaaccc ccactaccat tttgatttat ttccatcctg tcatgtgttc 52020
attgtatatt ttttaagttg agctatagtc aaatatgtat atatctgcaa aatgttgtat 52080
ttatgctgat tggttccatg ttacagtcag gtcagatgta ctaactgttg aaaatgtgct 52140
tctcatatag atttgcaact atgtggggcc tgcaaaggtt atcgttcagt tggtcacaaa 52200
tggaaaaaac atccacctgc acgcccacag cctggtgggc aagcactgtg aggacggggt 52260
atgcaccgta acagcaggac ccaaggacat ggtggttggg taagtgggct gtgtggtatg 52320
gagggagcgg gaacacgcgg gtgtgaaatc cataggcccc tttccttgtg gacctgtgca 52380
aagcctgaga gggtctcaca cacgcgcaag cagataaagc caggggaatg tgtgtcctct 52440
cctttgaact gctaaggaaa tagaggtgac cagatatctg tcctgcctga gacaatcctt 52500
gcatctgtag tcttggcctc tcaaggatgg gtttggacaa tgttataatt ataagccctt 52560
gacctaagag gcatttgtgc cactttcaat tatatttttc ttctcctgga attaaagatg 52620
aagaaaagaa tgaaacaact accttaacta tatagaaatg attaccatgg agatgctttt 52680
tgcttccttg aaacagcatc tccactatag tttaagctta ccttgaactc acatagatcc 52740
tgttgtctct acctcccaag tgtttgggtg ctgggattag agatgtgtac ccccccacac 52800
acacacagag ctctgatttt tgcctatttg ggattttctt aatagagttc tagtgggaac 52860
atttgagaca tgaagcagat aagtaaatat tgtttcaact ctgcgctact tataaaatgt 52920
gtgcttttta agtcaacaag acatgcaaat tggtttgggc actcatttga tctggaacat 52980
aagtagaaat tagtgtattc tttcaaagtc agtcaaatac aacaattttt agtgaatgaa 53040
attttataga agtaatctta cttcaaaaga atcacaaaat atttaataag acataattat 53100
gggctgagag aggatgagtt tagtttgcaa gtatgatggc ccaagttcaa accccctggcc 53160
tctcatagca gcgcttttca gagcacaggc atttatcctc ccagctcctg caggggagca 53220
agaggcaaag acaggagaat ctggaatctc atgcagtagc tggcctggtg tggtggacag 53280
acaccaaaca gatcccatgt caaacctgaa gttctcctca gacctccata tgcacagcac 53340
atcacatagt cagctgcgtt cgcacacata aaataagggc actcactatg ctaaggtttt 53400
atatttcatt atcctgaaac actaaaatag cagacaatgt agtaacttaa acagcaaaaa 53460
atgatgtaag cattcagtca tctcaaatac agataactcc tattttccag actcttaaaa 53520
agcaaagggg atgattcagt gggtaagtgc actttccacc aaatctgaga gcctgagtcc 53580
aactcctgta agccacatgg caggagagaa ccaacttcca caaattattc tccaacctcc 53640
acatgcaaca aaggtataag tgcacacaca caccatgtaa tataattttt aaataaaaaa 53700
aagttaacta aaatattttt aaagccagaa ggtaagaact ggggagacag gtcagtgggt 53760
aaagtatttg caatgcaaac ttcagggcct gagttctgag ctccagcgcc cacatgaaaa 53820
tccaggtgtt aaaaaaacca aactgataaa taaaataaaa ccgggaaagg gcagagcaca 53880
gcggtgcatg cctttaatcc tagcactcag gaggcagagt ctaaggccag cctggtctac 53940
agagtgagtt ccaggaccgc cagggctaca cagagagagt ctgtctcact gcccccaaa 54000
gccaggtgta gtgatgcacc tggaacccca atgtctggcc acagggatgg agcagatccg 54060
cggggctcac aagccagtca gaaagcacgg cccagtccat gaccctgtct caaaaacccg 54120
agaagtgact gaggaaaatt ccctgcctgt gttctctgtg gtgcatgcag atacattgtt 54180
gttaatttta tttaattgtg tacatcatca ttcagagttc tccagtctta gatcaagtag 54240
tgtctgggaa gaccccagtg aatgtgtgct ttttttagga gattgcttag ccagccttga 54300
tgaagtctga ctctgttgcc ctgcaatctt gggaggttca gctttcaaag tcccaggcat 54360
catttcttca tttgacgacg aagatgctca agtgtgagaa gctcccagag agagggccct 54420
tccctttaag tcattcatct ctggtgcttt ttgacgttag ggagaaaagt atccgaattg 54480
gaggaagctg aagttagttc tcgtggactt cagcttgtct cggcatctcc tggctgccct 54540
gcttctcaat ctgccgaaac tgacatgcag ggtgactgtc agttttagac aactgatcgg 54600
ttagtgtatt ccctgattga ttggccacat catagccaat cgttttgaag catttccgtt 54660
cttttcgtct tgcttaatct gtcttacaat catatgttct taaggaaatc acaggtgaac 54720
ttttctatca gtatttctgt cagtaatatt tattattata aaacttgcag agtgttgctt 54780

ctctgttggg tttaataatc tttcatgatt tagaagaaac taatggtggc tgtgtctgct 54840
gctctgaggt agacacaagg ccttgggttg ctctcccctg cagcactacc gccactaaaa 54900
acactaatgg ctgtttatgc tggagcctag gttgcagcga tggaggtaaa ttcagtacct 54960
gtaagcagag cggagaggtc cgtcggtgtc ttttagtgta gtggggcacg cggctctaga 55020
gttgggagtg tgcattactc atgatttagc aaacattttc gacatccgat tcctgactag 55080
gctccctgtg gtttgatcta gtcatagctg ggtgggattt tctaaggtga ggaaactttc 55140
tcggagagat gcagtcgagg agatgccgcc tgagtgatag gggtaacca cagtagcttt 55200
tcccacccaa gaatctctca ctgtagaatg tctgcagtcg ctttcccggt tggcatttag 55260
ctgagagaat tctgctcatg gtattttccc cagacaccat tttcttcgtg attcccttgt 55320
ctctgtgtct ctgagtttct aacacggtcc tacacctttt gccttttctt ctgtttctct 55380
tggatggaaa agcagcgtgc tagcagccgg gaagtcagag ctcctgtatg cttctgtcac 55440
tctgcacggc agaagggatg gaggccaagt tgcatgactt tctaaagact tgaattcctt 55500
tccttacaaa atcagaaaga cacaggctct gtctatctcc aggcactcag tgtttaaccc 55560
tctagaccaa gcccacatta gctaatgtta taaaacagca ggaggaaact tgccactatc 55620
tcctcatggt atgacatcgg ttattttatt tagttctgac ttgtaggcca gaagtttagg 55680
aaggcagcca aaggtgtttc ttagacttgt caatactaga aatagaacca agataacatt 55740
ttccaatact acagattaga ctgaatatag attcacaaac aaggcaccag tccaaactat 55800
caacatatgt aaaaatgacc ccaagaagac caagctttga acacatcaaa accaaacgga 55860
ccccctttgt ttacagtgtc tttcacactt tcttctctgt ttgaattctt aataagttta 55920
gtttctattt ttccaaatta ttttatcagt attagaacta tcatctcttg ggtttgattt 55980
atggatttga ttgttttaat atagggtcat taacttcagt tagctttcat tagacctctt 56040
gtctcttatg cacttagtta gacttcactg cctttcttaa taaaaaaaag aagaagaatc 56100
ctgtttagta ttctattttg accagaatgt taatattttt agcaacactc tagttctctg 56160
aaatttttttg ttcagtggaa aatgtctgtg catttctact ttgctgtcgg ccacgtccac 56220
caaaagtcac cacaccctgt ttgcttactg gcaacctggg ttgaaagaga tgtacagttc 56280
tttttatttc cccctcccaa ctaatttatg tagcttattg cagttggtaa gcaggagagc 56340
agagattctc aacctgtcgg ttgcaacccc tttgggcgtc aaatggtctt ttcacagagc 56400
atgcctatgg agatttatat taagctttat aacagtggca aaattacagt tatgaagcag 56460
caatggaata attttatggt tggaggtcac cacagcatga ggaactgtta aagtgttgaa 56520
gcatgaggaa ggctgagagc cactgccctg gatgatagta gatatgagaa cacacaggag 56580
agcctttggg ctccctcagg agaactgcta ccttaaatta ctgctggaca gatccagtgt 56640
aattgtgtag ctaaacacta gcccacagga gaggcactgc ctagagaagg ttggacaaat 56700
atggacttag gttcaaggtg tgccgtacca agttggtgta tgcactgtga gacgagagct 56760
gcttttgaac actgaaatca aggtctgaaa caacaaaggc caaagagggg ccactgtgag 56820
ccccaaccca gtgttagaca catcaaaatt tgagcaacag cttccaaatg atgatgaaga 56880
tgaaacatct gacctacttc agcatgttta ttgatggcta caggtttgag tcagcctggg 56940
tcacttcata gaaaactgat gccagcctcc atcccataga gtgggatttc acagagcagt 57000
gaaaagccag gccctccagc ctcagcattc ctcttgagtt aatggaaagg tttgtttgaa 57060
aagtcctgat tccgtagggt tggatggagc cccagatttt tagtaaattc taaaacaaca 57120
ctgatgttgc tagttaaaga ctcccatgtt gtgaatatga gaggaagaag caaggttgtg 57180
gtttcagccc ttttattagt ggttgtagcc tttctgtttt taaaatgtca ttgctgcaac 57240
actcctcctc tccagtgttc atttctgtga attttgataa tcatgtggct actcctgatc 57300
tgaggctgat gagttctctt tggttttatg tctgaaagtc gtcccttcat cttctctgtt 57360
caaaggtgtt ttttgaagag cagaggattc taagttgaca atatgtactt tcatgtgctg 57420
gttttatgtt gattcaccgt ctcaggtctt gacctgtttc cagcaagagc tctctgtagg 57480
tctgcctcgg ctctactgtt cccagagtgt gattgtttct ctagcagcgt cttgacttct 57540
ctttagcact cgtaagcagt gtgagttttg atgggtttga gggtagcttt ctttttaagc 57600
ttgagcttct tagtcgtcat agatccttgg atttacattt cttattaaat cttaaagctt 57660
ttcaactgtt aagttttctg tcactgtcac tgcccctgct ccccattcct gttctctgtc 57720
actttctctc ttccagacta gggttgagtt ggcacagtga ctaagagaat accttctgaa 57780
tagtcaatct gatgcctcat gtattccgag gcttcctgtt tatgagaata gtagctcttt 57840
cccttagcct aacagggctt tctcacaaat gtttcctttg gatttttgaa aatctgtgat 57900
gctctggata taaatcctaa tcttcttaac ctcaagtctt tgcaactgca gagccctagc 57960
tggctgcctg ggttctcctt cccatcctgc aacctaagca ttaagccagg taattgtaag 58020
agtacctttc tatttttttt tctcggatga ctgttgtgtg tcacctatct ccagagcatg 58080
acaatcgttt tctcgtgtgt tttggctatt taattctaga ggaaccgtct aggtctatat 58140
tactccacca tggcctgtgg tggaacactg gtgtgttgtt tactagggta aacccttcct 58200
tcatgattca tataataaat aagcatgatg cctttctcca gatgtgaagc tgggttacgt 58260
gtaagaagtg cagccgaggt gatggtcact gatgttccca ggagatgagc ctgggctcct 58320
agcgggggag cagtctgcac aagagtgaga cccaaaaggg cagacacaag atctgttaga 58380
cacaaggacc tccaagcaca ttagcaggtt gggtgcataa tgtttgtgat gctccctccg 58440
ttcaagcttt cctgtggtca gcaggcacct gctgaggacc cacatgtcag cctgcattct 58500
aagacctggg gatctctgtc ctgaaggagc tgtggaaaca cagcatgttg acttctctgt 58560
gtccctgtaa ctcactccac atctggcctt ctgtgtgtgt ctgtttcctt ctgccttcac 58620
ctcttctcct ccctttctcc agtttatatg acctttgcac agtagcttgt tctcatgtgt 58680
gcaggcaagt tgtcagatct cttttattgt cattttatat tacatttacc gtgtgtgtgt 58740
gtgtgtgtgt gtgtgtgtgt gtgtgtatgt gtatttctgc ttgtcggggg aggctatctg 58800
ttctctctct ctctctctct ctctctctct ctctctctct ctctctgtgt gtgtgtgtgt 58860

gtgtgtgtgt gtgtgtgtgt gtgtgtgtgt gtcagtgtca aagcccagaa caacttgtgg 58920
aagccgcttc tcttcccctc ctatgtgggt tctagggatt gaactcatgt caccaagctt 58980
ggcagctagt gttcttactg acttagccat ctcactgacc tgtatcattt tcttccttct 59040
actaatattt attttacttt tagagtaaaa gtccttacaa acatctatat ggctctgaga 59100
gacccaggcc ctgcactttt ctctttgagt tcatctgtcc cctctgactc tgctccttac 59160
cctcagccca tgtgccacct ttctgccact gtcaaaaaca gcagagtggc cctgccatgc 59220
ggtcttcaca ttcaccagag tggtcctgcc atgaggtcct cacactcacc tgtctgcagc 59280
tctgatactt agaatccact tcttctgacc tccctcagga ctggctcagc tgtgatctca 59340
gggtcctgtc tgggttctcc ctgtctggct ctcctaccct ctcacccctc ccaggcctgt 59400
cttctttttc ttgtttacat gtatttctct tttaagttac actgtgaaat ctatcatcgt 59460
gtacgctagc atgtgcattg tgtgagagca cagtgaagcc acacaagcct tccctagtaa 59520
agcatacgta caagggcacc cgtactctgt gactcctgag ttgacatcta atatgttctg 59580
agacgtaagg tgattcaagt tttcctccaa ctcttttagt aacccagttt aacctggtct 59640
gacaaatcca cttctgtgat gagtctggtt agaggcctct ctgcagaaat gtcaatgagc 59700
tgagtccatg gtgcacccca gccctgccaa cagtgtctag tgacgtacag catatgccat 59760
atatcaccct cataaagtcc caaattctga tgttcatctg actttggtct gtgtaagatg 59820
ctgagtgctt gtgtactttt ttcggtgtgc tgtctgctga cctgaagccc gcccccggc 59880
catgacctgc ctgacttctg ctctcactgt ttttctccag ctttgcaaac ctgggaatac 59940
ttcatgtgac taagaaaaag gtatttgaaa cactggaagc acggatgaca gaggcgtgta 60000
ttaggggcta taatcctgga cttctggtgc attctgacct tgcctatcta caagcagaag 60060
gcggaggaga ccggcaactc acaggtgagc cagctgaccc agccttaatt aaggcttcta 60120
tgttccagga tgactgcagc catcagcgtg ccaggctatc agagggaagg ggatcagtgc 60180
agcccggtct ttcctttctg tgctgggcac cgtggagaag cggagcccac acacaaagac 60240
tctgcatgct tgttctcacc tatgtctgcg cagccggcgg tgcagagacg tgcatttccc 60300
tgtactcctc gcattctatg tgtgccttaa ctcttagcaa gcgtaggaga gtagatgggt 60360
gagcatggga gaagacaata tttacaatac tccagagaaa acgcagctta cctttgatgg 60420
gtttttaggc aatttaataa gaccattgtg taaaatttta ctaacagttt aaagaatttc 60480
aggccaaagt aaccaaaaga aaaaagaaga ctcagttatt aaaattaaag gtgaaaatca 60540
gggaaacatt accagaggta atggtcaaat ccaaagaatc attagggctt tgaaaatatg 60600
tattacagca aattagaata tctcacagaa atgggtaagt tcacaggtac atctgacctg 60660
ccagtgttaa actaagaaga tagaaacaac ttacacagac ctaagacaag caataaaata 60720
tctcccaact gaaaaatacc cggtcctaaa tggaatcatg gctgaattct gtcagatatt 60780
tgaagagata acgtaaatcc tccttaaact gttccataaa gtagaaaggg gaagaatatc 60840
aacaaactta ttttccaaag ccagcattac ttttataccc aaaccaggta aaagaagaaa 60900
agaaaagaaa agaaaagaaa agaaaagaaa agaaaagaaa agaaaagaga aaaggaagga 60960
aaggataagc cgattcatac attcccaata aaataataga atgaaatgaa aaatatacaa 61020
acgggaaagg aagagatgaa agagccctat ccacagacaa tattgtccca cacacaagag 61080
cccagggctg cactggagag gcttagctct gattgaggat cagccctgcg tcatcaccac 61140
acagaccccg tggttttcct atatcgatag tgagtgtgtc gagaaagaag tcagcaaacg 61200
cgacggccga gcacagtcac aggcatcccc gatagcttca acatgagcaa ataaaactcc 61260
tggcaataaa ctaaccatgt gaaaagtcct ttacagtgaa catgttaaga cacggaagaa 61320
gaaagagatg tcaatcaatg atggaaaggc ctcccatgtt cctgggagtt aacagagtta 61380
ctaccacaaa aatatggcca agtgtggtct gtagattgaa tgcaatccac aatgccaata 61440
ccgttcctcc tagaaaataa agttcacttg ggacgacaaa aggtctcagg tagccaaagc 61500
tataggatag taagaatatt tcagaggtat cacagctgct gatatcagag gtatcatagc 61560
tgctgattcc aggtcacact acagagccat ggtaacaaaa aaagcatgga agtggggtca 61620
aaacagacgt gtaaatcagc agaagaggag agtcagaaag gaacacaatg tagccacctg 61680
atacccgaga aagatgccaa aatgtgcatt agagaaaaga cgggctggag aagaagtgat 61740
gccggtgggg aaccggctag cacatataag gaagggaacc agatcccagg ctcctacccct 61800
accaaccaat tcaacacgga gcagagatct taatggaaaa ggtaatggtc tgaaacagct 61860
aaaggaaaac atgcagaaaa cgcttcgtga tgtaggcaag ccctttccga aaagggctcc 61920
agcaactcag gaaataaaat ggagaactag caactgagat tcaaaggcgt cacagcagag 61980
gaaacagttt cctgagttgc agagacgaca gccttcagtg ggggaaagaa ttgttgctag 62040
ctatgtagct gacagattag tatctaggtt ctataaaaac tcaaaaaaaa ttaaattctg 62100
gaaacaaaca acccagtaaa taaatgcgct cagaagaaag agagggttct ccaacaatga 62160
gtgcaaacag ccaacaaatg tgaaagcatg cttcacaccc ttagtcttta aagttcctct 62220
gaggtcccac ctcaccctgg tcagaagtca ttaagaaaac aagtccagga gcctgccaag 62280
tagctcaggt ggtacagtac ttacctcttg tgtaagaagt cctgggtttg attcccagca 62340
ctccctacaa acggctgcac tcccagatga gttcaaggtc gtccccagat gtgtgtagca 62400
gatgtcttag caggtaaccc atacaaagcc tgctgccctg aattcagtct ccaggaccca 62460
catggtagaa ggagagaacc cctcccctca ggtggtcctc taccctaaac acacgtgtgt 62520
tatgaccaac aaatgcaaga atgtaaataa gaaaatcaaa atggagattt agaataacat 62580
gaagtaaaac tgcatagaca cagctatcca ctcctgcgga tatttacaaa actctaactc 62640
cacgtactat aacatacata tttccctgca catatgtgtt tatttcagcc atattcaaga 62700
gctacatcaa acaaccaccg tagctctcca ttagcagatg atggattaag aaagcatgat 62760
atacatgagt aatggagttt ttttttcagc cattaaaaaa aaaaaaagaa aaaagaaaga 62820
agggctggag agatggctca gcagttaaga gcactgactg ctcttccaga ggtcctgagt 62880
tcaaatccca gcaaccacat ggtggctcac aaccatctgt catggaactc gataccctct 62940

tctcttgtgt ctgaagacag ctatagtcta ctcacataca taaaataaaa ataaatcttt 63000
atttttaaaa aagaagggaa gaaaaagaaa agaaacattt gcagggcagt gtatgcaact 63060
ggagatctct gtatcaagag aaataagcca gaacaaagaa gatgaacatc acctaccgtc 63120
tctctcggtt ctagatccta ggttttatat agatgtacat aagatatttt gcatatgcgt 63180
gcataggata tgaaagtaag aggaaggtac tccgttggta gggaggggac gagtggatat 63240
gaggagggga gaaggggcct taggtacggt cacagtgctt gttctaggat gaaaatgttt 63300
tcagggagtt atcccgatga gcactgagta tatgccaaga aagactttaa ggaagtgttt 63360
ctagaggaaa agaattggtc tttgaggcca tttgctgtca aatgttcaca ctctgacctg 63420
cctgggcatg agcagactgt tctctgtgac cctagacaga gagaaggaga tcatccgcca 63480
ggcagccgtg cagcagacca aggagatgga cctgagcgtg gtgcgcctca tgttcacagc 63540
cttcctccct gacagcactg gcagcttcac tcggagactg gagcctgtgg tgtcagacgc 63600
catctatgat agcagtgagt actcgttccc aagatgggac agtttcatct agctgcatgg 63660
agggcagggc caccatgtca cttactcccc agagctgagg cctgcagact gggacctagt 63720
cacatagagc ctctagttta ttgtctacaa agttaatact gcagtctagg gtaaagggtc 63780
aatatccagt taagcgtctt aagaacatgt ctgccaaggt aacacgtctt cctcagagag 63840
tgcattatat tccttacatg taacaccagt ttcttactat gaacacatga ctgtaaggat 63900
agattgctgg tgctctaaaa aaaattacct taatttttgc acctggcagc acggaagaaa 63960
aattgataga agacaagtat ccctcttgtc tctctttatc cccagtttga aatacattta 64020
ttaagacagg aacgtcaagg taactaaata aatgaatagt atgtcaaaat catctttcag 64080
gctgcagata atagaatgtg cagggtttcc agtagcttgt agaatttact gtgtggctct 64140
attgttcgat atgcattggg cttaacaaac agttgtttca ggaaccctgc aagcctcact 64200
gaatggtcat agaatacgat gcacaaggaa atcccatgtt ctgcatccag ccacgcatgg 64260
gtctgtctga tttaaacaag tgtgaaggga agtgaaaagt ttattgaatg gaaaagagtc 64320
ttactgaaca gagatgagct acagctgttg gagtttttaa agggggaagc tttgccccac 64380
tcaccactat gggcagcttt gccccaaacc acacagaatg ctaatttagg ttgggggaaa 64440
aaaaaccaga acatcttcac ttttctttga agaatatgtg tgtgtgtgtg tgtgtgtgta 64500
aatgtgtgtg tatatatata tatatatata tatatatata tatatatatt agcttaaaat 64560
gtcattttct taaaattttt aagaatttta caaatgacat aaagttagca tgaaacaagc 64620
cattggtcca gaaacatcag acagctctct ccttcctcat cagcctgtgt gttagagaag 64680
ccatatgcca ccatccctcc ctgacctgct cctttctggg gaaccatgag tgccccagac 64740
ccaagaactg ctcctagtac cttggaagtc tttcttgctc tatttgtgtc ttgttaaagg 64800
cagtaagagt gagcaccatc atggaatctt tgtgaggcta atgctaagcc agggtatgca 64860
ttctcttctc cctttgaaca gaagccccga atgcatccaa cctgaaaatc gtgagaatgg 64920
acagaacagc aggatgtgtg acgggagggg aggagattta ccttctctgt gacaaggttc 64980
agaaaggtac gtacacacct ggtctctaga tgtagggtgt ggctgtcaga gttctctgga 65040
aacagcggac caggggtgtt ctccttttgc tgtgggagtc tcaaaaagac agcgttcttc 65100
aacaccatta cacgcattgc aatcacatgc ttgaatacgc ccgtttgcag atgacatcca 65160
gattcggttt tatgaagagg aagaaaatgg cggagtttgg gaaggatttg gggacttttc 65220
ccccacggat gttcatagac aggtaggtgg gttattattg cgggttttat tttaatgcac 65280
ttctgccttg gtgaaattca gtgctcagtt tccacatatt aacaaagaaa ccgtcataag 65340
tctctttgac atccatacag caatgctatg tttatggctc ttccagtact ctgacataga 65400
ggctgagaag tctaaagggc tgaagctgat gctgtcctga tgagctggtg tctctggctg 65460
attggttaca tgaccagggt ctcccagctg atgacatgag ccaccacagt aatagtcact 65520
ctttgacagt gactgagaaa tggtgtttca aataattggc aacaacccaa atgttagcca 65580
gtaaatgact aattagatag tggtgtatct atgtaatgga acatcataca gttactagaa 65640
aagaaggctc tgtgaatgaa caaagacctc aaaatagaat gcaaagattt gtaacaatcg 65700
atatggaaca cactgtatgt agaaacaatt ctatgtagaa acaattctgt gttcacgtgc 65760
acaggaagcc tgagacaata acaacatcag ctcccctctag gatctcaagt tagtgcatga 65820
gtgggaagcg gggaggcagg gagaactgtc agcatatgct acctattgct catcctatat 65880
ttgatgacat cttgatcctt ataaacatag aaagaccgaa actaagtctg tccagttcct 65940
tattatccca gcactgccca ggactagcat gtgacacctc ctgcgcagcc tcacttagca 66000
tgactgtctg cgggctgtca ggggcagccc tggttctgtc tgcttccaca tccactcctt 66060
ctcttgctgc tggcagtgat cctggatgtt ttcaaggcta atctgaaaga gaaatggctg 66120
agtcgcgtga tccgccatca tgctgttgtg ttttactttc acgggggcag tataagtgtg 66180
ggtataaggt actaactggg aactttctcc aggtgctggg tttgcacaca ctttatttaa 66240
gttcagagta tacatagacg tgtatagaat gtgcctgatg ctcaccgaca ctagaaaaca 66300
aatgtcctac actaatgttt gggtctttg caccctgcag tttgccattg tcttcaaaac 66360
gccaaagtat aaggatgtca acattacaaa gccagcttcc gtgtttgttc agcttcggag 66420
gaaatcagac ctggaaacta gtgaaccgaa accctttctc tactaccctg aaatcaaagg 66480
tacctgggtg gtttagactc ttgtgcttgc tctgaaagac cagtgggccc ctcctctact 66540
cagcctccca gaagctgctg cctggatgcc tggatgcacg ctgcatagtg ttaggggcaa 66600
ggaaggtcat atgccaaggt catggaatta gccccaggaa tttatgttac tatattgctg 66660
ccaaacttat atgagactta gacatttggc tattgttggg tattagaatc tagtatggag 66720
ccagcaagat gggtaaaagc acttgctgtc agggctgacc tgagcctgag tcccaaagct 66780
gtgtgctgaa aggagagacg tacctgccag aagatgtggg tggtcctcca ccaggagggg 66840
gctgggcggg gaagaaaggg aaagtaaaaa aaaaaaaaaa aaaagtaagg attttttcag 66900
ctactcagaa gcccttaaga attaaccaga aggcttagcc actgtccagc ccagaactat 66960
aaaatgagat ggtcactata ctttcctcac aaaagacacc tcaggaaagg tgcactatag 67020

ttaaaatgca gattacgatt ctttttgacg tgctagacat catattctct gggccttgta 67080
gacactaggc agctgctcta ccattgagct gtaaccctgg ttctcaaatg ataagtaccc 67140
aaagggaagc atccacaaac tgtatgaaat agttgaggag acagtcctgg attccttggt 67200
ctaaagaatc agccaaagaa tttctcttca gtgggaagcc acttaccaca ttatgaaggt 67260
gaatggaagt caaggctctg tttatgaaag agagatgttc agaggcagag gtgtggcagc 67320
tggtgtttgt cctgggagaa atgattactg tgaatctgtg tggactgagg aaggttgatt 67380
taggcgaggg aggccaggtc cacgcagtga agcgtttctg tggtagctgg aggtttttgc 67440
tttgactctg atggctaggc ctgcctgctc actgtctgga atgatgtggt cagaatgttg 67500
ctagaattca gtggtgcctc agcagagtta gaaagggttc tagcagccag aagttagcat 67560
ccagcagata cttctatgtt ggtgctgtgg agacctggac cttgggtgaa gagaaaggaa 67620
gagagactcg agagtctcag attgttctag ggtttccaga ctagaagtgt atgtatggag 67680
tggttaagtg gataagagga acaagagaga agagtctgag aatgaaagag aagcttagtg 67740
taaacatatt tcagttgggg tcaagtgcag gaagggggcc agtcaaaggc atgactcaag 67800
tgggtggggg ctcagcactg aagagaatcc cggccccaca gagtttaagt gtagggacgc 67860
acagtgctgc cgtcaacctc tctgtacctc tgtttcgtaa gttttgtctt gacggccatg 67920
gttctgagaa gagatccatg aacttggagg ctacacaagg cctgggatct gataaaatca 67980
gaaatgtctg gctcagagag aatggggctg gagaggatgt caccaggtac agggtcctag 68040
gtccagggcc accaaggccc ccaggtatcg gtcccttgaa ggcaaagcgt gatggtggcc 68100
aggagtttct gggaggccat gtctcttttt gaggtatggc ctccccattc ctgggtcagg 68160
cttatggtga gctctgtggc tggcctactt ccttcctcac cagcttgagg tcttctagat 68220
ccttctgtcc agacaaggca acatgaaatt actgctttac ctgaagcctc tgccctctga 68280
aaacaggaaa aaaagattat ttttctaaga aaaaaaaaaa aaaaaaaaca gtgtatctcc 68340
tgaaatgtgc ctttaaaaga gcgttgtcat tgctgatgca gaatcatgag aaaaagaaaa 68400
gaactgcagg ttccctgtgc aggatgtgag ccttgaaacg ctgagagttt ggttgccagg 68460
caacctctcc tgctagtgtc tgcatctgaa gagtacagta gacaaggccc agcttcctgg 68520
gcactccacg ccagcagagt gggagttttc taatgcctgg attagaggat tggggttccc 68580
tgaagtcctg gttacctcac atcttctagg tcatcatgaa tacaaatggt atacttgaaa 68640
tacaaatgag gctccaccta cacacaggat ggctgaggta cctgttgtgt tccgtgtttc 68700
tacactggaa acatttctc tgtgagatcc acagttcatc cactgactaa atgaatatag 68760
ctattggttt tggtataact ggtgggaaaa ctttataaat atttctccga gccatcagtt 68820
agaaagtaga cagtctcttc acctcagcct cactcctttc tggccgaaag ccgtttctt 68880
cctaatcatc ctggtgtgcc cagggttggt ttcccaggga ctggcaaagt ccaccatgct 68940
ctcctgcttt ccccatcagc tgcagtagac agtccaacat agtccaaaag tccttgtgcc 69000
acaccgtggg gtgatggaaa cacccacaga actagagaag tagggatctt tctgaacata 69060
gaccatgatg aaagccagca ttaatcagtc agacacagga cgattgttca gtattggcct 69120
atttcctgga tagaaagcac catgttgact atttaatgta aacacaaaac caccttcact 69180
tctcagcccc aaagacaaag aaaacaaaca aacaaatatc tgctaggata ctcccagacc 69240
acaaaggaac aggagcacca tgtcagatct cccacacagt aacaggactg agaacatggt 69300
ggctgaagat cacacgggcc atgacagaac tgagggcaca tggctgtcga tcacattgca 69360
gtaacagaat taggggggct gtctgggtta gtaaggaaca cagacagtga aagacaagag 69420
gaacacagaa ctctgtgaag agcaccaaga taagtctcgg agcacctgga cctgggcagg 69480
ccactgtccc agaggtcact cactgtccca gaggccatct ttattttgga gtccgagatc 69540
tcaggagcca aaacatctct gtacaccctc tctgtgtttc taggatcgac attatatctc 69600
ttaccacaat aaccagtaag gactcggtct ccattgcctc atgcctgcac tgtcttcctt 69660
agagtttgac tctagatgtc ctgtatacag tgatagatgt agctcctaaa atgctgtcct 69720
ctatgtcatt ctctgagaca ttcagcaagt ccctcacatt cctatccctg aggctcctca 69780
gcatgggtga ccccagtcct cctttaacct ttattatcaa aagcacccac ccatgttatc 69840
cctctgagaa gccctgtgtc ttcccagaat atcatgttct tcggccatag ttgttagcct 69900
ttatagaggc ttgtcatgct gtaaatctgt tttcacgtga tttcacatga tggtatgatg 69960
cataccacca taatccatcc cactaaatcc ctctttgcct tgaaattgac ggtccttgac 70020
agcagagatg tgtgttatgt ttctgtccat cccagcacct tgctcagtgt atgtacggaa 70080
taaatgtaag ctggttatta tagttgtctt aggtgaacaa ggacaactgt tctgaacgtt 70140
agctgggtgt ggtggtgcac acctctaatc ctagtgttca cgaggcaaag gcagttttga 70200
gtcttgttca aaaccaagct agtctacata gtgagttcca ggcagctaga gttacatggt 70260
gagaccctat ctcaaaataa agaagtaaaa gtgctttaaa aagaaaaaaa tatgtgtatc 70320
tttgggttat ctgaatcttg agaagcaatg agatctctca ccatcttgtt agcactggga 70380
acttaggagg gggcgctcag gagagttggg tgtgtgccgc actgtggtct ctgaggcgtg 70440
ctcttggtct tctcgtccat tggaataaaa accatgcttc ctcctcgggt gcctgttatt 70500
tccctttaat gcatttaaaa atatcatgag aatcaatcgt ctctctgcgg ctgctggtgg 70560
aacccgatca gtctttcccc cctggtttcc cttcctgtaa atacagacaa agaggaagtg 70620
caaaggaaac gccagaagct tatgccgaac ttctcggaca gcttcggcgg cggcagtgga 70680
gcgggagccg gtggtggagg catgttcggt agtggcggtg gcggagggag taccggaagc 70740
cctggcccag gtacgagaaa catctcttct atctaaagcc tttcatgaag aggaggggt 70800
agttttcttt cccaggttgc caaacatgtc tgcccatgga gtgatggtgt cattactaat 70860
tattagattt caattaatgg aaaatttcct acagatgtaa aagaattttt gttctcaaag 70920
aaatgtatgt ccaactcaag gcacagccac tccccaccca cccacccatc aagtttcact 70980
ttctgtggtt tcagttacac gcggtcagct tgagtctaaa aatattacca ttgtgttgac 71040
agagagagac cacacacaca aaactgtctt cacagtctgc tgttgtgact gccctattac 71100

```
tctgtgttct tggtctcctt ctgtcttttt acgttgtgaa ctttacccag gtctgtatgc 71160
atctgcagtt ttagggatac actgggggtc tcggacgttc tctttcagga taatatgtgt 71220
ctagtcccga aaagtgtaag aaaaaagtca actgaagggt ttctggaaga aaacctttct 71280
caaatcattt ttgagtgttt ccctcctccc cctccccttt gcttttggag aagcagttag 71340
tctattattc tgagaaaata ttaaaacaac tatcataccc accgcatggt agatgggtat 71400
aatatgccta cacagcatac tgtgggctgg agacactgta ctgtaaacaa actgagtcac 71460
agaaaatcat gccagggaag cacttcaatg gtgtgtaaac tactgctcct aactgccacg 71520
catacggcac agtcagtatt tatgtgttcc tctcgggtcc ggctatgatc cccgtgcgtg 71580
gatttccagc catctagtta cactcagttt ttaagcatcg ttgcatcatg cttgtggatg 71640
ctgagtaacg ctgcttttgc ctcagatcta gctagcttaa gcatttcaat ggaaatacag 71700
ttcttatagc agcataagtg tgctgtttaa tgactcacac ctgtagcgta agtgacttga 71760
atgctgtttc ctgtctcctg tgtaaaataa ctgtgctgag catggccttt ctttattcca 71820
gataacatgt aaatgcttta aatgtcagag agtgtagtgc taagtgctgg ctgcccctct 71880
aaatttatca ttcatttctc cactaaagta tcattatatt acgtattggg ttcttctcac 71940
caggggtctt catatttaac tatttttttc tgtaggtcta agtcttggtg tgagtactta 72000
ccgttagctt ttcccttcac ctggaagaaa gggacctgct cagccttaag tcctccaagc 72060
tgctagtctg ggcactgcct gtttgttcag ctgtctgttt cccacagtga ttctgctaac 72120
tctcgggcag agccagcctg tgtaggcttg tgcctcccca tgccacagcc tgcctccctg 72180
agtgggtccc catactgtta acaatctccc tgatgttccc tttgtctcat tgctggttca 72240
gggtatggct actcgaacta cggatttcct ccctacggtg ggattacatt ccatcccgga 72300
gtcacgaaat ccaacgcagg ggtcacccat ggtaagtcgg atcggaatac ccagagcact 72360
gctcgggcaa caggatcatc tctaaaagct ttggaaaatt actttccaat aaattaatat 72420
tctcaggtta ttctaggaca gcataaggga cacccctgac acgtatttgc tcatcacatt 72480
gtgtggaagc aggacagagg ctctagacaa aagatgagac ttgactacgc aaacacacaa 72540
ataccacctt cacatctgtc cctttacccc aagtcctatc cttctgaaat cagttttcat 72600
tttgtagggt tggccttttc ccactcttgg gcatcctctg agtagctgtg gctacaggct 72660
gctcgtggtt tatataaagc tgcattcttc tgtaccatgg aaattattct ataaagtctt 72720
tccatatata atgccaattc atgccagatg cgtacatcac tgtaatcttt tttaaagatt 72780
tttaactggc attgtcgttt atcctataat cgggtaacca ttcatcccat aaaatagaac 72840
tattctttcc attacctata acctcatgac ccctgtgcaa ggccagtgct aggcttggca 72900
ccttctggac agaggtgagg ttcagaaact tgactttcag aagacactgt agcaggattg 72960
ggttttgctc caggtttgcc caggagtctt actgtcactc tgtcacataa cgaggttcct 73020
acagcaacca cagtcagtct agtgatggtg ccctagagcc ctcctggcca cacagaatgg 73080
aaatgagctc tgctcggctc cctttccctt gccactcagg attaggaaag aaaataactg 73140
ctttcgagaa ctttccaact ggctatcagc gcacagaact ttatctcatt tttcttcttc 73200
acagataaac aaaatgagat ccatgccttg tttctgtttg gttttgtttg agagagagag 73260
agagagagag aatatcactg tgtaaccttg accttctggg acattgtggg tggagctcac 73320
tacagctggc tacagaatga gttttgggaa ggcaggcttt taaattgttt ctgaatattt 73380
aaagtatttc cgaaacatcc caggtttttt tggtacacac ctttaatccc agcacttttt 73440
gtgagtttaa ggccaaccaa agctacacag caagaccctg tctcaaaaag taaatagaga 73500
aggaaatata aactatgtcc agaacatatt atttgtgtta taaacattta tagttttcca 73560
tttcctgcag ataagccttc ttcctatttt ccttttttct gccctatgtt gagacagtga 73620
gctatagctt aatagatttt ctacgttttt ttccctaggc accataaaca ccaaatttaa 73680
aaatggccct aaagattgtg ccaagagtga tgacgaggag agtctgactc tccctgagaa 73740
ggaaactgaa ggtgaagggc ccagcctgcc catggcctgc accaagacgg aacccatcgc 73800
cttggcatcc accatggaag acaaggagca ggacatggga tttcagggta ggtgagcttg 73860
cccacgaggg cttccaggga cagctggggt ggggtttcag ggtaggtgag caagcccaca 73920
agggcttcca gggacagctg ggtaggggca attctaggta caggaagctg tctccagaag 73980
acaggtttcc ttcctgttaa tctaaagctg atgagtcagc atctcctctg agtgctctgc 74040
accgtgccct gtgccctgca agagaggcgt cctgtggatc cagtcatctt tgcagcctca 74100
gctctgatta taaagtgcag aaacttgcat gtacattgcc tagtctgtgc agttctgggt 74160
atgagaagag gcaactcact ttgtccctgg cccaggtaac aagtctccat gtccttcgcg 74220
cacctccctc ccagcagccg gctcagctgc cacactgaag acgtttgcct gaaaatggga 74280
gttttccttt attctcttgt cctccttgaa gaagtagaat tgaaagggaa cgttccatct 74340
gagcaggcct cattggcata tgagtcatga ttctcgatca gacgccatct gtaaagagat 74400
agtaactatt tacacatagt ggccgtacac atggttacta tcacatattt ttttcccttt 74460
tttctgtttt taaaatactt cttctgagat gcacagtata tgtgtacaga gccatatgga 74520
aacagggtag atttgcccat ttgggttgag gttagtctgc tgtcccttga ccttaagata 74580
ataaaatttc caaaacccct ggggtttctg agactgcctg gcacccaagg gtgtgtttcc 74640
tcactttgac tcagacagga ttccagaaga gactttgttt gtaaagtgat tggctattta 74700
aaattatggc aaaagccaag taccctgaga ttggttgctc cccttcaatc atagacactg 74760
attgttcttc tcaaaatagc agatgtttct agattgttcc tcacagacac tttaaaatca 74820
acctcttaac tgtattatct gtcagtgaga caatgcccga aatgcccgat tgtgcttgtt 74880
ctagtcaaca caatccagag gcagcaaaag gagcattttc agctaaaatg gtgtgccatg 74940
ccgcacacgc ggggcgagca aatgacactc agctgtgctt gtgggcatta ccttgcagat 75000
aacctctttc tcgagaaggc tctgcagctc gccaggcgac acgccaacgc ccttttcgac 75060
tacgcagtga cgggggatgt gaagatgttg ctggccgtgc aacgccatct caccgccgtg 75120
caggatgaga atggggacag gtgagttgag tggggactgt tgcacagcaa gctctgtttc 75180
```

aggacgggga aaggcaagga tactgccccc acaaactgcc tttgaaaggg agctgccctc 75240
atctgacaag acctcgagta tgctgtctcc ctaggcaaga aggctcagat gtgtgtaatc 75300
atgctaattt tacacccctg ctttgtgtac taaaatagta attgagaaaa ttattgacag 75360
ccctgcaaag tccctgaagt gttattgtca tagtgtaccc aagaaagcca ggtaagaaca 75420
acattagtag agtaactaag aggaaatgcc attctctcct agctactagc tgcttctctg 75480
tggcctctgt taagggtcac gaggatgtga gtgggtcagg ctctgcctca gctccaggcc 75540
aggctgtgtg ggagtctgac attccagatg ccatgcctgc tgtctcttcc tgaggttctg 75600
tgcacatagc tacctggctc cttcctaaat ggttctctta gtccttccat tgagacttct 75660
gaaatgatct aaaagcctat aaatatagcc agagacagca gtcttgagat tcaagtgctt 75720
tatctaagtg atcctaaaat agcgttaaca ctgaaaagaa gccctttgct gtggaagcat 75780
tctggagtgc tgtgtgctga gtctcagttt aaaaacaaag cgctttagga gaatgccaac 75840
ctcaggggtt tcgtacaagg acctttgtcc tcgtcacttg cagtgtaact aaggatgtgg 75900
tataaccgct ggtcttatcc tacacagtac aagaggaacc cagacgacct tctgtgaaaa 75960
aagctctgaa taacagaagg cgcctttaga aggaaatcat ccagttagca ttcaatagca 76020
aataaccaca aaacagagca gataaccaga gcaggacacc ggtgctccaa gggtcatctt 76080
taaggactgg cgaaattcat gagtagtgag ggcgagtgga ttaagcaatt aggaagtgag 76140
atgctgctgt ctgaaggaga ggctgtggaa agggatgcca ggcacatgca gtccagcaag 76200
tgtgtgcaga ggaatgcagg aacatgtgtg taaggacctg ggtgttcatg gggcagctga 76260
gacactggcc ttcattccca agtgggtaca atacccagag cctcgtcaag ccaagactta 76320
aacaagcagg ctccgtgtaa ggttaggggt atgagacctg ggagacacag aggacccact 76380
caggagtata cataagggac tattctggtt tctcttcttt attcatcttg tcatatttgg 76440
cttataatct ggaccctgtc accattagtc atcatagaaa tacaataaag agaaactata 76500
gaaccctctg gtatcgggga acttccccca ggggtatgaa gatctgaagg ggaggggacg 76560
tgcttctgtg aagtgctgag ctgcacatgg aaatgttgaa gtagcttttg aagccctttt 76620
actcgacgtg tgtctgtccg catcacctct cacaagcctc ctgtcagggt cccatcagga 76680
gcaccacagg agcctgaggt gtggctgcgg tgcttctcgt caaagcgtgc gtgcttcact 76740
tgcagttaac acaggcctgg ggtcatctaa agaggccacc atgatcttcc ctgagaagta 76800
ttgctaagtg aaatatctca gggataaata acaagacctt gtttagaaat agaagtcact 76860
tctcattgtt acggaggaag agagctcggc agctgagttt tccccaagctg ctcctaagcc 76920
taggcattac ctttcttcat ggtcttgtat gtcagggaga gaccagggag agctgccagg 76980
gacagaggca tggagacaga gctggcttgg agcttaatgc catttggttt ccgacccggc 77040
aggtgccttt gcacttcatg tgtctggtca gatgttaaca ctccgggggc aggtgggcac 77100
gatcagtgtg tagtagcctg cgataggcag tggagaggaa gttctccaga gaaataatgt 77160
cacctctgat cagtacattc agtacatggt agggccaagg aaagctacac atagtagcta 77220
gtcttcagct ccagtcattt cttgacatgt ttttgtttct gtcgcttgct acatccccag 77280
ttaattattg ccactgtgtg tcattccagt gtcttacact tagccatcat ccacctccac 77340
gctcagcttg tgagggatct gctggaagtc acatctggtt tgatctctga tgacatcatc 77400
aacatgagaa atgacctgta tcaggtaagc agccaccaca aggccgtcga gaagagaaaa 77460
ggttgcctta gtaacacgtg cgtgagctgt tctcagtgat gggaaacagg aaaacagcat 77520
cttctcagaa gtggcatctt aaaagcatcc ttagttggga atgtcatttt acagagagat 77580
gggttgggat catgggtttg atggcaatca cactccatgg aaattcacca tcttctagaa 77640
aaggcagact ttaagaccta gaagtcagag ttgataaagt tttgagggca aacctcagag 77700
gggaaaaagt cacagctgtc aggagagaca tgttaaaaag aaatccaagt ttcaaaagcc 77760
agagttacag agacaaagtt tggagctgag atgaaaggat ggaccatcca gagactgtcc 77820
cagccgggga tccatcccat aatcagccac aaaacgcaga cactattgca tatgcctgca 77880
agattttgcg gaaaggaccc tgatatagct gtttcttgtg aggctatgct ggggcctagc 77940
aaacacacaa gtggatgctc acattcagct attggatgga tcacagggct ccaaatggag 78000
gagctagaga aaacacccaa ggagctgaag gggtctgcaa ccctataggt ggaacaacaa 78060
tatgaactaa ccaatacccc cagagctcat gtctctagct gcatatgtag cagaaaatgg 78120
catagtcgac catcactgga aagagaggcc ccttggtctt acaaacttta tatgccccag 78180
tacaggggaa cgccagggcc aagaagcagg agtgggtagg taggggaaca gggcgggagg 78240
agggtatagg ggactttcgg gatagcattt gaaatgcaaa tgaagaaaat acctaataaa 78300
aaattggaaa aaaagaatat tttcaaatta aaaaaaaaaa aagaattttg aggtctgcgg 78360
aaggaagggt attcgcacag caactggatc aagtgtatgt caaagaaagg ctctgaggtc 78420
agctcctgga gaggtgaaag gctgtaagaa caggaagagg cacctcagag tcccatccag 78480
ctcaggtgtg agaaggtaag ggagccccga aaactccaga ggaaaccacc tggagatggc 78540
acagtgagct gaggacagag gcagatggta ggggaaagca aaccagaaac tccactggca 78600
gacacagtct gggagctgtg gcctggccca ggatggtggc ctgctctacg gctccttcct 78660
ctctcacctc tatttctctc caacccttttt gtgcccctac ccctctctct gggcctgttg 78720
gtcttatcct cttgttggct tctaagttcc tctgcactct cttgcccaga gtgtcattct 78780
gatccccaca gccaggccca tgctgagacc tctgcttcca ggtcatattg ctttcattga 78840
aaaactgtgg ccagcaaccg tgaataattt gttatttaga agagatgaga tgagaactag 78900
tggaattctt acttgtttgt ttgtttgttt gtgatgtttt ttgttgttgt tgttggtttt 78960
tttggtttgg tttggtgctt gggactgtac ccaaagctga gcacaaaccc ttccagcaat 79020
ctacaaccat gttactccat ttttaaaaca agttattcaa gttgttatct cttcttatcc 79080
atgcttctct ctgtctccct ctgtgtctct ctctgtctct ctcttgtctg tctgtctgtc 79140
tctgtgtgtg tgtgtatccc tctctatcaa tctctctctg tctctctgtc tctctctttc 79200
tctccctgtg tctgtgtatc tctctctatc aatctctctc tgtctctctg tctctctctt 79260

tctctccctg tgtctgtgta tctctctcta tcaatctctc tctgtcattc tgtctccgtc 79320
tctccccccc ccccaatgtg tgtgtatctc tctctgttga tctctctcta gcgtacccag 79380
gctggcactg agccatcagt gtcctacctc agcctcccaa atcataggct tgacaagcat 79440
gtgcctggca tgttatctca tttttaattt aaaaaaaaat cattatttta gaagaaatta 79500
aaaagaaaca aagagatgac tcagtggacg aagtatctgc tgtacaagtg tgaagaccat 79560
gttcagatcc ccagaccatc gccagccaga tttagtacca caccccccca caccccgccc 79620
ccgccccccc ctccccagtc agggcaggag acaagcatct tgcatatgcg gggggggggg 79680
ggggacaaaa gaccctgcct tataaccttg cacaaggcag ggggcagtac acacgttgcc 79740
ctctgacctc atgcacgtcc ctgcacccac acgctcagag aaggaaagaa gggaaggaag 79800
aaaggaggga gggagggagg gagggaggga ggaaaggaag gaaactaact gcacatacac 79860
actaaaacaa aagagctaaa aaatttttat tttaaaggaa atcattaaat tcttattttt 79920
taatacaata ttttaaagtt actttgaaaa tatgaattgg aggctggaag tggctcacat 79980
gcttaaagcc ttgagttcgt tcgctcccca caccaaagaa aaggctgaag ctgttagttc 80040
gttggcctgt agctctcaga cttttccgtt ttcagaggag cacagctgct ttctgcacag 80100
cactctgcac ttctctgtct gcagtgattc ctatccaggg gagatgacta ggctaaggtg 80160
ttattttaga tgtgttcttc aagatctgtc ttcctcgggc ttccatgcca tcttgggata 80220
ctattggtat ttggaaagtc attgggcccc gaaagttgaa ttctccaaac ttttaggcag 80280
atggcacttt ttaaaaactt tgtattgatt ctctgtgagt tttgcatcct tttttttttt 80340
tttttttttt tttttttgg tttttcgaga cagggtttct ctgtgtagcc ctggctgtcc 80400
tggcactcac tttgtagact aggcatcatg caccccagtc ccactcatct ccctgtcccc 80460
tcatatctgc cctctgccct tgcagcctcc ctcccacaaa ataaacactc aagtaacagg 80520
aaagacaaaa acaaaaaaag catagagaac atctcgatgt gggagcgata gtgtgtcacc 80580
gggcatccca cagtgtatcc ctctgtccac acatcttcac ttgcaatgag tgtttggttc 80640
tctgtgacac ggtaattctg agttccccaa agtataagct tttatgttat ttcaagatta 80700
tgcttatact gaatttacaa taataacaaa catgtggcaa aagtgagagc ctgagaacta 80760
acgacataaa aggccgtgtc acatgccgga gaatgcaaga agagaacgca gtttaaagac 80820
tcacacttgg gctagtctag agagtgggtg ggttccaaca gggatctgat agtgacagat 80880
ggtcacagaa atgtactatg gttagtggaa gatgtttggg gagaatgttt gagataattt 80940
acaaataatc caaattactt agagaactgt atacttttct gccaagaata gttattatgt 81000
catgcatgat ggtgcatatc tataattcca gcccagagga gtctgaggca ggaggattgt 81060
tgtgagtttt gggccattct ggcctataca gtgggttcca attttgtctg tattatatta 81120
tagggtcttg tttaaacaac aagaacaaca acaacacaaa accaaacaag caaagaaaaa 81180
aaaccctcca aattaaaaag aaactagttc ccaagctggg tgtggggcac acatttgtag 81240
ttctaacacc caagaagaca caggaggact gacagcttca tgtcaatctg ggctacacag 81300
caagttctgg gccagcctca aacacaggaa gaccctctct caaacaaaac aaagcaaaac 81360
aactccacaa agccaaacaa agaaacccaa aagttatgag aatcacaaac gctgcctgtc 81420
tatgtccttg aggctaagaa gctggtatgg cagggaatgt ttgtgcccat taactgagtg 81480
aagccaaggt gggaaaaact ggggagaaag gtctgagggc tccaaatcag agcagctttg 81540
ccttgagctc aggggagagg ttccatccac aaagagctgg gtcgcacaca atagagcctg 81600
cttccgcaga tcctatcacc aacagccagc agggcttcct gaatccaacc tccagcagcc 81660
gagggaagca ggttttgcaa agaggattgg gaagtagcaa cttcctcttc ttcagtttat 81720
tcttatactt gtgatggtta aaaatgcaat ctgccacttg tctgctatga ggtagcctgg 81780
gtgcaggggt gatgccttcc tccacctcgc ctctcactac ctgtacaggc agtcaggaga 81840
gctggcctgg ggtcatgcaa gggggtgagc tagccctgtc cttcacaggc tttcgagagc 81900
aggccttgca tctagcctgt gctgcagagt agagaggacc ctcgtggagg gagcacagtg 81960
agcagccctg agggtgtgag agcaggagaa ctggccctgc ccctcactca caggctgtag 82020
tgtatgggag agtgggtcct gccctttggg cagcacagtg gagccagctc tggaggcata 82080
gggagatccg acagctccct atttccagat gtgactgttg catgtataca ggaagagtgc 82140
tcttccagtg ttggctacag acagctgctc tgggaagaca tgtattcttt agtgtgctca 82200
catgcactcc acacggtttt atacatacat ttcctatccc acatcagcct ttccctttaa 82260
gtttcaattt aaaatacttt ctcaattcat aatttttgca aaattcaact gagaggacct 82320
gtatatttcc tgagattgaa ccagacgctt acctcctaag ttccccccagc agttacatcc 82380
tacttaacac aataatacaa ttaggaattg caacagattc tagtcagatc tcaatatttg 82440
tatgtactca tatgcatgga tagctctgta agattttttt ttcctaagct gaacatggtg 82500
gtattgtgtt ttagttcctg ttattcaggt agctaaggca ggaagagtcc ttgaatatag 82560
atgttccgat gtagcctggg cagaaagcaa gactgtttct aagcagagtg aatgaatgaa 82620
tgaatgaatg gtcaatccat gtgcttgtgg atgtgtatgt agcattgtac tgtgtccctg 82680
tcttgggtct tcacgctgtt tatcctttag tttcatagac agctggtttg ttctcattgc 82740
tcctgaagag aatgctgtca tgtcccctaa ctagctgctc actgtccaca ggctgtgcac 82800
tgcagtgagc acctgcactg tacccatggg tgccacaagt gggtttcaag ggcagtgtga 82860
gccctgagct ccacagatgg attctaggga ttccttgagg cacgcatttt atgcgtgtgt 82920
aaaatgtaac ccttgggtgt tggtggccca ccagccactg cttcccagca ggaagaactc 82980
cctgaatggg aggtcgggca agtcctcact acacagcctg ggcttcagtg taaagcgcct 83040
acaacacaga ctggctggca ttgagagttg gtgctactct ctaccagtga tctcagcttt 83100
ttgacagaca cttttgtgat tgggttagcc ccacttgttt ctgaaaacag ttttccatgc 83160
gtcctactta caaccatgga agcacagctt tgaggtcagg gtagtcagaa agacctccca 83220
gagcctatta tgcacagtgc ctgggattgt tttctgcctg aactttctta gggagagtgc 83280
tgtattggct aactatgccc tgaggatctg gtagcacctg tgatagtatt acagtggagc 83340

ttgtatctgt gcgtctcttg aagccagcct gcagcagtgt gttctggttt agcctatgta 83400
cttccagacc attgtggaag gatgacatct ttcttgattg ctttctttta atagtctatt 83460
tatgttttgt tgttaaaaac acagcattgg ttcgatttgc aaatgtatag aattaccagc 83520
tatagataga tacctatcag aaattcagag aaattaaatt ttcttctaca agagttaaaa 83580
aaaatcatgt agcaaacgta tcccataacc ccgactgttg aatgctggtt ccctccgtgg 83640
gcctttgagc ctggcatgca gaacaggaag ctccttgagt gacaaatgtc cctacagtat 83700
gactccataa cgtagcctga agccaggggc tacagacatc cctaagcaat ggctctccct 83760
ctccttctct gtgtagacac ctctgcactt ggccgtgatc accaagcagg aagatgtagt 83820
agaggatttg ctgagggttg gggctgacct gagccttctg gaccgctggg gcaactctgt 83880
cctgcaccta gctgccaaag aaggacacga cagaatcctc agcatcctgc tcaagagcag 83940
aaaagcagcg ccccttatcg accaccccaa tggggaaggt aagagcagtc gccttgctgg 84000
ctaaccgtcc cctctcggct gcagcataag ggcttggcat ttgacaaatg cgtctgacat 84060
gactttcacc atgaatactc tcctcggcca gctggagttt gtcatatctt gtggttattt 84120
ctgctgttta ttacagcaaa cgcccttaaa gagtatttgt gagtattgct tagtaagtgg 84180
aaaagcaagc ccgagcaaac attagtcttt ataaagcatc gtgtgtgtgt gtgtgtgtgt 84240
gtgtgtgtgt gtgtgtgtgt gtggagaacg tttacatagt agctcagtgt ctcccgagaa 84300
cacagtggga gcttgtggct tctgaaagta gaacagtgga ccgtctgagg ctttgaagca 84360
cgccctgaac agaaaccgga agtgctgcga cctggtggtg tccctcctcc ccgcagccca 84420
gctctgtcag tctcacccac atctctgcct ttgttcggcc ttaggtctaa atgccatcca 84480
catagctgtg atgagcaata gcctgccatg tctgctgctg ctggtggctg ccggggcaga 84540
agtcaatgct caggagcaga agtctgggcg cacagcgctg cacctggccg tggagtacga 84600
caacatctcc ttggctggct gcctgcttct ggaggtaaag gcgtacttgt gatcttgacc 84660
taaatccccc tggaagtttt agggaagtct tttcaaagaa tgactctagg gccagctcgc 84720
tgtcagtatt ccactgtctg ctttctgcat ccccagctcc agcccagctc tcaacctggg 84780
gatgccagtg acagagccag cccagtatag cagttgggtg aatgatgtaa cctgaggtgc 84840
aacggagatg tggaggtaga cagtggagga gagagcccct gctaactctt tctttgtggg 84900
cggatttagg gtgatgccca cgtggacagt accacctatg atgggactac acctctgcat 84960
atagcggccg gaagagggtc caccagactg gcagctcttc tcaaagcagc aggtaagacg 85020
ctgtggtcac tgataccagc aaagcaaact gcctgcaagt tcaaagctgc ctgcggaagc 85080
tgcggttctg ccctgggaaa gtgccggtaa aggagacctg caccccggcc actctgcagc 85140
ggctgcactg gaggctctgc ttggcctcgg gtttaccttg atgctcacgc tagttcccac 85200
tctctggtta tctacgttgc ctttgggttg ttgaggttta ctcggtacct ataaaatctt 85260
gagctgtgtc agcttctggt catattacaa gtctcccaga aggaaaatat ctaaagactt 85320
ccttttgcaa aaaaaaaaaa aaaaaaagtc tcttagaagc tgaaagcatg ggaaaagcaa 85380
atgagccctg tatttgtgtt actgtgcacg aggcactaag acttgctggc ctccactgcc 85440
tttcctcggc tccaggcacc atgctggcat cccatattca cagtctcctt taccacatct 85500
actcctcagt aactctctga gataagaact gttgctatta atcccatctg agcaccaaga 85560
aggcagacta caaagatgat caagccccac tcccagtgtc ccggaaagct gagtcacagc 85620
accgcagcct gactgcagac tcttattctt cacgctgtct ttctttctgc tctgcctggg 85680
gttctagtcc ttcctgtaaa gcactcagaa acattccagc tcatctcctg tctccggcgc 85740
tttgtgtgtt cagtgttaac acatgcttga tgaggttggc tggccttccg ggcctgccca 85800
gacacacctg atttacttca gaggttgata agggaggatt tgtgaacgac acattcctta 85860
tagattcagg caaagaggcc ttggttgtaa tgtgttgagt cattttggaa gtcactgatt 85920
atactggggg aaaaaaaaaa ggttgtctct tagtcagaca cctgaaatct caacattcag 85980
aaggctgagg caggagaatg gccacaagct taggctacct tggtccacat agttctaggc 86040
agaacaaaga tacatagtga gatactgtct cgacagtaaa tatataaatg taaacacttt 86100
tacttgaatt caccatgaaa aggtagcaaa taatatcttg tgtgacataa agaaaaccag 86160
acaaaaagcc tagccctgac ctcctcggag tcacaaaggt cacttcaggg acatgtgtag 86220
ccctcgggtc ttcaaactca tttcccagtc atgttcctgg ccatgttttg ctcacgttat 86280
aaaagagcaa gtgaggcagc agagagtggc aaggcagcag tgttggccca agtcagaggt 86340
tctgtcggca gaactgaagc aaacacttca ttccaaaagg ctgtgctgag cctgcctgcc 86400
tgcctgccac tgttggccaa cccggagccg gacacccata ggtagaatag ccaggaaggc 86460
atgacaggta actccactaa cattagggtt acgttttatg ttgtcttgct gggagctgaa 86520
gaacaggcaa tatccccaag atttgttagc acattttttc cccataacaa cacatcctgc 86580
ctagcatagc ggccttgtgg tgtccatttc tctgttgctt aggaactaga actagggccc 86640
caacagtaca gcactcaagg acagagctga ccaagcaaat ctgctccaca caagtaagcg 86700
agagtgtgag aagtgtgtat tggtagatgt aaatgtgaag gttcaggact ttacccctgt 86760
gggtgcataa attgccacct actagtcctt aggttaaaat gtatcctttg tagtataata 86820
ataataagaa taataatcct gcagagttac aaatcccagg agtacttgaa gtctttcatg 86880
tatttacttt acatcctgat cacgtcctcc cctcctccca gtgccctcca catggctcct 86940
cctccccacc ccctccctct ctcttcacag ggtccttggc tacctacttc catagaagtg 87000
aattaaaatt ttttaaaaag ttgttttaaa tttcactaga aatgaaaagt tatcctgtaa 87060
ttgccttaat gagctagagg taacctgtcc ctcgacatgg gaatcctgtg tgtgattgga 87120
ggcggacaag ctttttcttt tacacaacac tgtatttccc tgactccata ggagcagacc 87180
ccctggtgga gaactttgag cctctctatg acctggacga ctcttgggag aaggctggag 87240
aagatgaggg agtggtgcca ggtaccccac ccctggacat ggctgccaac tggcaggtga 87300
gctctggcca gcccatctca tgggtattta cgggggagcc agtggctcag gaatataaaa 87360
cagatggtct tcttggcatg ctcctcccta aagaacatgc ctttgttttt atgaggtctt 87420

gttaccgaga gggtcttaat cttgaaagat ggtcttcatc catgaaagat gaaggtccgg 87480
tgagccagag tgtaaagctg tttgcgtaac tggattagag ttcagatatg agaggttaga 87540
aaagcacaaa atgcacagaa gagttaccct gtcagcacac gaccagagtt cttgccattt 87600
ctgtcagaag ctagcttgct cagagcttaa gtaagaggtt tctgaacata aaaataaact 87660
agaaattctc ttgtggattt ccataaacat attaaaaacc ttccttaaaa cacaataggg 87720
cttacactta aaggagaaaa cgctaggtgt ttggaccaag agaaaagagc cagaaggcaa 87780
agtaggcagt gtacgggtcc aacacagtcg ggtttgtagg aatctagact ctctgtgcac 87840
gtctaggagg ctgtttgctg gctgggttcc tacagggaac acagaagact gacccctgac 87900
ctgattccta ccctgtgtac catcagcaaa ttcacctcag agaacaccag gacttttaaa 87960
aatcatttct gtgaatattt tctcatgttt ttctatccga caggtatttg acatactaaa 88020
tgggaaaccg tatgagcctg tgttcacatc tgatgatata ctaccacaag gtgaggtgtg 88080
gcaggacaga ttctctcagc cagtgttgcc ccatgctgtg gtttacagga ggagaggaga 88140
ggccagcctg ggcgagggtg tactgcaaac cgtctagatc ttatctcaga atctgagatc 88200
cctggaggtg tggccctgag ctgctagaag cacctactgc tgctgctgag cagggcctct 88260
ggacttggca gaagcttggt ggcctccatc agaagttgga caatgacctg tcctctcaga 88320
ttgattgtcc ctggaaggtt aaagcagtca tgtttcttcc ttaggggaca tgaagcagct 88380
gacagaagac acgaggctac aactctgcaa actgctggaa attcctgatc cagacaaaaa 88440
ctgggccact ctggcacaga agttgggtct ggggatattg aacaatgcct tccggctgag 88500
tcctgctcct tctaaaactc tcatggacaa ctatgaggta acgcgccgcc gtaccattta 88560
gagttgtctg tgtaactatt ggtagttgac cgtgtcagct ccactgagca gcacagccta 88620
ccccccttaca tagtaacttt atggaaggaa accagtcatt gcctggaagg caaagggagc 88680
caggccagga tggtgccttt ttcttgagaa gactggttac ccgtctctcg gcattccctg 88740
ttttcaaatt ctcagagcct tcctgtgaaa atactactgc ccacaggatg ggacccctc 88800
caagctgtga tcggtgtgtg catccctgtt tgcaagaggg agccccccag ggccatggct 88860
tcattgttct gtcgggtgac tcctattaga gcaattccaa actaaaaaat agacaaacat 88920
gggcgagcta ctccgaatga ggctgtcatt gctagctgct gtcttccctg gatttctgtg 88980
cgtagacatc accgtaggca gtctcctgtc tcctgtctcc atgaactcac aggcttcttt 89040
cccttccttc ccctcttttt tcttatctct ctccccnnnn nnnnnnnnnn nnnnnnnnnn 89100
nnnnnnnnnn nnnnnnnnnn nnnnnnnnnn nnnnnnnnnn nnnnnnnnnn nnnnnnnnnn 89160
nnnnnnnnnn nnnnnnnnnn nnnnnnnnnn nnnnnnnnnn nnnnnnnnnn nnnnnnnnnn 89220
nnnnnnnnnn nnnnnnnnnn nnnnnnnnnn nnnnnnnnnn nnnnnnnnnn nnnnnnnnnn 89280
nnnnnnnnnn nnnnnnnnnn nnnnnnnnnn nnnnnnnnnn nnnnnnnnnn nnnnnnnnnn 89340
nnnnnnnnnn nnnnnnnnnn nnnnnnnnnn nnnnnnnnnn nnnnnnnnnn nnnnnnnnnn 89400
nnnnnnnnnn nnnnnctaca cagaggccat tgaagtgatc caggcagcct tccgcacccc 89460
ggcaaccaca gcctccagcc ccgtgaccac tgctcaggtc cactgtctgc ctctctcgtc 89520
ttcctccacg aggcagcaca taggtaaaga ggctgagaga gttcctttcc tcgctggcaa 89580
ggtctttgtg ctctgggacg tggcactaca ctccctttcc ctagtctttt tcctgacatg 89640
tgttcctctg tggcatgcca cccgctccct ccccacagtg ctcctctgcg cacacaggaa 89700
caggcagttc ccacagacac tcacattggc atataggtca cttagaatcc cagtagacac 89760
tggacatggc ggggagggtt aaagaaacac tacttctcat atttgttaaa tttggagaaa 89820
gattctaaga aaggtgcaca gccctgggcc acaggacaag aagtaggacc caggaaatat 89880
gcatactgta tgacccttcc tttcatgcca ccacccttca ggtcccccac ctcctccctc 89940
cctcggcact ccccgtcctt cagctcccac cctgctcatg gtcctgcctt tgtcttccca 90000
cactaacatc cacctgtcac ccccaacaca tgcatttgta cgaatggatc tcggtacagg 90060
cttgaattat gtcattgttc ccggcccgtg ctttctctaa cctctgcacc ctttcttctt 90120
gttcattttc taccctagcc acaaactcca ctgagaccag gaacaactag cattctccca 90180
tctttgtatt atgccagcac cccggtggca ggccttccat ctgggctcag taggttatgt 90240
ctgccttcca cctggggcag tcctcccctg ggagcctgcc tgtcaccttc agcaggtcct 90300
ggacacctga ttcttgcctc tttgtcctgt actgtccttc cttccccacc tgctgtggag 90360
aacctcttgc taggatgtaa cttctcatct ttaaacactg gcccgcctgg ccacaggctg 90420
cttagccccg ctttgattaa tgttacatct caatgaatgg ctttgtccat tgatagcgct 90480
ttggttagct catgttttca cgtttgtatg atacatgctg ttatttttgt tcgcatttct 90540
catatggggg aatctacagg taaacacagc ctccaagttc agtagaaaaa tgtcatcatt 90600
aagagtatta tatccaataa acagtaaaca gattttttaa aagagaaaga aatttagaga 90660
aaaacgagac atcacttcct tgttacaccc tttagttgag ttcaaacttc tcagaagaat 90720
atgataactg ctggtcagct caccacctct gtctgccatc tgcttctgtc ccctcctctc 90780
ctannnnnnn nnnnnnnnnn nnntcccatt ttcacatgtg ttccctaccc tttccatccc 90840
tttaaagcct gttttccgcc ccctggaggc ctttgctagt tttctggcct ctgctcacgc 90900
tcacttccac atgaatatac atacgtgact gttggaagct aggatccaca cgtgagagag 90960
aacgaatggt atttatgtct ctgggctgcc ttgattagta ttctccatta acctgcaagt 91020
ttcatctctc tttacggccg aataaacttt tatcatcttc acacaactct tggctcttgc 91080
acaaactcat actggccatg actgtcaaaa gtatagccag gctgtaacaa aatctaagag 91140
tggcaatgaa tactttctgg cattcaggtc ctcatgctta accctgaaag ggcatctctt 91200
cttggcagat gccaagttcc gcccatcctc actgcttgcc tgaactccaa cagtggctcc 91260
tagtcagcct ctttgactct ttgcttgccc ctccatcagt tctctgcacc acagtgatgt 91320
gacatgatag ggctccagtc acaattaaaa actaaatact tagcagctag gaacacggca 91380
agtgtcccct cctctacctt tgggaaagcc aggtagggtg acaaatatgt acaacccgat 91440
agcccactca tagaggaaag gacaccgaga gaggtggatc cacgggactc actggccagt 91500

ctctaaaaac aagatggaga aggaacaagg aagtcacaca atgttgacct ccccacaccc 91560
cacacctgca catgagcaca catcccacaa acacaaagca aatacttaaa tactgtatta 91620
aatttccatc attgtgacca acttaggaga gatttggttg ggcacacagt ttttataggt 91680
cccatggtcc actgccttcg gggtatagca aaacccttca tcacggagga agcacatggt 91740
agaggaatcc tactcacttc aaggtgtttg gaagtacaca gagatgggga ggagccccag 91800
tacagatctc tttcaaagcc acgccctcca tgacctaact tccttctgtg aggccccata 91860
gtcaaagagt tgtatcacct ccagataatg tcacaggcta gaaaccaagc ctttacttag 91920
cacacagcct ttgtgggaca ttttaagatc cgaaccataa tacacagtaa gcctttcaaa 91980
tgctgcccag tccctgttac acaacagaca gttgactgga tgaggtaaca ccaggacacc 92040
gctctggcat ggtcattgag tctcagtgtt ctgacgttaa gattcaatga tgagagtcaa 92100
acatttgctt agtaagtgtt atgcttctgt ttctttagtc actctgtaca gaaataatct 92160
gctctggttc ccaggaatgc ttttcaggtg atagaattta acttaagctt ttggtataga 92220
cacatttaaa aaaaaaaaaa aaaagcctca ttttcttgca ccaaaagaaa attttctagg 92280
caatgtgtat tctttaaaag ctcatacagc tcagagaaag gtgccatttt tagttcctgc 92340
ttctgttatg agtttatttt gccttgtatt cagcagtttg cccagccgtt aaaaaaaaaa 92400
aaaaaaaaaa aaaagacaaa aggtaaaatg atagagcagt tagaaaaata tgcctcactt 92460
tacaaaggaa aacatgttta tcctgaacca agagaattca gaacagattc tgttcaacag 92520
aaagactgat gtaccctgta gcactctcta atgtcatttg ataaccctgc cattcacaca 92580
gccaagcagc accggtggca tgatggggag gtgggatgcc gtgctgcgca ggagctagca 92640
agcacaaccc tccataccat agtccacacg gagcctggga gggagcccac tgctgtggat 92700
ggtgctctag ggcactgtga caaagctctc tgggtttatg tcagaaggga ttcagggcag 92760
gaacccaaag actttgaatc tgaaactgct gagcaccccg gcttgctgcc tcatgggctc 92820
atgctccgct agctttcttt tcctttttttt gttttgtttt tttttttttt tttttcgaga 92880
cagggtttct ctacatagcc ctggctgtcc tggagctcac tttgtagacc aggctggcct 92940
cgaactcaga aatccgcctg cctctgcctc ccgagtgctg ggattaaagg cgtgcgccac 93000
catgcccgtc ttccgctagc tttcttacac agccctggac tgcctctcag ggagtggtgc 93060
tgtctacaga ggataggccc tcctcccgat cagttaactg tcaggacacg cccacagacc 93120
agtctcatct ggctgggccc tccctcatct gcacctccct cctcaagtga ttctagacgt 93180
gtgaagttga cagttgaagg tgacagggac agcaggcaga acaaagccct tgccatggcc 93240
taactcagcc cctcatgagt ctgtgctttc tccgcagatg aactccggga tagtgacagc 93300
gtctgtgaca gtggtgtgga gacatccttc cgcaaactca gctttacaga gtctcttact 93360
ggagacagcc cactgctatc tctgaacaaa atgccccacg gttatgggca ggaaggacct 93420
attgaaggca aaatttagcc tgctggccgt tccccccacac tgtgtaaacc aaagccctga 93480
cagtccattg catcgtccca aaggaggaag gcaaagcgaa tccaaaggtg ctggagaatc 93540
gccggcctgc agggtcactc gatttcattc aaggccttcc gaatttggcg tcctttcttg 93600
gttctgaaat gaaatgtagt tgccacgcac agacggtgtc tagcaatcat ggcgctcgct 93660
cgctcagctg cactctatgg ctcaggtgca gtgtcttgag ctttctctgc tgctactgga 93720
tcacatttgc tttgtgttgt tactgctgtc cctccgctgg gttcctgctg tcattaaaag 93780
gtgtcgctgt ccccacccgg tgtcctttct agccatctac tgtaagttgt gcattcaaat 93840
taagattaag gaaaaacata tttttaaatg agtaccttga tgcgcaataa aaaaaagaca 93900
tttctttttt taatgtggtt tatctgtgat ttaaaaataa aaaacacatg aacttacaat 93960
atttaaaaca tgctacaatc agtgctgaaa atagtatttt ccccgtttta tgcattttac 94020
tattgtaaat atgttttcta aatcaaatac tttaaaagaa gaaatgttga atttataaat 94080
gctatttact tttttatttt acttttataa taaaagtaca agcacattgt tgacctaatc 94140
ttgtctgatc tctgcaaatg ctttctcaga ggagtccctg tgactgtggg aacagcccca 94200
cagcccgttt gtcacatcca cattctcctt tagacacatt tctttgaagc tagaaatgtt 94260
caaactgtgg aaataaaagt gttctaaccc aagactgagg ttctgaccca ggcaacatca 94320
tctggacgcc tagcatcaac attccaatct ctacaccgtt cctctccagc gcatgctgtc 94380
tgtgtctatg aaggataaca gaccccagct tcagcacttc atcttctgga tacgatgtgg 94440
ccgttgcgtt catagctaac tacacaaaac ctgtacaaga tgtggtcctt catcatgtat 94500
agagagggac tgatgaaata cacacacaca cacacacaca cacacagaca ccacagcaac 94560
tggctgttaa tagttgctag gggaatggga gtcctttctt caaatggtat agcacatact 94620
ccagtaacta ttccctcccc atgttcatgc aggcagccct aattaaactc actcatacac 94680
acaagcacag aagcaggaaa gtaggaggcg gaaacgagaa ggcagcgggg tttgtggata 94740
tgatctcagt acatcctata catgtttaag atcatgagaa aatatacatt ttaattaaaa 94800
actaaattcc aacatatgta aaagattttc cagtatggag tttagtaagt ctgcatggct 94860
gaaagtagaa agagggaaga ttaatggctt gaaagaggtc ttagtttgga ctctggtgtg 94920
tgaccaaggt catttagtgt gtagtgtaaa gatcaaatcc aaaaccttct ggtgaagtgg 94980
cttggcagtt aagagcactg atcttccaga ggaccagtca gtaggtttaa ttcctagaac 95040
ccctcacagc tcacaaccac ctgtaacttc ggtcccaggg aatatgatgc cctcttcagg 95100
cttctgtggg caccaggcat ataaatggtg cacagatata catgcaggca aaacatccat 95160
acacacagaa agaaaaaacc aaaaacctaa ggccttgtca tgccacacaa gtgatctact 95220
actgatccca cggaagggcc tggaaaccca ccagctatta cctttgggaa ctgccttcac 95280
tacaaagagc tctcaccccc atttccatgg tagtcatatg aatgcctttc ccattgctgg 95340
gaccttcaga tcctgggaga caggctaaag tgatctgggc tatctccctc acacagctca 95400
aggtacccca aatagacatc ttgtatgtca gtgtccatat gacaaaatat tccaaactta 95460
caatgaagaa aatggaggtc cagagagcgc aactcagttg tggctaaggt gcatctgtgt 95520
ttatatccag atacttagcg cctaggcttg tgagttagag aatgatttta ttgggacaaa 95580

```
aaagatgata atgactgaat ctattagtag ggatttggta taagattaag taaaatatca 95640
ttaatgaaga aatgattaat tatttaaata tataatacaa gctacttaat aatacttaaa 95700
ttatttaata aatattctaa gtgcctaatc atcaattaat cggaatccct ttctaccaaa 95760
aattccatat gtatgaaaat ttaaaccata cgaattttag acataccacc aatcttagac 95820
aaacacccctt gacatcgaga aggccctaat cgagacttca ttctgtgatg taaaagcaga 95880
gcagattcta cctcttagaa tgcatcacaa atgaacagaa actggttaaa cagcaaattc 95940
tgttttccat tacagtaccc tctctccacc ctaacacacc cttattctgc gttggaaact 96000
ttaagacaaa actgtcttgc tttggtgttt cgtaatactt cctgggacag ccatgccacc 96060
atgccccagc caggactttc tatggctgtt agctgtgcgt cttaaaacat ggtgctgaag 96120
cccttcccac agagagacac cattgtttcc tgggtttagg ctcctgagtc caggccacct 96180
gtggcaaggc aggaacgatg ctgagtgact tccgaaacta ggtccgaatt tctgccaacc 96240
ttcccggaac taccttctcc tggaactcag tcaccctgtt cagagggtgc caagcagcca 96300
tgaagagaag gccagtacag gtgttcagcc aacagcctta accgaggtcc tagccaaagc 96360
ttgccaagcc gacggctgcc aggtgaccct gcctagcctt taggctgtct cagcagatgc 96420
tgaccacaat aaacatgggc tgttttgctg ttctcaaagg acagatttga aagcaaatta 96480
aatactagtg gggttttaa gccctgggtg tagagtggac accaccattc tccaggcatt 96540
tgaaaagaca gcttgcctac cataagttca gcagaaaaga tactgactgg agagacgc   96598
```

<210> 26
<211> 3892
<212> DNA
<213> Mus musculus

<220>
<221> misc_feature
<222> (1)...(3892)
<223> n = A,T,C or G

<400> 26

```
agcggccgcc gcgggcgcgc tctagcagcg caggccggag ctcagggccc cgcgcgcccg 60
gcccgccccg cgcttctccg cccgcgccgc agccatggcg cgccgctgag ccgcccgccc 120
gcccgcccgc gccccgaccc ggctcggctc ccgccggtcc gcgccgctcc gcagcggagc 180
ccgcaggcga ggagaggccg cgcgcatctc cagggtaccc tcagaggcca gaagagggtg 240
tcagagccct tgtaactgga gtttgacggt cgtgagctgc gcatcttcac catggcagac 300
gatgatccct acggaactgg gcaaatgttt catttgaaca ctgctttgac tcactcaata 360
tttaatgcag aattatattc accagaaata ccactgtcaa cagatggccc ataccttcaa 420
atattagagc aaccaaaaca gaggggattt cgattccgct atgtgtgtga aggcccatca 480
cacggagggc ttccgggagc ctctagtgag aagaacaaga aatcctaccc acaggtcaaa 540
atttgcaact atgtggggcc tgcaaaggtt atcgttcagt tggtcacaaa tggaaaaaac 600
atccacctgc acgcccacag cctggtgggc aagcactgtg aggacggggt atgcaccgta 660
acagcaggac ccaaggacat ggtggttggc tttgcaaacc tgggaatact tcatgtgact 720
aagaaaaagg tatttgaaac actggaagca cggatgacag aggcgtgtat taggggctat 780
aatcctggac ttctggtgca ttctgacctt gcctatctac aagcagaagg cggaggagac 840
cggcaactca cagacagaga gaaggagatc atccgccagg cagccgtgca gcagaccaag 900
gagatggacc tgagcgtggt gcgcctcatg ttcacagcct tcctccctga cagcactggc 960
agcttcactc ggagactgga gcctgtggtg tcagacgcca tctatgatag caaagccccg 1020
aatgcatcca acctgaaaat cgtgagaatg gacagaacag caggatgtgt gacgggaggg 1080
gaggagattt accttctctg tgacaaggtt cagaaagatg acatccagat tcggttttat 1140
gaagaggaag aaaatggcgg agtttgggaa ggatttgggg acttttcccc cacggatgtt 1200
catagacagt ttgccattgt cttcaaaacg ccaaagtata aggatgtcaa cattacaaag 1260
ccagcttccg tgtttgttca gcttcggagg aaatcagacc tggaaactag tgaaccgaaa 1320
ccctttctct actaccctga aatcaaagac aaagaggaag tgcaaaggaa acgccagaag 1380
cttatgccga acttctcgga cagcttcggc ggcggcagtg gagcgggagc cggtggtgga 1440
ggcatgttcg gtagtggcgg tggcggaggg agtaccggaa gccctggccc agggtatggc 1500
tactcgaact acggatttcc tccctacggt gggattacat tccatcccgg agtcacgaaa 1560
tccaacgcag ggtcacccca tggcaccata aacaccaaat ttaaaaatgg ccctaaagat 1620
tgtgccaaga gtgatgacga ggagagtctg actctccctg agaaggaaac tgaaggtgaa 1680
gggcccagcc tgcccatggc ctgcaccaag acggaaccca tcgccttggc atccaccatg 1740
gaagacaagg agcaggacat gggatttcag gataacctct ttctcgagaa ggctctgcag 1800
ctcgccaggc gacacgccaa cgcccttttc gactacgcag tgacggggga tgtgaagatg 1860
ttgctggccg tgcaacgcca tctcaccgcc gtgcaggatg agaatgggga cagtgtctta 1920
cacttagcca tcatccacct ccacgctcag ctcgtgaggg atctgctgga agtcacatct 1980
ggtttgatct ctgatgacat catcaacatg agaaatgacc tgtatcagac acctctgcac 2040
ttggccgtga tcaccaagca ggaagatgta gtagaggatt tgctgagggt tggggctgac 2100
ctgagccttc tggaccgctg gggcaactct gtcctgcacc tagctgccaa agaaggacac 2160
gacagaatcc tcagcatcct gctcaagagc agaaaagcag cgccccttat cgaccacccc 2220
```

aatggggaag gtctaaatgc catccacata gctgtgatga gcaatagcct gccatgtctg 2280
ctgctgctgg tggctgccgg ggcagaagtc aatgctcagg agcagaagtc tgggcgcacg 2340
ccgctgcacc tggccgtgga gtacgacaac atctccttgg ctggctgcct gcttctggag 2400
ggtgatgccc acgtggacag taccacctat gatgggacta cacctctgca tatagcggcc 2460
ggaagagggt ccaccagact ggcagctctt ctcaaagcag caggagcaga ccccctggtg 2520
gagaactttg agcctctcta tgacctggac gactcttggg agaaggctgg agaagatgag 2580
ggagtggtgc caggtaccac acccctggac atggctgcca actggcaggt atttgacata 2640
ctaaatggga aaccgtatga gcctgtgttc acatctgatg atatactacc acaaggggac 2700
atgaagcagc tgacagaaga cacgaggcta caactctgca aactgctgga aattcctgat 2760
ccagacaaaa actgggccac tctggcacag aagttgggtc tggggatatt gaacaatgcc 2820
ttccggctga gtcctgctcc ttctaaaact ctcatggaca actatgaggt ctctgggggt 2880
accatcaaag agctgatgga ggccctgcaa cagatgggct acacagaggc cattgaagtg 2940
atccaggcag ccttccgcac cccggcaacc acagcctcca gccccgtgac cactgctcag 3000
gtccactgtc tgcctctctc gtcttcctcc acgaggcagc acatagatga actccgggat 3060
agtgacagcg tctgtgacag tggtgtggag acatccttcc gcaaactcag ctttacagag 3120
tctcttactg gagacagccc actgctatct ctgaacaaaa tgccccacgg ttatgggcag 3180
gaaggaccta ttgaaggcaa aatttagcct gctggccgtt cccccacact gtgtaaacca 3240
aagccctgac agtccattgc atcgtcccaa aggaggaagg caaagcgaat ccaaaggtgc 3300
tggagaatcg ccggcctgca gggtcactcg atttcattca aggccttccg aatttggcgt 3360
ccttcttggt tctgaaatga aatgtagttg ccacgcacag acggtgtcta gcaatcatgg 3420
cgctcgctcg ctcagctgca ctctatggct caggtgcagt gtcttgagct ttctctgctg 3480
ctactggatc acatttgctt tgtgttgtta ctgctgtccc tccgctgggt tcctgctgtc 3540
attaaaaggt gtcgctgtcc ccacccggtg tcctttctag ccatctactg taagttgtgc 3600
attcaaatta agattaagga aaaacatatt tttaaatgag taccttgatg cgcaataaaa 3660
aaaaagacat ttcttttttt aatgtggttt atctgtgatt taaaaataaa aaacacatga 3720
acttatcaat atttaaaaca tgctacaatc agtgntgaaa atagtatttt ccccgtttta 3780
tgcattttac atttgtaaat atgttttcta atcaatactt taaaagaaga atgttgaatt 3840
tataaaatgc tatttacttt tttatttata ataaagtaca gcacatgtga ct 3892

<210> 27
<211> 2916
<212> DNA
<213> Mus musculus

<400> 27
atggcagacg atgatcccta cggaactggg caaatgtttc atttgaacac tgctttgact 60
cactcaatat ttaatgcaga attatattca ccagaaatac cactgtcaac agatggccca 120
taccttcaaa tattagagca accaaaaacag aggggatttc gattccgcta tgtgtgtgaa 180
ggcccatcac acggagggct tccgggagcc tctagtgaga agaacaagaa atcctaccca 240
caggtcaaaa tttgcaacta tgtggggcct gcaaaggtta tcgttcagtt ggtcacaaat 300
ggaaaaaaca tccacctgca cgcccacagc ctggtgggca agcactgtga ggacggggta 360
tgcaccgtaa cagcaggacc caaggacatg gtggttggct ttgcaaacct gggaatactt 420
catgtgacta agaaaaaggt atttgaaaca ctggaagcac ggatgacaga ggcgtgtatt 480
aggggctata atcctggact tctggtgcat tctgaccttg cctatctaca agcagaaggc 540
ggaggagacc ggcaactcac agacagagag aaggagatca tccgccaggc agccgtgcag 600
cagaccaagg agatggacct gagcgtggtg cgcctcatgt tcacagcctt cctccctgac 660
agcactggca gcttcactcg gagactggag cctgtggtgt cagacgccat ctatgatagc 720
aaagccccga atgcatccaa cctgaaaatc gtgagaatgg acagaacagc aggatgtgtg 780
acgggagggg aggagattta ccttctctgt gacaaggttc agaaagatga catccagatt 840
cggttttatg aagaggaaga aaatggcgga gtttgggaag gatttgggga cttttccccc 900
acggatgttc atagacagtt tgccattgtc ttcaaaacgc caaagtataa ggatgtcaac 960
attacaaagc cagcttccgt gtttgttcag cttcggagga aatcagacct ggaaactagt 1020
gaaccgaaac cctttctcta ctaccctgaa atcaaagaca aagaggaagt gcaaaggaaa 1080
cgccagaagc ttatgccgaa cttctcggac agcttcggcg gcggcagtgg agcgggagcc 1140
ggtggtggag gcatgttcgg tagtggcggt ggcggaggga gtaccggaag ccctggccca 1200
gggtatggct actcgaacta cggatttcct ccctacggtg ggattacatt ccatcccgga 1260
gtcacgaaat ccaacgcagg ggtcacccat ggcaccataa acaccaaatt taaaaatggc 1320
cctaaagatt gtgccaagag tgatgacgag gagagtctga ctctccctga gaaggaaact 1380
gaaggtgaag ggcccagcct gcccatggcc tgcaccaaga cggaacccat cgccttggca 1440
tccaccatgg aagacaagga gcaggacatg ggatttcagg ataacctctt tctcgagaag 1500
gctctgcagc tcgccaggcg acacgccaac gcccttttcg actacgcagt gacgggggat 1560
gtgaagatgt tgctggccgt gcaacgccat ctcaccgccg tgcaggatga gaatggggac 1620
agtgtcttac acttagccat catccacctc cacgctcagc tcgtgaggga tctgctggaa 1680
gtcacatctg gtttgatctc tgatgacatc atcaacatga gaaatgacct gtatcagaca 1740
cctctgcact tggccgtgat caccaagcag gaagatgtag tagaggattt gctgagggtt 1800
ggggctgacc tgagccttct ggaccgctgg ggcaactctg tcctgcacct agctgccaaa 1860
gaaggacacg acagaatcct cagcatcctg ctcaagagca gaaaagcagc gccccttatc 1920
gaccacccca atggggaagg tctaaatgcc atccacatag ctgtgatgag caatagcctg 1980

ccatgtctgc tgctgctggt ggctgccggg gcagaagtca atgctcagga gcagaagtct 2040
gggcgcacgc cgctgcacct ggccgtggag tacgacaaca tctccttggc tggctgcctg 2100
cttctggagg gtgatgccca cgtggacagt accacctatg atgggactac acctctgcat 2160
atagcggccg gaagagggtc caccagactg gcagctcttc tcaaagcagc aggagcagac 2220
cccctggtgg agaactttga gcctctctat gacctggacg actcttggga gaaggctgga 2280
gaagatgagg gagtggtgcc aggtaccaca cccctggaca tggctgccaa ctggcaggta 2340
tttgacatac taaatgggaa accgtatgag cctgtgttca catctgatga tatactacca 2400
caaggggaca tgaagcagct gacagaagac acgaggctac aactctgcaa actgctggaa 2460
attcctgatc cagacaaaaa ctgggccact ctggcacaga agttgggtct ggggatattg 2520
aacaatgcct tccggctgag tcctgctcct tctaaaactc tcatggacaa ctatgaggtc 2580
tctgggggta ccatcaaaga gctgatggag gccctgcaac agatgggcta cacagaggcc 2640
attgaagtga tccaggcagc cttccgcacc ccggcaacca cagcctccag ccccgtgacc 2700
actgctcagg tccactgtct gcctctctcg tcttcctcca cgaggcagca catagatgaa 2760
ctccgggata gtgacagcgt ctgtgacagt ggtgtggaga catccttccg caaactcagc 2820
tttacagagt ctcttactgg agacagccca ctgctatctc tgaacaaaat gccccacggt 2880
tatgggcagg aaggacctat tgaaggcaaa atttag 2916

<210> 28
<211> 96592
<212> DNA
<213> Homo sapiens

<400> 28
ttgacaagct ggttctaaaa tgaattcaga aatgcaaagg acctagaata gccaaaacaa 60
ccttgaaaaa gtgaacaaag ttggaggact aacacttcct gatttcaaga cttattgtaa 120
agctataaag taatcaaaaa ggtgtggtat tatcatatag atagagaaaa gatcaatgga 180
acaaaataga gtccagacat acagatacaa ctgattttta acaatttgca aaggcaattt 240
agtgaagaaa ggatagtttt tttttcaac aaatgctgca gaatcagttg gaaatcaata 300
atctaaaaag aaaaaacttc aatccatatc ttgtaccact atatgcaaaa cttaactcaa 360
aatgtatcat agacctgaat ttaagtcctc aaactacaaa agttctagaa gaaaatagag 420
gagaaaatct tagtgagttt gagtcaggca aagagtatgt ggatacaaca ctgaaaccac 480
aatgcagtaa aaaactgaca aattatactt catcaaattt aaaatcttct ctgcaaaaga 540
ggatgaaaag acaagtcaca ggctaaaaaa tatgtatttg caaatcatat atctggtaaa 600
atacttttat ccagaatata aaatgaactc tcaaaattca ataataagaa aaaaaataca 660
ccaaaagtaa acaaaacatt tgaacatgca tttcaccaaa tggcaaataa gcacataaac 720
agatgcccat catcattagt cattagggta aagcaaagta aagcacaatg agataccact 780
aaacatatca attgagacga ttaaaataaa aaagactgac cataccaagg attgcaaata 840
tatgaaggag ctagaactct catttcctgt agtgggaata taaaataata tactctttgg 900
gagaacagtc tgcagtttct taaaaagtta catgtatgct aactatacgc tctagccatt 960
tcacttgtag gtatttccca tgacaaaaga aagtatatgt ctatataaac agttaaatag 1020
ttacacacaa atgttttagt ggttttatta atatatccca aaactggaaa caacctaaat 1080
gtctatgaac aggaaaatgg ataaataaat tgtgactatc tatacaatgg aatgctactc 1140
aacaataaaa aggaattaac tactgataca acaaggtgga tgaattttaa gataatcatg 1200
ctcaatgaaa gaagtcagat aaaaaatagt acctactgta tgattccatt tatatgaaac 1260
tctagaaaat gcaaactaat ctatgacaga aagcaaatca gtggttgctt aggaatgtgg 1320
aggagtgagg aatggaaatg aggaactgca gagggccatg agaaaacttt tcagctgatg 1380
cacatggtca ctgtctttat tatggtgatg gtctcatggg tatatatata tatcaaaaac 1440
aactctctct ctctctctct ctccaatccc ccaaccccat ctcttacaac aagaaagtat 1500
gtccctagta tatctggaag ccatcttttg accaagagaa aagccagcat gaggaaactg 1560
ttaacacagg gacagaagaa aagaaaatgt ctctgaaggg aagagagcca gaattgtgat 1620
tacattaaac ctaaagattt tcaacgatgt gacctgatga aattccataa tggttaagtc 1680
attttgagtc ataattttac atcttttcac cttaggtgaa aagcatccta accaagataa 1740
gtatatgctc agcattatat tgataattta taatacgcca ttggaaaaaa actctcttcc 1800
tggcatttaa ttatattact tatttcaaag aaaatgcagt aaaaaactga tctgtatatc 1860
tactgatatg tctatcagta aaacagataa gtatatctat gtatacctac tatgcataag 1920
ccttacaggt gctacataga ggtgatatct gttgtttata ctctttgtct tcccttcttc 1980
cttttttga taacagtgcc ccaatttacg cttagctaac cataatctag cattactaat 2040
tcatgtcttc cagatgagat agaccctcct tgtcactaaa agtagacatg tgactcacag 2100
cattgtaaga agatagtaat ggaacacagg tccccaaatt taccctccca atctcttctc 2160
aattactttc cccaacaaga aaaaatgcta ctgcaggtaa gcaggggcat ctaaagcagg 2220
gtcacttgag gtcacaggct ccagggtctc taaaaggaaa gagatactct cactcttctc 2280
tgttttaggt agtctggagg gccttgttat agaaagactt aaaacaaagg tctcaatttt 2340
atggcttcca gtgaaagtta gcctgaggaa acctatgaat gccttcagga aagcattaca 2400
tgggcttatt tgtgtgtgtt cttgggaagg tcagactaat ctgggtagtg acaggtcata 2460
tggaggaacg ctgcactcat ggaagagttg cttgtagccc ctgaggttag gcaagcattc 2520
tcccttgcac ccagggagct ctgggagaag aaagactttt tttaaagctt cagctgggtg 2580
tggacagatt gccagttggt ttactgccag cccacaaaag cagaaacctt tcccctggca 2640
ggtttgccac tgcataaaag accctaacta cttgtcctgt aattataggt cagtgtatac 2700

```
agagcctgag gtctgaacag gaaatggaaa ataataactt gccctagtag tactagcaag 2760
ccagagagga aaactaagat aaacacttca aacaggtgtt ttgttgaaat agaactgtta 2820
gaagagccag gtgaaaactt ttctaaaaag taataaattc actcaagaag atataattaa 2880
agattaggag ttaaatatat gatagatgct agaatgataa aatatacata ttttaaaaat 2940
aggtaatgac tccatgtgtt tctgacagta tacccttcct ggttgaaata actaaaatga 3000
aataactaat ggaaataact gaaatgctac ataacatatt tttaaaaatt aatactgctt 3060
agccagcatg aaattcagga atctaaatat ttccaaaact gaaaaactgg agggaatagc 3120
tgagaaattg acttagtctg ttttgtgttg ctatcacaga aaaccacaga ctgggtaatt 3180
tataatgaaa agaaatttgt atctcacagt tctggaggct gggaagtcca atatcaaggt 3240
gccagcatct tgtgaaagct ttcttgctac atcatcccat agtgaaaggc agaagagcaa 3300
aagagcagac actcaagaga gaaagtaacc tactcctaca ataatggcat tcatccattt 3360
atgggggtag agacctcatg acccaatcag ctctcaaagg tctcacctct caacactgtt 3420
gaatggggag ttaagtccca acacaggaac tttgggggac atgttcaaac gataacactg 3480
cctatctagt ccttgtcact ggacagaaaa agaagaaatt ctcacctgtg aattcataac 3540
cacttacact atatgtgatg atcaaaagca cacaagtaaa aaatttttatt taaggttttt 3600
ccaagttaat agtataccag ataacaggca aaaagaagca caaatattcc ttactcaatt 3660
ttaacctaag cttcaaataa tttccagaga ttgtttcaag aaacgtaagc aatttattat 3720
ttataattca gttgacatat taaacagcat atgaaatata gctgaaaaga gaattgataa 3780
actagaaggc aaatataaag aaattatcca gaatgaaatc caaagagata agagatatta 3840
aaagacacag agattagatt gagaagatct aacatatacc taatttaaat tccagaaaac 3900
aagtctaggg aaaatgaaag agaggaaatg ttacaaaaga taatggacta ggaatgtaac 3960
tgttgaaaca tcaaatcttg agacccaggg agccatataa catgcttcca aaatccaagc 4020
ttattatatt aatatacaaa aacattaata ttaatattat atattaatgt tattgttaat 4080
ataatcatta tattattaaa catataattt atacataaac aatatattat atataaatgt 4140
atatgatata taatatacaa tagttataat atataataaa ttatatattg acttctatat 4200
aatatagaat tatataactt ataaattata tatagcttta tatataaatc aataattata 4260
tattgatgtg tacaattatt attttatatt attattaata attatataat tggtattata 4320
taaatattaa ttttatattg caatagttat aaagtaaata tcatataaca ttattaatat 4380
aataaatata gtatattaat attaataact tgtatttatt aacaatatat taataaatga 4440
cataatttca catttctttc agatttctgt aaaagcttat caggagttat atatatgcaa 4500
aaccatattt atcttattaa agaggcatgt ataagtgtca gaccatcact tatgaataga 4560
tttgacagcc agatatacaa cctttcagaa aacagaagca aacctcctcc ataaaaacaa 4620
gtaattggca gctgtctcaa tgatgtccag taactagcct tatttcactc agaacagttt 4680
tgtcggcttc atatagcatg accaatcacc ctggtttact agagcctgag ggtttcctgc 4740
gcaagggact ctctgtgcta aaagcaagat agtcctggat caaccgggat gagttaagtc 4800
tctatttcca tgccttcata gaaatcatta aaagatatct ctctgttatg ggctgaataa 4860
tgtcacccac cctgaaattc ctatgttgaa gtcctaaccc ccagtacctc agaatgtaac 4920
tggattttga gatagggttt ttaaagaggt atataagtta aaatgaggac agtgggatgg 4980
gccctaatct aatatgactg atgtccttat aagacgaaat gatttggacg tggacacaga 5040
gggacaacca catgaggaca gggagaagat ggccatctgc aagccaagga gagaagccct 5100
caagtaaacc aatgctgcca acacattgct gttgaacttc cagtctctag aacgataaaa 5160
aatatttctg ttgtttaagc cacccagtct gagatatttt gttttggcag ccctaacaaa 5220
cacattctct aagggtttat tctcattttt ggtttagtgt atttcagctg atgattatag 5280
cctaatcctc cccctcttct tggggcctct cttctctgta ctcttcctcc tcatatctca 5340
cacaggcaga tttgttcctg cctcctcacc tacattacga gctccgcaca gtctcctttc 5400
ttctggctcc ggctccctca ttaatctttc tgttattaac gctattaact cgttaaacta 5460
tctctccacc tttccaaact tccattaact ctggcactcc ataagtgtta gtcaaataaa 5520
tgaagaatca cactaactcc tctttccttc cctctcactc caaactcttg gtgagattct 5580
tttgcactta tgccctcgtt tccccatttc ccactcactt gtcagtctcc tcattcttgg 5640
ttttcctcat tatattgaaa cacatctgaa aaagtcatgt aagaccacag ccactgcgta 5700
tcagtgctat ttgagagtta ataaagttcc cttttcctcc aagacagcga gcagagtgaa 5760
cagaagaaag actggtttct gcccttggct gcacagccat gccccgaggc cgggcttagg 5820
attttataat tcacaaagtc aactgtctct tcccctctcc atcctttagg aactgtctgc 5880
agtatctgac actgttgaga gctctcatac cctttaaact ctctcttctc tggctttctg 5940
agctttcctc ccacttttga tggcactgca ggagttttta agcatctctc tctcatacca 6000
accccagaaa taaagtcatt cctcgaattc ctgtacttag ccccttgtct cctaactctt 6060
tgaccactaa tgttttagaa acactctcct gacaactatc gcatctttgc agttgtttcc 6120
atttccaacg ccatgtctct ctgagcagca gccttgagtt ttcaattgca tatttattag 6180
tcatttccat atggacatct cactaaatct gtcatactaa acatgtctaa aatatgtatg 6240
tttacatgtt acatgtatgt ttggtatgtg ggaatgcatt acattcccac agattttttc 6300
ccagttacag tgttactatt ctaatcactc agactagaac actcagagct atctatgagt 6360
cttcctctca ctcaaactgc atatataatc tataaacagg tcctgttgac ctcatcttct 6420
aaaaatctct tacatgctcc tttttatatc attggataat agtttagtgt ggtggttaat 6480
atcatgcaca ctggagccaa atctgttagg ttcaaatcct gactctcctt ctatttacta 6540
attatgaaac cttaggaaaa ttattcaact tttcatctgt aaatatgggg attataatgg 6600
tgatccactt tatatgactg acaaccaaat aagtgaatac gtgtatagca ggtattaacc 6660
atatgcttag ccgacctatt tcaacctcct aaccaaccag aagaactgta gttccctgct 6720
ctcttgcatc taagtggggc catggaatgt gggcagaagt gttgtgtcac ctttactcag 6780
```

```
aggcaattaa aaagaagaca tacttttcct atgttctcat tgccaatgaa cacactacac 6840
agatctcaac ctcagaattc tctccacact ggtatttcca aaatagcttc atgagaaggg 6900
aaatttgtgt agtttcaatg atttagtgat gctatggttt gggtatttgt ctcctctaaa 6960
cctcatgttg aaatgtgacc ccaattttgg aggtggggcc taatgggaga tgtttcggtc 7020
atggaggtgg atccctcaca aatagattga tgctattccc tgcaaggagg agaggatgag 7080
tgagttctca ctgcattagt tcctatgaga gctggttgct aaaaagtttg gcatctctcc 7140
cctctctctc gccatacgat ctctctgcac acaccacccg cctttgcctt ccattgagtg 7200
gaagcagcct gaggccctca ccagacgcat atgctggtgc catgcttctt gtacagtctg 7260
aagactgtga gcctaataaa cctcttttca ttataagcta cctagtcttg tgtattctgc 7320
catagcaaca caaatgaact aagacaagtg acaaacacaa aataacttgt cttttagaca 7380
atattgatgt ctgaaattcc acattgaggc atttttttcc tcttcgtagc ctgataatgg 7440
caaatacctt ctgacacctt aggatggatg aatttgtaag agatattcct ggaagcaggt 7500
ttgtacagtg tagagaaaat gttgggtaac aaagcaggaa tggaggaggg ttaatcagca 7560
agccaaaaag cttttatagt tttgcctgct gtgctgcatg gaattacatc cccccaaaac 7620
atatgttcaa gtcctaacca caggtacctg tgaacatgag tttatttgga actaggatct 7680
ttgcaaatgt tatcaagtta agatgaggta atactggatt ggggtgggct ctaaatccaa 7740
tgactggtgt cttcataaaa gaaaggtgag ggagagttga atatagcgac acaaaggaaa 7800
cacggggaag gagaccatgt gacaatgtag gcacagaatg gagtgttgca gccaagccaa 7860
gaatggcaag gattgctgga gctaccagaa gctaggagga ggcaaaggat tcttccctag 7920
agccttcaga gggatcatgg ccttaatggc accttgagtt cagaatttta ggctccagga 7980
ctgtgacaga ataaattccc gttgtaagct gttcagtcgg tggtaaattt ttacagcagc 8040
cccaggaaaa tatgcctgct caacacccta ccctgaattt taagttcatt ttatggaatc 8100
attttgagtt tggagtttga tttaatcctt ttcagtaatt aacgctcttc atttttttact 8160
cccaaatatg tcacactggc tctactctta taacaattgt atttatctat cttttggtaa 8220
tggggctgcc acccccttgca gaatgaaaag tagagtgtgt atgctcacac ctccctccct 8280
ccctccctct ctccctccct ctctctttct ttctttctca gagtatcgct ctgtctcgcc 8340
caggctggag tgcagtggcg caatcttggc tcactgcaac ctctgcctcc agggttcaag 8400
cgattctcct gcctcagcct ctggagtagc tgggattaca ggcgcccgcc accacgcccg 8460
gctaattttt ttgtattgtt agtagagatg gggtttcacc atattggtca ggctggtctc 8520
aaactcctga cctcagggga tccacccgcc ttggcctccc aaagtgctgg gattacaggt 8580
gtgagccacc gcacccggct cacagaattt cttaaaaagt cattgtgttc agtctttatg 8640
gatcctctaa attccagctg ctcagcctcc cggagctaaa tgagatcact taaaggtcga 8700
ctgttggggc ctccaaggtc gcctcggaga ctcacctcat aggcaatcaa atggatcgct 8760
gggtttttgt gtgtgtgggt ttttttgtt tatttttttt ttccagaaaa acactccacc 8820
aagaaggttt ttataaccta ctggaggagg aggatggaga gtgggcacga aaggactagg 8880
atgaacatgc cataagcaat ctaaatgcca ctgcaatatt gcctggtgag gacctgatta 8940
ctgatgtttt aaagctaagc tttcagttgt cactccaccc agtagtgaaa caatgagctc 9000
taaaatatat atttcggctc aagctttctt atgtggggag gtaatccacc cgaaggtatc 9060
cccagcctgt acctaataca gtgcccagca ctaaagcagc tcagatgcca gtgaatggtg 9120
gccactggga ggcctgtcag tgggtgccag tagcggtctc ttcagagaaa aagaaaactc 9180
ccctctgcca gatcagtatt ttatgagctg tgaaccaaaa ccattgctac caccatcact 9240
ataattctat ccacagtaat tatcataaag gcctaacaat gccttgtaga tgaacattct 9300
gagtaactgc tctataacca ggagatttaa gaccgcacca aaaaccagta gagggttata 9360
ctttactggg cacaagtcgt ttatgataac gaaattgtag tttaatctgt gaagagatgt 9420
gaatgtaact gagacacgct taaatggaat atacagatga gctttatttt tatatctggc 9480
atgcttggat ccatgccgac cctccagctg ctcgggcctg cccttagggg ctatggaccg 9540
catgactcta tcagcggcac tgccaccgcc gccgcctccg tgctgcctgc gttccccgac 9600
cattgattgg gcccggcagg cgcttcctgg gggcttccct accggctcca gcccttggga 9660
ttcgggagcg ccctgctagg aagccagagc cccgcagggg ccgcggcgtc caggccgcct 9720
aacgcgcgcc cctcgcccgg cgccccgaag cggccccgag gggcgggagc cgaggcgagc 9780
ggcaaggccg ggccgggggc gcacagcgcc cctagaagtg cgggcttccc ccacccccgg 9840
cagcgaccct acctcccgcc cccgctgcgt gcgcgcgtgt gtccgtctgt ctgtatgctc 9900
tctcgacgtc agtgggaatt tccagccagg aagtgagaga gtgagcgaga cagaaagaga 9960
gagaagtgca ccagcgagcc ggggcaggaa gaggaggttt cgccaccgga gcggcccggc 10020
gacgcgctga cagcttcccc tgcccttccc gtcggtcggg ccgccagccg ccgcagccct 10080
cggcctgcac gcagccaccg gccccgctcc cggagcccag cgccgccgag gccgcagccg 10140
cccggccagt aaggcggcgc cgccgcccgg ccaccgcgcg ccctgcgctt ccctccgccc 10200
gcgctgcggc catggcgcgg cgctgactgg cctggcccgg ccccgccgcg ctcccgctcg 10260
ccccgacccg cactcgggcc cgcccgggct ccggcctgcc gccgcctctt ccttctccag 10320
ccggcaggcc cgcgccgctt aggagggaga gcccacccgc gccaggaggc cgaacgcgga 10380
ctcgccaccc gggtaagcca aactcgggcg agtgggggcc cggcagggga cgcgtggccc 10440
aagtcctgcc gcccagccct ccgcaccccc tccagcccca ctcggacttc ctcattcctg 10500
cgctaaccgc tgggctagac cgtgggagga ggttgacagt agctgagagg cacatgggat 10560
tagcgacagc ggggaaagac acatccggac ctcgcagggg ctagtcggcc gaagggccgc 10620
ggccgcccgg cggtcatttc tcttcacgtc cctccgcggg gcgggaacgc gaaccggagg 10680
ggaactctta caaactttta aaatccccaa ccccagcccc acctggggga tggtgagaag 10740
gttggagtgc gctgcggcgc ggaggagaga gggaaggtgg ttggtgcagt gcagaaccag 10800
atttcaccta agggcgttcc atgaaatgaa agcagaagtg cttgtcagtc ctcttggctg 10860
```

ggaaagccct ccccagctcc ccagaattga ataggagagt ctattcatcc ctctcctact 10920
tctaaaagaa acctggctcg ctctctgtct ctctctctct ccccccctccc cccctccgtg 10980
cgcgcgcgcg cgcgcacaca cacacacaca cacacacaca cacacacgat atagtcacct 11040
gctataggac ttgattctga tcctccgggg cctggcattt gagagagaaa ataaattacc 11100
tacctggata cttagagcac tttttagact tgattatgta aaactcttgg acagtgccac 11160
catgcattat gcatgaccgc tgaaacaaaa ataatgtaaa aacaaggcct atcctaaatg 11220
caagtttttc tatggtcctt aaaaacagac accagggaat gtttgccctg acttgctggt 11280
ttatcctgaa actcaaatgg gacttaatga tacaggattt aattatcaag attgacatgt 11340
tcatggactt tgttaagtag aggttttcag cattacagtt tatgaaataa ccatatttaa 11400
ctcaaagatt tttttataaa gctcataatc tgaaaaaatg cttatggtat tactaaaaca 11460
tttaatagct ctggcatcaa aatattctac atactggtga tcttttagta aatagtaaag 11520
gttagggtca agatattcaa taattttttt ctagcagaat cccacaactg aatattgtca 11580
agcagtttaa actgcattcg tgtttgttaa aactttaaaa gggaaactta aaactaaagt 11640
atgttgtttt tctgatttta atattgtgct ttctagagat gaatcctttt actgtttgag 11700
gcactatagg aattatgtat ttaaatgtat gtatttaaat ttgaagcaat gtactttttt 11760
gagtttataa acttgggaag acaggaaata aaaagatttg tttcctgtag aattttacat 11820
tcatgattta attgaattgc taaaatggaa agaacatgta cgattaatgg agacttggaa 11880
tctgagatta ttcatccata ttgatacacc tgcaattcct aaatgccctc ccctgcttgt 11940
ttagataaaa tgtcttcctg ggctgcagcc tactggacaa acttgaacac aaaggaacac 12000
acgttatatg tacatttaat actggaaccg aaaagctgct tgcagtgaaa agcaaaccac 12060
ttctaaactc tttgagactt tttaaagaag gtagtataat ccttttaggg gttggtggtg 12120
atgtgaaaat ctattatctt tttgctgaaa tttcattctt atgttaggca ttggcactca 12180
cttgtgctga gtaaatgccc gtgtttttca aacccagata gtaaatactg gggtatacaa 12240
tacaaaaata gtccttcccc tttgtaagca ttgctttgat ctttgcactt cctttttact 12300
ctacctctta aacaagatct gtgttgattg agttgattaa agcacaatta atctgaaata 12360
ggcagaattt tagatttagt gattatattc cttacatttc tgttgttact gttcctggaa 12420
acaaattgaa gtagtttgag aaactaatat ttatgcaggt tgttttaact acacttttag 12480
acttgcagat aattgattaa gtaagttact accttgactt tcagggattg ctcattgtgg 12540
tagatacgag gtctagatag ggaattggca gctacatgaa tgttggacta tcattccatc 12600
agcaagacct tatttttacc taatttatga gaggtatttc tctgttcaga agaggtgaaa 12660
gttctggctt ctggggggaa gtggttactt cataaccttc aattggtttg aacttgggag 12720
aagtaagaaa agtagtcgat attttcaaac agtaaaataa ttgtatctga gttgctgtat 12780
ggatttttga tgaagtactc aaaatgtcat cttttgcatc ttcgggtctt agtagatgtg 12840
ctattctgag tatagaattg actgcagtag gaagtgtatc tggaaatgca cagtactgtt 12900
gtataaccaa tagagtaaat tctcagatta tgctcttctg gtccacagat tgatcagaaa 12960
tcacattaac aggaatggat gtctatgcaa aatttctcat taattttctt acttttagta 13020
ggaagtttgt tttattcggt tggtctgttg gttttctgtt tacaaggtga attgtctaca 13080
gtagtgggat tgttatatta gaagagaagg agaaggaaaa atagaagtta gtgctagagg 13140
atatgttaag atgttctgct cctacatcta catttgtgtc taatcagcat taattgatga 13200
ctacgtcttg gtaaacagta gaccatactg gagcaattta ctctgctttt tgggaattag 13260
ttctcatttg cactcttgca cctcccataa atactgaact cacttgtagt gttcttagag 13320
gtcagcaata aaccatcaag agcatatgat ctctctttga attttgtcac tactttggag 13380
acagacagtt tggaagaact gaaggtactc agcctcaggg agaaaggttt gaacgttttg 13440
gcatagtcct gcaagtcagt ttggctcctt gactgtgaat ttttttaatc aaaaaagatt 13500
aatatttgaa accacataat ttggacagtt aatagttctg gtaacataac caagcctttt 13560
atctttgtag tatttcatta attagtatcc agtgcctcac ttttagaaga aaaaaagaga 13620
aaaaagtagt aacattcttt taatgatgat gaaattgctt catacctttg gtctctgctg 13680
tattaggggt tgttcctggg tgcaaattaa tatactgtct tttacatttc cggaggttgt 13740
tgacctggcc ttgaagatca gccaacagtt tagaaaataa gttgtgcaaa ttgcattgct 13800
acacctcaaa taatttaaag cctgacagtt ctgtacagaa taagagtcac ataagatgtg 13860
atttgttaaa gattatagaa atctcagaag atagattatg gatgaaaata tttgtttaat 13920
ttagagtact aataataaaa atagtgcatt ttacatttta aaaagaaaac aatttttatt 13980
gtgtcatatt ttctgtagta acaaacagaa ttaattatca gtaatacaaa tttcatctgc 14040
tttaatttat tgcctgtttt tgaaattaga ataatgatat atttggttta attatccatt 14100
acaaattgcc ttattatttt tcattcatct ttcaccatta atccagaaac cttaaaatag 14160
cttttatttt gacccatgcc ttcagtagat atcaacagac ttgagttcat ctatggatcc 14220
tattagttta aactgtgaca aattttgact gcttaagctt tagaaaagaa tttattcttt 14280
aaaatagcca aaattataaa aaatgaaata aagaaaaagc tgtttgagag aatcaaatac 14340
tctagtttaa ctaaaaataa ataatatcta atcaatactg acgaaagaaa tatctgatgc 14400
ttagcattgt acttgttggt acatagtaag caatcattaa ctgttaaatg aatgaatgat 14460
atgaagttgt tgcaaaattt ttttttaaat ggacagccat cttctataag gaacaaaaat 14520
tacattattt tgctttgcct tctggaaaga aaggagcaca aaatagtcct tgtgtcttag 14580
atattatgga aatcttatct aaagaccact tgaagtttac tagaacaaat aatttattaa 14640
aaagtatctg gcgccttcca aaaggggaac atttcattct gaaagaggca tttatgaact 14700
agaaaaaaaa tgccttttag tttcaaagtt ttagaggcac cagagcaaga ttataaagta 14760
attggaatga taaaaggtgc ttgtaaaatt gaaaaatgta atttttttggt gaagttagaa 14820
tttctgtaca ctccattgaa actgtaatat caaatactaa tacctccaaa aaagaaacaa 14880
tgctacaagc actgcttggt aatctaagaa ttgttaacac ttatctaaat taatcaacca 14940

```
aaaagttaaa acaaaatatg ttattttgtt atgttacctc tagaattaaa cacctatcac 15000
ccataaatct gtgactgtta aaagttacta gatggcaaac ctcaggaaca agcaggcagt 15060
tagcagggtt tactgtactt tccactttca tactttaatg atttttccaa cactatagaa 15120
gctggggcct catcctagca cagtttgttc attatttttc agttgtagca tttttaaaa 15180
gttggccttg aatttctgaa ttgaaccatt tattttttg tggacacagt tttttaaatg 15240
aaatatacca tttagactag aaaagagcaa aatgccaata ctccctgtcc tagtgtcatt 15300
gtgccccctg ggaaccctaa tgcagtcctg tgggaaaaca accattttat tgagcgctgc 15360
tcacttcagt tcattctgct ttgctgccac ttagttctgg ttctgaccct gatctcccct 15420
cacatcactc acaacaaaca ttcaagttta aaaattaagt taaaagttag ttacatatat 15480
atgtaactaa tgtgtatatt aaatatactg atatatatta gttacatata cagatatggc 15540
aacaacaaaa aaggttatat atatacatat ttgtaactaa tatgtatatt aaatataatg 15600
atatatgtat aaatacatat ttgtaactaa tataattata tttaatatat attagttaca 15660
aatatgtata tataatattt tttgttgttg ccagcaaaat gagaaagaaa ttaaatgtta 15720
acatctataa caattgcact gtaaagccgt cattgttgaa aagtactccg aagattaata 15780
gcggcagtgt ttgttaaaat aataaaataa tccaggcact ctgcctgtga cttccccatg 15840
agatggcttc cagaaaaaga taaatttttg tgtgttccct tacgagttca gtctgcagct 15900
tctgggcatg aagagaagca taaacaaaac aaacaaggct ctaactttgt tataatcctt 15960
acgctactag tagaaatgta tggacaattc atgcatttca tattggaaaa taaagctatt 16020
agatttttct ttgagatgaa cctttttttt gtaatttatt ttttattttt tattttttttt 16080
cagtagagat gggattttgc tctgttggcc aggctggtct caaactcctg acctcaagtg 16140
atccacccgc ctcagcctcc caaagtgccg ggattacagg cgtgagccac ctagcttggc 16200
caaggtgaac ctttttgata aatgaggctc actaatattt ctaggattta cttttcacta 16260
acttgaacta aagaaagtat aagacctgca taaagttgag catacctcta taatgtccca 16320
ttttaaaaag caatttgttt ttaatgactt ttatttgtta aaagcagtta tttcaaaaaa 16380
ttatttttat tatttatcat gtcatttgga ctgaaaatac tataccatat ttcagtgtta 16440
gggcacacct atccctcttt atcccagacg tcaattctac catataacaa gtatgtaaaa 16500
aagaaaacat aaaagtgatg acagactccc taatcaacct atttattggt tctaagccca 16560
agcctgcagt tcatttaaat agtgaaaata gattacttaa ttgtcttaga aaatatgtct 16620
tccatgacaa gaattttagg aatgagaaaa taactatttt tggatgtttt aacacaaatt 16680
cccaaatcat gttttattga tatttgaaga acctctttgt ctaccagttc ttaagtatag 16740
gcttattttg taaggcttaa acatgtattg gtgtttgact gttaagactt gatctataac 16800
cttcagtgtc tttttctttt aaatggggta tggacttggt aatgaggcca gaggagaata 16860
ccttacaatt tatagcatct gcatataata caagaacctt catgatttat aaaattaaaa 16920
tgatgatgat gtggaatgat ggaagaagca tgtgtgggtg gctatttatg tagaggaaat 16980
tagaagttga aagcaatgtt aagaattaaa attaaattac acttaatttt ttgctcagtt 17040
ggatagtcag gaaagtactt aaatcagcaa ttgatttacg taaaatcaaa actgaaaact 17100
gaatatacaa tctaggaaat aggaaaaaaa tttttagtgt gttcatactg ctaatttcaa 17160
accaagcaaa aaacctgggg aactactttt cagaggagat ggcttaaaat gaaccaaaaa 17220
tatagccatt tctctaccct attcatttaa tgcattcatt tattatactg ctaatacatg 17280
ataaggtgaa taggttaagc atcgtttgta tatgtgtatg tgattttaaa cctgagtctt 17340
ttgaaagatt ggtcaaagca aaggataatg aaagcataaa gatttagggg gaagagtcct 17400
ctataaaact aagacttaaa ctttgttatc aacttgaatg tgaagataga ataatctaga 17460
aagtgaatga gaattataaa taaaaggaca aatcatatat ctcatggttg agaaatacaa 17520
tgagataaat tgtatatctc atctcatgga gggtcataat gaaagaagta agagagattg 17580
cccttggata gaacctttcg gcagacattt cataatggat tatcatattt ttgctaaact 17640
ggtaagaatg gacttttcac cacattatga aaaaacactt tagaagtcag ttgcgatcaa 17700
gcaaatataa gtacctggga aattaaaatc aaaagcacag cagtccaaca atagtcaaat 17760
gcatttactc actctcatac catatcatag cgttaatttg agataatgtc tttatggtgt 17820
ctctttatat atttactcga tttcccattt aagacattta accaaattta aaaagcaatc 17880
cttagaaact ttaaaaatat ttcctatttt aaaccattac ataatcattt accctgcagt 17940
tacaattctt tttttgtgat gggtggggaa gtatcccatt tctcttagaa agtcttctgt 18000
aatagccaaa taggagtcaa tccaagttgc ttagagaggt agttcaatcc tgtgttgaaa 18060
tagatgactt ttggagtaat tgtgctatat ttactgagta tctgttgaac tgtaggagtc 18120
cagtcattca aggcaagcta cagaaatgat tttattgctg aggcacttaa gtatcttcac 18180
atctatatgc caatattaaa tttctcatca tccttgatcc ccaccccttt gcacagattg 18240
gtatccccag cacattaggg aatgcggttg ttagcctgtt agggtccatt cagtagattt 18300
ctcatgccag aaatctagaa gcccttctta tacattcagc tttcttcttc agtcagtcac 18360
caagttctat caaggcagcc tcctcatctc cagaatccat cccttcctcg ttgaatttct 18420
agattttatt tttgcattgt ctcctgcttc taacccatcg ttcatttcgc ataatggtca 18480
gctttctaaa gtgtaaatcg gatcatgtct ctgcagtgaa tcccagttgt cttcacaagc 18540
tgattctcgc acacctcacc cccttactcc ctccggtgtt ccccaaccat cccctaatct 18600
caaatcctcc acaccgactt tcagtttcct atccacagtt tctgactttg aatgcctttg 18660
cttatgccct ccctgtatga gaagtgcctt ttctttccta gccatcctgc taacttctaa 18720
acccttaggc ctggtttgag catcaccatt tctctcactt gcctttctgt tttctccctc 18780
ccctttccat aaagagttat gtgcccccat tctgtgttct cctgaattcc tgtgggtcct 18840
agaaaagaaa catcttgagg gcaaggagtt catcttattt actttttatt tgcacagcac 18900
ctggtgtgtt tgctgaataa gtaaataccg aacaagatat caagagacag tgctgccaat 18960
taactgtgtg gctctgagca agttaattaa cttctctgag tcttattttt gttctgtaaa 19020
```

aaaaatgagg tttgacaaaa tgagcatctc tctctaaaat tatgtgtttc tttgatgtaa 19080
ttgtattaat tggaagactt cagattttct tgccatatca agataggtca acttaagaaa 19140
aatgcatgtg agtaagtacc aagccaattc ttgattatca gtatgataat atgaggaact 19200
gcccattttg tcgataataa gttataattt ttggtctcag tgtaggttct ttctcaaatc 19260
cagatgtttc tggtgttgca ttttttcatt attattcagt ctctcgcatt catatttctc 19320
caaagaaatt tatggaaaac ttgagacaaa aattgaatgt aaagtttaat atttaaatgc 19380
tagattgatt caaggtctca ctaattataa ctatatttgg agggagaaga aggggaggtg 19440
gtggtagtat gagctgaatc ctcctctgtt ccttcctaga aggaacacaa cgtcctacat 19500
caagaaaaaa gacagaagca tatcttggag atatgaaaag aaccaccagg taaactaaaa 19560
agaaaaagag tgttaaaaag tcagccttta aaaagcagga ttggagatga ggaggggtgg 19620
agtaggggaa gcattgcttg tttatagaag ctcttccatc ttcgtatagt gtgattttta 19680
tttttttatt attttttcct tccttttttt tgacctccag ctttagtata gtacagttga 19740
cagaaattat atatgtttat ggtacacaag atgatgtttt gatatacata tgcattgtga 19800
aatgattaaa tcaagctgat taacataagt agggttttat ttctttaaca gtgatttatt 19860
tgaatatatg ttgagtcctt tctatgtgcc agacacagtt ctaaggacaa ggcacacagt 19920
gccaaacaag acagaaatcc agaaattcat ggagcttaaa tcatgtagtc ttttcagttt 19980
ttaaaatttt cttgctgggc gtagtggctc atatctgtaa tcccagtact tcaggaggcc 20040
gagaagggag gattgcttaa acccaggaat ttgagaccag catggacaaa atagcgagac 20100
cccagtctct acaaaaaatt taaaaattag ctgggcatgg tggcacatgc ctgtggtccc 20160
agctcttcag gaggccgagg taggaggttc acttgagcct gggaggttga ggcttcagtg 20220
agcagcgatt gtgccactgc agtccagtct aggcaacaaa gcaagactgt ctcaaaaaag 20280
gaaaaaaaaa atagaaagag aaactttatt tttacattag ccttttttaa gttgaagaat 20340
cccatagcta aaaaagtaca caaatattaa tgctgcaact ttagtgattt atctcaaggt 20400
gaacacgttt aacctagtgg gtggtcaaga aatagaacac tggcagcact gcaaaagcat 20460
cctatctgcc ctttcttagt taggacccct tccctccact gtcatttaaa agatttcaca 20520
aacaactttt ggattatatg tatgtcaatt ctgtgactca tatatatcca catagacaat 20580
ggagcgttga ctgaatgaat tcattattcc aaggtgctaa taatgagctt cacaccagcg 20640
ggaactctgc tgtgtcagtt gggctctaag ctcagttcat ttcagatatt cctcactaaa 20700
caaaatactg ttttttcctca gttgttagta aaaaatagaa aacagattac attctcattt 20760
acttattact tatgaatgta aaagtaaata ttttgcttaa taccaagtag ccagggtcac 20820
tgacagagac atgggagaaa gaagtattca catctgatgt tatcagacat agaagagaga 20880
ggtatagttc acctatttct ccccactcag tttatacatt gacatctgta tgtgtgttgt 20940
tttgttttgt tttgtttgtt tgttttgaga tgtggtgtcg ctctgtcaca caggctggaa 21000
tgcagtggcg caatcatggc tcactgcagc ctcgacttcc tggtcccaag ctatcctccc 21060
acctcagcca cccaagtagg tgggactaca ggcatgcacc accacacctg gcaaaatttt 21120
aattttattt tactttttaa aatttttgta gagatggggt ctccctatgt tgccaaggct 21180
tgtccccaaa tacctggacc tcccaaagtg ctgggattat aggtgtgagc caccacaccc 21240
agccatgtat atgttaaagc gtttatctgt atgagtggtc agtgtagaaa gggcttcatg 21300
gagcctgatt ttgatggctt gcaaattatt atatttgtgt atgtgtaatc ggaaggcctt 21360
cgccgtgatt atttcttcaa aggaaccctt tgtccctcca gggtgattgt acccatgttc 21420
ataggaccaa acattgctgg ttcacataat gactcttttt cttttaaaac ataaaggaca 21480
tatttttatg gatttttgag gattaataga ataaaaatct aaagtcatat tcagattcaa 21540
gagaactgga tcagtttctc tgtatttgaa caaaagaata ctaatctcta attcataatg 21600
catgaggtct acttcttcct cagtctgaaa atatgaatat tccaaaatgg tactcaaggc 21660
agaggctata tatcaaaatt aaaattctgt gatactatgt ttttagggaa gaagaaaact 21720
gtagcattag aaatgtagac gcaaaaggtc agaacaaagt ccagtatgtc aagtcccttt 21780
ataatgccat ctgaatgaaa gacctcacac catgaacaga ggtattgaag cagtttttat 21840
gtggaaatgg taggcagcaa tatctcagga gtgactttaa ttataacatt taaggtttta 21900
tctaacttat tataatcctt agagaacttc tctagctcta caagttattc ttggcagaat 21960
ttgtcctaac atagtattaa taaaaataaa ttttacttga gtatgatata aattactctt 22020
aattacaata taattttaaa atgcccaaaa catcttttga tcttatgaat ttcagtcagc 22080
tatgattaag aagacaacca taggcaaaaa tttctttctt atggaacacg ttggaatgca 22140
ttctaatcac ttctctaaga aaatccctgt cccccatgat tatagtgaat atgatacagc 22200
aataatttag atgcttcttt caggggggtg aggttatgta ttctacagtc tattgattct 22260
aagaggcact tttttctccc ccaatttaac agttttgttt aattatatac atttcaggat 22320
tttttgggga tatttttcta tcactggttt ctgatttaat tccactgtat ttagataaca 22380
tattccgcat gctttcaatt ctttgaaatt tattgagact tactttatgg ctcgggacgt 22440
ggtctatctt cgtgaatatt ccgtgtgtat ttaaaaataa tgtggactct gggccgggcg 22500
cggtggctca cggctgtaat cctagcactt tgggagtccg aggcaggcaa atcatgaggt 22560
caggagatcg agaccatcct ggctcacacg gtgaaacccc atctctacta aaaaaataca 22620
aaaacttagc caggcgttgt ggtgggcgcc tgtagtccca gctactcagg aggctgaggc 22680
aggagaacag cgtgaaccca ggaggcagag cttgcagtga gccgagatgg cgccactgca 22740
ctccagcctg ggtgacagag cgagactcca tctcaaaaaa aaaaagaaaa taataataat 22800
aataatgtgg actctaaaat cattcagtgg ggtgttgtag acacactagg ttcttttgat 22860
tgacagtttt gttcctaagc cttctatatc tttcctacca tggtttgaat gtgtccccac 22920
caaattcagg tgttgaaaca ggatgaccaa tgtgatggta ggaaaaggta gagcctgtaa 22980
gaagtgatgg gattaggtgc ccttttaaaa gggcttgctg gaggaaattt gtctcctctt 23040
gcctttccgc cctctgccag ttgcggacac agcattcctc ccctctagag gatgcatccc 23100

tcaccagata ccaaatgctg gtgccttgat cttgggcttc ccgttgccag aactgtgaga 23160
aataaacctg ggctctttat aaattaccca gtctcagcta ttctgttata gcagcattga 23220
cagactaaga catttcctga cactctgttg atttgctttg atcaattacc gagagaggaa 23280
tgttaaaaat cttaactgta attgtggatt tgtcattttt tccattagtt ctgccacgtt 23340
tttgcttcat gtattttgaa gctttgttaa taggtgcgta cacaataaga tcattgtttt 23400
tctttgatga gttggccctt tgattcttat atgcaatgtt cctctttgtc cctggtaatg 23460
tttattctct tgaagtctac tttatctgat attttagaat gtagcctgct tttcttatat 23520
ttagcatttg catgatataa ctagcccagt gtaccatctt catacattta aactgtgtct 23580
ttaatattaa agtacaattc ttatgaaaag cgtatacttt aatcttgttt tttaaaaatc 23640
cagtctgatc atctctgtct tctagatgag ttcagtccag ttccatgtaa tattattatt 23700
attattattt tttttttttt ttttttttga gacggagtct cgctctgtcg cccaggctgg 23760
agtgcagtgg cgggatctcg gctcactgca agctccgcct cccgggttca cgccattctc 23820
ctgcctcagc ctcccaagta gctgggacta caggcgcccg ccactacgcc cggctaattt 23880
tttgtatttt tagtagagac ggggtttcac cgttttagcc gggatggtct cgatctcctg 23940
acctcgtgat ccgcccgcct cggcctccca aagtgctggg attacaggcg tgagccaccg 24000
cgcccggccc catgtaatat tattactggt atgttgggtt taagtctctc atcttggtaa 24060
ttaattatct atttgtgcta tctcttcttt attctttttt ttctcctttc ctgccatcct 24120
ttgaataaat caagtttagt gttttaaatt ccattttatc tccttcattg gttactttct 24180
aaagtggtta ctttaccact ttagaaaagt atttgtttat tacaatatgc atctttgagt 24240
ctctaattaa tgtgagaact tgacaatgtt atacttctgt ttgtccttct gtgctgttat 24300
tgttatacat tttacttctt tatatgttat aaactccaaa atatattgtc ttgttttgct 24360
gtgaacagtc aatcgtctgt ttaggaaatt aagaaagaag aaataaaagt cttctgctta 24420
ctcacatatt tatcattatt gatgcttttt attctttcat acaggtccaa gtgtctatca 24480
tttcccttca gcatgaagaa cttcttttta gcatgtcttt tagcaggtct gctggtgatg 24540
aatactctta gctcttgttt acttgaaaat gtcaatttca cctttatttt taaaagattt 24600
atttgctgaa tattctggac caatcttttt ttttctttta tcactgtaaa ggtgccttta 24660
cattgccttc tagcttgtac tgtatctaat gatcatcaga aacatcatgc ttatcatgct 24720
tatcaattgt tccccccatat ataatatgtc ctttcttgtc ctttgctatt ttgggatttt 24780
ttttttctct agcattagtt tttaacagtt tggctgtaat atgcctaacc atggttttat 24840
ttgtctttat ccagcagaga gatctttgag attcttggat ctgtgggttg attttttaaa 24900
aatcaaattt ataaaaattc tcaactgtta tttttttgaa tatttttct gtcttttttc 24960
tgtttctggg attctatgtt acaaatacgt aagactgctt aatattgtcc tgtaggtcac 25020
tgaagctgtg ttcacttctt tctgcctttt tttctctgta attcagtttg ggtagttttt 25080
attgaccttt cttctagttt attggtactt tcttctataa tgttaaatat gctaagtcca 25140
tctagtgaaa tttcatttca gttctaaaat ttccattctt ttttttaaaa gaaaggtatg 25200
catttctttg ctgatattgc ctatctgttc tctcattatg ttcatctttt cctttacatt 25260
attgaccata tgctattagc tatttttgtc atttgtgtca tttttttct cagtcttatt 25320
ttcctatttc tttgcaggct tcattatgtt ttattgtaca ttgtggagag tttgggtttt 25380
ttgtcttcct ttaaaacgtg ttgagctttg ttgtaacagg cagtgaattt actggtggat 25440
cacctagttt tattaagata ggtttagagt agcccttatt ttaggatgtt tctaactccc 25500
atcgtgtggt ctttctgaaa tcacaactga atgctcacaa tgttcagtga tgtctttcca 25560
ccctggatta ttacaaattc agtgtctctc agcactatat gatgtctgga atttccattt 25620
agctcttaga tgcccagaaa ctgttttcta ttaggttttg cagggtcttg tcctacacta 25680
tcacagcttg gtatttggcc agtgaccaga gatgacccct atgtagattt ctgaggctct 25740
tgctttttgt gtaaagcctt tctttgtgat attctgccca ttggtgttct ctctttgggc 25800
tctccctgtg tcatgatttg gaaagtgtcc tcagtcaaaa agtgagggcg aatttagaat 25860
ttacctaaat caccttataa tatatagccc tatgaagtcc aatgtcttaa attacatctt 25920
taggtatttt gtatggtttc acagttgagt atagcaagga gggaggttca gataactgtt 25980
aatctgtgat aactaaaacc agaatttgac attctgacaa acttgaaatt gcagtgcttc 26040
ttaaaattaa tacagtatta cttaaaaaac aaaaacaaaa aaaccagtgt tttccaattt 26100
gccaatacaa aaaagcaaaa ctcccatctc tacttttag gaacttctat ccatttgaga 26160
agatgataaa tatataggta aagttagatt gcagtaaaac tatgctgtct agtgttaaat 26220
caccagtata tataagtttt atagatatta tgtgactaca gagctatagg ttataggaaa 26280
aatataggat ttcttggcag aaattggatt taactagagc cttgaagagt ggatgctatt 26340
gaggtagaaa agatggcatt tcaggatgca gggcatattg tgaacaaatg tacaagatgg 26400
aaatgagcaa gtcattctga atgattaatc tgtctatcat agagggtttt gattaggaca 26460
tagtagtagt tgatgcttat aatatgattt aatataataa tgtaatgtgc tgagtggttg 26520
aagtctttgg actttatctt ataagtaata tggcaataat gtgaaagatt cttgaagagg 26580
aagaaaccct gcagtgaggg agaacagtac agagcctatt actgtcattc agaggtgaag 26640
caataaaggt ctctcctagg atgttggaaa gaaggggcag atccaagaaa caacatgaag 26700
aaaataatca gcagaaattg gttattgact agatatgtgt gaaaatgata tattggcaga 26760
aattgcaaag tcaaaaatgg ggctcattgc agcaggaatg atgaaattgc ttttagatag 26820
agaggtgaag ggatttaaaa cattaacttg ttttaaaggt atcactgctt aaagctaaca 26880
gtgatttatt aatatttgca ctctgaaagt tttaacagtt ttaattttat gtatacacca 26940
cagtgttttg gctactatac ttgcctcttt tgttcagttg ggaattgagt aaactaatag 27000
agtaaaaaaa tgagtacaaa gtgggctttg actagaactc taacaaagtg tgatcactga 27060
attaccaaga taattaaccc ataccccaca tatactccat ttcccatgac acatattata 27120
ttgcattgtt tttgcctatt tgccttccta cttcctaaac tgttgaatta gtttgctttt 27180

gttgcattaa aaaaaaggag aatcaccaca aagcaaatta aaagatactg attacttctt 27240
ccaattctgt gggctgccta gttcatctgg cctggcctgc tttggctaat ctctgaggtc 27300
ttttggctac ttgactggta ctgtttgtag ctactcagct ggtgccctac tgctgtttgt 27360
ctgaagttgt tttggctact cagctggtgc tagggagcct cagctaggac tgcctgtctg 27420
tgctccacat ggtctttgat tttccagtgg ctggtgcagg cttgtttgca tggtaacaat 27480
gttccaagaa agcaagaatg atcattgcaa ggcctcttat aagctgggtt tagaacttgt 27540
acttctacct cttcctattg gtcagagtat gtccgtaggt ctgccaagac tcaaagcaca 27600
gagaaataga tgtttcctct tgttgagtgg catataaaaa aaagtgtggc cattgatttc 27660
aatctgccac aaccgtaagc tctgtgagta caggaactgc atcactcttt tgcactttgg 27720
tgttgttggc atagtaggca taaatatttt taggtgaatg actaaattaa tgaaggaagg 27780
aaaacattca ggtagtattt caatatactg tagttttaaa ttatatatat tatatacatt 27840
tgacatagtt taatatataa gtatatacac atatagtttt catatataag tatattatat 27900
attatataaa atatttatat gttatatata aagtatatat ataaaatata tagttttaat 27960
atgtaagtat atgtatataa ttatatataa tattataagt atatatactt ttatcatacc 28020
atcagtgagt atagacatgg ttcagggaga atctctttat tctccttcta tcttttgaga 28080
tagaccaaag aaaatttttt tttaaaaaaa gaactatcac ttgagttaac attgtttggt 28140
atttttatct tcataggtga ttgccttgtg aataactcat tttgtgtatt gtcagaatcc 28200
tattctgctt ttcaggctgt gctatgggta gactagctct aaccagaaca cttgggagac 28260
ctgaagcaaa gatactatat taaaataagt aatgttatat gccaaagagg aaaaccactt 28320
ctgcctccta aacaactgaa agaaccagag actgtggggt cagattagct agcattacta 28380
tatgaacctg agcaaatgac ttactctctt tcagcttcag tgtcttatct aggacattgg 28440
ttctcaggat tggtgtgaga attaagagaa atatgtgaag ttctagctac agttctttac 28500
ccatgaggta gtcagtaaag tttacttctt tctccatgtg aaccaaagca ctgtcatgat 28560
gtgatgatgt tcattagggt ttctcatgct cagcgttatt gatatttagg gttgcataat 28620
gctttattgt gaggggccat tctgtgcagt ataggatgtg tagcagcatc tgtagcctct 28680
atacactaga tgccagtagg accacctatc cagctgtgac aggcaaaaat gtcttcaaac 28740
gttgccaaat gacccctggg ggcaaaatgc aaacagttta tctagcagtg acacatagcc 28800
agccacctta gaaagggaat tgtcagcgta cccataagtg tagaatggga aacaaaatac 28860
ttgagtcttc tcagaatgtc taaacctttt gctgtctata ttcctctttt tataatttac 28920
cacaaacata tattgcttat tatgccatct aataaaatgg ctaacatttg ctgtgtatta 28980
tactcggtgt taataagctt tgtatgaact cactagctga atgttgtata tgtatctgcc 29040
atacctgttg gattgacatt ccactgtaaa caggtacctt atctttacca gctccaagca 29100
caatgtattt ggtatatcag gtagctattc tacatgttaa ttgatcacat tgcacccaaa 29160
tgaaacatgg atgtggaagt ggaaggagga gctttaaaac atggaaaatt agtttataca 29220
aatctaaatg ttgtagtgca ccaaattgat tcctttttaa aaactttcag aaatcagctt 29280
ttaaaaaaaa aaagtaacta agccatgatc aacttttctt tcatcaccac taagccaata 29340
tcagaaagtt gtcaatcaac agctctttta aagttgaaat ttttctattg tatttcttat 29400
gaagatttca gtagtgctta gaagtttttg gctcatttat catgttttat tcactgtatg 29460
tcattaattg ttaaagatgt cagagtcaaa ccttaaattc aggagagatt taaatttttc 29520
ctttttccta tttaaatata tgtttctaat tacataattc agttagaaaa ttacaatata 29580
gaaacatata acttagtaaa agtttccagt tactgtatac agattatatt gtatactgta 29640
ttatttttaa cagttgcata tttaacttat aaatgtggat aattataatt aaacaatctg 29700
ttatcaaagg acatacagat tatttccagt tccttgctgc agtgaatatc cttgtactta 29760
tttttttaca cacttgctca gataattttt aagtacaaat tcctgaggta gaattgcaga 29820
atcaaaagat actctcattt gagtgtttca cattttatgc aaagtatttt gtttcctcca 29880
gaaaattcct ctgtggaaga ggcttcctca cattctttat cagtttatta gccagcagag 29940
aaatgatgaa aagccacccc ttttcttgta gtttcatcct cattgctatc caagatgagg 30000
tattaaaatc acacctaagt ggtgtctgtt gtggctgaat ataatcttga aagtagagac 30060
acttctctct gattcccaac tgtactctct ggatggcacc taaaatttta ttttttcaaa 30120
ctgaaaatca ttttaatgag ttcgaaaagg ttttattttt taaaatgaaa tcctatagaa 30180
agaatacatt ttcttacagt gggtcatggt caaaagtgtg aatatctctg atcctagaaa 30240
gtttccccag ttacaaggac aggcgtgccc ccaactccat ccttaatggc tcaattctgt 30300
ggctttcaag ttaatccact cagtcctaca gccttcacca agaagctcat gtgctacact 30360
ttggtgttag tgggggcatt gtcacacagg ttcagaaaat aacccattag gcattttata 30420
gaactcatca catgttcaga actaagctag ttgtaacatt atgtttttca tgtgaaaaaa 30480
cacaagcaat tcactggcta ttttatttgt tgctagctag ctatactttc tatttctttt 30540
tccatggtgt gaaaatcttc ctagaaacct cactactggg aaaagagatg cagagattag 30600
tcccatcttc tcccctggaa atttactatc gcccttttgg cttcaaggtc cttcctcaaa 30660
agcaaagaat tgaacaaggg aaagtgaggg gaagggctgg gggaacaggg aggtagaagg 30720
ggttttccct ggtcttttct aatcctttag ttccagacgc ttagttggta tttcaacatt 30780
ttagaaataa aacctgcttt tataattaat gcaaatatta cttagtgatt catcctttcc 30840
aaatgattct tggtttcttt tgacatttca tttttaaatt gtgaattaac attgacatca 30900
ccatagcgtt actgttacct cagctcttta cagtgaactg tattgcaaag gctataaaag 30960
ttcaaatttt aactatcacc cacgtgagcc ttggactgct gttaggcttt tgaaactgtg 31020
atcctttggc aagaattggt gtataaatca tggggaggat attgaagggg aagatcatgt 31080
ttaatttggc agttgggtgc ctgcaattga cagattttca ttaaggtttt agctatagta 31140
gtgatcatgc atcagttcta ataaagtagg tttcgtttga attttaaaaa ttagagtgag 31200
gactgcttta tgttaactgt ttacattcag aagtctggaa aaccatccac aggtttaaat 31260

gtatataatc agtataatta acacgtcaac agcctcacct ctgtttttga ggaccatagg 31320
ctaatattat ttatgaaggt tcaggagatc agtctagatc gacagaaagc ttaagggcaa 31380
agatggtttt aatatttttc ttggaagttt gagcttctgt ggtattctaa ggatagggat 31440
tttgacattg attgttctag ctggtattca gtactgtatt taagtgtaat gtttttacta 31500
gtattcttct tataactgaa aactatgcta atgattcttt actatagata tatactcata 31560
aatacgtgta aaaatccaag tttaccaaaa aatattaaga cttaggggat atgttctttg 31620
aataggcatt tatttgctaa ttaacatatt ctaatctcaa caaggttata ttcttttcaa 31680
aaatccattc catcaaaatc tgtcattttt acagatgaag aaacaaaggc cacagtgagt 31740
ttagttaata tccaagatca cacagccagc atgatagaac tcttaaattt caagcgactt 31800
accctcttta ctctatcagg tcaaatttcc cttttttaac tgcttgtctt aattgagatt 31860
tgtttaattg tggctgtctg tatatcttca atcaatactt tcttgtttta gacaaatgtt 31920
tccctgcaaa tctgcatgaa cttcatgttc tgaaggactc agagctgccc tttccttttt 31980
gttaaagttg tacactgctc tgcatccccc tagaatgctc atcagctttc tcactcttcc 32040
tcactcacac agtgctccag caggagagga tttctttgaa aatcagattc tttttggag 32100
ggctttgtgc ttattggagt gttatgtgat atattcatta cggtgagggc taaactcttt 32160
gaacaaagag acctgacagt gcatttgggt taaagaacaa agtttatttc tttctctcat 32220
caccaccagc atgagcagcc tggatggtca gagcagatct gtctcatggc attgttcagg 32280
gatcctcatt ccttccctct catttatctg ccatccccta ggctattaat catcatgttt 32340
ttggtagaag ccaggtcttc tccatgtctg agtttttatg gaggaagcat gtcaagggcc 32400
ttaagcccag acctggaagt ggtatgcaag agttctgaca ttgcactggt gagcatttgg 32460
tcagatggcc atctctaact ccaacgaagt gtagggtata cactaggtaa gcagtcacat 32520
ttccagctac aatactaatt ctatggaaaa ggagacaaca tattttgatg ggtagctgac 32580
attctcatct gcagtcctaa gtactcatga ctgctagata gactttatga aaaaaaaaaa 32640
gaaaaataac cccaggaatt aatatttacc ctcagccttg tgtttcttta attttctcat 32700
ttttcatgtc acatatctaa aatttgatct aaataaattt aaataaaatt aacctttatt 32760
tgaataataa taaggacatt tctcaaggaa gtaaatctca aatgatatgt gattttagat 32820
actatcttta tctgaccatg tggttccttg cttctatcag ggagggcctc tgggtaatag 32880
aagattgcta gggcctgcag tatattaatg tttagctgga gttgattaaa tctgagacct 32940
atagccttga taataaggac ttttattttt gtatttttat tgcactttta atattacaga 33000
gtttctgtac cagcttgaca acagcagaaa catggcttgc ttctgtggaa cacgctagag 33060
cagcagtccc caaccttttt ggcatcaggg accagtttcg tggaagacag cgtttccaca 33120
gactgggggc tgggggtgat tttgtgatga ttcaagcaca ttacatatat tgtgcacttt 33180
atttctatta ttattaacat tgtaatatgt aattaaataa ttatacaact caccataatg 33240
tagaatcagt aggagacctg agctgctttt cccacaacta gacagtccca tctgggagtg 33300
acgggagaca gtgacccatc tggagacagt gacagtgatc atcaggcgtt agattctcat 33360
aaggagcggg caatctagat cccttgcatg cacagttcac aatagggttt atgctcctat 33420
gagaatctaa tgccactgct gatctgacag gacacggagc tcaggcagta atgcaagcga 33480
tggggagcag ctgtatatac agatgaagct tggctggctt gcctactgct cacctctttt 33540
tgtgctgcgt tatccatagc ccagggtttg gggacgcctg cactagagga cagagcctgg 33600
tacactatag cagtcctccc cttgtgttat gcagagtact gatcagtccc ttttgagctt 33660
ctcaaccttg tataaaactt tgagacagtt ctgggaccat cacaaggttt atgaagggtt 33720
agaaaacaat acacttaagg aactaagact gtaaatcgtt gaagttcaga gggcgataaa 33780
aagaaatggc ttctaagaat cccagtatgt atgatttttt atacaaagca taaagatgat 33840
ttgtaccaaa ctcaggacac tgtggcgagc ttaacacgag ggtattgaat catacgtaag 33900
tggtaattcc tagattatgc aatctccttg ctgttagatt tagataccaa agaggaacta 33960
catcatgtcc tcctcctatg gtcttcaaaa aaggattata tttggtctta ctggtataat 34020
acagtttatt cctgcatgaa ttccatggtg atagaatttt taaagttcat tctagtgtta 34080
cagttttgtt ttgttttgtt ttaatacaca gcttcagaat ggcagaagat gatccatatt 34140
tgggaaggcc tgaacaagta agtgtcataa tctcactgat aactttattt aaatatattc 34200
atatttcaaa aatatgcaaa ggcaacatta gtaagttagc attaggaaaa ttctaaaatt 34260
agtaaatttt tttttaattt cagtttctct gtcctttcat gtgaagttgg agaggatatg 34320
tcatgtgaaa tgagtaactt acacccgctt tcacacatat tgttgcacaa aaattgtata 34380
tgctacagga tagctattgt attagctttc tagagctacc ataacagatc accacaaacc 34440
tgatggctta aaacaacaaa atacattatt tcacagttct gggggctaga agtatgaaat 34500
caaggtgttg gcagggttgg ttccttctgg aggctctcag gaagaatctg ttccatgcct 34560
ctcccctagc ttctggttgg ttgctgggaa tccttggctt gtagctttat cgctccaatc 34620
tcttcctcca ttgtcacgtg cgcttcttcc cggcgtgtct ctactgtgtc tctaaaccta 34680
aatctcctct ttttttcttc caaaggcatc attggattta gggtacaccc ttatccaata 34740
tgacttcatc ttaatttgtt tatatctgcg aagaccctat ttccaaataa agtcacattc 34800
acaggtaccg gggattagga gttaaaggtg tctttttggg agggaacaca gtttaaccca 34860
ctacagcccc ttgacatatt tatttaatgt gtacacatgg atgtagagag tggaatgaca 34920
gaccatggag attcagaaag gtgagggatg ggagggcagt gggatggggg tggatgataa 34980
gaagttactt aatgggtaca atgtatgtta tttgagtgtt ggataccta aaaaccctgt 35040
tttgactact atacaatcta tgcatgtaac aaaattgcac ttgtacccca tcaatttata 35100
ccaaaaaaaa attttattta aaactcatct agtaaggaga cagattgttg tagtgaaaag 35160
atctttgaac tcagaatttg aagatcttta tataagtact gactactaca gatcagctcc 35220
tcagacttga agaatcactt agtccccgta atcctcactt ttctcatttg taaaatagaa 35280
atgctagcac cacactaaca gggatgagtc aaaaatattc aatgaatgtg ctttgtaagc 35340

ataaaactgt tcctatcatt atattagtag ttatgaaagg atagcagtgt cctttttct 35400
ttttctccta tattttattt tgaaaaatgt tggaaaaaat aaatagaaat gttggaataa 35460
tctaacaaat ctacattggt tcttaaaacc gacattttcg cttttctctt tgctctcttt 35520
ttcgtgtgtg tgtgtgtgtg tatatatgta tatatgtata tgtatataca taatcatttg 35580
gaagcaattt tcagactctg gagcaattat aatggcaata ttaaattatt tatgtaatga 35640
ttccaagaag tatttaaaac atggagattt gttcattcat cagctattta ttgaggtatt 35700
actacgtgcc agaacattgg cttagtgggg aaaaaataag tgatatatac actctaattt 35760
caggaaccta ctattttaat aggaagaaca gagacattgt tttcatatgg tgatatatgc 35820
tgtagtagta gtctgcgcag agcgggatag aaatgttaat aagcagcatc actaggtgct 35880
accttggagg aagagcagag ttacggagga ctttccgaag gaagtcttcg taagctgtgt 35940
tttgaaagat gtagtttgct agactagaaa aggtgaagga acactaggtg ccaggatagc 36000
ctggtcacat ttaaatttta gtggcaatca tttaaaatag tggatttctg aaattacacg 36060
tgcaaagaat cagccagaaa aataaaaaca gaagtggaat atgttttaaa ggctctggta 36120
tcatttctat aaataaaatg gacatatata gcatttatca cttcgggtga tattctgatt 36180
gatttagaaa gaatgactgc attgcttttc acattaagat gctccttttct aattttattt 36240
ttaaaatggt gatttaggat aaatctagga acattttctt ttattgcagc aaagacattt 36300
ggctaattgt attatcaaaa ccgtttgttt tacctttcgc ttttaatgtg ctttctctaa 36360
caattaaggg cgaactaacc agcatgagga ttgtgtctgc ttgattttaa accatccttt 36420
cctgtctgta cacaggaaat cttatcaaca agagatgatt ctttatgcca cagaagagta 36480
aaacctgctg aaaacattca ttttgaaagt ttttcttatg tgggcatctt tgcgatcatc 36540
agtaacaggg gtggggcata ttctgacatt tccatttttg aggcaagcgg tcttgaagtt 36600
tttttctttc ttggcttgag cttggtggtt tatatttcct aaagtttgca tgtcctcagt 36660
gttttagtgg agtctggaat ttgctctcca ccctgaccta ataaacaatg caagctgcag 36720
cacaacacaa cttctctctt agtctgaagt gaagagtata tctctctcct aaggtgagcc 36780
cttttgccca ctcgccagtc atttagccac cacaggaaag agttttcctg ctaggttatc 36840
acgtttgctc tgagactgtt aaaatcctgt atgaaatgag gcccattttt gcagaacatg 36900
gtttttcatt cctgtatttc cttttcagca aacaatgaat agcatcagtg agcttttaat 36960
cttataagca ttgaagtgaa gcgcattctt tgttcctcag ttgaaaattt gcatccctca 37020
aatggatctg aagtactgtt tatttaaacg agatttttct tactcttaat ttaactaggc 37080
atacgaagat cttcagtacc ccagaacttt caataattta ctactaaggg caggagacaa 37140
actgtaatac tggctactaa aagaaacgaa aagctagttc agcacactta ctagtctctc 37200
ataaaaagta aaatatggct ggtttccatg ctgtccacag acatttcaaa catataactt 37260
ctttaggcta aaataaatag caagctcaga aatacagttt taaagtccta aatgagccag 37320
cggttacaat gtttttttgat aagtaatgaa tagtaattta ttaagataaa atgtactttc 37380
ttcaaaatat tctgcatgcc cactctgttg ctgccctaac acattaccac atacttgggg 37440
cttaaaacaa cacaaattta tacttctgag tcaggagctc agaaatctag aatcaaggtg 37500
ttggcagggc tgctttcctt cttgcggctc tcatctcttt gccttttgag cttctagtgg 37560
ctgcctgctt tccctgtgca ccctctcatc actccagcct cttgcttcca ttgtcatact 37620
tcctactatt cagtgtgatt tcctgaaccc ctctcatcag gcccacctgg acaattcaag 37680
acactctctt catcttatta tcattacctt aatgacatac gtaaatttcc ttttgtcata 37740
taaggtagca ctcacagttt ctgaggatta ggatgcagac atgtttaggg ggtcgttatt 37800
cagcttaaca cacccactaa attccaggta ttttgtgtta tgctaggaaa ctgaaggtaa 37860
aagaaatgag tgagctcagt gcttagttgt ctactccttg tactgtcagt tatattttca 37920
gttattctgt aaatagtccc agaggtgctc tctagcattc tttcaggcct tgacattgga 37980
tagctagatg aggggtatat ttaccaccat agaggaaata ctgatttcta ggattatatt 38040
attttgttta tacttctaag agagtacaag cttcaaatca ttcattcaac aactatttaa 38100
gtatctgttc tgtgataagc atgtacttgg ccctgaagat ataccagtaa gcaagctaga 38160
ccaggtccct gcctctgggg actttatagc caagttatgc ttctcaaatc cctttgttac 38220
gtaaacgttg tccaacccta tgtaaagttt tgtacacatg agaactacct aggtctcatg 38280
aagatctttt aaaaccctga tttaaaatat tttcagattt ttgtttttgg ctttagtttc 38340
attcctatta ttacatttta ggtgtcccaa cttcaaattt ttcatataat ataggaaaaa 38400
taatgattga aacatttaaa tgttcttctt tacagatgtt tcatttggat ccttctttga 38460
ctcatacaat atttaatcca gaagtatttc aaccacagat ggcactgcca acaggtaaga 38520
aaactcatcc ctgttaccct gttgttctgc tttcagtctt agtaaaatgc aggatttgtt 38580
aatagtctgt cctagaaact cagttgtgta cctagaaagg aaatggtgat ttgttttaaa 38640
aaccaatctt ttaacatact ttcttgaaat atatttgcac aaaaatattt catctcaatt 38700
acccccttgta tggtttactg catttcatat ctgctaggac tactcagaag gaatttcttt 38760
agtcaacata aggattttct ttattatctc tgtattcctt ttaagtcctt tctcccagat 38820
tcacacgagg cagtctccac tcttcatttc tgtttcaaac atttaagagg gcctgttgtg 38880
tactaccagt gtgccagtca ctggggaatc aaagtgtaca gaaatcataa aaatcatctc 38940
attagaaaaa tcatctttat aaaagcttat aactttgcac tctgacaagt tggataaaac 39000
tcataatcct tccttctacc atgactgtat taattgtgcc gattggcaac tgtttaattt 39060
cgtaagagta ataaaaaaaa aactctcttt cttaaaaaaa gtgatttatt tgtatgtaag 39120
cttggtgtga tggctctttt tatgttctgt ttctctcctc acttcacccg tcaccagtgg 39180
gacttaacag atgttcaaaa gcagtgtgag tgaattatgg aagttatgtg taaaacagag 39240
tttggtaatc gctgaaggag gcatcagctc ttttatttac cagggacttg cttgtcttca 39300
ttgggggact ttatcccatt tgtattattc tcatttaaaa ttatcatggt tactacagta 39360
cttacttttc ttttgtttta gatcagaaaa ctttttctgc atttatatct ttagtatttc 39420

taggagttca tgggagggaa aaaagtagat ctactatact ttcaccttca ctagtaaagg 39480
tgtatttcag aggtttttct gtctttaccc ctacaaaaca ttatttttag gctgccatgt 39540
ctgagatctt gtgaagtgct gtccaatttg agctctaggt cagcttcctg tgaacaagta 39600
aatgtgttgg tagtgtgtgt gtttcttggg aactttttaa ttctggagtt caaagtattt 39660
ttcttagtat taatgtatta ctgaaaatat aagaacttgg ttctgtccac aggaaataca 39720
cacattttca cttaatgtct tgtgaataca aaacagtatc ctgaggcaga cgttttctta 39780
attttattcc ttcggattta tgggcatgaa aataagatta tcaagataac catctcagta 39840
aatattctgt ggtcacagct ttatgttgta gaaattattt tgccataata ttagaaaaac 39900
tattcaaatt acacctaaaa atctaatttg ttagttgttg ttacaccatc atttttacct 39960
gtatgttttt ccctcttcat tgcagtgtca tagtacaatt tttatagtcc aagatatata 40020
ggatgttgta ttcaaatttt taaagaggta gagaggttga ttatctaaac caatattaac 40080
ttgtaactct aaaatggatt attttagtct tctagatttt ttgaaatcta aaacctttag 40140
tagatctagt acttgttgaa gactatggga tgcgacataa ctcttaggtc taaagttaaa 40200
tttctctgca cagtggaatg tccttttcat ggcacagaaa tgtggccaaa agaatgttgg 40260
ccttaaaaca acaaatacat gcatatcaaa tctaattttc tagttgattt aactttaatg 40320
tgttgcctga aagaggaaaa atggctctat tttacatgaa ttttcacata tttaaaggaa 40380
attaaatcct acaggaaaaa taatagcaaa tttgcagttg tttttagcag agttaataaa 40440
aagtgagaga cttgaagttt aatgtaaaca tgcttccaac tcctctgtat ttttcatgac 40500
atccaaatat tgctctgtta gaggactgtc ttgatatgtt ctttcaccat tttaacaggg 40560
aatgttttta taagtgttac attgtcatac aggctgtatc ttagtgtgaa aatatttcta 40620
tttctaatca attttatttc catatccaaa attaaattct agccatttgg tgggggaatt 40680
tttaaattat gattatgttt ctaaaagtaa tttaaaatgt ccaggggtga atttcataca 40740
gacaaaagat ggtaatttaa tacattgacc agtgactccc cctttttttt cttgtgtatg 40800
cttcatttca gatgcattca tttttcaact cgcttccttg taaatttctt gaataataac 40860
aggaagtaat tataccettg ttttctccag ttctctagcc ttatcctgat ttatcataat 40920
attgaaaaga taaaaataaa ttctctggcc gggtgaggtg gctcacgcct gtaatcccag 40980
cactttggga ggccaaggca ggtagatcac gagctcagga gttcaagacc agcctggcca 41040
agatggtgaa accccgtctc tactaaaaaa atacaaaaat tagctgggca tggtggtagg 41100
cgcctgtaat tccagctcct ctggaggctg aggcagagaa ttgcttgaac ccaggaagca 41160
gagattgtgg tgagccgaga ttgtgccatg gcactccagc ctgggtgaca gagtgagact 41220
ctgtctcaaa aaaaaataaa aaaaatttg agaaaattct cttttgtgtt atagtttatt 41280
tttaatttca acctgaaggt ttatctcctt acaaatttat ttaaaataag aggtcttgag 41340
cttctttctg agagaaaatg aagattaaaa tggacataca agcattctcc taaaatattt 41400
ttagcaccaa acatcttaat ttacattcaa ataaatgaga accaccatat gcctttattt 41460
attgcaaagc tatggattat tgtactattg acataaaaat cagctgggaa gcttaaggac 41520
tttttcatat tgaacagttt gtacttatgt tgtcagagat tctgattttc ctttgaattt 41580
tcaagtcaga taccattagg gtatggatta agcctctagt tttctttgcg tttttacaat 41640
aactaatttg catgtaccta tcttcaatta aattattttc agtattttat aaaatgcctg 41700
agatgtttta gaacacagtt tatatgatag cacaatattc aaaattgctt ataaacatca 41760
aaagttatgg attaatatga aaattttacc atgtctgata gaccagatta cattgggagt 41820
gtcggggagg aaaggggaaa caaactaacc agttaaggaa tgaaggcagt actttccctt 41880
caagtaaaga cgggaacaga aaacctagtg taaggaaaaa gagatagcct gagatcatta 41940
tctgtctatc agacgtatac atacaggttg ctagatcagt atcttttaat ttcacattaa 42000
gcacactctc tttgaggaag gaaagtcagt gtctaatgat gtgaaggcca aagaaaatgt 42060
catagtgccc agaagtggtt gctgctttta attggtattt gtcattttaa aatgctttgt 42120
taggagacaa gattatgaag gctgttgaca tcaattcttg ccggccatat gccttttatt 42180
gggttaggaa atgtgccatc acctttcaac aaacatttac ttattgtggt tcgctaaact 42240
cgtaaaacta ttgttaaaat aagggttaat ttaatatttc agacaaggaa gagaaattat 42300
atccttgtgg aattttaacc aaaaattgat ttgaaatttc tacataaaaa cataatttca 42360
gtttttttact tgctttacgt tttataccaa atttgagaag cctcacagtt tcttttggtt 42420
tctgtttgtt gtttttgttt ttagcagatg gcccatacct tcaaatatta gagcaaccta 42480
aacaggtaag attaaagggg tgggacttta aatgttagat tccagtgtct aatattgaga 42540
tcataagcac tgaagaatag taaatgagtt ctatgaagga agtagtttat ctgaaagatc 42600
aacaacctga caaattatgt aacagcttta ttcattcact ttacatttct cttactcatt 42660
gttcacactg tcctgagcct cagtatgtag ttctgtaaaa ctgcagttaa ttacagtatt 42720
agaattacag ttaattacag tattctgctg tctctaccag cttaacttac cagccactcc 42780
caggaaaggc agaggtgatt tccattcatt ttcatttata aggaaatacg tatgcataca 42840
gttcacatgc actgagcgat atccatctat tatccttgca catgtagtac ctcttagcct 42900
tcctccttcc tttggtagcc ccagcctttt ctgtgctgcc tttggaacat gtaacctaac 42960
atactgaaag taccagtggg aagtaaaatg ttagaatgaa ctattgaatt ctcttccctc 43020
tggtgtcagc ctctttggag tcaggagagg aacatttttt caggtgcttc cccttaccac 43080
tagagtctgt agagaatagc tcagaactga aatggcctcc tcactgtatc caactctgtg 43140
gatatttata ggaaattgga tgggaccaat gctgtgaaag agaaagaaag gcaatcttta 43200
atgtctgcct ataggaggtt tcagttatta gggaatggaa ataatggtgt agttccccac 43260
agaatgagga cagtcagtaa atctttggat ggataaacta atcacatttg tgtgtgactg 43320
aggatatgga tacctgtcct cttgcagctg tattacccaa tacattttct aagtattctt 43380
ttcattagct caggtgagtg aaacactttt gcattttgta acatttaaca catgctaact 43440
aggtcacaag cttaaataaa gcatccactg cgttgtggtt tccagaatcg tgacattata 43500

```
tatgtgttat atttggtcag catttgatat aaatgtctta atgattgctg aacaggctat 43560
ccttataagg ttgaaattag atgaaagttt ttgctgtggg ggcaagaatg ccacatgaaa 43620
ctttggggca tggttctagt tcttgcaggt gtcacatcct gactcctcag gttgccagta 43680
acactccatt gctctacctc catgttcaac tcttttaagc agttatttct cagagaaggt 43740
catcaattcc cagtgaaatt ttatccctta aagttctgtg ggctctggca tcctggaaaa 43800
tttcccagct tttaagatct aggtagtcta tgaaacagaa atcaactgaa atttgtctgc 43860
atatgccaaa gtatttttc caatatcatt ttcataagca tagcactaca ataagaattt 43920
ttaaatgtaa ttccttatta tggatccaac agttataagg aaaaattggc atttatgatt 43980
tacctgaagg gttaatgtag ttccaaaatt caaaatttaa ttcatataaa agcttatgtg 44040
agtaaaacaa tgtgtttacc aaagtgatgc tattttgata tctgaattca gtgaaagtaa 44100
gaaggttgta ttcaagtcag actttctgac tgaatggatg tagccttgcc tttgaggttg 44160
atgactcatt ttaagcaaat ggagttactg agatgagtga atgtggatta aaggcaaaat 44220
tttgatctca gaaatttaga atcagaaatg catccagaca atgcatttga cgacatcttc 44280
ctgaaaacag ttgtaaattt tcatcctcag attaaaaact tctgaggatt tagatactcg 44340
tatgtaacca tgaaaaatat ctattaagta ttgtctattt gacaccactc ccaataagat 44400
ataaacacat acgtgtctat atttttccaa ctgtcccaaa ggaattttgt gattaaagat 44460
aatgaattgt gtgtgtgtgg ttttggggtt ttttttgttt ttttttttta agttagaaaa 44520
agattctttt ttctttggga cctcttagcc acagattttc cccagctctg acggagatga 44580
gtcatagcat agactactaa tttttagaac atccagttct gttgcatatt aatcagtgtt 44640
aattaactaa tactgttcaa aaactcaagt tgtgtttaaa ttagcagtca agtaaatctc 44700
tattcttatg gttagctaat tctgcaggct attatacatg tgttgttttt ttcatggttt 44760
tagaaatttc atttaattat ttaaaaaacc aacagtgttt gcttacaaaa ggcatagggc 44820
tgaaattgta aattattttt aaatatgaat tgtgtgagaa ccaaaacaaa ggaattacct 44880
ctgaaacttc atctccaaag gcttcccttg tgtttagatc tggaatctcc caaggaattg 44940
ttttaggtct cactgtgatg agttgatcat aaactttaga catttgtgtg cattaaggaa 45000
atttcccaga ccggggaatt tactttatgc cttacttatc tggtaaatgt gtttgaggac 45060
tgatctttgg aaatcaggaa gtttttggat aatataggaa cagttccaga tgcttcccag 45120
agatttaaat cagttcaagg cacctggact gctcagagac tgcaagaccc tacttggcat 45180
aatgaaatgt agtatagtct aagtagtgca tacgttttac atttgttcat gagctcaaag 45240
ctgtcaaaat ctgcatgttt gctggggttc cacaatttaa tgagtaaggg agtgaacccc 45300
caccagcagc tagtgggtgt gatgagctga tgggcagatg atctagtgct gtatatgcag 45360
agttggtccc acaatagaaa caaaaggtga gaatgttcct taaaaaatct taacgtattt 45420
tcttttcacc aaaaagtgaa tctgtaaaaa gaaaatcatc tgcttaagcg actatgtggt 45480
tataaactac taacactacc acaaattgat tgacatcatt ataaagttga atcaggaaat 45540
acagcgaaac ctcccttaat atgatgatga ggtcataatt atccttgttt tattctttgt 45600
atacaaatat gaataagtta gctttataaa tcataaaaag ttagaactgg agtagacctt 45660
aaagatggtc tggtataatc ttccctttg tacaaatgaa aaaatatgac acttcttagg 45720
tgtttatcag cccacagtag ttatatagt gaaggcttcc agctaacttc attaattaac 45780
tatatctata aaatttctgg gttagatagt atttaaggga actacctagt tttggaatca 45840
ttctggtagg ttaatattca atcttgagct tgaccatatt aataatcata aaaacaaaaa 45900
tcttatcctc tgaaatgctg agagaagctt aacagatgca gggtctagca cagggtgtgt 45960
tctacaacgc tgaaacagta tatctaaata aacatgtctg tagtccctgc tgaaccagct 46020
gtaatcagag tagataaagg aatgtcttta agtaagagtc aaggaagcat aacttttatt 46080
aatatggtac agttcagagc ttggcagcag caatttaaga caaggaagct ctgactagaa 46140
caagccgtaa catgttaagt ctaaagccta aactcttcag cagattactc tccacacatg 46200
catagcatga gaggttccat gggcttaggt acctggcttt ttagccatat cttagtgtac 46260
aaatatcgat taataccatt tttcgtagta agattacggg aaaagtgatt cttgtttaca 46320
gagccctctt tcagtttcat gtttttcttc tctcatttag tagacataag atttaaaaat 46380
ttgtatgtac ctttgttgcc gtaattttta ataagtattt ctcaaactta attggcttaa 46440
cgttcacctt tgcagagagg atttcgtttc cgttatgtat gtgaaggccc atcccatggt 46500
ggactacctg gtgcctctag tgaaaagaac aagaagtctt accctcaggt caaagtaagt 46560
ttgtggtagc tctccttcta tttgaattct ggaaattttg atttcctacg atttccaagg 46620
aattgcttta aatgagtacg ggttgccttc gctcctaagc tgaagtgttc acatgattat 46680
taaaattttt aaatagaaat ttgtctccta gcaatagaag tgacagatac taaaactttg 46740
ttaacatttc aatttagtag aaatgtcttc agcattagct aacaaactta ctatttcttg 46800
atcatcttaa atatttttaa aaattggata cttcctgaaa ctttagtaag tctttgaaag 46860
aaatggttga ttttgactct ttggagattt agtgaaaaac caaacaagtg actgagtgta 46920
tggattatat agtatattat tttaagattat gaatttggat ccagactttg ttattagctg 46980
tgtagctttg aataggttta accaatactt ctaaacttca gtctcttaat gtataaaata 47040
caaaaatatt aatagtatat acttcataag gtcctttaga gattaaatga gaaaatgtgt 47100
acatgttcca tagagagcct gaaagtaccc tagggccttg gcctgagcag atgtgtggcc 47160
ctcttggaat gaactgctac gtgacatgct ggtggtgtat ttggtcatac cttttaccat 47220
gttcaggctg tttgtttatt tgagaatatt tttctttaag ttaatatgag acttttaatt 47280
tcataatatg ctatgtttca tagactgact gatgataaac atagactgat aatcattttg 47340
cagataaacg cagtcaacaa ttgtaattta gtttttata aaagttatta tttagtcagt 47400
aattcaaggc ttggacaaaa tagttctcac tatgtgtgtg tgcccctcag ggtgtaacat 47460
acttttggac tcagtctttt tattggtttg tttttgtaag tagatgaagc tcagtagagc 47520
aggccctcag caaagttgtg atgtaaagaa atctggccgg gctcgttgac tcatgtctgt 47580
```

```
aatcccagca ctttgggagg ccgaggctag cagatcacaa ggtcaggagt tcgagaccag 47640
cctggccaat atggtgaaac cccatctcta ctaaaaatac aaaaattagc tgggtgtggt 47700
ggtgtacacc tgtagtccca gctagtcagg aggctgaggc acaagaatcg cttgaacctg 47760
ggaagcggag gttgcagtga gccgagactg tgccactgga ctccatcctg gacaacagag 47820
cgagactctg tctcaaaaaa aaaaaaaaaa aaaaaagaaa gaaatcagtc tttggttttg 47880
ccagccttgc attttgtttc ttccttttcc tcttatccat cttctaggcc caggcctgtt 47940
aaatttctta tccttattcc caaacaaaag acaaaatgtt gtgacgtcgt tctttacctt 48000
tcaagtgaaa ctcacccatt atttagtatg ggtactacta gtcatagttg cattttaagt 48060
ctcttagctc caattgtgga atgaaagatg tttttctat taaaggtaat taggcgggta 48120
aatatgatgg cttaagtcca cagaagcaga ccacggtctt atggcctcac atcctttaga 48180
accttgatga ggaatgagga gcagcaataa agaactaatt tgcacttatt aaacaccaaa 48240
gacttgccaa gtggtttgta taacattatc tcttttaatc tcttagaaat ctcagtaggt 48300
aaaaagtgca atgcctgtga acccaaacag actcagagaa tggaattata ataactggcc 48360
tttctcacaa gactagtggc tctatatcct gcagcaggct attgctcacc gaacctctca 48420
gatataatgt gtgcatttgg ggtactgggg gcagggagat aagggacaaa ttatgggcag 48480
acaatggtgt aaaaaggcat ggtgaatgat aactcttgaa aacaagggag agagaggctc 48540
actggtcaat gggtactcag gtattgggga agccaggccc agcacttacc tttttaacac 48600
atgtgtctgt gtgtcacatg tataaatatt gtgcctttat gtatgtgtat atttgtgaga 48660
aggggattga aagacacatg ttatcagcat tgacactgac tataatgact gaagaattaa 48720
ctttaagcga aaaactggga gtttttttctg actggcttgg cacaaaaaat ataaattatg 48780
gtattttgtt tacttctatt gctctagtat tttgtgacta agtaacaatt cttgatacta 48840
acaaacacat ggcattaact ttcttataaa tttattggct aagaaaaaac tttatacatc 48900
catttattcc cagtcttcat acatctttct catgtagagg tttcatctac tcaaaaacat 48960
attgctgcct cagtcacaaa ataatttaca aatgaaacaa cttagtgggc attagatgtt 49020
cttgtagaat gaaagtttct atatcaagga gtttagtact tactgggaaa tatagatatg 49080
taaacgacta ataataaagc aggttcaact acagtcacat tatttcacca aatcatttat 49140
tttttattca ctaattcagt atccatttat taagcattta atcaacttgt aagaggagga 49200
ggaacttaga ggttagagaa tatgactata caagctgtca ttgttccctt agcttgtcat 49260
tgttcactga aaattgaaat ggttctgctg ttcctaacag ttataggaag taattttatt 49320
tatccaagaa attcttaatt ggaaggatgg gtaggagaga aagaaagaga caacggccag 49380
gtattttttt tttccagagc tcttctccaa atatcccttt taatagttat ttctgctcag 49440
gatgaccttt aagtttacaa aggaatttta aatccccttt tcctctaatg tttaagcaga 49500
ggaaaaaatt gttctccatg catctttcta atcaggaaga cctttaaaat tgtgcatgtc 49560
ttttctgtgg tactttacta agcacctgct cggcgagaga ctgtaaggga aacagtggga 49620
tagatacatc taaagtttct caaacttggc attattgaca tttggggctt gaaaattctg 49680
tgttgtggga ggctgtctga cacgttataa aatatttagc agcctccctg accttgactc 49740
actagatgct aataggattc ctataattgt gacatccaaa aagtctccac attgacaaat 49800
gtcccctggg gggcaaaatc acccctcttt gagaaccact gatgtggatg caacaaagaa 49860
tcaaccttaa ccttacccat ttgcatactt ttctactata aagttgtctc gcatagatta 49920
cgtagaattt ccaagtgtat tattttataa acgtgccttt cttggttatt agtagatatt 49980
tcttattttc aagatggtga tcatggtgtc tgtataattt ctgttagaat atcagagtta 50040
gtgtacctgg atactaagga gaaagataga aaagataaaa tatgtatctt caacattttg 50100
tattctgtga tgaccctaaa agctaatgtg ggagttctat tgactgttat aaactattac 50160
agaagaaaaa gtgaaagtgg agagttaggt ccactgtgtg tgaacaatgg gaggtctat 50220
ggaatgatgc tttcaggtgg gcagaggcct cgtggagggt ggccccacac tcaccccag 50280
aatccagaag ccattcccac aggaacttca gagggagagc atttacattt acaggaaaga 50340
aatgatactt ctgagcctac atttgccaca tattcattaa gtgcatttgt tcaggcatta 50400
cagtaaaatg ccatgggctg tgcttttcct cagctacagc ctctctccct ccaacctcac 50460
accctgtctt cctgtcatgc ctttgtgaga gcgccaccac tctgcggtgc ctggagagga 50520
acctggaaac gctgatagtt cagtgcatac tcggtcatat ggagacagca ggaaagagtc 50580
aggggccaac ttgaaagatt tggggcctgg ctgtggtttg caggctgtag acaccaaact 50640
atactttata gaacatgtga aaatatagcc tgtatgggtt actctcttac tgattgtcta 50700
gatttgatgc cttacattaa atcattttaa aaccaactct agaagtctac tcaaatcctg 50760
cattttctag tggaatagtg tcccatgcat aaaaccctgt ttcgcatagg tgtaggctag 50820
cttacagacc ctcttaccat gttgagatta tttctagaac tcaggctact gaaccatctg 50880
gtgctctgcc tgtgtctttc acatagctaa gaagaaaatt tccatttagc tttctgatat 50940
cttgctgtgc aagtcttcat gctctttttt taaaaaaaat tttaattata aattctttca 51000
agcatataga aagtttaaaa attgacataa cagacactat gtactacaac acaaatttaa 51060
taggtattag catcttacct tatttgcttt tgatagctct ctctctctct ctctgtcctc 51120
tctctctctc aaataaaata gttacagcta aaaccccact tatccatcac tttgtcctcc 51180
ttccttctct taggtaagct gctactctga actttcactc cccaagatgt ttttatattt 51240
ttactacata agaatgcatc tataaatact ggcattttca agaaagttaa tttttactaa 51300
cttcaaccca aatttgccat tgtataggat ggctcctttt cagggcaaac ataggttcaa 51360
actggacgcc attgaagatt aactggtact cactttgatc ctattgattc ctctttgctg 51420
tctagataaa aatgtatttg tcacaatgta gtttgtacta actcttatag caaaaaataa 51480
tattcagaga aaaatctagg ggaagtaagt atgcagacta actcttatat cttgtccttt 51540
cataagaaaa atttcccatc attaaacatt taataatcta ttatactgcc tataaaattt 51600
ccctttaaag tttttccat tctaatttca tcacggagct cctaatagga gaggctggca 51660
```

gaaaagcaca ggaaccttcc tctaggccaa gcttgtccct gcctgctttt gactcagcaa 51720
ggagtgccag ggaaggctca cttccctatt tttcccgaaa gccacgcgtg aagttttaaa 51780
aatcaaactg tgttcccttt cacagaggtg ggtgacaaga gaagcagccc catgggactt 51840
cagaaactct caccaatcct cctctagagt gaccagaaag taccccttcc tatcctaact 51900
aatgtgacac gtccttctgt gtggtccact ctatgcaaaa ccagaaattg acaagaatga 51960
ctttgattgt aaagtacaaa tagcttacca gttagtattc ttagtttctc tttagttata 52020
aatatcaaat gcaagggact tgaaacccag ctgatagaag tgtaactatt tttaatcaaa 52080
cattttctga aagctggttc tcacatatgt tgtctttcct gagtgtttta tgttgagtgg 52140
aagacgtgtt gaaatgttta ggtcaacaac agctttttttg ttgtcccctt ccactactga 52200
tttaaaaaaa aaaaaaaaag gctccactct tcgtgccttt ccttaaaaat cctaaatgtt 52260
aagtttctgt cgaggtcgaa tagagactac ccgatctgct gtggcatttg aggatatcac 52320
atatccttgc ataatctccc tttcctttgt atagacttgt ctgattcacg tggtttggat 52380
ctatttggag tactaattta aaataattta acctgactca attacttaaa acaacagggg 52440
agaaaatcac caataatcag ctatgtgtgc gtatagaacc tagttcatat catttattag 52500
gatttccatt gaacccctaa gctgttcact gcgcaagtat ttaattaggg aaaataacca 52560
tacccctatg tacattatac tgtgtgtttt gtaaaccatg agaaaagctg cttattgaac 52620
agaagggctt tgtaacctgg ggtctatgga tccctgttgg tggtttcaga cgctttttag 52680
attcctaaat ttatatgcag acttacacat gtatgtatgt gcatttttc ttgggagatg 52740
agtataactt tttatcatat tttcaaaggg aaagatttaa catggttaag aatcatgcta 52800
tagcttgtat cactttagct ttttgatagc agctatcttt ttaaatgatc tatgtaatat 52860
tttttaagat tttttctttt tttattcttg catttttaat caattttaaa tgaagtgaaa 52920
tgctttatgt tgctcaacac atagcccaga aagcctccct tcagttttcc agtaggtgga 52980
aaaaagatga aactgaattg atggccaaaa gagttgaatg ggcttcactg ctttttcttt 53040
cttgctcttc catacatttg cagattaagc atgaagctca ccattagtga ttgaagccaa 53100
aagagagaag gcaattcatt gaacaaatat tgattgagca ctgtgtgtca ggtactgctc 53160
tagacaagca gacaaaactc cctcccatat gtaaccacaa ttgagactta taggtaattt 53220
ttgttgcaag tagttcagaa gttttgtatt ttgtcctaaa acaaaatatt actttgtaaa 53280
atattttttt aaatagccct ctaaatccgc tgtatatatt caaagtcact attataaagt 53340
ggagaaagct actcattaga gaaaacctat cacacagata atagctggga agcttaaccc 53400
ttagaaatta ttaggagaga tatacactcc ttgaatttaa gaactgtctg aattttaaat 53460
tttgttttgt cagttctggc ttggtgacct tcctctattg gccattccca tttttctgcc 53520
ctccactctt ccttgacaca tgattatttc tgggaccact gttcaatccg aaacaaatct 53580
ctggtggctt cttttaaacc cagtggtctc agtctgaagg tatagtgggg caactctcgg 53640
cattccattc tggagtcttg acctcataaa gtcacatatt ggcaattcgc cttgataagg 53700
tcaagtgaac acacagaagc actaagatcc ccacatgtgt tccactgcct cctccacaga 53760
caagttgttt ttcaacccat ctgcaggtcc agaggtgaag ctggaggcta ccctgcagtc 53820
tcagtctttt ctccccaccc tccagatgga ttccaaataa gaagcctcca gactaccttc 53880
acctccctgg ggtctcatgg acatgggctg gaaagtgctt gcagactttc acagacaatg 53940
ctttttgcct ttaggtcccc ttctacccaa cagaatgatt ataataatgg cttgatgtgc 54000
ttacttacat catctcattt aatcatcaca gtaatccact gatgcaggtt ctgtgatccg 54060
cattttataa atgaggaaat tgaagcctag agaaattaag aagcttaact tgggtctcac 54120
aggcttttag agatggagcc caagatttca gttcatgcca tatggtttaa gggcctgcac 54180
tcttaaccat cgtggaatac cattcattat cataatgtta aggatctgtt tctactttgc 54240
aggaatcttt taaatctatg gttcacaatc atgggtgggc ttcagaatcg tccaaaaatg 54300
tttcaaccac agagacgcct gggaaccact gcacaattgc taaatcacag tgcatcatct 54360
agccaatctt ggattctaca ataattctat gacagtttct aagaatacat gccattatat 54420
aatggcaaca tattacaaca gataattgaa tagatattca atgtataccg tggaatagaa 54480
tattatgcca ctgtgtttta aaaagataat taagaggata cggcacaacg tggaaaactg 54540
cttataacat aatattaagg ataaacctca gctgggcata gtggctcacg cctataatcc 54600
caacactttt gggaggccaa ggcaggagga ttgcttgagc ccaggatttt gaggccagca 54660
tgggcaacat agtgagaccc tgtctctata aaaaaaattt tttaaagaaa aaaaacagga 54720
tttcaaatta ttgctaatat ttgtagtgat tacaaatatt agcttctttt tataaagcct 54780
gggtggaagc atatagatgt atctttagct ttatttattc atccatgact taaagaagat 54840
tggttaacac ctgactccac cttgagtagc agaacattgg actgaaggaa aaaaaaaaat 54900
gtgaagctgt cttacttagc tacagtttcc agcatgtttt ctcaggagct ctatgggaga 54960
cagtgcagtg tggctctcaa accttactac ccataaaaat ctgcttcaaa acctacttaa 55020
aatatcgatt ttattccatt tccaaagcct ggtagagtag gtaattctcc cactacaata 55080
cacttagtaa cactagaaaa aaaatataac attttcttta atgcatagct gagtttatta 55140
gaaagtatga aaactactgg ggcgccgtaa gacaaaagaa aactttggga aaacacaata 55200
ggatatgggt ggactttggg gccacagctg ccctggaaat atttgctgac ctagataatc 55260
aagaagcttg gtttttaaca gcttcctaga gacaaaagat aaggccttgg gcctttccaa 55320
tctgaggaat tgaaactgca atcacccact taattcagga ctctctcagg gctatagttt 55380
ctgttgatgg gtgatcctca ggcagagagg tgatgaggaa gttgcctgtc tcagcctagg 55440
gggaatcaga agataaaatg cctctcttga gaatcaataa ctacaagccc aattcttaca 55500
gattcagagt taagtgtatg ctccctgaat agagtaggaa accccaagtc tagaaatgaa 55560
tatgaagttg ttccacattg gggataccct cagagccttg cagaaaagca taaaaatgta 55620
tgtggctcaa agaatcatga gctcctgatc aaaaattaga aaatatgtat gcaaataagc 55680
cactctgttt aagaactggc agaacaatga aaagcagaat taaatcctca gaatcttcag 55740

ataattgaat taccaattgt ttgatatata aatacaaaat aagtttggaa tagaaaacag 55800
accaggcaga atgaaaataa aaccaagtcg aatttttaaa attaaatgtg cgacactgaa 55860
aatgattcaa catagttaac atggataatt tgaaagaaag gatgagctgg caggtacaaa 55920
agaaggagta agaggtgtct agttgaagtc ctagaaagaa gtattacaac tgcagatttc 55980
tggctctaca tttacagttt ctaggtctgg ggtgaggttt aggtggttgt ggaccacact 56040
tttaggtttt aaagcattgg ctctgaagtc atttggttca gggctgggca tgatgttggt 56100
tcctagtttt gcgacttgca gtgttactta acttggctaa gcatcagctt ccttatctat 56160
gaaactgaaa aaataatacc tatgtcatag gatatatgtg taaatcaagt tattcagtac 56220
atgccacaca gtaagcactt aaaaatgaca gttgcttcta tttttatata accaaataca 56280
ctactacaaa tattgagtgg cagctttgct ggttcattta tggattcatg gagctacaca 56340
aaaccttcac cagtctatta atttattttt agtacagtct ctctaatcag tattgcttcc 56400
actgtatatt gaccccatat cctttcttct gtctggggtt aagataggag actgagatca 56460
gctgtgtcaa aagctttctt ttagatgtaa cacatgattt cttatgaaaa tctcataggt 56520
aggataccag aaaggaattt attaaattct tgttaggctc cttagttgta ttagttttgt 56580
agggcagtgt tttaatttag gatacttatg atagagagca ccccttccat aatcaggaga 56640
aacacaagat ggaaataaag attttattac ttagtggtcc tggggagcac acagcactcc 56700
agggaaggcc acacaccaag atcagggagc atgtggagaa agagagagaa aggaactcac 56760
gggccattgc ctttactggg tccaggacat tatccaaaca ggtttcccac agggagtttt 56820
aataggtggg tttcaagcaa gcaggtatga gttccaggag gtcacagtgt gatgaagaag 56880
aagtcactgt ggcatatctg cacagtccat gcgaggtgtg agcatcagtg aagaccagtc 56940
aggtaggcta tatgtagctg gcccatgggg aggcgatcac caggtggcaa ttgtataatg 57000
cagatatcta gattggccac atagaggaac cgggaggagg tgtagtactg gaaactgcgt 57060
cgacggtcac tgagccctgc tttaggtatg agaaagtcca gcttatattc agaatggatg 57120
caaaggcagc ataaaattaa aagcattcac tgccagctgc cataacaagt taccacaaac 57180
taggtggttt aaagcaaccg aaatttattc tttcacagtt ctggatgcta gaagtccaat 57240
atcatggtct tcgcagggcc gtgccccctt tgaaggctct aggcaataat ccttccttgc 57300
ctgtttctag cttcttgtgg ttgctgacaa ttcatggcag tttgtgactt gcagctgcat 57360
cattccaatt tctgtctcca tcttcacatg aatactgtct tctctctgtg tccctgtgtc 57420
caaatctccc tcttctttct cttagagacc tgtcattgtg tttacggcat ccataacccct 57480
atgtgacctc atcttaacta atcataattg caacgacccc atttccagat aatacaatat 57540
tctgaggctc tgggtagatg tgaattctgg gggtacacca ttcagcccac tacaaacata 57600
ttgtggcatt gaggttataa gccagtagcc ctacaagtat aggatgatgc ccatttcagc 57660
ctgataatac actccgaaag agatcacatg gtaacattgt tgcctcagtt aatcatagaa 57720
atactcaatt agtatagaaa taacaagaaa aatgctttgt tattctcatg tgtcatttat 57780
cgagttctta ctatgtatct gttattgtta tccttataca cttctgcaca aaagatggtt 57840
ttatcccaag ttttagctta gaaactgagg cttaatgagt aacttgacca agaatggctt 57900
ctctttctcc atttccttcc tctcatggtg ttgtttata tacataaaat aatatatatt 57960
attctattaa tatagattaa tatatgtatt tatatatgta cattttatat atgtataaaa 58020
cttatgttgt aagttttata tacatataat atatattctg tttatatatt gaatttatat 58080
atatataaaa cttaacacta ttaagtctta acactattaa gtttcatggt gttgagttat 58140
atagttctat taattatata tatatggggg gaggggtgtt tctccagcct agacttccac 58200
tttgaaatgt agtctccagg ctcagccact tgtttgacat tgccacttgg atgtctggta 58260
gacttaacat attcacaact gaatttcaaa tcttccccac caatctgctc tgcctcatag 58320
cttccccttt ccggaatgtg gttgctccat ccttccagtt gtacaggccc caaatcttgg 58380
agttttcctt gactcctctc ttcttctcaa atcccacatg taatccatca ggtagtctta 58440
ttggctgtgc cttcacactt ttttcagaaa ctaattattc ttcagcacct ccaatgctgc 58500
caccctggta taagctacca tcctcagcat ctttcttcca gtggatttta gcatccatga 58560
atgattattt cctaaatcaa ttatcaataa ttgttataaa tttgtgattt ttctatttct 58620
atccttcctg tcacttttcc tggttttttt aaaagagctt tcttcattct tctttaaaaa 58680
aaattaaaat taacatcagt atgaactcgt ggattctttt gttattcatt gtactataag 58740
ccattcctgt cattattcat tttgattctt aaattgtcct attcttggcc agtgggagtc 58800
tttcatgcta gctcctatgt ctttttgata tgtccctatc tttttttttcc taacactttc 58860
cttatttctg aaattataag ctattctagg ttcaccctat accttcacta ccccagtcca 58920
ggcatcgtct gtctctctaa gaagtcctag ttccttttag caggagaagt ttttctagac 58980
tctttcatta ggcagagcta caaaattgat tattttaaaa atctcgagtt catacactaa 59040
ttcataattc taattccaat ccaacactgc aggcttcttc cctccctccc ctcatcccat 59100
acttgtattt ggatttgaga tatgaaatgt gtatctcata tcccatattt cttcagtaac 59160
tgaaaaccct catcatcaat gatagcagca tgcttactca ttttgctcag tccaataatt 59220
cacacacatt tgtagaaaaa tagaatagtt ttagaatggc tataccaata tctgcaagct 59280
tactatattc ggttcaaaat ttctttgtag ttcttttgac caaatgtatt caacagagag 59340
cattcagtca gagtaatgtg tacaaaagtt acttggatta aattttttcc ttctgtgtat 59400
ttatgtttcc attgagctat agagttaagc tattttttta tttctattcc attttagggg 59460
ttttcccatc cctgttgatt tagtaatttt attttttgaa tatcaacatt tgtcagtttc 59520
acatctttca tttatatgag attaactaga ccctttagaa atcattataa agtcaggttg 59580
ttctattaat ttttaaaata ggtgtataaa aaccaaaggg atcaatcaga gccttgattt 59640
aaagtacgtt ctgaattatt tcagacaccc gaatccatct aaacccagaa catttcattt 59700
gtagatttca aagacttctt ggccttccta ccttccttac atttatgtag catgtgttta 59760
tccaaggcac accacctgcc aggcacagtg ctgggctctg tgttgacaca catgaatgtg 59820

```
atcatctttc tgtagccccc aggttacatg gcaccacagc caagatacag acatgtgtga 59880
gagagtaatc agggtattac tcaggatttg ctggaggaat ccctcttcaa aggatattct 59940
aaattggtgt gtgtgtgtgt gtgtgcgcgc gcgcgcgcat gtgtgtgtgt gtgtgtgttg 60000
aggtggactg ggagaaaata taccctcctt gagatcacct cctggctaga tagtctctcc 60060
cttctctgtt tgtgatagaa tgcaagtgga atttgacagg tgtacttctc aaactagaaa 60120
ctagagagct gctatctcag tcaccgtaag aagaacgaat cacaccaaac ctacatcatt 60180
gtcttctatg acaggatttc tacaagggtg agatagtgag atgctggaga tccctgtttt 60240
ggtagttcag ggattcacag catctcccat gctggtgatg tcaacctgaa gagatatttc 60300
tagtgatagg ttccagggat ctttcaccga acactttaat atattttatc attgtcctga 60360
atacagccct ataaggttct tactaaattt gcaggtgaca tgaagtgata gacaacaaac 60420
aatttgaatt caaattaatt ttgaaagctg agacaatgga cccaattctt cagcaattat 60480
atttaaaaga tataaataca tgcagaaaac aaattacaag tacagagtgg aagagagatg 60540
atttagcagc aacgcatgca aagagatcta ggagttttag ttaataacag actcaacatg 60600
aaccagcaat gcaatgtgac tacgaaacaa gtaattcctc attaggctgc attaatggaa 60660
acacgataca taagaccaag gaagtggtgg tctcatttgc actctgtgat gaaaggccac 60720
accaggaata cagtgtagta ctgctgggca ccacatttgt agagaaatag aaaccaactt 60780
gagtgtcttc agagcagaat ggtgagagaa ctcaaacttc attcaaatga ggagtagtta 60840
ctgagcctga gaaatttata gcctggagga tttggttgtc ttcaaatata tgaatggctg 60900
tgatggggaa aaaaggatta aatgtgtcct gttgtcacaa gaggatgaaa taaggagcgg 60960
tgaatggaag ccccaggcga tagaggtttc agttgctgct gacagtcagg gctgtctgag 61020
gatggaatgt attgcctcag gacaaagtga gttcattgtc actgaattgg ttcaaaaatg 61080
accaggaaga ctacagggca gggaagctaa cttgtattac aataagtaat ttttttcttt 61140
tttatttgta tgcccttaga aagatgttta cttaacttga taaatctgta acaaggata 61200
atcaagattt ggttatagga caattacaat tttctgacag atcccccaaa ataatagaaa 61260
ttaatagctt acacactact ttatccaagc tgtagaatgt ataacttact tcatatactt 61320
tgtgtttagt cagtttatac aggctgaaca acataaagtt aagacataaa agggtttttt 61380
ttgcaggttt aaagtactac cattttttcca aattactggc tgatcctaga actttgagaa 61440
actttggtgt ttccccccctt cctacttttg tttacatcat agtacgtaga tggtacaata 61500
ttttggttaa cctcagtctg acacttagat tgtgttctct tttgccctat taatgtctag 61560
agtgtaaaaa gaccagtaaa gtagcaccaa gaaatcaaag aaaatttccc agttctactc 61620
acttgtaatt ctcttaattt agtaggggct ttccacagga aatcacaata gaatagttga 61680
aatgctcttt atcttatgta ctttccttct gaagaaaggg acagttaatt ggctttgttc 61740
ccaacatggt ccaaattttt atacagggca gctgtattta ctgtagtaca aaggagattc 61800
tacaccgaat gatgtctcta agtattttca ataattaaga acctaattgc ttctctagcc 61860
agtgggtttc agtgaaagtg ataaaataga ttttctaagc aagaaaaata cataatgagg 61920
gtggttgtat acctttaaga aaaaagaaat cagtgatatt caatcccaag taaatttgga 61980
tatcacagag tcagagatat tcttaattat tttaaattaa atttgagtca aaaacaatgt 62040
gtttaaagac tttaaaatca ttttaaaaga atgttagaaa tttacattaa taatttctat 62100
tttcaatgaa taaatgagaa acctagaatt tttgttttgg acacactttg atcatatcta 62160
atgtgttcta cttcctctaa ttattttttat gttattagga tttttaaaaa acctcttaga 62220
ttaaaatttc tcagtgtatc tttgtttgcg tgtctttttt tctctctcca cttatgtaat 62280
tgaagcattt ctgacatatc ctcagaacac acagattctt atcaggactt aactacccag 62340
ggcagggccc tccttggatt tactgaaaat aaacactttg ggtactcagt ctcagtttta 62400
atgacaaaag ttgaaatctt tgcctaacat gaaggttttt ctttgtctct aaattttgat 62460
tttccatgct atcagaggtc ccattgcaac aggtaatttg agtgttgatt gtatgttttc 62520
tcagtttgta attgtggtga aatgtattta aaatgttcac aattttaaag ttaagtttac 62580
ctataaggtt ctttccaact cagaaattct gtgatggtaa actttttttt tgtagtttga 62640
aaatgtgatc tttggatgtt gtcttctgtg tcctatgcat atgactagga atcatagaat 62700
tttagagctg gaaaatgagt gagacattat ctagtgattc tgcaaatgaa gaattaaaag 62760
gcccaaagag gttaggtgac tcacgcagta tcatagcagt acctggtgac tagaactctg 62820
gccacctact gcagcactaa aaattaaccc aaatgttttt atctgaaaat ttgcagactt 62880
caggctaggc ctgtaaataa ataaataaga agttaaaaca tatttgagaa aggtaagctt 62940
aatatgccac atttgggaaa agatgtgcct gacacaaatg tatttggtgt aatagagaac 63000
attcttccat tataccgaaa tgaaaatttt gaacattctc aaaattcttg aattttgaga 63060
catttatgtt aagcacagtt tattcagact ctctataaac tgtgctgtca tccctgttgt 63120
tttctccaag cggaatcata cttaaattac aactttaggg agtttaatgt ttcctatttt 63180
tgcactcctg ggatattttt cagtgactca gaagcataaa ctgacactgt gaagttaagg 63240
ctgtgttcca gtgggaatga cagcatgtct ctaggacgct gatggagaaa gccctcctca 63300
tgcagctgtc ttgcggagtc tccctgctgg tggtgaatgt gagatcacac agggccagca 63360
gccagggcag tacatctctg agtctcgctt ctggttttac ctagagccaa cccttggtga 63420
ttgtttgcct ccttggccaa gcccccacac aataggaaaa ctgtgtctca aggtcgaatt 63480
ctattaatac ctgagaggtg aatttataaa catttaagat gttttagcat ctctgcagcc 63540
agtgtctctg taaatgaaaa ttgcttctcg aagtagaaaa gcataaggga gtacaagggc 63600
ttcaaaaaaa aatcctttta ggaataagaa tattgccagg gttaggtgtt cctaactggt 63660
ttcaagcaga ggagttagta aaattccaaa agttaatcag cttcatgtac tcaacttttg 63720
ctaggacttt gcagaaactc ctcagggatc cctcagagtt agattaggga gatgaaccct 63780
gccaaaggac agtaaccatt caacagctgg atagcctgtt gggccccact gtcctcagcc 63840
tttcctaatg ttgcctggga acaccacttt tctgctccct cctttttcag tgtagcctgg 63900
```

```
agacaattat ttatttattc actagctctt ttaacaaata ttgattaagt aggtactggt 63960
tctgttctag gggttggaaa aacagagaaa attaaatagt gcctttgtcc tcaggaaaca 64020
atccatttaa tagagagacg aagatatgcc cttaaatacc tgaatcacca gcataaccag 64080
cacctgcggc tgccatgtgg gaattgttga acaagataat ttttgaatga aagagtaaat 64140
gacctaattg ctctaatgta aagataaatt tctaagagtc ggctagttgg gatgattata 64200
gaaggattca agggagagat gtatgaaagc atagatctaa cgagagaaaa cagcacagca 64260
gaaaaagcag caaaggctca aaagtagaaa tctctgagat cgaatggaga atagtaaata 64320
atttgctgga atgaagagca cgtggaaggc aagggtggta agaaggctgg agatagtaga 64380
taaggccaaa tcatgcaagg atttaaactg tatgttttta gaaaatgggt aagctataga 64440
aattttatat aggaatgaga aattatcagc agcattgtgc tttaggacta caaatctggt 64500
agcaatctgc taactgatat tggaagggaa aggcccagag acactagttg gaagaattgc 64560
agtgattctt catgagctaa actaagagtt caagctaaga tagataaact aaggaatgag 64620
aatggaagaa agggacaaac tgagattttt ggggtggtac tcagctgaag tttggcaact 64680
ggttaaacat aaaagacaag agacagagag gaatccaaga tgaaaagacg attttgctgc 64740
actttttag aaggcagcat ttgtatttcc atttccagga ggtggagtag ctttgagaag 64800
aaaggtgatt agttcagttt tgggcacatt gagctgtaac ggtattatgg acaggaagat 64860
accctacaaa cagctggaaa ttttgcacat gcttaaaaag aagatgagaa catgagattt 64920
caatttagcc atcatctgca tggagtagac aattcaatcc aggagacttg ataagaatgt 64980
caagggagtg atcacgataa aggtgagaaa tggactgagc atcgatctct gctgaaacat 65040
ctctttgagc aggaagaaga gtgccttagc aaaagcagcc agtgaaatgg ggagttgggg 65100
gagactttca aggagggaat ggtaacatat cccattccca aagaggtcaa gtagggttga 65160
ggaataataa atggctagtg ggtttggaaa catggaatct gtcatgacct ttacacactt 65220
tttcatttga tggatcgggg gattacttac atttgcaaag ggcctcagat taagtggtaa 65280
tggttcagca ttagtaatta gagattttct tatgtacttt gtagggagaa atagaatggc 65340
attcaaacag gtttaagttc ctcttaagaa cctttctcat tggattgtag agaagagcaa 65400
atgaaagaat cgttcagtat ttcttggtat catgcccagc ccataggttt caattaatgt 65460
taacctttac agttgtcatc atcattatcc agaaggggct ttttttaga ataaggaaga 65520
aaagcagtgc tcatctgtag acaagggaaa aaatcctttg gaagagagaa agagaagggt 65580
aattcaagaa gatcaggaat aaacattaag ggtaaaaaca gaatggttag ccctggaaaa 65640
gggaacaaac atgtttctct gaattaggag aggatatgtg aagtaacagg gatgttttga 65700
gcctcagtaa cagtaaggag aagagagagt gaaggaactt gcacgcagtg acttctacct 65760
tctcattaaa gtcaagagag aatctcaact gcagatatct gcttaaggta ggaagagaga 65820
aataagaatt gctgtcattt attttgtggt tttgatatag caaaaactgt actagacact 65880
ttacctcctt taatcctcag aaaacttaga gattatatat ttgtattcac attctacaaa 65940
tgagaaagca agtaaaaaag ttaagtaact ctcccagagt cccccagatg gtcatgggag 66000
cccagattca gagccagcta tgtcttactc caaagcctgt gctctttatt gaaacatact 66060
ccactagttt gagatcttgg aggtgtaccc ctgcactact atcaggacta cccctgataa 66120
agtcctgtgg ggtagttgtt cctatccatc accacagtga atatcaaaga agggccctct 66180
catttgagga ataacagaca gtgaaattga gatggaccag aaatgcaaat cctggatccc 66240
tgagggaaac aacaaaatga agcatagagg aaatatctga gtctggtgcc aagactcatc 66300
agtacaagag agaatgccta atgggcaata gcagttaggg tgggaaaaga gaagtacaag 66360
ggggtgtctt ggaatttgag ggaagaactg ctgccttagt tttcgaagga tgattattcg 66420
agagttgccc tcccttcact ttctcacagc tggcagaatt cctacctctt agtggttaga 66480
ttcctttta ccagatactg tagggccagt gcagcaacca gagcccccag ccagaaaccc 66540
tgcctccaat ttagaccagt gttctgctct ggtgtgtaat aatcatattc taatttaaat 66600
agcctattga gatgctacat tgatatctag ccttttttt tttttttt acttttaagt 66660
tttatgtgca ggatgtgcag gtttgttaca taggtagacg tgtcatgggg gtttgttgta 66720
ctgattattt catcactcag gtattaagcc taatatctgt tagttctttt tcctggtcct 66780
gtctgtcctc ccaccctcca ccctttgata ggccccagtg tgtgttgttt ccctctatgt 66840
ggcaatgtgt tctcatcatt tagctcccac ttataagtga gaatatgcag tatctgtaat 66900
aatgggtttt ctgttctcgt gttagtttgc taaggataat gacctccagc tccatccatg 66960
tccctgcaaa ggacatgatc ttgttctttt tcatttctgc atagtattca attggccatt 67020
ttttaaaaga caaaataaca aaaaaaagag tacagttatc tccactcctc acccactacc 67080
tgccacctaa cccaaccctt tatattttag taattgcctt ccataaataa cattttatcc 67140
tgtcttaatt atgcattcat tagtgtatta attattgaat gcccacaata tgacaagcat 67200
tatttcaagc actgaggata cagcagagaa taaatcaata tcctcactgt ttttattcca 67260
tttttttaag agacagggtc tcaccctgtt gcccaggctg gagtgcagtg gtgcgatcat 67320
agcccaatgc caccttgaac ttctgggctc aaaggacact cctacctcag tctcccaagt 67380
agccggaact acaggtgcac actaccacac ccagcttccc cactctgagc ctccatccta 67440
atggagaaga gaggcagtaa tcaaaataca tgaaatgtgt agttattaga tggttattaa 67500
tgtatagaga aaaattaagc agagaaagga gcaagggatt agggggtcaa agttttggta 67560
ggggtagagt ggtcagtgaa ggccaccttg agagatggca attatgtaaa gcctgaaagc 67620
aggtaagaaa tatacaggta tacagatgtc cctggaaaga gtgttctagg cagaagaaat 67680
agaaagtgca aaagctaaga ttgctcacct ggcaagttca agaacagtga gaaactgatg 67740
tggctgcata ggagtaagaa gaaaaaggac ggagaggaca tgagatggtc agatagccta 67800
gggccttgta agccattata acgattttgt cttttaatat agagaaatat taaaggtttt 67860
tgttttaact ttttaacata gaaaaatgta atcatataca aacacagaat agtataatga 67920
gccccttgc actaatcact cagctttaac agctttcact ttgggaggat tttgagtgaa 67980
```

ggagtgataa gacatggcat ggcatgacat ggcttgaaca ggatcactca ggaaaataag 68040
ccatggggag acaggacaag cagaggaaac acaggagcaa aagcttagga gacagatgat 68100
ggtggctgga aaccaaggga gtagcagtga aggtggtgag aagtgatcag atagaggata 68160
tttttttgaa gatttgaatt aatattatat atattatact ttaaagaaga atttgaaaat 68220
gcatgtagtg tagtaaagat ggtaagaaat acagaaaaga ggccaggcac agtggctcat 68280
gccagtaatc ctagcacttt aggaggctta ggtgggaagc tcccttgagg ccatgagttc 68340
aaggccagcc cgggcaacat agtgagactc tgtctatatg gaaaaaaaaa ttaaccaggt 68400
gtggtggcat gcacctgtag tcctagctac ctaggaggct gaggcaggaa gattgcctga 68460
gccaggaggt tgagtcttca gtgagccatg atcataccac tgtactccag cctgaatggc 68520
agagcaagac cctgtccctt taaaaaaaaa aaaaaagaaa gaaagaaaaa gaaatataag 68580
aaatggcact agaatatcag tattatatac attattttct gtttaaaaat ccacacaact 68640
ggcaaaaaaa agtgacatac atgaacattt ataagacagt gcatttataa attacaaact 68700
ctctgctcct cccatattat tttgcagtga tgaatgatta ggccatccaa acgtaaagat 68760
ttatttgtaa tttgctaatg atggaatgac accttttgag gtacaagttc ccagcaattc 68820
ttggaattag caatagtgtg ttacttcttt ctcgaaagaa gcatgatttg taagcagcta 68880
tatctgatgt agtaaaactg aaagttaaaa taaaaaagga aaccgtgggt caaagtatga 68940
gggaaaagat aaaactttca gagcaaagtt atacatagag attttattca tcgtctctgt 69000
gtaactcacg agaacttctt agcttattta ttatgtcaaa tggcagtttg ctcttctaat 69060
cccagcctga tatcatgagc catatatgct gttggtcatt caaaaagggc actaaacaag 69120
gtgaaagaat gtcagtgaat caatgcaact ccatgagtac taccaggaaa agaaaaacaa 69180
aaaaacatgc cattaatagt gggtcaaaga ggattttttt catgccatcc cctaccttac 69240
tgagaatacc acatttatat tcagaagact cataatgaat tccagagtcc tctgtgtctt 69300
ggctgtatga ctttgggtta tgtcactttg ttaatctgtt tgaacttctg ttttcttatc 69360
tacaaaatag gggtaataga gtcaactctg cctccctcac agggactttc ggaggcccaa 69420
gggtctttac aaataataga gaaggaaaga aactggcact ttaatagtac ctaccatgta 69480
ccagggactg cgtagtcccc acggcagccc tctgaagtca tgtgattggc ccattgtgta 69540
gatgtagagc ttaagactca gagaggttat ttaatctgtg taaggccaca gagctaggag 69600
ggggaaaagc tgggattgga tcactgacta gatcaacttg aaatctcctg cctgacagag 69660
aaagaagaga ctaactttac taagcatctt tttatgccag aaactgtgtt ggacataatt 69720
ctgtactatc ttatttggta aaggtcattc tgtccttcct ggttgccctg gttgctcaac 69780
aagtaataaa caataaagcc tgactgtttt tcatccctga gagaagaaat attattaaaa 69840
tagcgttatt ctctttcttt ctttcctttt tttttttttt gtgtgtgtgt gtgtgtgcgc 69900
ctgataatct gacttgcaac ctcacagaaa agaatgaatg catggtttca tgtctgtggc 69960
atgactctgt gaaggagcct tgcaagggga cagatacacc tctaccactt cttatagcca 70020
ttcctgagac ctctcttctc acaacacata ccagcaaagc caaacaaaca aggtgtttag 70080
gaataactgt catctatcaa cactctctag gcaaagtaga actatgaaac actgaggaga 70140
ctgcacacac acagaagaaa aaatgatcat gtcacatggt agcacagagc aatgggaaat 70200
gtgaatagtt tgttgaggtg gacagaccca agctcaagga ttgcctgatc gctccattta 70260
taggaaccat ggcaacctac cttatcttac tgagcctcag ttcctccagc tgtgaaatgg 70320
ggtacaacct ctgaccccac agggctgttg tggggattat ctgagttagc atgttccata 70380
aaacacagta agttagaaag tatagctgta taggctctgg gagtttaaca agcaagagaa 70440
atttgacagt aatcatttag ttactgaacc ctgtcttcat attgcgctag gttgcatatg 70500
gttttcctgc ctgtcccttc caatcacatt tcagaaaaaa ggaggcagta gtggagcctt 70560
gtgggctaaa gtggtgagga gggtgtcatg gaagattcag gttcaggtct tcctcttgaa 70620
gaatgggtgc ttctcaatag ggaagtggag gaacaacctg ggagttgtca gcttgaagaa 70680
aatcctgaga cgtgataatg ggtaggtcca aggaggtcct gagtaggact ctgagcacag 70740
agcctttctt tgcatgtgct gtttttcctg ccagcaagac cctctaaacc ctttggcctc 70800
acccaccccc ttgcctggtt atactcctag ccttctttca gatctccacc ctcagtactc 70860
cctcaggaag ctttccctga cctcctgacc cagtcaagtg cttcttatat atctcctttg 70920
taagctcttc tcagttacac ttttacattt atttgtgaga ttatttgatt agtgtcagtc 70980
ccactcttta attctccaca agaacagaga ttgtgtctgt tctgcattca ccatgcatcg 71040
cttcatcccc tagtttactg cagtgtgtgg aacataggac gggctttaaa tactcgctga 71100
ataaatagag aaatgttggt tattagtgag gtattaccta ggtgggagag aggatttata 71160
tttgggattg ctaatcccat taagtgccaa gcactgcatt aactcattta atcctaaaaa 71220
aaagtcctat gaaatcaagt gtattattcc ttttaacttt aaagacagag aaactgaaat 71280
ccagagatgt taaccatctt tccccgttga cacagctaag tggtaacagt cggtcttgag 71340
ccgggcagcc tggcttcaga cttcctagac tttaccacat tgttctgctg cttagtgcag 71400
tagaggacaa agtgaggtgc acacgtgaag tcaggacaga tttccgtttg aaaattgtgt 71460
atttcccct cctcttcttc attaccattg tcatcatcat catcatcaat cacttcacaa 71520
gcaaagctaa gcagaaaaga actacttcat gtatcacctc ttacattcat gttaactcta 71580
cattctgcca aagtgatttg gctgaattta cccaaagagc aaagtggaca tttcaaatcc 71640
tgattcctag aaaattatag actacatcct gtaaccacct ttgaagaacc agcatgtact 71700
ttaccccaac gtgacaaaat tgttttaaat aaaaagaggt aatttgacta agattggtat 71760
gatctgagac ccatggtttc tacatcttat tcaaaaagca gatccagcat gtggtcatca 71820
gtaggtattg agtcataaag ctcctggaag aaaatcttaa gtttcatcaa gaatgccaag 71880
aagtcctggg tgtctccaag aatgatagct gttactataa taacataatt actactggaa 71940
aattagcaaa gcccaataaa atatggcatg caaaaaaata tttaccctgg aaatagaagt 72000
gacctaaatc agtcgtttat tccagccacc tgcttttaga tagtacttaa aaaaaaaact 72060

ttcaggatgg tcatattgtt taaagttctc taggtggaag agtactttac agtaataaat 72120
taataaataa gacaagttaa tgcttttaaa ctactattat ttgttgagca ttgttgtaaa 72180
ggctttacat gcattaatgt atttagtcct cacaacaacc ctttgagata ctattatcat 72240
catctccctt ttacagatgg ggaaactgag cagtgcccaa agtcacacaa ccaggaaatg 72300
gggaactatc ttcaagccca gctggtttgc gcctaaagtc catgctctta cagtgtccct 72360
gggtagctga gtactcagtt ttgtcacctt tgtctccttg actttaactg aaagcttaac 72420
tctttttttt tttttttttt tttaaacgga gtcttgctct gtcacccagg ctggaggggc 72480
agtgtcttga tcttggctca ctgcaacctc cacctcctgg gttcaagtga ttctcctgcc 72540
gcctgccgaa tagctgggat tacaggtgca caccaccatg cccggctaat attttgtatt 72600
tttagtaaag acagggcttc accatgttgg ccaggctggt ctcaaactcc tgacctcagg 72660
tgatctaccc gtctcggcct cccaaagtgc tgggattaca ggcatgagcc accacgcccg 72720
gcccgaaagc ttaactctta ataagagcct gcaatgttca gacgctttga aaaaaaccta 72780
acagacacag ttgctctcta taaacttaat tttcaaagga accactattc aaagaagtaa 72840
aagggagact gaataaaaag aactaagttc tccgtagagc aaatgaattt ctgaatcgat 72900
tgcaaacagt tctccgtcat catgacatcc tatgaagtat ggtctccccc atgttagagt 72960
gccagtgtct tcatgtacca tataacttgc aaatagtatc actgtcttct cacacttcag 73020
ctttcttctt tcatcagtat tttggaaagt ccacaaaacc caggggggctc cctgtctgtt 73080
gccctgtggt ctctgtgaaa accagatgat gcgatgctga cttggactct ttcaaatgga 73140
tccaacagca catgagtcac tggcagcatg ttcagctcct ttacctcagg aattacccag 73200
ttcctcattc ttgccaccct tcagccgtga agtagtaata tgaaaaatac atgagtactt 73260
tttgaggata cctcggacct ctttccttca gtaccagaga ctaattgcct cataatggta 73320
acattgaaga gcccagttat ctgacgtgat tgggctttaa ttatggattc cttctggtgt 73380
cctattaaat tggaaagcct agctgatttc acccaaatga agcattacct catgccttgc 73440
tctcttgcat tcctgtgctg gtgtcactta aaagtgtaat tacatggttt atttcgtgtt 73500
ctttcactct tgatttattg ctgaagaagc ccctgtggga tatatcatca taacaaagac 73560
tgatttcaga tgtcaacacc aagaaacaca actggcatca gcaatctata gagagtatct 73620
agggacagca gtcagctttg gagaaaaccc agaacttctc attccccatc ccacatttaa 73680
gggcaaagac tttctccttg ttaaagggaa agcactgagt tcataattag ctttataccc 73740
agagtttttt tttttttaaat gttatacctg tttaagaaat tttttgtgct ttgaggtggt 73800
caagatcaaa tttaattagc aaccctcttc gtgattggct tgctctgggg accctgctct 73860
ttgagaagga ggttagaagg tttagaagca aacctttgca gcagttctag ccaagtgcat 73920
gcatacaggc cttgaggtgg agctgcacag ttgactgtag gggagtctgt cctttgttga 73980
cgagtgaaac ttctctccct gcaaaccctc tggggaataa tgccaactct gatcagcttt 74040
tcgactcttg gatgaaatat tgggttggga aaaagtccct actaagaaaa ttgtaccaaa 74100
accctgcttc aataaaatat gaatccattt aatgaagttt atagtagaag taggctagtc 74160
gaaaaggatt ctgtctcttt ccaagtatta ttggcaggtt tagggtcttc ctcaagttta 74220
tatcattagg acttccatca aagagttatc tttaggtgtg attcccatcc tccctccctc 74280
tctccctccc tccctccgtc ccatcgttcc ttcctccctt tcctccttcc ttttctacct 74340
ttctttttct ctctgtctct ctttctttca agctgtgggc tgcttgtaca gatttatgca 74400
ttttgaatta ctgcatagtt gagagatttg gggcttaggt tattcagttc ttcaaaaagt 74460
acagtatgag ctaaggagat agggtgacca actaatctag gttttggctc caaaaaccct 74520
gtgtcccaga aaacccctca gtcctaggca aaccaggata gtagttcatc ttgtgaggag 74580
attatagtgc atttccaagt atttagctga atttatcaca gggctctgac ttgataggat 74640
ttaagagggt taaaataagg tcttttctgt tatgttagtg tgactttagt cttggcactt 74700
tctcaaggaa ggaatccagt ctactacagg ggctaggtga ctaaggagaa gaaagaggat 74760
atctaagtgt tttttgctaa aaatgtcatt atctccatgc tatttccttt ttattgctat 74820
tgtcatcatt ttaaagacat accaataaga aataaattac tttaaataga aaactctaat 74880
tttatcaagc aacagcaaac aggggtgaaa tctaaggatt tgtacttaag gagtacactt 74940
ttttgaattg aagaaactta ctatgacctg agcatatgta actcaattat cagaagtcag 75000
agagggctac tgaaggctca ggttttctaa gagatgactg gaggccaagt cctcaggctt 75060
gagtgtttat agccaaactt cagaaccagg tccctggcaa gattctcctg tctaatcaca 75120
tggcacagag ttaggatgga ctgtttctgc ttacctgcct tcttcctttg atttccatca 75180
accaccagtt tctttctcat caatcattta cccagctttt gtgaccaaga attttgatga 75240
ggatgcagag gaaggcatgt tcttggttaa taggaaagga ggcttcatga caacacagca 75300
tcctctaaga tgtgttgctc caactttcac aatacatatt gtgttttaag tggcctgctt 75360
cttggagtgt atcttcccac tacacaaaac aaccaccatt aacattttca tttacttcca 75420
ccctgtcatt tgttcatcac atacttttaa aaacagggct gtaatcatgt atatacatat 75480
atgaataact ttatattgta ctttttatac ttactggctt atcataaaca tgtttccatg 75540
ttgctggaga gtcagatatg ctaacttttg gaatgtgctt cttatataga tctgcaacta 75600
tgtgggacca gcaaaggtta ttgttcagtt ggtcacaaat ggaaaaaata tccacctgca 75660
tgcccacagc ctggtgggaa aacactgtga ggatgggatc tgcactgtaa ctgctggacc 75720
caaggacatg gtggtcgggt aagtaggggt atatgatgct gtggaaggta ggaacagata 75780
gaatatggga tgcaggaact tagaatgaac aggccccttt cattagggac ctttctgaga 75840
ccctcagatg acctcaaaaa actgtgtaaa cttgtgcttt catttggact ccaggaagta 75900
aggtgaccac atgtctggat ttgcttgaga cagcccagtt tgcccctgac ctaattgttt 75960
atagcaccct cttctactct taaaggtggc ccaatttgga tgatcactta tatgtggtca 76020
cttgacctac aaggtatttg tgccactatt aatttttttt ctcctagaaa taaaaataag 76080
gacaaattta aagaaacata actatagcag aaatcatcac tttatgtata tttttacatt 76140

cttgatgatt tcttcagtgg atttatggtg tgaatatgta gggctttaag tgaatgcata 76200
aatatcattt cagctctctg ctgcttagaa agtaatgcac agataagaag ccaaggaatg 76260
caaatttatt ttggcattca ttaggcttga aatgtaaata aaaaatgacg tattcattta 76320
agggttgatc aaataaaaca agcaattttt aatgaatgaa gttttataga agtaatttca 76380
atccaaaaga attaagcttt taaataagac atatttattg tgctaaaaaa cttgtatttt 76440
catcttccta atacactaaa atggcacaca gaataatagt gtagcacaca atacaaactg 76500
atatgtgcaa tcaataattc taaaaattgt tgttttaagt ctttctttgg aagtccttaa 76560
atgcaaaaga agaaattttt aattttgttt aatttttaaa aacatttttc agggttttct 76620
aatcctggtt taaatagttt atgctgaaat cccagttaat ctggcttatt cactcaaaag 76680
aaaggaattg tttaaatcaa ctataattaa gctgatggtt gcatttcagg ctttgaattc 76740
cgatcaagtt tcaaattggt tattattttg gaggttgtgc tttcatgttc ctaggtattt 76800
ctttgtttga ctgtgaaaat atttaagtgt gaaaaaccta gagagagagc tttcttttaa 76860
gtcactaaaa tcggattctt ttgatgttag aaaaaaaaaa gactctagat tgaaggaaga 76920
agcttaaatt gatttgaaaa attaaagtgt gctttatatg aacttgtttc aacatcctct 76980
tgaaagactg gcgtgtctcc tgttgtatgt cacagtttgt tcatcatgtg ttgtggaata 77040
cccttccagt tcctgattga cctaggagag ctttgaaatg caggctaatt atcacagttt 77100
tagactgatt gatcacttag tgtttctgca gatttattcc agtagggttc tgtcagtttg 77160
aagtgtttat tttctttggt cttgtttaat ccctattata atcatatcct ttagaggaaa 77220
ttttagatga agtttttctac tagtatttat tattataaaa cctccagtat ggtgtctata 77280
atttggtgtt tctgtggttt gctttagtaa tctttcagga ttttagaaag aactaatggt 77340
tatttaaatt tgagacctta aagcgggaat agaagtaaat tgtttctaag gaaagtgagg 77400
gattctttca ctcttgtagt gcagtggggt gtgggctcag tagttgggag aactcatcag 77460
tcattcactc attcaacaaa cattttgagc atctcagctc ctgaatttga tcattatgat 77520
gtctggttgt agagcagtga gattttctaa ccctatataa tttcagtgag aaatttaatc 77580
aagatgccac ttgagagtta caatagactc agtagtctta acgtttttag tgtttcctat 77640
gaaaagtcag tactcacttt ctaatttgat ttttagctga gaaatttttg cttacaatgt 77700
ttcccaaaat acactatttc tttcataatt catttgtttc tatctctctc tatatataca 77760
tttttaagat tttcaatgtc aaaacttatt gcatttatgt agaaagaaca tcgtgctagc 77820
attctggaaa ccagtatttt attcccagta ctgttgctct caacagagga ctgggggcaa 77880
gttacatgac ttcttgggga ccttaatgtc ttccctcata aaatgagaga gtttcactac 77940
agaatcttat cttcaggctg tttacatctc cagaattctg gactgaacta acattacgta 78000
atattatgaa ataacatgag caaacttgcc acacacatat ttatggtatg acattgatta 78060
ttagtattta gttctgactt gtagataaga gtatcacata gagaaactta ggaagatagc 78120
caaaattatg tcttagactt gtcaatacta gaaatgaagc taagataact tgttttcaat 78180
aatacagatt aggcaaagag tatagattca caagcaagac actaatccaa cataccaaaa 78240
aagatatcca aaaaatggca atgagaagac aaataacttt taaattgtca aaaccaaaag 78300
aattactttc tttggagtgt ttatcttcat ctttccctca gtatgaattc ttaataaact 78360
tagtttctac tttcaaaagg attttatcta tgttataaat attcatgttt taaatagcca 78420
agtcttgatt ctggtttgat tgctgtaatg taggggattt gtcattttaa tattttttaa 78480
gttagcttta attttgtctt ttttaaaatt catttatata attagccttt actgcctttc 78540
ttggtaaaaa ataataataa taatcttagt ttggtgtttt attttaaacc ctgaatatca 78600
acaatttgtt tcaacatatt ttagttcgtt gacattttta ttccatggaa aatatctgtg 78660
ttccttgact ttgctttagg ccagttcact taccagtaat caccacactc tctctgcttt 78720
ctggcagttt agagacatgg aattttttttc caattaatac atatagcata taccaactgg 78780
taagcactag gagtagatat ataaatatca aaatacagag gagaaacaag gccttcttca 78840
gtgtctgttg ttccccttttt tgtccatgag ttctcatcat ttagctccca cttacaagtg 78900
agaacatgca gtatttgttt ttctgttcct gcattagttt gctaaggaca gtggcctcta 78960
gctccatcca tgttcctgca aaacatatga tcttattctt ttttatggct gcatagtatt 79020
ccatggtata tatgtaccac attttcttta tttatttatg ttattgatag ggaatttagg 79080
ttgattccat gtctttgcta ttgtggatac tgccgcagtg aacattaaca tgcatgtgtc 79140
tttatggtag aatgatttat attcctttgg ttgtgtaccc aacagtggga ttgctgggtc 79200
aaatggtagt tctattttta gctctttgaa aaatcaccac actgctacaa aatcaatgtg 79260
caaaaatcac aagcattcct atacaccaat aacagacaaa cagagagcca aatcatgagt 79320
gaactcccat tcacaattgc ttcaaagaga ataaaatacc taggaatcca acttacaggg 79380
gatgtgaagg acctcttcaa ggagaactac aaaccccctgc ttaatgaaat aaaagaggac 79440
acaaactaat ggaagaacat tccatgctca tggataggaa gaatcaatat cgtgaaaatg 79500
gccatactgc ccaaggtaat ttatagattc aatgccatcc ccatcaagct accaatgact 79560
ttcttcacag aattggaaaa aactactttc aagttcatat ggaaccaaaa aagagcctgc 79620
attgccaaga caatcctaag ccaaaagaac aaagctggag gcgtcacact acctgacttc 79680
aaactatact acaaggctac agtaaccaaa acagcatggt actggtacca aaacagagat 79740
atagaccaat ggaacagaac agagccctca gaaataatac cgtacatcta caactatctg 79800
atctttgaca aacctgacaa aaacaagaaa tggggaaagg attcctattt aataaatggt 79860
gctgggaaaa ctggctagcc atatgtagaa agctgaaact ggatcccttc cttacacctt 79920
atacaaaaat taattcaaga tggattaaag acttaaatgt tatatctaaa accataaaaa 79980
ccctagaaga agacctaggc aataccattc agcacatggg catgggcaaa gacttcatga 80040
ctaaaacacc aaaagcaatg gcaaccaaag ccaaaattga caaatgggat ctaattaaac 80100
taaagagctt ctccgcagca aaagaaacta ccatcagagt gaacaggcaa cctacagaat 80160
gggagaaaat ttttacaatg tatccatctg acaaagggct aatatccaga atctataaag 80220

```
aacttaaata aatttacaag aaaaaatcaa acaaccccat caaaaagtgg gcaaaggata 80280
tgaacagaca cttctcaaaa gaagacattt atgcagccaa aagacacatg aaaaaatgct 80340
ctcattatca ctggccatca gagaaatgcc aatcaaaacc acaatgagat accatctcac 80400
accagttaga atggcaatca ttaaaaagtc gaaacaacag gtgctggaga ggttgtggag 80460
aaataggaac acttttacac tgttggtggg actgtaaact agttcagcca ttgtggaaga 80520
cagtgtggcg attcctcaag gatctagaac tagaaatgcc atttgaccca gccatcccat 80580
tactgggtat atacccaaag gtttataaat catgctgcta taaagacaca tgcacactta 80640
tgtttattgc agcattattc acaatagcaa agacttggaa ccaacccaaa tgtccatcag 80700
tgataaactg gattaagaaa atgtggcata tatacatcat ggaatactat gcagccataa 80760
gaaaggatga gctcatgtcc tttgtaggga cgtgggtgaa gctggaaacc atcattctga 80820
gcaaactatc gcaaggacag aaaaccaaac actgcatgtt ctcactcata ggtggaaatt 80880
gaacaatgag aacacttgga cacagggtgg ggaatatcac acccctgggc ctgtggtggg 80940
gtgggggag ggatagcatt aggagatata cctaatgtaa atgctgagtt actgggtgca 81000
gcacaccaac atggcacgtg tatacatatg taacaaacct gcacattgtg cacatgtacc 81060
ctagaactta aagtataata aaaaataagc aagtttacaa gggcaaaaat aaaacaaaaa 81120
agaaaaagca ccacactgct tccacagtgg ctgaactaat ttgcactccc accagcagta 81180
tataagtgta ccctcttctc cacagccgtg ccagcatctg ttatcttttg actttttaat 81240
aaaagccatt ctgacaggtg tgagatcata tctcattgtg ttttaatttg cgtttctcta 81300
gtgagctttt tccatatgtt tgttggtggc atgtgtgtct tctcttgaaa agtatctaaa 81360
acagtcactt atcttttaaa gaaacttttt aaatcagaaa aaaggtgttt atatttaacc 81420
acgtatttat catttgcagt gctgttcatt ctgtttctta ggtcaaaatt tctatctggt 81480
atcattttct tctgcctcag gcacttcctt ttattatact gctgatctga tgctgattaa 81540
ttctttcagt aggtgtatgt tttcatagct ttttatttta tctttgtttt tcaaagatat 81600
tttgaagagt atagaatttt aggtagacag gccgggcgca gtggctcacg cctgaaatcc 81660
cagcactttg gaaggtcgag gcgggcaggt cacctgaggt caggagttca agaccaacct 81720
gaccaacatg gagaaacccc gtctctacta aaaatacaaa attagccagg catggtggtg 81780
catgcctgta atcccagcta ctcgagaggc cgaggcagga gaatcacttg aacctgggag 81840
gcagaggttg cggtgagccg agatcgcaca attgcactcc ggcctgcgca acaagaaaga 81900
aactccgtct caaaaaaaaa gaattatagg ttgacagtat tattctttca catccttaaa 81960
ctatgttgtt ccactgtctt ctgatttgcc ttgtttccaa gaagtcacct gtcaatctaa 82020
tctttgttcc tctgtatata attttttttt ctctctagca gcttttcaga ttttctcttc 82080
ctcactcgtt ttaagcaatt tgattatatg gatattagca tagtttcctt catgttgctt 82140
gtgcttgggg ttcatcgaga tccttagatc tctgggttta tatatttagt acgttttaaa 82200
actttttggc cattattttt tcaaatatat tttctgtcca ctcctcttca tcttcttctg 82260
gaaccccagt tgcacatata tttggctatg tgaaatttcc tacagctcac tgatgacctg 82320
ttttttaaaa aatctttttt ttctctttca ttttggaaag ttttattact gtgtctgcac 82380
gttcaccaat atttttgtct gtagtgtcta atatgttctt aattccatct agtgtatttt 82440
ttcctcttag acattgtaat ttgcaatttc tatagatttg tttggggtct ttttttttca 82500
tatctgccat gttactcctt aacataccaa tgctttcttc tttttctgat catatggaat 82560
atataatagc tattcaatgt acttgtgtac aaattctgtc ttctggatct atttttgttt 82620
agtttttgtc ctaattatga attatatttt tctatttctt tccatgcctg ttagtttttt 82680
attggatgac aggtattttg aattttgtat cgttaagtct tggattcctt tttttttttt 82740
tttttttga tgtgctttta aatacgtttg aggattggga tgaagttaaa tttgggaaca 82800
gtttgatttt tttgattctt tctaaactta cttttaagct ttatcagaga gaccagagaa 82860
acctttactc tagggttaat ttgacatcat tgctaaggca gtacctttct gaattttcaa 82920
cctgatgctc catgtattac aagatttctt gaactattcc agccccatat gtgttacaat 82980
aatttttctg cctccacctc tgtggtggtt cttttcccag ctttgacata tttcctcaca 83040
cacagcactc agctgaagac tccagtgatc tctgagtgta tctctgtttg cggtttcttc 83100
ctttccggta ctctgcttgt gagttttagc ctccttggct tcctccaata tttaactgtg 83160
tctcctcaac ctcagagatt gccaggctct gttggggttc ctccttcctg tgctgcagcc 83220
tgggactctt taggcattaa gccagagtaa ttacagggtt caccttattt atttcccttt 83280
tcttaaagat tactgttctg tgctgcctgt tttctagtgt ctaaaaacca tttcttcatg 83340
tattttagat atttaaggtt caaaccagtc tgttttactc tatcgttacc tgaagcagaa 83400
gactgacttc tgtactgttt catttgttaa ccagagtaaa tccttcatta ttcacataat 83460
aaattaataa ggatgatgtt tttctcacag ggactagatt aggcaatata catgaaaagc 83520
atattattga cagtaaagtg taagatgcta cacagatgtt tatcattgct attacaaagg 83580
agataacccc gttttcctgc agttagggaa gttctatatg ggagtaaggc tgaaagggcc 83640
aaaagatata ggtattgttt ctgaaaaact gcctatgctt ctatgcatat aagtatgtca 83700
tgttgcatat ttttctgtgc tgtattaatt catgcattcc tttatcaaca gatacttatt 83760
aaacactcat atgtcaggca ttgttctagg gactagagat ctctgccttc aaggagctta 83820
ttttctagtg gtatatttc tgttctgtgt cttagctatc cactttttc atctgcctgg 83880
acacgtgact tattctgtct ctgggcctct ggtatgagtg ctcatttcat tctgccttat 83940
aactcctatt ttcttcccta ctttatctga ccttcctacc ttagcttgtt cattctttcc 84000
ttcaatccag ttgtcatgaa atctctttct ttcctctact aatttttttt ttctttcttt 84060
ctttctgagt aaaagccaga gatctggccc cctgcttacc tctctgaatt ctttacttac 84120
ttctgacaac ttgctcattt cactccagct acattgacct ccttgccttt gttgtgtttt 84180
gaaaacaccg gcgtggtcct accgcaggaa ttttgtactt actgttcctt ttgccacatt 84240
catcattcat atgttcatgt cttccccatc gcttccttca agtttttgct cagttgtcat 84300
```

cttttttactg agtgtccttc ctgacctctc cctactttaa aatgttatgc ttttttctac 84360
tacctctcct gttcctcatt cctaccttat ttttctggat aacacttatt gccttctaaa 84420
ttgtatcgta taatttactt ctttgtttct tctccgttgt cacactagca tataaattca 84480
gtgaaggtag agattctttt actgctgaca aaagcatcta ggacatttcc tggcactgat 84540
aaggatctgc ataaatattt gttgagtgaa tgaatctcct tggtaaagtc ctttttttgtt 84600
tgcctgttat atttattgaa tagacttctt tgtttcatgt actttaattt caaaagtgaa 84660
tggctggaac attttatata ttttcttata aatcattttc attgcttttt aagcttatca 84720
catattttgt tttataaata tgtagccttc gtgagataaa agattctcca tccctgttta 84780
cgtgtatact tagatgacaa ctctaatggt cataaataat tccaacctta tagataactc 84840
aggagaagat gagattataa gtagacttta aatcccattc agaaacccaa ggacaattca 84900
aaaggaaaat aatcattcca aatataatat tcttcttatt cttaagaagt tgttagtatt 84960
ttgaattttg aatttttaat acagctccca tagaaaatac tttaaaatga acccggtaag 85020
acttcctcat taagacatat agacacatat gctcccactc ccgtaatcaa atttggaagt 85080
caaataagct ctgaaaacca aaacattctt ccaagtttat tgcagactca tttggtagta 85140
aaaaccaatc tgacctgatg tgtagctgtt tatagtcttt atttttccat ttggtgagtc 85200
caattatata tttcgctgag gaaatactaa catgtttgac tacattgtgt gccccagacc 85260
tgcttagagt attatgtaat atgcagtata ggccatatat tgccttttta acatcaaaaa 85320
taccctaaat tctgaaatgc atgtagcccc aagagatttg tataaagcat tgagggcctg 85380
tgtatttttt tttaatttac aagtgaagcc agattcccta gacattaagt gcctaacttt 85440
tggttgttgt tgctgctgct gttactgttt tttctccagc ttcgcaaacc tgggtatact 85500
tcatgtgaca aagaaaaaag tatttgaaac actggaagca cgaatgacag aggcgtgtat 85560
aaggggctat aatcctggac tcttggtgca ccctgacctt gcctatttgc aagcagaagg 85620
tggaggggac cggcagctgg gaggtaagca tcattttcct ggccttgatc ctccaagggg 85680
tccaggcttt ggttttcatc tgtatgaatt atatgttcat ctgcatccct tccagtctct 85740
accccacact gctgtcacct tttccttgct cagaaacctt tgatggcatc ctgctacttc 85800
taggataaaa actatagctc catagcctgt catacaaagc cctgcttctc tggccccagc 85860
aactttcagc ctcatctcca gcccaccttg catcctcctc tcgccatctc tgctctctga 85920
ataactgagg catatttata cctttgtatg tgtcttcctt taccaaaaac accccaccgt 85980
ttccatttca ccctctcgaa aaccaatgca gaaatcaaga ctttgtctaa agaacacctc 86040
tgttttacct tccccaactc accttattat aaggcatttt tctttctttg ggctctgcag 86100
acacattata aactattatc tttatcataa aaattgtatg attttccatt ttcttgcctt 86160
ttctcaaatt ttttttcaa ccccattccc tgtcccacca catgccccat ttctactttt 86220
gtattttgtc ctttactctg gccgtccagt cgtcatctga tactcatgtg attcaaaacc 86280
gattatcagt tttccactaa atatactcca tcttctgata cccagactca aaaccttccc 86340
atcctcactg actccccctc ctcgcctgtg ctgcgaggct gtggtccagc cattccgtgc 86400
tctctggcat tccacactac attccatttt cattgttcca cttatagtag tcttctccct 86460
ggtctctgtc cctccccact tctaatcaag tttacatctt gtagccagat agatattact 86520
gaagttctgt atcttgttgc tgctccgtgt ctccgttttg cctacaaaat ccaaattctg 86580
taagttggct ttttggatct cccaagagct ggcctcaggc tgcatttcca atctatttcc 86640
cgttttgccc cttcaggtca aagtctacta cttggtagga gcaccatgat ttctcacttt 86700
cctgccttgg cacattcttt ctatttttgc tctctcctct tcctggaatg cctttatcca 86760
ctctcttata tgaatgtgtt caaattctgc cacctttca atattcagag caaatacctc 86820
ttctgctcca aagccctctg aactctctcc ttctttgaaa tcccacatca cttcatttgt 86880
agctctcttg tagtacttgt ctcttgaatc actacctttt atgatattat agcttaatta 86940
tgactatatc catcagcgtg aaaactccag caggagggaa ggaaacagat gtccctgagt 87000
cattcagttc cttcagaagt tgtttttgag tacctactat gtgccaggct gtctgcttgg 87060
cactgtggaa tgagctcact ccctgtgccc aagcagctct tggaataatt tgcacacttc 87120
tcaatacagc tggtgcctag tagggttatt caattccatt ccatcacatc ctgtgtgcct 87180
ttaactctta ctgtagagag gaaagggaaa ggtgggggaa ggacaggaga ggagggaggc 87240
gatctgatac acggaagaga gtttttactg taatccggag aaaatgcagg tcttctttga 87300
tgcctttcat attggatgac ataaatgaga ctgtttttaa cactttatta gcaatatgaa 87360
gagtttcaaa agaggaaaaa tgggttttta ttgtaagttt acattatttg ggctttataa 87420
aagcatggtc ttttaaatgt tcacacttcc ctgggcatga atggactgtg ctgtatggcc 87480
ctagatcggg aaaaagagct aatccgccaa gcagctctgc agcagaccaa ggagatggac 87540
ctcagcgtgg tgcggctcat gtttacagct tttcttccgg atagcactgg cagcttcaca 87600
aggcgcctgg aacccgtggt atcagacgcc atctatgaca gtagtgagta cttcacttcc 87660
aacaggggggc acaccaagaa tagacttcca gccctgccct gccatttact tgctagctga 87720
gtcctgggga aggtaactta atcactttaa gcctcagctt tatcatctgt aaaatgtgaa 87780
taggaaaatc tagcttgaaa ggttgatgtc caggttaaat gaggtatttt aagaggggct 87840
caatacatgt aattcttttt cctttgatgt acattttta gtcttttaca tgaaatgtca 87900
catctcatct tatttatgaa tgtcttgcta taaagatata tggttgatac ttttagaagg 87960
gagaataatc ccaattttct ttggtggtgg ggggactctg gaaaggagtt tataaaagac 88020
aactattatt cttacccatt ttctttccca tatgttacta gctttatcaa gagagaaact 88080
tgaagttaaa tgaatgagta gcatagacag catgtgactt caaaatccta tttcaagccg 88140
ggtttggcgt catgccctgt agtctcacct acttgggagg ctgaggtggg aggatatgga 88200
gtttcagccc agcatgagca acataatgag accctatctc aaaaaaaaaa aatatatata 88260
tatatatgta tatatatatt aatttatagt tcctaggact ttttaagagt tttacagtaa 88320
ccttcagcat ttaccatgca actttcttgt ttgattatgc ataggattga ttagacaggt 88380

```
atttcaaaa agcctgcaag catagtactc agactcattg aaagtttata gaatttggcc 88440
tgtgtggaaa actctgtgct ccaagtacaa caactaactg aattctctaa tttaaacaag 88500
tttgaaggga agtgaaaggt tataaaatga tacagactca taccgcagaa tcaccttaaa 88560
atgcagctgt tggagaaaaa aaaaaaggaa gctttatgct attaccacaa gaaagttttg 88620
cctcttcacc acacagaatc ctaatttagt ttgggaaaaa gaaacataac atccccattt 88680
tccttacctg taaaataaaa ggtagtaagt agtttaagaa aactgtactt ttcctaaagt 88740
ttttaggact cttgtaaata gaataatagc agaatcatgg aaacaaattt ggaattgaag 88800
tcttgacctc attgaaagcc cagaaatcaa ttagcttggt acttcccaat acatcagaaa 88860
agctctttct ctttttgtgt cagtctctct gtgttagagg gagaaacaaa tacctgttcc 88920
tttctacctc atttgttttg gtgatcataa atgacccatg cccaagaagt gttccgagaa 88980
tttcagaagc cattcttgtt ttatttactt tgttagtgaa agcatgtatt taagctttgt 89040
tttggtcatg tgtgctaagg ggagggtcct acctaaagga ctggcttgtt gagctgagga 89100
ttaatcatgt tatttgttgt ttttccccctg tgaacagaag cccccaatgc atccaacttg 89160
aaaattgtaa gaatggacag gacagctgga tgtgtgactg gaggggagga aatttatctt 89220
ctttgtgaca aagttcagaa aggtaaatac attctgtgat ctctgatctc aagaggtgtg 89280
atcttgacac actacagttc tgagtgtgtc tgtgagtcac atttcagcag tggacaagaa 89340
acatccctct gctgccacag aaagtcttaa aaagcattta cgttttacct cttccaaatg 89400
taaattgtct gtgttatttt ttcctaagtc aactaaatca cttttagatt tcccatggaa 89460
gtaaaaagaa ataagaaaac cgtgataatt ataatcagtg actttcagta tctttataca 89520
ttaaaattaa tatctgtact ttgttaaaca aaaataaata atagcccatt cttgacacac 89580
atacaaacac acacacagaa taatgtttta catagtatta aattgttttt atttcttttg 89640
caatgctttg atataacact gagagaactt ctctgaacac ttccacagat gtgatgttta 89700
agaaaaggaa agaaaacagg gaatacaggg aaattctcag agcacatttc tctcacaatt 89760
ttccctttga ggaaaggttc ttggcgttta tctttcatta gatatattac aacattgata 89820
gattagtaaa agctgaggaa aatgtgttat tttctcaaac tgcattcttt ttttaaataa 89880
aatgatagtt ggaaagtgta gtataatatc tatttactac agggtgagca ttttcaaagt 89940
aagcagatac ctaactgaaa atataaataa gtaaaataaa tcaataaaag acatgtggct 90000
tttaaaaaaa ttatcatatg agattaaatt ctctgaggta tatagtcaga aaaaaatggg 90060
atcagatttt aaagtaaaca catatatact ttatccaaaa aaatttacag gccaggcatg 90120
gtaactcatg cctgtaatcc cagcactttg ggaggctgag gtgggaggat tgcttgaacc 90180
caggagtttg aggctgtagt gagctattat catgctgctg tactccagcc tagatgacag 90240
agcgagaccc tgtctcaaaa aaaagaagtt tacactttct ctgcctacac tagtgtcagc 90300
cttcttccaa tccactctgt ccaagtaata ctcatttcca gattcctggg tgtacatact 90360
gtgagtaaac acaatctgag tacctttatt tgtaaatgag atccttagtc attatcatta 90420
cattggaatg gcttagtaag aaatgtcttg tacttggctt aaagaatctc aataactagg 90480
tccagtgtag tcctacctga aggcaaagga gattgtaaga tgttttatcc attcctagta 90540
tttctaaaat tctacttaca tagagataaa aactttgaac aaagtcaata gtgtggccct 90600
ttctatgaaa cggaaagtga agccaggaag atgccaacct aagttacatt gtcacatttc 90660
tgtagaagtg aatagagtat ttataaacat gctgaacatg gagtatttat aaacacactc 90720
catgactaga gtgtgcaatt taagcattgc attttaatta gaagggacct gattattata 90780
gtattttgac caattactca atatgaacta agagactatt atagtgagac agtttaatag 90840
tgtgttaaga gcctggactc tgaaattata ctgtctatgt taaaatcctg gttcctccaa 90900
gtactagctg ttcagccttg gataagtact taacctcttt ggaccccagt ttctcttttg 90960
caaagtgagg ataataatag tacccatcta catcacaagg ttgtggtgag gatgaaatca 91020
gttaatatgt gtatatatga agcacttaga atagcatctg ccatacatta atagtaaaac 91080
ttatataagt taattatttt tatgtacatc tatatattga attgcactgc ctcgttggat 91140
tctaatattc ttccaccatc gttcgtgttt ctcaggattg gcttaaggat ggagaagcag 91200
taaggtcaaa gattaaaaga aaattcaagt taactaggta aatctagtat tctgtcattg 91260
tgaatgaaag ttggggcgca ttactgtggt aaataatgaa aaaagtcagt gatttgtgaa 91320
gattttaaaa gactgaacct tttgatcttg tttttaaaa gtgtaaatag atagtaggta 91380
gaatattaaa ccagttttat ttttcagcat gtttataaat actatttaca ctatgtgaaa 91440
ttacacactt caatgtgatt gtttgcagat gacatccaga ttcgatttta tgaagaggaa 91500
gaaaatggtg gagtctggga aggatttgga gatttttccc ccacagatgt tcatagacaa 91560
gtaagtgatt tattattatt attaatcctt attattttta gagatgggat ctcactctga 91620
cacccaggct gcagtgcagt ggtacaatca cagctcactg tatcccccaa ctgttgggct 91680
tgagggatcc tcctgtctca acctaccaaa tatctgggac tacaggcatg ctaccatgcc 91740
cagctagttt cttcagtttt attttttttg tagagatgat gtcttgccat cttgctcagg 91800
ctcgtcttga actcctggac ttaagcgatt atcccacatt ggcctcccaa aatgctggga 91860
ttataggcat gatccatatt actatcatca ttttatactt tctgccttat tgaaatttag 91920
tacttagctt ccatctataa aaaagaaaaa tcataaatat ttaacttcca taaaacagta 91980
ctatttttaa gactcatgca ggtcatccaa aaaagttacc tactgtagag ttatgggcaa 92040
ggttctgtta gatcgtttgc tctgagatgt tggcaagata tttaattgct gcctaatccc 92100
ctaacccata agacagtgcc ccacaggtcc tttaatgaaa atgtttttaa gtgttctacc 92160
aaagtaatta tgatgagacc tattttatga tagcacctaa ttatgataac accttaaaag 92220
ggtgttctag gagtcatctt ctaattgaag ctgatgtcat cctgttaaat gaagatttcc 92280
caatggcaac tgattaaatg atcagtttaa tcatttaata acatgataat gctgagcccc 92340
caccttaatt attattctcc tatttacaag taattgaaga gtattctcct atttacaagt 92400
aattatctgg attggaaaca atccagatat tcatcagtaa gtggccagtt aaattatggt 92460
```

ctgtcctttt gatgaaatat catgtagtta ttcaaaagaa tgagtactct gcgaactaat 92520
atagaaagat ctcagttata cagtgttaaa atattttaaa agatgggtgt agaacaaagt 92580
gtgtgtagaa atgattctat gttcatgcat aaagaaattc tggaaggata cctaagaaat 92640
taataacagt atttaccact ggctggtaca ctgtaggaac tgcatggatg gggaacaaga 92700
atgtcaaaga gaataccact gtatgctttt ttatatttta taagatcttt atccctgtga 92760
atatattaca tagtcaaaaa atcaaactga aaaatgctta aatccctaac ataccaccaa 92820
gaactaatgt gttatgatgc caatgtaagc aatgtttagt ttcttctctt catcactggc 92880
ctgtagccat ctctctcttt ccatacccag tctttctgat gttgctctaa ataatcttgg 92940
aggcttttaa ggctgctttg gaagagaaga aagtatatgc agtgaatttt ataagcatga 93000
tatttacaac tagaagacat ttgaaataga caaatgaaat aataaagctt cgtagtacat 93060
tagcatagca ttgtattttg attttacagt agcaatttca taaaaatgta tcggtataga 93120
attctgagtt tggaactttc tccaggacac ggtgtttttt agtacactgt gtctaaacat 93180
tgggtataaa gaaaagcata aggaatgtgt ttaatgagta gcattactgc aaaaaaaaaa 93240
aaaatgtagt cctacaccaa catgtggttc ttcgtatcct gcagtttgcc attgtcttca 93300
aaactccaaa gtataaagat attaatatta caaaaccagc ctctgtgttt gtccagcttc 93360
ggaggaaatc tgacttggaa actagtgaac caaaaccttt cctctactat cctgaaatca 93420
aaggtaagtc agttgtttaa aatcttatgc tcatatttta ttttatttta tttttggttt 93480
ttgttttgtt ttgttttgtt ttgagacaga gtctgactct gttgcccagg ctggagtgca 93540
atggtgcaat catagctcac tgcaacttcg aactcctggc ctcaaacagt tctcctgccc 93600
cagcatctca aatagctggg actacaggtg tgcgccacca tgccgggcta attttttttt 93660
tttttttactt ttaatagaaa tgaggacttc ctacattgtc caggctggcc ttaaactcct 93720
ggccttaagc agtcctccgg ccttggcctc ccaaagtgct gggattacag gcttgagctg 93780
ccatgtcttg ccattgtgct tgttctgaga agaggacaag ctaattagaa aagatcagtt 93840
agtcacctcc tttgaacagc tttctagtaa caggtccctg gatccatggt gcttattttt 93900
agaagagaca gtagtatatt attttgaggt catggaatta gtctagcttt ttaaaataat 93960
gttatttcca ccaagcttat atgagattta gacattgaga atttgtcttt gaatttgaga 94020
atctaacatt tattgactaa ctcagagttc aagcagctga gatgaaaaaa tcaggcttgc 94080
tgtttaggag aatcagccag ttagtcaacc agcctcaggc ggtggaacag aaaacttggc 94140
caacatgcac tgtgaaacta tgaaaatgag taaggcatca ttctttctct cacaaaagat 94200
gctttaagaa agggcactca taggtttctt aaatagttat aatgtaaatt atgattttaa 94260
taagtaccga aagtgaagca tccataaaaa tataagagaa tgttgaagag agagcccttg 94320
gtccctttat tctaaagcag cctccaaaaa gaaggcttcg tcaaggagat atcatttggc 94380
cctcagcatt tggggtatat ttcagcagtt gggtgttaaa cagagaagga gagggttctc 94440
taggtttaag ttacttgagg agcttaaaga gagaggtaaa cacagcgttt atgcagaggg 94500
aaaaaagtgt tctgagcgct cggatggaca tgaaggccaa tgtgggtaag gaaggccagg 94560
gccaggctaa agagggctgt gaatggcagc ttaagaattt tgcattaatt ctggagaggg 94620
atagccgggc ctgcctaggt ttggaatgat ggctctgact tctttatgtc accactgagg 94680
gtaccattta ggaggcagat gatgagtcag aagagtaatg caagaatcca agtgagaaac 94740
agtgcagtta gtaaaggtcc tagtgccagg aatgaaagga gatggtggac ttaagagaga 94800
gtgcagtgat actgtgatac agtgatactg tcaagagggc ctagaggtcg gaggtgtagg 94860
agagacgggg agtcatagat gggtctaaga ctccctgacc aggaagcttg tgatgtggtt 94920
gacttgatga aaggaacaag ggagcaaaag tagatttggg tgggaaaaga aggagctcag 94980
agtaaaacat gttatatttt aggtccctgt gggaaaatca agtgtaagtt cttagtaggc 95040
agatcaaaag gcagaactgg aactcaagag aggacagggt gagaaattta gcattagaat 95100
catctcaaaa cgagatataa caggcaccaa ctgagtcatc attcaaaggt atttcttaac 95160
ctagagtcca ctgaccccca aagagtctgt ggatagaaat gtactttgat atcatcagct 95220
tcttttataa tcctgttttg tattttacat actgaaaacc atgattctga gaagagatcc 95280
atagagttca ccagactgca gaagggggctg tggcacaaac aaaattagaa acccctcagt 95340
tggagggaag tgagactggg aagggaggtc agtaaaatga ttgccacggg gcacccagat 95400
ccttgctgag gttggaaacc gcaaaggtgc agcagcaaca agccacatgg gtgggtgttg 95460
tctcctgtga gagcaagatg tgataggtaa tctttgaaga gggctttcct ttcctagaat 95520
tcgtgctgcc acaggagtgt ttagaaaacg gttctacaag ttcttgtttc ttttctgagg 95580
ttgtggcctc tccattgttt ggtcagattt gtagtgcact ctatatcctg gtggtttgaa 95640
aacattgtca gcacaataac ttttctttct tcacatgaga tttcatacaa aatcccagtg 95700
tatggacatg gtaggctttg attaaaatag gggtgaaggg ccctgccagc tcggccttcc 95760
gagaggctgt cccacctctc cttacttccc tatcaggagc tgctggaggt cttttgaatg 95820
cttctgccta cacaaaatga tgtgaaaatc actgctttaa tttaaacacc cttctttaaa 95880
agtagacaca gaagaaaaat atggataatt tttttagact ttctgaggaa aaaaatagat 95940
ttcttctcca gaaatatgtc ttcaaataat gccagttttt gctaacacag agaatcatga 96000
agaaagaaaa cccaggttac ctgtatgtgt atgaacctag aaacattcaa agctcagctt 96060
ggtatctagg ctgcctgtct ccctttccca gtggtatcta tgcctcagaa aagtatgtta 96120
tagtgggggt atattcaggt gattacttta atgcctcgtt atcatagtag gaactatcca 96180
atgcagggat tagagaatgg ggtcacctga aagttcaaaa ttgcacctgt atgcttcatg 96240
cacctacctt ccaggctgtc attgatgaaa agcttcatac aatacaaagt ttaagaactg 96300
tctggagaaa tacttaccca ttgagctctg aaggaaagaa tggacaacat atatatatat 96360
gaggacaaca aattacttga ttctatagaa accttctgga ttataaaaac tcaagataat 96420
aggaccaagg agaaataatc tgaccaggca atataactgg tcagaaaagt agtaggccat 96480
ttagcattag agtaagtaga tagaaatgga tccaaactgt acaacccagg gaaacattgc 96540

atacatttct cagaagtcat tgaacacact tttccttacc cccagtctct gt 96592

<210> 29
<211> 3625
<212> DNA
<213> Homo sapiens

<400> 29
ggccaccgga gcggcccggc gacgatcgct gacagcttcc cctgcccttc ccgtcggtcg 60
ggccgccagc cgccgcagcc ctcggcctgc acgcagccac cggccccgct cccggagccc 120
agcgccgccg aggccgcagc cgcccggcca gtaaggcggc gccgcccgcg gccaccgcgg 180
gccctgccgt tccctccgcc gcgctgcgcc atggcgcggc gctgactggc ctggcccggc 240
cccgccgcgc tcccgctcgc cccgacccgc actcgggccc gcccgggctc cggcctgccg 300
ccgcctcttc cttctccagc cggcaggccc cgccgcttag gagggagagc ccacccgcgc 360
caggaggccg aacgcggact cgccacccgg cttcagaatg gcagaagatg atccatattt 420
gggaaggcct gaacaaatgt ttcatttgga tccttctttg actcatacaa tatttaatcc 480
agaagtattt caaccacaga tggcactgcc aacagatggc ccataccttc aaatattaga 540
gcaacctaaa cagagaggat ttcgtttccg ttatgtatgt gaaggcccat cccatggtgg 600
actacctggt gcctctagtg aaaagaacaa gaagtcttac cctcaggtca aaatctgcaa 660
ctatgtggga ccagcaaagg ttattgttca gttggtcaca aatggaaaaa atatccacct 720
gcatgcccac agcctggtgg gaaaacactg tgaggatggg atctgcactg taactgctgg 780
acccaaggac atggtggtcg gcttcgcaaa cctgggtata cttcatgtga caaagaaaaa 840
agtatttgaa acactggaag cacgaatgac agaggcgtgt ataaggggct ataatcctgg 900
actcttggtg caccctgacc ttgcctattt gcaagcagaa ggtggagggg accggcagct 960
gggagatcgg gaaaaagagc taatccgcca agcagctctg cagcagacca aggagatgga 1020
cctcagcgtg gtgcggctca tgtttacagc ttttcttccg gatagcactg gcagcttcac 1080
aaggcgcctg gaacccgtgg tatcagacgc catctatgac agtaaagccc ccaatgcatc 1140
caacttgaaa attgtaagaa tggacaggac agctggatgt gtgactggag gggaggaaat 1200
ttatcttctt tgtgacaaag ttcagaaaga tgacatccag attcgatttt atgaagagga 1260
agaaaatggt ggagtctggg aaggatttgg agatttttcc cccacagatg ttcatagaca 1320
atttgccatt gtcttcaaaa ctccaaagta taaagatatt aatattacaa aaccagcctc 1380
tgtgtttgtc cagcttcgga ggaaatctga cttggaaact agtgaaccaa aacctttcct 1440
ctactatcct gaaatcaaag ataaagaaga agtgcagagg aaacgtcaga agctcatgcc 1500
caatttttcg gatagtttcg gcggtggtag tggtgccgga gctggaggcg gaggcatgtt 1560
tggtagtggc ggtggaggag ggggcactgg aagtacaggt ccagggtata gcttcccaca 1620
ctatggattt cctacttatg gtgggattac tttccatcct ggaactacta aatctaatgc 1680
tgggatgaag catggaacca tggacactga atctaaaaag gaccctgaag gttgtgacaa 1740
aagtgatgac aaaaacactg taaacctctt tgggaaagtt attgaaacca cagagcaaga 1800
tcaggagccc agcgaggcca ccgttgggaa tggtgaggtc actctaacgt atgcaacagg 1860
aacaaaagaa gagagtgctg gagttcagga taacctcttt ctagagaagg ctatgcagct 1920
tgcaaagagg catgccaatg cccttttcga ctacgcggtg acaggagacg tgaagatgct 1980
gctggccgtc cagcgccatc tcactgctgt gcaggatgag aatggggaca gtgtcttaca 2040
cttagcaatc atccaccttc attctcaact tgtgagggat ctactagaag tcacatctgg 2100
tttgatttct gatgacatta tcaacatgag aaatgatctg taccagacgc ccttgcactt 2160
ggcagtgatc actaagcagg aagatgtggt ggaggatttg ctgagggctg gggccgacct 2220
gagccttctg gaccgcttgg gtaactctgt tttgcaccta gctgccaaag aaggacatga 2280
taaagttctc agtatcttac tcaagcacaa aaaggcagca ctacttcttg accacccccaa 2340
cggggacggt ctgaatgcca ttcatctagc catgatgagc aatagcctgc catgtttgct 2400
gctgctggtg gccgctgggg ctgacgtcaa tgctcaggag cagaagtccg ggcgcacagc 2460
actgcacctg gctgtggagc acgacaacat ctcattggca ggctgcctgc tcctggaggg 2520
tgatgcccat gtggacagta ctacctacga tggaaccaca cccctgcata tagcagctgg 2580
gagagggtcc accaggctgg cagctcttct caaagcagca ggagcagatc ccctggtgga 2640
gaactttgag cctctctatg acctggatga ctcttgggaa aatgcaggag aggatgaagg 2700
agttgtgcct ggaaccacgc ctctagatat ggccaccagc tggcaggtat ttgacatatt 2760
aaatgggaaa ccatatgagc cagagtttac atctgatgat ttactagcac aaggagacat 2820
gaaacagctg gctgaagatg tgaagctgca gctgtataag ttactagaaa ttcctgatcc 2880
agacaaaaac tgggctactc tggcgcagaa attaggtctg gggatactta ataatgcctt 2940
ccggctgagt cctgctcctt ccaaaacact tatggacaac tatgaggtct ctgggggtac 3000
agtcagagag ctggtggagg ccctgagaca aatgggctac accgaagcaa ttgaagtgat 3060
ccaggcagcc tccagcccag tgaagaccac ctctcaggcc cactcgctgc ctctctcgcc 3120
tgcctccaca aggcagcaaa tagacgagct ccgagacagt gacagtgtct gcgacacggg 3180
cgtggagaca tccttccgca aactcagctt taccgagtct ctgaccagtg gtgcctcact 3240
gctaactctc aacaaaatgc cccatgatta tgggcaggaa ggacctctag aaggcaaaat 3300
ttagcctgct gacaatttcc cacaccgtgt aaaccaaagc cctaaaattc cactgcgttg 3360
tccacaagac agaagctgaa gtgcatccaa aggtgctcag agagccggcc cgcctgaatc 3420
attctcgatt taactcgaga ccttttcaac ttggcttcct ttcttggttc ataaatgaat 3480

tttagtttgg ttcacttaca gatagtatct agcaatcaca acactggctg agcggatgca 3540
tctggggatg aggttgctta ctaagctttg ccagctgctg ctggatcaca gctgctttct 3600
gttgtcattg ctgttgtccc tctgc 3625

<210> 30
<211> 2907
<212> DNA
<213> Homo sapiens

<400> 30
atggcagaag atgatccata tttgggaagg cctgaacaaa tgtttcattt ggatccttct 60
ttgactcata caatatttaa tccagaagta tttcaaccac agatggcact gccaacagat 120
ggcccatacc ttcaaatatt agagcaacct aaacagagag gatttcgttt ccgttatgta 180
tgtgaaggcc catcccatgg tggactacct ggtgcctcta gtgaaaagaa caagaagtct 240
taccctcagg tcaaaatctg caactatgtg ggaccagcaa aggttattgt tcagttggtc 300
acaaatggaa aaaatatcca cctgcatgcc cacagcctgg tgggaaaaca ctgtgaggat 360
gggatctgca ctgtaactgc tggacccaag gacatggtgg tcggcttcgc aaacctgggt 420
atacttcatg tgacaaagaa aaaagtattt gaaacactgg aagcacgaat gacagaggcg 480
tgtataaggg gctataatcc tggactcttg gtgcaccctg accttgccta tttgcaagca 540
gaaggtggag gggaccggca gctgggagat cgggaaaaag agctaatccg ccaagcagct 600
ctgcagcaga ccaaggagat ggacctcagc gtggtgcggc tcatgtttac agctttttctt 660
ccggatagca ctggcagctt cacaaggcgc ctggaacccg tggtatcaga cgccatctat 720
gacagtaaag cccccaatgc atccaacttg aaaattgtaa gaatggacag gacagctgga 780
tgtgtgactg gaggggagga aatttatctt ctttgtgaca aagttcagaa agatgacatc 840
cagattcgat tttatgaaga ggaagaaaat ggtggagtct gggaaggatt tggagatttt 900
tcccccacag atgttcatag acaatttgcc attgtcttca aaactccaaa gtataaagat 960
attaatatta caaaaccagc ctctgtgttt gtccagcttc ggaggaaatc tgacttggaa 1020
actagtgaac caaaaccttt cctctactat cctgaaatca aagataaaga agaagtgcag 1080
aggaaacgtc agaagctcat gcccaatttt tcggatagtt tcggcggtgg tagtggtgcc 1140
ggagctggag gcggaggcat gtttggtagt ggcggtggag gagggggcac tggaagtaca 1200
ggtccagggt atagcttccc acactatgga tttcctactt atggtgggat tactttccat 1260
cctggaacta ctaaatctaa tgctgggatg aagcatggaa ccatggacac tgaatctaaa 1320
aaggaccctg aaggttgtga caaaagtgat gacaaaaaca ctgtaaacct ctttgggaaa 1380
gttattgaaa ccacagagca agatcaggag cccagcgagg ccaccgttgg gaatggtgag 1440
gtcactctaa cgtatgcaac aggaacaaaa gaagagagtg ctggagttca ggataacctc 1500
tttctagaga aggctatgca gcttgcaaag aggcatgcca atgccctttt cgactacgcg 1560
gtgacaggag acgtgaagat gctgctggcc gtccagcgcc atctcactgc tgtgcaggat 1620
gagaatgggg acagtgtctt acacttagca atcatccacc ttcattctca acttgtgagg 1680
gatctactag aagtcacatc tggtttgatt tctgatgaca ttatcaacat gagaaatgat 1740
ctgtaccaga cgcccttgca cttggcagtg atcactaagc aggaagatgt ggtggaggat 1800
ttgctgaggg ctggggccga cctgagcctt ctggaccgct tgggtaactc tgttttgcac 1860
ctagctgcca aagaaggaca tgataaagtt ctcagtatct tactcaagca caaaaaggca 1920
gcactacttc ttgaccaccc caacggggac ggtctgaatg ccattcatct agccatgatg 1980
agcaatagcc tgccatgttt gctgctgctg gtggccgctg ggctgacgt caatgctcag 2040
gagcagaagt ccgggcgcac agcactgcac ctggctgtgg agcacgacaa catctcattg 2100
gcaggctgcc tgctcctgga gggtgatgcc catgtggaca gtactaccta cgatggaacc 2160
acacccctgc atatagcagc tgggagaggg tccaccaggc tggcagctct tctcaaagca 2220
gcaggagcag atcccctggt ggagaacttt gagcctctct atgacctgga tgactcttgg 2280
gaaaatgcag gagaggatga aggagttgtg cctggaacca cgcctctaga tatggccacc 2340
agctggcagg tatttgacat attaaatggg aaaccatatg agccagagtt tacatctgat 2400
gatttactag cacaaggaga catgaaacag ctggctgaag atgtgaagct gcagctgtat 2460
aagttactag aaattcctga tccagacaaa aactgggcta ctctggcgca gaaattaggt 2520
ctggggatac ttaataatgc cttccggctg agtcctgctc cttccaaaac acttatggac 2580
aactatgagg tctctggggg tacagtcaga gagctggtgg aggccctgag acaaatgggc 2640
tacaccgaag caattgaagt gatccaggca gcctccagcc cagtgaagac cacctctcag 2700
gcccactcgc tgcctctctc gcctgcctcc acaaggcagc aaatagacga gctccgagac 2760
agtgacagtg tctgcgacac gggcgtggag acatccttcc gcaaactcag ctttaccgag 2820
tctctgacca gtggtgcctc actgctaact ctcaacaaaa tgccccatga ttatgggcag 2880
gaaggacctc tagaaggcaa aatttag 2907

<210> 31
<211> 167163
<212> DNA
<213> Homo sapiens

<220>
<221> misc_feature
<222> (1)...(167163)

<223> n = A,T,C or G

<400> 31

```
gagttgtcag caacctagag ccctgaatga ctgcctggaa gagctgcctc accacctgag 60
catctgtgtt agaccagcgg gacattcgct aaagcaatta aaacatattt tattcaatac 120
tactcacagt cagggaaaga cctgagctcc attctaattt gtgcccaggt atctgggcat 180
tttaaaggga gaaggaggga tgggggatgg cagggcttga aatgagttag ggaagtgaaa 240
aattacaaag agcaggaagg gaaaagggct tggcccatgt gactgagact agcacaggac 300
cctatcttca ggtgttggct ggaacaaaca gtaagttctt tgaattttgt aggcaggcac 360
tttcagggtg ggctcaaatc gtgctgggga ggcatcctcg tgctgttaga accatgttag 420
tgtgtgtcca aggctttttt tttttctttt tttcagagac agggtctcac tctgttgccc 480
aggctggagt gcagtggtgc aatcacagct cactacagcc ttgaattctt aagcttaagt 540
gatcctccca cctcaacctc ctgagtacct gacaccacaa gtgcacacca ccatgtctga 600
ctagtttttt taaaaatatt tttgtgagat ggggtctccc tctgtttctt gggctggtct 660
tgaactgctg ggctcaagta atcctacttc ctcggccttc caaagtgctg ggattacagg 720
cgtgcaccac catgcccagc ctgtccaagt ctttttaggc tgaggttaag acctggtaag 780
agattggtca agaagagaat ctttgctatc accctattgg gcttattcca ttagtttaca 840
aatgaagccc aggaaatact aatgtagatg ggtcccagca agaatttaag tactggtaat 900
tcttaacaaa tgtttatgaa gagtttatta tgtaccaaac tccctaccac acctacatcc 960
acaatcttca cacctacatc cacaatcttc acacctacat ccacaatacc acatctacat 1020
ccacaatctt catttgctcc tctatttgca agatcttgtc tttacaaaaa aaattttaaa 1080
aatcattagc caggtatggt ggtgtgttcc tgtggtccca gctactcagg aggttgaggt 1140
gggagcatcg cttgagccca ggagtttgag gctgcagtga gctaggattg cactatgcac 1200
tccagtccgg gtgacagact gagaccctgt ctcaaaatat aataataaat taaacaaaca 1260
aacaaacaaa caaacaaaca aataagtaat ctggccgacg cagatactga acgttggtta 1320
acacaatccc aattcctaat ccctttcctc cttgactgct tttgctacag aggttggaaa 1380
actccaacac ttgctatccc agctttactt gcagctagca gaggccatat gagagctcta 1440
gccaatgagg ctgaatgttt taccacgggt tggttggaag gtttttgctt tttctggcaa 1500
acaggacata cttggtttag gctgcttctt ttacttattc ctgctttaac catggatttg 1560
atgcccaaga ccaagaatat gctgctctga caatactaag ctcaaacaac aaagagtatt 1620
gtttttaca taagaagagg aaaaatgatc tcttctttta tctctcactt acaattgtct 1680
aagttttccc agaagctccc caatagcttt cctttcacat gccattggcc agaattggat 1740
cagctgagcc cattcctgaa ttgatcacta agaaaggaag tagaatcaca tgctaggctt 1800
agactgatca ctgggtagaa tggatactgg agcatcaatc aaagcatgcc ctatagcagg 1860
taaatgtgat cacaagacaa ataatgactt tctgaatata aaaaccatag gaataaataa 1920
caaattaaat gactggtgaa tatgagctta ttgacattaa agcaaattta taaaaccact 1980
cataaacaaa aggaaaaggc aaataagaca tcagggatat attttgcaa tttacatggc 2040
aaataaagtg ttaatatctc ataagtcact tctaaaaatt caccttaagg aaatcattaa 2100
ggtcatatgc aatgatttag tcacaaagat gtttgtaaca gaactgtgtt taataacgga 2160
agtttgcaag caagatgaat tcctagcaat aaggtactgg ttaggtaaaa tatgatacaa 2220
acctacaatg gaatgcaaca ggactattga aaaacaacgt tgcagaaata tatgtaccgt 2280
tatgaaaaga tgattgtgat gtgttattaa gtggcaaaat cagaatacaa ataatgcaca 2340
tggtttgttt ctatatacct gaagaaaaat gtaggagtat gtgtgtgtgt atttgtgtgt 2400
aattatgtag aaggatgtgt attccagagt taacaatggc catttctgat ggatttgttg 2460
acttttcttt ttctttcttt cttttttttt tttttttttg agacagagtc tcgctctgtc 2520
acccaggctg gagtgcaatg gcacaatctc ggctcattgc aacctccgcc tcccaggttc 2580
aagcgattct cctgccctca gtctctcaag tagctaggat tataggcacc caccaccacg 2640
cccagctaat ttttgtattt ttagtagaga tagggtttca ccaagttagt caggctgatc 2700
ttgaactcct gacctcaggc gatccaccca cctcagcctc ccaaagtgct gggattacaa 2760
gcaccgctcc cggatatatt tgttgatttt ttaaactagt gagcattcag gttgcatttg 2820
taataaaata aacaagtaaa ggaacttaat ttagggaaca tgtatcaaaa agtaaatgag 2880
catctcaaca gcaaagtggt caatgggcct aaggaaaata tgttcataaa ataaagtaca 2940
atgagccaat gatcaggtgt gtaagaattt attcttggta ataaatcagt atgtaattaa 3000
aacaagaatg taatgtctct ctttttattc ccaatccatt gaactggaat gctttccccg 3060
catgaaaaga atagagcctg gttgggagta cagggaaatg tacaagttca caggtaaaac 3120
tgtgctgaag aacaatttgt caataggtag acattcaaaa atgccaattt tcttttgtct 3180
gagttattat tattattatt attattttta tttttgagat ggagtctcac tctgctgccc 3240
aggctagaat gcaggggtgc cgtctcggct cactgcaacc tctgcctccc aggttcatgc 3300
agttctcctg cctcacctcc tgagtagctg ggattacagg cacaggccac cacaccaagc 3360
taattttttt ttcttttttt tttttttttt agtagagatg ggatttcacc atgttgtcca 3420
ggctggtctc aaacttctga cctcaggtga tcagactgcc tcggcctccc aaagcgctgg 3480
gattataggt gtgagccact gcacctggcc ctggtctagg caattatttt taaattattt 3540
ttatttttat tttttgagac aggatctcac tctgtcaccc aggctgtagt gcagtagtgt 3600
gatcatagct cactgcagcc ttgacctcct tgggctcagg tgatcctcct actcagactc 3660
cccagtagct gggactacag gcttgcacca gcatgcctgg ctaatttttg tactttttgt 3720
agagatgggg ttttgccatg ttgcccagtc tggtttcaaa atcctggctc aggtgatctt 3780
cccgcctcac cttctcaaag tgctgggatt acaagcatga gccatcatgc ctggtctgat 3840
tttttttttt tttttttttt tttttgaga ctgagtctct atctgttgcc caggctaaag 3900
```

tgcactggtg cgatatcagc tcactgcaac cttcacctcc cgggttcaag cgattctcct 3960
gcctcagcct catgagtagc tgggattaca ggcttgcacc accaagccca gatggttttt 4020
tgtgttttta gtggagacgg ggttgcacca tgttagccag gctggtctta cactcctaac 4080
ctcaggagat ccacctgcct caatcttacg aagtgttggg atggtgggag aataaaaatg 4140
atcaatgtgg ttccaggaag cgctgattca ttaggagctg gctttctttg tctctgttgc 4200
ttgaaataaa actagtcttg gcttcctgtc acctcagggg cagctgtgct gtagaagaca 4260
caggtgactt gttcctggtt ctgccacttg ctgatttgct tagacaagac ttctttgggc 4320
ctcggcttgc ctgtgtgact tgacaagtga gaattataac aatgtccctg cccgggagct 4380
gttcagaggc tgaagaggaa gaccagactc agtaaagcct ggaggctgcc aggtagggct 4440
ggggccagca tgacctgagt cctagcagga gaagaagagg cagcctagag tcttctgtga 4500
agtgcacata gaagagagac tggggccaag ccacaaaaga tagaatgcac agctgggccc 4560
taaagaagga ttttctaaga gaaaagaaaa agccaatgag acaggacagc tagatactgt 4620
gtggttagag tgagacaaga aaaatctgca tcttcataag ataaatccgg agagactatt 4680
gagagtctat tagtggcaga gaattgaatt cagaggggcc agttgtgctg ctgcagaatc 4740
cagaaaaaaa aagacagaag ccgacaaggc acagtggctc acacctgtaa taccagaact 4800
ttgggaggcc caggtgggag gattgcttga gcccaggagt ttgaggctgc agtgagccac 4860
agttgcacca ctgcactcca gcctagggtg acagagtgag actctgtccc gaagagagag 4920
agagagagat tgattgaaga gatggaagct tattccagag caatgaccgt gagcatgaag 4980
aaaggtgggt gggtgaatct ataggactaa tcagtagaca gacattggga agaggagaac 5040
tcaggttcat ggcctcagca agtggtggta cctgtcataa ccactcttga agccttgaag 5100
cctgttatgt caggcagaaa gtaggggaca aactttcaaa tcatacatct acaaagaaca 5160
ttcgtcatgc actccagcca tcctgaattg ctagcagtac atcaacctat gcacatacac 5220
actgttccct ttgctaaaat gctccccatt ctcttggaca aatggcaaac tccaacctat 5280
tttttgagtt tcagctctgg ccccacctcc ctggggcagt cttttcagcc tttgccaaat 5340
agatacctct ctgtgcttcc accacacctg ggttacccc ctctcattct gttctcacaa 5400
gatctaggat gtctatccct tcgttcactc attggttcta tatggagcta ctgatatgtg 5460
attcctgacc caatagaaaa tgatgagggt ggctgtttgt ctgactcaag cctttgtgaa 5520
atgactgatg gttctctagg ccttttctg ctgttctctc tgcctggaac actctaacac 5580
tctatctggt ttttatggct ggctccttct tttgtttttt tttttttttt tttcctttga 5640
gatcgagcct ctacctgtca cccggtggag tggagtggta tgatctcagc ttactgcaac 5700
ctccaccttc caggctcaag tgattctcca acctctgcct cccaagtagc tgggactaca 5760
aggtgcgttg ccaccacact cggctaattt ttgtattttt tgtagagatg gggtttggct 5820
acgttggcca ggctggtctc gaactcctgg cctcaagtga tctgcctgcc ttgggcttcc 5880
aaagtgctgg gattataggc atgagccacc acacctggac ggctggcttc ttcttgttgg 5940
tcaaattatc tagtatcaat taccttcttg ccccatttc tctttagctc tctgccgaga 6000
tttactgtct caatagcatt gaccattgtt gaaattacct tgcttattgg cttgtctgct 6060
tcttctgact agaatgtcaa cctcacaaaa gcagggactt gggctgtctt attgcactca 6120
tagtcctgag gactagcaca gtgcctggca cataagagga atttgataat gtttgataaa 6180
ggaatgaatg ggttttgtag ataaggatgc agatataggg ccaggcatgg tggttcatgc 6240
ctgtaatccc agcactttgg gaggctgaga tgggcagatt acttgaggtc aggagttcaa 6300
gaccagcctg gtcaacatag tgaaacccct gtctactgaa aatacaaaaa gtagctgggt 6360
gtggtggcag gtgcctgtaa tccccactca ggaggctgag gcaagagaat cgcttgaagc 6420
caggaggcgg agattgcagt gagcccagat cgtgcctggg tgacagagct agactccatc 6480
tcaaaaaaaa aaaaaaaaaa aaaaaaaaga ggatgcagat atagagaagg taaagactgg 6540
tactgagcct aaaattattt aattatagaa ccaatactaa acaaccatga gaactgcatt 6600
tacagaatgg aagtggtaat cgaaagacaa tagcacaact aggctagcgc agtggctcat 6660
gcctgtaatc tcaacacttt gggaggccga ggcgggtgaa tcacttgagc ccgggagtaa 6720
gccaggcatg gtggcacata cctactcagg aggctgaggt gggaggactg cttgagcctg 6780
agaggtggag gctgctgtga gtcgagtttg caccactgct cttcagtctg gatgacagag 6840
tgagatcctg tctcaaaaca aaaacaaaaa caaaaacaga aaacaacgaa caaatacagg 6900
tgaggtaaag ggagatgtga gaaaatgtgt gaatgtaata gcaaaaagtt gcctggactt 6960
tacaatgatg aggcctacaa gtaaaacatc atcatgccac tctttccttg cttttcatta 7020
tcttttctga acctttgtag ccttatttag aaagcaatgc ttctcatgtg gtaaataaat 7080
atttaaaaca caacaattcc tttagtttta tgttagcttc cttttcttct actaaaaaaa 7140
ttagatttct taaaaattgc aactatgaga cattaacatt gtaaaatatt tttcctatct 7200
atttgtcaat cctgcatcca tctatatatc tatatcaaga tctatgccta tatgtatcca 7260
tctgtgccca aaaaggcctc atctgatatt cagaaaaatt atttctggct gataaaattt 7320
gtttttcttc tctatttttt tttctgcaga gttttaatgt tttacaacgt gtatgtatgt 7380
gtgtgtgtgt atatatatat ataatatcac ttttgcaaga agaaaagtga cttttaaaaa 7440
atgaaagcag gcctgggcac ggtggctcac gcctgtaatc ccagcacttt gggaagctga 7500
agcgggtgga tcacgaggtt aggagatcga gaccatcctg gccaacatgg tgaaaccccg 7560
tctctattaa aatacaaaaa attagcaggg cgtggtggcg cacgcctgta gtcccagcta 7620
catgggaggc tgaggcaggg gaatcgctta aacccaggag gtggaggttg cagtgagccg 7680
agattgagct gctgcattca agcccggtaa cacagcaaga ctccatttaa aaacaaacaa 7740
acaaacaaac aaacaaacaa aacaaaacaa aagcagatat accaaaatgt ttattttgga 7800
tggtaggaat attaaaatat tattttttct ttgtattttt actgaaaaaa tgttttaatt 7860
ctaaaaaaga aattaataaa aaggaaaatg tgaatctaag attagtgcat gagagctgga 7920
gacccagaga gggcaaacag catcaaggtc aaagttgagt gagtcctggg tgaaagtgtc 7980

ttttcagcca ggggagattt tccatgcaga agggactggg catcaactgt caggacatgg 8040
acagagacgt ctgccattag aagctggtcc tctcatagcc caccagcatc cccaggagg 8100
acactggctt cctcaaggtc acagcctttg gtttctggaa aaaatgatta agcagtgaaa 8160
ggattaaggt ctcacagctc ctatcgcatg atgtatagat gcagaggttt ctcatcatct 8220
tctttttata ttttaaaaga tggatgacaa ctagatgcca actgcagctc aggcacctgc 8280
ccagtccctc tcagagccgg gcacaaaaca gacacctgtc tagcccctca cagagagcca 8340
ggcacaaaac agacacctgt ccagcccctc acagagagcc aggcacaaag cggagacttg 8400
tccagctcct ggcaaggagc caggcacaaa agagatgccc atgagtgtaa atgaatctgt 8460
gaaacaggca gaacaggagc atcaactgag taattgtttt ttctttttca aataagaaca 8520
tttgcgttga gaagccgcca cgggaagaga tgtgatgaaa cactacacag aaagagggag 8580
gagcctccgg gtaggttgga gtgagtagga tgtagggtag ccggcagggg agaagagggg 8640
caaccttctg ttccataata ttgataccac ccatttgtgt gtccaatttt tgctggtttg 8700
tgacgtcctt ctagggtcca tgagtaaccc ccaccccatt gcctagctgg gtgcctgact 8760
ccccttaagt gctcagtaga cgttgaactg gacgggcgct ggcgtggtgg ctcacgcctg 8820
taatcccagc aatttgggag gccgaagcga gtggatcatc tcaggttcgg agttcgagac 8880
cagcctggcc aacatggtga aaccttgtct ctgctaaaat tacaaaaatt agctgggcgt 8940
ggtggcgcgc gcctgtgatc ccagctactc cggaggctga ggcaggagaa tcgcttgaac 9000
caggcaggcg gaggttgcag tgagccatga tcgggccact gcactccagc ctgggtgaca 9060
gagtgaaact tcatctctaa ataaataaat aaataaataa ataaaataaa ctttgaactg 9120
aacaggggca gctcttacat ctgtccagtc tcgggtttat gcagatagaa caagataacc 9180
acatcccaca tgggaggcca gtgtccactt gctcctttgg aggaactact gggcatttaa 9240
gggactcgtt gaacacactc tgtaggtcaa ctctgcccgg agtggaggga gctgcgcaga 9300
ccgctggccc tctgcacggg tttgcaactc ggcttctgga aacagcggga gacgtcgaag 9360
gaggcgagaa ggtgcgcgcg cccttctcac caggccggcg gatgctgggt ggtacccaaa 9420
gaggcgctca gatcactgga gctggagctc agtcggcggt cttctgcggg aactgcacct 9480
atcccggagc atctctggcc ctcctatttc actgaactcg ccttcctcag caggaaagtg 9540
ggaagaccgc ttgcttccag gacggtgtgg ggagggtaag agggctcagg gaaagaccgt 9600
ggaaagctcg gcgcacaagc gagctggcat caccggggat tccctccggc ccggtgggac 9660
tccggcgcgc ttgtagcgag gacccccagc ccagggcaga gcctaccctc cgctccccca 9720
gtctgggccc ttggcggccg cagtcgccca agcccgcccc gcctccgcgc gcggccctct 9780
ccggctcagt gccctgcgct gccgggaagc aggctgaggg gcgcaccggg cggcgggcgg 9840
ggacctgggg aaggccggga gcgccgggac cgaggacgca cgcggcgggc cgggccgcgc 9900
gcgcctcccc cctcccccga ggcccgagcg cgagccgccg tgacgtcacg ggcaacccgc 9960
cagccccgcg ctcctccggg cagagcgcgt gtggcggccg agcacatggg cccgcgggcc 10020
gggcgggctc ggggcggccg ggacgaggag gggcgacgac gagctgcgag caaagatgtg 10080
ccccgggacc cccggcacct tccagtggat ttccttgcgg aaaggatgtt ggcggtccct 10140
gtgacctgtg gagacacggc cagatctgcc ctccagtaag ttccaatttt gtcccctgcg 10200
tttctggaaa ctctcgagac ttggccctga gtagggatgc gctgtgagta gtcggacgga 10260
ggaaaggggc tttcggaatc caatatgtgt gtgctaggcg tgcactgtcc tcatcaccag 10320
cctcaagact ggtggcgagt ttgggcgttt ggggaggggg aggggagggg gatgacaccg 10380
aatggccggt gcatagaggc aattctggat ttatgaactt gatcaaaggc atattaacta 10440
atttctaaaa atagacccct ctgcagtcag aacagatttg gagccatgat gtggtttgga 10500
ggcccctctg gcgaaactag aggtctgtga atgtgtgatc ctccctccat cctttgacca 10560
tcactccccg cttcctctca caccccctca gggctgggcg aaggtgattt ccaaaaactc 10620
agtaactccg tgttttgaga ctttctcagt ttttgcatcg catgtgaggg tttgttgggt 10680
gctcctaaac cctcatcatg gtcattcttc ttggctgggc tgttgattta attaggtttg 10740
ccttctcttg ctaacgtgct cttatttatg ccagtgtttg tggttctggg tgtagacttt 10800
caggaagcac agccatgaag ctgcatgcgt ctgtgtgtgg atgcctcatg ccttttcctg 10860
gtggtttgca ggcatggaag attcttctgt tcatctaagc tcctgacttt aggccccttct 10920
agaacattag agaggagaag aaatcacgtg caaggattta gggacaagag gatgaggttt 10980
tggtgtttcc tttctggtat taggttttcc agagagaggc tgttagcctg tctctcgccc 11040
actccaccac tgtgaatcct ggctactggg gctagttcct tgctaggaaa aaaaaactct 11100
agagaaatgt gtttgtgtgt ggtgggtggg cgggggccct gaggatgcag ggactgtgtc 11160
tgctgtgttg tctgttataa gatgtttcag acctattttg catactggca gcgacaagtt 11220
gagacttgtt caacttgaca cagtcctgtg gtcatagcga atctttctaa agctctgatc 11280
agtcaagaag ggggttgtat caatcctcag aaccctgagt ggaactttct acaggattta 11340
ttaggagaaa aaccttcccg gaagctgcag aaggacaaat acagaatccg tgtctgggag 11400
aaacctcgtg gcctggtctc cattatttga gatgagttac atcttggagg tgaggacgtg 11460
cctcgtggtc taaagcttcg gcacaagggc ccaactggaa ttccacttac gggtatgact 11520
gtggggtata tgctgtaccc attgaattcc cagagggatt tgttaagtgg ttgtgttgct 11580
gttttgcctg gttaagagtg gcttattctt gttgcctcat ctatcttgag tgcagaattt 11640
ttgcataaac ggcttctctt gaaaaataga agtccgtttt tgttttgttt tgtttttttt 11700
ctgattaact ttccttttcc tctgtcttaa acaccttccc ccgcccctcg ccacctccat 11760
gctgtgtttc tgtggctgga gcttttctgc actggaaagg aggagttctc ccctctgttc 11820
tgacatatct gatccccact agtttatatg attgctggtc tgaaaacctg gtgaattctc 11880
actgcccaca tcaaatcaat tccctgttct ttagttgttt ttttttccg cctcctgagc 11940
ggtaggcagg cactctggct ggattctgga gcctgtgttt gccatgcctg ggccttctgc 12000
ctgcccaaga tggacacgtt tctctggcag cttcgtcaag agatctcctg gactataaga 12060

cacccctgttc tttcctcctg tttgtgtgag tggccctgca tttgggcaga gtattggcag 12120
aatatctggg ggaagtggaa gactttggat agttgccggt ccaggtcgtc tggagaggca 12180
gtggcaggcc tgctgatggc tgcccctttg ctatttgttt tatatcaggt ggcccttctg 12240
gatgtgtggc ctttttattt tgcatttcct ttcatagttg gctgcagaca taagattctt 12300
ctttttctct tgttttgctt ttttttttt ttttttgaga cagagtttca ctcttgttgc 12360
ccaggctgga gtaaaatggc tggatttcag ctcactgcaa cttccgcctc ccaggttcaa 12420
gcgattctcc tgcctcagcc tcccaagtag ctaggattac aggcatgcat caccacaccc 12480
agctaatttt gtatttttag tagagacgga gtttctccag attggtcagg ctggtcttga 12540
actcccagcc tcaggtgatc cgcccgcctt ggcctcccaa aatgctggga ggcctcccaa 12600
gttgctggga ttacaggcat ggaccaccat gcctggctaa ttttgtattt ttagtagaga 12660
cggagtttct ccagattggt caagctggtc tcgaacatct gacctcaggt gatctgccct 12720
cctcggcctc ccaaagtact gggattacag gagccacagt acccggcctt gttttgcttt 12780
ttattgtttc ctgatgcagg tttgtgatct ctttgaggga tcaggaccac acccccccacc 12840
ccaatttata ttcccagtgt ccatggtggg accctgactg aggatgggaa cagagcaaca 12900
atctggaatc atttggacca ttgagtcctg tggacagtca cttcaatatc atccctagag 12960
agtctcagaa attcaaggcc ttttggaatg ctgccaaaat gggtcctttg gcaggagccc 13020
cctttgtctc ttctgttttc tgacctcaaa taatctaagg aactaataac ggtaataaat 13080
aacaattaat aataagaaca ataatagtgc atgtataaca tggacattgt ttatcacgca 13140
aagcatgttc ttcaccttct actccctaca actccatgag gcatttataa tgaaatgtct 13200
agcaatggta gacttcttgt tgtggggcag ggttggttct aacacttcaa attatatgat 13260
ctcatttaac tttcctataa aataggatga ttattgttgt ggttttgcag atgaggaacc 13320
cacagcccag agaggttaaa aatatcctcc caggtcacac agcttgggag aggcagagct 13380
gtagagattt ggaccacagt cttcgacttc ttagtgtgac tctcagaagt tagaggataa 13440
tgaggttaag aacaagcaaa gggccgggcg cggtggctca cacctgtaat cccagcactt 13500
tgggaggctg aggcaggtgg atcacctgag gtcaggagtt cgagaccagc ctggccaaca 13560
tggtgaaacc ccggtctctac tacaaataca aaaaattagc cgggcatggt ggcgcacact 13620
tgtaattcca gctacttggg agtctgaggc aggagaattg cttgaacccg ggaggcggag 13680
gttgccggga gccgagatcg caccattgta ttccagcctg ggcaacaaga gcaaaactca 13740
gtctcaaaaa aaaaacaaca agaaaagggt atacccccttg gccttgagca ggagaacaca 13800
gtgtgtcccc caccctcacc gtgcacctta ggcacaaaca gaaagctgaa catcttgcat 13860
gaagatacct catgctctgc ctgccgacct tttctgcagc cacggagcag tcagagacaa 13920
aggggccaag aggccttagg tgagggctgc catgctggaa agtttgggct gtgggtgtgg 13980
gcaggagtca tgggtagagg tcagaaccct tagtcttggc tgggtgcagt agctcacacc 14040
cgtaatccca gcactttggg aggctgaggc cggtagatta cctgaggtca ggagttcaag 14100
accagcctgg ccagcatggt gaaaccctgt ctctactaaa aatccaaaaa aaaaaaaaaa 14160
aaaaaaaaat tagcccggcg tggtggcaca cgcctgtagt cctagctact cgggaggctg 14220
aggtaggaag atctcttgaa cccgggagac ggagtttgca gtgagctgag attgccagtg 14280
cactcctgcc tgggagatag agtaagactt tgtctcaaaa aacaaacaac cgcctcggtc 14340
tttgtgcaaa atctataatc atttgcaaga ggctgcaatc aggtatagca ctggattttg 14400
tgaccaaaac taggtttcag gatttaatta aaaacaaaaa caatgtcagg tcataaaaca 14460
caagacatat acatgatgtg gaaatggtct tattcatttt ttaattgcga aattccgaac 14520
tatctgtttt tttttccttt tatcttttcc tttttgtgtg tgtgtggagt taggtggggg 14580
gggaggtatg gcatattgtt ttgtttatct gcctctttat tatcactgac tgtgaaattc 14640
ttgagaacag gggcgtctaa gtgcccaaca cagccctggt actttgttga atcaggtggc 14700
tgtcaagagc tgctgcttca gcccggcatg gtggcttatg cctataattt ttgcactttg 14760
agaggctgag gcaggaggat tgcttgagct gagaagtttg agaccagcct gggcagcaga 14820
gtgagactct gtctctgtaa aaaataataa aataaaaatt aaaaaataaa gaaagaactg 14880
ctttgtcttc aaggaaacaa gatacacagt caacagtcat tatcttttta gatatgagac 14940
agcttgacat ggtgtggaag tggggggctt gcctgtaggg aaagtatgct gatgaaaaag 15000
aaggaaaaca gacagtagca ggaggcagtg gggatgtgtc agggagctga ggagtgtaga 15060
tctggttact tttgggcaag ttacttctct aagcttcagt ttcctcatct ataaaatggt 15120
ttgctgtgat gacgaaagag atgatgaaca gaatgtgtct ggcagaggac ctgggctcat 15180
aagtgagcac acagtaaaag gtagctaata tcattactgt tccaattatt gagctttatt 15240
acttcctgtg gctgcatgaa gtagtggttt ctgaatagaa aggcaacatt ctagttgcct 15300
agatcttaag atggcagtga cttcactgta gttctctgca agcatcagaa acaagccctg 15360
gcttatgtaa gttagaaaca gatgagaaag taaataaagc tgcagaagtg ggtctcagga 15420
aggacaggaa tcaaagggct gtccctcgga tgtcagactg cctttgcaag ttacttcagg 15480
tcactgtgcc tccatttcct cagctgctga gcaggggagg agaatcactt ctccctccct 15540
gggatggtgc agagcactta gcagagcact tagcacagag gaataagccc ttcagtaaaa 15600
catttgagta aagcaattat tatttattat tcttcagggc tctttttttt tctggtgcat 15660
tagccctaac attgactact tggttcaaga gtgatggatg cattcatttc ctgagcagtt 15720
gctgtatgcc tggcaccgtt ttagctgctg gcgatccagc agggagcaga agagacaaaa 15780
atccctgccc tcctgaagct tctgtcctag tggatgtcac acagtagtgg ggcaggctgg 15840
tccccagaga cagctggggg acagatggag gcagtgcagg caaaagctaa ctcctgtgca 15900
aagtctcccc ggccccactg tgttaacagt aaagtgctta gcagaggctc ccacacatag 15960
tagttactat gtagtggtag ttgctatgtt tattattatt atattattct cctccctcat 16020
aaaatggcag cctccctttc ttccattccc cttcctgagt tatttttttct cgatagggct 16080
tatcttccta gtataccata tgtctctctt ctcccactaa attgtaaccc attcattttt 16140

```
ccaggatata tttgttgggt gtctactctg tgcctggcat tgctgtagat gttggggatg 16200
cagaaaggaa gaaaaaggac agatttccat gctagagctt aggttacagc tgggttagac 16260
agataaggaa cagcagacat aataaagaag gacatgaatt gtgtattcaa gattatgaag 16320
tgctgtggga acaggcctag cagggtcagt gtctggaggg ctggggcgtg gtgctgtgtg 16380
gttaagttgt ccggtcagag tcagggcagg ctccatggag gagctgacgt taacctccac 16440
ctgaagtagg ggaaggacag gcgtttcagg ctctgggaag agctaggaca gaggtggagg 16500
cagaagtgag cctgtggggg tgaggagcag ccaggagaag cgtgagatga gaatggcgtg 16560
agcaagggag gtaggagaac ccgagcagca tgcagtgtgc agcctcacag agagcctgca 16620
gggcattcca aagattctgt ctttactctt gtaaaatggg gagcccttgc ggtgtttttt 16680
gtttttgttt tttgtttgtt atgaggcaga gtcttgctct gttgcccagg ctggagtgca 16740
gtggggagat cttggttcac tgcaacctct gcctcctggg tttaagcaat ccttacacct 16800
cagcctcctg agtagctggg attacaggtg cgtgccacca cgcctggcta atgtttttat 16860
ttttgtagag atggggtttc accatgttgg ccaggcaggt ctcaaactcc tgaactcaag 16920
tgatccaccc acctcagcct cccaaagtgt tgggattaca ggtgtgaacc actgtgactg 16980
gcttccttgc agtgttctaa aaaggaaagt aatttgatct gacttggctt ttgaaacggt 17040
cagtctggct gctgtgttga gaagaaacta aatcagggtc aggagctaga gcaccaggac 17100
aaacatgcac agagcgttaa gtgccaggtc caggctggca gtggtggatg gggccacagg 17160
aatggacttc taggctgttc actgttgtat ccccagcatc ttaaactgtg ctctccatat 17220
ggtagctgtg agccctatgt gggtattgag cacttgaaag gaaattgata tgtactgtgt 17280
gttaaacgca cattggattt cagagactta gtgagaagag taagatatct cattatttat 17340
ttatttattt atttatttat ttatttactg agacagagtc tcacctgttg cccagcctgg 17400
agtgcagtgg catgatctcg gctcattgca acttccgcct cccgggttca agcgattctc 17460
ctgcctcagc ctcccaagta gctgggatta caggtgccca ccaccacacc tggctaattt 17520
tgtattttta gtagatatgg ggtttcacta tgttggccag gctggtcttg aacttctgac 17580
ctcaggtgat ccatcccact cagctttcca aagtgctgga attacaggtg tgagccactg 17640
caccaggcct atttgtttat ttatatgaga cacggtctca ctctgtcacc caggctggaa 17700
tgcagtgatg tgttcataga tcattatagc ctcgacctcc caggctcagg tgatcctccc 17760
acctcagcct cctgggcagc tgggaccaca ggtctgtgtc accacgccca gctaattttt 17820
ttgtattttt ggtaaagctg gggtctccct gtgttgccca ggctggtctt gagctcctgg 17880
gctccagcaa tctgccttcc tcagcctcct atagttctag ggttacaggc gtgagccact 17940
gcaccctgcc aagttatctc atgaaccaat tctttgatgt taattacata ttgaactgat 18000
aatgttttgg atatattggg ttaaattatt aaaattaatt tcatctgttt tttctaactt 18060
tatggctact agaaaatcta aaattacatg tgggttgaat catattttcg ctggatggaa 18120
caatgccttg gacgaggtca gcatttagtg aatcaaatgt gtgggtgagg cacaggtgtt 18180
ctgccgtgaa tgagaaaccg ttgccatctt cgggaagctt ccagtgtggg agggaggcac 18240
actgcagatg taactgctag gagagaaaga aggcaggttg aaatgggacc acagccaacc 18300
aggcctgctg tagcccagaa gctggaagag actcgcccag gagggaagga agacactggg 18360
gtgttgggtc tcagtctggg tcttggtgac agcagtgggc tggcagtgca gaggttcaag 18420
gccttggact gcaagtccac taaggtggct ggtggtgtgg gtggaggtgg aaaaggcagg 18480
tggggcgagg gtgaggggca ggacatcctg gagagcctga ataaccaggt ataagtgatt 18540
tctttttctt tctttctttc tttttttttt tttttttttt ttttaagacg gagtctcctt 18600
ctgtcgccca ggctggagtg cagtggcgag atctcggctc actgcaagct ccgcctcccg 18660
ggttcacgcc attctcctgc ctcagcctcc cgagtagctg ggactacagg cacccgccca 18720
ccacgcccgg ctaagttttt gtatttttag tagagacgag gtttcaccat gttagccagg 18780
atggtttcga tctcctgacc tcgtgatccg cccgccttgg cctcccaaag tgctgggatt 18840
acaggcgtga gccgccgcgc ccagccccag gcggaaatta tttcaactgg gtcctgagaa 18900
ctgggcactt ttcaaggatt gtcagcaagg agcaacttcc caaatataca tctaaaaaga 18960
tgacaaactc atgtggaatg tggatttaag gaggaaggta cttgacttaa gaattccagg 19020
attttggtgg aagaaggaaa tatgggccag gcactgtggc ttatgcctgt aatcccatca 19080
ctttgggaga tagaggtggg aggattagtt gagcccatct gagaccagcc tgggcaacat 19140
agtgagatcc tgtctctaca gttttttttt ttttttgaga cggaatcttg ctctgtcatc 19200
aaggctggag tgcagtggtg agatctcagc tcactgcaac ctccgcctcc tgggttcaag 19260
cgattctcct gcctcagcct tccgagtagc tggagttaca gccttgtgcc accatgcttg 19320
gctaattttt gtatttttag cagagacggg gtttcaccat gttggtcagg ctgatctcga 19380
actcctgacc ttgtgatctg cctgcctcgg cctcccaaag tgctgggatt acaggcatga 19440
gccactgcac ctggcctcaa aaattcttat ttttaattaa ctgggcttgg tggcatgtac 19500
ctgtagttcc atctttggtg gcacgcacct gtattcccat ctacttggga ggctgaggca 19560
ggaagcttgc tcgaacccag aagtttgagg ctgcagtgag ttatgattac accactgtac 19620
tccagtctgg gtgacagaat gagaccctgt ctctaaaata aataaataaa tttttaaaag 19680
ctgggaggtg gggttggtaa attcataaaa aataatattt cttgagttga atacaaacaa 19740
tacaatcact gtttttttctt tgccttgttt tatttttact tatttattta tttttgcaga 19800
cattgggcct cactcctagg ctggtcttta actcccggct tcaagtgacc ctcccacttt 19860
ggcctcccaa agtgttgaga ttacaggtgt gagccactgt gcccaggctg tggtttttta 19920
tttttaatt ttttattttt ttgtattata atcatagtgt atatgttgca tcctgatttt 19980
tttttttttt ttttttgaga cagcatctca ttctgttatc caggctggag tgcagtggca 20040
caatctcagc tcactgcaac ctctacctcc caggttcaag cgattcttct gccttggcct 20100
ccgagtagct gggatgccac cgtgcccggc taaatttttt ttctttcttt ctttttcttt 20160
tttttttgt atttttagtt cagacagggt ttcaccatgt tggccaggct ggtcttgaac 20220
```

tcctgacctc aagtgatccc acctcggctt cccaaagtgc tgggattaca gcgtgagcca 20280
ccacatctgg acacatcctg attttttttct tagttgctat taggacacag gcatttttga 20340
atattatatg cactttgtcc atgtcttttt tttttttttt tttttgagac ggagtttcac 20400
tcttgttgcc caggctggag ggcaatggcg cgatctcggc tcactgcaac ctctgcctcc 20460
cgggttcaag tgattctcct gcctcagcct cctgggtagc tggcattaca ggcgctcacc 20520
accacgcccg gctaattttg tgtttttagt agagacgggg tttctccacg ttggtcaggc 20580
tggtctcgaa ctcctgacct caggtgatcc acccgccttg gcctcccaaa gtgctgggat 20640
tacaggagtg agccactgtg cccagccgtc catgtcattt ttattgggta gatatttctg 20700
ttcagtgact gtttcgttgc ttactttatc attcctttga tttggatgct tagcttcata 20760
acagcctgtt tatatagatt atgttttcta tttttcagtt aattaaaaaa aaaacaaatt 20820
ctcagtccta ggattattgt gcataatgtc tgaatttttt agaatttcag gtaaatattg 20880
tggttcatga gatttgcaca gaagctgaat ttgctgcttt ctggtgaggc acgtgatgtt 20940
ttttgctgtc cgtgatgcac taacatgtca ttaacatgta tgtacagtgt tatatgctcg 21000
cacatgttta tgtgcagcct atgcacctgt attggatttt tcttcactct catttattgg 21060
caggtatttg tggaatgcct gtggtgtgcc aagttctgct tgaggccttg ggtattcagt 21120
agtgatcaaa gtagatcaac tagtaccagt tcttaagagg cttattgtta agtgggggaa 21180
acaagcaaat agacaaagaa aaaataacat tattgtaagg aatatgaaat aatatgtact 21240
agaataatat gaagggatgg ttattgtgag gtaagggggt tcatactgaa atactgtctg 21300
aggaatcagg atttgagtga gacacgaaga ctcagtagaa gttaaccaga agaacaaagt 21360
gttagcagaa aagacaagat tgatcaatat gactacatat taaagaaaaa ggcacatcta 21420
agcaacaaaa actgcaatgg tattaaaagc caaatacaag acttagaaga tatttgcaat 21480
atttataaca cattacaaaa cactaatatc tagaatgtat gcagaattcc caaaagtcaa 21540
caacaaaaag acaaacagcc ttatagcaaa gtgggtgaaa gataggacca tgcagtgctc 21600
atagaaagca aagtagatgg ctgggcaccg tggctcatgc ctataatccc agcactttgg 21660
aaggctgagg cgggcggatc acaaggtcag gcgttcgaga ccatcctggc caacatggta 21720
aaacctcatt tctactaaaa atacaaaaat tagctgggtg tggtggcgtg tgcctgtagt 21780
cccagctact tgggaggctg aggcaggaga atcgcttgaa cctgggaggc ataggttgca 21840
gtgaacccag atcttgccac tgcatgccag cctggacaca gagaacgact ccgtctaaaa 21900
aattaaaaaa aaaagcaaag tggatgataa atgtgaaaag attctgaatt ttgctaacaa 21960
agtcaggttt tggagatcta atttctagtc agtaaaattc atcctttcta ggatacaatg 22020
ctaagagttt tgataaatgc atagaactgt gttacactgt tgtgtaatta ccacaatcat 22080
ggcttagaac atttccatcg ttcacccaga ttccctcctg tcccttcaca gccagtctcc 22140
ttctccaccc ccagctttaa ccaacccctg agttgtttgc tgaccttaca gtgttgcctt 22200
tttcagagtg tcctataaat ggattcatac agtgtgtagt ctctcggatt tgtcttcttt 22260
cacctggcat aatgcattcg ggacccttcc atgttgttct atgcatcagg ggtttggtag 22320
tttttattgc tgactgggac tctgttgtat ggatatacca ctgtttgttt atctgttctc 22380
catttgaaga gcatctgggt gtttccacaa gatacatgtt tcatgcttta caacgtgcca 22440
gaaataggat gtctgactca agtgttgaca aggcacaggg tgttcagcag ctccgctagt 22500
gagtgtgtga ggtcacttta gagaccacct tgccagcagg tagtgaaatt ggaaacatgt 22560
gcagccccgt tcctaggtaa gattgagaat tcacagttcg tgctgtatgt tacttaagga 22620
tatttagaga tgaaagtgaa ggtttaaaaa atgggctgga aagatctata tcaaactcac 22680
tgtcatcatc tggggagaga aggggagtgg gctgattgtg aaggtcaaag cctactttaa 22740
ctttgccttt attgcttttt ttaaattcaa ttaattaatt tacttgtggg gacagaatct 22800
cactctgtca aggttgctgg agtgtgagtg gtgtgatcgt ggctcactgc agcctctact 22860
ttccaggctc aagtgattct cctgcctcag cctccggagt agctgggact acaggtgtga 22920
gccaccatgc ctgtattttt tgtagagatg agatctcact atattgccca ggctggtctt 22980
gaactcctgg cctcaagcca tcctcctgcc cagttctccc aaagtgctgg gattacaggg 23040
aggacccagt gtctggccta tctttaatgc ttttttgaa aagaaggtgg atacctacac 23100
atatcactta cacagtttga aaatatgtag aaaagaaaaa aagtttgaaa atacatatga 23160
caagnnnnnn nnnnnnnnnn nnnnnnnnnn nnnnnnnnnn nnnnnnnnnn nnnnnnnnnn 23220
nnnnnnnnnn nnnnnnnnnn nnnnnnnnnn nnnnnnnnnn nnnnnnnnnn nnnnnnnnnn 23280
nnnnnnnnnn nnnnnnnnnn nnnnnnnnnn nnnnnnnnnn nnnnnnnnnn nnnnnnnnnn 23340
nnnnnnnnnn nnnnnnnnnn nnnnnnnnnn nnnnnnnnnn nnnnnnnnnn nnnnnnnnnn 23400
nnnnnnnnnn nnnnnnnnnn nnnnnnnnnn nnnnnnnnnn nnnnnnnnnn nnnnnnnnnn 23460
nnnnnnnnnn nnnnnnnnnn nnnnnnnnnn nnnnnnnnnn nnnnnnnnnn nnnnnnnnnn 23520
nnnnnnnnnn nnnnnnnnnn nnnnnnnnnn nnnnnnnnnn nnnnnnnnnn nnnnnnnnnn 23580
nnnnnnnnnn nnnnnnnnnn nnnnnnnnnn nnnnnnnnnn nnnnnnnnnn nnnnnnnnnn 23640
nnnnnnnnnn nnnnnnnnnn nnnnnnnnnn nnnnnnnnnn nnnnnnnnnn nnnnnnnnnn 23700
nnnnnnnnnn nnnnnnnnnn nnnnnnnnnn nnnnnnnnnn nnnnnnnnnn nnnnnnnnnn 23760
nnnnnnnnnn nnnnnnnnnn nnnnnnnnnn nnnnnnnnnn nnnnnnnnnn nnnnnnnnnn 23820
nnnnnnnnnn nnnnnnnnnn nnnnnnnnnn nnnnnnnnnn nnnnnnnnnn nnnnnnnnnn 23880
nnnnnnnnnn nnnnnnnnnn nnnnnnnnnn nnnnnnnnnn nnnnnnnnnn nnnnnnnnnn 23940
nnnnnnnnnn nnnnnnnnnn nnnnnnnnnn nnnnnnnnnn nnnnnnnnnn nnnnnnnnnn 24000
nnnnnnnnnn nnnnnnnnnn nnnnnnnnnn nnnnnnnnnn nnnnnnnnnn nnnnnnnnnn 24060
nnnnnnnnnn nnnnnnnnnn nnnnnnnnnn nnnnnnnnnn nnnnnnnnnn nnnnnnnnnn 24120
nnnnnnnnnn nnnnnnnnnn nnnnnnnnnn nnnnnnnnnn nnnnnnnnnn nnnnnnnnnn 24180
nnnnnnnnnn nnnnnnnnnn nnnnnnnnnn nnnnnnnnnn nnnnnnnnnn nnnnnnnnnn 24240
nnnnnnnnnn nnnnnnnnnn nnnnnnnnnn nnnnnnnnnn nnnnnnnnnn nnnnnnnnnn 24300

nnnnnnnnnn nnnnnnnnnn nnncgtggtg gctcatgcct gtaatcccag cactttggca 24360
ggctgaggcg ggcagatcac ttgaggtcag gagttcagga ccagcctggc caacatggtg 24420
aaaccccatc tctactaaaa atacaaaaat tagccaggag tggtggcagg tgcctgtaat 24480
ccgagctact tgggaagctg aggcaggaaa atagcttgaa cccgggaggc ggaggttgca 24540
gtgagctgag attgagccat tgtattccag cctgggcaac aaagtgagat tccatctcaa 24600
aaaaaaaaaa aaaaaaaaaa aaaagaaaga aagaaaagaa aaagaaaagg acttcttgga 24660
gggcaaagtt tgagggaaga gttagctagc tagagacctt ccaaactgtg ctcaggcatt 24720
tggattttac cctgtgggtg atagggaact attgaaagtt ttcatttttt tctttttgga 24780
gacagagtct tgctctgttg cttcaggcag gagtgcagtg agccatagtg gctcattgca 24840
gcctggaccc cctgtgctca agcaatcctc ccacctcagc ctccagagta gctgggacta 24900
caggtgcatg gcaccacccc cagctaatgt tttgttttta attttttata aagatagggt 24960
ctcactttgt tgtccaggct taaaactcct ggctttaagc gatcctccca tcttgacctc 25020
ccaaagtgtt ggaattacag gaatgagcca tcatgctcgg ctctatggaa ggttttgagg 25080
tggggcaggg atgagattag agtgatgtgc ctttgccaga gagatttgtc cagaggaagg 25140
gtgaccagcc tggagtctct ttggaatgtt ctaggtaatg tagcagagat tgtgtcagtt 25200
accctctggt gggatgcttg gcttttccca ttttttcaaa aggaaatggg aaaaggaaaa 25260
ggtcctctgt atggaaagtc cccaggaatt tataggagag tggctaacga agacagagtt 25320
cccttccggc atttattgtg actgagtttg ttccatagtt ttagtcccac ttaggtcatg 25380
attcatccca ctgggccagc agcagcattg ggatcacgtc agccaatctt cgcacttaga 25440
gattttgaag agagatatct cctatccatg tggtacattg cttttcacaa aacaaaagag 25500
ttcattttgt cggtgaaaag ccctggccag acccagccgg tggagaccag tagcttcctc 25560
tggcaggatg ggcttcctat ggtcattgca gtccctcccc atttataagg ctgccttccc 25620
tgagaacaca tgggacagat gccccccaga agcatgccct ctggaaaggg agaggagcag 25680
gagggggctt caggcatgct gggcctgggg ggccaccccc ctggcctttg ataagttcct 25740
ggctggtatg agccatcttc tttcctctcc ccaggccttg caaaggccat atctcttcct 25800
tttcatgact ctggaaattc catacatact ttctgcatta ggaagacatt ctctgcatgc 25860
tgatctcgtt aaaaaggaaa agccaaggag catcctgcac ctccctcctg taagcgagct 25920
ttgcaaatgg taaactagat cctgaaaggc aaccacttaa aagcttttaa tggcttccca 25980
ttattagctt gagtgcctgg tgcgtcagag gctctcagtg agcgtgtatt gaattgctga 26040
atgcatggtg gccctgggcg aggaatctgg ccctcactgt cccaaggctg agtgcctttg 26100
ccctgtggag gaaagcactg ccttggggtc agaaataagg attggcagga ttgcctgggg 26160
ttagaaatag tgatcatggg atcagaggca gcaggtgaat gtggggtgtg aatattttg 26220
aaggcttgga atgaaatgat tcttccagac tttagaggat ttgtgtctgc agattcttct 26280
ctaaggtttg tgtctcacat agtgagagaa tcagccagcc agggaaatgg gacaggattt 26340
tgtctctttc cccatttcct cttctaatga actcctacct atgctttatg actgcattaa 26400
ggggtcacct cctccaggaa gcattccctc atctccttac gctggttctg agtgtgtcgt 26460
tcctgcttga tcccctccca gcaccatgtg tgtgagttct cacagctcac accccatagt 26520
attactatgt tgtttctaca gtgggttcct gtcaggggtt ccaaacttag ctgtttagga 26580
ggatgagtag attgtacaac cacaccaggc gggcctgatc aaatacagta agaagtgtcc 26640
ggattggcaa accagcgggt ccctcttcta gatgttatca gagagatact gttagagaga 26700
ggttgctaga tgttaataga ggctgctgtt agattccagc tccctcctcc cctggtccct 26760
tttctctcct catgccaaaa gatagaccca gtgttgccag agcttccact tttaaaagag 26820
aagcctttta aaatataaac tctcctgatt tttaaatgtt ttcagttaag tttttaaagg 26880
aacattcata ggccaaacca aatacctgtg ggccgtatgt ggcccccggg gccagcagtt 26940
tgcagctcct tggttatagg tgcctctaac ccattgcaca agctccctca ggcatttcat 27000
gtctttcctt tccattcccc ctgggcctag cagggagctg gcatgtcaca gtgggtgctc 27060
cctaattatt ggttattgac ttgaatgcta gactgagcaa gcacctccct gctcacaggg 27120
tgttaacatt tttccctcag aactgtttgt atgtaatgct attctgaatt gcttcagttc 27180
cttacatact ttagtgatag tctgtatgtg cctggaaaag tcatcattcc ttagattcta 27240
gacaactctg ttcttctgtt cttttccttt ttcctttctc tctccgtccc ctcaacatat 27300
gtttttgttt tttgtttttt gccagttctt ttgtatctgg ccggttaaca cagggcttca 27360
taacatgggg tcggctgtta ttaagcaaca gtggtttccg gtagctcact gactttgccc 27420
tctctgctgg taagccattg ctgatttcca tcagggtaac aagcatgatt ttcagattta 27480
ccttcatttg cagtgaacaa ttatagcaaa attgcttaaa gaaatgaacc ttcaggtgtg 27540
gcggctcgtg cctgtaatcc cagcactttg ggaggccgag gcgggaggat cacttgagcc 27600
caggaggtct ctaggctgca gtaagctgta gttgcaccac tgcactccag cctgggtgac 27660
agggtgagac atcgtctcaa aagaaaagaa aggaaaagaa aagaaaagaa aaggagggga 27720
ggagaggggga ggggagggga agagggagag agggagggag ggagggacgg aggaaggagg 27780
aaaggcagga aggcaggaag gcaggaagga aggaaggcag gaaggcagga aggcaggaag 27840
gcaggaagga aggaaggaag gaagagaatg aaaggctggg cgtggtagct cttgcctgtt 27900
gtagtcctgg cactttggga ggctgagatg ggaggaattg gaaaccagcc tgggcaacat 27960
aatgagactt catctctaca aaacaaattt ttttttaatt agccagtggc atgcacctgt 28020
ggtcccagga ggccaaagct ggaggattgc ttgaggatgg gaagttgagg ctgcagtgac 28080
ctatgttccc accactgtac tccagccaga gtcacagaac aagaccttgt caaaaaaaga 28140
aaagaaagaa aaggagagag ggagggaagg aaggaagaaa aaaaagaagg aaagggagag 28200
aggaaggaag gaaggaaggg aagggaaggg aaggaaggga gggaagggag gaaggtagga 28260
aaatggaagg aagggaggga aggagggaag aaaagaaaga aggaaggaaa aaaccggtcg 28320
gctatcttga cgaaaaaagt ctcagtccac tccattttgt gcagcatttc ccgtcttgtt 28380

atctttctgg aaaaactcag taagtctctg gctgggaatt atagaagctt aatatgcact 28440
tgtaactgtg tcaggctctc acagggaaca aacaagagga actatagatt tttatagtct 28500
gcaagagccc agtccatgca tttaggaggc acagagtgag agaaagagca ttgacaatga 28560
taatatagca ttaatatagg tacaatctgg agtaatgtta tctagtacta ctaatagcga 28620
tattaaaatg gcattatata gatattaagc cctgacttcg tgccggtttg aacttttcct 28680
gtattaattc atgtgaatca attttgagac tctgtctcaa aaaaaatata tgtatataat 28740
atatacaaat atatataata tatacaaata tatataatat acaaatatat atatataata 28800
tatacaaata tataatatac aaatatatat aatatataca aatatatata tataatatat 28860
agatgtcatg gtgatgggaa gactgggga ggagaggagc agtttaatcc caggatccca 28920
tggagaggaa acttgtaggt ctcttaccca gtgtcttctc ctgctctctg gctctgattt 28980
ctgaactaag aaaagtgtgc aaacatcatc tgctctcctg gttgtatttg taaatgcctc 29040
ccctagaccc agctctactt taggccacgt gagtgacagt gaccacatca gtcagggatt 29100
ctcctactgt ccctgaccca tgtccgcttt tgtctcctcc cactctaata acagaccgtg 29160
tggcttgaac ctcagccctg ctgccaagta gctgtgtgag cttcagagcc tggtgcctca 29220
gttccctcat ctgtaaaatg ggaatacaaa tggtccctgt gtgtttgggt attcaagaaa 29280
attaagtgag ttgctgcaca cctcacctct ggctccttca acaaaaacat ggtcaccctc 29340
aaaccaaacg tgaacaggag ggtggtgacg caaaccacat ggaaacgaga ggcagaccca 29400
ggctggagtg caattgcacg atctcggctc actgcaccct ctgccttcca ggttcaagca 29460
attcttctgc ctcagccttc tgagtagctg ggattacagg cacctgctgc caagtctggc 29520
taatttttgt atttttaata gagacggggt ttcgctatgt tggccaggct ggtctcgaac 29580
tcctgatctc aggtgatcca cccgccttga cctcccaaag tgctgggatt acaggcatga 29640
accaccgccc ccggccgcag atgggaaatt atttttgcac tttcagttgt cctaaatcta 29700
gagttttcct ttatccagac agctggtgaa agtggacgtg agaatagaga gatttcctgg 29760
tggggaagac ctccatcctc cttctccagc cccttcctca ttattcccaa gaaaagagct 29820
tctgggtttc tgggagaggc tgagcaggtg gagacacctg ggccatatct gggtctctcc 29880
agaatccagg agcctgcctg acgcgtcagc cggggagggt ttctgtgtga agtcggcttg 29940
agtgagccag cctttcatgc atccttgtct ttatttcttc cccagctcag gaggggctga 30000
gttgtgtctt aatgagcagt tccagctgca tgctgggcat ctcctaattt actctcattt 30060
atttagctcc tgctgcatgc tgggcaatgc tctaagtgct ctgcaggttt tggctaattt 30120
aatctcccta gcaaaccctt tgaggggact gacaggatga ccatcctcat tttacagatg 30180
tcgggattga ggcacagaca gtttattttt aatttttatg tgtaaaatgt ttttactgtg 30240
gtaaaattta cataataaaa tttgccattg taaccatttc taactgtaca attcagtggc 30300
attaaggcca ttggtattgt tgtgtaacca tcaccgctaa tctaaccatc tccggaacat 30360
ttttcatcat tccaaactga aactctgcac tcattaaaca cttactctcc tctccccctc 30420
ccctcatccc ctggtatcca cagtttctat gaatctaact accctaagca tccgtatagg 30480
tggaatcata gtattcatcc ttttgtgact ggctcattac acttggtgta aacttttttt 30540
tatttttat tttttttga gacagaatct tgttctgttg cccaggctgg agtgcagtgg 30600
cacaatttca gctcaccaca acctctgcct cccaattcaa gagattctcc tgcctcagcc 30660
tccctagtag ctgggattat agtcgtgcac caccacgcgt ggctaatttt tgtatttttt 30720
agtagagacg gggcttcgcc atgttggcca ggctggtctc aaactcctga cctcaggtga 30780
tccaccctcc tcggcctccc taagtgctgg gattacaggc atgagccacc gtgcccccacc 30840
ttagtgtaat ctttcaaagg tatatctccg ttgtagcctg tgtcacaatt ttatgccctt 30900
ttaagactga atgagttttt aaattcattc ccatttaact ctttgttttg ttgcctgtgc 30960
ttttggtgtg aggcacagac attttaagaa acttagccaa ggccattcaa ccagaagtgg 31020
cagagcctgg atcttgacca catggctttg ctgctcccag agtcttgacc ctgtgctggg 31080
gctgacttct gcttttacat gagttcttgt cagtgctttc ggcagcttga tcaggtagat 31140
gctgcttttc cactttgcaa atatggaagg gagcacccag ctctcttggg catggcagaa 31200
attcatcagg ggcggaggag gcacagggta cccacccaaa aaatcccaac atattgaatc 31260
accttccaat aatctatagc atcatttatg ttatgctgcc cacaacataa atggctagca 31320
aataatttgg atgcgacaga attggtgaat gaatttaatg tagcttcaac ctgaagacca 31380
ttatacacta ttaataccta caaaattata gatgtaaggc tgggtgtggt ggctcatgcc 31440
tgtaattcca gcactttggg aggccaaggt gggcagataa tgagttcagg agttcgagac 31500
cagcctggcc aacatagtga aaccccgtct ctactaaaaa tacaaaaatt agctgggctt 31560
ggtggcacat gcctgtagtc ccagctactc agcaggctga gggaggagaa tctcttgaac 31620
ccgggaggcg gaggttatgg tgagctgaga tcacgccact gcactccagc ctaggcaaca 31680
gagtgagact ccgtctcaac acaaacaaaa ttatagatgt cacctacagg ccagttggga 31740
ggcatattgg taagtaaata cccatgacct ctccaccctg cctaggatcc aaactatgaa 31800
cactgatggt tgactctaac tccatattct tttcagtttt ctcccatgcc tacccccctgc 31860
ccccgagcat tctccctcat tggtggccct gattttcttt ttgttgagat aggatctcac 31920
tatgttgccc aggctggtcc caaactcctg ggctcgagag atcctcttat ctcggcctct 31980
caaagtgttg agattacagg cgtgagccgc tatgccaggc caactcttat cttgaatgtt 32040
gatgtttatc atgtcatgga tgaaaaaaaa aaaacccatg cgtattcttt tttttttctt 32100
tttttttga gatggtgttt cgctcttgtc acccaggatg gggatggagt gcaatagcat 32160
gatctcggct aactgtaacc tccacctcct gggttcaagc gattctcctg cctcagtctc 32220
ctgagtagct gggattacgg gcactcgcca ccatgcctgg ctaatttttg tatttttagt 32280
agagatgagg tttcaccatg ttggccaggc tggtcttgaa cacctgacct caggtgatcc 32340
acccgcctcg gcctcccgtt tgtattctta aatagtgtat tgtttccttt acttccttta 32400
gagctttaca gaaattccat gctgccttca gcttcctttg ccgctctgca tcatgttggt 32460

aagtctctgc tgatgcctgc tgctgtagct tatttttcac tgtgataatc tgttccattg 32520
tgtgaactga ccacagttga ttgatctgtt ctccaaggac atgtgagttg ttcgcaggtg 32580
tccccccacc ccctcccgat acaaacagtg ctgctgtgga catttcatac atctggttgc 32640
ctatgtcaga atttccaggg tatacagcta ggacgcagta ctgctgttgg attttcctgg 32700
gattgtagac tctcaaattt ggaatggact tctatggcca agtagttcac cttcagcaat 32760
catctcaaaa gaaatgctct tggtgtgatg gttgagagcg ctgttctgga ataggagaga 32820
cacagacctg ctgggccttt gcctcatttt attacttagt tcattatgca atgaacttaa 32880
catctgtcta cctcagtagt ttccctgtct gtgaattgag tctgttacat atatatatat 32940
ctatttaaat atatatatct atttaatata tattaaataa tatatattta aatagatata 33000
tatatattac agactcaatt taacccttag gaccccagga gcggatgaat cgtaacccag 33060
ttcnnnnnnn nnnnnnnnnn nnnnnnnnnn nnnnnnnnnn nnnnnnnnnn nnnnnnnnnn 33120
nnnnnnnnnn nnnnnnnnnn nnnnnnnnnn nnnnnnnnnn nnnnnnnnnn nnnnnnnnnn 33180
nnnnnnnnnn nnnnnnnnnn nnnnnnnnnn nnnnnnnnnn nnnnnnnnnn nnnnnnnnnn 33240
nnnnnnnnnn nnnnnnnnnn nnnnnnnnnn nnnnnnnnnn nnnnnnnnnn nnnnnnnnnn 33300
nnnnnnnnnn nnnnnnnnnn nnnnnnnnnn nnnnnnnnnn nnnnnnnnnn nnnnnnnnnn 33360
nnnnnnnnnn nnnnnnnnnn nnnnnnnnnn nnnnnnnnnn nnnnnnnnnn nnnnnnnnnn 33420
nnnnnnnnnn nnnnnnnnnn nnnnnnnnnn nnnnnnnnnn nnnnnnnnnn nnnnnnnnnn 33480
nnnnnnnnnn nnnnnnnnnn nnnnnnnnnn nnnnnnnnnn nnnnnnnnnn nnnnnnnnnn 33540
nnnnnnnnnn nnnnnnnnnn nnnnnnnnnn nnnnnnnnnn nnnnnnnnnn nnnnnnnnnn 33600
nnnnnnnnnn nnnnnnnnnn nnnnnnnnnn nnnnnnnnnn nnnnnnnnnn nnnnnnnnnn 33660
nnnnnnnnnn nnnnnnnnnn nnnnnnnnnn nnnnnnnnnn nnnnnnnnnn nnnnnnnnnn 33720
nnnnnnnnnn nnnnnnnnnn nnnnnnnnnn nnnnnnnnnn nnnnnnnnnn nnnnnnnnnn 33780
nnnnnnnnnn nnnnnnnnnn nnnnnnnnnn nnnnnnnnnn nnnnncttcc aggcttccca 33840
ttttagactt gaaatgtagg cctgttgggg cctcctccag tagatgtgtg cccttgagct 33900
tggatacccc cctgccaaga tacgcctttc aaaaacagca taaacttatc gcaactgaat 33960
agaaggggct cccaaggggt cccaccctgc tccggaagtc tcagtgagga atcaatagct 34020
gttaattcct gtgctgcccc ctgcctgaca ttccatccac acttgaggcc cagattgtaa 34080
gaagggagag caggcaggtc tctcttccag gagagggacc cggttcatca gcaggattta 34140
ttcattcatg tattacgtca ggctcagcgc ctgggctttt tataaatgag ggctctgctg 34200
gcgttctgtg ttattcagat attcggtgag agaccaccag aagccacaaa aaattgctga 34260
catctgttgg aaggcaatca tgcataatgg ataagggtgc actctgcagt tccagcagca 34320
ttgatttgaa ttccccctct gccgcttcaa aactctgaga tctcagcgag ttgcttaccc 34380
tctagagcct ccacgtcctc atctataaaa ctagaaaata agtggtactc actagatcct 34440
taccttgttg tgagagtaga atgacgtgca tgtagagctt tcccagagct ggcatgtggt 34500
aggtgctcaa taaacgctgg ttattctctc tctctctctc actctgttgc ctaggctgga 34560
gtgcagtggc gtgatcatag ctcacaacag cctctgtctg ccaggctcaa gggatactcc 34620
tgcctcagcc tcctgagtag ctgggacttg caggcaagtg ctaccgctcc tagctaattt 34680
gttttattct tagtacagat ggggtcttgc tatgtggtcc tcctggagt gctggttgct 34740
ctctaactgg ctgcgagccc cggggtaggc acactggtac tggggctata gcaaaatccg 34800
tgctcggtaa atcatcattg aaggagcaag ggtgctttac agttgtagaa tctcatagta 34860
cacgttactt tggtttttct cttcccagct ctggagtcct gggatccaag tgtgagcccc 34920
atgtctgcaa aatgaggcta gtcacttgaa tcctcttgga tcttgctttc ctcatctgtt 34980
agtgagcttg tagatagctc tgactgtcac caggctttgc tctgagcact ctgcatctgt 35040
attcaacctg tatacttaat cttcacaaca gttgaggtgg ttgatccata gtaatttctc 35100
gttgtacaaa cggagaaact gaggcagaga gataagtgtc ttcccaaggt taacagctaa 35160
ccagtagcag ggcacgctgg gatttgaaag actctctatg tgtcctaact ggtatgctct 35220
cctgtctaga ggtgatctct tcctccaggg ttccaggtag attggttgag atgtgcaatg 35280
cggaagagcc tcgggataag tcggctaatg acaccctcag gctacccagc cctcgggagc 35340
tcacagcaga gcagttgggg atgccttgca aggttacctg tgccagagtt cctggggtac 35400
atattaaata ggtaggaccc aatactactt tgcattttcc tggaatccta gactcttaac 35460
attggaaagg atttctttgc caagtaattc gccctcatca ttaacctcta agaaatgcag 35520
aggtggaatt agaaggtgcg tgtctctttc ttttgcacca cagattctat cgagtctcat 35580
ctggcagctt caaggatcat ccgcattctt tcatcttcat ggaatgtcac tagattgaga 35640
tgattctaag ggtgaggtca agcatttgga gtttggaact ctgccttttg tagagatgct 35700
cccgtgggac ttgccctgca cccagttcag cttgcttggg atgcatctgc tgagcctgga 35760
agagaagtat gggttcaagg ccagcggtcc agcactccca cagacctgat ctgtaatagg 35820
gaagggttgc ctctgtgtgg gttgggagtt taatcagcaa gagtggatta gcagcaggct 35880
cattctgttt actccagtag gtcacggggc caagggcaaa catacacccc tcccagccca 35940
gaaactgata gctgcaaagt tgcacacccct tctccccaaa ttgacaaata tggttttcat 36000
tgttggagta ataaattatg ctttttaagg ataaaaatgt tgcccagctg ttgagtggtt 36060
taaacttcca gatgttcctc tgctcagtaa tcagagcagg atgcatgatg ggatctcttt 36120
aaaagttgat gaacagaatg gttcattaaa tttcaacaat gtgcctggaa tgccaagggg 36180
aggtgtagca tctccctcga gggaaggagg tacacatttc ctagggtgct tgggtgaaga 36240
attagcaagc agtaactgaa gaccagttac tggtcaggca gggaagccca agttctggcc 36300
cctgctgact gtgtgacttt gggctgagca gttggcctct ctgagtctta ggttttgttt 36360
tttttttttc ttttgtgttt ttgagataga gtctcgcttt gtcgcccagg ctggaatgca 36420
gtggcgcgat cttggctcac tgcaacctct gcctcccagg ttcaagtggt tctcatgctt 36480
cagcctcccg agtagctgga ttacagacat gcaccaccat gcctaactaa tttttgtatt 36540

```
tttagtagag ttggggtttt accatgttgg ccaggctggt cttaaagtcc tggcctcaag 36600
tgatccgccc gccttggtct cccaaagtgt tgggattaca gacatgagcc actgcgccga 36660
gcctgagcct cagtttctga tacttgcaac tgggagcatg ttttgagagc tagagaaagt 36720
attgttgtct tgtgtaacat gaaaaaagct gtgcggatac tagctaagat caatgaaagg 36780
ttatcaaact caatctgatg gtgaaagaag atacagagat caagcccgtc cgcattttta 36840
acgtggggac actgaggccc aggaaacttg gggcttcttc aagtagtagc taggcctgtg 36900
ctgtcccata tggtagccgc tagccactag ccgcatgtgg ctattgagca gtcgaaatgt 36960
gcgaaaattc aaattaaaat atgttgtata tgtaaattgc acaatggatt ataagactca 37020
gtttaaaaaa aagaaaaaat tcataacaac ttttgaaata ttgattacat gttgagatga 37080
taagatttcg atatactaag ttaaataaaa cattaaaaat gaatttcacc tttggtttct 37140
gcttagtatt ttttaatgta gcaactagaa aattaaaaat tactgccggg tgcggtggct 37200
cacgcctgta atcccagcac tttgggaggc tgaggtgggc tgatcacgag gtcagaagat 37260
cgagaccatc ctggctaaca cggtgaaacc tcatctctac taaaaataca caaaaaatta 37320
gctgggcatg gtggtgggcg cctgtagtcc cagctactgg ggaggctgag gcaggagaat 37380
ggcgtgaatc cgggaggcgg agcttgcagt gagctgagat cgcaccactg aactccagcc 37440
tgggcgacag agggtctctg tctcaaaaaa aaaaagaaag aaaattaaca attacatgtg 37500
tagtttgcat tatatttcca ctgggcagtg ctatgctaaa ctgtgacaca aacggtggac 37560
tttagagttg tgcagggtac aatctgcacg gccatatgtg gcaggcctac caggtgtgga 37620
tgaggctgtc tgggttcgaa tcctggctct accagtaatc agttgtgtgg tattgactaa 37680
attgtttaaa ctctcagagc ttcagttgtt ctatctgtaa aatgagtatt gcaatagtgc 37740
atgcttcata gagctgttgt aaggattaaa tgaattagtg catataaagt gcctagaata 37800
gtgtctggcc cgttttaaga gctcagtgag tcttggttgt tgtagttgtt actttagtgg 37860
ttgtttttta gaacctgagg acacaaggat ggaaagactc agtctgtctg tggcaaacca 37920
cgaaaggaaa gattaaacaa ttcaatgcag gctgggtgca gtgggtcaca cttgtaatcc 37980
cagcactttg ggaggccaag acgagcggat agttgagatc agaagttctt gaccagcctg 38040
gccagcatgg tgaaatccca tatctactaa aaatacaaaa attagccaga cgtagtggcg 38100
agtgcctgta gtcccagcta ctcgggagac tgaggcacaa gaattgcttg aacctgggag 38160
gtggaggttg cagtgagctg agatcacgct actgcactcc agcctgggcg acagagcagg 38220
actctgtctc agaaaagaaa gaaagaaaaa ataaattcaa tgcagtgatt attagagggg 38280
taagtccatc gggacaaatc agggggcatt cccactgagg caggacttga gatgaatctc 38340
ggaagcagaa atgctgagct tgttgggctg gccagagata agaaggacat tgtgggcggg 38400
agaaacagca cctacagggc tgagatggat gaaagctcag ttcagtctgg gaactgcaga 38460
aactcagttt gcctgagcaa actgcaatgt ggcaagtggg aaatgaaccc aagctaaaag 38520
aggacagcag ctgggtgtaa attttgtcct gtaacccatg gagagccaag aaggattttg 38580
agccagggag tgacatgatt ggatttcatg atcagatcat tctaactgct ttgaggaggt 38640
tgtcttggaa ggaaagcaag agtctttggc aagaagatca agagaagaaa gattgctatt 38700
tccaaagctc aagtagtgta tttttcattg ttgttgttaa agttcaaaag tctttatatt 38760
gaaagtggca cgtatgctat gatctcacac ctgtaatccc ttcccagttt atctgcatga 38820
gaaaagcgct tggaatggtg catatctaaa ggtggtttct gctcaatggg gatgtggatt 38880
cttgtgtcag gttgttgggg aggctttagg ctgtggctgc cagcccatcc cactgacagc 38940
caacttcata tggatgcaga gctttttaac catatcaaag agaaggatag gagcttaaaa 39000
tatgaaactc ctgcttttga tatgcttaaa ttctgggtct gatatctttt tgaaagaatt 39060
aggtttttaa ttgaaatagc acatgcatat atttaacatc aaatgggtaa ggcttttatg 39120
gaaaatccag attcccctgc ccttttttctg cctgtcctta gccctgtttc ccagagtgaa 39180
tcagggacag gtcagtacct ctaaagaata agctcatgtt gctccttgtc tgttgactta 39240
gacttctgga ccctcagcaa tgtcccggga ctcacactgg gggaggcagg gagaccagca 39300
tgccccctct tgccacttgt acttcccatt ttccccctgg caaggtgtgg aacatttata 39360
ctcccacgcc cacacttaag actcctgtgc tttatctgca aggttaattc caacaattga 39420
taaatggatg aacagtgttt acattaagag gcttgtcaat ggccttcact gcccgtcgaa 39480
gcagtcggct ttgatcacat gtcctttgtg ttcagctctc tctttgccct cagctttcta 39540
atcgcccttt aaggggctat ctgaggctgg gtgaggtggc tcacgcctgt aatcccagca 39600
gttcaggagg ctgaggcggg aggatctctt gagcccagga gttcaacact aacctgggca 39660
acaaagcgag agcctgtctc tacaaaacat taaaaaatta gccatacatg gtggagtgcg 39720
cctgtggtcc tagctgaggt gagaggattg ctcgagccca ggagtttgag gctgcagtga 39780
gctgtaattg caccactgca ttccagcctg ggtgacagag tgagacccta tctcaaaaaa 39840
aaggaactat ctggcttcat cctaagagta gttgcatttt aaactctcag tcagcttctt 39900
ttactgtaaa atgggaataa taattaacag tctaaattcc taaagtgatt atgaaggctg 39960
aacaggtgat gcctgttcct caggcccctc ccttggggcc ccttcagccc catgtgtccc 40020
aggctaatct caaactcctg ggctcaagtg atctgcctac tttggcctcc cgaagtgttg 40080
ggattacaga cacgagccac cactcccagc caggttgcta ttaaatttta cctttttatc 40140
caggagcgtg ggtcatcatt tctttcaaat aatgcttcta tttatgagat ttttctttgt 40200
agaaaatgtg ggaaatgtta agagtataag gaagaaaata agtcgtgtta acgcaattgc 40260
tgcatttttc ttttttttctt tgcatatttc ttttaaactt gagatcatgt aatgtcaaca 40320
agtttattgg atttttctcc taatgtagta ttagtatttc catgtgttgt aaagagcaag 40380
cttttaggta tcatttttaa tgcaatataa tgttgtaaca tcctattgca atgacagctt 40440
gtctttctcc caattttatc tgcactatta ggtattaaga atatttaatg tggccgggca 40500
tgaaggctcc tgcctgtaat cccagcactt tgggaggccg aggtgggtgg aacacttgag 40560
gtcaggagtt cgagaccagc ttgggcaaca tggtgaaacc gcatctccac tcaaaataca 40620
```

aaaattagct gggtgtggtg atgcatgcct ctaatcccag ctactcggaa ggctaaggct 40680
ggaaaatcat ttgaacccag gaggtggagg ttgcagtgag tcgagatcat gcactgcact 40740
tcagcctggg taacagagcc aaacactagc taaaaaaaaa aaaaaaaaaa aaaagaacat 40800
tgtgtatttt cctcttttaa ataatgttga gatgagtgtt ttggtgtata tgtctgtgca 40860
tgtgtgcaca tatgtgtgca gaaacctata ggatagattt ctgcaagaat tatcaagtca 40920
aaggttctag acatttttaa ggctctcaat gcatgttgtt cagttgtttc cccatttggt 40980
tacaccaagt tacagtcaca aaattacacc aaatattttt tcctccatgg agttgctttc 41040
tcatgaaatt gggtttctga ggccaggaag acttgggtct tgttgaggtg aaggggataa 41100
ttcttcagca tttgtcacta tttttcacta tcattttgag tttgatttaa atagcacagt 41160
ttgctgcgtg gaaaatgaga gtagctttga aggggcacag ctttgaaggg cacacagtag 41220
atgatgacat ctacttcaga actctggctg ctacgtgggg tacgaccatg ttgcattttc 41280
aggatgcttt agtggaaatc aaaagcatgg gatgcgaatg caaaaccaaa gggcttaggt 41340
tctgcatctg acatatgtgg ctagctgtat gttccttgcc gcgtcactca accttgctca 41400
gtacatacct tcttcatatg gcgtgcatct cttcctcagc tatatgggtg tcctcgaaaa 41460
tgctgagcat aagaaaagtg catttgtatc ctctttattt atttattatt tatttatttt 41520
ttgagatggg gtcttactct gttgcccaag ctggagtgca gtcgcacaat catggttcat 41580
tgcagcctca aattcctggg ctcaatggat tctcttgtct tagcctcctc agttgctggg 41640
actacaggag tgtgccacca tgcccagctt tttttttttt tttttttttt ttgatagtag 41700
agatgaggtc ttgttatgtt gcccaggcca gtttcgaact ccttggctca aacaattccc 41760
cctcctctgc ctcccaaagt gctgggatta taggcatgag ctaccgctac acccagccta 41820
tcctgtttac ctacaacttg ccagtaacct tgggcaaatt gcttggcctc aggatcttgg 41880
ttttgttttc tgtaaaatgg gaataataat aataataata gtccctaatt cctaagggga 41940
ttgggaaggt tgaacaaagt gcttccgcta gattgcttga cctggtgcct aatcctggtg 42000
atttctcgta aaaatcagtt gttcccacta gtggagtcca ccagacctga tgattcccca 42060
gcgttgagtt cagccaacct gcactttagt tcttcaggct ggttgtatat ggctggctag 42120
attcatcgtt tggaatcttc agagtcagaa gggacccatc caagtttgcc actcaatgga 42180
tgacaaagta aggnnnnnnn nnnnnnnnnn nnnnnnnnnn nnnnnnnnnn nnnnnnnnnn 42240
nnnnnnnnnn nnnnnnnnnn nnnnnnnnnn nnnnnnnnnn nnnnnnnnnn nnnnnnnnnn 42300
nnnnnnnnnn nnnnnnnnnn nnnnnnnnnn nnnnnnnnnn nnnnnnnnnn nnnnnnnnnn 42360
nnnnnnnnnn nnnnnnnnnn nnnnnnnnnn nnnnnnnnnn nnnnnnnnnn nnnnnnnnnn 42420
nnnnnnnnnn nnnnnnnnnn nnnnnnnnnn nnnnnnnnnn nnnnnnnnnn nnnnnnnnnn 42480
nnnnnnnnnn nnnnnnnnnn nnnnnnnnnn nnnnnnnnnn nnnnnnnnnn nnnnnnnnnn 42540
nnnnnnnnnn nnnnnnnnnn nnnnnnnnnn nnnnnnnnnn nnnnnnnnnn nnnnnnnnnn 42600
nnnnnnnnnn nnnnnnnnnn nnnnnnnnnn nnnnnnnnnn nnnnnnnnnn nnnnnnnnnn 42660
nnnnnnnnnn nnnnnnnnnn nnnnnnnnnn nnnnnnnnnn nnnnnnnnnn nnnnnnnnnn 42720
nnnnnnnnnn nnnnnnnnnn nnnnnnnnnn nnnnnnnnnn nnnnnnnnnn nnnnnnnnnn 42780
nnnnnnnnnn nnnnnnnnnn nnnnnnnnnn nnnnnnnnnn nnnnnnnnnn nnnnnnnnnn 42840
nnnnnnnnnn nnnnnnnnnn nnnnnnnnnn nnnnnnnnnn nnnnnnnnnn nnnnnnnnnn 42900
nnnnnnnnnn nnnnnnnnnn nnnnnnnnnn nnnnnnnnnn nnnnnnnnnn nnnnnnnnnn 42960
nnnnnnnnnn nnnnnnnnnn nnnnnnnnnn nnnnnnnnnn nnnnnnnnnn nnnnnnnnnn 43020
nnnnnnnnnn nnnnnnnnnn nnnnnnnnnn nnnnnnnnnn nnnnnnnnnn nnnnnnnnnn 43080
nnnnnnnnnn nnnnnnnnnn nnnnnnnnnn nnnnnnnnnn nnnnnnnnnn nnnnnnnnnn 43140
nnnnnnnnnn nnnnnnnnnn nnnnnnnnnn nnnnnnnnnn nnnnnnnnnn nnnnnnnnnn 43200
nnnnnnnnnn nnnnnnnnnn nnnnnnnnnn nnnnnnnnnn nnnnnnnnnn nnnnnnnnnn 43260
nnnnnnnnnn nnnnnnnnnn nnnnnnnnnn nnnnnnnnnn nnnnnnnnnn nnnnnnnnnn 43320
nnnnnnnnnn nnnnnnnnnn nnnnnnnnnn nnnnnnnnnn nnnnnnnnnn nnnnnnnnnn 43380
nnnnnnnnnn nnnnnnnnnn nnnnnnnnnn nnnnnnnnnn nnnnnnnnnn nnnnnnnnnn 43440
nnnnnnnnnn nnnnnnnnnn nnnnnnnnnn nnnnnnnnnn nnnnnnnnnn nnnnnnnnnn 43500
nnnnnnnnnn nnnnnnnnnn nnnnnnnnnn nnnnnnnnnn nnnnnnnnnn nnnnnnnnnn 43560
nnnnnnnnnn nnnnnnnnnn nnnnnnnnnn nnnnnnnnnn nnnnnnnnnn nnnnnnnnnn 43620
nnnnnnnnnn nnnnnnnnnn nnnnnnnnnn nnnnnnnnnn nnnnnnnnnn nnnnnnnnnn 43680
nnnnnnnnnn nnnnnnnnnn nnnnnnnnnn nnnnnnnnnn nnnnnnnnnn nnnnnnnnnn 43740
nnnnnnnnnn nnnnnnnnnn nnnnnnnnnn nnnnnnnnnn nnnnnnnnnn nnnnnnnnnn 43800
nnnnnnnnnn nnnnnnnnnn nnnnnnnnnn nnnnnnnnnn nnnnnnnnnn nnnnnnnnnn 43860
nnnnnnnnnn nnnnnnnnnn nnnnnnnnnn nnnnnnnnnn nnnnnnnnnn nnnnnnnnnn 43920
nnnnnnnnnn nnnnnnnnnn nnnnnnnnnn nnnnnnnnnn nnnnnnnnnn nnnnnnnnnn 43980
nnnnnnnnnn nnnnnnnnnn nnnnnnnnnn nnnnnnnnnn nnnnnnnnnn nnnnnnnnnn 44040
nnnnnnnnnn nnnnnnnnnn nnnnnnnnnn nnnnnnnnnn nnnnnnnnnn nnnnnnnnnn 44100
nnnnnnnnnn nnnnnnnnnn nnnnnnnnnn nnnnnnnnnn nnnnnnnnnn nnnnnnnnnn 44160
nnnnnnnnnn nnnnnnnnnn nnnnnnnnnn nnnnnnnnnn nnnnnnnnnn nnnnnnnnnn 44220
nnnnnnnnnn nnnnnnnnnn nnnnnnnnnn nnnnnnnnnn nnnnnnnnnn nnnnnnnnnn 44280
nnnnnnnnnn nnnnnnnnnn nnnnnnnnnn nnnnnnnnnn nnnnnnnnnn nnnnnnnnnn 44340
nnnnnnnnnn nnnnnnnnnn nnnnnnnnnn nnnnnnnnnn nnnnnnnnnn nnnnnnnnnn 44400
nnnnnnnnnn nnnnnnnnnn nnnnnnnnnn nnnnnnnnnn nnnnnnnnnn nnnnnnnnnn 44460
nnnnnnnnnn nnnnnnnnnn nnnnnnnnnn nnnnnnnnnn nnnnnnnnnn nnnnnnnnnn 44520
nnnnnnnnnn nnnnnnnnnn nnnnnnnnnn nnnnnnnnnn nnnnnnnnnn nnnnnnnnnn 44580
nnnnnnnnnn nnnnnnnnnn nnnnnnnnnn nnnnnnnnnn nnnnnnnnnn nnnnnnnnnn 44640
nnnnnnnnnn nnnnnnnnnn nnnnnnnnnn nnnnnnnnnn nnnnnnnnnn nnnnnnnnnn 44700

nnnnnnnnnn nnnnnnnnnn nnnnnnnnnn nnnnnnnnnn nnnnnnnnnn nnnnnnnnnn 44760
nnnnnnnnnn nnnnnnnnnn nnnnnnnnnn nnnnnnnnnn nnnnnnnnnn nnnnnnnnnn 44820
nnnnnnnnnn nnnnnnnnnn nnnnnnnnnn nnnnnnnnnn nnnnnnnnnn nnnnnnnnnn 44880
nnnnnnnnnn nnnnnnnnnn nnnnnnnnnn nnnnnnnnnn nnnnnnnnnn nnnnnnnnnn 44940
nnnnnnnnnn nnnnnnnnnn nnnnnnnnnn nnnnnnnnnn nnnnnnnnnn nnnnnnnnnn 45000
nnnnnnnnnn nnnnnnnnnn nnnnnnnnnn nnnnnnnnnn nnnnnnnnnn nnnnnnnnnn 45060
nnnnnnnnnn nnnnnnnnnn nnnnnnnnnn nnnnnnnnnn nnnnnnnnnn nnnnnnnnnn 45120
nnnnnnnnnn nnnnnnnnnn nnnnnnnnnn nnnnnnnnnn nnnnnnnnnn nnnnnnnnnn 45180
nnnnnnnnnn nnnnnnnnnn nnnnnnnnnn nnnnnnnnnn nnnnnnnnnn nnnnnnnnnn 45240
nnnnnnnnnn nnnnnnnnnn nnnnnnnnnn nnnnnnnnnn nnnnnnnnnn nnnnnnnnnn 45300
nnnnnnnnnn nnnnnnnnnn nnnnnnnnnn nnnnnnnnnn nnnnnnnnnn nnnnnnnnnn 45360
nnnnnnnnnn nnnnnnnnnn nnnnnnnnnn nnnnnnnnnn nnnnnnnnnn nnnnnnnnnn 45420
nnnnnnnnnn nnnnnnnnnn nnnnnnnnnn nnnnnnnnnn nnnnnnnnnn nnnnnnnnnn 45480
nnnnnnnnnn nnnnnnnnnn nnnnnnnnnn nnnnnnnnnn nnnnnnnnnn nnnnnnnnnn 45540
nnnnnnnnnn nnnnnnnnnn nnnnnnnnnn nnnnnnnnnn nnnnnnnnnn nnnnnnnnnn 45600
nnnnnnnnnn nnnnnnnnnn nnnnnnnnnn nnnnnnnnnn nnnnnnnnnn nnnnnnnnnn 45660
nnnnnnnnnn nnnnnnnnnn nnnnnnnnnn nnnnnnnnnn nnnnnnnnnn nnnnnnnnnn 45720
nnnnnnnnnn nnnnnnnnnn nnnnnnnnnn nnnnnnnnnn nnnnnnnnnn nnnnnnnnnn 45780
nnnnnnnnnn nnnnnnnnnn nnnnnnnnnn nnnnnnnnnn nnnnnnnnnn nnnnnnnnnn 45840
nnnnnnnnnn nnnnnnnnnn nnnnnnnnnn nnnnnnnnnn nnnnnnnnnn nnnnnnnnnn 45900
nnnnnnnnnn nnnnnnnnnn nnnnnnnnnn nnnnnnnnnn nnnnnnnnnn nnnnnnnnnn 45960
nnnnnnnnnn nnnnnnnnnn nnnnnnnnnn nnnnnnnnnn nnnnnnnnnn nnnnnnnnnn 46020
nnnnnnnnnn nnnnnnnnnn nnnnnnnnnn nnnnnnnnnn nnnnnnnnnn nnnnnnnnnn 46080
nnnnnnnnnn nnnnnnnnnn nnnnnnnnnn nnnnnnnnnn nnnnnnnnnn nnnnnnnnnn 46140
nnnnnnnnnn nnnnnnccag tacagatgtc tggcatttta ttgcagacaa catctgggca 46200
gatgacagca tctggtattt ttatgattgt cacttaccag aggtatggat agacatttct 46260
tgaagaagat aggagcaagc ctgattattt gaggaacacg agggccagat agcagaagtg 46320
gagggagagg tgtggttggc ttggcaggtg gagagggtca gggatggaag caaggagctt 46380
gctatgtggg ggtgtgggag tggggttggg gaaaccatat tgctttattt caggagagag 46440
tgatgtggcc tgtgtcttgg tggtagcaaa gaaagcggag agaaaaacgt agattttaca 46500
tttatctggg caattgtgcc aataggactt gcacacggat tggatatgga ggtgagaaaa 46560
taagggaatc aagaatgact cctacgagtc aggctgggtg gttcatgcct actatagtcc 46620
tagcacgttg cggggctgtg atggttaata ctgagtgtca acttgattgg attgaaggat 46680
gcaaagtgtt gtttccaggt gtgtctgtga gggtgttgcc aaaggagatt aacatttgag 46740
tcagtgggct ggggaaggca gacccaccct taagctggtg ggcacaatct aatcagctgc 46800
cagtgaatat aaagcaggca gaaaaacatg aaaatgcaag actggcctag cctcttagcc 46860
tacatctttc tcccatactg gatgcttcct gccctcagac atcggactcc aagttcttca 46920
gttttgaggc ttggactggc tctccttgcc cctcaaactt gcagacagac tattgtggga 46980
ccttgtgatc gtataagtta atatttaata aactcctctt tatatacata cgtgtgtgtg 47040
tgtgtgtgtg tgtatatata tatagataaa actcctcttt atatacatac gtgtgtgtgt 47100
gtatgtatat atctatatct atatctatct ctctctctct ctctatatat atatatatct 47160
cctattagtt ctgtccctcc agggaaccct gactaataca gaggctgagg caggagggcc 47220
gtttgaggtc aggagttcaa gaccagtctg gacaacttag agagaccttg tctctacaaa 47280
aaattaaaaa aaaaaaagaa tgactcctag gtttttggca tcagcaacct ggcagatgct 47340
ggtgtcttca atggacacgg ggaagatggg cagtagattg tgggcgcaga gagttctgtg 47400
caggcatgtt aaattgcaga tgcccattaa tggccacagg agatgccaac taggtacttg 47460
gcaaagtgag tctgggaatt gagaccatca tggtaggaga tagcatttgg aagattttgg 47520
cagaggtggg atccaaatcc atgggagtgg agtatagata gaaaagtgga cataggacta 47580
gacatggcca ctagtggtag gggtagaagt tggggagctg gaaaaggtga ttacaaggag 47640
ccactggtga ggcagaaaaa catgccggga gcctagagaa ggaagacctt cttaaggaag 47700
gaggagagtc atcacttgca atgttgctaa gaggtcgggt agaatgagtc acacagaagt 47760
gactgttaga tttggcatca aggaggttgt tgactaactt ggcaagagcg cctcatggga 47820
gcaatcaaga ggaagccagt gaccaatgaa ggtgactgct cttgcgttct gctctctctt 47880
cattgccttt tgctctttgc ttaggcaaat cagtgtcttc agtaggttcc ttgaaggcta 47940
atgtcaccat tagtatttac ctacccatta gctcatttga ttctcaaaac aacatctttg 48000
cagaaaaggc tgggcagggc aggcaggtat tattcccatt ttacagatga gaaagccaca 48060
acttaaaaac gtcaagtgtt tttttttcag gctgtttctt aaaagtaaga gttatgtgtc 48120
aggcacccct cctccacccc catctcggca tacatcccag tgttttgtgg tttgctacat 48180
ttcttggttt tcttagctag attgtgaacc cagtgaaggg aggatctgaa tgacatctct 48240
atattcccac gcagagatag caaatgcttg ccacacttgt tactttctct tgggtaccca 48300
tggcaggcat cactagctga tcatgacctt ctcttccttt gagcccagac tcagcttaga 48360
attctaccca gcattccaga tagcctgtca acctgctgac agataaggta tcatcaacag 48420
gcatttttac tgtagtactt aatgtcatgc ctggttcagc aagtggtttt gtgactgatt 48480
tagcaaataa gcattctctg aaaacacagt gagtgagttc tatgtcacta gaggtaatca 48540
agaagagatc aaattcatgc acatggccgg gtgtggtggc tcacgcctgt aatcccagca 48600
ctttgggagg ccaaggcggg cggatcacga ggtcaggaga tagagaccat cctggctaac 48660
atggtgaaac cccatctcta ctaaaaatac aaaaaattag ccgggcgtgg tggcgggtgc 48720
ctgtagtccc agctacttgg gaggctgagg caggagaatg gtgtgaaccc gggaggcgga 48780

gcttgcagtg agctgagatc gcaccactcc actccagcct gggcgacaga gtgagactct 48840
gtttcaaaag aaaaaaaaaa aattcttgca caattggata aggtaaatag aattcaggta 48900
ttgggtaaaa ggttatccaa ctctgagatt atctgatatt catggagtta tttaatgaac 48960
agtagtatct cttaccattc tggaacaaaa caagaaacct ttgagaggaa tctcccccca 49020
cctccccaga tatataggag gggacagtgg gcaacactat tatggctcct ttcttgttgt 49080
cttggccact tggggctctg gtctcagcct ttctggattg ccataatctg ttgggcccta 49140
tggagccaaa gggagtaaac agaatagtac tgttctgggt tgttgaggga ttcggtgaga 49200
ttccctaggt aggttttggt caacaagcag gcatttcata atcctctcta cctcctgccc 49260
tgccctgggg attgtgagga ttgtctgcat tcctctgtgg aactggaatg tggctgcagt 49320
gatggtgtct gcagagatgt tgaagctgct cctctctcct gccctctatc ttggcctcgc 49380
ccggtcctag ccggggtaag tgaacatcaa tcccatggac agagtcttct ctgaaaactg 49440
ctttatggat tttttttttt ttcccagaca gggtcttgct ctgtcgccca ggctggagtg 49500
cagtggcaca atctcactgc aacctcagct tcctgggctt aagtaatcct cccacctcag 49560
ccacccgagt agttgggact acaggtgcat gctatgatgc ccagctaatt tttgtatctt 49620
tagtagagac aaggtttcac catgttgccc aggctggtct tgaactcctg gactcaagcg 49680
atctacctgc ctcagcctcc caaagtgctg ggattacaag cctgccttat ggatcttaat 49740
gtcttggccc gagggcattg gctctgcctg gctccagagg gatatgaagc cattcctggg 49800
ggacaccatt gttttctagt taatattatc agtcctgtag tccagtgatt ttcaaatttt 49860
agtgtagtca gaatcaccag aagggcttgt tgaaacccag acagctggac ccctccctag 49920
agacttagga tgaggcctga gaattggatc taacaagctc acaggtgaca ctgaagttga 49980
cgaccttgta tctcacttta agcaaagtct tcccatagga accattgttc ctccctgccc 50040
ataggtaata tcttctactt gggccctctg cttccactgt ggcctgctta caacctcacc 50100
tacacttatt aactagcaga gttcactgag tctgcttaaa accttaaact tcaaactata 50160
ccacaaggct acagtagcca aaacagcatg atactagtac caaaacagag atatagacca 50220
atggaacaga atggaggcct cagaaataat accacacatc tacaaccatc tgaactttga 50280
caaacctgac aaaaacaagc aatggggaaa gaattcccta tttaataatg gtgtcaggaa 50340
aaccggctag ccatatgtag aaaactgaaa ctggacccct tccttatacc ttatacaaaa 50400
attaactcaa gatggattaa aggcttaaac ataagaccta aaaccataaa aaccctagat 50460
gaaaacctag gcaacaccat tcaggacata ggcatgggca aagacttcat gactaaaaca 50520
ccaaaagcaa tggcaacaaa agccagaatt gacaaatggg atctaattaa actaaagggc 50580
ttctgcacag caaaagaaac tactatcaga gtcaacaagg aacctacaga atgggagaaa 50640
atttttgcaa tctatccatc tgaccaatgg ctaatattca gaatctacaa agaacttaaa 50700
tttacagggg aaaaaacaaa caaacaaaca aacaaaaacc ccatcaaaaa gtgggcaaag 50760
gatgtgaaca gacacttctc aaaagaagac atttatatgg ccaacaaaca aatgaaaaac 50820
agaaaacaaa aaacaactca tcattactgg tcattagaga aatgcaatca aaaccacaat 50880
gagataccat ctcatgccag ttagaatggt gatcattaaa aagtcaggga acaacagatg 50940
ctggagagga tgtggagaaa tagggacgct tttacactgt tggtgggaat gtaaattagt 51000
tcaaccattg tggaagacag tggggtgatt cctcaaggat ctagaaccag aaataccatt 51060
tgacccagca atccaattac tgggtatata cccaaaagat tataaatcat tctactataa 51120
agacacatgc acatatatgt ttatagcagc actttttaca ataacaaaga cctggaacca 51180
acccaaatgc ccatcactga tagactggat aaagaaaatg tggcacatat acaccatgga 51240
atactatgca gccataaaaa agaatgcatt catgtccttt gcagggactt ggatgaagct 51300
ggaaaccatc attctcagca aattaacaca ggaacgaaaa accaaacaca gcatgttctc 51360
attcagaagt gggagttgaa caatgagaac atatggacac agggagggga acatcacaca 51420
ctggggcctg ttgggggatg gggggcaagg ggagggatgg cattaggaga aatacccagt 51480
gcatgcgggg cttaaaacct agaagatggg ttgatgggtg cagcaaacca ccatggcaca 51540
cgcatatcta tgtaacaaac ctgcgtgttc tgtacatgta tcccagaact taaagtacaa 51600
acaaacaaac aaaaaacctc cacaaaaacc ctatctccag gcttcccatt gtatctcctg 51660
acttttttga aatcctgagc agcctgttcc tgttgatcga gtgaatgagc attctctgaa 51720
attgcagtga gtgagttctc catcactaga ggtagtcaag aagaggttgg aatcacacac 51780
aattggatga ggtaaataga attcatttgt tagatagact atgcaggcct aagattgttt 51840
agtattcacg gagttatttt gtgaacacta gtgtcttcca gcctcatcaa gtggtccctc 51900
cctctgtagc cccacatcct ggtctttgat cagttccctg acacccttcc ctgggcacca 51960
caggaccttt gcacttgctg gtcccagatc ctagaatgct ttttccccag cttgttgaaa 52020
agctgattct catcctaact tcaatatcac ctcctcagac cacccccttct aatagactca 52080
ttccctaacc tccatttctc atcactggat gctgctgaat ttcactgtgg caccagtcat 52140
agtctgtaaa aaaaatttat taaattattt ggctctttga ttttggtgtg actcctcccc 52200
tagagaaaag aaactgactt tgtccttgaa ctctgtgttg tctgacactc agtgggtggt 52260
tcatacatat tcttttttta aaattaattt taattttttat tttacattcc gaggtacatg 52320
tgcaggatgt gcagatttgt tgcataggta aacgtgtgcc atggtggttt gctgcaccta 52380
tcaacccatc acctagttat taagcccagc atgcattagc tattttttct atgaaaggtg 52440
agcaaatgct tgtatacagg ctgcagaggg ttaggtcaag gctgaaccat gtagtctaaa 52500
ttttggagtg actgcagtga agcccctcca tggtggaaga aaatggaatg tccttgttgg 52560
agctggccag aactgggcag atccagaggc tggaggtaat ggcaataact ctgactgcta 52620
ataataaaat aagaacgtaa aagagtgctt acagtgtgtt acaggcactg ctttaagtgc 52680
attacgtcat ttagtttcca cagttgtctc catgttacag aggagaggac tgaggcacag 52740
agtattacaa taagaaactt ggcccacagt cagcgagcaa gaaagcctag attcatacct 52800
gggcatatgg cttcagagtc cacacgcttg gcgcctgcac tgcaccgaga ggggctgctg 52860

ggccgggagt cccccacctg ctctctgttt tcaaactccc catttcacac ccatgccagg 52920
tgtgatgaaa tcctctaggt gatgggcagt ggacatgagt ggttctggtt acagttggac 52980
ttcactggcc tgtcccacac ctgaggatat ggattctagc ccatgggacc ataagccagg 53040
ccccctgagg gtctgttatt tgtctgggaa gtggcagcgc tctttggaat cttgttcatg 53100
actgacaaac acccccacgc cccttcagcc ataattatca cctagtcccc agagaagggc 53160
catgggtggg ctgactgtag agatagacgc ttgggtctct ggctgggaac accacatgca 53220
agagaagagg ccatgggctc gccttccatc cagctgaagt ctttctggcc gggctgcagg 53280
cttcatgcaa cacctttcaa cctgtaaaag gaaaaaagaa ggccgggggt gggcctcagc 53340
tgctgccact ggcattgtat cactgaagcg ttttttgttg ttgttttttt tttttttgaat 53400
accggaaatt aaggggaaat tcggaaacct cataatggct tcatgaacca ttgcacttgg 53460
aaaatgtact tttgaaaaat tcccttgatt tattgttttt cggaggctgt agataacatt 53520
agagtatatt attcctatat aatttcctga gttttctatg tcaattactt tgttttatta 53580
aagagaaaaa aggaactaat attgattgag gccctcctgt gtgccaggct aaannnnnnn 53640
nnnnnnnnnn nnnnnnnnnn nnnnnnnnnn nnnnnnnnnn nnnnnnnnnn nnnnnnnnnn 53700
nnnnnnnnnn nnnnnnnnnn nnnnnnnnnn nnnnnnnnnn nnnnnnnnnn nnnnnnnnnn 53760
nnnnnnnnnn nnnnnnnnnn nnnnnnnnnn nnnnnnnnnn nnnnnnnnnn nnnnnnnnnn 53820
nnnnnnnnnn nnnnnnnnnn nnnnnnnnnn nnnnnnnnnn nnnnnnnnnn nnnnnnnnnn 53880
nnnnnnnnnn nnnnnnnnnn nnnnnnnnnn nnnnnnnnnn nnnnnnnnnn nnnnnnnnnn 53940
nnnnnnnnnn nnnnnnnnnn nnnnnnnnnn nnnnnnnnnn nnnnnnnnnn nnnnnnnnnn 54000
nnnnnnnnnn nnnnnnnnnn nnnnnnnnnn nnnnnnnnnn nnnnnnnnnn nnnnnnnnnn 54060
nnnnnnnnnn nnnnnnnnnn nnnnnnnnnn nnnnnnnnnn nnnnnnnnnn nnnnnnnnnn 54120
nnnnnnnnnn nnnnnnnnnn nnnnnnnnnn nnnnnnnnnn nnnnnnnnnn nnnnnnnnnn 54180
nnnnnnnnnn nnnnnnnnnn nnnnnnnnnn nnnnnnnnnn nnnnnnnnnn nnnnnnnnnn 54240
nnnnnnnnnn nnnnnnnnnn nnnnnnnnnn nnnnnnnnnn ttgcaaggct atggacagat 54300
actggtaatg gaacctgtgc tcagaattgt tgggaatctg tagtttaaag gtttgtcatt 54360
tggaggaata tcaagtaaac cctatggggt caaaaacaag gtctagccag gtgcggtggt 54420
tcatgcctgt aattccagca ttttgggagg ctgaggcgag tggattacct gaggtcagga 54480
gttcaagacc agcctggcca acatggtgaa accccgtttc tactaaaaat acaaaactta 54540
gctgggtgtg ctggcaggca cctgtaatcc cagctactca ggaagctgaa acaggagaat 54600
cacttgaact cgggaggtgg aagtttcagt gagtcgagat ggtgccactg gactccaacc 54660
tggtcaacag agtgagcctg actctgatga tagtaataaa taataatggt agcttctatt 54720
tattgtttac tactatgtgc cagacactat tctaggtgct tttcatcaat tagctcattt 54780
catcttcgtt ataaccctat gttctaagta cagtcatgta ccacataatg atgtgtggat 54840
cagcaatgga ccgtatatac aacggtggcc ctatgagatt ataatacagt atttttactg 54900
tacttcttct atgtttagat atgattagat acacaaatgc tcaccattgt attaagttga 54960
ctccgatgtt cagtacagta acatgccatt caggactgca gcctagaagc agtaggctgt 55020
accatatagt ccagatgtgt agtgagtagg ctgtcatcta gttttgtaca aactccctct 55080
gtaatgttcg cacaacgaaa ttgcctaacg ccgcatttct cagaatgtat ctctaccctt 55140
aagcaacgtg tgactgtcct attatcatca ccccatttta cagatgaagg agctgtggca 55200
agaaattgac taacttggcc caaggtcacc catataataa aaggcgggct gggtgcagtg 55260
gctcgtgcct gtaattccag cactttggga gtccaagggg aataggatca cttcaggaga 55320
ggagtttgag accagcctgg gcaacagagg gagaccctac ctctaccaaa attataaaaa 55380
aattagccgg gcatggtggc atagacttgt ggtcccagct gcccagaagg ctgaagtgga 55440
aggatggctt gagcctggga ggtcaagcct gcagtgagcc gtgattgtgc cactgcactc 55500
cagcctgggt gagagagtga gatcctatct caaaaaaaaa aaataaaaaa aaaatgaaaa 55560
aaaaaataaa aaaaaataaa taaaataatt aaaaaaagag ctgggacttg aagccagata 55620
ataaggcagg cagagggcat acgaaacacc cagatgggtc atgagcccgt gcatcagtca 55680
gggtctaatg gctcagctga ggaagggagg tttaaggaag agactgtgga cagaggtgtt 55740
agggaaggtg tcagagaagg ttaaggagcc aacatggatc atgggggtgg tacagtgttg 55800
ccagggctgg ggaggattgg ctgcagtgtg gggtacccag ccgctgccat gtggagaggg 55860
acctgtcact cctgctgtga actctccctt cttctgccct ctgacctcct gctggtgcct 55920
cccattggct aaacacagtt gatggccagt gcactgggga gctgttcttg gagcccacag 55980
gcatctgctt cttggcacag agcagacaat ggattgagtc gggagggagg ggaactagag 56040
aatacccaag tcccaatggc tggagaggag tgagcaggta aagggggtag aaggtgaagt 56100
cagagaggcc ggggtccaca ctacacaggg cctttggctt tcttcggaag gagcaggaag 56160
ccagtggagg gtttgagcag atgtgtgcat cacggccact gccatcagga gaacgtcttt 56220
ctctatgcta ggtgtgaaca gtcacccctt cctctccctt tctcaggatg agtgctgagg 56280
aggaaaaggc cctttcctcc aatgtcttca gaggagacgt ctgcccagtc cagactacgt 56340
gcctcatgcc agccagcctg taaatgacag gaaagagaaa ggagcagtgt ctcaaaagag 56400
gatattctgg aacgtttgat aatcagagcc cattgtaaag tcatgttttg agttacagca 56460
tttaaatctt tgttccagtg gaaaactttt acaggccgtc ctgggcaagg cttgaatcct 56520
gtttggatct ccaaaggcct taaaatagtg tgggatgccg ggagactggc tccaaaaggg 56580
aggagacctg tgctggcagc cctggccaca ccttggtggg accggctcct ggccttgggg 56640
accctgccac tctcctagct gtgcttccgc agagctggac acacaaaagc tttcttgtct 56700
ggatttgttc ccctaagagg gtggcaccga actggtcctg cccatgcagg aagctggtcc 56760
tgtccaacca gggaatgaaa agttggccag tatcttctca ggccacgcta cagccaagtc 56820
tcttttaatg agctggttct cttaggatgc ccaggggagg gagaacacac ttgagtggga 56880
tggcgaatga agaaggaagc ggctgttcag acagcttcca gttctgtcag gttctgctgc 56940

tgtgaccaag gcaccaagtc atctggaaac ccactgcagg cagcaacctc tttttcacct 57000
ttgtgtgccc caaagcttgc tccgggaatc gatggtttgg gaaacaatgc ttgctgtctt 57060
tgagtcctca gtcctgtaaa atggggaaag tatacatcag agggcatttg agagtaaggc 57120
aagaagcgct tggcatgtgc ctggcacata gtacctgttc agcagattcc ctttcagctc 57180
agggttagat cttcatccac ctcttgttgg cctgaaggaa ggggaggtag gtactattag 57240
ccccatttat tttattttat ttttttattt ttcttgagac agtcttgctc tatcacccag 57300
gctggagtgc agtagtgcca tcttggctca ctgcaacctt cacctcccag gttcaagtga 57360
tcttcccgcc tcagcctctc aagtagctgg gattacaggc atgcaccacc acgcttggct 57420
aatttttgta tttttagtag agagggggtt ttgccatgtt ggccagtctg gtcttgaact 57480
cctgacctca agtgatctgc ccacttcagc gagattatag gcgtgagcca ccacatgtgg 57540
cttattagct ccattttata ggaggtgaaa ttagacagca agaggctaaa tgccatggct 57600
gaggtaagat ctgtggtagc atccaagtct tgaccccaga cttggtgtac ttctcatcac 57660
agatgcaatt ttgtgcctca tcatctgacc caggagctac acatctgctt tttttttct 57720
tctttctgag acagtctcac tctgtcaccc aggctggagt gcagtggcac aatcttggct 57780
cactgcaacc tctgcctccc aggttcaagt gattctcatg cctcagcctc ccaagtagct 57840
gggaccacag gtgtgcgcca ctacccccag ctaatttttg tctttttagt agagacgggg 57900
tttcaccgtg ttggccaggc tggtcttgaa ctcctgacct tgtgatccac cctcctcggc 57960
ctcccaaagt gcttggatta caggcgtgag ccactgcgcc tggctaaagt tgttatttta 58020
aaattattat ttatttatat ttgttattat attttttatt tatatatatt ttgttatctt 58080
atttttaaaa gttgtatggc ccatttaaac ttttgcagaa atagtttttt gaatccatgg 58140
atttaatgct ttcctcttta aatttttttt aagtcaattt agagttttta agacaaaaac 58200
tggaaaacgg ctttgctctt ttgtggaact gctctggatt tttttttttt tttaagaaag 58260
aattttgctc catcacccag gctggagtgc agaagtacaa tcatatctca ctgcaggttt 58320
caactcctgg gatccagtga tcctcttgcc tcagcttcct gaatagctgg gaatacaggt 58380
gtgcgctacc gcaccaggct attttttttt taatttaaat tttttttgta gagatagggt 58440
ctctctttgt tgctcaggct ggtcttgaac tcctggcttc aagcaatcct accacctcag 58500
cctcccagcg agttgagatt aaaggtgtga gccactgtgc atggcctgtg ttgaattttt 58560
tgtctcccca ttcccaagta gcaactctgg actccacaga aaggccgagt ctctccaaga 58620
cccagccgtc ttggagggtc atctgtccac tcctccaaag agggtgagac agggccaata 58680
atgaagtgat cactgtgcac caaccctccc tcttgcttca gaagagacct tttcatccct 58740
gagccaatta atcaatttca agagatagca tctgagtgag atgggtcacc aagtcacaga 58800
ggccctcagc tcctgtgccg gctctcaaag gggttgaat gaggtggcct taacagattg 58860
cctagtaccc aacttcctag agttcgtata gtagagagca agtagcatag ggctagaagg 58920
ccaggtcaca ataccctctc tggagtcttc tgaatgtatg accaataggc aagagcccca 58980
gtttcctggt ctacagaagt gacagatggg accctaccta ccacacaggt tctgaggata 59040
aaaatagtga tatgggctgg gcacggtggc tcgtgcctgt aatctcagca ctttgggagg 59100
ctgaggcggg tggatcactt gaggtcagga gttcgagacc aacctgggca acatggcaaa 59160
accccatttc tactaaaaat acaaaaatta gctgagtgtg atggtgcatg cctgtaatcc 59220
cagctactca ggagggaact gcttgaaccc gggaggtgga ggttgcggtg agctgggatc 59280
atgccactgc actccagctt gcgcaacaga gcgagactct gtctcaaaaa acaaacaaac 59340
aacaaaacag gtaatatggt tggaagaggt ttatccttgc caagttttg taaatcaaac 59400
agctcttgag agatgcctgt ttggctgttt ttattttttt agagatggag tcttgctcag 59460
ttgcccaggc tggagtgcag tggcgcggtc tcggctcact gtaagctccg cttcccgggt 59520
tcacgccatt ctcctgcctc agcctcttga gtagctggga ctacaggcgc ccgccaccac 59580
gaccggctaa tttttttttg tatttttagt ggagacgcgg tttcaccatg ttagccagga 59640
gggtctcgat ctcctgacct tgtgatccac cctctccggc ctcccaaagt gctgggatta 59700
caggcgtgag ccaccacgcc tggcctgttt ggctgttttt ttaataggag gtgggaatcc 59760
cttgtcttcc tgggcagact agatgtgatg tgttcacagg gagctttgta cagattgctt 59820
gagagaagca cctttctttc cagagtggct gcttggacga tgccttccaa agctgctaag 59880
tcctgtggca ggggattgtt gatattcttc atggtttctc tggggagcct cctagcctct 59940
gaattattga tgcatgtcta cattattatt attttttttt tgagacggag tcttgctctg 60000
tcacccaggc tgtagtgcag tggcgcaatc tcggctcact gcaagctctg cctcccgggt 60060
tcatgccgtt ctcctgcctc agcctcctga gtagctggga ctacaggcgc ccaccaccat 60120
gcccggctaa ttttttttgt atttttagta gagttggggt ttcaccgtgt tagccaggat 60180
ggtcttgatc tcctggcatg tctacattat taaaagggtc tctttgcatg agttagcaga 60240
atgtagtgcc ctctcgtgaa tgagaaaagc cgcccagtcc tgattgcagc ctcataatgg 60300
gtctgaaatg acaagtgtga agcagtgggc agagccaaga ccacctttat ttgtgcatcc 60360
gtgctcttgg gtgggtatgt ttcacacagt ttgttgtcct tctgtccctt gcccatgagg 60420
ccttagaaac tcagcttgtc tccacccatt cctctacaac ccacacaaca acttgtaacc 60480
ttttaatctt ctttgcttcc taaagatgca actcatgcca cctcccccag gaagttctcc 60540
ctgacttcca atctttagaa taggctgcca gagatccctg tgttaactgt gaacagagcc 60600
cttttcatcc tcttgcatca gaattcctgt ctcatccact cgactgaact tcttgggtag 60660
gaatggaggt gtgttttttt tttgttttgt ttttgttttt ttgtttgttt ttaagatgga 60720
gtctcactct gttgcccagg ctggagtgca gtggcacgat ctcggctcac tgcaagctcc 60780
gcctcctggg ttcatgccat tctcctgcct cagcctcccg agtagctgga actacaggcg 60840
cccaccacca cgcctggtta attttctgta tttttagtag agacggggtt tcactgtgtt 60900
agccaggatg gtctcgatct cctgacctcg tgatccacct gcctcggcct cccaaagtgc 60960
tgggattaca ggcgtgagct accacgcccg gccggaggtg tgttttacat gcatctcact 61020

gcctgggaca gcgcctggaa cctagcaggt gttttatgtg tggggggtgt tggttgtagt 61080
agtggggaaa ggtttcttaa atgaggtgtg tttagaattt agccctttgc agggtataaa 61140
atctgagaaa taaggtgtgt gttgtgacga gggatggcac tgggggcctg cctaaagcac 61200
tggcttcatt gtgggggctg aggtggcttg tttccttgga gctaatggtc agagtgagga 61260
gctggggact gagcttggat ctagattatt gttgcatgaa tggcctaaat gaagagtgtt 61320
ttctcaagtc cctatagaag agggagatcc tcatggcttt caaggagtca tgcccaagat 61380
acaggaatgg ctctctggtg gactggctgg gacagagggg agagggtgaa agtggggagg 61440
aggaggaagt ttaacagtat ttgaagcatt tgaaagccta gacccatcgt acgctgaaac 61500
agagaagaat ggctagccaa aaatactgtg tgagtctagg aacttaagaa tgctagtcct 61560
tgaccccaaa catacttata tagcagactc aacattattg tcttccacct accggatggg 61620
attatgcaag tgatatgacc cagagcattc tggaagccgg caggggcata aataatacat 61680
tagatatgca gatgaactgc ccagggtgtc tctggtcttg ggaagaattc ttagtagacc 61740
tgagataatt gcagaacaga gtgtcctgtt ttcccatcca cagcctccag gcctggcgag 61800
gattttgtgt tcaaaagaga tttccaaggt ggacatctct ctgacactgg cgatttttct 61860
ctctcacttg aacaaaactc ggtggaaagg tcactcctat ttgtcttagc ttctctgtca 61920
catcccaccc acactttgaa aggtctgttc ttagcggggt cccaggaagg ctgtgctggc 61980
cctgtcagcc ctgagctggg tggtagtcct ttatcagaga tacccatgag aatcactcac 62040
cctggagnnn nnnnnnnnnn nnnnnnngtg actggagctt taagagctac tgaactaagt 62100
ggtaataaaa gtgaacaaag taaccagtct ggaaatgtca gtagaatgga aagaaagaac 62160
aggtgggaga gttctccatt ttttcacggt aagacccggt tacttggctt ttgttgagtc 62220
cctggtgggt gtccatggtg tctttggcct tatctcccta ctaaacagga ttacctgtgt 62280
ggggcacctt tcctggtctc ctctctctcc aaggagcagg tagtacttgg aggccgtggt 62340
taatcacatt tcctaagtga gcttttcctt tctagaataa tccttgctct tacccttgca 62400
ttcctactgc atgttccctc aggctgaagc cttcctttac atgtgagcat tctcctggat 62460
caactccctg atgactggag cctgtgccgt gtgtttccaa gttgcaccca ccacccacgg 62520
agactagctc atggccgagt ggcagctggt gtagctgata gctgtgtggg aaatgggtct 62580
gcacaatata aagacagctc acaggaaagg ggccccaaat ggtactctct ttttttttga 62640
gatgttgccc aggctggagt gcagtggcgc aatctcggct cactgcaacc tctgccttcc 62700
ggattcaagc gattctcctg cctcacccctc ccaagtagct gggattacag gtgcctgcca 62760
ccacacccag caaatttttt ttttttttgt atttttggta gagatggggt ttcgccattt 62820
gggacaggct ggtcttgaac tcctgtcctc aggtgatccg cccacctcgg cctcccaaag 62880
tgctgggatt acaagcgtga gccattgcac atggcctttt tttttttttt tttttttttt 62940
gttgttgttg ttgttgagaa ggagtattgc tttgttgccc aggctggagt gcagtggtga 63000
gatctcagct cactgcaacc tccgcttccc aggttcaagc aattctcctg cctcagcctc 63060
tggagtggct aggattatag gcgcccacca ccacacctgg ctaatttttg tacttttagt 63120
agagacaggg tttcaccatg ttggccagga tagtcgaact cttggccacc gcgcctggcc 63180
ccaaatgaca ctttgataag ttgctctacc caatcctaaa aaaggaaata cagataaaaa 63240
ataccaacgt tgtataatac acatgttgtt gatgcgctct gttgttggca aaagctgtgt 63300
tgctggacgg aatgcaagtg gatgtggccc tttgggaaga attatggcct tttggcattc 63360
tctatccaaa ctacaaaagc atgtgccctt tgacctggcc atctcatgtt gggacattgg 63420
gatatttccc ctacagagat aacctataga tatttgtagg aaaagaacag tgcccaaggt 63480
tattcatgga acatttttat aaaagcaaaa gactgggagt aactcaaaca tctgtgagtt 63540
gaagactggc aaatgatgac acattgtgct aggcaagatg cggaaaggaa taaggaagtg 63600
ctgtgtgcac aaacacgggg agatctcagg atgtattgtt gacacaaaaa gcacaatgtg 63660
gagcggggag tatggcatgc catcttttct gtaaaaaaaa agcatgggtg tagaggggat 63720
gtaaaaatgt gtctttgcac aaactctgtt aggatgcaca ggaaaccaac acaagtggtg 63780
gcctgtgcgt ggtgggagga gggagatgca gggaggggag actgggttag ggaggaagat 63840
gcatgaagaa gatttttttt ttcttttcag agataaggtt tcactctgtg gcccaggctg 63900
gagtgcagtg gtgtgatcat agcttgctgc aacctcagac tatgaaggag actcttgatt 63960
gcgtatcttt gggtatcatt tgaattttga agcatgaata tattgaaatc gggagcagct 64020
caaacattga gtatattatc tatcatacat ttaatttaaa aagtttataa ccttgtcatg 64080
attaggctgg ggttatgcat ttttaggagg gagaccccag aggtaaaacg tcagttctta 64140
tcacatgtca aggttatgtt ctattcacgc gactgatata aatcccggtt gcatatccct 64200
ataatacacc tgaccaaagc caggtgtggt ggcatgtgcc tgtagtctca gctactgggg 64260
aggctgaggc aggggggttgc ttgagcccag gaattcgagg ccagcctggg caacatagca 64320
agacctcatc tctccaaaac cacaaatttg gctgggcgca gtggctcacg cctgtaatcc 64380
cagcactttg ggaggctgag gtgggcggat cacctgaggt cgggagttca agaccagcct 64440
gaccaacatg gagaaacccc atccctacta aaaatacaaa attagccggg cgtggtggca 64500
catgcctgta atcccagcta cttgggaggc tgaggcagga gaatcgcttg aacccgggag 64560
gcagaggttg cagtgagctg agatcgcgcc attgcactcc agcctgggca acaagaccga 64620
aactccgtct cagataaaaa acaaacaaac aaacaaaaaa ggccaacaaa tttgaccaat 64680
actcctcaaa actggaaaag ctggtgacca ccacccctag tgctccaata aagggctgtg 64740
ggaacagttg tgctcagggt ggtactggta cagcctggca gaggacatga tgcttgcata 64800
gtgtggaaag gccctctgag atattgctgg gccttagggg aggaggtcca ggcctagaga 64860
ggactgaagc cctccccagg gcatggtgtg gaccacgctg aagggaggtg gggttggaga 64920
cacagctggg agtcagatcc caggggctct cctgtgtgat gctgaattgc tgggaagggg 64980
atgccagcta gtctctttct ctgtgccaac cctgattggt tgggtggctg ctgagagcct 65040
tatggtgccc tggctcagta ggaagagtgt gtgttcattt agtgagtttg ccaaagacaa 65100

```
gggaaggggg ttagagagta ggtggcagat aatgccggcc tggggatcct ggaggtttta 65160
ggctggcaga ccctggctga ggctgttttc tgttcttttc cttaggcctt gggagacctt 65220
taccttttca gtgcctctaa gacagggtca gagcagtttg gtgacaaagt ggaagtaaac 65280
catgggtgaa aaacagctct ggggaccctc ataggcttcc acttattgag gattaggcag 65340
aacttgcagc ttggcccagc tttcgaaaca aggtgttccc aggaggtgga aagactggtt 65400
ttcaatcaga taaaggtagg atcctatgaa aaactcctag ggccttgccg ttcctatgaa 65460
gtggtagcta tcattcacac tcctagttat gccatcgagg taaaaagagg tggaataaga 65520
atcagggaca cttgaaaata agaaaagaga aagactgcgt gcatgtgcat gggtgcacag 65580
cctgtacata tgtgtgtgtg tgcccctgcg tgcacgtgca tgggtgcaca gcctgtacat 65640
atgtgtgtgt gtgcccctgc gtgcacgtgc gtgggtgcac agcctgtaca tatgtgtgtg 65700
tgtgcccctg cgtgcacgtg cgtgggtgca cagcctgtac atatgtgtgt gtgtgcccct 65760
gcgtgcacgt gcgtgggtgc acagcctgta catatgtgtg tgtgtgctcc tgcgtgcacg 65820
tgcgtgggtg cacagcctgt acatatgtgt atttgtgctc ctgtgtgcac gtgcatgggt 65880
gcacagcctg tacatatgtg tatgtgtgct cctgcttgca tatgcatggg tacacagcct 65940
gtaactacat gtgtgtgagc ctgtgggtat gtgaggggag tgagggaccc tgagggacta 66000
gaaacttcct taaaggccat ttgagagagt gctgctttct ataccaccca ccctagggca 66060
gttaggcaaa ggtgtgcctc cctttaagag gacctagcgg tcggtctggt ttacaacata 66120
ggttatcagg gagtagtact ttggcctgga aaaggaacct ttattttgga aaagatttgt 66180
aatctctgtg gcttgtttga tacacattgg aaacctgact ggaaactcag gccaccagca 66240
tctgttgggg gcacatgttt gcacttttgg ttatgatgcc acggccctgg ttcaggtatc 66300
ctaaagaaga taattaatga tgaaaatgat tggtgtagat ttcataccac agagcactgt 66360
ggtttgtata gtaagacaca gtgatggtgt ggacccatgc acagccagct ttgcagatgt 66420
taagaatagc gactttgcct gacataattg gtgtctgcac atgggattaa acaagtaaga 66480
ttgatgtgaa ttttattctt tcatttttta tgtagactca ctgattgggt aactgtggaa 66540
ataatgtcac agtgcctttt taagttaaga acatgtagtt gtgatcagga gcggtggctc 66600
acgcctgtaa tcccagcagt ttgggaggct gaagtgggtg gatcacctga ggtcaggagt 66660
tcaagaccag cctggccaac atggcaaaac cccgcctcta ctaaaaatac aaaaagtagg 66720
tgggcgcctg taattccagc tactggggag gctgaggcag gagaatcact tgaacccagg 66780
agctggagat tgcaatgagc caagatcgtg ccatagcact ccactttggg tgatagaggg 66840
agactctgtc tcaaaaaaaa aaattaaaaa aaaataaaaa aagataaaat gtagttatga 66900
aaagatttct aagcattttt aagcatttta tattaaaaca ggaattcaga acaaatcaaa 66960
acgaaacaat aaaactgggg atgaaggaaa ctcaatttta aatataaact ttggcaggtg 67020
ggtggtgaat ccgggtgttg tgagtcattt gctggcttgt ttgtcacatg gcctgctcta 67080
taagtgtcac cctaattgat gtattcgttt ggagtgggtg ctcttctgag gtttttgttt 67140
ccccctttct ttggggtgca ctgtgtgtgc atggtaagaa caaggggcca tggaattaga 67200
gaatcttgag tttctaatgc aaattcagtt ccaagcagtg tgacctggtt gtttaactcc 67260
tttgagccac ccccgcccat ggccctcatc tgtaccctga ggataatagt gtgggctttg 67320
caggcttttg gtgagcaagt gagatgatgt agcaaaacac ccagcccaga gcctagcacc 67380
aattggtctg taatccatgc tgcacggaca cagccattct ctggatgtgg cctcttctgc 67440
ctccactgtg aggtcagaga ctgagtcact gcaggagtaa cctctccttg gcaagcagcg 67500
ggagtcattt catcccagcc tttcaggagg gtgaatctgc acctggggtc cagagtctca 67560
gagatgagac gtgagccagg cgctgattca tcatgatgca ggctgtggag actctagcca 67620
tggtttctcc atgcaggagt gaggttggga taaggggtct ttctgggggc tctgtgctct 67680
gtggcccctg ctgctccgga ctggttcatt ggagaaacct gtcacattct ctagaccggt 67740
tgccacgcca tgctcacagt ctctgttctt gccttcctag gtgggaagtg agtgatgacc 67800
ctgaagtgag gactcatctc tagatctcca agggctgcag ctcagccagc actttacaag 67860
ggtgatctgg agccaaactg gcctgttggc tgaccatagg tgactctggg tagcccatac 67920
ccaggctcag cagcagttgg ggagctgcct cgatttctgg ttacagaatt cctggaactg 67980
agtcactgca gtaattgctg tgatgaattg tgtttacttt gtgtgggatt ccaaactgta 68040
gcagcagtga ctacagctgg aagacagcat gatcagcagc ttccaaggca gagcctggcg 68100
tcagaaagct gcattgcgct aatgctgaag cctgtgggag cctgttggag agacacttgg 68160
atgtttagcg agctggtgac tctccttgtc atgagtaagc ttaggacctt gggcaagtca 68220
tccaaactct tctgggcaag tcattctcct gcttggatgc cttgaggcag agaggcagtg 68280
aggtgaagtg gtcagtgcgt cgactctgcc tctagcctgc tggggtttga atccacctgt 68340
gtgatgttgt atgatattga cctttctggc tctcagcatc ctcttgtgtg aaataggaga 68400
ttttaacagt atctatttcg tagggttggt gtttgaatga gttaacatat gtaaagtgaa 68460
tggtacagtg cctggcttcc tggcaagatt gctatcagga ttaaggcagg ttaagccctt 68520
ggcacacact aagagctcaa taaatgtgag ctgatgttat tggtcccttta ttactattca 68580
agaagcctgc ccagccctcc tccctctcca tccacacagc agcctggtac ccgctgttct 68640
ctaggttctg gacacacgtt atgacatgtt ctgatgatct ggcttagaca gtggggccct 68700
cgaggtaggc ccagaggact tggtcctcac tgcctctgtg gcgccttgca ctgggtccag 68760
ctgacgtgga gagagactca ggaaacagtg gctgagtgtg actttggctg gcatagtggt 68820
tgctgagaga acagacaagg ttctctctca cgacatacag atttcagatc agggaaagtc 68880
ccagctggca taagtttatc gagcatctcc catggacaag atcagctgtg ggtggagcct 68940
tgaagtacat ggtagaagga cagcgagtct tcccaggcca gggcttcaag tgaggagaca 69000
agatatagcc tcccagagaa ttcctataat gcaatcgtga aagaaccata cccagcagga 69060
ggccggggaa agtgactcct gcaactctag gaaggcttcc tggaagaggt ggaacgtgag 69120
cagcatagga ttttgagaga agaaatggaa tgggctgagg gagattctgc tggtggaggt 69180
```

```
tcaggttgac ctaagggctg gcagcagtgg aggcccccca cgagtgagtt tgaggggcct 69240
ctttagctca gtccagttga ggcagcagag cctttccata ggggtgtggt gtgacctgaa 69300
tgttgggcac gtggtcgtaa ctgagcttta aaagtgaatg agaggagcca tgcgtgatgg 69360
ctcgagcctg taatcccagc actttgggag atcaaagctg gggatcacc tgaggtcagg 69420
agttcgagac caacctgggc aacatggtga aaccctgtct gtactaaaaa tacaaaaatc 69480
agttgggtgt ggtggtgggt gcctgtaatc ccagctactc aggaggctga ggcaggagaa 69540
tcgctccaac ctgggaggca gagactgtaa tgagccaaga ttgtgctgct ctactctagc 69600
ctgtctcaaa acaaaaaaca agaaacaaaa acaaaacaaa acaaaaaaac actgtctcaa 69660
aaaaaaaaag tgaataaaga gagaagaaag gcggggcacg atgcttttta agtctacgag 69720
catcttatag cattgtttac atcccagctt ttcactgacc cttcctttac ccccgccccc 69780
atcccgtggc ccttgctctg tcctgccctt tctgtacggc gttttctctt cccggctcct 69840
ctgcccccag ccccacgtgc caacttgttt cagcctggca aggaggcctg ctggtatccc 69900
agccatttcc tgccccaagg ccgcccaccc tcctccgatc tagcatggcc ttgccctccg 69960
taggcaggcc tcacccctgc ggcagccggg aagcagggct cctgttttgc ctgtttaccc 70020
tggcgcaggg acaggtagat catgtagcac tgctgggatg ccagctgcgt aagtctccgg 70080
cagtgggcac aggcggaggg caggggggcat tcctctgggc tgactcgagc ccggcctgcc 70140
tggcacaaga ccccctgctc ctggaggcag ctcaggggct tgctcatggc tgccctggct 70200
ctgcgctggg ccgctgagtc atcgctcagc agtgaggcct ctgagtcaca gtgcagccct 70260
ggactggaca gtgagtgcct tgtgggaccc tcctgggctt tcctgtccct cagagcattt 70320
aaaggggtcc caaattgtca tgtgaccctc tctgatgcca gagagacact ctaggtcttt 70380
cgcgccctgg tcattttaca gaactgtggc tggatgccag gcattcagag atacattcct 70440
caatctcggt ccgccttaca ggatccagca tcgaagagtt cagttcaagt cagcagccga 70500
gttacaggaa agcagccagg gcaatgattc agcaagtcca ggcaggagtg aaccaggcaa 70560
gatttcggga ggagagggct gggtcgcgtg tcctggcagg gccacaggaa gagcatacac 70620
atctcctgcg tcttcgtatt ttgtgagtgt cttgaaatga ccttgagaat ctacgcaata 70680
ccatgaagaa gcaggagcag gaataccatt tatccattgg cacagggtag catgctcagt 70740
gtgcactgag gttgtgaatt cttgtcctga cccaaagatg ctgatgctgt ccttataccc 70800
atttcacggt tgaagaaact gaaggtgtag taggagaaga tactgactca gaggcacaga 70860
gttagaaagc agcatgtctg ggatttgacc ccagacctgt ctgactcagt ttactatttt 70920
aaactacccg attcaagtta agtttctctc ctctctctct gtctctcttt ctcccacgtg 70980
gctgctggaa tcttccctaa ggaccatagc tgtctgcagt gcaggaagcc aactattaag 71040
gggaaacaaa agtgttctta ggagtcctgc tgtcactgtg gattgagccg gtgaagccct 71100
gagggatttc atcgctgagg tggatggaga agcagctggg ggccttggct gtaaggaccc 71160
ctaagggtta gtgtctggag cttgggggat gggaatgggt cccggtgtgc ggaaattgga 71220
tggctcgctg accccaaggg gaaacccttg ttgacgctgt aaagtgtctt ctggccttag 71280
agaaaccctc tagggttcag gttccagctt caccctcctt tagtcaggtg gctttggggg 71340
ggacatttct gctggccctg gcagacactg gactagagca gtgtttctaa agtgtagccc 71400
cttctctctc ccgggaaggg agacaagtgg gtgaggggat tggaatctgc attcataaca 71460
cactccccag gagagactgg agagcacctt tcccggtgaa ctcccgtcat gtcaggcctc 71520
agcagactga gcgggaaccc ccaccagcga ggttgctcag gacagcgttt ctcttgggcc 71580
tgtgtttgga acaagtatcc tttgaactaa tcactaacct gtgaaaatgg atagacctcg 71640
tgtgaaaatg aaaatgtctg gcttcccttc tgaggagcgg aggcatgaaa ggtgtggccg 71700
gtaggggtgg gggccccaca cctgtgcagc cgccctggcc agagcccaca agcagcgtcc 71760
cctggtcatg ccccactccc ttctccataa tctcgcccct ccctgaccag gccacctgac 71820
gttgccattt atattcaagt tcattccgtt gttattcttt tagaaaagag agatgagaaa 71880
tatttcttgt gcctacatct ttatcaaaag tagagcaaga aacttgagga ctctgtttct 71940
agaaaaatga gagtgggcat ccctcttctc tgaaaggaag aatttccata cctgccctct 72000
acccaatgtg tagacgaaag gatgagtgtc cttttggtgt gtggagagct ttgatggggc 72060
atgtgatgtg tagggcttag ggggtatgct ggggtggctg ggaaaggtgg ttgggtgtcc 72120
tgggaaggct tttatagaca ctgcgtggca ggcctaggac tctaagtgct tccagccagc 72180
tgtacatacg tatatatttt tttttctttt tttttttga gacggagtct tgctctggag 72240
tgcaatggca tgaacttggc tcactgtaac ctccgcctcc caggttcaag tgattctcct 72300
gtctcagcct cccaagtagc tgggattaca ggcacctgcc accacgccca gctaattttg 72360
tatttttgat agagacgggg tttctccatg ttggtcaggc tggtctcgaa ctcccgacct 72420
caggtgatcc tcctgcctcg gccttccaaa gtgctgggat tacaggcgtg agctaccacg 72480
cccagcccca gccatctata ttaactcaat ggaattcttg ttgaggacta aaatggagtt 72540
tttgttgaag actaaataag taaatgacag tggagtattg tcattatccc cattcaatag 72600
ataaggaaat taaggtttag agagtcacag taactggtcc tggctacaca gccagtcagg 72660
agcaaggcta acatttaggc ctagagtgct ctcctaaccg ctatgcctgg ggtgaggagg 72720
gaaaccttcc atccttgtgt catcccattt gatatattag ttactgaagc tgaagctaaa 72780
ataatccgag ggctgggcga ggtggctcat gcctgtaatt ccagcacttt gggaggccga 72840
ggcaggtgga ttgcttgagg tcagaagttc gagaccagcc tggccaacat ggcgaaaccc 72900
tgtctctgct aaaaatgcaa aaattagctg agcgtggtgg caggcacctg taatcccagc 72960
tactcgagat gctgaggcag gagaattgct tgaacatggg aggaagaagt tgcagtgagc 73020
caacattgtg ccactgtact ccaccctggg atacggagca ggactctgcc taaaaaataa 73080
taataatagt aatataataa taatccgaga gtgcttacca tgtgcctaca ttgttctggg 73140
atactgagat gaataagacc tcttttgggc tcttgatgac ttggttataa gaagaggcag 73200
ctcggttccc ccagggactc tcaacagtta gggacaggct ggggcatggt ggctcctgcc 73260
```

tgtaatccca gcacttttag aggctgaggc aggaggatgg agtatgtcca ggagtttgag 73320
actagcctgg acagcatagt gaggtcccat ctctacaaaa aataaaaaat tagctgggca 73380
tggtggtgtg tgcctatagt cccagctact cagtaggtga aagaggagga gatgggagaa 73440
ttggttgagc tctggagttg gaagctgcag tgtgcagtga tcacaccact gcactcagtc 73500
taggccacag agtgagactg cctcaaaaaa caaaacaaaa caaagcaaaa acaattaggt 73560
acagaaaggg aggagcatcc acattttctg acattgttta ttgcttttttt accctcatgt 73620
tctcccctct cctggcattg cctggcactt gggagaagtt tcataaatat tggtcgaatg 73680
aatgaatgga tattaaatca tttgtgtttt cttttgtgag ttgaaaccgt cttcaaatga 73740
gtcttcaaag tgctctgatt cgtatggtgg agtgtgatga agttaaactc tgggttgaga 73800
agagaggaaa tgtgagttaa ggaaataaag agaatggagt tgaatgttca gaatctttgg 73860
agtgctgtca gggatccatg attctgaatt cagttatcat ctttggtgtg agaaatcccc 73920
aaagattcaa acttgtcacc agaaaggtgg tcttaggctg ggtgtggtgg ctcatgccta 73980
taatcacggc actttgggag gccaaggcgg ctggatcatt tgaggtcagg agtttaagac 74040
cagccggtcc aacatggtga aatacaacat ggtgtatttt gtgtctctat tggaaataca 74100
aaaattagcc gggcatggtg gcgggtgcct gtaatcccag ctaccaggga ggctgaggca 74160
ggagaatcac ttgaacctgg gaggcggagg ttgcagtgag cggatattgt gccattgcac 74220
tccagcctgg gtgacagagt gagactctgt ctcaaaataa ataaataaat aaataaataa 74280
ataaataaat aaataaataa aagatggtct tgaggtttcg gtttgggcat aggttacctt 74340
ctgcagaatg catgtgagtg atatggagac aagttttaac atgggcactt tttgaaaaca 74400
gacatcttaa atattggcca aaaggccaga tgtcaaagtg gactttctat ttttgatgga 74460
aaatttcaag cctgtttttct gtaaaggaat gattattgta caaggatttc tggaactgca 74520
actggcccat agatggtggc cattatagta gtttcctatg aaatctacta atgtacagca 74580
agcagttctg tgactttcag tcaccacttg gtttcttttt ttttcttttt ctcttgtgga 74640
ctacacttga agaatccttt tttttttttt tttttttttt tttttttgag acagggtctt 74700
gcttaggccg gagagcagtg gtgctatcat agctcattgt aaccttgagc ttctgggttc 74760
aaatgattct cctgcctcag cctcctgaat agctgggact ttgggtgtgt gccaccacgc 74820
ttgactaatt tgtttacttt ttgtagagac agggtcttgc tatgttgccc aggctggtct 74880
tcaactccag agctcgagtg attctcccgt cttagcttcc caaagtgctg ggattaccag 74940
cataagccac cacacctggc ctgaaggctc gtttgtaatg gcactcttaa gtgagataaa 75000
ttgagtattt tcttcctcac aaccttctta tgcttgtctg tagttggctg tctcattttg 75060
gacaattttg attgtcggct tggctcttca actgcctgtg ttgttgtatt cttagaacac 75120
acagaacaac caggtctggg cttcttatct tgcgaggcag gtgaagaaag actaggaaca 75180
atgtatttgg gtgcccacct cataccggcc atgaccttct attgaggaac agcaggtaga 75240
tgaagcagct ttctcatatt acgccgtggc tgactccaaa cttgaactct ggtctctgtt 75300
tttggagctt gcttccttcc tacgacccta gactgctcct cctaaataat taagtggcaa 75360
acatggcaca gacagtaacc tcacataaga agctatgagg ttaggacaga aatatccact 75420
gctggcctgc gaactattca cacaggtctg actccccgcc agagtcctgg ctgccctggt 75480
cctgtggaat cccgtcatgg cttctgtcta aacggagcag gctcagggcc atgctgaact 75540
tggagaaatc tgtgcaggag tcagatttgg gctttagagt cacctggggt tgaatctcgg 75600
gcctgctatt tattgaacct gtcctagcct caatcgcctt atctgtacca tggggatgat 75660
accatctagg tcgaaggtag ttgcaggaga ttctcatgca cggaggccct tggccgtcat 75720
ttgtttcttt gtctctacaa gcagagaggc tgagtcatac agatcctgag ggagatcac 75780
tttctcttcc ctcctttcgt gtgtcatctc cacctgggtg tggtgagccc caggaataat 75840
gctgtgacat caagccccgt gaacacctcc tttcacacgg accccgtgac tggctgcggc 75900
tgccagcggt gatgagaact gatgacactc cagggctacc aggaacggtg tgtgtgcctc 75960
tgggcagggc cacttcctag aaccatgggg ctgctctgtg aagagaaccg gctcccagtg 76020
agctccagga gacgaggagc aggtcctggt ggcggaggtg ctggcgcagg aggccggctc 76080
ctggcagaat cggcttccag ctctgggcac tggggtgcgg tgtgggacca gcaacaggct 76140
gctcctctgc caggaaccgc cagaaccaaa gtcttctcga aggcatcttc agaaggcaag 76200
cacagggctg agcactccgg agaggagcct ttgggggaaa actttttctc ggagcttcgg 76260
agctcaggga gtactatggg ggagattcac taggttgatg ctttgctgat aggcagccct 76320
gtagagaaga caaggcagct gggcatggtg gctcaggcct gtaatcccag cacttcggga 76380
ggccaaggtg ggcggatcac aaggtcagga gttcaagact agcctggtca acatggtgaa 76440
accccgtctc tactaaaaat acaaaaaaat tagctgggcg tggtggcacg cgcctgtaat 76500
cccagctact cggaaggctg aggcagaaga attgcttgaa cccaggaggt ggaggttgta 76560
gtaagtcgag atcacgccac tgcactccag cctgggtgac agagcaagac ccaatctcaa 76620
aaaaaaaaaa aaaaaaaagg gagaagacaa ggcgacaact gagctttctg tctcctgtgg 76680
agtttagcac ttggtcaggt ggccatttct ctccctccta ctcggtgacc ttctcaaggg 76740
caaggggctc cttgaagagt gtctttcaca ttgaaacatg cacagaatgg tggcttatga 76800
cccatggtag aggcgtgaac ttgggatgcc actgtggacg tgtccccagc ctccctcctc 76860
attggtgtac tgatatcgtg ttgccttgga cctattacct cacctcttcc tggtgatttc 76920
acctcaatat cccctttctc ctatgcttct gtttttcctt ctacccttcc atgttttttt 76980
ctcccaacca ttcttctgta ttttattcca cccagccacc ctttcatcta accagccttc 77040
cacctagcta ggcttccaac taactatcct tttttattga gacagagttt caagtggcac 77100
agttgggcca cttgtttctg atgggagcca cttctcaaac aggtcgatga ctaggacttt 77160
ggacaaaagc atgggtgcag gctgggcgcc gtggctcaca cctgtaatcc cagcactttg 77220
ggaggccaag gcgggtggat cacctgaggt caggagtttg agaccagcct ggccaacatg 77280
gcaaaaccct gcctctacta aaaattgatc aaatatagat cacccgtaat tatcacccat 77340

cattctgaca cttagcaata gtcaccctta acatcttgtt gtcttgcttt cagatggttt 77400
tgtttttgac atgtttttt tttaacttat attatgacca tctcctctat gtcttttaaa 77460
gttttaaaaa cttgttttga atatcttttc catatttcat tatgcacctg tacaggaaag 77520
gtactccata agtaggtttt tcttctctgt aaaatgggga aaataagaac cccttctaca 77580
tggggttgtt gcgaggatga ggtgaagaaa gttgtcaaag cccctcgcta gtaccaggca 77640
cagagcaggt actgggcgca tgttgcctgc tactggtatt attgtcattg tcattgctaa 77700
tcatcagcca agtcagtcct gcggggcttt cagatttcct ctgcttttac ctgttgcaag 77760
gtataaacct tttcatgtgt ctctgattgt tttcttggca gagccaccca caccccagga 77820
atttaatagg gtcttgatgc attcccagct tgctgttgag gagtatttaa ccagtcacca 77880
ctccacaggc aatattaggg gttgctgagg cagcctccaa tagtgtgagc attctaatta 77940
ctgcagcaaa ctcttttaaa aaaattttaa cttttctggc tgggcgcgtt ggctcacgcc 78000
tgtaatccca gcactttggg aggtcgaggt gggtggatca cttgaggtca ggagttcgag 78060
accagcctgg ccaacatggt gaaaccccgt ctctactaaa aatacaaaaa ttagccgggc 78120
atagtggcac atgcctgcaa tcccagctac ttgggagtct gagggagtag aatcgcttga 78180
gcccgggagg cgtaggttgc agtgagccaa gatcacgcca ctgcacccca gcctgggtga 78240
cagagtgaga ctccatctca aaagcaaaac aaaacaaaca aacaaaaaac aaagcacccct 78300
ggagtactac tcagcaacaa gaaggaatga actactgata acactcagtg actcggagga 78360
atctctaaag gatgatgctg agggaaaaac ccagtcccac aagtttacct gctctatgat 78420
tccagttacg tataacatcg ttgaaatgag aaaatttcag aattggaggg cagattagtg 78480
ggagaaacag gcagagtcta caagggatcc ctccgttatt tcttacaact gcatgtcaat 78540
ctataatgat ctcaagaaat aaaaattcaa tttgagaaac ctgcagttcc aagtctgcat 78600
tgcagctgga ggtagtcann nnnnnnnnnn nnnnnnnngg cgcgttggct cacgcctgta 78660
atcccagcac tttgggaggt cgaggtgggt ggatcacttg aggtcaggag ttcgagacca 78720
gcctggccaa catggtgaaa ccccatcttt attaaatata caaagattag ccaggcgtgg 78780
tggcaggtgc ctataatccc agctattcag gaggctgaga caggagaatt gcttgaaccc 78840
aggaggcaga ggttgcagta agctgagatc ataccactgc actccagcct gaaagacaga 78900
gtgagactcc atctcaaaaa aaaaaaatta acttttaatt tttattttt agagacaggg 78960
tctcgctttt tcacccaggc tggattgcaa tgatacgttc atagctcagt tcatagttca 79020
tagcttagac ctcccaagct caagagattc ttccacctca gcctcccaca ggtgcacacc 79080
actatgccca gctaatttta aaatattttt tgtacacatg aggtctcact atgttaccag 79140
gctggtctca aactcctggc ctcgggcaat cctggactcc ccagatgctg gaattatggg 79200
ttgagccata gcctgcagca aactcttaaa acatgagggg aactatcttg ttataaagtt 79260
gcttttcttt gactactggt gtggttggcc agcttttcat gtttatggct tatttgtgtt 79320
tgttaactgg tcatttcctt tggggtgtta gcaatttttt tctcattgat ttgtaagatc 79380
tgtcttgatt aacttgtgaa ttttgcagat ggtggaaccg gggtgcccca cagtaagtcc 79440
ccttggttag gcatcctgtc cctggattcg acacctccat ttctatgggg gacatttctt 79500
tacaaatctg catctgattg tgataccaaa ccatgctttg caagtgaata caaggatcta 79560
ccccattaat ctgggcagtt gctagtttta tttatttatt tatttattta tttatttatt 79620
tatttattta tttatttttc cgagaccgtg tcttgctctg tcacccaggc tggagtgcag 79680
tggcatgatc tcggctcact gcaacctccg cctcttgggt tcaagcaatt atcctgcttc 79740
agcctcccga gttgctggga ttacaggtgt ccgccactgc gccagctaat tttttgcatt 79800
tttagtagag atggggtttc actatgttgg ccaggctggt ctcgaactcc tgatctcatg 79860
atctttctgc ctcagcctcc caaaatgctg ggatgacagg catgagccac cgtgcctggc 79920
cagttgccag tttttaatgt tctcaggtga gtggcatttg ccaaggaact tctctcttcc 79980
cttctgtctt cataaacaaa aaatcctact aacttcaggt gtcagtttaa aagtcatctc 80040
ttctgggagc cccaccttaa acccctaaat cagctgttgg cgtcgggggt ggacttctcc 80100
tttgttgcac caattgcaat ttcttactta ggtgtgtgct tgtgggaaca tttttgtaat 80160
atcagcttct ctgctaagga cagcgatcat gtctctttta tcatttggta tgtgttgctg 80220
cagcacttag cacagtgctt agcttggaag ccaaagccca gaggggtaga atgacttgct 80280
tgaggcccct cagcttggac ctggcagatc tgggacccgg tgtctgggcc caccttcaag 80340
ctggatccct caggaccctg tgtacctgtg gccagggctt tgctttggtg cctggatgag 80400
agtggtaaga gttgctgagg tgccaggcct ttgcccccat gcccgctggc atcactgatt 80460
ccaggttatt ttggctcagc gcggatggag cgagatgtag gggaaaggtt gggaccggct 80520
tccaggtgat tggcacatcc ctgccctaaa ccctgcatgg ctttcctggg cctgccatcc 80580
aaggggaaag aagcctcttt cctcacaagc tgaagcttga aaccaatcaa gctccaggcc 80640
agagaagctg caggtggggt gtggtttgca gtgggggtgg ggcaggaaag aaaggttacg 80700
tggggtgaag gggtcaggtc aattcagtgt gggttggaat gctggggtcc tgggtgggca 80760
gggatgaagg acacctgggg aggggagaga gcctgccggc tggaactggg gccctgccag 80820
tcccccgctg catgagtctg ggcatgcttc caatctctgg gagcctcagc ctcctgtctg 80880
taaaatggag ctaatgaggc ctgccgtcag ggtttgtggg caagattcag taactggatg 80940
cctgctcctg gcccttggca agcattcaat aaatgttgat ttctctccca cccctcgctc 81000
cttctcttat tcttttcctg ttgcttttca cacggacatg tgttgaatgt ggcaggatat 81060
ttctttcttt ttttttgag acagagtctt gctctgtcac ccaggctgga gtgcagtggc 81120
gcgatctcgg ctcactgcca gctccgcctc ccgggttcac gccattctcc tgcctcagcc 81180
ccctgagtag ctgggactac aggcgcccac caccacgccc ggctattttt tttgtatttt 81240
tagtacatat ggggtttcac catgttagcc aggatggtct cgatctcctg acctcgtgat 81300
ccgcccgcct cagcctccca aagtgctggg attccaggca tgagtcactg cgcccagcca 81360
ggatatttct aacacagaga aattctcagt ggaggccgcg ggaactagag atagaggtcc 81420

tcttgcctac caaagcacca gatggaggtg acagaggcca tcttgtccta gagggacctg 81480
gctaagagga tctaagacca gaactagtct gagtgcttgg gattcatatg gggctgtaga 81540
cggcaggcct gcttgaacac cagtgttcca atcccacatc tcattcttag agctgtgtgg 81600
ccctgggcga gtctcttgcc actatttctt catcctgaag tgggcaccac attactgcac 81660
ctcaatttgg acgaggattc tcaggatggg gaggtggttg aaatcatcca ggggtcactg 81720
gaatcaggat ggtcctggct ttttcagaag gacaaggtgg taggtgccaa tctgggggct 81780
ttgcagatat tgaacggcag cctgaggatg gggataaacc tgggtgtggc agacagaatt 81840
atgcttcccc caaagaggtt cacatcctga tctctggaac atggggatgt catatgccat 81900
acagagaagg gaaattaaag tcacaggtgg aattaaagct gaccttaaaa gagggagatc 81960
cttttagatt atccagaggg cccagtctca tcacagagtc cttacaagtg ggcgagagag 82020
gcagaagaag ggttcagaaa catgtgaggt gacaagtaac attgctggct ttgaggatgg 82080
aggaaggagc catgagacaa ggaataaccg tccctagaag ttggaaaagg caaggaaata 82140
ggaactcccc cagagcctcc aggaaggaac acagtcctgc taacactcag tgagacccac 82200
tttgaacttt gtatgttcag aactgtatca taataaatgt gggtggattt aagcctctga 82260
gtttgtacta gtttgttccg gcagccctag gaagtgaatg caatgggcgc agcatttgat 82320
ggccagcctg gcatccagcc tgtggggagt gggagctgag ggcagtccaa gggaaatgtc 82380
ctttgagtgt ggcaccggca ggcaaaccct tgtttgggtg tgatcatcag tgagttgggc 82440
cactgaagct gcctggtgac ctggcaggga tggcttaagt aggcagggtt tgcagatcag 82500
cgagataaag gggcttgcat aaattcacat gcccatggct tctccctctg acagcgcagc 82560
cctgcctccg gctgcctggc caggatggcg tgttggtgca ttagggaccc cttcctctcc 82620
tgccctgctg tttaaattgt tggtctgttg gcgttctcag gagcccttta ccagtaccct 82680
taggccccag acgacctact cctcaagctg atcatatctg ctggctgtgt ccctgctctg 82740
ggcccagcac cctcatggtt ttccataaca cgtatcccaa ctcctgctga tgggaccctc 82800
gtgagctgtc tgtgtaggga gcatggctgc ccctgggtct gctttcctgc ccctgaccca 82860
caggaatagg atgtggcaga gtgggggcca gatgacagag atgctggtgc tctctgagct 82920
gaggcccact gctgacctgc tggtggcttg tcctgctctt ggatcatagg cactgggaca 82980
tcctgccttc cttcagtgtc ccctccccact ccctctcctt cagcatcaga ggaatgaagg 83040
aggcctggag gagtagcccc tatctaatag agacaggagt gggctgggtg agcatggggt 83100
ggtcgcaggg ggaaggggat aggagatgga gagaggaacc tgctatctgg tgggttccag 83160
gatggtgaca gacagtggca gaagcagtgc agagacctta gtttgtgctg gaggttcagt 83220
taatcactgg tgaaggcaga catcactgca ggagaccaga ctgttatgta ttgtctgtgc 83280
attcatatta gtacacaggg aagatcttgg gctccgagac ctaggcagat cttgcctccc 83340
atctgccgac cctccgcaga tgctacacat tatgccaggt aaacacatta tcaaggagcc 83400
ttgggccgtg ttagaggaag gctccttgtg cctgtgagga gtttgcacta atgctatgtg 83460
tgtttgtgtg taaggcttgc attcactgat tcactgtaga ggatgacttg aaacccaagt 83520
catcagctgg gcagtgcatg ggctgctcgc ctgaatggtg aagctctacc aagtggtttc 83580
cacctagggc ctggcttagc tggcctcaga tccccttctc agttctgctc aagacctgcc 83640
tgcggccttc ctcgggcttg cttgcccaga atgctaggaa tggccaggga tactgcctgg 83700
gcatgtgttt ggcctcagcc aaggtcaggc tgtcaggaag gaagaagggg gagggggcaca 83760
cttgcctggg agggtggagg aggaggggtc caagccgcca cagcctggca gtcctttttt 83820
gtgcaaagga gacccatcag ctcctctgcc tggaacaccc ccatttactc ctccgcagtt 83880
agaggggtgg gaggtgagga attttgtgag gggaattatc tctgatcttg atgtgcacat 83940
tcctcggcag tatcccgggc ttggccagct tgctgacacc tggctggaat aacaatgagc 84000
gatgcttctg gaggatgctc cgctggggag ggctctgctg aaccagcact tactccctgc 84060
ttttcgatcc tcactctgcc ttcctgtctg acaaaggccg ggctgttggg atggtaatgc 84120
cctctgggct ggccttgatt gaaggtaaaa aatgaacttc gaggatagcc cctgtggctg 84180
tgcagtctca ttttttatgc agatatgttg ggggatgcag agacatggtg gcgggtgggg 84240
tggggagaag gggactggga aatctgagac ctgaagctaa gatctgagat gtcatccttt 84300
atggagcatc tccggatgaa aatgtcccag aggccctagg tcactcaccc tctgccccct 84360
aataaaatca ctgttgctgc ctcctgctga atctgaagta tgccttcact gagggcagga 84420
cgtccatctc aactattatc aaaggaaaac ataacaagag gcaacagcac agcccccggg 84480
gcatgaggct ccttcctctg tgatccctct gtccttgtct gccagtgttt ttttccttga 84540
cttttaaaat aatctttttc ttattatgaa atagatattt attttgctta acaaagaatt 84600
ctgtagtcct tctagaagct aggtaactct tctaagtcct ttgcaaatat taagtcattt 84660
aatttaatcc tagagcagtt ctgcgaggtg gtgggaagac ctagagaggt tagggtccac 84720
cttagcacta gctctctgag ctataaggcc tcagggtctg tggcattccc tactcactga 84780
aaagaaggga atttaaaaca tgagttccac cacccagaaa tgaaacagtg ttcatttcca 84840
gaatccaggc atatggttga ttttaccaat ttcctatcac ttgaatcaca gttttttttg 84900
tttgtttgtt tttgtttgag atggagtctc actctattgc ccaggttgga gtacaatggt 84960
atgatcttgg ctcactacaa ccccgacctc ctgggttcaa gcaattttcg tgcctcagct 85020
tccagagtag ctggggttac gggtgcatgc caccacaccc agctaatttt tttgtatttt 85080
tagtagagat gggttttcgc catgttggcc aggctggtct ccaactcctg acctcaggtt 85140
atccaccctc agattatcca ccctcctaca cctcccacag tgctgggatt acaggtgtga 85200
gtcactgcac ctggcccaca ggtttttttt aatagtcctg tgtgtataat tttgcatcct 85260
attatttctc cttcctttta acatatatat atatatatat atacgtatat atatatgtat 85320
atacatatat ataatatatg tatatacata tatatataca tatatatgta tacatatata 85380
tacatatata tatatatata tgtatgtatt atctctttac tttttaaaag tttctcagac 85440
tttaagaatt acttaaaatt tcccaaaaca aacccacagg cactgctgtg atcttcaggc 85500

acactttggt attcaggagt tgcttctgct ggaccaaagt caataacaag tagcttaaga 85560
agctgtagat ggcagagata atattgactg gaggcttagg ggtctggttc agggtggact 85620
caacaatttt gcaagttcta tattgttaaa attacaatga attatttata agcagttgtt 85680
agtaaatcat gaagttggca ttattattct ttatttattt atttattttg agatggagtc 85740
tcgctctgtt gcccaggctg aagcgcaatg gcatgatttc ggctcactgc aacctccacc 85800
tcccaggttc cagtgattct cctgcctcag cctcctgagt agctgggatt acaggcatgc 85860
accaccacac ccggctaatt tttgtatttt tagtagagat ggagtttcat catgttgccc 85920
aggctggtct cgaactcctg acctcaggtg atgcacccac ctcctcctct caaagtgctg 85980
ggattacagg tgtgagccac cacgcttggc ctattttttta ttattaaaaa acaaaatgat 86040
gctgatgctt tcaaatatgt attgcagtct tattacttag tattgagctt tgcagtgttg 86100
gactaatatc acaagtcgaa gggacaaaga agatggtaga caataccaac tgtttcccat 86160
taggctcttt ccctcctctt gtttttctga gaccttaagg gcctggttgt ttctactgtt 86220
ccctgctact tcataacctg caagcttgag aatgtttgtc atttacaaat tttaccttat 86280
agtagaagaa catagtaaat aattgtacca gattgagtag tgtcccccca aaattcatgc 86340
ctgtatgaac ttcagaacat ggcattattt ggacataggg tttttgtttt tgtttttgtt 86400
ttgagatgga gtctcgctct gtcactcagg aaggagtgca gtggcgtgat ctcggcttac 86460
tgcaacctct gcctcctggg ttcaagctat tctcctgcct cagccgtccg agtagctgag 86520
attacaggca cccgccacca ggcttggcta tttttgtac ttttagtaga gacagggtgt 86580
caccatgttg gccaggctgg tctcgaactc ctgaccttca gtgatcctcc tgtctcggct 86640
tcccaaagtg ttggggttac aggcacgagc cactgtgcct ggtggacatt gggtcttttt 86700
agatgtcatt acttaagatg atatcatatt ggattagggt gggggctaaa gccaatgacc 86760
catgtcctca gaggagaaga gagggacata ggaaagaagg atttgtagag atacagggag 86820
aaggccatgt gaccacagag gcagagatta gagtgacaca gctacaagcc aaggagctcc 86880
aaggattgca ggaagccacc agatgccaga aagaggcaag gaaggatctt tcatggagcc 86940
ttcagaggga gcacagtccc gctgacacct tgatcttgga catctagtcc ccagaactgt 87000
gagagagtgc atctgctgat gtaagccacc cagtttgtag caatttgtta tggctgccct 87060
cagaaactaa gacagtagcc aacacagaac caaatacoct ttggggattg agggaagtgg 87120
agcgggagaa tgagcggcac tccctccctt tacataggac tacttggtaa atatttactg 87180
aatgaaggaa agctgaagta tctgtagaac tggccatttc attctgcatt tagagctctg 87240
ggagctcttg aaatgtctag acaaacatcc tcttcagcta gaggaagctc cttctcccac 87300
ttactagcta tgagatcttg ggcagatggc ttaacatttc tgggcctcag tgaccttact 87360
tgcaaagttg tctgggtcaa ggcaatgatt aaatgcgatg atgcatgtaa agccccagca 87420
tgcagcctga aaatgtgagc ccttattatt tatactgttt gccaaagtgg tgagtagtgt 87480
tgctgaatat gccgtgtgtc ctattaactc tgtgacggcc acagtcacgt ctcctttctg 87540
cctcctcctc ctcctcctct ctgtccagtg actagggagg gcaggaagtt cagagggtga 87600
aatgctgaat taattcctgt cctcctggtc tatattccag atcaccctgt gggtgggttg 87660
aaaaccatgc cctgtctacc tcctctgcaa ggaaaaaata aagacagaga gcaaatctaa 87720
aacacgcaat gcagaggaat ggttactgcc tgctgtgtgc ctctccaggg agcaccagga 87780
ggggctgtgt gggacaggga cactgcgtat aagtgacact cctccacatt ttggcctgga 87840
gttagcacat acaactgcct ttttgctgac gcgatgccca ggagccctct caggggatgg 87900
caaatctcag aataaatgtg ttttttactc atggaatgca ggggaatgga tatgaaaact 87960
tttgtatcaa atagacatta gcgaggagag ggatgcttca catcctaggt ttgtgtgatg 88020
tcggcacagc tgatgaggtc tttgcaaatt ttgctgctga ggaagggatg cgctggactc 88080
ccagtgtcca ggggccctct gcttgtctgt ccttaggcag agtccaccag aatcgggtaa 88140
agtatgtgac tgcctcttag tgtagggcat gacgtcaact gtgaggagaa gagatcaaga 88200
tacagctctt tggtggtgtt ttttgaccca tatccaggaa ctgttcccac acccagagag 88260
gaatatctgc aagttctggt gccagatgtt gattcaaaaa caaaacccag cgaagcatct 88320
gtactttttt ttctgattgt attagaagta cagtccacaa taatatattg tacatttaaa 88380
aataactggc tgagcgcagt ggctcacgcc tgtaatccca gcattttggg aggccaaggc 88440
aggcggatcc cctgaggtca ggagttggag accaacctgg ccaacatggc aaaaccccgt 88500
ctccactaaa aatacaaaaa aaattagcca gacgtggtga tgggcacctg taatcccagc 88560
tattctggag gctgaggcag gagaatcact tgaagccggg aggcggaggt tgcagtgagc 88620
tgagatcatg ccattgcact ccagcctggg ggacaagagt gagactccgt ctcaaaaaaa 88680
caaaaacaaa agaaccctca aaaaactaaa agaggccggg tgtggtggct catgcctgta 88740
atcccagcac ttggagaggc cgaggcaggc agatcactga ggtcaggagt tcaagaccag 88800
cctgggtaac atggtgaaac cttatctcca ctaaaaatac aaaaatagcc gggcttggtg 88860
gtgggtgcct gtgacccaag ctactctgga ggctgaggtg ggagaatcgc ttgaacccag 88920
gaggtggaga ttgcagtgag ctgagattgc gccactgcac tccagcctgg gtggcagaga 88980
gagtatccgt ctcaaataaa taaataagta aataagagtt taattggatt gttcgtaaca 89040
caaagaaagg gtaaatgttt gaggtgatgg atgccccatt taccctgaag ggattattat 89100
gcattgtgtg cctctatcaa aatatctcat gtaccctata aatatatatg cctgtgtacc 89160
cacaaaaatt aaaaattaaa taaaaagttc attaaaaata acttcaaaca ctacagaaat 89220
gttaaaagta gaagtgacct ttccgtagaa tgtcatgcct gtactttggc agagaatttc 89280
aatggaagca tccagaaaac cctggctcgg aatctgagct tgattatttc agtgtttcag 89340
gtctcacatg ggggacagtc ttgccactgt caaaggccat catgagagtt acagcagact 89400
tttgatgaaa ttatgtccac agtggtccct ggtcctttgt ggtttgagat atattctttg 89460
tccccacgcc tgccccttgc ttcagatctt ctaggaatcc agatacatta aagcaaagtg 89520
acgggacagc cctgtccatt tcctgatctg ttgctgactg taccactgag ctgctctctc 89580

ctactgagcc ccgcatggtt cctcctgttt tcctgccctc tcacttgctg gtgttggcct 89640
gctctggctg ttctgcttgg ggttaggtga ggatatgggg cagaaggctt ccagcctgga 89700
gcgcctatgg tgtcccaaga caagggcctg cctctggcct ctgggtgtgt cctcctgcga 89760
gttttagaga aatctgggca ggctcagact gaatgcctgc ttctccttgc agccttctga 89820
agattcccat gacacacacc tgatttacct tcagctcttt tacttgctgg gtaagcacag 89880
ccctggatta cttggcatct ggaattaact gaagggtaac actttcttaa ccttatttgg 89940
aaaatgaata ggaaggagac cccttctaac ttctggaagc acaaaagtgt gtttatcctt 90000
caggagcatt cttggctgct gaaaaatcat catggctagc tttcactttc acgtattgag 90060
ctagtaggtg gtatcaatag tagctaatat ctatttcatg ttttttgtg taggagggac 90120
ccggcgtgca atttgcatgg attgtctcat tttatcctcc cagcaggtaa aagagctggg 90180
agggagtaaa gggagttgtt aatttctgag aaggaaggtg aaacttagga atgttaaata 90240
cccctcttgg tgtacacagt atgcaatgga ggcagggtgc aaatgtgcta tggagatttc 90300
agagcctggg cttcccagag gaggcctttg ccccacagaa tctttgctcc atttctccac 90360
tttgctgcct ctatgaatca atgattctgt gttgtccatt tttcctttgc aatcttttgc 90420
catctcttca ttaaaaatgg tctccatcat tctggccaag cacagtgact aacatacaat 90480
aggtgtgcag taaatgtcta gtggatgaat acccagtgtg aacattaatt gagcaccact 90540
agctgccaag cagccaagca ccctatatct gggagatgta gaatttaagg ctgtgtagtc 90600
actgtcctca agctgctcac agctttgtgt aagacactca agggaacatg caaagctcag 90660
ggcagctcaa tttggggcga gggttatgga gaaaatacag tctgcatgct tttagtacat 90720
gcagttctgc atcttggggt gcagtgctag agtgaatgac acagtagagg tgacaggctt 90780
aggatttaaa tttgataatg tcatgatgcc atgggagcac tggagaaggg gcagcagtgg 90840
attcagctga gagtcgcaag ggttaatga gaagattggg aaaggctgca cagaggagga 90900
gaagggcttt gtaggatgag tagaggttca ccaagggtgg gtaacagtta gaggaacagc 90960
aggtacagaa gcccagagga gaatcagtga atgacacatc caccatgtcc agagatcagg 91020
gatggttgca gctgaagcta aaagtgtaag agcttggact gtggatcttc aatcccagtt 91080
catcttattc ccacccatat ggctggtgct atggttttgt cacctgtaac acaagtatga 91140
gactggtacc ttcctaggag gggtgttgag agtctgagcc acactcaaat tcttggtgtg 91200
cagtgtctct gatctgaaat gtgcaccgtg aatgttagca gtgggtatta cctttggcct 91260
gattgtgaag cgggtttgag acagaagtga agcagttcaa tttgttgtgt gatcttcttc 91320
ttccccaaaa gaaggttttc atcctcctta aggcttttgc ccacccttcc cttccttcac 91380
atccctggtg ggtacccttc ccacctgcag gaagcctact gatctttgag ttttggcctg 91440
ccacctggcc cttccctcgc tctgcagtct tggttggcct ccaggtgtgt gtgtgtgtgt 91500
gtgtgtgtgt gtgtgtgggt gtgtggccct gggctgcact gtgcctgctg gagcaggtgc 91560
agttgccctt tcaccatgcg tgcttctttg gatgtgaccc cttgggtgcc tgtgtattta 91620
gagtggagga acgtcctcca ctataatgat gagaagtgtc attgctagtt gcactcagtt 91680
ttctatggtg tttgggggag aaattggggt gctggatttt ggttttaact ctttcccacc 91740
ttaaccagtg actgtggaaa gttgcacatc gtccccttcc tcacagctgg ggtctgttct 91800
tcctccgggt gttacggttt gtgagagcca ggagggtaca cagaggtggc ctgatttctg 91860
gagccatagc aagaagggag aggtgtgtgt cctctctgtc tctgtctctg tctctgtctc 91920
tgactctgtc tgtctctgtc tctgtctctc tctctctctc tctcttcttt ctccctctcc 91980
ctttttcaca tgttgttttc tgattataag aggaatactt catcattaga catatttcga 92040
gcctccgttc ctcatctatg aaataagcat agtaaaggac tgaaggattt aaagggatgt 92100
ggccaagtgt gcaaagtcat atgtgctcaa taatttattt tccttgtttt cttttccatt 92160
tccttcttct cctctaaggc gctccacatc tccgtgatat agcgtgtact cctttcctca 92220
ttttcttttc tctgagacaa agggaagttc ctagttctgc tatttcctag ttcagtgacc 92280
ttggacaagg caccctgggg agccttagct gctcatatgc acaagcttct agcaactgct 92340
tccaagggaa gggtggggag gaagggagac cctgagtgtt tggaccccgc ctgtgctcag 92400
cacagaatgg agctcagtaa gggcaggtcg tgggctcctt cttctccctt tcagcaacca 92460
tcactcgtat tctttttat ctccaaggcc tcatgctgga aagagattga ttgctctctg 92520
agcagtaagt cctctgaaac agggtttctc tggggagttc tgtggcaccc ctgccttacc 92580
tgggggacct ggcaaagagg caccctccac ccccctcac cctcagccag gtttactgaa 92640
cagagttacg ggggtgggcc tccggatctg cattggagca agtccacagg tggtgcttag 92700
gcacactcag gtttgagagc cactgaccta gaggggaaga ctgggagaag ccttttaaga 92760
gaccagtact gtgacttgca ccatggagac acctggaaaa catacagtga ttcacacaca 92820
cgccccaaac agcagtgtgt gtgtgtgtgt gtgtgtgtgt atgtgtggtt tcacacttgg 92880
tttgctatga atgctgtaat gccacccagt tcccacagag ctctggggac tcaggttccc 92940
caagtcaggg ttcttttctg gagtgctatt ctgacaggtt tttatttttta tttttttaat 93000
tattttattt tattttttatt ttattagtat tattatatct gacaggtttt taatacagtg 93060
aatttgtatg tctcctgtgt atttatttta gctgctccct tctttcccct tctctccctt 93120
tcctctcatc tcctcccgct ttcgtccttt acctttccct ggatgatatt agcattaccc 93180
gtacactctt cccatctcct ggcccctcca gttctgaagg gatggggact aagtgggatc 93240
tgtcctctaa gcaggcggag gagggaacca tgtcagctcc taggtgcccc cagagcacct 93300
gctgccagtt ttccacaact cctgtcctcc gtggcgcttg ggtgacagca gagcagagcc 93360
gatgtgcctg ctcctggtca tgggctgttc gtgctgtcat cgctttcatt tgccccatcc 93420
ccccaatttg gacttaccca gaatccttgg gcaagctaga tggctgccaa agtttactta 93480
gcgagatgtg cctgcgtgac ttgagtagag acctgcttct gagaaagtca gaagtgcatt 93540
ccaaagtcag gagcgtgacc tccccggaag cccagctaat ccaggtttta aagaggagtt 93600
ggaacaaagg tggtgcttat gggatgtatg gagctgtggg atgtgggaga ggacaggttt 93660

agcagagtgg cgtggtggaa agtttcctct cgccgaggag gacaactccc cagcgcctga 93720
gacagcggga gctcagagcg cctagaatgt gcccagattc taaagtgaag caggctgtgg 93780
tgtgatgatg gcccccgagg ggacttgcag tcttatggac ctgggttcaa ggccaccctc 93840
ttcagccaca caaactgggc aaggggcccg ccctttcac gctgctgttt ctgtaacttt 93900
cagatatgag acctgtctgt ggggttgtga ggatacatgg ggattaagtg ggaaattaaa 93960
agggttttct ttgtgactgg tgactgataa atgccatttc ctttcatcct ccttattttg 94020
acattagccc caacctctac cccgagggtc tctctctctc tcttcccaac ttagattcct 94080
ctcaggggac acatagacaa cccagaaaag cagctctgat ctctcgtgga gcttccctga 94140
actccacacc gcacatctac tactgtttag gataacattt tgacttagtc cttaaacgga 94200
ctatttctca tagtcctgtt ttgtaaatga gattttaagc tctttgcaga cagggaccag 94260
tataaactct tgagtctctg tcaaacttag tgaactttct ttccagcctg ggcaacatga 94320
tgaaaacctc tctctacaaa aaaaatacaa aaattagcag ggcatggtgg ctcgcacccg 94380
tagtcccagc tactctggag gctgaggtgg gagaattgct tgagcctggg agtttgaggc 94440
tgcagtgagc catgatcacg ccactgcact ccagcctggg tgacagaacc ggactctgtc 94500
tcaaaaaaac aacaaccccc ctcccccaca aaaaacctta ctgaactttc ttggagaaca 94560
tgcacactgt agaaccttct gtatttggaa tattcaagtg caggtcctgc tgttctgtaa 94620
agaactgtca ccttgagtca ttgtttgacg gctcaggtcc ttggcacttt acccctttgt 94680
tcatgattga ttgacatttg gacctttgct gacctttccc tgagggattt tgtgggcata 94740
gtcaaacaaa aggaaattga aaaaaaaaac cccacaaatt ttgaaggtct gtcacttgga 94800
gaagaacagt gttcatgact cttcttcgtc atttcagcat cttaatcatc ttgagcctaa 94860
agatgaagga gagtggaggt gagcctgatt aaatgggagc ttttcctata ttgaggatct 94920
actaggtgca aggacctcac ttgggttatc tctagccttc ccccaactct gcaaggcatt 94980
tcacagatat ggatgcaggt tcaaagaact gaagcttaaa gcttcgcaga tttggaagcc 95040
tcagagctag aattctcccc ctagcctgtt tgtctctgaa gcgttggtta ttgcttgaca 95100
ttgcatccct ccctcttttt tccctctctt cctctttccc ttccctgcca ctgtctgcct 95160
gtcttcctgt ctgcctctct ttctgtctta aaattactgt tgctattcct tactggtctg 95220
gtctggtcta gttaattaaa aagaaggcta tcgtgaggta accctgggat ccttattggc 95280
acagtcatat tcccataact tttaggtgcc tgagtcctaa tggatctgta agacagtgcc 95340
catcagcgag aaataccac agcctccctg gatgctgccc acaggcatct tgcagatgag 95400
ggtgtttgcc ttctcacctg caaggggtg gggagaagtg gccaggggtg ggctgttctg 95460
gggacgcctg cctccccact ccctgagcct agggggaatg atattatggc ttcaggccac 95520
aactagacac attatctact ctgacaggga gccagtctgg agaaggatgt catggatgac 95580
gtgaagaaat tactgaggga aaaagagcaa ggcgtgagcc ctattggggc tgttttggtc 95640
aactcctggc agtagatgat attgatgctt tggaaatctc tattttcctt gcatttccca 95700
gcaaagtgcc attcccatct ctttctttta gaagcttctt atcctatggc tctgcctcca 95760
gtctttctca tcacataatt cttcaaaggc aaggcagact agtggttaag aacaaagacc 95820
cagggctggg cgtggtggct cctgcctgta atcccagcac ttcgggaggc caaggccggg 95880
gtatcagttg aggtcaggag ttggaggcca gctttgccaa catggtgaaa ccctgtctct 95940
gctaaaaata caaaaattag ccgggcgtgg tggcaggtgc ctgtaatcct agctacttgg 96000
gaggctgagg caggagaatc acttgaaccc aggaggtgga ggttgtagtg agctgagatc 96060
acgccactgc actccagcct gggtgacaga gcaagactct atctcaatta aacaaaacaa 96120
aacaaaacaa acaaacaaaa aagaacaaag tctccggagg cagtgcctgg gctcgtatcc 96180
tagctctgtc tcttgctgac tcctgcatgg cttgggcata cttgggcttt gctgtgcacc 96240
agtgttctcg tttcttagtg agttcgtatc tcacagcatt gttggagatt acaggaacac 96300
aaccaatgct ctcagaacca gcactcagga agtgtggtaa ttacatgctc ttctgggctt 96360
ctggtcatgc caccttcgac ggacaatgcg ccagcaccca ccagtttaag gaccagttcc 96420
tcctctaggc attgtgccca gcgcaccctt ctcatggaga tttgcttctc actctgcatc 96480
tgcaggtctt cgctgggtga tcttgggcaa gtcactttac ttctccaggc cttacttctc 96540
agctcttcag tgaggacatt gaacgagatt atttccaagg tttcttgtgt gctgacgtac 96600
tggggcctct cacattctat gtggtccaac ctttcctcct gttcctcagc ttctgccagc 96660
ccgccagggc aacctggtta tcaacaaatg tcctcgctta tggacatgtc ctgcttctct 96720
ggaaatctta gtacagcctg aaagctccat gaagtcagga ttgtgtcttt catgtttgat 96780
atgatttctg gcttctagca ctgtgctagg agctagcacg tgaacactgt ctgtgtagct 96840
ggaattaatc acccactatt ccagtcaaca attccttttt tttttgagac ggagtcttgc 96900
tctgtccccc aggctggagt gcagtggcat gatcttggct cactgcaacc tccacttccg 96960
ggttcacgcc attctcctgc ctcagcctcc tgagtagctg ggactacagg cgctcgccac 97020
cacgcccggc taattttttt tttttgtat ttttagtaga ggtggggttt cactgtgtta 97080
gccaggatgg tctcgatctc ctgacctcat gatccgcctg tctcggtctc ccaaagtgct 97140
gggattatag gcatgagcca tcgcccccgg ccctttttttt ttttttttt ttccgagatg 97200
gagtctcact ctgtctccca ggctggagtg cagtggcatg atcactgctc attgcaactt 97260
ccgcctgctg ggttcaagca attatcctgc cttagcctcc cgagtagctg cgactatagg 97320
cgtgcaccac catgactggc taattttttg tactttagta gagacggggt tttgccatgc 97380
tggccaggct ggtcttgaac tcctgacctc gtgatccaca cgcctcagcc tcccaacgtg 97440
ctgggattac aggcgtgagc cactgcgctc agctttccag tcatttctta agcgatgttg 97500
agttcccaag cactgtgccg gacacgcatg ggttgctgga ggctggcaga acccgaatgt 97560
cagtattcct gaagaatggc tctaagcaat gtaccttgca gagctcaagc agggctctgc 97620
tgcatcatgc ccatgaaggt gcctgctggt agaaggctgg ttctatactg gctgcttgcc 97680
tcctccttgt ggagacccac attgtgtctg gcaaagtgca gaaggaaatc aagcccttaa 97740

taacattagg gaactggact gggttcggtt tgcatctccc gagtctgact cgtgggagta 97800
accaccataa tagcagcgct gtgctgttga gggtgacttt gactatctgc ttgtgttgtg 97860
agcccactgt gtgccggagc actctctata cattcagtct tgtccgcaga tgaactccag 97920
tggacacatg tttctattcc ttattattat tattatcatt tgtttttctg agataataat 97980
aataatatta ttattaatat tattgttatt atattattat tattattatt attattatta 98040
tttagatgga gtttcactct tgtcgcccag gttggagtgc agtggcacaa tcacaatctc 98100
agctcactgc aacctccgcc ttctgggttc aagtgattct cctgcctcag cctcccgagt 98160
agctgagatt acaggtgccc gccaccacac tcagctaatt tttgtatttt tggtagcgat 98220
ggagtttcaa catgttagtt aggccggtct cgaacttctg accttaggtg atccacttgc 98280
cttggcctcc caaagtgctg ggatcacagg tgtgagccac cacacccggc ctattattat 98340
tattttttga gacagggtct tactttgtca cctaggctgt tggctcactg caacctccac 98400
tgacttcctg gactcaagga atcctcttac ctcagcctcc tgagtagctg ggactacagg 98460
tgcgccacca ctgccagcta atttttgtat tttttgtaga gatatggttt caccatgttg 98520
tccaggctgg tctcaaactc ctgggttcaa gtgatctgcc cgcctccacc ttccaaagtg 98580
ctaggattac aggcatgagc cgctgtgccc ggcttattct ttgttttatt gaacagatga 98640
ggaaacactt tcaatgagct taagggatgt gcccaaggcc acacggttgt gaccacagca 98700
tgaggattca caccttggtt cctgtggctc ccatcccccc tcccctgaat gaggaatcgg 98760
gtgagtgtga cgggagcagg cactcgaagg cttgggaatt ccaaagcagc tgatccatgt 98820
catcccagca cacgttaggg aggaagtgaa acgaagcaaa aatagagcct gtcagcgggg 98880
ggacagctgc cgctttctct gggaacagat ctccccgatt tatcttccag gttaggaaag 98940
ccccagctcc tggggtacca cagagaaata atgtgagcac agccagagaa attgccacct 99000
ctcccttcct cagaagaaga taaaggagac atcgtctttt tggcatcagt tttgaagaaa 99060
tctttttttt tttttgagat ggagtctcgc tcctgttgcc cagcctggag tgcaatggca 99120
caatctcggc tcactgcaac ctccgcctcc caggttcaag cgattctcct gactcagcct 99180
cccaggtagc tgggattaca ggtgcccgcc accacgccca gctaattttt gtatttttag 99240
tagagagggg gtttcgccat gttggccagt ctggtctcga actcatgacc tcaggtgatc 99300
cacctcctcg gcctcccaaa gtgctgggat tacaggcgtg agccaccaca cccagccagt 99360
tttgaagaaa tcttacttca caatcctgag agaaagagaa attgaggcac acttagcatg 99420
tgagactgat ttctcacttc tgctgctaca actgctgccc cattgaccgg gtctcagagg 99480
tgttataatt aacatcctct gactcttgct ggtactaaaa ttaacaacat gactggtccc 99540
ttctgcaaga gagggaaaga gatacacaca tgtgcacata cacgcacaca catgcctgca 99600
cacacacact tgcacgcaca catgcatgca cacacatcac acacacacac acacacacac 99660
acacagacac atgcagagtc cagaaggctc agagacagag agagagagag gaaggcacag 99720
ccacacacac actgggagac acttattatg gctcccccctg caaacggtgc tcccctcaaa 99780
gctttaggac cagggacgcc ttccctcact ttctgtgttc tgtacctcat accccattta 99840
ccagcctgtg ccaaccctgt atactttaag tcttcagtta ccaaagcatc catctgcccg 99900
tcctatattg agggggagtg gatgcgcaga ggtggtctgt tggtgttggg tagggaatag 99960
acgcagcctg tttttcgttt ttcttccaaa tgtcttcggc agggcctcag ctagtggaaa 100020
tacctggtgg ggagggtcaa gaaggaaatg acagtggggg gaatggggag ggatagcatt 100080
aggagatata cctaatgtta aatgacgagt taatgggtgt agcacacaag catggcacat 100140
gtatacatat gtaacaaacc tgcacgttgt gcacatgtac cctaaaactt aaagtataat 100200
aaaaaaaaat aaaattaaaa aaataaaata aaaaaagaat tcctatttta actttggaaa 100260
aaaagaaaaa aagaaggaaa tgacagattt ggtcctgaca gcctgggagg cagagaggag 100320
agagatggag ccaagagaat ggggctgggg ccacgttttc ttgtggggca attccgtgtc 100380
actgggccag agcatgctta ttgtgaagac ttcccctcag tgtcctctgg ggaagggtca 100440
gctgtgaggc agtggggcgt gagaatacga tgaaacaggg aaacctggaa gctgccgatt 100500
cccaggcctt gaagggggtgt tctgatgtct ttgacattcc agcaaacata ttttcacagg 100560
aaaactcaaa aaatataaac tcccttttta tagaaataat gtgcatggag aaaagtgcac 100620
atgtcatatg tgtatcgata gcttgacgaa tgttctcaaa ctgaacctga gtgtggagcc 100680
agcacctgga tcaatagaac atttccagca ttttggaagc cccctcccag tcagtcactc 100740
aggagaactt tgtcctgact tctaaccaca cagattagtt taacctgttt atgtacttaa 100800
tctaaataac atcatcacac actcccctgt gtcttgcttc tttcactcaa aattatgttt 100860
gtgggatata ttcatgtggt acgtgcattt tcagatcatt ctcactggtc gctagattgt 100920
gtgcatattt taaaaaatcc attctactgt tgatgggcat ttgagtagtt tccagtgttt 100980
ggctgttatg aatagagctg ctgtgatatc cttagacttg tcttctggtg aacatatgca 101040
cacatatctg tgcattgata ccacggagtg gagctgcagg gtcatagggt ttgggtatat 101100
tcagcattag taaatagtgc agaacagttt cccaaagtgg ttgtttatcc tttttcagag 101160
tgtctgttca ccagcacgta tgaattccag ttccacatca aagccaatta ttggcattgt 101220
gtggttttta aattttagcc aacccattac agaaagagca aatgaaagga atctaacagc 101280
aagtaatcag gagagaaaag aaaatgctcc ctgagaaagc attatgattt catgatacgg 101340
gaaatcccgg tgtacaatgc cacacattgt gtacttttgc aggcggaggg cgaggtgcag 101400
ccaccttctt gtgagcatca gggcagtcct tgccattggt cagggaccac cacttgactg 101460
cagtaatacc caacagacca tgtcataatt cttcctcagg tgacagagct ttaatggatc 101520
cgaccccaac agaacaaact tggcttcaga cttgcctgtg ggcctcagta aatattcaac 101580
agactgtgtc cccctgttca ctcccctgtc attagctgag gctggtacca taaatcacag 101640
ctccccttcc tgttgtaagt ggcccctctc agctgacggt actccagtcc tgacattcag 101700
gagcagtgcc tctcctgggc tgcttcccag ggctggcagg tcttggtggt cacttgtagg 101760
agccgaggag tgagtgtgtg cagtcctgga gtcactgctg tggggtgact tgcaggagcc 101820

tcttccattt gatgtgagaa tgatagatcc gtcaagctct taatatgtgc ccagcaacat 101880
gctaagtcct ttatgttgtt tattgcaacc tgtcacagtc cattcaggct ggtataacaa 101940
accaccttag actggtagct tgtaaacaac agaactttat ttgtcatggt tctggaggtg 102000
gggaagtcca agattaaggc tcagacatat ttggtgtctg gtgagggcct gtttcctggt 102060
ttagcaatga tgccttcttg ttgggtcttt atatggtgaa aggatcaaat aaggtatttt 102120
atacctcttt tataagggca ctaagccctc cagcctcatg accgaatcac ttttcaaagg 102180
cctccccttt tgagatcatc accttggcag ttaagagttc aacgtatgaa tttaggggtc 102240
gggggtgtag gtaacaaaca ttcaaaacat catacaacct cacagtagtt ctatgaaatg 102300
agtagatgtt gtttaccacc attttcagat gaggaaatta agggttaaaa acatttcccc 102360
agagtaagaa acgtagtaaa tttgggagtc taacttcaaa ctgtaatttt taattactgt 102420
acttccttct tcttttctct taggggctgt ttagatactt gagcttcctt ttgaagccat 102480
cttcctcttg ggtctaaaag ctttcaggta gatttaaatg ggacaagaag gccagatgca 102540
gtggctaaca cctctaatcc cagctatttg ggaggctgag gcaggagaat cacttgaacc 102600
tgggaggtgg aagttgcagt gagctgagat ggcaccgttg cactccagcc tgggcaacag 102660
agcaagactc tgtctcaagg aaaaaataaa taaaataaat aaataaataa atgggacaag 102720
aggaagtgac ctgcagagca catattgggt tcagatgtaa ttatctgctt tttcaatgat 102780
tttaagaaat tagaaccact gtttttctct ggctcttttc atgacttttt tcaaatgttg 102840
gttttcagca gttcaactat tatatactta ggtgtgattt aaaaaaatat atatcctgca 102900
tgggatttgc tgatattctt atatctataa attaatgtct ttcactgaat ttagaacact 102960
ttcatccctt cccacccccg atattgatct tgctccattc tcttctctcc ttttgggact 103020
aaaattacat gtctgttagg ctttttgata ttgtcctaca ggtctctgag gctctcttca 103080
atttttcaaa tttttttctg ttattcagat taaataattt ctattggcct ctcctcaaat 103140
tgtactgact cttggttcct cttcagtctg ccgttaaatg gatccaattg attgtttcag 103200
attttttttt taattgtaga atttccattt ggctctttct ttgctaagct ttcttatctt 103260
tttatttact aagaatacat tttcctttga gtctttgagc atagttataa tagctgctta 103320
aaaaaccctt gtctgctaat tccagaaact gggtcatctc aggatcatgg ggacggtccc 103380
cataactatc ttttttctta agaatggatt acatttcctg tttttgtttt ctttttagtt 103440
gtttgttata ttgagctatt ataaattata tcacagacat tgtgagtgat acttggtaag 103500
gactgattct gttttgttcc tccaaaagagt agtgcttttt tgtttcagta ggtagttaat 103560
tcagtaatct aagacttcaa attctgactc cctagtattg ggcagcaata taaattgctg 103620
tttagttctt ttggctttaa ctgggctact ccatacaaac ttggctcagg agtcacttag 103680
tgacttaggg aacatttgta cacagaattt gaactcccct ttttggaaac tctcttttta 103740
ccaggatttc ttctttcact ttcacctatt gtgttgcctt gatctctgtc ctgatgtttc 103800
agtgcagcaa caccatctga gtgttagcca ctcacatgtt gcagaaccgg gcctactctc 103860
agactaaaat tgaccaaaag caggaactca ctggcttgct gaatgtccag gtcttgatca 103920
cactccagaa tgtcctactt ttggtcatca ggtgcctaga ttttttagaa atctttgttc 103980
taatttttag aaatatttgt tcagagttta tagttgctat ctaccaggga actgcttcaa 104040
taggaactac tcaaccattg ccagaacctt cttgaacttt ctagaaacac tgtcttattt 104100
attttatttt attttttaga caaaatctca ctctgtgctc tgccacccag gctggggtgc 104160
aatggtgtaa tcttggttca ctgcaacctc cacctcccga gttcaagtga ttctcgttcc 104220
tcagcctccc aggtagctgg gattacaggc acctgccacc acacctggct aattgtttgt 104280
atttttagta gagacggggt ttcaccacgt tggccagtct ggtctcaaac tcctgacttc 104340
aggcgatcca cccatctcag cctcccaaag tgctgggatt tacaggcatg agccaccacg 104400
cttggcctag aaacactgtc ttatttaaat aagaaagagt gtctctcaga atggaaagaa 104460
aacagctttg gagtcaaacc caacttggat gcagaatctc actctatctt ggtttttttt 104520
tttttttttt tttttttttg agactgaatc tcactctgtc acctaggcta gagtgcagtg 104580
gcatgatctt ggctcactac aacctcggcc tcctgggttc aagccattct tctgccttag 104640
cctcctgagt agctgtgatt acaggtgtgt gccaccacgc ctggctgatt ttttgtaatt 104700
ttagtagaga cggggtttca ccgtgttagc caggatgatc tcgatctcct gacctcgtga 104760
tccacccgcc tcggcctccc aaagtgctgg gattacaggc gtgagccacc gcactgggct 104820
ggatatgttt tttaagaaga aaacgtacct tactgtttcc ttcgtgattt cttcactagc 104880
cgctgcaagt gtgtgtggct ctgtgcattg tgggcagggt gaggtagcta gcttgatttc 104940
cattttggga atgagtggtg cctcctcatg gaagacttct taaaacagga gatgggtgcc 105000
ctctgttatt cttgtatggc aggcagaata acgtccccct aaatgtccac atccgaatcc 105060
ctagaacctg cgaatatgtt gcatgtcaaa agggactttg cagatgctag taaggctaag 105120
gaccctgagg tggacggatg attttggatc atctgggtga gcccaatata atcacatgcg 105180
tccttaggag tggaagaggg aggcagagag ttattcagag acagagatat ggccacagaa 105240
gcagagtcag agatgccttg gtgttgtatt tgaagatgga ggaagaagcc acagccaagg 105300
cctatgaagc agcctttaga agcttggaaa agcaaggaaa cacattccct ctcagccacc 105360
agaaagaaat gcagccctcc caacagcctg attttagctc agtgagaccc atgtcagact 105420
tccagaaatg taagatcata agtgtgtgct gttttatttt ttatttcttt ttctgtttta 105480
aaccactacg tttgtaaaca tttctttggc agtaatcgtt gcttttgatg gaagttggag 105540
cagtggtcaa gctcgtgggc tttggagcca gtctgccagt ccgccttgtt tcaaacccct 105600
tttcctttct ctctttcttc cttccttcct tccttccttc cttccttcct tccttccttc 105660
cttccctcct tccttccctc tctctctttc tttctttctt cttttttttt ttgagataga 105720
gtctcattct gtggcccagg ctggaatgca gtggtgtgat cttggctcac tgcaacctcc 105780
gtctccttgg tttaagcgat tctcattcct cggcctccca aatagctgga attacaggca 105840
tgcaccacca cacccagcta atttttgtat ttttagtaga gacagggttt caccatgttg 105900

gccaggctgg tcttgaattc ctggcctcaa gggatccacc agcctcggca tcacaaagtg 105960
ctaggattac aggcgtgagc caccgcgccc agcctcaaac ccttttcctg tgattttttt 106020
tttttttttt aaagctgtgt gaccttgtgg aagttgcttc acttctctgg gcctcaaatt 106080
tctcatctct aaagtcggtt aacaataatc tacaactcat agtttttgt gaggatgaaa 106140
tgagggtttc atacatgcag aaagcctgga agtaagtttt agctgtggcc attctgggca 106200
ggaatccctc actaggtaac ttagagcggc ggatcagggc acagacttgg gagacagact 106260
gtctagcttc agatttaggc ttcttactag tctgaagtag aatggttgtc tgttcctcca 106320
ctgcttcatc tggaaaacag gggcaggcat cccagggtgt ttgagagatc atatcagatg 106380
gagaggccag agtttttagc cgatacatca tagacattcc ccaaaggaca gttattgtta 106440
tcattgggtg gatggggaat attgagcctc cagccctagg cattgtggat caatttgttc 106500
ttcagttatc tttggaataa tttaaataca ctggattggg aagacaaagc tatcaccctc 106560
ctgatgggag agtgctgggt acctagcagg tgctcagtag ttagtgaatg aatgagtaag 106620
aacagaacag gtcctaggcc ctgccctgaa gaggctcaca gtgcaggaca aggcctggag 106680
tcaggccagg gttttgtggt tgtctttaaa aagaaacaaa acaaagaact acttcctacc 106740
agctgaccct aaacccctca gctccctgca gggacctctc agccctgcag gggagcccag 106800
gctatgtgcc aggcctggcc ttgctgtcag ctttctaagc cagtcacaaa cattctcctc 106860
tcaactttat aatggatgcc ccccagcctc ctctcccctc tcagggtgct cctgaggcta 106920
tgagcagctg gcagttccct gtctgagagg tccttcgtac cgcaggagag gcagctctca 106980
cctgaaggcc tccctggaca ccggtctcaa ctttgtgtgt taccagttat ggagaattat 107040
ttttagtgag ttacactctg ttagggccaa acacaaggag gaaagagtga ctgaatactg 107100
ctctgagtca gtgactgtga caagactgtt tttttcttct tccctgttgc aggcctgatc 107160
ttttggccag aaggagatta aaaagatgcc cctcaagatg gctgtgcctg tcagctgcat 107220
ggagcttcgt tcaagtattt tctgagcctg atggatttac agtgatcttc agtggtctgg 107280
ggaataacgc tggtggaacc atgcactgga atgacacacg cccggcacat ttcaggatac 107340
taaaagtggt tttaagggag gctgtggctg aatgcctcat ggattcttac agcttggatg 107400
tccatggggg acgaaggact gcagctggct gagagggttg agatctctgt ttacttagat 107460
ctctgccaac ttcctttggg tctccctatg gaatgtaaga ccccgactct tcctggtgaa 107520
gcatctgatg cacgttccat ccggcgctca gctgggcttg aggtgaggct gtcctggctg 107580
gtcacggagg gttcactggg atgtggctgg tgttctcaac gaggtggcat ttaatgagca 107640
ccctctgtgt ggttggcgtg atgctgggtg cctgggggag acagatggaa ctaaagaggt 107700
ccttcctgga agccgttact gttccctgcc ctgacgtgtg ttattgtacc tgtcaccatt 107760
ttctgatgtt cccagtttac gtatctgtca ccatctacct ccctataaag tgcctctgct 107820
gttggttctg tgtccttaag acattgtgtg gcatttggca cagtctctca tgctgcagaa 107880
agggtaaatt aagacaggat tgttaatgaa tgaagacatg ggagattcaa tggaaagata 107940
tgcatggggg gataatttct tggctctgtg ctcagagcta ggaacaacat aaggccatat 108000
ggctggtgct gtgagggttg tatgtgggca gatgcatgtg gaggcagaca ggcctgggta 108060
tgactcttta acacatacac actgcatgcc tccaggcaga ctgatggatt cctttccctg 108120
cagtgaaggg ttaagtatta tcacacgtca caggactgga attgaagatc gccaagtaga 108180
cattgaaatg ctgaccacat gacagacact cagcatgtgg tgggtgatgt tggctggggg 108240
tagcttttgg ggatggtgga catttcttgg ccctgatctt tctgagcaga gccagggagg 108300
cagtgctgga ctctgacctg ggcacagctg gttggcgtgg acagttgatt aaaggtcaca 108360
gtttccctgc agcccatggg aaggcagagt gacaagtcag tggcagatga ggaaatggag 108420
tctcatgcca gctggaggga gggagcagct gggttggcca caaaggcctg ttgcagttct 108480
gcagctgaca agtggaagga ctggcctctc aggtccaggc ctggtggcct gggaggctgc 108540
tagctctgag cgatgagtgg tgccaaggtg cctgaaggag agttgcagct caaacagcaa 108600
gtggtccagc tctcagacca cttgaggcca cctggcatgt ggcggccaag accacagttg 108660
gggcggggcc ttgagtctga ttttgattgg attcctagtt cggctcatgc ctgacagcac 108720
tttctcagac aggcttccct gacctcctaa ggtaaataat gtccctctgg gtgttacatg 108780
ctgttctcac ccatagctct tagatgtgtt ctaattgtat attcatatgt gcaatttact 108840
gggtttcctg tctttctctc acactagaaa gtaatctttc tgagttgagg aaatgcattt 108900
gccttgcaca caggctagac acatacatag taggcatttt atttgttgca tcacagttgc 108960
cttgatcaaa tatggtgact ctccgtggtt ttctcttggt ttctttgaaa aggtcagaca 109020
gaagggaaac actcttttct tgagtatata tatgacttag cccagcaatc ccactaggag 109080
agcaatggtt tatccattat acagttgagc gcctggaggc tctgaggggc catgtacctt 109140
ccccaaggtt atttatccag gagaagacag agccagaagc tgagaactgc ctcgtgtttt 109200
ataccaaaca aggccccttc agctgaagac ttttccttgc ggtcttggga agggtgtgtg 109260
tgacggtgag ctgcagcagg tgctgggctg tgcttgactg tggtcacgta ggggcaggca 109320
gaggagccat ttcggggggc tgctgagggt ctggagagag gctcacagtg gtgagcaaag 109380
aggttctggg atgggagggg atcagttggg aaagacagag gcagatactc cccactcctt 109440
tgtgccttcc cagtcctctc tcacccctc ctgccctctc cccacaccct atcgtggtgg 109500
cagctgtgcc tcacttatca catggtccat agctctgttt tttcccttt tatcagttac 109560
tgcctagaac aacccaattg tggtggtctg ggttatctcc cactcttcac agtagggcca 109620
gagtgggctc agacaccaaa ggacacagaa tggttatggc aatgaataag attccttctc 109680
tgtgtcagca aggatgctga tgtagtaata atctccattt attaagccat gctgtctaca 109740
gggcacatag cacatgtttt tccattcaaa cctcaaaaca gagctatgag atgcgcacca 109800
ttatgatgat tatctgtttt actgtttttt gttttgtttt gtttttgttg tttttttga 109860
gactgagtct tactctatca tccaggctgg agtgcagtgg cgcaatctcg gctcactgca 109920
acctccacct ccttggttca agtgattctt gtgcgtcagc ctcccaagta gctgggacta 109980

caggcgtgcg ccaccatgcc tggctaattt ttgttggcca ggctggtctt gaactcctga 110040
cttcaagtga tctgcctgcc tcggcctcct aaagagctgc gattacaagc gtgacccacc 110100
ttgcccggtc tgttttactg ttgaagtaac tggagcaccc agagggaaat caagtgtccc 110160
tagagaatac aagcagcatt tggacagccc tgtctgactc caaagccact gcacaacatt 110220
gcctggagct gagagatgca cattctccct tcttggccac tggtgactgt gaccttgggc 110280
aaactacctc tgctttttgg gccttagtac attcattcat agagaagctg gctaatttct 110340
cttctcagcc tggagacttg agagtgaaag gggcactgta tcacttactc tgggatgaca 110400
ggtgtatacg aggatgtagc tgggcaagcc aggcctagtg gctcgcacac tcatcagtta 110460
gatggtgggt gctggtgctg ctggtgcatc atgcatacaa ggctggctct cccagttttc 110520
cctggacttt tgcctgaaca gtttgcattt ctactcgtgg tgcaggagct tgctgttctg 110580
atttcatgtc acaagcctgc tttcagccac ttagaagagc cctgttatcc ctgactgccg 110640
ctacagcagt cccaacctgt gttgttttca cttgatgggc tcctggaatt attgacagca 110700
cactctctag tcgtacgggc tgccctgcca cctacactga catcctggcc aggttcctgg 110760
agatggcctg gcatcttgga cctgttcccc acggggtgcc tggagcctct gatggtctac 110820
ccagggtggc aatgagggaa aggctgtgac ttgatcatct ccaggggttc ttcggtgaac 110880
agcccaaggt tgcttaacag ctggtgtgta tgaatacctg gaatgtgcca gttcaccttc 110940
taatctggca aggagaatgt gtctcctcct tatgcatatg aacaaactga ggttcagtga 111000
gatccagaaa gctgccacgg ttactgatgg agagagcaga gaagctggtg tttgcagtcc 111060
catctgtcag ccttgacacc cctactcctg tccagccagt gtttctcaaa gcgtgctgat 111120
gagcaatgca agatgatttc atgttataga taagaataaa aaaattgttt tgtgtttaac 111180
tcaaattaga aaaaggcaac aattggtatg tgcgacctgt ggttttgcag atgatactgc 111240
ttaggatgtt ggtacttaag aaaaggtcaa cttttcaaaa atactattag tgacatgtgg 111300
acctagtcct cctgaagagg actacattgg ggcaccggta attgtttcta tttgcggtac 111360
tctggctgtg tggctctggc cacgccactg gaggcagtgt ctgagcctgt gacttgagta 111420
gtagctctgt gtcatgtctg ctgattctcc ccaaatcctg aagattcatg atgaagtgac 111480
tggccggctt ggtctgaagc tagattgaaa acaataagga tcccagaacg atagcacttt 111540
acaatcctat aatttggctc aaattgcctg cagttactat ctcagccctg cctgttatgt 111600
tcattgagca cccaaagttt ttcagtcaat tcctgagtta attatttctt gggattgaat 111660
tatgaaatag taaatatttc cactatgcaa tcaattggtg acttattcat gtattcattt 111720
cattcattta gtctcaataa attgaagata agggctgagc acagtggctc acacatgtaa 111780
tcccagcact ttgggaggcc gaggtgggca gatcacctga ggtcaggagt tcaagaccag 111840
cctaaccaac atagcgaaac cccatcacta ctaaaaatag aaaaattagc tgggtgtggt 111900
ggcatgcgcc tgtaatccca gctactcagg aggctgaggc aggagaatca tttgaatctg 111960
ggaggcggag gttgcagtga gccgagattg caccactgca ctccagcctg cgtgacaaac 112020
tgagactcca tctcccaaat aaataaataa ataaataaaa gataatatta cctaccccat 112080
gagtttatta tgagaattaa ataagagaac atattaaaag gtttcattca gtgccaggca 112140
tataatatgt actcagggaa tactagtttt ttttaaataa aattttaaaa tgggattaga 112200
aggtcaaagc atataggcat aaaggtataa aaaatattga agatgagtga gttactaaaa 112260
tttaacatta cgtttagctc tgagcttcct aattagcaca acatgctaag taggttatat 112320
ttgtatctaa agtaaagatt ggcaaacttt ttatatcaag ggttaggtga caaatgtttt 112380
cagctttgca ggccacacag ctctgtactg cttgtcagtt ctgcctttgt atctggaaag 112440
tagccataga taatatgtaa atgagtgggt gtggctgtgt gccaataaaa ctttatttac 112500
aagaactggc aataggcctt taggctgtag tttgtccttg gcctaaataa aggaaacatg 112560
tttgtctttc aaaggcagaa actcctcctg gatcataaac attgaaaaaa aaattgttac 112620
aagatgcaat atttctgtga gacttgttaa gcagtatatg cgcagtgctt tcattaggat 112680
tttacagaaa acttagaaga tggccttcac atggctagtt tccttactgt tcattcagca 112740
gacatttact gagggcttac tatgtcctag gctctgatat ccatacatgg taaaacgtaa 112800
aggcatgtgc attttggcag gggtgctgc attgacaagg agtggtctcg gtgacagttt 112860
tgggaagtca gtttagaaca gtattggaca cactgttcca tccctgaatt acacacaggc 112920
ctcaatgcta acttgagtgg gcttttggtc cagcaggcct gggctccagg ggcagtcact 112980
cagcgtcctg cctcaagagt ggttgtgtca tgaaatcttg gccctctttg actgatagct 113040
acaattattt gaagagtgtg gtttttagaa caaaactaaa tctgttttga agctctgctg 113100
ttttatacct gctgtgtgat cttgctcaag tttttatttt tattttttc tgtataaggg 113160
attttatttt atttttattt attatgtatg tatttatttt attttaagtt ctggggtaca 113220
tgtgcaggat gtgcaggttt gttacatggt agacgtgtgc catgtgattt gctgcatcta 113280
tcaccccatc acctagatat taagcccagc atgcattagc tattttctct gatgcctttc 113340
ctcccccccc ccgcccccga cccacaggcc ccaatgtgct caagtgtttt aatctctctg 113400
agcctcactt tcttcatctg caaaccataa cacctctgaa tctatttgtt actctaatgc 113460
tagctatcta gtgtttgctc tctaaatgtt agttcctcca cctggggct gggcgggcgg 113520
tggctgacct caccccatga agctgtctcc tggctttccc agctagagcc tgtgggtatg 113580
aagattgcag cccctcctc ctgggggagt attcagtgta acaagccttg agaaaacact 113640
ggcctgtgga attccaaagt taatgaaggg aacagattcc aagagggtct ctgtttgacc 113700
ttccactctt tccccaaccc cgttacccct gagcgagttt gggggcaagc ataggtttgt 113760
aggcactgca aagaagaact ctcacgctgt ggtgcaggac cgtggccttg gcctctggat 113820
atctgggcca ggtgggtgca tttgggcctc ttccctacag cgcctggtca gactgcaaga 113880
gacataggaa gcagccaggg caggctatct taagaggagg gagcatattt gataaagtta 113940
gaaaatgatt ctcagctgtc cataggaaga gcagagtaag gggctatgtg agaagtttgg 114000
aagatggccc agttccaaaa atgaagaaag aaagtaagaa agaaagagag aaagagagaa 114060

agaaaggaag gaaggaagga aggaaggaaa gaaagaggaa agaaagaaag aggaaagaaa 114120
gaaggaaaga aagaaggaag gaaagaaaga aagaagagag ggagaggaag gaaggaagaa 114180
aggaaagaaa gaaagaaaga cagacaaaga aagaaagaca ggaaggaagg aagaaaggaa 114240
agaaagaaag aaaaaggaag gaaggaaaga aggaaggagg ggaagaagga agaaagaagg 114300
aaagaaagat tcattattct gtcctctgaa cctgagtgaa gaaaaatacc ctgtcctttg 114360
tacctgcgtg aagagagaaa agaaaagaaa agaaaagaaa gaaagaaaga aagaaaagaa 114420
agacaggaag gaagaaagga aagaaagaaa gaaagaaaga aagaaaaaaa gacagacagg 114480
aaggaagaaa ggaaagaaag aaagaaaagg gaaggaagga aggaaagaag gaaggaagga 114540
agaaagaagg aaagaaagaa gattcattat tctgtcctct gaacctgagt gaagaaatat 114600
actctgtcct ttgtacctgc gtgaagaaag aaaagaaaag aaagaaaagg gaaggaagga 114660
aggaaagaag gaaggaagga agaaagaagg aaagaaagaa gattcattat tctgtcctct 114720
gaacctgagt gaagaaatat actctgtcct ttgtacctgc gtgaagaaag aagagaaaag 114780
aaagaaaaga aagaaagaaa gaaagaaacg aaggaaggaa gaaagaaaga gtgtaaagaa 114840
ataaaaggct gtaaagaaat aaaataatgt aaagaaagaa aataatgtaa agaaagaaaa 114900
gaaaggaaag aaagattcat tactatcctt tggacctgag tggtgttttc agttaaaatg 114960
agtggagtag aacccagaga tcaggctttt gaggacggta aagcctcaag atacattctg 115020
tttctggctc agggaacctt cagggaaaag gctttggaga ccaagctagg cagggaagcg 115080
cctttgctta tcatgctgga gaccagactg aggcagtgaa gtcaccataa ttgatgggcc 115140
acactagact ggagccaacc atggcccatt gctgagactc tgggcctcag ggtggccttg 115200
gctggaacct gtattccagt ggccctggtg gctctcttcc ctctcacctt cccaggaggc 115260
agattatttc cctgtggtgg gggtgaggcg gtggcgtggg ggcttcttcc ctctctctct 115320
gtcttgtagc cttgttaaaa tagtggccac acgctgatag ctggatttcc agtgcagttg 115380
ctggggatgg gggtctttcc ctctcatctg tctgtcaccc tttatcactc cacaaggggt 115440
gggggtactg gacagaaaga gtgtgtccca cctgattact gggaagtgca atggacaccc 115500
ccaccccccg ttgacttatc tggctccaac cgaagaacag ctgttggtgg tcaggtctct 115560
gagtcactca gtagcttggg actgggttca gtgggagatg atggcataaa caaataaaca 115620
ctggctggcc cccagctctc agctttctgc tcactggggg gccccagaag cttcctgttg 115680
cgtctgcagg gaaaataagg atacccataa tgaaaatagt tttcctgatg gagaaatgct 115740
ctggggaggt tgttgaggca tgttagaaaa tccctgaggc tttctggctc tgtgagctaa 115800
gggaggacac ttggcctctc tgggctttcg cttgctcctt tgggaagcct gttattgtga 115860
aagtgagatg atctgggagg taaaactggc cagttgtcca tctcccctct ctgaacatga 115920
tgcatagtaa gccctgaatc tcatttaaat ttggaagtca gactgacttt gtgactgtcc 115980
tagtttccgg ctgttgagtc agctcaggca gttcttggga cttctgtgag tctctggctc 116040
cccgtctctc aatggggtga cagtcctcgt cttgtgcccc tcacgcgctt gttgagatga 116100
cagatgagtc acctctggga aactgcctga gagctggatg acaccactct gtgggattgc 116160
atttcatcca cagagacatc tggaaaagga ggcagagcag agcctgactt ttgattattt 116220
ggggagggag cctggagctg gtggaagtga gggcatagag cacattcctt tcaggccagg 116280
cagtgcttgg ctgctgaggc accggtggag acctcatgat ctggtggtcc gcctccctct 116340
tgtgctgtgt gtgcctggag ctctctggct tccttggctt tccactgtct ttcccaaagc 116400
ctccatgtat tctagaatct gctatggagt ttcaggggga tctctgtata gtgtgaatat 116460
ctagctctga aacacaaaag agaaagctac aaagggcaac gtaacaaaag ccgtattccc 116520
acccaaggat tagcacatgc tcatgaggtg gcatgctttg ctttggatca ttgtctttat 116580
tttattttat tttgtttatt tatttattta ttatttattt tttgagatgc agtctcgctc 116640
tgttgcccag gctggaatgc agtggtgcaa tctcagctca ctgcagcctc tgcctcccag 116700
gttcaagcga ttctcctgcc tcagcctcct gagtagctgg gactgcaggc gcgtaccacc 116760
acacctggct aattttttat atatttagta gagatggggt ttcactgtgt tagccaggat 116820
ggtcttgatc ttctgacctc atgatccccc cacctcggcc tcccaaagtg ctgggattac 116880
aggcatgagc caccacaccc agcctggatc attgtcttta aagaaattta aaaaaaaaaa 116940
tagataaatg ggaagtctat gagccttgtc tcccattccc catccttctc ttcatacaag 117000
tacctctcct gaacttgggg tataacaggt catgctctta caatttcatt acaaatctat 117060
ttgaagatca ctggtagcac cattttgtgt gtttttaaat tcttaggtag gtgacataac 117120
gtataagatg tctgcacttt cctcttttct cccggcgcta tgctgggcag atgatgctac 117180
tatgcttgtt cattcattcc ctgggccaca gagtcttggg ttgtttccag aattttgctt 117240
ttgtaaacaa tgctgtcatg agcatcctag cacagggctg caggtgcatg tctgggtgtg 117300
tcccaggcag tacagggcac agagcatgca catttgcagc ctgactggag gttgccacat 117360
cactctccag ttggcactat ttcctaggtt ccttccctga gagcctggga acttctggaa 117420
ggcaggagtg ggtggtgttc accactatat acctctgcca gtcagcatgc ccccacaccat 117480
gtaccagctc atgacacgcc tgaatggggt gaattctgga aggggacaag gcaagtgcca 117540
gggcaggtcc agagtctggg tgtggaagga gcaacataat gggggcccct tgcagaagca 117600
gcagaaagaa caaagaaata ggggcctgga gtgggggtgt gaatgaagac attgctgttg 117660
agaagatact tgtgaagaag ccaacagcgt tgtctgggcc tggctggata tcatgaggct 117720
ggacatggag gtcccaggca ccattgctgt gggcttttgg ggagttggat gcacagtgga 117780
gagctgagga ggaagttggg cttgctgcag agggctgctg gaggggctgg ctggagtctg 117840
gctctggccc tcactcactg agggttatcc aagtgaccca ttgcaggaag aggggtgggg 117900
gacccagagt tactgtaacc actttatgag tctgtctcag ccaggatctg gggcttctag 117960
caaagcccac agcacaggca cattttagga aaggcaagat tgagatgccc tcggtggccc 118020
agaaggacac ttcagggcag cagggctgta aaggtcagga gggggaaact tctggaagga 118080
agttgaatgt tgtgggaaaa agctgacata aatgaacaag gaatgatttt tttcttttct 118140

tttttttatt tttcttttta gagagacagt cttgatatgt tgcctaggct ggtctcaaac 118200
ttttgaactt gagtgatcct cccaccttgg cctcccaaag tgctggaatt ataggcatga 118260
accaccattg tacagccaca agggaagatt aaacatagac agtttgagaa gagttttttt 118320
ttttttaaat ttctaatttt atatttaata gagatggagt ctcactatgt tgctcaggct 118380
agtcttaaag tcctgagctc aagcgatcct cctgccttgt tctcccaaag tgctgggatt 118440
acaggcttga gccactgcac ctggccaagg gtactttttt tttttttaag aaaaatatcg 118500
ttagaaatag aaaatttgga tttggtaatg gatagtgcat actatgaaat aacctttttta 118560
cttcttcaac acttcccatg ataataacac taatacaaga tctttggtga aaacttagaa 118620
tgcgtagaaa tgtgttggtg gttgaaagag agccccataa ccctccactg aggagtaacc 118680
tctgtttgac cttagaaata atacctgtga acagaatggc caacttgtcc tggtttgcca 118740
gagactttcc tggttttggc acggaaagtc caacactctg ggaacctcct cagtcctgtg 118800
caaactggga tggtcgttca ccctacctgt gaattgaatg ataccactga gcatcaagca 118860
ctttgacagt tcacaagtca tttctagtgc tgtcattgtg tatacttggt tccggctgtg 118920
tgcaaggcat tgtgcgaggc tgcatgttct ggagctacac cagagctcca gagctgaagt 118980
tctattttct tttttttttt ttttcacttt ttattttaga atcaagagat gcatatgcag 119040
gtttgttaca gaggtatatg ggatgcggtt gaagtttgga gtatgattga actcatcacg 119100
caggtactga gcatagtatc cagtagtttt tcaatccttg tccccctctt ttcctcccct 119160
ttctagtagc cctcagcatc tattgttccc atctttatgc ccatgtgtac ccaatgttta 119220
gttcccactt ataagtgaga acatgcggta ttttgttttc tgtttcagca ttagtttgct 119280
tatgataatg gcctccagct gcatccatgt tgctgcagag agcgtgattc cattcttttta 119340
tatggttgca tagtatttct tggtgtatat gtgccacatt ttctttatcc agtccaccac 119400
tgacgggcat ttatgttgat tccatgtctt tgctattgtg aatagtgctg ccatgaacac 119460
atgggtgcgt gtgtactttt ggtagaacaa tttattttcc tttgagtata cacccaggta 119520
tgagattggt gggttaaatg gtagttcaac ttggagtttt ctgagaaatc tctaaactgc 119580
tcctcacagt ggctggacta atttacattc ccaccaacag tgtgtcagtg tctccttttc 119640
tacacaggcc caccaacatc tgttattttt tgacttttta atgaaaacca ttctgattgg 119700
catgaaatgt atctcattgt ggttttgatt tgaatttttc caatgactag tgatgttgtg 119760
catttttttca tgtttggtgg ctgcatgaat gtcttctttt gagcagtgtc cgtccttgtg 119820
ctttgcccac tttttaatgg gattatttgc ttctttgctt tttgatttaa gttccttatc 119880
gattctggat attggaactt tgtcaaatgc atactttgtg aatattttct cccattctgt 119940
agcttgtctg tgtactccct tgatagtttc ttttgctctg cagaaactct ttagtttaat 120000
taggccccaa ttgtcaattt ttgtttttgt tgcagttgtt tttgaggact tagccataaa 120060
ttcctgtcaa ggcctatatt gagaggggta tttcctcggt tttcttctag gatttttata 120120
gtttgaagtc ttaagtcttt aatccatgtt gagttatttt ttgtgtggtg ataaataggg 120180
attctgtttc attttcttct gcatatggat agccatttat cccagcacca tttattgaat 120240
aggaagtctt ttccccattg cctcttttttg tcaagtttgt tgaagatcag atggttgtag 120300
ataagtggct ttatttctgg attctctatt ctgttccgtt ggtctgtgta tttgtttttg 120360
caccagtacc atgctgtttg ggttactgta gccttgtagt ataatttgaa gtcaggtaat 120420
gtgaggtctc tagctttgtt cttatttctt agaattgctt tggtcattca ggctcttttt 120480
tggttccatg tgaattttag aatagttttt tctacttctg tgaaaaatga tgttgatagt 120540
ttgaaagaaa taacattgaa tctataaatt gctttggaca gtatggccat tttaatgatt 120600
ttgattcttc caatcaatga gcatgaaatg tttttccatt tatttgtgtc atgtctgatt 120660
tctttcaaca atgttttgta gtccttgtag agatctttca cctccttggt tagaaagtat 120720
tactagatat tttatttatg tgtgtatgaa gccctgtttt ctatccttaa ggagcttaca 120780
acttgtcttt ggtatgcagt aggggcattt cacttgtgag agtgggggtc ttactgtagc 120840
ctgggcaata ccctctttta ttattgggta ctaaggattt aaggatattt ttgttctggt 120900
gggttatcca gttttgcagg ttggtaccag gagagaggat gcatgttggt accaatgctg 120960
aatttgactg cccctggccc tgctgtgcag cactttcaga cccctaaatg atgtgaccca 121020
ttctggggcc agctcagtgt tttacagtga tgaagatgat gttgatgatg atgatgatga 121080
tgtcaacatc catcattctg aggtgtgtgt ctatctctgg cctcgttata cctgcagaac 121140
attcatgtgg tagccaatac ctgggaaaat aatcccactt gccaggcagc aaaccgtggc 121200
cctccgttag gggatggca tctcacactc atgaagtgtt gagtgtatgg gtccctggcc 121260
agctttggca actgtgaggc aggcattatg cttccatttt accactgaca gactgaggcc 121320
ggtgatgggg agcacctggg cctgttggct tgtctgtggt aggccaggct ggctacataa 121380
tatgtggggc tcagtgcaaa atgaaatatg ggctctcttg tgtaaaaatt aggaagaatt 121440
tctagatgct gacagcagag ctttaaacca agctcgggcc cctctgagcc aggagccctg 121500
tgtgcctgca caggctgtat gtccaaggag ctggtcctag ggcaggacta aggttcaggc 121560
ttcgtattga cttccatgac caagcatcct gggtccaggc tgtgttttgt gtgtctgcag 121620
agctctggtc tatctgctga taagtccctg tggtgctcaa aggtacctgg cttccttgga 121680
gaaaagtttc tctctcccct gcccttcccc agcctcaaaa tacacaaatg ctcagatgaa 121740
tacttaagaa tccaactgga ccagcttctg tctgtgactt gatgacttaa tgtgattttg 121800
ggggatgcaa cattgggggt gatgctttag catgccttct ctctcccact caggaacatg 121860
ggcaaatgcc atgggctggg agctctcacc tgctcttttg tgctgagctt cactatctct 121920
tctgggcaca gattggttac agggatatag accacctgtg tggtgccctg gggttatgt 121980
ccacggggag cctcacatga agaagagaat ttcctttggg atgtgtattt cttgtggtct 122040
tagcatgctg gcaattctta tttttggcat catttaatcc ttacaacaac tttgtaaggt 122100
tttcacatcc attggtcatt tctgagttga gcacctcaac tcaggtgaat gtgtttgtat 122160
gaatgtttgt tgagcaccta ctatgtgcag ggcagtgtag taaaaaattg aggacctgtg 122220

ggtttttttt tgagtatata taattgtgtt cttctgtcat tttacatgac tttcacctca 122280
tttcattgct gtagcaacac tgtgaggttg ttgctgtcac ccccatttct caggcgagga 122340
aatagaggtt gagtcaatct cccatcttgg atttacctga catcacagtc tgagtctttt 122400
gtggcatgtg caagagtgca ctggctttcg gggcctgagc tggctggcct ggattgaatc 122460
ctggctttgc catttgcagc tgtggtctga ggcaagttat ttaacttctc ttgacctcgg 122520
tttccccatc tgtaaaatgg ggctatacta gttgctctgt gtaggaaggg tctgaggttt 122580
cagtaagata cattttgcac aaaccccaca cacaccaagt atccatgaag cctggttatc 122640
cttataacct cagagcggtt tggagagggt taggaacttg cagatggtca cataatcggt 122700
gtatggcaga tcttgggcag agctggactg gtcttcctaa tgagagctcc tttcccccact 122760
ctgctttcat ggacagaccc taatgaccac ctgggttatg tttttgtccc tgtcaattcc 122820
tttgtccttg ctgataacct aagggaacct tgagagccag aacgagacag gaagaaaatg 122880
cctggctgct gcttgcgggg ttaattaaac cattcctggg gtttcatttg ctctctagtt 122940
agcaccaaaa taaatcctcc aaatggcttt gagccttcca gttgacattg cattttgaat 123000
tctgacagca ccagcatttt acatcttcct gaggatgata aatgtttaga tagcaccgaa 123060
gataatggta aaaggagaaa ctgggggtag ttcaggctgt ggctgagcag attgtaactc 123120
tttggtgtga cgaatgggtg agaagttgct taaaggacac atctgatgtc ccctgctcct 123180
aggggttgag agggggatgg ggggtggact ctgttttgcc atccggagaa tccattgcct 123240
tcccgatgct tctggggtct catgcttgag tcttgtttcc atgcgttctg tcagatgtta 123300
aggtcttgtg cataatggtt gaggtgtcaa tgtgcaggcg ttaatgtcac cattcttatg 123360
taattgttac cctttattga gtgcctacta tgtgccagga tttaaatatt tctctcagca 123420
acccaaagat gtaaatgtca tatcaccttc attttacaga tgtgaaaaca ggctcggaga 123480
ggctgagaca cccagccatt aaggactcag ctgggattcc cagccttccc tggcactctc 123540
tctttttttt tttttttttt ctgttttgag acagagtctt gctctgtcag ccaggctgga 123600
gtgcagtggc acaatatcag ctcacttcaa cctccctctc ccgggctcaa gcaattctcc 123660
tgtctcagcc tcccgagtag ctggatttgc aggcatgtgc caccgcaccc ggctaatttt 123720
tatttttaat tttttttggt agagacgggg tttcaccatg ttggccaggc tagtcttgaa 123780
ctcctgacct caggtaatct gccagcctcg gcctcccaaa gtgctgggat tacaggtgtg 123840
agccaccatg cccggccttc cctggcactc ttgagcatgt ttccagaccg cctgcttcct 123900
accccagaca tgtcaaaatg cggtgctgta aatgacaggg ctctggtccc agatggacag 123960
cccgggccct ttgaagacac tctggccttc aggagtctgg atctttatgc ctgatggaac 124020
tgacacgggc ctgctatgtt tacagtgcct gttctcctgc agggtctccg gaagtcatca 124080
gccgtgctgg ccagcactcc ggaggatgac aggaaattct gctgcagctc tgtgggctgg 124140
ctggggtgca tgctggacag ggagcgggcg gacagggctg gtagggagga aaacaccact 124200
gaaatagttc agaccgtatt gtcagtgaat tcctggatta gtatgcagtg ctgccaatga 124260
tcagatttgg gtcaggaagc ccagatggca gaaagattgt gttaaatctt tatatttttc 124320
cacgaaatcc tcaccgtttc agaccctgtt ttctaagcta tcaccctaat aattgtggat 124380
tttggatttc caaggcacgt tttatagtgg ttgtaaggag gggatggttg ggttggggat 124440
tgggatggga ggggggtagc tgaagaaata agaaagtgct gcagaaagga gcacattcca 124500
cacccagctt cttcctgatt gtgtttccct ccaagcagcg aacaagaggc tgattcaagt 124560
ggctcagtgg cctccctgcc tggagttggg ttcctatgcc ttccagtgac taaggaggaa 124620
attccatttc agttaatctg tggccgcgca tcacccatgg cattctctat cttaatttta 124680
tggctgcctc atctttttgg aattcgcctt ctttttgtgt gttgggtgat aagtcaagga 124740
gacatatggc acagctccac aaaatacaac cacattttag tattccgtac acagtccttt 124800
ctgggcatcc caggctttgt attttcacgt tggtcggccg tgaggagggt ggatcttcat 124860
tatctgtgca tgcatgtgca tcccttcatc ccttcctctt cagtttcttt tctttttttt 124920
tctttttttct ttctttcttt tttttttttga gatggaatct tgctctgtca cccaggctgg 124980
agtacagtgg cgtgatattg actcgctgca acctccgcct cctgggttca agcgattctc 125040
ctgcctcagc ctcctgagta gctgggatta cagggttgtg ccaccacgcc cggctaattt 125100
ttgtattttt agtaaagacg gggtttcacc atattgacca ggctggtctc aaactcctga 125160
ccttgtgatc cgcccacctc agcctcccaa agtgttggga ttacaggcgt gagccaccac 125220
atccagcctc ctgttcagtt tcttaaccct tgatgggact cccggctaaa cacccccatt 125280
gtctgggacc ttgagattag atgcagtagc actgctggtt tcatggtgga ctttgagtcg 125340
gatggaggaa ctccttggag tgcgggagga agggctgcgg aaggtgtctg aactatcgta 125400
gcagtagggt ccctgccaca gggaggtgag gcagccatca tcaccagtcc taagaggtac 125460
ggtcaccctc cgcctctgtc tccctctcca gtccccatcc aatttcagct ttgaacccag 125520
ctgccgggtc ttcaaagcct gttctgactt acccagcaga gttggttgct ctgtcctctt 125580
agcttataga cgctgcccgt tttttcccca cgtggcccct gccatttagg tccatgtctc 125640
tcgtgatcct gtgtgtattt ccagtggaag gacaaggttt ggcccactgt atatttgaag 125700
gccctcaata tggcgttagt tcgtatttac tgatggatga agaactctga ggagatctgg 125760
ttgaaagaga cttttaaaaa ttaaagggaa atgggtcacg caggccgcgt ttttgtcctt 125820
gcctattggc tacttttgct attcagatca tctgtgaatg taagtgactg tctgctgtgt 125880
tcgcagtgtg gttactggga aaacagagct ctctctcccc aaatcaggtg aaactacagg 125940
aaaacagtag gcaagagtct tcaaagtgat gcatctcgag agagagaggg cctgggtctg 126000
aatcatggct ttgtgcctta ccagcctgtg attctagtca ctttaccttc tgagccttgt 126060
ttttcccagc tgcaaaatga gtgtgataat agcgctcgct tcaggggatt gtttcaagga 126120
tcagttgaaa tagtgcacac cgaatgtttg gcatatgtgc ggagctcttt gggggtgact 126180
catgggcaac tgttttttcat ttaagaactg agtggattgg acctttaaga actgctttgg 126240
tcccactgtc atgggaggtt agtgctgtct ggagcaggca tggggatgtg gtggtacagg 126300

gtagggtgta ggggtcccca gaattggggg cctccgtgtt ggaaagggac agtgggcaga 126360
gtccatgcag gcgctctggg gctgacaggg acctgggact tctttctgag aggcagtgac 126420
cagccaactg gagtggaggg ttgtggggaa aatgtttggc caaaggattt aggaaccaga 126480
gagaagcagg cacatgatgc tccttttctt ggtggggtgt tcatcaggcc tgtagctgcc 126540
acagtcatca ctgtggctga gattcatacc tgtagcacca gagtgggagg caatttttcc 126600
aacatggggc agaggctggg tgtgtgggga ttggttgcaa ggtgctggga gtggggtgca 126660
gggaagggcc tctgcacacc acgtccgtcc tcatcctgct atgaggtcag ctctgtacat 126720
gtgctggaag agcaattagc ggggtgccta agcagtgtcc atcatggacg tgggtctctg 126780
ctggccacgc aaggggaaga tgggactggt gggaagattg gggagatgga agcgggggca 126840
gttgtgtgcg aaaggtacag ggtcgtggaa acatgcacaa gaaagtgaca ttttagaagg 126900
tggtttttgt tccataataa atttattagc tttggaatct gactctattg ccttggcttc 126960
tccaaacttt ggtgtcctca tctgcaaact ggggatcaca atctcagttt tgcagggaat 127020
gtgaaatcct gacctgaaat gatcacatgt gtaggaagct gacaaagaat gctaattttc 127080
ttccttcttt tctttatggc ctttccttgg taaatctctg tgtgattgat aactcatgaa 127140
gaacctacca gtcaaatcag ttaaattaaa tgacttccta atgataataa ttagattagt 127200
aactaataat catagctgac atttatctag tgcttattaa cttctgggca gtgcgaattt 127260
ggtctcatct gttaaataat ttaattcctg taataatcct aggaggatgg tgctataatt 127320
agccccattt tacaaacaag aaaactggag gatcagagag gctaagtata atgtctaatg 127380
tcacactgct gaaagtggct tgctggtgtt ccagccccaa cagtctggct ccagagtttg 127440
tgctattaac cattatatca gtctgctgtg tgtgtgtgtg tttgtgtgtg tgtgtgtgtg 127500
tgagagagag agagagagag attgtgcatt gtacggtgaa gagcaataga aatcattata 127560
aggcattcct gtgctacagt cttggtgggt caggcttact tatagaagct gtgagaagga 127620
tggggctatg ggctttaact cagttctgac aatctgtcac tcccagaacc tgaggcaggc 127680
tggacctata gacttccacc tcctcttcct cctgccctga accgtccagg gactcagagg 127740
aggtcagaca ggtgggactc ccagaatagt gacctcctct gccacttgca gcctatgtcg 127800
ggctcctctt atagagccac ccccttgctt atgctgtagt gttcctttgt tctggccata 127860
gttttcctgg gcctgtgtca gttgagtgcg tccagatgca gctgggttga gactcaaagc 127920
cctgaagata ccgcaagggg agtgctcctg tgtgcagttg ctctgacctg cacagcaggt 127980
cggagtggcc gccggtcccc ggctgccagc tgtttacctg gccggccttg caaaggctct 128040
tcagggccag gccccagccg gtgaatcact gaaactgatc cccgtggcca ctgtgaagta 128100
cgggactgga ggtctccgga ggagatggca gctctaataa aaacaggctg ccaaaccgca 128160
gaaactgtat aagcctctgt ttctcatcct cgctaccacc cctgcgtagt ccctgtttta 128220
cagttgagga aaccgtggcc cagggaaggt cgggaacttg tccaaaggca cagtcataga 128280
actagggctt acaccagctg tgtctaatag catgcgtgcc ccctctgctg tgcttttatt 128340
tgcttcttgg agtgctgttt agagaaattg atggttcttt tgttgtgact aggggtccgc 128400
actatgggtg acctccctgc tccctttggc ctcaggccct tatcttaggc gatggccaaa 128460
agaacagctt tacattcagt aaactgtttt aactgaacct caccacaggt ttacgggtgg 128520
tgctcgtttc ataatggata aagaatgggt gtggctcagt gtggggaagt tggtggatgg 128580
ggagagggca gcagagtaac cagcttttag tggagacctg gatttgagtg ccctgagtta 128640
ggtgagcctt tgtacctgtt tcttcacttc ttggagcaga acattagttt ctccagatcc 128700
cacatgtgat aaagttctgt gatcctgcag tttgaacatc ctggcattca gctcagccca 128760
ctgtgcagaa atctatacta aacatgtaga cagtttagga agagggattg tttattgtgt 128820
tgaatgttat agaataatag ctacttaaat tttggagcac ttgccattca tcttccaagt 128880
gatttctacg tgttgactca tgtaacacag gcaaccaccc tataataagt actatgttta 128940
cacacccact ctacagatga ggaaactgag gctcagagga attaagtaat ctgccaaaga 129000
tcccttggca agtaagtggc taagggggga ttggaaccca gggagagagc ataatcctag 129060
gaggagattc tgccaaggta cagccctcca gcatggaagg gcagcagcct cctgtgagct 129120
gactcacttg agttattcca caccatccca gtggagaagt tggattatcc ctattgtgat 129180
agaggaagaa aggtgaagga atttgccaga aattataaat ggtgacattc gcattgaaca 129240
catgtccact gaccccaaat actgggtcat tttatatctt tccctccctt cttttttttt 129300
tttttagact agtctcactc tgtcacccag gctggagtgc catggcacga tcttggctca 129360
ctgcaacctc cacctcccgg gttcaagcaa ttcttctgcc tcagccttcc aagtagctgg 129420
gattacagac acctgccacc aggccagacg aatttttttgg atttttagta gaaatggggt 129480
ttcaccatgt tggccaggct ggtcttgaac tcctgacctc aggtaatcca cctgccttca 129540
cctcccaaag tgttgggatt acaggcgtga gccacgccca cccacccccct tcacacccca 129600
ccttgttttt ttctttttag tagagatggg gttttaccgt gttggccagg ctggtcttga 129660
actcctgatc tcaagtgatc cgcttgcctc ggcctcccaa agtgctggga ttactggtgt 129720
gagccaccac gcttggcctc ttccctccct tcttgctaac tttgaagctc agctccttgt 129780
ccaagagcct ttctgtccag gtgttttcat ggtgcctgtg ccttctgctg gtttggagtg 129840
agcggtttcc acagcatcct cccatcagcc atgcattcct cagggtcagg cgcaacatgc 129900
tcttcatatc tgctccctgg cacttggcct tcaaaccagg aattcactgc acctagcctg 129960
atccattgac ctgggcccag tataggccct cccatgttgg gaggccatgg ggtgttaagg 130020
ttaatgctgg gccacacatg actgtgatga gtccctttct ctctgctgaa gaacgtgttt 130080
ccatgggggga caggggcagt taagcctggg ttctgttggg cgctgctgct tgggtgggtc 130140
cagtctgccc catgtggggt ctgcattcta tgccaagtgt tggcaggttc tgccaggtag 130200
agctcttcag tatggaagca cttccctgtg ctcagcctct ccccgactgt gcacatttcc 130260
ttctcttctg ctggggaaat ggaaaacagc tgaccactga ttccttgaaa tcacctactc 130320
agacactcca gggtctgtgt gggattaagt cgtccgcagt ctttccctcc cttaatctct 130380

tctaaccttc ctcgaaagtg ccgtttccca ccccatttta atcatgccct ttgctctcgc 130440
ctggacatct ccaggggctc cagtgcctct ctaagtgaag ggtttggggc cacacacatg 130500
gctgtctgtt ctctccaggc ccacatgcag aatccagcag ccccctggcc tcacacctgc 130560
ttttcagtag tggctgggac tgccagatga ataggcttga ggcagcatac gagtttgcta 130620
tacacttgct caaaacatta gtggactcaa aaccaaaaag acaacccagt ttcagaatgg 130680
gcagaagact tgaacacaca tttctccaga gaagttatat aaatggccaa taaacacatg 130740
aaaagatgct cagtagccat ggttgttagg gagatgcaaa ttaaaaccat gagataccccc 130800
ttcataccta ctaggatggc tgtaattaaa caccaccacc accaacagca aaaaaaaaaa 130860
aaaaaaaaaa aaggcagaaa atagcaagtg ttggtgagga tgtagagaaa ttggaaccct 130920
tgtacattgc tggtaggaat gtaagatgat acagtctctg tggaagacag tttggtggtt 130980
cctcaaaaag tcaagtatag aattacatat gacccagcaa ttccactcct aggtgtatat 131040
ccaagagagt tgaaggcgta tgtccacaaa aagcttttac aggcatgtgc actactcaca 131100
acagccagaa ggtgggaaca acccagatgt ccaatgtctg tccgcggaag aatagcaaac 131160
agtattcaac tgtaaaatgg aatgagacat tgatacatgt tacaacatgg atgaacctca 131220
aaaatgctgt tctaggtgac agaagctagg cacaaaaagt cacatattgt ctgattccat 131280
ttttatgaat tttcaagaat aagcaaaatt atatagacag aaagcagatt aatggttgct 131340
gggggccatg gggagtagct gcttaatggg tacaaagttg ttgttggttt ttttttttt 131400
ttttttgaga ccgagtttcc ctcttgtttc ccaggctgga gtgctggagt gcagtggtgc 131460
aatctcagct cactgcaacc tccacctcct gtgttcaagt gattctcctg cctcagcctc 131520
ctgagcggtt agggttacag tcatgtgcca tcacacccgg ctaattttgt atttttggta 131580
gagatggggt ttcaccatgt tggccaggct ggtctcaaac tcctgacttc aggtgatcca 131640
cccacctcgg cctcccaaag tgttgggatt acaggtgtga gccaccacac ctggccaatg 131700
agtacaaagt tttatttgag ggtgatgaaa atgttttgga accagataga catggtgatt 131760
ataccacatt gtgaatgtac tagacaaacg tcactgattt tgtacacttt aaaatggtta 131820
atattacttt atgcagattt tacctccatt aaaacagnnn nnnnnnnnnn nnnnnnnnnn 131880
nnnnnnnnnn nnnnnnnnnn nnnnnnnnnn nnnnnnnnnn nnnnnnnnnn nnnnnnnnnn 131940
nnnnnnnnnn nnnnnnnnnn nnnnnnnnnn nnnnnnnnnn nnnnnnnnnn nnnnnnnnnn 132000
nnnnnnnnnc ctgtaatccc agcactttgg gtaggccgag gcgggcggca gtatcacctg 132060
aagtcgggag tttgagacca gcctgaccaa catggtagaa atcctttccc tactaaaaat 132120
acgaaattag ccaggcgtgg tggcacgtgc ctgtaatccc agctacttgg gaggctgagg 132180
caggagaatt gcttgaatct gggaggcaga ggttgtggtg agccgagatt gcatcattgc 132240
atttcagcct gggcaacaag agcgaaactc catctcaaaa aaaaaaaaaa aaaaaaaaaa 132300
gaggatatgt ggacactatg tctggcactt aaactggtgg attctataga gatgggatgg 132360
gttcattagc cccattctac agatgagaaa gtagaggctc cattagagca cacagtgtag 132420
aagtggaacc aggattggtc tgactctaga atgctgcttt tgatcacttt tctatgttcc 132480
tcttcctcta cagtggacag gttttgtttt gtttcgcttt gcttcagatt cttggctcat 132540
gccaagtgct gtgtgccatg ggtacatcac ctgaccctga agagcccaga tctatgtctt 132600
ttcatgcaac cttgccctgt gctttccaca tattcacatc cttcacagtc aatcccaccc 132660
tggccactag gccaataggg actccccaca tcccacttac agggggcaa atatttatag 132720
ccaaaagaaa acagaagatg tgggtttgag tcacagttcc actatgtggt catggatgag 132780
tgccttcaac cctcagtacc tcagtttccc taaatgtgca gtgggagagc atgagaacac 132840
ttatctgatg tcttccctgg ggttttgtga ggattaaaca caccatgtag gggcaagtgc 132900
tttgagaatg gaaaagcact gtgcagacat gagtgatgtg catgatggtt attgttagat 132960
gttctctcct ggaatcaagt tcaagcttct tgcatactca gcagagaaca gacccaaact 133020
cccgaatcta gtgctgtggc atgtggttgg tgctcaataa tgatttgttg tgtgaacaag 133080
tgcatgaatg agtagtaggt caatgacctc aatgtgggcc tggtcaggat ttagatttct 133140
tgtctttgtg tgtgctcagc ccaggactga atgcacagca ggctgtcagg aagtagtcat 133200
ggattgatgt gtccgtattc gccataacca agcaggagat agatgatttc gttctggtct 133260
ctggattttg gctgcatttt ctctcatagt aggaattatt cccggaagat ccaaatgctc 133320
agctttgtcc tcctctcatc ctggtgaggc gtgtgatcca ggtgagaagc agggagaact 133380
ggccttcctg gtgggaagtt gtgggccatc accttaagct tggcttgtag tggtgggaga 133440
cctgtgttcc ttccttcctg agccccactt cctcttgatt ttattgtatt tttagcagca 133500
ctgagctgaa gctttctggg ttgcatgtta gaggcagcac ttcttgcaca agctagggga 133560
gcgtgagtat cgtttcggtt ctgatagcag gatggattgt tatgtatcct ttctgatgga 133620
ggctaccaaa agctttagat gttaggatgg attatctcac cttgcacaag acagtgagtg 133680
ctagagttta agttttctcc tctgcaaaat aaggagtggc agtgagactt agagcatctc 133740
tcaagcccac tttggcccaa aagttccatg actgtggcag tctagagtaa atgtgtttga 133800
tagcccctag ttacaactct cttctgtctc ttcccctttc tgcctgcgct accatctaat 133860
ccatccacgc aaccacccat ccacccaccc acatttccta gccagctctc tggaccaggc 133920
cttgtgttaa gtcctgaaga cacagaaaag gctaaaacag cccctggtct taaggactca 133980
cagatcatga ggaggcgata gatactggaa tagaaggcaa aagtcaaatg tcatgagtgc 134040
tgggattcag aaaggtgcag gctgctctgg tagcaataaa ggaaaccagc tctcccttga 134100
gatccctttc atgagggtta tagcctggat cctgtgatga cccaggacag actggtcttg 134160
atccagggga accatatcag tgacctcaag aggccccttt tagccacagt aatctatgat 134220
gtggagttct caattgctgg ctttgctgga gaaagagaga aggaagaaat agaggggccg 134280
gtcagccctc ctgtacattt agtttgcggt ctcaaacctc tgtttcctgg agctgcaggc 134340
tgtatgtgtg ggaggtgcac agcagtggct tggccgcccc aacgcgcctg cagcccgctc 134400
tggggacagc cgtcttttgg cagagtctgc ctctgcagtg gtggcagctg gcccaggcag 134460

acctcctctc tgtccggtta gtcagcgtct ggtggccaag ggcagagtgg agcctgtgtt 134520
tccgacaggc cagctgcccc tcagagtgag gcagcgccgt gattctgagg gtgatgctgg 134580
cgttcgcccc tcaaccctgt cccccacaag gagattacgg acaaggcagc atttctaata 134640
agcagcctct gcttgccccg tagcagatga aagactgcgt gtaagagtgg gtaattttgg 134700
gttttcctga tgaaagtagt tgacatttgt gctgagacct catgcgctct gcagcgctgc 134760
ctttcatctc tgctctcaaa gccctgctca tctctgccat gactcggggg cggcctgcgc 134820
caccggaatt cagttgggcc agtggtgaaa tatagaggaa atggtggaat gacccctgaa 134880
ggcagtggat gggggagtgg agacaagtcc tgactttcct ggtggagggg atgttcctgg 134940
gggtgtggaa ctgtgtatcg gcccatgcag cacagccagg gaaagagtgt ggatcttgga 135000
gcttggtatg gctggctaga ttggaaaggc agctcctcca cggacttgct cggtgaatgg 135060
agggcatcct ttctcttccc tgatctgtgt cctcatttgt cagtgggaat aatagaataa 135120
cattaccacc aacgacgatg gctttcacaa ctggaaccac taggccctca ggaatgttta 135180
ctggcttcta gtctcattga taaactgaag ccattgtgcc atagctctag agaactggca 135240
ctaggggctt gcatgattca tctgagttcc ccagacaatg ctggcacata gtaggccctc 135300
cataaagtaa gaagggacac tttgctcctg acatccatac agctcctcta gctttgcaga 135360
gaaagcccta gagagaaaga atgtgaagga acaaagggtc aggttaggtt gtgcaacctt 135420
tctgatatgc atttgtaatg cagtgagcaa atgcacaagc cttgagtcgc tgcattcttt 135480
tctaaccacc caagagataa gttgaacccc cactcgtagc aacctggcca cttgtctgtt 135540
tagtcagccc tggaatacct ccactgacgg ggcactcact accactgaaa gatggtgtgc 135600
agaattctaa tgttcttcct ctcgcggggt cgtgggggct ttcttccagt tctctgggtt 135660
ctgatcgttg gggctgtcct gacacccttc ttcagatgac cgctgtcagg ttggtgaagg 135720
cagtgcgcgt tgggccttcc ctccctagag atgtgattgc acgtcgggta ccaggagcct 135780
ggccagccct cagcttctgc taaccaggga gcagtgaagc ccggtttact tttgttggct 135840
tgtttatttt ctaatgtcac ctgcctctgc actttaagta gtatttgggc tgctatggag 135900
tgtgcacatt ctaacacagc accttgtcag ctggctgaga agctgaaaat tctcggcatt 135960
cctccttgat gtcaattact tcaaggagca ggaaggtgac ttcttttaac aagtggaaag 136020
gattcaggta acaaagccat ttcctcttct taaccctcta ctgtggaaag ttattgactt 136080
agcgacacag tctgtttatc tgggcttcag gagcagggct ttggagactg aaggcttctc 136140
cccacttttg tgcaatgtta tatatttttt ctcactcttc atccagtctg agtggggtct 136200
gccccctccc ctgagcagtc accatcctgc tacccactcc ggctctattg gcgactgtgg 136260
catagggtgg tggaaaggac gctggactga gagcccagag atcagatgtc tgaccttccc 136320
tctgcctgga agagctgcct ggcctctgat cagggtccct accttcgttt tctggcctca 136380
ctcactgtgg cccaccatcc agcagtgcag gtgtcctggg gactcgggga aggtcagaag 136440
tggaaggaaa gcctccaagc cctcgcatgc aggcccacca ttccactgtc aggttttctt 136500
ccctttcctt ctgctctaca ttttgtgcca gcagtttggg ggtctcctga atcttatccc 136560
ttcgcagcgg taggcaggtg caatttctgg ccagtcactg aacatgaaga agcatctata 136620
ggcggagtta aatccaaaac acttggcctg acacacctgg ccttgaagtc tcccccagcc 136680
tctcctcttg tccccaacag tggttcatgt tctagccaca cagaaattga gggggtcctg 136740
tgttatctgt gttcctgcag acagagcttg cctaggacag caaaagtcat cctaacataa 136800
aaatatgcat gaataaactc actgccaaca ttcagcccac ccacatccaa tgataatcag 136860
tcatttactg atggccagac gttgtaaaca tctgtcagtc cactgcaaga acagcagaaa 136920
ctcgtagctg acctccattt ggcctactca ctgaattaag tgatgttact cattcgcttt 136980
gtaaaagcaa actgagtgag gccccaggct cattgaagac tctgtgggtt acccagaagc 137040
ccaactttcc agattcaata ttttttctat ttcttagctg tgttaccttg ggcaagttaa 137100
ttaacctctc tgtgcctgca tgttgcatct gtaaatgaga ctaatactag tacccacctt 137160
ctaaagtgat tatgagcatt aaatgaatta gtacgtttaa aggcttagaa cagtgtttta 137220
tgatacgata aacactcaat aaatgttagc tattgatatt ggtgtgccca gaaggcttgt 137280
tactactagt tgatttatgt gcttgccaaa agttgttgtg ttggtaatta agtacgacat 137340
aaactaatga aaattgagtt tataaagagt tgttttatga aaagtttagt actttggata 137400
gaatggataa tattgatttg ctaaaaaaaa attgtcacca aataacttaa aggcaaaaca 137460
gctgtaaaaa attgtaaaac catagtttgt attccatatg ctttacaggt gattaacttt 137520
ggcactgctt taaagaaact gaaaccagga attgtagatg ttgacttgtg agtgtgtatt 137580
ttgcatggaa gatgatttgc atctctagtc agcgtgccaa aaaaagatcc taccctttga 137640
ttggttaata aatatgcaac tataataaaa tatttaagtg ttggtcctta atgattgcct 137700
acattttcaa tgaaatgaca gtggcagaac tgaggaacag gggtttttct ctccaaggca 137760
gatcagctgc cagaaattgc acgttttgta ccagctgcag ggtttcctgg aatcagcaaa 137820
ggcctgtgag aggctgcatg aaccacacac gcttactgac acctcctgtg tgccgagcgc 137880
tgagtagtgc tgaggatata gcaattgggg agcactagca gaatcagggt tgttaaccgt 137940
ggagaagaga aaggaaagat gatgtctctc tatgcaaatc aagggaaagt gatttaaaaa 138000
tgttaaaaaa aaaaagttaa gagaggtctt cgaaaggtgt tttgcaaaat gattccttgg 138060
attaatgatg aaggagtgga gtgaaggaag tttatatttt gtgagaaaac gtgtgctaag 138120
gaaggcctcg acattagatt cattctgtac acaactcagc aaattagatc atagtttaaa 138180
atgataattt caggggccgg gcacagtggc tcctgcctgt aatcccaaca ctttgggagg 138240
ccgaggaggg aggatcattt gaggccagga gtttgagacc agtctggcca acatatcgaa 138300
accccatctc tattaaaaaa tacagaaaat tagctgggct ttgtggtgca tgcctatctt 138360
cctgtctact tgggaggctg tgcatgagaa tcannnnnnn nnnnnnnnnn nnnnnnnnnn 138420
nnnnnnnnnn nnnnnnnnnn nnnnnnnnnn nnnnnnnnnn nnnnnnnnnn nnnnnnnnnn 138480
nnnnnnnnnn nnnnnnnnnn nnnnnnnnnn nnnnnnnnnn nnnnnnnnnn nnnnnnnnnn 138540

nnnnnnnnnn nnnnnnnnnn nnnnnnnnnn nnnnnnnnnn nnnnnnnnnn nnnnnnnnnn 138600
nnnnnnnnnn nnnnnnnnnn nnnnnactgc aacctctgcc tcctgggttc aagcaattta 138660
cctgcctctg cctcccgagt agctaggact acaagcatgt gccaccacgc ccggttaatt 138720
tttgtatttt tagtagagac ggtgtttcac catattggtc agactggtct tgaactcttg 138780
acctcgtgat ctgcccgcct tggcctccca aaatgcaggg attacaggtg tgagccactg 138840
cacctggcca gttcaggttt caaagtataa gatcaatctt attttatgta tagtttatgt 138900
gtaattataa attgtatact aataaagtca tttttataat tctttatttc tttttccaag 138960
atacccaccc cccccccacc aaggagggct aggacaagtg acaaaagtca ccccagatca 139020
cagccaaatg ggtagagcta ggaatccgaa gtaggtgatg aagcttgatt tttcaagatg 139080
ctgcaacccc tctcccccat atgctacatt aaatatctgg acctatttta agtgggttct 139140
tttctggggt aatgaggttc tcttctgcct cagttttcca tggctgcaga gctcctgctg 139200
ttccttctgc ctagttcact cttctttgcc ttgactgcac ctctctgagc taccttccta 139260
cctccatcct tgcattcatt aggggtttct ttatgtgtcc agctcactgt gggacccagg 139320
actccttgaa gagggagatc ttgctcatcc atctgtgtcc cccactctac tgccaacaca 139380
gtggtaggaa gggaaggcgg caggcttagg cgaggtcctg caggctggac aggatctgtc 139440
accattactt agcccaggcc catctcttta cttccagacc aactggtgtc accataatgc 139500
cttctggcaa tttgaagatt ctggcaacct cttaccggat gcagctggca ggataatccc 139560
tgtgcattag ccactttcag aggtatctgg gcccccaaaa gagtcacggg agtcccagac 139620
atgaaggagc attagtggcc atctccccac cttgctgtat cagcaaaacc gctaagccct 139680
gacccatatt ttgggcagtg ctcaccctat ctcattttat cttactgtat atctcgtttg 139740
tatcatttta ttaggtatct tattttatct cattatatgg taatctcatt tgctgggtag 139800
accagtgagg agggctggga caagtgactg gaattgccca aggtcacaga caagtgaggt 139860
ttagagatag gactccgaag caggtggcgg agtcaccgtt gagttcatca tagcgatgct 139920
ggctgcctca aagtactgtt ttagttaatg ctcacaagca actctgcgaa gcagacgggc 139980
cattcatttg tgcttcacag tgtgggaaac tgagtcagag caggttcatg tcacacagct 140040
gataaatgac aaagccagat gtgaagcctg tgagttggat tccaacattc atctttgatg 140100
aagcgttcag gttggggcct ggtgtgggac atgctcagag gaggacgagg aggatgattt 140160
gggggaggcc tcagaaggcg ttcctggtct gagtgtgctg ctctcactgc cttggtttgg 140220
gtccccgctt ctgagcagga tcttctattg gcagagggtt caatgcagtg ggtgccatga 140280
aggaagatgt ctccccaaca gcatcacagt ctcagggact gttcacatca cattaaaagc 140340
gctgagactc tggcttttgg tgaaaacggc tattttgtct tgttccacgg gagctgggaa 140400
gaggagacta aacacaggag cctcccccta acccaggggc acatggagaa ggaggaaggc 140460
agcccatgct ggggagcaaa agggagagaa ggcgagggcc agagccctgg gaagtgtcag 140520
atcctggtgt gccgcgcttc tcccaggttg tataatcagc tgtcacttca gcatttccct 140580
gcatcagcct tcccaagtac agattgaaca ttaaacaaac agtgacggct ttataaatat 140640
ttaactccca ctgtttagga attgctcggg gcaggagaca aaataagagt tcctggctgt 140700
ctgctccatg ccccctctgg catggtgttg gaggccgggg gtcagagatt tggggagtgg 140760
tgggcagcag ctattctctt cttcctttcc agggggatgt gtttggggac acatgtctgt 140820
gtgttcatgg agctgttagg gcctcttcta gaagccacca gtttgcgttt tcatacagtg 140880
gctgggatcc atcattgctg gtgttattcc ttcacatcag agcaaacttc ttcctttctt 140940
ttttatatat agcagcttta ttgagatata attcacatat aattcacctg aaactcaccc 141000
atttaaggta tacaacacaa tttttttttgt ttgtttgttt ctttgagaca gagtcttgct 141060
ctgtgaccca ggctggagtg cagtggtgcc atcttggctc actgcaacct ccgcctcccg 141120
ggttcaggct attctgcttc agcctcccga gtagctgaga ctacaggcac acaccaccat 141180
gtctggctaa cttttgtatt tttttttttt tttttttgga gacagagtct cactctgttg 141240
cccaggctgg agtgcagtgg ctcgatgtcg gctcactgta agctccgcct cccgtgttca 141300
tgccattctc ctgcctcagc ctcctgagta gctgggacta caggcgccca ccaccacgcc 141360
cgactaattt tttgtatttt tagtggagat ggggtttcgc cgtgttagcc aggatggtct 141420
tgatctcctg acctcgtgat ccgcccacct cggcctccca aagtgctggg attacaggtg 141480
tgaaccaccg caccagaccc caacttttgt atttttagta gagatggggt tttgccacgt 141540
tgcccagact ggtcttgaac tcctggcctt aagcgatcca cccacctcgg cctcccttag 141600
tgctgggatt ataggcagga gccacggtgc tcggccaaca caattgttct taatatattc 141660
atggagttgt gcaaccatca ccacaatcaa tttgagagca ttttgatcac cccaaaaaga 141720
aatcgttttt attagcagtc acctctttat tcccacaatt ctctgcgtgc taagaaattg 141780
ttgagctact tgtactacaa atatacctat cctggaatgg tcttttgtgc ctggcttctt 141840
tcacttggtg taatgttttc aaggtttatc tgtgttgtag catagatcag catgtcattt 141900
ctccttaggg ctgaatactg ttccctagca caagtatatt gcatttttaa aaatctactc 141960
atctgttgat gaacatttgg ggacagcaaa gctctagttg aaaaatcaag atgaaaaatc 142020
aaaaaaatat agttgaaaaa atcaaaaaaa tctagttgaa aaatccagca tcatgtgatt 142080
aaggccaaac tcaacttcac aaaagagagc ctctactatt ttgggaaaga tctagaaggt 142140
tatctgtaag catatggtgg atgttacaat gtgttcagtg gacacgccat tcagctttta 142200
ttcagcactt accatatggt gggcactatg gtaggattca ttaattaatt tattgaaaat 142260
aattattgag attatatagt cttgtgaaaa gatttatcag aggctgttct gtgaatttta 142320
gaggtttttt gtttttgttt tttttgaaa tggagtttta cttttgttgc ccaggctgga 142380
gtgcagtggt gccatctcgg ctcaccgcaa cctctgcctc ctgggttcaa gcgattctcc 142440
tgcctcagcc tcccaagtag ctgggactat aggcatacac caccatgcct ggctaatttt 142500
gtatttttag tagagatggg gtttcaccat gttggccggg ctgctctcga actcctgacc 142560
tcaggtgagc cacccacctc gggctcccaa agtgctggga ttacaggcat gagccaccgt 142620

gcccaggtga gttttagact tttttggggt gtaagaatgg gagcatgagt ttgtgttctc 142680
agctcgtttc atttgatttc agatgtcagc tctctttagg agagaggcgt ggagaaaagg 142740
tgggactctc tgcagtcctg ttcttgttct gcccacttcc ctccagtcat ggtctctggc 142800
cggctctctt ctccccagag tggttctttt cctgcccctg gtgtccctct aagacccaga 142860
tgtttccagc acccatttga gaggctgatt cagcccaggc agtgtctggc ctagggaagg 142920
tgactgcagg aaatgttaaa aaatgggggc tgttatcatg cctgagcatc gcacagtccc 142980
tccgttgtga ctaattgtag tggttgttgt tgaagggctg ccccaggtcc ccctccctgg 143040
accggtacac tcaccccagc tgccgagacg gcggctgcta atgtccactg ctgctaatgt 143100
ccacagcacc acttctctgg agattagcct tcctccaact ggagctgctt catccgggaa 143160
gttgctcacc ctgcaccagc cctggctgac tcaggggtgc aaagggctgc tcctccttgt 143220
ttccatgtgg ggtaaactcc atggtgcggt tcatgttcca gggcatcctg tggatggcct 143280
gagttggtcc ccagccgaga ccacctcctt cccttgcctg gctctgccac cttcccttcc 143340
tttctcctga ggttgctccc tgttaaatca cacagacaga cattccctcc tcaggatctg 143400
tccccgggtg cccaccctca gacactatcc ggcaacacgt tccctttcct gagtcccctc 143460
tgatgatgta tctgacttgg agatccttgg cctgctggct ctcttttcag ttctgttctg 143520
gagaatagaa gcttcctggg ctggcctgtt gagtgttttg tttggttggt tttgatttcc 143580
aagtcttttc aggctaatcg gacacgagga aaggtctgca ttgaatcaca gccttggcac 143640
tgttcccccc ttccttccct cctccgttca ttcccccgaa catccttttc tgtgccgaca 143700
atgacctcag cagaaagggg aaaaggacgt gggaaggctg catgggaaac tcatggaact 143760
tggtagcaaa cagctctggg ttcaagtccg ggccctgttg ctcaggagcc aggcactcag 143820
ttgtggtggc ttctctaacc gggaacttct tctatgaaaa tgggactatt cgtctctact 143880
ccttgcatta ttgtggggat cggaaataat ctgggtcaat gatgtggtgc agtgactagc 143940
atcgagggga caccctctcc acacaggctg gaaagacatc cagaaggagt cctgggctcc 144000
ctcaccacgc aggggctcag cctcgccaga tagttgtcat cacccatcca ggcccgtttc 144060
tggtctcatt gcttttgttc atgtactttc ctcctgggtt gctcttcctt ttctctctga 144120
cctgtcaaaa agccccattg taagcaacat ttaaatgcta cctcttccat gaagcccttt 144180
tggatgcctc ttagggggat gagttctttc catcctagca ctgcttgctg catggtccag 144240
gatctgtggg gcctgtcttc tttaccactt tgctcacttc atgaatgtgg gggtgtatga 144300
agagtgtgat gaaattagag gctatgttct ggagtcagac tgatcagtac tgggattctg 144360
gccatacccc ctccttagct gggtgacttc aggcaactga ctgtaccatc ctgaaagtca 144420
tctgtaagcc gtgctgaccc cccaaccaca ggacacttgt gcccgctgca ggtgctctgt 144480
cagcagtgac ttctcactac ctggcatcct ctgaattcac gagcagtttg aagaatgagc 144540
aaatactgag caaagctcat aggattaaat acacacagac ctgtgtaagt taacaccaag 144600
ctaagggtta aatactttac aaaaattctc agtgcggttg gtgggtgaga agggctgtcg 144660
tcccctctgg ctgccttgcc aagctggtag aggggccatc tcccactacc tactgacaaa 144720
gattctttgc ttgacccaat tacccaattt aatcagacac ttaagtcttt tcctaggccc 144780
atctgtgtac ttcctgtaaa atctagtttt agcaaaaaac tctgctgagt cagtttggta 144840
aggaccaccg gccgtcaata tcttccatat ctgatagata gggttcctca tcttctacca 144900
ccctccagat gatgtctggt caccctggcc tgcctttagc aagaatcttg ataggttgat 144960
ttaaccagaa tccccttac tcctgatgtt gcctgttagt aattttctat cctctgaccc 145020
cccaccctgc ttcttggctg tgaatttcca cttgctcagg ctgtattgag ttgagcccta 145080
tctttctcct acactgcaaa ttcccattgc cgtggtccct atacctatca ggatggtcct 145140
gaattgtctt ctttatcatc tgctctgctc tgccagagct taagcccagg cagctgagtt 145200
aaaagtgtga ccaaatggac catcttgtcc ctgaaaattg ggacctaagc tcgattccga 145260
gatttaatca tggcagagga gtttactctg ctaaccacca tcaccccacc ctatccccca 145320
gaggggtgcg gtgagtaagg gggtggcaac tggttgtttc caatctccca tctctctctg 145380
gccctcctct gtctctggcc tcccttctcc ctttgaaagg gacacagtgc tgctcacagc 145440
ctgtcctgct gcggtggctg cagagagggg ctgagaataa gcctgtgtcc gaggcagccc 145500
tccaaataca gcagggctgg gttgaccttt aaatagatgg gctctgggcc actcagttcc 145560
tggaagtttg aagaaagtca gcctttcaag tgatgcgact tccaacctca ggaaagatcg 145620
ctgtgtttgt aaagggtata atttactgtt gcttttgcac ttcatgatat gcggctgcac 145680
agccagtggg agccagcatg ccagaattag gaggggaaaa tcctcaagta ttttaatata 145740
cccctactta gaagcaagat aaacatatgt attgtacttt gggacagaag caaggggggt 145800
ttagcgggtg ggaaagggag agtcctggca ttgcatgaaa ggtcttttta agctgaactc 145860
tgagggtttt cagtttttca tttttctgtt ttttttttt ttttatttgt aagcaaagct 145920
ggcatgagca aatgatgcca ggatctactt ggctgtgtaa aagctgtagt ccctcctcag 145980
acaggcagac tgctagaacc ctccctaagc cataaactag tacaccgttt tgcctcgccc 146040
aattaaaaat gattgggtgt gggttgtcat cacagttgac ttagatgatt tactttcttc 146100
caggaaatct tcctcccaac tcttgctttg caattaggca aaagaaaaat aaatgttaat 146160
atgttctgtg ctcccaggga aaattttttt aaaaagaaaa taaaacactt tgggaggccg 146220
aggcaggcgg atcacgaggt caggagatcg agaccatcct ggctaacacg gtgaaacccc 146280
gtctctactg aaaatacaaa aaaaattagc cgggcatggt ggcgggcgcc tgtagtccca 146340
gctactcggg aggctgaggc aggagaatgg cgtgaacccg ggaggcggag cttgcagtga 146400
gccgagatcg caccactgga ctccagcctg ggtgacagag cgagactccg tctcaaaaaa 146460
aaaaaagaag tgtcaaggtc atgagtgtca gggaaagact gagaaatggt tccagactga 146520
aagagactaa agagatgtga gaattaaatg caaagcttga ttctggacaa ttgtcaaacc 146580
ttggatggct gggagcgtta gatgttagtt acgtatctgt gtcagcttcc tgatcttcct 146640
atagaacatg gtcctaaggg agaatgtctt tgctttagaa aaatacacat ggaagtactc 146700

tgtagtgata gggcttcagg tccactactc cctctcacat gctgtagggg aaaaggattt 146760
gctgtgtgtt cccaacacgt ttgtgattat ttcacaaata acaataatta aaaaaaacca 146820
acctctggtg gggcggtgcg gaattattat tattttttt tgggacagag tctcactctg 146880
ttgctcaggt tggagggcag tggcatgatc ttagctcact acaaccctca actcctgagt 146940
tcaagcgatt cttgtgcctc agcctcccaa ttagctggga ttacaggcat gcgccaccag 147000
gtctggctaa tttttttgca tttttagtag agacgaagtt ttgccatgtt ggccaggctg 147060
gtctcaaact cctgacctca ggtgatccaa ccacctcggc ctcccaaagt gctgagatta 147120
gaggcgtgag tcatcatgcc tggcagtaat cttttcagtg gagaataaaa taaacccggg 147180
tttgaattct gaccctttaa atttactcag ggtttgactt cagccaggga acataacctt 147240
tctgtgcctc agtttcctca tttttcaaaa caaagatggt gtgtgtgtgc atctccatct 147300
atgagttgtt gtgaatagga atgtaggggc cccagcaggg tgtgtaaagg taccagttcc 147360
tggggcgtca aaggtttttc tttccctttg taccccctca ctgacatgcc caggtctgtc 147420
tgtgtagagc agtgggtgga tttacagact ccgctgagaa ccccccttata gcatattttc 147480
aggttcgtga tgatatcatg ggctgtgacc ctgggagttt aattggaatg aatttcaagg 147540
ttttgttaaa cttggctaga atcttcctat atctccagca agatattgat ggggtttttg 147600
ttgttgctgt tgtttttgag acagggtctt gctctgtcac ccaggctgga gttcagtggt 147660
gccgtcactg cagcctcaac ctcccaggct caagggatcc tccccttcag cgtccctagt 147720
agctgggact acaggtgcgc tccaccacac ccagctagtt tttgaatttt tttgtagtga 147780
tggggtttca ctatgttgcc cacgctggtc tcaaactcct gggctcaagc catctgccta 147840
ccttagcctc ataaagtgct ggaattgcag gtgtgagcca ctgcacctgg ccaagaactt 147900
ggtgtttctt atttccaagc ttatctcctc tgttatttgc tcccagggag accccttttc 147960
catttatgtt tccagggtgc tgggggggaag tgattgccct catctctttt gcccagccac 148020
caaacccagt gcagactctg ctgaatgcag gccccatctc tgtattttac ttattccatt 148080
tcctgtcttg aaggtggggg cactatgttt atctctagga ccctgactgg ctcatcattg 148140
aaagcttatt ttgtgcaaag cattgttctg agcactgtgt gtgtatgatt ctacttcatc 148200
ctctgtaacc ctagcagtcc ggtcaaatag tatcatttta tagacaaggc aactgaggca 148260
cagagcagct cagcagcttg cccagatcac acagctggaa agtggtagag ctggaatctg 148320
gatgggtgct ggcagcctgg cctcagagtc ggtgctaacc actgagcggc actgccaccc 148380
aacggatggg agcctgcagg atgaggccat gggaattgat ttcactcagc gcgagccaag 148440
cagaactccc gtccagacct ctgtccacca ccctaataaa gccagtgttt acacaggctg 148500
gaggtgctca tcagatgttt ttgccaatcc ccaaatacgg ccagggccag aaagtgagag 148560
gggtgcctat tatgtaaggc cctgcggctg gtccccagac aatgccactt cctcgcttgg 148620
cgctgtgtga ctgcataaca acgggttact catttcctct ggaaaacttc acagggcctc 148680
tttgccccat ttcctcctcc caccccatcc tcaggcttcc tctgttgaat tttctccaga 148740
ttcctgggaa caccaccatg gagggcctgg cctttggcgg tggacagtct gtggctgggt 148800
gggaacgtca ggagtggggc ctgcctcttt gctgggaatg ccgtgcactc ccagcccact 148860
tgccccatgc cagcactcga ggcaggtgag gcccagccat gggtataagg tgctcccagg 148920
gcgggattag cctcaaggag gcaggggttc catgtttctg ctcctctccc attactaacg 148980
ggtgtctgct ttaggtttga tctccctccc ttctgcccct ctcctggtaa agggatgaat 149040
aatgaaggcc ttccttggga agtctgagat ggattacccc aaggaggctc gaacatggga 149100
ggacatgcga gaactattgt cacatgagtt ttctttctat cctgtctgca ggacttcgca 149160
ggtgagcagt aacggaaatc agccccgttt attcctccca gcacctgcct tatccaactc 149220
cccacgctgt ggctgagtcc cagcctgcta tggaagcatc actggactcc cattgaactc 149280
tgtgcagatt cgctgttcgt agacatggta cctgatggac accaagctac gtacagcttc 149340
aaggcccctt ctttctttgc taataaaaaa taaaaaataa aaacataata cacgttatat 149400
ttctatctta taatacacat ttcttatttt tatctcaata agctgataga tatatagtag 149460
atatacacat ctctgactgt acatacatac acaaacacac acacacgtac atgcatgtat 149520
atgcgtatct atctccaatg catgtggagt gttgtgccag gtcatcctca catttaattt 149580
ttctgttcta tttatcattc ttaaggattg tcaggcatta gggatttatc tatagcaatt 149640
tggatcttag cagttctttt catgggttat cttatttttt ccttccaaca accttggagg 149700
ctataaaagg gcatattatt gccctttttat agatgggaat taatttatta gcaaactcag 149760
tttcatgatt ttttttttct tttgagatgg agtttcgctc ttgttgccca agctggagtg 149820
caatggcacg atctcggctc actgcaacct ctgcctcccg ggttcaagcg attctcctgc 149880
ctcagccact ggagtagctg ggattacagg catgcgccac catatctggc taattttata 149940
tgtttagtag agatggggtt tctccatgtt ggtcaggctg gtctcgaact cccaacctca 150000
ggtgatccac ctgccttggc ctcccaaagt gctggggtta caggcgcgag ccattgtgcc 150060
tggccagtat catggatatt tttaaagtgc ttgttgtgtg ttgggcacta ctatgtcctg 150120
catttagaaa tagaactcat tcctattctc tgctttccca tggagagagg cacaaacctg 150180
cgggtatagg tgggtatagt ttcttaatgt gcacaccatc gtaaagtgga gaccctgtgc 150240
gtgagatgtg tgctgtgtac agggtgggca ggagagagac tggtgagggg atgatgccct 150300
tatgctggaa ggggcaagat gaagcctctc tgggtttggt gggaccttgg agacatctgc 150360
ctagtgtgtg gtcacccaga ggccaaggat gtgctgtttc tacttgcttg ctgctttctc 150420
tgggagtggc catttcttcc aaggcagtgt aagagcctgg gcataggagc caggcagaac 150480
tgtgtgggga tgttgcctcc atagctttct agatgagcaa ccttaggaaa gtttgaacag 150540
tttctctaca tcctgggttc ttcatccatg aaatgggaat tattgtttga gcctctaaag 150600
tgagacagtc aaatgagatg ggcatttgaa gccttcagca gggttccagc ctcacagcta 150660
actctcaact agctgtgatg gttacagagc tgtaggttgc tcataagtca acattgtagt 150720
tgctggagcc atcaactcag ctataggata caacactgag ccctttttcac tatcctttga 150780

tgggtctcct tttccctagt atgggccatc ggctaaggtg attgaaaaag ggcctttcat 150840
gttttcaaag gggcaaagaa acttcagtag ttggttgggc attttattga gtagaggtga 150900
ctccatgcag ttctttttgg gggaaggatg ctgcacattc cagggtttaa caaaaattta 150960
ttgaatgctt actttgcccc agatacctgt cttggtaaag gtgataaata agacagcctc 151020
atcctcatct tcatcaagcc accagtctgg tggtggtgac tggggtgggg actgctgagg 151080
cattgggccc cttgaagcct tccctctggg tgtcttcctc cccaggatgg tctgggctct 151140
ctcgggatgg gaggaagttg gtctgaaagt gcctcagtga tcgctgaaat aggtttgcca 151200
aagatagagg ggaaagagat ggccagcctt tgtttaggga ctcccagaat ggtcttaggt 151260
caggaaagag cgaatgggta catttctccc aatttctggg ctggagattc caaactgact 151320
ccttcatggc aggcttcctt atctctgcaa catttgttta catgttccct tcatagattt 151380
ctaacaggat gaagactttg ttccttgggg taagggtcta cctccaggtg tgtggggaaa 151440
agttgagaag ctgctaaatt cttggctttg acttatcaat ggtttttcgg agggcctgga 151500
gctgggctgg gactgatcaa atttggtaat tgcttaggca gcctccactt ttgttgctcc 151560
tggggccaga tggcgttggg ctagaaacaa tatcggggag ttctgtttgg ttttaactgc 151620
tgtacctggg tgctctgtta tccaaccagg ccctctccta agttctcttc tttttttttg 151680
agacagagtt tcactcttgt tgcccaggct ggagtgtaat ggcgctatct cacagcaacc 151740
tctgcctccc aggttcaagc cattctcctg cctcagccgc cgtagtagct gggattacag 151800
gcatgcacca ccacacccag ctaattttgt atttttagta gagacggggt ttctccatgt 151860
tggtcaggct ggtctcgagc tccggacctc aggtgatctg cccgcctcgg cctcccaaag 151920
tgctgggatt acaggcgtga gccctgtaat cccggtgccc agccagttct cttctttttt 151980
taagcctctt cctttagaag gaagaaaaca taatctatat gctggctggc tctacacagc 152040
agcaggcatc acaaggggtt tttgtctgct tttgcttctg aaggtgaacc cactatctat 152100
ttagagaggc caggtcaggg tgtgtgtgtg tgtatgtgtc tttttcccta gggtgggtat 152160
tttcattcan nnnnnnnnnn nnnnnnnnnt gttgttatga tcccctgttg tagaagaagc 152220
agtgaagttt tagagagatg aagtgctttg tctgatgtca cacaactgat agatgacaga 152280
atcacatatg tattcagggg tctggtccaa agcctagaag aaggtgagcc atgcatggcc 152340
cgatggcaca gagcatgagc tctggttgca caatcgtggg aggttactta atcttgagaa 152400
acctcagttt tcttatctgt aaaatgggac agtatgtatc ccaaagggcc atttgacca 152460
tttatcaaag tcagtttctc ctccgtatgg agcggagaac agtgtccagc atgcagtacg 152520
tgctctgatg atcttggctg taatgaccat cctggttagg gtggatttgg ccaaaagctg 152580
gagtaactgg tagcttcctg atctgagagg tgtcagtggg tggtgtggcc caaagattga 152640
gaatcttgtc aggggtggcc acgtgagggt ggaagtggtt tgagtgagga atggtcttga 152700
ggtaagagtt ggacaaacag tgtcttctct ctctctctct tttttttttt tttttttttt 152760
tttacttttt tttgagatgg agttttgctc tttttgccca ggccagagtg caatggcgcg 152820
atcttggctc accacaacct ccgcgtccca ggttcaagtg attctcttgc ctcagcctcc 152880
cgagtagctg ggattacaga catgcaccac ccacgcccgg ctaattttgt atttttagga 152940
gagatggggt ttctccatgt tggtcgggct ggtctccaac tcccgacctc aggtgttctg 153000
cctgccttgg cctcccaaag tgctgggatt ataggcgtga gccaccccac ctggtcgaca 153060
gtgtcttctc taagcagaac tgtagccctg aagcgcccag gctagaactc tctattgaat 153120
gaggatcact ccattgtgcc agctgctctc atattgcttt gatggccttt ctcagaaagg 153180
agaaaggtgg tgctgggtgt ggctttggct tcctatggcc gccccaatgc tggtctgaca 153240
ctttctgaat ctggtttgtg gttggatctg tgcatcctac cgctccactc catgtcctgg 153300
ccttggagaa atcatttccc tcccgccgtg attgttcacc ttcaaaggtc aacaaaggca 153360
gtaggcactg ctgagctctt gcagccttca gggctgagct gcttcctctc ctgcttccag 153420
tgaggggttg cttgagggtg cctttaggaa ggaacctcac ccctaatgta acttgagcag 153480
gtctttgctc taaactcttc gctgggaaca taccctcccg tgggacttgg catggcaccc 153540
acctctgcag agccacctgt gccacagaat aacctcttct ccctagacac actgtgtgtc 153600
cttggttgga ttctctagat gggagggacc ggctgaagca taactgtgta tgttcacata 153660
tgtgccttgg aagagtttta tgcaggaaag gatttgtaaa tcagaaaaat gaatgctggt 153720
atctaatttc ctgtccccgt tttaagtcca gaccaagtca cctaacccct cgggcttgca 153780
gcccgcatca tccactgggt gattaccttg agttatctgg ttcagtccct ttgctgtggg 153840
caccctcttt acctctacaa gtaaactgca attaatctga cagcaggggc aagggaactg 153900
acgagtcttg ggtgcaaatc ttgagatgct ttggcacatt atctaattca ttgtttttct 153960
tttctttttt cttttctttt cttttttttt tagacaggct ggagtgcagt ggcacgatct 154020
tggctcactg caacctctgc ctcctgggtt caagtgattt ttgtgcctct agcctcctga 154080
gtagctggga ttccaggtgt gtgccatcac gcccagctaa tttttgtatt tttagtagag 154140
acagggtttt gccatgtagg tcaggctagt ctagaagtct ggccttaagt gatccacccct 154200
ccttgggcag ggtgccacct gcctgaaaga aaatgcccac cctagggaaa aagacacaca 154260
cacacacaca cacacacaca cacacagaga gagagagaga gagagagaga gagagaccaa 154320
cgtggcctct ctaaataggt agtgggttaa ccttcagaag caaaggcaga caaaaaccac 154380
ttgtgatacc tgctgctgcg tagagccagc cagcatatag attatgcttt cttccttctg 154440
aaggaagact ctaggattac aggagtgagc ctggccccaa ttcaattcaa tctttatggg 154500
catcgaacaa gggcattatt tattacccat ggtttacaga agtcaaaccc aagtctcaga 154560
gagggaagac attttttagt ctcactgcga aagtgacaga gctagaattt agtctctgtc 154620
acgctaaatc gtgctacatg ctccgtctag ttcagagcct gtgtggaggt cgacactgga 154680
gtccctcagt tttcctcatc aggctcctgg tctgattctt ccaagatcag tctctttccc 154740
tcagcggaag ccagctggga atcactggcc ttgtaacctc ttcaggcgac ctccttggga 154800
tgttcacaag gagcatagac atctggagaa tgatactgtt ttttcctagg gctgggatgg 154860

gggtggtatc cctcacatca ttccagaaac agcgccagcc ctgcttttat cagcgggggc 154920
cacaaagagg cgacaagccc tgatcaaatg tgagcgagat tgacaatctc agcactttcc 154980
agggaaagga tgccgtccca catggcttca ggcctccaag gaagccccat cctgagacag 155040
agagacccca aatcacagtg tgtttccttt tggccagaga tgaggcttac tcatctcaga 155100
cagctgtgtc cacggcatgt tcctcaagga cagctgctcc agatgaaaag aagagatgct 155160
cccagagcct tgaggggctg ctgataggaa cccaaggcct caccctgggg ctgtatcctg 155220
tggcttcaaa aatatatgta tttgaaagct tctagcaaaa cagattacac aagaacgcaa 155280
atgtaagatc cctttgggat tctaactttt tttcttaagg tgtttaaaga aaaatgacac 155340
ccttaaaaag aaatgccatt tgttttcaaa gctgcagcac cagaggacat ggaagagaaa 155400
ggctgaagtg tccctgaaaa tgagtgaaga ttccagtctg tttttactaa tgtccaaaat 155460
cttaggttta ttgcctttgc gttcctctgc cccagggtct ctgtgtggca ggcgagtggg 155520
tctcgttcga tggtgcagtc atcgtgtggc ggaatgcatg ctcacacagg gaagttggtt 155580
gagcgtctcc tttgagcagt ccttcctctg gtgaggtgca caggcgggca tcatgcccat 155640
ttcatagatg aggaaactga ggctgagagg gatgctgcaa tttaggactt ctgggtgata 155700
tcacagaagc ctcattctct tttgtggcat gtaccttctc tatttgggaa gttaaattgg 155760
actatgggga ttggaccaag gggtcttctt gcttccgctt tctctaggcc aaggctttgc 155820
agttatggag gaagaaagag ctggggtctg tcttggcaac cctgtgactc ttctccccta 155880
tcctcacctg ccttctcact tgaccaacat attttaaggg ctccatgtac aaaggtaggg 155940
ctgtggccct ttcccaacca gcaccttggc cctgtaccct gatatgggat ggttaaggca 156000
cacaggcttc atggatgggg gcctacgacc gcagatggag cagaggtgga cagggtatgg 156060
tccggcaggt ggaggtgctg gagaaaagcc gcgttaggca gcacgggagc caggtgcggg 156120
gtctctcaaa gcctcgagtc cctggcagtg ctgtgctctg tgaggactgc ttttgactgc 156180
agggcactga cactcatacc ttttgcttct gcagctgacc atactccctg gagccttctc 156240
ccgaggtgcg cgggtgacct tggcacatac agccatcatg atggtacttt aagtggaggc 156300
tgaatcatct cccctttgag ctgcttggca cgtggctccc ttggtgttcc ccttttactg 156360
ccaggacact gagatttgga gaggtaagtg gcttacctga ggccatgtgc taacagagaa 156420
gatgaagaga tgattgaaac aggcctaaga ccagacctaa gggtctgtac attttccaca 156480
tactttccat atctttagag gcctgaccaa agcagatctt ttcctttctt ctaggtaagt 156540
ccaaaggcac ctgcctgctg ggcccactgt tttctaactt tcctaacttt ctgatccctt 156600
ggaggtgata atcaaatatt ctagtctgag gcattgggat acatggtgct aggttctgag 156660
actctgcgtc aggcctgaac cctgcatttt gtggaggtgg gtgggagaat gttcccctgg 156720
ggaacatgcc tagacacggg ggacaacagt tgccctcatg gggaggtacc tgtttactcg 156780
ctgttatggg accgctttca caaaaccact gcaggtgagt gagttcctgc tgaatatcag 156840
gcctggtgtc tctagactca ttatttcccc cacccaaccc ctatgttagt tcatctcgag 156900
ccacattttt attgccataa tccaggcctg gacaggccaa gatcttttaa caattttaat 156960
tactgaaaat aataactgca tttttttaa agcccaactt ttggtagagt cagcccaaaa 157020
tacagtcttt gtgttgccat ctgggaactg gatttggaat tgttcttcca tgagactgca 157080
gagcagaacg gcagggccag aggtcccacg agctggtcag acccggttct gctccttgct 157140
ggctgagtga ccttgggcat tgtgattcat attctcatct gccttcaagt gtggttacga 157200
atgcttttta tcctgcttat caatttctat gattgccata aaatattaac acaaatttag 157260
catttatata acataatata ttaatgcata gtttttttgt gggggggggc ggtggagggg 157320
cagtgttgag acagagtctt gctctgtcac ccaggctgga gtgcatggtg tgatcctggc 157380
ccactgaacc ttgacctcca gggctcaagg ggtcctccca cctcagcctc cagagtagct 157440
gggacttcag gcatgagcta tcacacctgg ctaatttttt gtatttttg tagagacgag 157500
gtctcactat cttgcccagg caggcctgga actcctggac tcaagcgatc ctcccacctc 157560
ggccttctaa agtgctgtga ttacaggtgt gagccactat gtccggctca cgtccagtta 157620
tctagggcag aggtcagaca tggatctctc caggctaaca tcaaggaacc tgtaggctgc 157680
cttccattct ggagactgca ggggagagtc tgttccttgc tcattctgga tgttggcaga 157740
atttgtttcc ttgaggttgt agggctgaga tcccgttttc ttgctggctg tccagatgca 157800
ttgagcttct ggaagcctcc tcgctaggct tgtggcctcc tctctccatt ttcaagtcca 157860
gcaatggtgg gtccagttcc ttttaggatt taaatctttt ctccctcttt cttttcacct 157920
ctgtgatctc agctagaaaa gattatctaa ttttaaggat tcatgtgatt ggactggggc 157980
caccctgata atccagaata atccctactt caaggtcctt aggtttaatc acatctagga 158040
agttctattg ccatgggagg caacatattc acaggttcca ggaaattccg acatgggcat 158100
ctctttgatt ggaatgaggg gtggcaggta ttctgacaca gtacctcaga agggttgctg 158160
tgatgatgaa gtgaatggag acacgtaaag cgcttaaaat attatgtaga gccatgtgaa 158220
tttggcagca ttgaattgtt gacctataaa aggtcaactt aatagtatct ggtctatagt 158280
aaatcttcag taaatgtttg ctgtcattac tctagtggta gaaatgtcat gctttattta 158340
gccagtttgc tattgttggg cattactttt taaagagtta ttattttgtt attatagtat 158400
atatgtgaac accgtgatca tcatgccttt gcaaatttta ttctgattta gggcaatgca 158460
tgtctttcca ggccacactc agtctcctgc tctggtgcca gtgtctctgt ggcctgaggg 158520
gcccctcttt cttgcagagc ccctcatcca gcaccagctc agctctgggc catgtgtgtg 158580
gggtgggact tttctctttt gggaagtggg ccatgcctct ccttcctgag accatcccct 158640
ttggcttcct cggggccctt ggctctttga accagcctgc agggcaccac acctgtgggc 158700
ccatttttgt caggaaacga ctcccttccc aagggcaccc tgagtcccag gtccccaggc 158760
ccacagatgt tgggccctca gagtgtgatc ccatgtcccc agctctgtaa ctccctgcat 158820
gtggctctta agactcacaa gcccatgttt gccttggcca gacactttcg cctgtcagtt 158880
ctgccagttg ggaagtgtca tccccactgt gtgaatgtgg aagggataat gaagaatttc 158940

cacggcttgc ctagggtcgt tgagcaatgt ggaagggatc atggagaatt tccatggctt 159000
gcctaaggtc atcaagctgg catctcaacc caggttcatc tgcctgtgat gacaggaaag 159060
aaatcaggtg tgattctgtt cttgttgacc catagccacc tctctcatta aggcttctcc 159120
tgcaggcaac tacacagcca gtggtgggaa agcacctaat ttacatcttc atgcattgaa 159180
actgatgtca ctcacctctc cccettgacc tggaagagcc cctgaaggct gccatagggg 159240
aaatgataac ccattctaca gctgctgaaa ctgaggccca gggagtgtcc agagctttct 159300
gaggtccacc atgtactgtt gaccaggatg agtgatagcc acctggtgac aatgttgaag 159360
gttctcattc agtagagctg ggttcaattc tgacttgcct gaccagctgg tgcctcattt 159420
tgctctaagt attggagcct ccagctttat tttctttcag tgggaaagac tgtgagattc 159480
agtccctcct tgaagctgcg catcgactct cctgtcctgg gtactcttta ggtagtggct 159540
gttctgaagt ggctttggaa accctggaat gaaactgtca tgtccaatgc tgagatttgt 159600
ataaactgtc attttaactg tgattattat tccttaggat ttgagaaata aaatctgagg 159660
agggagcagg acctgcctca gggcctcctc tccaagttga gtgacccatc ttcccaggtc 159720
tccttttgtt ctgtcttgag cctctctcct gaggtcctga aagaagagaa tgtgctgttg 159780
taggggggcct ggaaatgtcc catggaccat ctcgtgactc agtaagatga tgtgtgtcct 159840
ctggggacag aaggaggtcc tgcacagagt gttcctgccc gttgacgccc tccccagatc 159900
ctaacgcgaa gtctgacaca tagtagcctc ccggtaatct taataagcaa aaggacacat 159960
ttctcaagtg atcttagcac catttcatgc aatcaattcc agcccagccc tttattctgg 160020
ccagccctgg tgggtccatt agctgcaaaa attagtcgtg ctcagtttta atgcaaatga 160080
ctaagtatta ctttattgaa ttgattaata aagaagctct ctttgtatga tatcaagacc 160140
atgaagaaat tgcaaagaca taggtagggt ctacagcaga gtttctcagc cttggcgctg 160200
ttcatatttt gggtcagatg atttgttgtg ggggctgtcc tatgtattgt aggatgttgc 160260
tcggcatctc tggcctctac ccactggatg ccagtagctc tgtcagtcgc aaccccagct 160320
tccagttggg acaacctaaa gtgtttccag acattgccac atgttctttt gtggtggtgt 160380
tggtggtggt gcgtacatat gtgtgtgtaa aaatctccct ttgttgagaa ccactaatct 160440
agagaccacc taattagggc tgacgtgggc tcttccacag agccttctcc ttaaagacag 160500
ataggttgat ggacagttgg atttagggaa aagaaaactg agttgagggt ttaaagtgaa 160560
tttcatcccc aatgtggaag atgagcgttc cgttcctggc tgtaggatgt ggagcatgtt 160620
actccacatg tgggagaaaa aaatcatccc atctacttca ggacttttgt gtgattaaag 160680
aggcaagata gtctagtgct tttcattcca tgcgcactca ataaaaaata ggggttatat 160740
ttatgaatag taacaaaaag cctcttaagg aaaattattt tgggcccttg ttcaaggttc 160800
agttgaccat ttatttttaa agagatgggg gtggcccagg ctggagtaga gtggtgccat 160860
cctagttcac tgcatcctca aactcctggg ctcaatcgat cctcctgctt caacatccca 160920
aagtgctagt attataggca tgagccatta cacctggccc atttgactat tttttctgct 160980
tgattgtaga gaagggaatg taagtcatga agatctaaac tataaaacct gcgtggctta 161040
gaggctgagc atggattgtg atttaaggaa gccccaggtt caatttctgg ttctgccact 161100
tgctagccat ggtgagacct tgcccagctt tcatactttc aggttctttg tctgtaaaat 161160
gggaacaata agagtactga cctcaccggg ctgttgtgct actggctgtg ctgcctgaat 161220
gggccctcct ttatttgaat ccctctgcca cagttgtccc attttttttt tttttaaat 161280
ggtgacccaa gttccattat ggacgatcag gcattcaggg gaaggaggga tccaaagcag 161340
agacaaaagc agccagccag ctcatcccct ctgcagctga taccgaggag gaggatgcca 161400
gtgggaagaa gcgtggccat atggctggca ggatgcctca ctggtaacag tcagcccggc 161460
ccctttgaag tggttttaag accaatttgg taacttgatt tggaaagtgg tatcgatgac 161520
tccgagggcg caggctgctc attagctgct gtctgggccc ggcaaattgg tttaagtgga 161580
atgaaattgg ttccttccct ccccgtgctc atggctgagg tccaggcccg attgtaatgg 161640
gaacctctcc tgggcaaagg acatagaaaa gctttccggg gagtctgacc ctcttttttc 161700
atatgtgaat tctactttcc cctgggggtc aaggcaatag gctgataaaa cataaaaatg 161760
aacctgaagt gggtggggtg agtgtgtgtg tgggaagttg gatcagagga gaatgaacaa 161820
tgaacagtgg ccgagtgttt ttcaagggcc agttaaccat tcccctgttg agacactgtg 161880
cttgatgcat ttttttcttt ttctttcttt cttttttatt ctcccatatt acaaacactg 161940
ctgggaggaa catccgcaca tgctttcttg gtcacttatg tgagagctca tccaggattt 162000
ataccctgag gcgacttcac tgccgtggag gctaaggaac attttctgtt tcataagacg 162060
cgatcacatt gccctccaga acaatcatgc cagtttacac tcccactgac tgtacacaag 162120
agccctgttt tcccccatct ggtcacccac atttgatagg tcagaatttt gctttttgcc 162180
agtctcatgg gggtgatgcc atttctggta gttcccctga ttgccagggg gatgagcagc 162240
ctgccctcaa ctgccccatg ctgaaggccc tccaaaggca gcatgaatcc tcacagttcc 162300
cagcctgtgc cctgacaacc taggaggaca cgctggtctg gtccattgct tatctgaaac 162360
gattcccttt ccctgttcct tttgttgcct tgagtgtcag ttttggacct cccactgagc 162420
agttttttct gtcacgtagc catagacagg actgagttct ttggaaaata atcacatccc 162480
actgttgttc ccattcgttc attttttcct tcaactaacc aacattattc agcactttac 162540
tcagcacagg atgtggaacc gaaggcctgg cttttgggac actcaccgtc cccagtcagc 162600
tctgtgtcct agggagagac ccatgagctc tctgggcctc cttttgtttc tctgtaataa 162660
aggactaaag ctaccagcct cgctgcgggt tggaaaaaag aaagagttag tgtaacacat 162720
gtgaatgtgt acgaaggggt ttagagacag acaaaacatt gcaccaaggc agggctcgct 162780
ggagtgcaga cccaggaaaa acactgcagg ccaaattgct tctggtctgt cagggtacca 162840
gccacacatc caaggcaggg aacttagggc gattggcctt tctgctcatg cttactctcc 162900
caagcttgtt gggttattat catgcacaag ccattcaatc gattgtattg ggtgcctaat 162960
atatcagaca ctcttaggtg ctagggatgt aagagcaagc aaaacagaca tggttctgcc 163020

cttgtgggct gtatagtcta gtggaggaag ggggaagagg ttaagtattt gtaggaaggt 163080
gaaccccatg ttaagaaagc atcttccatc tttcaaatgt ttccaagtag ctggttattc 163140
atgatagcga ggaaatggtg gtcatctcca ttgctctggt aactagtttg tgtctgagaa 163200
agtgctgatt tccaagaaaa ggaaagagac cacggatgaa tgatagcaag actattaaac 163260
aggaatacaa agaagagctc acatttattg agcacctact atatgtgctg gaccctcttg 163320
taagagcttc atacaaatta ggcagacagg caatattatt atccccattt gatctacaag 163380
gaaactgagt acaaagtgat agcttttctg cagagcttgt gcacaggggc caaagtttgg 163440
gaacaatgcc tttctttaat ttgaaagtat ctgttgttgg tgtcacttgt tggaattcct 163500
agaatcccag aaaagtaagt ttaagttttc ttctgccagc atcggtcagc ctacctttga 163560
cccaaggtca cgactccctc cctcctccct tcccttccct gctctgacat tcttaccttg 163620
tttttgagtt ccttattccc tagcttcctc caagcagcag gctctgggaa cagtcccgtt 163680
catttatata acaaatattt attgaaatgc agacttgttg ttttcataat acctgtgtac 163740
aagttaaacc cgtggtcact tgttcatgtg cctcttgttt atgacaagag gtggtccctt 163800
gttgaaagcc tgtcacatgg caggtactca gtcttttctt tccttctaat aagtaggtga 163860
gtattttcct tggtttgcac acgaggaaat ggaggctcag agagcttagg tagcttactc 163920
aaagccacac agctgggtta aatgggttca gcaccatgaa aatctgacct ttaatgtctg 163980
cactcttatt ctctggctta ttgggaaaga attggacagg gatggtaaat ggagttgatg 164040
gaggcaaaga gataattact gttgctcaaa ggtggatcga gagagtttgg gcaaaaacgg 164100
aaccacgaac aagccgttga aagtcccctc atcctggacc tgtggtctgt cctttgtaac 164160
tgctcttttt ttggcattcc ctctgtctcc tctgcccatc ccaatgccag gcacactgag 164220
cctgtaggga tttacaaggt gggcctcagg tttctcccct cccaggtggg cataggaatc 164280
actgcttagc ttagatttgg cagaatgaaa gagtcaggca gagtctcacc cggccctgtt 164340
tcttagctgc tttgacacct ggggcaggtt tttggacctc tctggacatg gccagctgtg 164400
ttgtgactgc ccaccagagg ctgggtctgt gaatggaaga ctgaagaaaa ggcgatgatc 164460
acagtccagg gacaaggttt ctgaatcttg tgaaatagtt acaaatacta tgaaacagga 164520
atacaaagaa aagctcacat ttattaagca cttactttgt gctataccct cttgtaagag 164580
ccttgtacaa attaggcaga cagacaatat tattatcccc attttaccta taaggaaact 164640
gagtgcaaag tgacagcttt tctgcagagc ctgtgcacag ggtcaaagt ttgggaacac 164700
attgcctttc tttaatctga aactatctgc tgttggtctc atttgttgaa attcctagaa 164760
tcccagagaa gtaagtttta gctttcttct gccagccccg gtcagcctgc ctttgaccca 164820
aggccatagt aagttccaga gtgacttccc gtctgtttta ttatccctga caaaggata 164880
cagtattgtg cggtggctgt ggatggcgtg tagtagtgtt ttccttctct tgaaaagcag 164940
ctgctgagtg gtggagcaca gaggtattcc ctgagcccca gcttgtgccc atggtaggtc 165000
cagggcgttt gggggaagga gaccgactct aggtggtccc gtctggtttc tcaggggaga 165060
aaattcatag atccatctcc tagctgtcat ccaattctgt gtagaagggg aaagccaagg 165120
ttgccctcag attttgatct tggttcactc tgatttctct atgccagact agtagagctt 165180
cagggaagct tggtgactat gactggatga attctgtccc ccttaaaacc ataggttgaa 165240
gtcctactca cccatacctc agaacgtgac tgtgttcgga aatagggtct ctagagaggt 165300
acagttaact gagctcacgt ggcggggctc caatatggtc agactggtgg gtgtccttat 165360
cagaagaaga gattaagatg acaaaggagg agtgaagacc acgtgtagac acagggagga 165420
ggtggccatc tacaagccca ggagagaggc ctcagaagaa acaactctga ccacaccttg 165480
atcctggact tctggctttc agaattgtga ggaaataaac ctctgttgtc tgagcaaccc 165540
agcctgtgaa aatttgttgt agcagccctg gcggatcaat acagtgacca tgagcaagtc 165600
actcaccctc tctgaacccc cttggtctca gctctaaaaa gaagcctgag aatttctgcc 165660
tcacaggctg tggtaagagg catcagaaat agccgacttt catcggaggc tcactgcgag 165720
ccagggcctg tttaagcctt ttccatgtct gaacacactt catcttcatg tgtagccttt 165780
ggctggggag gggagatact attgttccca ttttacaggt gaggagagtg gagcacagag 165840
ctgttaatta acctgtccca tgttagtcag caaggaaggg gctggtccat atgtggaact 165900
tcgtagttgt actctggtgc ccacacacca gatcactatg ccgtcccagc tgttgttaca 165960
ataagactat gtaggtgaag ggcccaccca gtagtgcctg gcctatggga cacattgaag 166020
accagccccc tgtgaccaga acctcccatt tgctgcattg agtttggaat gtttgaactc 166080
caggagctgt gtgtgtgtgt gtgtgtgtgt gtgtgtgtgt gtgtgtgtgt gtgtatctgt 166140
ctgtctccct ctctctcctc tctcccctac cttccttttc ttccctcctg tttttgggag 166200
ggagctcagc ttcccttcac tgccactgcc catcagcatc ctctccctat tcagccaccc 166260
ccacccccca tccctggtat taattgactt tggctcctgt cctggagctc agcctggcac 166320
ccagacctgc cgtgctatac taacacctct cacatggtta aaccaatggg ggtggcattt 166380
agcagatgac gctgaaaccc gtgaatctgc cctttctcac tgggccctca ctgctctaga 166440
gtccccgcct cttttacgaa gcggctgtca tggttgatgg gtgagtgtca ggccccaccc 166500
agggacaggg aggcccagtg ccggctgtgg agttacccag accttacggt aaaaacgcat 166560
caccctgaat gggggccagg ccaggcatcg gcccaagccg gccctgccca gcggctggat 166620
tctgactcgc tgtgccttag tcacaggcca gagactcttc tgcttccagc tgaggctgtc 166680
ccctgccttc cctggcggcc cgggttcctg gaaaagctgt gtgccagagg acgggagttt 166740
ccctccaact taagccatta gaaagctcat ttattgagca cttactaagt gtcagagact 166800
gtgctaggca ctttacatga atcttctcat ttaatccttt taataaactt ccggggcaca 166860
tgctcttcta atctcccttt tactgatgat gcaggcttga cagtgggaat gctaaccgca 166920
gaaagcagct cacctcactt gggtgctact gtgtgccaag tcctggggtt agcacccaat 166980
agccccgtgg cggcatgctg ttattactct ccctgcatag gagtgggagg ctgagacctg 167040
ggtgtttctg ggtgtcccag ctcctgtatg ccatctgcct gtccagccct tagccacaaa 167100

gctgttgtgc catgtgggga gatgcggcca gtttcccagg tgaggctggt cctgcttcaa 167160
gcg 167163

<210> 32
<211> 1537
<212> DNA
<213> Homo sapiens

<400> 32
agcacatggg cccgcgggcc gggcgggctc ggggcggccg ggacgaggag gggcgacgac 60
gagctgcgag caaagatgtg ccccgggacc cccggcacct tccagtggat ttccttgcgg 120
aaaggatgtt ggcggtccct gtgacctgtg gagacacggc cagatctgcc ctccacaggc 180
ctgatctttt ggccagaagg agattaaaaa gatgcccctc aagatggctg tgcctgtcag 240
ctgcatggag cttcgttcaa gtattttctg agcctgatgg atttacagtg atcttcagtg 300
gtctggggaa taacgctggt ggaaccatgc actggaatga cacacgcccg gcacatttca 360
ggatactaaa agtggtttta agggaggctg tggctgaatg cctcatggat tcttacagct 420
tggatgtcca tgggggacga aggactgcag ctggctgaga gggttgagat ctctgtttac 480
ttagatctct gccaacttcc tttgggtctc cctatggaat gtaagacccc gactcttcct 540
ggtgaagcat ctgatgcacg ttccatccgg cgctcagctg ggcttgagct gaccatactc 600
cctggagcct tctcccgagg tgcgcgggtg accttggcac atacagccat catgatggta 660
ctttaagtgg aggctgaatc atctcccctt tgagctgctt ggcacgtggc tcccttggtg 720
ttcccctttt actgccagga cactgagatt tggagaggta agtggcttac ctgaggccat 780
gtgctaacag agaagatgaa gagatgattg aaacaggcct aagaccagac ctaagggtct 840
gtacattttc cacatacttt ccatatcttt agaggcctga ccaaagcaga tcttttcctt 900
tcttctaggt aagtccaaag gcacctgcct gctgggccca ctgttttcta actttcctaa 960
ctttctgatc ccttggaggt gataatcaaa tattctagtc tgaggcattg ggatacatgg 1020
tgctaggttc tgagactctg cgtcaggcct gaaccctgca ttttgtggag gtgggtggga 1080
gaatgttccc ctggggaaca tgcctagaca cgggggacaa cagttgccct catggggagg 1140
tacctgttta ctcgctgtta tgggaccgct ttcacaaaac cactgcaggt gagtgagttc 1200
ctgctgaata tcaggcctgg tgtctctaga ctcattattt cccccaccca acccctatgt 1260
tagttcatct cgagccacat ttttattgcc ataatccagg cctggacagg ccaagatctt 1320
ttaacaattt taattactga aaataataac tgcatttttt ttaaagccca acttttggta 1380
gagtcagccc aaaatacagt ctttgtgttg ccatctggga actggatttg gaattgttct 1440
tccatgagac tgcagagcag aacggcaggg ccagaggtcc cacgagctgg tcagacccgg 1500
ttctgctcct tgctggctga gtgaccttgg gcattgt 1537

<210> 33
<211> 150
<212> PRT
<213> Homo sapiens

<400> 33
Met Gly Pro Arg Ala Gly Arg Ala Arg Gly Gly Arg Asp Glu Glu Gly
1 5 10 15
Arg Arg Arg Ala Ala Ser Lys Asp Val Pro Arg Asp Pro Arg His Leu
20 25 30
Pro Val Asp Phe Leu Ala Glu Arg Met Leu Ala Val Pro Val Thr Cys
35 40 45
Gly Asp Thr Ala Arg Ser Ala Leu His Arg Pro Asp Leu Leu Ala Arg
50 55 60
Arg Arg Leu Lys Arg Cys Pro Ser Arg Trp Leu Cys Leu Ser Ala Ala
65 70 75 80
Trp Ser Phe Val Gln Val Phe Ser Glu Pro Asp Gly Phe Thr Val Ile
85 90 95
Phe Ser Gly Leu Gly Asn Asn Ala Gly Gly Thr Met His Trp Asn Asp
100 105 110
Thr Arg Pro Ala His Phe Arg Ile Leu Lys Val Val Leu Arg Glu Ala
115 120 125
Val Ala Glu Cys Leu Met Asp Ser Tyr Ser Leu Asp Val His Gly Gly
130 135 140
Arg Arg Thr Ala Ala Gly
145 150

<210> 34
<211> 5

```
<212> PRT
<213> Unknown

<220>
<223> Cytokine receptor extracellular motif found in
      many species

<221> VARIANT
<222> 3
<223> Xaa = any amino acid

<400> 34
Trp Ser Xaa Trp Ser
 1              5
```